(19) Europäisches Patentamt
European Patent Office
Office européen des brevets

(11) EP 4 419 525 B1

# (12) EUROPEAN PATENT SPECIFICATION

(45) Date of publication and mention of the grant of the patent:
**23.07.2025 Bulletin 2025/30**

(21) Application number: **22797828.5**

(22) Date of filing: **21.10.2022**

(51) International Patent Classification (IPC):
***C07D 487/04*** *(2006.01)* ***A61P 35/00*** *(2006.01)*
***A61K 31/407*** *(2006.01)*

(52) Cooperative Patent Classification (CPC):
**C07D 487/04; A61P 35/00**

(86) International application number:
**PCT/GB2022/052687**

(87) International publication number:
**WO 2023/067353 (27.04.2023 Gazette 2023/17)**

## (54) HETEROCYCLIC COMPOUNDS FOR USE IN THE TREATMENT OF CANCER

HETEROCYCLISCHE VERBINDUNGEN ZUR VERWENDUNG BEI DER BEHANDLUNG VON KREBS

COMPOSÉS HÉTÉROCYCLIQUES À UTILISER DANS LE TRAITEMENT DU CANCER

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **21.10.2021 GB 202115158**

(43) Date of publication of application:
**28.08.2024 Bulletin 2024/35**

(73) Proprietor: **Artios Pharma Limited**
**Cambridge CB22 3AT (GB)**

(72) Inventors:
- **DAVIS, Owen**
  **Cambridge CB22 3AT (GB)**
- **HEALD, Robert**
  **Cambridge CB22 3AT (GB)**
- **STOCKLEY, Martin**
  **Cambridge CB22 3AT (GB)**
- **NISSINK, Johannes Wilhelmus Maria**
  **Cambridge CB22 3AT (GB)**
- **FINCH, Harry**
  **Cambridge CB22 3AT (GB)**
- **MANN, Sam**
  **Cambridge CB22 3AT (GB)**

(74) Representative: **Sagittarius IP**
**Marlow International**
**Parkway**
**Marlow SL7 1YL (GB)**

(56) References cited:
**WO-A1-2021/028643**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### FIELD OF THE INVENTION

The invention relates to heterocyclic derivatives and such compounds for use in the treatment and prophylaxis of cancer, and to compositions containing said derivatives and processes for their preparation.

### BACKGROUND OF THE INVENTION

Robust repair of DNA double-strand breaks (DSBs) is essential for the maintenance of genome stability and cell viability. DSBs can be repaired by one of three main pathways: homologous recombination (HR), non-homologous end-joining (NHEJ) and alternative NHEJ (alt-NHEJ). Microhomology-mediated end-joining (MMEJ) is the most well characterised alt-NHEJ mechanism. HR-mediated repair is a high-fidelity mechanism essential for accurate error-free repair, preventing cancer-predisposing genomic stability. Conversely, NHEJ and MMEJ are error-prone pathways that can leave mutational scars at the site of repair. MMEJ can function parallel to both HR and NHEJ pathways (Truong et al. PNAS 2013, 110 (19), 7720-7725).

The survival of cancer cells, unlike normal cells, is often dependent on the mis-regulation of DNA damage response (DDR) pathways. For example, an increased dependency on one pathway (often mutagenic) to cope with either the inactivation of another one, or the enhanced replication stress resulting from increased proliferation. An aberrant DDR can also sensitise cancer cells to specific types of DNA damage, thus, defective DDR can be exploited to develop targeted cancer therapies. Crucially, cancer cells with impairment or inactivation of HR and NHEJ become hyper-dependent on MMEJ-mediated DNA repair. Genetic, cell biological and biochemical data have identified Polθ (UniProtKB - 075417 (DPOLQ_HUMAN) as the key protein in MMEJ (Kent et al. Nature Structural & Molecular Biology (2015), 22(3), 230-237, Mateos-Gomez et al. Nature (2015), 518(7538), 254-257). Polθ is multifunctional enzyme, which comprises an N-terminal helicase domain (SF2 HEL308-type) and a C-terminal low-fidelity DNA polymerase domain (A-type) (Wood & Doublié DNA Repair (2016), 44, 22-32). Both domains have been shown to have concerted mechanistic functions in MMEJ. The helicase domain mediates the removal of RPA protein from ssDNA ends and stimulates annealing. The polymerase domain extends the ssDNA ends and fills the remaining gaps.

Therapeutic inactivation of Polθ would thus disable the ability of cells to perform MMEJ and provide a novel targeted strategy in an array of defined tumour contexts. Firstly, Polθ has been shown to be essential for the survival of HR-defective (HRD) cells (e.g. synthetic lethal with FA/BRCA-deficiency) and is up-regulated in HRD tumour cell lines (Ceccaldi et al. Nature (2015), 518(7538), 258-262). *In vivo* studies also show that Polθ is significantly over-expressed in subsets of HRD ovarian, uterine and breast cancers with associated poor prognosis (Higgins et al. Oncotarget (2010), 1, 175-184, Lemée et al. PNAS (2010), 107(30), 13390-13395, Ceccaldi *et al.* (2015), *supra*). Importantly, Polθ is largely repressed in normal tissues but has been shown to be upregulated in matched cancer samples thus correlating elevated expression with disease (Kawamura et al. International Journal of Cancer (2004), 109(1), 9-16). Secondly, its suppression or inhibition confers radio-sensitivity in tumour cells. Finally, Polθ inhibition could conceivably prevent the MMEJ-dependent functional reversion of BRCA2 mutations that underlies the emergence of cisplatin and PARPi resistance in tumours.

There is therefore a need to provide effective Polθ inhibitors for the treatment of cancer.

### SUMMARY OF THE INVENTION

According to a first aspect of the invention, there is provided a compound of formula (I):

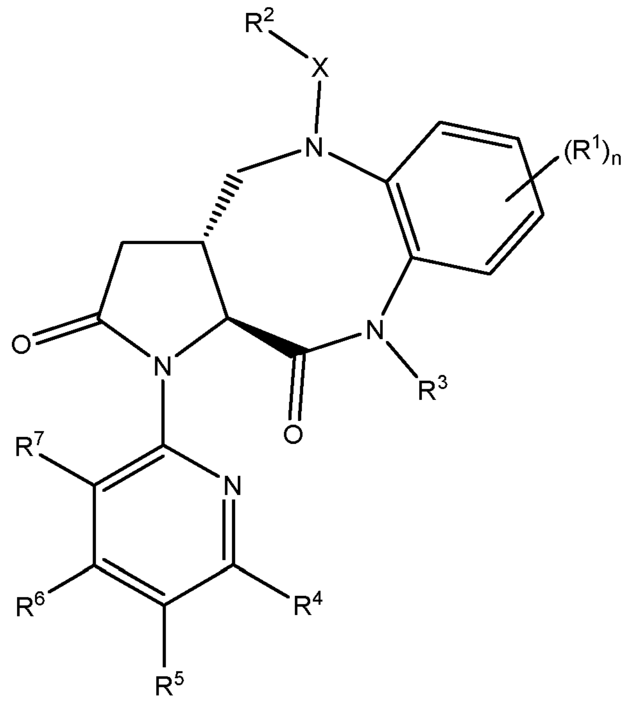

(I)

or a tautomeric or a stereochemically isomeric form, a pharmaceutically acceptable salt or a solvate thereof, wherein:

n represents an integer selected from 0, 1, 2, 3 or 4;
$R^1$ represents $C_{1-6}$ alkyl, $C_{2-6}$ alkenyl, $C_{1-6}$ alkoxy, hydroxy, halogen, halo$C_{1-6}$ alkyl, halo$C_{1-6}$ alkoxy, $C_{3-8}$ cycloalkyl, cyano or $-NR^xR^y$;
X represents a bond or a $C_1$-$C_6$ alkylene group optionally substituted by one or more $C_{1-6}$ alkyl, $NR^x$, O, hydroxy, halogen or CO groups;
$R^2$ represents a -Ring$^A$ or -Ring$^B$-Y-Ring$^C$ group;
or -X-$R^2$ represents a $-C_1$-$C_{12}$ alkylene-$NR^xR^y$ group, wherein said alkylene group may be optionally substituted by one or more $C_{1-6}$ alkyl, $C_{2-6}$ alkenyl, $-NR^x$, O, hydroxy or CO groups; Ring$^A$ represents carbocyclyl, heterocyclyl or heteroaryl which requires a substituent selected from a $-(CH_2)_m$-CO-$C_{1-6}$ alkyl, $-(CH_2)_m$-NHCO-$C_{1-6}$ alkyl, $-(CH_2)_m$-CO-$C_{2-6}$ alkenyl,$-(CH_2)_m$-NHCO-$C_{2-6}$ alkenyl, $-(CH_2)_m$-CO-$C_{2-6}$ alkynyl or $-(CH_2)_m$-NHCO-$C_{2-6}$ alkynyl group, wherein said alkyl, alkenyl or alkynyl group may be optionally substituted by one or more (e.g. 1, 2 or 3) halogen, hydroxy, CO or $-NR^xR^y$ groups and wherein said carbocyclyl, heterocyclyl or heteroaryl groups may be optionally further substituted by one or more (e.g. 1, 2 or 3) substituents selected from halogen, $C_{1-6}$ alkyl, hydroxy, $C_{1-6}$ alkoxy, -CO-$C_{1-6}$ alkyl, oxo, $C_{1-6}$ alkylamino or cyano, wherein said $C_{1-6}$ alkyl group may be optionally substituted by one or more halogen, hydroxy or cyano groups;
m represents an integer selected from 0, 1, 2, 3 or 4;
Ring$^B$ represents carbocyclyl, heterocyclyl or heteroaryl, each of which may be optionally substituted by one or more (e.g. 1, 2 or 3) substituents selected from hydroxy, oxo, $C_{1-6}$ alkyl, $C_{2-6}$ alkenyl, $C_{2-6}$ alkynyl, $C_{1-6}$ alkoxy, halo$C_{1-6}$ alkyl, -CO-$C_{1-6}$ alkyl, cyano or halogen;
Ring$^C$ represents heterocyclyl substituted by one or more (e.g. 1, 2 or 3) substituents selected from halogen, $C_{1-6}$ alkyl, -CO-$C_{1-6}$ alkyl, and/or $-(CH_2)_m$-CO-$C_{2-6}$ alkenyl optionally substituted by an $-NR^xR^y$ group;
Y represents a bond, -O-, -NHCO-, CO, or a $C_1$-$C_6$ alkylene group optionally substituted by one or more $C_{1-6}$ alkyl, $C_{2-6}$ alkenyl, $-NR^x$, O, hydroxy or CO groups;
$R^3$ represents hydrogen or $C_{1-6}$ alkyl;
$R^4$, $R^5$, $R^6$ and $R^7$ independently represent hydrogen, $C_{1-6}$ alkyl, $C_{2-6}$ alkenyl, $C_{1-6}$ alkoxy, halogen, halo$C_{1-6}$ alkyl, halo$C_{1-6}$ alkoxy, $C_{3-8}$ cycloalkyl, cyano or $-NR^xR^y$; and
$R^x$ and $R^y$ independently represent hydrogen, $C_{1-6}$ alkyl, $C_{2-6}$ alkenyl, $C_{2-6}$ alkynyl, $C_{1-6}$ alkoxy, or $R^x$ and $R^y$ together with the nitrogen atom to which they are attached join to form a nitrogen containing heterocyclic ring which may be optionally substituted by one or more $C_{1-6}$ alkyl groups.

**DETAILED DESCRIPTION OF THE INVENTION**

***Definitions***

The term 'halo' or 'halogen' as used herein refers to fluorine, chlorine, bromine or iodine.

The term 'cyano' as used herein refers to a group where a carbon atom is triple bonded to a nitrogen atom.

The term '$C_{1-6}$ alkyl' as used herein as a group or part of a group refers to a linear or branched saturated hydrocarbon group containing from 1 to 6 carbon atoms. Examples of such groups include methyl, ethyl, n-propyl, *iso*-propyl, butyl, *iso*-butyl, *tert-butyl,* pentyl, hexyl and the like.

The term '$C_{2-6}$ alkenyl' as used herein as a group or part of a group refers to a linear or branched unsaturated hydrocarbon group containing from 2 to 6 carbon atoms and at least one double bond. Examples of such groups include ethenyl, propenyl, butenyl, pentenyl, hexenyl and the like.

The term '$C_{2-6}$alkynyl' as used herein as a group or part of a group refers to a linear or branched hydrocarbon group having from 2 to 6 carbon atoms, respectively, and containing a carbon carbon triple bond. Examples of such groups include $C_{3-4}$alkynyl or $C_{3-6}$alkynyl groups such as ethynyl and 2 propynyl (propargyl) groups.

The term '$C_{1-6}$ alkoxy' as used herein as a group or part of a group refers to a $C_{1-6}$ alkyl group which contains an oxygen atom wherein $C_{1-6}$ alkyl is as defined herein. Examples of such groups include methoxy, ethoxy or propoxy.

The term "$C_1$-$C_x$ alkylene" as used herein as a group or part of a group refers to a $-(CH_2)_{1-x}$ group. Examples of such groups include methylene, ethylene, propylene and butylene.

The term 'halo$C_{1-6}$ alkyl' as used herein as a group or part of a group refers to a $C_{1-6}$ alkyl group as defined herein wherein one or more than one hydrogen atom is replaced with a halogen. The term 'halo$C_{1-6}$ alkyl' therefore includes monohalo$C_{1-6}$ alkyl and also polyhalo$C_{1-6}$ alkyl. There may be one, two, three or more hydrogen atoms replaced with a halogen, so the halo$C_{1-6}$ alkyl may have one, two, three or more halogens. Examples of such groups include fluoroethyl, fluoromethyl, trifluoromethyl or trifluoroethyl and the like.

The term "$C_{3-8}$ cycloalkyl" as used herein refers to a saturated monocyclic hydrocarbon ring of 3 to 8 carbon atoms. Examples of such groups include cyclopropyl, cyclobutyl, cyclopentyl, cyclohexyl, cycloheptyl, cyclooctyl and the like.

The term 'oxo' as used herein refers to the group =O.

The term 'hydroxy' as used herein refers to the group -OH. The term 'carbocyclyl' includes aromatic and non-aromatic ring systems comprising solely carbon and hydrogen (i.e. not containing any heteroatoms). Carbocyclyl ring systems have from 5 to 12 ring members, more usually from 5 to 10 ring members. In one embodiment, carbocyclyl includes an aryl ring which refers to carbocyclyl aromatic groups including phenyl, naphthyl, indanyl, indenyl, and tetrahydronaphthyl groups. The term "aryl" embraces polycyclic (e.g. bicyclic) ring systems wherein one or more rings are non-aromatic, provided that at least one ring is aromatic. Examples of polycyclic (e.g. bicyclic) aryl groups containing an aromatic ring and a non-aromatic ring include indanyl groups. In such polycyclic systems, the group may be attached by the aromatic ring, or by a non-aromatic ring. One particular example of an aromatic carbocyclic ring system includes phenyl.

In an alternative embodiment, carbocyclyl includes a non-aromatic carbocyclic group such as cycloalkyl and cycloalkenyl groups as defined herein and spiro and bridged derivatives thereof. Particular examples of non-aromatic carbocyclic ring system include cyclobutyl, cyclohexyl, 2,3-dihydro-1H-inden-2-yl and bicyclo[1.1.1]pentan-1-yl.

The term 'heteroaryl' as used herein refers to a monocyclic or bicyclic aromatic ring system containing for example 3 to 12 ring members. Each ring may contain up to five heteroatoms typically selected from nitrogen, sulfur and oxygen. Particular examples of heteroaryl include imidazolyl, pyrazolyl, triazolyl, pyridinyl, pyrimidinyl, oxazolyl and oxadiazolyl.

The term 'heterocyclyl' as used herein refers to a monocyclic, bicyclic or tricyclic non-aromatic, partially saturated or fully saturated ring system containing for example 3 to 12 ring members. Each ring may contain up to five heteroatoms typically selected from nitrogen, sulfur and oxygen. Particular examples of bicyclic or tricyclic heterocyclyl include monocyclic or bicyclic heterocyclic rings which may be fused to monocyclic carbocyclic and heteroaromatic rings.

Particular examples of 'heterocyclyl' include morpholine, piperidine (*e.g.* piperidin-1-yl, piperidin-2-yl, piperidin-3-yl and piperidin-4-yl), piperidinone, pyrrolidine (*e.g.* pyrrolidin-1-yl, pyrrolidin-2-yl and pyrrolidin-3-yl), pyrrolidone, azetidine, pyran (2H-pyran or 4H-pyran), dihydrothiophene, dihydropyran, dihydrofuran, dihydrothiazole, tetrahydrofuran, tetrahydrothiophene, dioxane, diazepane, tetrahydropyran (*e.g.* tetrahydropyran-4-yl), tetrahydropyridine, imidazoline, imidazolidinone, oxazoline, thiazoline, pyrazolin-2-yl, pyrazolidine, piperazinone, piperazine, hexahydropyrrolo[3,4-*b*][1,4]oxazin-6(2*H*)-yl, octahydro-2*H*-pyrazino[1,2-a]pyrazin-2-yl, octahydro-2H-pyrazino[1,2-a]pyrazin-3-yl, octahydro-5H-pyrrolo[3,4-c]pyridin-5-yl, tetrahydro-1*H*-imidazo[4,5-c]pyridin-1-yl, tetrahydro-3H-imidazo[4,5-*c*]pyridin-3-yl, isoindolin-1-yl, isoindolin-5-yl, tetrahydroisoquinolin-6-yl, tetrahydro-[1,2,4]triazolo[1,5-a]pyrazin-2-yl, tetrahydro-[1,2,4]triazolo[4,3-a]pyrazin-3-yl, tetrahydroimidazo[1,2-a]pyrazin-2-yl, dihydro-5H-pyrrolo[3,4-b]pyrazin-2-yl, dihydro-5H-pyrrolo[3,4-b]pyridin-3-yl, dihydro-5H-pyrrolo[3,4-b]pyridin-2-yl, dihydro-5H-pyrrolo[3,4-b]pyridin-7-yl, dihydro-5H-pyrrolo[3,4-b]pyridin-5-yl, tetrahydropyrrolo[3,4-d]imidazol-2-yl, dihydro-2H-benzo[b][1,4]oxazin-6-yl, dihydro-2H-benzo[b][1,4]oxazin-7-yl, dihydro-4H-pyrrolo[3,4-d]imidazol-1-yl, dihydro-1H-pyrrolo[3,4-c]pyridin-6-yl, dihydro-5H-pyrrolo[3,4-d]pyrimidin-2-yl, dihydro-4H-pyrrolo[3,4-d]thiazol-2-yl, dihydro-4H-pyrrolo[3,4-d]oxazo-2-yl, tetrahydropyrrolo[3,4-c]pyrazol-3-yl, tetrahydroimidazo[1,2-a]pyridin-6-yl, tetrahydroimidazo[1,2-a]pyridin-7-yl, and tetrahydropyrazolo[1,5-a]pyrazin-2-yl.

It will be appreciated that the term 'heterocyclyl" includes reference to spiro and bridged heterocyclic derivatives. Examples of such spiro and bridged heterocyclic derivatives include: hexahydropyrrolo[2,3-c]pyrrolidinyl, diazaspiro[3.3]heptanyl, azaspiro[3.3]heptan-6-yl, diazaspiro[3.4]octanyl, diazaspiro[4.4]nonyl, oxa-azaspiro[3.4]octanyl, oxa-azaspiro[4.4]nonyl, tetrahydrofuro[3,4-c]pyrrolidinyl, oxa-azaspiro[3.3]heptyl, diazaspiro[3.5]nonanyl, diazaspiro[4.4]nonanyl, diazaspiro[4.5]decanyl, diazaspiro[3.5]nonanyl, octahydro-naphthyridinyl, tetrahydropyrazino-oxazinyl, oxadiazaspiro[3.4]octanyl, oxadiazaspiro[3.5]nonanyl, oxadiazaspiro[4.5]decanyl, oxadiazospiro[5.5]undecanyl, triazaspiro[3.5]nonanyl, diazabicyclo[2.2.1]heptan-2-yl, diazabicyclo[3.1.1]heptan-6-yl, diazabicyclo[3.2.1]octan-8-yl, oxabicyclo[2.2.1]heptanyl, oxa-2,8-diazaspiro[3.5]nonan-8-yl, oxa-3,7-diazabicyclo[3.3.1]nonan-3-yl, tetrahydro-1'H-spiro[azetidine-3,4'-pyrrolo[1,2-a]pyrazin]-2'(3'*H*)-yl, tetrahydro-1'*H*-spiro[azetidine-3,6'-pyrazino[2,1-*c*][1,4]oxazin]-8'(7'*H*)-yl, triazaspiro[3.5]nonan-8-yl, spiro[azetidine-3,3'-imidazo[1,2-a]imidazol]-1'(2'H)-yl and spiro[azetidine-3,5'-imidazo[1,2-a]pyrazin]-7'(8'H)-yl).The term 'optionally substituted' as used herein refers to a group which may be substituted or unsubstituted by a substituent as herein defined.

***Embodiments***

According to one particular aspect of the invention which may be mentioned, there is provided a compound of formula (I):

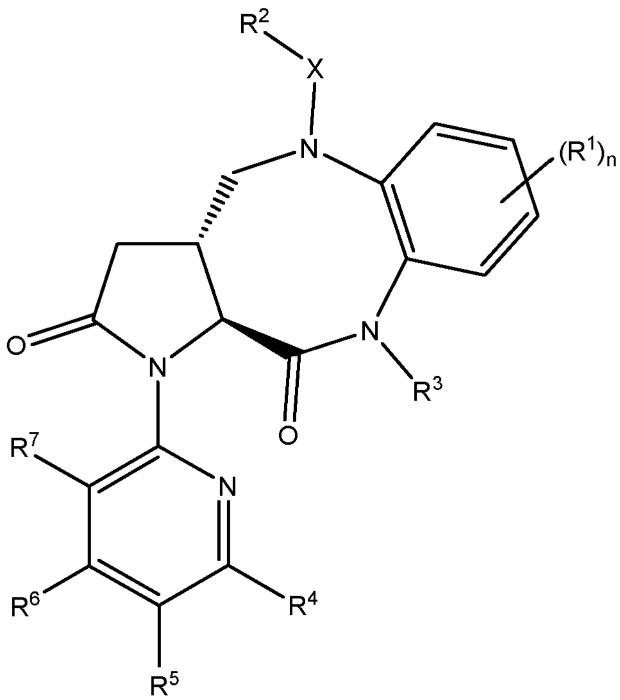

(I)

or a tautomeric or a stereochemically isomeric form, a pharmaceutically acceptable salt or a solvate thereof, wherein:

n represents an integer selected from 0, 1, 2, 3 or 4;
$R^1$ represents $C_{1-6}$ alkyl, $C_{2-6}$ alkenyl, $C_{1-6}$ alkoxy, hydroxy, halogen, halo$C_{1-6}$ alkyl, halo$C_{1-6}$ alkoxy, $C_{3-8}$ cycloalkyl, cyano or $-NR^xR^y$;
X represents a bond or a $C_1$-$C_6$ alkylene group optionally substituted by one or more $C_{1-6}$ alkyl, $NR^x$, O, hydroxy or CO groups;
$R^2$ represents a -Ring$^A$ or -Ring$^B$-Y-Ring$^C$ group;
or -X-$R^2$ represents a -$C_1$-$C_{12}$ alkylene-$NR^xR^y$ group, wherein said alkylene group may be optionally substituted by one or more $C_{1-6}$ alkyl, $C_{2-6}$ alkenyl, $-NR^x$, O, hydroxy or CO groups; Ring$^A$ represents carbocyclyl, heterocyclyl or heteroaryl which requires a substituent selected from a $-(CH_2)_m$-CO-$C_{1-6}$ alkyl, $-(CH_2)_m$-NHCO-$C_{1-6}$ alkyl, $-(CH_2)_m$-CO-$C_{2-6}$ alkenyl,$-(CH_2)_m$-NHCO-$C_{2-6}$ alkenyl, $-(CH_2)_m$-CO-$C_{2-6}$ alkynyl or $-(CH_2)_m$-NHCO-$C_{2-6}$ alkynyl

group, wherein said alkyl, alkenyl or alkynyl group may be optionally substituted by one or more (e.g. 1, 2 or 3) halogen, hydroxy, CO or $-NR^xR^y$ groups and wherein said carbocyclyl, heterocyclyl or heteroaryl groups may be optionally further substituted by one or more (e.g. 1, 2 or 3) substituents selected from halogen, $C_{1-6}$ alkyl, hydroxy, $C_{1-6}$ alkoxy, $-CO-C_{1-6}$ alkyl, oxo, $C_{1-6}$ alkylamino or cyano;

m represents an integer selected from 0, 1, 2, 3 or 4;

$Ring^B$ represents carbocyclyl, heterocyclyl or heteroaryl, each of which may be optionally substituted by one or more (e.g. 1, 2 or 3) substituents selected from hydroxy, oxo, $C_{1-6}$ alkyl, $C_{2-6}$ alkenyl, $C_{2-6}$ alkynyl, $C_{1-6}$ alkoxy, halo$C_{1-6}$ alkyl, $-CO-C_{1-6}$ alkyl, cyano or halogen;

$Ring^C$ represents heterocyclyl substituted by one or more (e.g. 1, 2 or 3) substituents selected from $C_{1-6}$ alkyl (such as methyl) and/or $-(CH_2)_m-CO-C_{2-6}$ alkenyl optionally substituted by an $-NR^xR^y$ group;

Y represents a bond, -O-, CO, or a $C_1$-$C_6$ alkylene group optionally substituted by one or more $C_{1-6}$ alkyl, $C_{2-6}$ alkenyl, $-NR^x$, O, hydroxy or CO groups;

$R^3$ represents hydrogen or $C_{1-6}$ alkyl;

$R^4$, $R^5$, $R^6$ and $R^7$ independently represent hydrogen, $C_{1-6}$ alkyl, $C_{2-6}$ alkenyl, $C_{1-6}$ alkoxy, halogen, halo$C_{1-6}$ alkyl, halo$C_{1-6}$ alkoxy, $C_{3-8}$ cycloalkyl, cyano or $-NR^xR^y$; and

$R^x$ and $R^y$ independently represent hydrogen, $C_{1-6}$ alkyl, $C_{2-6}$ alkenyl, $C_{2-6}$ alkynyl, $C_{1-6}$ alkoxy, or $R^x$ and $R^y$ together with the nitrogen atom to which they are attached join to form a nitrogen containing heterocyclic ring which may be optionally substituted by one or more $C_{1-6}$ alkyl groups.

In one embodiment, n represents 0, 1, 2 or 3.

In one embodiment, $R^1$ represents $C_{1-6}$ alkyl (such as methyl), halogen (such as fluorine or chlorine) or $C_{3-8}$ cycloalkyl (such as cyclopropyl). In a further embodiment, $R^1$ represents $C_{1-6}$ alkyl (such as methyl) or halogen (such as fluorine or chlorine). In a yet further embodiment, $R^1$ represents halogen (such as fluorine or chlorine). In an alternative embodiment, $R^1$ represents $C_{1-6}$ alkyl (such as methyl). In a further embodiment, $R^1$ represents methyl.

In a further embodiment, n represents 0.

In a further embodiment, n represents 1. In a yet further embodiment, n represents 1 and $R^1$ represents $C_{1-6}$ alkyl (such as methyl) or halogen (such as fluorine or chlorine). In a yet further embodiment, n represents 1 and $R^1$ represents methyl, fluorine or chlorine. In a yet further embodiment, n represents 1 and $R^1$ represents $C_{1-6}$ alkyl (such as methyl). In a still yet further embodiment, n represents 1 and $R^1$ represents methyl.

In an alternative embodiment, n represents 2. In a yet further embodiment, n represents 2 and $R^1$ represents $C_{1-6}$ alkyl (such as methyl), halogen (such as fluorine or chlorine) or $C_{3-8}$ cycloalkyl (such as cyclopropyl). In a yet further embodiment, n represents 2 and $R^1$ represents methyl, fluorine, chlorine, or cyclopropyl. In a still yet further embodiment, n represents 2 and:

both $R^1$ groups are halogen (such as both represent fluorine or one represents fluorine and the other represents chlorine); or

one $R^1$ group represents halogen (i.e. fluorine) and the other represents $C_{1-6}$ alkyl (such as methyl); or

one $R^1$ group represents halogen (i.e. fluorine) and the other represents $C_{3-8}$ cycloalkyl (such as cyclopropyl).

In an alternative embodiment, n represents 3. In a yet further embodiment, n represents 3 and $R^1$ represents halogen (such as fluorine or chlorine). In a yet further embodiment, n represents 3 and $R^1$ represents fluorine or chlorine. In a still yet further embodiment, n represents 3 and two $R^1$ groups represent fluorine and one $R^1$ group represents chlorine.

In one embodiment, $R^2$ represents $-Ring^A$.

In one embodiment, $Ring^A$ represents carbocyclyl (such as phenyl, cyclobutyl, 2,3-dihydro-1H-inden-2-yl or bicyclo[1.1.1]pentan-1-yl), a heterocyclyl ring (such as azetidinyl, pyrrolidinyl, piperidinyl, piperazinyl, isoindolin-1-yl, isoindolin-5-yl, tetrahydroisoquinolin-6-yl, diazaspiro[3.4]octan-6-yl, octahydro-2*H*-pyrazino[1,2-a]pyrazin-2-yl, octahydro-5H-pyrrolo[3,4-c]pyridin-5-yl, 5-oxa-2,8-diazaspiro[3.5]nonan-8-yl, 2,6-diazaspiro[3.4]octane, 9-oxa-3,7-diazabicyclo[3.3.1]nonan-3-yl, tetrahydro-1*H*-imidazo[4,5-c]pyridin-1-yl, tetrahydro-1'H-spiro[azetidine-3,4'-pyrrolo[1,2-a]pyrazin]-2'(3'*H*)-yl, tetrahydro-1'*H*-spiro[azetidine-3,6'-pyrazino[2,1-*c*][1,4]oxazin]-8'(7'*H*)-yl, tetrahydro-3*H*-imidazo[4,5-c]pyridin-3-yl, tetrahydro-[1,2,4]triazolo[1,5-a]pyrazin-2-yl, tetrahydroimidazo[1,2-a]pyrazin-2-yl, tetrahydropyrazolo[1,5-a]pyrazin-2-yl, octahydro-2H-pyrazino[1,2-a]pyrazin-3-yl, tetrahydro-[1,2,4]triazolo[4,3-a]pyrazin-3-yl, 2,5,8-triazaspiro[3.5]nonan-8-yl, spiro[azetidine-3,3'-imidazo[1,2-a]imidazol]-1'(2'H)-yl, dihydro-5H-pyrrolo[3,4-b]pyrazin-2-yl, dihydro-5H-pyrrolo[3,4-b]pyridin-3-yl, dihydro-5H-pyrrolo[3,4-b]pyridin-2-yl, dihydro-5H-pyrrolo[3,4-b]pyridin-7-yl, dihydro-5H-pyrrolo[3,4-b]pyridin-5-yl, tetrahydropyrrolo[3,4-d]imidazol-2-yl, dihydro-2H-benzo[b][1,4]oxazin-6-yl, dihydro-2H-benzo[b][1,4]oxazin-7-yl, dihydro-4H-pyrrolo[3,4-d]imidazol-1-yl, diazabicyclo[3.1.1]heptan-6-yl, diazabicyclo[3.2.1]octan-8-yl, diazabicyclo[2.2.1]heptan-2-yl, dihydro-1H-pyrrolo[3,4-c]pyridin-6-yl, dihydro-5H-pyrrolo[3,4-d]pyrimidin-2-yl, dihydro-4H-pyrrolo[3,4-d]thiazol-2-yl, dihydro-4H-pyrrolo[3,4-d]oxazo-2-yl, tetrahydropyrrolo[3,4-c]pyrazol-3-yl, diazaspiro[3.3]heptan-6-yl, tetrahydroimidazo[1,2-a]pyridin-6-yl, tetrahydroimidazo[1,2-a]pyridin-7-yl, or spiro[azetidine-3,5'-imidazo[1,2-a]pyrazin]-7'(8'H)-yl) or a heteroaryl ring (such as pyridyl or pyrimidinyl), each being substituted by a

- $-(CH_2)_m-CO-C_{2-6}$ alkyl optionally substituted by a halogen group (such as $-CO-CH_2-Cl$);
- $-(CH_2)_m-CO-C_{2-6}$ alkenyl optionally substituted by one or more halogen, hydroxy, CO or $-NR^xR^y$ group (such as $-COCH=CH_2$, $-CO-C(=CH_2)-Me$, $-CO-CH=CH-CH_2-N(Me)_2$, $-CO-C(=CH_2)-CH_2-N(Me)_2$, $-CO-C(=CH-Me)-CH_2-N(Me)_2$, $-CO-C(=CH_2)-CH_2-N(Me)_2$, $-CO-C(Cl)=CH-CH_2-N(Me)_2$, $-CO-C(=CH_2)-CH_2-OH$, $-CO-C(=CH_2)-CH_2-O-C(=O)-Me$), $-CO-CH=CH-CHF_2$, $-CO-C(=CH_2)-CH_2-NEt_2$;
- $-(CH_2)_m-NHCO-C_{2-6}$ alkenyl optionally substituted by a $-NR^xR^y$ group (such as $-NH-CO-CH=CH_2$, $-NH-CO-C(=CH_2)-CH_2-N(Me)_2$, $-NH-CO-C(=CH-Me)-CH_2-N(Me)_2$, $-NH-CO-CH=CH-CH_2-N(Me)_2$, $-CH_2-NH-CO-CH=CH_2-$, $-CH_2-NH-CO-CH=CH-CH_2-N(Me)_2$, $-CH_2-NH-CO-C(=CH_2)-CH_2-N(Me)_2$);
- $-(CH_2)_m-CO-C_{2-6}$ alkynyl (such as -CO-ethynyl or -CO-ethynyl-Me); or
- $-(CH_2)_m-NHCO-C_{2-6}$ alkynyl group (such as -NH-CO-ethynyl),

wherein said carbocyclyl, heterocyclyl or heteroaryl group may be optionally substituted by one or more (e.g. 1, 2 or 3) further substituents selected from: halogen (such as fluorine); $C_{1-6}$ alkyl (such as methyl or ethyl) optionally substituted by one or more halogen (such as $-CF_3$ or $-CHF_2$), hydroxy (such as $-CH_2-OH$) or cyano (such as $-CH_2-CN$) groups; hydroxy; $C_{1-6}$ alkoxy (such as methoxy and $-CH_2-OMe$); $-CO-C_{1-6}$ alkyl (such as -CO-Me); oxo; $C_{1-6}$ alkylamino (such as $-N(Me)-(CH_2)_2-N(Me)_2$); or cyano).

In a further embodiment, $Ring^A$ represents carbocyclyl (such as phenyl or bicyclo[1.1.1]pentan-1-yl), a heterocyclyl ring (such as azetidinyl, pyrrolidinyl, piperidinyl, piperazinyl, isoindolin-5-yl, tetrahydroisoquinolin-6-yl, diazaspiro[3.4]octan-6-yl, octahydro-2*H*-pyrazino[1,2-a]pyrazin-2-yl, octahydro-5H-pyrrolo[3,4-c]pyridin-5-yl, 5-oxa-2,8-diazaspiro[3.5]nonan-8-yl, 2,6-diazaspiro[3.4]octane, 9-oxa-3,7-diazabicyclo[3.3.1]nonan-3-yl, tetrahydro-1*H*-imidazo[4,5-c]pyridin-1-yl, tetrahydro-1'H-spiro[azetidine-3,4'-pyrrolo[1,2-a]pyrazin]-2'(3'*H*)-yl, tetrahydro-1'*H*-spiro[azetidine-3,6'-pyrazino[2,1-*c*][1,4]oxazin]-8'(7'*H*)-yl, tetrahydro-3*H*-imidazo[4,5-c]pyridin-3-yl, tetrahydro-[1,2,4]triazolo[1,5-a]pyrazin-2-yl, tetrahydroimidazo[1,2-a]pyrazin-2-yl, tetrahydropyrazolo[1,5-a]pyrazin-2-yl, octahydro-2H-pyrazino[1,2-a]pyrazin-3-yl, tetrahydro-[1,2,4]triazolo[4,3-a]pyrazin-3-yl, 2,5,8-triazaspiro[3.5]nonan-8-yl, spiro[azetidine-3,3'-imidazo[1,2-a]imidazol]-1'(2'H)-yl or spiro[azetidine-3,5'-imidazo[1,2-a]pyrazin]-7'(8'H)-yl) or a heteroaryl ring (such as pyridyl), each being substituted by a

- $-(CH_2)_m-CO-C_{2-6}$ alkyl optionally substituted by a halogen group (such as $-CO-CH_2-Cl$);
- $-(CH_2)_m-CO-C_{2-6}$ alkenyl optionally substituted by one or more halogen, hydroxy, CO or $-NR^xR^y$ group (such as $-COCH=CH_2$, $-CO-C(=CH_2)-Me$, $-CO-CH=CH-CH_2-N(Me)_2$, $-CO-C(=CH_2)-CH_2-N(Me)_2$, $-CO-C(=CH-Me)-CH_2-N(Me)_2$, $-CO-C(=CH_2)-CH_2-N(Me)_2$, $-CO-C(Cl)=CH-CH_2-N(Me)_2$, $-CO-C(=CH_2)-CH_2-OH$, $-CO-C(=CH_2)-CH_2-O-C(=O)-Me$);
- $-(CH_2)_m-NHCO-C_{2-6}$ alkenyl optionally substituted by a $-NR^xR^y$ group (such as $-NH-CO-CH=CH_2$, $-NH-CO-C(=CH_2)-CH_2-N(Me)_2$, $-NH-CO-C(=CH-Me)-CH_2-N(Me)_2$, $-NH-CO-CH=CH-CH_2-N(Me)_2$, $-CH_2-NH-CO-CH=CH_2-$, $-CH_2-NH-CO-CH=CH-CH_2-N(Me)_2$, $-CH_2-NH-CO-C(=CH_2)-CH_2-N(Me)_2$);
- $-(CH_2)_m-CO-C_{2-6}$ alkynyl (such as -CO-ethynyl or -CO-ethynyl-Me); or
- $-(CH_2)_m-NHCO-C_{2-6}$ alkynyl group (such as -NH-CO-ethynyl),

wherein said carbocyclyl, heterocyclyl or heteroaryl group may be optionally substituted by one or more (e.g. 1, 2 or 3) further substituents selected from halogen (such as fluorine), $C_{1-6}$ alkyl (such as methyl or ethyl), hydroxy, $C_{1-6}$ alkoxy (such as methoxy and-$CH_2$-OMe), $-CO-C_{1-6}$ alkyl (such as -CO-Me), oxo, $C_{1-6}$ alkylamino (such as $-N(Me)-(CH_2)_2-N(Me)_2$) or cyano).

In a further embodiment, $Ring^A$ represents a heterocyclyl ring (such as azetidinyl, pyrrolidinyl, piperidinyl, piperazinyl, isoindolin-1-yl, isoindolin-5-yl, tetrahydroisoquinolin-6-yl, diazaspiro[3.4]octan-6-yl, octahydro-2*H*-pyrazino[1,2-a]pyrazin-2-yl, octahydro-5H-pyrrolo[3,4-c]pyridin-5-yl, 5-oxa-2,8-diazaspiro[3.5]nonan-8-yl, 2,6-diazaspiro[3.4]octane, 9-oxa-3,7-diazabicyclo[3.3.1]nonan-3-yl, tetrahydro-1*H*-imidazo[4,5-c]pyridin-1-yl, tetrahydro-1'H-spiro[azetidine-3,4'-pyrrolo[1,2-a]pyrazin]-2'(3'*H*)-yl, tetrahydro-1'*H*-spiro[azetidine-3,6'-pyrazino[2,1-*c*][1,4]oxazin]-8'(7'*H*)-yl, tetrahydro-3*H*-imidazo[4,5-c]pyridin-3-yl, tetrahydro-[1,2,4]triazolo[1,5-a]pyrazin-2-yl, tetrahydroimidazo[1,2-a]pyrazin-2-yl, tetrahydropyrazolo[1,5-a]pyrazin-2-yl, octahydro-2H-pyrazino[1,2-a]pyrazin-3-yl, tetrahydro-[1,2,4]triazolo[4,3-a]pyrazin-3-yl, 2,5,8-triazaspiro[3.5]nonan-8-yl, spiro[azetidine-3,3'-imidazo[1,2-a]imidazol]-1'(2'H)-yl, dihydro-5H-pyrrolo[3,4-b]pyrazin-2-yl, dihydro-5H-pyrrolo[3,4-b]pyridin-3-yl, dihydro-5H-pyrrolo[3,4-b]pyridin-2-yl, dihydro-5H-pyrrolo[3,4-b]pyridin-7-yl, dihydro-5H-pyrrolo[3,4-b]pyridin-5-yl, tetrahydropyrrolo[3,4-d]imidazol-2-yl, dihydro-2H-benzo[b][1,4]oxazin-6-yl, dihydro-2H-benzo[b][1,4]oxazin-7-yl, dihydro-4H-pyrrolo[3,4-d]imidazol-1-yl, diazabicyclo[3.1.1]heptan-6-yl, diazabicyclo[3.2.1]octan-8-yl, diazabicyclo[2.2.1]heptan-2-yl, dihydro-1H-pyrrolo[3,4-c]pyridin-6-yl, dihydro-5H-pyrrolo[3,4-d]pyrimidin-2-yl, dihydro-4H-pyrrolo[3,4-d]thiazol-2-yl, dihydro-4H-pyrrolo[3,4-d]oxazo-2-yl, tetrahydropyrrolo[3,4-c]pyrazol-3-yl, diazaspiro[3.3]heptan-6-yl, tetrahydroimidazo[1,2-a]pyridin-6-yl, tetrahydroimidazo[1,2-a]pyridin-7-yl, or spiro[azetidine-3,5'-imidazo[1,2-a]pyrazin]-7'(8'H)-yl) each being substituted by a

$-(CH_2)_m-CO-C_{2-6}$ alkyl optionally substituted by a halogen group (such as $-CO-CH_2-Cl$);
$-(CH_2)_m-CO-C_{2-6}$ alkenyl optionally substituted by one or more halogen, hydroxy, CO or $-NR^xR^y$ group (such as $-COCH=CH_2$, $-CO-C(=CH_2)-Me$, $-CO-CH=CH-CH_2-N(Me)_2$, $-CO-C(=CH_2)-CH_2-N(Me)_2$, $-CO-C(=CH-Me)-CH_2-N(Me)_2$, $-CO-C(=CH_2)-CH_2-N(Me)_2$, $-CO-C(Cl)=CH-CH_2-N(Me)_2$, $-CO-C(=CH_2)-CH_2-OH$, $-CO-C(=CH_2)-CH_2-O-C(=O)-Me)$, $-CO-CH=CH-CHF_2$, $-CO-C(=CH_2)-CH_2-NEt_2$;
$-(CH_2)_m-NHCO-C_{2-6}$ alkenyl optionally substituted by a $-NR^xR^y$ group (such as $-NH-CO-CH=CH_2$, $-NH-CO-C(=CH_2)-CH_2-N(Me)_2$, $-NH-CO-C(=CH-Me)-CH_2-N(Me)_2$, $-NH-CO-CH=CH-CH_2-N(Me)_2$, $-CH_2-NH-CO-CH=CH_2-$, $-CH_2-NH-CO-CH=CH-CH_2-N(Me)_2$, $-CH_2-NH-CO-C(=CH_2)-CH_2-N(Me)_2)$;
$-(CH_2)_m-CO-C_{2-6}$ alkynyl (such as -CO-ethynyl or -CO-ethynyl-Me); or
$-(CH_2)_m-NHCO-C_{2-6}$ alkynyl group (such as -NH-CO-ethynyl),
wherein said heterocyclyl or heteroaryl group may be optionally substituted by one or more (e.g. 1, 2 or 3) further substituents selected from: halogen (such as fluorine); $C_{1-6}$ alkyl (such as methyl or ethyl) optionally substituted by one or more halogen (such as $-CF_3$ or-$CHF_2$) hydroxy (such as $-CH_2-OH$) or cyano (such as $-CH_2-CN$) groups; hydroxy; $C_{1-6}$ alkoxy (such as methoxy and $-CH_2-OMe$); $-CO-C_{1-6}$ alkyl (such as -CO-Me); oxo; $C_{1-6}$ alkylamino (such as $-N(Me)-(CH_2)_2-N(Me)_2$); or cyano).

In a further embodiment, Ring$^A$ represents dihydro-5H-pyrrolo[3,4-b]pyridinyl, such as dihydro-5H-pyrrolo[3,4-b]pyridin-3-yl, dihydro-5H-pyrrolo[3,4-b]pyridin-2-yl, dihydro-5H-pyrrolo[3,4-b]pyridin-7-yl, or dihydro-5H-pyrrolo[3,4-b]pyridin-5-yl each being substituted by a

$-(CH_2)_m-CO-C_{2-6}$ alkenyl optionally substituted by one or more halogen, hydroxy, CO or $-NR^xR^y$ group (such as $-COCH=CH_2$, $-CO-C(=CH_2)-Me$, $-CO-CH=CH-CH_2-N(Me)_2$, $-CO-C(=CH_2)-CH_2-N(Me)_2$, $-CO-C(=CH-Me)-CH_2-N(Me)_2$, $-CO-C(=CH_2)-CH_2-N(Me)_2$, $-CO-C(Cl)=CH-CH_2-N(Me)_2$, $-CO-C(=CH_2)-CH_2-OH$, $-CO-C(=CH_2)-CH_2-O-C(=O)-Me)$, $-CO-CH=CH-CHF_2$, $-CO-C(=CH_2)-CH_2-NEt_2$;
wherein said heterocyclyl group may be optionally substituted by one or more (e.g. 1, 2 or 3) further substituents selected from: halogen (such as fluorine); $C_{1-6}$ alkyl (such as methyl or ethyl) optionally substituted by one or more halogen (such as $-CF_3$ or $-CHF_2$), hydroxy (such as $-CH_2-OH$) or cyano (such as $-CH_2-CN$) groups; hydroxy; $C_{1-6}$ alkoxy (such as methoxy and $-CH_2-OMe$); $-CO-C_{1-6}$ alkyl (such as -CO-Me); oxo; $C_{1-6}$ alkylamino (such as $-N(Me)-(CH_2)_2-N(Me)_2$); or cyano).

In a yet further embodiment, Ring$^A$ represents dihydro-5H-pyrrolo[3,4-b]pyridinyl, such as dihydro-5H-pyrrolo[3,4-b]pyridin-3-yl, dihydro-5H-pyrrolo[3,4-b]pyridin-2-yl, dihydro-5H-pyrrolo[3,4-b]pyridin-7-yl, or dihydro-5H-pyrrolo[3,4-b]pyridin-5-yl each being substituted by a $-COCH=CH_2$.

In a still yet further embodiment, Ring$^A$ represents dihydro-5H-pyrrolo[3,4-b]pyridin-2-yl substituted by a $-COCH=CH_2$.

In a still yet further embodiment, Ring$^A$ represents 6,7-dihydro-5H-pyrrolo[3,4-b]pyridin-2-yl substituted by a $-COCH=CH_2$.

In an alternative embodiment, $R^2$ represents -Ring$^B$-Y-Ring$^C$.

In one embodiment, Ring$^B$ represents carbocyclyl (such as cyclohexyl or phenyl), heterocyclyl (such as azetidinyl, pyrrolidinyl, piperidinyl, piperazinyl, diazepanyl or hexahydropyrrolo[3,4-*b*][1,4]oxazin-6(2*H*)-yl) or heteroaryl (such as imidazolyl, pyrazolyl, triazolyl, pyridinyl, pyrimidinyl, oxazolyl or oxadiazolyl), each of which may be optionally substituted by one or more (e.g. 1, 2 or 3) substituents selected from hydroxy, oxo, $C_{1-6}$ alkyl (such as methyl), halo$C_{1-6}$ alkyl (such as $-CH_2-F$), cyano or halogen (such as fluorine).

In a further embodiment, Ring$^B$ represents cyclohexyl, phenyl, azetidinyl, pyrrolidinyl, piperidinyl, piperazinyl, diazepanyl, hexahydropyrrolo[3,4-*b*][1,4]oxazin-6(2*H*)-yl, imidazolyl, pyrazolyl, triazolyl, pyridinyl, pyrimidinyl, oxazolyl or oxadiazolyl, each of which may be optionally substituted by one or more (e.g. 1, 2 or 3) substituents selected from hydroxy, oxo, methyl, $-CH_2-F$, cyano or fluorine.

In a further embodiment, Ring$^B$ represents carbocyclyl (such as cyclohexyl or phenyl), heterocyclyl (such as azetidinyl, pyrrolidinyl, piperazinyl, diazepanyl or hexahydropyrrolo[3,4-*b*][1,4]oxazin-6(2*H*)-yl) or heteroaryl (such as imidazolyl, pyrazolyl, triazolyl, pyridinyl, pyrimidinyl, oxazolyl or oxadiazolyl), each of which may be optionally substituted by one or more (e.g. 1, 2 or 3) substituents selected from hydroxy, oxo, $C_{1-6}$ alkyl (such as methyl), halo$C_{1-6}$ alkyl (such as $-CH_2-F$), cyano or halogen (such as fluorine).

In one embodiment, Y represents a bond, -O-, -NHCO-, CO, or a $C_1$-$C_6$ alkylene group optionally substituted by one or more $C_{1-6}$ alkyl, $C_{2-6}$ alkenyl, $-NR^x$, O, hydroxy or CO groups (such as $-CO-C(=CH_2)-CH_2-$ or $-NHCO-CH=CH-CH_2-$). In a further embodiment, Y represents a bond, -O-, -NHCO-, CO, or a $C_1$-$C_6$ alkylene group optionally substituted by one or more $C_{1-6}$ alkyl, $C_{2-6}$ alkenyl, $-NR^x$, O, hydroxy or CO groups (such as $-CO-C(=CH_2)-CH_2-$).

In a further embodiment, Y represents a bond. In an alternative embodiment, Y represents-O-.

In one embodiment, Ring$^C$ represents a monocyclic heterocyclyl ring having at least one nitrogen atom. In a further embodiment, Ring$^C$ represents azetidinyl, pyrrolidinyl, piperazinyl, piperidinyl, morpholinyl or tetrahydropyridinyl, each of which being substituted by a halogen (such as fluorine), $C_{1-6}$ alkyl (such as methyl), -CO-$C_{1-6}$ alkyl (such as -CO-methyl) and/or a-$(CH_2)_m$-CO-$C_{2-6}$ alkenyl optionally substituted by an -$NR^xR^y$ group (such as -COCH=$CH_2$,-CO-C(=$CH_2$)-$CH_2$-N$(Me)_2$, -CO-C(=CH-Me)-$CH_2$-N$(Me)_2$, or -CO-CH=CH-$CH_2$-N$(Me)_2$).

In a further embodiment, Ring$^C$ represents azetidinyl, pyrrolidinyl, piperazinyl, piperidinyl, morpholinyl or tetrahydropyridinyl, each of which being substituted by a fluorine, methyl,-CO-methyl, and/or a -COCH=$CH_2$, -CO-C(=$CH_2$)-$CH_2$-N$(Me)_2$, -CO-C(=CH-Me)-$CH_2$-N$(Me)_2$, or -CO-CH=CH-$CH_2$-N$(Me)_2$.

In a further embodiment, Ring$^C$ represents azetidinyl, pyrrolidinyl, piperazinyl, piperidinyl, morpholinyl or tetrahydropyridinyl, each of which being substituted by:

one methyl group; or
one -CO-methyl group; or
one -COCH=$CH_2$ group; or
one -CO-C(=$CH_2$)-$CH_2$-N$(Me)_2$ group; or
one -CO-C(=CH-Me)-$CH_2$-N$(Me)_2$ group; or
one -CO-CH=CH-$CH_2$-N$(Me)_2$ group; or
one methyl group and one -COCH=$CH_2$ group; or
one fluorine group and one -COCH=$CH_2$ group.

In a further embodiment, Ring$^C$ represents azetidinyl, pyrrolidinyl, piperazinyl or tetrahydropyridinyl, each of which being substituted by a $C_{1-6}$ alkyl (such as methyl) and/or a -$(CH_2)_m$-CO-$C_{2-6}$ alkenyl optionally substituted by an -$NR^xR^y$ group (such as -COCH=$CH_2$,-CO-C(=$CH_2$)-$CH_2$-N$(Me)_2$, -CO-C(=CH-Me)-$CH_2$-N$(Me)_2$, or -CO-CH=CH-$CH_2$-N$(Me)_2$).

In one embodiment, X represents a bond or a $C_1$-$C_4$ alkylene group optionally substituted by one or more $C_{1-6}$ alkyl (such as methyl), -$NR^x$ (such as -NH or -N-methyl), O, hydroxy, halogen (such as fluorine) or CO groups.

In a further embodiment, X represents a bond or a $C_1$-$C_4$ alkylene group optionally substituted by one or more $C_{1-6}$ alkyl (such as methyl), -$NR^x$ (such as -NH or -N-methyl), O, hydroxy or CO groups.

In a further embodiment, X represents a bond.

In an alternative embodiment, X represents a $C_1$-$C_4$ alkylene group (such as -$CH_2$-, -$(CH_2)_2$-, -$(CH_2)_3$-, -$(CH_2)_4$-, or -$(CH_2)_5$-) optionally substituted by one or more $C_{1-6}$ alkyl (such as-($CH_2$)-C(Me)-), O (such as -$(CH_2)_2$-O-, -$(CH_2)_3$-O- or -$(CH_2)_2$-O-$CH_2$-) or CO (such as-($CH_2$)-CO- or -$(CH_2)_2$-CO-) -$NR^x$ (such as -$(CH_2)_2$-NH-, -$(CH_2)_3$-NH-, -$(CH_2)_2$-N(Me)- or-$(CH_2)_3$-N(Me)-) groups or a CO and -$NR^x$ group (such as -$(CH_2)_2$-N(Me)-CO-) or a hydroxy and -$NR^x$ group (such as -($CH_2$)-CH(OH)-$CH_2$-N(Me)- or halogen (such as -$(CH_2)_3$-CHF-,-$(CH_2)_2$-CHF-$CH_2$-, or -$CH_2$-CHF-$(CH_2)_2$-).

In a further embodiment, X represents a $C_1$-$C_4$ alkylene group (such as -$CH_2$-, -$(CH_2)_2$-,-$(CH_2)_3$- or -$(CH_2)_4$-) optionally substituted by one or more $C_{1-6}$ alkyl (such as -($CH_2$)-C(Me)-), O (such as -$(CH_2)_2$-O-, -$(CH_2)_3$-O- or -$(CH_2)_2$-O-$CH_2$-) or CO (such as -($CH_2$)-CO- or-$(CH_2)_2$-CO-) -$NR^x$ (such as -$(CH_2)_2$-NH-, -$(CH_2)_3$-NH-, -$(CH_2)_2$-N(Me)- or -$(CH_2)_3$-N(Me)-) groups or a CO and -$NR^x$ group (such as -$(CH_2)_2$-N(Me)-CO-) or a hydroxy and -$NR^x$ group (such as -($CH_2$)-CH(OH)-$CH_2$-N(Me)-).

In a yet further embodiment, X represents a bond, -$CH_2$-, -$(CH_2)_2$-, -$(CH_2)_3$-, -$(CH_2)_4$-,-($CH_2$)-C(Me)-, -$(CH_2)_2$-O-, -$(CH_2)_3$-O-, -$(CH_2)_2$-O-$CH_2$-, -($CH_2$)-CO-, -$(CH_2)_2$-CO-, -$(CH_2)_2$-NH-, -$(CH_2)_3$-NH-, -$(CH_2)_2$-N(Me)-, -$(CH_2)_3$-N(Me)-, -$(CH_2)_2$-N(Me)-CO-, -($CH_2$)-CH(OH)-$CH_2$-N(Me)-, -$(CH_2)_3$-CHF-, -$(CH_2)_2$-CHF-$CH_2$-, or -$CH_2$-CHF-$(CH_2)_2$-.

In a yet further embodiment, X represents a bond, -$CH_2$-, -$(CH_2)_2$-, -$(CH_2)_3$-, -$(CH_2)_4$-,-($CH_2$)-C(Me)-, -$(CH_2)_2$-O-, -$(CH_2)_3$-O-, -$(CH_2)_2$-O-$CH_2$-, -($CH_2$)-CO-, -$(CH_2)_2$-CO-, -$(CH_2)_2$-NH-, -$(CH_2)_3$-NH-, -$(CH_2)_2$-N(Me)-, -$(CH_2)_3$-N(Me)-, -$(CH_2)_2$-N(Me)-CO- or -($CH_2$)-CH(OH)-$CH_2$-N(Me)-.

In a still yet further embodiment, X represents -$CH_2$-.

In one embodiment, -X-$R^2$ represents a -$C_1$-$C_{10}$ alkylene-$NR^xR^y$ group, wherein said alkylene group may be optionally substituted by one or more $C_{2-6}$ alkenyl, $NR^x$, O or CO groups, such as -$(CH_2)_2$-O-$(CH_2)_2$-NH-CO-C(=$CH_2$)-$CH_2$-N$(Me)_2$, -$(CH_2)_5$-NH-CO-CH=CH-$CH_2$-N$(Me)_2$,-$(CH_2)_5$-NH-CO-C(=$CH_2$)-$CH_2$-N$(Me)_2$, -$(CH_2)_4$-NHCOCH=CH-$CH_2$-$NMe_2$, or -$CH_2$-CHF-$(CH_2)_3$-NHCO-CH=CH$CH_2$$NMe_2$, -$(CH_2)_3$-CHF-$CH_2$NHCO-CH=CH$CH_2$$NMe_2$, or -$(CH_2)_3$-$CF_2$-$CH_2$NHCO-CH=CH$CH_2$$NMe_2$.

In a further embodiment, -X-$R^2$ represents a -$C_1$-$C_{10}$ alkylene-$NR^xR^y$ group, wherein said alkylene group may be optionally substituted by one or more $C_{2-6}$ alkenyl, $NR^x$, O or CO groups, such as -$(CH_2)_2$-O-$(CH_2)_2$-NH-CO-C(=$CH_2$)-$CH_2$-N$(Me)_2$, -$(CH_2)_5$-NH-CO-CH=CH-$CH_2$-N$(Me)_2$ or -$(CH_2)_5$-NH-CO-C(=$CH_2$)-$CH_2$-N$(Me)_2$.

In one embodiment, $R^3$ represents hydrogen or $C_{1-6}$ alkyl (such as methyl or ethyl). In a further embodiment, $R^3$ represents hydrogen or $C_{1-6}$ alkyl (such as methyl). In a yet further embodiment, $R^3$ represents hydrogen or methyl. In a yet further embodiment, $R^3$ represents $C_{1-6}$ alkyl (such as methyl). In a still yet further embodiment, $R^3$ represents methyl.

In one embodiment, $R^4$ represents:

hydrogen;
$C_{1-6}$ alkyl (such as methyl or ethyl);
$C_{2-6}$ alkenyl (such as ethenyl);$C_{1-6}$ alkoxy (such as methoxy);
halogen (such as chlorine); or
$-NR^xR^y$ (such as $-N(Me)_2$ or -N(Me)(Et)).

In a further embodiment, $R^4$ represents $C_{1-6}$ alkyl (such as methyl). In a yet further embodiment, $R^4$ represents methyl.
In one embodiment, $R^5$ represents:

hydrogen;
halogen (such as chlorine); or
$C_{1-6}$ alkyl (such as methyl).

In one embodiment, $R^5$ represents hydrogen.
In one embodiment, $R^6$ represents:

$C_{1-6}$ alkyl (such as methyl, ethyl or isopropyl);
$C_{2-6}$ alkenyl (such as $-C(=CH_2)(Me)$);
halogen (such as bromine);
halo$C_{1-6}$ alkyl (such as trifluoromethyl or $-C(H)(Me)-CF_3$); or
halo$C_{1-6}$ alkoxy (such as difluoromethoxy).

In a further embodiment, $R^6$ represents halo$C_{1-6}$ alkyl (such as trifluoromethyl). In a yet further embodiment, $R^6$ represents trifluoromethyl.
In one embodiment, $R^7$ represents:

hydrogen; or
cyano.

In a further embodiment, $R^7$ represents hydrogen.
In one particular embodiment, $R^4$ represents methyl, $R^5$ represents hydrogen, $R^6$ represents trifluoromethyl, and $R^7$ represents hydrogen. Thus, in one embodiment, the compound of formula (I) is a compound of formula $(I)^a$:

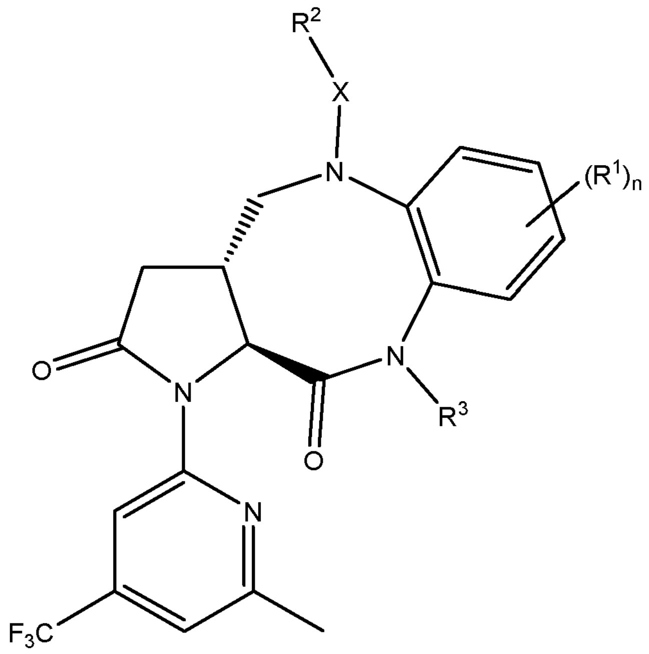

(I)[a]

or a tautomeric or a stereochemically isomeric form, a pharmaceutically acceptable salt or a solvate thereof, wherein n, $R^1$, X, $R^2$ and $R^3$ are as defined herein.

In one embodiment, $R^x$ and $R^y$ independently represent hydrogen or $C_{1-6}$ alkyl (such as methyl or ethyl). In a further embodiment, $R^x$ and $R^y$ independently represent $C_{1-6}$ alkyl (such as methyl). In a further embodiment, both of $R^x$ and $R^y$ represent $C_{1-6}$ alkyl (such as methyl).

In one embodiment, the invention provides a compound of formula (I) which is the free base of a compound of Examples 1-332 or a pharmaceutically acceptable salt or solvate thereof.

In a further embodiment, the invention provides a compound of formula (I) which is the free base of a compound of Examples 1-227 or a pharmaceutically acceptable salt or solvate thereof.

A reference to a compound of the formula (I) and sub-groups thereof also includes ionic forms, salts, solvates, stereochemical isomers, tautomers, N-oxides, thereof, for example, as discussed below; preferably, the salts or tautomers or stereochemical isomers or N-oxides or solvates thereof; and more preferably, the salts or tautomers or N-oxides or solvates thereof, even more preferably the salts or tautomers or solvates thereof. Hereinafter, compounds and their ionic forms, salts, solvates, stereochemical isomers, tautomers, N-oxides, thereof as defined in any aspect of the invention (except intermediate compounds in chemical processes) are referred to as "compounds of the invention".

***Salts***

Certain compounds of the formula (I) can exist in the form of salts, for example acid addition salts or, in certain cases salts of organic and inorganic bases such as carboxylate, sulfonate and phosphate salts. All such salts are within the scope of this invention, and references to compounds of the formula (I) include the salt forms of the compounds. The salts of the present invention can be synthesized from the parent compound that contains a basic or acidic moiety by conventional chemical methods such as methods described in Pharmaceutical Salts: Properties, Selection, and Use, P. Heinrich Stahl (Editor), Camille G. Wermuth (Editor), ISBN: 3-90639-026-8, Hardcover, 388 pages, August 2002. Generally, such salts can be prepared by reacting the free acid or base forms of these compounds with the appropriate base or acid in water or in an organic solvent, or in a mixture of the two; generally, non-aqueous media such as ether, ethyl acetate, ethanol, isopropanol, or acetonitrile are used.

Acid addition salts (*mono-* or *di*-salts) may be formed with a wide variety of acids, both inorganic and organic. Examples of acid addition salts include *mono-* or *di*-salts formed with an acid selected from the group consisting of acetic, 2,2-dichloroacetic, adipic, alginic, ascorbic (*e.g.* L-ascorbic), L-aspartic, benzenesulfonic, benzoic, 4-acetamidobenzoic, butanoic, (+) camphoric, camphor-sulfonic, (+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, D-gluconic, glucuronic (*e.g.* D-glucuronic), glutamic (*e.g.* L-glutamic), α-oxoglutaric, glycolic, hippuric, hydrohalic acids (*e.g.* hydrobromic, hydrochloric, hydriodic), isethionic, lactic (*e.g.* (+)-L-lactic, (±)-DL-lactic), lactobionic, maleic, malic, (-)-L-malic, malonic, (±)-DL-mandelic, methanesulfonic, naphthalene-2-sulfonic, naphthalene-1,5-disulfonic, 1-hydroxy-2-naphthoic, nicotinic, nitric, oleic, orotic, oxalic, palmitic, pamoic, phosphoric, propionic, pyruvic, L-pyroglutamic, salicylic, 4-amino-salicylic, sebacic, stearic, succinic, sulfuric, tannic, (+)-L-tartaric, thiocyanic, p-toluenesulfonic, undecylenic and valeric acids, as well as acylated amino acids and cation exchange resins.

One particular group of salts consists of salts formed from acetic, hydrochloric, hydriodic, phosphoric, nitric, sulfuric, citric, lactic, succinic, maleic, malic, isethionic, fumaric, benzenesulfonic, toluenesulfonic, methanesulfonic (mesylate), ethanesulfonic, naphthalenesulfonic, valeric, acetic, propanoic, butanoic, malonic, glucuronic and lactobionic acids. One particular salt is the hydrochloride salt.

Where the compounds of the formula (I) contain an amine function, these may form quaternary ammonium salts, for example by reaction with an alkylating agent according to methods well known to the skilled person. Such quaternary ammonium compounds are within the scope of formula (I).

The compounds of the invention may exist as *mono-* or *di*-salts depending upon the $pK_a$ of the acid from which the salt is formed.

It will be appreciated that for use in medicine the salts of the compounds of formula (I) should be pharmaceutically acceptable. Suitable pharmaceutically acceptable salts will be apparent to those skilled in the art. Pharmaceutically acceptable salts include those described by Berge, Bighley and Monkhouse, J. Pharm. Sci. 1977, 66, pp. 1-19. Such pharmaceutically acceptable salts include acid addition salts formed with inorganic acids e.g. hydrochloric, hydrobromic, sulfuric, nitric or phosphoric acid and organic acids *e.g.* succinic, maleic, acetic, fumaric, citric, tartaric, benzoic, p-toluenesulfonic, methanesulfonic or naphthalenesulfonic acid. Other salts *e.g.* oxalates or formates may be used, for example in the isolation of compounds of formula (I) and are included within the scope of this invention. However, salts that are not pharmaceutically acceptable may also be prepared as intermediate forms which may then be converted into pharmaceutically acceptable salts. Such non-pharmaceutically acceptable salts forms, which may be useful, for example, in the purification or separation of the compounds of the invention, also form part of the invention.

Certain of the compounds of formula (I) may form acid addition salts with one or more equivalents of the acid. The present invention includes within its scope all possible stoichiometric and non-stoichiometric forms.

### *Solvates*

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Pharmaceutically acceptable solvates of the compound of the invention are within the scope of the invention. In one embodiment, the pharmaceutically acceptable solvates of the compounds of the invention include the hydrate thereof.

In one embodiment, said crystalline form of the compounds of formula (I) is a cocrystal or coformer. Such a cocrystal or coformer may be prepared using water-soluble molecules such as saccharin, caffeine, nicotinamide or carboxylic acids. Coformers may be prepared as described in Emami S et al (2018) BioImpacts 8(4), 305-320.

### *N-Oxides*

Compounds of the formula (I) containing an amine function may also form N-oxides. A reference herein to a compound of the formula (I) that contains an amine function also includes the N-oxide.

Where a compound contains several amine functions, one or more than one nitrogen atom may be oxidised to form an N-oxide. Particular examples of N-oxides are the N-oxides of a tertiary amine or a nitrogen atom of a nitrogen-containing heterocycle.

N-Oxides can be formed by treatment of the corresponding amine with an oxidizing agent such as hydrogen peroxide or a per-acid (*e.g.* a peroxycarboxylic acid), see for example Advanced Organic Chemistry, by Jerry March, 4th Edition, Wiley Interscience. More particularly, N-oxides can be made by the procedure of L. W. Deady (Syn. Commun. 1977, 7, 509-514) in which the amine compound is reacted with m-chloroperoxybenzoic acid (mCPBA), for example, in an inert solvent such as dichloromethane.

Also included within the scope of the compound and various salts of the invention are polymorphs thereof.

### *Enantiomers*

Where chiral centres are present in compounds of formula (I), the present invention includes within its scope all possible enantiomers and diastereoisomers, including mixtures thereof. The different isomeric forms may be separated or resolved one from the other by conventional methods, or any given isomer may be obtained by conventional synthetic methods or by stereospecific or asymmetric syntheses. The invention also extends to any tautomeric forms or mixtures thereof.

***Purity***

Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are given on a weight for weight basis). Impure preparations of the compounds may be used for preparing the more pure forms used in the pharmaceutical compositions.

***Processes***

According to a further aspect of the present invention there is provided a process for the preparation of compounds of formula (I) and derivatives thereof. The following schemes are examples of synthetic schemes that may be used to synthesise the compounds of the invention. In the following schemes reactive groups can be protected with protecting groups and de-protected according to well established techniques.

According to a further aspect of the invention there is provided a process for preparing a compound of formula (I) as herein defined which comprises:

(a) deprotection of a compound of formula (II);

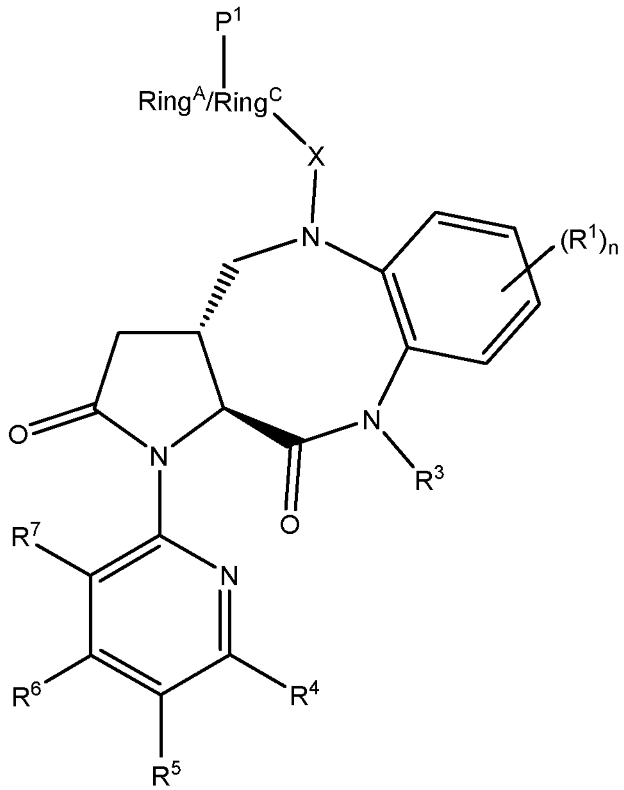

(II)

wherein $R^1$, n, X, $R^3$, $R^4$, $R^5$, $R^6$, $R^7$, $Ring^A$ and $Ring^C$ are as defined herein and $P^1$ represents a suitable protecting group, such as Boc;

(b) reacting a compound of formula (III):

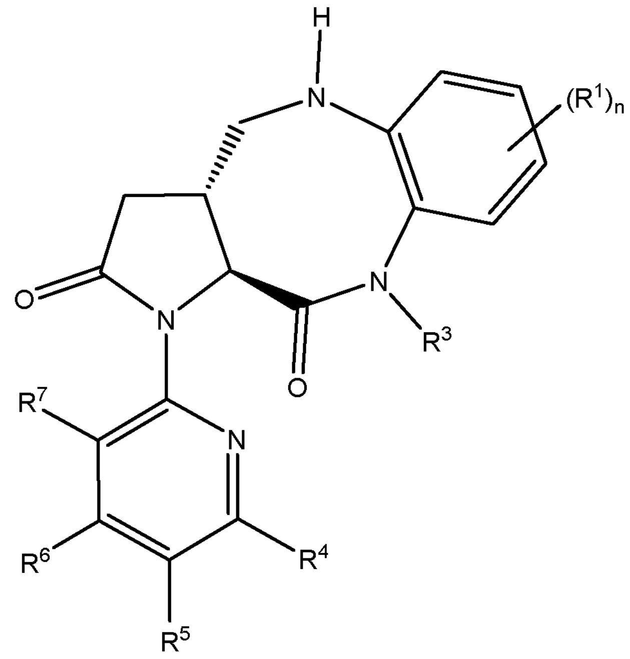

(III)

wherein $R^1$, n, $R^3$, $R^4$, $R^5$, $R^6$ and $R^7$ are as defined herein, with a compound of formula $L^1$-X-$R^2$, wherein X and $R^2$ are as defined herein and $L^1$ represents a suitable leaving group such as halogen (i.e. chlorine) or a hydroxy group;

(c) deprotection of a protected derivative of a compound of formula (I);

(d) interconversion of a compound of formula (I) or protected derivative thereof to a further compound of formula (I) or protected derivative thereof; and

(e) optional formation of a pharmaceutically acceptable salt of a compound of formula (I).

Process (a) typically comprises a deprotection reaction to prepare the compound of formula (I). For example, when $P^1$ represents Boc, process (a) typically comprises the use of an acid, such as TFA.

Process (b) typically comprises reacting a compound of formula (II) with the compound of formula $L^1$-X-$R^2$ in a suitable solvent, such as DCM in the presence of suitable reagents, such as HATU and DIPEA.

Compounds of formula (II) and (III) may be prepared in accordance with the procedures described herein. For example, compounds of formula (II) may be prepared in accordance with the experimental procedure described in Example 1, compounds of formula (III) may be prepared in accordance with the experimental procedure described in Example 27.

Compounds of formula $L^1$-X-$R^2$ are either known or may be prepared in accordance with known procedures.

A wide range of well known functional group interconversions for process (d) are known by a person skilled in the art for converting a precursor compound to a compound of formula (I) and are described in Advanced Organic Chemistry by Jerry March, 4th Edition, John Wiley & Sons, 1992. For example possible metal catalysed functionalisations such as using organo-tin reagents (the Stille reaction), Grignard reagents and reactions with nitrogen nucleophiles are described in 'Palladium Reagents and Catalysts' [Jiro Tsuji, Wiley, ISBN 0-470-85032-9] and Handbook of OrganoPalladium Chemistry for Organic Synthesis [Volume 1, Edited by Ei-ichi Negishi, Wiley, ISBN 0-471-31506-0].

If appropriate, the reactions described herein are followed or preceded by one or more reactions known to the skilled of the art and are performed in an appropriate order to achieve the requisite substitutions on each of the variables defined herein to afford other compounds of formula (I). Non-limiting examples of such reactions whose conditions can be found in the literature include:

protection of reactive functions,
deprotection of reactive functions,
halogenation,
dehalogenation,

dealkylation,
alkylation of amine, aniline, alcohol and phenol,
Mitsunobu reaction on hydroxyl groups,
cycloaddition reactions on appropriate groups,
reduction of nitro, esters, cyano, aldehydes,
transition metal-catalyzed coupling reactions,
acylation,
sulfonylation/introduction of sulfonyl groups,
saponification/hydrolysis of esters groups,
amidification or transesterification of ester groups,
esterification or amidification of carboxylic groups,
halogen exchange,
nucleophilic substitution with amine, thiol or alcohol,
reductive amination,
oxime formation on carbonyl and hydroxylamine groups,
S-oxidation,
N-oxidation,
salification.

It is recognised that the sequence of reactions involving aryl coupling and reduction may be varied. It is also recognised that a wide range of palladium based catalysts are suitable for conducting aryl coupling reactions.

It may also be recognised that isomer separation may occur at any suitable stage in the synthetic sequence. It should be stressed that such chiral separation forms a key aspect of the invention and that such separation may be conducted in accordance with the methodology described herein or may be conducted in accordance with known methodology. It is also recognised that it may be beneficial to temporarily form a protected derivative of an intermediate in the synthesis, for example, a Boc-protected amine, or SEM-protected amide, in order to facilitate chromatographic separation, chiral resolution or to give improved solubility or yields in particular steps.

In many of the reactions described above, it may be necessary to protect one or more groups to prevent reaction from taking place at an undesirable location on the molecule. Examples of protecting groups, and methods of protecting and de-protecting functional groups, can be found in Protective Groups in Organic Synthesis (T. Green and P. Wuts; 4th Edition; John Wiley and Sons, 2007).

A hydroxy group may be protected, for example, as an ether (-OR) or an ester (-OC(=O)R), for example, as: a *tert-butyl* ether; a tetrahydropyranyl (THP) ether; a benzyl, benzhydryl (diphenylmethyl), or trityl (triphenylmethyl) ether; a trimethylsilyl or *tert*-butyldimethylsilyl ether; or an acetyl ester (-OC(=O)$CH_3$).

An amine group may be protected, for example, as an amide (-NRCO-R) or a carbamate (-NRCO-OR), for example, as: a methyl amide (-NHCO-$CH_3$); a benzyl carbamate (-NHCO-$OCH_2C_6H_5$, -NH-Cbz or NH-Z); as a *tert*-butyl carbamate (-$NHCOOC(CH_3)_3$, NH-Boc); a 2-biphenyl-2-propyl carbamate (-NHCO-$OC(CH_3)_2C_6H_4C_6H_5$, NH-Boc), as a 9-fluorenylmethyl carbamate (-NH-Fmoc), as a 6-nitroveratryl carbamate (-NH-Nvoc), as a 2-trimethylsilylethyl carbamate (-NH-Teoc), as a 2,2,2-trichloroethyl carbamate (-NH-Troc), as an allyl carbamate (-NH-Alloc), or as a 2(-phenylsulfonyl)ethyl carbamate (-NH-Psec).

Other protecting groups for amines, such as cyclic amines and heterocyclic N-H groups, include toluenesulfonyl (tosyl) and methanesulfonyl (mesyl) groups, benzyl groups such as a para-methoxybenzyl (PMB) group and tetrahydropyranyl (THP) groups.

A carboxylic acid group may be protected as an ester for example, as: an $C_{1-7}$ alkyl ester (*e.g.* a methyl ester; a *tert*-butyl ester); a $C_{1-7}$ haloalkyl ester (*e.g.* a $C_{1-7}$ trihaloalkyl ester); a tri$C_{1-7}$ alkylsilyl-$C_{1-7}$ alkyl ester; or a $C_{5-20}$ aryl-$C_{1-7}$ alkyl ester (*e.g.* a benzyl ester; a nitrobenzyl ester; *para*-methoxybenzyl ester.

It will be understood by those skilled in the art that certain compounds of the invention can be converted into other compounds of the invention according to standard chemical methods.

Pharmaceutically acceptable salts may be prepared conventionally by reaction with the appropriate acid or acid derivative.

***Therapeutic Utility***

The compounds of the invention, subgroups and examples thereof, are inhibitors of Polθ polymerase activity, and which may be useful in preventing or treating disease states or conditions described herein. In addition, the compounds of the invention, and subgroups thereof, will be useful in preventing or treating diseases or condition mediated by Polθ. References to the preventing or prophylaxis or treatment of a disease state or condition such as cancer include within their scope alleviating or reducing the incidence of cancer.

Thus, for example, it is envisaged that the compounds of the invention will be useful in alleviating or reducing the incidence of cancer.

The compounds of the present invention may be useful for the treatment of the adult population. The compounds of the present invention may be useful for the treatment of the pediatric population.

As a consequence of their inhibition of Polθ, the compounds will be useful in providing a means of disabling the ability of cells to perform MMEJ. It is therefore anticipated that the compounds may prove useful in treating or preventing proliferative disorders such as cancers. In addition, the compounds of the invention may be useful in the treatment of diseases in which there is a disorder associated with cell accumulation.

Without being bound by theory it is expected that the Polθ inhibitors of the present invention will demonstrate certain properties for them to be of particular utility in the therapeutic treatment of certain cancers. For example, in one embodiment, the Polθ inhibitors of the present invention are suitably lethal in BRCA1 and BRCA2 deficient primary and secondary solid tumours, including breast, ovarian, prostate and pancreas.

In a further embodiment, the Polθ inhibitors of the present invention are suitably lethal in a variety of primary and secondary solid tumours which are HRD by mechanisms other than BRCA deficiency, such as those with promoter hypermethylation. In these tumours where no DSB repair pathway may be fully down regulated the Polθi may be given along with another DDR modulator such as a PARP inhibitor, a DNA-PK inhibitor, an ATR inhibitor, an ATM inhibitor, a wee1 inhibitor or a CHK1 inhibitor.

In a further embodiment, the Polθ inhibitors of the present invention are suitably lethal in primary and secondary breast, ovarian, prostate and pancreatic tumours retaining BRCA1 deficiency but which, following or not following exposure to PARPi medication, are resistant to PARPi treatment.

In a further embodiment, the Polθ inhibitors of the present invention suitably increase the ORR including CRR, will delay the onset of PARPi resistance, will increase the time to relapse and DFS, and will increase the OS of HRD (BRCA1/2 deficient and other HRD mechanisms) primary and secondary tumours (breast, ovarian, prostate and pancreas) when given with PARPi treatment programmes.

In a further embodiment, the Polθ inhibitors of the present invention suitably show synthetic sickness and/or synthetic lethality in a variety of tumours with loss of ATM activity ($ATM^{-/-}$) particularly in the context of WT p53. Tumour types will include around 10% of all solid tumours including gastric, lung, breast, and CRC, along with CLL. Co-medicating with another DDR modifier, such as a DNA-PK inhibitor, PARP inhibitor or ATR inhibitor, may further enhance such activity. Polθ inhibitors will resensitise CLL to classical chemotherapy and chemo-immunotherapy where drug resistance has emerged. Thus, according to a further embodiment, the pharmaceutical composition of the present invention additionally comprises a DNA-PK inhibitor, PARP inhibitor, or ATR inhibitor.

In a further embodiment, the Polθ inhibitors of the present invention suitably show synthetic sickness and/or synthetic lethality in a variety of tumours deficient in the DNA double strand break repair process of non-homologous end-joining (NHEJ-D). Tumour types will include approximately 2-10% of all solid tumours including prostate, pancreatic, cervical, breast, lung, bladder and oesophageal. Co-medicating with another DDR modifier, such as a PARP inhibitor, ATM inhibitor, wee1 inhibitor, CHK inhibitor, or ATR inhibitor, may further enhance such activity. Polθ inhibitors will further sensitise NHEJD cancer cells to DNA DSB inducing chemotherapies and to ionising radiation based therapies. Thus, according to a further embodiment, the pharmaceutical composition of the present invention additionally comprises a PARP inhibitor, ATM inhibitor, wee1 inhibitor, CHK inhibitor, or ATR inhibitor.

In a further embodiment, the Polθ inhibitors of the present invention suitably reduce the DNA replication stress response during the chemotherapy of HR proficient tumours such as ovarian, NSCL and breast tumours over expressing Polθ. This will increase the ORR to treatment and increase OS. Such effects are particularly likely with cytarabine (Ara-C) and hydroxyurea used in a wide variety of leukemias including CML, and the management of squamous cell carcinomas.

In a further embodiment, the Polθ inhibitors of the present invention suitably selectively sensitise solid tumours to radiotherapy, including EBRTand brachytherapy, with little or no sensitisation of normal tissues. In a fractionated curative-intent setting this will increase loco-regional control driving increased survival. This will be particularly evident in the management of NSCLC, SCCH&N, rectal cancer, prostate cancer and pancreatic cancer.

In a further embodiment, the Polθ inhibitors of the present invention suitably show synthetic sickness and/or synthetic lethality in PTEN deleted tumours such as CaP, with or without comedication with a PARPi. Furthermore, such tumours will exhibit exquisite sensitivity to radiotherapy both by dint of the PTEN deletion as well as the Polθ inhibitor induced radiosensitivity.

In a further embodiment, the Polθ inhibitors of the present invention suitably suppress TLS polymerase activity, sensitising primary and secondary solid tumours (e.g. breast, lung, ovarian, CRC) to drugs (e.g. cisplatin, mitomycin and cyclophosphamide) as well as reducing the acquisition of drug-induced mutations implicated in tumour resistance leading to prolongation of remission and increased TTR.

In a further embodiment, the Polθ inhibitors of the present invention suitably resensitise BCR-ABL-positive CML which is has developed imatinib resistance, as well as other solid tumours with elevated ligase IIIα levels, reduced ligase IV levels and increased dependence upon altEJ DSB repair.

In a further embodiment, the Polθ inhibitors of the present invention suitably show synthetic sickness and/or synthetic lethality in aromatase inhibitor resistant $ER^-$ primary and secondary breast cancers, again showing elevated ligase IIIα levels, reduced ligase IV levels and increased dependence upon altEJ DSB repair.

According to a further aspect of the invention there is a provided a compound of formula (I) as defined herein for use in the treatment of tumours characterised by a deficiency in homologous recombination (HRD).

It will be appreciated that references herein to "deficiency in homologous recombination (HRD)" refer to any genetic variation which results in a deficiency or loss of function of the resultant homologous recombination gene. Examples of said genetic variation include mutations (e.g. point mutations), substitutions, deletions, single nucleotide polymorphisms (SNPs), haplotypes, chromosome abnormalities, Copy Number Variation (CNV), epigenetics, DNA inversions, reduction in expression and mis-localisation.

In one embodiment, said homologous recombination genes are selected from any of: ATM, ATR, BRCA1, BRCA2, BARD1, RAD51C, RAD50, CHEK1, CHEK2, FANCA, FANCB, FANCC, FANCD2, FANCE, FANCF, FANCG, FANCI, FANCL, FANCM, PALB2 (FANCN), FANCP (BTBD12), ERCC4 (FANCQ), PTEN, CDK12, MRE11, NBS1, NBN, CLASPIN, BLM, WRN, SMARCA2, SMARCA4, LIG1, RPA1, RPA2, BRIP1 and PTEN.

It will be appreciated that references herein to "non-homologous end-joining deficiency (NHEJD)" refer to any genetic variation which results in a deficiency or loss of function of the resultant homologous recombination gene. Examples of said genetic variation include mutations (e.g. point mutations), substitutions, deletions, single nucleotide polymorphisms (SNPs), haplotypes, chromosome abnormalities, Copy Number Variation (CNV), epigenetics, DNA inversions, reduction in expression and mis-localisation.

In one embodiment, said non-homologous end-joining genes are selected from any one or more of: LIG4, NHEJ1, POLL, POLM, PRKDC, XRCC4, XRCC5, XRCC6, and DCLRE1C.

According to a further aspect of the invention there is a provided a compound of formula (I) as defined herein for use in the treatment of tumours which overexpress Polθ.

According to a further aspect of the invention there is a provided a compound of formula (I) as defined herein for use in the treatment of tumours which have elevated ligase IIIα levels, reduced ligase IV levels and increased dependence upon altEJ DSB repair.

Examples of cancers (and their benign counterparts) which may be treated (or inhibited) include, but are not limited to tumours of epithelial origin (adenomas and carcinomas of various types including adenocarcinomas, squamous carcinomas, transitional cell carcinomas and other carcinomas) such as carcinomas of the bladder and urinary tract, breast, gastrointestinal tract (including the esophagus, stomach (gastric), small intestine, colon, rectum and anus), liver (hepatocellular carcinoma), gall bladder and biliary system, exocrine pancreas, kidney, lung (for example adenocarcinomas, small cell lung carcinomas, non-small cell lung carcinomas, bronchioalveolar carcinomas and mesotheliomas), head and neck (for example cancers of the tongue, buccal cavity, larynx, pharynx, nasopharynx, tonsil, salivary glands, nasal cavity and paranasal sinuses), ovary, fallopian tubes, peritoneum, vagina, vulva, penis, cervix, myometrium, endometrium, thyroid (for example thyroid follicular carcinoma), adrenal, prostate, skin and adnexae (for example melanoma, basal cell carcinoma, squamous cell carcinoma, keratoacanthoma, dysplastic naevus); haematological malignancies (i.e. leukemias, lymphomas) and premalignant haematological disorders and disorders of borderline malignancy including haematological malignancies and related conditions of lymphoid lineage (for example acute lymphocytic leukemia [ALL], chronic lymphocytic leukemia [CLL], B-cell lymphomas such as diffuse large B-cell lymphoma [DLBCL], follicular lymphoma, Burkitt's lymphoma, mantle cell lymphoma, MALT lymphoma, T-cell lymphomas and leukaemias, natural killer [NK] cell lymphomas, Hodgkin's lymphomas, hairy cell leukaemia, monoclonal gammopathy of uncertain significance, plasmacytoma, multiple myeloma, and post-transplant lymphoproliferative disorders), and haematological malignancies and related conditions of myeloid lineage (for example acute myelogenous leukemia [AML], chronic myelogenous leukemia [CML], chronic myelomonocytic leukemia [CMML], hypereosinophilic syndrome, myeloproliferative disorders such as polycythaemia vera, essential thrombocythaemia and primary myelofibrosis, myeloproliferative syndrome, myelodysplastic syndrome, and promyelocytic leukemia); tumours of mesenchymal origin, for example sarcomas of soft tissue, bone or cartilage such as osteosarcomas, fibrosarcomas, chondrosarcomas, rhabdomyosarcomas, leiomyosarcomas, liposarcomas, angiosarcomas, Kaposi's sarcoma, Ewing's sarcoma, synovial sarcomas, epithelioid sarcomas, gastrointestinal stromal tumours, benign and malignant histiocytomas, and dermatofibrosarcoma protuberans; tumours of the central or peripheral nervous system (for example astrocytomas, gliomas and glioblastomas, meningiomas, ependymomas, pineal tumours and schwannomas); endocrine tumours (for example pituitary tumours, adrenal tumours, islet cell tumours, parathyroid tumours, carcinoid tumours and medullary carcinoma of the thyroid); ocular and adnexal tumours (for example retinoblastoma); germ cell and trophoblastic tumours (for example teratomas, seminomas, dysgerminomas, hydatidiform moles and choriocarcinomas); and paediatric and embryonal tumours (for example medulloblastoma, neuroblastoma, Wilms tumour, and primitive neuroectodermal tumours); or syndromes, congenital or otherwise, which leave the patient susceptible to malignancy (for example Xeroderma Pigmentosum).

Many diseases are characterized by persistent and unregulated angiogenesis. Chronic proliferative diseases

are often accompanied by profound angiogenesis, which can contribute to or maintain an inflammatory and/or proliferative state, or which leads to tissue destruction through the invasive proliferation of blood vessels. Tumour growth and metastasis have been found to be angiogenesis-dependent. Compounds of the invention may therefore be useful in preventing and disrupting initiation of tumour angiogenesis. In particular, the compounds of the invention may be useful in the treatment of metastasis and metastatic cancers.

Metastasis or metastatic disease is the spread of a disease from one organ or part to another non-adjacent organ or part. The cancers which can be treated by the compounds of the invention include primary tumours (i.e. cancer cells at the originating site), local invasion (cancer cells which penetrate and infiltrate surrounding normal tissues in the local area), and metastatic (or secondary) tumours ie. tumours that have formed from malignant cells which have circulated through the bloodstream (haematogenous spread) or via lymphatics or across body cavities (trans-coelomic) to other sites and tissues in the body.

Particular cancers include hepatocellular carcinoma, melanoma, oesophageal, renal, colon, colorectal, lung e.g. mesothelioma or lung adenocarcinoma, breast, bladder, gastrointestinal, ovarian and prostate cancers.

A further aspect provides the use of a compound for the manufacture of a medicament for the treatment of a disease or condition as described herein, in particular cancer.

The compounds may also be useful in the treatment of tumour growth, pathogenesis, resistance to chemo- and radio-therapy by sensitising cells to chemotherapy and as an anti-metastatic agent.

The potency of the compounds of the invention as inhibitors of Polθ can be measured using the biological and biophysical assays set forth in the examples herein and the level of affinity exhibited by a given compound can be defined in terms of the $IC_{50}$ value. Particular compounds of the present invention are compounds having an $IC_{50}$ value of less than 1μM, more particularly less than 0.1 μM.

A role for the loss of Polθ enhancing the efficacy of CRISPR mediated gene editing has been described in WO 2017/062754. Thus, Polθ inhibitory compounds are likely to be useful in enhancing the efficiency of CRISPR based editing methodologies and/or CRISPR based editing therapeutics. Furthermore, compound mediated Polθ inhibition is likely to reduce the frequency of random integration events and thus provide a route to ameliorate any safety concerns of CRISPR mediated technology. Thus, according to a further aspect of the invention, there is provided the use of a compound of formula (I) as defined herein in a CRISPR based editing methodology and/or CRISPR based editing therapeutics, such as the enhancement of efficiency of CRISPR based editing methodology and/or CRISPR based editing therapeutics.

***Pharmaceutical Compositions***

While it is possible for the active compound to be administered alone, it is preferable to present it as a pharmaceutical composition (e.g. formulation). In one embodiment this is a sterile pharmaceutical composition.

Thus, the present invention further provides pharmaceutical compositions, as defined above, and methods of making a pharmaceutical composition comprising (e.g admixing) at least one compound of formula (I) (and sub-groups thereof as defined herein), together with one or more pharmaceutically acceptable excipients and optionally other therapeutic or prophylactic agents, as described herein.

The pharmaceutically acceptable excipient(s) can be selected from, for example, carriers (e.g. a solid, liquid or semi-solid carrier), adjuvants, diluents, fillers or bulking agents, granulating agents, coating agents, release-controlling agents, binding agents, disintegrants, lubricating agents, preservatives, antioxidants, buffering agents, suspending agents, thickening agents, flavouring agents, sweeteners, taste masking agents, stabilisers or any other excipients conventionally used in pharmaceutical compositions. Examples of excipients for various types of pharmaceutical compositions are set out in more detail below.

The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

Pharmaceutical compositions containing compounds of the formula (I) can be formulated in accordance with known techniques, see for example, Remington’s Pharmaceutical Sciences, Mack Publishing Company, Easton, PA, USA.

The pharmaceutical compositions can be in any form suitable for oral, parenteral, topical, intranasal, intrabronchial, sublingual, ophthalmic, otic, rectal, intra-vaginal, or transdermal administration. Where the compositions are intended for parenteral administration, they can be formulated for intravenous, intramuscular, intraperitoneal, subcutaneous administration or for direct delivery into a target organ or tissue by injection, infusion or other means of delivery. The delivery can be by bolus injection, short term infusion or longer term infusion and can be via passive delivery or through the utilisation of a suitable infusion pump or syringe driver.

Pharmaceutical formulations adapted for parenteral administration include aqueous and non-aqueous sterile

injection solutions which may contain anti-oxidants, buffers, bacteriostats, co-solvents, surface active agents, organic solvent mixtures, cyclodextrin complexation agents, emulsifying agents (for forming and stabilizing emulsion formulations), liposome components for forming liposomes, gellable polymers for forming polymeric gels, lyophilisation protectants and combinations of agents for, *inter alia,* stabilising the active ingredient in a soluble form and rendering the formulation isotonic with the blood of the intended recipient. Pharmaceutical formulations for parenteral administration may also take the form of aqueous and non-aqueous sterile suspensions which may include suspending agents and thickening agents (R. G. Strickly, Solubilizing Excipients in oral and injectable formulations, Pharmaceutical Research, Vol 21(2) 2004, p 201-230).

The formulations may be presented in unit-dose or multi-dose containers, for example sealed ampoules, vials and prefilled syringes, and may be stored in a freeze-dried (lyophilised) condition requiring only the addition of the sterile liquid carrier, for example water for injections, immediately prior to use. In one embodiment, the formulation is provided as an active pharmaceutical ingredient in a bottle for subsequent reconstitution using an appropriate diluent.

The pharmaceutical formulation can be prepared by lyophilising a compound of formula (I), or sub-groups thereof. Lyophilisation refers to the procedure of freeze-drying a composition. Freeze-drying and lyophilisation are therefore used herein as synonyms.

Extemporaneous injection solutions and suspensions may be prepared from sterile powders, granules and tablets.

Pharmaceutical compositions of the present invention for parenteral injection can also comprise pharmaceutically acceptable sterile aqueous or non-aqueous solutions, dispersions, suspensions or emulsions as well as sterile powders for reconstitution into sterile injectable solutions or dispersions just prior to use.

Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as sunflower oil, safflower oil, corn oil or olive oil), and injectable organic esters such as ethyl oleate. Proper fluidity can be maintained, for example, by the use of thickening or coating materials such as lecithin, by the maintenance of the required particle size in the case of dispersions, and by the use of surfactants.

The compositions of the present invention may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol, sorbic acid, and the like. It may also be desirable to include agents to adjust tonicity such as sugars, sodium chloride, and the like. Prolonged absorption of the injectable pharmaceutical form may be brought about by the inclusion of agents which delay absorption such as aluminum monostearate and gelatin.

In one particular embodiment of the invention, the pharmaceutical composition is in a form suitable for i.v. administration, for example by injection or infusion. For intravenous administration, the solution can be dosed as is, or can be injected into an infusion bag (containing a pharmaceutically acceptable excipient, such as 0.9% saline or 5% dextrose), before administration.

In another particular embodiment, the pharmaceutical composition is in a form suitable for sub-cutaneous (s.c.) administration.

Pharmaceutical dosage forms suitable for oral administration include tablets (coated or uncoated), capsules (hard or soft shell), caplets, pills, lozenges, syrups, solutions, powders, granules, elixirs and suspensions, sublingual tablets, wafers or patches such as buccal patches.

Thus, tablet compositions can contain a unit dosage of active compound together with an inert diluent or carrier such as a sugar or sugar alcohol, eg; lactose, sucrose, sorbitol or mannitol; and/or a non-sugar derived diluent such as sodium carbonate, calcium phosphate, calcium carbonate, or a cellulose or derivative thereof such as microcrystalline cellulose (MCC), methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Tablets may be designed to release the drug either upon contact with stomach fluids (immediate release tablets) or to release in a controlled manner (controlled release tablets) over a prolonged period of time or with a specific region of the GI tract. Capsule formulations may be of the hard gelatin or soft gelatin variety and can contain the active component in solid, semi-solid, or liquid form. Gelatin capsules can be formed from animal gelatin or synthetic or plant derived equivalents thereof.

The solid dosage forms (eg; tablets, capsules etc.) can be coated or un-coated. Coatings may act either as a protective film (e.g. a polymer, wax or varnish) or as a mechanism for controlling drug release or for aesthetic or identification purposes. The coating (e.g. a Eudragit ™ type polymer) can be designed to release the active component at a desired location within the gastro-intestinal tract. Thus, the coating can be selected so as to degrade under certain pH

conditions within the gastrointestinal tract, thereby selectively release the compound in the stomach or in the ileum, duodenum, jejenum or colon.

Instead of, or in addition to, a coating, the drug can be presented in a solid matrix comprising a release controlling agent, for example a release delaying agent which may be adapted to release the compound in a controlled manner in the gastrointestinal tract. Alternatively the drug can be presented in a polymer coating e.g. a polymethacrylate polymer coating, which may be adapted to selectively release the compound under conditions of varying acidity or alkalinity in the gastrointestinal tract. Alternatively, the matrix material or release retarding coating can take the form of an erodible polymer (e.g. a maleic anhydride polymer) which is substantially continuously eroded as the dosage form passes through the gastrointestinal tract. In another alternative, the coating can be designed to disintegrate under microbial action in the gut. As a further alternative, the active compound can be formulated in a delivery system that provides osmotic control of the release of the compound. Osmotic release and other delayed release or sustained release formulations (for example formulations based on ion exchange resins) may be prepared in accordance with methods well known to those skilled in the art.

The compound of formula (I) may be formulated with a carrier and administered in the form of nanoparticles, the increased surface area of the nanoparticles assisting their absorption. In addition, nanoparticles offer the possibility of direct penetration into the cell. Nanoparticle drug delivery systems are described in "Nanoparticle Technology for Drug Delivery", edited by Ram B Gupta and Uday B. Kompella, Informa Healthcare, ISBN 9781574448573, published 13th March 2006. Nanoparticles for drug delivery are also described in J. Control. Release, 2003, 91 (1-2), 167-172, and in Sinha et al., Mol. Cancer Ther. August 1, (2006) 5, 1909.

The pharmaceutical compositions typically comprise from approximately 1% (w/w) to approximately 95% (w/w) active ingredient and from 99% (w/w) to 5% (w/w) of a pharmaceutically acceptable excipient or combination of excipients. Particularly, the compositions comprise from approximately 20% (w/w) to approximately 90%,% (w/w) active ingredient and from 80% (w/w) to 10% of a pharmaceutically acceptable excipient or combination of excipients. The pharmaceutical compositions comprise from approximately 1% to approximately 95%, particularly from approximately 20% to approximately 90%, active ingredient. Pharmaceutical compositions according to the invention may be, for example, in unit dose form, such as in the form of ampoules, vials, suppositories, pre-filled syringes, dragées, tablets or capsules.

The pharmaceutically acceptable excipient(s) can be selected according to the desired physical form of the formulation and can, for example, be selected from diluents (e.g solid diluents such as fillers or bulking agents; and liquid diluents such as solvents and co-solvents), disintegrants, buffering agents, lubricants, flow aids, release controlling (e.g. release retarding or delaying polymers or waxes) agents, binders, granulating agents, pigments, plasticizers, antioxidants, preservatives, flavouring agents, taste masking agents, tonicity adjusting agents and coating agents.

The skilled person will have the expertise to select the appropriate amounts of ingredients for use in the formulations. For example tablets and capsules typically contain 0-20% disintegrants, 0-5% lubricants, 0-5% flow aids and/or 0-99% (w/w) fillers/ or bulking agents (depending on drug dose). They may also contain 0-10% (w/w) polymer binders, 0-5% (w/w) antioxidants, 0-5% (w/w) pigments. Slow release tablets would in addition contain 0-99% (w/w) release-controlling (e.g. delaying) polymers (depending on dose). The film coats of the tablet or capsule typically contain 0-10% (w/w) polymers, 0-3% (w/w) pigments, and/or 0-2% (w/w) plasticizers.

Parenteral formulations typically contain 0-20% (w/w) buffers, 0-50% (w/w) cosolvents, and/or 0-99% (w/w) Water for Injection (WFI) (depending on dose and if freeze dried). Formulations for intramuscular depots may also contain 0-99% (w/w) oils.

Pharmaceutical compositions for oral administration can be obtained by combining the active ingredient with solid carriers, if desired granulating a resulting mixture, and processing the mixture, if desired or necessary, after the addition of appropriate excipients, into tablets, dragee cores or capsules. It is also possible for them to be incorporated into a polymer or waxy matrix that allow the active ingredients to diffuse or be released in measured amounts.

The compounds of the invention can also be formulated as solid dispersions. Solid dispersions are homogeneous extremely fine disperse phases of two or more solids. Solid solutions (molecularly disperse systems), one type of solid dispersion, are well known for use in pharmaceutical technology (see (Chiou and Riegelman, J. Pharm. Sci., 60, 1281-1300 (1971)) and are useful in increasing dissolution rates and increasing the bioavailability of poorly water-soluble drugs.

This invention also provides solid dosage forms comprising the solid solution described above. Solid dosage forms include tablets, capsules, chewable tablets and dispersible or effervescent tablets. Known excipients can be blended with the solid solution to provide the desired dosage form. For example, a capsule can contain the solid solution blended with (a) a disintegrant and a lubricant, or (b) a disintegrant, a lubricant and a surfactant. In addition a capsule can contain a bulking agent, such as lactose or microcrystalline cellulose. A tablet can contain the solid solution blended with at least one disintegrant, a lubricant, a surfactant, a bulking agent and a glidant. A chewable tablet can contain the solid solution blended with a bulking agent, a lubricant, and if desired an additional sweetening agent (such as an artificial sweetener), and suitable flavours. Solid solutions may also be formed by spraying solutions of drug and a suitable polymer onto the surface of inert carriers such as sugar beads ('non-pareils'). These beads can subsequently be filled into capsules

or compressed into tablets.

The pharmaceutical formulations may be presented to a patient in "patient packs" containing an entire course of treatment in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in patient prescriptions. The inclusion of a package insert has been shown to improve patient compliance with the physician's instructions.

Compositions for topical use and nasal delivery include ointments, creams, sprays, patches, gels, liquid drops and inserts (for example intraocular inserts). Such compositions can be formulated in accordance with known methods.

Examples of formulations for rectal or intra-vaginal administration include pessaries and suppositories which may be, for example, formed from a shaped moldable or waxy material containing the active compound. Solutions of the active compound may also be used for rectal administration.

Compositions for administration by inhalation may take the form of inhalable powder compositions or liquid or powder sprays, and can be administrated in standard form using powder inhaler devices or aerosol dispensing devices. Such devices are well known. For administration by inhalation, the powdered formulations typically comprise the active compound together with an inert solid powdered diluent such as lactose.

The compounds of the formula (I) will generally be presented in unit dosage form and, as such, will typically contain sufficient compound to provide a desired level of biological activity. For example, a formulation may contain from 1 nanogram to 2 grams of active ingredient, e.g. from 1 nanogram to 2 milligrams of active ingredient. Within these ranges, particular sub-ranges of compound are 0.1 milligrams to 2 grams of active ingredient (more usually from 10 milligrams to 1 gram, e.g. 50 milligrams to 500 milligrams), or 1 microgram to 20 milligrams (for example 1 microgram to 10 milligrams, e.g. 0.1 milligrams to 2 milligrams of active ingredient).

For oral compositions, a unit dosage form may contain from 1 milligram to 2 grams, more typically 10 milligrams to 1 gram, for example 50 milligrams to 1 gram, e.g. 100 miligrams to 1 gram, of active compound.

The active compound will be administered to a patient in need thereof (for example a human or animal patient) in an amount sufficient to achieve the desired therapeutic effect.

***Methods of Treatment***

The compounds of the formula (I) and sub-groups as defined herein may be useful in the prophylaxis or treatment of a range of disease states or conditions mediated by Polθ. Thus, according to a further aspect of the invention there is provided a compound for use in a method of treating a disease state or condition mediated by Polθ (e.g. cancer) which comprises administering to a subject in need thereof a compound of formula (I) as described herein. Examples of such disease states and conditions are set out above, and in particular include cancer.

The compounds are generally administered to a subject in need of such administration, for example a human or animal patient, particularly a human.

The compounds will typically be administered in amounts that are therapeutically or prophylactically useful and which generally are non-toxic. However, in certain situations (for example in the case of life threatening diseases), the benefits of administering a compound of the formula (I) may outweigh the disadvantages of any toxic effects or side effects, in which case it may be considered desirable to administer compounds in amounts that are associated with a degree of toxicity.

The compounds may be administered over a prolonged term to maintain beneficial therapeutic effects or may be administered for a short period only. Alternatively they may be administered in a continuous manner or in a manner that provides intermittent dosing (e.g. a pulsatile manner).

A typical daily dose of the compound of formula (I) can be in the range from 100 picograms to 100 milligrams per kilogram of body weight, more typically 5 nanograms to 25 milligrams per kilogram of bodyweight, and more usually 10 nanograms to 15 milligrams per kilogram (e.g. 10 nanograms to 10 milligrams, and more typically 1 microgram per kilogram to 20 milligrams per kilogram, for example 1 microgram to 10 milligrams per kilogram) per kilogram of bodyweight although higher or lower doses may be administered where required. The compound of the formula (I) can be administered on a daily basis or on a repeat basis every 2, or 3, or 4, or 5, or 6, or 7, or 10 or 14, or 21, or 28 days for example.

The compounds of the invention may be administered orally in a range of doses, for example 1 to 1500 mg, 2 to 800 mg, or 5 to 500 mg, e.g. 2 to 200 mg or 10 to 1000 mg, particular examples of doses including 10, 20, 50 and 80 mg. The compound may be administered once or more than once each day. The compound can be administered continuously (i.e. taken every day without a break for the duration of the treatment regimen). Alternatively, the compound can be administered intermittently (i.e. taken continuously for a given period such as a week, then discontinued for a period such as a week and then taken continuously for another period such as a week and so on throughout the duration of the treatment regimen). Examples of treatment regimens involving intermittent administration include regimens wherein administration is in cycles of one week on, one week off; or two weeks on, one week off; or three weeks on, one week off; or two weeks on, two weeks off; or four weeks on two weeks off; or one week on three weeks off - for one or more cycles, e.g. 2,

3, 4, 5, 6, 7, 8, 9 or 10 or more cycles.

In one particular dosing schedule, a patient will be given an infusion of a compound of the formula (I) for periods of one hour daily for up to ten days in particular up to five days for one week, and the treatment repeated at a desired interval such as two to four weeks, in particular every three weeks.

More particularly, a patient may be given an infusion of a compound of the formula (I) for periods of one hour daily for 5 days and the treatment repeated every three weeks.

In another particular dosing schedule, a patient is given an infusion over 30 minutes to 1 hour followed by maintenance infusions of variable duration, for example 1 to 5 hours, e.g. 3 hours.

In a further particular dosing schedule, a patient is given a continuous infusion for a period of 12 hours to 5 days, an in particular a continuous infusion of 24 hours to 72 hours.

In another particular dosing schedule, a patient is given the compound orally once a week.

In another particular dosing schedule, a patient is given the compound orally once-daily for between 7 and 28 days such as 7, 14 or 28 days.

In another particular dosing schedule, a patient is given the compound orally once-daily for 1 day, 2 days, 3 days, 5 days or 1 week followed by the required amount of days off to complete a one or two week cycle.

In another particular dosing schedule, a patient is given the compound orally once-daily for 2 weeks followed by 2 weeks off.

In another particular dosing schedule, a patient is given the compound orally once-daily for 2 weeks followed by 1 week off.

In another particular dosing schedule, a patient is given the compound orally once-daily for 1 week followed by 1 week off.

Ultimately, however, the quantity of compound administered and the type of composition used will be commensurate with the nature of the disease or physiological condition being treated and will be at the discretion of the physician.

It will be appreciated that Polθ inhibitors can be used as a single agent or in combination with other anticancer agents. Combination experiments can be performed, for example, as described in Chou TC, Talalay P. Quantitative analysis of dose-effect relationships: the combined effects of multiple drugs or enzyme inhibitors. Adv Enzyme Regulat 1984;22: 27-55.

The compounds as defined herein can be administered as the sole therapeutic agent or they can be administered in combination therapy with one of more other compounds (or therapies) for treatment of a particular disease state, for example a neoplastic disease such as a cancer as hereinbefore defined. For the treatment of the above conditions, the compounds of the invention may be advantageously employed in combination with one or more other medicinal agents, more particularly, with other anti-cancer agents or adjuvants (supporting agents in the therapy) in cancer therapy. Examples of other therapeutic agents or treatments that may be administered together (whether concurrently or at different time intervals) with the compounds of the formula (I) include but are not limited to:

- Topoisomerase I inhibitors;
- Antimetabolites;
- Tubulin targeting agents;
- DNA binder and topoisomerase II inhibitors;
- Alkylating Agents;
- Monoclonal Antibodies;
- Anti-Hormones;
- Signal Transduction Inhibitors;
- Proteasome Inhibitors;
- DNA methyl transferase inhibitors;
- Cytokines and retinoids;
- Chromatin targeted therapies;
- Radiotherapy; and
- Other therapeutic or prophylactic agents.

Particular examples of anti-cancer agents or adjuvants (or salts thereof), include but are not limited to any of the agents selected from groups (i)-(xlvi), and optionally group (xlvii), below:

(i) Platinum compounds, for example cisplatin (optionally combined with amifostine), carboplatin or oxaliplatin;

(ii) Taxane compounds, for example paclitaxel, paclitaxel protein bound particles (Abraxane™), docetaxel, cabazitaxel or larotaxel;

(iii) Topoisomerase I inhibitors, for example camptothecin compounds, for example camptothecin, irinotecan(CPT11),

SN-38, or topotecan;
(iv) Topoisomerase II inhibitors, for example anti-tumour epipodophyllotoxins or podophyllotoxin derivatives for example etoposide, or teniposide;
(v) Vinca alkaloids, for example vinblastine, vincristine, liposomal vincristine (Onco-TCS), vinorelbine, vindesine, vinflunine or vinvesir;
(vi) Nucleoside derivatives, for example 5-fluorouracil (5-FU, optionally in combination with leucovorin), gemcitabine, capecitabine, tegafur, UFT, S1, cladribine, cytarabine (Ara-C, cytosine arabinoside), fludarabine, clofarabine, or nelarabine;
(vii) Antimetabolites, for example clofarabine, aminopterin, or methotrexate, azacitidine, cytarabine, floxuridine, pentostatin, thioguanine, thiopurine, 6-mercaptopurine, or hydroxyurea (hydroxycarbamide);
(viii) Alkylating agents, such as nitrogen mustards or nitrosourea, for example cyclophosphamide, chlorambucil, carmustine (BCNU), bendamustine, thiotepa, melphalan, treosulfan, lomustine (CCNU), altretamine, busulfan, dacarbazine, estramustine, fotemustine, ifosfamide (optionally in combination with mesna), pipobroman, procarbazine, streptozocin, temozolomide, uracil, mechlorethamine, methylcyclohexylchloroethylnitrosurea, or nimustine (ACNU);
(ix) Anthracyclines, anthracenediones and related drugs, for example daunorubicin, doxorubicin (optionally in combination with dexrazoxane), liposomal formulations of doxorubicin (eg. Caelyx™, Myocet™, Doxil™), idarubicin, mitoxantrone, epirubicin, amsacrine, or valrubicin;
(x) Epothilones, for example ixabepilone, patupilone, BMS-310705, KOS-862 and ZK-EPO, epothilone A, epothilone B, desoxyepothilone B (also known as epothilone D or KOS-862), aza-epothilone B (also known as BMS-247550), aulimalide, isolaulimalide, or luetherobin;
(xi) DNA methyl transferase inhibitors, for example temozolomide, azacytidine or decitabine, or SGI-110;
(xii) Antifolates, for example methotrexate, pemetrexed disodium, or raltitrexed;
(xiii) Cytotoxic antibiotics, for example antinomycin D, bleomycin, mitomycin C, dactinomycin, carminomycin, daunomycin, levamisole, plicamycin, or mithramycin;
(xiv) Tubulin-binding agents, for example combrestatin, colchicines or nocodazole;
(xv) Signal Transduction inhibitors such as Kinase inhibitors (e.g. EGFR (epithelial growth factor receptor) inhibitors, VEGFR (vascular endothelial growth factor receptor) inhibitors, PDGFR (platelet-derived growth factor receptor) inhibitors, MTKI (multi target kinase inhibitors), Raf inhibitors, mTOR inhibitors for example imatinib mesylate, erlotinib, gefitinib, dasatinib, lapatinib, dovotinib, axitinib, nilotinib, vandetanib, vatalinib, pazopanib, sorafenib, sunitinib, temsirolimus, everolimus (RAD 001), vemurafenib (PLX4032/RG7204), dabrafenib, encorafenib or an IκB kinase inhibitor such as SAR-113945, bardoxolone, BMS-066, BMS-345541, IMD-0354, IMD-2560, or IMD-1041, or MEK inhibitors such as Selumetinib (AZD6244) and Trametinib (GSK121120212);
(xvi) Aurora kinase inhibitors for example AT9283, barasertib (AZD1152), TAK-901, MK0457 (VX680), cenisertib (R-763), danusertib (PHA-739358), alisertib (MLN-8237), or MP-470;
(xvii) CDK inhibitors for example AT7519, roscovitine, seliciclib, alvocidib (flavopiridol), dinaciclib (SCH-727965), 7-hydroxy-staurosporine (UCN-01), JNJ-7706621, BMS-387032 (a.k.a. SNS-032), PHA533533, PD332991, ZK-304709, or AZD-5438;
(xviii) PKA/B inhibitors and PKB (akt) pathway inhibitors for example AKT inhibitors such as KRX-0401 (perifosine/NSC 639966), ipatasertib (GDC-0068; RG-7440), afuresertib (GSK-2110183; 2110183), MK-2206, MK-8156, AT13148, AZD-5363, triciribine phosphate (VQD-002; triciribine phosphate monohydrate (API-2; TCN-P; TCN-PM; VD-0002), RX-0201, NL-71-101, SR-13668, PX-316, AT13148, AZ-5363, Semaphore, SF1126, or Enzastaurin HCl (LY317615) or MTOR inhibitors such as rapamycin analogues such as RAD 001 (everolimus), CCI 779 (temsirolemus), AP23573 and ridaforolimus, sirolimus (originally known as rapamycin), AP23841 and AP23573, calmodulin inhibitors e.g. CBP-501 (forkhead translocation inhibitors), enzastaurin HCl (LY317615) or PI3K Inhibitors such as dactolisib (BEZ235), buparlisib (BKM-120; NVP-BKM-120), BYL719, copanlisib (BAY-80-6946), ZSTK-474, CUDC-907, apitolisib (GDC-0980; RG-7422), pictilisib (pictrelisib, GDC-0941, RG-7321), GDC-0032, GDC-0068, GSK-2636771, idelalisib (formerly CAL-101, GS 1101, GS-1101), MLN1117 (INK1117), MLN0128 (INK128), IPI-145 (INK1197), LY-3023414, ipatasertib, afuresertib, MK-2206, MK-8156, LY-3023414, LY294002, SF1126 or PI-103, or sonolisib (PX-866);
(xix) Hsp90 inhibitors for example AT13387, herbimycin, geldanamycin (GA), 17-allylamino-17-desmethoxygeldanamycin (17-AAG) e.g. NSC-330507, Kos-953 and CNF-1010, 17-dimethylaminoethylamino-17-demethoxygeldanamycin hydrochloride (17-DMAG) e.g. NSC-707545 and Kos-1022, NVP-AUY922 (VER-52296), NVP-BEP800, CNF-2024 (BIIB-021 an oral purine), ganetespib (STA-9090), SNX-5422 (SC-102112) or IPI-504;
(xx) Monoclonal Antibodies (unconjugated or conjugated to radioisotopes, toxins or other agents), antibody derivatives and related agents, such as anti-CD, anti-VEGFR, anti-HER2, anti-CTLA4, anti-PD-1 or anti-EGFR antibodies, for example rituximab (CD20), ofatumumab (CD20), ibritumomab tiuxetan (CD20), GA101 (CD20), tositumomab (CD20), epratuzumab (CD22), lintuzumab (CD33), gemtuzumab ozogamicin (CD33), alemtuzumab (CD52), galiximab (CD80), trastuzumab (HER2 antibody), pertuzumab (HER2), trastuzumab-DM1 (HER2), ertumaxomab (HER2 and CD3), cetuximab (EGFR), panitumumab (EGFR), necitumumab (EGFR), nimotuzumab (EGFR), bevacizumab (VEGF), catumaxumab (EpCAM and CD3), abagovomab (CA125), farletuzumab (folate receptor), elotuzumab (CS1), denosumab (RANK ligand), figitumumab (IGF1R), CP751,871 (IGF1R), mapatumumab (TRAIL receptor), metMAB (met), mitumomab (GD3 ganglioside), naptumomab estafenatox (5T4), siltuximab (IL6), or immunomodulating agents such as CTLA-4 blocking antibodies and/or antibodies against PD-1 and PD-L1 and/or PD-L2 for example ipilimumab (CTLA4), MK-3475 (pembrolizumab, formerly lambrolizumab, anti-PD-1), nivolumab (anti-PD-1), BMS-936559 (anti- PD-L1), MPDL320A, AMP-514 or MEDI4736 (anti-PD-L1), or tremelimumab (formerly ticilimumab, CP-675,206, anti-CTLA-4);

(xxi) Estrogen receptor antagonists or selective estrogen receptor modulators (SERMs) or inhibitors of estrogen synthesis, for example tamoxifen, fulvestrant, toremifene, droloxifene, faslodex, or raloxifene;

(xxii) Aromatase inhibitors and related drugs, such as exemestane, anastrozole, letrazole, testolactone aminoglutethimide, mitotane or vorozole;

(xxiii) Antiandrogens (i.e. androgen receptor antagonists) and related agents for example bicalutamide, nilutamide, flutamide, cyproterone, or ketoconazole;

(xxiv) Hormones and analogues thereof such as medroxyprogesterone, diethylstilbestrol (a.k.a. diethylstilboestrol) or octreotide;

(xxv) Steroids for example dromostanolone propionate, megestrol acetate, nandrolone (decanoate, phenpropionate), fluoxymestrone or gossypol,

(xxvi) Steroidal cytochrome P450 17alpha-hydroxylase-17,20-lyase inhibitor (CYP17), e.g. abiraterone;

(xxvii) Gonadotropin releasing hormone agonists or antagonists (GnRAs) for example abarelix, goserelin acetate, histrelin acetate, leuprolide acetate, triptorelin, buserelin, or deslorelin;

(xxviii) Glucocorticoids, for example prednisone, prednisolone, dexamethasone;

(xxix) Differentiating agents, such as retinoids, rexinoids, vitamin D or retinoic acid and retinoic acid metabolism blocking agents (RAMBA) for example accutane, alitretinoin, bexarotene, or tretinoin;

(xxx) Farnesyltransferase inhibitors for example tipifarnib;

(xxxi) Chromatin targeted therapies such as histone deacetylase (HDAC) inhibitors for example panobinostat, resminostat, abexinostat, vorinostat, romidepsin, belinostat, entinostat, quisinostat, pracinostat, tefinostat, mocetinostat, givinostat, CUDC-907, CUDC-101, ACY-1215, MGCD-290, EVP-0334, RG-2833, 4SC-202, romidepsin, AR-42 (Ohio State University), CG-200745, valproic acid, CKD-581, sodium butyrate, suberoylanilide hydroxamide acid (SAHA), depsipeptide (FR 901228), dacinostat (NVP-LAQ824), R306465/ JNJ-16241199, JNJ-26481585, trichostatin A, chlamydocin, A-173, JNJ-MGCD-0103, PXD-101, or apicidin;

(xxxii) Proteasome Inhibitors for example bortezomib, carfilzomib, delanzomib (CEP-18770), ixazomib (MLN-9708), oprozomib (ONX-0912) or marizomib;

(xxxiii) Photodynamic drugs for example porfimer sodium or temoporfin;

(xxxiv) Marine organism-derived anticancer agents such as trabectidin;

(xxxv) Radiolabelled drugs for radioimmunotherapy for example with a beta particle-emitting isotope (e.g. , Iodine -131, Yittrium -90) or an alpha particle-emitting isotope (e.g., Bismuth-213 or Actinium-225) for example ibritumomab or Iodine tositumomab;

(xxxvi) Telomerase inhibitors for example telomestatin;

(xxxvii) Matrix metalloproteinase inhibitors for example batimastat, marimastat, prinostat or metastat;

(xxxviii) Recombinant interferons (such as interferon-$\gamma$ and interferon $\alpha$) and interleukins (e.g. interleukin 2), for example aldesleukin, denileukin diftitox, interferon alfa 2a, interferon alfa 2b, or peginterferon alfa 2b;

(xxxix) Selective immunoresponse modulators for example thalidomide, or lenalidomide;

(xl) Therapeutic Vaccines such as sipuleucel-T (Provenge) or OncoVex;

(xli) Cytokine-activating agents include Picibanil, Romurtide, Sizofiran, Virulizin, or Thymosin;

(xlii) Arsenic trioxide;

(xliii) Inhibitors of G-protein coupled receptors (GPCR) for example atrasentan;

(xliv) Enzymes such as L-asparaginase, pegaspargase, rasburicase, or pegademase;

(xlv) DNA repair inhibitors such as PARP inhibitors for example, olaparib, velaparib, iniparib, rucaparib (AG-014699 or PF-01367338), talazoparib or AG-014699;

(xlvi) DNA damage response inhibitors such as ATM inhibitors AZD0156 MS3541, ATR inhibitors AZD6738, M4344, M6620 wee1 inhibitor AZD1775;

(xlvii) Agonists of Death receptor (e.g. TNF-related apoptosis inducing ligand (TRAIL) receptor), such as mapatumumab (formerly HGS-ETR1), conatumumab (formerly AMG 655), PRO95780, lexatumumab, dulanermin, CS-1008, apomab or recombinant TRAIL ligands such as recombinant Human TRAIL/Apo2 Ligand;

(xlviii) Prophylactic agents (adjuncts); i.e. agents that reduce or alleviate some of the side effects associated with chemotherapy agents, for example

- anti-emetic agents,
- agents that prevent or decrease the duration of chemotherapy-associated neutropenia and prevent complications that arise from reduced levels of platelets, red blood cells or white blood cells, for example interleukin-11 (e.g. oprelvekin), erythropoietin (EPO) and analogues thereof (e.g. darbepoetin alfa), colony-stimulating factor analogs such as granulocyte macrophage-colony stimulating factor (GM-CSF) (e.g. sargramostim), and granulocyte-colony stimulating factor (G-CSF) and analogues thereof (e.g. filgrastim, pegfilgrastim),
- agents that inhibit bone resorption such as denosumab or bisphosphonates e.g. zoledronate, zoledronic acid, pamidronate and ibandronate,
- agents that suppress inflammatory responses such as dexamethasone, prednisone, and prednisolone,
- agents used to reduce blood levels of growth hormone and IGF-I (and other hormones) in patients with acromegaly or other rare hormone-producing tumours, such as synthetic forms of the hormone somatostatin e.g. octreotide acetate,
- antidote to drugs that decrease levels of folic acid such as leucovorin, or folinic acid,
- agents for pain e.g. opiates such as morphine, diamorphine and fentanyl,
- non-steroidal anti-inflammatory drugs (NSAID) such as COX-2 inhibitors for example celecoxib, etoricoxib and lumiracoxib,
- agents for mucositis e.g. palifermin,
- agents for the treatment of side-effects including anorexia, cachexia, oedema or thromoembolic episodes, such as megestrol acetate.

In one embodiment the anticancer is selected from recombinant interferons (such as interferon-$\gamma$ and interferon $\alpha$) and interleukins (e.g. interleukin 2), for example aldesleukin, denileukin diftitox, interferon alfa 2a, interferon alfa 2b, or peginterferon alfa 2b; interferon-$\alpha$2 (500 $\mu$/ml) in particular interferon-$\beta$; and signal transduction inhibitors such as kinase inhibitors (e.g. EGFR (epithelial growth factor receptor) inhibitors, VEGFR (vascular endothelial growth factor receptor) inhibitors, PDGFR (platelet-derived growth factor receptor) inhibitors, MTKI (multi target kinase inhibitors), Raf inhibitors, mTOR inhibitors for example imatinib mesylate, erlotinib, gefitinib, dasatinib, lapatinib, dovotinib, axitinib, nilotinib, vandetanib, vatalinib, pazopanib, sorafenib, sunitinib, temsirolimus, everolimus (RAD 001), vemurafenib (PLX4032/RG7204), dabrafenib, encorafenib or an I$\kappa$B kinase inhibitor such as SAR-113945, bardoxolone, BMS-066, BMS-345541, IMD-0354, IMD-2560, or IMD-1041, or MEK inhibitors such as Selumetinib (AZD6244) and Trametinib (GSK121120212), in particular Raf inhibitors (e.g. vemurafenib) or MEK inhibitors (e.g. trametinib).

Each of the compounds present in the combinations of the invention may be given in individually varying dose schedules and via different routes. As such, the posology of each of the two or more agents may differ: each may be administered at the same time or at different times. A person skilled in the art would know through his or her common general knowledge the dosing regimes and combination therapies to use. For example, the compound of the invention may be using in combination with one or more other agents which are administered according to their existing combination regimen. Examples of standard combination regimens are provided below.

The taxane compound is advantageously administered in a dosage of 50 to 400 mg per square meter ($mg/m^2$) of body surface area, for example 75 to 250 $mg/m^2$, particularly for paclitaxel in a dosage of about 175 to 250 $mg/m^2$ and for docetaxel in about 75 to 150 $mg/m^2$ per course of treatment.

The camptothecin compound is advantageously administered in a dosage of 0.1 to 400 mg per square meter ($mg/m^2$) of body surface area, for example 1 to 300 $mg/m^2$, particularly for irinotecan in a dosage of about 100 to 350 $mg/m^2$ and for topotecan in about 1 to 2 $mg/m^2$ per course of treatment.

The anti-tumour podophyllotoxin derivative is advantageously administered in a dosage of 30 to 300 mg per square meter ($mg/m^2$) of body surface area, for example 50 to 250$mg/m^2$, particularly for etoposide in a dosage of about 35 to 100 $mg/m^2$ and for teniposide in about 50 to 250 $mg/m^2$ per course of treatment.

The anti-tumour vinca alkaloid is advantageously administered in a dosage of 2 to 30 mg per square meter ($mg/m^2$) of body surface area, particularly for vinblastine in a dosage of about 3 to 12 $mg/m^2$ , for vincristine in a dosage of about 1 to 2 $mg/m^2$ , and for vinorelbine in dosage of about 10 to 30 $mg/m^2$ per course of treatment.

The anti-tumour nucleoside derivative is advantageously administered in a dosage of 200 to 2500 mg per square meter ($mg/m^2$) of body surface area, for example 700 to 1500 $mg/m^2$, particularly for 5-FU in a dosage of 200 to 500$mg/m^2$, for gemcitabine in a dosage of about 800 to 1200 $mg/m^2$ and for capecitabine in about 1000 to 2500 $mg/m^2$ per course of treatment.

The alkylating agents such as nitrogen mustard or nitrosourea is advantageously administered in a dosage of 100 to 500 mg per square meter ($mg/m^2$) of body surface area, for example 120 to 200 $mg/m^2$, particularly for cyclophosphamide in a dosage of about 100 to 500 $mg/m^2$ , for chlorambucil in a dosage of about 0.1 to 0.2 mg/kg, for carmustine in a dosage of about 150 to 200 $mg/m^2$ , and for lomustine in a dosage of about 100 to 150 $mg/m^2$ per course of treatment.

The anti-tumour anthracycline derivative is advantageously administered in a dosage of 10 to 75 mg per square

meter ($mg/m^2$) of body surface area, for example 15 to 60 $mg/m^2$, particularly for doxorubicin in a dosage of about 40 to 75 $mg/m^2$, for daunorubicin in a dosage of about 25 to 45$mg/m^2$ , and for idarubicin in a dosage of about 10 to 15 $mg/m^2$ per course of treatment.

The antiestrogen agent is advantageously administered in a dosage of about 1 to 100 mg daily depending on the particular agent and the condition being treated. Tamoxifen is advantageously administered orally in a dosage of 5 to 50 mg, particularly 10 to 20 mg twice a day, continuing the therapy for sufficient time to achieve and maintain a therapeutic effect. Toremifene is advantageously administered orally in a dosage of about 60mg once a day, continuing the therapy for sufficient time to achieve and maintain a therapeutic effect. Anastrozole is advantageously administered orally in a dosage of about 1mg once a day. Droloxifene is advantageously administered orally in a dosage of about 20-100mg once a day. Raloxifene is advantageously administered orally in a dosage of about 60mg once a day. Exemestane is advantageously administered orally in a dosage of about 25mg once a day.

Antibodies are advantageously administered in a dosage of about 1 to 5 mg per square meter ($mg/m^2$) of body surface area, or as known in the art, if different. Trastuzumab is advantageously administered in a dosage of 1 to 5 mg per square meter ($mg/m^2$) of body surface area, particularly 2 to 4$mg/m^2$ per course of treatment.

Where the compound of the formula (I) is administered in combination therapy with one, two, three, four or more other therapeutic agents (particularly one or two, more particularly one), the compounds can be administered simultaneously or sequentially. In the latter case, the two or more compounds will be administered within a period and in an amount and manner that is sufficient to ensure that an advantageous or synergistic effect is achieved. When administered sequentially, they can be administered at closely spaced intervals (for example over a period of 5-10 minutes) or at longer intervals (for example 1, 2, 3, 4 or more hours apart, or even longer periods apart where required), the precise dosage regimen being commensurate with the properties of the therapeutic agent(s). These dosages may be administered for example once, twice or more per course of treatment, which may be repeated for example every 7, 14, 21 or 28 days.

In one embodiment is provided a compound of formula (I) for the manufacture of a medicament for use in therapy wherein said compound is used in combination with one, two, three, or four other therapeutic agents. In another embodiment is provided a medicament for treating cancer which comprises a compound of formula (I) wherein said medicament is used in combination with one, two, three, or four other therapeutic agents. The invention further provides use of a compound of formula (I) for the manufacture of a medicament for enhancing or potentiating the response rate in a patient suffering from a cancer where the patient is being treated with one, two, three, or four other therapeutic agents.

It will be appreciated that the particular method and order of administration and the respective dosage amounts and regimes for each component of the combination will depend on the particular other medicinal agent and compound of the present invention being administered, their route of administration, the particular tumour being treated and the particular host being treated. The optimum method and order of administration and the dosage amounts and regime can be readily determined by those skilled in the art using conventional methods and in view of the information set out herein.

The weight ratio of the compound according to the present invention and the one or more other anticancer agent(s) when given as a combination may be determined by the person skilled in the art. Said ratio and the exact dosage and frequency of administration depends on the particular compound according to the invention and the other anticancer agent(s) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention. A particular weight ratio for the present compound of formula (I) and another anticancer agent may range from 1/10 to 10/1, more in particular from 1/5 to 5/1, even more in particular from 1/3 to 3/1.

The compounds of the invention may also be administered in conjunction with non-chemotherapeutic treatments such as radiotherapy, photodynamic therapy, gene therapy; surgery and controlled diets.

The compounds of the present invention also have therapeutic applications in sensitising tumour cells for radiotherapy and chemotherapy. Hence the compounds of the present invention can be used as "radiosensitizer" and/or "chemosensitizer" or can be given in combination with another "radiosensitizer" and/or "chemosensitizer". In one embodiment the compound of the invention is for use as chemosensitiser.

The term "radiosensitizer" is defined as a molecule administered to patients in therapeutically effective amounts to increase the sensitivity of the cells to ionizing radiation and/or to promote the treatment of diseases which are treatable with ionizing radiation.

The term "chemosensitizer" is defined as a molecule administered to patients in therapeutically effective amounts to increase the sensitivity of cells to chemotherapy and/or promote the treatment of diseases which are treatable with chemotherapeutics.

In one embodiment the compound of the invention is administered with a "radiosensitizer" and/or "chemosensitizer". In one embodiment the compound of the invention is administered with an "immune sensitizer".

The term "immune sensitizer" is defined as a molecule administered to patients in therapeutically effective

amounts to increase the sensitivity of cells to a Polθ inhibitor.

Many cancer treatment protocols currently employ radiosensitizers in conjunction with radiation of x-rays. Examples of x-ray activated radiosensitizers include, but are not limited to, the following: metronidazole, misonidazole, desmethylmisonidazole, pimonidazole, etanidazole, nimorazole, mitomycin C, RSU 1069, SR 4233, EO9, RB 6145, nicotinamide, 5-bromodeoxyuridine (BUdR), 5- iododeoxyuridine (IUdR), bromodeoxycytidine, fluorodeoxyuridine (FudR), hydroxyurea, cisplatin, and therapeutically effective analogs and derivatives of the same.

Photodynamic therapy (PDT) of cancers employs visible light as the radiation activator of the sensitizing agent. Examples of photodynamic radiosensitizers include the following, but are not limited to: hematoporphyrin derivatives, Photofrin, benzoporphyrin derivatives, tin etioporphyrin, pheoborbide-a, bacteriochlorophyll-a, naphthalocyanines, phthalocyanines, zinc phthalocyanine, and therapeutically effective analogs and derivatives of the same.

Radiosensitizers may be administered in conjunction with a therapeutically effective amount of one or more other compounds, including but not limited to: compounds of the invention; compounds which promote the incorporation of radiosensitizers to the target cells; compounds which control the flow of therapeutics, nutrients, and/or oxygen to the target cells; chemotherapeutic agents which act on the tumour with or without additional radiation; or other therapeutically effective compounds for treating cancer or other diseases.

Chemosensitizers may be administered in conjunction with a therapeutically effective amount of one or more other compounds, including but not limited to: compounds of the invention; compounds which promote the incorporation of chemosensitizers to the target cells; compounds which control the flow of therapeutics, nutrients, and/or oxygen to the target cells; chemotherapeutic agents which act on the tumour or other therapeutically effective compounds for treating cancer or other disease. Calcium antagonists, for example verapamil, are found useful in combination with antineoplastic agents to establish chemosensitivity in tumor cells resistant to accepted chemotherapeutic agents and to potentiate the efficacy of such compounds in drug-sensitive malignancies.

Examples of immune sensitizers include the following, but are not limited to: immunomodulating agents, for example monoclonal antibodies such as immune checkpoint antibodies [e.g. CTLA-4 blocking antibodies and/or antibodies against PD-1 and PD-L1 and/or PD-L2 for example ipilimumab (CTLA4), MK-3475 (pembrolizumab, formerly lambrolizumab, anti-PD-1), nivolumab (anti-PD-1), BMS-936559 (anti- PD-L1), MPDL320A, AMP-514 or MEDI4736 (anti-PD-L1), or tremelimumab (formerly ticilimumab, CP-675,206, anti-CTLA-4)]; or Signal Transduction inhibitors; or cytokines (such as recombinant interferons); or oncolytic viruses; or immune adjuvants (e.g. BCG).

Immune sensitizers may be administered in conjunction with a therapeutically effective amount of one or more other compounds, including but not limited to: compounds of the invention; compounds which promote the incorporation of immune sensitizers to the target cells; compounds which control the flow of therapeutics, nutrients, and/or oxygen to the target cells; therapeutic agents which act on the tumour or other therapeutically effective compounds for treating cancer or other disease.

For use in combination therapy with another chemotherapeutic agent, the compound of the formula (I) and one, two, three, four or more other therapeutic agents can be, for example, formulated together in a dosage form containing two, three, four or more therapeutic agents i.e. in a unitary pharmaceutical composition containing all agents. In an alternative embodiment, the individual therapeutic agents may be formulated separately and presented together in the form of a kit, optionally with instructions for their use.

In one embodiment is provided a combination of a compound of formula (I) with one or more (e.g. 1 or 2) other therapeutic agents (e.g. anticancer agents as described above). In a further embodiment is provided a combination of a Polθ inhibitor as described herein and a PI3K/AKT pathway inhibitor selected from: apitolisib, buparlisib, Copanlisib, pictilisib, ZSTK-474, CUDC-907, GSK-2636771, LY-3023414, ipatasertib, afuresertib, MK-2206, MK-8156, Idelalisib, BEZ235 (dactolisib), BYL719, GDC- 0980, GDC-0941, GDC-0032 and GDC-0068.

In another embodiment is provided a compound of formula (I) in combination with one or more (e.g. 1 or 2) other therapeutic agents (e.g. anticancer agents) for use in therapy, such as in the prophylaxis or treatment of cancer.

In one embodiment the pharmaceutical composition comprises a compound of formula (I) together with a pharmaceutically acceptable carrier and optionally one or more therapeutic agent(s).

In another embodiment the invention relates to the use of a combination according to the invention in the manufacture of a pharmaceutical composition for inhibiting the growth of tumour cells.

In a further embodiment the invention relates to a product containing a compound of formula (I) and one or more anticancer agent, as a combined preparation for simultaneous, separate or sequential use in the treatment of patients suffering from cancer.

## EXAMPLES

The invention will now be illustrated, but not limited, by reference to the specific embodiments described in the following examples.

**Abbreviations**

| | |
|---|---|
| AIBN | Azobisisobutyronitrile |
| aq. | Aqueous |
| Boc | *tert*-Butyloxycarbonyl |
| Cbz | Benzyloxycarbonyl |
| CDI | 1,1'-Carbonyldiimidazole |
| DABCO | 1,4-Diazabicyclo[2.2.2]octane |
| DAST | Diethylaminosulfur trifluoride |
| DBU | 1,8-Diazabicyclo[5.4.0]undec-7-ene |
| DCE | 1,2-Dichloroethane |
| DCM | Dichloromethane |
| DEAD | Diethyl azodicarboxylate |
| DIPEA | N,N-Diisopropylethylamine |
| DMA | Dimethylacetamide |
| DMAP | 4-Dimethylaminopyridine |
| DMF | Dimethylformamide |
| DMP | Dess-Martin Periodinane |
| DMSO | Dimethylsulfoxide |
| DPPP | 1,3-Bis(diphenylphosphino)propane |
| EDCI | *N*-(3-Dimethylaminopropyl)-N'-ethylcarbodiimide |
| EtOH | Ethanol |
| EtOAc | Ethyl acetate |
| FA | Formic acid |
| Fmoc | Fluorenylmethoxycarbonyl |
| h | Hour(s) |
| HATU | 1-[Bis(dimethylamino)methylene]-1H-1,2,3-triazolo[4,5-b]pyridinium 3-oxide hexafluorophosphate |
| HFIP | 1,1,1,3,3,3-Hexafluoropropan-2-ol |
| HMDS | Bis(trimethylsilyl)amine |
| HOBt | Hydroxybenzotriazole |
| HPLC | High-performance liquid chromatography |
| LDA | Lithium diisopropylamide |
| *m*-CPBA | *meta*-Chloroperoxybenzoic acid |
| Me | Methyl |
| MeCN | Acetonitrile |
| MeOH | Methanol |
| min | Minutes |
| Ms | Methanesulfonyl |
| MTBE | Methyl *tert-butyl* ether |
| NBS | *N*-Bromosuccinimide |
| NCS | *N*-Chlorosuccinimide |
| NIS | *N*-Iodosuccinimide |
| NMO | 4-Methylmorpholine 4-oxide |
| NMP | *N*-Methylpyrrolidinone |
| NMR | Nuclear magnetic resonance |
| PE | Petroleum ether |
| PPTS | Pyridinium *p*-toluenesulfonate |
| rt | Room temperature or ambient temperature |
| sat. | Saturated solution |
| SFC | Supercritical Fluid Chromatography |
| T3P | Propanephosphonic acid anhydride |
| TBAB | Tetrabutylammonium bromide |
| TBAF | Tetra-*n*-butylammonium fluoride |
| TBDPS | *tert*-Butyldiphenylsilyl |
| TBS | *tert*-Butyldimethylsilyl |
| TEA | Triethylamine |

| | |
|---|---|
| TFA | Trifluoroacetic acid |
| THF | Tetrahydrofuran |
| TLC | Thin layer chromatography |
| TsOH | *p*-Toluenesulfonic acid monohydrate |

**Typical Preparative HPLC method**

For purification of samples by HPLC the following columns were typically used; SunFire C18, Xtimate C18, Phenomenex Gemini, Phenomenex Synergi C18, Phenomenex Luna, Waters Xbridge C18, Boston Prime C18 and Shim-pack C18. Typical mobile phases used were water and MeCN, with either acidic or basic additives, such as formic acid (0.1% v/v) or ammonium hydroxide (0.05% v/v). A typical method started with 95% water:5% MeCN and decreasingly polar ratios of water and MeCN over a period of 5 to 12 min, with a typical flow rate of 25 mL/min.

**Intermediate 1: 2-(*tert*-butyl) 3-methyl (2*S*,3*S*)-5-oxopyrrolidine-2,3-dicarboxylate**

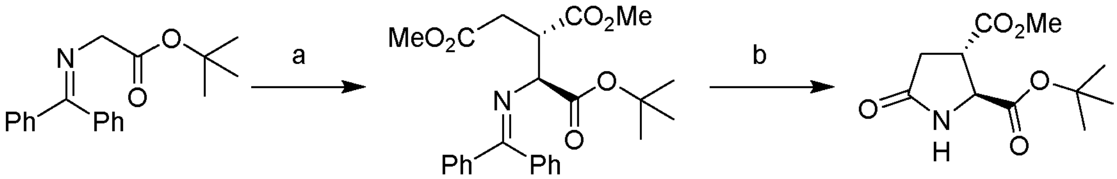


**Step a.** To a solution of *tert-butyl* 2-((diphenylmethylene)amino)acetate (CAS: 81477-94-3; 100 g, 339 mmol) and dimethyl fumarate (CAS: 624-49-7; 73.2 g, 508 mmol) in EtOAc (1 L) was added (*S*)-2-((2,3-bis(dicyclohexylamino) cycloprop-2-en-1-ylidene)amino)propan-1-ol (CAS: 1808186-23-3, prepared as described in J. S. Bander et. al. Chem. Sci. 2015, 6, 1537; 18.5 g, 33.9 mmol). The reaction mixture was stirred at rt for 48 h. Upon completion, the reaction mixture was evaporated and the crude product was purified by column chromatography (hexane/EtOAc = 4/1) to give *1*-(*tert*-butyl) 2,3-dimethyl (1S,2S)-1-((diphenylmethylene)amino)propane-1,2,3-tricarboxylate as a colourless oil (140 g, 94% yield).

**Step b.** To a solution of *1*-(*tert*-butyl) 2,3-dimethyl (1*S,*2*S*)-1-((diphenylmethylene)amino)propane-1,2,3-tricarboxylate (140 g, 318.5 mmol) in THF (1.4 L) was added 15% w/w aq. citric acid (1.4 L). The reaction mixture was stirred at rt for 48 h. Upon completion, the reaction was quenched with sat. aq. $NaHCO_3$ and extracted with EtOAc (3 x 1 L). The combined organic layers were dried over $Na_2SO_4$ and evaporated. The crude product was purified by column chromatography (hexane/EtOAc = 3/7) to give the title compound as a white solid (70 g, 90% yield).

$^1$H NMR (400 MHz, $CDCl_3$) δ ppm 6.63 (br s, 1H), 4.46 (d, *J* = 6.0 Hz, 1H), 3.77 (s, 3H), 3.37 (dt, *J* = 6.0, 8.0, 9.2 Hz, 1H), 2.76 - 2.55 (m, 2H), 1.47 (s, 9H).

**Intermediate 2a: *tert*-butyl (2-fluoro-6-(methylamino)phenyl)carbamate**

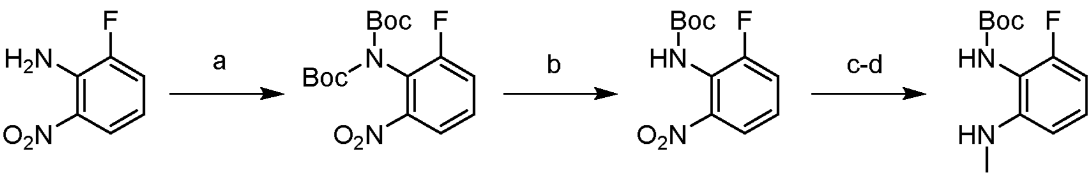


**Step** a. To a solution of 2-fluoro-6-nitroaniline (CAS: 17809-36-8; 100 g, 641 mmol) in DCM (500 mL) was added di-*tert*-butyl dicarbonate (308 g, 1.41 mol), TEA (130 g, 1.28 mol) and DMAP (7.83 g, 64.1 mmol). The mixture was stirred at 40 °C for 12 h. Upon completion, the reaction mixture was quenched with sat. aq. $NH_4Cl$ (500 mL) at 25 °C, diluted with water (500 mL) and extracted with DCM (3 x 1 L). The combined organic layers were washed with brine (3 x 500 mL), dried over $Na_2SO_4$ and evaporated. The crude product was triturated (PE/EtOAc = 20/1, 500 mL) to give *tert-butyl* (*tert*-butoxycarbonyl)(2-fluoro-6-nitrophenyl)carbamate (210 g, 92% yield) as a yellow solid.

$^1$H NMR (400 MHz, $CDCl_3$) δ ppm 7.87 (d, *J* = 8.0 Hz, 1H), 7.53 - 7.36 (m, 2H), 1.41 (s, 18H). **Step b.** To a solution of *tert-butyl* (*tert*-butoxycarbonyl)(2-fluoro-6-nitrophenyl)carbamate (210 g, 589 mmol) in DCM (2.1 L) was added TFA (87.4 g, 766 mmol) and the mixture was stirred at 25 °C for 12 h. Upon completion, the reaction mixture was quenched with sat. aq. $Na_2CO_3$ (500 mL), diluted with water (500 mL) and extracted with DCM (3 x 1 L). The combined organic layers were

washed with brine (3 x 500 mL), dried over $Na_2SO_4$ and evaporated to give *tert*-butyl (2-fluoro-6-nitrophenyl)carbamate (145 g, 96% yield) as a yellow solid.

$^1H$ NMR (400 MHz, $CDCl_3$) δ ppm 7.82 - 7.78 (m, 1H), 7.38 - 7.30 (m, 2H), 7.23 - 7.15 (m, 1H), 1.43 (s, 9H).

**Step c.** To a solution of *tert*-butyl (2-fluoro-6-nitrophenyl)carbamate (145 g, 567 mmol) in MeOH (2 L) was added 10% Pd/C (15 g) and the mixture was stirred at 25 °C for 12 h under a $H_2$ atmosphere (15 psi). Upon completion, the reaction mixture was filtered and evaporated to give *tert*-butyl (2-amino-6-fluorophenyl)carbamate (128 g, 99% yield) as an off-white solid. $^1H$ NMR (400 MHz, $CDCl_3$) δ ppm 7.01 - 6.95 (m, 1H), 6.60 - 6.46 (m, 2H), 6.10 (br s, 1H), 4.02 (br s, 2H), 1.51 (s, 9H).

**Step d.** To a solution of *tert*-butyl (2-amino-6-fluorophenyl)carbamate (128 g, 566 mmol) in MeOH (1.5 L) was added paraformaldehyde (25.5 g, 849 mmol) and sodium methoxide (306 g, 5.66 mol). The reaction mixture was stirred at 25 °C for 12 h, and then $NaBH_3CN$ (107 g, 2.83 mol) was slowly added and stirred at 25 °C for 12 h. Upon completion, the reaction mixture was quenched with sat. aq. $NH_4Cl$ (500 mL), diluted with water (500 mL) and extracted with EtOAc (3 x 1 L). The combined organic layers were washed with brine (3 x 500 mL), dried over $Na_2SO_4$ and evaporated. The crude product was purified by column chromatography (PE/EtOAc = 10/1) to give the title compound (56 g, 41% yield) as a yellow solid.

m/z ES+ $[M+H]^+$ 241.2; $^1H$ NMR (400 MHz, $CDCl_3$) δ ppm 7.14 - 7.07 (m, 1H), 6.50 - 6.43 (m, 2H), 5.81 (br s, 1H), 4.49 (br s, 1H), 2.87 (s, 3H),1.51 (s, 9H).

**Intermediate 2b: *tert*-butyl (2-chloro-6-(methylamino)phenyl)carbamate**

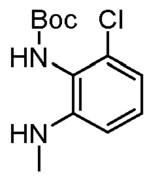

The title compound was prepared in a similar manner to **Intermediate 2a,** using 2-chloro-6-nitroaniline (CAS: 769-11-9) in step a.

$^1H$ NMR (400 MHz, $CDCl_3$) δ ppm 7.10 (t, *J* = 8.0 Hz, 1H), 6.76 (dd, *J* = 1.2, 8.0 Hz, 1H), 6.58 (d, *J* = 8.0 Hz, 1H), 5.91 (br s, 1H), 4.52 (br s, 1H), 2.87 (s, 3H), 1.51 (s, 9H).

**Intermediate 2c: *tert*-butyl (4-fluoro-2-(methylamino)phenyl)carbamate**

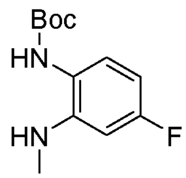

The title compound was prepared in a similar manner to **Intermediate 2a,** using 4-fluoro-2-nitroaniline (CAS: 364-78-3) in step a.

$^1H$ NMR (400 MHz, $CDCl_3$) δ ppm 7.12 - 7.08 (m, 1H), 6.42 - 6.32 (m, 2H), 5.85 (br s, 1H), 4.12 (br s, 1H), 2.83 (s, 3H), 1.50 (s, 9H).

**Intermediate 2d: *tert*-butyl (2,4-difluoro-6-(methylamino)phenyl)carbamate**

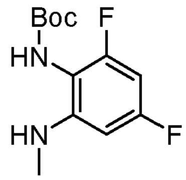

The title compound was prepared in a similar manner to **Intermediate 2a,** using 2,4-difluoro-6-nitroaniline (CAS: 364-30-7) in step a.

$^{1}$H NMR (400 MHz, $CDCl_3$) δ ppm 6.24 - 6.07 (m, 2H), 5.66 (br s, 1H), 4.58 (br s, 1H), 2.84 (d, *J* = 5.2 Hz, 3H), 1.49 (s, 9H).

**Intermediate 3: *tert*-butyl (2-chloro-4-fluoro-6-(methylamino)phenyl)carbamate**

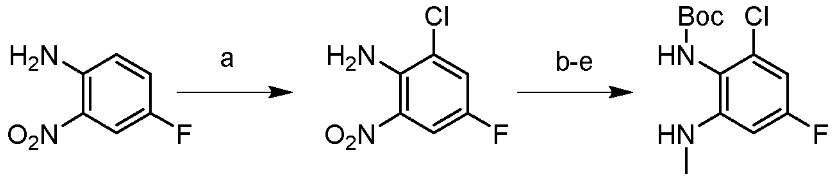


**Step a**. To a solution of 4-fluoro-2-nitroaniline (16.5 g, 106 mmol) in DMF (330 mL) was added *N*-chlorosuccinimide (15.5 g, 116 mmol) in DMF (330 mL) and the mixture was stirred at 40 °C for 12 h. Upon completion, the reaction mixture was quenched with sat. aq. $NH_4Cl$ (100 mL) at 25 °C, diluted with water (300 mL) and extracted with EtOAc (3 x 300 mL). The combined organic layers were washed with brine (3 x 200 mL), dried over $Na_2SO_4$ and evaporated. The crude product was purified by column chromatography (PE/EtOAc = 50/1) to give 2-chloro-4-fluoro-6-nitroaniline (17 g, 84% yield) as a brown solid.

$^{1}$H NMR (400 MHz, $CDCl_3$) δ ppm 7.85 (dd, *J* = 8.8, 3.2 Hz, 1H), 7.40 (dd, *J* = 7.2, 3.2 Hz, 1H), 6.44 (br s, 2H).

**Steps b-e.** These 4 steps were conducted in a similar manner to **Intermediate 2a,** steps a-d. m/z ES+ [M-*t*Bu+H]$^{+}$ 219.4; $^{1}$H NMR (400 MHz, $CDCl_3$) δ ppm 6.48 (dd, *J* = 8.4, 2.4 Hz, 1H), 6.26 (dd, *J* = 11.2, 2.4 Hz, 1H), 5.74 (br s, 1H), 4.61 (br s, 1H), 2.84 (s, 3H) ,1.50 (s, 9H).

**Intermediate 4: *tert*-butyl (6-chloro-3,4-difluoro-2-(methylamino)phenyl)carbamate**

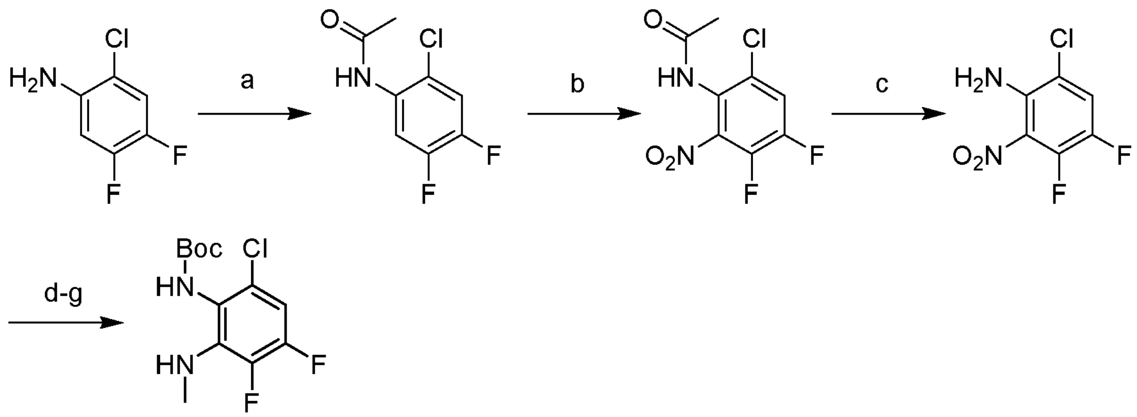


**Step a.** To a solution of 2-chloro-4,5-difluoroaniline (CAS: 2613-32-3; 19.0 g, 116 mmol) in EtOAc (400 mL) was added DIPEA (30.0 g, 232 mmol) and acetic anhydride (17.8 g, 174 mmol) dropwise. The mixture was stirred at 50 °C for 16 h. Upon completion, the reaction mixture was washed with water (4 x 100 mL), dried over $Na_2SO_4$, and evaporated to give *N*-(2-chloro-4,5-difluorophenyl)acetamide (23 g, crude) as brown solid.

$^{1}$H NMR (400 MHz, $CDCl_3$) δ ppm 8.38 (dd, *J* = 8.4, 12.4 Hz, 1H), 7.53 (br s, 1H), 7.26 - 7.19 (m, 1H), 2.25 (s, 3H).

**Step b.** To a solution of *N*-(2-chloro-4,5-difluorophenyl)acetamide (23.0 g, 112 mmol) in conc. $H_2SO_4$ (200 mL)

was added 80% $HNO_3$ (32.2 g, 409 mmol) at 0 °C. The mixture was allowed to warm to 25 °C over 1 h. Upon completion, the reaction mixture was poured into ice water (600 mL), and extracted with EtOAc (3 x 200 mL). The combined organic layers were washed with brine (3 x 200 mL), dried over $Na_2SO_4$ and evaporated. The crude product was purified by column chromatography (PE/EtOAc = 3/1) to give *N*-(6-chloro-3,4-difluoro-2-nitrophenyl)acetamide (26.0 g, 92% yield) as yellow solid.

$^1H$ NMR (400 MHz, $CDCl_3$) δ ppm 7.54 (dd, *J* = 7.6, 8.8 Hz, 1H), 7.35 (br s, 1H), 2.23 (s, 3H). **Step c.** To a solution of *N*-(6-chloro-3,4-difluoro-2-nitrophenyl)acetamide (26.0 g, 104 mmol) in 1,4-dioxane (250 mL) was added 12 M aqueous hydrochloric acid (17 mL). The mixture was stirred at 80 °C for 18 h. Upon completion, the reaction mixture was diluted with water (60 mL) and extracted with EtOAc (2 x 40 mL). The combined organic layers were dried over $Na_2SO_4$, and evaporated to give 6-chloro-3,4-difluoro-2-nitroaniline (22 g, crude) as yellow solid which was used directly in the next step.

**Steps d-g.** These 4 steps were conducted in a similar manner to Intermediate 2a, steps a-d. m/z ES+ [M-*t*Bu+H]$^+$ 236.7; $^1H$ NMR (400 MHz, $CDCl_3$) δ ppm 6.64 - 6.59 (m, 1H), 5.72 (br s, 1H), 4.31 (br s, 1H), 3.08 (d, *J* = 4.4 Hz, 3H), 1.49 (s, 9H).

**Intermediate 5a: (3a*R*,11a*S*)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

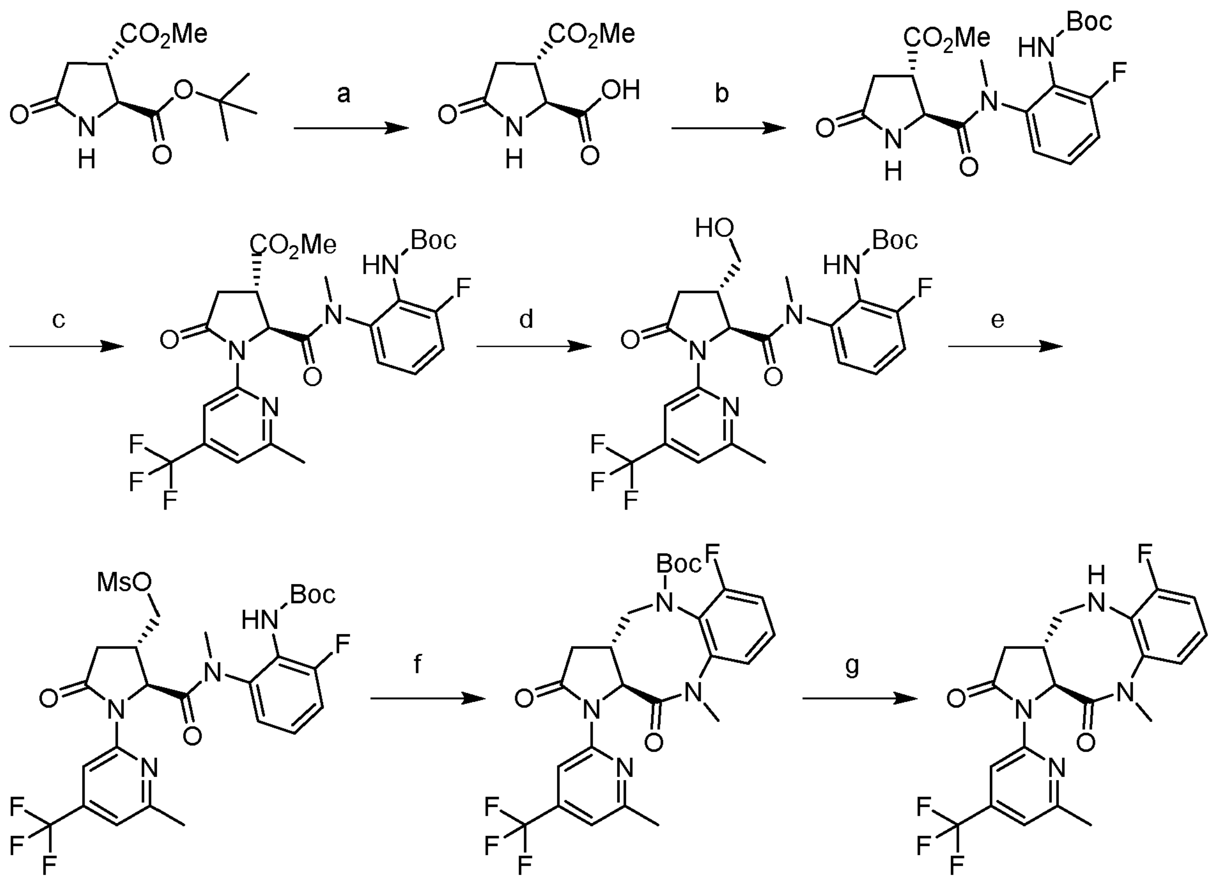


**Step a.** A mixture of **Intermediate 1** (40.0 g, 164 mmol) and TFA (250 mL) was stirred at 25 °C for 12 h under a $N_2$ atmosphere. Upon completion, the reaction mixture was evaporated. The residue was triturated (PE/EtOAc = 10/1) for 60 min. The suspension was filtered to give (2S,3S)-3-(methoxycarbonyl)-5-oxopyrrolidine-2-carboxylic acid (27.0 g, 87% yield) as a white solid.

$^1H$ NMR (400 MHz, DMSO-$d_6$) δ ppm 13.13 (br s, 1H), 8.15 (s, 1H), 4.26 (d, *J* = 0.8 Hz, 1H), 3.68 (s, 3H), 3.37 - 3.33 (m, 1H), 2.56 - 2.52 (m, 1H), 2.36 - 2.32 (m, 1H).

**Step b.** To a solution of (2S,3S)-3-(methoxycarbonyl)-5-oxopyrrolidine-2-carboxylic acid (5 g, 26.7 mmol) in MeCN (50 mL) was added Ghosez's reagent (CAS: 26189-59-3; 3.93 g, 29.3 mmol) at 0 °C. The mixture was stirred at rt for 1 h and then added dropwise to a mixture of **Intermediate 2a** (6.42 g, 26.7 mmol) and *N,N*-dimethylpyridin-2-amine (6.53 g, 53.4 mmol) in MeCN (50 mL) at 0 °C. The reaction mixture was stirred at 0 °C for 30 min. Upon completion, the mixture

was quenched with water (6 mL) and then evaporated to remove MeCN. The residue was diluted with additional water (35 mL) and extracted with EtOAc (3 x 50 mL). The combined organic layers were washed with brine (100 mL), dried over $Na_2SO_4$ and evaporated. The crude product was purified by column chromatography (PE/EtOAc = 0/1) to give methyl (2*S*,3*S*)-2-((2-((*tert*-butoxycarbonyl)amino)-3-fluorophenyl)(methyl)carbamoyl)-5-oxopyrrolidine-3-carboxylate (8.7 g, 78% yield) as a white solid.

m/z ES+ $[M+H]^+$ 410.0; $^1H$ NMR (400 MHz, $CDCl_3$) δ ppm 7.29 (s, 1H), 7.25 - 7.17 (m, 1H), 7.09 (d, *J* = 7.6 Hz, 1H), 6.69 - 6.43 (m, 2H), 4.57 (d, *J* = 4.0 Hz, 1H), 3.72 - 3.66 (m, 3H), 3.54 - 3.45 (m, 1H), 3.25 - 3.18 (m, 3H), 2.84 (m, 1H), 2.61 - 2.43 (m, 1H), 1.48 (s, 9H).

**Step c.** A mixture of methyl (2S,3S)-2-((2-((*tert*-butoxycarbonyl)amino)-3-fluorophenyl)(methyl)carbamoyl)-5-oxopyrrolidine-3-carboxylate (22.0 g, 53.7 mmol), 2-bromo-6-methyl-4-(trifluoromethyl)pyridine (CAS: 451459-17-9; 15.4 g, 64.4 mmol), $Pd_2(dba)_3$ (4.92 g, 5.37 mmol), Xantphos (6.22 g, 10.7 mmol) and $K_2CO_3$ (18.5 g, 134 mmol) in 1,4-dioxane (220 mL) was degassed and purged with $N_2$ 3 times. The reaction mixture was stirred at 100 °C for 3 h under a $N_2$ atmosphere. Upon completion, the mixture was filtered, evaporated, water (150 mL) was added, and the mixture was extracted with EtOAc (3 x 170 mL). The combined organic layers were washed with brine (300 mL), dried over $Na_2SO_4$ and evaporated. The crude product was purified by column chromatography (PE/EtOAc = 1/1) to give methyl (2*S*,3*S*)-2-((2-((*tert*-butoxycarbonyl)amino)-3-fluorophenyl)(methyl)carbamoyl)-1-(6-methyl-4-(trifluoromethyl)pyridin-2-yl)-5-oxopyrrolidine-3-carboxylate (27.7 g, 89% yield) as a yellow solid.

m/z ES+ $[M+H]^+$ 569.2; $^1H$ NMR (400 MHz, DMSO-$d_6$) δ ppm 8.95 (s, 1H), 8.41 (s, 1H), 7.75 (d, *J* = 8.0 Hz, 1H), 7.50 - 7.44 (m, 2H), 7.41 - 7.36 (m, 1H), 3.76 (s, 1H), 3.55 (s, 1H), 3.49 (m, 1H), 3.31 (s, 3H), 3.14 - 3.08 (m, 3H), 2.66 (s, 3H), 2.62 - 2.58 (m, 1H), 1.45 (s, 9H).

**Step d.** To a solution of (2S,3S)-2-((2-((*tert*-butoxycarbonyl)amino)-3-fluorophenyl)(methyl)carbamoyl)-1-(6-methyl-4-(trifluoromethyl)pyridin-2-yl)-5-oxopyrrolidine-3-carboxylate (53.4 g, 93.9 mmol) in THF (530 mL) and MeOH (53 mL) was added $NaBH_4$ (7.11 g, 187 mmol) portion-wise at 0 °C. The reaction mixture was stirred at 20 °C for 1 h. Upon completion, the mixture was slowly quenched with sat. aq. $NH_4Cl$ 50 (mL). The mixture was stirred for a further 30 min, evaporated, water (200 mL) was added and the mixture was extracted with EtOAc (3 x 250 mL). The combined organic layers were washed with brine (500 mL), dried over $Na_2SO_4$ and evaporated. The crude product was purified by column chromatography (PE/EtOAc = 0/1) to give *tert*-butyl (2-fluoro-6-((2S,3S)-3-(hydroxymethyl)-N-methyl-1-(6-methyl-4-(trifluoromethyl)pyridin-2-yl)-5-oxopyrrolidine-2-carboxamido)phenyl)carbamate (47 g, 93% yield) as a yellow solid.

m/z ES+ $[M+H]^+$ 541.1; $^1H$ NMR (400 MHz, $CDCl_3$) δ ppm 8.60 (s, 1H), 7.81 (d, *J* = 8.0 Hz, 1H), 7.42 (m, 1H), 7.27 (s, 2H), 7.11 (s, 1H), 6.13 (s, 1H), 3.41 - 3.39 (m, 1H), 3.28 (s, 3H), 3.25 - 3.16 (m, 2H), 2.97 - 2.93 (m, 1H), 2.63 (s, 3H), 2.23 - 2.14 (m, 1H), 2.09 (d, *J* = 17.6 Hz, 1H), 1.53 (s, 9H).

**Step e.** To a solution of *tert*-butyl (2-fluoro-6-((2*S*,3*S*)-3-(hydroxymethyl)-*N*-methyl-1-(6-methyl-4-(trifluoromethyl)pyridin-2-yl)-5-oxopyrrolidine-2-carboxamido)phenyl)carbamate (47.0 g, 86.9 mmol) in DCM (500 mL) was added TEA (35.2 g, 347 mmol) at 0 °C. Then MsCl (14.9 g, 130 mmol) was added dropwise at 0 °C. The reaction mixture was stirred at 0 °C for 1 h. Upon completion, the reaction mixture was quenched with water (400 mL) and extracted with EtOAc (2 x 400 mL). The combined organic layers were washed with brine (800 mL), dried over $Na_2SO_4$ and evaporated to give ((2*S*,3*S*)-2-((2-((*tert*-butoxycarbonyl)amino)-3-fluorophenyl)(methyl)carbamoyl)-1-(6-methyl-4-(trifluoromethyl)pyridin-2-yl)-5-oxopyrrolidin-3-yl)methyl methanesulfonate (52.0 g, 98% yield) as a yellow solid.

m/z ES+ $[M+H]^+$ 619.1.

**Step f.** To a solution of ((2*S*,3*S*)-2-((2-((*tert*-butoxycarbonyl)amino)-3-fluorophenyl)(methyl)carbamoyl)-1-(6-methyl-4-(trifluoromethyl)pyridin-2-yl)-5-oxopyrrolidin-3-yl)methyl methanesulfonate (26.0 g, 42.0 mmol) in NMP (400 mL) was added $K_3PO_4$ (26.7 g, 126 mmol). The mixture was stirred at 60 °C for 12 h. Upon completion, the mixture was diluted with water (1.2 L) and extracted with EtOAc (3 x 800 mL). The combined organic layers were washed with water (2 x 1 L), brine (2 x 1 L), dried over $Na_2SO_4$ and evaporated. The crude product was purified by column chromatography (PE/EtOAc = 3/1) to give *tert*-butyl (3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocine-5-carboxylate (31.0 g, 67% yield) as a yellow solid.

m/z ES+ $[M+H]^+$ 523.0.

**Step g.** To a solution of *tert*-butyl (3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocine-5-carboxylate (31.0 g, 59.3 mmol) in DCM (320 mL) was added TFA (123 g, 1.08 mol). The mixture was stirred at 20 °C for 2 h. Upon completion, the mixture was basified to pH 8 with sat. aq. $NaHCO_3$. The organic layer was separated and the aqueous was further extracted with EtOAc (3 x 600 mL). The combined organic layers were washed with brine (2 x 500 mL), dried over $Na_2SO_4$ and evaporated to give the title compound (22.3 g, 90% yield) as a yellow solid.

m/z ES+ $[M+H]^+$ 423.1; $^1H$ NMR (400 MHz, $CDCl_3$) δ ppm 8.35 (s, 1H), 7.32 - 7.27 (m, 1H), 7.24 - 7.20 (m, 1H), 7.20 - 7.14 (m, 1H), 7.06 (s, 1H), 4.79 (d, *J* = 9.2 Hz, 1H), 3.78 - 3.73 (m, 1H), 3.36 (s, 3H), 2.87 - 2.70 (m, 3H), 2.50 (s, 3H), 2.29 - 2.21 (m, 1H).

**Intermediate 5b: (3a*R*,11a*S*)-6-chloro-10-methyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione**

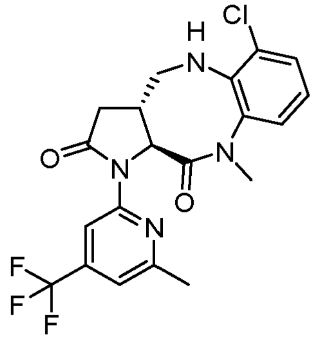

The title compound was prepared in a similar manner to **Intermediate 5a,** using **Intermediate 2b** in step b.

$^1$H NMR (400 MHz, $CDCl_3$) δ ppm 8.36 (s, 1H), 7.50 (dd, *J* = 1.6, 8.0 Hz, 1H), 7.39 - 7.33 (m, 1H), 7.30 - 7.25 (m, 1H), 7.08 (s, 1H), 4.78 (d, *J* = 9.2 Hz, 1H), 3.75 (dd, *J* = 4.0, 13.2 Hz, 1H), 3.37 (s, 3H), 2.93 - 2.74 (m, 3H), 2.52 (s, 3H), 2.33 - 2.23 (m, 1H).

**Intermediate 5c: (3a*R*,11a*S*)-8-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

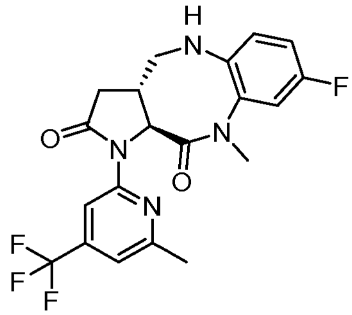

The title compound was prepared in a similar manner to **Intermediate 5a,** using **Intermediate 2c** in step b.

$^1$H NMR (400 MHz, $CDCl_3$) δ ppm 8.35 (s, 1H), 7.28 (d, *J* = 6.0 Hz, 1H), 7.15 - 7.03 (m, 3H), 4.83 - 4.74 (m, 1H), 3.79 (d, *J* = 8.8 Hz, 1H), 3.36 (s, 3H), 2.87 - 2.68 (m, 3H), 2.52 (s, 3H), 2.27 - 2.20 (m, 2H).

**Intermediate 5d: (3a*R*,11a*S*)-6,8-difluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

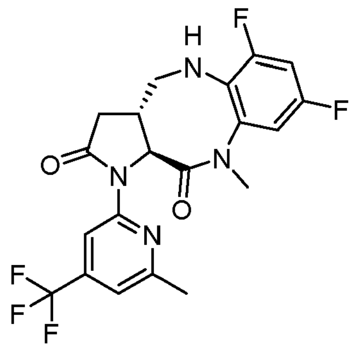

The title compound was prepared in a similar manner to **Intermediate 5a,** using **Intermediate 2d** in step b.
m/z ES+ $[M+H]^+$ 441.1.

**Intermediate 5e: (3a*R*,11a*S*)-6-chloro-8-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

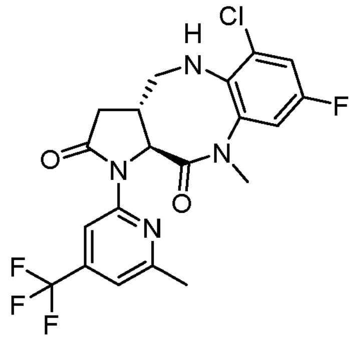

The title compound was prepared in a similar manner to **Intermediate 5a,** using **Intermediate 3** in step b.
m/z ES+ $[M+H]^+$ 457.1; $^1$H NMR (400 MHz, $CDCl_3$) δ ppm 8.35 (s, 1H), 7.25 (d, *J* = 2.8 Hz, 1H), 7.10 - 7.04 (m, 2H), 4.73 (d, *J* = 9.2 Hz, 1H), 3.72 (dd, *J* = 13.2, 3.6 Hz, 1H), 3.34 (s, 4H), 2.89 - 2.75 (m, 2H), 2.74 - 2.67 (m, 1H), 2.51 (s, 3H), 2.32 - 2.20 (m, 1H).

**Intermediate 5f: (3a*R*,11a*S*)-6-chloro-8,9-difluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

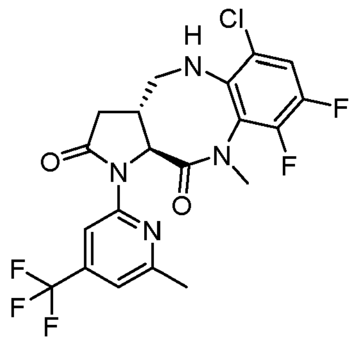

The title compound was prepared in a similar manner to **Intermediate 5a,** using **Intermediate 4** in step b.
m/z ES+ $[M+H]^+$ 475.0.

**Intermediate 6a: (3a*R*,11a*S*)-8-fluoro-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

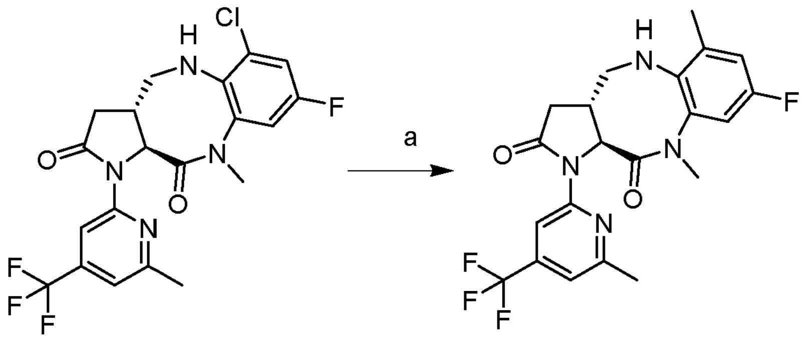


**Step a.** To a solution of **Intermediate 5e** (200 mg, 0.44 mmol), methylboronic acid (262 mg, 4.38 mmol) and $Na_2CO_3$ (139 mg, 1.31 mmol) in 1,4-dioxane (1.5 mL) and water (0.2 mL) was added XPhos-Pd-G2 (34.4 mg, 0.044 mmol). The mixture was degassed and purged with $N_2$ 3 times. The reaction mixture was stirred at 110 °C for 1 h under a $N_2$ atmosphere. Upon completion, the reaction mixture was diluted with water (5 mL) and extracted with EtOAc (3 x 25 mL). The combined organic layers were washed with brine (5 mL), dried over $Na_2SO_4$, and evaporated. The residue was purified by Prep-TLC (PE/EtOAc = 3/1) to give the title compound (150 mg, 72% yield) as yellow solid.

$^1H$ NMR (400 MHz, $CDCl_3$) δ ppm 8.35 (s, 1H), 7.07 (s, 1H), 6.97 (m, 2H), 4.83 (d, *J* = 9.2 Hz, 1H), 3.73 (dd, *J* = 4.8, 13.6 Hz, 1H), 3.34 (s, 3H), 2.84 - 2.71 (m, 3H), 2.62 - 2.54 (m, 1H), 2.51 (s, 3H), 2.42 (s, 3H), 2.27 - 2.16 (m, 1H).

**Intermediate 6b: (3a*R*,11a*S*)-6-cyclopropyl-8-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

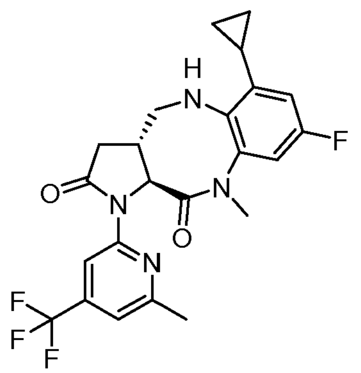

The title compound was prepared in a similar manner to **Intermediate 6a,** using cyclopropylboronic acid in step a.

m/z ES+ $[M+H]^+$ 463.2; $^1H$ NMR (400 MHz, $CDCl_3$) δ ppm 8.35 (s, 1H), 7.07 (s, 1H), 6.91 (dd, *J* = 2.8, 8.8 Hz, 1H), 6.59 (dd, *J* = 2.8, 9.6 Hz, 1H), 4.84 (d, *J* = 8.4 Hz, 1H), 3.77 (m, 1H), 3.34 (s, 3H), 3.09 - 2.90 (m, 1H), 2.86 - 2.69 (m, 3H), 2.51 (s, 3H), 2.33 - 2.18 (m, 2H), 1.18 - 1.05 (m, 2H), 0.80 - 0.63 (m, 2H).

**Intermediate 6c: (3a*R*,11a*S*)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

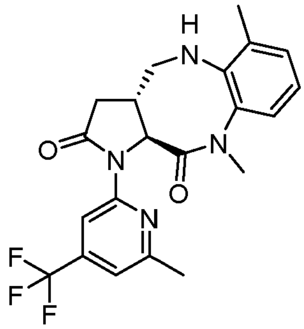

The title compound was prepared in a similar manner to **Intermediate 6a,** using **Intermediate 5b** and methylboronic acid in step a.

m/z ES+ $[M+H]^+$ 418.9; $^1H$ NMR (400 MHz, $CDCl_3$) δ ppm 8.34 (s, 1H), 7.26 - 7.14 (m, 3H), 7.05 (s, 1H), 4.85 (d, *J* = 9.2 Hz, 1H), 3.81 - 3.69 (m, 1H), 3.37 (s, 3H), 2.84 - 2.72 (m, 2H), 2.67 - 2.58 (m, 1H), 2.51 (s, 3H), 2.42 (s, 3H), 2.28 - 2.16 (m, 1H).

**Intermediate 7a: 2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)acetaldehyde**

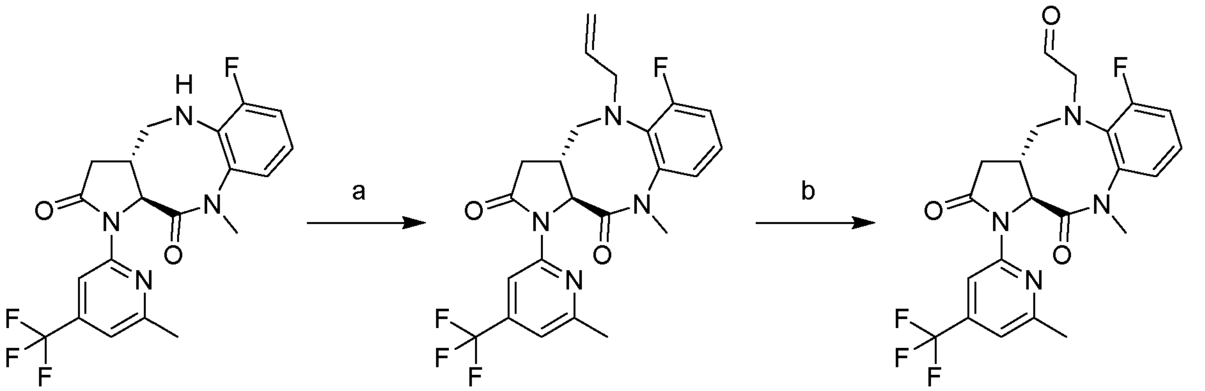


**Step a.** A mixture of **Intermediate 5a** (7.6 g, 17.9 mmol), $Na_2CO_3$ (5.72 g, 53.9 mmol), TBAB (580 mg, 1.80 mmol) in DMF (70 mL) was degassed and purged with $N_2$ 3 times, and then 3-bromoprop-1-ene (8.71 g, 71.9 mmol) was added dropwise at 20 °C. The mixture was stirred at 100 °C for 2 h under a $N_2$ atmosphere. Upon completion, the reaction mixture was diluted with water (200 mL) and extracted with EtOAc (3 x 200 mL). The combined organic layers were washed with water (3 x 200 mL), brine (2 x 200 mL), dried over $Na_2SO_4$ and evaporated. The crude product was purified by column chromatography (PE/EtOAc = 5/1) to give (3a*R*,11a*S*)-5-allyl-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione (7 g, 82% yield) as a yellow solid.

m/z ES+ $[M+H]^+$ 463.2; $^1H$ NMR (400 MHz, $CDCl_3$) δ ppm 8.32 (s, 1H), 7.36 - 7.29 (m, 1H), 7.23 - 7.21 (m, 1H), 7.15 - 7.11 (m, 1H), 7.05 (s, 1H), 5.78 - 5.65 (m, 1H), 5.23 - 5.18 (m, 1H), 5.06 (dd, *J* = 1.6, 10.0 Hz, 1H), 4.66 - 4.61 (m, 1H), 3.77 - 3.63 (m, 2H), 3.59 - 3.49 (m, 1H), 3.34 (s, 3H), 2.92 - 2.82 (m, 2H), 2.75 - 2.66 (m, 1H), 2.50 (s, 3H), 2.24 - 2.14 (m, 1H).

**Step b.** To a solution of (3a*R*,11a*S*)-5-allyl-6-fluoro-10-methyl-1-(6-methyl-4-(trifluoromethyl)pyridin-2-yl)-1 ,3a,4,5, 1 0, 11 a-hexahydro-2*H*-benzo[*b*]pyrrolo[2,3-*f*[1,4]diazocine-2,11(3*H*)-dione (7.0 g, 15.1 mmol) in THF (140 mL) and water (70 mL) was added $NaIO_4$ (9.71 g, 45.4 mmol) and $OsO_4$ (385 mg, 1.51 mmol) at 0 °C. The mixture was stirred at 0 °C for 1 h. Upon completion, sat. aq. $Na_2S_2O_3$ (100 mL) was added, stirred at rt for a further 30 min and then the aqueous mixture was extracted with EtOAc (3 x 200 mL). The combined organic layers were washed with sat. aq. $Na_2S_2O_3$ (2 x 150 mL), sat. $NaHCO_3$ (2 x 150 mL), brine (300 mL), dried over $Na_2SO_4$ and evaporated to give the title compound (7 g, crude) as a yellow solid.

m/z ES+ $[M+H]^+$ 465.2.

**Intermediate 7b: 2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)acetaldehyde**

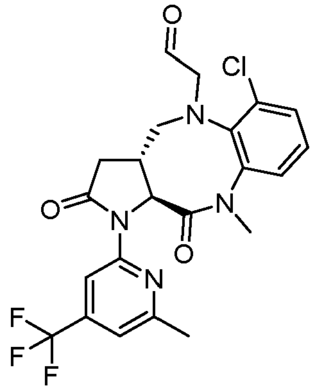

The title compound was prepared in a similar manner to **Intermediate 7a,** using **Intermediate 5b** in step a.
$^1$H NMR (400 MHz, $CDCl_3$) δ ppm 9.67 (t, *J* = 1.6 Hz, 1H), 8.34 (s, 1H), 7.52 - 7.46 (m, 1H), 7.39 - 7.31 (m, 2H), 7.06 (s, 1H), 4.68 (d, *J* = 9.2 Hz, 1H), 3.88 - 3.71 (m, 2H), 3.56 (dd, *J* = 4.8, 14.8 Hz, 1H), 3.37 (s, 3H), 3.15 (dd, *J* = 12.0, 14.4 Hz, 1H), 3.07 - 2.94 (m, 1H), 2.75 (dd, *J* = 8.8, 16.8 Hz, 1H), 2.50 - 2.46 (m, 3H), 2.24 (dd, *J* = 11.6, 16.8 Hz, 1H).

**Intermediate 7c: 2-((3a*R*,11a*S*)-8-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)acetaldehyde**

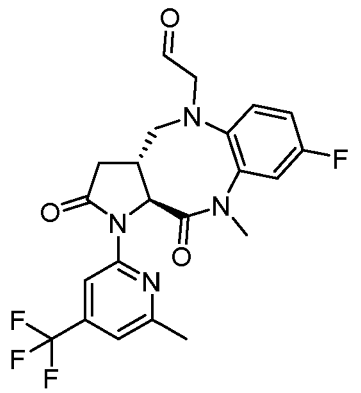

The title compound was prepared in a similar manner to **Intermediate 7a,** using **Intermediate 5c** in step a.
m/z ES+ $[M+H]^+$ 465.2.

**Intermediate 7d: 2-((3a*R*,11a*S*)-6,8-difluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)acetaldehyde**

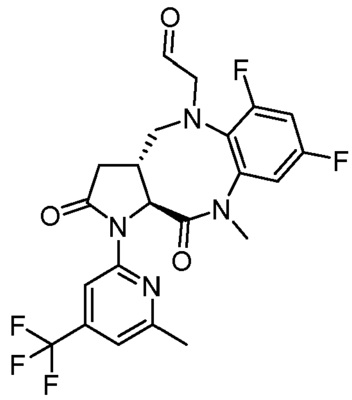

The title compound was prepared in a similar manner to **Intermediate 7a,** using **Intermediate 5d** in step a.

m/z ES+ $[M+H]^+$ 483.4.

**Intermediate 7e: 2-((3aR,11aS)-6-chloro-8-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)acetaldehyde**

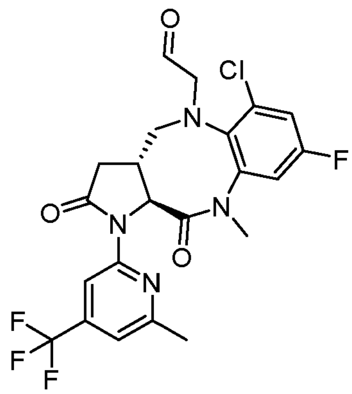

The title compound was prepared in a similar manner to **Intermediate 7a,** using **Intermediate 5e** in step a.

m/z ES+ $[M+H]^+$ 499.2.

**Intermediate 7f: 2-((3a*R*,11a*S*)-6-chloro-8,9-difluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)acetaldehyde**

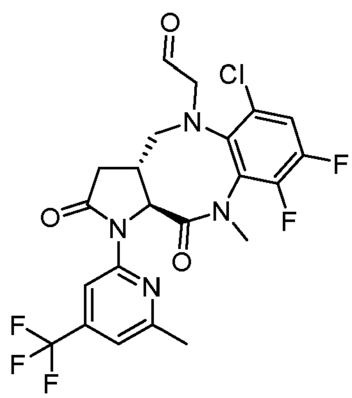

The title compound was prepared in a similar manner to **Intermediate 7a,** using **Intermediate 5f** in step a.

m/z ES+ $[M+H]^+$ 516.9.

**Intermediate 7g: 2-((3a*R*,11a*S*)-8-fluoro-6,10-dimethyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)acetaldehyde**

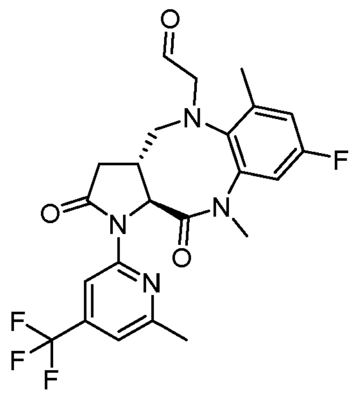

The title compound was prepared in a similar manner to **Intermediate 7a,** using **Intermediate 6a** in step a.

$^1$H NMR (400 MHz, $CDCl_3$) δ ppm 9.63 (s, 1H), 8.35 (s, 1H), 7.07 (s, 1H), 7.02 - 6.98 (m, 1H), 6.93 (dd, *J* = 2.8, 8.4 Hz, 1H), 4.75 (d, *J* = 9.2 Hz, 1H), 3.84 (d, *J* = 18.4 Hz, 1H), 3.67 - 3.57 (m, 2H), 3.36 (s, 3H), 3.01 - 2.85 (m, 2H), 2.75 (dd, *J* = 8.4, 16.8 Hz, 1H), 2.50 (s, 6H), 2.25 - 2.18 (m, 1H).

**Intermediate 7h: 2-((3a*R*,11a*S*)-6-cyclopropyl-8-fluoro-10-methyl-1-(6-methyl-4-(trifluoromethyl)pyridin-2-yl)-2,11-dioxo-1,2,3,3a,4,10,11,11a-octahydro-5H-benzo[*b*]pyrrolo[2,3-*f*][1,4]diazocin-5-yl)acetaldehyde**

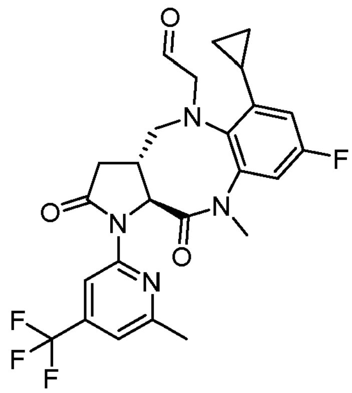

The title compound was prepared in a similar manner to Intermediate 7a, using Intermediate **6b** in step a.

m/z ES+ $[M+H]^+$ 505.4.

**Intermediate 7i: 2-((3aR,11aS)-6,10-dimethyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)acetaldehyde**

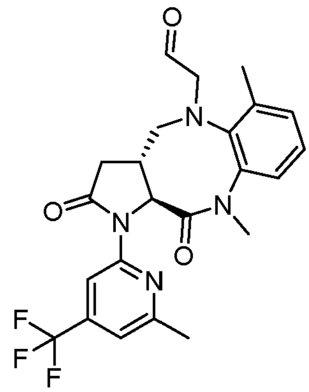

The title compound was prepared in a similar manner to **Intermediate 7a,** using **Intermediate 6c** in step a.

m/z ES+ $[M+H]^+$ 461.3; $^1$H NMR (400 MHz, $CDCl_3$) δ ppm 9.65 (t, *J* = 1.6 Hz, 1H), 8.34 (s, 1H), 7.27 - 7.19 (m, 3H), 7.05 (s, 1H), 4.75 (d, *J* = 9.2 Hz, 1H), 3.84 (dd, *J* = 18.0, 1.6 Hz, 1H), 3.68 - 3.59 (m, 2H), 3.40 - 3.35 (m, 3H), 3.04 - 2.89 (m, 2H), 2.78 - 2.71 (m, 1H), 2.50 (d, *J* = 2.4 Hz, 6H), 2.26 - 2.18 (m, 1H).

**Intermediate 8: (3a*R*,11a*S*)-6-fluoro-5-(2-iodoethyl)-10-methyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione**

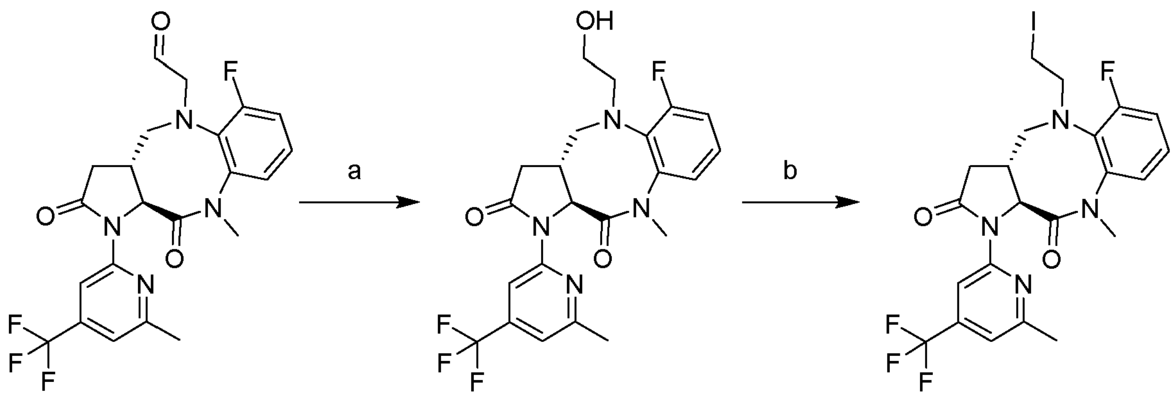


**Step a.** To a solution of **Intermediate 7a** (1.00 g, 2.15 mmol) in MeOH (10 mL) was added $NaBH_3CN$ (244 mg, 6.46 mmol) and the mixture was stirred at 0 °C for 30 min. Upon completion, the reaction mixture was quenched with sat. aq. $NH_4Cl$ (20 mL) at 0 °C. The mixture was diluted with water (30 mL) and extracted with EtOAc (3 x 50 mL). The combined organic layers were washed with brine (3 x 20 mL), dried over $Na_2SO_4$ and evaporated to give (3a*R*,11a*S*)-6-fluoro-5-(2-hydroxyethyl)-10-methyl-1-(6-methyl-4-(trifluoromethyl)pyridin-2-yl)-1,3a,4,5,10,11a-hexahydro-2H-benzo[*b*]pyrrolo [2,3-*f*][1,4]diazocine-2,11(3*H*)-dione (1 g, 99% yield) as a yellow solid.

m/z ES+ $[M+H]^+$ 467.2; $^1$H NMR (400 MHz, $CDCl_3$) δ ppm 8.33 (s, 1H), 7.41 - 7.33 (m, 1H), 7.24 (d, *J* = 8.0 Hz, 1H), 7.17 (t, *J* = 9.2 Hz, 1H), 7.06 (s, 1H), 4.65 (d, *J* = 9.2 Hz, 1H), 4.13 (q, *J* = *7.2* Hz, 1H), 3.59 (dd, *J* = 13.6, 4.4 Hz, 1H), 3.55 - 3.42 (m, 2H), 3.39 - 3.35 (m, 3H), 3.32 - 3.23 (m, 2H), 3.05 - 2.95 (m, 1H), 2.94 - 2.80 (m, 1H), 2.75 (dd, *J* = 16.8, 8.2 Hz, 1H), 2.50 (s, 3H), 2.27 - 2.17 (m, 1H).

**Step b.** To a solution of (3a*R*,11a*S*)-6-fluoro-5-(2-hydroxyethyl)-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione (1 g, 2.14 mmol) in THF (10 mL) was added $I_2$ (816 mg, 3.22 mmol), $PPh_3$ (844 mg, 3.22 mmol) and imidazole (292 mg, 4.29 mmol). The reaction mixture was stirred at 0 °C for 12 h. Upon completion, the reaction mixture was quenched with sat. aq. $NH_4Cl$ (20 mL), diluted with water (30 mL) and extracted with EtOAc (3 x 50 mL). The combined organic layers were washed with brine (3 x 30 mL), dried over $Na_2SO_4$ and evaporated. The crude product was purified by column chromatography (PE/EtOAc = 4/1) to give the title compound (1 g, 80% yield) as a yellow solid.

m/z ES+ $[M+H]^+$ 577.1.

**Intermediate 9a: 2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)acetic acid**

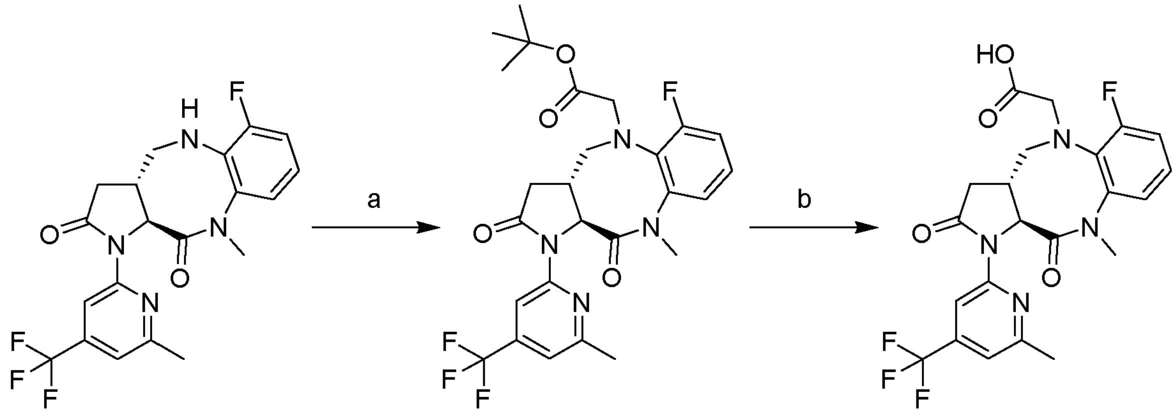


**Step a.** To a solution of **Intermediate 5a** (300 mg, 0.71 mmol) and *tert*-butyl 2-bromoacetate (277 mg, 1.42 mmol) in NMP (4 mL) was added $K_2CO_3$ (294 mg, 2.13 mmol) and TBAI (26.2 mg, 0.071 mmol). The mixture was stirred at 120 °C for 24 h. Upon completion, the reaction mixture was diluted with EtOAc (50 mL) and washed with brine (3 x 10 mL). The organic layer was dried over $Na_2SO_4$ and evaporated. The crude product was purified by column chromatography (PE/EtOAc = 5/1) to give *tert-butyl* 2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)acetate (210 mg, 54% yield) as a yellow gum.

$^1H$ NMR (400 MHz, $CDCl_3$) δ ppm 8.33 (s, 1H), 7.38 - 7.33 (m, 1H), 7.22 (d, *J* = 8.0 Hz, 1H), 7.17 - 7.14 (m, 1H), 7.05 (s, 1H), 4.65 (d, *J* = 9.2 Hz, 1H), 3.87 - 3.76 (m, 1H), 3.68 - 3.56 (m, 2H), 3.38 - 3.34 (m, 3H), 3.03 - 2.90 (m, 2H), 2.85 (s, 1H), 2.77 - 2.70 (m, 1H), 2.49 (s, 3H), 1.43 (s, 9H).

**Step b.** To a solution of *tert-butyl* 2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)acetate (100 mg, 0.19 mmol) in DCM (2 mL) was added TFA (3.08 g, 27.0 mmol). The mixture was stirred at 20 °C for 30 min. Upon completion, the reaction mixture was evaporated to give the title compound (100 mg, crude) as a yellow oil.

m/z ES+ $[M+H]^+$ 481.3.

**Intermediate 9b: 2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)acetic acid**

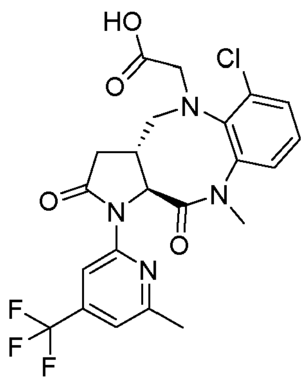

The title compound was prepared in a similar manner to **Intermediate 9a,** using **Intermediate 5b** in step a.

m/z ES+ $[M+H]^+$ 497.1.

**Intermediate 10a: Benzyl 3-((*R*)-4-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2-(fluoromethyl)piperazin-1-yl)azetidine-1-carboxylate and**

**Intermediate 10b: Benzyl 3-((*S*)-6-fluoro-4-(2-((3a*R*,11a*S*)-6-fluoro-10-methyl-1-(6-methyl-4-(trifluoromethyl) pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-1,4-diazepan-1-yl)azetidine-1-carboxylate**

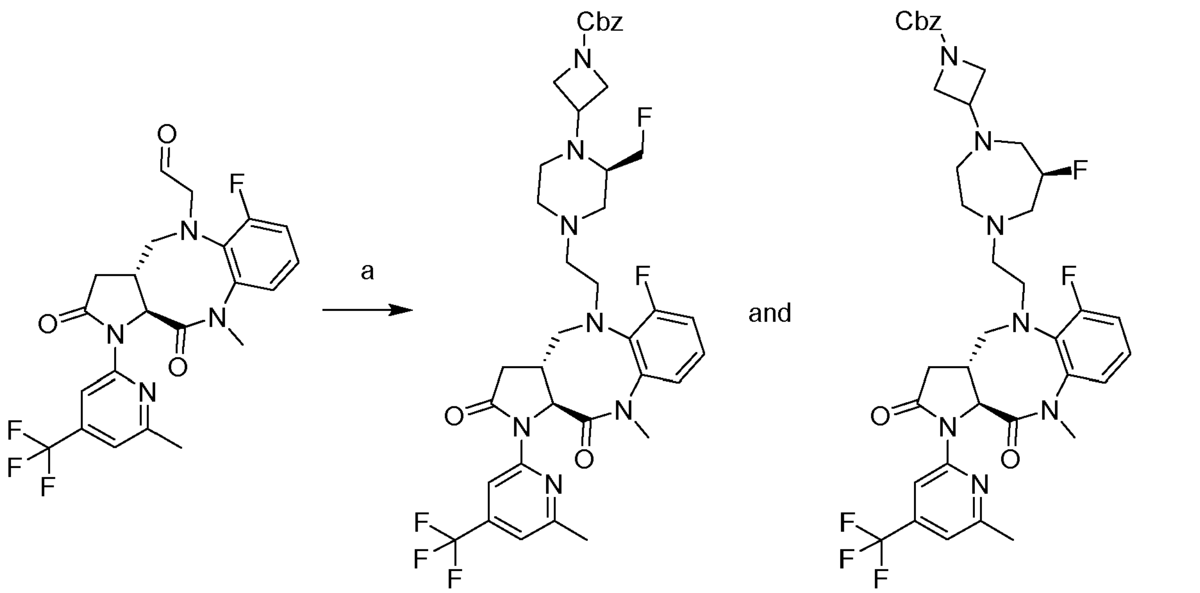

and

**Step a.** To a solution of **Intermediate 7a** (100 mg, 0.22 mmol), **Intermediate A3** (99 mg, 0.32 mmol) in MeOH (2 mL), 4 Å molecular sieves (100 mg) and acetic acid (65 mg, 1.08 mmol) were added. The mixture was stirred at 25 °C for 30 min, after which, $NaBH_3CN$ (27 mg, 0.43 mmol) was added. The mixture was stirred at 25 °C for 30 min. Upon completion, the mixture was diluted with DCM (5 mL), and basified to pH 8 with sat. aq. $NaHCO_3$. The organic phase was separated and the aqueous phase was further extracted with DCM (2 x 5 mL). The combined organic layers were dried over $Na_2SO_4$ and evaporated. The residue was purified by column chromatography (PE/EtOAc = 1/4) and further purified by chiral SFC (column: Daicel ChiralPak AS (250x30mm,10μm); mobile phase: A: 0.1% $NH_4OH$/MeOH; B%: 50% $CO_2$) to give two products.

**Intermediate 10a:** benzyl 3-((*R*)-4-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2-(fluoromethyl)piperazin-1-yl) azetidine-1-carboxylate (22 mg, 22% yield) as a yellow solid.

m/z ES+ $[M+H]^+$ 756.3; $^1H$ NMR (400 MHz, $CDCl_3$) δ ppm 8.33 (s, 1H), 7.37 - 7.29 (m, 6H), 7.22 (d, *J* = 8.0 Hz, 1H), 7.17 - 7.11 (m, 1H), 7.06 (s, 1H), 5.09 (s, 2H), 4.71 - 4.64 (m, 0.5H), 4.60 (d, *J* = 9.2 Hz, 1H), 4.58 - 4.53 (m, 0.5H), 4.42 - 4.38 (m, 0.5H), 4.30 - 4.28 (m, 0.5H), 4.05 - 3.98 (m, 2H), 3.96 - 3.85 (m, 2H), 3.68 - 3.59 (m, 1H), 3.58 - 3.53 (m, 1H), 3.35 (s, 3H), 3.22 - 3.12 (m, 2H), 2.96 - 2.78 (m, 3H), 2.76 - 2.64 (m, 2H), 2.52 - 2.42 (m, 6H), 2.42 - 2.32 (m, 3H), 2.31 - 2.24 (m, 1H), 2.24 - 2.15 (m, 1H).

**Intermediate 10b:** Benzyl 3-((*S*)-6-fluoro-4-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)ethyl)-1,4-diazepan-1-yl)azetidine-1-carboxylate (50 mg, 48% yield) as a yellow solid.

m/z ES+ $[M+H]^+$ 756.3; $^1H$ NMR (400 MHz, $CDCl_3$) δ ppm 8.32 (s, 1H), 7.39 - 7.29 (m, 6H), 7.22 (d, *J* = 8.0 Hz, 1H), 7.14 (dt, *J* = 1.2, 9.2 Hz, 1H), 7.05 (s, 1H), 5.09 (s, 2H), 4.65 (t, *J* = 5.2 Hz, 0.5H), 4.62 - 4.57 (m, 1H), 4.52 (t, *J* = 5.2 Hz, 0.5H), 4.05 - 3.96 (m, 2H), 3.78 (m, 2H), 3.62 - 3.53 (m, 1H), 3.40 (t, *J* = 5.2 Hz, 1H), 3.35 (s, 3H), 3.17 (t, *J* = 6.8 Hz, 2H), 2.96 - 2.84 (m, 4H), 2.79 - 2.67 (m, 4H), 2.66 - 2.58 (m, 1H), 2.58 - 2.50 (m, 3H), 2.49 (s, 3H), 2.45 - 2.36 (m, 1H), 2.23 - 2.11 (m, 1H).

**Intermediate 11a: *tert*-butyl 3-(5-(((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-1-methyl-1*H*-1,2,4-triazol-3-yl)azetidine-1-carboxylate and**

**Intermediate 11b: *tert*-butyl 3-(3-(((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-1-methyl-1*H*-1,2,4-triazol-5-yl)azetidine-1-carboxylate**

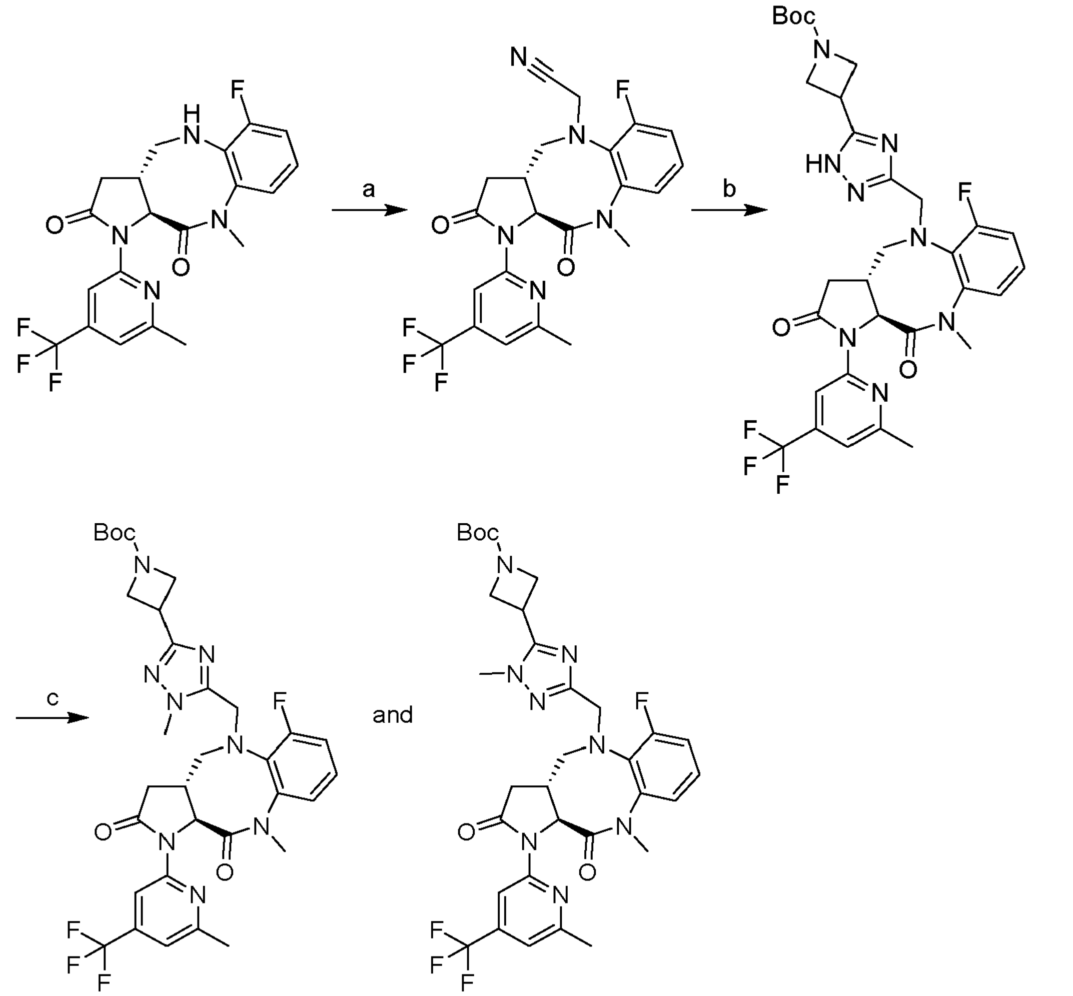


and

**Step** a. To a solution of **Intermediate 5a** (400 mg, 0.95 mmol) and 2-bromoacetonitrile (1.14 g, 9.47 mmol) in DMF (5 mL) was added $Na_2CO_3$ (402 mg, 3.79 mmol) and TBAB (31 mg, 0.095 mmol). The reaction mixture was stirred at 100 °C for 12 h. Upon completion, the reaction mixture was diluted with water (20 mL) and extracted with EtOAc (3 x 30 mL). The combined organic layers were washed with brine (20 mL), dried over $Na_2SO_4$, evaporated and purified by column chromatography (PE/EtOAc = 2/1) to give 2-((3a*R*,11aS*)*-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)acetonitrile (410 mg, 94% yield) as a brown solid.

m/z ES+ $[M+H]^+$ 462.3; $^1$H NMR (400 MHz, $CDCl_3$) δ ppm 8.33 (s, 1 H), 7.47 - 7.44 (m, 1H), 7.31 (d, *J* = 8.0 Hz, 1H), 7.24 - 7.22 (m, 1H), 7.07 (s, 1H), 4.61 (d, *J = 8.8* Hz, 1H), 4.21 - 4.14 (m, 1H), 4.04 - 3.96 (m, 1H), 3.62 - 3.58 (m, 1H), 3.42 (s, 3H), 3.09 - 3.00 (m, 1H), 2.97 - 2.92 (m, 1H), 2.79 -2.73 (m, 1H), 2.50 (s, 3H), 2.22 (dd, *J* = 16.8, 11.6 Hz, 1H).

**Step b.** To a solution of 2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)acetonitrile (200 mg, 0.43 mmol) in MeOH (4 mL) was added sodium methoxide (30 mg, 0.56 mmol) and *tert*-butyl 3-(hydrazinecarbonyl)azetidine-1-carboxylate (CAS: 1001907-44-3; 280 mg, 1.3 mmol), the mixture was stirred at 80 °C for 12 h. Upon completion, the reaction mixture was quenched with sat. aq. $NH_4Cl$ (20 mL), diluted with water (30 mL) and extracted with EtOAc (3 x 50

mL). The combined organic layers were washed with brine (3 x 20 mL), dried over $Na_2SO_4$, evaporated and purified by column chromatography (EtOAc) to give *tert*-butyl 3-(3-(((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-1*H*-1,2,4-triazol-5-yl)azetidine-1-carboxylate (110 mg, 39% yield) as a yellow solid.

m/z ES+ $[M+H]^+$ 659.5; $^1H$ NMR (400 MHz, $CDCl_3$) δ ppm 8.32 (s, 1H), 7.40 - 7.36 (m, 1H), 7.26 - 7.17 (m, 2H), 7.07 (s, 1H), 4.67 (d, *J* = 8.8 Hz, 1H), 4.55 - 4.47 (m, 1H), 4.42 - 4.35 (m, 1H), 4.29 - 4.21 (m, 2H), 4.11 - 4.02 (m, 2H), 3.89 - 3.76 (m, 1H), 3.64 - 3.61 (m, 1H), 3.33 (s, 3H), 3.10- 3.02 (m, 1H), 3.00- 2.98 (m, 1H), 2.73 (dd, *J* = 16.8, 8.4 Hz, 1H), 2.51 (s, 3H), 2.21 (dd, *J* = 16.8, 11.6 Hz, 1H), 1.44 (s, 9H).

**Step c.** To a solution of *tert*-butyl 3-(3-(((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-1*H*-1,2,4-triazol-5-yl)azetidine-1-carboxylate (100 mg, 0.15 mmol) in DMF (3 mL) was added $K_2CO_3$ (63 mg, 0.46 mmol) and MeI (43 mg, 0.30 mmol). The reaction mixture was stirred at 25 °C for 2 h. Upon completion, the reaction mixture was diluted with water (20 mL) and extracted with EtOAc (3 x 30 mL). The combined organic layers were washed with brine (20 mL), dried over $Na_2SO_4$, evaporated and purified by prep-TLC (EtOAc/MeOH = 10/1) to give two products. The regiochemistry was confirmed by 2D NMR.

**Intermediate 11a:** *tert*-butyl 3-(5-(((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-1-methyl-1*H*-1,2,4-triazol-3-yl) azetidine-1-carboxylate (50 mg, 49% yield) as a yellow solid.

m/z ES+ $[M+H]^+$ 673.2; $^1H$ NMR (400 MHz, $CDCl_3$) δ ppm 8.30 (s, 1H), 7.39 - 7.36 (m, 1H), 7.22 - 7.15 (m, 2H), 7.10 (s, 1H), 4.61 (d, *J* = 8.8 Hz, 1H), 4.40 (d, *J* = 8.8 Hz, 1H), 4.36 - 4.33 (m, 1H), 4.23 - 4.49 (m, 2H), 4.19 - 4.07 (m, 2H), 3.84 (s, 3H), 3.77 - 3.74 (m, 1H), 3.59 - 3.27 (m, 1H), 2.99 (s, 3H), 2.99 - 2.96 (m, 1H), 2.99 - 2.96 (m, 1H), 2.95 - 2.73 (m, 1H), 2.72 (s, 3H), 2.20 - 2.19 (m, 1H), 1.47 (s, 9H).

**Intermediate 11b:** *tert*-butyl 3-(3-(((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-1-methyl-1*H*-1,2,4-triazol-5-yl) azetidine-1-carboxylate (20 mg, 20% yield) as a yellow solid.

m/z ES+ $[M+H]^+$ 673.2; $^1H$ NMR (400 MHz, $CDCl_3$) δ ppm 8.30 (s, 1H), 7.34 - 7.28 (m, 1H), 7.20 - 7.10 (m, 2H), 7.04 (s, 1H), 4.64 (d, *J* =9.2 Hz, 1H), 4.37 - 4.28 (m, 2H), 4.23 - 4.13 (m, 2H), 4.10 - 4.03 (m, 2H), 3.86 - 3.73 (m, 3H), 3.70 (s, 3H), 3.30 (s, 3H), 3.03 - 2.98 (m, 1H), 2.69 (dd, *J* = 16.8, 8.4 Hz, 1H), 2.48 (s, 3H), 2.19 (dd, *J* = 16.8, 11.6 Hz, 1H), 1.43 (s, 9H).

**Intermediate 12a: tert-*butyl* 3-(((*S*/*R*)-3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-2-hydroxypropyl)(methyl)amino)azetidine-1-carboxylate and**

**Intermediate 12b: *tert*-butyl 3-(((*R*/*S*)-3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-2-hydroxypropyl)(methyl)amino)azetidine-1-carboxylate**

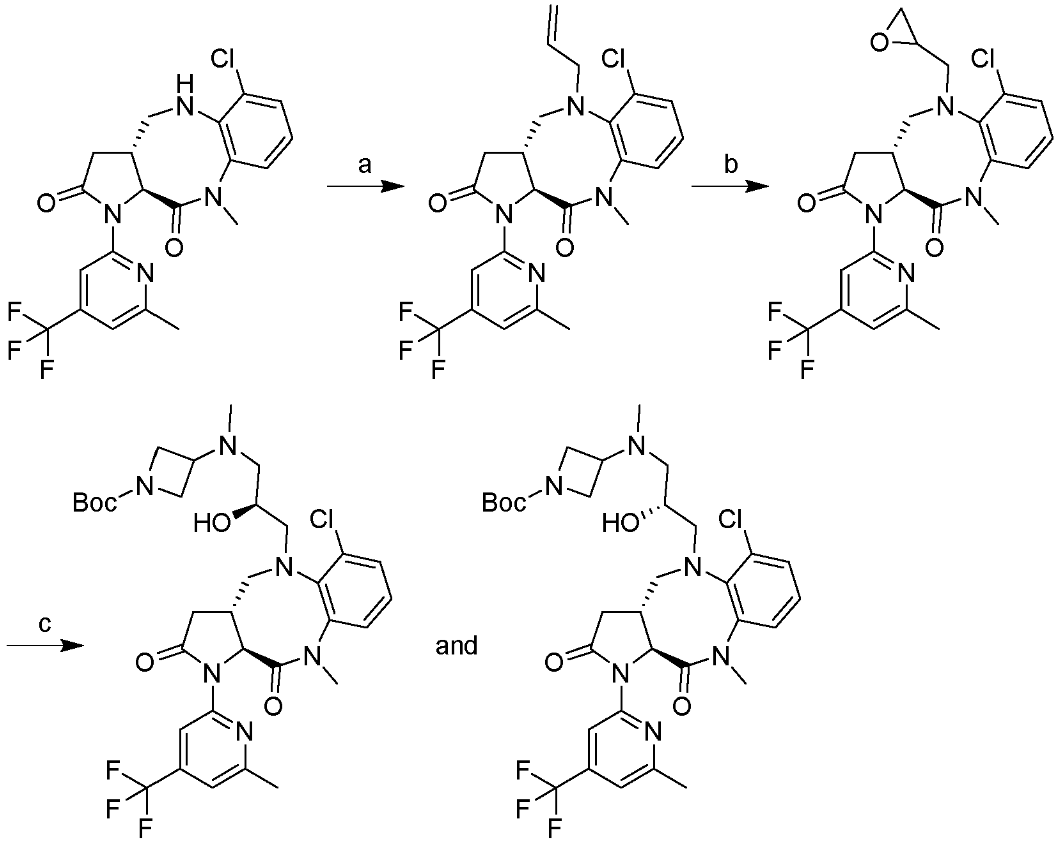


**Step a.** This step was conducted in a similar manner to **Intermediate 7a,** step a, using **Intermediate 5b.**

Step b. To a mixture of (3a*R*,11a*S*)-5-allyl-6-chloro-10-methyl-1-(6-methyl-4-(trifluoromethyl)pyridin-2-yl)-1 ,3a,4,5, 1 0, 11a-hexahydro-2H-benzo[b]pyrrolo[2,3-*f*][1,4]diazocine-2,11(3*H*)-dione (100 mg, 0.21 mmol) in DCM (5 mL) was added m-CPBA (144 mg, 0.63 mmol). The reaction mixture was stirred at 30 °C for 20 h. Upon completion, the reaction mixture was diluted with water (50 mL) and extracted with EtOAc (3 x 30 mL). The organic layers were combined, washed with brine (3 x 50 mL), dried over $Na_2SO_4$ and evaporated. Purification by Prep-TLC (PE/EtOAc = 1/2) gave (3a*R*,11a*S*)-6-chloro-10-methyl-1-(6-methyl-4-(trifluoromethyl)pyridin-2-yl)-5-(oxiran-2-ylmethyl)-1,3a,4,5,10,11a-hexahydro-2*H*-benzo[*b*]pyrrolo[2,3-*f*][1,4]diazocine-2,11(3*H*)-dione (90 mg, 87% yield) as a yellow solid. m/z ES+ $[M+H]^+$ 495.0; $^1H$ NMR (400 MHz, $CDCl_3$) δ ppm 8.34 (s, 1H), 7.48 - 7.45 (m, 1H), 7.36 - 7.30 (m, 2H), 7.05 (s, 1H), 4.67 - 4.62 (m, 1H), 3.72 - 3.62 (m, 1H), 3.39 (d, *J* = 2.4 Hz, 3H), 3.36 - 3.30 (m, 1H), 3.13 - 2.96 (m, 4H), 2.80 - 2.68 (m, 2H), 2.53 - 2.50 (m, 1H), 2.49 (s, 3H), 2.27 - 2.18 (m, 1H).

**Step c.** To a mixture of (3a*R*,11a*S*)-6-chloro-10-methyl-1-(6-methyl-4-(trifluoromethyl)pyridin-2-yl)-5-(oxiran-2-ylmethyl)-1,3a,4,5,10,11a-hexahydro-2*H*-benzo[*b*]pyrrolo[2,3-*f*][1,4]diazocine-2,11(3*H*)-dione (208 mg, 0.42 mmol) in MeOH (5 mL) was added *tert*-butyl 3-(methylamino)azetidine-1-carboxylate (CAS 454703-20-9; 391 mg, 2.10 mmol). The reaction mixture was stirred at 70 °C for 18 h. Upon completion, the reaction mixture was evaporated and the residue was purified by Prep-HPLC to give *tert*-butyl 3-(((*rac*)-3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-2-hydroxypropyl)(methyl)amino)azetidine-1-carboxylate (150 mg, 52% yield) as a yellow solid. m/z ES+ $[M+H]^+$ 681.4; $^1H$ NMR (400 MHz, $CDCl_3$) δ ppm 8.34 (s, 1H), 7.49 - 7.44 (m, 1H), 7.38 - 7.28 (m, 2H), 7.06 (s, 1H), 4.68 - 4.58 (m, 1H), 4.00 - 3.89 (m, 2H), 3.87 - 3.71 (m, 3H), 3.68 - 3.60 (m, 1H), 3.46 - 3.26 (m, 5H), 3.24 - 2.86 (m, 5H), 2.81 - 2.70 (m, 1H), 2.48 (s, 3H), 2.45 - 2.35 (m, 1H), 2.34 - 2.17 (m, 4H), 1.44 (s, 9H).

The racemic material (150 mg) was purified by chiral SFC (column: Daicel ChiralPak IC (250x30mm, 10 μm); mobile phase: A: 0.1% $NH_4OH$/MeOH; B: 55% $CO_2$) to give two products. **Intermediate 12a:** *tert-butyl* 3-(((*S*/*R*)-3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)-2-hydroxypropyl)(methyl)amino)azetidine-1-carboxylate (60 mg, 40% yield) as a colourless oil.

m/z ES+ $[M+H]^+$ 681.3. **Intermediate 12b:** *tert*-butyl 3-(((*R*/*S*)-3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)-2-hydroxypropyl)(methyl)amino)azetidine-1-carboxylate (75 mg, 50% yield) as a colourless oil.

m/z ES+ $[M+H]^+$ 681.3.

**Intermediate 13: *tert*-butyl 3-((3-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propyl)amino)azetidine-1-carboxylate**

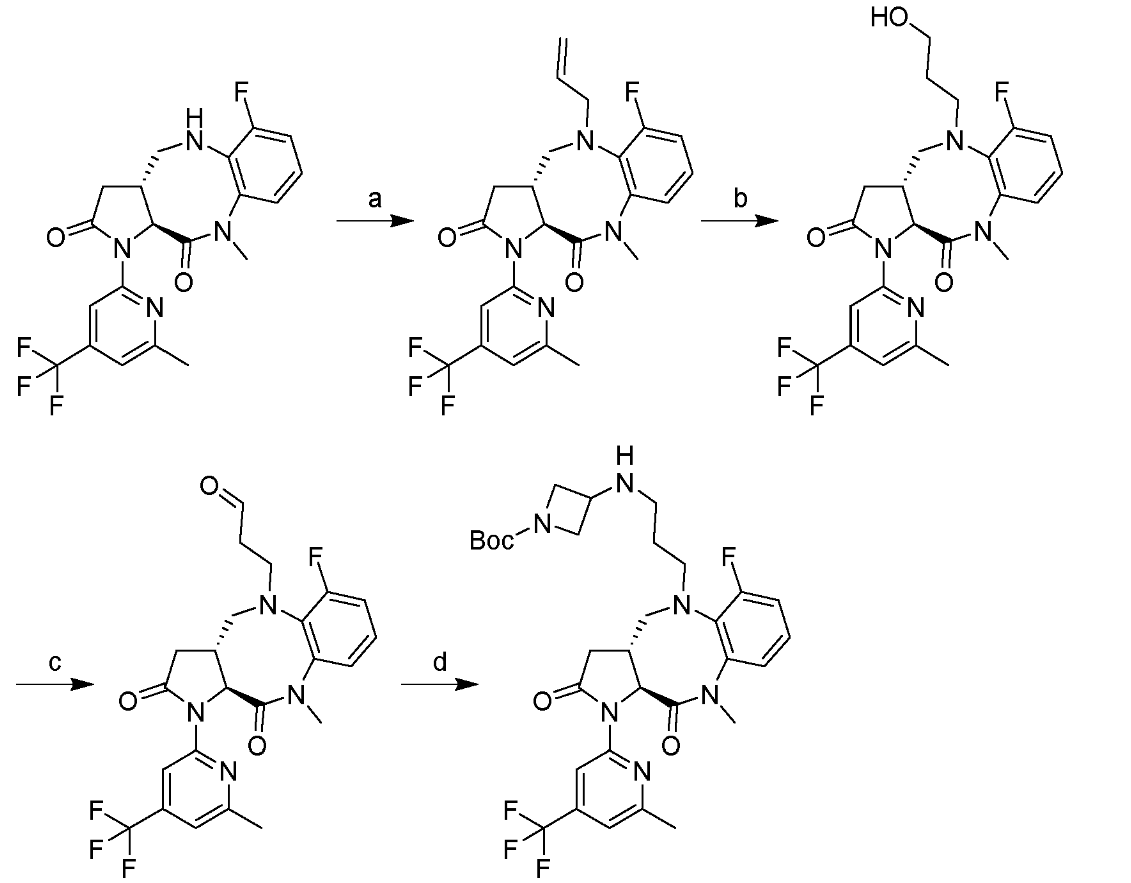


**Step a.** This step was conducted as described in **Intermediate 7a,** step a.

**Step b.** To a solution of (3a*R*,11a*S*)-5-allyl-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (100 mg, 0.22 mmol) in tetrahydrofuran (3 mL) was added a solution of borane dimethyl sulfide complex (10 M in THF, 65 μL) in DCM (0.59 mL). The reaction mixture was stirred at 25 °C for 2 h. The reaction mixture was quenched with methanol (2 mL) and evaporated. The residue was dissolved in THF (4 mL) and 1 M sodium hydroxide (0.65 mL) and 30% hydrogen peroxide (221 mg, 1.95 mmol) were added. The resulting mixture was stirred at 25 °C for 1 hr. Upon completion, the reaction mixture was diluted with water (30 mL) and extracted with EtOAc (30 mL). The organic layer was washed with water (2 x 30 mL), 1 M sodium thiosulfate (30 mL), brine (30 mL), dried over $Na_2SO_4$ and evaporated. The residue was purified by Prep-HPLC to give (3a*R*,11a*S*)-6-fluoro-5-(3-hydroxypropyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (14 mg, 69% yield) as a white solid.

m/z ES+ $[M+H]^+$ 481.1; $^1H$ NMR (400 MHz, $CDCl_3$) δ ppm 8.32 (s, 1 H), 7.35 - 7.31 (m, 1H), 7.22 (d, *J* = 8.0 Hz, 1H), 7.18 - 7.14 (m, 1H), 7.05 (s, 1H), 4.63 (d, *J* = 9.0 Hz, 1H), 3.71 (t, *J* = 6.0 Hz, 2H), 3.62 - 3.54 (m, 1H), 3.37 (s, 3H), 3.29 - 3.17 (m, 2H), 2.98 - 2.85 (m, 2H), 2.79 - 2.67 (m, 1H), 2.49 (s, 3H), 2.27 - 2.14 (m, 1H),1.67 - 1.55 (m, 2H).

**Step c.** To a solution of (3a*R*,11a*S*)-6-fluoro-5-(3-hydroxypropyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (300 mg, 0.62 mmol) in DCM (5 mL) was added DMP (397 mg, 0.94 mmol). The reaction mixture was stirred at 25 °C for 1 h. Upon completion, the reaction mixture was quenched with sat. aq. $Na_2S_2O_3$ (20 mL), diluted with water (30 mL) and extracted with DCM (3 x 50 mL). The combined organic layers were washed with sat. aq. $NaHCO_3$ (20 mL), dried over $Na_2SO_4$ and evaporated to give 3-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propanal (290 mg, crude) as a yellow solid.

m/z ES+ $[M+H]^+$ 479.2.

**Step d.** To a solution of 3-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)propanal (0.29 g, 0.61 mmol) and *tert*-butyl 3-aminoazetidine-1-carboxylate (CAS: 193269-78-2; 209 mg, 1.21 mmol) in MeOH (5 mL) was added acetic acid (182 mg, 3.03 mmol), 4 Å molecular sieves (300 mg) and $NaBH(OAc)_3$ (257 mg, 1.21 mmol). The reaction mixture was stirred at 25 °C for 12 h. Upon completion, the reaction mixture was evaporated and purified by column chromatography (EtOAc) to give the title compound (370 mg, 96% yield) as a yellow solid.

m/z ES+ $[M+H]^+$ 635.2; $^1H$ NMR (400 MHz, $CDCl_3$) δ ppm 8.32 (s, 1H), 7.37 - 7.29 (m, 1H), 7.21 (d, *J* = 8.0 Hz, 1H), 7.14 (t, *J* = 9.2 Hz, 1H), 7.06 (s, 1H), 4.61 (d, *J* = 8.8 Hz, 1H), 4.10 - 4.03 (m, 2H), 3.83 - 3.74 (m, 2H), 3.60 (dd, *J* = 9.2, 5.2 Hz, 4H), 3.55 (d, *J* = 8.8 Hz, 1H), 3.35 (s, 3H), 3.21 - 3.09 (m, 2H), 2.95 - 2.84 (m, 2H), 2.75 - 2.62 (m, 1H), 2.65 - 2.54 (m, 2H), 2.49 (s, 3H), 2.25 - 2.19 (m, 1H), 1.43 (s, 9H).

**Intermediate 14: (3a*R*,11a*S*)-6-chloro-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

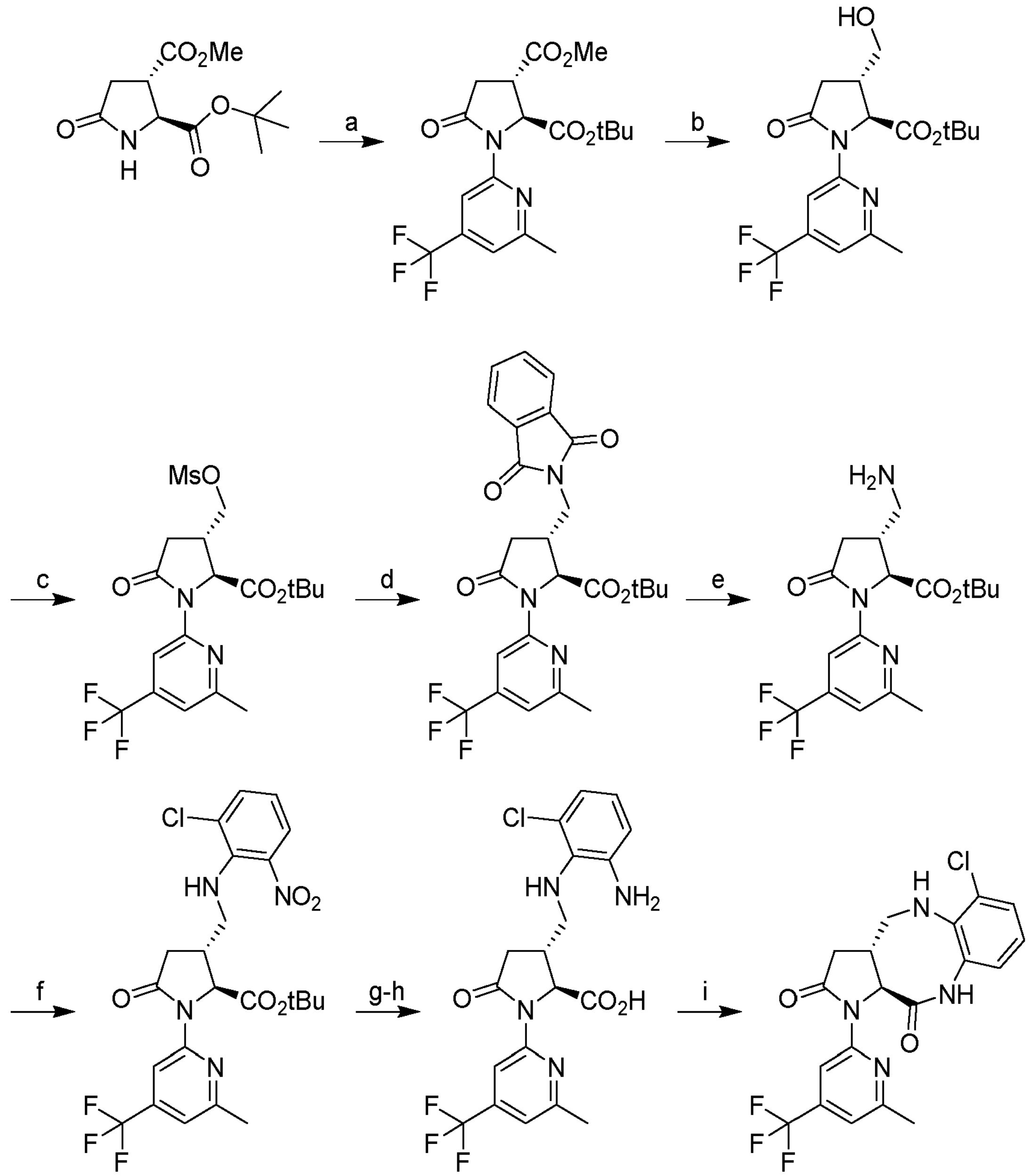


**Step a.** A mixture of **Intermediate 1** (100 g, 411 mmol), 2-bromo-6-methyl-4-(trifluoromethyl)pyridine (CAS: 451459-17-9; 128 g, 534 mmol), $K_2CO_3$ (114 g, 822 mmol), CuI (15.7 g, 82.2 mmol) and $N^1$,$N^2$-dimethylethane-1,2-

diamine (14.5 g, 164.4 mmol) in toluene (1.4 L) was stirred at 110 °C for 12 h under a $N_2$ atmosphere. Upon completion, the reaction mixture was filtered. The filter cake was washed with EtOAc (2 L). The combined filtrate was washed with water (2 L), brine (2 L), dried over $Na_2SO_4$, evaporated and purified by column chromatography (PE/EtOAc = 8/1) to give 2-(*tert*-butyl) 3-methyl (2S,3S)-1-(6-methyl-4-(trifluoromethyl)pyridin-2-yl)-5-oxopyrrolidine-2,3-dicarboxylate (108 g, 63% yield) as a yellow solid.

m/z ES+ $[M+H]^+$ 403.2; $^1H$ NMR (400 MHz, $CDCl_3$) δ ppm 8.46 (s, 1H), 7.11 (s, 1H), 5.21 (d, *J* = 2.8 Hz, 1H), 3.80 (s, 3H), 3.25 - 3.16 (m, 1H), 3.10 - 2.93 (m, 2H), 2.50 (s, 3H), 1.46 (s, 9H). **Step b.** *Note: The following process was conducted in 6 parallel batches.*

To a solution of 2-(*tert*-butyl) 3-methyl (2S,3S)-1-(6-methyl-4-(trifluoromethyl)pyridin-2-yl)-5-oxopyrrolidine-2,3-dicarboxylate (50 g, 124 mmol) in THF (450 mL) and MeOH (50 mL) was added $NaBH_4$ (7.05 g, 186 mmol) portion-wise at 0 °C. The mixture was stirred at 0-5 °C for 3 h. Upon completion, the reaction mixture was quenched with sat. aq. $NH_4Cl$ (3 L) and extracted with EtOAc (2 x 3 L). The organic layers from the parallel batches were combined, washed with brine (3 L), dried over $Na_2SO_4$ and evaporated to give *tert*-butyl (2S,3S)-3-(hydroxymethyl)-1-(6-methyl-4-(trifluoromethyl)pyridin-2-yl)-5-oxopyrrolidine-2-carboxylate (300 g, crude) as a yellow gum.

m/z ES+ $[M+H]^+$ 375.0; $^1H$ NMR (400MHz, $CDCl_3$) δ ppm 8.49 (s, 1H), 7.09 (s, 1H), 4.82 (d, *J* = 3.2 Hz, 1H), 3.77 (d, *J* = 6.0 Hz, 2H), 2.99 - 2.87 (m, 1H), 2.58 - 2.51 (m, 2H), 2.49 (s, 3H), 2.24 - 2.08 (m, 1H), 1.43 (s, 9H).

Step c. To a solution of *tert*-butyl (2*S*,3*S*)-3-(hydroxymethyl)-1-(6-methyl-4-(trifluoromethyl)pyridin-2-yl)-5-oxopyrrolidine-2-carboxylate (100 g, 267 mmol) and TEA (54.1 g, 534 mmol) in DCM (1 L) was added MsCl (33.7 g, 294 mmol) dropwise at 0 °C. The reaction mixture was stirred at 20 °C for 1 h. Upon completion, the reaction mixture was diluted with water (1 L). The layers were separated, the organic layer was washed with brine (3 x 1 L), dried over $Na_2SO_4$, filtered through a pad of silica gel (100-200 mesh) and evaporated to give *tert-butyl* (2*S*,3*S*)-1-(6-methyl-4-(trifluoromethyl)pyridin-2-yl)-3-(((methylsulfonyl)oxy)methyl)-5-oxopyrrolidine-2-carboxylate (120 g, 99% yield) as a yellow gum.

m/z ES+ $[M+H]^+$ 453.0; $^1H$ NMR (400 MHz, $CDCl_3$) δ ppm 8.47 (s, 1H), 7.12 (s, 1H), 4.81 (d, *J* = 3.2 Hz, 1H), 4.33 (d, *J* = 6.0 Hz, 2H), 3.08 (s, 3H), 3.06 - 2.96 (m, 1H), 2.84 - 2.75 (m, 1H), 2.60 - 2.53 (m, 1H), 2.51 (s, 3H), 1.45 (s, 9H).

**Step d.** A mixture of *tert*-butyl (2S,3S)-1-(6-methyl-4-(trifluoromethyl)pyridin-2-yl)-3-(((methylsulfonyl)oxy)methyl)-5-oxopyrrolidine-2-carboxylate (50 g, 111 mmol), isoindoline-1,3-dione (16.4 g, 112 mmol) and $K_2CO_3$ (25.1 g, 181 mmol) in NMP (400 mL) was stirred at 100 °C for 12 h. Upon completion, the reaction mixture was diluted with water (1 L) and filtered. The filter cake was dissolved with EtOAc (1 L), washed with brine (3 x 1 L), dried over $Na_2SO_4$ and evaporated. The crude product was triturated (PE/MTBE = 1/1, 3.0 L) at 25 °C for 12 h to give *tert*-butyl (2*S*,3*R*)-3-((1,3-dioxoisoindolin-2-yl)methyl)-1-(6-methyl-4-(trifluoromethyl)pyridin-2-yl)-5-oxopyrrolidine-2-carboxylate (25 g, 44% yield) as a white solid. m/z ES+ $[M+H]^+$ 504.1; $^1H$ NMR (400 MHz, $CDCl_3$) δ ppm 8.46 (s, 1H), 7.92 - 7.85 (m, 2H), 7.80 - 7.73 (m, 2H), 7.09 (s, 1H), 4.74 (d, *J* = 3.2 Hz, 1H), 4.07 - 3.94 (m, 1H), 3.86 - 3.74 (m, 1H), 3.02 - 2.89 (m, 2H), 2.61 - 2.51 (m, 1H), 2.48 (s, 3H), 1.34 (s, 9H).

**Step e.** *Note: The following process was conducted in 4 parallel batches.*

To a solution of *tert*-butyl (2*S*,3*R*)-3-((1,3-dioxoisoindolin-2-yl)methyl)-1-(6-methyl-4-(trifluoromethyl)pyridin-2-yl)-5-oxopyrrolidine-2-carboxylate (25.0 g, 49.7 mmol) in MeOH (250 mL) was added hydrazine monohydrate (85% purity; 4.68 g, 79.5 mmol). The reaction mixture was stirred at 70 °C for 12 h under a $N_2$ atmosphere. Upon completion, the reaction mixture was cooled to rt, filtered and the filtrate was evaporated. The resulting residues from the parallel batches were combined, diluted with water (1 L), extracted with EtOAc (2 x 1 L), washed with brine (1 L), dried over $Na_2SO_4$ and evaporated to give *tert-butyl* (2*S*,3*R*)-3-(aminomethyl)-1-(6-methyl-4-(trifluoromethyl)pyridin-2-yl)-5-oxopyrrolidine-2-carboxylate (76 g, crude) as a brown gum.

m/z ES+ $[M+H]^+$ 374.0; $^1H$ NMR (400 MHz, $CDCl_3$) δ ppm 8.49 (s, 1H), 7.09 (s, 1H), 4.77 (d, *J* = 3.2 Hz, 1H), 3.01 - 2.85 (m, 3H), 2.46 (s, 3H), 2.43 - 2.38 (m, 2H), 1.43 (s, 9H).

**Step f.** *Note: The following process was conducted in 4 parallel batches.*

A mixture of *tert*-butyl (2*S*,3*R*)-3-(aminomethyl)-1-(6-methyl-4-(trifluoromethyl)pyridin-2-yl)-5-oxopyrrolidine-2-carboxylate (40 g, 107 mmol), 1-chloro-2-fluoro-3-nitrobenzene (CAS: 2106-49-2; 17.8 g, 102 mmol) and $NaHCO_3$ (18.0 g, 214 mmol) in EtOH (380 mL) and water (20 mL) was stirred at 105 °C for 12 h. Upon completion, the reaction mixture was evaporated, diluted with EtOAc (5 L) and washed with water (2 L). The organic layers from the parallel batches were combined, dried over $Na_2SO_4$, evaporated and purified by column chromatography (PE/EA = 10/1) to give *tert-butyl* (2*S*,3*R*)-3-(((2-chloro-6-nitrophenyl)amino)methyl)-1-(6-methyl-4-(trifluoromethyl)pyridin-2-yl)-5-oxopyrrolidine-2-carboxylate (175 g, 77% yield) as a yellow oil.

m/z ES+ $[M+H]^+$ 529.1; $^1H$ NMR (400 MHz, $CDCl_3$) δ ppm 8.41 (s, 1H), 7.93 (dd, *J* = 7.6, 1.6 Hz, 1H), 7.47 (dd, *J* = 7.6, 1.6 Hz, 1H), 7.03 (s, 1H), 6.78 (t, *J* = 8.4 Hz, 1H), 4.76 (d, *J* = 2.4 Hz, 1H), 3.68 - 3.63 (m, 1H), 3.48 - 3.42 (m, 1H), 2.97 - 2.90 (m, 1H), 2.61 - 2.52 (m, 1H), 2.47 - 2.38 (m, 4H), 1.36 (s, 9H).

**Step g.** To a solution of *tert*-butyl (2*S*,3*R*)-3-(((2-chloro-6-nitrophenyl)amino)methyl)-1-(6-methyl-4-(trifluoromethyl)pyridin-2-yl)-5-oxopyrrolidine-2-carboxylate (110 g, 208 mmol) in THF (1.1 L) was added 3% Pt-V/C (11 g, 1.26 mmol). The reaction mixture was stirred at 25 °C for 16 h under a $H_2$ (30 psi) atmosphere. Upon completion, the reaction mixture was filtered and evaporated to give *tert*-butyl (2*S*,3*R*)-3-(((2-amino-6-chlorophenyl)amino)methyl)-1-(6-methyl-4-(trifluoromethyl)pyridin-2-yl)-5-oxopyrrolidine-2-carboxylate (102 g, 98% yield) as a brown oil.

m/z ES+ $[M+H]^+$ 499.0; $^1H$ NMR (400 MHz, $CDCl_3$) δ ppm 8.48 (s, 1H), 7.10 (s, 1H), 6.91 - 6.76 (m, 2H), 6.68 - 6.61 (m, 1H), 4.88 (d, *J* = 4.0 Hz, 1H), 3.29 - 3.22 (m, 1H), 3.17 - 3.10 (m, 1H), 3.07 - 2.98 (m, 1H), 2.68 - 2.53 (m, 2H), 2.50 (s, 3H), 1.43 (s, 9H).

**Step h.** *Note: The following process was conducted in 3 parallel batches.*

To a solution of *tert-butyl* (2*S*,3*R*)-3-(((2-amino-6-chlorophenyl)amino)methyl)-1-(6-methyl-4-(trifluoromethyl) pyridin-2-yl)-5-oxopyrrolidine-2-carboxylate (34.0 g, 68.2 mmol) in DCM (170 mL) was added HCl in 1,4-dioxane (4 M, 170 mL). The reaction mixture was stirred at 25 °C for 12 h. Upon completion, the reaction mixture was diluted with water (1 L). The pH of the reaction mixture was adjusted to pH 5 with solid NaOH and then evaporated to remove the DCM. The resulting residues from the parallel batches were combined, extracted with EtOAc (3 x 1 L), washed with brine (1 L), dried over $Na_2SO_4$ and evaporated to give (2S,3R)-3-[(2-amino-6-chloro-anilino)methyl]-1-[6-methyl-4-(trifluoromethyl)-2-pyridyl]-5-oxo-pyrrolidine-2-carboxylic acid (90 g, 99% yield) as a yellow oil.

m/z ES+ $[M+H]^+$ 442.9; $^1H$ NMR (400 MHz, DMSO-$d_6$) δ ppm 8.39 (s, 1H), 7.40 (s, 1H), 6.74 - 6.66 (m, 1H), 6.61 - 6.51 (m, 2H), 4.92 (d, *J* = 2.8 Hz, 1H), 3.14 - 3.07 (m, 1H), 3.03 - 2.95 (m, 1H), 2.87 - 2.78 (m, 1H), 2.70 - 2.61 (m, 1H), 2.53 - 2.52 (m, 1H), 2.47 (s, 3H).

**Step i.** *Note: The following process was conducted in 2 parallel batches.*

To a solution of (2*S*,3*R*)-3-(((2-amino-6-chlorophenyl)amino)methyl)-1-(6-methyl-4-(trifluoromethyl)pyridin-2-yl)-5-oxopyrrolidine-2-carboxylic acid (30.0 g, 67.8 mmol) and DIPEA (17.5 g, 136 mmol) in DCM (1800 mL) and EtOAc (600 mL) was added T3P (50% in EtOAc; 43.1 g, 67.8 mmol). The reaction mixture was stirred at 20 °C for 1 h. Upon completion, the reaction mixture was quenched with water (500 mL) and extracted with EtOAc (3 x 1 L). The organic layers from the parallel batches were combined, washed with brine (1 L), dried over $Na_2SO_4$ and evaporated. Purification by column chromatography (PE/EtOAc/$NH_4OH$ = 1/1/0.002) gave the title compound (40 g, 67% yield) as an off-white solid.

m/z ES+ $[M+H]^+$ 425.1; $^1H$ NMR (400 MHz, $CDCl_3$) δ ppm 8.32 (s, 1H), 7.80 (s, 1H), 7.49 - 7.43 (m, 1H), 7.23 - 7.14 (m, 2H), 7.06 (s, 1H), 4.75 (d, *J* = 8.8 Hz, 1H), 3.84 - 3.72 (m, 1H), 3.67 - 3.34 (m, 1H), 2.87 - 2.73 (m, 3H), 2.44 (s, 3H), 2.36 - 2.21 (m, 1H).

**Intermediate A1: *tert*-butyl 3-(piperazin-1-yl)azetidine-1-carboxylate**

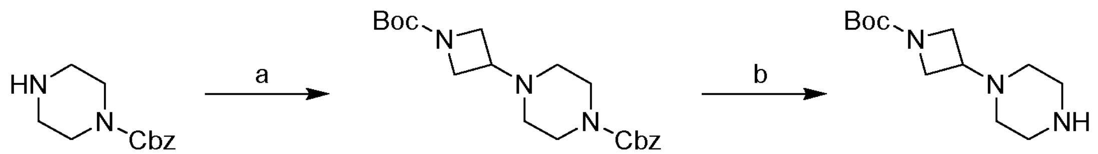


**Step a.** To a solution of *tert*-butyl 3-oxoazetidine-1-carboxylate (CAS: 398489-26-4; 29.2 g, 170 mmol) and benzyl piperazine-1-carboxylate (CAS: 31166-44-6; 25 g, 114 mmol) in MeOH (500 mL) was added 4 Å molecular sieves (25 g) and acetic acid (6.8 g, 114 mmol). The mixture was stirred at rt for 12 h, and then $NaBH_3CN$ (21.4 g, 341 mmol) was added. The mixture was stirred at rt for 1 h. Upon completion, the mixture was filtered and evaporated. The crude product was purified by reverse phase flash chromatography (water (0.1% TFA)/MeCN). The product containing fractions were combined, basified to pH 8 with sat. aq. $Na_2CO_3$ and evaporated to remove MeCN and water. The resulting residue was extracted with EtOAc (3 x 500 mL). The combined layers were washed with brine (800 mL), dried over $Na_2SO_4$ and evaporated to give obtain benzyl 4-(1-(*tert*-butoxycarbonyl)azetidin-3-yl)piperazine-1-carboxylate (30 g, 67% yield) as a yellow solid.

m/z ES+ $[M+H]^+$ 376.4; $^1H$ NMR (400 MHz, $CDCl_3$) δ ppm 7.40 - 7.30 (m, 5H), 5.14 (s, 2H), 3.99 - 3.90 (m, 2H), 3.82 (s, 2H), 3.57 (s, 4H), 3.09 (s, 1H), 2.22 (s, 4H), 1.43 (s, 9H).

**Step b.** A mixture of benzyl 4-(1-(*tert*-butoxycarbonyl)azetidin-3-yl)piperazine-1-carboxylate (60 g, 160 mmol) and 10% Pd/C (9 g) in MeOH (600 mL) was stirred at 40 °C for 16 h under a $H_2$ atmosphere (50 psi). Upon completion, the mixture was filtered and evaporated to give the title compound (37 g, crude) as a white solid.

$^1H$ NMR (400 MHz, $CDCl_3$) δ ppm 3.93 - 3.87 (m, 2H), 3.79 (m, 2H), 3.09 - 3.01 (m, 1H), 2.92 - 2.89 (m, 4H), 2.32 (s, 4H), 1.41 (s, 9H).

**Intermediate A2: *tert*-butyl 3-(1,4-diazepan-1-yl)azetidine-1-carboxylate**

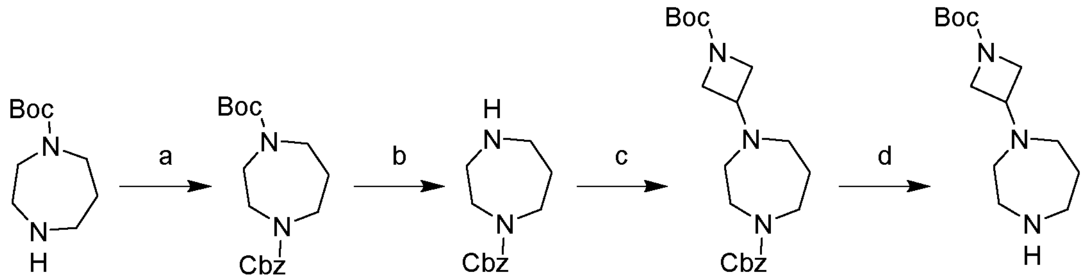


**Step a.** To a solution of *tert*-butyl 1,4-diazepane-1-carboxylate (CAS: 112275-50-0; 10.0 g, 49.9 mmol) in THF (100 mL) and water (30 mL) was added CbzCl (8.52 g, 49.9 mmol) and $Na_2CO_3$ (15.9 g, 150 mmol). The mixture was stirred at 25 °C for 2 h. Upon completion, the reaction mixture was quenched with sat. aq. $NH_4Cl$ (100 mL), diluted with water (100 mL) and extracted with EtOAc (3 x 200 mL). The combined organic layers were washed with brine (3 x 100 mL), dried over $Na_2SO_4$ and evaporated. The residue was purified by column chromatography (PE/EtOAc = 4/1) to give 1-benzyl *4*-(*tert*-butyl) 1,4-diazepane-1,4-dicarboxylate (15 g, 89% yield) as a yellow oil.

$^1$H NMR (400 MHz, DMSO-$d_6$) δ ppm 7.38 - 7.31 (m, 5H), 5.11 - 5.01 (m, 2H), 3.51 - 3.48 (m, 2H), 3.45 - 3.36 (m, 4H), 3.31 - 3.28 (m, 2H), 1.74 - 1.57 (m, 2H), 1.41 - 1.30 (m, 9H).

Step b. To a solution of 1-benzyl 4-(*tert*-butyl) 1,4-diazepane-1,4-dicarboxylate (5 g, 15.0 mmol) in DCM (50 mL) was added TFA (10 mL) and the mixture was stirred at 25 °C for 30 min. Upon completion, the reaction mixture was evaporated to give benzyl 1,4-diazepane-1-carboxylate as a TFA salt (5.1 g, crude) as a colourless oil.

m/z ES+ $[M+H]^+$ 235.2.

**Step c.** To a solution of benzyl 1,4-diazepane-1-carboxylate (5.1 g, 14.6 mmol) and *tert-butyl* 3-oxoazetidine-1-carboxylate (3.01 g, 17.6 mmol) in MeOH (50 mL) was added acetic acid (4.40 g, 73.2 mmol), 4 Å molecular sieves (5 g) and $NaBH_3CN$ (1.84 g, 29.3 mmol). The mixture was stirred at 25 °C for 2 h. Upon completion, the reaction mixture was filtered and evaporated. The residue was diluted with sat. aq. $NaHCO_3$ (50 mL) and water (100 mL), and extracted with EtOAc (3 x 150 mL). The combined organic layers were washed with brine (3 x 100 mL), dried over $Na_2SO_4$, filtered and evaporated. The residue was purified by column chromatography (PE/EtOAc = 3/1) to give benzyl 4-(1-(tert-butoxycarbonyl)azetidin-3-yl)-1,4-diazepane-1-carboxylate (3.5 g, 61% yield) as a colourless oil.

m/z ES+ $[M+H]^+$ 390.2. $^1$H NMR (400 MHz, $CDCl_3$) δ ppm 7.30 - 7.40 (m, 5H), 5.14 (s, 2H), 4.60 - 4.57 (m, 4H), 3.98 - 3.87 (m, 2H), 3.77 - 3.72 (m, 2H), 3.59 - 3.51 (m, 4H), 3.31 - 3.25 (m, 1H), 2.38 (d, *J* = 4.8 Hz, 2H), 1.66 (s, 9H).

**Step d.** To a solution of benzyl 4-(1-(*tert*-butoxycarbonyl)azetidin-3-yl)-1,4-diazepane-1-carboxylate (3.5 g, 8.99 mmol) in MeOH (50 mL) was added 10% Pd/C (954 mg) and the mixture was stirred at 25 °C for 1 h under a $H_2$ atmosphere (15 psi). Upon completion, the reaction mixture was filtered and evaporated to give the title compound (2.1 g, 91% yield) as a colourless oil.

$^1$H NMR (400 MHz, DMSO-$d_6$) δ ppm 5.63 (s, 1H), 4.37 - 4.35 (m, 1H), 3.98 (t, *J* = 7.6 Hz, 2H), 3.59 - 3.55 (m, 2H), 3.32 - 3.23 (m, 2H), 2.78 - 2.65 (m, 1H), 2.54 - 2.50 (m, 4H), 2.45 - 2.30 (m, 1H),1.37 - 1.35 (m, 11H).

**Intermediate A3: A mixture of benzyl (*S*)-3-(6-fluoro-1,4-diazepan-1-yl)azetidine-1-carboxylate and benzyl (*R*)-3-(2-(fluoromethyl)piperazin-1-yl)azetidine-1-carboxylate**

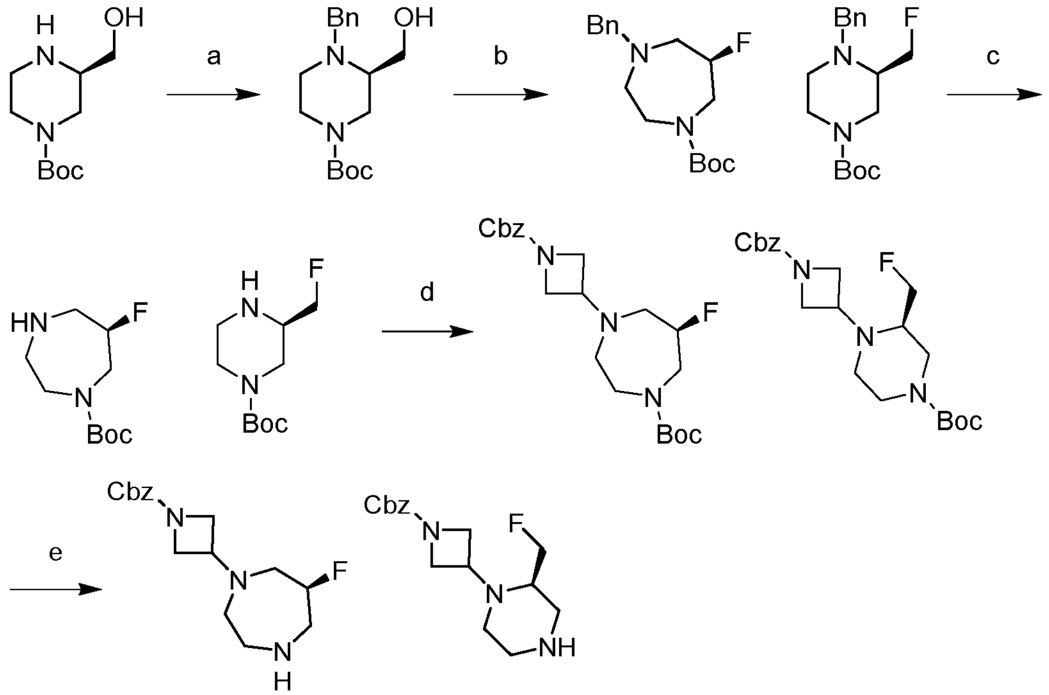


**Step a.** To a solution of *tert-butyl* (*R*)-3-(hydroxymethyl)piperazine-1-carboxylate (CAS: 278788-66-2; 5.00 g, 23.1 mmol,) and benzaldehyde (2.94 g, 27.7 mmol) in DCE (50 mL) was added acetic acid (2.08 g, 34.6 mmol) and 4 Å molecular sieves (5 g). The mixture was stirred at 25 °C for 1 h, and then $NaBH(OAc)_3$ (5.88 g, 27.7 mmol) was added. The mixture was stirred at 25 °C for 12 h. Upon completion, the mixture was filtered, diluted with water (20 mL) extracted with EtOAc (3 x 20 mL). The combined layers were dried over $Na_2SO_4$ and evaporated. The residue was purified by column chromatography (PE/EtOAc = 3/1) to give *tert-butyl* (R)-4-benzyl-3-(hydroxymethyl)piperazine-1-carboxylate (3.5 g, 47% yield) as a yellow solid.

m/z ES+ $[M+H]^+$ 307.2.

Step b. To a solution of *tert*-butyl (*R*)-4-benzyl-3-(hydroxymethyl)piperazine-1-carboxylate (500 mg, 1.63 mmol) in DCM (10 mL) was added DAST (1.32 g, 8.16 mmol) dropwise at 0 °C. The mixture was stirred at 25 °C for 5 h. Upon completion, the mixture was diluted with sat. aq. $NaHCO_3$ (20 mL) and extracted with EtOAc (2 x 30 mL). The combined organic layers were dried over $Na_2SO_4$ and evaporated. The residue was purified by column chromatography (PE/EtOAc = 20/1) to give an inseparable mixture of *tert-butyl* (R)-4-benzyl-6-fluoro-1,4-diazepane-1-carboxylate and *tert-butyl* (R)-4-benzyl-3-(fluoromethyl)piperazine-1-carboxylate (450 mg, 86% yield) as a brown oil.

m/z ES+ $[M+H]^+$ 309.3.

**Step c.** A mixture of *tert*-butyl (*R*)-4-benzyl-6-fluoro-1,4-diazepane-1-carboxylate and *tert*-butyl (*R*)-4-benzyl-3-(fluoromethyl)piperazine-1-carboxylate (400 mg, 1.30 mmol), 10% Pd/C (100 mg), and 20% $Pd(OH)_2$/C (100 mg) in MeOH (10 mL) was stirred at 40 °C for 24 h under a $H_2$ atmosphere (15 psi). Upon completion, the reaction mixture was filtered and evaporated to give an inseparable mixture of *tert*-butyl (*R*)-6-fluoro-1,4-diazepane-1-carboxylate and *tert*-butyl (*R*)-3-(fluoromethyl)piperazine-1-carboxylate (280 mg, 99% yield) as a colourless oil.

m/z ES+ $[M+H]^+$ 219.1.

**Step d.** To a solution of *tert*-butyl (*R*)-6-fluoro-1,4-diazepane-1-carboxylate and *tert*-butyl (*R*)-3-(fluoromethyl) piperazine-1-carboxylate (280 mg, 1.28 mmol) and benzyl 3-oxoazetidine-1-carboxylate (395 mg, 1.92 mmol) in MeOH (5 mL) was added 4 Å molecular sieves (280 mg) and acetic acid (385 mg, 6.41 mmol). The mixture was stirred at 25 °C for 30 min, then $NaBH_3CN$ (121 mg, 1.92 mmol) was added. The mixture was stirred at 25 °C for 12 h. Upon completion, the reaction mixture was diluted with DCM (5 mL), basified to pH 8 with sat. aq. $NaHCO_3$. The organic layer was separated and the aqueous was further extracted with DCM (2 x 5 mL). The combined organic layers were dried over $Na_2SO_4$ and evaporated. The residue was purified by column chromatography (PE/EtOAc = 5/1) and then re-purified by Prep-TLC (PE/EtOAc = 1/1) to give an inseparable mixture of *tert*-butyl (*R*)-4-(1-((benzyloxy)carbonyl)azetidin-3-yl)-6-fluoro-1,4-diazepane-1-carboxylate and *tert*-butyl (*R*)-4-(1-((benzyloxy)carbonyl)azetidin-3-yl)-3-(fluoromethyl)piperazine-1-carboxylate (250 mg, 37% yield) as a colourless oil.

m/z ES+ $[M+H]^+$ 408.2.

**Step e.** To a solution of *tert-butyl* (*R*)-4-(1-((benzyloxy)carbonyl)azetidin-3-yl)-6-fluoro-1,4-diazepane-1-carboxylate and *tert-butyl* (*R*)-4-(1-((benzyloxy)carbonyl)azetidin-3-yl)-3-(fluoromethyl)piperazine-1-carboxylate (220 mg, 0.54 mmol) in DCM (3 mL) was added TFA (1 mL). The mixture was stirred at rt for 30 min. Upon completion, the mixture was

diluted with DCM (5 mL) and basified to pH 8 with sat. aq. $NaHCO_3$. The organic layer was separated and the aqueous was further extracted with DCM (2 x 5 mL). The combined organic layers were dried over $Na_2SO_4$ and evaporated to give an inseparable mixture of the title compounds (100 mg, crude) as a colourless oil.

m/z ES+ $[M+H]^+$ 307.9.

**Intermediate A4: 5-benzyl 2-(*tert*-butyl) 2,5,8-triazaspiro[3.5]nonane-2,5-dicarboxylate**

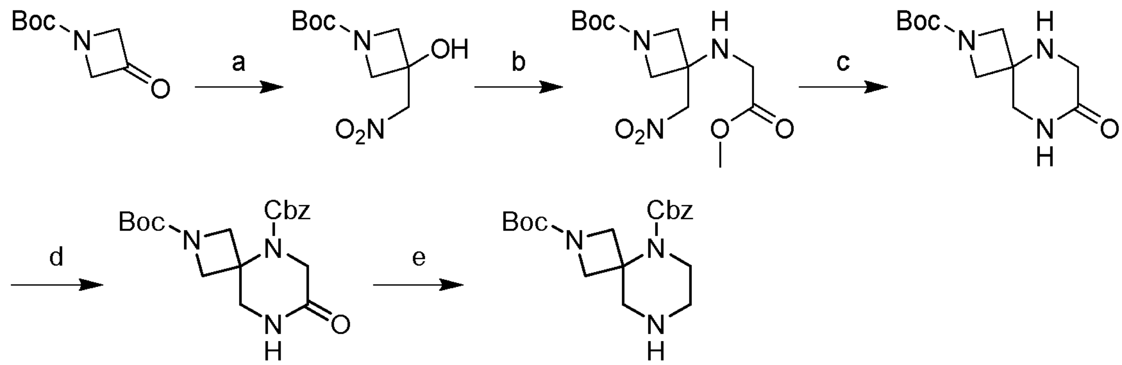


**Step a.** To a solution of *tert*-butyl 3-oxoazetidine-1-carboxylate (100 g, 584 mmol) in $MeNO_2$ (678 g, 11.1 mol) was added TEA (11.8 g, 117 mmol). The mixture was stirred at 25 °C for 1 h. Upon completion, the mixture was evaporated to give *tert-butyl* 3-hydroxy-3-(nitromethyl)azetidine-1-carboxylate (135 g, crude) as a yellow solid.

$^1H$ NMR (400 MHz, DMSO-$d_6$) δ ppm 6.45 (s, 1H), 4.86 (s, 2H), 4.04 (d, J= 8.8 Hz, 2H), 3.74 (d, *J* = 9.2 Hz, 2H), 1.38 (s, 9H).

**Step b.** To a solution of *tert*-butyl 3-hydroxy-3-(nitromethyl)azetidine-1-carboxylate (50 g, 215 mmol) in DCM (1 L) was added TEA (43.6 g, 431 mmol). Then MsCl (24.7 g, 215 mmol) in DCM (500 mL) was added dropwise at -78 °C. The mixture was stirred at -78 °C for 1.5 h. Methyl 2-aminoacetate hydrochloride (CAS: 34582-32-6; 54.0 g, 430 mmol) and TEA (43.6 g, 431 mmol) was dissolved in DCM (500 mL) and stirred for 10 min, and this solution was added into the reaction mixture at -78 °C. The mixture was stirred at 25 °C for 16 h. Upon completion, the mixture was diluted with water (400 mL) and extracted with DCM (2 x 500 mL). The combined organic layers were washed with brine (300 mL), dried over $Na_2SO_4$ and evaporated. The crude product was purified by silica gel chromatography column (PE/EtOAc = 5/1) to give *tert-butyl* 3-((2-methoxy-2-oxoethyl)amino)-3-(nitromethyl)azetidine-1-carboxylate (41.5 g, 63% yield) as a white solid.

m/z ES+ $[M\text{-}tBu+H]^+$ 248.1; $^1H$ NMR (400 MHz, $CDCl_3$) δ ppm 4.70 (d, *J* = 4.4 Hz, 2H), 3.97 - 3.90 (m, 2H), 3.89 - 3.85 (m, 2H), 3.76 (s, 3H), 3.48 (s, 2H), 1.45 (s, 9H).

**Step c.** To a solution of *tert-butyl* 3-((2-methoxy-2-oxoethyl)amino)-3-(nitromethyl)azetidine-1-carboxylate (20 g, 66.0 mmol) in MeOH (500 mL) was added Raney-Ni (20 g, 233 mmol) and the reaction mixture was stirred at 25 °C for 12 h under a $H_2$ atmosphere (50 psi). Upon completion, the mixture was filtered and evaporated to give *tert-butyl* 7-oxo-2,5,8-triazaspiro[3.5]nonane-2-carboxylate (63 g, crude) as a colourless oil.

m/z ES+ $[M+H]^+$ 242.1.

**Step d.** To a solution of *tert-butyl* 7-oxo-2,5,8-triazaspiro[3.5]nonane-2-carboxylate (63 g, 261 mmol) and $NaHCO_3$ (65.8 g, 783 mmol) in THF (600 mL) and water (200 mL) was added CbzCl (66.8 g, 392 mmol) and the mixture was stirred at 25 °C for 12 h. Upon completion, the mixture was diluted with water (200 mL) and extracted with EtOAc (2 x 200 mL). The combined organic layers were washed with brine (200 mL), dried over $Na_2SO_4$ and evaporated. The crude product was purified by column chromatography (PE /EtOAc = 1/1) to give 5-benzyl 2-(*tert*-butyl) 7-oxo-2,5,8-triazaspiro[3.5]nonane-2,5-dicarboxylate (46 g, 42% yield) as a colourless oil.

m/z ES+ $[M+Na]^+$ 398.2; $^1H$ NMR (400 MHz, $CDCl_3$) δ ppm 7.37 - 7.23 (m, 5H), 6.49 (s, 1H), 5.08 (s, 2H), 4.21 (d, *J* = 9.2 Hz, 2H), 4.10 - 4.06 (m, 2H), 3.73 (d, *J* = 9.2 Hz, 2H), 3.56 (d, *J* = 1.6 Hz, 2H), 1.37 (s, 9H).

**Step e.** To a solution of 5-benzyl 2-(*tert*-butyl) 7-oxo-2,5,8-triazaspiro[3.5]nonane-2,5-dicarboxylate (13 g, 34.6 mmol) in THF (100 mL) was added borane dimethyl sulfide complex (10 M in THF, 10.39 mL) at 0 °C, and the mixture was stirred at 25°C for 1 h. Upon completion, the mixture was quenched with MeOH (10 mL) and evaporated. The crude product was purified by column chromatography (PE/EtOAc = 1/1) to give the title compound (2 g, 16% yield) as a yellow oil.

$^1H$ NMR (400 MHz, DMSO-$d_6$) δ ppm 7.41 - 7.33 (m, 5H), 5.09 (s, 2H), 3.96 (d, *J* = 9.2 Hz, 2H), 3.70 (d, *J* = 8.8 Hz, 2H), 3.36 - 3.33 (m, 2H), 3.24 - 3.19 (m, 2H), 2.78 (s, 2H), 1.38 (s, 9H).

**Intermediate A5: 5-benzyl 2-(*tert*-butyl) (*R*)-6-methyl-2,5,8-triazaspiro[3.5]nonane-2,5-dicarboxylate**

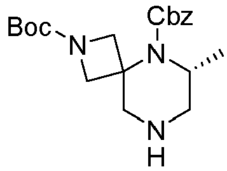

The title compound was prepared in a similar manner to **Intermediate A4,** using methyl *D*-alaninate (CAS: 14316-06-4) in step b.

m/z ES+ $[M+H]^+$ 376.2.

**Intermediate A6: 5-benzyl 2-(*tert*-butyl) (*S*)-6-methyl-2,5,8-triazaspiro[3.5]nonane-2,5-dicarboxylate**

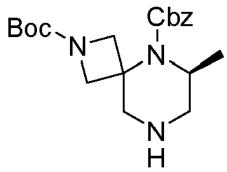

The title compound was prepared in a similar manner to **Intermediate A4,** using methyl L-alaninate (CAS: 2491-20-5) in step b.

m/z ES+ [M-*t*Bu+H]$^+$ 320.2.

**Intermediate A7: 5-benzyl 2-(*tert*-butyl) 9-methyl-2,5,8-triazaspiro[3.5]nonane-2,5-dicarboxylate**

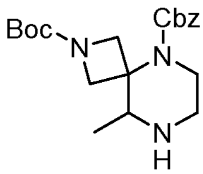

The title compound was prepared in a similar manner to **Intermediate A4,** using $EtNO_2$ in step a.

m/z ES+ $[M+H]^+$ 376.2.

**Intermediate A8: 5-benzyl 2-(*tert*-butyl) (6R)-6,9-dimethyl-2,5,8-triazaspiro[3.5]nonane-2,5-dicarboxylate**

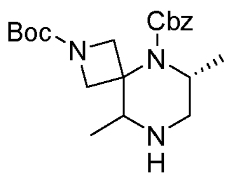

The title compound was prepared in a similar manner to Intermediate A4, using $EtNO_2$ in step a, and methyl *D*-alaninate in step b.

m/z ES+ $[M+H]^+$ 390.3.

**Intermediate A9: *tert*-butyl (*R*)-6-methyl-2,5,8-triazaspiro[3.5]nonane-2-carboxylate**

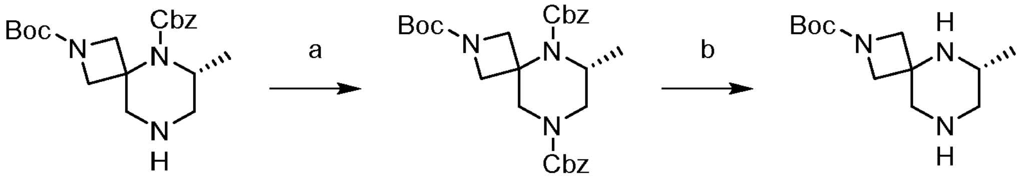


**Step a.** To a solution of **Intermediate A5** (0.8 g, 2.13 mmol) in THF (9 mL) and water (3 mL) was added $Na_2CO_3$ (226 mg, 2.13 mmol) and CbzCl (545 mg, 3.20 mmol). The mixture was stirred at 25 °C for 12 h. Upon completion, the reaction mixture was quenched with sat. aq. $NH_4Cl$ (20 mL), further diluted with water (30 mL) and extracted with EtOAc (3 x 50 mL). The combined organic layers were washed with brine (3 x 20 mL), dried over $Na_2SO_4$ and evaporated. The residue was purified by column chromatography (PE/EtOAc = 5/1) to give 5,8-dibenzyl 2-(*tert*-butyl) (*R*)-6-methyl-2,5,8-triazaspiro[3.5]nonane-2,5,8-tricarboxylate (1 g, 92% yield) as a colourless solid.

m/z ES+ $[M+H]^+$ 510.4; $^1H$ NMR (400 MHz, $CDCl_3$) δ ppm 7.37 - 7.27 (m, 10H), 5.25 - 5.08 (m, 4H), 4.51 - 4.98 (m, 1H), 4.37 - 4.23 (m, 2H), 4.00 - 3.96 (m, 1H), 3.77 - 3.48 (m, 4H), 3.26 - 3.22 (d, *J = 13.2* Hz, 1H), 1.44 (s, 9H), 1.17 (d, *J* = 6.0 Hz, 3H).

**Step b.** To a solution of 5,8-dibenzyl 2-(*tert*-butyl) (*R*)-6-methyl-2,5,8-triazaspiro[3.5]nonane-2,5,8-tricarboxylate (1.0 g, 1.96 mmol) in THF (10 mL) was added 10% Pd/C (208 mg) and the mixture was stirred at 25 °C for 12 h under a $H_2$ atmosphere (15 psi). Upon completion, the reaction mixture was filtered and evaporated. The residue was purified by reverse phase flash chromatography (water (0.1% FA)/MeCN) to give the title compound (450 mg, 95% yield) as a colourless oil.

$^1H$ NMR (400 MHz, $CDCl_3$) δ ppm 3.94 (d, *J* = 8.8 Hz, 1H), 3.76 - 3.68 (m, 2H), 3.56 (d, *J* = 9.2 Hz, 1H), 3.04 (d, *J* = 11.8 Hz, 1H), 2.89 - 2.84 (m, 1H), 2.82 - 2.78 (m, 1H), 2.73 - 2.66 (m, 1H), 2.34 - 2.28 (m, 1H), 1.44 (s, 9H), 1.02 (d, *J* = 6.0 Hz, 3H).

**Intermediate A10: *tert*-butyl (*R*)-hexahydro-1'*H*-spiro[azetidine-3,4'-pyrrolo[1,2-a]pyrazine]-1-carboxylate**

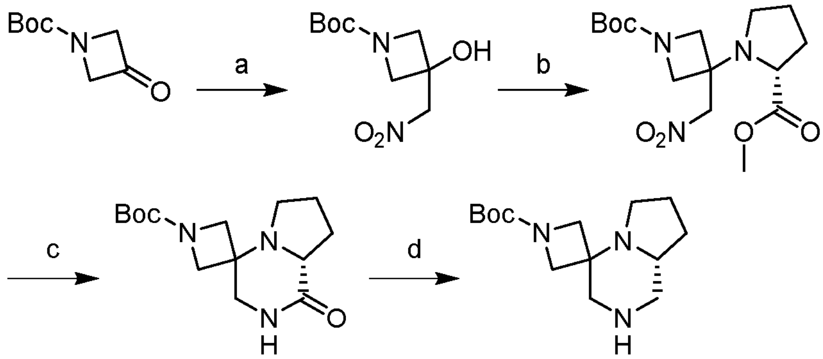


**Step a.** This step was conducted as described in **Intermediate A4,** step a.

**Step b.** To a solution of *tert-butyl* 3-hydroxy-3-(nitromethyl)azetidine-1-carboxylate (5.0 g, 21.5 mmol) and TEA (4.4 g, 43.1 mmol) in DCM (100 mL) was added MsCl (2.5 g, 21.5 mmol) at -78 °C under a $N_2$ atmosphere. The mixture was stirred at -78 °C for 2 h. A solution of methyl *D*-prolinate hydrochloride (CAS: 184719-80-0; 5.6 g, 33.6 mmoll) and TEA (4.4 g, 43.1 mmol) in DCM (100 mL) was then added to the reaction mixture at -78 °C. The reaction mixture was stirred at 25 °C for 12 h under a $N_2$ atmosphere. Upon completion, the reaction mixture was diluted with DCM (100 mL), washed with brine (3 x 100 mL), dried over $Na_2SO_4$ and evaporated. The crude product was purified by column chromatography (PE/EtOAc = 1/1) to give methyl (1-(*tert*-butoxycarbonyl)-3-(nitromethyl)azetidin-3-yl)-D-prolinate (5.0 g, 68% yield) as a yellow oil.

$^1H$ NMR (400 MHz, $CDCl_3$) δ ppm 4.83 - 4.68 (m, 2H), 4.15 - 4.10 (m, 2H), 4.08 - 3.99 (m, 2H), 3.90 - 3.80 (m, 2H), 3.71 (s, 3H), 3.27 - 3.18 (m, 1H), 2.04 - 1.89 (m, 4H), 1.47 (s, 9H).

**Step c.** To a solution of methyl (1-(*tert*-butoxycarbonyl)-3-(nitromethyl)azetidin-3-yl)-*D*-prolinate (5.0 g, 14.6 mmol) in MeOH (100 mL) was added Raney-Ni (1.3 g, 14.6 mmol) and the mixture was at 25 °C for 12 h under a $H_2$ atmosphere (50 psi). Upon completion, the reaction mixture was filtered and evaporated to give *tert*-butyl (*R*)-1'-oxohexahydro-1'*H*-spiro[azetidine-3,4'-pyrrolo[1,2-a]pyrazine]-1-carboxylate (3.5 g, 85% yield) as a yellow solid. m/z ES+ $[M+H]^+$ 282.2.

**Step d.** To a mixture of *tert*-butyl (*R*)-1'-oxohexahydro-1'*H*-spiro[azetidine-3,4'-pyrrolo[1,2-a]pyrazine]-1-carboxylate (400 mg, 1.42 mmol) in THF (5 mL) was added borane dimethyl sulfide complex (10 M in THF, 0.43 mL). The mixture was stirred at 25 °C for 1 h under a $N_2$ atmosphere. Upon completion, the reaction mixture was quenched with MeOH (10 mL) at 0 °C and stirred at 25 °C for 2 h. The mixture was evaporated to give the title compound (400 mg, crude) as a white solid.

$^1$H NMR (400 MHz, $CDCl_3$) δ ppm 4.09 - 3.91 (m, 2H), 3.86 - 3.70 (m, 1H), 3.67 - 3.54 (m, 2H), 3.53 - 3.33 (m, 1H), 3.28 - 3.09 (m, 1H), 2.97 - 2.73 (m, 1H), 2.73 - 2.56 (m, 1H), 2.52 - 2.17 (m, 2H), 2.03 - 1.70 (m, 4H), 1.52 - 1.43 (m, 9H).

**Intermediate A11: *tert*-butyl (*S*)-6-(methoxymethyl)-2,5,8-triazaspiro[3.5]nonane-2-carboxylate**

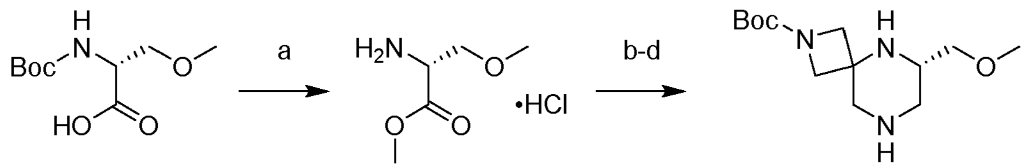


**Step a.** To a solution of *N*-(*tert*-butoxycarbonyl)-O-methyl-D-serine (CAS Number 86123-95-7; 500 mg, 2.28 mmol) in MeOH (5 mL) was added $SOCl_2$ (597 mg, 5.02 mmol) dropwise at 0 °C. The reaction mixture was stirred at 20 °C for 2 h. Upon completion, the mixture was evaporated. The residue was triturated with EtOAc (30 mL), filtered and the filter cake was dried to give methyl *O*-methyl-*D*-serinate hydrochloride (310 mg, 80% yield) as a white solid. $^1$H NMR (400 MHz, DMSO-$d_6$) δ ppm 8.74 (br s, 3H), 4.27 (t, *J* = 3.6 Hz, 1H), 3.78 (d, *J* = 3.6 Hz, 2H), 3.74 (s, 3H), 3.29 - 3.26 (s, 3H).

**Steps b-d.** These 3 steps were conducted in a similar manner to **Intermediate A10,** steps b-d.

m/z ES+ $[M+H]^+$ 271.9.

**Intermediate A12: *tert*-butyl hexahydro-1'*H*-spiro[azetidine-3,6'-pyrazino[2,1-*c*][1,4]oxazine]-1-carboxylate**

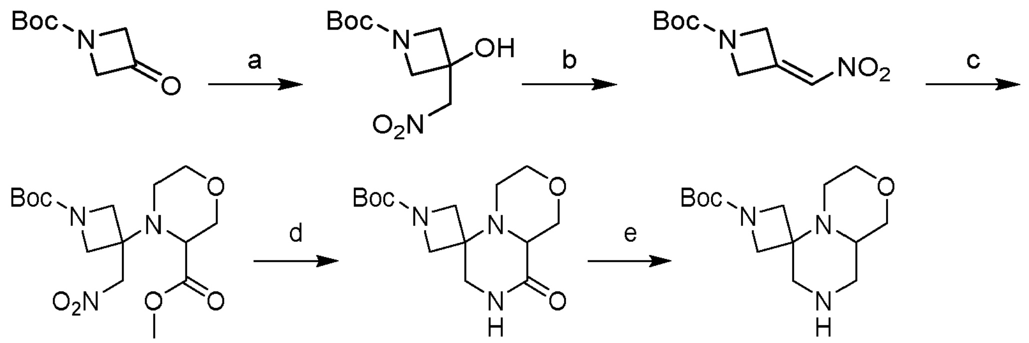


**Step a.** This step was conducted as described in **Intermediate A4,** step a.

**Step b.** A To a solution of *tert*-butyl 3-hydroxy-3-(nitromethyl)azetidine-1-carboxylate (2 g, 8.61 mmol) in DCM (20 mL) was added DAST (2.08 g, 12.9 mmol) dropwise at 0 °C. The reaction mixture was stirred at 0 °C for 30 min. Upon completion, the mixture was quenched with water (20 mL), and the layers were separated. The organic layer was washed with brine (50 mL), dried over $Na_2SO_4$ and evaporated. The crude product was purified by column chromatography (PE/EtOAc = 8/1) to give *tert*-butyl 3-(nitromethylene)azetidine-1-carboxylate (1.55 g, 84% yield) as a yellow solid.

$^1$H NMR (400 MHz, $CDCl_3$) δ ppm 7.08 - 7.02 (m, 1H), 5.06 - 5.00 (m, 2H), 4.75 - 4.67 (m, 2H), 1.48 (s, 9H).

**Step c.** To a solution of *tert*-butyl 3-(nitromethylene)azetidine-1-carboxylate (1.35 g, 6.30 mmol) in DCM (20 mL) was added methyl morpholine-3-carboxylate (CAS: 126264-49-1; 1.37 g, 9.45 mmol). The reaction mixture was stirred at 20 °C for 12 h. Upon completion, the mixture was evaporated and the crude product was purified by column chromatography (PE /EtOAc = 5/1) to give methyl 4-(1-(*tert*-butoxycarbonyl)-3-(nitromethyl)azetidin-3-yl)morpholine-3-carboxylate (1.2 g, 53% yield) as yellow oil.

$^1$H NMR (400 MHz, $CDCl_3$) δ ppm 4.77 - 4.65 (m, 2H), 4.33 (d, *J* = 8.8 Hz, 1H), 4.23 (d, *J* = 10.8 Hz, 1H), 4.07 (d, *J* = 9.6 Hz, 1H), 3.94 (d, *J* = 9.6 Hz, 1H), 3.84 (dd, *J* = 2.8, 10.8 Hz, 1H), 3.76 (s, 3H), 3.71 (d, *J* = 8.8 Hz, 1H), 3.62 (dd, *J* = 3.2,

11.2 Hz, 1H), 3.57 - 3.49 (m, 2H), 3.37 - 3.26 (m, 1H), 2.43 (d, *J* = 11.8 Hz, 1H), 1.46 (s, 9H).

**Steps d-e.** These 2 steps were conducted in a similar manner to **Intermediate A10,** steps c-d.

m/z ES+ $[M+H]^+$ 284.0.

**Intermediates A13a and A13b: *tert*-butyl (7'*R*/S,9a'*S*)-7'-methylhexahydro-1'*H*-spiro[azetidine-3,6'-pyrazino [2,1-*c*][1,4]oxazine]-1-carboxylate and *tert*-butyl**

**(7'*S*/*R*,9a'*S*)-7'-methylhexahydro-1'*H*-spiro[azetidine-3,6'-pyrazino[2,1-*c*][1,4]oxazine]-1-carboxylate**

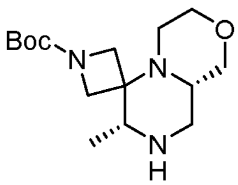

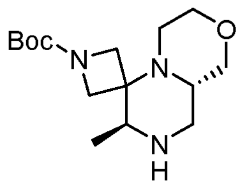

The title compound was prepared in a similar manner to **Intermediate A12,** using nitroethane in step a, and methyl (R)-morpholine-3-carboxylate (CAS: 1187933-47-6) in step c. The two diastereoisomers of unknown absolute configuration were separated during step d using Prep-HPLC. The separated enantiomers were then used separately in step e.

**Intermediate A13a**

m/z ES+ $[M+H]^+$ 298.0.

**Intermediate A13b**

m/z ES+ $[M+H]^+$ 298.0.

**Intermediate A14a and A14b: *tert*-butyl (3*S*,9a*S*/*R*)-3-methyloctahydro-2*H*-pyrazino[1,2-*a*]pyrazine-2-carboxylate** and **ter*t*-butyl (3*S*,9a*R*/*S*)-3-methyloctahydro-2*H*-pyrazino[1,2-*a*]pyrazine-2-carboxylate**

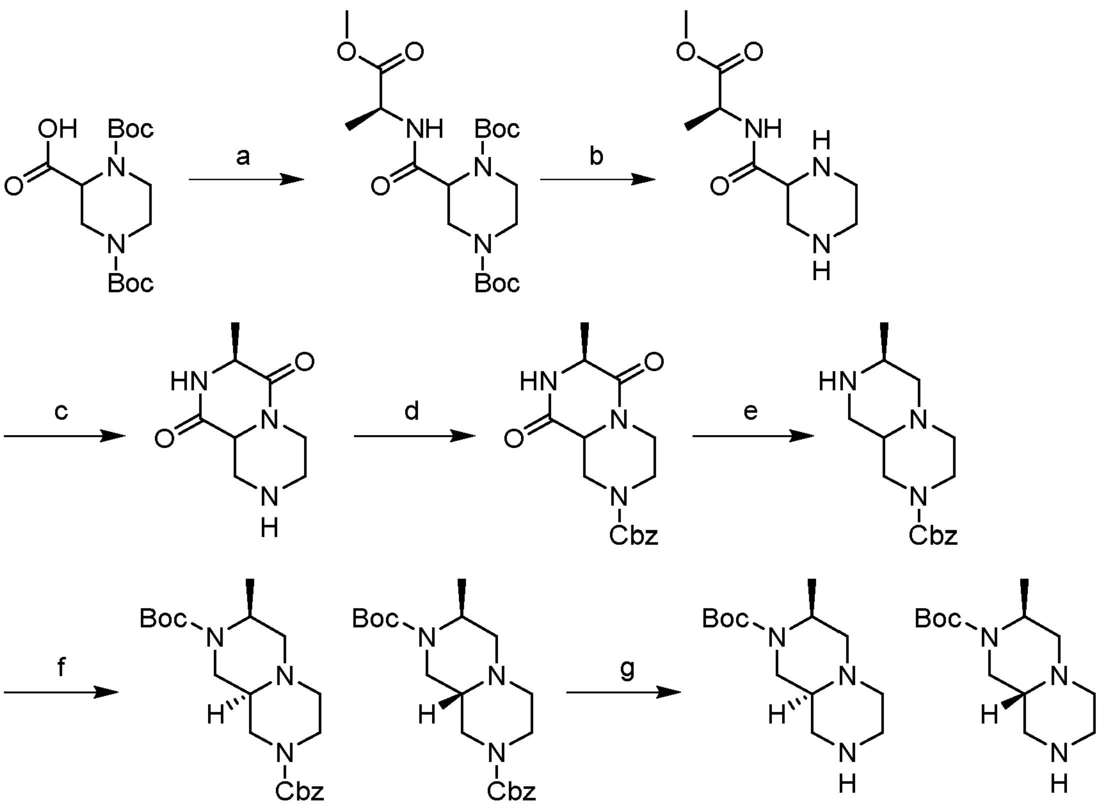


**Step a.** A mixture of 1,4-bis(*tert*-butoxycarbonyl)piperazine-2-carboxylic acid (CAS: 181955-79-3; 5 g, 15.1 mmol), methyl L-alaninate hydrochloride (CAS: 2491-20-5; 2.53 g, 18.2 mmol), HATU (5.75 g, 15.1 mmol) and DIPEA (9.78 g, 75.7 mmol) in DCM (50 mL) was stirred at 25 °C for 1 h. Upon completion, the reaction mixture was evaporated and the crude product was purified by column chromatography (PE/EtOAc= 1/1) to give di-tert-butyl 2-(((S)-1-methoxy-1-oxopropan-2-yl)carbamoyl)piperazine-1,4-dicarboxylate (6.0 g, 95% yield) as a colourless oil. m/z ES+ $[M+H]^+$ 416.5; $^1H$ NMR (400 MHz, $CDCl_3$) δ ppm 7.01 - 6.57 (m, 1H), 4.65 - 4.61 (m, 1H), 4.58 - 4.37 (m, 2H), 3.99 - 3.80 (m, 2H), 3.76 - 3.71 (m, 3H), 3.29 - 2.85 (m, 3H), 1.49 (s, 9H), 1.45 (s, 9H), 1.40 (d, *J* = 7.2 Hz, 3H).

**Step b.** A mixture of di-*tert*-butyl 2-(((*S*)-1-methoxy-1-oxopropan-2-yl)carbamoyl)piperazine-1,4-dicarboxylate (2.0 g, 4.8 mmol) and TFA (15.4 g, 135 mmol) in DCM (10 mL) was stirred at 0 °C for 2 h. Upon completion, the reaction mixture was evaporated to give methyl (piperazine-2-carbonyl)-*L*-alaninate as a TFA salt (1.6 g, crude) as a yellow oil.

m/z ES+ $[M+H]^+$ 216.1.

**Step c.** A mixture of methyl (piperazine-2-carbonyl)-*L*-alaninate (1.6 g, 4.86 mmol) and TEA (2.46 g, 24.3 mmol) in THF (20 mL) was stirred at 60 °C for 4 h. Upon completion, the reaction mixture was evaporated and the crude product was purified by column chromatography (DCM/MeOH = 10/1) to give (7S)-7-methylhexahydro-2*H*-pyrazino[1,2-a] pyrazine-6,9-dione (1.0 g, crude) as a yellow oil.

m/z ES+ $[M+H]^+$ 184.2; $^1H$ NMR (400 MHz, $CDCl_3$) δ ppm 4.74 - 4.60 (m, 1H), 4.50 - 4.45 (m, 1H), 4.23 - 4.14 (m, 2H), 3.85 - 3.79 (m, 1H), 3.42 - 3.30 (m, 1H), 3.07 - 2.85 (m, 3H), 1.55 (t, *J* = 6.8 Hz, 3H).

**Step d.** A mixture of (7S)-7-methylhexahydro-2*H*-pyrazino[1,2-a]pyrazine-6,9-dione (1.0 g, 5.46 mmol), CbzCl (1.12 g, 6.55 mmol), $NaHCO_3$ (2.29 g, 27.3 mmol) in THF (10 mL) and water (10 mL) was stirred at 25 °C for 12 h. Upon completion, the reaction mixture was evaporated, water (100 mL) was added and the mixture was extracted with EtOAc (3 x 50 mL). The combined organic layers were washed with brine (50 mL), dried over $Na_2SO_4$ and evaporated. The crude product was purified by column chromatography (PE/EtOAc = 1/1) to give benzyl (7*S*)-7-methyl-6,9-dioxooctahydro-2*H*-pyrazino[1,2-*a*]pyrazine-2-carboxylate (550 mg, 32% yield) as a colourless oil.

$^1H$ NMR (400 MHz, $CDCl_3$) δ ppm 7.38 - 7.23 (m, 5H), 6.54 - 6.41 (m, 1H), 5.22 - 5.02 (m, 2H), 4.64 - 4.62 (m, 1H), 4.48 (t, *J* = 12.4 Hz, 1H), 4.24 - 4.02 (m, 2H), 3.98 - 3.85 (m, 1H), 2.83 - 2.78 (m, 2H), 2.72 - 2.56 (m, 1H), 1.46 (t, *J* = 7.6 Hz, 3H).

**Step e.** A mixture of benzyl (7*S*)-7-methyl-6,9-dioxooctahydro-2*H*-pyrazino[1,2-*a*]pyrazine-2-carboxylate (500 mg, 1.58 mmol) and borane dimethyl sulfide complex (10 M in THF, 0.47 mL) in THF (5 mL) was stirred at 20 °C for 1 h. Upon completion, the reaction mixture was quenched with MeOH (10 mL) and stirred at 25 °C for 2 h. The reaction mixture was evaporated to give benzyl (7*S*)-7-methyloctahydro-2*H*-pyrazino[1,2-*a*]pyrazine-2-carboxylate (500 mg, crude) as a colourless oil.

m/z ES+ $[M+H]^+$ 290.5.

**Step f.** A mixture of benzyl (7*S*)-7-methyloctahydro-2*H*-pyrazino[1,2-*a*]pyrazine-2-carboxylate (500 mg, 1.73 mmol), di-*tert*-butyl dicarbonate (377 mg, 1.73 mmol) and DIPEA (669 mg, 5.18 mmol) in THF (10 mL) was stirred at 25 °C for 1 h. Upon completion, the reaction mixture was evaporated and purified by column chromatography (PE/EtOAc= 1/1) to give two diastereoisomers of unknown absolute configuration.

8-Benzyl 2-(*tert*-butyl) (3*S*,9a*S*/*R*)-3-methylhexahydro-2*H*-pyrazino[1,2-*a*]pyrazine-2,8(1*H*)-dicarboxylate (110 mg, 16% yield) as a yellow oil.

m/z ES+ $[M+H]^+$ 390.1; $^1$H NMR (400 MHz, $CDCl_3$) δ ppm 7.46 - 7.31 (m, 5H), 5.15 (s, 2H), 4.00 - 3.88 (m, 3H), 3.61 - 3.56 (m, 1H), 3.17 - 3.07 (m, 2H), 2.99 - 2.96 (m, 1H), 2.90 - 2.84 (m, 1H), 2.76 - 2.72 (m, 1H), 2.62 - 2.51 (m, 1H), 2.45 - 2.37 (m, 1H), 2.15 (dd, *J* = 7.2, 11.6 Hz, 1H), 1.47 (s, 9H), 1.34 (d, *J* = 6.4 Hz, 3H).

8-Benzyl 2-(*tert*-butyl) (3*S*,9a*R*/*S*)-3-methylhexahydro-2*H*-pyrazino[1,2-*a*]pyrazine-2,8(1*H*)-dicarboxylate (110 mg, 16% yield) as a yellow oil.

m/z ES+ $[M+H]^+$ 390.1; $^1$H NMR (400 MHz, $CDCl_3$) δ ppm 7.38 - 7.32 (m, 5H), 5.15 (s, 2H), 4.22 - 3.91 (m, 3H), 3.87 - 3.60 (m, 1H), 3.08 (s, 1H), 2.79 - 2.51 (m, 4H), 2.32 - 2.29 (m, 1H), 2.16 - 2.04 (m, 1H), 1.96 - 1.90 (m, 1H), 1.47 (s, 9H), 1.28 - 1.21 (m, 3H).

**Step g.** To a solution of 8-benzyl 2-(*tert-butyl*) (3*S*,9a*R*/*S*)-3-methylhexahydro-2*H*-pyrazino[1,2-*a*]pyrazine-2,8(1*H*)-dicarboxylate (100 mg, 0.26 mmol) in 2-propanol (10 mL) was added 10% Pd/C (10 mg) and the mixture was stirred at rt for 4 h under a $H_2$ atmosphere (15 psi). Upon completion, the reaction mixture was filtered and evaporated to give *tert*-butyl (3*S*,9a*R*/*S*)-3-methyloctahydro-2*H*-pyrazino[1,2-*a*]pyrazine-2-carboxylate (50 mg, 76% yield) as a colourless oil.

**Intermediate A14a:**

m/z ES+ $[M+H]^+$ 256.5.

**Intermediate A14b** was prepared in a similar manner to **Intermediate A14a,** using 8-benzyl 2-(*tert*-butyl) (3*S*,9a*S*/*R*)-3-methylhexahydro-2*H*-pyrazino[1,2-*a*]pyrazine-2,8(1*H*)-dicarboxylate in step g.

**Intermediate A14b:**

m/z ES+ $[M+H]^+$ 256.5.

**Intermediate A15: *tert*-butyl 3-(((*rel-trans*)-4-hydroxypyrrolidin-3-yl)oxy)azetidine-1-carboxylate**

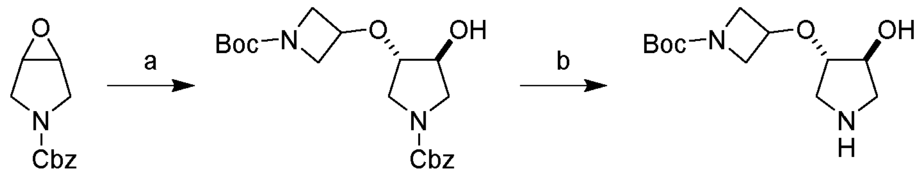


**Step a.** To a solution of benzyl 6-oxa-3-azabicyclo[3.1.0]hexane-3-carboxylate (CAS: 31865-25-5; 10.0 g, 45.6 mmol) in DMSO (40 mL) and water (40 mL) was added $Cs_2CO_3$ (22.3 g, 68.4 mmol) and *tert*-butyl 3-hydroxyazetidine-1-carboxylate (CAS: 141699-55-0; 9.5 g, 54.7 mmol). The reaction mixture was then stirred at 110 °C under microwave irradiation for 45 min. Upon completion, the reaction mixture was poured into cold water (50 mL) and extracted with EtOAc (3 x 100 mL). The combined organic layers were washed with cold water, brine, dried over $Na_2SO_4$ and evaporated. Purification by column chromatography gave benzyl (*rel-trans*)-3-((1-(*tert*-butoxycarbonyl)azetidin-3-yl)oxy)-4-hydroxypyrrolidine-1-carboxylate as an off-white solid (3.7 g, 21% yield).

m/z ES+ $[M-CO_2tBu]^+$ 293.6.

**Step b.** To a solution of benzyl (*rel-trans*)-3-((1-(*tert*-butoxycarbonyl)azetidin-3-yl)oxy)-4-hydroxypyrrolidine-1-carboxylate (3.7 g, 9.4 mmol) in MeOH (100 mL) was added 10% Pd/C (3.6 g) and the mixture was stirred at rt for 2 h under a $H_2$ atmosphere. Upon completion, the reaction mixture was filtered and evaporated to give the title compound as an off-white solid (1.5 g, 62% yield).

m/z ES+ $[M+H]^+$ 259.6.

**Intermediate A16: benzyl 3-((*rel-trans*)-hexahydropyrrolo[3,4-b][1,4]oxazin-4(4aH)-yl)azetidine-1-carboxylate**

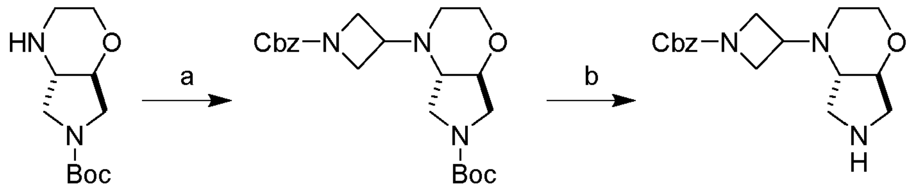


**Step a.** To a solution of *tert*-butyl (*rel-trans*)-hexahydropyrrolo[3,4-*b*][1,4]oxazine-6(2*H*)-carboxylate (CAS: 138026-93-4; 950 mg, 4.16 mmol) and benzyl 3-oxoazetidine-1-carboxylate (CAS: 105258-93-3; 1.28 g, 6.24 mmol) in DCE (15 mL) was added 4 Å molecular sieves (1 g). The mixture was stirred at 20 °C for 30 min, then $NaBH(OAc)_3$ (2.65 g, 12.8 mmol) was added. The mixture was stirred at 20 °C for a further 12 h. Upon completion, the mixture was filtered and evaporated. The residue was purified by column chromatography (PE/EtOAc = 1/1) to give *tert*-butyl (*rel-trans*)-4-(1-((benzyloxy)carbonyl)azetidin-3-yl)hexahydropyrrolo[3,4-*b*][1,4]oxazine-6(2*H*)-carboxylate (1.8 g, crude) as a colourless oil.
$^1H$ NMR (400 MHz, $CDCl_3$) δ ppm 7.36 - 7.32 (m, 5H), 5.11 (s, 2H), 4.68 - 4.61 (m, 1H), 4.27 - 4.23 (m, 1H), 4.05 - 3.99 (m, 2H), 3.90 (dd, *J* = 4.4, 10.4 Hz, 2H), 3.86 - 3.76 (m, 1H), 3.65 - 3.54 (m, 2H), 3.31 - 3.19 (m, 1H), 3.14 - 3.03 (m, 2H), 2.79 - 2.67 (m, 1H), 2.29 - 2.12 (m, 2H), 1.46 (s, 9H).
**Step b.** To a solution of *tert*-butyl (*rel-trans*)-4-(1-((benzyloxy)carbonyl)azetidin-3-yl)hexahydropyrrolo[3,4-*b*][1,4]oxazine-6(2*H*)-carboxylate (1.0 g, 2.40 mmol) in DCM (10 mL) was added TFA (2 mL). The mixture was stirred at 20 °C for 30 min. Upon completion, the mixture was diluted with DCM (5 mL) and basified to pH 8 with sat. aq. $NaHCO_3$. The layers were separated, and the aqueous phase was further extracted with DCM (4 x 15 mL). The combined organic layers were washed with brine (20 mL), dried over $Na_2SO_4$ and evaporated to give the title compound (760 mg, crude) as a colourless oil.
m/z ES+ $[M+H]^+$ 318.2.

**Intermediate A17a: 2-benzyl 8-(*tert*-butyl) (rel-3*R*,9a*R*)-3-methyl-4-oxohexahydro-2*H*-pyrazino[1,2-*a*]pyrazine-2,8(1*H*)-dicarboxylate and**

**Intermediate A17b: 2-benzyl 8-(*tert*-butyl) (rel-3*R*,9a*S*)-3-methyl-4-oxohexahydro-2*H*-pyrazino[1,2-*a*]pyrazine-2,8(1*H*)-dicarboxylate**

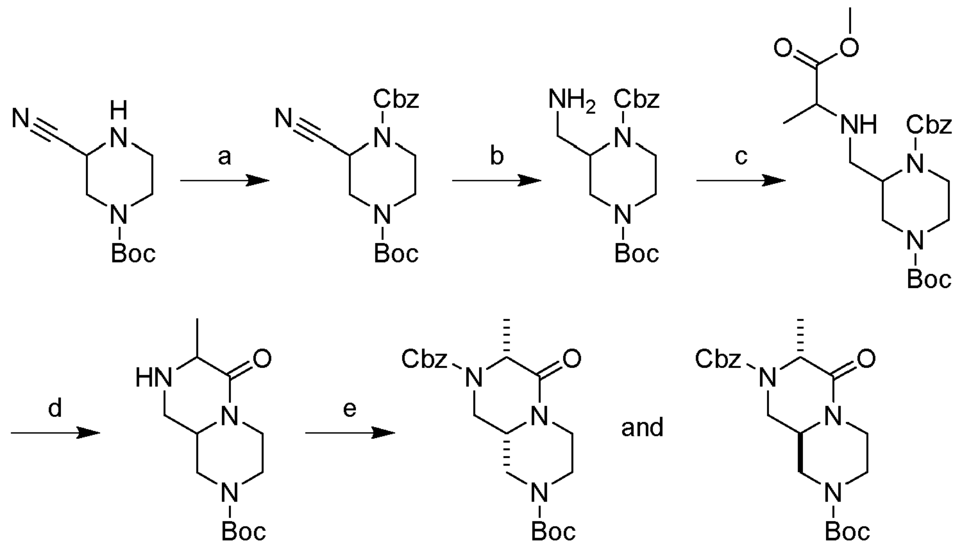


**Step a.** To a solution of *tert*-butyl 3-cyanopiperazine-1-carboxylate (CAS: 859518-35-7; 1.3 g, 6.15 mmol) in THF (10 mL) and water (10 mL) was added $Na_2CO_3$ (1.3 g, 12.3 mmol) and CbzCl (1.15 g, 6.77 mmol). The mixture was stirred at 25 °C for 12 h. Upon completion, the reaction mixture was diluted with $H_2O$ (20 mL) and extracted with EtOAc (3 x 30 mL). The conbined organic layers were washed with brine (50 mL), dried over $Na_2SO_4$, evaporated and purified by prep-TLC

(PE/EtOAc = 6/1) to give 1-benzyl 4-(*tert*-butyl) 2-cyanopiperazine-1,4-dicarboxylate (100 mg, 43% yield) as an off-white solid.

m/z ES+ $[M+Na]^+$ 368.1.

**Step b.** A mixture of 1-benzyl 4-(*tert*-butyl) 2-cyanopiperazine-1,4-dicarboxylate (2.1 g, 6.08 mmol), Raney-Ni (1 g, 11.7 mmol) and 30% $NH_4OH$ (71 mg, 0.61 mmol) in MeOH (25 mL) was degassed and purged with $H_2$ 3 times. The reaction mixture was stirred at 25 °C for 5 h under a $H_2$ atmosphere (50 psi). Upon completion, the reaction mixture was filtered and evaporated to give 1-benzyl 4-(*tert*-butyl) 2-(aminomethyl)piperazine-1,4-dicarboxylate (2.1 g, crude) as a colourless oil.

$^1H$ NMR (400 MHz, $CDCl_3$) δ ppm 7.36 - 7.28 (m, 5H), 5.22 - 5.11 (m, 2H), 4.15 - 3.84 (m, 4H), 3.12 - 2.80 (m, 5H), 1.47 (s, 9H), 1.07 (s, 2H).

Step c. To a solution of 1-benzyl 4-(*tert*-butyl) 2-(aminomethyl)piperazine-1,4-dicarboxylate (2.1 g, 6.01 mmol) in THF (25 mL) was added TEA (1.22 g, 12.0 mmol) and methyl 2-bromopropanoate (CAS: 5445-17-0; 1.30 g, 7.81 mmol). The reaction mixture was stirred at 80 °C for 12 h. Upon completion, the mixture was diluted with water (40 mL) and extracted with EtOAc (3 x 40 mL). The combine organic layers were washed with brine (100 mL), dried over $Na_2SO_4$, evaporated and purified by column chromatography (PE/EtOAc = 1/1) to give 1-benzyl 4-(*tert*-butyl) 2-(((1-methoxy-1-oxopropan-2-yl)amino)methyl)piperazine-1,4-dicarboxylate (1.5 g, 57% yield) as a colourless oil.

m/z ES+ $[M+H]^+$436.2; $^1H$ NMR (400 MHz, $CDCl_3$) δ ppm 7.39 - 7.31 (m, 5H), 5.20 - 5.09 (m, 2H), 4.23 - 4.06 (m, 2H), 4.02 - 3.83 (m, 2H), 3.70 (s, 3H), 3.44 - 3.27 (m, 1H), 3.09 - 2.90 (m, 2H), 2.80 (dd, *J* = 12.0, 8.0 Hz, 2H), 2.56 (dd, *J* = 11.6, 8.0 Hz, 1H), 1.67 - 1.53 (m, 1H), 1.47 (d, *J* = 3.6 Hz, 9H), 1.30 - 1.20 (m, 3H).

**Step d.** A mixture of 1-benzyl 4-(*tert*-butyl) 2-(((1-methoxy-1-oxopropan-2-yl)amino)methyl)piperazine-1,4-dicarboxylate (2.9 g, 6.66 mmol), 10% Pd/C (300 mg) in MeOH (40 mL) was degassed and purged with $H_2$ 3 times. The reaction mixture was stirred at 45 °C for 12 h under a $H_2$ atmosphere (15 psi). Upon completion, the reaction mixture was filtered and evaporated to give *tert*-butyl 7-methyl-6-oxooctahydro-2*H*-pyrazino[1,2-a]pyrazine-2-carboxylate (1.75 g, crude) as a colourless oil.

$^1H$ NMR. (400 MHz, $CDCl_3$) δ ppm 4.64 - 4.54 (m, 1H), 4.11 - 3.91 (m, 2H), 3.57 - 3.32 (m, 2H), 3.31 - 3.13 (m, 1H), 2.98 - 2.69 (m, 3H), 2.68 - 2.45 (m, 2H), 1.48 (s, 9H), 1.40 (dd, *J* = 6.8, 4.0 Hz, 3H).

**Step e.** To a solution of *tert*-butyl 7-methyl-6-oxooctahydro-2*H*-pyrazino[1,2-a]pyrazine-2-carboxylate (1.2 g, 4.46 mmol) in THF (12 mL) and water (12 mL) was added $Na_2CO_3$ (944 mg, 8.91 mmol) and CbzCl (1.14 g, 6.68 mmol). The reaction mixture was stirred at 25 °C for 12 h. Upon completion, the reaction mixture was diluted with water (20 mL) and extracted with EtOAc (3 x 30 mL). The combined organic layers were washed with brine (60 mL), dried over $Na_2SO_4$, evaporated and purified by column chromatography (PE/EtOAc = 3/2) to give two products of unknown absolute configuration.

**Intermediate A17a:** 2-benzyl 8-(*tert*-butyl) (*rel*-3*R*/*S*,9a*R*/*S*)-3-methyl-4-oxohexahydro-2*H*-pyrazino[1,2-a]pyrazine-2,8(1*H*)-dicarboxylate (550 mg, 29% yield) as a white solid.

m/z ES+ [M-*t*Bu+H]$^+$ 348.0; $^1H$ NMR (400 MHz, $CDCl_3$) δ ppm 7.43 - 7.30 (m, 5H), 5.16 (s, 2H), 4.84 - 4.65 (m, 1H), 4.52 (d, *J* = 12.4 Hz, 1H), 4.39 - 3.97 (m, 3H), 3.51 - 3.50 (m, 1H), 2.99 - 2.77 (m, 2H), 2.75 - 2.63 (m, 1H), 2.50 - 2.49 (m, 1H), 1.48 (s, 9H), 1.46 (d, *J* = 7.2 Hz, 3H).

**Intermediate A17b:** 2-benzyl 8-(*tert*-butyl) (*rel*-3*R*/*S*,9a*S*/*R*)-3-methyl-4-oxohexahydro-2*H*-pyrazino[1,2-*a*]pyrazine-2,8(1*H*)-dicarboxylate (430 mg, 23% yield) as a yellow oil.

m/z ES+ [M-*t*Bu+H]$^+$348.0; $^1H$ NMR (400 MHz, $CDCl_3$) δ ppm 7.43 - 7.30 (m, 5H), 5.22 - 5.11 (m, 2H), 4.75 (d, *J* = 6.0 Hz, 1H), 4.59 (d, *J* = 11.4 Hz, 1H), 4.10 - 3.90 (m, 3H), 3.45 (dd, *J* = 14.0, 4.8 Hz, 1H), 3.37 (d, *J* = 11.6 Hz, 1H), 2.88 - 2.73 (m, 2H), 2.72 - 2.62 (m, 1H), 1.47 (s, 9H), 1.45 (d, *J* = 7.2 Hz, 3H).

**Intermediate A18a: 2-benzyl 8-(*tert*-butyl) (3*R*/*S*,9a*R*/*S*)-3-methyl-4-oxohexahydro-2*H*-pyrazino[1,2-*a*]pyrazine-2,8(1*H*)-dicarboxylate and**

**Intermediate A18b: 2-benzyl 8-(*tert*-butyl) (3*S*/*R*,9a*S*/*R*)-3-methyl-4-oxohexahydro-2*H*-pyrazino[1,2-*a*]pyrazine-2,8(1*H*)-dicarboxylate**

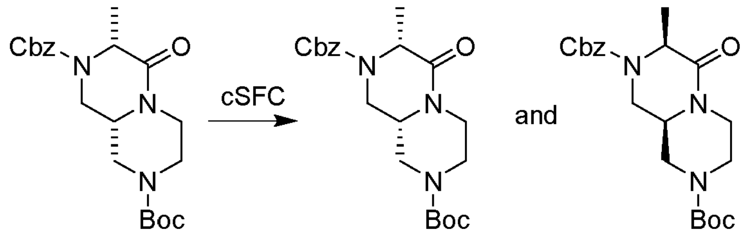


The title compounds were prepared by chiral SFC separation of **Intermediate.**

**Intermediate A18a:**

m/z ES+ $[M+H]^+$ 404.0; $^1H$ NMR (400 MHz, $CDCl_3$) δ ppm 7.40 - 7.30 (m, 5H), 5.16 (s, 2H), 4.84 - 4.63 (m, 1H), 4.52 (d, *J* = 12.4 Hz, 1H), 4.39 - 4.01 (m, 3H), 3.51 - 3.50 (m, 1H), 3.00 - 2.78 (m, 2H), 2.74 - 2.62 (m, 1H), 2.51 - 2.40 (m, 1H), 1.48 (s, 9H), 1.46 (d, *J* = 7.2 Hz, 3H).

**Intermediate A18b:**

m/z ES+ $[M-tBu]^+$ 347.9; $^1H$ NMR (400 MHz, $CDCl_3$) δ ppm 7.40 - 7.30 (m, 5H), 5.16 (s, 2H), 4.85 - 4.63 (m, 1H), 4.52 (d, *J* = 12.4 Hz, 1H), 4.39 - 4.02 (m, 3H), 3.51 - 3.49 (s, 1H), 2.99 - 2.78 (m, 2H), 2.74 - 2.63 (m, 1H), 2.50 - 2.48 (s, 1H), 1.48 (s, 9H), 1.46 (d, *J* = 7.2 Hz, 3H).

**Intermediate A18c: 2-benzyl 8-(*tert*-butyl) (3*R*/*S*,9a*S*/*R*)-3-methyl-4-oxohexahydro-2*H*-pyrazino[1,2-*a*]pyrazine-2,8(1*H*)-dicarboxylate and**

**Intermediate A18d: 2-benzyl 8-(*tert*-butyl) (3*S*/*R*,9a*R*/*S*)-3-methyl-4-oxohexahydro-2*H*-pyrazino[1,2-*a*]pyrazine-2,8(1*H*)-dicarboxylate**

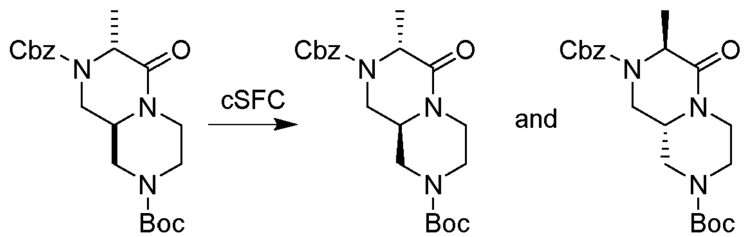


The title compounds were prepared by chiral SFC separation of **Intermediate A17b.**

**Intermediate A18c:**

m/z ES+ $[M+H]^+$404.0.

**Intermediate A18d:**

m/z ES+ $[M+H]^+$404.0.

**Intermediate A19a: benzyl (3*R*/*S*,9a*R*/*S*)-3-methyl-4-oxooctahydro-2*H*-pyrazino[1,2-*a*]pyrazine-2-carboxylate**

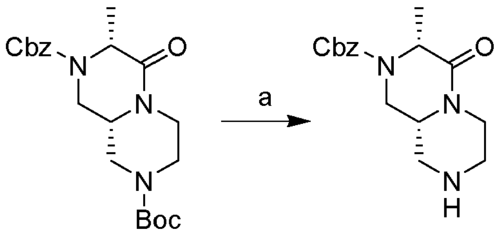


**Step a.** To a solution of **Intermediate A18a** (110 mg, 0.27 mmol) in DCM (1.5 mL) was added TFA (508 mg, 4.46 mmol). The mixture was stirred at 25 °C for 1 h. Upon completion, the reaction mixture was basified to pH 8 with sat. aq. $NaHCO_3$ and extracted with EtOAc (3 x 15 mL). The combine organic layers were washed with brine (30 mL), dried over $Na_2SO_4$ and evaporated to give the title compound (68 mg, crude) as a yellow solid.
m/z ES+ $[M+H]^+$ 304.0

**Intermediate A19b: benzyl (3*S*/*R*,9a*S*/*R*)-3-methyl-4-oxooctahydro-2*H*-pyrazino[1,2-*a*]pyrazine-2-carboxylate**

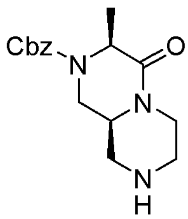

The title compound was prepared in a similar manner to **Intermediate A19a,** using

**Intermediate A18b.**

m/z ES+ $[M+H]^+$304.1.

**Intermediate A19c: benzyl (3*R*/*S*,9a*S*/*R*)-3-methyl-4-oxooctahydro-2*H*-pyrazino[1,2-*a*]pyrazine-2-carboxylate**

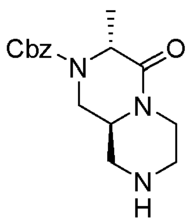

The title compound was prepared in a similar manner to **Intermediate A19a,** using **Intermediate A18c.**
m/z ES+ $[M+H]^+$304.1.

**Intermediate A19d: benzyl (3*S*/*R*,9a*R*/*S*)-3-methyl-4-oxooctahydro-2*H*-pyrazino[1,2-a]pyrazine-2-carboxylate**

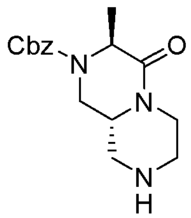

The title compound was prepared in a similar manner to **Intermediate A19a,** using **Intermediate A18d.**
m/z ES+ [M+H]+ 304.2.

**Intermediate A20a: *tert*-butyl (7*R*/*S*,9a*S*/*R*)-7-methyl-6-oxooctahydro-2*H*-pyrazino[1,2-a]pyrazine-2-carboxylate**

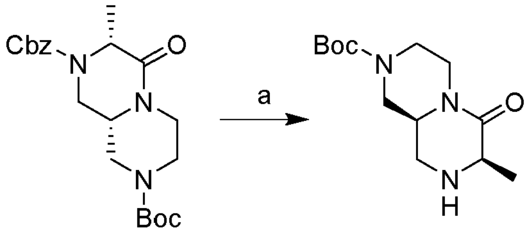


**Step a.** A mixture of **Intermediate A18a** (110 mg, 0.27 mmol) and 10% Pd/C (10 mg) in MeOH (2 mL) was degassed and purged with $H_2$ 3 times. The mixture was stirred at 30 °C for 1 h under a $H_2$ atmosphere (15 psi). The mixture was filtered and evaporated to give the title compound (65 mg, crude) as a yellow solid.
m/z ES+ [M+H]+270.0.

**Intermediate A20b: *tert*-butyl (7*S*/*R*,9a*R*/*S*)-7-methyl-6-oxooctahydro-2*H*-pyrazino[1,2-*a*]pyrazine-2-carboxylate**

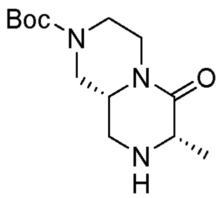

The title compound was prepared in a similar manner to Intermediate A20a, using **Intermediate A18b.**
m/z ES+ [M+H]+270.1.

**Intermediate A20c: *tert*-butyl (7*R*/*S*,9a*R*/*S*)-7-methyl-6-oxooctahydro-2*H*-pyrazino[1,2-*a*]pyrazine-2-carboxylate**

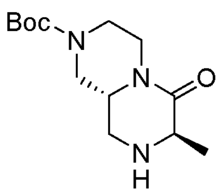

The title compound was prepared in a similar manner to **Intermediate A20a,** using **Intermediate A18c.**
m/z ES+ [M+H]+270.1.

**Intermediate A20d: *tert*-butyl (7*S*/*R*,9a*S*/*R*)-7-methyl-6-oxooctahydro-2*H*-pyrazino[1,2-a]pyrazine-2-carboxylate**

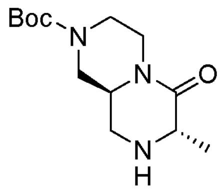

The title compound was prepared in a similar manner to **Intermediate A20a,** using **Intermediate A18d.**
m/z ES+ $[M+H]^+$270.1.

**Intermediate A21: *tert*-butyl 6-oxooctahydro-2*H*-pyrazino[1,2-*a*]pyrazine-2-carboxylate**

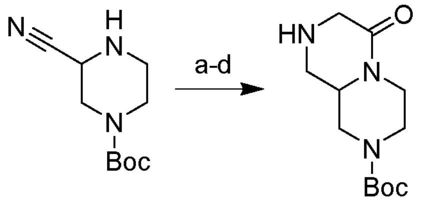


**Steps a-d.** These 4 steps were conducted in a similar manner to **Intermediate A17a** and **Intermediate A17b,** steps a-d, using methyl 2-bromoacetate in step c.
m/z ES+ $[M+H]^+$ 256.2; $^1$H NMR (400MHz, $CDCl_3$) δ ppm 4.62 (d, *J* = 13.2 Hz, 1H), 4.06 (s, 2H), 3.61 - 3.46 (m, 2H), 3.45 - 3.36 (m, 1H), 3.30 - 3.20 (m, 1H), 2.83 - 2.78 (m, 1H), 2.77 - 2.70 (m, 1H), 2.69 - 2.53 (m, 2H), 1.48 (s, 9H).

**Intermediate A22: benzyl 4-oxooctahydro-2*H*-pyrazino[1,2-*a*]pyrazine-2-carboxylate**

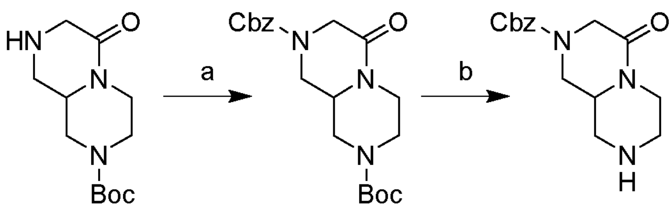


**Step a.** This step was conducted in a similar manner to **Intermediate A17a** and **Intermediate A17b,** step e, using **Intermediate A21.**
**Step b.** This step was conducted in a similar manner to **Intermediate 19a.**
m/z ES+ $[M+H]^+$290.1; $^1$H NMR (400MHz, $CDCl_3$) δ ppm 7.40 - 7.30 (m, 5H), 5.16 (s, 2H), 4.58 (d, *J* = 10.8 Hz, 1H), 4.41 (d, *J* = 18.0 Hz, 1H), 4.09 (s, 1H), 3.97 (d, *J* = 18.0 Hz, 1H), 3.55 - 3.45 (m, 1H), 3.21 - 2.96 (m, 3H), 2.78 - 2.63 (m, 2H), 2.49 (t, *J* = 11.2 Hz, 1H).

**Intermediate A23a: methyl (*rel*-2*R*,6*R*)-4-benzyl-6-methylpiperazine-2-carboxylate and**

**Intermediate A23b: methyl (rel-2*S*,6*R*)-4-benzyl-6-methylpiperazine-2-carboxylate**

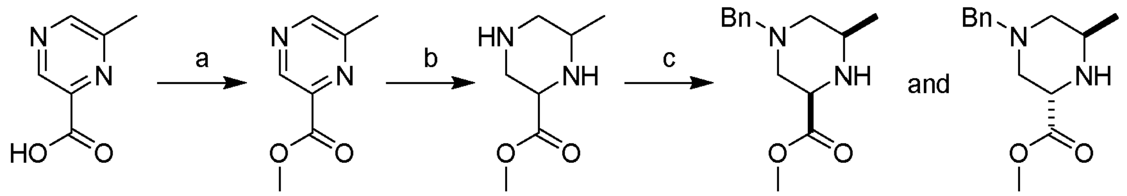


**Step a.** To a mixture of 6-methylpyrazine-2-carboxylic acid (10.0 g, 72.4 mmol) and $Cs_2CO_3$ (47.1 g, 144 mmol) in DMF (100 mL) was added MeI (15.4 g, 108 mmol). The reaction mixture was stirred at 25 °C for 1 h. Upon completion, the reaction mixture was quenched with water (100 mL) and extracted with EtOAc (3 x 150 mL). The combined organic layers were washed with brine (100 mL), dried over $Na_2SO_4$ and evaporated to give methyl 6-methylpyrazine-2-carboxylate (6.5 g, 59% yield) as a yellow solid.

$^1H$ NMR (400 MHz, $CDCl_3$) δ ppm 9.15 (s, 1H), 8.67 (s, 1H), 4.06 (s, 3H), 2.71 (s, 3H).

**Step b.** A mixture of methyl 6-methylpyrazine-2-carboxylate (6.50 g, 42.7 mmol), $PtO_2$ (650 mg, 2.86 mmol) and acetic acid (5.13 g, 85.4 mmol) in MeOH (120 mL) was stirred at 50 °C for 48 h under a $H_2$ atmosphere (50 psi). Upon completion, the reaction mixture was filtered and evaporated to give methyl 6-methylpiperazine-2-carboxylate (6.0 g, 88% yield) as a yellow oil.

m/z ES+ $[M+H]^+$ 158.9.

**Step c.** A mixture of methyl 6-methylpiperazine-2-carboxylate (5.0 g, 15.8 mmol) and benzaldehyde (1.68 g, 15.8 mmol) in MeOH (100 mL) was stirred at 0 °C for 30 min. After which, $NaBH_3CN$ (1.19 g, 18.9 mmol) was added and stirred at 0 °C for a further 1 h. Upon completion, the reaction mixture was evaporated, dissolved in water (50 mL) and extracted with EtOAc (2 x 50 mL). The combined organic layers were washed with brine (50 mL), dried over $Na_2SO_4$ and evaporated. The residue was purified by reverse phase flash chromatography (water (0.1% $NH_4OH$)/MeCN) to give two products.

**Intermediate A23a:** methyl (rel-2R,6R)-4-benzyl-6-methylpiperazine-2-carboxylate (2.10 g, 53% yield) as yellow oil

m/z ES+ $[M+H]^+$ 249.1; $^1H$ NMR (400 MHz, $CDCl_3$) δ ppm 7.43 - 7.28 (m, 5H), 3.74 (s, 3H), 3.64 (s, 1H), 3.59 - 3.51 (m, 1H), 3.47 - 3.40 (m, 1H), 3.29 - 3.15 (m, 2H), 2.70 (d, *J* = 10.6 Hz, 1H), 2.34 (dd, *J* = 11.2, 3.2 Hz, 1H), 1.86 (t, *J* = 10.4 Hz, 1H), 1.04 (d, *J* = 6.4 Hz, 3H). **Intermediate A23b:** methyl (rel-2*S*,6*R*)-4-benzyl-6-methylpiperazine-2-carboxylate (1.20 g, 31% yield) as yellow oil.

m/z ES+ $[M+H]^+$249.1; $^1H$ NMR (400 MHz, $CDCl_3$) δ ppm 7.41 - 7.28 (m, 5H), 3.72 (s, 3H), 3.60 - 3.55 (m, 2H), 3.17 - 3.14 (m, 1H), 3.00 - 2.85 (m, 1H), 2.80 - 2.75 (m, 1H), 2.10 - 1.95 (m, 1H), 1.75 - 1.67 (m, 2H), 1.07 (d, *J* = 6.4 Hz, 3H).

**Intermediate A24a: (rel-4*R*,9a*S*)-2-benzyl-4-methyloctahydro-2*H*-pyrazino[1,2-*a*]pyrazine**

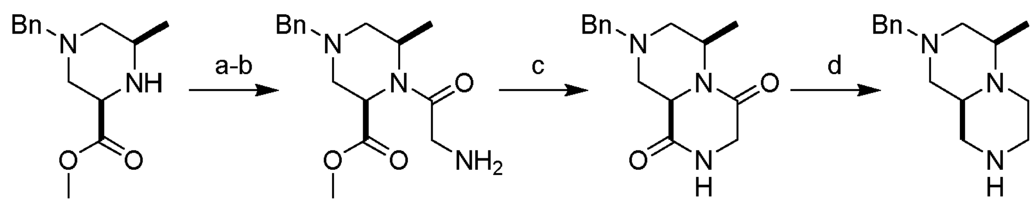


**Step** a. A mixture of (tert-butoxycarbonyl)glycine (1.69 g, 9.66 mmol), HATU (3.67 g, 9.66 mmol) and DIPEA (3.12 g, 24.1 mmol) in DMF (15 mL) was stirred at 25 °C for 30 min. **Intermediate A23a** (2.00 g, 8.05 mmol) in DMF (15 mL) was then added and the reaction mixture was stirred at 25 °C for a further 2 h. Upon completion, the reaction mixture was diluted with water (50 mL) and extracted with EtOAc (2 x 50 mL). The combined organic layers were washed with brine (20 mL), dried over $Na_2SO_4$, evaporated and purified by column chromatography (PE/EtOAc = 3/1) to give methyl (*rel*-2*R*,6*R*)-4-benzyl-1-((*tert*-butoxycarbonyl)glycyl)-6-methylpiperazine-2-carboxylate (2.3 g, 70% yield) as a yellow oil. m/z ES+ $[M+H]^+$ 406.2; $^1H$ NMR (400 MHz, $CDCl_3$) δ ppm 7.37 - 7.30 (m, 5H), 5.55 (s, 1H), 5.17 - 5.09 (m, 1H), 4.26 - 4.16 (m, 1H), 4.07 - 3.97 (m, 1H), 3.95 - 3.82 (m, 1H), 3.79 - 3.71 (m, 3H), 3.68 - 3.46 (m, 3H), 2.75 - 2.58 (m, 1H), 2.31 - 2.17 (m, 2H), 1.47 (s, 9H), 1.36 (d, *J* = 6.4 Hz, 2H), 1.23 (d, *J* = 4.4 Hz, 1H).

**Step b.** To a solution of methyl (*rel*-2*R*,6*R*)-4-benzyl-1-((tert-butoxycarbonyl)glycyl)-6-methylpiperazine-2-carboxylate (2.0 g, 4.93 mmol) in MeOH (20 mL) was added 4 M HCl in MeOH (10 mL) and the reaction mixture was stirred at 25 °C for 16 h. Upon completion, the mixture was evaporated to give methyl (*rel*-2*R*,6*R*)-4-benzyl-1-glycyl-6-methylpiperazine-2-carboxylate hydrochloride (1.50 g, 88% yield) as a white solid.

m/z ES+ $[M+H]^+$ 306.0.

**Step c.** A mixture of methyl (*rel*-2*R*,6*R*)-4-benzyl-1-glycyl-6-methylpiperazine-2-carboxylate hydrochloride (1.50 g, 4.91 mmol) and TEA (2.49 g, 24.5 mmol) in MeOH (30 mL) was stirred at 70 °C for 2 h. Upon completion, the reaction mixture was evaporated and triturated with THF (5 mL) to give (*rel*-4*R*,9a*R*)-2-benzyl-4-methylhexahydro-2*H*-pyrazino[1,2-*a*]pyrazine-6,9-dione (1.20 g, 89% yield) as a yellow solid.

m/z ES+ $[M+H]^+$ 274.0; $^1H$ NMR (400 MHz, $CDCl_3$) δ ppm 7.31 - 7.20 (m, 5H), 6.02 (s, 1H), 4.12 - 3.95 (m, 1H), 3.88 (s, 2H), 3.69 - 3.47 (m, 3H), 3.38 - 3.26 (m, 1H), 2.84 - 2.81 (m, 1H), 2.26 - 2.25 (m, 1H), 2.13 -2.05 (m, 1H), 1.45 (d, *J* = 6.4 Hz, 3H).

**Step d.** To a solution of (*rel*-4*R*,9a*R*)-2-benzyl-4-methylhexahydro-2*H*-pyrazino[1,2-*a*]pyrazine-6,9-dione (400 mg, 1.46 mmol) in THF (1 mL) was added $LiAlH_4$ (555 mg, 14.6 mmol) and the reaction mixture was stirred at 0 °C for 1 h. Upon completion, the reaction mixture was slowly quenched with water (1 mL), 15% aq. NaOH solution (3 mL) and additional water (1 mL) at 0 °C and then $Na_2SO_4$ was added. The mixture was filtered and the filtrate was evaporated to give the title compound (330 mg, 91% yield) as yellow oil.

m/z ES+ $[M+H]^+$246.1; $^1H$ NMR (400 MHz, $CDCl_3$) δ ppm 7.36 - 7.30 (m, 5H), 3.76 - 3.69 (m, 1H), 3.53 - 3.45 (m, 2H), 3.16 - 3.09 (m, 1H), 3.08 - 3.00 (m, 1H), 2.96 - 2.87 (m, 1H), 2.82 - 2.75 (m, 2H), 2.70 - 2.62 (m, 1H), 2.60 - 2.51 (m, 1H), 2.39 - 2.32 (m, 1H), 2.30 - 2.24 (m, 1H), 2.03 - 1.94 (m, 2H), 1.91 - 1.85 (m, 1H), 1.04 (d, *J* = 6.4 Hz, 3H).

**Intermediate A24b: (*rel*-4*R*,9a*R*)-2-benzyl-4-methyloctahydro-2*H*-pyrazino[1,2-a]pyrazine**

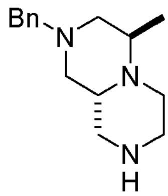

The title compound was prepared in a similar manner to **Intermediate A24a,** using **Intermediate A23b** in step a.

m/z ES+ $[M+H]^+$246.1; $^1H$ NMR $^1H$ NMR (400 MHz, $CDCl_3$) δ ppm 7.27 - 7.16 (m, 5H), 3.68 - 3.63 (m, 1H), 3.46 - 3.43 (m, 1H), 3.36 - 3.30 (m, 1H), 2.93 - 2.82 (m, 3H), 2.73 (dd, *J* = 11.6,

1.6 Hz, 1H), 2.67 - 2.46 (m, 6H), 2.42 - 2.35 (m, 1H), 2.29 (dd, *J* = 10.8, 3.2 Hz, 1H), 1.10 (d, *J* = 6.4 Hz, 3H).

**Intermediate A25: 1-(1-benzhydryl-3-methylazetidin-3-yl)piperazine**

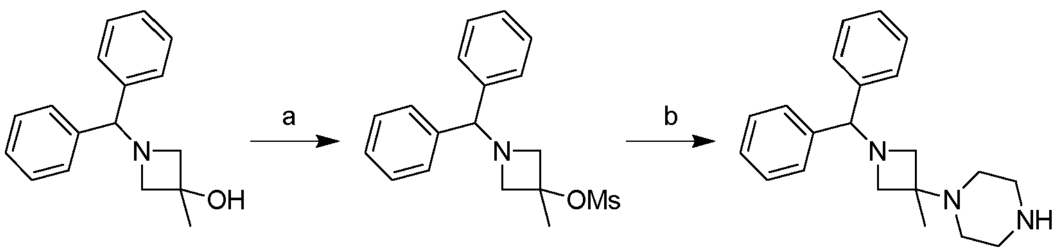


**Step a.** To a mixture of 1-benzhydryl-3-methyl-azetidin-3-ol (CAS: 40320-63-6; 500 mg, 1.97 mmol) and TEA (399 mg, 3.95 mmol) in DCM (10 mL) was added MsCl (339 mg, 2.96 mmol) at 0 °C. The reaction mixture was stirred at 20 °C for 1 h under a $N_2$ atmosphere. Upon completion, the mixture was diluted with water (50 mL) and extracted with EtOAc (3 x 20 mL). The combined organic layers were washed with brine (3 x 50 mL), dried over $Na_2SO_4$ and evaporated to give 1-benzhydryl-3-methylazetidin-3-yl methanesulfonate (650 mg, 99% yield) as a yellow solid.

$^1H$ NMR (400 MHz, $CDCl_3$) δ ppm 7.42 - 7.37 (m, 4H), 7.31 - 7.25 (m, 4H), 7.24 - 7.16 (m, 2H), 4.44 (s, 1H), 3.35 (s, 4H), 3.04 (s, 3H), 1.92 (s, 3H).

**Step b.** A mixture of 1-benzhydryl-3-methylazetidin-3-yl methanesulfonate (1.0 g, 3.02 mmol) and piperazine (2.60 g, 30.1 mmol) in 2-propanol (10 mL) was stirred at 70 °C for 16 h. Upon completion, the reaction mixture was evaporated and purified by reverse phase flash chromatography (water (0.1% $NH_4OH$)/MeCN) to give the title compound (950 mg, 97% yield) as a yellow solid.

m/z ES+ $[M+H]^+$322.2; $^1H$ NMR (400 MHz, $CDCl_3$) δ ppm 7.48 - 7.37 (m, 4H), 7.32 - 7.28 (m, 4H), 7.23 - 7.17 (m,

2H), 4.44 (s, 1H), 3.13 - 3.06 (m, 6H), 2.86 (d, *J* = 6.8 Hz, 2H), 2.58 - 2.51 (m, 4H), 1.38 (s, 3H).

**Intermediate A26: *tert*-butyl 6-oxo-2,5,8-triazaspiro[3.5]nonane-2-carboxylate**

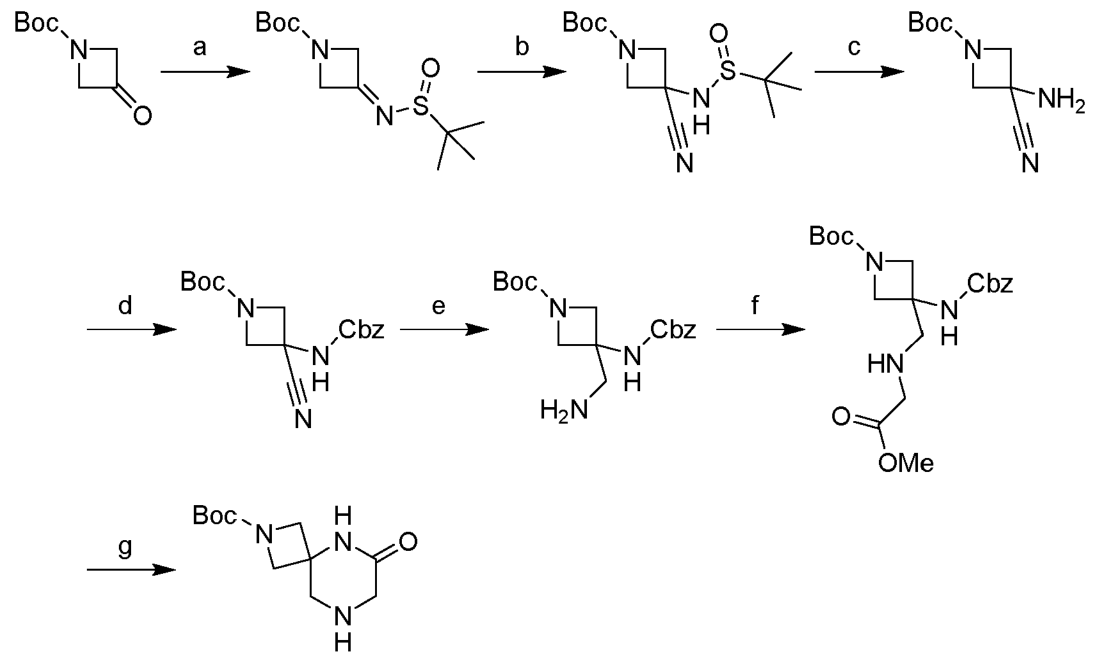


**Step a.** To a solution of *tert*-butyl 3-oxoazetidine-1-carboxylate (5.0 g, 29.2 mmol) in THF (50 mL) was added $Ti(OEt)_4$ (20 g, 87.6 mmol) and 2-methylpropane-2-sulfinamide (4.25 g, 35.1 mmol). The reaction mixture was stirred at 25 °C for 12 h. Upon completion, the mixture was diluted with EtOAc (300 mL) and quenched with water (150 mL) and filtered. The filtrate was extracted with EtOAc (3 x 150 mL) and the combined organic layers were washed with brine (150 mL), dried over $Na_2SO_4$, evaporated and purified by column chromatography (PE/EtOAc = 5/1) to give *tert*-butyl 3-((*tert*-butylsulfinyl) imino)azetidine-1-carboxylate (7.5 g, 93.6% yield) as a yellow solid.

m/z ES+ $[M+H]^+$275.2; $^1H$ NMR (400 MHz, $CDCl_3$) δ ppm 5.16 - 5.06 (m, 1H), 5.00 - 4.90 (m, 1H), 4.74 (t, *J*=2.4 Hz, 2H), 1.45 (s, 9H), 1.25 (s, 9H).

**Step b.** To a solution of *tert*-butyl 3-((*tert*-butylsulfinyl)imino)azetidine-1-carboxylate (7.1 g, 25.9 mmol) in DCM (100 mL) was added $Ti(OEt)_4$ (11.8 g, 51.8 mmol) and trimethylsilanecarbonitrile (6.42 g, 64.7 mmol). The reaction mixture was stirred at 25 °C for 12 h. Upon completion, the mixture was quenched with water (200 mL) and extracted with DCM (300 mL and 3 x 200 mL). The combined organic layers were washed with brine (3 x 100 mL), dried over $Na_2SO_4$, evaporated and purified by column chromatography (PE/EtOAc = 1/1) to give *tert*-butyl 3-((*tert*-butylsulfinyl)amino)-3-cyanoazetidine-1-carboxylate (6.6 g, 84% yield) as a yellow solid.

m/z ES+ $[M+Na]^+$324.2; $^1H$ NMR (400 MHz, $CDCl_3$) δ ppm 4.50 - 4.38 (m, 2H), 4.31 - 4.24 (m, 1H), 4.16 - 4.14 (m, 1H), 4.04 (s, 1H), 1.46 (s, 9H), 1.29 (s, 9H).

**Step** c. To a solution of *tert*-butyl 3-((*tert*-butylsulfinyl)amino)-3-cyanoazetidine-1-carboxylate (2.0 g, 6.64 mmol) in THF (25 mL) and water (5 mL) was added iodine (337 mg, 1.33 mmol) and the mixture was stirred at 50 °C for 12 h. Upon completion, the reaction mixture was quenched with sat. aq. $NaHCO_3$ (20 mL), diluted with water (30 mL) and extracted with EtOAc (3 x 50 mL). The combined organic layers were washed with brine (20 mL), dried over $Na_2SO_4$, evaporated and purified by column chromatography (PE/EtOAc = 1/1) to give *tert*-butyl 3-amino-3-cyanoazetidine-1-carboxylate (950 mg, 73% yield) as a yellow solid.

$^1H$ NMR (400 MHz, $CDCl_3$) δ ppm 4.35 (d, *J* =8.8 Hz, 2H), 3.89 (d, *J* =8.8 Hz, 2H), 2.03 (br s, 2H), 1.45 (s, 9H).

**Step d.** A mixture of *tert*-butyl 3-amino-3-cyanoazetidine-1-carboxylate (300 mg, 1.52 mmol), CbzCl (389 mg, 2.28 mmol), and $Na_2CO_3$ (484 mg, 4.56 mmol) in THF (6 mL) and water (2 mL) was stirred at 25 °C for 2 h. Upon completion, the reaction was quenched with sat. aq. $NH_4Cl$ (20 mL), diluted with water (20 mL) and extracted with EtOAc (3 x 30 mL). The combined organic layers were washed with brine (20 mL), dried over $Na_2SO_4$, evaporated and purified by column chromatography (PE/EtOAc = 5/1) to give *tert*-butyl *3*-(((benzyloxy)carbonyl)amino)-3-cyanoazetidine-1-carboxylate (450 mg, 89% yield) as a yellow solid.

m/z ES+ $[M+Na]^+$354.1; $^1H$ NMR (400 MHz, $CDCl_3$) δ ppm 7.38 - 7.30 (m, 5H), 5.66 - 5.46 (m, 1H), 5.18 (s, 2H),

4.43 - 4.41 (m, 2H), 4.11 - 4.00 (m, 2H), 1.45 (s, 9H).

**Step e.** To a solution of *tert*-butyl 3-(((benzyloxy)carbonyl)amino)-3-cyanoazetidine-1-carboxylate (300 mg, 0.91 mmol) in MeOH (30 mL) was added Raney-Ni (78 mg, 0.91 mmol) The suspension was degassed and purged with $H_2$ 3 times and the reaction mixture was stirred at 25 °C for 12 h under a $H_2$ atmosphere (15 psi). Upon completion the reaction mixture was filtered and evaporated to give *tert*-butyl 3-(aminomethyl)-3-(((benzyloxy)carbonyl)amino)azetidine-1-carboxylate (300 mg, crude) as a yellow solid which was used in the next step directly.

m/z ES+ $[M+H]^+$ 336.2.

**Step f.** To a solution of *tert*-butyl 3-(aminomethyl)-3-(((benzyloxy)carbonyl)amino)azetidine-1-carboxylate (300 mg, 0.90 mmol) in THF (5 mL) was added TEA (272 mg, 2.68 mmol) and methyl 2-bromoacetate (274 mg, 1.79 mmol). The reaction mixture was stirred at 25 °C for 12 h. Upon completion, the mixture was quenched with sat. aq. $NH_4Cl$ (20 mL), diluted with water (20 mL) and extracted with EtOAc (3 x 30 mL). The combined organic layers were washed with brine (20 mL), dried over $Na_2SO_4$, evaporated and purified by column chromatography (PE/EtOAc = 1/2) to give *tert*-butyl 3-(((benzyloxy)carbonyl)amino)-3-(((2-methoxy-2-oxoethyl)amino)methyl)azetidine-1-carboxylate (300 mg, 82% yield) as a yellow solid.

m/z ES+ $[M+H]^+$ 408.2; $^1$H NMR (400 MHz, $CDCl_3$) δ ppm 7.41 - 7.29 (m, 5H), 5.81 (br s, 1H), 5.12 (s, 2H), 4.19 - 4.10 (m, 2H), 3.82 (d, *J* =8.8 Hz, 2H), 3.75 (s, 3H), 3.48 (s, 2H), 3.08 (s, 2H), 1.44 (s, 9H).

**Step g.** To a solution of *tert*-butyl 3-(((benzyloxy)carbonyl)amino)-3-(((2-methoxy-2-oxoethyl)amino)methyl) azetidine-1-carboxylate (1.3 g, 3.19 mmol) in MeOH (50 mL) was added 10% Pd/C (300 mg) and the mixture was stirred at 40 °C for 12 h under a $H_2$ atmosphere (15 psi). Upon completion, the reaction mixture was filtered and evaporated to give the title compound (670 mg, crude) as a white solid.

$^1$H NMR (400 MHz, DMSO-$d_6$) δ ppm 8.27 (s, 1H), 3.81 - 3.69 (m, 4H), 3.11 (s, 2H), 2.90 (s, 2H), 2.75 (br s, 1 H), 1.38 (s, 9H).

**Intermediate A27: *tert*-butyl 5-methyl-6-oxo-2,5,8-triazaspiro[3.5]nonane-2-carboxylate**

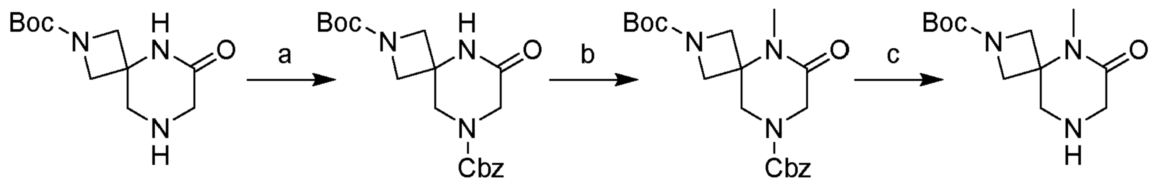


**Step a.** This step was conducted in a similar manner to **Intermediate A26,** step d, using **Intermediate A26.**

**Step b.** To a solution of 8-benzyl 2-(*tert*-butyl) 6-oxo-2,5,8-triazaspiro[3.5]nonane-2,8-dicarboxylate (0.25 g, 0.67 mmol) in DMF (5 mL) was added $Cs_2CO_3$ (651 mg, 2.0 mmol) and MeI (284 mg, 2.00 mmol). The reaction mixture was stirred at 25 °C for 12 h. Upon completion, the reaction mixture was quenched with sat. aq. $NH_4Cl$ (20 mL), diluted with water (30 mL) and extracted with EtOAc (3 x 30 mL). The combined organic layers were washed with brine (20 mL), dried over $Na_2SO_4$, evaporated and purified by column chromatography (PE/EtOAc = 1/2) to give 8-benzyl 2-(*tert*-butyl) 5-methyl-6-oxo-2,5,8-triazaspiro[3.5]nonane-2,8-dicarboxylate (220 mg, 84% yield) as a colourless oil.

m/z ES+ $[M+H]^+$390.1; $^1$H NMR (400 MHz, DMSO-$d_6$) δ ppm 7.44 - 7.28 (m, 5H), 5.12 (s, 2H), 4.08 (d, *J* = 9.2 Hz, 2H), 4.02 (s, 2H), 3.87 (s, 2H), 3.77 (d, *J* = 9.6 Hz, 2H), 2.96 (s, 3H), 1.38 (s, 9H).

**Step c.** This step was conducted in a similar manner to **Intermediate A26,** step g.

m/z ES+ $[M+H]^+$256.1.

**Intermediate A28: *tert*-butyl 7',8'-dihydro-6'*H*-spiro[azetidine-3,5'-imidazo[1,2-*a*]pyrazine]-1-carboxylate**

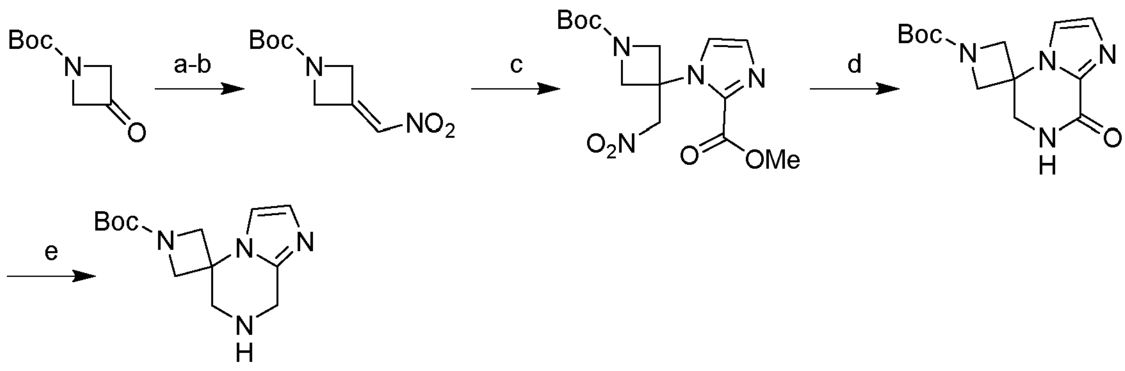


**Steps a-b.** These 2 steps were conducted as described in **Intermediate A12,** steps a-b. $^1$H NMR (400 MHz, $CDCl_3$) δ ppm 7.05 (s, 1H), 5.03 (d, *J* = 3.2 Hz, 2H), 4.71 (s, 2H), 1.48 (s, 9H).

**Step c.** To a solution of methyl 1*H*-imidazole-2-carboxylate (2.59 g, 20.5 mmol) in toluene (40 mL) was added NaH (971 mg, 24.3 mmol, 60% dispersion in mineral oil). The mixture was stirred at 0 °C for 30 min, after which *tert*-butyl 3-(nitromethylene)azetidine-1-carboxylate (4 g, 18.7 mmol) in toluene (40 mL) was slowly added. The mixture was stirred at 0 °C for a further 30 min. Upon completion, the reaction mixture was quenched with sat. aq. $NH_4Cl$ (100 mL) at 25 °C, diluted with water (100 mL) and extracted with EtOAc (3 x 150 mL). The combined organic layers were washed with brine (50 mL), dried over $Na_2SO_4$, evaporated and purified by column chromatography (PE/EtOAc = 1/2) to give methyl 1-(1-(*tert*-butoxycarbonyl)-3-(nitromethyl)azetidin-3-yl)-1*H*-imidazole-2-carboxylate (0.72 g, 2.12 mmol, 11% yield) as a yellow solid.

m/z ES+ $[M+H]^+$341.1; $^1$H NMR (400 MHz, $CDCl_3$) δ ppm 7.23 (s, 2H), 5.25 (s, 2H), 4.56 - 4.30 (m, 4H), 3.97 (s, 3H), 1.46 (s, 9H).

**Step d.** This step was conducted in a similar manner to **Intermediate A10,** step c.

**Step e.** To a solution of *tert*-butyl 8'-oxo-7',8'-dihydro-6'*H*-spiro[azetidine-3,5'-imidazo[1,2-a]pyrazine]-1-carboxylate (200 mg, 0.72 mmol mmol) in THF (3 mL) was added a solution of borane dimethyl sulfide complex (10 M in THF, 0.36 mL). The reaction mixture was stirred at 25 °C for 12 h. Upon completion, the reaction mixture was quenched with MeOH (2 mL) at 0 °C, evaporated and purified by reverse phase flash chromatography (water (0.1% FA)/MeCN) to give the title compound (90 mg, 47% yield) as a yellow solid.

m/z ES+ $[M+H]^+$265.1.

**Intermediate A29: *tert*-butyl 1',2'-dihydrospiro[azetidine-3,3'-imidazo[1,2-*a*]imidazole]-1-carboxylate**

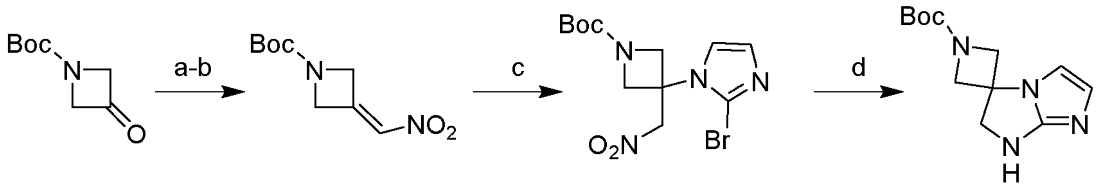


**Steps a-b.** These 2 steps were conducted as described in **Intermediate A12,** steps a-b.

**Step c.** This step was conducted in a similar manner to **Intermediate A28,** step c, using 2-bromo-1*H*-imidazole (CAS: 16681-56-4).

**Step d.** To a solution of *tert*-butyl 3-(2-bromo-1*H*-imidazol-1-yl)-3-(nitromethyl)azetidine-1-carboxylate (700 mg, 1.94 mmol) in MeOH (20 mL) was added cobalt (II) chloride (1.51 g, 11.6 mmol) and $NaBH_4$ (440 mg, 11.6 mmol). The reaction mixture was stirred at 0 °C for 1 h. Upon completion, the mixture was quenched with sat. aq. $NH_4Cl$ (1 mL), filtered and the filtrate was evaporated. The residue was purified by reverse phase flash chromatography (water (0.1% FA)/MeCN) to give the title compound (300 mg, 61.9% yield) as a white solid.

m/z ES+ $[M+H]^+$ 250.9.

**Intermediate A30: *tert*-butyl 3-(2-oxopiperazin-1-yl)azetidine-1-carboxylate**

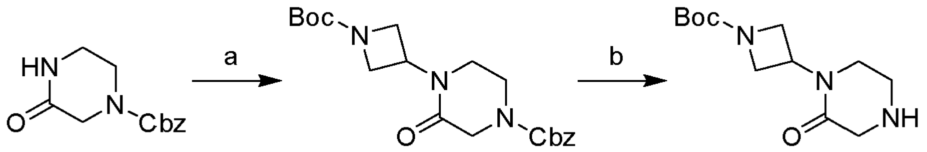


**Step a.** To a suspension of NaH (128 mg, 3.20 mmol, 60% dispersion in mineral oil) in DMF (5 mL) was added benzyl 3-oxopiperazine-1-carboxylate (CAS: 78818-15-2; 0.5 g, 2.13 mmol) at 0 °C. The mixture was stirred at 0 °C for 30 min under a $N_2$ atmosphere, after which *tert*-butyl 3-iodoazetidine-1-carboxylate (CAS: 254454-54-1; 724 mg, 2.56 mmol) was added. The reaction mixture was stirred at 20 °C for a further 1 h. Upon completion, the reaction mixture was partitioned between water (40 mL) and EtOAc (40 mL). The aqueous layer was further extracted with EtOAc (2 x 40 mL) and the combined organic layers were dried over $Na_2SO_4$ and evaporated. The residue was purified by reverse phase flash chromatography (water (0.1% FA)/MeCN) to give benzyl 4-(1-(*tert*-butoxycarbonyl)azetidin-3-yl)-3-oxopiperazine-1-carboxylate (60 mg, 7% yield) as a yellow oil.

m/z ES+ $[M+H]^+$ 390.1

**Step b.** To a solution of benzyl 4-(1-(*tert*-butoxycarbonyl)azetidin-3-yl)-3-oxopiperazine-1-carboxylate (60 mg, 0.15 mmol) in 2-propanol (2 mL) was added 10% Pd/C (5 mg). The reaction mixture was stirred at 40 °C for 12 h under a $H_2$ (15 psi) atmosphere. Upon completion, the reaction mixture was filtered and the filtrate was evaporated to give the title compound (44 mg, crude) as a yellow oil which was used without further purification.

**Intermediate A31: *tert*-butyl (*rel*-3*R*,4*R*)-4-hydroxy-3-(methylamino)piperidine-1-carboxylate**

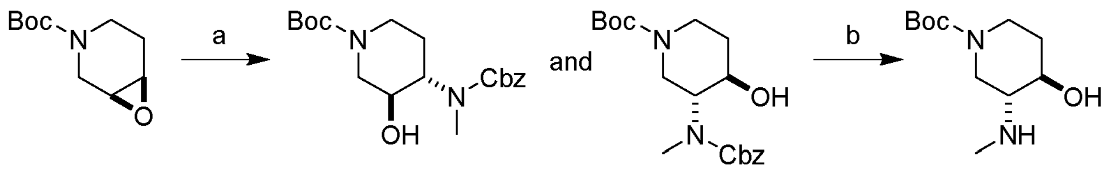


**Step a.** A solution of *tert*-butyl 7-oxa-3-azabicyclo[4.1.0]heptane-3-carboxylate (CAS: 161157-50-2; 1.0 g, 5.02 mmol) in methanamine (2M in THF, 50.2 mL) was stirred at 100 °C for 12 h. Upon completion, the reaction mixture was filtered and evaporated to give a yellow oil (1.15 g, crude). The oil was dissolved in THF (12 mL) and water (6 mL) and CbzCl (1.22 g, 7.16 mmol) and $Na_2CO_3$ (1.52 g, 14.3 mmol) were added. The reaction mixture was stirred at 25 °C for 1 h. Upon completion, the mixture was diluted with water (30 mL) and extracted with EtOAc (3 x 50 mL). The combined organic layers were washed with brine (3 x 20 mL), dried over $Na_2SO_4$ and evaporated. The residue was purified by column chromatography (PE/EtOAc = 1/1) to give *tert*-butyl (rel-3*R*,4*R*)-3-(((benzyloxy)carbonyl)(methyl)amino)-4-hydroxypiperidine-1-carboxylate (200 mg, 11% yield) as a colourless oil. The regiochemistry was confirmed by 2D NMR.

m/z ES+ $[M+H]^+$365.2; $^1$H NMR (400 MHz, $CDCl_3$) δ ppm 7.44 - 7.29 (m, 5H), 5.22 - 5.10 (m, 2H), 4.23 - 3.98 (m, 2H), 3.82 - 3.74 (m, 2H), 2.93 (s, 3H), 2.89 - 2.74 (m, 1H), 2.70 - 2.64 (m, 1H), 2.05 - 1.99 (m, 1H), 1.59 - 1.50 (m, 1H), 1.46 (s, 9H).

**Step b.** To a solution of *tert*-butyl (rel-3*R*,4*R*)-3-(((benzyloxy)carbonyl)(methyl)amino)-4-hydroxypiperidine-1-carboxylate (200 mg, 0.55 mmol) in THF (5 mL) was added 10% Pd/C (30 mg). The mixture was stirred at 25 °C for 30 min under a $H_2$ atmosphere (15 psi). Upon completion, the reaction mixture was filtered and the filtrate was evaporated to give the title compound (110 mg, crude) as a colourless oil which was used without further purification.

**Intermediate A32: *tert*-butyl 2,5,8-triazaspiro[3.5]nonane-2-carboxylate**

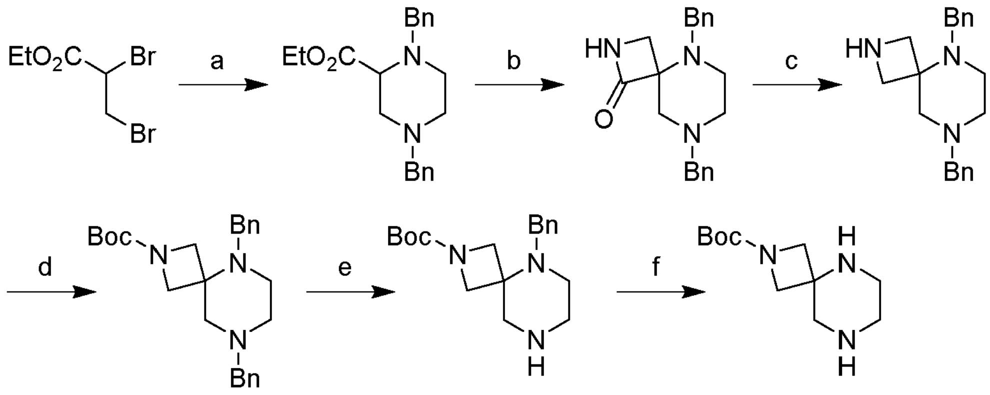


**Step a.** Ethyl 2,3-dibromopropanoate (CAS: 3674-13-3; 21.6 g, 83.2 mmol) was added dropwise to a solution of *N1*,*N2*-dibenzylethane-1,2-diamine (CAS: 140-28-3; 20 g, 83.2 mmol) and triethylamine (26 mL, 164 mmol) in toluene (300 mL) at 40 °C. The reaction mixture was stirred at 80 °C for 4 h. Upon completion, the reaction mixture was cooled to rt and the solvent was evaporated under vacuum. The reaction mixture was diluted with EtOAc (300 mL) and washed with water (2 x 100 mL). The organic layer was further washed with brine, dried over dried over $Na_2SO_4$ and evaporated. Purification by column chromatography (n-hexane/EtOAc = 4/1) gave ethyl 1,4-dibenzylpiperazine-2-carboxylate (20 g, 73% yield) as a pale yellow oil. m/z ES+ $[M+H]^+$339.7.

**Step b.** To a solution of ethyl 1,4-dibenzylpiperazine-2-carboxylate (10 g, 29.6 mmol) in THF (200 mL), cooled to -78 °C, was added paraformaldehyde (0.89 g) followed by a solution of LiHMDS (1 M in THF, 88.8 mL, 88.8 mmol) under a $N_2$ atmosphere. The reaction mixture was stirred at -78 °C for 15 min and then stirred at rt 2 h. Upon completion, the reaction mixture was quenched with water (200 mL) and the aqueous mixture was extracted with EtOAc (2 x 500 mL). The organic layers were combined, dried over dried over $Na_2SO_4$ and evaporated. Purification by column chromatography (n-hexane/EtOAc = 3/2) gave 5,8-dibenzyl-2,5,8-triazaspiro[3.5]nonan-1-one (5.6 g, 60% yield) as a white solid.

m/z ES+ $[M+H]^+$ 322.2.

**Step c.** To a solution of 5,8-dibenzyl-2,5,8-triazaspiro[3.5]nonan-1-one (5.6 g, 17.4 mmol) in THF (60 mL), cooled to 0 °C, was added a solution of $LiAlH_4$ (2.5 M in THF, 20.9 mL, 52.3 mmol). The reaction mixture was stirred at 60 °C for 15 h. Upon completion, the reaction mixture was cooled to 0 °C and quenched with the slow addition of sat. aq. $NH_4Cl$. The reaction mixture was extracted with EtOAc (2 x 100 mL). The organic layers were combined, dried over dried over $Na_2SO_4$ and evaporated to give 5,8-dibenzyl-2,5,8-triazaspiro[3.5]nonane (5.2 g, 97% crude) as a yellow solid.

m/z ES+ $[M+H]^+$ 308.2.

**Step d.** To a solution of 5,8-dibenzyl-2,5,8-triazaspiro[3.5]nonane (5.2 g, 16.9 mmol) in methanol (52 mL) were added triethylamine (4.7 mL, 33.8 mmol), DMAP (0.12 g, 1.0 mmol) and di-*tert*-butyl decarbonate (4.6 g, 21.1 mmol). The reaction mixture was stirred at rt for 3 h. Upon completion, the reaction mixture was quenched with water (50 mL) and extracted with EtOAc (2 x 100 mL). The organic layers were combined, dried over $Na_2SO_4$ and evaporated. Purification by column chromatograpy (*n*-hexane/EtOAc = 4/1) gave *tert*-butyl 5,8-dibenzyl-2,5,8-triazaspiro[3.5]nonane-2-carboxylate (5.1 g, 74% yield) as a yellow solid.

m/z ES+ $[M+H]^+$408.3.

**Step e.** To a solution of *tert*-butyl 5,8-dibenzyl-2,5,8-triazaspiro[3.5]nonane-2-carboxylate (5.0 g, 12.3 mmol) in 1,2-dimethoxyethane (50 mL), cooled to 0 °C, was added 1-chloroethyl chloroformate (0.69 g, 18.4 mmol). The reaction mixture was stirred at 80 °C for 2 h. Upon completion, the reaction mixture was poured into cold water (50 mL) and extracted with DCM (2 x 100 mL). The organic layers were combined, washed with cold water and brine, dried over $Na_2SO_4$ and evaporated. The crude was triturated with diethyl ether to give *tert*-butyl 5-benzyl-2,5,8-triazaspiro[3.5]nonane-2-carboxylate (1.5 g, 39% yield) as white solid.

m/z ES+ $[M+H]^+$318.7.

**Step f.** To a solution of *tert*-butyl 5-benzyl-2,5,8-triazaspiro[3.5]nonane-2-carboxylate (2.5 g, 7.88 mmol) in MeOH (25 mL) was added 10% Pd/C (250 mg) under a $N_2$ atmosphere. The suspension was degassed and purged with $H_2$ 3 times. The mixture was stirred at 25 °C for 4 h under a $H_2$ atmosphere (15 psi). Upon completion, the reaction mixture was filtered and the filtrate was evaporated to give the title compound (1.2 g, crude) as yellow solid.

m/z ES+ $[M+H]^+$ 228.2; $^1H$ NMR (400 MHz, $CDCl_3$) δ ppm 4.11 - 4.05 (m, 2H), 3.82 - 3.75 (m, 2H), 3.29 (s, 2H), 3.23 - 3.15 (m, 4H), 1.44 (s, 9H).

**Intermediate A33: *tert*-butyl 8-cyano-2,6-diazaspiro[3.4]octane-2-carboxylate**

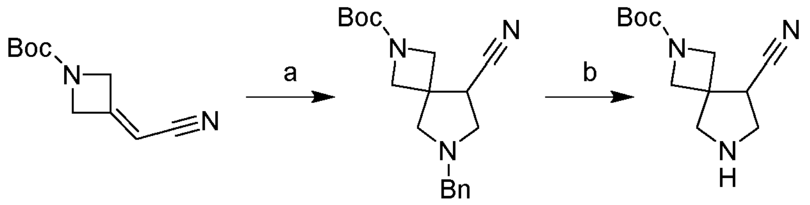


**Step a**. *N*-benzyl-1-methoxy-*N*-((trimethylsilyl)methyl)methanamine (CAS: 93102-05-7; 4.40 g, 18.5 mmol) and LiF (1.20 g, 46.3 mmol) were added to a solution of *tert*-butyl 3-(cyanomethylene)azetidine-1-carboxylate (CAS: 1153949-11-1; 3 g, 15.4 mmol) in MeCN (30 mL). The mixture was stirred at 60 °C for 72 h. Upon completion, the mixture was filtered and the filtrate was evaporated. The residue was purified by column chromatography (PE/EtOAc = 3/1) to give *tert*-butyl 6-benzyl-8-cyano-2,6-diazaspiro[3.4]octane-2-carboxylate (3.7 g, 73% yield) as a colourless oil.

m/z ES+ $[M+H]^+$ 328.1.

**Step b**. A mixture of *tert*-butyl 6-benzyl-8-cyano-2,6-diazaspiro[3.4]octane-2-carboxylate (3.2 g, 9.77 mmol), 10% Pd/C (1.0 g) and ammonium formate (3.08 g, 48.8 mmol) in EtOH (30 mL) was stirred at 80 °C for 12 h. Upon completion, the mixture was filtered and evaporated to give the title compound (2 g, crude) as a light yellow oil.

m/z ES+ $[M+H]^+$ 238.2.

**Intermediate A34: *tert*-butyl 8-cyano-8-methyl-2,6-diazaspiro[3.4]octane-2-carboxylate**

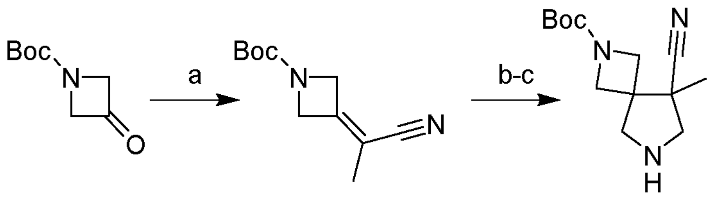


**Step a.** To a solution of diethyl (1-cyanoethyl)phosphonate (1.0 g, 5.23 mmol) in THF (50 mL) was added NaH (313 mg, 7.85 mmol, 60% dispersion in mineral oil) at 0 °C. The mixture was stirred for 30 min, after which *tert*-butyl 3-oxoazetidine-1-carboxylate (1.79 g, 10.46 mmol) was added and the reaction mixture was slowly warmed to 15 °C and stirred for 12 h. Upon completion, the reaction was quenched with water (50 mL) and extracted with EtOAc (2 x 50 mL). The combined organic layers were evaporated and the residue was purified by column chromatography (PE/EtOAc = 5/1) to give *tert*-butyl 3-(1-cyanoethylidene)azetidine-1-carboxylate (600 mg, 55 % yield) as a colourless oil.

$^1H$ NMR (400 MHz, $CDCl_3$) δ ppm 4.60 - 4.53 (m, 2H), 4.45 - 4.46 (m, 2H), 1.74 (t, *J* = 1.6 Hz, 3H), 1.39 (s, 9H).

**Steps b-c.** These 2 steps were conducted in a similar manner to **Intermediate A33,** steps a-b.

m/z ES+ $[M+H]^+$ 252.2; $^1H$ NMR (400 MHz, $CD_3OD$) δ ppm 4.06 - 3.93 (m, 2H), 3.81 (d, *J* = 9.2 Hz, 1H), 3.68 (d, *J* = 9.2 Hz, 1H), 3.26 - 3.17 (m, 2H), 3.16 - 3.08 (m, 1H), 2.86 (d, *J* = 11.6 Hz, 1H), 1.40 - 1.35 (m, 12H).

**Intermediate B1: *tert*-butyl 3-(4-formyl-1*H*-imidazol-1-yl)azetidine-1-carboxylate**

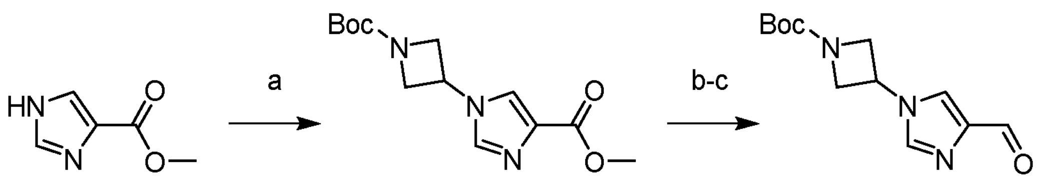


**Step a.** A mixture of *tert*-butyl 3-iodoazetidine-1-carboxylate (CAS: 254454-54-1; 10.0 g, 35.3 mmol), methyl 1H-imidazole-4-carboxylate (CAS: 17325-26-7; 8.91 g, 70.6 mmol) and $Cs_2CO_3$ (34.5 g, 106 mmol) in DMF (100 mL) was stirred at 100 °C for 1 h. Upon completion, the reaction mixture was diluted with water (300 mL) and extracted with EtOAc (300 mL). The organic layer was washed with brine (300 mL), dried over $Na_2SO_4$ and evaporated. The residue was purified by reverse phase flash chromatography (water (0.1% $NH_4OH$)/MeCN) to give methyl 1-(1-(*tert*-butoxycarbonyl)azetidin-3-yl)-1*H*-imidazole-4-carboxylate (5.5 g, 55% yield) as a white solid. The regiochemistry was confirmed by 2D NMR.

m/z ES+ $[M+H]^+$ 282.2; $^1H$ NMR (400 MHz, $CDCl_3$) δ ppm 7.85 (d, *J* = 1.6 Hz, 1H), 7.62 (d, *J* = 1.6 Hz, 1H), 4.96 -

4.89 (m, 1H), 4.50 - 4.42 (m, 2H), 4.10 - 4.06 (m, 2H), 3.88 (s, 3H), 1.45 (s, 9H).

**Step b.** To a solution of methyl 1-(1-(tert-butoxycarbonyl)azetidin-3-yl)-1*H*-imidazole-4-carboxylate (2.00 g, 7.11 mmol) in THF (20 mL) was added $LiBH_4$ (465 mg, 21.3 mmol) at 0 °C. The reaction mixture was stirred at 0 °C for 1 h, and then MeOH (2 mL) was added. The reaction mixture was stirred at 25 °C for a further 12 h. Upon completion, the reaction mixture was poured into sat. aq. $NH_4Cl$ (50 mL) and extracted with EtOAc (50 mL). The organic layer was washed with brine (50 mL), dried over $Na_2SO_4$ and evaporated. The residue was purified by column chromatography (DCM/MeOH = 10/1) to give *tert*-butyl 3-(4-(hydroxymethyl)-1*H*-imidazol-1-yl)azetidine-1-carboxylate (0.65 g, 36% yield) as a white solid.

$^1H$ NMR (400 MHz, $CDCl_3$) δ ppm 7.56 (d, *J* = 1.2 Hz, 1H), 7.12 (s, 1H), 4.92 - 4.81 (m, 1H), 4.61 (s, 2H), 4.46 - 4.37 (m, 2H), 4.14 - 4.04 (m, 2H), 1.46 (s, 9H).

**Step c.** To a solution of *tert-butyl* 3-(4-(hydroxymethyl)-1*H*-imidazol-1-yl)azetidine-1-carboxylate (50 mg, 0.20 mmol) in DCM (1 mL) was added $MnO_2$ (52 mg, 0.59 mmol). The reaction mixture was stirred at 25 °C for 2 h. Upon completion, the reaction mixture was filtered and evaporated to give the title compound (40 mg, 81% yield) as a brown oil.

$^1H$ NMR (400 MHz, $CDCl_3$) δ ppm 9.91 (s, 1H), 7.88 (s, 1H), 7.72 (s, 1H), 5.05 - 4.86 (m, 1H), 4.60 - 4.40 (m, 2H), 4.20 - 4.04 (m, 2H), 1.49 (s, 9H).

**Intermediate B2: *tert*-butyl 3-(3-formyl-1H-pyrazol-1-yl)azetidine-1-carboxylate**

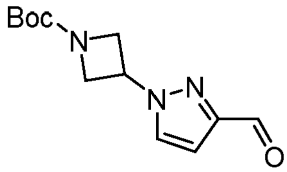

The title compound was prepared in a similar manner to **Intermediate B1,** using methyl 1*H*-pyrazole-3-carboxylate (CAS: 15366-34-4) in step a.

m/z ES+ $[M+Na]^+$ 274.1.

**Intermediate B3: *tert*-butyl 3-(3-formyl-1*H*-1,2,4-triazol-1-yl)azetidine-1-carboxylate**

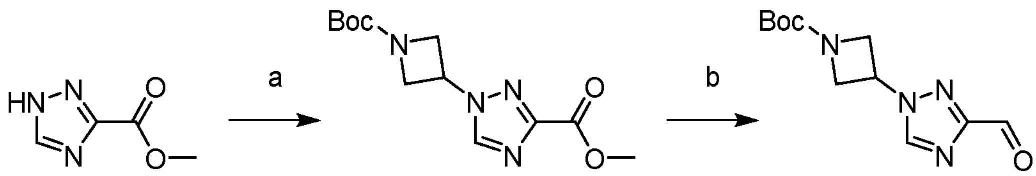


**Step a.** This step was conducted in a similar manner to **Intermediate B1,** step a, using methyl 1H-1,2,4-triazole-3-carboxylate (CAS: 4928-88-5).

Step b. To a solution of methyl 1-(1-(*tert*-butoxycarbonyl)azetidin-3-yl)-1*H*-1,2,4-triazole-3-carboxylate (250 mg, 0.89 mmol) in DCM (10 mL) was added DIBAL-H (1 M in DCM, 2.66 mL). The reaction mixture was stirred at -70 °C for 1 h. Upon completion, the mixture was quenched with MeOH (0.5 mL) and stirred at 20 °C for 20 min. The mixture was filtered and evaporated to give the title compound (170 mg, 76% yield) as a white solid.

$^1H$ NMR (400 MHz, $CDCl_3$) δ ppm 10.08 (s, 1H), 8.32 (s, 1H), 5.28 - 5.17 (m, 1H), 4.61 - 4.20 (m, 4H), 1.51 (s, 9H).

**Intermediate B4: *tert*-butyl 3-(3-oxopropoxy)azetidine-1-carboxylate**

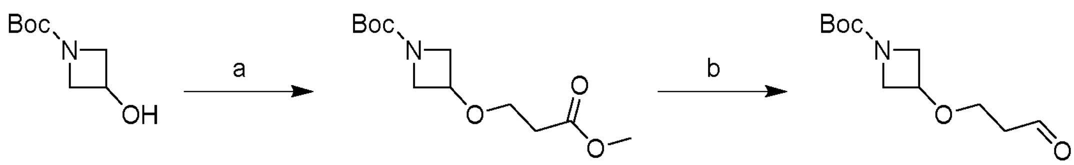

**Step a**. To a solution of *tert*-butyl 3-hydroxyazetidine-1-carboxylate (CAS: 141699-55-0; 10 g, 57.7 mmol) in THF (100 mL) was added NaH (346 mg, 8.66 mmol, 60% dispersion in mineral oil). The mixture was stirred at 20 °C for 30 min. Then methyl acrylate (CAS: 96-33-3; 24.9 g, 289 mmol) was added and the reaction mixture was stirred at 20 °C for 12 h. Upon completion, the mixture was quenched with sat. aq. $NH_4Cl$ (100 mL) and extracted with EtOAc (2 x 100 mL). The combined organic layers were washed with brine (200 mL), dried over $Na_2SO_4$, and evaporated. The crude product was purified by column chromatography (PE/EtOAc = 5/1) to give *tert*-butyl 3-(3-methoxy-3-oxopropoxy)azetidine-1-carboxylate (6 g, 40% yield) as a colourless oil.

$^1H$ NMR (400 MHz, $CDCl_3$) δ ppm 4.27 - 4.19 (m, 1H), 4.09 - 4.01 (m, 2H), 3.82 (dd, *J* = 4.4, 9.6 Hz, 2H), 3.71 (s, 3H), 3.64 (t, *J* = 6.4 Hz, 2H), 2.59 (t, *J* = 6.4 Hz, 2H), 1.43 (s, 9H).

**Step b.** To a solution of *tert*-butyl 3-(3-methoxy-3-oxopropoxy)azetidine-1-carboxylate (2 g, 7.71 mmol) in DCM (50 mL) was added DIBAL-H (1 M in THF, 23.1 mL) dropwise at -70 °C. Then the reaction mixture was stirred at -70 °C for 30 min. Upon completion, the mixture was quenched with MeOH (5 mL) at -70 °C, and allowed to warm to 20 °C over 30 min. The mixture was filtered and evaporated to give the title compound (1.4 g, 79% yield) as a colourless oil, which was used for the next step directly without further purification.

$^1H$ NMR (400 MHz, $CDCl_3$) δ ppm 9.80 (t, *J* = 1.6 Hz, 1H), 4.26 - 4.20 (m, 1H), 4.12 - 4.04 (m, 2H), 3.84 - 3.78 (m, 2H), 3.74 - 3.66 (m, 2H), 2.76 - 2.64 (m, 2H), 1.44 (s, 9H).

**Intermediate B5: tert-butyl 3-(4-formyl-1H-pyrazol-1-yl)azetidine-1-carboxylate**

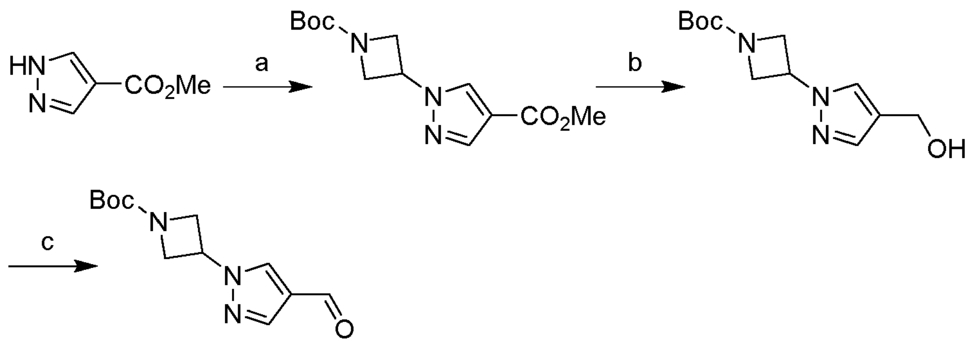


**Step** a. This step was conducted in a similar manner to **Intermediate B1,** step a, using methyl 1H-pyrazole-4-carboxylate (CAS: 51105-90-9).

**Step b.** This step was conducted in a similar manner to **Intermediate B3,** step b.

**Step c.** This step was conducted in a similar manner to **Intermediate B1,** step c.

$^1H$ NMR (400 MHz, $CDCl_3$) δ ppm 9.91 (s, 1H), 8.08 (s, 1H), 8.07 (s, 1H), 5.13 - 5.07 (m, 1H), 4.47 - 4.42 (m, 2H), 4.37 - 4.32 (m, 2H), 1.49 (s, 9H).

**Intermediate B6: *tert*-butyl 3-(4-formyl-1-((2-(trimethylsilyl)ethoxy)methyl)-1*H*-imidazol-2-yl)azetidine-1-carboxylate**

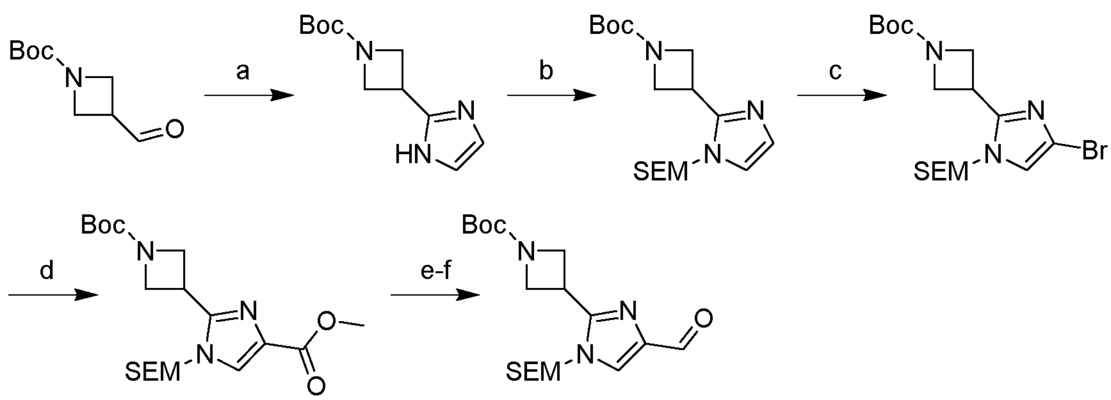

**Step a.** To a solution of *tert*-butyl 3-formylazetidine-1-carboxylate (4.8 g, 25.9 mmol) and glyoxal (40 wt% in water; 18.0 g, 129 mmol) in MeOH (50 mL) was added 30% ammonium hydroxide (18.1 g, 155 mmol). The reaction mixture was stirred at 20 °C for 12 h. Upon completion, the mixture was evaporated and the residue was purified by reverse phase flash chromatography (water (0.1% $NH_4OH$)/MeCN) to give *tert*-butyl 3-(1*H*-imidazol-2-yl)azetidine-1-carboxylate (5.79 g, crude) as a brown oil.

m/z ES+ $[M+H]^+$ 224.2.

**Step b**. To a solution of *tert*-butyl 3-(1*H*-imidazol-2-yl)azetidine-1-carboxylate (5.79 g, 25.9 mmol) in THF (3 mL) was added NaH (1.24 g, 31.1 mmol, 60% dispersion in mineral oil). The mixture was stirred at 0 °C for 30 min, after which SEMCl (5.62 g, 33.7 mmol) was added and the reaction mixture was stirred for a further 1 h. Upon completion, the mixture was quenched with sat. aq. $NH_4Cl$ (5 mL), diluted with water (100 mL) and extracted with EtOAc (3 x 100 mL). The combined organic layers were evaporated and the residue was purified by reverse phase flash chromatography (water (0.1% $NH_4OH$)/MeCN) to give *tert*-butyl 3-(1-((2-(trimethylsilyl)ethoxy)methyl)-1*H*-imidazol-2-yl)azetidine-1-carboxylate (6.0 g, 65 % yield) as a brown oil.

m/z ES+ $[M+H]^+$ 354.0.

**Step c.** A mixture of *tert*-butyl 3-(1-((2-(trimethylsilyl)ethoxy)methyl)-1*H*-imidazol-2-yl)azetidine-1-carboxylate (3.1 g, 8.77 mmol), NBS (1.56 g, 8.77 mmol) in DCM (30 mL) and DMF (30 mL) was stirred at 25 °C for 12 h under a $N_2$ atmosphere. Upon completion, the mixture was filtered and the filtrate was evaporated. The residue was purified by reverse phase flash chromatography (water (0.1% $NH_4OH$)/MeCN) to give *tert*-butyl 3-(4-bromo-1-((2-(trimethylsilyl) ethoxy)methyl)-1*H*-imidazol-2-yl)azetidine-1-carboxylate (3.2 g, 84% yield) as a yellow oil.

m/z ES+ $[M+H]^+$ 434.2; $^1H$ NMR (400 MHz, $CDCl_3$) $\delta$ ppm 7.03 - 6.93 (m, 1H), 5.21 - 5.10 (m, 2H), 4.29 - 4.17 (m, 4H), 3.99 - 3.85 (m, 1H), 3.55 - 3.42 (m, 2H), 1.45 (s, 9H), 0.89 (t, *J* = 8.4 Hz, 2H), 0.00 (s, 9H).

**Step d.** A mixture of *tert*-butyl 3-(4-bromo-1-((2-(trimethylsilyl)ethoxy)methyl)-1*H*-imidazol-2-yl)azetidine-1-carboxylate (3.2 g, 7.40 mmol), Pd(dppf)$Cl_2$ (270 mg, 0.37 mmol), TEA (793 mg, 7.84 mmol) in MeOH (30 mL) was stirred at 60 °C for 12 h under a CO atmosphere (50 psi). Upon completion, the mixture was filtered and the filtrate was evaporated. The residue was purified by by reverse phase flash chromatography (water (0.1% $NH_4OH$)/MeCN) to give the title compound (3.0 g, 69% yield) as a yellow oil.

m/z ES+ $[M+H]^+$ 412.2.

**Step e.** This step was conducted in a similar manner to **Intermediate B3,** step b.

**Step f.** This step was conducted in a similar manner to **Intermediate B1,** step c.

m/z ES+ $[M+H]^+$ 382.2.

**Intermediate B7: *tert*-butyl 4-(4-formyloxazol-2-yl)piperazine-1-carboxylate**

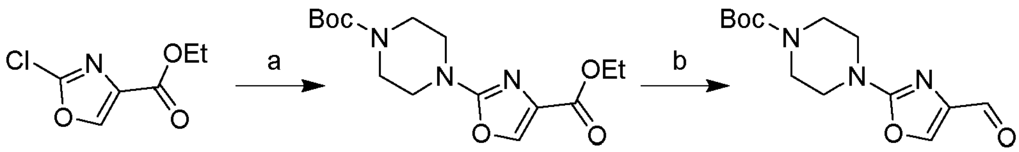


**Step a.** To a solution of ethyl 2-chlorooxazole-4-carboxylate (CAS: 460081-18-9; 1.0 g, 5.70 mmol) in DMF (10 mL) was added $K_2CO_3$ (1.57 g, 11.3 mmol) and *tert*-butyl piperazine-1-carboxylate (1.27 g, 6.83 mmol). The mixture was stirred at 25 °C for 5 h. Upon completion, the reaction mixture was diluted with water (20 mL) and extracted with EtOAc (3 x 10mL). The combined organic layers were evaporated and the residue was purified by column chromatography (PE/EtOAc = 3/1) to give ethyl 2-(4-(*tert*-butoxycarbonyl)piperazin-1-yl)oxazole-4-carboxylate (1.3 g, 68% yield) as a light yellow solid.

m/z ES+ $[M+H]^+$326.2; $^1H$ NMR (400 MHz, $CDCl_3$) $\delta$ ppm 7.79 (s, 1H), 4.41 - 4.32 (m, 2H), 3.55 - 3.49 (m, 8H), 1.48 (s, 9H), 1.38 - 1.33 (m, 3H).

**Step b.** This step was conducted in a similar manner to **Intermediate B3,** step b.

m/z ES+ $[M+H]^+$282.1.

**Intermediate B8: *tert*-butyl 4-(4-formyl-1*H*-imidazol-2-yl)piperazine-1-carboxylate**

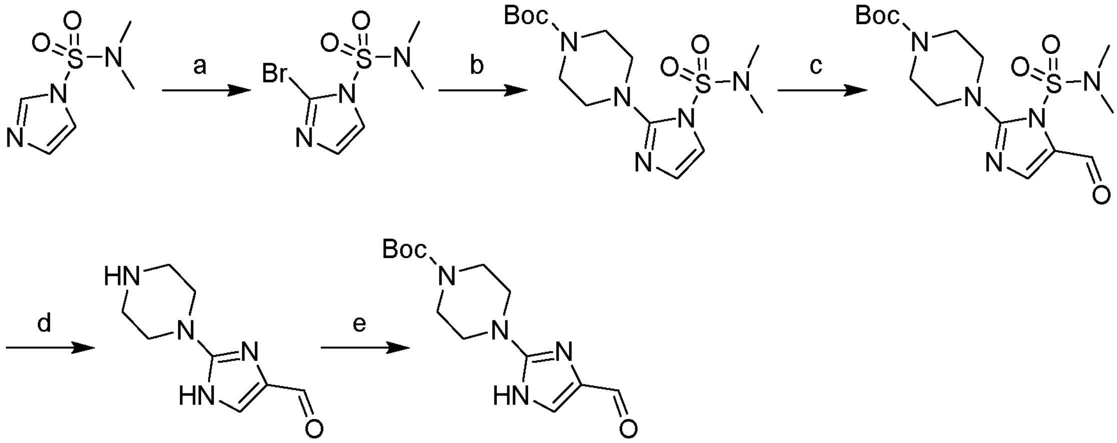


**Step a.** To a mixture of *N,N*-dimethyl-1*H*-imidazole-1-sulfonamide (CAS: 78162-58-0; 9 g, 51.3 mmol) in THF (90 mL) was added n-BuLi (2.5 M in THF, 24.6 mL) dropwise at -70 °C. The mixture was stirred at -70 °C for 30 min, after which $CBr_4$ (18.7 g, 56.5 mmol) was added at -70 °C. The reaction mixture was stirred at 20 °C for 16 h. Upon completion, the reaction mixture was quenched with sat. aq. $NH_4Cl$ (300 mL) and extracted with EtOAc (3 x 200 mL). The combined organic layers were washed with brine (3 x 500 mL) and evaporated. The residue was purified by column chromatography (PE/EtOAc = 2/1) to give 2-bromo-N,N-dimethyl-1*H*-imidazole-1-sulfonamide (10.0 g, 77% yield) as a red solid.

m/z ES+ $[M+H]^+$ 255.9; $^1H$ NMR (400 MHz, $CDCl_3$) δ ppm 7.42 (d, *J* = 1.6 Hz, 1H), 7.01 (d, *J* = 1.6 Hz, 1H), 3.02 (s, 6H).

**Step b**. A mixture of 2-bromo-*N,N*-dimethyl-1*H*-imidazole-1-sulfonamide (2 g, 7.87 mmol) and *tert*-butyl piperazine-1-carboxylate (CAS: 143238-38-4; 14.6 g, 78.7 mmol) was stirred at 90 °C for 20 h. Upon completion, the reaction mixture was evaporated and the residue was purified by reverse phase flash chromatography (water (0.1% FA)/MeCN) to give *tert*-butyl 4-(1-(*N,N*-dimethylsulfamoyl)-1*H*-imidazol-2-yl)piperazine-1-carboxylate (1 g, 35% yield) as a yellow solid.

m/z ES+ $[M+H]^+$ 359.9; $^1H$ NMR (400 MHz, $CDCl_3$) δ ppm 7.09 (d, *J* = 2.0 Hz, 1H), 6.78 (d, *J* = 2.0 Hz, 1H), 3.59 - 3.52 (m, 4H), 3.23 - 3.18 (m, 4H), 2.89 (s, 6H), 1.48 (s, 9H).

**Step c.** To a mixture of *tert*-butyl 4-(1-(*N,N*-dimethylsulfamoyl)-1*H*-imidazol-2-yl)piperazine-1-carboxylate (647 mg, 1.80 mmol) in THF (6 mL) was added *n*-BuLi (2.5 M in THF, 1.44 mL) dropwise at -70 °C. The mixture was stirred at -70 °C for 30 min, after which DMF (789 mg, 10.8 mmol) was added at -70 °C. The reaction mixture was stirred at 20 °C for 2 h. Upon completion, the reaction mixture was quenched with sat. aq. $Na_2CO_3$ (30 mL) and extracted with EtOAc (3 x 10 mL). The combined organic layers were evaporated and the residue was purified by reverse phase flash chromatography (water (0.1% FA)/MeCN) followed by Prep-TLC (EtOAc) to give *tert*-butyl 4-(1-(*N,N*-dimethylsulfamoyl)-5-formyl-1*H*-imidazol-2-yl)piperazine-1-carboxylate (154 mg, 22% yield) as a yellow solid.

$^1H$ NMR (400 MHz, $CDCl_3$) δ ppm 9.83 (s, 1H), 7.63 (s, 1H), 3.62 - 3.57 (m, 4H), 3.47 - 3.42 (m, 4H), 2.84 (s, 6H), 1.49 (s, 9H).

**Step d.** A mixture of *tert*-butyl 4-(1-(*N,N*-dimethylsulfamoyl)-5-formyl-1*H*-imidazol-2-yl)piperazine-1-carboxylate (160 mg, 0.41 mmol) in conc. HCl (2 mL) was stirred at 60 °C for 2 h. Upon completion, the reaction mixture was basified to pH 7 with sat aq. $Na_2CO_3$ and extracted with EtOAc (3 x 10 mL). The combined organic layers were evaporated to give 2-(piperazin-1-yl)-1H-imidazole-4-carbaldehyde (70 mg, 94% yield) as a yellow solid.

m/z ES+ $[M+H]^+$ 181.2.

**Step e.** To a mixture of 2-(piperazin-1-yl)-1*H*-imidazole-4-carbaldehyde (70 mg, 0.39 mmol) and $Na_2CO_3$ (82 mg, 0.78 mmol) in THF (3 mL) was added $Boc_2O$ (169 mg, 0.78 mmol). The reaction mixture was stirred at 20 °C for 30 min. Upon completion, the reaction mixture was filtered and the filtrate was evaporated. The residue was purified by reverse phase flash chromatography (water (0.1% FA)/MeCN) to give the title compound (80 mg, 73% yield) as a white solid.

m/z ES+ $[M+H]^+$ 281.3; $^1H$ NMR (400 MHz, $CDCl_3$) δ ppm 9.83 (s, 1H), 9.31 (s, 1H), 7.61 (s, 1H), 3.57 (s, 8H), 1.49 (s, 9H).

**Intermediate B9: *tert*-butyl 4-(3-formyl-1H-1,2,4-triazol-1-yl)piperidine-1-carboxylate**

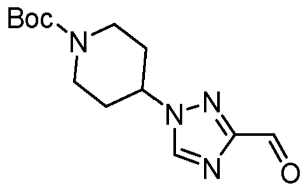

The title compound was prepared in a similar manner to **Intermediate B3,** using methyl 1*H*-1,2,4-triazole-3-carboxylate (CAS: 4928-88-5) and *tert*-butyl 4-iodopiperidine-1-carboxylate (CAS: 301673-14-3) in step a.

$^1$H NMR (400 MHz, $CDCl_3$) δ ppm 10.01 s, 1H), 8.24 (s, 1H), 4.49 - 4.45 (m, 1H), 4.32 - 4.28 (m, 2H), 2.97 - 2.91 (m, 2H), 2.22 - 2.17 (m, 2H), 2.08 - 1.98 (m, 2H), 1.49 (s, 9H).

**Intermediate B10: *tert*-butyl 3-(3-formylphenyl)azetidine-1-carboxylate**

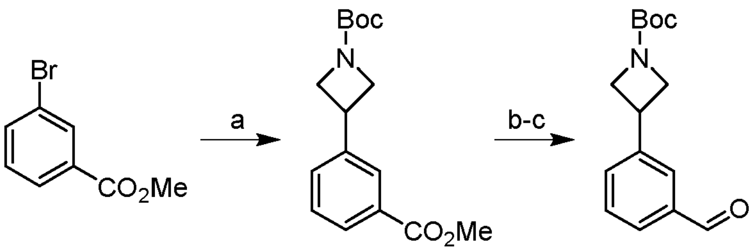


**Step a.** A mixture of methyl 3-bromobenzoate (500 mg, 2.33 mmol), *tert*-butyl *3*-bromoazetidine-1-carboxylate (CAS: 1064194-10-0; 714 mg, 3.02 mmol), $(Ir[dF(CF_3)ppy]_2(dtbpy))PF_6$ (CAS: 870987-63-6; 26 mg, 0.023 mmol), [4,4-bis(1,1-dimethylethyl)-2,2'-bipyridine] nickel (II) dichloride (CAS: 1034901-50-2; 4.6 mg, 0.012 mmol), tris(trimethylsilyl) silane (CAS: 1873-77-4; 578 mg, 2.33 mmol) and $Na_2CO_3$ (493 mg, 4.65 mmol) in 1,2-dimethoxyethane (50 mL) was degassed and purged with $N_2$ 3 times and then the mixture was stirred at 25 °C for 14 h under a $N_2$ atmosphere. Upon completion, the reaction mixture was filtered and evaporated. The residue was diluted with water (20 mL) and extracted with EtOAc (3x 30 mL). The combined organic layers were dried over $Na_2SO_4$, evaporated and purified by column chromatography (PE/EtOAc = 9/1) to give *tert*-butyl 3-(3-(methoxycarbonyl)phenyl)azetidine-1-carboxylate (590 mg, 75% yield) as a light yellow oil.

$^1$H NMR (400 MHz, $CDCl_3$) δ ppm 8.00 - 7.98 (m, 1H), 7.95 - 7.92 (m, 1H), 7.54 - 7.49 (m, 1H), 7.46 - 7.41 (m, 1H), 4.35 (t, *J* = 8.8 Hz, 2H), 4.00 - 3.97 (m, 2H), 3.93 (s, 3H), 3.83 - 3.75 (m, 1H), 1.47 (s, 9H).

**Step b.** A mixture of *tert*-butyl 3-(3-(methoxycarbonyl)phenyl)azetidine-1-carboxylate (100 mg, 0.34 mmol) in DCM (5 mL) was degassed and purged with $N_2$ 3 times. DIBAL-H (1 M in toluene, 1.03 mL) was added and the reaction mixture was stirred at -60 °C for 1 h under a $N_2$ atmosphere. Upon completion, the reaction mixture was quenched with MeOH (0.5 mL) at - 60 °C, filtered and evaporated to give *tert*-butyl 3-(3-(hydroxymethyl)phenyl)azetidine-1-carboxylate (90 mg, crude) as a light yellow oil.

$^1$H NMR (400 MHz, $CDCl_3$) δ ppm 7.37 - 7.33 (m, 2H), 7.27 - 7.23 (m, 2H), 4.73 (s, 2H), 4.33 - 4.30 (m, 2H), 4.02 - 3.95 (m, 2H), 3.84 - 3.68 (m, 1H), 1.48 (s, 9H).

**Step c.** To a solution of *tert*-butyl 3-(3-(hydroxymethyl)phenyl)azetidine-1-carboxylate (90 mg, 0.34 mmol) in DCM (1 mL) was added $MnO_2$ (594 mg, 6.84 mmol). The mixture was stirred at 25 °C for 16 h. Upon completion, the reaction mixture was filtered and evaporated to give the title compound (70 mg, crude) as a light yellow oil.

$^1$H NMR (400 MHz, $CDCl_3$) δ ppm 10.04 (s, 1H), 7.87 (s, 1H), 7.85 - 7.78 (m, 1H), 7.65 - 7.52 (m, 2H), 4.42 - 4.31 (m, 2H), 4.03 - 3.96 (m, 2H), 3.87 - 3.78 (m, 1H), 1.48 (s, 9H).

**Intermediate B11: *tert*-butyl 3-(6-formylpyridin-2-yl)azetidine-1-carboxylate**

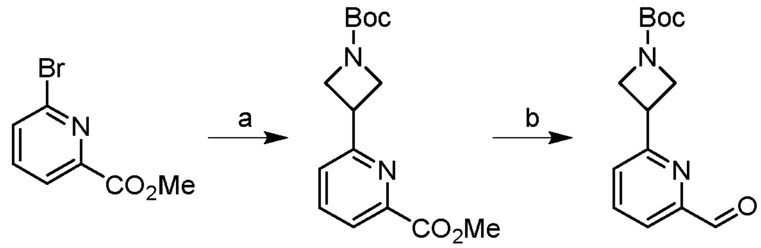


**Step a.** This step was conducted in a similar manner to **Intermediate B10,** step a, using methyl 6-bromopicolinate (CAS: 26218-75-7).

**Step** b. To a solution of methyl 6-(1-(tert-butoxycarbonyl)azetidin-3-yl)picolinate (100 mg, 0.34 mmol) in DCM (5 mL) was added DIBAL-H (1 M in THF, 1.03 mL) at -70 °C. The reaction mixture was stirred at -70 °C for 1 h. Upon completion, the mixture was quenched with MeOH (0.2 mL), stirred at 20 °C for 30 min, filtered and the filtrate was evaporated to give the title compound (80 mg, 89% yield) as a white solid.

$^1$H NMR (400 MHz, $CDCl_3$) δ ppm 10.09 (s, 1H), 7.89 - 7.80 (m, 2H), 7.50 - 7.43 (m, 1H), 4.38 - 4.33 (m, 2H), 4.26 - 4.20 (m, 2H), 4.04 - 3.91 (m, 1H), 1.48 (s, 9H).

**Intermediate B12: tert-butyl 3-(4-formylpyrimidin-2-yl)azetidine-1-carboxylate**

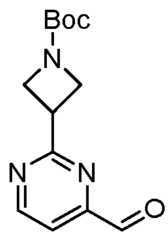

The title compound was prepared in a similar manner to Intermediate B10, using methyl 2-bromopyrimidine-4-carboxylate (CAS: 1209459-78-8) in step a.

$^1$H NMR (400MHz, $CDCl_3$) δ ppm 10.05 (s, 1H), 9.01 (d, *J* = 4.8 Hz, 1H), 7.70 (d, *J* = 4.8 Hz, 1H), 4.46 - 4.26 (m, 4H), 4.18 - 4.10 (m, 1H), 1.48 (s, 9H).

**Intermediate B13: *tert*-butyl 3-(4-formylphenyl)azetidine-1-carboxylate**

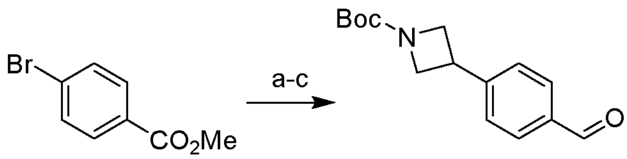


**Step a.** This step was conducted in a similar manner to **Intermediate B10,** step a, using *tert*-butyl 3-bromoazetidine-1-carboxylate and methyl 4-bromobenzoate.

**Steps b-c.** These 2 steps were conducted in a similar manner to **Intermediate B1,** steps b-c. m/z ES+ [M-tBu+H]$^+$206.1; $^1$H NMR (400 MHz, $CDCl_3$) δ ppm 10.01 (s, 1H), 7.88 (d, *J* = 8.0 Hz, 2H), 7.49 (d, *J* = 8.0 Hz, 2H), 4.38 (t, *J* = 8.8 Hz, 2H), 3.99 (t, *J* = 8.8 Hz, 2H), 3.86 - 3.78 (m, 1H), 1.48 (s, 9H).

**Intermediate B14: *tert*-butyl 3-(4-formylcyclohexyl)azetidine-1-carboxylate**

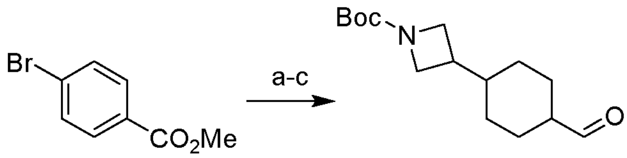


**Step a.** This step was conducted in a similar manner to **Intermediate B10,** step a, using *tert*-butyl 3-bromoazetidine-1-carboxylate and methyl 4-bromobenzoate.

**Step b.** This step was conducted in a similar manner to **Intermediate A23a** and **Intermediate A23b,** step b.

**Step c.** This step was conducted in a similar manner to Intermediate B3, step b.

m/z ES+ $[M-tBu+H]^+$212.0; $^1$H NMR (400 MHz, $CDCl_3$) δ ppm 9.72 - 9.59 (m, 1H), 3.98 - 3.89 (m, 2H), 3.69 - 3.56 (m, 2H), 2.48 - 1.93 (m, 5H), 1.90 - 1.75 (m, 2H), 1.64 - 1.49 (m, 2H), 1.43 (d, *J* = 2.0 Hz, 9H), 1.33 - 1.11 (m, 1H), 1.01 - 0.81 (m, 1H).

**Intermediate B15: *tert*-butyl 3-(2-fluoro-5-formylphenyl)azetidine-1-carboxylate**

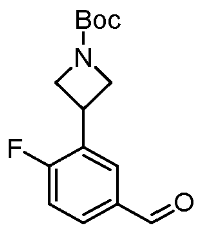

The title compound was prepared in a similar manner to **Intermediate B13,** using *tert*-butyl 3-bromoazetidine-1-carboxylate and methyl 3-bromo-4-fluorobenzoate (CAS: 82702-31-6). $^1$H NMR (400 MHz, $CDCl_3$) δ ppm 9.99 (s, 1H), 7.95 (dd, *J* = 7.2, 2.0 Hz, 1H), 7.85 - 7.79 (m, 1H), 7.22 (dd, *J* = 9.6, 8.8 Hz, 1H), 4.40 - 4.32 (m, 2H), 4.10 - 4.01 (m, 3H), 1.48 (s, 9H).

**Intermediate B16: tert-butyl 2-(4-formylphenyl)pyrrolidine-1-carboxylate**

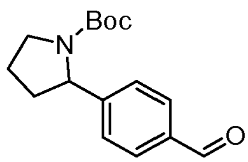

The title compound was prepared in a similar manner to **Intermediate B10,** using methyl 4-bromobenzoate and (tert-butoxycarbonyl)proline (CAS: 59433-50-0) in step a.

$^1$H NMR (400 MHz, $CDCl_3$) δ ppm 10.00 (s, 1H), 7.83 (d, *J* = 8.0 Hz, 2H), 7.34 (d, *J* = 8.0 Hz, 2H), 5.00 - 4.83 (m, 1H), 3.66 - 3.64 (m, 2H), 2.39 - 2.37 (m, 1H), 1.92 - 1.80 (m, 3H), 1.48 - 1.17 (m, 9H).

**Intermediate B17: *tert*-butyl 6-formyl-3,4-dihydroisoquinoline-2(1*H*)-carboxylate**

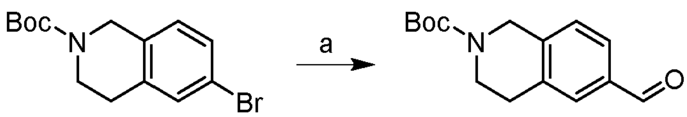


**Step a.** A mixture of *tert*-butyl 6-bromo-3,4-dihydroisoquinoline-2(1*H*)-carboxylate (CAS: 893566-74-0; 1.0 g, 3.20 mmol), N-formylsaccharin (CAS: 50978-45-5; 1.35 g, 6.41 mmol), $Pd(OAc)_2$ (58 mg, 0.26 mmol), 1,4-bis(diphenylphosphino)butane (CAS: 7688-25-7; 163 mg, 0.38 mmol), triethylsilane (819 mg, 7.05 mmol) and $Na_2CO_3$ (848 mg,

8.01 mmol) in DMF (20 mL) was stirred at 80 °C for 16 h under a $N_2$ atmosphere. Upon completion, the reaction mixture was filtered, evaporated and purified by column chromatography (PE/EtOAc = 5/1) to give the title compound (100 mg, 11% yield) as a colourless oil.

$^1$H NMR (400 MHz, $CDCl_3$) δ ppm 9.90 (s, 1H), 7.69 - 7.54 (m, 2H), 7.21 (s, 1H), 4.58 (s, 2H), 3.61 (t, *J* = 5.6 Hz, 2H), 2.85 (t, *J* = 5.6 Hz, 2H), 1.43 (s, 9H).

**Intermediate B18: *tert*-butyl 5-formylisoindoline-2-carboxylate**

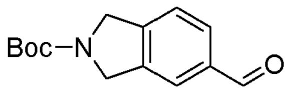

The title compound was prepared in a similar manner to **Intermediate B17,** using *tert*-butyl 5-bromoisoindoline-2-carboxylate (CAS: 201940-08-1).

$^1$H NMR (400 MHz, $CDCl_3$) δ ppm 10.00 (s, 1H), 7.81 - 7.75 (m, 2H), 7.45 - 7.38 (m, 1H), 4.75 - 4.72 (m, 4H), 1.52 (s, 9H).

**Intermediate B19: *tert*-butyl 2-formyl-5,6-dihydroimidazo[1,2-*a*]pyrazine-7(8*H*)-carboxylate**

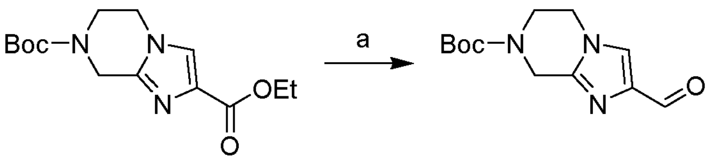


**Step a**. To a solution of 7-(*tert*-butyl) 2-ethyl 5,6-dihydroimidazo[1,2-a]pyrazine-2,7(8*H*)-dicarboxylate ( CAS: 1053656-22-6; 300 mg, 1.07 mmol) in DCM (8 mL) was added DIBAL-H (1 M in toluene, 3.20 mL). The reaction mixture was stirred at -70 °C for 1 h. Upon completion, the mixture was quenched with MeOH (0.7 mL), stirred at 20 °C for 30 min, filtered and the filtrate was evaporated to give the title compound (210 mg, 78% yield) as a white solid.

$^1$H NMR (400 MHz, $CDCl_3$) δ ppm 9.84 (s, 1H), 7.56 (s, 1H), 4.74 (s, 2H), 4.09 (t, *J* = 7.2 Hz, 2H), 3.89 (t, *J* = 7.2 Hz, 2H), 1.49 (s, 9H).

**Intermediate B20: *tert*-butyl 2-formyl-5,6-dihydro-[1,2,4]triazolo[1,5-*a*]pyrazine-7(8*H*)-carboxylate**

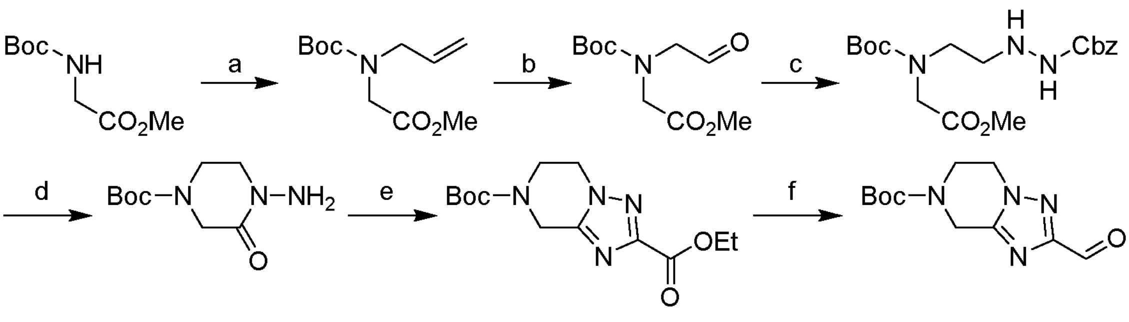


**Step a.** To a solution of NaH (1.59 g, 39.6 mmol, 60% dispersion in mineral oil) in DMF (100 mL) was added methyl (*tert*-butoxycarbonyl)glycinate (CAS: 31954-27-5; 5 g, 26.4 mmol) at 0 °C. The reaction mixture was stirred at 0 °C for 30 min, then 3-bromoprop-1-ene (3.20 g, 26.4 mmol) was added. The reaction mixture was stirred at 0 °C for a further 3 h. Upon completion, the reaction mixture was quenched with sat. aq. $NH_4Cl$ (20 mL) at 0 °C and then diluted with water (100 mL) and extracted with EtOAc (3 x 50 mL). The combined organic layers were washed with brine (50 mL), dried over $Na_2SO_4$, evaporated and purified by column chromatography (PE/EtOAc = 5/1) to give methyl *N*-allyl-*N*-(*tert*-butoxycarbonyl)glycinate (3.0 g, 50% yield) as a colourless oil.

$^1H$ NMR (400 MHz, $CDCl_3$) δ ppm 5.76 - 5.65 (m, 1H), 5.11 - 5.03 (m, 2H), 3.93 - 3.73 (m, 4H), 3.66 (s, 3H), 1.40 - 1.32 (m, 9H).

**Step b.** A mixture of methyl *N*-allyl-*N*-(*tert*-butoxycarbonyl)glycinate (500 mg, 2.18 mmol), $OsO_4$ (55.4 mg, 0.22 mmol), $NaIO_4$ (1.40 g, 6.54 mmol) in THF (5 mL) and water (5 mL) was stirred at 0 °C for 3 h under a $N_2$ atmosphere. Upon completion, the mixture was quenched with sat. aq. $Na_2S_2O_3$ (5 mL), stirred at 25 °C for a further 30 min, then extracted with EtOAc (3 x 20 mL). The combined organic layers were washed with sat. aq. $Na_2S_2O_3$ (2 x 15 mL) and brine (20 mL), dried over $Na_2SO_4$, filtered and evaporated to give methyl *N*-(tert-butoxycarbonyl)-*N*-(2-oxoethyl)glycinate (350 mg, 1.51 mmol, 69% yield) as a black oil.

$^1H$ NMR (400 MHz, $CDCl_3$) δ ppm 9.69 (d, *J* = 7.2 Hz, 1H), 4.15 - 3.91 (m, 4H), 3.77 (s, 3H), 1.47 (s, 9H).

**Step c.** A mixture of benzyl hydrazinecarboxylate (862 mg, 5.19 mmol), methyl *N*-(tert-butoxycarbonyl)-*N*-(2-oxoethyl)glycinate (1.2 g, 5.19 mmol), $NaBH_3CN$ (326 mg, 5.19 mmol), 4 Å molecular sieves (300 mg) and acetic acid (1.56 g, 25.9 mmol) in MeOH (30 mL) was stirred at 25 °C for 6 h under a $N_2$ atmosphere. Upon completion, the reaction mixture was filtered and the filtrate was evaporated. The residue was purified by column chromatography (PE/EtOAc = 1/1) to give benzyl 2-(2-((*tert*-butoxycarbonyl)(2-methoxy-2-oxoethyl)amino)ethyl)hydrazine-1-carboxylate (1.5 g, 76% yield) as a colourless oil.

$^1H$ NMR (400 MHz, $CDCl_3$) δ ppm 7.33 - 7.23 (m, 5H), 7.20 (br s, 1H), 5.07 (s, 2H), 3.95 - 3.77 (m, 2H), 3.66 (s, 3H), 3.42 - 3.29 (m, 2H), 2.99 - 2.83 (m, 2H), 1.43 - 1.31 (m, 9H).

**Step d.** To a solution of benzyl 2-(2-((*tert*-butoxycarbonyl)(2-methoxy-2-oxoethyl)amino)ethyl)hydrazine-1-carboxylate (1.5 g, 3.93 mmol) in MeOH (30 mL) was added 10% Pd/C (150 mg) under a $N_2$ atmosphere. The suspension was degassed and purged with $H_2$ 3 times. The mixture was stirred under a $H_2$ atmosphere (15 psi) at 25 °C for 5 h. Upon completion, the reaction mixture was filtered and evaporated to give *tert*-butyl 4-amino-3-oxopiperazine-1-carboxylate (0.8 g, 95% yield) as a yellow solid.

$^1H$ NMR (400 MHz, $CDCl_3$) δ ppm 4.16 (s, 2H), 3.76 - 3.68 (m, 2H), 3.58 - 3.54 (m, 2H), 1.49 (s, 9H).

**Step e.** A mixture of *tert*-butyl 4-amino-3-oxopiperazine-1-carboxylate (0.8 g, 3.72 mmol), ethyl 2-ethoxy-2-iminoacetate (CAS: 816-27-3; 540 mg, 3.72 mmol) in EtOH (10 mL) was degassed and purged with $N_2$ 3 times. The mixture was stirred at 25 °C for 1 h under a $N_2$ atmosphere. Upon completion, the mixture was evaporated and purified by reverse phase flash chromatography (water (0.1% FA)/MeCN) to give 7-(*tert*-butyl) 2-ethyl 5,6-dihydro-[1,2,4]triazolo[1,5-a] pyrazine-2,7(8H)-dicarboxylate (500 mg, 45% yield) as a colourless oil. m/z ES+ $[M+H]^+$ 297.1; $^1H$ NMR (400 MHz, $CDCl_3$) δ ppm 4.81 (s, 2H), 4.47 (q, *J* = 7.2 Hz, 2H), 4.31 (t, *J* = 5.6 Hz, 2H), 3.96 (t, *J* = 5.2 Hz, 2H), 1.50 (s, 9H), 1.43 (t, *J* = 7.2 Hz, 3H).

**Step f.** To a solution of 7-(*tert*-butyl) 2-ethyl 5,6-dihydro-[1,2,4]triazolo[1,5-*a*]pyrazine-2,7(8*H*)-dicarboxylate (100 mg, 0.34 mmol) in THF (5 mL) was added DIBAL-H (1 M in toluene, 1.01 mL). The mixture was stirred at -78 °C for 2 h. Upon completion, the reaction mixture was quenched with MeOH (1 mL) at -78 °C and then stirred for a further 2 h. The mixture was filtered and evaporated to give the title compound (60 mg, 71% yield) as a white solid.

$^1H$ NMR (400 MHz, $CDCl_3$) δ ppm 10.01 (s, 1H), 4.84 (s, 2H), 4.34 (t, *J* = 5.4 Hz, 2H), 4.02 (t, *J* = 5.4 Hz, 2H), 1.52 (s, 9H).

**Intermediate B21: *tert*-butyl 3-formyl-5,6-dihydro-[1,2,4]triazolo[4,3-*a*]pyrazine-7(8*H*)-carboxylate**

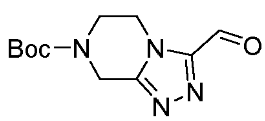

The title compound was prepared in a similar manner to **Intermediate B19,** using 7-(*tert*-butyl) 3-ethyl 5,6-dihydro-[1,2,4]triazolo[4,3-*a*]pyrazine-3,7(8*H*)-dicarboxylate (CAS: 1215852-11-1). $^1H$ NMR (400 MHz, $CDCl_3$) δ ppm 10.10 (s, 1H), 4.93 (s, 2H), 4.43 - 4.38 (m, 2H), 3.90 - 3.87 (m, 2H), 1.52 (s, 9H).

**Intermediate B22: *tert*-butyl 3-(4-formylpiperidin-1-yl)-3-methylazetidine-1-carboxylate**

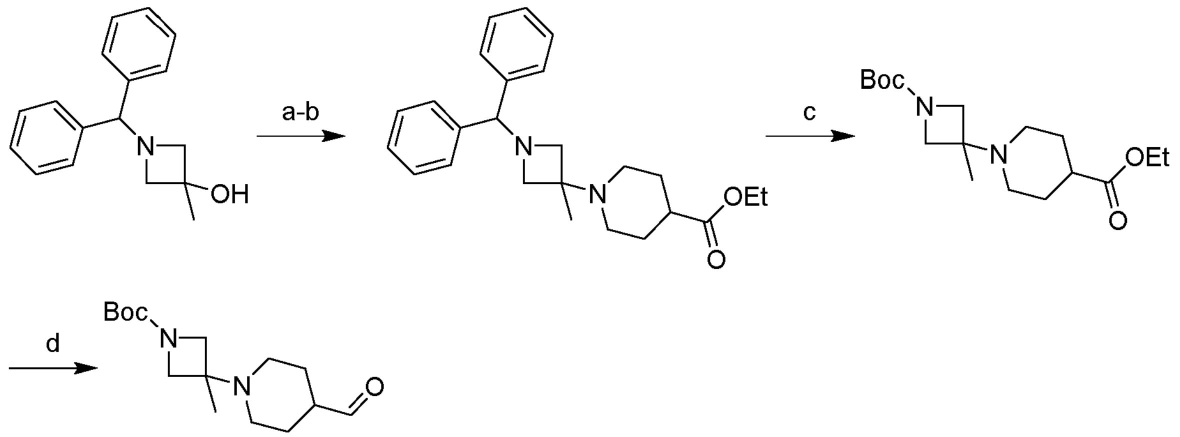


**Steps a-b.** These 2 steps were conducted in a similar manner to **Intermediate A25,** steps a-b, using ethyl piperidine-4-carboxylate (CAS: 1126-09-6) in step b.

**Step c.** A mixture of ethyl 1-(1-benzhydryl-3-methylazetidin-3-yl)piperidine-4-carboxylate (580 mg, 1.48 mmol), 10% Pd/C (10 mg), 10% $Pd(OH)_2$/C (10 mg) and 4 M HCl in MeOH (0.37 mL, 1.48 mmol) in MeOH (5 mL) was stirred at 25 °C for 16 h. Upon completion, the mixture was filtered and the filtrate was evaporated to give ethyl 1-(3-methylazetidin-3-yl) piperidine-4-carboxylate (320 mg, 95% yield) as colourless oil. A mixture of ethyl 1-(3-methylazetidin-3-yl)piperidine-4-carboxylate (220 mg, 0.97 mmol), $Boc_2O$ (318 mg, 1.46 mmol) and TEA (295 mg, 2.92 mmol) in THF (2 mL) was stirred at 25 °C for 16 h. Upon completion, the reaction mixture was evaporated and the residue was purified by column chromatography (PE/EtOAc = 2/1) to give ethyl 1-(1-(*tert*-butoxycarbonyl)-3-methylazetidin-3-yl)piperidine-4-carboxylate (300 mg, 94% yield) as white solid.

m/z ES+ $[M+H]^+$ 327.1; $^1H$ NMR (400 MHz, $CDCl_3$) δ ppm 4.21 - 4.11 (m, 2H), 3.78 (d, *J*= 8.0 Hz, 2H), 3.52 (d, *J* = 7.6 Hz, 2H), 2.71 - 2.55 (m, 2H), 2.37 - 2.07 (m, 3H), 2.00 - 1.88 (m, 2H), 1.84 - 1.69 (m, 2H), 1.46 (s, 9H), 1.28 - 1.20 (m, 6H).

**Step d.** This step was conducted in a similar manner to **Intermediate B3,** step b.

$^1H$ NMR (400 MHz, $CDCl_3$) δ ppm 9.67 (s, 1H), 3.77 (d, *J* = 8.0 Hz, 2H), 3.58 - 3.48 (m, 2H), 2.70 - 2.53 (m, 2H), 2.32 - 2.16 (m, 3H), 2.01 - 1.87 (m, 2H), 1.76 - 1.62 (m, 2H), 1.46 (s, 9H), 1.26 (s, 3H).

**Intermediate B23: *tert*-butyl 3-(methyl(3-oxopropyl)amino)azetidine-1-carboxylate**

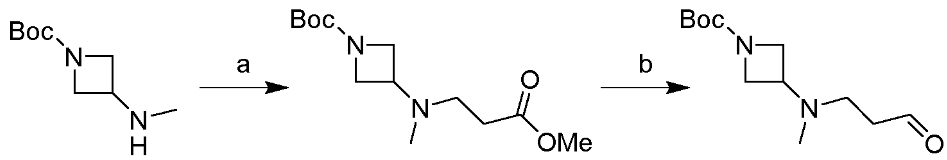


**Step a.** A mixture of *tert*-butyl 3-(methylamino)azetidine-1-carboxylate (100 mg, 0.54 mmol), TEA (271 mg, 2.68 mmol) and methyl acrylate (138 mg, 1.61 mmol) in MeOH (1 mL) was stirred at 25 °C for 16 h. Upon completion, the reaction mixture was diluted with water (10 mL) and extracted with EtOAc (3 x 10 mL). The combined organic layers were washed with brine (10 mL), dried over $Na_2SO_4$ and evaporated. The residue was purified by column chromatography (EtOAc) to give *tert*-butyl 3-((3-methoxy-3-oxopropyl)(methyl)amino)azetidine-1-carboxylate (100 mg, 68% yield) as a yellow oil.

$^1H$ NMR (400 MHz, $CDCl_3$) δ ppm 3.97 - 3.88 (m, 2H), 3.86 - 3.74 (m, 2H), 3.70 (s, 3H), 3.29 - 3.13 (m, 1H), 2.68 - 2.42 (m, 4H), 2.15 (s, 3H), 1.44 (s, 9H).

**Step b.** This step was conducted in a similar manner to **Intermediate B3,** step b.

$^1H$ NMR (400 MHz, $CDCl_3$) δ ppm 9.81 (s, 1H), 3.98 - 3.91 (m, 2H), 3.85 - 3.75 (m, 2H), 3.26 - 3.12 (m, 1H), 2.68 - 2.60 (m, 4H), 2.21 (m, 3H), 1.46 (s, 9H).

**Intermediate B24: *tert*-butyl (7*S*)-7-formyl-8-methyloctahydro-2*H*-pyrazino[1,2-*a*]pyrazine-2-carboxylate**

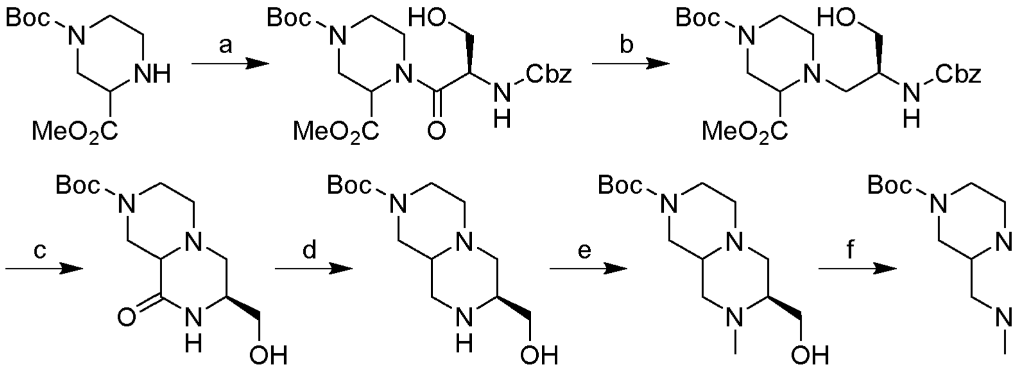


**Step** a. To a solution of 1-(*tert*-butyl) 3-methyl piperazine-1,3-dicarboxylate (5.0 g, 20.4 mmol) and ((benzyloxy) carbonyl)-D-serine (4.90 g, 20.4 mmol) in DCM (100 mL) was added HATU (7.78 g, 20.4 mmol) and DIPEA (5.29 g, 40.9 mmol). The mixture was stirred at rt for 1 h. Upon completion, the reaction mixture was diluted with water (100 mL) and extracted with EtOAc (3 x 100 mL). The combined organic layers were evaporated and the residue was purified by reverse phase flash chromatography (water (0.1% FA)/MeCN) to give 1-(*tert*-butyl) 3-methyl 4-(((benzyloxy)carbonyl)-D-seryl) piperazine-1,3-dicarboxylate (4.3 g, 44% yield) as an off-white solid.

m/z ES+ $[M+H]^+$ 466.1; $^1$H NMR (400 MHz, DMSO-$d_6$) δ ppm 7.38 - 7.31 (m, 5H), 5.07 - 4.92 (m, 3H), 4.92 - 4.71 (m, 1H), 4.67 - 4.45 (m, 1H), 4.42 - 4.13 (m, 2H), 3.98 - 3.73 (m, 2H), 3.71 - 3.53 (m, 4H), 3.48 - 3.38 (m, 1H), 3.21 - 3.08 (m, 1H), 3.04 - 2.62 (m, 2H), 1.39 - 1.37 (m, 9H).

**Step b.** A solution of borane dimethyl sulfide complex (10 M in THF, 13.9 mL) was added dropwise to a solution of *1*-(*tert*-butyl) 3-methyl 4-(((benzyloxy)carbonyl)-*D*-seryl)piperazine-1,3-dicarboxylate (6.5 g, 13.9 mmol) in THF (60 mL) at 0 °C. The mixture was stirred at 25 °C for 12 h. Upon completion, MeOH (30 mL) was slowly added and the mixture was diluted with sat. aq. $NaHCO_3$ (100 mL) and extracted with DCM (3 x 100 mL). The combined organic layers were washed with brine (300 mL), dried over $Na_2SO_4$ and evaporated. The residue was purified by reverse phase flash chromatography (water (0.1% $NH_4OH$)/MeCN) to give 1-(*tert*-butyl) 3-methyl 4-((*S*)-2-(((benzyloxy)carbonyl)amino)-3-hydroxypropyl) piperazine-1,3-dicarboxylate (4.7 g, 74% yield) as a yellow solid.

m/z ES+ $[M+H]^+$452.2; $^1$H NMR (400 MHz, DMSO-$d_6$) δ ppm 7.41 - 7.27 (m, 5H), 6.95 - 6.85 (m, 1H), 5.07 - 4.94 (m, 2H), 4.66 - 4.57 (m, 1H), 4.06 - 3.82 (m, 1H), 3.77 - 3.49 (m, 5H), 3.46 - 3.35 (m, 2H), 3.13 -3.09 (m, 1H), 3.05 - 2.75 (m, 2H), 2.72 - 2.61 (m, 1H), 2.60 (br s, 1H), 2.48 - 2.32 (m, 2H), 1.36 (s, 9H).

**Step c.** A mixture of 1-(*tert*-butyl) 3-methyl 4-((*S*)-2-(((benzyloxy)carbonyl)amino)-3-hydroxypropyl)piperazine-1,3-dicarboxylate (4.7 g, 10.4 mmol), 10% Pd/C (470 mg) in MeOH (80 mL) was stirred at 20 °C for 2 h under a $H_2$ (15 psi) atmosphere. Upon completion, the reaction mixture was filtered and the filtrate was evaporated to give *tert*-butyl (7*S)*-7-(hydroxymethyl)-9-oxooctahydro-2*H*-pyrazino[1,2-a]pyrazine-2-carboxylate (2.97 g, crude) as an off-white solid.

$^1$H NMR (400 MHz, DMSO-$d_6$) δ ppm 7.91 - 7.58 (m, 1H), 4.85 (br s, 1H), 4.30 - 4.17 (m, 1H), 3.84 (br s, 1H), 3.47 - 3.40 (m, 1H), 3.32 - 3.26 (m, 1H), 3.19 - 3.08 (m, 2H), 3.01 - 2.93 (m, 1H), 2.87 - 2.72 (m, 2H), 2.43 - 2.35 (m, 2H), 2.15 - 2.01 (m, 1H), 1.40 (s, 9H).

**Step d.** A solution of borane dimethyl sulfide complex (10 M in THF, 6.7 mL) was added dropwise to a solution of *tert*-butyl (7*S*)-7-(hydroxymethyl)-9-oxooctahydro-2*H*-pyrazino[1,2-a]pyrazine-2-carboxylate (1.9 g, 6.66 mmol) in THF (30 mL) at 0 °C. The mixture was stirred at 50 °C for 12 h. Upon completion, MeOH (20 mL) was slowly added to the reaction mixture, diluted with sat. aq. $NaHCO_3$ (50 mL) and extracted with DCM (3 x 50 mL). The combined organic layers were washed with brine (120 mL), dried over $Na_2SO_4$ and evaporated to give *tert-butyl* (7*S*)-7-(hydroxymethyl)octahydro-2*H*-pyrazino[1,2-*a*]pyrazine-2-carboxylate (1.8 g, crude) as a yellow solid.

$^1$H NMR (400 MHz, DMSO-$d_6$) δ ppm 6.14 - 5.70 (m, 1H), 5.02 - 4.74 (m, 1H), 3.86 - 3.57 (m, 5H), 2.90 - 2.76 (m, 3H), 2.74 - 2.70 (m, 1H), 2.64 - 2.61 (m, 1H), 2.59 - 2.52 (m, 2H), 2.42 - 2.38 (m, 1H), 2.01 - 1.92 (m, 1H), 1.40 (s, 9H).

**Step e.** To a solution of *tert*-butyl (7*S*)-7-(hydroxymethyl)octahydro-2*H*-pyrazino[1,2-*a*]pyrazine-2-carboxylate (1.8 g, 6.63 mmol) and formaldehyde (37 wt.% in $H_2O$; 2.69 g, 33.1 mmol) in MeOH (25 mL) was added $NaBH_3CN$ (833 mg, 13.2 mmol). The mixture was stirred at 20 °C for 12 h. Upon completion, the reaction mixture was evaporated and the residue was purified by reverse phase flash chromatography (water (0.1% $NH_4OH$)/MeCN) to give *tert*-butyl (7*S*)-7-(hydroxymethyl)-8-methyloctahydro-2*H*-pyrazino[1,2-*a*]pyrazine-2-carboxylate (750 mg, 33% yield) as a white solid.

m/z ES+ $[M+H]^+$ 286.2.

**Step f.** A solution of DMSO (657 mg, 8.41 mmol) in DCM (0.5 mL) was added dropwise to a solution of oxalyl chloride (533 mg, 4.20 mmol) in DCM (6 mL) at -78 °C. The mixture was stirred at -78 °C for 10 min, after which a solution of *tert*-butyl (7S)-7-(hydroxymethyl)-8-methyloctahydro-2*H*-pyrazino[1,2-*a*]pyrazine-2-carboxylate (400 mg, 1.40 mmol) in DCM (2 mL) was added dropwise at -78 °C. The mixture was stirred at -78 °C for 20 min, and then TEA (1.42 g, 14.0 mmol) was added at -78 °C. The mixture was stirred and warmed to 20 °C for 30 min. Upon completion, the reaction mixture was

diluted with sat. aq. $NaHCO_3$ (10 mL) and the layers separated. The aqueous mixture as further extracted with DCM (2 x 5 mL). The combined organic layers were washed with brine (10 mL), dried over $Na_2SO_4$ and evaporated to give the title compound (360 mg, crude) as a brown solid.

m/z ES+ $[M+H]^+$ 284.2.

**Intermediate B25: *tert*-butyl 2-formyl-8-methyl-5,6-dihydroimidazo[1,2-a]pyrazine-7(8H)-carboxylate**

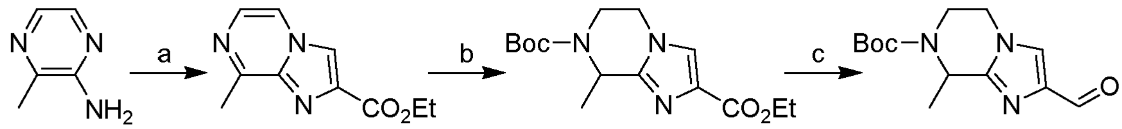


**Step a.** A mixture of 3-methylpyrazin-2-amine (CAS: 19838-08-5; 3.00 g, 27.5 mmol) and ethyl 3-bromo-2-oxopropanoate (6.43 g, 33.0 mmol) in 1,2-dimethoxyethane (30 mL) was stirred at 25 °C for 16 h. Upon completion, the mixture was cooled to 0 °C and filtered. The filter cake was dissolved in EtOH (40 mL) and stirred at 80 °C for 2 h. Upon completion, the reaction mixture was cooled to 0 °C and filtered to give ethyl 8-methylimidazo[1,2-a]pyrazine-2-carboxylate (3.2 g, crude) as a white solid.

m/z ES+ $[M+H]^+$ 205.9; $^1H$ NMR (400 MHz, DMSO-$d_6$) δ ppm 8.86 (s, 1H), 8.70 (d, *J* = 4.8 Hz, 1H), 7.96 (d, *J* = 4.8 Hz, 1H), 4.39 - 4.30 (m, 2H), 2.88 (s, 3H), 1.34 (t, *J* = 7.2 Hz, 3H).

**Step b.** To a solution of ethyl 8-methylimidazo[1,2-a]pyrazine-2-carboxylate (1.2 g, 5.85 mmol) in EtOH (40 mL) was added 10% Pd/C (320 mg). The mixture was stirred at 50 °C for 16 h under a $H_2$ (50 psi) atmosphere. Upon completion, the reaction mixture was filtered and evaporated to give ethyl 8-methyl-5,6,7,8-tetrahydroimidazo[1,2-*a*]pyrazine-2-carboxylate (1.2 g, crude) as a yellow solid. To a mixture of ethyl 8-methyl-5,6,7,8-tetrahydroimidazo[1,2-a]pyrazine-2-carboxylate (1.2 g, 5.73 mmol) and $Na_2CO_3$ (1.52 g, 14.3 mmol) in DCM (15 mL) was added $Boc_2O$ (1.88 g, 8.60 mmol). The reaction mixture was stirred at 20 °C for 2 h. Upon completion, the reaction mixture was quenched with water (30 mL) and extracted with EtOAc (3 x 20 mL). The combined organic layers were washed with brine (3 x 20 mL), dried over $Na_2SO_4$ and evaporated. The residue was purified by column chromatography (PE/EtOAc = 1/1) to give 7-(*tert*-butyl) 2-ethyl 8-methyl-5,6-dihydroimidazo[1,2-*a*]pyrazine-2,7(8*H*)-dicarboxylate (1.1 g, 61% yield) as a white solid.

m/z ES+ $[M+H]^+$ 310.4; $^1H$ NMR (400 MHz, $CDCl_3$) δ ppm 7.52 (s, 1H), 5.43 - 5.40 (m, 1H), 4.50 - 4.32 (m, 3H), 4.06 - 3.97 (m, 2H), 3.39 - 3.25 (m, 1H), 1.56 (d, *J* = 6.8 Hz, 3H), 1.49 (s, 9H), 1.38 (t, *J* = 7.2 Hz, 3H).

**Step c.** This step was conducted in a similar manner to **Intermediate B3,** step b.

$^1H$ NMR (400 MHz, $CDCl_3$) δ ppm 9.86 (s, 1H), 7.53 (s, 1H), 5.43 - 5.39 (m, 1H), 4.52 - 4.39 (m, 1H), 4.14 - 3.94 (m, 2H), 3.39 - 3.26 (m, 1H), 1.58 (d, *J* = 6.8 Hz, 3H), 1.51 (s, 9H).

**Intermediate B26: *tert*-butyl 2-formyl-6-methyl-5,6-dihydroimidazo[1,2-*a*]pyrazine-7(8*H*)-carboxylate**

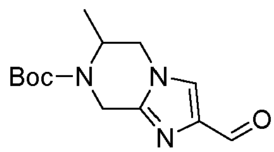

The title compound was prepared in a similar manner to **Intermediate B25,** using 5-methylpyrazin-2-amine (CAS: 5521-58-4) in step a.

$^1H$ NMR (400 MHz, $CDCl_3$) δ ppm 9.86 (s, 1H), 7.56 (s, 1H), 5.07 (d, *J* = 17.6 Hz, 1H), 4.94 - 4.82 (m, 1H), 4.35 (d, *J* = 17.6 Hz, 1H), 4.21 (dd, *J* = 12.4, 4.8 Hz, 1H), 3.90 (d, *J* = 12.4 Hz, 1H), 1.50 (s, 9H), 1.21 (d, *J* = 7.2 Hz, 3H).

**Intermediate B27: *tert*-butyl 4-(2-oxoethoxy)piperidine-1-carboxylate**

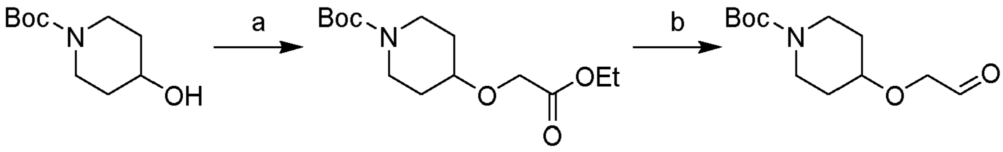


**Step a**. A solution of ethyl 2-diazoacetate (8.50 g, 74.5 mmol) in DCM (80 mL) at 10 °C was added dropwise over 2 h to a solution of *tert*-butyl 4-hydroxypiperidine-1-carboxylate (5.0 g, 24.8 mmol) and $Rh(OAc)_2$ (275 mg, 1.24 mmol) in DCM (80 mL). The mixture was stirred at 20 °C for 46 h. Upon completion, the reaction mixture was evaporated and the residue was purified by column chromatography (PE/EtOAc = 8/1) to give *tert*-butyl 4-(2-ethoxy-2-oxoethoxy)piperidine-1-carboxylate (3 g, 40% yield) as a light yellow oil.

$^1H$ NMR (400 MHz, $CDCl_3$) δ ppm 4.25 - 4.20 (m, 2H), 4.12 (s, 2H), 3.84 - 3.76 (m, 2H), 3.61 - 3.53 (m, 1H), 3.12 - 3.07 (m, 2H), 1.91 - 1.82 (m, 2H), 1.60 - 1.57 (m, 2H), 1.46 (s, 9H), 1.30 (t, $J$ = 7.2 Hz, 3H).

**Step b.** This step was conducted in a similar manner to **Intermediate B3,** step b.

$^1H$ NMR (400 MHz, $CDCl_3$) δ ppm 9.74 (s, 1H), 3.85 - 3.71 (m, 2H), 3.58 - 3.51 (m, 2H), 3.14 - 3.01 (m, 2H), 1.95 - 1.80 (m, 2H), 1.69 - 1.65 (m, 1H), 1.63 - 1.53 (m, 2H), 1.46 (s, 9H).

**Intermediate B28: *tert*-butyl (*S*)-3-((2-oxoethoxy)methyl)pyrrolidine-1-carboxylate**

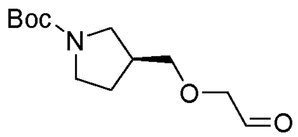

The title compound was prepared in a similar manner to **Intermediate B27,** using *tert*-butyl (*S*)-3-(hydroxymethyl)pyrrolidine-1-carboxylate (CAS: 199174-24-8) in step a.

$^1H$ NMR (400 MHz, $CDCl_3$) δ ppm 9.73 (s, 1H), 4.10 (s, 2H), 3.59 - 3.51 (m, 3H), 3.47 - 3.43 (m, 1H), 3.35 - 3.31 (m, 1H), 3.15 - 3.10 (m, 1H), 2.55 - 2.50 (m, 1H), 2.04 - 1.94 (m, 1H), 1.78 - 1.67 (m, 1H), 1.47 (s, 9H).

**Intermediate B29: *tert*-butyl (*R*)-3-((2-oxoethoxy)methyl)pyrrolidine-1-carboxylate**

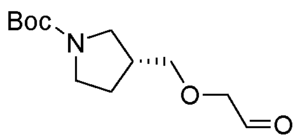

The title compound was prepared in a similar manner to **Intermediate B27,** using *tert*-butyl (*R*)-3-(hydroxymethyl)pyrrolidine-1-carboxylate (CAS: 138108-72-2) in step a.

$^1H$ NMR (400 MHz, $CDCl_3$) δ ppm 9.72 (s, 1H), 4.09 (s, 2H), 3.47 - 3.43 (m, 4H), 3.34 - 3.30 (m, 1H), 3.13 - 3.08 (m, 1H), 2.54 - 2.49 (m, 1H), 2.02 - 1.96 (m, 2H), 1.46 (s, 9H).

**Intermediate B30: *tert*-butyl 3-(4-oxobutyl)azetidine-1-carboxylate**

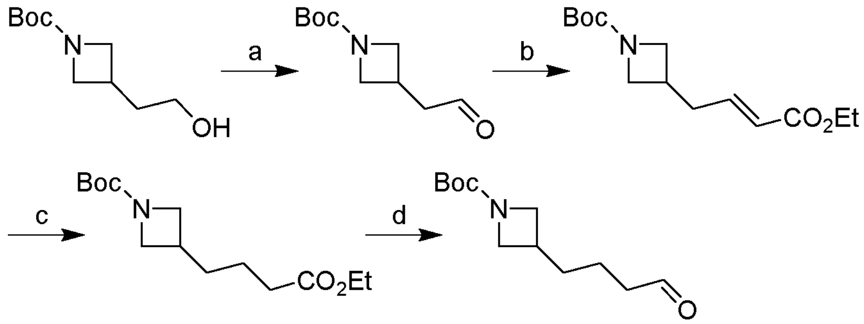


**Step a**. To a solution of oxalyl chloride (946 mg, 7.45 mmol) in DCM (5 mL) was added DMSO (1.16 g, 14.9 mmol) at -78 °C. The mixture was stirred at -78 °C for 10 min. A solution of *tert*-butyl 3-(2-hydroxyethyl)azetidine-1-carboxylate (CAS: 152537-03-6; 500 mg, 2.48 mmol) in DCM (4 mL) was then added and the mixture was stirred at -78 °C for a further 30 min. After which, TEA (2.51 g, 24.8 mmol) was added and the mixture was stirred at -78 °C for an additional 30 min. Upon completion, the reaction mixture was warmed to 20 °C, diluted with water (50 mL) and extracted with DCM (20 mL). The combined organic layers were washed with brine (50 mL), dried over $Na_2SO_4$ and evaporated to give *tert*-butyl 3-(2-oxoethyl)azetidine-1-carboxylate (500 mg, crude) as a colourless liquid.

$^1H$ NMR (400 MHz, $CDCl_3$) δ ppm 9.78 (s, 1H), 4.13 (t, *J* = 8.4 Hz, 2H), 3.57 (dd, *J* = 8.8, 5.6 Hz, 2H), 2.99 - 2.87 (m, 1H), 2.86 - 2.80 (m, 2H), 1.44 (s, 9H).

**Step b.** To a solution of *tert*-butyl 3-(2-oxoethyl)azetidine-1-carboxylate (500 mg, 2.51 mmol) in DCM (10 mL) was added ethyl 2-(triphenyl-$\lambda^5$-phosphaneylidene)acetate (CAS: 1099-45-2; 962 mg, 2.76 mmol). The mixture was stirred at 20 °C for 16 h. Upon completion, the reaction mixture was filtered and evaporated. The residue was purified by column chromatography (E/EtOAc = 5/1) to give *tert*-butyl (*E*)-3-(4-ethoxy-4-oxobut-2-en-1-yl)azetidine-1-carboxylate (440 mg, 63% yield) as a colourless liquid.

$^1H$ NMR (400 MHz, $CDCl_3$) δ ppm 6.90 - 6.82 (m, 1H), 5.80 (d, *J* = 15.6 Hz, 1H), 4.23 - 4.16 (m, 2H), 4.06 - 4.02 (t, *J* = 8.4 Hz, 2H), 3.60 - 3.55 (m, 2H), 2.71 - 2.60 (m, 1H), 2.50 (t, *J* = 6.8 Hz, 2H), 1.44 (s, 9H), 1.30 (t, *J* = 7.2 Hz, 3H).

**Step c.** To a solution of *tert*-butyl (*E*)-3-(4-ethoxy-4-oxobut-2-en-1-yl)azetidine-1-carboxylate (440 mg, 1.63 mmol) in EtOH (5 mL) was added 10% Pd/C (50 mg). The mixture was stirred at 25 °C for 16 h under a $H_2$ atmopshere (50 psi). Upon completion, the reaction mixture was filtered and evaporated to give *tert*-butyl 3-(4-ethoxy-4-oxobutyl) azetidine-1-carboxylate (400 mg, crude) as a colourless liquid.

$^1H$ NMR (400 MHz, $CDCl_3$) δ ppm 4.17 - 4.11 (m, 2H), 4.02 - 3.97 (m, 2H), 3.56 - 3.52 (m, 2H), 2.30 (t, *J* = 6.8 Hz, 2H), 1.67 - 1.58 (m, 5H), 1.44 (s, 9H), 1.27 (t, *J* = 7.2 Hz, 3H).

**Step d.** This step was conducted in a similar manner to **Intermediate B3,** step b.

$^1H$ NMR (400 MHz, $CDCl_3$) δ ppm 9.78 (t, *J* = 1.6 Hz, 1H), 4.01 (t, *J* = 8.4 Hz, 2H), 3.54 (dd, *J* = 8.8, 5.6 Hz, 2H), 2.47 (m, 3H), 1.65 - 1.55 (m, 4H), 1.44 (s, 9H).

**Intermediate B31: tert-butyl 3-((2-oxoethoxy)methyl)azetidine-1-carboxylate**

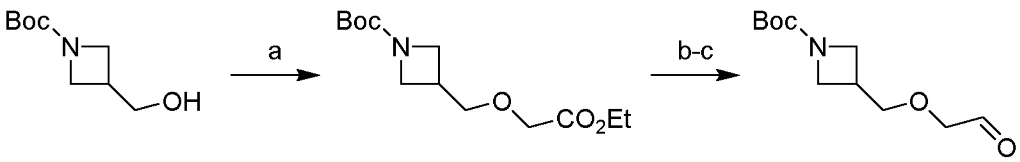


**Step a.** This step was conducted in a similar manner to **Intermediate B27,** step a, using *tert*-butyl 3-(hydroxymethyl)azetidine-1-carboxylate (CAS: 142253-56-3)

**Step b.** To a solution of *tert*-butyl 3-((2-ethoxy-2-oxoethoxy)methyl)azetidine-1-carboxylate (300 mg, 1.10 mmol) in THF (3 mL) was added $LiBH_4$ (72 mg, 3.29 mmol) at 0 °C. The mixture was stirred at 0-20 °C for 16 h. Upon completion, the reaction mixture was quenched with sat. aq. $NH_4Cl$ (1 mL) at 0 °C, filtered and evaporated to give *tert*-butyl 3-((2-hydroxyethoxy)methyl)azetidine-1-carboxylate (210 mg, crude) as a colourless oil.

m/z ES+ $[M+H]^+$ 232.2; $^1H$ NMR (400 MHz, $CDCl_3$) δ ppm 4.01 (t, *J* = 8.4 Hz, 2H), 3.77 - 3.72 (m, 2H), 3.68 - 3.64 (m, 2H), 3.63 (d, *J* = 6.8 Hz, 2H), 3.60 - 3.56 (m, 2H), 2.83 - 2.70 (m, 1H), 1.95 (br s, 1H), 1.45 (s, 9H).

**Step c.** To a solution of oxalyl chloride (346 mg, 2.72 mmol) in DCM (10 mL) was added DMSO (426 mg, 5.45 mmol) in DCM (5 mL) at -78 °C. The mixture was stirred at -70 °C for 15 min. A mixture of *tert*-butyl 3-((2-hydroxyethoxy) methyl)azetidine-1-carboxylate (210 mg, 0.91 mmol) in DCM (5 mL) was then added and the mixture was stirred at -70 °C

for a further 2 h 30 min. After which, TEA (827 mg, 8.17 mmol) was added and the reaction mixture was stirred at -70 °C for a further 30 min. Upon completion, the reaction mixture was diluted with water (5 mL) and extracted with DCM (3 x 5 mL). The combined organic layers were dried over $Na_2SO_4$ and evaporated to give the title compound (180 mg, crude) as a light yellow oil. $^1H$ NMR (400 MHz, $CDCl_3$) δ ppm 9.72 (s, 1H), 4.12 (s, 2H), 4.06 - 3.99 (m, 2H), 3.73 - 3.68 (m, 2H), 3.14 - 3.11 (m, 2H), 2.87 - 2.76 (m, 1H), 1.44 (s, 9H).

**Intermediate B32: *tert*-butyl (3-formylbicyclo[1.1.1]pentan-1-yl)carbamate**

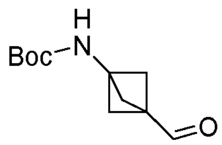

The title compound was prepared in a similar manner to to **Intermediate B3,** step b, using methyl 3-((tert-butoxycarbonyl)amino)bicyclo[1.1.1]pentane-1-carboxylate (CAS: 676371-64-5).

$^1H$ NMR (400 MHz, $CDCl_3$) δ ppm 9.67 (s, 1H), 5.00 (br s, 1H), 2.29 (s, 6H), 1.46 (s, 9H).

**Intermediate B33: *tert*-butyl 2-formyl-6,7-dihydropyrazolo[1,5-*a*]pyrazine-5(4*H*)-carboxylate**

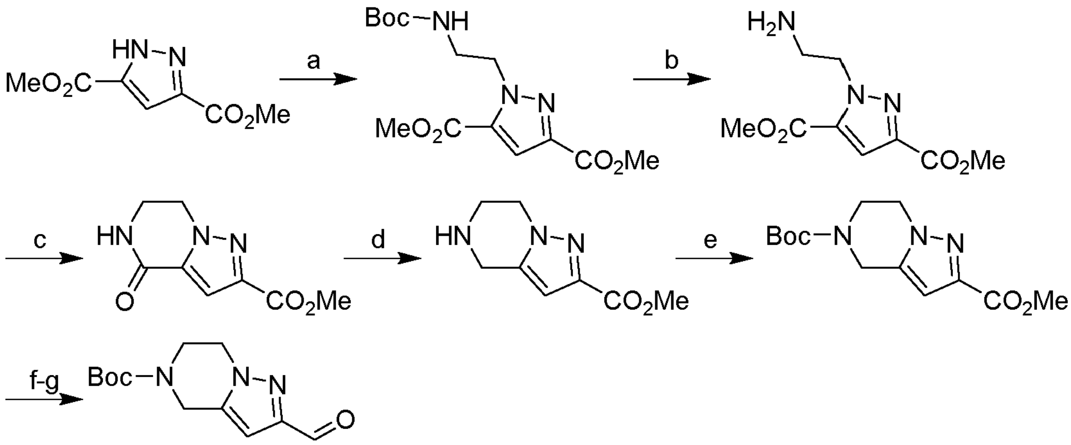


**Step a**. A mixture of dimethyl 1*H*-pyrazole-3,5-dicarboxylate (5.0 g, 27.2 mmol), *tert*-butyl (2-bromoethyl) carbamate (7.30 g, 32.6 mmol) and $K_2CO_3$ (5.6 g, 40.7 mmol) in DMF (100 mL) was stirred at 0 °C for 2 h under a $N_2$ atmosphere. Upon completion, the reaction mixture was quenched with water (100 mL) and filtered. The filter cake was washed with water (50 mL) to give dimethyl 1-(2-((*tert*-butoxycarbonyl)amino)ethyl)-1*H*-pyrazole-3,5-dicarboxylate (8.50 g, 96% yield) as a yellow solid.

m/z ES+ $[M+H]^+$ 328.1; $^1H$ NMR (400 MHz, $CDCl_3$) δ ppm 7.38 (s, 1H), 4.79 - 4.75 (m, 3H), 3.95 (s, 3H), 3.93 (s, 3H), 3.64 (d, *J* = 4.0 Hz, 2H), 1.39 (s, 9H).

**Step b.** A mixture of dimethyl 1-(2-((*tert*-butoxycarbonyl)amino)ethyl)-1*H*-pyrazole-3,5-dicarboxylate (1 g, 3.06 mmol) and TFA (6.97 g, 61.1 mmol) in DCM (10 mL) was stirred at 25 °C for 1 h under a $N_2$ atmosphere. Upon completion, the mixture was evaporated to give dimethyl 1-(2-aminoethyl)-1*H*-pyrazole-3,5-dicarboxylate as a TFA salt (1.0 g, 96% yield) as a yellow oil.

m/z ES+ $[M+H]^+$ 228.0.

**Step c.** A mixture of dimethyl 1-(2-aminoethyl)-1*H*-pyrazole-3,5-dicarboxylate (TFA salt, 1.0 g, 2.93 mmol) and TEA (2.97 g, 29.3 mmol, 4.08 mL) in MeCN (100 mL) was stirred at 60 °C for 12 h under a $N_2$ atmosphere. Upon completion, the reaction mixture was diluted with water (100 mL) and filtered to give methyl 4-oxo-4,5,6,7-tetrahydropyrazolo[1,5-*a*] pyrazine-2-carboxylate (500 mg, 87% yield) as a white solid.

m/z ES+ $[M+H]^+$ 196.1; $^1H$ NMR (400 MHz, DMSO-$d_6$) δ ppm 8.43 (s, 1H), 7.10 (s, 1H), 4.40 (s, 2H), 3.82 (s, 3H), 3.64 (s, 2H).

**Step d.** A mixture of methyl 4-oxo-4,5,6,7-tetrahydropyrazolo[1,5-*a*]pyrazine-2-carboxylate (500 mg, 2.56

mmol) and a solution of borane dimethyl sulfide complex (10 M in THF, 1.28 mL) in THF (5 mL) was stirred at 25 °C for 12 h under a $N_2$ atmosphere. Upon completion, the reaction mixture was quenched with MeOH (10 mL) at 0 °C and stirred for a further 2 h. The mixture was evaporated to give methyl 4,5,6,7-tetrahydropyrazolo[1,5-*a*]pyrazine-2-carboxylate (0.5 g, crude) as a yellow oil.

m/z ES+ $[M+H]^+$ 182.1.

**Step e.** To a solution of methyl 4,5,6,7-tetrahydropyrazolo[1,5-a]pyrazine-2-carboxylate (8.0 g, 44.1 mmol,) in DCM (80 mL) was added $Boc_2O$ (14.4 g, 66.2 mmol) and TEA (13.4 g, 132 mmol). The reaction mixture was stirred at 25 °C for 1 h. Upon completion, the mixture was diluted with water (100 mL) and extracted with EtOAc (3 x 40mL). The combined organic layers were dried over $Na_2SO_4$ and evaporated. The residue was purified by column chromatography (PE/EtOAc = 1/1) to give 5-(*tert*-butyl) 2-methyl 6,7-dihydropyrazolo[1,5-a]pyrazine-2,5(4*H*)-dicarboxylate (2.5 g, 19% yield) as a white solid.

m/z ES+ $[M+H]^+$ 282.1; $^1H$ NMR (400 MHz, $CDCl_3$) δ ppm 6.63 (s, 1H), 4.68 (s, 2H), 4.26 (t, *J* = 5.6 Hz, 2H), 3.93 (s, 4H), 3.92 - 3.89 (m, 1H), 1.50 (s, 9H).

**Steps f-g.** These 2 steps were conducted in a similar manner to **Intermediate B5,** steps b-c. m/z ES+ $[M+H]^+$251.8.

**Intermediate B34: *tert*-butyl ((3-formylbicyclo[1.1.1]pentan-1-yl)methyl)carbamate**

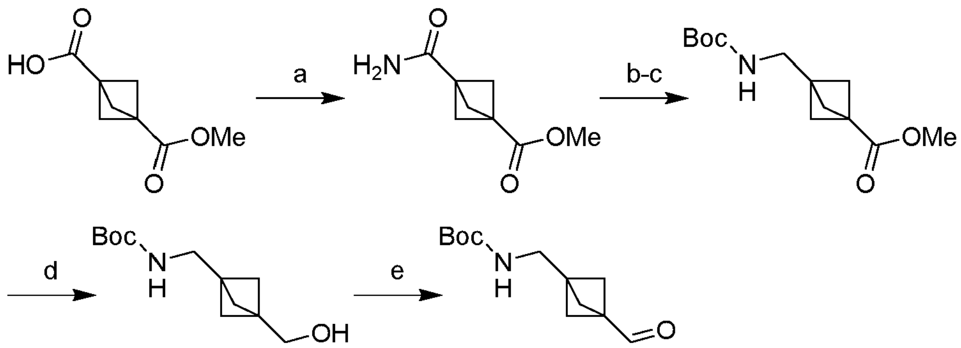


**Step a**. Oxalyl chloride (4.48 g, 35.3 mmol) and DMF (380 mg, 5.20 mmol) were added to a solution of 3-(methoxycarbonyl)bicyclo[1.1.1]pentane-1-carboxylic acid (CAS: 83249-10-9; 5.00 g, 29.4 mmol) in DCM (50 mL). The mixture was stirred at 25 °C for 2 h. Upon completion, the reaction mixture was evaporated to give the acid chloride (5.5 g, crude) as a white solid. Ammonia gas was bubbled through DCM (40 mL) for 30 mins, after which, a solution of the acid chloride in DCM (10 mL) was added. The reaction mixture was stirred at 25 °C for 2 h. Upon completion, the reaction mixture was diluted with water (50 mL) and extracted with EtOAc (3 x 20 mL). The combined organic layers were dried over $Na_2SO_4$, filtered and evaporated. The residue was purified by column chromatography (PE/EtOAc = 1/1) to give methyl 3-carbamoylbicyclo[1.1.1]pentane-1-carboxylate (3.7 g, 75% yield) as a white solid.

$^1H$ NMR (400 MHz, DMSO-$d_6$) δ ppm 7.35 (s, 1H), 7.04 (s, 1H), 3.60 (s, 3H), 2.12 (s, 6H).

**Step b.** To a mixture of methyl 3-carbamoylbicyclo[1.1.1]pentane-1-carboxylate (1.5 g, 8.9 mmol) in THF (20 mL) was added a solution of borane dimethyl sulfide complex (10 M in THF, 2.66 mL). The reaction mixture was stirred at 25 °C for 4 h under a $N_2$ atmosphere. Upon completion, the reaction mixture was quenched with MeOH (5 mL) at 0 °C, filtered and evaporated to give methyl 3-(aminomethyl)bicyclo[1.1.1]pentane-1-carboxylate (1.4 g, crude) as a white solid which was used without further purification.

**Step c.** To a solution of methyl 3-(aminomethyl)bicyclo[1.1.1]pentane-1-carboxylate (1.4 g, 9.02 mmol) in THF (10 mL) and water (10 mL) was added TEA (2.74 g, 27.1 mmol) and $Boc_2O$ (2.95 g, 13.5 mmol). The mixture was stirred at 25 °C for 16 h. Upon completion, the reaction mixture was diluted with water (30 mL) and extracted with EtOAc (3 x 50 mL). The combined organic layers were dried over $Na_2SO_4$, and evaporated. The residue was purified by column chromatography (PE/EtOAc = 3/1) to give methyl 3-(((*tert*-butoxycarbonyl)amino)methyl)bicyclo[1.1.1]pentane-1-carboxylate (400 mg, 17% yield) as a light yellow oil.

$^1H$ NMR (400 MHz, $CDCl_3$) δ ppm 4.54 (br s, 1H), 3.66 (s, 3H), 3.26 - 3.17 (m, 2H), 1.95 (s, 6H), 1.43 (s, 9H).

**Step d.** This step was conducted in a similar manner to **Intermediate B1,** step b.

$^1H$ NMR (400 MHz, $CDCl_3$) δ ppm 4.68 (s, 1H), 4.51 (br s, 1H), 3.59 (s, 2H), 3.20 (d, *J* = 5.6 Hz, 2H), 1.61 (s, 6H), 1.44 (s, 9H).

**Step e.** To a solution of oxalyl chloride (212 mg, 1.67 mmol) in DCM (3 mL) was added DMSO (261 mg, 3.34 mmol) in DCM (3 mL) at -78 °C. The mixture was stirred at -60 °C for 15 min, A mixture of methyl 3-(((*tert*-butoxycarbonyl)amino)methyl)bicyclo[1.1.1]pentane-1-carboxylate (190 mg, 0.84 mmol) in DCM (5 mL) was then added. The mixture was stirred at -60 °C for a further 2 h 30 min. After which, TEA (507 mg, 5.02 mmol) was added and the reaction mixture was stirred at -60 °C for a further 30 min. Upon completion, the reaction mixture was diluted with water (15 mL) and extracted with DCM (3 x 10 mL). The combined organic layers were dried over $Na_2SO_4$ and evaporated to give the title compound (180 mg, crude) as a colourless oil which was used without further purification.

$^1H$ NMR (400 MHz, $CDCl_3$) δ ppm 9.57 (s, 1H), 4.54 (br s, 1H), 3.23 (d, *J* = 5.6 Hz, 2H), 1.95 (s, 6H), 1.45 (s, 9H).

**Intermediate B35: 2-(2-(1,3-dioxoisoindolin-2-yl)ethoxy)acetaldehyde**

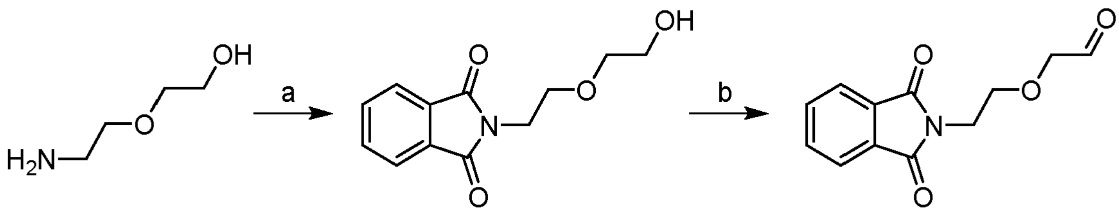


**Step a**. To a solution of 2-(2-aminoethoxy)ethanol (CAS: 929-06-6; 10.0 g, 95.1 mmol) in toluene (100 mL) was added isobenzofuran-1,3-dione (14.1 g, 95.1 mmol). The reaction mixture was stirred at 110 °C for 4 h. Upon completion, the reaction mixture was evaporated. The residue was triturated with PE/EtOAc (5/1; 40 mL) and filtered to give 2-(2-(2-hydroxyethoxy)ethyl)isoindoline-1,3-dione (21 g, 92% yield) as a white solid.

$^1H$ NMR (400 MHz, $CDCl_3$) δ ppm 7.88 - 7.82 (m, 2H), 7.76 - 7.69 (m, 2H), 3.95 - 3.87 (m, 2H), 3.79 - 3.72 (m, 2H), 3.71 - 3.66 (m, 2H), 3.63 - 3.56 (m, 2H), 2.80 (br s, 1H).

**Step b.** To a solution of oxalyl chloride (3.24 g, 25.5 mmol) in DCM (30 mL) was added a solution of DMSO (3.99 g, 51.0 mmol) in DCM (5 mL) dropwise at -78 °C. After stirring for 10 min, a solution of 2-(2-(2-hydroxyethoxy)ethyl) isoindoline-1,3-dione (2 g, 8.50 mmol) in DCM (20 mL) was added dropwise. The mixture was stirred at -78 °C for 30 min, after which TEA (7.74 g, 76.5 mmol) was added. The reaction mixture was stirred at -78 °C for a further 30 min. Upon completion, the reaction mixture was warmed to 20 °C and water (50 mL) was added. The layers were separated and the organic layer was washed with brine (50 mL), dried over $Na_2SO_4$ and evaporated to give the title compound (1.5 g, 76% yield) as a yellow oil.

$^1H$ NMR (400 MHz, $CDCl_3$) δ ppm 9.66 (s, 1H), 7.93 - 7.78 (m, 2H), 7.77 - 7.62 (m, 2H), 4.14 - 4.12 (m, 1H), 4.00 - 3.94 (m, 1H), 3.94 - 3.86 (m, 1H), 3.85 - 3.81 (m, 1H), 3.80 - 3.65 (m, 1H), 3.63 - 3.39 (m, 1H).

**Intermediate B36: 5-(1,3-dioxoisoindolin-2-yl)pentanal**

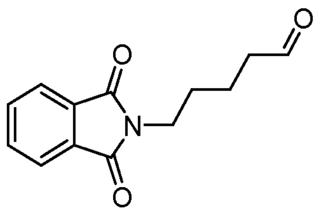

The title compound was prepared in a similar manner to **Intermediate B35,** using 5-aminopentan-1-ol (CAS: 2508-29-4) in step a.

m/z ES+ $[M+H]^+$232.1 $^1H$ NMR (400 MHz, $CDCl_3$) δ ppm 9.77 (t, *J* = 1.6 Hz, 1H), 7.89 - 7.82 (m, 2H), 7.76 - 7.70 (m, 2H), 3.72 (t, *J* = 6.8 Hz, 2H), 2.54 - 2.50 (m, 2H), 1.79 - 1.65 (m, 4H).

**Intermediate B37: 3-methoxy-4-nitrobenzaldehyde**

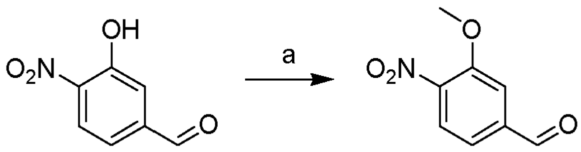


**Step a**. To a solution of 3-hydroxy-4-nitrobenzaldehyde (CAS: 704-13-2; 1.00 g, 6.0 mmol) in DMF (12 mL) was added $K_2CO_3$ (910 mg, 6.6 mmol) and MeI (1.70 g, 12.0 mmol). The mixture was stirred at 20 °C for 2 h. Upon completion, the reaction mixture was diluted with water (50 mL) and extracted with EtOAc (3 x 50 mL). The combined organic layers were washed with water (2 x 100 mL), brine (2 x 200 mL), dried over $Na_2SO_4$ and evaporated. The residue was purified by column chromatography (PE/EtOAc = 6/1) to give the title compound (1.1 g, 99% yield) as a yellow solid.
$^1H$ NMR (400 MHz, $CDCl_3$) δ ppm 10.07 (s, 1H), 7.94 (d, *J* = 8.0 Hz, 1H), 7.61 (d, *J* = 1.2 Hz, 1H), 7.55 (dd, *J* = 8.0, 1.6 Hz, 1H), 4.05 (s, 3H).

**Intermediate B38: *tert*-butyl 4-fluoro-4-formylpiperidine-1-carboxylate**

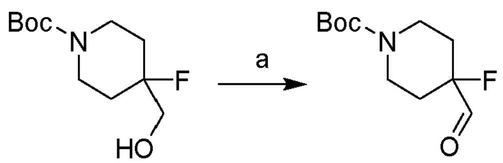


**Step a.** To a solution of oxalyl chloride (1.63 g, 12.9 mmol) in DCM (5 mL) was added DMSO (2.01 g, 25.7 mmol) in DCM (5 mL) at -78 °C. The mixture was stirred at -70 °C for 15 min, after which, a mixture of *tert*-butyl 4-fluoro-4-(hydroxymethyl)piperidine-1-carboxylate (CAS: 614730-97-1; 1.5 g, 6.43 mmol) in DCM (5 mL) was added and the mixture was stirred at - 70 °C for a further 2h 30 min. After this, TEA (3.90 g, 38.6 mmol) was added and the reaction mixture was stirred at -70 °C for a further 30 min. Upon completion, the reaction mixture was diluted with water (40 mL) and extracted with DCM (2 x 20mL). The combined organic layers were washed with brine (3 x 20 mL), dried over with $Na_2SO_4$ and evaporated. The residue was purified by column chromatography (PE/EtOAc = 3/1) to give the title compound (1.1 g, 74% yield) as a light yellow oil.
$^1H$ NMR (400 MHz, $CDCl_3$) δ ppm 9.73 (d, *J* = 5.2 Hz, 1H), 4.05 - 3.95 (m, 2H), 3.17 - 2.97 (m, 2H), 1.94 - 1.79 (m, 2H), 1.73 - 1.55 (m, 2H), 1.46 (s, 9H).

**Intermediate B39: tert-butyl 3-((*tert*-butyldiphenylsilyl)oxy)-4-formylpiperidine-1-carboxylate**

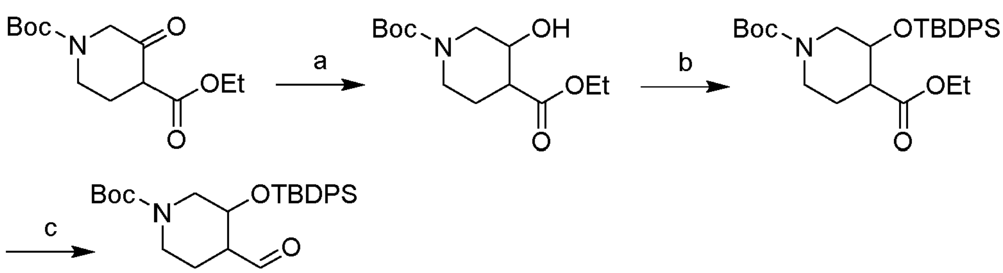


**Step a.** To a solution of *1*-(*tert*-butyl) 4-ethyl 3-oxopiperidine-1,4-dicarboxylate (CAS: 71233-25-5; 10 g, 36.9 mmol) in EtOH (60 mL) was added $NaBH_4$ (697 mg, 18.4 mmol). The reaction mixture was stirred at 20 °C for 2 h. Upon completion, the reaction mixture was slowly poured into sat. aq. $NH_4Cl$ (50 mL) and extracted with EtOAc (3 x 50 mL). The combined organic layers were washed with brine (50 mL), dried over $Na_2SO_4$ and evaporated. The crude product was purified by column chromatography (PE/EtOAc = 5/1) to give 1-(*tert*-butyl) 4-ethyl 3-hydroxypiperidine-1,4-dicarboxylate (7.5 g, 74% yield) as a colourless oil.
$^1H$ NMR (400MHz, $CDCl_3$) δ ppm 4.31 - 4.15 (m, 3H), 4.07 - 3.97 (m, 2H), 3.04 - 2.91 (m, 1H), 2.89 - 2.79 (m, 1H), 2.78 - 2.59 (m, 1H), 2.58 - 2.49 (m, 1H), 2.12 - 1.99 (m, 1H), 1.79 - 1.68 (m, 1H), 1.48 - 1.45 (m, 9H), 1.30 - 1.25 (m, 3H).
**Step b.** To a solution of 1-(*tert*-butyl) 4-ethyl 3-hydroxypiperidine-1,4-dicarboxylate (1.5 g, 5.49 mmol) and imidazole (1.12 g, 16.46 mmol) in DCM (15 mL) was added TBDPSCl (1.96 g, 7.13 mmol) at 0 °C. The reaction mixture was

stirred at 10 °C for 12 h. Upon completion, the mixture was filtered and evaporated. The residue was purified by reverse phase flash chromatography (water (0.1% FA)/MeCN) to give 1-(*tert*-butyl) 4-ethyl 3-((*tert*-butyldiphenylsilyl)oxy)piperidine-1,4-dicarboxylate (1.9 g, 64% yield) as a yellow oil.

m/z ES+ $[M+H]^+$ 512.2.

**Step c.** This step was conducted in a similar manner to **Intermediate B3,** step b.

$^1H$ NMR (400MHz, $CDCl_3$) δ ppm 9.44 - 9.40 (m, 1H), 7.69 - 7.53 (m, 5H), 7.44 - 7.25 (m, 5H), 4.27 - 4.12 (m, 1H), 3.87 - 3.53 (m, 2H), 3.26 - 2.99 (m, 1H), 2.95 - 2.75 (m, 1H), 2.45 - 2.20 (m, 1H), 1.80 - 1.45 (m, 2H), 1.30 - 1.23 (m, 9H), 1.06 - 0.94 (m, 9H).

**Intermediate B40: *tert*-butyl ((5-formylpyridin-2-yl)methyl)carbamate**

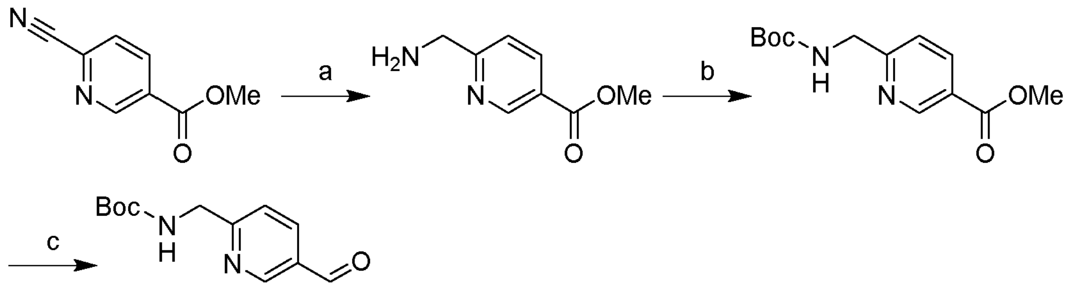


**Step a.** To a solution of methyl 6-cyanonicotinate (CAS: 89809-65-4; 1 g, 6.17 mmol) and 10% Pd/C (0.2 g) in MeOH (20 mL) was added HCl (729 mg, 7.40 mmol). The reaction mixture was stirred at 20 °C for 15 h under a $H_2$ atmosphere (15 psi). Upon completion, the mixture was filtered and the filtrate was evaporated to give methyl 6-(aminomethyl)nicotinate as the HCl salt (1.2 g, 96% yield) as a yellow oil.

$^1H$ NMR (400 MHz, DMSO-$d_6$) δ ppm 9.07 (d, *J* = 1.6 Hz, 1H), 8.73 (br s, 3H), 8.38 - 8.30 (m, 1H), 7.70 (d, *J* = 8.4 Hz, 1H), 4.30 - 4.20 (m, 2H), 3.89 (s, 3H).

**Step b.** To a solution of methyl 6-(aminomethyl)nicotinate hydrochloride (1.2 g, 5.92 mmol) and TEA (1.80 g, 17.8 mmol) in MeOH (20 mL) was added $Boc_2O$ (1.94 g, 8.88 mmol). The reaction mixture was stirred at 20 °C for 12 h. Upon completion, the mixture was evaporated, water (50 mL) was added and the aqueous mixuture was extracted with EtOAc (2 x 50 mL). The combined organic layers were evaporated. The residue was purified by column chromatography (PE/EtOAc = 6/1) to give methyl 6-(((*tert*-butoxycarbonyl)amino)methyl)nicotinate (1.4 g, 89% yield) as a white solid.

$^1H$ NMR (400 MHz, $CDCl_3$) δ ppm 9.15 (d, *J* = 2.0 Hz, 1H), 8.41 - 8.29 (m, 1H), 7.46 (d, *J* = 8.0 Hz, 1H), 5.66 (br s, 1H), 4.56 (d, *J* = 5.2 Hz, 2H), 3.97 (s, 3H), 1.46 (s, 9H).

**Step c.** This step was conducted in a similar manner to **Intermediate B3,** step b.

$^1H$ NMR (400 MHz, $CDCl_3$) δ ppm 10.11 (s, 1H), 9.00 (d, *J* = 1.6 Hz, 1H), 8.16 (dd, *J* = 8.0, 2.0 Hz, 1H), 7.47 (d, *J* = 8.0 Hz, 1H), 5.55 (br s, 1H), 4.55 (d, *J* = 5.2 Hz, 2H), 1.48 (s, 9H).

**Intermediate B41: *tert*-butyl (*R*)-3-(2-oxoethyl)pyrrolidine-1-carboxylate**

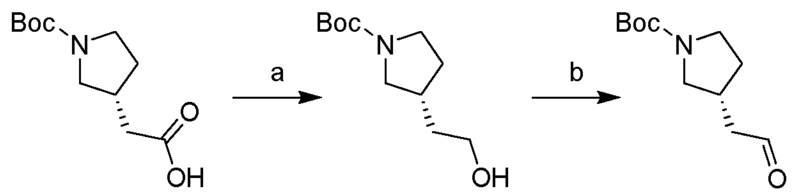


**Step a**. To a solution of (*R*)-2-(1-(*tert*-butoxycarbonyl)pyrrolidin-3-yl)acetic acid (CAS: 204688-60-8; 1.00 g, 4.36 mmol) in THF (10 mL) was added a solution of borane tetrahydrofuran complex (1 M in THF, 5.23 mL) at 0 °C. The reaction mixture was stirred at 20 °C for 1 h. Upon completion, the reaction mixture was quenched with 10% aq. NaOH (2 mL). The reaction mixture was diluted with water (30 mL) and extracted with EtOAc (30 mL). The organic layer was dried over $Na_2SO_4$ and evaporated to give *tert*-butyl (*R*)-3-(2-hydroxyethyl)pyrrolidine-1-carboxylate (0.93 g, 99% yield) as a colourless oil.

$^1H$ NMR (400 MHz, $CDCl_3$) δ ppm 3.74 - 3.66 (m, 2H), 3.60 - 3.52 (m, 1H), 3.50 - 3.41 (m, 1H), 3.31 - 3.21 (m, 1H),

2.94 - 2.86 (m, 1H), 2.34 - 2.19 (m, 1H), 2.07 - 1.97 (m, 1H), 1.71 - 1.63 (m, 2H), 1.58 - 1.49 (m, 1H), 1.46 (s, 9H), 1.45 - 1.42 (m, 1H).

**Step b**. To a solution of *tert*-butyl (*R*)-3-(2-hydroxyethyl)pyrrolidine-1-carboxylate (0.3 g, 1.39 mmol) in DCM (3 mL) was added Dess-Martin periodinane (1.18 g, 2.79 mmol). The reaction mixture was stirred at 20 °C for 1 h. Upon completion, the pH of the reaction mixture was adjusted to ~pH 9 with sat. aq. $NaHCO_3$ (20 mL). The layers were separated and the organic layer was washed with brine (20 mL), dried over $Na_2SO_4$, evaporated and purified by column chromatography (PE/EtOAc = 3/1) to give the title compound (150 mg, 50% yield) as a colourless oil.

$^1H$ NMR (400 MHz, $CDCl_3$) δ ppm 9.79 (s, 1H), 3.62 - 3.56 (m, 1H), 3.50 - 3.42 (m, 1H), 3.34 - 3.30 (m, 1H), 3.00 - 2.85 (m, 1H), 2.68 - 2.57 (m, 3H), 2.11 - 2.08 (m, 1H), 1.56 - 1.49 (m, 1H), 1.48 (s, 9H).

**Intermediate B42: *tert*-butyl (*S*)-3-(2-oxoethyl)pyrrolidine-1-carboxylate**

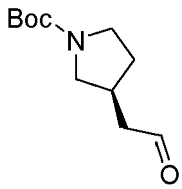

The title compound was prepared in a similar manner to **Intermediate B41,** using *tert*-butyl (S)-3-(hydroxymethyl)pyrrolidine-1-carboxylate (CAS: 204688-61-9) in step a.

$^1H$ NMR (400 MHz, $CDCl_3$) δ ppm 9.80 (s, 1H), 3.65 - 3.29 (m, 3H), 3.02 - 2.85 (m, 1H), 2.66 - 2.54 (m, 3H), 2.12 - 2.07 (m, 1H), 1.56 - 1.50 (m, 1H), 1.46 (s, 9H).

**Intermediate B43: tert-butyl (*S*)-3-formylpyrrolidine-1-carboxylate**

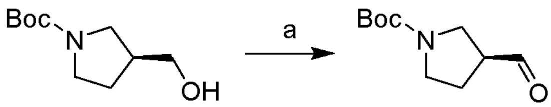


**Step a.** This step was conducted in a similar manner to **Intermediate B41,** step b, using *tert*-butyl (*S*)-3-(hydroxymethyl)pyrrolidine-1-carboxylate (CAS: 199174-24-8).

$^1H$ NMR (400MHz, $CDCl_3$) δ ppm 9.70 (s, 1H), 3.80 - 3.63 (m, 1H), 3.59 - 3.46 (m, 1H), 3.43 - 3.40 (m, 2H), 3.10 - 2.95 (m, 1H), 2.29 - 2.07 (m, 2H), 1.47 (s, 9H).

**Intermediate B44: tert-butyl (*R*)-3-formylpyrrolidine-1-carboxylate**

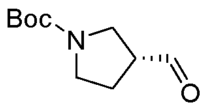

The title compound was prepared in a similar manner to **Intermediate B43,** using *tert-butyl* (*R*)-3-(hydroxymethyl)pyrrolidine-1-carboxylate (CAS: 138108-72-2).

$^1H$ NMR (400MHz, $CDCl_3$) δ ppm 9.69 (s, 1H), 3.79 - 3.63 (m, 1H), 3.55 - 3.45 (m, 1H), 3.43 - 3.30 (m, 2H), 3.10 - 2.95 (m, 1H), 2.20 - 2.04 (m, 2H), 1.47 (s, 9H).

**Intermediate B45: *tert*-butyl 4-(3-oxopropyl)piperidine-1-carboxylate**

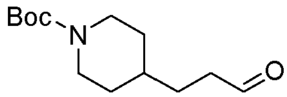

The title compound was prepared in a similar manner to **Intermediate B43,** using *tert*-butyl 4-(3-hydroxypropyl) piperidine-1-carboxylate (CAS: 156185-63-6).

$^1$H NMR (400MHz, $CDCl_3$) δ ppm 9.79 (t, *J* = 1.6 Hz, 1H), 4.17 - 4.00 (m, 2H), 2.70 - 2.62 (m, 2H), 2.52 - 2.43 (m, 2H), 1.68 - 1.56 (m, 4H), 1.45 (s, 9H), 1.44 - 1.34 (m, 1H), 1.13 - 1.08 (m, 2H).

**Intermediate B46: benzyl 4-(1-oxopropan-2-yl)piperazine-1-carboxylate**

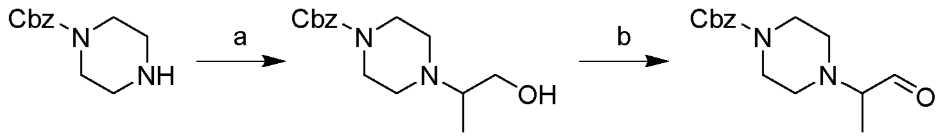


**Step a.** To a solution of benzyl piperazine-1-carboxylate (3.00 g, 13.6 mmol) and 1-hydroxypropan-2-one (1.11 g, 15.0 mmol) in MeOH (40 mL) was added acetic acid (82 mg, 1.36 mmol) and 4 Å molecular sieves (1 g). The mixture was stirred at 15 °C for 30 min, after which, $NaBH_3CN$ (2.57 g, 40.9 mmol) was added. The reaction mixture was stirred at 15 °C for 90 min. Upon completion, the reaction mixture was filtered and the filtrate was evaporated. Sat. aq. $NaHCO_3$ (10 mL) was added and the aqueous mixture was extracted with EtOAc (3 x 20 mL). The combined organic layers were washed with brine (10 mL), dried over $Na_2SO_4$ and evaporated. The crude product was purified by column chromatography (PE /EtOAc = 5/1) to give benzyl 4-(1-hydroxypropan-2-yl)piperazine-1-carboxylate (1.7 g, 43% yield) as a yellow oil.

m/z ES+ $[M+H]^+$ 279.2; $^1$H NMR (400 MHz, $CDCl_3$) δ ppm 7.40 - 7.31 (m, 5H), 5.12 (s, 2H), 3.64 - 3.45 (m, 6H), 2.81 - 2.75 (m, 1H), 2.69 - 2.59 (m, 3H), 2.39 - 2.24 (m, 2H), 0.90 (d, *J* = 6.8 Hz, 3H).

**Step b.** DMSO (505 mg, 6.47 mmol) was added to a solution of oxalyl chloride (410 mg, 3.23 mmol) in DCM (5 mL) at -78 °C. The mixture was stirred at -78 °C for 10 min. After which, a solution of benzyl 4-(1-hydroxypropan-2-yl) piperazine-1-carboxylate (300 mg, 1.08 mmol) in DCM (2 mL) was added. The mixture was stirred at -78 °C for a further 30 min, and then TEA (1.09 g, 10.8 mmol) was added. The mixture was stirred at -78 °C for a further 60 min. Upon completion, the reaction mixture was quenched with water (30 mL) and extracted with EtOAc (3 x 25 mL). The combined organic layers were washed with brine (25 mL), dried over $Na_2SO_4$ and evaporated to give the title compound (300 mg, crude) as a yellow oil.

$^1$H NMR (400 MHz, $CDCl_3$) δ ppm 9.67 (s, 1H), 7.39 - 7.30 (m, 5H), 5.14 (s, 2H), 3.62 - 3.50 (m, 4H), 3.13 - 3.09 (m, 1H), 2.61 - 2.45 (m, 4H), 1.15 (d, *J* = 6.8 Hz, 3H).

**Intermediate B47a: 1-(tert-butyl) 4-ethyl (*rac-cis*)-3-cyanopiperidine-1,4-dicarboxylate and**

**Intermediate B47b: 1-(*tert*-butyl) 4-ethyl (*rac-trans*)-3-cyanopiperidine-1,4-dicarboxylate**

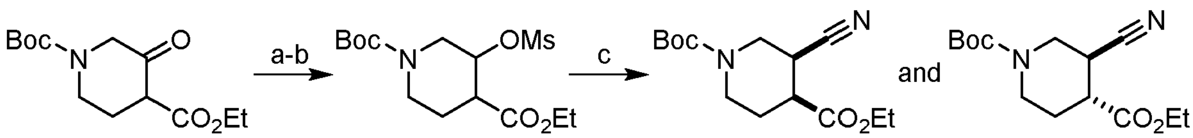


**Step a.** To a solution of 1-(*tert*-butyl) 4-ethyl 3-oxopiperidine-1,4-dicarboxylate (CAS: 71233-25-5; 10 g, 36.9 mmol) in EtOH (60 mL) was added $NaBH_4$ (697 mg, 18.4 mmol). The reaction mixture was stirred at 20 °C for 2 h. Upon completion, the reaction mixture was slowly added into sat. aq. $NH_4Cl$ (50 mL) and the aqueous mixture was extracted with EtOAc (3 x 50 mL). The combined organic layers were washed with brine (50 ml), dried over $Na_2SO_4$, evaporated and purified by column chromatography (PE/EtOAc = 5/1) to give 1-(*tert*-butyl) 4-ethyl 3-hydroxypiperidine-1,4-dicarboxylate (7.5 g, 74% yield) as a colourless oil.

$^1$H NMR (400 MHz, $CDCl_3$) δ ppm 4.31 - 4.15 (m, 4H), 4.07 - 3.97 (m, 1H), 3.04 - 2.91 (m, 1H), 2.89 - 2.79 (m, 1H), 2.78 - 2.59 (m, 1H), 2.58 - 2.49 (m, 1H), 2.12 - 1.99 (m, 1H), 1.79 - 1.68 (m, 1H), 1.48 - 1.45 (m, 9H), 1.30 - 1.25 (m, 3H).

**Step b.** To a solution of 1-(*tert*-butyl) 4-ethyl 3-hydroxypiperidine-1,4-dicarboxylate (7.5 g, 27.4 mmol) and TEA

(11.1 g, 110 mmol) in DCM (75 mL) was added MsCl (6.29 g, 54.9 mmol) at 0 °C. The reaction mixture was stirred at 20 °C for 12 h. Upon completion, the reaction mixture was diluted with water (50 mL) and extracted with DCM (100 mL). The organic layer was washed with brine (50 mL), dried over $Na_2SO_4$ and evaporated to give 1-(*tert*-butyl) 4-ethyl 3-((methylsulfonyl)oxy)piperidine-1,4-dicarboxylate (9.6 g, crude) as a yellow oil.

m/z ES+ $[M+Na]^+$ 374.0.

**Step c.** A mixture of 1-(*tert*-butyl) 4-ethyl 3-((methylsulfonyl)oxy)piperidine-1,4-dicarboxylate (9.6 g, 27.3 mmol), TBAF (1 M in THF, 41.0 mL) and trimethylsilyl cyanide (4.07 g, 41.0 mmol) in MeCN (90 mL) was stirred at 80 °C for 12 h. Upon completion, the reaction mixture was diluted with water (40 mL) and extracted with EtOAc (3 x 40 mL). The combined organic layers were washed with brine (40 mL), dried over $Na_2SO_4$ and evaporated. The residue was purified by column chromatography (PE/EtOAc = 5/1) to give two isomers.

The first eluting isomer was confirmed by 2D NMR to be **Intermediate B47b:** 1-(*tert*-butyl) 4-ethyl (*rac-trans*)-3-cyanopiperidine-1,4-dicarboxylate (1.4 g, 18% yield) as a yellow oil.

$^1H$ NMR (400 MHz, $CDCl_3$) δ ppm 4.27 - 4.21 (m, 3H), 3.91 - 3.87 (m, 1H), 3.28 - 3.10 (m, 1H), 3.09 - 3.03 (m, 1H), 3.00 - 2.92 (m, 1H), 2.78 - 2.74 (m, 1H), 2.06 - 2.04 (m, 1H), 1.70 - 1.65 (m, 1H), 1.47 (s, 9H), 1.32 - 1.27 (m, 3H).

The second eluting isomer was confirmed by 2D NMR to be **Intermediate B47a:** 1-(*tert*-butyl) 4-ethyl (*rac-cis*)-3-cyanopiperidine-1,4-dicarboxylate (5 g, crude) as a yellow oil.

$^1H$ NMR (400 MHz, $CDCl_3$) δ ppm 4.51 - 4.38 (m, 1H), 4.28 - 4.19 (m, 3H), 3.25 - 3.21 (m, 1H), 3.03 - 2.98 (m, 1H), 2.95 - 2.68 (m, 1H), 2.66 - 2.60 (m, 1H), 2.05 - 2.03 (m, 1H), 1.99 - 1.91 (m, 1H), 1.48 (s, 9H), 1.32 - 1.28 (m, 3H).

**Intermediate B48a: *tert*-butyl (*rac-cis*)-3-cyano-4-formylpiperidine-1-carboxylate**

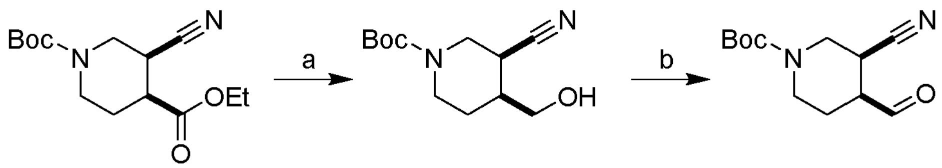


**Step a.** To a solution of **Intermediate B47a** (0.8 g, 2.83 mmol) in THF (8 mL) and MeOH (1 mL) was added $LiBH_4$ (185 mg, 8.5 mmol) and the mixture was stirred at 10 °C for 12 h. Upon completion, the mixture was quenched with sat. aq. $NH_4Cl$ (0.2 mL), diluted with EtOAc (30 mL), filtered and evaporated. The residue was purified by column chromatography (PE/EtOAc = 1/3) to give *tert*-butyl (*rac-cis*)-3-cyano-4-(hydroxymethyl)piperidine-1-carboxylate (500 mg, 73% yield) as a colourless oil.

$^1H$ NMR (400 MHz, $CDCl_3$) δ ppm 4.50 - 4.36 (m, 1H), 4.35 - 4.20 (m, 1H), 3.67 - 3.53 (m, 2H), 3.06 - 3.00 (m, 1H), 2.92 - 2.73 (m, 1H), 2.72 - 2.54 (m, 1H), 1.89 - 1.86 (m, 1H), 1.56 - 1.52 (m, 3H), 1.42 (s, 9H).

**Step b.** To a solution of *tert*-butyl (*rac-cis*)-3-cyano-4-(hydroxymethyl)piperidine-1-carboxylate (450 mg, 1.87 mmol) in DCM (10 mL) was added Dess-Martin periodinane (1.59 g, 3.75 mmol) and the mixture was stirred at 15 °C for 1 h. Upon completion, the mixture was filtered, evaporated and purified by column chromatography (PE/EtOAc = 1/1) to give the title compound (434 mg, crude) as a yellow oil which was used directly in subsequent steps.

**Intermediate B48b: *tert*-butyl (rac-trans)-3-cyano-4-formylpiperidine-1-carboxylate**

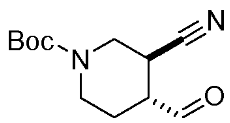

The title compound was prepared in a similar manner to **Intermediate B48a,** using **Intermediate B47b.**

$^1H$ NMR (400 MHz, $CDCl_3$) δ ppm 9.74 (s, 1H), 4.51 - 4.13 (m, 1H), 4.12 - 3.87 (m, 1H), 3.81 - 3.56 (m, 1H), 3.52 - 3.10 (m, 1H), 3.07 - 2.90 (m, 1H), 2.88 - 2.77 (m, 1H), 2.23 - 2.12 (m, 1H), 1.72 - 1.54 (m, 1H), 1.48 (s, 9H).

**Intermediate B49: benzyl 2-formyl-5,7-dihydro-6*H*-pyrrolo[3,4-*b*]pyrazine-6-carboxylate**

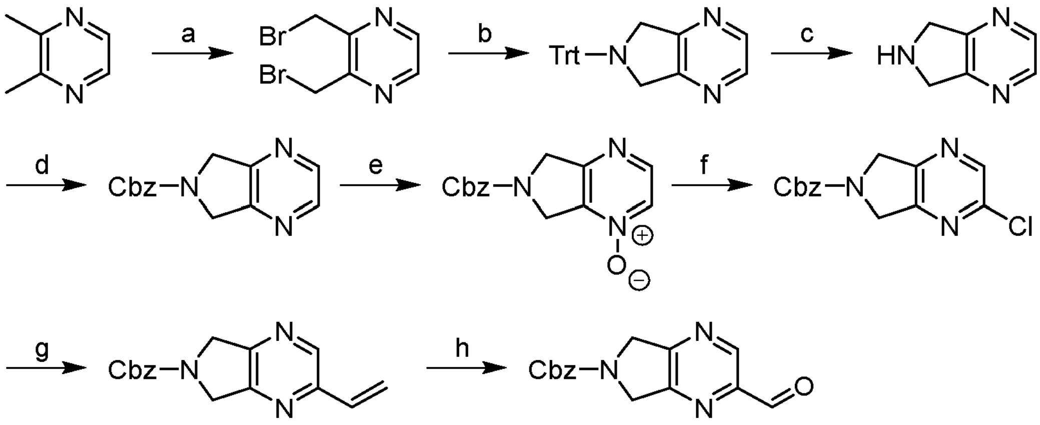


**Step** a. To a solution of 2,3-dimethylpyrazine (10 g, 92.5 mmol) in $CCl_4$ (320 mL) was added NBS (41.2 g, 231 mmol) and AIBN (106 mg, 0.65 mmol). The mixture was stirred at 80 °C for 1 h under a $N_2$ atmosphere. After 1 h, additional AIBN (106 mg, 0.65 mmol) was added and stirred at 80 °C for a further 5 h under a $N_2$ atmosphere. Upon completion, the mixture was filtered and the filter cake was washed with $CCl_4$ (100 mL). The filtrate was evaporated and purified by column chromatography (PE/EtOAc = 20/1) to give 2,3-bis(bromomethyl)pyrazine (20 g, 31% yield) as a purple oil.

m/z ES+ $[M+H]^+$266.8; $^1H$ NMR (400 MHz, $CDCl_3$) δ ppm 8.51 (s, 2H), 4.72 (s, 4H).

**Step b.** To a solution of 2,3-bis(bromomethyl)pyrazine (40 g, 116 mmol) in DMF (1500 mL) was added a solution of triphenylmethanamine (90.1 g, 347 mmol) in DMF (500 mL) at 0 °C. The mixture was stirred at 20 °C for 1 h followed by 60 °C for 12 h. Upon completion, the mixture was evaporated, diluted with water (400 mL) and extracted with EtOAc (3 x 400 mL). The combined organic layers were washed with brine (2 x 500 mL), dried over $Na_2SO_4$, evaporated and purified by column chromatography (PE/EtOAc = 7/1) followed by trituration with MeOH (100 mL) at 20 °C to give 6-trityl-6,7-dihydro-5*H*-pyrrolo[3,4-*b*]pyrazine (16 g, 37% yield) as a yellow solid.

m/z ES+ $[M+H]^+$ 364.0; $^1H$ NMR (400 MHz, $CDCl_3$) δ ppm 8.22 (s, 2H), 7.64 - 7.56 (m, 6H), 7.35 - 7.28 (m, 6H), 7.23 - 7.16 (m, 3H), 4.08 (s, 4H).

**Step c.** A solution of 6-trityl-6,7-dihydro-5*H*-pyrrolo[3,4-*b*]pyrazine (16.0 g, 44.0 mmol) in 4 M HCl in MeOH (120 mL) was stirred at 20 °C for 2 h. Upon completion, the mixture was evaporated, diluted with water (200 mL) and extracted with EtOAc (3 x 150 mL). The aqueous phase was lyophilized to give 6,7-dihydro-5*H*-pyrrolo[3,4-*b*]pyrazine hydrochloride (7 g) as a black solid.

$^1H$ NMR (400 MHz, DMSO-$d_6$) δ ppm 10.60 (br s, 2H), 8.60 (s, 2H), 4.58 - 4.48 (m, 4H).

**Step d.** To a solution of 6,7-dihydro-5*H*-pyrrolo[3,4-*b*]pyrazine hydrochloride (8.67 g, 55.0 mmol) in THF (200 mL) and water (100 mL) was added $Na_2CO_3$ (14.6 g, 138 mmol) and CbzCl (11.3 g, 66.0 mmol). The reaction mixture was stirred at 20 °C for 5 h. Upon completion, the mixture was diluted with water (100 mL) and extracted with EtOAc (3 x 200 mL). The combined organic layers were washed with brine (500 mL), dried over $Na_2SO_4$, evaporated and purified by column chromatography (PE/EtOAc = 2/1) to give benzyl 5,7-dihydro-6*H*-pyrrolo[3,4-*b*]pyrazine-6-carboxylate (7.5 g, 64% yield) as a yellow solid.

m/z ES+ $[M+H]^+$ 256.1; $^1H$ NMR (400 MHz, $CDCl_3$) δ ppm 8.46 (s, 2H), 7.45 - 7.33 (m, 5H), 5.26 (s, 2H), 4.85 - 4.83 (m, 4H).

**Step e.** To a solution of benzyl 5,7-dihydro-6*H*-pyrrolo[3,4-*b*]pyrazine-6-carboxylate (10 g, 39.2 mmol) in DCM (150 mL) was added *m*-CPBA (80%; 9.3 g, 43.1 mmol). The reaction mixture was stirred at 25 °C for 12 h. Upon completion, the mixture was diluted with sat. aq. $Na_2S_2O_3$ (150 mL) and extracted with DCM (200 mL). The combined organic layers were washed with sat. aq. $Na_2S_2O_3$ (150 mL), brine (300 mL), dried over $Na_2SO_4$, evaporated and purified by column chromatography (PE/EtOAc = 0/1) to give 6-((benzyloxy)carbonyl)-6,7-dihydro-5H-pyrrolo[3,4-b]pyrazine 1-oxide (8.3 g, 76% yield) as a yellow solid,.

m/z ES+ $[M+H]^+$ 272.0; $^1H$ NMR (400 MHz, $CDCl_3$) δ ppm 8.39 - 8.36 (m, 1H), 8.01 (d, *J* = 4.0 Hz, 1H), 7.45 - 7.32 (m, 5H), 5.24 (d, *J* = 3.2 Hz, 2H), 4.90 (s, 2H), 4.89 - 4.84 (m, 2H).

**Step f.** To a solution of 6-((benzyloxy)carbonyl)-6,7-dihydro-5*H*-pyrrolo[3,4-*b*]pyrazine 1-oxide (14.3 g, 52.7 mmol) in DMF (200 mL) was added oxalyl chloride (16.7 g, 132 mmol) dropwise at 0 °C. The reaction mixture was stirred at 40 °C for 12 h. Upon completion, the mixture was basified to pH 8 with sat. aq. $NaHCO_3$, diluted with water (500 mL) and extracted with EtOAc (3 x 500 mL). The combined organic layers were washed with brine (2 x 800 mL), dried over $Na_2SO_4$, evaporated and purified by column chromatography (PE/EtOAc = 7/1) to give benzyl 2-chloro-5,7-dihydro-6*H*-pyrrolo [3,4-*b*]pyrazine-6-carboxylate (11.6 g, 76% yield) as a yellow solid.

m/z ES+ $[M+H]^+$ 290.1; $^1H$ NMR (400 MHz, $CDCl_3$) δ ppm 8.48 (d, *J* = 4.0 Hz, 1H), 7.49 - 7.32 (m, 5H), 5.25 (s, 2H), 4.84 - 4.77 (m, 4H).

**Step g.** A mixture of benzyl 2-chloro-5,7-dihydro-6*H*-pyrrolo[3,4-*b*]pyrazine-6-carboxylate (10 g, 34.5 mmol), potassium trifluoro(vinyl)borate (CAS: 13682-77-4; 9.25 g, 69.0 mmol), Pd(dppf)$Cl_2$ (2.53 g, 3.45 mmol) and $Na_2CO_3$ (9.15 g, 86.3 mmol) in 1,4-dioxane (200 mL) and water (30 mL) was degassed and purged with $N_2$ 3 times. The reaction mixture was stirred at 80 °C for 12 h under a $N_2$ atmosphere. Upon completion, the mixture was diluted with water (250 mL) and extracted with EtOAc (3 x 250 mL). The combined organic layers were washed with brine (600 mL), dried over $Na_2SO_4$, evaporated and purified by column chromatography (PE/EtOAc = 9/1) to give benzyl 2-vinyl-5,7-dihydro-6*H*-pyrrolo[3,4-*b*]pyrazine-6-carboxylate (9.04 g, 93% yield) as an off-white solid.

m/z ES+ $[M+H]^+$282.2; $^1H$ NMR (400 MHz, $CDCl_3$) δ ppm 8.43 (s, 1H), 7.46 - 7.31 (m, 5H), 6.82 - 6.79 (m, 1H), 6.41 - 6.34 (m, 1H), 5.63 - 5.69 (m, 1H), 5.26 (s, 2H), 4.81 (s, 4H).

**Step h.** To a solution of benzyl 2-vinyl-5,7-dihydro-6*H*-pyrrolo[3,4-*b*]pyrazine-6-carboxylate (8.8 g, 31.3 mmol) in THF (150 mL) and water (150 mL) was added NaIO, (20.1 g, 93.9 mmol) and $OsO_4$ (795 mg, 3.13 mmol) at 0 °C. The reaction mixture was stirred at 0 °C for 1 h. Upon completion, the mixture was quenched with sat. aq. $Na_2S_2O_3$ (200 mL) and stirred at 25 °C for 10 min. The mixture was diluted with water (50 mL) and extracted with EtOAc (3 x 250 mL). The combined organic layers were washed with sat. aq. $Na_2S_2O_3$ (400 mL), brine (500 mL), dried over $Na_2SO_4$, evaporated and purified by column chromatography (PE/EtOAc = 3/1) to give the title compound (6.4 g, 72% yield) as a yellow solid.

m/z ES+ $[M+H]^+$284.0; $^1H$ NMR (400 MHz, $CDCl_3$) δ ppm 10.14 (d, *J* = 6.4 Hz, 1H), 9.06 (d, *J* = 4.0 Hz, 1H), 7.48 - 7.32 (m, 5H), 5.27 (s, 2H), 4.92 (d, *J* = 3.2 Hz, 4H).

**Intermediate B50a: *tert*-butyl (*R*/*S*)-7-(((*tert*-butyldimethylsilyl)oxy)methyl)-3-vinyl-5,7-dihydro-6*H*-pyrrolo[3,4-*b*]pyridine-6-carboxylate and**

**Intermediate B50b: *tert*-butyl (*S*/*R*)-7-(((*tert*-butyldimethylsilyl)oxy)methyl)-3-vinyl-5,7-dihydro-6*H*-pyrrolo[3,4-*b*]pyridine-6-carboxylate**

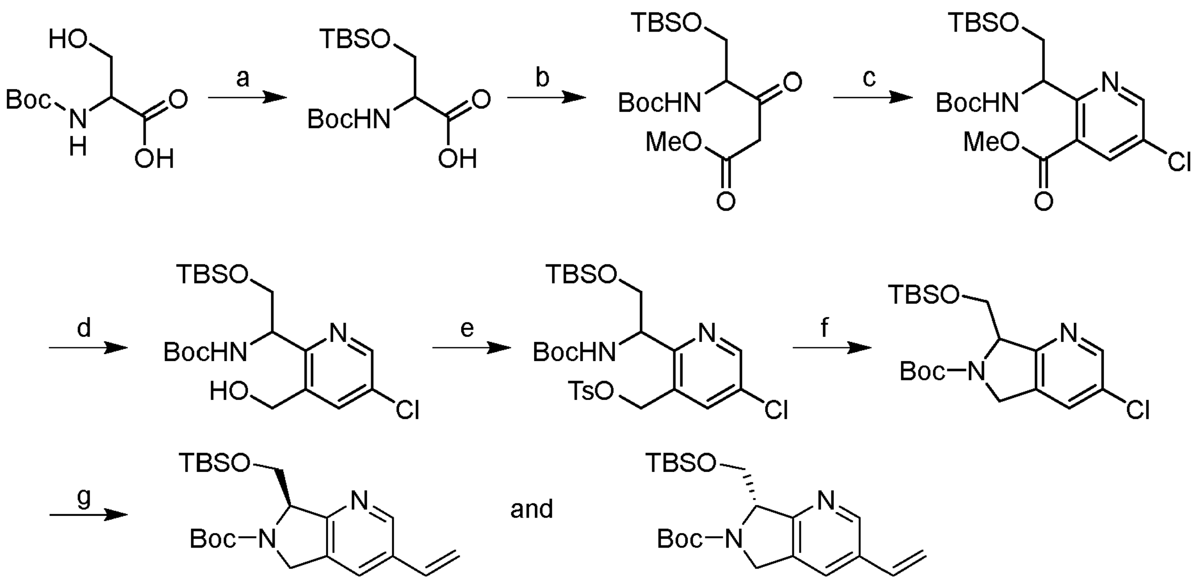


**Step a.** To a solution of (*tert*-butoxycarbonyl)serine (50 g, 244 mmol) and imidazole (33.2 g, 487 mmol) in DMF (500 mL) was added TBSCl (36.7 g, 244 mmol) at 0 °C. The reaction mixture was stirred at 20 °C for 12 h. Upon completion, the mixture was diluted with water (1.5 L) and extracted with EtOAc (3 x 1 L). The combined organic layers were washed with brine (3 x 1 L), dried over $Na_2SO_4$ and evaporated to give *N*-(*tert*-butoxycarbonyl)-*O*-(*tert*-butyldimethylsilyl)serine (64 g, 82% yield) as a colourless oil.

$^1H$ NMR (400 MHz, $CDCl_3$) δ ppm 5.49 - 5.30 (m, 1H), 4.37 - 4.21 (m, 1H), 4.10 - 4.02 (m, 1H), 3.89 - 3.81 (m, 1H), 1.46 (s, 9H), 0.89 (s, 9H), -0.06 (s, 6H).

**Step b.** A solution of *N*-(*tert*-butoxycarbonyl)-*O*-(*tert*-butyldimethylsilyl)serine (66.9 g, 209 mmol) and CDI (40.8 g, 251 mmol) in THF (670 mL) was stirred at 20 °C for 1 h, after which potassium 3-methoxy-3-oxopropanoate (32.7 g, 209 mmol) and $MgCl_2$ (19.9 g, 209 mmol) was added. The reaction mixture was stirred at 50 °C for a further 16 h. Upon completion, the mixture was diluted with water (500 mL) and extracted with EtOAc (500 mL). The organic layer was washed with brine (500 mL), dried over $Na_2SO_4$, evaporated and purified by column chromatography (PE/EtOAc = 10/1) to give methyl 4-((*tert*-butoxycarbonyl)amino)-5-((*tert*butyldimethylsilyl)oxy)-3-oxopentanoate (27.6 g, 35% yield) as a colour-

less oil.

$^1H$ NMR (400 MHz, $CDCl_3$) δ ppm 5.49 - 5.39 (m, 1H), 4.45 - 4.36 (m, 1H), 4.12 - 4.04 (m, 1H), 3.83 - 3.79 (m, 1H), 3.74 (s, 3H), 3.62 (d, *J* = 3.6 Hz, 2H), 1.46 (s, 9H), 0.88 (s, 9H), 0.06 (s, 6H).

**Step c.** To a solution of methyl 4-((*tert*-butoxycarbonyl)amino)-5-((*tert*-butyldimethylsilyl)oxy)-3-oxopentanoate (27.6 g, 73.5 mmol) in THF (280 mL) was added t-BuOK (9.07 g, 80.9 mmol) at 0 °C. The mixture was stirred at 0 °C for 45 min, after which DABCO (9.07 g, 80.9 mmol) and (*Z*)-*N*-(2-chloro-3-(dimethylamino)allylidene)-*N*-methylmethanaminium hexafluorophosphate (23.7 g, 77.2 mmol) was added at 0 °C. The mixture was stirred at 20 °C for 3 h and then ammonium acetate (6.23 g, 80.9 mmol) was added. The reaction mixture was stirred at 20 °C for a further 16 h. Upon completion, the mixture was diluted with water (500 mL) and extracted with EtOAc (3 x 300 mL). The combined organic layers were washed with brine (300 mL), dried over $Na_2SO_4$, evaporated and purified by column chromatography (PE/EtOAc = 10/1) to give methyl 5-chloro-2-(2,2,3,3,10,10-hexamethyl-8-oxo-4,9-dioxa-7-aza-3-silaundecan-6-yl)nicotinate (23 g, 69% yield) as a white solid.

m/z ES+ $[M+H]^+$ 445.1; $^1H$ NMR (400 MHz, $CDCl_3$) δ ppm 8.63 (d, *J* = 2.4 Hz, 1H), 8.19 (d, *J* = 2.4 Hz, 1H), 6.01 - 5.78 (m, 2H), 3.94 (s, 3H), 3.90 - 3.83 (m, 1H), 3.82 - 3.74 (m, 1H), 1.44 (s, 9H), 0.77 (s, 9H), -0.09 (s, 3H), -0.11 (s, 3H).

**Step d.** To a solution of methyl 5-chloro-2-(2,2,3,3,10,10-hexamethyl-8-oxo-4,9-dioxa-7-aza-3-silaundecan-6-yl)nicotinate (13 g, 29.2 mmol) in EtOH (130 mL) was added $NaBH_4$ (2.21 g, 58.4 mmol) and $CaCl_2$ (3.24 g, 29.2 mmol) at 0°C. The reaction mixture was stirred at 0 °C for 2 h. Upon completion, the mixture was quenched with sat. aq. $NH_4Cl$ (300 mL) and extracted with EtOAc (3 x 300 mL). The combined organic layers were washed with brine (300 mL), dried over $Na_2SO_4$, evaporated and purified by column chromatography (PE/EtOAc = 5/1) to give *tert*-butyl (2-((*tert*-butyldimethylsilyl)oxy)-1-(5-chloro-3-(hydroxymethyl)pyridin-2-yl)ethyl)carbamate (6.6 g, 53% yield) as a colourless oil.

m/z ES+ $[M+H]^+$417.3; $^1H$ NMR (400 MHz, $CDCl_3$) δ ppm 8.48 (d, *J* = 2.4 Hz, 1H), 7.69 (d, *J* = 2.4 Hz, 1H), 5.57 (d, *J* = 8.0 Hz, 1H), 5.18 - 5.06 (m, 1H), 4.80 - 4.66 (m, 2H), 4.02 - 3.95 (m, 1H), 3.75 - 3.62 (m, 2H), 1.42 (s, 9H), 0.75 (s, 9H), -0.05 (s, 3H), -0.11 (s, 3H).

**Step e.** To a solution of *tert*-butyl (2-((*tert*-butyldimethylsilyl)oxy)-1-(5-chloro-3-(hydroxymethyl)pyridin-2-yl) ethyl)carbamate (5.6 g, 13.4 mmol) in DCM (60 mL) was added TEA (4.08 g, 40.3 mmol) and TsCl (5.12 g, 26.9 mmol) at 0 °C. The reaction mixture was stirred at 0 °C for 1 h. Upon completion, the mixture was quenched with sat. aq. $NaHCO_3$ (100 mL) and extracted with EtOAc (100 mL). The organic layer was washed with brine (100 mL), dried over $Na_2SO_4$, evaporated and purified by column chromatography (PE/EtOAc = 10/1) to give (5-chloro-2-(2,2,3,3,10,10-hexamethyl-8-oxo-4,9-dioxa-7-aza-3-silaundecan-6-yl)pyridin-3-yl)methyl 4-methylbenzenesulfonate (5.3 g, 69% yield) as a colourless oil.

m/z ES+ $[M+H]^+$571.1; $^1H$ NMR (400 MHz, $CDCl_3$) δ ppm 8.45 (d, *J* = 2.4 Hz, 1H), 7.84 (d, *J* = 8.0 Hz, 2H), 7.69 (d, *J* = 2.4 Hz, 1H), 7.36 (d, *J* = 8.0 Hz, 2H), 5.68 (d, *J* = 7.2 Hz, 1H), 5.31 - 5.16 (m, 2H), 4.87 - 4.80 (m, 1H), 3.93 - 3.84 (m, 1H), 3.52 - 3.44 (m, 1H), 2.46 (s, 3H), 1.60 (s, 9H), 1.43 (s, 9H), -0.16 - -0.13 (m, 3H), -0.20 (s, 3H).

**Step f.** To a solution of (5-chloro-2-(2,2,3,3,10,10-hexamethyl-8-oxo-4,9-dioxa-7-aza-3-silaundecan-6-yl)pyridin-3-yl)methyl 4-methylbenzenesulfonate (4.8 g, 8.40 mmol) in DMF (150 mL) was added NaH (1.01 g, 25.2 mmol, 60% dispersion in mineral oil) at 0 °C. The reaction mixture was stirred at 0 °C for 1 h. Upon completion, the mixture was diluted with water (500 mL) and extracted with EtOAc (500 mL). The organic layer was washed with brine (500 mL), dried over $Na_2SO_4$, evaporated and purified by reverse phase flash chromatography (water (0.1% $NH_4OH$)/MeCN) to give *tert*-butyl 7-(((*tert*-butyldimethylsilyl)oxy)methyl)-3-chloro-5,7-dihydro-6*H*-pyrrolo[3,4-*b*]pyridine-6-carboxylate (2.2 g, 66% yield) as a colourless oil.

m/z ES+ $[M+H]^+$ 399.0; $^1H$ NMR (400 MHz, $CDCl_3$) δ ppm 8.46 (s, 1H), 7.57 - 7.49 (m, 1H), 4.99 - 4.83 (m, 1H), 4.80 - 4.63 (m, 1H), 4.60 - 4.50 (m, 1H), 4.44 - 4.18 (m, 1H), 4.07 - 3.98 (m, 1H), 1.53 (s, 9H), 0.68 (d, *J* = 2.4 Hz, 9H), -0.07 (d, *J* = 4.0 Hz, 3H), -0.18 (d, *J* = 8.4 Hz, 3H).

**Step g.** This step was conducted in a similar manner to **Intermediate B49,** step g, and an additional chiral SFC step was conducted to afford the title compounds.

**Intermediate B50a:**

m/z ES+ $[M+H]^+$391.3; $^1H$ NMR (400 MHz, $CDCl_3$) δ ppm 8.50 - 8.45 (m, 1H), 7.66 - 7.54 (m, 1H), 6.79 - 6.68 (m, 1H), 5.86 - 5.76 (m, 1H), 5.42 - 5.33 (m, 1H), 5.00 - 4.83 (m, 1H), 4.81 - 4.65 (m, 1H), 4.62 - 4.51 (m, 1H), 4.20 - 4.45 (m, 1H), 4.10 - 4.00 (m, 1H), 1.53 (s, 9H), 0.67 (d, *J* = 3.2 Hz, 9H), -0.08 (d, *J* = 5.6 Hz, 3H), -0.20 (d, *J* = 10.4 Hz, 3H).

**Intermediate B50b:**

m/z ES+ $[M+H]^+$391.3; $^1H$ NMR (400 MHz, $CDCl_3$) δ ppm 8.48 (d, *J* = 2.8 Hz, 1H), 7.67 - 7.54 (m, 1H), 6.65 - 6.80 (m, 1H), 5.75 - 5.90 (m, 1H), 5.37 (d, *J* = 11.2 Hz, 1H), 5.02 - 4.84 (m, 1H), 4.82 - 4.64 (m, 1H), 4.61 - 4.49 (m, 1H), 4.46 - 4.16 (m, 1H), 4.12 - 3.98 (m, 1H), 1.53 (s, 9H), 0.67 (d, *J* = 3.2 Hz, 9H), -0.08 (d, *J* = 5.6 Hz, 3H), -0.20 (d, *J* = 10.4 Hz, 3H).

**Intermediate B51a: *tert*-butyl (*R*/*S*)-7-(((*tert*-butyldimethylsilyl)oxy)methyl)-3-formyl-5,7-dihydro-6*H*-pyrrolo [3,4-*b*]pyridine-6-carboxylate**

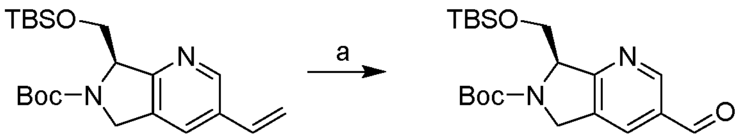


**Step a.** This step was conducted in a similar manner to **Intermediate B49,** step h, using **Intermediate B50a.**

m/z ES+ $[M+H]^+$393.4; $^1$H NMR (400 MHz, $CDCl_3$) δ ppm 10.12 (s, 1H), 8.96 (s, 1H), 8.08 - 7.95 (m, 1H), 5.11 - 4.92 (m, 1H), 4.90-4.71 (m, 1H), 4.68 - 4.56 (m, 1H), 4.50 - 4.21 (m, 1H), 4.00 - 4.18 (m, 1H), 1.54 (s, 9H), 0.65 (d, *J* = 2.8 Hz, 9H), -0.07 (d, *J* = 4.0 Hz, 3H), -0.19 (d, *J* = 8.4 Hz, 3H).

**Intermediate B51b: *tert*-butyl (*S*/*R*)-7-(((*tert*-butyldimethylsilyl)oxy)methyl)-3-formyl-5,7-dihydro-6*H*-pyrrolo [3,4-*b*]pyridine-6-carboxylate**

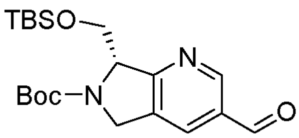

The title compound was prepared in a similar manner to **Intermediate B51a,** using **Intermediate B50b.**

m/z ES+ $[M+H]^+$ 393.4; $^1$H NMR (400 MHz, $CDCl_3$) δ ppm 10.12 (s, 1H), 8.96 (s, 1H), 8.09 - 7.97 (m, 1H), 5.10 - 4.93 (m, 1H), 4.90 - 4.73 (m, 1H), 4.68 - 4.58 (m, 1H), 4.49 - 4.21 (m, 1H), 4.14 - 4.07 (m, 1H), 1.54 (s, 9H), 0.65 (d, *J* = 2.8 Hz, 9H), -0.07 (d, *J* = 4.4 Hz, 3H), -0.19 (d, *J* = 8.8 Hz, 3H).

**Intermediate B52: *tert*-butyl 5-(((*tert*-butyldiphenylsilyl)oxy)methyl)-2-formyl-5,7-dihydro-6*H*-pyrrolo[3,4-*b*]pyridine-6-carboxylate**

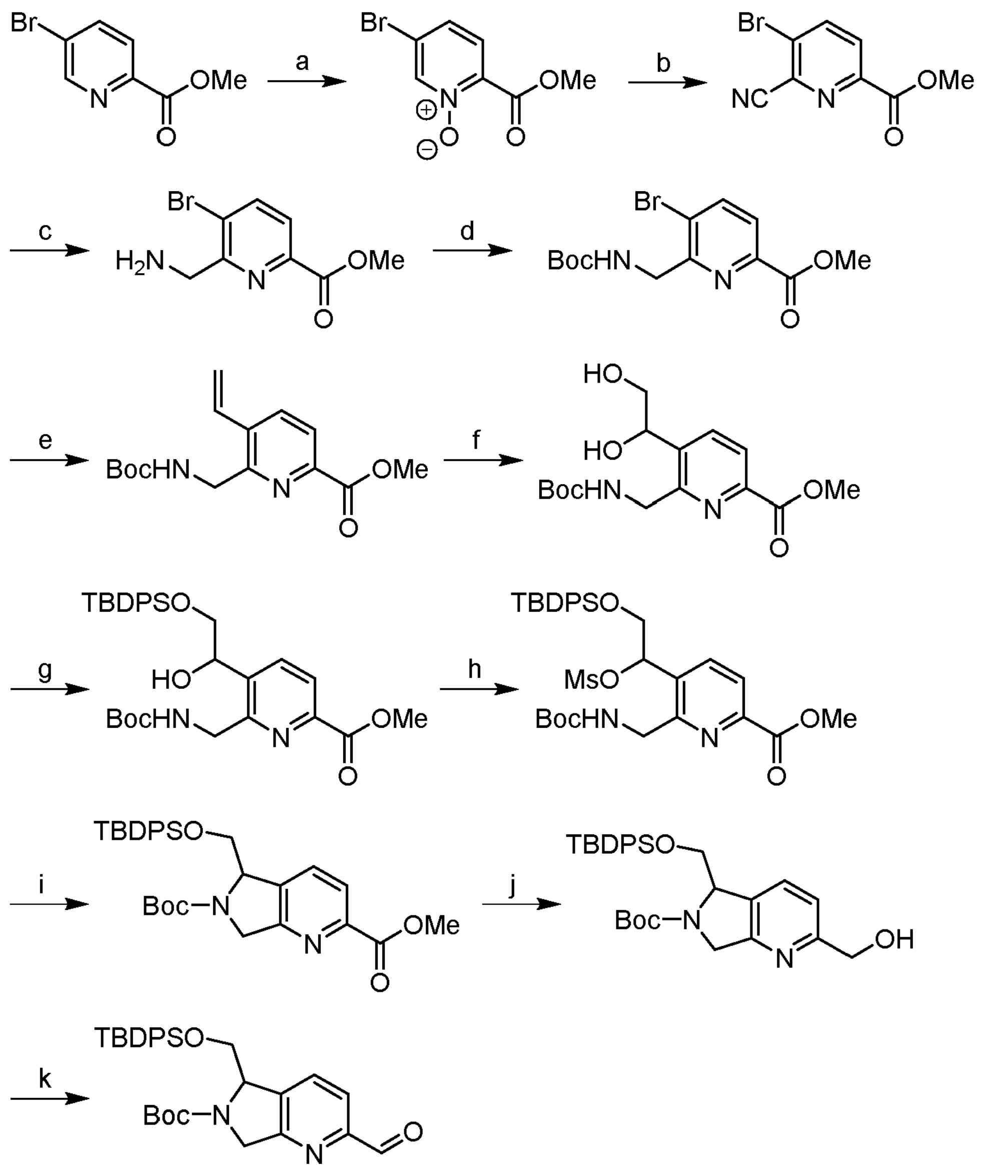


**Step a.** To a solution of methyl 5-bromopicolinate (15 g, 69.4 mmol) in DCM (250 mL) was added *m*-CPBA (80%; 30.0 g, 139 mmol). The reaction mixture was stirred at 25 °C for 12 h. Upon completion, the mixture was diluted with sat. aq. $Na_2SO_3$ (500 mL) and extracted with DCM (500 mL). The combined organic layers were washed with sat. aq. $Na_2SO_3$ (300 mL), brine (500 mL), dried over $Na_2SO_4$, evaporated and purified by column chromatography (PE/EtOAc = 1/2) to give 5-bromo-2-(methoxycarbonyl)pyridine 1-oxide (11.7 g, 70% yield) as an off-white solid.

m/z ES+ $[M+H]^+$ 234.0; $^1H$ NMR (400 MHz, $CDCl_3$) δ ppm 8.41 (s, 1H), 7.55 (d, *J* = 8.4 Hz, 1H), 7.41 (d, *J* = 8.4 Hz, 1H), 4.00 (s, 3H).

**Step b.** A mixture of 5-bromo-2-(methoxycarbonyl)pyridine 1-oxide (11.7 g, 50.4 mmol), TMSCN (50.0 g, 504 mmol) and dimethylcarbamoyl chloride (54.2 g, 504 mmol) was stirred at 50 °C for 16 h under a $N_2$ atmosphere. Upon completion, the mixture was basified to pH 8 with 1 M NaOH and extracted with EtOAc (3 x 250 mL). The combined organic layers were washed with brine (600 mL), dried over $Na_2SO_4$, evaporated and purified by column chromatography (PE/EtOAc = 7/1) to give methyl 5-bromo-6-cyanopicolinate (10.4 g, 85% yield) as an off-white solid.

m/z ES+ $[M+H]^+$ 241.0; $^1H$ NMR (400 MHz, $CDCl_3$) δ ppm 8.24 - 8.15 (m, 2H), 4.04 (s, 3H). **Step c.** To a solution of methyl 5-bromo-6-cyanopicolinate (10.4 g, 43.2 mmol) in THF (120 mL) was added a solution of borane tetrahydrofuran complex (1 M in THF, 215.73 mL) dropwise at 0 °C. The reaction mixture was stirred at 20 °C for 3 h. Upon completion, the mixture was quenched with MeOH (50 mL) at 0 °C and evaporated to give methyl 6-(aminomethyl)-5-bromopicolinate (12.0 g, crude) as a yellow solid.

**Step d.** To a solution of methyl 6-(aminomethyl)-5-bromopicolinate (12.0 g, 42.6 mmol) in THF (150 mL) was added $NaHCO_3$ (7.16 g, 85.3 mmol) and $Boc_2O$ (11.2 g, 51.2 mmol). The reaction mixture was stirred at 20 °C for 12 h. Upon completion, the mixture was diluted with water (150 mL) and extracted with EtOAc (3 x 150 mL). The combined

organic layers were washed with brine (300 mL), dried over $Na_2SO_4$, evaporated and purified by column chromatography (PE/EtOAc = 7/1) to give methyl 5-bromo-6-(((*tert*-butoxycarbonyl)amino)methyl)picolinate (4.5 g, 29% yield) as a yellow solid.

m/z ES+ $[M+Na]^+$ 367.0; $^1H$ NMR (400 MHz, $CDCl_3$) δ ppm 8.01 (d, *J* = 8.0 Hz, 1H), 7.90 (d, *J* = 8.4 Hz, 1H), 5.96 (s, 1H), 4.60 (s, 2H), 4.00 (s, 3H), 1.50 (s, 9H).

**Step e.** A mixture of methyl 5-bromo-6-(((tert-butoxycarbonyl)amino)methyl)picolinate (2.5 g, 7.24 mmol), potassium trifluoro(vinyl)borate (CAS: 13682-77-4; 1.94 g, 14.5 mmol), Pd(dppf)$Cl_2$ (265 mg, 0.36 mmol) and $Na_2CO_3$ (1.92 g, 18.1 mmol) in 1,4-dioxane (40 mL) and water (4 mL) was degassed and purged with $N_2$ 3 times. The reaction mixture was stirred at 80 °C for 24 h under a $N_2$ atmosphere. Upon completion, the mixture was diluted with water (50 mL) and extracted with EtOAc (3 x 50 mL). The combined organic layers were washed with brine (150 mL), dried over $Na_2SO_4$, evaporated and purified by column chromatography (PE/EtOAc = 8/1) to give methyl 6-(((*tert*-butoxycarbonyl)amino) methyl)-5-vinylpicolinate (1.68 g, 76% yield) as a yellow solid.

m/z ES+ $[M+Na]^+$ 315.0; $^1H$ NMR (400 MHz, $CDCl_3$) δ ppm 8.03 (d, *J* = 8.0 Hz, 1H), 7.92 (d, *J* = 8.0 Hz, 1H), 6.99 - 6.92 (m, 1H), 5.95 (br s, 1H), 5.83 (d, *J* = 17.2 Hz, 1H), 5.59 (d, *J* = 11.2 Hz, 1H), 4.58 (s, 2H), 4.01 (s, 3H), 1.49 (s, 9H).

**Step f.** To a solution of methyl 6-(((*tert*-butoxycarbonyl)amino)methyl)-5-vinylpicolinate (1.7 g, 5.82 mmol) in MeCN (15 mL), tBuOH (15 mL) and water (15 mL) was added $OsO_4$ (148 mg, 0.58 mmol) and NMO (818 mg, 6.98 mmol). The reaction mixture was stirred at 20 °C for 2 h. Upon completion, the mixture was quenched with sat. aq. $Na_2SO_3$ (25 mL) at 20 °C and extracted with EtOAc (3 x 25 mL). The combined organic layers were washed with sat. aq. $Na_2SO_3$ (35 mL), brine (60 mL), dried over $Na_2SO_4$, evaporated and purified by column chromatography (PE/EtOAc = 1/2) to give methyl 6-(((*tert*-butoxycarbonyl)amino)methyl)-5-(1,2-dihydroxyethyl)picolinate (1.5 g, 78% yield) as a yellow solid.

m/z ES+ $[M+H]^+$ 327.2; $^1H$ NMR (400 MHz, $CDCl_3$) δ ppm 8.10 - 8.01 (m, 2H), 6.04 (s, 1H), 5.26 - 5.17 (m, 1H), 4.69 - 4.57 (m, 1H), 4.53 - 4.43 (m, 1H), 4.15 - 4.03 (m, 1H), 3.99 (s, 3H), 3.84 - 3.81 (m, 1H), 3.70 - 3.67 (m, 1H), 3.00 (br s, 1H), 1.44 (s, 9H).

**Step g.** This step was conducted in a similar manner to **Intermediate B50a** and **Intermediate B50b,** step a, using TBDPSCl.

m/z ES+ $[M+H]^+$ 565.3; $^1H$ NMR (400 MHz, $CDCl_3$) δ ppm 8.05 - 8.01 (m, 2H), 8.00 - 7.96 (m, 2H), 7.52 - 7.49 (m, 2H), 7.46 - 7.41 (m, 6H), 5.84 (s, 1H), 5.14 (s, 1H), 4.52 - 4.51 (m, 1H), 4.26 - 4.25 (m, 1H), 3.89 (s, 3H), 3.85 - 3.82 (m, 1H), 3.70 - 3.66 (m, 1H), 3.31 (s, 1H), 1.44 (s, 9H), 1.05 (s, 9H).

**Step h.** To a solution of methyl 6-(((*tert*-butoxycarbonyl)amino)methyl)-5-(2-((*tert*-butyldiphenylsilyl)oxy)-1-hydroxyethyl)picolinate (2 g, 3.54 mmol) in DCM (50 mL) was added TEA (1.08 g, 10.6 mmol) and methanesulfonic anhydride (925 mg, 5.31 mmol). The reaction mixture was stirred at 20 °C for 2 h. Upon completion, the mixture was diluted with water (50 mL) and extracted with DCM (2 x 50 mL). The combined organic layers were washed with brine (120 mL), dried over $Na_2SO_4$, evaporated and purified by column chromatography (PE/EtOAc = 4/1) to give methyl 6-(((*tert*-butoxycarbonyl)amino)methyl)-5-(2-((*tert*-butyldiphenylsilyl)oxy)-1-((methylsulfonyl)oxy)ethyl)picolinate (2 g, 88% yield) as a white solid. m/z ES+ $[M+H]^+$ 643.2; $^1H$ NMR (400 MHz, $CDCl_3$) δ ppm 8.06 - 8.00 (m, 1H), 7.99 - 7.92 (m, 1H), 7.60 - 7.51 (m, 4H), 7.48 - 7.41 (m, 2H), 7.41 - 7.34 (m, 4H), 6.08 (s, 1H), 5.68 (t, *J* = 4.8 Hz, 1H), 4.62 - 4.57 (m, 1H), 4.39 - 4.35 (m, 1H), 4.01 (s, 3H), 3.99 - 3.93 (m, 1H), 3.89 - 3.83 (m, 1H), 3.02 (s, 3H), 1.45 (s, 9H), 1.02 (s, 9H).

**Step i.** To a solution of methyl 6-(((*tert*-butoxycarbonyl)amino)methyl)-5-(2-((*tert*-butyldiphenylsilyl) oxy)-1-((methylsulfonyl)oxy)ethyl)picolinate (150 mg, 0.23 mmol) in NMP (5 mL) was added $K_3PO_4$ (149 mg, 0.70 mmol). The reaction mixture was stirred at 60 °C for 48 h. Upon completion, the mixture was diluted with water (5 mL) and extracted with EtOAc (3 x 5 mL). The combined organic layers were washed with brine (2 x 15 mL), dried over $Na_2SO_4$, evaporated and purified by prep-TLC (PE/EtOAc = 1/1) to give 6-(*tert*-butyl) 2-methyl 5-(((*tert*-butyldiphenylsilyl)oxy)methyl)-5,7-dihydro-6*H*-pyrrolo[3,4-*b*]pyridine-2,6-dicarboxylate (30 mg, 23% yield) as a brown solid.

m/z ES+ $[M+H]^+$ 547.3; $^1H$ NMR (400 MHz, $CDCl_3$) δ ppm 8.14 - 8.04 (m, 1H), 7.72 - 7.65 (m, 1H), 7.53 - 7.47 (m, 3H), 7.44 - 7.38 (m, 3H), 7.38 - 7.31 (m, 4H), 5.23 - 5.08 (m, 1H), 4.97 - 4.82 (m, 1H), 4.80 - 4.72 (m, 1H), 4.05 (s, 3H), 4.02 - 3.95 (m, 1H), 3.95 - 3.87 (m, 1H), 1.54 - 1.41 (m, 9H), 0.90 (d, *J* = 12.8 Hz, 9H).

**Step j.** To a solution of 6-(*tert*-butyl) 2-methyl 5-(((*tert*-butyldiphenylsilyl)oxy)methyl)-5,7-dihydro-6H-pyrrolo [3,4-b]pyridine-2,6-dicarboxylate (320 mg, 0.59 mmol) in THF (5 mL) and MeOH (0.5 mL) was added $LiBH_4$ (64 mg, 2.93 mmol). The reaction mixture was stirred at 20 °C for 2 h. Upon completion, the mixture was quenched with sat. aq. $NH_4Cl$ (10 mL) at 20 °C and extracted with EtOAc (3 x 10 mL). The combined organic layers were washed with brine (25 mL), dried over $Na_2SO_4$, evaporated and purified by column chromatography (PE/EtOAc = 6/1) to give *tert*-butyl 5-(((*tert*-butyldiphenylsilyl)oxy)methyl)-2-(hydroxymethyl)-5,7-dihydro-6H-pyrrolo[3,4-b]pyridine-6-carboxylate (210 mg, 66% yield) as a yellow solid.

m/z ES+ $[M+H]^+$ 519.3; $^1H$ NMR (400 MHz, $CDCl_3$) δ ppm 7.68 - 7.48 (m, 4H), 7.46 - 7.29 (m, 7H), 7.20 - 7.15 (m, 1H), 5.19 - 5.04 (m, 1H), 4.87 - 4.74 (m, 3H), 4.71 - 4.61 (m, 1H), 4.08 - 3.81 (m, 2H), 3.52 - 3.39 (m, 1H), 1.55 - 1.41 (m, 9H), 0.91 (d, *J* = 13.2 Hz, 9H).

**Step k.** To a solution of *tert*-butyl 5-(((*tert*-butyldiphenylsilyl)oxy)methyl)-2-(hydroxymethyl)-5,7-dihydro-6*H*-pyrrolo[3,4-*b*]pyridine-6-carboxylate (210 mg, 0.41 mmol) in DCM (5 mL) was added $MnO_2$ (704 mg, 8.10 mmol). The

reaction mixture was stirred at 20 °C for 12 h. Upon completion, the mixture was filtered, evaporated and purified by Prep-TLC (PE/EtOAc = 2/1) to give the title compound (120 mg, 57% yield) as a white solid.

m/z ES+ $[M+H]^+$ 517.2; $^1H$ NMR (400 MHz, $CDCl_3$) δ ppm 10.10 (d, *J* = 6.8 Hz, 1H), 7.92 - 7.90 (m, 1H), 7.81 - 7.67 (m, 1H), 7.55 - 7.43 (m, 4H), 7.41 - 7.30 (m, 6H), 5.25 - 5.09 (m, 1H), 4.96 - 4.75 (m, 2H), 4.01 - 3.90 (m, 2H), 1.56 (d, *J* = 6.8 Hz, 9H), 0.89 (d, *J* = 10.8 Hz, 9H).

**Intermediate B53: *tert*-butyl 2-formyl-5,7-dihydro-6*H*-pyrrolo[3,4-*b*]pyridine-6-carboxylate**

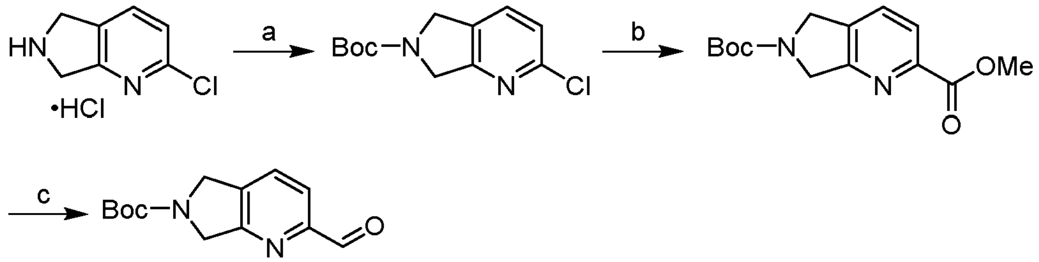


**Step a.** To a solution of 2-chloro-6,7-dihydro-5*H*-pyrrolo[3,4-*b*]pyridine hydrochloride (CAS: 1841081-37-5; 24.0 g, 125 mmol) in 1,4-dioxane (240 mL) was added $Boc_2O$ (41.1 g, 188 mmol), TEA (38.1 g, 376 mmol) and DMAP (1.53 g, 12.5 mmol). The reaction mixture was stirred at 25°C for 30 min. Upon completion, the mixture was diluted with water (250 mL) and extracted with EtOAc (2 x 150 mL). The combined organic layers were washed with brine (200 mL), dried over $Na_2SO_4$, evaporated and purified by column chromatography (PE/EtOAc = 3/1) to give tert-butyl 2-chloro-5,7-dihydro-6H-pyrrolo[3,4-b]pyridine-6-carboxylate (30.0 g, 93% yield) as a white solid.

m/z ES+ $[M+H]^+$ 255.0; $^1H$ NMR (400 MHz, DMSO-$d_6$) δ ppm 7.82 (t, *J* = 7.2 Hz, 1H), 7.42 (d, *J* = 8.0 Hz, 1H), 4.47 - 4.56 (m, 4H), 1.46 (s, 9H).

**Step b.** To a solution of *tert*-butyl 2-chloro-5,7-dihydro-6*H*-pyrrolo[3,4-*b*]pyridine-6-carboxylate (30.0 g, 117 mmol) in DMSO (150 mL) and MeOH (150 mL) was added $Pd(OAc)_2$ (5.29 g, 23.5 mmol), DPPP (9.72 g, 23.5 mmol) and TEA (119 g, 1.18 mol). The reaction mixture was stirred at 60 °C for 12 h under a CO atmosphere (50 psi). Upon completion, the mixture was filtered, evaporated and purified by column chromatography (PE/EtOAc = 2/1) to give 6-(*tert*-butyl) 2-methyl 5,7-dihydro-6*H*-pyrrolo[3,4-*b*]pyridine-2,6-dicarboxylate (32.0 g, 96% yield) as a yellow solid.

m/z ES+ $[M+H]^+$ 279.1; $^1H$ NMR (400 MHz, DMSO-$d_6$) δ ppm 7.89 - 8.01 (m, 2H), 4.67 (d, *J* = 11.6 Hz, 2H), 4.59 (d, *J* = 6.4 Hz, 2H), 3.88 (s, 3H), 1.46 (s, 9H).

**Step c.** To a solution of 6-(*tert*-butyl) 2-methyl 5,7-dihydro-6*H*-pyrrolo[3,4-*b*]pyridine-2,6-dicarboxylate (43.5 g, 156 mmol) in THF (545 mL) at -78 °C was slowly added DIBAL-H (1 M in toluene, 390 mL) over 30 min. The reaction mixture was stirred at -78 °C for 3 h. Upon completion, the mixture was added dropwise to MeOH (700 mL) at -40 °C under a $N_2$ atmosphere. The mixture was stirred for 30 min, after which 0.5 M HCl (800 mL) was added and the mixture was stirred at 15 °C for a further 30 min. The aqueous mixture was extracted with EtOAc (2 x 500 mL) and the combined organic layers were dried over $Na_2SO_4$, evaporated and purified by column chromatography (PE/EtOAc = 5/1) to give the title compound (25.0 g, 64% yield) as a white solid.

m/z ES+ $[M+H]^+$ 249.2; $^1H$ NMR (400 MHz, DMSO-$d_6$) δ ppm 9.97 (d, *J* = 2.0 Hz, 1H), 7.96 - 8.04 (m, 1H), 7.87 (d, *J* = 7.6 Hz, 1H), 4.63 - 4.76 (m, 4H), 1.47 (s, 9H).

**Intermediate B54: *tert*-butyl 6-formyl-1,3-dihydro-2*H*-pyrrolo[3,4-*c*]pyridine-2-carboxylate**

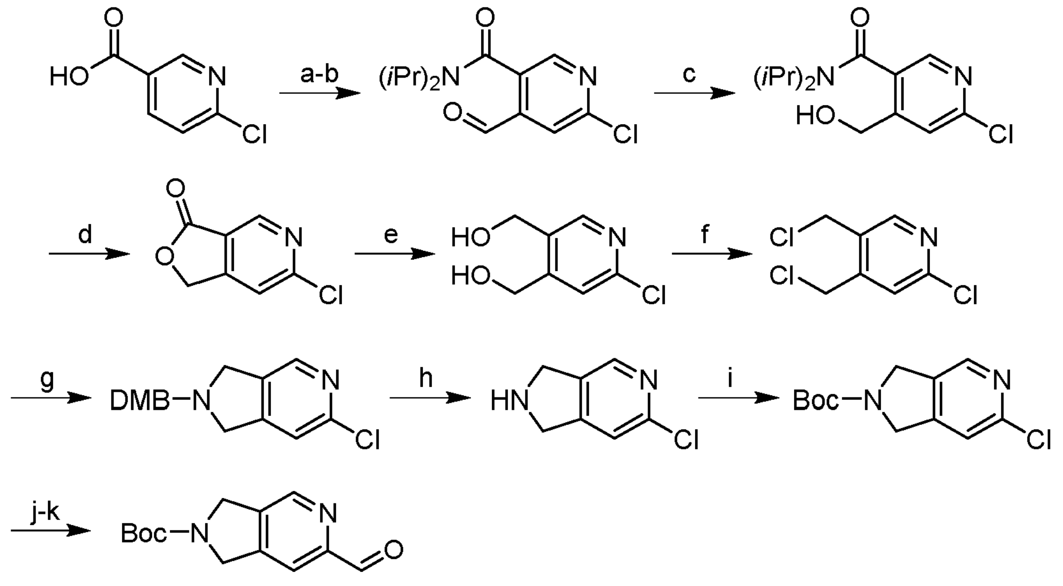


**Step a.** To a solution of 6-chloronicotinic acid (CAS: 5326-23-8; 23.0 g, 145 mmol) in toluene (200 mL) was added DMF (872 mg, 11.9 mmol) at 0 °C, after which $SOCl_2$ (86.8 g, 729 mmol) was added to the mixture slowly, keeping the temperature between 0 - 5 °C. The reaction mixture was stirred at 0 °C for 30 min followed by 85 °C for a further 5 h. Upon completion, the mixture was evaporated to give 6-chloronicotinoyl chloride (25 g, crude) as yellow oil. To a solution of the acid chloride (25.0 g, 142.1 mmol) in DCM (250 mL) was added diisopropylamine (57.5 g, 568 mmol). The mixture was stirred at 0 °C for 2 h. Upon completion, the reaction mixture was quenched with water (50 mL) at 0 °C and extracted with DCM (3 x 50 mL). The combined organic layers were washed with brine (60 mL), dried over $Na_2SO_4$ and evaporated to give 6-chloro-*N*,*N*-diisopropylnicotinamide (32.0 g, 93% yield) as a yellow solid. $^1H$ NMR (400 MHz, $CDCl_3$) δ ppm 8.37 (d, *J* = 2.0 Hz, 1H), 7.72 - 7.54 (m, 1H), 7.37 (d, *J* = 8.4 Hz, 1H), 3.89 - 3.45 (m, 2H), 1.58 - 1.11 (m, 12H).

**Step b.** A solution of LDA (2 M in THF, 93.4 mL) was added dropwise to a solution of 6-chloro-*N*,*N*-diisopropylnicotinamide (30.0 g, 124 mmol) in THF (300 mL) at -78 °C under a $N_2$ atmosphere. The mixture was stirred at -78 °C for 2 h, after which DMF (23.3 g, 318 mmol) was added slowly. The reaction mixture was stirred at -78 °C for 1 h, slowly warmed to 25 °C and stirred at that temperature for an additional 30 min. Upon completion, the mixture was slowly quenched with 10% aq. citric acid (200 mL) and extracted with EtOAc (3 x 250 mL). The combined organic layers were washed with brine (250 mL), dried over $Na_2SO_4$, evaporated and purified by column chromatography (PE/EtOAc = 3/1) to give 6-chloro-4-formyl-N,N-diisopropylnicotinamide (24.0 g, 60% yield) as a yellow solid.

m/z ES+ $[M+H]^+$ 269.0; $^1H$ NMR (400 MHz, DMSO-$d_6$) δ ppm 9.99 (s, 1H), 8.54 (s, 1H), 8.04 (s, 1H), 3.62 - 3.56 (m, 1H), 3.51 - 3.46 (m, 1H), 1.47 (d, *J* = 6.4 Hz, 6H), 1.10 (d, *J* = 6.4 Hz, 6H).

**Step c.** To a solution of 6-chloro-4-formyl-*N*,*N*-diisopropylnicotinamide (24.0 g, 89.3 mmol) in EtOH (500 mL) was slowly added $NaBH_4$ (23.9 g, 634 mmol) at 25 °C. The reaction mixture was stirred at 25 °C for 2 h. Upon completion, mixture was slowly poured into water (1000 mL) at 0 °C, quenched with 1 M HCl (100 mL) and extracted with EtOAc (3 x 250 mL). The combined organic layers were washed with brine (300 mL), dried over ($Na_2SO_4$), evaporated and purified by column chromatography (PE/EtOAc = 3/1) to give 6-chloro-4-(hydroxymethyl)-*N*,*N*-diisopropylnicotinamide (10.0 g, 33% yield) as a light yellow solid.

m/z ES+ $[M+H]^+$ 271.1; $^1H$ NMR (400 MHz, $CDCl_3$) δ ppm 8.18 (s, 1H), 7.45 (s, 1H), 4.76 - 4.41 (m, 2H), 3.77 - 3.68 (m, 1H), 3.57 - 3.52 (m, 2H), 1.55 (d, *J* = 6.4 Hz, 6H), 1.17 (s, 6H). **Step d.** A solution of 6-chloro-4-(hydroxymethyl)-*N*,*N*-diisopropylnicotinamide (9.00 g, 33.2 mmol) in 6 M HCl (180 mL) was stirred at 100 °C for 1 h. Upon completion, the mixture was basified to pH 7-8 with sat. aq. $Na_2CO_3$ at 0 °C and extracted with EtOAc (3 x 100 mL). The combined organic layers were washed with brine (100 mL), dried over $Na_2SO_4$ and evaporated to give 6-chlorofuro[3,4-*c*]pyridin-3(1*H*)-one (3.00 g, 53% yield) as a yellow solid.

m/z ES+ $[M+H]^+$ 170.0.

**Step e.** To a solution of 6-chlorofuro[3,4-*c*]pyridin-3(1*H*)-one (2.80 g, 16.5 mmol) in EtOH (60 mL) was slowly added $NaBH_4$ (4.37 g, 115 mmol) in batches at 25 °C. The reaction mixture was stirred at 25 °C for 2 h. Upon completion, the mixture was poured into water (100 mL), quenched with 1 M HCl (50 ml) and extracted with EtOAc (3 x 100mL). The combined organic layers were dried over $Na_2SO_4$ and evaporated to give (6-chloropyridine-3,4-diyl)dimethanol (1.40 g, 48% yield) as a yellow solid.

m/z ES+ $[M+H]^+$ 174.1.

**Step f.** To a solution of (6-chloropyridine-3,4-diyl)dimethanol (1.10 g, 6.34 mmol) in DCM (60 mL) was added $SOCl_2$ (3.77 g, 31.6 mmol). The mixture was stirred at 50 °C for 2 h. Upon completion, the mixture was evaporated to give 2-chloro-4,5-bis(chloromethyl)pyridine (1.33 g, crude) as a yellow oil that was used directly without further purification.

**Step g.** To a solution of 2-chloro-4,5-bis(chloromethyl)pyridine (1.33 g, 6.32 mmol) in DCM (30 mL) was added DIPEA (2.45 g, 18.9 mmol) and (2,4-dimethoxyphenyl)methanamine (1.58 g, 9.48 mmol). The reaction mixture was stirred at 25 °C for 6 h. Upon completion, the mixture was evaporated and purified by column chromatography (PE/EtOAc = 3/1) to give 6-chloro-2-(2,4-dimethoxybenzyl)-2,3-dihydro-1H-pyrrolo[3,4-c]pyridine (1.20 g, 57% yield) as a yellow solid.

m/z ES+ $[M+H]^+$ 305.1; $^1H$ NMR (400 MHz, $CDCl_3$) δ ppm 8.18 (s, 1H), 7.26 - 7.23 (m, 1H), 7.15 (s, 1H), 6.51 - 6.48 (m, 2H), 3.95 - 3.93 (m, 4H), 3.87 (s, 2H), 3.83 (s, 6H).

**Step h.** A solution of 6-chloro-2-(2,4-dimethoxybenzyl)-2,3-dihydro-1*H*-pyrrolo[3,4-*c*]pyridine (1.00 g, 3.28 mmol) in TFA (3 mL) was stirred at 80 °C for 2 h. Upon completion, the mixture was evaporated to give 6-chloro-2,3-dihydro-1*H*-pyrrolo[3,4-*c*]pyridine (500 mg, 98% yield) as yellow oil that was used directly without further purification.

**Step i.** To a solution of 6-chloro-2,3-dihydro-1*H*-pyrrolo[3,4-c]pyridine (500 mg, 3.23 mmol) in DCM (10 mL) was added $Boc_2O$ (1.06 g, 4.85 mmol) and DIPEA (835 mg, 6.47 mmol). The reaction mixture was stirred at 25 °C for 6 h. Upon completion, the mixture was evaporated and purified by column chromatography (PE/EtOAc = 3/1) to give *tert*-butyl 6-chloro-1,3-dihydro-2*H*-pyrrolo[3,4-*c*]pyridine-2-carboxylate (700 mg, 84% yield) as yellow solid

m/z ES+ $[M+H]^+$ 255.0; $^1H$ NMR (400 MHz, $CDCl_3$) δ ppm 8.35 - 8.30 (m, 1H), 7.32 - 7.24 (m, 1H), 4.73 - 4.66 (m, 4H), 1.54 (s, 9H).

**Steps j-k.** These 2 steps were conducted in a similar manner to **Intermediate B49,** steps g-h.

m/z ES+ $[M+H]^+$ 249.1.

**Intermediate B55: *tert*-butyl 2-formyl-5,7-dihydro-6*H*-pyrrolo[3,4-*b*]pyridine-6-carboxylate**

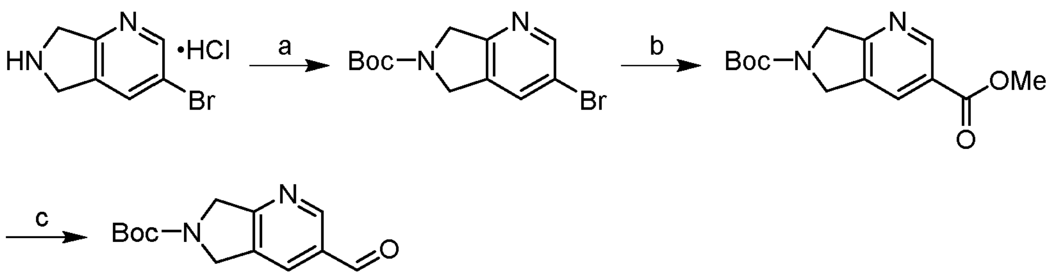


**Step a.** To a solution of 3-bromo-6,7-dihydro-5*H*-pyrrolo[3,4-*b*]pyridine hydrochloride (CAS: 1394117-24-8; 1 g, 4.25 mmol) in DCM (15 mL) was added DIPEA (1.21 g, 9.34 mmol) and $Boc_2O$ (1.11 g, 5.10 mmol). The reaction mixture was stirred at 30 °C for 1 h. Upon completion, the mixture was evaporated and purified by column chromatography (PE/EtOAc = 2/1) to give *tert*-butyl 3-bromo-5,7-dihydro-6*H*-pyrrolo[3,4-*b*]pyridine-6-carboxylate (1.2 g, 94% yield) as a white solid.

$^1H$ NMR (400 MHz, $CDCl_3$) δ ppm 8.55 (s, 1H), 7.82 - 7.63 (m, 1H), 4.71 - 4.61 (m, 4H), 1.53 (s, 9H).

**Step b.** A mixture of *tert*-butyl 3-bromo-5,7-dihydro-6*H*-pyrrolo[3,4-*b*]pyridine-6-carboxylate (1 g, 3.34 mmol), $Pd(OAc)_2$ (150 mg, 0.67 mmol), DPPP (276 mg, 0.67 mmol) and TEA (3.38 g, 33.4 mmol) in MeOH (10 mL) and DMSO (10 mL) was degassed and purged with $N_2$ and CO 3 times. The reaction mixture was stirred at 60 °C for 12 h under a CO atmosphere (50 psi). Upon completion, the reaction mixture was evaporated, dissolved in EtOAc (100 mL) and washed with water (2 x 100 mL). The organic layer was dried over $Na_2SO_4$, evaporated and purified by column chromatography (PE/EtOAc = 4/1) to give 6-(*tert*-butyl) 3-methyl 5,7-dihydro-6*H*-pyrrolo[3,4-*b*]pyridine-3,6-dicarboxylate (900 mg, 97% yield) as a white solid.

m/z ES+ $[M+H]^+$279.1; $^1H$ NMR (400 MHz, $CDCl_3$) δ ppm 9.11 (s, 1H), 8.20 - 8.15 (m, 1H), 4.81 - 4.68 (m, 4H), 3.96 (s, 3H), 1.53 (s, 9H).

**Step c.** To a solution of 6-(*tert*-butyl) 3-methyl 5,7-dihydro-6*H*-pyrrolo[3,4-*b*]pyridine-3,6-dicarboxylate (0.1 g, 0.36 mmol) in DCM (4 mL) was added DIBAL-H (1 M in toluene, 1.08 mL) at -78 °C. The reaction mixture was stirred at -78 °C for 30 min. Upon completion, the mixture was quenched with MeOH (0.5 mL) at -78 °C and the mixture was warmed to 20 °C until a solid was formed. The solid was filtered and the filtrate was evaporated and purified by column chromatography (PE/EtOAc=2/1) to give the title compound (0.1 g, crude) as a yellow oil.

$^1H$ NMR (400 MHz, $CDCl_3$) δ ppm 10.12 (s, 1H), 8.96 (s, 1H), 8.08 - 8.03 (m, 1H), 4.82 - 4.75 (m, 4H), 1.54 (s, 9H).

**Intermediate B56: *tert*-butyl 2-formyl-5,7-dihydro-6*H*-pyrrolo[3,4-*d*]pyrimidine-6-carboxylate**

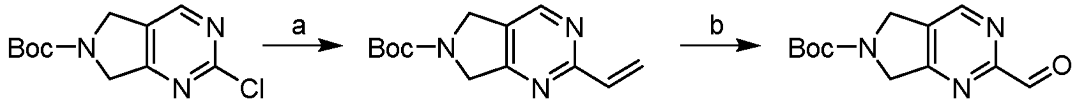


**Steps a-b.** These 2 steps were conducted in a similar manner to **Intermediate B49,** steps g-h, using *tert*-butyl 2-chloro-5,7-dihydro-6*H*-pyrrolo[3,4-*d*]pyrimidine-6-carboxylate.

$^1$H NMR (400 MHz, $CDCl_3$) δ ppm 10.05 (s, 1H), 8.83 - 8.76 (m, 1H), 4.78 - 4.69 (m, 4H), 1.48 (s, 9H).

**Intermediate B57: *tert*-butyl 1-(cyanomethyl)-5-formylisoindoline-2-carboxylate**

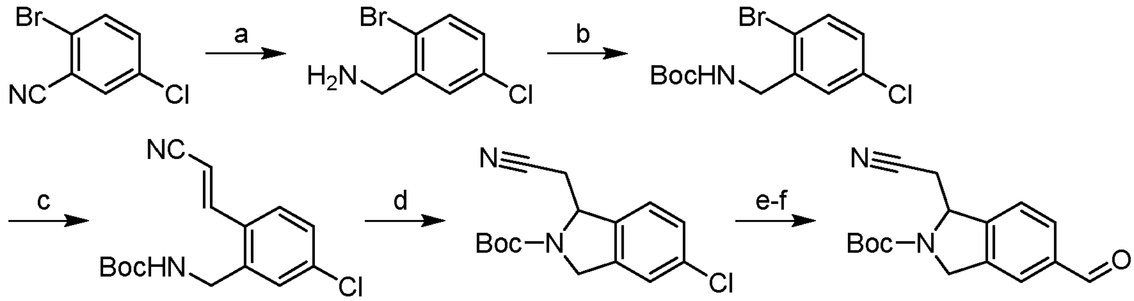


**Step a.** To a solution of 2-bromo-5-chlorobenzonitrile (CAS: 57381-37-0; 5 g, 23.1 mmol) in THF (100 mL) was added a solution of borane dimethyl sulfide complex (10 M in THF, 6.93 mL) at 0 °C. The reaction mixture was stirred at 20 °C for 12 h. Upon completion, the mixture was quenched with MeOH (50 mL) at 0 °C, evaporated and purified by column chromatography (PE/EtOAc = 10/1) to give (2-bromo-5-chlorophenyl)methanamine (3 g, crude) as a yellow solid.

**Step b.** To a solution of (2-bromo-5-chlorophenyl)methanamine (8 g, 36.3 mmol) and $Boc_2O$ (9.50 g, 43.5 mmol) in DCM (200 mL) was added TEA (7.34 g, 72.5 mmol). The reaction mixture was stirred at 15 °C for 12 h. Upon completion, the mixture was quenched with water (40 mL) and extracted with EtOAc (2 x 30 mL). The combined organic layers were washed with brine (30 mL), dried over $Na_2SO_4$, evaporated and purified by column chromatography (PE/EtOAc = 5/1) to give *tert*-butyl (2-bromo-5-chlorobenzyl)carbamate (8 g, 58% yield) as a white solid.

m/z ES+ $[M\text{-}tBu+H]^+$ 265.9; $^1$H NMR (400 MHz, DMSO-$d_6$) δ ppm 7.63 (d, *J* = 8.4 Hz, 1H), 7.53 (t, *J* = 5.6 Hz, 1H), 7.35 - 7.22 (m, 2H), 4.14 (d, *J* = 6.0 Hz, 2H), 1.41 (s, 9H).

**Step c.** To a solution of *tert*-butyl (2-bromo-5-chlorobenzyl)carbamate (2 g, 6.24 mmol), acrylonitrile (1.66 g, 31.2 mmol) and t-$Bu_3$P-Pd-G2 (CAS: 1375325-71-5; 160 mg, 0.31 mmol) in DMF (20 mL) was added *N*-cyclohexyl-*N*-methylcyclohexanamine (CAS: 7560-83-0; 1.34 g, 6.86 mmol). The reaction mixture was purged with $N_2$ 3 times and stirred at 110 °C for 16 h under a $N_2$ atmosphere. Upon completion, the mixture was quenched with water (50 mL) and extracted with EtOAc (3 x 50 mL). The combined organic layers were washed with brine (60 mL), dried over $Na_2SO_4$, evaporated and purified by column chromatography (PE/EtOAc = 3/1) to give *tert*-butyl (E)-(5-chloro-2-(2-cyanovinyl) benzyl)carbamate (1.4 g, 77% yield) as a white solid.

m/z ES+ $[M+Na]^+$ 315.1; $^1$H NMR 400 MHz, $CDCl_3$) δ ppm 7.79 (d, *J* = 8.4 Hz, 1H), 7.61 (d, *J* = 16.4 Hz, 1H), 7.35 (d, *J* = 8.4 Hz, 1H), 5.74 (d, *J* = 16.4 Hz, 1H), 5.50 (d, *J* = 12.0 Hz, 1H), 4.75 (br s, 1H), 4.31 - 4.26 (m, 2H), 1.40 (s, 9H).

**Step d.** To a solution of *tert*-butyl (*E*)-(5-chloro-2-(2-cyanovinyl)benzyl)carbamate (300 mg, 1.02 mmol) in THF (3 mL) was added NaH (49 mg, 1.23 mmol, 60% dispersion in mineral oil) at 0 °C and the reaction mixture was stirred for at 0 °C for 30 min. Upon completion, the mixture was quenched with sat. aq. $NH_4Cl$ (0.1 mL), diluted with water (30 mL) and extracted with EtOAc (2 x 30 mL). The combined organic layers were washed with brine (30 mL), dried over $Na_2SO_4$, evaporated and purified by column chromatography (PE/EtOAc = 3/1) to give *tert*-butyl 5-chloro-1-(cyanomethyl)isoindoline-2-carboxylate (180 mg, 60% yield) as a white solid.

$^1$H NMR (400 MHz, $CDCl_3$) δ ppm 7.37 - 7.27 (m, 3H), 5.23 - 5.15 (m, 1H), 4.80 - 4.63 (m, 2H), 3.29 - 3.22 (m, 1H), 3.00 - 2.90 (m, 1H), 1.53 - 1.51 (m, 9H).

**Steps e-f.** These 2 steps were conducted in a similar manner to **Intermediate B49,** steps g-h.

$^1$H NMR (400 MHz, $CDCl_3$) δ ppm 10.05 (s, 1H), 7.96 - 7.79 (m, 2H), 7.56 (d, *J* = 8.0 Hz, 1H), 5.35 - 5.26 (m, 1H), 4.93 - 4.73 (m, 2H), 3.36 - 3.28 (m, 1H), 3.03 - 2.96 (m, 1H), 1.57 - 1.53 (m, 9H).

**Intermediate B58: *tert*-butyl 1-(cyanomethyl)-6-formylisoindoline-2-carboxylate**

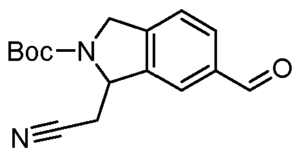

The title compound was prepared in a similar manner to **Intermediate B57,** using 2-bromo-4-chlorobenzonitrile (CAS: 57381-49-4) in step a.

$^1$H NMR (400 MHz, $CDCl_3$) δ ppm 10.05 (s, 1H), 7.91 (d, *J* = 8.0 Hz, 1H), 7.88 (s, 1H), 7.56 - 7.44 (m, 1H), 5.36 - 5.23 (m, 1H), 4.93 - 4.73 (m, 2H), 3.46 - 3.06 (m, 1H), 3.00 - 2.90 (m, 1H), 1.57 - 1.51 (m, 9H).

**Intermediate B59: *tert*-butyl 7-(cyanomethyl)-2-formyl-5,7-dihydro-6*H*-pyrrolo[3,4-*b*]pyridine-6-carboxylate**

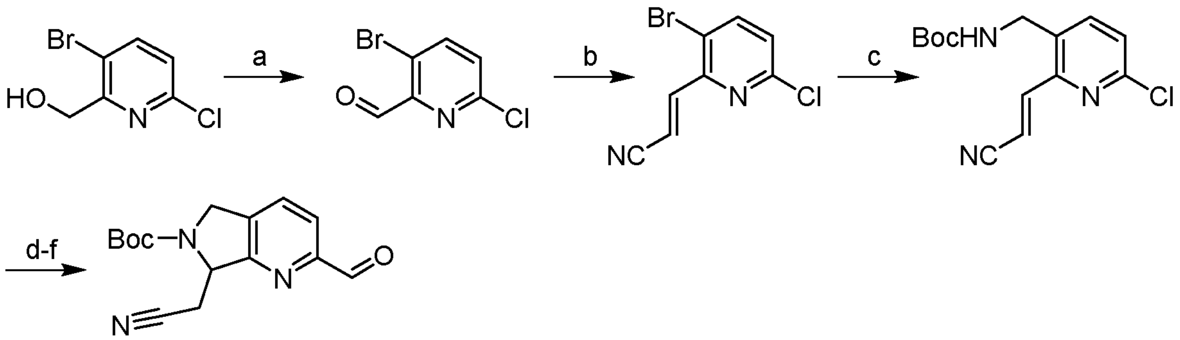


**Step a.** A mixture of (3-bromo-6-chloropyridin-2-yl)methanol (CAS: 1227601-71-9; 1 g, 4.50 mmol) and $MnO_2$ (3.91 g, 45.0 mmol) in DCM (5 mL) was stirred at 25 °C for 12 h under a $N_2$ atmosphere. Upon completion, the mixture was evaporated and purified by column chromatography (PE/EtOAc = 5/1) to give 3-bromo-6-chloropicolinaldehyde (300 mg, 30% yield) as a white solid.

$^1$H NMR (400 MHz, $CDCl_3$) δ ppm 10.04 (s, 1H), 7.92 (d, *J* = 8.4 Hz, 1H), 7.33 (d, *J* = 8.4 Hz, 1H).

**Step b.** A mixture of 3-bromo-6-chloropicolinaldehyde (800 mg, 3.63 mmol) and 2-(triphenyl-λ$^5$-phosphaneylidene)acetonitrile (CAS: 16640-68-9; 1.64 g, 5.44 mmol) in DCM (5 mL) was stirred at 25 °C for 4 h under a $N_2$ atmosphere. Upon completion, the mixture was evaporated and purified by column chromatography (PE/EtOAc = 5/1) to give (E)-3-(3-bromo-6-chloropyridin-2-yl)acrylonitrile (0.85 g, 96% yield) as a white solid.

$^1$H NMR (400 MHz, $CDCl_3$) δ ppm 7.91 - 7.87 (m, 1H), 7.79 (d, *J* = 15.6 Hz, 1H), 7.24 (d, *J* = 8.4 Hz, 1H), 6.72 (d, *J* = 11.6 Hz, 1H).

**Step c.** A mixture of potassium (((*tert*-butoxycarbonyl)amino)methyl)trifluoroborate (CAS: 1314538-55-0; 535 mg, 2.26 mmol), (*E*)-3-(3-bromo-6-chloropyridin-2-yl)acrylonitrile (500 mg, 2.05 mmol), cataCXium-A-Pd-G3 (CAS: 1651823-59-4; 149 mg, 0.21 mmol) and $Cs_2CO_3$ (2.01 g, 6.16 mmol) in 1,4-dioxane (2 mL) and water (0.4 mL) was degassed and purged with $N_2$ 3 times. The reaction mixture was stirred at 100 °C for 7 h under a $N_2$ atmosphere. Upon completion, the mixture was filtered, evaporated and purified by column chromatography (PE/EtOAc = 3/1) to give *tert*-butyl (*E*)-((6-chloro-2-(2-cyanovinyl)pyridin-3-yl)methyl)carbamate (0.38 g, 63% yield) as a white solid.

m/z ES+ $[M+H]^+$ 294.1; $^1$H NMR (400 MHz, $CDCl_3$) δ ppm 7.69 - 7.63 (m, 2H), 6.96 - 6.72 (m, 2H), 4.75 (br s, 1H), 4.42 (d, *J* = 6.0 Hz, 2H), 1.48 (s, 9H).

**Steps d-f.** These 3 steps were conducted in a similar manner to **Intermediate B57,** steps d-f.

$^1$H NMR (400 MHz, $CDCl_3$) δ ppm 10.06 - 9.92 (m, 1H), 7.91 (d, *J* = 7.6 Hz, 1H), 7.83 - 7.69 (m, 1H), 5.21 - 5.10 (m, 1H), 4.88 - 4.70 (m, 2H), 3.61 - 3.21 (m, 1H), 3.08 - 3.06 (m, 1H), 1.51 - 1.45 (m, 9H).

**Intermediate B60: *tert*-butyl 5-(cyanomethyl)-2-formyl-5,7-dihydro-6*H*-pyrrolo[3,4-*b*]pyridine-6-carboxylate**

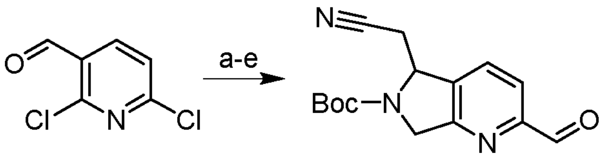


**Steps a-e.** These 5 steps were conducted in a similar manner to **Intermediate B59,** steps b-f, using 2,6-dichloronicotinaldehyde (CAS: 55304-73-9) in step a.

$^1$H NMR (400 MHz, $CDCl_3$) δ ppm 10.22 - 9.97 (m, 1H), 8.00 - 7.92 (m, 2H), 5.39 - 5.28 (m, 1H), 4.82 (s, 2H), 3.30 - 3.03 (m, 2H), 1.58 - 1.55 (m, 9H).

**Intermediate B61: *tert*-butyl 5-(cyanomethyl)-3-formyl-5,7-dihydro-6*H*-pyrrolo[3,4-*b*]pyridine-6-carboxylate**

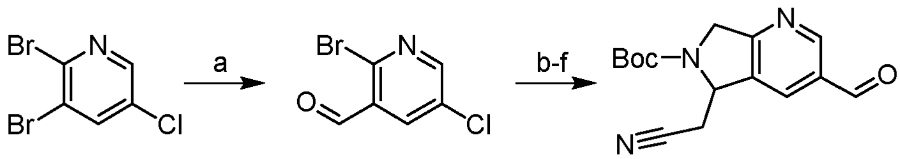


**Step a.** A solution of isopropylmagnesium chloride lithium complex (1.3 M in THF, 31.2 mL) was added dropwise to a solution of 2,3-dibromo-5-chloropyridine (CAS: 137628-17-2; 10 g, 36.8 mmol) in THF (100 mL) at -40 °C under a $N_2$ atmosphere. The mixture was stirred at - 40 °C for 1 h, after which DMF (10 mL) was added dropwise and the reaction mixture was allowed to warm to 25 °C over 30 min. Upon completion, the mixture was quenched with 1 M HCl and extracted with EtOAc (2 x 80 mL). The combined organic layers were washed with brine (60 mL), dried over $Na_2SO_4$, evaporated and purified by column chromatography (PE/EtOAc = 9/1) to give 2-bromo-5-chloronicotinaldehyde (9.5 g, 58% yield) as a pink solid. $^1$H NMR (400 MHz, $CDCl_3$) δ ppm 10.29 (s, 1H), 8.55 (d, *J* = 2.8 Hz, 1H), 8.13 (d, *J* = 2.8 Hz, 1H).

**Steps b-f.** These 5 steps were conducted in a similar manner to **Intermediate B59,** steps b-f.

m/z ES+ $[M+H]^+$ 288.1; $^1$H NMR (400 MHz, $CDCl_3$) δ ppm 10.16 (s, 1H), 9.07 (s, 1H), 8.20 (s, 1H), 5.37 - 5.32 (m, 1H), 4.99 - 4.79 (m, 2H), 3.41 - 3.01 (m, 2H), 1.58 - 1.53 (m, 9H).

**Intermediate B62: *tert*-butyl 7-(cyanomethyl)-3-formyl-5,7-dihydro-6*H*-pyrrolo[3,4-*b*]pyridine-6-carboxylate**

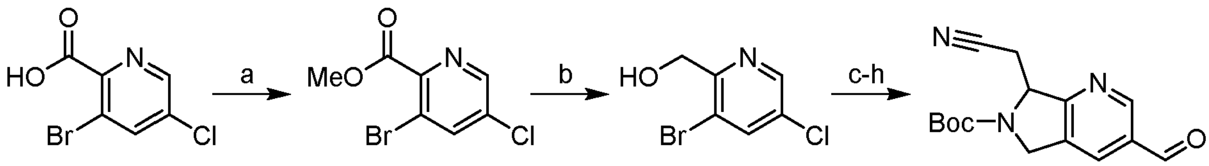


**Step a.** To a solution of 3-bromo-5-chloropicolinic acid (CAS: 1189513-50-5; 10 g, 42.3 mmol) in MeOH (100 mL) was added $H_2SO_4$ (20.7 g, 211 mmol) at 0 °C. The reaction mixture was stirred at 90 °C for 4 h. Upon completion, the mixture was quenched with water (500 mL) at 0°C and extracted with EtOAc (2 x 500 mL). The combined organic layers were washed with brine (2 x 1 L), dried over $Na_2SO_4$ and evaporated to give the methyl 3-bromo-5-chloropicolinate (9.4 g, 89% yield) as a white solid.

$^1$H NMR (400 MHz, DMSO-$d_6$) δ ppm 8.72 (s, 1H), 8.56 (s, 1H), 3.91 (s, 3H).

**Steps c-h.** These 6 steps were conducted in a similar manner to **Intermediate B59,** steps a-f.

m/z ES+ $[M+H]^+$ 288.2; $^1$H NMR (400 MHz, DMSO-$d_6$) δ ppm 10.13 (s, 1H), 9.08 (s, 1H), 8.30 (d, *J* = 6.0 Hz, 1H), 5.27 - 5.16 (m, 1H), 4.83 - 4.57 (m, 2H), 3.50 - 3.32 (m, 1H), 3.25 - 3.13 (m, 1H), 1.50 (s, 9H).

**Intermediate B63: *tert*-butyl 2-formyl-1-methyl-4,6-dihydropyrrolo[3,4-*d*]imidazole-5(1*H*)-carboxylate**

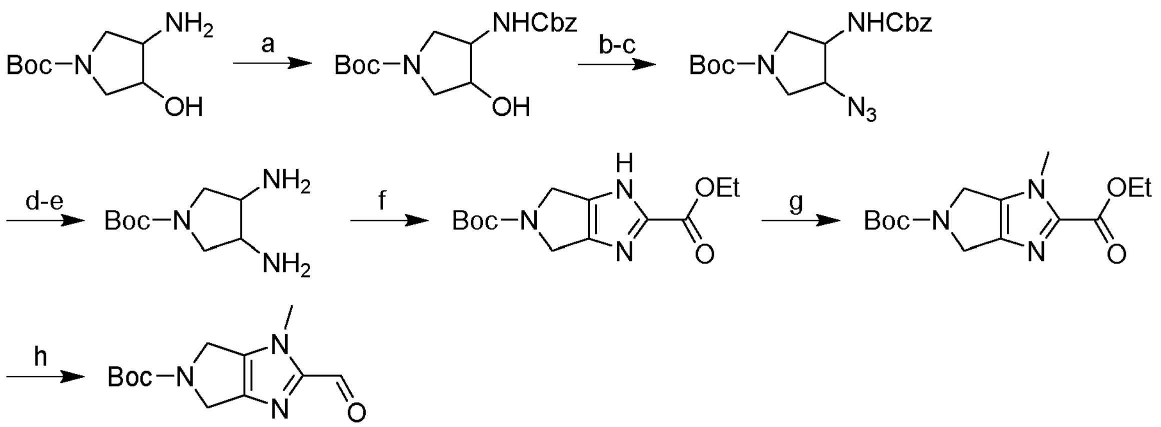


**Step a.** To a mixture of *tert*-butyl 3-amino-4-hydroxypyrrolidine-1-carboxylate (CAS: 190141-99-2; 37 g, 183 mmol) and $NaHCO_3$ (46.1 g, 548.8 mmol) in THF (300 mL) and water (100 mL) was added CbzCl (37.5 g, 219 mmol). The reaction mixture was stirred at 25 °C for 12 h under a $N_2$ atmosphere. Upon completion, the mixture was diluted with water (600 mL) and extracted with EtOAc (3 x 200 mL). The combined organic layers were washed with brine (200 mL), dried over $Na_2SO_4$, evaporated and purified by column chromatography (PE/EtOAc = 5/1 to 1/1) to give *tert*-butyl 3-(((benzyloxy) carbonyl)amino)-4-hydroxypyrrolidine-1-carboxylate (60 g, 97% yield) as a yellow oil.

$^1H$ NMR (400 MHz, $CDCl_3$) δ ppm 7.38 - 7.32 (m, 5H), 5.10 (s, 2H), 5.06 - 4.87 (m, 1H), 4.25 (br s, 1H), 4.05 - 3.92 (m, 1H), 3.85 - 3.75 (m, 1H), 3.68 - 3.55 (m, 1H), 3.50 - 2.98 (m, 3H), 1.45 (s, 9H).

**Step b.** To a solution of *tert*-butyl 3-(((benzyloxy)carbonyl)amino)-4-hydroxypyrrolidine-1-carboxylate (40 g, 118 mmol) and TEA (36.1 g, 356 mmol) in DCM (400 mL) was added MsCl (27.8 g, 242 mmol). The reaction mixture was stirred at 0 °C for 1 h. Upon completion, the mixture was quenched with water (500 mL) at 0 °C and extracted with DCM (2 x 300mL). The combined organic layers were washed with brine (250 mL), dried over $Na_2SO_4$ and evaporated to give *tert*-butyl 3-(((benzyloxy)carbonyl)amino)-4-((methylsulfonyl)oxy)pyrrolidine-1-carboxylate (50 g, crude) as a yellow oil.

$^1H$ NMR (400 MHz, $CDCl_3$) δ ppm 7.38 - 7.32 (m, 5H), 5.21 - 5.01 (m, 4H), 4.30 - 4.13 (m, 1H), 3.84 - 3.56 (m, 3H), 3.48 - 3.30 (m, 1H), 3.14 (s, 3H), 1.45 (s, 9H).

**Step c.** A mixture of *tert*-butyl 3-(((benzyloxy)carbonyl)amino)-4-((methylsulfonyl)oxy)pyrrolidine-1-carboxylate (25 g, 60.3 mmol) and $NaN_3$ (19.3 g, 296.6 mmol) in DMF (250 mL) was stirred at 100 °C for 12 h under a $N_2$ atmosphere. Upon completion, the mixture was quenched with sat. aq. $NaHCO_3$ (50 mL) at 25 °C, diluted with water (1000 mL) and extracted with EtOAc (3 x 200 mL). The combined organic layers were washed with brine (200 mL), dried over $Na_2SO_4$ and filtered to give a solution of *tert-butyl 3*-azido-4-(((benzyloxy)carbonyl)amino)pyrrolidine-1-carboxylate (21 g, crude) in EtOAc as a yellow liquid that was used directly in the next step without further purification.

m/z ES+ $[M+Na]^+$ 384.0.

**Step d.** To a solution of *tert*-butyl 3-azido-4-(((benzyloxy)carbonyl)amino)pyrrolidine-1-carboxylate (21 g, 58.1 mmol) in EtOAc was added MeOH (500 mL) and 10% Pd/C (2 g) under a $N_2$ atmosphere. The suspension was degassed, purged with $H_2$ 3 times and then stirred at 25 °C for 12 h under a $H_2$ atmosphere (15 psi). Upon completion, the mixture was filtered and evaporated to give *tert*-butyl 3-amino-4-(((benzyloxy)carbonyl)amino)pyrrolidine-1-carboxylate (20 g, crude) as a yellow oil.

m/z ES+ $[M+H]^+$ 336.1; $^1H$ NMR (400 MHz, $CDCl_3$) δ ppm 7.43 - 7.32 (m, 5H), 5.57 - 5.38 (m, 1H), 5.13 (s, 2H), 4.22 - 4.10 (m, 1H), 3.71 - 3.67 (m, 1H), 3.64 - 3.50 (m, 2H), 3.33 - 3.07 (m, 2H), 1.47 (s, 9H).

**Step e.** To a solution of *tert*-butyl 3-amino-4-(((benzyloxy)carbonyl)amino)pyrrolidine-1-carboxylate (40 g, 119 mmol) in MeOH (500 mL) was added 10% Pd/C (6 g) under a $N_2$ atmosphere. The suspension was degassed and purged with $H_2$ 3 times and the reaction mixture was stirred at 25 °C for 12 h under a $H_2$ atmosphere (40 psi). Upon completion, the reaction mixture was filtered and evaporated to give *tert*-butyl 3,4-diaminopyrrolidine-1-carboxylate (16 g, crude) as a yellow oil.

$^1H$ NMR (400 MHz, $CDCl_3$) δ ppm 3.71 - 3.67 (m, 1H), 3.64 - 3.48 (m, 2H), 3.37 - 3.34 (m, 1H), 3.20 - 3.07 (m, 2H), 1.47 (s, 9H).

**Step f.** A mixture of *tert*-butyl 3,4-diaminopyrrolidine-1-carboxylate (5 g, 24.8 mmol) and ethyl 2-ethoxy-2-iminoacetate (CAS: 816-27-3; 3.61 g, 24.8 mmol) in HFIP (50 mL) was stirred at 60 °C for 12 h under a $N_2$ atmosphere. Upon completion, the reaction mixture was evaporated to give 5-(*tert*-butyl) 2-ethyl 3a,4,6,6a-tetrahydropyrrolo[3,4-*d*] imidazole-2,5(1*H*)-dicarboxylate (7.04 g, crude) as a colourless oil.

m/z ES+ $[M+H]^+$ 284.1.

To a solution of oxalyl chloride (7.42 mL, 84.7 mmol) in DCM (100 mL) at -65 °C was added DMSO (13.2 mL, 169 mmol). The mixture was stirred for 30 min, after which, the crude 5-(*tert*-butyl) 2-ethyl 3a,4,6,6a-tetrahydropyrrolo[3,4-*d*]imidazole-2,5(1*H*)-dicarboxylate (8 g, 28.2 mmol) in DCM (20 mL) was added at -65 °C. The mixture was stirred for a further 30 min and then TEA (25.7 g, 254.1 mmol) was added and the reaction mixture was allowed to warm to 25 °C over 1 h. Upon completion, the reaction mixture was evaporated, diluted with water (100 mL) and extracted with EtOAc (3 x 50 mL). The combined organic layers were washed with brine (50 mL), dried over $Na_2SO_4$ and evaporated to give *5-(tert-butyl)* 2-ethyl 4,6-dihydropyrrolo[3,4-*d*]imidazole-2,5(1*H*)-dicarboxylate (8 g, crude) as a yellow oil.

m/z ES+ $[M+H]^+$ 282.1; $^1H$ NMR (400 MHz, $CDCl_3$) δ ppm 4.53 - 4.33 (m, 6H), 1.51 - 1.40 (m, 9H), 1.23 - 1.17 (m, 3H).

**Step g.** To a mixture of 5-(*tert*-butyl) 2-ethyl 4,6-dihydropyrrolo[3,4-*d*]imidazole-2,5(1*H*)-dicarboxylate (7 g, 24.8 mmol) and $K_2CO_3$ (10.3 g, 74.7 mmol) in DMF (100 mL) was added MeI (3.53 g, 24.9 mmol). The reaction mixture was stirred at 25 °C for 12 h under a $N_2$ atmosphere. Upon completion, the mixture was quenched with water (200 mL) at 25 °C and extracted with EtOAc (3 x 100 mL). The combined organic layers were washed with brine (50 mL), dried over $Na_2SO_4$, evaporated and purified by column chromatography (PE/EtOAc = 1/1 to 0/1) to give 5-(*tert*-butyl) 2-ethyl 1-methyl-4,6-dihydropyrrolo[3,4-*d*]imidazole-2,5(1*H*)-dicarboxylate (2.4 g, 33% yield) as a yellow solid.

m/z ES+ $[M+H]^+$ 296.1; $^1H$ NMR (400 MHz, $CDCl_3$) δ ppm 4.52 - 4.38 (m, 6H), 3.96 (s, 3H), 1.51 (s, 9H), 1.46 - 1.42 (m, 3H).

**Step h.** To a solution of 5-(*tert*-butyl) 2-ethyl 1-methyl-4,6-dihydropyrrolo[3,4-*d*]imidazole-2,5(1*H*)-dicarboxylate (2.1 g, 7.11 mmol) in DCM (100 mL) was added DIBAL-H (1 M in toluene, 21.3 mL). The reaction mixture was stirred at -78 °C for 1 h. Upon completion, the mixture was quenched by with MeOH (4 mL) at -65 °C, and then stirred at 25 °C for 1 h. The mixture was filtered and the filtrate was evaporated to give the title compound (1.1 g, 62% yield) as a yellow solid.

$^1H$ NMR (400 MHz, $CDCl_3$) δ ppm 9.72 (s, 1H), 4.59 - 4.41 (m, 4H), 3.98 (d, *J* = 2.8 Hz, 3H), 1.53 (s, 9H).

**Intermediate B64: *tert*-butyl 1-ethyl-2-formyl-4,6-dihydropyrrolo[3,4-*d*]imidazole-5(1*H*)-carboxylate**

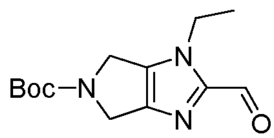

The title compound was prepared in a similar manner to **Intermediate B63,** using iodoethane in step g.

$^1H$ NMR (400 MHz, $CDCl_3$) δ ppm 9.73 (s, 1H), 4.63 - 4.37 (m, 6H), 1.54 (s, 9H), 1.46 - 1.42 (m, 3H).

**Intermediate B65: *tert*-butyl 2-formyl-4,6-dihydro-5*H*-pyrrolo[3,4-*d*]thiazole-5-carboxylate**

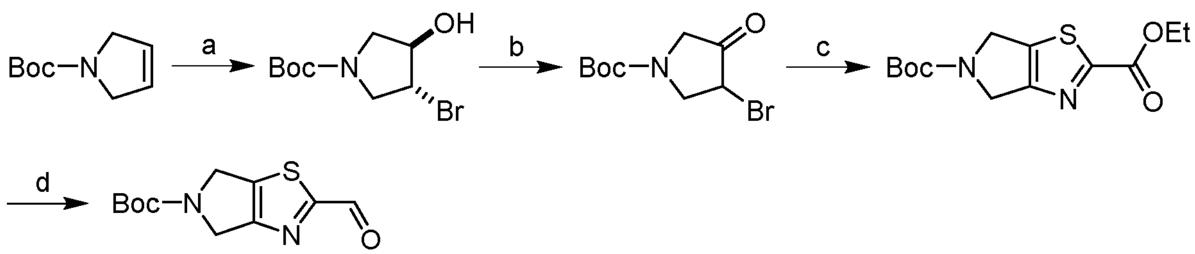


**Step a.** To a mixture of *tert*-butyl 2,5-dihydro-1*H*-pyrrole-1-carboxylate (5 g, 29.6 mmol) in water (2.5 mL) and DMSO (50 mL) was added NBS (6.84 g, 38.4 mmol) at 0 °C. The reaction mixture was stirred at 25 °C for 2 h. Upon completion, the mixture was diluted with water (200 mL) and extracted with EtOAc (2 x 100 mL). The combined organic layers were washed with brine (200 mL), dried over $Na_2SO_4$, evaporated and purified by column chromatography (PE/EtOAc = 1/1) to give *tert*-butyl (*rel-trans*)-3-bromo-4-hydroxypyrrolidine-1-carboxylate (6.85 g, 87% yield) as a colourless oil.

$^1H$ NMR (400 MHz, $CDCl_3$) δ ppm 4.47 (s, 1H), 4.22 - 4.09 (m, 1H), 4.05 - 3.95 (m, 1H), 3.92 - 3.67 (m, 2H), 3.48 - 3.33 (m, 1H), 3.01 - 2.78 (m, 1H), 1.47 (s, 9H).

**Step b.** To a solution of *tert*-butyl (*rel-trans*)-3-bromo-4-hydroxypyrrolidine-1-carboxylate (6.85 g, 25.7 mmol) in DCM (70 mL) was added DMP (21.8 g, 51.5 mmol). The reaction mixture was stirred at 20 °C for 12 h. Upon completion, the mixture was filtered, evaporated and purified by column chromatography (PE/EtOAc = 1/1) to give *tert*-butyl 3-bromo-4-oxopyrrolidine-1-carboxylate (6.4 g, 94% yield) as a black solid.

$^1$H NMR (400 MHz, $CDCl_3$) δ ppm 4.41 - 4.36 (m, 1H), 4.20 - 4.12 (m, 1H), 4.11 - 4.03 (m, 1H), 4.02 - 3.90 (m, 1H), 3.86 - 3.72 (m, 1H), 1.49 (s, 9H).

**Step c.** A mixture of *tert*-butyl 3-bromo-4-oxopyrrolidine-1-carboxylate (3 g, 11.4 mmol), ethyl 2-amino-2-thioxoacetate (CAS: 16982-21-1; 1.51 g, 11.4 mmol), PPTS (856 mg, 3.41 mmol) and 4 Å molecular sieves (6 g, 3.79 mmol) in toluene (50 mL) was stirred at 120 °C for 16 h under a $N_2$ atmosphere. Upon completion, the reaction mixture was evaporated, diluted with water (30 mL) and extracted with EtOAc (30 mL). The organic layer was washed with brine (30 mL), dried over $Na_2SO_4$, evaporated and purified by column chromatography (PE/EtOAc = 5/1) followed by reverse phase flash chromatography (water (0.1% FA)/MeCN) to give 5-(*tert*-butyl) 2-ethyl 4,6-dihydro-5*H*-pyrrolo[3,4-*d*]thiazole-2,5-dicarboxylate (740 mg, 22% yield) as a yellow solid.

m/z ES+ $[M+H]^+$ 299.3; $^1$H NMR (400 MHz, $CDCl_3$) δ ppm 4.79 - 4.72 (m, 2H), 4.70 - 4.65 (m, 1H), 4.63 - 4.58 (m, 1H), 4.53 - 4.45 (m, 2H), 1.53 (s, 9H), 1.49 - 1.43 (m, 3H).

**Step d.** This step was conducted in a similar manner to **Intermediate B63,** step h.

$^1$H NMR (400 MHz, $CDCl_3$) δ ppm 9.95 (s, 1H), 4.83 - 4.72 (m, 2H), 4.72 - 4.62 (m, 2H), 1.53 (s, 9H).

**Intermediate B66: *tert*-butyl 2-formyl-4,6-dihydro-5*H*-pyrrolo[3,4-*d*]oxazole-5-carboxylate**

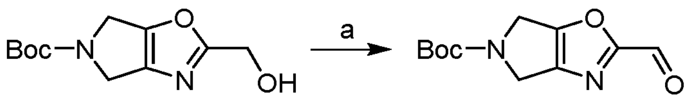


**Step a.** This step was conducted in a similar manner to **Intermediate B1,** step c, using *tert*-butyl 2-(hydroxymethyl)-4,6-dihydro-5*H*-pyrrolo[3,4-*d*]oxazole-5-carboxylate (CAS: 1251012-01-7) in step a.

$^1$H NMR (400 MHz, $CDCl_3$) δ ppm 9.71 (s, 1H), 4.61 - 4.57 (m, 2H), 4.51 - 4.46 (m, 2H), 1.53 (s, 9H).

**Intermediate B67a: 5-(*tert*-butyl) 3-methyl 1-methyl-4,6-dihydropyrrolo[3,4-*c*]pyrazole-3,5(1*H*)-dicarboxylate and**

**Intermediate B67b: 5-(*tert*-butyl) 3-methyl 2-methyl-2,6-dihydropyrrolo[3,4-*c*]pyrazole-3,5(4*H*)-dicarboxylate**

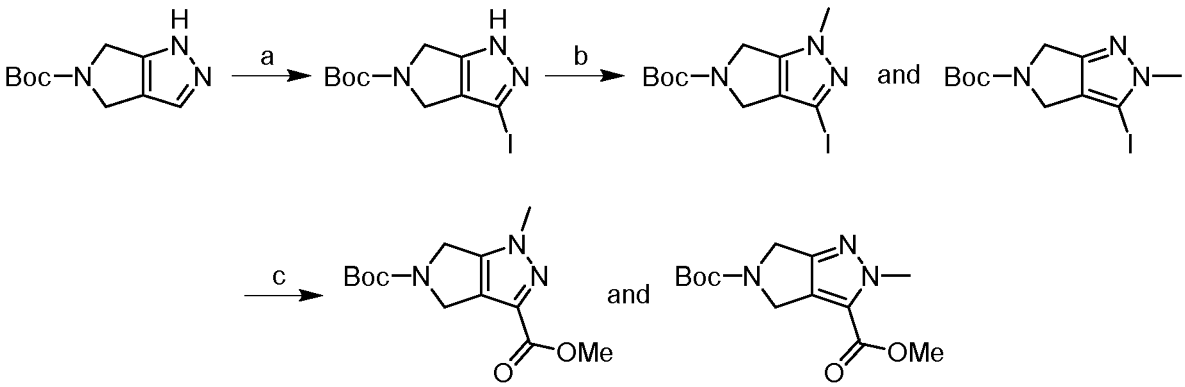


**Step a.** To a solution of *tert*-butyl 4,6-dihydropyrrolo[3,4-c]pyrazole-5(1*H*)-carboxylate (CAS: 657428-42-7; 15.0 g, 71.7 mmol) in DCE (100 mL) was added NIS (24.2 g, 107.5 mmol). The reaction mixture was stirred at 80 °C for 12 h under a $N_2$ atmosphere. Upon completion, the mixture was quenched with water (120 mL) and extracted with EtOAc (3 x 150 mL). The combined organic layers were washed with brine (150 mL), dried over $Na_2SO_4$, evaporated and purified by column chromatography (PE/EtOAc = 1/1) to give *tert*-butyl 3-iodo-4,6-dihydropyrrolo[3,4-c]pyrazole-5(1*H*)-carboxylate (1.50 g, 6% yield) as a yellow solid.

m/z ES+ $[M+H]^+$ 336.0; $^1$H NMR (400 MHz, $CDCl_3$) δ ppm 4.62- 4.52 (m, 2H), 4.39 - 4.36 (m, 2H), 1.53 - 1.51 (m, 9H).

**Step b.** To a mixture of *tert*-butyl 3-iodo-4,6-dihydropyrrolo[3,4-*c*]pyrazole-5(1*H*)-carboxylate (1.50 g, 4.48 mmol) and $K_2CO_3$ (1.86 g, 13.4 mmol) in DMF (6 mL) was added MeI (6.99 g, 49.2 mmol). The reaction mixture was stirred at 25 °C for 12 h under a $N_2$ atmosphere. Upon completion, the mixture was quenched with water (60 mL) and

extracted with EtOAc (2 x 100 mL). The combined organic layers were washed with brine (60 mL), dried over $Na_2SO_4$, and evaporated to give an inseparable mixture of *tert*-butyl 3-iodo-1-methyl-4,6-dihydropyrrolo[3,4-*c*]pyrazole-5(1*H*)-carboxylate and *tert*-butyl 3-iodo-2-methyl-2,6-dihydropyrrolo[3,4-*c*]pyrazole-5(4*H*)-carboxylate (3:2 ratio; 1.2 g, 77% yield) as a red solid.

m/z ES+ $[M+H]^+$ 349.9

**Step c.** A mixture of *tert*-butyl 3-iodo-1-methyl-4,6-dihydropyrrolo[3,4-*c*]pyrazole-5(1*H*)-carboxylate and *tert*-butyl 3-iodo-2-methyl-2,6-dihydropyrrolo[3,4-*c*]pyrazole-5(4*H*)-carboxylate (1.15 g, 3.3 mmol), Pd(dppf)$Cl_2$ (241 mg, 0.33 mmol) and TEA (3.33 g, 33 mmol) in MeOH (100 mL) was stirred at 50 °C for 16 h under a CO atmosphere (50 psi). Upon completion, the mixture was evaporated and purified by column chromatography (PE/EtOAc = 1/1) to give two products. The regiochemistry was confirmed by 2D NMR.

**Intermediate B67a:** 5-(*tert*-butyl) 3-methyl 1-methyl-4,6-dihydropyrrolo[3,4-*c*]pyrazole-3,5(1*H*)-dicarboxylate (450 mg, 49% yield) as a red solid.

m/z ES+ $[M+H]^+$ 282.2; $^1H$ NMR (400 MHz, $CDCl_3$) δ ppm 4.58 - 4.48 (m, 4H), 3.92 (s, 3H), 3.89 (s, 3H), 1.51 (s, 9H).

**Intermediate B67b:** 5-(*tert*-butyl) 3-methyl 2-methyl-2,6-dihydropyrrolo[3,4-*c*]pyrazole-3,5(4*H*)-dicarboxylate (270 mg, 29 % yield) as a white solid.

m/z ES+ $[M+H]^+$ 282.2; $^1H$ NMR (400 MHz, $CDCl_3$) δ ppm 4.58 - 4.43 (m, 4H), 4.18 (s, 3H), 3.88 (d, *J* = *12.8* Hz, 3H), 1.52 (d, *J* = 3.2 Hz, 9H).

**Intermediate B68: *tert*-butyl 3-formyl-1-methyl-4,6-dihydropyrrolo[3,4-*c*]pyrazole-5(1*H*)-carboxylate**

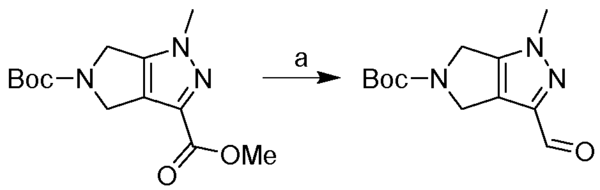


**Steps a.** This step was conducted in a similar manner to **Intermediate B53,** step c, using **Intermediate B67a.**

m/z ES+ $[M+H]^+$ 252.3; $^1H$ NMR (400 MHz, $CDCl_3$) δ ppm 9.89 (s, 1H), 4.62 - 4.47 (m, 4H), 3.92 (s, 3H), 1.50 (s, 9H).

**Intermediate B69: 6-(difluoromethyl)imidazo[1,2-*a*]pyrazine-2-carbaldehyde**

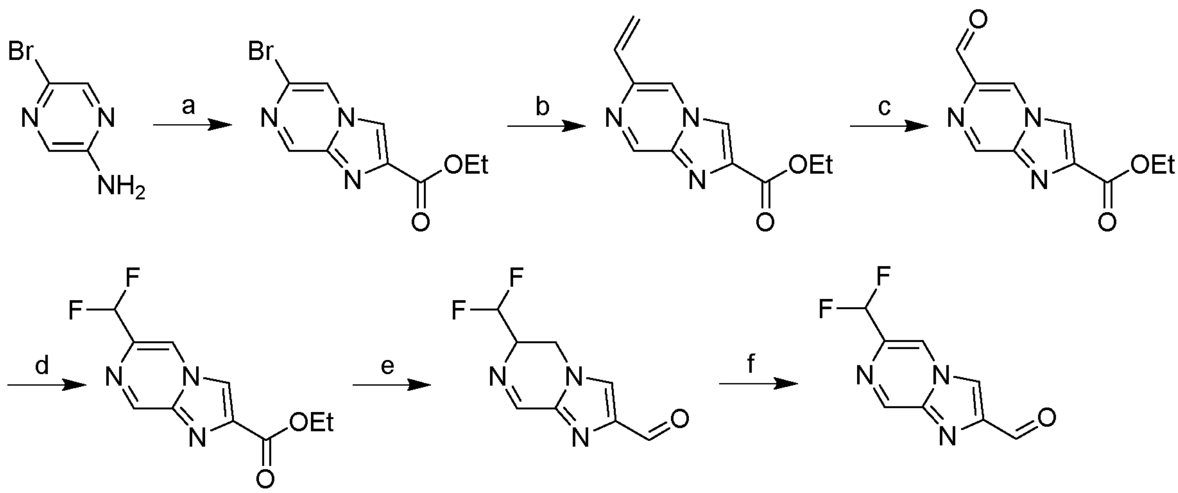

**Step a.** To a solution of 5-bromopyrazin-2-amine (59489-71-3; 15 g, 86.2 mmol) in 1,4-dioxane (150 mL) was added $NaHCO_3$ (21.7 g, 259 mmol) and ethyl 3-bromo-2-oxopropanoate (25.2 g, 129 mmol). The reaction mixture was stirred at 100 °C for 16 h. Upon completion, the mixture was filtered, evaporated and purified by column chromatography (EtOAc to EtOAc/MeOH = 20/1) to give ethyl 6-bromoimidazo[1,2-a]pyrazine-2-carboxylate (11.5 g, 49% yield) was obtained as yellow solid.

m/z ES+ $[M+H]^+$ 272.0; $^1$H NMR (400 MHz, $CDCl_3$) δ ppm 9.01 (s, 1H), 8.31 (s, 1H), 8.24 (s, 1H), 4.53 - 4.47 (m, 2H), 1.46 (t, *J* = 7.2 Hz, 3H).

**Step b.** A mixture of ethyl 6-bromoimidazo[1,2-a]pyrazine-2-carboxylate (10.5 g, 38.9 mmol), potassium trifluoro(vinyl)borate (CAS: 13682-77-4; 52.1 g, 389 mmol), $Cs_2CO_3$ (38.0 g, 117 mmol) and XPhos-Pd-G2 (3.06 g, 3.89 mmol) in 1,4-dioxane (150 mL) and water (150 mL) was degassed and purged with $N_2$ 3 times. The reaction mixture was stirred at 100 °C for 16 h under a $N_2$ atmosphere. Upon completion, the mixture diluted with water (800 mL) and extracted with EtOAc (3 x 300 mL). The combined organic layers were dried over $Na_2SO_4$, evaporated and purified by column chromatography (PE/EtOAc = 4/1 to 2/1) to give ethyl 6-vinylimidazo[1,2-*a*]pyrazine-2-carboxylate (5.5 g, 65% yield) as a yellow solid.

$^1$H NMR (400 MHz, $CDCl_3$) δ ppm 9.17 (s, 1H), 8.21 (s, 1H), 7.96 (s, 1H), 6.74 - 6.67 (m, 1H), 6.39 - 6.35 (m, 1H), 5.56 - 5.53 (m, 1H), 4.52 - 4.46 (m, 2H), 1.46 (t, *J* = 7.2 Hz, 3H).

**Step c.** This step was conducted in a similar manner to **Intermediate B49,** step h.

m/z ES+ $[M+H]^+$ 220.0; $^1$H NMR (400 MHz, $CDCl_3$) δ ppm 10.17 (s, 1H), 9.29 (s, 1H), 8.81 (s, 1H), 8.41 (s, 1H), 4.55 - 4.49 (m, 2H), 1.48 (t, *J* = 7.2 Hz, 3H).

**Step d.** To a solution of ethyl 6-formylimidazo[1,2-*a*]pyrazine-2-carboxylate (2 g, 9.12 mmol) in DCM (100 mL) was added DAST (5.88 g, 36.5 mmol) at -78°C. The reaction mixture was allowed to warm to 0 °C over 16 h. Upon completion, the mixture was diluted with sat. aq. $NaHCO_3$ (50 mL) and extracted with EtOAc (3 x 20 mL). The combined organic layers were washed with brine (3 x 20 mL), dried over $Na_2SO_4$, evaporated and purified by column chromatography (PE/EtOAc = 3/1 to 1/1) to give ethyl 6-(difluoromethyl)imidazo[1,2-a]pyrazine-2-carboxylate (1.24 g, 56% yield) as a yellow solid.

$^1$H NMR (400 MHz, $CDCl_3$) δ ppm 9.22 (s, 1H), 8.46 (s, 1H), 8.36 (s, 1H), 6.95 - 6.65 (m, 1H), 4.54 - 4.48 (m, 2H), 1.47 (t, *J = 7.2* Hz, 3H).

**Step e.** This step was conducted in a similar manner to **Intermediate B63,** step h.

$^1$H NMR (400 MHz, $CDCl_3$) δ ppm 9.76 (s, 1H), 7.47 (s, 1H), 6.26 (d, *J* = 1.6 Hz, 1H), 6.21 - 5.92 (m, 1H), 4.89 - 4.84 (m, 1H), 4.62 (d, *J* = 2.0 Hz, 2H).

**Step f.** To a solution of 6-(difluoromethyl)-5,6-dihydroimidazo[1,2-*a*]pyrazine-2-carbaldehyde (660 mg, 3.31 mmol) in DCM (50 mL) was added $MnO_2$ (5.76 g, 66.3 mmol). The reaction mixture was stirred at 25 °C for 16 h. Upon completion, the mixture was filtered and evaporated to give the title compound (490 mg, crude) as a red solid.

$^1$H NMR (400 MHz, $CDCl_3$) δ ppm 10.24 (s, 1H), 9.25 (s, 1H), 8.50 (s, 1H), 8.32 (s, 1H), 6.96 - 6.65 (m, 1H).

**Intermediate B70: 6-(((*tert*-butyldiphenylsilyl)oxy)methyl)-5,6-dihydroimidazo[1,2-*a*]pyrazine-2-carbaldehyde**

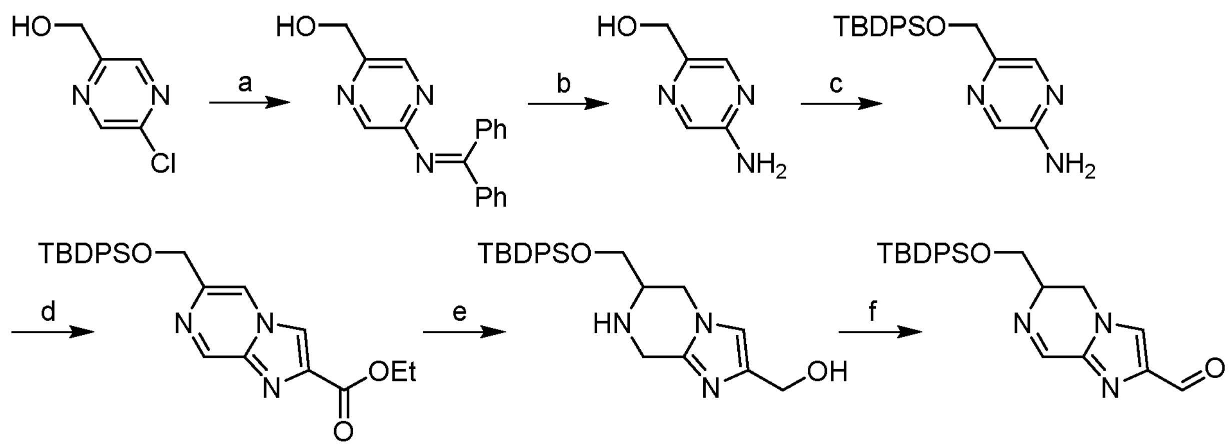


**Step a.** A mixture of (5-chloropyrazin-2-yl)methanol (CAS: 72788-94-4; 8.4 g, 58.1 mmol), diphenylmethanimine (12.6 g, 69.7 mmol), $Pd_2(dba)_3$ (2.66 g, 2.91 mmol), Xantphos (3.36 g, 5.81 mmol) and $Cs_2CO_3$ (56.8 g, 174 mmol) in 1,4-dioxane (180 mL) was degassed and purged with $N_2$ 3 times. The reaction mixture was stirred at 110 °C for 36 h under a $N_2$ atmosphere. Upon completion, the reaction mixture was diluted with water (200 mL) and extracted with EtOAc (3 X 100

mL). The combined organic layers were dried over $Na_2SO_4$, evaporated and purified by column chromatography (PE/EtOAc = 4/1 to 1/1) to give (5-((diphenylmethylene)amino)pyrazin-2-yl)methanol (3.32 g, 20% yield) as a yellow oil.

m/z ES+ $[M+H]^+$ 290.1; $^1H$ NMR (400 MHz, $CDCl_3$) δ ppm 8.22 (d, *J* = 1.2 Hz, 1H), 7.83 (d, *J* = 1.2 Hz, 1H), 7.75 (d, *J* = 7.6 Hz, 2H), 7.51 - 7.43 (m, 1H), 7.37 (t, *J* = 7.2 Hz, 2H), 7.31 - 7.20 (m, 3H), 7.09 (d, *J* = 6.8 Hz, 2H), 4.65 (d, *J* = 4.4 Hz, 2H), 2.75 - 2.65 (m, 1H).

**Step b.** To a solution of 5-((diphenylmethylene)amino)pyrazin-2-yl)methanol (2.1 g, 7.26 mmol) in DCM (30 mL) was added TFA (6 mL). The reaction mixture was stirred at 20 °C for 16 h. Upon completion, the mixture was evaporated to give (5-aminopyrazin-2-yl)methanol (1.8 g, crude) as a red oil.

$^1H$ NMR (400 MHz, $CDCl_3$) δ ppm 8.50 (s, 1H), 7.95 (s, 1H), 5.39 (s, 2H).

**Step c.** This step was conducted in a similar manner to **Intermediate B50a** and **Intermediate B50b,** step a, using TBDPSCl.

m/z ES+ $[M+H]^+$ 364.2; $^1H$ NMR (400 MHz, $CDCl_3$) δ ppm 8.20 (s, 1H), 7.89 (d, *J* = 1.2 Hz, 1H), 7.75 - 7.65 (m, 4H), 7.46 - 7.41 (m, 2H), 7.41 - 7.35 (m, 4H), 4.77 (s, 2H), 4.54 (s, 2H), 1.10 (s, 9H).

**Step d.** This step was conducted in a similar manner to **Intermediate B69,** step a.

m/z ES+ $[M+H]^+$ 460.3; $^1H$ NMR (400 MHz, $CDCl_3$) δ ppm 9.07 (s, 1H), 8.28 (s, 1H), 8.23 (d, *J* = 1.6 Hz, 1H), 7.71 - 7.69 (m, 4H), 7.49 - 7.43 (m, 2H), 7.43 - 7.37 (m, 4H), 4.92 (d, *J* = 1.6 Hz, 2H), 4.53 - 4.47 (m, 2H), 1.47 (t, *J* = 7.2 Hz, 3H), 1.16 (s, 9H).

**Step e.** To a solution of ethyl 6-(((*tert*-butyldiphenylsilyl)oxy)methyl)imidazo[1,2-a]pyrazine-2-carboxylate (130 mg, 0.28 mmol) in THF (5 mL) was added $LiBH_4$ (19 mg, 0.85 mmol) at 0 °C. The reaction mixture was allowed to warm to 20 °C over 5 h. Upon completion, the reaction mixture was quenched with sat. aq. $NH_4Cl$ (0.5 mL) at 0 °C, diluted with water (10 mL) and extracted with EtOAc (3 x 5 mL). The combined organic layers were dried over $Na_2SO_4$ and evaporated to give (6-(((*tert*-butyldiphenylsilyl)oxy)methyl)-5,6,7,8-tetrahydroimidazo[1,2-*a*]pyrazin-2-yl)methanol (120 mg, crude) as a light yellow solid.

m/z ES+ $[M+H]^+$ 422.3.

**Step f.** To a solution of (6-(((*tert*-butyldiphenylsilyl)oxy)methyl)-5,6,7,8-tetrahydroimidazo[1,2-a]pyrazin-2-yl) methanol (120 mg, 0.29 mmol) in DCE (5 mL) was added $MnO_2$ (990 mg, 11.4 mmol). The reaction mixture was stirred at 60 °C for 16 h. Upon completion, the reaction mixture was filtered and evaporated to give the title compound (80 mg, crude) as a light yellow solid.

m/z ES+ $[M+H]^+$ 418.2; $^1H$ NMR (400 MHz, $CDCl_3$) δ ppm 9.91 (s, 1H), 8.38 (s, 1H), 7.64 - 7.16 (m, 4H), 7.45 - 7.38 (m, 7H), 4.40 - 4.31 (m, 1H), 4.21 - 4.10 (m, 3H), 3.74 - 3.70 (m, 1H), 1.06 (s, 9H).

**Intermediate B71: *tert*-butyl 2-formyl-6-(trifluoromethyl)-5,6-dihydroimidazo[1,2-*a*]pyrazine-7(8*H*)-carboxylate**

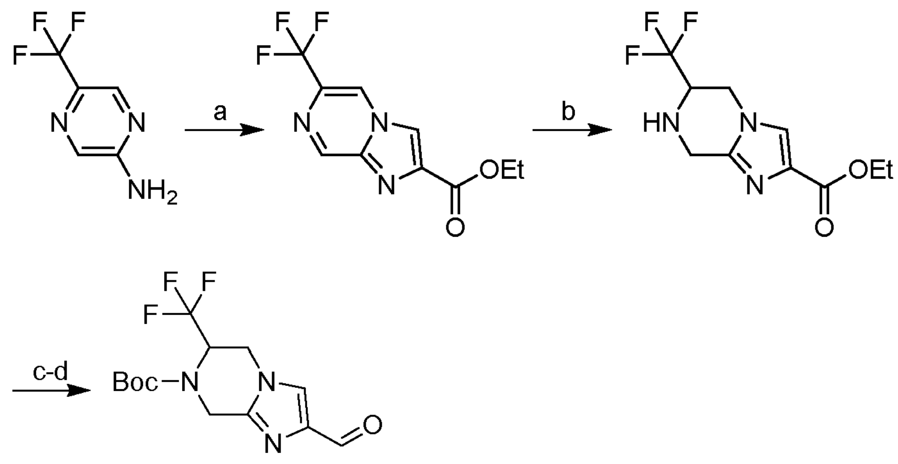


**Step a.** This step was conducted in a similar manner to **Intermediate B69,** step a, using 5-(trifluoromethyl) pyrazin-2-amine (CAS: 69816-38-2).

m/z ES+ $[M+H]^+$ 260.0; $^1H$ NMR(400 MHz, $CDCl_3$) δ ppm 8.97 (s, 1H), 8.78 (s, 1H), 8.39 (s, 1H), 4.26 (d, *J* = 7.2 Hz, 2H), 1.26 - 1.19 (m, 3H).

**Step b.** To a solution of ethyl 6-(trifluoromethyl)imidazo[1,2-a]pyrazine-2-carboxylate (5.8 g, 22.4 mmol) and acetic acid (4.03 g, 67.1 mmol) in EtOH (100 mL) was added $PtO_2$ (508 mg, 2.24 mmol) under a $N_2$ atmosphere. The suspension was degassed and purged with $H_2$ and the mixture was stirred at 50 °C for 12 h under a $H_2$ atmosphere (50 psi).

Upon completion, the mixture was filtered and evaporated to give ethyl 6-(trifluoromethyl)-5,6,7,8-tetrahydroimidazo[1,2-a]pyrazine-2-carboxylate (5.8 g, crude) as a yellow oil.

m/z ES+ $[M+H]^+$ 264.1.

**Steps c-d.** These 2 steps were conducted in a similar manner to **Intermediate B40,** steps b-c.

m/z ES+ $[M+H]^+$ 320.2; $^1H$ NMR (400 MHz, $CDCl_3$) δ ppm 9.94 (s, 1H), 7.68 (s, 1H), 5.50 - 5.15 (m, 2H), 4.61 - 4.42 (m, 3H), 1.61 (s, 9H).

**Intermediate B72: *tert*-butyl 2-formyl-6-methyl-6,7-dihydropyrazolo[1,5-*a*]pyrazine-5(4*H*)-carboxylate**

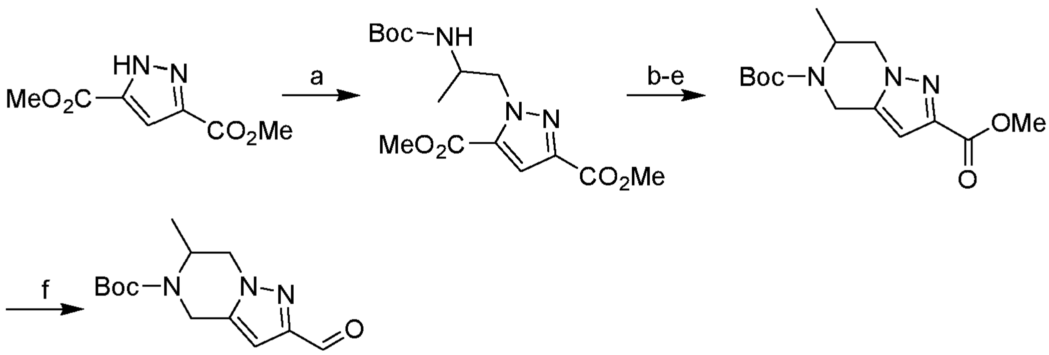


**Step a.** To a solution of dimethyl 1*H*-pyrazole-3,5-dicarboxylate (5 g, 27.1 mmol), *tert*-butyl (1-hydroxypropan-2-yl)carbamate (5.71 g, 32.5 mmol) and $PPh_3$ (14.2 g, 54.3 mmol) in THF (10 mL) under a $N_2$ atmosphere was added DEAD (7.09 g, 40.7 mmol). The reaction mixture was stirred at 25 °C for 12 h. Upon completion, the mixture was evaporated and purified by column chromatography (PE/EtOAc = 10/1 to 2/1) to give dimethyl 1-(2-((*tert*-butoxycarbonyl)amino)propyl)-1*H*-pyrazole-3,5-dicarboxylate (9.0 g, 97% yield) as a white solid.

$^1H$ NMR (400 MHz, $CDCl_3$) δ ppm 7.35 (s, 1H), 4.81 - 4.65 (m, 2H), 4.58 - 4.45 (m, 1H), 4.27 - 4.21 (m, 1H), 3.93 (d, *J = 6.8* Hz, 6H), 1.30 (s, 9H), 1.20 (d, *J* = 6.8 Hz, 3H).

**Steps b-e.** These 4 steps were conducted in a similar manner to **Intermediate B33,** steps be.

m/z ES+ $[M+H]^+$ 296.2; $^1H$ NMR(400 MHz, $CDCl_3$) δ ppm 6.65 (s, 1H), 5.00 - 4.85 (m, 2H), 4.35 - 4.24 (m, 2H), 4.21 - 4.16 (m, 1H), 3.93 (s, 3H), 1.50 (s, 9H), 1.26 (t, *J* = 7.2 Hz, 3H).

**Step f.** This step was conducted in a similar manner to **Intermediate B3,** step b.

m/z ES+ $[M+H]^+$ 265.9; $^1H$ NMR (400 MHz, $CDCl_3$) δ ppm 9.95 (s, 1H), 6.64 (s, 1H), 5.03 - 4.88 (m, 2H), 4.35 - 4.30 (m, 2H), 4.20 - 4.16 (m, 1H), 1.51 (s, 9H), 1.20 (d, *J* = 7.2 Hz, 3H).

**Intermediate B73: *tert*-butyl (2-formyl-5,6,7,8-tetrahydroimidazo[1,2-*a*]pyridin-6-yl)carbamate**

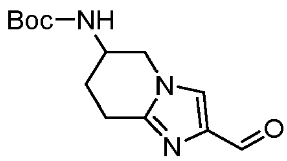

The title compound was prepared in a similar manner to **Intermediate B71,** using 5-nitropyridin-2-amine (CAS: 4214-76-0) in step a.

$^1H$ NMR (400 MHz, $CDCl_3$) δ ppm 9.82 (s, 1H), 7.49 (s, 1H), 4.75 (d, *J* = 5.6 Hz, 1H), 4.33 - 4.29 (m, 1H), 4.20 (br s, 1H), 3.93 - 3.88 (m, 1H), 3.10 - 3.00 (m, 2H), 2.25 - 2.06 (m, 2H), 1.46 (s, 9H).

**Intermediate B74: *tert*-butyl (2-formyl-5,6,7,8-tetrahydroimidazo[1,2-*a*]pyridin-7-yl)carbamate**

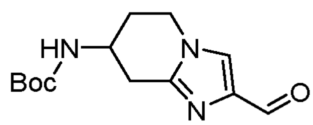

The title compound was prepared in a similar manner to **Intermediate B71,** using 4-nitropyridin-2-amine (CAS: 4487-50-7) in step a.

$^1$H NMR (400 MHz, $CDCl_3$) δ ppm 9.87 (s, 1H), 7.57 (s, 1H), 4.73 (s, 1H), 4.30 - 4.15 (m, 3H), 3.34 - 3.27 (m, 1H), 2.90 - 2.84 (m, 1H), 2.35 - 2.30 (m, 1H), 2.25 - 2.13 (m, 1H), 1.51 (s, 9H).

**Intermediate B75: *tert*-butyl 6-formyl-2,3-dihydro-4*H*-benzo[*b*][1,4]oxazine-4-carboxylate**

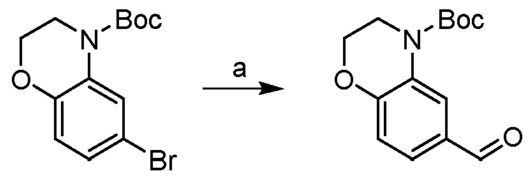


**Step a.** A solution of *n*-BuLi (2.5 M in THF, 1.15 mL) was added dropwise to a solution of *tert*-butyl 6-bromo-2,3-dihydro-4*H*-benzo[*b*][1,4]oxazine-4-carboxylate (CAS: 719310-31-3; 600 mg, 1.91 mmol) in THF (20 mL) at -70 °C under a $N_2$ atmosphere. The reaction mixture was stirred at -70 °C for 30 min, after which DMF (1.47 mL, 19.1 mmol) in THF (10 mL) was added dropwise at -70 °C. The resulting mixture was stirred at -70 °C for a further 1 h. Upon completion, the reaction mixture was quenched with water (10 mL) and extracted with EtOAc (5 x 2 mL). The combined organic layers were dried over $Na_2SO_4$, filtered, evaporated and purified by column chromatography (PE/EtOAc = 7/1) to give the title compound (450 mg, 90% yield) as a light yellow oil.

$^1$H NMR (400 MHz, $CDCl_3$) δ ppm 9.87 (s, 1H), 8.38 (br s, 1H), 7.55 (d, *J* = 8.4 Hz, 1H), 6.99 (d, *J = 8.4* Hz, 1H), 4.35 - 4.30 (m, 2H), 3.93 - 3.88 (m, 2H), 1.58 (s, 9H).

**Intermediate B76: *tert*-butyl 7-formyl-2,3-dihydro-4*H*-benzo[*b*][1,4]oxazine-4-carboxylate**

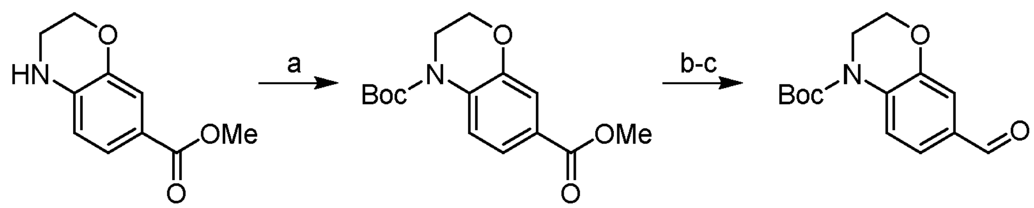


**Steps a-c.** These 3 steps were conducted in a similar manner to **Intermediate B33,** steps eg, using methyl 3,4-dihydro-2*H*-benzo[*b*][1,4]oxazine-7-carboxylate (CAS: 142166-01-6) in step a.

$^1$H NMR (400 MHz, $CDCl_3$) δ ppm 9.79 (s, 1H), 7.99 (d, *J* = 8.4 Hz, 1H), 7.38 - 7.27 (m, 2H), 4.22 - 4.18 (m, 2H), 3.85 - 3.82 (m, 2H), 1.49 (s, 9H).

**Intermediate B77: *tert*-butyl (6-formylpyridin-3-yl)carbamate**

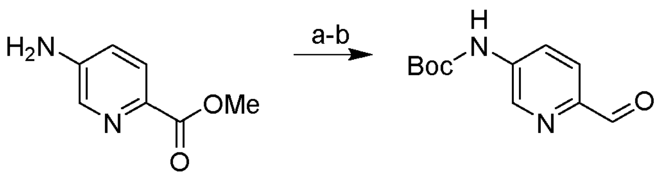

**Steps a-b.** These 2 steps were conducted in a similar manner to **Intermediate B40,** steps b-c, using methyl 5-aminopicolinate (CAS: 67515-76-8) in step a.

$^1$H NMR (400 MHz, $CDCl_3$) δ ppm 9.92 (s, 1H), 8.53 (d, *J* = 2.0 Hz, 1H), 8.12 (d, *J* = 8.0 Hz, 1H), 7.89 (d, *J* = 8.8 Hz, 1H), 6.77 (s, 1H), 1.48 (s, 9H).

**Intermediate B78: benzyl (2-formylpyrimidin-5-yl)carbamate**

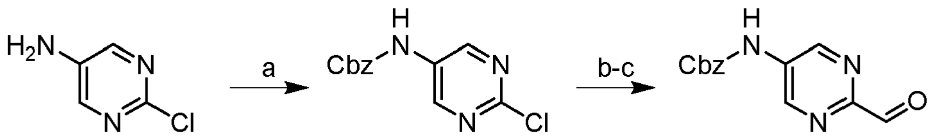


**Step a.** To a mixture of 2-chloropyrimidin-5-amine (CAS: 56621-90-0; 0.5 g, 3.86 mmol) and $NaHCO_3$ (973 mg, 11.6 mmol) in THF (5 mL) and water (2 mL) was added CbzCl (988 mg, 5.79 mmol). The reaction mixture was stirred at 25 °C for 12 h. Upon completion, the mixture was evaporated and the residue was purified by column chromatography (PE/EtOAc = 2/1) to give benzyl (2-chloropyrimidin-5-yl)carbamate (0.7 g, 69% yield) as a white solid.

m/z ES+ $[M+H]^+$ 264.0.

**Steps b-c.** These 2 steps were conducted in a similar manner to **Intermediate B49,** steps g-h.

m/z ES+ $[M+H]^+$ 258.0; $^1$H NMR (400 MHz, $CDCl_3$) δ ppm 10.05 (s, 1H), 9.10 (s, 2H), 7.46 - 7.39 (m, 5H), 7.12 (s, 1H), 5.28 (s, 2H).

**Intermediate B79: *tert*-butyl (2,6-difluoro-4-formylphenyl)carbamate**

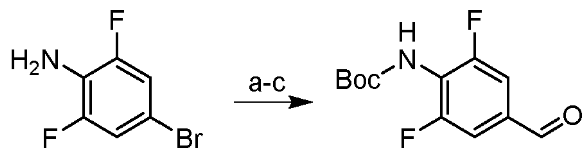


**Step a.** This step was conducted in a similar manner to **Intermediate B40,** steps b, using 4-bromo-2,6-difluoroaniline (CAS: 67567-26-4).

$^1$H NMR (400 MHz, $CDCl_3$) δ ppm 7.19 - 7.11 (m, 2H), 5.85 (br s, 1H), 1.45 (s, 9H).

**Steps b-c.** These 2 steps were conducted in a similar manner to **Intermediate B55,** steps b-c.

$^1$H NMR (400 MHz, $CDCl_3$) δ ppm 9.90 (t, *J* = 1.6 Hz, 1H), 7.53 - 7.43 (m, 2H), 6.19 (br s, 1H), 1.53 (s, 9H).

**Intermediate B80: *tert*-butyl 3-fluoro-3-((2-oxoethoxy)methyl)azetidine-1-carboxylate**

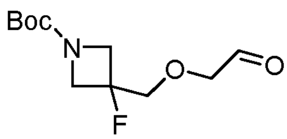

The title compound was prepared in a similar manner to **Intermediate B27,** using *tert*-butyl 3-fluoro-3-(hydroxymethyl)azetidine-1-carboxylate (CAS: 1126650-66-5) in step a.

$^1$H NMR (400 MHz, $CDCl_3$) δ ppm 9.79 (s, 1H), 4.31 - 4.28 (m, 1H), 4.18 - 4.14 (m, 1H), 4.13 - 4.03 (m, 1H), 3.97 - 3.90 (m, 2H), 3.65 - 3.52 (m, 3H), 1.54 (s, 9H).

**Intermediate B81: *tert*-butyl (5-formyl-2,3-dihydro-1*H*-inden-2-yl)carbamate**

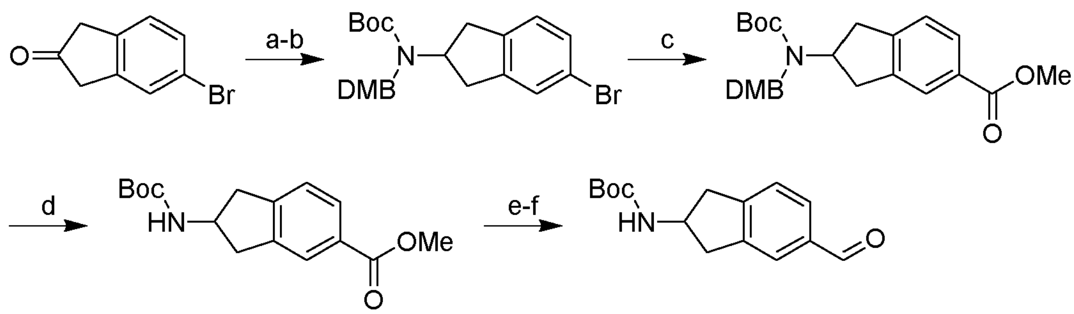


**Step a.** A mixture of 5-bromo-1,3-dihydro-2*H*-inden-2-one (CAS: 174349-93-0; 0.5 g, 2.37 mmol), (2,4-dimethoxyphenyl)methanamine (594 mg, 3.55 mmol), $NaBH(OAc)_3$ (1.00 g, 4.74 mmol) and acetic acid (14 mg, 0.24 mmol) in DCE (5 mL) was degassed and purged with $N_2$ 3 times. The reaction mixture was stirred at 25 °C for 12 h under a $N_2$ atmosphere. Upon completion, the mixture was quenched with sat. aq. $NH_4Cl$ (1 mL), diluted with water (30 mL) and extracted with EtOAc (2 x 30 mL). The combined organic layers were washed with brine (30 mL), dried over $Na_2SO_4$ and evaporated to give 5-bromo-*N*-(2,4-dimethoxybenzyl)-2,3-dihydro-1*H*-inden-2-amine (140 mg, 16% yield) as a brown oil.

m/z ES+ $[M+H]^+$ 362.0.

**Step b.** To a solution of 5-bromo-*N*-(2,4-dimethoxybenzyl)-2,3-dihydro-1*H*-inden-2-amine (4.3 g, 11.9 mmol), TEA (2.40 g, 23.7 mmol) and DMAP (145 mg, 1.19 mmol) in DCM (70 mL) was added $Boc_2O$ (3.89 g, 17.80 mmol). The reaction mixture was stirred at 20 °C for 1 h. Upon completion, the mixture was evaporated and purified by column chromatography (PE/EtOAc = 5/1) to give *tert*-butyl (5-bromo-2,3-dihydro-1*H*-inden-2-yl)(2,4-dimethoxybenzyl)carbamate (1.7 g, 29% yield) as a brown oil.

m/z ES+ $[M+Na]^+$ 484.0; $^1H$ NMR (400 MHz, $CDCl_3$) δ ppm 7.25 - 7.19 (m, 2H), 7.10 (d, *J* = 8.0 Hz, 1H), 6.97 (d, *J* = 8.0 Hz, 1H), 6.50 - 6.45 (m, 1H), 6.42 (d, *J* = 2.4 Hz, 1H), 5.10 - 4.56 (m, 1H), 4.39 (s, 2H), 3.82 (s, 3H), 3.75 (s, 3H), 3.08 - 2.99 (m, 2H), 2.97 (d, *J* = 8.4 Hz, 2H), 1.41 (s, 9H).

**Step c.** This step was conducted in a similar manner to to **Intermediate B55,** step b.

m/z ES+ $[M+Na]^+$ 464.3; $^1H$ NMR (400 MHz, $CDCl_3$) δ ppm 7.79 (d, *J* = 2.8 Hz, 1H), 7.25 - 7.20 (m, 1H), 7.16 (d, *J* = 7.6 Hz, 1H), 7.12 (d, *J* = 8.8 Hz, 1H), 6.50 - 6.45 (m, 1H), 6.42 (d, *J* = 2.0 Hz, 1H), 4.48 - 4.33 (m, 2H), 3.89 (s, 3H), 3.82 (s, 3H), 3.77 - 3.73 (m, 3H), 3.14 - 2.91 (m, 5H), 1.42 - 1.37 (m, 9H).

**Step d.** A mixture of methyl 2-((*tert*-butoxycarbonyl)(2,4-dimethoxybenzyl)amino)-2,3-dihydro-1*H*-indene-5-carboxylate (900 mg, 2.04 mmol), 10% $Pd(OH)_2$ (286 mg, 2.04 mmol) and 10% Pd/C (217 mg, 2.04 mmol) in MeOH (5 mL) was degassed and purged with $H_2$ 3 times. The reaction mixture was stirred at 25 °C for 12 h under a $H_2$ atmosphere (15 psi). Upon completion, the mixture was filtered, evaporated and purified by column chromatography (PE/EtOAc = 6/1) followed by prep-HPLC to give methyl 2-((*tert*-butoxycarbonyl)amino)-2,3-dihydro-1*H*-indene-5-carboxylate (190 mg, 32% yield) as a white solid.

m/z ES+ $[M\text{-}tBu+H]^+$ 236.1; $^1H$ NMR (400 MHz, $CDCl_3$) δ ppm 8.00 - 7.78 (m, 2H), 7.30 (s, 1H), 4.81 - 4.68 (m, 1H), 4.60 - 4.46 (m, 1H), 3.92 (s, 3H), 3.42 - 3.25 (m, 2H), 2.98 - 2.73 (m, 2H), 1.47 (s, 9H).

**Steps e-f.** These 2 steps were conducted in a similar manner to **Intermediate B5,** steps b-c. m/z ES+ $[M\text{-}tBu+H]^+$ 206.0; $^1H$ NMR (400 MHz, $CDCl_3$) δ ppm 9.89 (s, 1H), 7.71 - 7.58 (m, 2H), 7.30 (d, *J* = 7.6 Hz, 1H), 4.69 - 4.67 (m, 1H), 4.50 - 4.45 (m, 1H), 3.40 - 3.20 (m, 2H), 2.83 - 2.72 (m, 2H), 1.38 (s, 9H).

**Intermediate B82: 4-(1,3-dioxoisoindolin-2-yl)butanal**

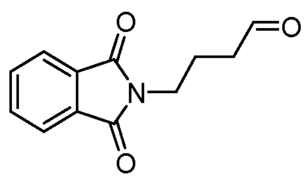

The title compound was prepared in a similar manner to Intermediate B35, using 4-aminobutan-1-ol (CAS: 13325-10-5) in step a.

$^1H$ NMR (400 MHz, $CDCl_3$) δ ppm 9.71 (s, 1H), 7.80 - 7.76 (m, 2H), 7.67 - 7.64 (m, 2H), 3.70 - 3.66 (m, 2H), 2.47 (t, *J* = 7.2 Hz, 2H), 2.00 - 1.97 (m, 2 H).

**Intermediate B86: 5-(1,3-dioxoisoindolin-2-yl)-2-fluoropentanal**

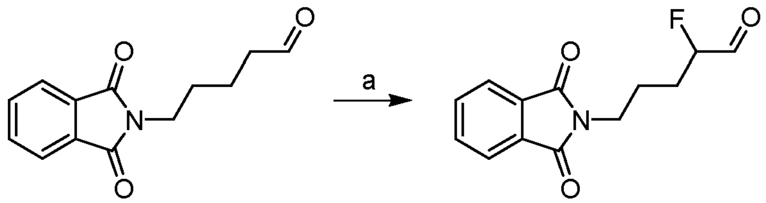


**Step a.** To a solution of **Intermediate B36** (770 mg, 3.33 mmol) and proline (115 mg, 1.00 mmol) in MeCN (10 mL) was added Selectfluor (1.77 g, 4.99 mmol) followed by TFA (114 mg, 1.00 mmol) at 0 °C. The reaction mixture was stirred at 25 °C for 16 h. Upon completion, the mixture was diluted with sat. aq. $NaHCO_3$ (20 mL) and extracted with EtOAc (3 x 20 mL). The combined layers were washed with brine (50 mL), dried over $Na_2SO_4$ and evaporated to give the title compound (800 mg, crude) as a yellow oil.

m/z ES+ $[M+H]^+$ 250.3; $^1H$ NMR (400 MHz, $CH_3OD$) δ ppm 9.77 (d, *J* = 5.6 Hz, 1H), 7.90 - 7.79 (m, 2H), 7.77 - 7.67 (m, 2H), 5.16 - 4.98 (m, 1H), 3.80 - 3.64 (m, 2H), 2.05 - 1.65 (m, 4H).

**Intermediate B87: 3-(benzyloxy)propanal**

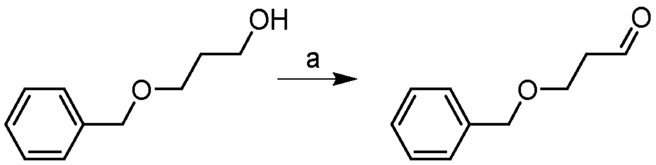


**Step a.** This step was conducted in a similar manner to **Intermediate B35,** step b, using 3-(benzyloxy)propan-1-ol (CAS: 4799-68-2).

$^1H$ NMR (400 MHz, $CDCl_3$) δ ppm 9.73 (t, *J* = 1.6 Hz, 1H), 7.32 7.27 (m, 2H), 7.27 - 7.19 (m, 3H), 4.47 (s, 2H), 3.75 (t, *J* = 6.0 Hz, 2H), 2.66 - 2.60 (m, 2H).

**Intermediate B88: *tert*-butyl (2-fluoro-5-oxopentyl)carbamate**

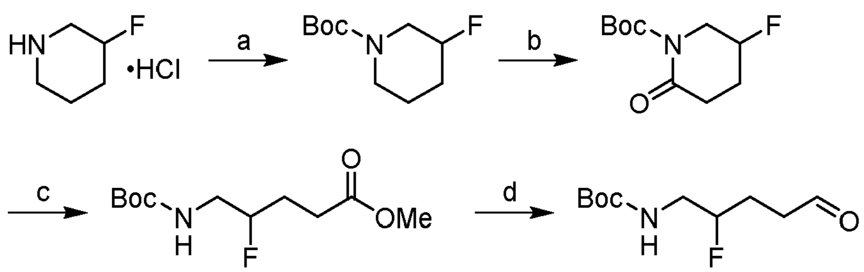


**Step a.** This step was conducted in a similar manner to **Intermediate B55,** step a, using 3-fluoropiperidine hydrochloride (CAS: 737000-77-0).

$^1H$ NMR (400 MHz, $CDCl_3$) δ ppm 4.73 - 4.46 (m, 1H), 3.70 - 3.39 (m, 3H), 3.38 - 3.19 (m, 1H), 1.95 - 1.75 (m, 3H), 1.52 - 1.43 (m, 10H).

**Step b.** To a mixture of $RuCl_3$ (1.78 g, 8.56 mmol) and $NaIO_4$ (30.5 g, 143 mmol) in water (160 mL) was added a solution of *tert*-butyl 3-fluoropiperidine-1-carboxylate (5.8 g, 28.5 mmol) in EtOAc (230 mL). The reaction mixture was stirred at 25 °C for 12 h. Upon completion, the reaction mixture was quenched with sat. aq. $Na_2S_2O_3$ (200 mL), diluted with water (150 mL) and extracted with EtOAc (3 x 250 mL). The combined organic layers were dried over $Na_2SO_4$, evaporated and purified by column chromatography (PE/EtOAc = 5/1) to give *tert*-butyl 5-fluoro-2-oxopiperidine-1-carboxylate (4.4 g, 71% yield) as a colourless oil.

$^1H$ NMR (400MHz, $CDCl_3$) δ ppm 5.17 - 4.96 (m, 1H), 4.19 - 4.05 (m, 1H), 3.78 - 3.59 (m, 1H), 2.79 - 2.65 (m, 1H), 2.56 - 2.49 (m, 1H), 2.25 - 2.12 (m, 1H), 2.02 - 1.87 (m, 1H), 1.54 (s, 9H). **Step c.** To a solution of *tert*-butyl 5-fluoro-2-oxopiperidine-1-carboxylate (2.00 g, 9.21 mmol) in MeOH (20 mL) was added NaOMe (995 mg, 18.4 mmol). The reaction mixture was stirred at 25 °C for 30 min. Upon completion, the mixture was quenched with 1 M HCl (20 mL) and extracted with EtOAc (2 x 40 mL). The combined organic layers were washed with brine (40 mL), dried over $Na_2SO_4$, evaporated and purified by column chromatography (PE/EtOAc = 5/1) to give methyl 5-((tert-butoxycarbonyl)amino)-4-fluoropentanoate (1.1 g, 48% yield) as a colourless oil.

$^1H$ NMR (400 MHz, $CDCl_3$) δ ppm 4.86 (br s, 1H), 4.72 - 4.46 (m, 1H), 3.69 (s, 3 H), 3.58 - 3.35 (m, 1H), 3.31 - 3.13 (m, 1H), 2.60 - 2.39 (m, 2H), 2.03 - 1.85 (m, 2H), 1.45 (s, 9H).

**Step d.** This step was conducted in a similar manner to **Intermediate B3,** step b.

$^1H$ NMR (400 MHz, $CDCl_3$) δ ppm 9.81 (s, 1H), 4.86 (br s, 1H), 4.73 - 4.69 (m, 1H), 3.54 - 3.48 (m, 2H), 3.25 - 3.10 (m, 2H), 2.03 - 1.85 (m, 2H) 1.45 (s, 9H).

**Intermediate B89: *tert*-butyl (2,2-difluoro-5-oxopentyl)carbamate**

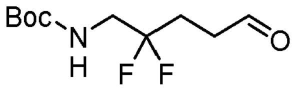

The title compound was prepared in a similar manner to **Intermediate B88,** using 3,3-difluoropiperidine hydrochloride (CAS: 496807-97-7) in step a.

$^1H$ NMR (400 MHz, $CDCl_3$) δ ppm 9.83 (s, 1H), 4.85 (br s, 1H), 3.54 (d, *J* = 6.8 Hz, 2H), 2.76 (t, *J* = 7.6 Hz, 2H), 2.30 - 2.13 (m, 2H), 1.47 (s, 9H).

**Intermediate B90: *tert*-butyl 3-(2-oxoethyl)azetidine-1-carboxylate**

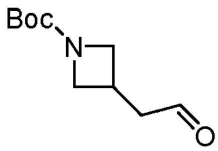

The title compound was prepared as described in **Intermediate B30,** step a.

$^1H$ NMR (400 MHz, $CDCl_3$) δ ppm 9.78 (s, 1H), 4.13 (t, *J* = 8.4 Hz, 2H), 3.61 - 3.52 (m, 2H), 2.99 - 2.87 (m, 1H), 2.86 - 2.80 (m, 2H), 1.44 (s, 9H).

**Intermediate B91: *tert*-butyl 3-(3-oxopropyl)azetidine-1-carboxylate**

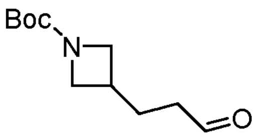

The title compound was prepared in a similar manner to **Intermediate B43,** using *tert*-butyl 3-(3-hydroxypropyl) azetidine-1-carboxylate (CAS: 158602-43-8).

$^1H$ NMR (400 MHz, CDCl3) δ ppm 9.79 (t, *J* = 1.2 Hz, 1H), 4.01 (t, *J* = 8.4 Hz, 2H), 3.59 - 3.50 (m, 2H), 2.58 - 2.48 (m, 1H), 2.45 - 2.43 (m, 2H), 1.97 - 1.87 (m, 2H), 1.44 (s, 9H).

**Intermediate B92: *tert*-butyl 3-(1-fluoro-4-oxobutyl)azetidine-1-carboxylate**

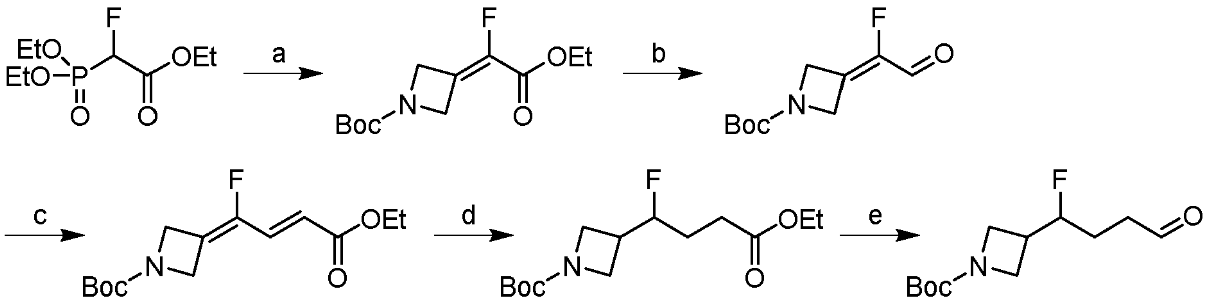


**Step a.** A mixture of ethyl 2-(diethoxyphosphoryl)-2-fluoroacetate (CAS: 2356-16-3; 10 g, 41.3 mmol), *tert*-butyl 3-oxoazetidine-1-carboxylate (CAS: 398489-26-4; 8.48 g, 49.6 mmol) and DBU (9.43 g, 61.9 mmol) in THF (100 mL) was degassed and purged with $N_2$ 3 times. The reaction mixture was stirred at 25 °C for 1 h under a $N_2$ atmosphere. Upon completion, the mixture was evaporated and purified by column chromatography (PE/EtOAc = 1/1) to give *tert*-butyl 3-(2-ethoxy-1-fluoro-2-oxoethylidene)azetidine-1-carboxylate (7.5 g, 70% yield) as a colourless oil.

$^1$H NMR (400 MHz, $CDCl_3$) δ ppm 4.71 - 4.63 (m, 2H), 4.58 - 4.56 (m, 2H), 4.22 (q, *J* = 7.2 Hz, 2H), 1.39 (s, 9H), 1.26 (t, *J* = 7.2 Hz, 3H).

**Step b.** This step was conducted in a similar manner to **Intermediate B3,** step b.

$^1$H NMR (400 MHz, $CDCl_3$) δ ppm 9.43 (d, *J* = 8.8 Hz, 1H), 4.88 - 4.85 (m, 2H), 4.78 - 4.68 (m, 2H), 1.47 (s, 9H).

**Step c.** A mixture of *tert*-butyl 3-(1-fluoro-2-oxoethylidene)azetidine-1-carboxylate (2.2 g, 10.2 mmol) and ethyl 2-(triphenyl-$\lambda^5$-phosphaneylidene)acetate (CAS: 1099-45-2; 3.56 g, 10.2 mmol) in DCM (100 mL) was degassed and purged with $N_2$ 3 times. The reaction mixture was stirred at 25 °C for 12 h under a $N_2$ atmosphere. Upon completion, the mixture was evaporated and purified by column chromatography (PE/EtOAc = 5/1) to give *tert*-butyl (*E*)-3-(4-ethoxy-1-fluoro-4-oxobut-2-en-1-ylidene)azetidine-1-carboxylate (1.0 g, 34% yield) as a yellow solid.

$^1$H NMR (400 MHz, $CDCl_3$) δ ppm 6.99 - 6.77 (m, 1H), 6.12 (d, *J* = 15.6 Hz, 1H), 4.64 (d, *J* = 2.8 Hz, 4H), 4.24 (q, *J* = 7.2 Hz, 2H), 1.46 (s, 9H), 1.31 (t, *J* = 7.2 Hz, 3H).

**Step d.** To a solution of *tert*-butyl (*E*)-3-(4-ethoxy-1-fluoro-4-oxobut-2-en-1-ylidene)azetidine-1-carboxylate (1.0 g, 3.50 mmol) in EtOH (20 mL) was added 10% Pd/C (100 mg) under a $N_2$ atmosphere. The suspension was degassed and purged with $H_2$ 3 times. The mixture was stirred at 25 °C for 12 h under a $H_2$ atmosphere (15 psi). Upon completion, the reaction mixture was evaporated to give *tert*-butyl 3-(4-ethoxy-1-fluoro-4-oxobutyl)azetidine-1-carboxylate (1.0 g, 99% yield) as a white oil.

$^1$H NMR (400 MHz, $CDCl_3$) δ ppm 4.76 - 4.51 (m, 1H), 4.21 - 4.09 (m, 2H), 4.04 - 3.94 (m, 2H), 3.93 - 3.83 (m, 1H), 3.81 - 3.69 (m, 1H), 2.80 - 2.61 (m, 1H), 2.56 - 2.40 (m, 2H), 1.96 - 1.76 (m, 1H), 1.63 - 1.53 (m, 1H), 1.44 (s, 9H), 1.32 - 1.20 (m, 3H).

**Step e.** This step was conducted in a similar manner to **Intermediate B3,** step b.

$^1$H NMR (400 MHz, $CDCl_3$) δ ppm 9.83 - 9.62 (m, 1H), 4.70 - 4.42 (m, 1H), 3.95 - 3.87 (m, 2H), 3.85 - 3.77 (m, 1H), 3.72 - 3.62 (m, 1H), 3.51 - 3.38 (m, 1H), 2.66 - 2.56 (m, 2H), 2.45 - 2.33 (m, 1H), 1.79 - 1.69 (m, 1H), 1.37 (s, 9H).

**Intermediate B93: *tert*-butyl 3-(2-fluoro-4-oxobutyl)azetidine-1-carboxylate**

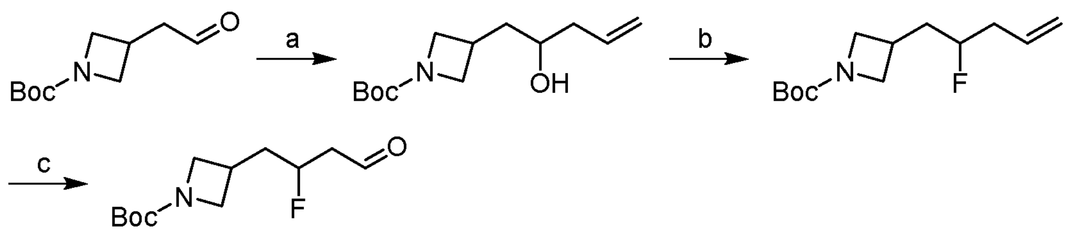


**Step a.** Allylmagnesium bromide (1 M in $Et_2O$, 40.1 mL) was added to a solution of **Intermediate B90** (4 g, 20.1 mmol) in THF (120 mL) at - 40 °C. The reaction mixture was at 0 °C for 5 h. Upon completion, the reaction mixture was quenched with sat. aq. $NH_4Cl$ and extracted with EtOAc (3 x 50 mL). The combined organic layers were washed with brine, dried over $Na_2SO_4$, evaporated and purified by column chromatography (PE/EtOAc = 4/1) to give *tert*-butyl 3-(2-hydroxypent-4-en-1-yl)azetidine-1-carboxylate (800 mg, 16% yield) as a brown oil.

$^1$H NMR (400 MHz, $CDCl_3$) δ ppm 5.91 - 5.73 (m, 1H), 5.24 - 5.03 (m, 2H), 4.08 - 4.00 (m, 2H), 3.69 - 3.58 (m, 3H), 2.80 - 2.68 (m, 1H), 2.33 - 2.25 (m, 1H), 2.20 - 2.10 (m, 1H), 1.78 - 1.73 (m, 2H), 1.60 - 1.55 (m, 1H), 1.44 (s, 9H).

**Step b.** To a solution of *tert*-butyl 3-(2-hydroxypent-4-en-1-yl)azetidine-1-carboxylate (0.8 g, 3.32 mmol) in DCM (20 mL) was added DAST (641 mg, 3.98 mmol) at -60 °C. The reaction mixture was stirred at -60 °C for 5 h. Upon completion, the mixture was quenched with sat. aq. $NaHCO_3$ and extracted with EtOAc (3 x 10 mL). The combined organic layers were washed with brine (10 mL), dried over $Na_2SO_4$, evaporated and purified by column chromatography (PE/EtOAc = 20/1) to give *tert*-butyl 3-(2-fluoropent-4-en-1-yl)azetidine-1-carboxylate (400 mg, 49% yield) as a light yellow oil.

$^1$H NMR (400 MHz, $CDCl_3$) δ ppm 5.84 - 5.76 (m, 1H), 5.17 - 5.12 (m, 2H), 4.60 - 4.40 (m, 1H), 4.08 - 4.03 (m, 2H), 3.62 - 3.58 (m, 2H), 2.75 - 2.73 (m, 1H), 2.44 - 2.35 (m, 2H), 2.00 - 1.89 (m, 2H), 1.44 (s, 9H).

**Step c.** This step was conducted in a similar manner to **Intermediate B49,** step h.

$^1$H NMR (400 MHz, $CDCl_3$) δ ppm 9.80 (s, 1H), 5.10 - 4.90 (m, 1H), 4.18 - 3.96 (m, 2H), 3.69 - 3.51 (m, 2H), 2.96 - 2.57 (m, 3H), 2.18 - 1.67 (m, 2H), 1.44 (s, 9H).

**Intermediate B94: *tert*-butyl 3-(3-fluoro-4-oxobutyl)azetidine-1-carboxylate**

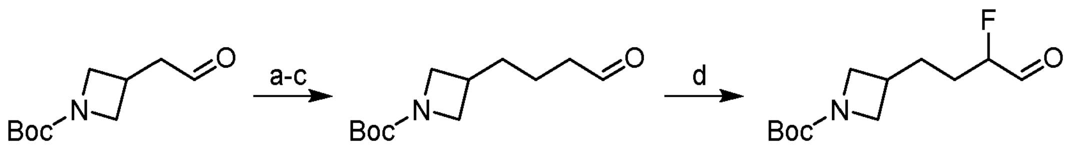


**Steps a-c.** These 3 steps were conducted in a similar manner to **Intermediate B92,** steps c-e, using **Intermediate B90** in step a.

$^1$H NMR (400 MHz, $CDCl_3$) δ ppm 9.78 (t, *J* = 1.6 Hz, 1H), 4.01 (t, *J* = 8.4 Hz, 2H), 3.54 - 3.51 (m, 2H), 2.52 - 2.46 (m, 3H), 1.65 - 1.55 (m, 4H), 1.44 (s, 9H).

**Step d.** A mixture of *tert*-butyl 3-(4-oxobutyl)azetidine-1-carboxylate (400 mg, 1.76 mmol) and proline (61 mg, 0.53 mmol) in MeCN (1 mL) was added Selectfluor (935 mg, 2.64 mmol) and TFA (60 mg, 0.53 mmol). The reaction mixture was stirred for 12 h under a $N_2$ atmosphere. Upon completion, the reaction mixture was diluted with $H_2O$ (10 mL) and extracted with EtOAc (3 x 10 mL). The combined organic layers were washed with brine (15 mL), dried over $Na_2SO_4$, evaporated and purified by column chromatography (PE/EtOAc = 1/1) to give the title compound (130 mg, crude) as a colourless oil.

$^1$H NMR $^1$H NMR (400 MHz, $CDCl_3$) δ ppm 9.80 (s, 1H), 4.04 - 4.00 (m, 1H), 3.58 - 3.54 (m, 2H), 2.52 - 2.45 (m, 2H), 2.40 - 2.36 (m, 1H), 1.69 - 1.61 (m, 4H), 1.45 (s, 9H).

**Intermediate B95: *tert*-butyl 3-(4-formylphenyl)morpholine-4-carboxylate**

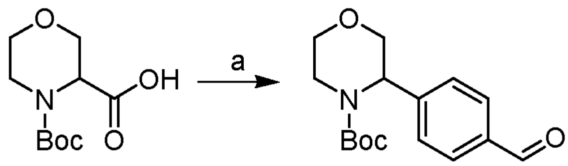


**Step a.** A mixture of 4-tert-butoxycarbonylmorpholine-3-carboxylic acid (1 g, 4.32 mmol), 4-bromobenzaldehyde (1.20 g, 6.49 mmol), Ir[dF($CF_3$)ppy]$_2$(dtbpy)($PF_6$) (97 mg, 0.087 mmol), phthalimide (636 mg, 4.32 mmol), [4,4'-bis(1,1-dimethylethyl)-2,2'-bipyridine] nickel (II) dichloride (86 mg, 0.22 mmol) and 2-(*tert*-butyl)-1,1,3,3-tetramethylguanidine in DMSO (10 mL) was degassed and purged with N2 for 5 min. The reaction was stirred under irradiation from a 34 W 455 nm LED lamp (7 cm away) at 25 °C (fan cooling) for 14 hr. Upon completion, the reaction mixture was diluted with $H_2O$ (30 mL) and extracted with EtOAc (2 x 25 mL). The combined organic layers were washed with brine (30 mL), dried over $Na_2SO_4$, evaporated and purified by column chromatography (PE/EtOAc = 5/1) to give the title compound (0.6 g, crude) as a colourless oil.

m/z ES+ $[M-Boc+H]^+$ 192.2; $^1$H NMR (400 MHz, $CDCl_3$) δ ppm 10.02 (s, 1H), 7.88 (d, *J* = 8.4 Hz, 2H), 7.63 (d, *J* = 8.4 Hz, 2H), 5.14 (s, 1H), 4.36 (d, *J* = 12.0 Hz, 1H), 3.97 - 3.88 (m, 2H), 3.84 (d, *J* = 13.2 Hz, 1H), 3.68 - 3.56 (m, 1H), 3.17 - 3.07 (m, 1H), 1.48 (s, 9H).

**Intermediate B96: *tert*-butyl 3-(5-formylpyridin-2-yl)morpholine-4-carboxylate**

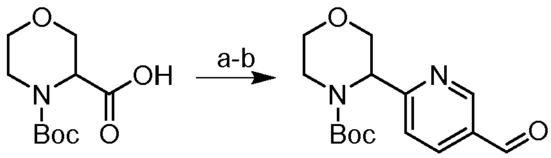


**Step a.** This step was conducted in a similar manner to **Intermediate B95,** step a, using 4-(tert-butoxycarbonyl) morpholine-3-carboxylic acid and methyl 6-bromonicotinate (CAS: 26218-78-0).

m/z ES+ $[M+H]^+$ 323.0; $^1H$ NMR (400 MHz, $CDCl_3$) $\delta$ ppm 9.22 (s, 1H), 8.33 - 8.23 (m, 1H), 7.27 - 7.25 (m, 1H), 5.13 (s, 1H), 4.77 (d, *J* = 11.2 Hz, 1H), 3.96 (s, 3H), 3.94 - 3.83 (m, 3H), 3.66 - 3.54 (m, 1H), 3.34 - 3.22 (m, 1H), 1.44 (s, 9H).

**Step b.** This step was conducted in a similar manner to **Intermediate B3,** step b.

m/z ES+ [M-*t*Bu+H]$^+$237.0; $^1H$ NMR (400 MHz, $CDCl_3$) $\delta$ ppm 10.12 (s, 1H), 9.07 (d, *J* = 1.6 Hz, 1H), 8.20 - 8.13 (m, 1H), 7.36 (d, *J* = 8.0 Hz, 1H), 5.15 (s, 1H), 4.82 - 4.72 (m, 1H), 3.99 - 3.82 (m, 3H), 3.67 - 3.56 (m, 1H), 3.35 - 3.24 (m, 1H), 1.46 (s, 9H).

**Intermediate B97a: ethyl 1-((*rac-trans*)-1-(*tert*-butoxycarbonyl)-4-fluoropyrrolidin-3-yl)-1*H*-pyrazole-4-carboxylate and**

**Intermediate B97b: ethyl 1-((*rac-cis*)-1-(*tert*-butoxycarbonyl)-4-fluoropyrrolidin-3-yl)-1*H*-pyrazole-4-carboxylate**

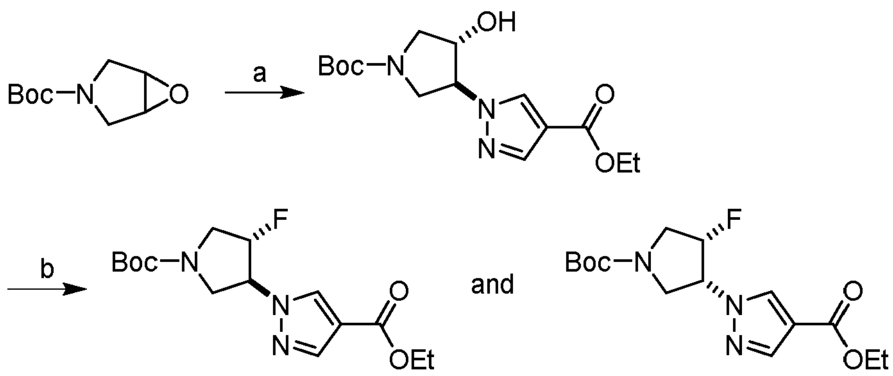


**Step a.** To a mixture of *tert*-butyl 6-oxa-3-azabicyclo[3.1.0]hexane-3-carboxylate (CAS: 114214-49-2; 2.00 g, 10.80 mmol) and ethyl 1*H*-pyrazole-4-carboxylate (CAS: 37622-90-5; 1.51 g, 10.8 mmol) in DMF (20 mL) was added $Cs_2CO_3$ (7.04 g, 21.6 mmol). The reaction mixture was stirred at 60 °C for 12 h. Upon completion, the mixture was quenched with water (100 mL) and extracted with EtOAc (3 x 100 mL). The combined organic layers were washed with water (120 mL), brine (2 x 150 mL), dried over $Na_2SO_4$, evaporated and purified by reverse phase flash chromatography (water (0.1% $NH_4OH$)/MeCN) to give ethyl 1-((*rac-trans*)-1-(*tert*-butoxycarbonyl)-4-hydroxypyrrolidin-3-yl)-1*H*-pyrazole-4-carboxylate (2.10 g, 60% yield) as a white solid.

m/z ES+ $[M+Na]^+$ 348.3; $^1H$ NMR (400 MHz, $CDCl_3$) $\delta$ ppm 7.94 (s, 2H), 4.68 - 4.63 (m, 2H), 4.30 (q, *J* = 7.2 Hz, 2H), 4.10 - 3.97 (m, 1H), 3.91 - 3.71 (m, 2H), 3.46 - 3.35 (m, 1H), 3.00 (br s, 1H), 1.48 (s, 9H), 1.35 (t, *J* = 7.2 Hz, 3H).

**Step b.** A solution of ethyl 1-((*rac-trans*)-1-(*tert*-butoxycarbonyl)-4-hydroxypyrrolidin-3-yl)-1*H*-pyrazole-4-carboxylate (2.10 g, 6.45 mmol) in DCM (25 mL) was degassed and purged with $N_2$ 3 times, after which DAST (2.08 g, 12.9 mmol) in DCM (5 mL) was added dropwise at -70 °C. The reaction mixture was warmed to 25 °C and stirred at that temperature for 16 h. Upon completion, the mixture was quenched with sat. aq. $NaHCO_3$ (20 mL) and extracted with DCM (3 x 15 mL). The combined organic layers were washed with brine (40 mL), dried over $Na_2SO_4$, evaporated and purified by column chromatography (PE/EtOAc = 7/1) followed by reverse phase flash chromatography (water (0.1% $NH_4OH$ )/MeCN) to give two products. The relative stereochemistry was confirmed by 2D NMR.

**Intermediate B97a:** ethyl 1-((*rac-trans*)-1-(*tert*-butoxycarbonyl)-4-fluoropyrrolidin-3-yl)-1*H*-pyrazole-4-carboxylate (680 mg, 31% yield) as a white solid.

m/z ES+ [M-*t*Bu+H]$^+$ 272.4; $^1$H NMR (400 MHz, DMSO-$d_6$) δ ppm 8.48 (s, 1H), 7.94 (s, 1H), 5.47 - 5.32 (m, 1H), 5.26 - 5.23 (m, 1H), 4.25 - 4.19 (m, 2H), 3.85 - 3.50 (m, 4H), 1.41 (s, 9H), 1.26 (t, *J* = 7.2 Hz, 3H).

**Intermediate B97b:** ethyl 1-((*rac-cis*)-1-(*tert*-butoxycarbonyl)-4-fluoropyrrolidin-3-yl)-1*H*-pyrazole-4-carboxylate (580 mg, 27% yield) as a white solid.

m/z ES+ [M-*t*Bu+H]$^+$ 272.1; $^1$H NMR (400 MHz, DMSO-$d_6$) δ ppm 8.49 (s, 1H), 7.93 (s, 1H), 5.52 - 5.35 (m, 1H), 5.28 - 5.15 (m, 1H), 4.25 - 4.19 (m, 2H), 4.00 - 3.93 (m, 1H), 3.90 - 3.76 (s, 1H), 3.74 - 3.58 (m, 2H), 1.43 (s, 9H), 1.27 (t, *J* = 7.2 Hz, 3H).

**Intermediate B98a: ethyl 1-((3*S*/*R*,4*S*/*R*)-1-(*tert*-butoxycarbonyl)-4-fluoropyrrolidin-3-yl)-1*H*-pyrazole-4-carboxylate and**

**Intermediate B98b: ethyl 1-((3*R*/*S*,4*R*/*S*)-1-(*tert*-butoxycarbonyl)-4-fluoropyrrolidin-3-yl)-1*H*-pyrazole-4-carboxylate**

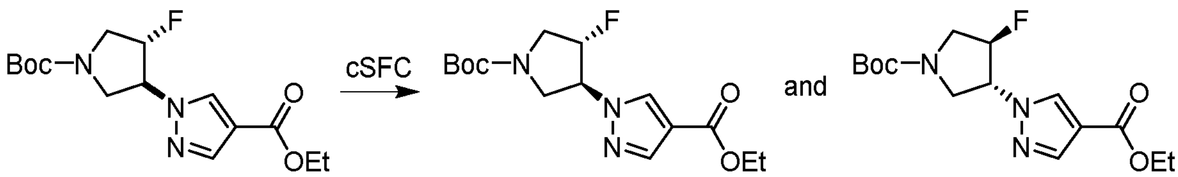


The title compounds were prepared by chiral SFC separation of **Intermediate B97a.**

**Intermediate B98a:**

m/z ES+ [M-*t*Bu+H]$^+$ 272.3; $^1$H NMR (400 MHz, $CDCl_3$) δ ppm 7.95 - 7.92 (m, 2H), 5.46 - 5.19 (m, 1H), 4.99 - 4.85 (m, 1H), 4.40 - 4.23 (m, 2H), 4.07 - 3.65 (m, 4H), 1.50 (s, 9H), 1.27 (t, *J* = 7.2 Hz, 3H).

**Intermediate B98b:**

m/z ES+ [M-*t*Bu+H]$^+$ 272.4; $^1$H NMR 400 MHz, $CDCl_3$) δ ppm 7.96 - 7.92 (m, 2H), 5.47 - 5.16 (m, 1H), 5.04 - 4.81 (m, 1H), 4.43 - 4.24 (m, 2H), 4.04 - 3.61 (m, 4H), 1.50 (s, 9H), 1.27 (t, *J* = 7.2 Hz, 3H).

**Intermediate B98c: ethyl 1-((3*R*/*S*,4*S*/*R*)-1-(*tert*-butoxycarbonyl)-4-fluoropyrrolidin-3-yl)-1*H*-pyrazole-4-carboxylate and**

**Intermediate B98d: ethyl 1-((3*S*/*R*,4*R*/*S*)-1-(*tert*-butoxycarbonyl)-4-fluoropyrrolidin-3-yl)-1*H*-pyrazole-4-carboxylate**

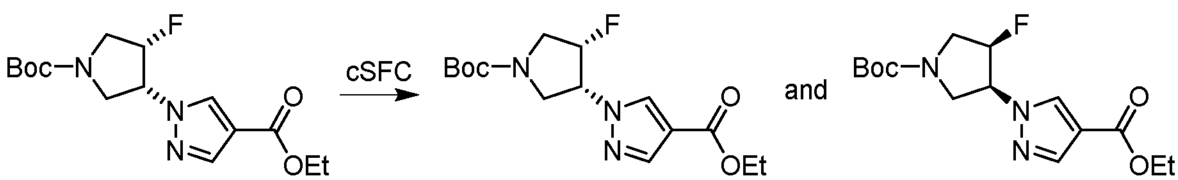


The title compounds were prepared by chiral SFC separation of **Intermediate B97b.**

**Intermediate B98c:**

m/z ES+ $[M\text{-}tBu+H]^+$ 272.3; $^1H$ NMR (400 MHz, $CDCl_3$) δ ppm 8.08 (d, *J* = 9.2 Hz, 1H), 7.96 (s, 1H), 5.39 - 5.20 (m, 1H), 5.05 - 4.88 (m, 1H), 4.36 - 4.28 (m, 2H), 4.19 - 4.07 (m, 1H), 3.99 - 3.61 (m, 3H), 1.50 (s, 9H), 1.36 (t, *J* = 7.2 Hz, 3H).

**Intermediate B98d:**

m/z ES+ $[M\text{-}tBu+H]^+$ 272.3; $^1H$ NMR (400 MHz, $CDCl_3$) δ ppm 8.08 (d, *J* = 9.2 Hz, 1H), 7.96 (s, 1H), 5.40 - 5.19 (m, 1H), 5.05 - 4.89 (m, 1H), 4.35 - 4.26 (m, 2H), 4.19 - 4.07 (m, 1H), 4.00 - 3.60 (m, 3H), 1.50 (s, 9H), 1.35 (t, *J* = 7.2 Hz, 3H).

**Intermediate B99a: *tert*-butyl (3*S*/*R*,4*S*/*R*)-3-fluoro-4-(4-formyl-1*H*-pyrazol-1-yl)pyrrolidine-1-carboxylate**

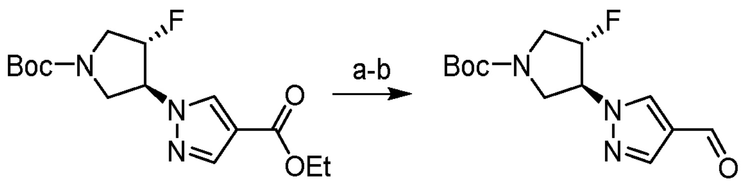


**Steps a-b.** These 2 steps were conducted in a similar manner to **Intermediate B10,** steps b-c, using **Intermediate B98a** in step a.

m/z ES+ $[M\text{-}tBu+H]^+$ 228.1; $^1H$ NMR (400 MHz, $CDCl_3$) δ ppm 9.88 (s, 1H), 8.01 (d, *J* = 6.8 Hz, 2H), 5.47 - 5.22 (m, 1H), 5.04 - 4.90 (m, 1H), 4.07 - 3.69 (m, 4H), 1.50 (s, 9H).

**Intermediate B99b: *tert*-butyl (3*R*/*S*,4*R*/*S*)-3-fluoro-4-(4-formyl-1*H*-pyrazol-1-yl)pyrrolidine-1-carboxylate**

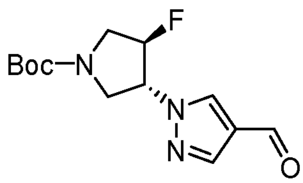

The title compound was prepared in a similar manner to **Intermediate B99a,** using **Intermediate B98b** in step a.

$^1H$ NMR (400 MHz, $CDCl_3$) δ ppm 9.88 (s, 1H), 8.01 (d, *J* = 7.6 Hz, 2H), 5.46 - 5.21 (m, 1H), 5.05 - 4.90 (m, 1H), 4.07 - 3.63 (m, 4H), 1.50 (s, 9H).

**Intermediate B99c: *tert*-butyl (3*S*/*R*,4*R*/*S*)-3-fluoro-4-(4-formyl-1*H*-pyrazol-1-yl)pyrrolidine-1-carboxylate**

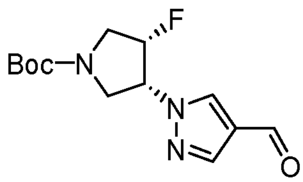

The title compound was prepared in a similar manner to **Intermediate B99a,** using **Intermediate B98c** in step a.

m/z ES+ $[M\text{-}tBu+H]^+$ 228.4; $^1H$ NMR (400 MHz, $CDCl_3$) δ ppm 9.90 (s, 1H), 8.13 (d, *J* = 13.2 Hz, 1H), 8.02 (s, 1H), 5.40 - 5.23 (m, 1H), 5.08 - 4.93 (m, 1H), 4.23 - 4.12 (m, 1H), 4.01 - 3.63 (m, 3H), 1.50 (s, 9H).

**Intermediate B99d: *tert*-butyl (3*R*/*S*,4*S*/*R*)-3-fluoro-4-(4-formyl-1*H*-pyrazol-1-yl)pyrrolidine-1-carboxylate**

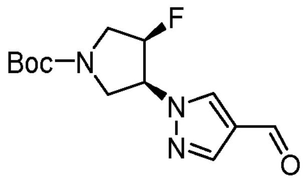

The title compound was prepared in a similar manner to **Intermediate B99a,** using **Intermediate B98d** in step a.

m/z ES+ [M-*t*Bu+H]$^+$ 228.3, $^1$H NMR (400 MHz, $CDCl_3$) δ ppm 9.90 (s, 1H), 8.18 - 8.09 (m, 1H), 8.02 (s, 1H), 5.40 - 5.24 (m, 1H), 5.08 - 4.93 (m, 1H), 4.24 - 4.09 (m, 1H), 4.01 - 3.84 (m, 1H), 3.83 - 3.61 (m, 2H), 1.50 (s, 9H).

**Intermediate B100: ethyl 1-((*rac-cis*)-1-(*tert*-butoxycarbonyl)-4-fluoropyrrolidin-3-yl)-1*H*-imidazole-4-carboxylate**

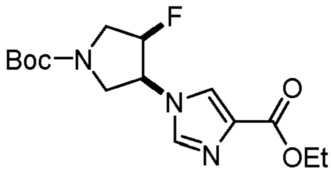

The title compound was prepared in a similar manner to **Intermediate B97a** and **Intermediate B97b,** using *tert*-butyl 6-oxa-3-azabicyclo[3.1.0]hexane-3-carboxylate and ethyl 1*H*-imidazole-4-carboxylate in step a.

m/z ES+ [M+H]$^+$ 328.1; $^1$H NMR (400 MHz, $CDCl_3$) δ ppm 7.77 (s, 1H), 7.64 (s, 1H), 5.34 - 5.12 (m, 1H), 4.82 - 4.63 (m, 1H), 4.43 - 4.31 (m, 2H), 4.10 - 4.01 (m, 1H), 3.99 - 3.80 (m, 1H), 3.79 - 3.64 (m, 2H), 1.49 (s, 9H), 1.38 (t, *J* = 7.2 Hz, 3H).

**Intermediate B101a: ethyl 1-((3*S*/*R*,4*R*/*S*)-1-(*tert*-butoxycarbonyl)-4-fluoropyrrolidin-3-yl)-1*H*-imidazole-4-carboxylate and**

**Intermediate B101b: ethyl 1-((3*R*/*S*,4*S*/*R*)-1-(*tert*-butoxycarbonyl)-4-fluoropyrrolidin-3-yl)-1*H*-imidazole-4-carboxylate**

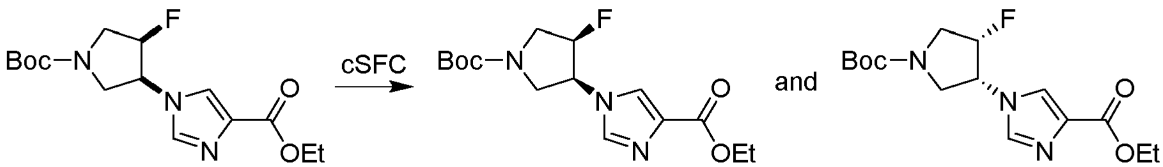


The title compounds were prepared by chiral SFC separation of **Intermediate B100.**

**Intermediate B101a:**

m/z ES+ [M+H]$^+$ 328.4; $^1$H NMR (400 MHz, $CDCl_3$) δ ppm 7.77 (s, 1H), 7.67 (s, 1H), 5.33 - 5.11 (m, 1H), 4.79 - 4.64 (m, 1H), 4.43 - 4.32 (m, 2H), 4.17 - 4.00 (m, 1H), 3.99 - 3.82 (m, 1H), 3.76 - 3.65 (m, 2H), 1.50 (s, 9H), 1.39 (t, *J* = 7.2 Hz, 3H).

**Intermediate B101 b:**

m/z ES+ [M+H]$^+$ 328.4; $^1$H NMR (400 MHz, $CDCl_3$) δ ppm 7.77 (s, 1H), 7.65 (s, 1H), 5.34 - 5.12 (m, 1H), 4.80 - 4.63 (m, 1H), 4.41 - 4.33 (m, 2H), 4.13 - 4.01 (m, 1H), 3.99 - 3.80 (m, 1H), 3.76 - 3.63 (m, 2H), 1.49 (s, 9H), 1.38 (t, *J* = 7.2 Hz, 3H).

**Intermediate B102a: *tert*-butyl (3*R*/*S*,4*S*/*R*)-3-fluoro-4-(4-formyl-1*H*-imidazol-1-yl)pyrrolidine-1-carboxylate**

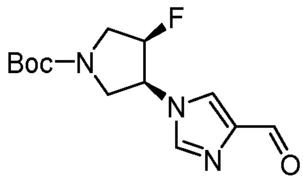

The title compound was prepared in a similar manner to **Intermediate B99a,** using **Intermediate B101a** in step a.
m/z ES+ $[M+H]^+$ 284.3, $^1H$ NMR (400 MHz, $CDCl_3$) δ ppm 9.90 (s, 1H), 7.80 (s, 1H), 7.73 (s, 1H), 5.34 - 5.16 (m, 1H), 4.85 - 4.67 (m, 1H), 4.18 - 4.03 (m, 1H), 4.01 - 3.64 (m, 3H), 1.50 (s, 9H).

**Intermediate B102b: *tert*-butyl (3*S*/*R*,4*R*/*S*)-3-fluoro-4-(4-formyl-1*H*-imidazol-1-yl)pyrrolidine-1-carboxylate**

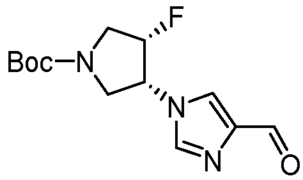

The title compound was prepared in a similar manner to **Intermediate B99a,** using **Intermediate B101b** in step a.
m/z ES+ $[M+H]^+$284.2; $^1H$ NMR (400 MHz, $CDCl_3$) δ ppm 9.91 (s, 1H), 7.87 - 7.69 (m, 2H), 5.35 - 5.13 (m, 1H), 4.82 - 4.67 (m, 1H), 4.18 - 4.04 (m, 1H), 3.94 - 3.66 (m, 3H), 1.50 (s, 9H).

**Intermediate C1: *tert*-butyl (*S*)-9-oxooctahydro-2*H*-pyrazino[1,2-*a*]pyrazine-2-carboxylate**

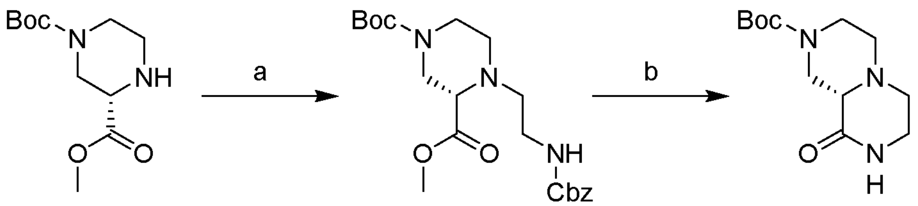


**Step a.** A mixture of 1-(*tert*-butyl) 3-methyl (S)-piperazine-1,3-dicarboxylate (CAS: 314741-39-4; 1.0 g, 4.09 mmol), benzyl (2-bromoethyl)carbamate (CAS: 53844-02-3; 1.27 g, 4.91 mmol), $K_2CO_3$ (1.70 g, 12.3 mmol) and potassium iodide (68 mg, 0.41 mmol) in MeCN (20 mL) was stirred at 80 °C for 4 h. Upon completion, the reaction was filtered and evaporated. The crude product was purified by column chromatography (PE/EtOAc = 3/1) to give 1-(*tert*-butyl) 3-methyl (*S*)-4-(2-(((benzyloxy)carbonyl)amino)ethyl)piperazine-1,3-dicarboxylate (0.9 g, 52% yield) as a colourless oil.
$^1H$ NMR (400 MHz, $CDCl_3$) δ ppm 7.47 - 7.29 (m, 5H), 5.30 (br s, 1H), 5.11 (s, 2H), 3.96 - 3.35 (m, 7H), 3.32 - 3.19 (m, 3H), 3.12 - 3.01 (m, 1H), 2.82 - 2.70 (m, 1H), 2.65 - 2.52 (m, 1H), 2.44 - 2.27 (m, 1H), 1.45 (s, 9H).
**Step b.** To a solution of 1-(*tert*-butyl) 3-methyl (*S*)-4-(2-(((benzyloxy)carbonyl)amino)ethyl)piperazine-1,3-dicarboxylate (900 mg, 2.14 mmol) in MeOH (5 mL) was added 10% Pd/C (100 mg), and the mixture was stirred at 50 °C for 3 h under a $H_2$ atmosphere (15 psi). Upon completion, the reaction was filtered and evaporated to give the title compound (500 mg, crude) as a white solid.
$^1H$ NMR (400 MHz, $CDCl_3$) δ ppm 6.03 (br s, 1H), 4.64 - 4.39 (m, 1H), 4.07 - 4.03 (m, 1H), 3.69 - 3.51 (m, 1H), 3.29 - 3.17 (m, 1H), 3.00 - 2.87 (m, 2H), 2.86 - 2.84 (m, 1H), 2.81 - 2.72 (m, 1H), 2.70 - 2.63 (m, 1H), 2.60 - 2.50 (m, 1H), 2.33 - 2.17 (m, 1H), 1.47 (s, 9H).

**Intermediate C2: *tert*-butyl (*R*)-9-oxooctahydro-2*H*-pyrazino[1,2-*a*]pyrazine-2-carboxylate**

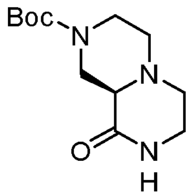

The title compound was prepared in a similar manner to **Intermediate C1,** using 1-(*tert*-butyl) 3-methyl (*R*)-piperazine-1,3-dicarboxylate (CAS: 438631-77-7) in step a.

$^1H$ NMR (400 MHz, $CDCl_3$) δ ppm 5.75 (br s, 1H), 4.61 - 4.42 (m, 1H), 4.10 - 3.98 (m, 1H), 3.64 - 3.53 (m, 1H), 3.29 - 3.18 (m, 1H), 3.00 - 2.73 (m, 4H), 2.71 - 2.64 (m, 1H), 2.62 - 2.51 (m, 1H), 2.31 - 2.20(m, 1H), 1.46 (s, 9H).

**Intermediate C3: *tert*-butyl (*R*)-7-oxooctahydro-2*H*-pyrazino[1,2-*a*]pyrazine-2-carboxylate**

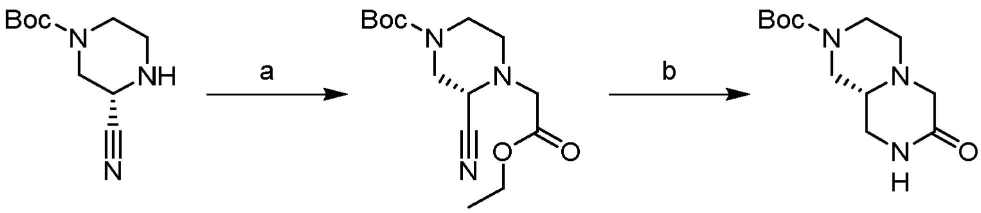


**Step a.** To a solution of *tert*-butyl (*S*)-3-cyanopiperazine-1-carboxylate (CAS: 1217650-60-6; 0.5 g, 2.37 mmol) and $Rh(OAc)_2$ (52 mg, 0.24 mmol) in DCM (10 mL) was added ethyl 2-diazenylacetate (550 mg, 4.7 mmol) in DCM (10 mL) over 30 min at 0 °C. The reaction mixture was stirred at 25 °C for 4 d under a $N_2$ atmosphere. Upon completion, the mixture was evaporated and the crude product was purified by column chromatography (PE/EtOAc = 2/1) to give *tert*-butyl (*S*)-3-cyano-4-(2-ethoxy-2-oxoethyl)piperazine-1-carboxylate (470 mg, 67% yield) as a colourless oil.

$^1H$ NMR (400 MHz, $CDCl_3$) δ ppm 4.15 - 4.08 (m, 3H), 4.00 (s, 2H), 3.28 (d, *J* = 6.0 Hz, 2H), 3.09 - 2.84 (m, 2H), 2.69 - 2.67 (m, 1H), 2.59 - 2.47 (m, 1H), 1.41 (s, 9H), 1.20 (t, *J* = 6.4 Hz, 3H).

**Step b.** To a solution of *tert*-butyl (S)-3-cyano-4-(2-ethoxy-2-oxoethyl)piperazine-1-carboxylate (470 mg, 1.58 mmol) in MeOH (80 mL) was added Raney-Ni (0.4 g), and the reaction mixture was stirred at 25 °C for 12 h under a $H_2$ atmosphere (45 psi). Upon completion, the mixture was filtered and evaporated to give the title compound (240 mg, 59% yield) as a white solid.

m/z ES+ $[M+H]^+$ 256.3.

**Intermediate C4: *tert*-butyl (*S*)-7-oxooctahydro-2*H*-pyrazino[1,2-*a*]pyrazine-2-carboxylate**

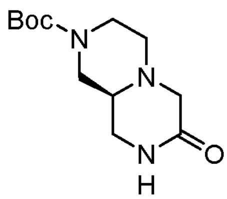

The title compound was prepared in a similar manner to Intermediate C3, using *tert*-butyl (*R*)-3-cyanopiperazine-1-carboxylate (CAS: 1217791-74-6) in step a.

$^1H$ NMR (400 MHz, $CDCl_3$) δ ppm 5.97 (br s, 1H), 4.09 - 3.97 (m, 1H), 3.91 - 3.70 (m, 1H), 3.53 - 3.48 (m, 1H), 3.32 - 3.24 (m, 1H), 3.24 - 3.12 (m, 1H), 3.11 - 2.99 (m, 1H), 2.94 (d, *J* = 16.4 Hz, 1H), 2.90 - 2.82 (m, 1H), 2.78 - 2.57 (m, 1H), 2.53 - 2.39 (m, 1H), 2.29 - 2.13 (m, 1H), 1.49 (s, 9H).

**Intermediate C5: *tert*-butyl 4,6-dihydropyrrolo[3,4-*d*]imidazole-5(1*H*)-carboxylate**

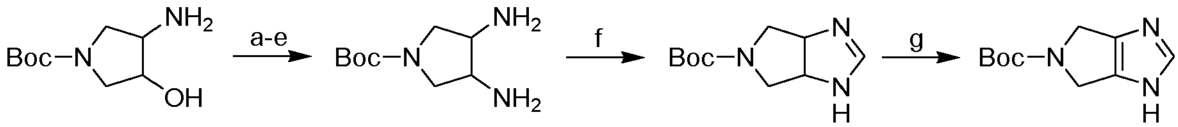


**Steps a-e.** These 5 steps were conducted as described in **Intermediate B63,** steps a-e.

**Step f.** A mixture of *tert*-butyl 3,4-diaminopyrrolidine-1-carboxylate (500 mg, 2.48 mmol) and trimethoxymethane (263 mg, 2.48 mmol) in HFIP (6 mL) was stirred at 34 °C for 12 h. Upon completion, the reaction mixture was evaporated to give *tert*-butyl 3a,4,6,6a-tetrahydropyrrolo[3,4-*d*]imidazole-5(1*H*)-carboxylate (500 mg, crude) as a white oil.

m/z ES+ $[M+H]^+$ 212.1.

**Step g.** To a mixture of oxalyl chloride (874 mg, 6.89 mmol) in DCM (15 mL) was added DMSO (1.08 g, 13.7 mmol) at -65 °C and the mixture was stirred for 30 min, after which *tert*-butyl 3a,4,6,6a-tetrahydropyrrolo[3,4-*d*]imidazole-5(1*H*)-carboxylate (485 mg, 2.30 mmol) in DCM (3 mL) was added at -65 °C. The mixture was stirred for a further 30 min and then TEA (2.09 g, 20.6 mmol) was added and the reaction mixture was allowed to warm to 25 °C over 1 h. Upon completion, the reaction mixture was quenched with $H_2O$ (30 mL) and extracted with EtOAc (2 x 30 mL). The combined organic layers were washed with brine (2 x 30 mL), dried over $Na_2SO_4$ and evaporated. The residue was purified by column chromatography (PE/EtOAc = 1/1) to give the title compound (110 mg, 8% yield) as a yellow oil.

m/z ES+ $[M+H]^+$ 210.0.

**Intermediate D1: tert-butyl 3-((6-bromopyridin-2-yl)oxy)azetidine-1-carboxylate**

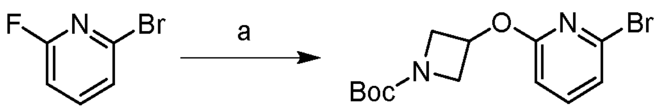


**Step a.** To a solution of *tert*-butyl 3-hydroxyazetidine-1-carboxylate (1.0 g, 5.8 mmol) in THF (15 mL) was added NaH (300 mg, 7.5 mmol, 60% dispersion in mineral oil) at 0 °C. The mixture was stirred at rt for 30 min, and then a solution of 2-bromo-6-fluoropyridine (CAS: 144100-07-2; 914 mg, 5.2 mmol) in THF (2 mL) was added dropwise at 0 °C. The mixture was stirred at rt for 30 min. Upon completion, the reaction mixture was quenched with sat. aq. $NH_4Cl$ (30 mL) at 0 °C and extracted with EtOAc (3 x 35 mL). The combined organic layers were washed with brine (70 mL), dried over $Na_2SO_4$, and evaporated. The crude product was purified by column chromatography (PE/EtOAc = 15/1) to give the title compound (720 mg, 38% yield) as a colourless oil.

m/z ES+ $[M+H]^+$ 329.3.

**Intermediate E1: 2-((dimethylamino)methyl)acrylic acid**

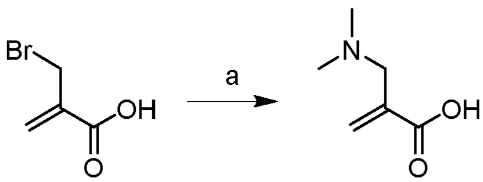


**Step a.** To a solution of 2-(bromomethyl)acrylic acid (CAS: 72707-66-5; 350 mg, 2.12 mmol) in THF (10 mL) was added $Me_2NH$ (2 M in THF, 3.18 mL). The reaction mixture was stirred at 15 °C for 12 h. Upon completion, the mixture was evaporated to give the title compound (300 mg, crude) as a white solid, which was used without further purification.

$^1H$ NMR (400 MHz, DMSO-$d_6$) δ ppm 6.11 (s, 1H), 5.66 (s, 1H), 3.47 (s, 2H), 2.55 (s, 3H), 2.50 (s, 3H).

**Intermediate E2: 2-(hydroxymethyl)acrylic acid**

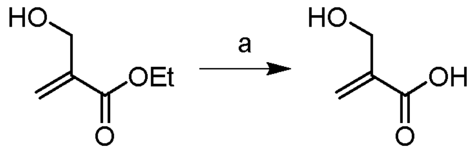


**Step a.** To a solution of ethyl 2-(hydroxymethyl)acrylate (CAS: 10029-04-6; 1 g, 7.68 mmol) in THF (10 mL) and water (10 mL) was added lithium hydroxide monohydrate (644 mg, 15.3 mmol). The reaction mixture was stirred at 20 °C for 12 h. Upon completion, the reaction mixture was acidified to pH 5 with 0.5 M HCl and extracted with EtOAc (3 x 20 mL). The combined organic layers were washed with brine (60 mL), dried over $Na_2SO_4$ and evaporated to give the title compound (450 mg, crude) as a yellow oil, which was used without further purification.
$^1H$ NMR (400 MHz, DMSO-$d_6$) δ ppm 12.50 (br s, 1H), 6.07 (s, 1H), 5.78 (s, 1H), 4.97 (br s, 1H), 4.09 (s, 2H).

**Intermediate E3: (*Z*)-4-((*tert*-butoxycarbonyl)(methyl)amino)-2-chlorobut-2-enoic acid**

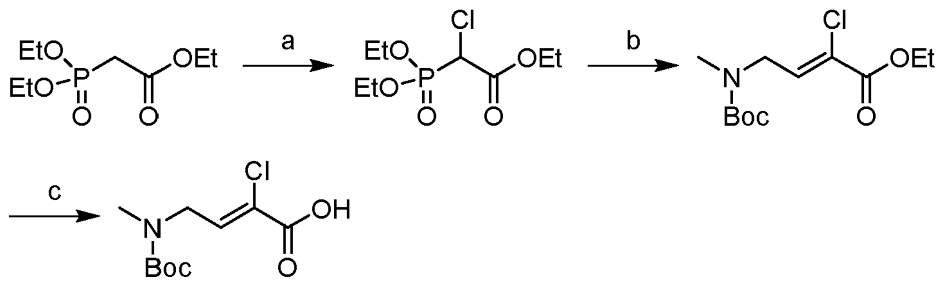


**Step a.** To a solution of ethyl 2-(diethoxyphosphoryl)acetate (CAS: 867-13-0; 10 g, 44.6 mmol) in THF (600 mL) was added KHMDS (1 M in THF, 98.1 mL) dropwise over 10 min at -78 °C under a $N_2$ atmosphere. The reaction mixture was stirred for 30 min at -78 °C, after which, a solution of NCS (7.15 g, 53.5 mmol) in THF (200 mL) was added dropwise. The reaction mixture was stirred for 30 min at -78 °C and then warmed to 25 °C and stirred for a further 2 h. Upon completion, the reaction mixture was quenched with 1 M HCl (100 mL) and extracted with EtOAc (2 x 500 mL). The combined organic layers were washed with brine (500 mL), dried over $Na_2SO_4$ and evaporated. The crude product was purified by column chromatography (PE/EtOAc = 5/1) to give ethyl 2-chloro-2-(diethoxyphosphoryl)acetate (6.5 g, 56% yield) as a yellow oil.
m/z ES+ $[M+H]^+$ 259.0; $^1H$ NMR (400 MHz, $CDCl_3$) δ ppm 4.51 (d, *J* = 16.4 Hz, 1 H) 4.25 - 4.34 (m, 6 H) 1.33 - 1.40 (m, 9 H).
**Step b.** To a solution of ethyl 2-chloro-2-(diethoxyphosphoryl)acetate (1 g, 3.87 mmol) in THF (20 mL) was added NaH (309 mg, 7.73 mmol, 60% dispersion in mineral oil) at 0 °C. The mixture was stirred for 1 h, after which, a solution of *tert-butyl* methyl(2-oxoethyl)carbamate (CAS: 123387-72-4; 670 mg, 3.87 mmol) in THF (4 mL) was added. The reaction mixture was stirred at 0 °C for a further 1 h. Upon completion, the reaction mixture was quenched with sat. aq. $NH_4Cl$ (40 mL) and extracted with EtOAc (3 x 40 mL). The combined organic layers were washed with brine (40 mL), dried over $Na_2SO_4$ and evaporated. The residue was purified by column chromatography (PE/EtOAc = 5/1) followed by Prep-TLC (PE/EtOAc = 5/1) to give ethyl (*Z*)-4-((*tert*-butoxycarbonyl)(methyl)amino)-2-chlorobut-2-enoate (130 mg, 12% yield) as a yellow oil.
m/z ES+ $[M+H]^+$ 278.1; $^1H$ NMR (400 MHz, $CDCl_3$) δ ppm 6.39 (s, 1 H) 4.38 - 4.14 (m, 4 H), 2.86 (s, 3 H), 1.44 (s, 9 H) 1.34 (t, *J* = 7.2 Hz, 3 H).
**Step c.** To a solution of ethyl (*Z*)-4-((*tert*-butoxycarbonyl)(methyl)amino)-2-chlorobut-2-enoate (150 mg, 0.54 mmol) in 1,4-dioxane (2 mL) and water (2 mL) was added KOH (151 mg, 2.70 mmol). The reaction mixture was stirred at 60 °C for 2 h. Upon completion, the reaction mixture was acidified to pH 2 with 1 M HCl and extracted with EtOAc (3 x 20 mL). The combined organic layers were washed with brine (20 mL), dried over $Na_2SO_4$ and evaporated to give the title compound (0.13 g, 96% yield) as a yellow oil.
$^1H$ NMR (400 MHz, $CDCl_3$) δ ppm 6.40 (s, 1 H) 4.33 - 4.10 (m, 2 H) 2.93 (s, 3 H) 1.47 (s, 9 H).

**Intermediate E4: 3-hydroxy-2-methylenebutanoic acid**

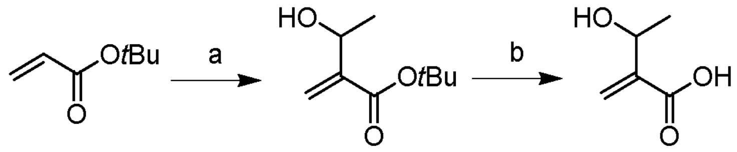


**Step a.** A mixture of *tert-butyl* acrylate (10 g, 78.0 mmol), acetaldehyde (50% purity; 13.7 g, 17.5 mL, 156 mmol) and DABCO (1.75 g, 15.6 mmol) in 1,4-dioxane (50 mL) was stirred at 25 °C for 96 h under a $N_2$ atmosphere. Upon completion, the reaction mixture was quenched with water (200 mL) and extracted with EtOAc (3 x 120 mL). The combined organic layers were washed with brine (100 mL), dried over $Na_2SO_4$ and evaporated. The residue was purified by column chromatography (PE/EtOAc = 3/1) to give *tert-butyl* 3-hydroxy-2-methylenebutanoate (12 g, 89% yield) as a colourless oil.

$^1$H NMR (400 MHz, $CDCl_3$) δ ppm 6.11 (d, *J* = 0.4 Hz, 1H), 5.72 (d, J = 0.4 Hz, 1H), 4.57 (q, *J* = 6.4 Hz, 1H), 2.43 (br s, 1H), 1.51 (s, 9H), 1.37 (d, *J* = 6.4 Hz, 3H).

**Step b.** A mixture of *tert*-butyl 3-hydroxy-2-methylenebutanoate (500 mg, 2.90 mmol) and TFA (6.62 g, 58.0 mmol) in DCM (5 mL) was stirred at 25 °C for 12 h under a $N_2$ atmosphere. Upon completion, the reaction mixture was evaporated. The residue was dissolved in DCM (100 mL), washed with water (3 x 50 mL), dried over $Na_2SO_4$ and evaporated. The residue was purified by Prep-TLC (EtOAc) to give the title compound (100 mg, 29% yield) as a colourless oil.

$^1$H NMR (400 MHz, DMSO-$d_6$) δ ppm 12.5 (br s, 1H), 6.03 (d, *J* = 1.2 Hz, 1H), 5.81 (d, *J* = 1.2 Hz, 1H), 4.95 (d, *J* = 4.4 Hz, 1H), 4.50 - 4.38 (m, 1H), 1.23 - 1.15 (m, 3H).

**Intermediate E5: (*E*)-2-((dimethylamino)methyl)but-2-enoic acid**

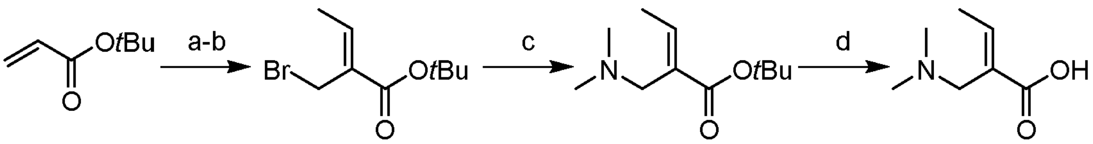


**Step a.** This step was conducted as described in **Intermediate E4,** step a.

**Step b.** Dimethyl sulfide (649 mg, 10.4 mmol) was added dropwise to a solution of NBS (1.71 g, 9.58 mmol) in DCM (3 mL) at 0 °C. The mixture was stirred for 10 min, after which, *tert*-butyl 3-hydroxy-2-methylenebutanoate (1.5 g, 8.71 mmol) was added. The reaction mixture was stirred at 25 °C for 12 h. Upon completion, the reaction mixture was quenched with water (1 mL) and evaporated. The residue was purified by column chromatography (PE/EtOAc = 5/1) to give *tert-butyl* (Z)-2-(bromomethyl)but-2-enoate (1.5 g, 73% yield) as a yellow oil.

$^1$H NMR (400 MHz, $CDCl_3$) δ ppm 6.99 (q, *J* = 7.2 Hz, 1H), 4.23 (s, 2H), 1.91 (d, *J* = 7.2 Hz, 3H), 1.53 (s, 9H).

**Step c.** A mixture of *tert-butyl* (*Z*)-2-(bromomethyl)but-2-enoate (1.4 g, 5.95 mmol) and $Me_2NH$ (2 M in THF, 29.7 mL) in THF (5 mL) was stirred at 25 °C for 1 h under a $N_2$ atmosphere. Upon completion, the reaction mixture was evaporated to give *tert*-butyl *(E)*-2-((dimethylamino)methyl)but-2-enoate (1.0 g, crude) as a yellow oil.

$^1$H NMR (400 MHz, $CDCl_3$) δ ppm 6.99 (q, *J* = 7.2 Hz, 1H), 3.23 (s, 2H), 2.29 (s, 6H), 1.89 (d, *J* = 7.2 Hz, 3H), 1.50 (s, 9H).

**Step d.** A mixture of *tert-butyl* (*E*)-2-((dimethylamino)methyl)but-2-enoate (1.0 g, 5.02 mmol) and 1 M HCl (25 mL) was stirred at 100 °C for 15 min under a $N_2$ atmosphere. Upon completion, the reaction mixture was evaporated to give the title compound (1.5 g, crude) as a yellow solid.

$^1$H NMR (400 MHz, $D_2O$) δ ppm 7.35 (q, *J* = 7.2 Hz, 1H), 3.91 (s, 2H), 2.75 (s, 6H), 1.85 (d, *J* = 7.2 Hz, 3H).

**Intermediate E6: (E)-2-((dimethylamino)methyl)but-2-enoyl chloride hydrochloride**

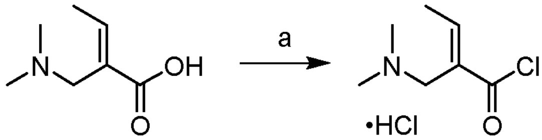


**Step a.** A mixture of **Intermediate E5** (100 mg, 0.70 mmol), $SOCl_2$ (249 mg, 2.10 mmol) in DCE (1 mL) was

degassed and purged with $N_2$ 3 times. The reaction mixture was stirred at 60 °C for 2 h under a $N_2$ atmosphere. Upon completion, the reaction mixture was evaporated to give the title compound (120 mg, crude) as a colourless oil.

$^1$H NMR (400 MHz, DMSO-$d_6$) δ ppm 8.70 (br s, 1H), 7.21 (q, $J$ = 7.2 Hz, 1H), 3.88 (d, $J$ = 5.6 Hz, 2H), 2.65 (d, $J$ = 4.8 Hz, 6H), 1.91 (d, $J$ = 7.2 Hz, 3H).

**Intermediate E7: 2-((diethylamino)methyl)acrylic acid**

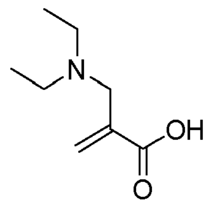

The title compound was prepared in a similar manner to **Intermediate E1,** using diethyl amine (CAS: 109-89-7).

m/z ES+ $[M+H]^+$ 158.1; $^1$H NMR (400 MHz, DMSO-$d_6$) δ ppm 9.00 (br s, 1H), 6.53 (s, 1H), 6.29 (s, 1H), 3.94 (s, 2H), 3.13 - 3.07 (m, 4H), 1.23 - 1.16 (m, 6H).

**Intermediate E8: 2-((4-methylpiperazin-1-yl)methyl)acrylic acid**

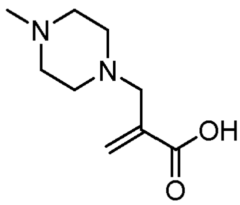

The title compound was prepared in a similar manner to **Intermediate E1,** using 1-methylpiperazine.

$^1$H NMR (400 MHz, $CD_3OD$) δ ppm 6.19 (d, $J$ = 1.6 Hz, 1H), 5.70 (d, $J$ = 1.6 Hz, 1H), 3.54 (s, 2H), 2.83 - 2.74 (m, 8H), 2.65 (s, 3H).

**Intermediate E9: (*E*)-4,4-difluorobut-2-enoic acid**

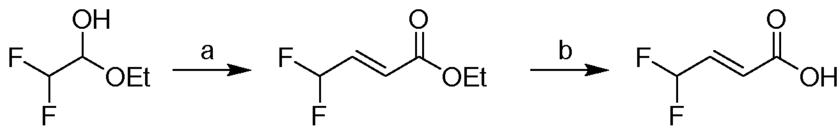


**Step a.** A mixture of 1-ethoxy-2,2-difluoroethan-1-ol (CAS: 148992-43-2; 2 g, 15.9 mmol), ethyl 2-(triphenyl-$\lambda^5$-phosphaneylidene)acetate (CAS: 1099-45-2; 6.63 g, 19.0 mmol) and 4-methylbenzenesulfonic acid monohydrate (362 mg, 1.90 mmol) in toluene (32 mL) was stirred at 120 °C for 16 h. Upon completion, the crude reaction mixture was used directly in the next step without further purification.

**Step b. To** a solution of ethyl (*E*)-4,4-difluorobut-2-enoate in toluene (2.4 g, 16.0 mmol, crude), THF (8 mL) and 4 M aq. NaOH (8.0 mL) were added. The reaction mixture was stirred at 20 °C for 1 h. Upon completion, the reaction mixture was washed with EtOAc (30 mL) and the organic layer was discarded. The aqueous layer was acidified to pH 3-4 with 1 M HCl and extracted with EtOAc (3 x 20 mL). The combined organic layers were dried over $Na_2SO_4$, evaporated and purified by column chromatography (PE/EtOAc = 3/1) to give the title compound (1.3 g, 67% yield) as a yellow oil.

$^1$H NMR (400 MHz, DMSO-$d_6$) δ ppm 13.06 (br s, 1H), 7.33 - 6.96 (m, 1H), 6.75 - 6.67 (m, 1H), 6.36 - 6.32 (m, 1H).

**Intermediate E10: (E)-4-(4-(tert-butoxycarbonyl)piperazin-1-yl)but-2-enoic acid**

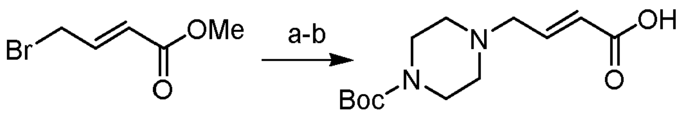


**Step a.** A mixture of methyl (E)-4-bromobut-2-enoate (CAS: 1117-71-1; 1.38 g, 6.14 mmol), *tert-butyl* piperazine-1-carboxylate (1.04 g, 5.59 mmol) and DIPEA (1.80 g, 13.97 mmol) in DCM (10 mL) was stirred at 20 °C for 12 h under a $N_2$ atmosphere. Upon completion, the reaction mixture was quenched with water (80 mL) and extracted with EtOAc (70 mL). The combined organic layers were washed with brine (70 mL), dried over $Na_2SO_4$, evaporated and purified by column chromatography (PE/EtOAc = 2/1) to give *tert-butyl* (*E*)-4-(4-methoxy-4-oxobut-2-en-1-yl)piperazine-1-carboxylate (1.0 g, 57% yield) as a white solid.

m/z ES+ $[M+H]^+$285.0; $^1H$ NMR (400 MHz, $CDCl_3$) δ ppm 6.90 - 6.71 (m, 1H), 5.95 - 5.78 (m, 1H), 3.67 - 3.56 (m, 3H), 3.33-3.30 (m, 4H), 3.07 - 2.96 (m, 2H), 2.30 - 2.26 (m, 4H), 1.47 (s, 9H).

**Step b.** This step was conducted in a similar manner to **Intermediate E2,** step a.

m/z ES- $[M-H]^-$269.0; $^1H$ NMR (400 MHz, $CDCl_3$) δ ppm 6.79 - 6.65 (m, 1H), 5.98 - 5.86 (m, 1H), 3.51-3.40 (m, 4H), 3.19 - 3.10 (m, 2H), 2.41 - 2.31 (m, 4H), 1.48 (s, 9H).

**Intermediate F1: *tert*-butyl (*R*)-2-(*N*-hydroxycarbamimidoyl)pyrrolidine-1-carboxylate**

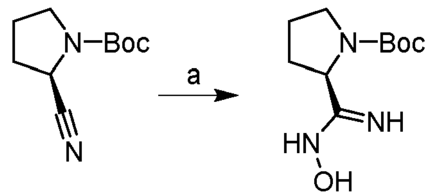


**Step a.** A mixture of *tert*-butyl (*R*)-2-cyanopyrrolidine-1-carboxylate (CAS: 228244-20-0; 500 mg, 2.55 mmol), hydroxylamine hydrochloride (266 mg, 3.82 mmol) and $Na_2CO_3$ (405 mg, 3.82 mmol) in EtOH (5 mL) was stirred at 60 °C for 90 min. Upon completion, the mixture was diluted with EtOAc (20 mL), filtered and the filtrate was evaporated to give the title compound (0.65 g, crude) as a white solid.

m/z ES+ $[M+H]^+$230.1; $^1H$ NMR (400 MHz, $CDCl_3$) δ ppm 5.30 (br s, 1H), 4.75 - 4.07 (m, 2H), 3.54 - 3.33 (m, 2H), 2.41 - 2.23 (m, 1H), 2.20 - 2.07 (m, 1H), 2.05 - 1.95 (m, 1H), 1.94 - 1.79 (m, 2H), 1.47 (s, 9H).

**Intermediate F2: tert-butyl (S)-2-(N-hydroxycarbamimidoyl)pyrrolidine-1-carboxylate**

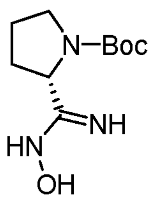

The title compound was prepared in a similar manner to **Intermediate F1,** using *tert*-butyl *(S)*-2-cyanopyrrolidine-1-carboxylate (CAS: 228244-04-0).

m/z ES+ $[M+H]^+$230.1; $^1H$ NMR (400 MHz, $CDCl_3$) δ ppm 5.35 (br s, 1H), 5.03 - 4.21 (m, 2H), 3.57 - 3.29 (m, 2H), 2.39 - 2.11 (m, 2H), 2.05 - 1.82 (m, 3H), 1.47 (s, 9H).

**Intermediate G1: *tert*-butyl (*R*)-2-(hydrazinecarbonyl)pyrrolidine-1-carboxylate**

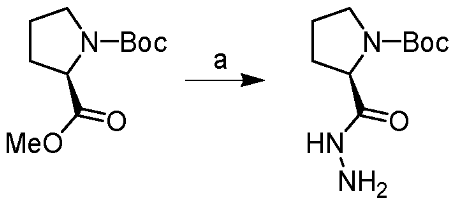


**Step a.** To a solution of *1-(tert-butyl)* 2-methyl (R)-pyrrolidine-1,2-dicarboxylate (CAS: 73323-65-6; 1 g, 4.36 mmol) in water (15 mL) was added hydrazine monohydrate (3.85 g, 65.4 mmol). The reaction mixture was stirred at 100 °C for 3 h. Upon completion, the mixture was extracted with DCM (3 x 15 mL) and the combined organic layers were dried over $Na_2SO_4$, filtered and evaporated to give the title compound (350 mg, 35% yield) as a colourless oil.

m/z ES+ $[M+Na]^+$ 252.1; $^1H$ NMR (400 MHz, DMSO-$d_6$) δ ppm 9.02 (br s, 1H), 4.16 (s, 2H), 4.06 - 3.90 (m, 1H), 3.31 - 3.22 (m, 2H), 2.09 - 1.95 (m, 1H), 1.90 - 1.63 (m, 3H), 1.44 - 1.28 (m, 9H).

**Intermediate G2: *tert*-butyl (*S*)-2-(hydrazinecarbonyl)pyrrolidine-1-carboxylate**

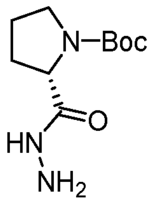

The title compound was prepared in a similar manner to **Intermediate G1,** using *1-(tert-butyl)* 2-methyl (S)-pyrrolidine-1,2-dicarboxylate (CAS: 59936-29-7).

m/z ES+ $[M+Na]^+$252.1; $^1H$ NMR (400 MHz, DMSO-$d_6$) δ ppm 9.02 (br s, 1H), 4.18 - 4.09 (m, 2H), 4.06 - 3.92 (m, 1H), 3.35 - 3.22 (m, 2H), 2.09 - 1.99 (m, 1H), 1.89 - 1.78 (m, 1H), 1.77 - 1.70 (m, 2H), 1.41 - 1.29 (m, 9H).

**Intermediate G3: *tert*-butyl (R)-2-(hydrazinecarbonyl)azetidine-1-carboxylate**

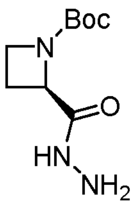

The title compound was prepared in a similar manner to **Intermediate G1,** using *1-(tert-butyl)* 2-methyl (R)-azetidine-1,2-dicarboxylate (CAS: 1260593-39-2).

m/z ES+ $[M+Na]^+$238.2; $^1H$ NMR (400 MHz, DMSO-$d_6$) δ ppm 9.21 (br s, 1H), 4.50 - 4.42 (m, 1H), 4.34 (s, 2H), 3.94 - 3.84 (m, 1H), 3.80 (br s, 1H), 2.45 - 2.31 (m, 1H), 2.08 - 1.99 (m, 1H), 1.40 (s, 9H)

**Intermediate G4: *tert*-butyl (S)-2-(hydrazinecarbonyl)azetidine-1-carboxylate**

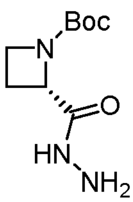

The title compound was prepared in a similar manner to **Intermediate G1,** using *1-(tert-butyl)* 2-methyl (*S*)-azetidine-1,2-dicarboxylate (CAS: 107020-12-2).

$^{1}$H NMR (400 MHz, $CDCl_3$) δ ppm 8.58 - 8.01 (m, 1H), 4.65 (t, *J* = 8.0 Hz, 1H), 3.94 - 3.87 (m, 2H), 3.84 - 3.76 (m, 2H), 2.44 (d, *J* = 7.2 Hz, 2H), 1.45 (s, 9H).

**Example 1**

**(3a*R*,11a*S*)-5-(2-(4-(1-Acryloylazetidin-3-yl)piperazin-1-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

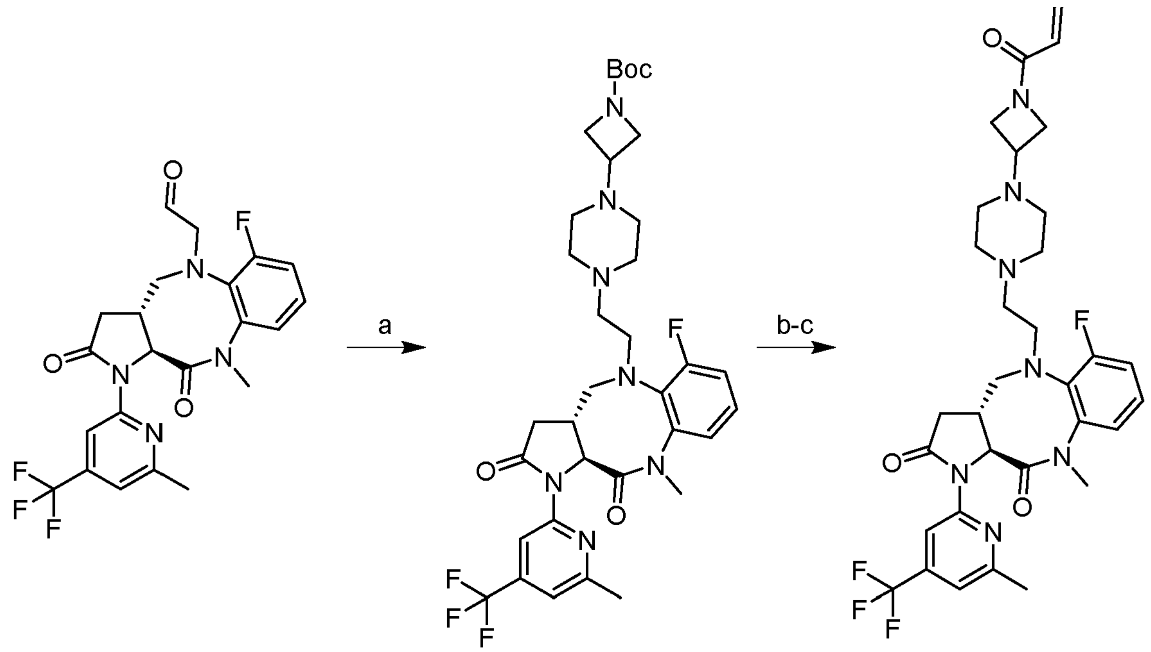


**Step** a. To a solution of **Intermediate 7a** (5 g, 10.8 mmol) and **Intermediate A1** (5.2 g, 21.5 mmol) in MeOH (50 mL) was added 4 Å molecular sieves (5 g) and acetic acid (3.2 g, 53.8 mmol). The mixture was stirred at rt for 30 min, and then $NaBH_3CN$ (1.69 g, 26.9 mmol) was added at 0 °C. The mixture was stirred at rt for 30 min. Upon completion, the mixture was filtered and evaporated under vacuum. Purification by column chromatography (PE/EtOAc = 0/1 to DCM/MeOH = 15/1) to obtain *tert-butyl* 3-(4-(2-((3aR,11aS)-6-fluoro-10-methyl-1-(6-methyl-4-(trifluoromethyl)pyridin-2-yl)-2,11-dioxo-1,2,3,3a,4,10,11,11a-octahydro-5H-benzo[*b*]pyrrolo[2,3-*f*][1,4]diazocin-5-yl)ethyl)piperazin-1-yl) azetidine-1-carboxylate (5 g, 65% yield) as a white solid.

m/z ES+ $[M+H]^+$ 690.3; $^{1}$H NMR (400 MHz, $CDCl_3$) δ ppm 8.31 (s, 1H), 7.46 - 7.38 (m, 1H), 7.29 - 7.27 (m, 1H), 7.23 - 7.18 (m, 1H), 7.08 (s, 1H), 4.61 (d, *J* = 8.8 Hz, 1H), 3.96 - 3.93 (m, 2H), 3.79 - 3.74 (m, 2H), 3.71 - 3.65 (m, 1H), 3.62 - 3.55 (m, 1H), 3.51 - 3.50 (m, 1H), 3.49 (s, 4H), 3.43 (s, 3H), 3.25 - 3.22 (m, 2H), 3.19 - 3.18 (m, 1H), 3.12 - 3.11 (m, 1H), 2.98 - 2.92 (m, 2H), 2.90 - 2.82 (m, 2H), 2.61 - 2.60 (m, 2H), 2.51 (s, 3H), 2.27 - 2.23 (m, 1H), 1.42 (s, 9H).

**Step b.** To a solution of *tert-butyl* 3-(4-(2-((3aR,11aS)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-t][1,4]diazocin-5-yl)ethyl)piperazin-1-yl) azetidine-1-carboxylate (3 g, 4.35 mmol) in DCM (40 mL) was added TFA (15.4 g, 135 mmol). The mixture was stirred at 20 °C for 1 h. Upon completion, the mixture was evaporated under vacuum to obtain (3a*R*,11a*S*)-5-(2-(4-(azetidin-3-yl) piperazin-1-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione as a TFA salt (2.55 g, crude) as a brown gum.

m/z ES+ $[M+H]^+$ 590.1.

**Step c.** To a solution of (3aR,11aS)-5-(2-(4-(azetidin-3-yl)piperazin-1-yl)ethyl)-6-fluoro-10-methyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione (TFA salt, 2.55 g, 4.32 mmol) in DCM (25 mL) was added DIPEA (2.79 g, 21.62 mmol) and subsequently cooled to 0 °C. Acryloyl chloride (CAS: 814-68-6; 705 mg, 7.78 mmol) in DCM (2 mL) was then added dropwise. The mixture was stirred at 0 °C for 10 min. Upon completion, the mixture was diluted with water (100 mL) and basified to pH 8 with sat. aq. $NaHCO_3$. The organic layer was separated and the aqueous layer was extracted with DCM (2 x 150 mL). The

combined organic layers were washed with brine (200 mL), dried over $Na_2SO_4$ and evaporated. The crude product was purified by column chromatography (DCM/MeOH = 10/1) to give the title compound (2.14 g, 77% yield) as a yellow solid.

m/z ES+ $[M+H]^+$ 644.3; $^1H$ NMR (400 MHz, $CDCl_3$) δ ppm 8.32 (s, 1H), 7.35 - 7.32 (m, 1H), 7.22 (d, *J* = 8.0 Hz, 1H), 7.17 - 7.11 (m, 1H), 7.06 (s, 1H), 6.36 - 6.29 (m, 1H), 6.22 - 6.12 (m, 1H), 5.66 (dd, *J* = 1.6, 10.0 Hz, 1H), 4.60 (d, *J* = 8.8 Hz, 1H), 4.23 - 4.17 (m, 1H), 4.11 - 4.02 (m, 2H), 3.91 (dd, *J* = 5.6, 10.6 Hz, 1H), 3.61 - 3.51 (m, 1H), 3.35 (s, 3H), 3.31 - 3.23 (m, 1H), 3.22 - 3.13 (m, 2H), 2.95 - 2.86 (m, 2H), 2.73 - 2.68 (m, 1H), 2.61 - 2.13 (m, 14H).

The **Examples** in **Table 1** were prepared using methods similar to those described in the synthesis of **Example 1,** using the listed **Intermediates** in step a.

The **Examples** in **Table 2** were prepared using methods similar to those described in the synthesis of **Example 1,** using the listed **Intermediates** in step a, and an additional chiral SFC step conducted after step a.

**Table 1**

| Example Number | Structure | Name | Intermediates | $^1$H NMR (400 MHz) δ ppm | MI |
|---|---|---|---|---|---|
| **2** | 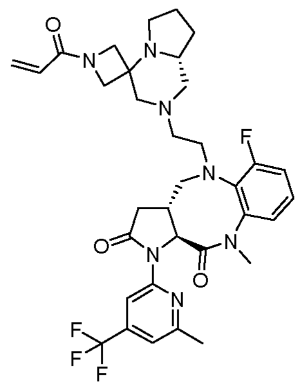 | (3a*R*,11a*S*)-5-(2-((*R*)-1-Acryloyltetrahydro-1'H-spiro[azetidine-3,4'-pyrrolo[1,2-a]pyrazin]-2'(3'*H*)-yl)ethyl)-6-fluoro-10-methyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione | **Intermediate 7a** and **Intermediate A10** | ($CDCl_3$) 8.34 (s, 1H), 7.40 - 7.33 (m, 1H), 7.24 (d, *J* = 8.0 Hz, 1H), 7.16 (t, *J* = 8.4 Hz, 1H), 7.07 (s, 1H), 6.41 - 6.15 (m, 2H), 5.75 - 5.67 (m, 1H), 4.81 (s, 2H), 4.62 (d, *J* = 9.2 Hz, 1H), 4.27 (s, 1H), 4.13 (d, *J* = 9.6 Hz, 1H), 3.85 - 3.80 (m, 1H), 3.75 - 3.70 (m, 1H), 3.60 (d, *J* = 9.2 Hz, 1H), 3.37 (d, *J* = 3.2 Hz, 3H), 3.29 - 3.05 (m, 3H), 2.95 - 2.80 (m, 4H), 2.76 - 2.70 (m, 1H), 2.51 (s, 7H), 2.25 - 2.18 (m, 2H), 1.89 - 1.80 (m, 2H), 1.45-1.40 (m, 1H) | 670.5 |
| **3** | 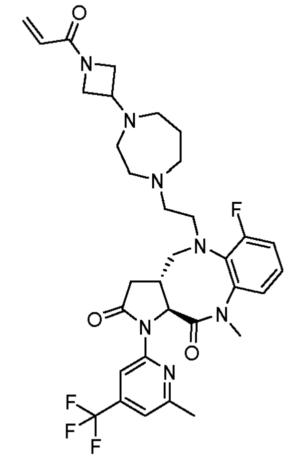 | (3a*R*,11a*S*)-5-(2-(4-(1-Acryloylazetidin-3-yl)-1,4-diazepan-1-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7a** and **Intermediate A2** | ($CDCl_3$) 8.31 (s, 1H), 7.35 - 7.32 (m, 1H), 7.22 (d, *J* = 8.0 Hz, 1H), 7.14 (t, *J* = 9.2 Hz, 1H), 7.05 (s, 1H), 6.38 - 6.29 (m, 1H), 6.23 - 6.11 (m, 1H), 5.68 - 5.64 (m, 1H), 4.59 (d, *J* = 9.2 Hz, 1H), 4.19 (t, *J* = 7.6 Hz, 1H), 4.11 - 4.04 (m, 1H), 3.99 (d, *J* = 3.2 Hz, 1H), 3.90 - 3.80 (m, 1H), 3.56 (d, *J* = 9.2 Hz, 1H), 3.53 - 3.36 (m, 2H), 3.35 (s, 3H), 3.30 - 3.15 (m, 2H), 2.97 - 2.81 (m, 3H), 2.80 - 2.67 (m, 4H), 2.55 (s, 6H), 2.49 (s, 3H), 2.49 - 2.45 (m, 1H), 2.24 - 2.14 (m, 1H) | 658.3 |

(continued)

| Example Number | Structure | Name | Intermediates | $^1$H NMR (400 MHz) δ ppm | MI |
|---|---|---|---|---|---|
| **4** | 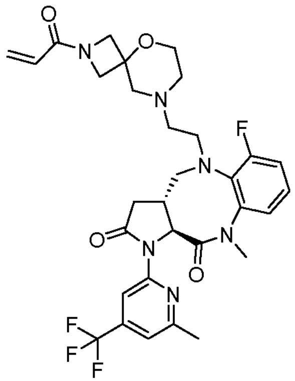 | (3a*R*,11a*S*)-5-(2-(2-acryloyl-5-oxa-2,8-diazaspiro[3.5]nonan-8-yl) ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7a** and *tert-butyl* 5-oxa-2,8-diazaspiro [3.5]no nane-2-carboxylate (CAS: 1251011-05-8) | ($CDCl_3$) 8.32 (s, 1H), 7.37 (m, 1H), 7.24 (d, *J* = 8.0 Hz, 1H), 7.16 (t, *J* = 9.2 Hz, 1H), 7.06 (s, 1H), 6.39 - 6.30 (m, 1H), 6.26 - 6.14 (m, 1H), 5.72 - 5.68 (m, 1H), 4.60 (d, *J* = 9.2 Hz, 1H), 4.24 - 4.03 (m, 2H), 4.00 - 3.87 (m, 2H), 3.76 - 3.74 (m, 2H), 3.64 - 3.49 (m, 1H), 3.38 - 3.25 (m, 5H), 2.99 - 2.42 (m, 12H), 2.25 - 2.17 (m, 1H) | 631.3 |
| **5** | 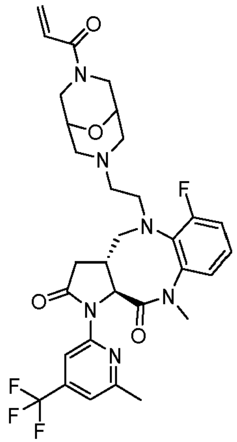 | (3a*R*,11a*S*)-5-(2-(7-acryloyl-9-oxa-3,7-diazabicyclo[3.3.1]nonan-3-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7a** and *tert-butyl* 9-oxa-3,7-diazabicyclo [3.3.1 ]nonane-3-carboxylate (CAS: 478647-20-0) | ($CDCl_3$) 8.32 (s, 1H), 7.38 - 7.29 (m, 1H), 7.21 (d, *J* = 8.0 Hz, 1H), 7.13 (m, 1H), 7.05 (s, 1H), 6.51 (dd, *J* = 10.8, 16.8 Hz, 1H), 6.26 - 6.13 (m, 1H), 5.72 - 5.58 (m, 1H), 4.66 - 4.54 (m, 2H), 3.95 - 3.80 (m, 3H), 3.67 (m, 1H), 3.56 - 3.43 (m, 1H), 3.34 (d, *J* = 2.8 Hz, 3H), 3.21 - 3.04 (m, 3H), 2.93 - 2.68 (m, 5H), 2.62 - 2.50 (m, 2H), 2.49 (s, 3H), 2.26 - 2.01 (m, 3H). | 631.3 |

(continued)

| Example Number | Structure | Name | Intermediates | $^1$H NMR (400 MHz) δ ppm | MI |
|---|---|---|---|---|---|
| **6** | 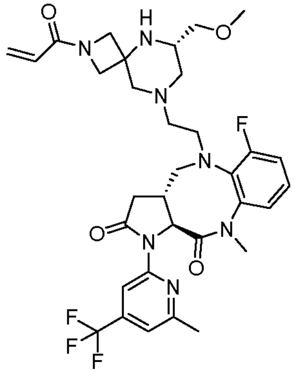 | (3a*R*,11a*S*)-5-(2-((S)-2-acryloyl-6-(methoxymethyl)-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7a** and **Intermediate A11** | ($CDCl_3$) 8.33 (s, 1H), 7.38 - 7.30 (m, 1H), 7.25 - 7.19 (m, 1H), 7.18 - 7.09 (m, 1H), 7.06 (s, 1H), 6.39 - 6.30 (m, 1H), 6.27 - 6.12 (m, 1H), 5.73 - 5.62 (m, 1H), 4.65 - 4.55 (m, 1H), 4.14 - 3.82 (m, 3H), 3.79 - 3.69 (m, 1H), 3.62 - 3.50 (m, 1H), 3.40 - 3.31 (m, 7H), 3.27 - 3.14 (m, 3H), 3.07 - 2.55 (m, 7H), 2.49 (s, 3H), 2.46 - 2.37 (m, 1H), 2.36 - 2.26 (m, 1H), 2.26 - 2.16 (m, 1H), 2.13 - 2.04 (m, 1H), 1.86 - 1.79 (m, 1H) | 674.4 |
| **7** | 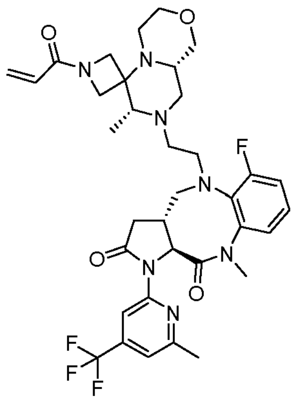 | (3a*R*,11a*S*)-5-(2-((7'*R*/S,9a'*S*)-1-acryloyl-7'-methyltetrahydro-1'*H*-spiro[azetidine-3,6'-pyrazino[2,1-*c*][1,4]oxazin]-8'(7'*H*)-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7a** and **Intermediate A13a** | ($CDCl_3$) 8.33 (s, 1H), 7.40 - 7.29 (m, 1H), 7.23 (d, *J* = 7.2 Hz, 1H), 7.19 - 7.11 (m, 1H), 7.06 (s, 1H), 6.43 - 6.12 (m, 2H), 5.75 - 5.64 (m, 1H), 4.60 (d, *J* = 9.6 Hz, 1H), 4.43 - 4.04 (m, 2H), 3.91 (d, *J* = 11.2 Hz, 1H), 3.78 - 3.52 (m, 5H), 3.35 (s, 3H), 3.25 - 3.04 (m, 3H), 2.98 - 2.79 (m, 4H), 2.78 - 2.66 (m, 1H), 2.60 - 2.40 (m, 5H), 2.35 - 2.30 (m, 1H), 2.25 - 2.15 (m, 2H), 1.58 - 1.53 (m, 1H), 1.49 - 1.42 (m, 1H), 1.02 - 0.81 (m, 3H) | 700.4 |

(continued)

| Example Number | Structure | Name | Intermediates | $^1$H NMR (400 MHz) δ ppm | MI |
|---|---|---|---|---|---|
| **8** | 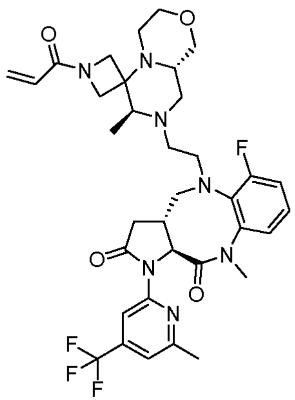 | (3a*R*,11a*S*)-5-(2-((7'*S*/*R*,9a'*S*)-1-acryloyl-7'-methyltetrahydro-1'*H*-spiro[azetidine-3,6'-pyrazino[2,1-*c*][1,4]oxazin]-8'(7'*H*)-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7a** and **Intermediate A13b** | ($CDCl_3$) 8.33 (s, 1H), 7.40 - 7.31 (m, 1H), 7.25 - 7.20 (m, 1H), 7.19 - 7.12 (m, 1H), 7.06 (s, 1H), 6.39 - 6.31 (m, 1H), 6.29 - 6.12 (m, 1H), 5.75 - 5.64 (m, 1H), 4.65 - 4.55 (m, 1H), 4.21 - 4.11 (m, 1H), 4.08 - 3.99 (m, 1H), 3.93 - 3.80 (m, 2H), 3.78 - 3.61 (m, 3H), 3.57 - 3.49 (m, 1H), 3.36 (s, 3H), 3.28 - 3.11 (m, 3H), 3.01 - 2.84 (m, 3H), 2.80 - 2.65 (m, 2H), 2.64 - 2.35 (m, 7H), 2.33 - 2.01 (m, 3H), 1.21 - 1.05 (m, 3H) | 700.3 |
| **9** | 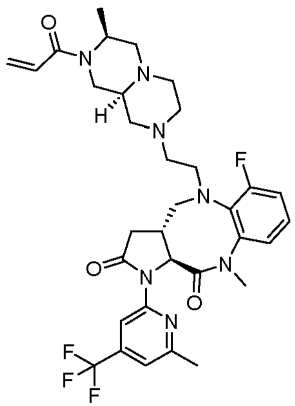 | (3a*R*,11a*S*)-5-(2-((7*S*,9a*S*/*R*)-8-acryloyl-7-methyloctahydro-2*H*-pyrazino[1,2-a]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7a** and **Intermediate A14a** | ($CDCl_3$) 8.34 (s, 1H), 7.39 - 7.32 (m, 1H), 7.24 (d, *J* = 8.2 Hz, 1H), 7.19 - 7.12 (m, 1H), 7.07 (s, 1H), 6.56 (dd, *J* = 10.4, 16.8 Hz, 1H), 6.31 (d, *J* = 16.8 Hz, 1H), 5.74 - 5.65 (m, 1H), 4.61 (d, *J* = 9.2 Hz, 1H), 4.31 (s, 1H), 3.77 (s, 1H), 3.59 - 3.55 (m, 1H), 3.41 - 3.37 (m, 4H), 3.36 - 3.18 (m, 2H), 3.15 - 3.06 (m, 1H), 2.98 - 2.83 (m, 3H), 2.82 - 2.69 (m, 3H), 2.66 - 2.53 (m, 2H), 2.51 (s, 3H), 2.49 - 2.14 (m, 6H), 1.35 (d, *J* = 6.4 Hz, 3H) | 658.3 |

(continued)

| Example Number | Structure | Name | Intermediates | $^1$H NMR (400 MHz) δ ppm | MI |
|---|---|---|---|---|---|
| **10** | 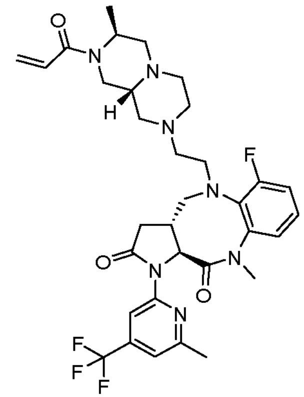 | (3aR,11aS)-5-(2-((7*S*,9a*R*/*S*)-8-acryloyl-7-methyloctahydro-2H-pyrazino[1,2-a]pyrazin-2-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Intermediate 7a** and **Intermediate A14b** | ($CDCl_3$) 8.33 (s, 1H), 7.38 - 7.36 (m, 1H), 7.28 - 7.22 (m, 1H), 7.17 (t, *J* = 8.8 Hz, 1H), 7.08 (s, 1H), 6.62 - 6.47 (m, 1H), 6.36 - 6.22 (m, 1H), 5.71 (d, *J* = 10.8 Hz, 1H), 4.85 - 4.81 (m, 0.5H), 4.67 - 4.59 (m, 1H), 4.38 - 4.30 (m, 0.5H), 4.21 - 4.12 (m, 0.5H), 3.68 - 3.54 (m, 1.5H), 3.52 - 3.43 (m, 1H),3.39 (s, 3H), 3.37 - 3.28 (m, 1H), 3.27 - 3.13 (m, 1H), 3.10 - 3.03 (m, 1H), 2.97 - 2.84 (m, 3H), 2.79 - 2.66 (m, 3H), 2.66 - 2.54 (m, 3H), 2.51 (s, 3H), 2.49 - 2.31 (m, 3H), 2.27 - 2.00 (m, 2H), 1.34 - 1.28 (m, 3H) | 658.3 |
| **11** | 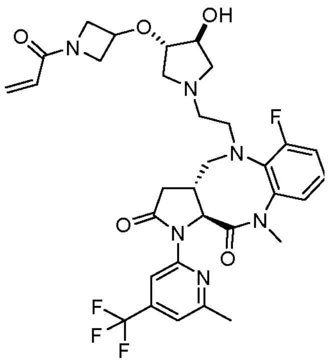 | (3a*R*,11as)-5-(2-((*rel-trans*)-3-((1-acryloylazetidin-3-yl)oxy)-4-hydroxypyrrolidin-1-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Intermediate 7a** and **Intermediate A15** | ($CDCl_3$) 8.31 (d, *J* = 6.4 Hz, 1H), 7.39 - 7.31 (m, 1H), 7.24 (d, *J* = 8.0 Hz, 1H), 7.18 - 7.10 (m, 1H), 7.06 (s, 1H), 6.37 - 6.25 (m, 1H), 6.23 - 6.09 (m, 1H), 5.67 (d, *J* = 10.4 Hz, 1H), 4.57 (d, *J* = 9.2 Hz, 1H), 4.44 (s, 1H), 4.41 - 4.32 (m, 1H), 4.30 - 4.20 (m, 1H), 4.14 - 4.01 (m, 2H), 3.98 - 3.86 (m, 1H), 3.85 - 3.76 (m, 1H), 3.61 - 3.48 (m, 1H), 3.36 (d, *J* = 8.4 Hz, 3H), 3.33 - 3.27 (m, 1H), 3.26 - 3.12 (m, 2H), 2.98 - 2.85 (m, 2H), 2.84 - 2.76 (m, 1H), 2.76 - 2.65 (m, 2H), 2.64 - 2.52 (m, 2H), 2.50 (s, 3H), 2.39 - 2.29 (s, 1H), 2.25 - 2.14 (m, 2H) | 661.3 |

(continued)

| Example Number | Structure | Name | Intermediates | $^1$H NMR (400 MHz) δ ppm | MI |
|---|---|---|---|---|---|
| **12** | 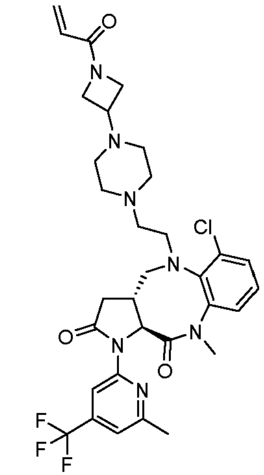 | (3aR,11aS)-5-(2-(4-(1-acryloylazetidin-3-yl)piperazin-1-yl)ethyl)-6-chloro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Intermediate 7b** and **Intermediate A1** | ($CDCl_3$) 8.33 (s, 1H), 7.45 (dd, *J* = 2.0, 7.2 Hz, 1H), 7.34 - 7.28 (m, 2H), 7.05 (s, 1H), 6.36 - 6.28 (m, 1H), 6.23 - 6.12 (m, 1H), 5.66 (dd, *J* = 1.6, 10.4 Hz, 1H), 4.61 (d, *J* = 8.8 Hz, 1H), 4.24 - 4.17 (m, 1H), 4.13 - 4.02 (m, 2H), 3.92 (dd, *J* = 5.6, 10.0 Hz, 1H), 3.63 - 3.52 (m, 1H), 3.37 (s, 3H), 3.25 - 3.06 (m, 3H), 3.04 - 2.90 (m, 2H), 2.72 - 2.61 (m, 1H), 2.59 - 2.25 (m, 13H), 2.30 - 2.20 (m, 1H) | 660.3 |
| **13** | 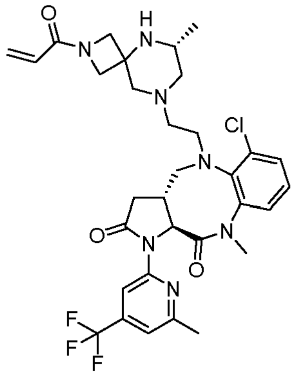 | (3a*R*,11a*S*)-5-(2-((R)-2-acryloyl-6-methyl-2,5,8-triazaspiro[3.5]nonan-8-yl)ethyl)-6-chloro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7b** and **Intermediate A9** | ($CDCl_3$) 8.34 (s, 1H), 7.46 (dd, *J* = 2.4, 7.2 Hz, 1H), 7.36 - 7.28 (m, 2H), 7.05 (s, 1H), 6.38 - 6.30 (m, 1H), 6.23 - 6.13 (m, 1H), 5.74 - 5.66 (m, 1H), 4.61 (d, *J* = 9.2 Hz, 1H), 4.16 - 3.80 (m, 4H), 3.63 - 3.53 (m, 1H), 3.38 (s, 3H), 3.14 - 3.13 (m, 2H), 3.05 - 2.92 (m, 3H), 2.91 - 2.68 (m, 3H), 2.57 - 2.30 (m, 6H), 2.29 - 2.18 (m, 2H), 1.93 - 1.85 (m, 1H), 1.16 - 1.14 (m, 3H) | 660.3 |

(continued)

| Example Number | Structure | Name | Intermediates | $^1$H NMR (400 MHz) δ ppm | MI |
|---|---|---|---|---|---|
| **14** | 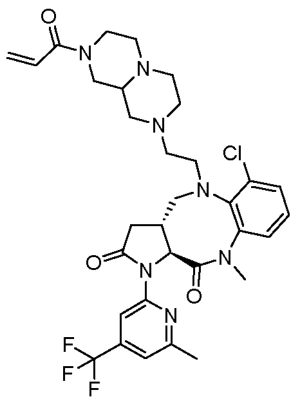 | (3a*R*,11a*S*)-5-(2-(8-acryloyloctahydro-2H-pyrazino[1,2-a]pyrazin-2-yl) ethyl)-6-chloro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Intermediate 7b** and *tert-butyl* octahydro-2*H*-pyrazino[1,2-a]pyrazine-2-carboxylate (CAS: 1159825-34-9) | ($CDCl_3$) 8.33 (s, 1H), 7.47 - 7.42 (m, 1H), 7.35 - 7.28 (m, 2H), 7.05 (s, 1H), 6.60 - 6.50 (m, 1H), 6.34 - 6.24 (m, 1H), 5.75 - 5.66 (m, 1H), 4.65 - 4.41 (m, 2H), 3.95 - 3.69 (m, 1H), 3.63 - 3.52 (m, 1H), 3.40 - 3.35 (m, 3H), 3.21 - 3.06 (m, 2H), 3.03 - 2.83 (m, 3H), 2.82 - 2.68 (m, 5H), 2.49 (s, 3H), 2.43 - 2.35 (m, 2H), 2.31 - 2.14 (m, 4H), 2.12 - 2.00 (m, 1H), 1.91 - 1.76 (m, 1H), 1.10 - 0.96 (m, 1H) | 660.4 |
| **15** | 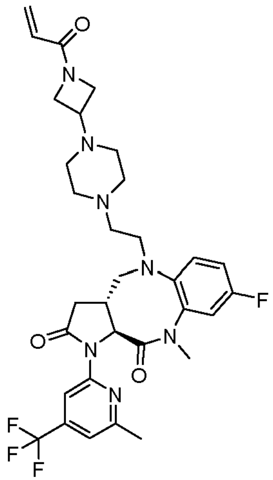 | (3aR,11aS)-5-(2-(4-(1-acryloylazetidin-3-yl)piperazin-1-yl)ethyl)-8-fluoro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Intermediate 7c** and **Intermediate A1** | ($CDCl_3$) 8.32 (s, 1H), 7.42 (dd, *J* = 5.6, 8.4 Hz, 1H), 7.17 - 7.05 (m, 3H), 6.37 - 6.28 (m, 1H), 6.22 - 6.12 (m, 1H), 5.70 - 5.62 (m, 1H), 4.60 (d, *J* = 9.2 Hz, 1H), 4.25 - 4.16 (m, 1H), 4.13 - 4.01 (m, 2H), 3.91 (dd, *J* = 5.2, 10.4 Hz, 1H), 3.55 - 3.53 (m, 1H), 3.32 (s, 3H), 3.23 - 3.12 (m, 3H), 2.95 - 2.65 (m, 4H), 2.57 - 2.29 (m, 12H), 2.17 (dd, *J* = 11.6, 16.8 Hz, 1H) | 644.3 |

(continued)

| Example Number | Structure | Name | Intermediates | $^1$H NMR (400 MHz) δ ppm | MI |
|---|---|---|---|---|---|
| **16** | 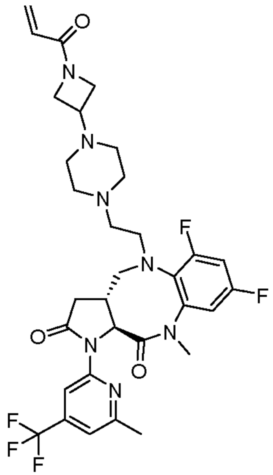 | (3a*R*,11a*S*)-5-(2-(4-(1-acryloylazetidin-3-yl)piperazin-1-yl)ethyl)-6,8-difluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7d** and **Intermediate A1** | ($CDCl_3$) 8.32 (s, 1H), 7.07 (s, 1H), 6.98 - 6.88 (m, 2H), 6.37 - 6.27 (m, 1H), 6.23 - 6.11 (m, 1H), 5.71 - 5.62 (m, 1H), 4.59 (d, *J* = 8.8 Hz, 1H), 4.20 (t, *J* = 7.2 Hz, 1H), 4.14 - 3.99 (m, 2H), 3.94 - 3.83 (m, 1H), 3.63 - 3.49 (m, 1H), 3.42 - 3.15 (m, 6H), 2.95 - 2.82 (m, 2H), 2.80 - 2.30 (m, 14H), 2.26 - 2.15 (m, 1H) | 662.3 |
| **17** | 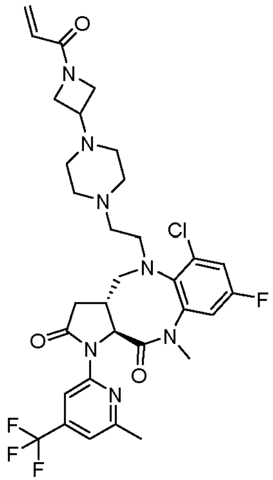 | (3a*R*,11a*S*)-5-(2-(4-(1-acryloylazetidin-3-yl)piperazin-1-yl)ethyl)-6-chloro-8-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3H)-dione | **Intermediate 7e** and **Intermediate A1** | ($CDCl_3$) 8.34 (s, 1H), 7.22 (dd, *J* =7.8, 3.2 Hz, 1H), 7.08 - 7.04 (m, 2H), 6.37 - 6.28 (m, 1H), 6.22 - 6.11 (m, 1H), 5.72 - 5.60 (m, 1H), 4.60 (d, *J* =8.8 Hz, 1H), 4.27 - 4.19 (m, 1H), 4.15 - 4.02 (m, 2H), 3.90 (dd, *J* =10.4, 5.2 Hz, 1H), 3.58 (d, *J* = 9.2 Hz, 1H), 3.39 (s, 3H), 3.25 (s, 2H), 3.01 - 2.93 (m, 2H), 2.85 (s, 1H), 2.74 (dd, *J* =17.2, 7.6 Hz, 1H), 2.63 (d, *J* = 9.2 Hz, 2H), 2.57 - 2.54 (m, 4H), 2.49 (s, 3H), 2.34 - 2.30 (m, 2H), 2.22 (dd, *J*=16.8, 10.8 Hz, 2H), 1.97 - 1.90 (m, 1H) | 678.3 |

(continued)

| Example Number | Structure | Name | Intermediates | $^1$H NMR (400 MHz) δ ppm | MI |
|---|---|---|---|---|---|
| **18** | 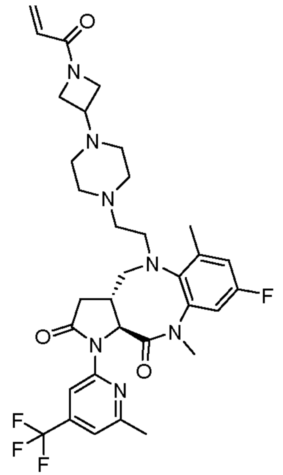 | (3a*R*,11a*S*)-5-(2-(4-(1-acryloylazetidin-3-yl)piperazin-1-yl)ethyl)-8-fluoro-6,10-dimethyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3H)-dione | **Intermediate 7g** and **Intermediate A1** | ($CDCl_3$) 8.34 (s, 1H), 7.06 (s, 1H), 6.97 (dd, *J* = 2.8, 8.8 Hz, 1H), 6.91 (dd, *J* = 2.8, 8.8 Hz, 1H), 6.37 - 6.29 (m, 1H), 6.24 - 6.12 (m, 1H), 5.66 (dd, *J* = 2.0, 10.4 Hz, 1H), 4.70 (d, *J* = 9.6 Hz, 1H), 4.21 (m, 1H), 4.13 - 4.02 (m, 2H), 3.92 (m, 1H), 3.65 - 3.56 (m, 1H), 3.35 (s, 3H), 3.18 (m, 1H), 3.12 - 2.92 (m, 3H), 2.81 - 2.66 (m, 2H), 2.59 - 2.24 (m, 16H), 2.24 - 2.18 (m, 1H) | 658.3 |
| **19** | 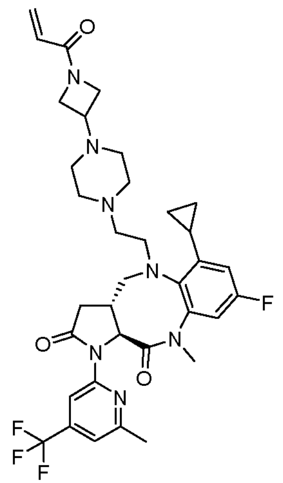 | (3a*R*,11a*S*)-5-(2-(4-(1-acryloylazetidin-3-yl)piperazin-1-yl)ethyl)-6-cyclopropyl-8-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7h** and **Intermediate A1** | ($CDCl_3$) 8.36 (s, 1H), 7.08 (s, 1H), 6.89 (d, *J* = 3.6 Hz, 1H), 6.46 (dd, *J* = 2.8, 9.6 Hz, 1H), 6.40 - 6.28 (m, 1H), 6.26 - 6.13 (m, 1H), 5.78 - 5.63 (m, 1H), 4.72 (d, *J* = 8.4 Hz, 1H), 4.24 (d, *J* = 6.4 Hz, 1H), 4.18 - 4.00 (m, 2H), 3.96 - 3.84 (m, 1H), 3.75 - 3.54 (m, 2H), 3.48 - 3.33 (m, 4H), 3.28 - 2.70 (m, 9H), 2.51 (s, 4H), 2.48 - 2.16 (m, 5H), 1.57 - 1.45 (m, 1H), 1.27 - 1.04 (m, 2H), 0.89 - 0.62 (m, 2H) | 684.3 |

(continued)

| Example Number | Structure | Name | Intermediates | $^1$H NMR (400 MHz) δ ppm | MI |
|---|---|---|---|---|---|
| **20** | 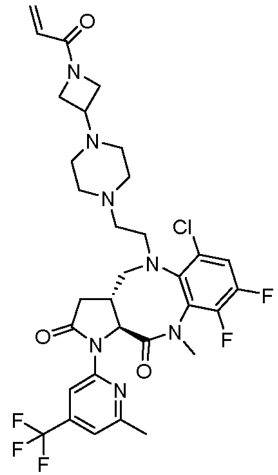 | (3a*R*,11a*S*)-5-(2-(4-(1-acryloylazetidin-3-yl)piperazin-1-yl)ethyl)-6-chloro-8,9-difluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7f** and **Intermediate A1** | ($CDCl_3$) 8.33 (s, 1H), 7.41 - 7.34 (m, 1H), 7.07 (s, 1H), 6.37 - 6.29 (m, 1H), 6.22 - 6.12 (m, 1H), 5.68 (d, *J* = 10.4 Hz, 1H), 4.61 (d, *J* = 8.4 Hz, 1H), 4.26 - 4.18 (m, 1H), 4.14 - 4.08 (m, 1H), 4.07 - 4.01 (m, 1H), 3.91 - 3.87 (m, 1H), 3.63 - 3.61 (m, 1H), 3.59 - 3.52 (m, 1H), 3.37 (s, 4H), 3.30 - 3.28 (m, 1H), 3.01 - 2.89 (m, 3H), 2.77 - 2.71 (m, 4H), 2.70 - 2.57 (m, 4H), 2.47 (s, 3H), 2.26 - 2.18 (m, 1H), 2.05 - 1.81 (m, 2H) | 696.1 |

Table 2

| Example Number | Structure | Name | Intermediates | $^1$H NMR (400 MHz) δ ppm | MI |
|---|---|---|---|---|---|
| 21a | 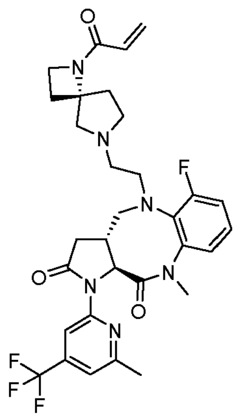 | (3aR,11aS)-5-(2-((S/R)-1-acryloyl-1,6-diazaspiro[3.4]octan-6-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Intermediate 7a** and *rac-tert*-butyl 1,6-diazaspiro[3.4]oct ane-1-carboxylate (CAS: 1148044-31-8) | ($CDCl_3$) 8.32 (s, 1H), 7.32 (m, 1H), 7.21 (t, *J* = 7.6 Hz, 1H), 7.18 - 7.09 (m, 1H), 7.08 - 7.03 (m, 1H), 6.41 - 6.27 (m, 1H), 6.19 - 6.08 (m, 1H), 5.69 - 5.56 (m, 1H), 4.60 (dd, *J* = 3.6, 9.2 Hz, 1H), 4.07 (t, *J* = 7.6 Hz, 1H), 3.96 - 3.81 (m, 1H), 3.56 (d, *J* = 9.2 Hz, 1H), 3.36 - 3.31 (m, 3H), 3.29 - 3.27 (m, 1H), 3.26 - 3.14 (m, 2H), 3.00 (d, *J* = 13.6 Hz, 1H), 2.85 (d, *J* = 2.0 Hz, 3H), 2.76 - 2.67 (m, 1H), 2.66 - 2.59 (m, 1H), 2.55 (td, *J* = 7.2, 12.4 Hz, 1H), 2.51 - 2.48 (m, 3H), 2.47 - 2.36 (m, 2H), 2.35 - 2.29 (m, 2H), 2.24 - 2.16 (m, 1H), 2.03 - 1.81 (m, 1H) | 615.3 |
| 21b | 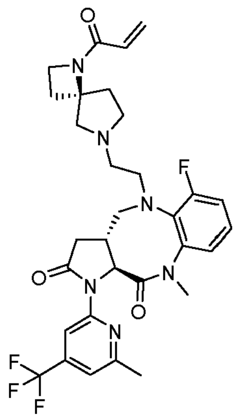 | (3a*R*,11aS)-5-(2-((R/S)-1-acryloyl-1,6-diazaspiro[3.4]octan-6-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-f][1,4]diazocine-2,11(3H)-dione | | ($CDCl_3$) 8.37 - 8.27 (m, 1H), 7.37 - 7.29 (m, 1H), 7.24 - 7.19 (m, 1H), 7.18 - 7.09 (m, 1H), 7.07 - 7.03 (m, 1H), 6.40 - 6.27 (m, 1H), 6.18 - 6.09 (m, 1H), 5.66 - 5.57 (m, 1H), 4.64 - 4.57 (m, 1H), 4.07 (t, *J* = 7.6 Hz, 1H), 3.95 - 3.82 (m, 1H), 3.60 - 3.50 (m, 1H), 3.36 - 3.33 (m, 3H), 3.24 - 3.17 (m, 2H), 2.98 (s, 1H), 2.95 - 2.83 (m, 3H), 2.76 - 2.67 (m, 1H), 2.66 - 2.62 (m, 1H), 2.62 - 2.51 (m, 1H), 2.49 (s, 3H), 2.47 - 2.30 (m, 4H), 2.29 - 2.14 (m, 2H), 1.96 - 1.87 (m, 1H) | 615.3 |

(continued)

| Example Number | Structure | Name | Intermediates | $^1$H NMR (400 MHz) δ ppm | MI |
|---|---|---|---|---|---|
| **22a** | 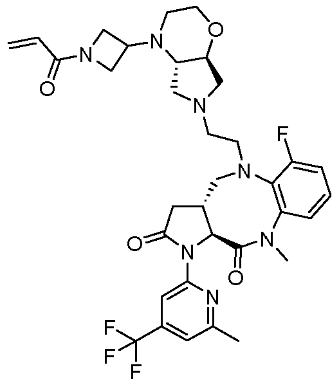 | (3aR,11aS)-5-(2-((4aS/R,7aS/R)-4-(1-acryloylazetidin-3-yl)hexahydropyrrolo[3,4-b][1,4]oxazin-6(2H)-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3H)-dione | **Intermediate 7a** and **Intermediate A16** | ($CDCl_3$) 8.32 (s, 1H),7.40 - 7.32 (m, 1H), 7.23 (d, *J* = 8.0 Hz, 1H), 7.18 - 7.15 (m, 1H), 7.06 (s, 1H), 6.39 - 6.29 (m, 1H), 6.21 - 6.11 (m, 1H), 5.68 (dd, *J* =10.2, 1.8 Hz, 1H), 4.59 (d, *J* =9.0 Hz, 1H), 4.26 - 4.18 (m, 1H), 4.09 (d, J=9.6 Hz, 1H), 4.02 - 3.91 (m, 1H), 3.85 - 3.62 (m, 2H), 3.61 - 3.51 (m, 2H), 3.36 (s, 3H), 3.33 - 3.18 (m, 3H), 2.99 - 2.82 (m, 5H), 2.79 - 2.60 (m, 5H), 2.49 (s, 3H), 2.35 - 2.27 (m, 1H),2.27 - 2.10 (m, 3H) | 686.4 |
| **22b** | 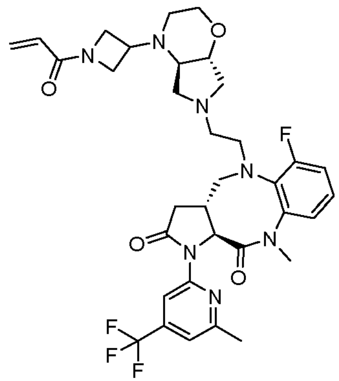 | (3a*R*,11a*S*)-5-(2-((4aR/S,7aR/S)-4-(1-acryloylazetidin-3-yl)hexahydropyrrolo[3,4-*b*][1,4]oxazin-6(2*H*)-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3H)-dione | | ($CDCl_3$) 8.32 (s, 1H), 7.41 - 7.33 (m, 1H), 7.25 - 7.22 (m, 1H), 7.21 - 7.13 (m, 1H), 7.06 (s, 1H), 6.38 - 6.29 (m, 1H), 6.23 - 6.12 (m, 1H), 5.69 (dd, *J* = 1.6, 10.4 Hz, 1H), 4.60 (d, *J* = 9.2 Hz, 1H), 4.24 - 4.21 (m, 1H), 4.11 - 4.07 (m, 1H), 4.03 - 3.92 (m, 2H), 3.84 - 3.68 (m, 2H), 3.67 - 3.55 (m, 2H), 3.38 (s, 3H), 3.33 - 3.31 (m, 3H), 3.18 - 2.91 (m, 4H), 2.88 - 2.65 (m, 6H), 2.50 (s, 3H), 2.36 (m, 1H), 2.21 (m, 2H) | 686.4 |

(continued)

| Example Number | Structure | Name | Intermediates | $^1$H NMR (400 MHz) δ ppm | MI |
|---|---|---|---|---|---|
| **23a** | 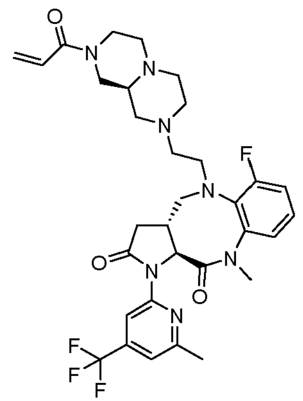 | (3a*R*,11a*S*)-5-(2-((*S*/*R*)-8-acryloyloctahydro-2*H*-pyrazino[1,2-a]pyrazin-2-yl) ethyl)-6-fluoro-10-methyl-1-(6-methyl-4-(trifluoromethyl)pyridin-2-yl)-1,3a,4,5,10,11a-hexahydro-2*H*-benzo [*b*]pyrrolo[2,3-*f*][1,4]diazocine-2,11(3*H*)-dione | **Intermediate 7a** and *rac-tert*-butyl octahydro-2H-pyrazino[1,2-a]pyrazine-2-carboxylate (CAS: 1159825-34-9) | ($CDCl_3$) 8.32 (s, 1H), 7.38 - 7.30 (m, 1H), 7.22 (d, *J* = 8.0 Hz, 1H), 7.18 - 7.11 (m, 1H), 7.06 (s, 1H), 6.55 (dd, *J* = 10.4, 16.8 Hz, 1H), 6.29 (dd, *J* = 2.0, 16.8 Hz, 1H), 5.71 (dd, *J* = 1.6, 10.4 Hz, 1H), 4.68 - 4.43 (m, 2H), 4.00 - 3.69 (m, 1H), 3.65 - 3.45 (m, 1H), 3.45 - 3.07 (m, 6H), 3.00 - 2.65 (m, 8H), 2.50 (s, 3H), 2.48 - 1.85 (m, 8H) | 644.3 |
| **23b** | 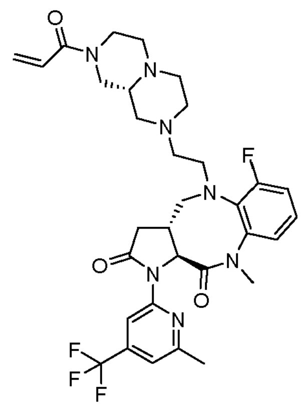 | (3a*R*,11a*S*)-5-(2-((R/S)-8-acryloyloctahydro-2*H*-pyrazino[1,2-a]pyrazin-2-yl) ethyl)-6-fluoro-10-methyl-1-(6-methyl-4-(trifluoromethyl)pyridin-2-yl)-1,3a,4,5,10,11a-hexahydro-2*H*-benzo [b]pyrrolo[2,3-*f*][1,4]diazocine-2,11(3H)-dione | | ($CDCl_3$) 8.33 (s, 1H), 7.39 - 7.31 (m, 1H), 7.23 (d, *J* = 7.6 Hz, 1H), 7.15 (t, *J* = 9.2 Hz, 1H), 7.07 (s, 1H), 6.57 (dd, *J* = 10.4, 16.8 Hz, 1H), 6.30 (d, *J* = 16.4 Hz, 1H), 5.72 (d, *J* = 10.4 Hz, 1H), 4.68 - 4.42 (m, 2H), 4.01 - 3.71 (m, 1H), 3.67 - 3.50 (m, 1H), 3.36 (s, 3H), 3.32 - 3.16 (m, 2H), 2.96 - 2.69 (m, 8H), 2.51 (s, 3H), 2.48 - 2.24 (m, 5H), 2.24 - 2.16 (m, 2H), 2.14 - 2.04 (m, 1H), 1.90 - 1.81 (m, 1H) | 644.3 |

(continued)

| Example Number | Structure | Name | Intermediates | $^1$H NMR (400 MHz) δ ppm | MI |
|---|---|---|---|---|---|
| **24a** | 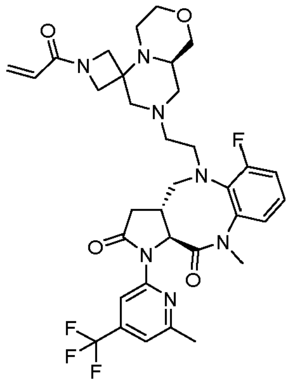 | (3aR,11aS)-5-(2-((R/S)-1-acryloyltetrahydro-1'*H*-spiro[azetidine-3,6'-pyrazino[2,1-*c*][1,4]oxazin]-8'(7'*H*)-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Intermediate 7a** and **Intermediate A12** | ($CDCl_3$) 8.33 (s, 1H), 7.30 - 7.39 (m, 1H), 7.23 (d, *J* = 8.0 Hz, 1H), 7.15 (t, *J* = 8.8 Hz, 1H), 7.06 (s, 1H), 6.12 - 6.41 (m, 2H), 5.59 - 5.78 (m, 1H), 4.60 (d, *J* = 9.2 Hz, 1H), 4.08 - 4.41 (m, 2H), 3.98 - 3.88 (m, 1H), 3.53 - 3.82 (m, 5H), 3.35 (s, 3H), 3.08 - 3.28 (m, 3H), 2.53 - 3.04 (m, 8H), 2.49 (s, 3H), 2.14 - 2.45 (m, 4H), 1.72 - 1.87 (m, 1H) | 686.3 |
| **24b** | 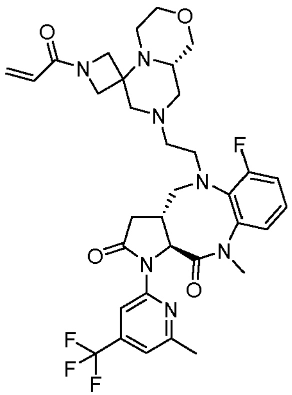 | (3a*R*,11a*S*)-5-(2-((*S*/*R*)-1-acryloyltetrahydro-1'*H*-spiro[azetidine-3,6'-pyrazino[2,1-c][1,4]oxazin]-8'(7'*H*)-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | | ($CDCl_3$) 8.34 (s, 1H), 7.42 - 7.32 (m, 1H), 7.24 (d, *J* = 8.4 Hz, 1H), 7.16 (t, *J* = 9.2 Hz, 1H), 7.07 (s, 1H), 6.43 - 6.33 (m, 1H), 6.31 - 6.17 (m, 1H), 5.79 - 5.66 (m, 1H), 4.66 - 4.58 (m, 1H), 4.41 - 4.08 (m, 2H), 3.97 - 3.81 (m, 2H), 3.75 - 3.54 (m, 4H), 3.38 (s, 3H), 3.32 - 3.14 (m, 3H), 3.04 - 2.84 (m, 4H), 2.75 (dd, *J* = 8.4, 16.4 Hz, 1H), 2.60 - 2.53 (m, 1H), 2.51 (s, 3H), 2.46 - 2.34 (m, 2H), 2.32 - 2.15 (m, 4H), 1.89 - 1.73 (m, 1H) | 686.3 |

(continued)

| Example Number | Structure | Name | Intermediates | $^1$H NMR (400 MHz) δ ppm | MI |
|---|---|---|---|---|---|
| **25a** | 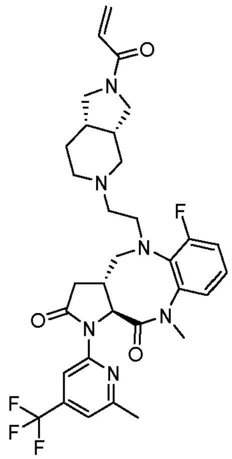 | (3a*R*,11a*S*)-5-(2-((3aS/R,7aS/R)-2-acryloyloctahydro-5H-pyrrolo[3,4-c]pyridin-5-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Intermediate 7a** and *tert*-butyl *rel-cis*-octahydro-2H-pyrrolo[3,4-c]pyridine-2-carboxylate (CAS: 236406-56-7) | ($CDCl_3$) 8.32 (s, 1H), 7.37 - 7.29 (m, 1H), 7.22 (d, *J* = 8.4 Hz, 1H), 7.13 (t, *J* = 9.2 Hz, 1H), 7.06 (s, 1H), 6.49 - 6.32 (m, 2H), 5.70 - 5.63 (m, 1H), 4.60 (d, *J* = 8.4 Hz, 1H), 3.62 - 3.46 (m, 5H), 3.46 - 3.36 (m, 1H), 3.35 (s, 3H), 3.27 - 3.10 (m, 2H), 2.97 - 2.85 (m, 2H), 2.77 - 2.66 (m, 1H), 2.50 (s, 3H), 2.43 - 2.34 (m, 3H), 2.34 - 2.24 (m, 3H), 2.24 - 2.15 (m, 2H), 1.79 - 1.66 (m, 1H), 1.58 - 1.45 (m, 1H) | 629.3 |
| **25b** | 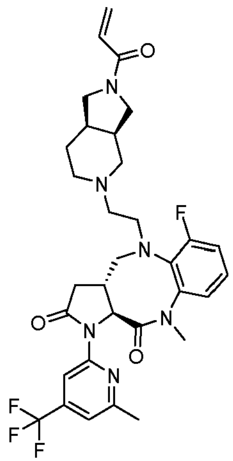 | (3a*R*,11a*S*)-5-(2-((3a*R*/*S*,7a*R*/*S*)-2-acryloyloctahydro-5H-pyrrolo[3,4-c]pyridin-5-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | | ($CDCl_3$) 8.32 (s, 1H), 7.38 - 7.29 (m, 1H), 7.25 - 7.19 (m, 1H), 7.18 - 7.10 (m, 1H), 7.06 (s, 1H), 6.49 - 6.32 (m, 2H), 5.72 - 5.62 (m, 1H), 4.60 (d, *J* = 9.2 Hz, 1H), 3.60 - 3.37 (m, 5H), 3.35 (d, *J* = 2.4 Hz, 3H), 3.28 - 3.12 (m, 2H), 2.98 - 2.81 (m, 2H), 2.77 - 2.64 (m, 1H), 2.50 (s, 3H), 2.46 - 2.10 (m, 9H), 1.79 - 1.65 (m, 1H), 1.57 - 1.42 (m, 1H) | 629.2 |

(continued)

| Example Number | Structure | Name | Intermediates | $^1$H NMR (400 MHz) δ ppm | MI |
|---|---|---|---|---|---|
| **26a** | 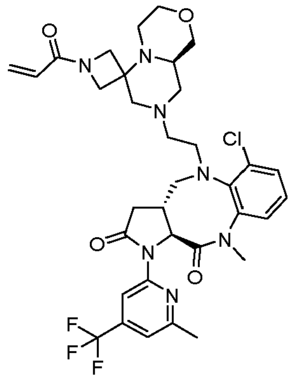 | (3aR,11aS)-5-(2-((R/S)-1-acryloyltetrahydro-1'*H*-spiro[azetidine-3,6'-pyrazino[2,1-c][1,4]oxazin]-8'(7'H)-yl)ethyl)-6-chloro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Intermediate 7b** and **Intermediate A12** | ($CDCl_3$) 8.35 (s, 1H), 7.48 - 7.45 (m, 1H), 7.37 - 7.30 (m, 2H), 7.07 (s, 1H), 6.41 - 6.32 (m, 1H), 6.29 - 6.14 (m, 1H), 5.73 - 5.70 (m, 1H), 4.64 - 4.61 (m, 1H), 4.38 - 4.09 (m, 2H), 3.98 - 3.85 (m, 2H), 3.76 - 3.66 (m, 2H), 3.61 - 3.57 (m, 2H), 3.39 (s, 3H), 3.25 - 3.10 (m, 3H), 3.07 - 2.96 (m, 2H), 2.95 - 2.85 (m, 2H), 2.80 - 2.69 (m, 1H), 2.64 - 2.52 (m, 2H), 2.50 (s, 3H), 2.47 - 2.32 (m, 3H), 2.31 - 2.18 (m, 2H), 1.88 - 1.72 (m, 1H) | 702.2 |
| **26b** | 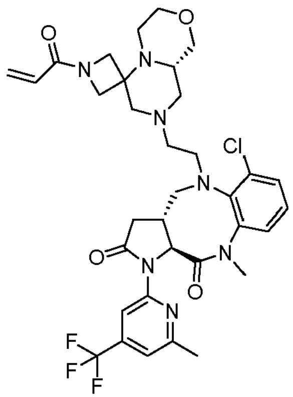 | (3aR,11 aS)-5-(2-((S/R)-1-acryloyltetrahydro-1'*H*-spiro[azetidine-3,6'-pyrazino [2,1-*c*][1,4]oxazin]-8'(7'*H*)-yl)ethyl)-6-chloro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | | ($CDCl_3$) 8.34 (s, 1H), 7.47 - 7.44 (m, 1H), 7.36 - 7.29 (m, 2H), 7.06 (s, 1H), 6.41 - 6.31 (m, 1H), 6.28 - 6.11 (m, 1H), 5.76 - 5.64 (m, 1H), 4.61 (d, *J* = 9.2 Hz, 1H), 4.39 - 4.22 (m, 1H), 4.19 - 4.10 (m, 1H), 3.93 - 3.90 (m, 1H), 3.76 - 3.61 (m, 3H), 3.61 - 3.50 (m, 2H), 3.42 - 3.37 (m, 3H), 3.22 - 3.20 (m, 2H), 3.15 - 3.12 (m, 1H), 3.06 - 2.94 (m, 2H), 2.93 - 2.90 (m, 2H), 2.77 - 2.64 (m, 1H), 2.61 - 2.51 (m, 2H), 2.48 (s, 3H), 2.42 - 2.34 (m, 3H), 2.26 - 2.23 (m, 2H), 1.91 - 1.72 (m, 1H) | 702.2 |

**Example 27**

**(3aR,11aS)-5-(2-(2-((E)-4-(dimethylamino)but-2-enoyl)-5-oxa-2,8-diazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione hydrochloride**

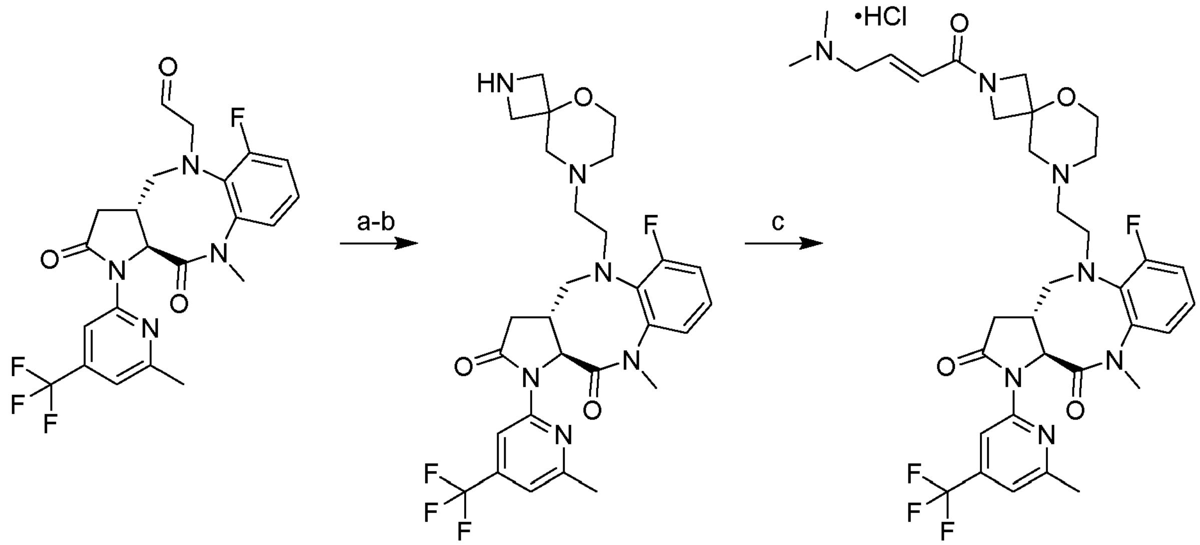


**Steps a-b.** These 2 steps were conducted in a similar manner to **Example 1,** steps a-b, using **Intermediate 7a** and *tert-butyl* 5-oxa-2,8-diazaspiro[3.5]nonane-2-carboxylate (CAS: 1251011-05-8) in step a.

**Step c.** A mixture of (3aR,11aS)-5-(2-(5-oxa-2,8-diazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (40 mg, 0.069 mmol), (*E*)-4-(dimethylamino)but-2-enoic acid hydrochloride (CAS: 848133-35-7; 14 mg, 0.083 mmol), HATU (26 mg, 0.069 mmol) and DIPEA (9 mg, 0.069 mmol) in DCM (2 mL) was stirred at 25 °C for 1 h under a $N_2$ atmosphere. Upon completion, the mixture was quenched with water (0.1 mL) and evaporated. The residue was purified by Prep-HPLC. The combined product containing fractions were evaporated, and the residue was lyophilised with 0.1 N hydrochloric acid to give the title compound as a HCl salt (22 mg, 43% yield) as a white solid.

m/z ES+ $[M+H]^+$ 688.5; $^1H$ NMR (400 MHz, $CD_3OD$) δ ppm 8.32 (s, 1H), 7.56 - 7.44 (m, 2H), 7.31 (t, *J* = 9.2 Hz, 1H), 7.26 (s, 1H), 6.81 - 6.70 (m, 1H), 6.54 (d, *J* = 16.0 Hz, 1H), 4.70 (d, *J* = 9.6 Hz, 1H), 4.60 - 4.44 (m, 1H), 4.32 (d, *J* = 8.8 Hz, 1H), 4.21 - 4.13 (m, 1H), 4.05 - 3.96 (m, 5H), 3.77 - 3.66 (m, 2H), 3.60 - 3.50 (m, 2H), 3.42 (d, *J* = 3.6 Hz, 3H), 3.40 - 3.38 (m, 2H), 3.29 - 3.20 (s, 3H), 3.04 (t, *J* = 12.4 Hz, 1H), 2.95 - 2.79 (m, 7H), 2.78 - 2.67 (m, 1H), 2.57 (s, 3H), 2.45 - 2.38 (m, 1H).

**Example 28**

**(3a*R*,11a*S*)-5-(2-((*R*)-4-(1-acryloylazetidin-3-yl)-3-(fluoromethyl)piperazin-1-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

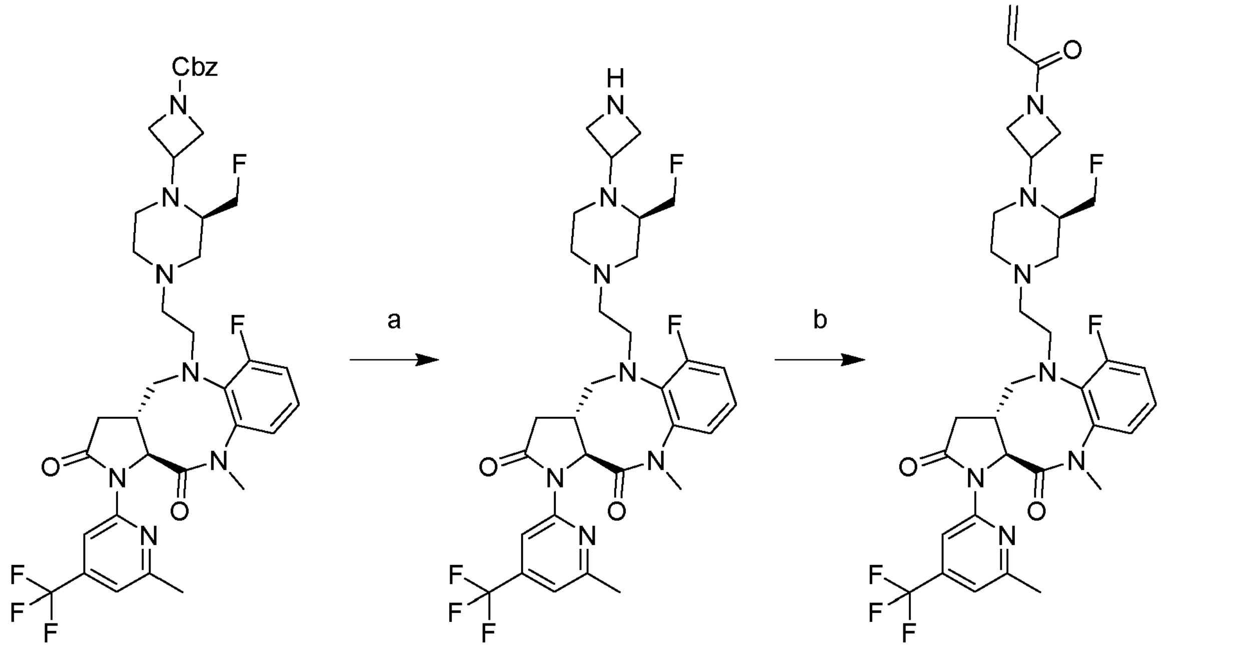


**Step a.** To a solution of **Intermediate** 10a (20 mg, 0.03 mmol) in DCM (1 mL) was added $BCl_3$ (1 M in DCM; 0.26 mL) at 0 °C dropwise. The mixture was stirred at 25 °C for 30 min. Upon completion, the reaction mixture was poured into sat. aq. $NaHCO_3$ (2 mL). The mixture was extracted with EtOAc (3 x 2 mL). The combined organic layers were dried over $Na_2SO_4$ and evaporated to give (3a*R*,11aS)-5-(2-((R)-4-(azetidin-3-yl)-3-(fluoromethyl)piperazin-1-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-f][1,4]diazocine-2,11(3H)-dione (16 mg, crude) as a yellow solid.

m/z ES+ $[M+H]^+$ 622.6.

**Step b.** To a solution of (3aR,11aS)-5-(2-((R)-4-(azetidin-3-yl)-3-(fluoromethyl)piperazin-1-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-f][1,4]diazocine-2,11(3H)-dione (15 mg, 0.02 mmol) and DIPEA (6 mg, 0.05 mmol) in DCM (0.5 mL) was added a solution of acryloyl chloride (2 mg, 0.02 mmol) in DCM (0.1 mL) at 0 °C. The mixture was stirred at 0 °C for 10 min. Upon completion, the reaction mixture was evaporated. The residue was purified by Prep-HPLC to give the title compound (4 mg, 23% yield) as a white solid.

m/z ES+ $[M+H]^+$ 676.3; $^1H$ NMR (400 MHz, $CDCl_3$) δ ppm 8.33 (s, 1H), 7.41 - 7.33 (m, 1H), 7.27 - 7.23 (m, 1H), 7.21 - 7.13 (m, 1H), 7.06 (s, 1H), 6.37 - 6.29 (m, 1H), 6.22 - 6.12 (m, 1H), 5.70 - 5.66 (m, 1H), 4.79 - 4.31 (m, 3H), 4.30 - 4.05 (m, 3H), 4.03 - 3.91 (m, 1H), 3.82 - 3.54 (m, 3H), 3.39 (s, 5H), 3.18 - 2.53 (m, 11H), 2.50 (s, 3H), 2.25 - 2.20 (m, 1H).

## Example 29

**(3a*R*,11a*S*)-5-(2-((*S*)-4-(1-acryloylazetidin-3-yl)-6-fluoro-1,4-diazepan-1-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

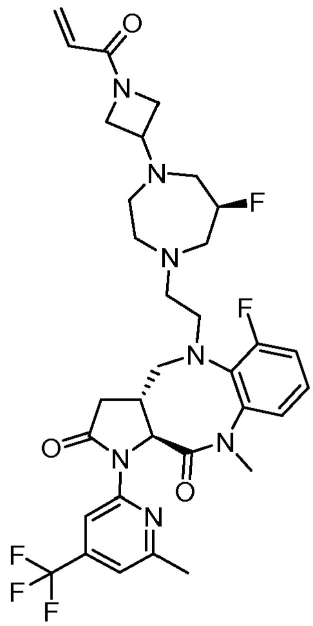

The title compound was prepared in a similar manner to **Example 28,** using **Intermediate 10b** in step a.

m/z ES+ $[M+H]^+$ 676.3; $^1H$ NMR (400 MHz, $CDCl_3$) δ ppm 8.32 (s, 1H), 7.40 - 7.32 (m, 1H), 7.23 (d, *J* = 8.0 Hz, 1H), 7.19 - 7.12 (m, 1H), 7.06 (s, 1H), 6.38 - 6.30 (m, 1H), 6.23 - 6.13 (m, 1H), 5.71 - 5.64 (m, 1H), 4.60 (m, 1H), 4.23 (m, 1H), 4.14 - 4.06 (m, 1H), 4.04 - 3.93 (m, 1H), 3.83 (m, 1H), 3.64 - 3.56 (m, 1H), 3.55 - 3.46 (m, 1H), 3.44 - 3.12 (m, 6H), 3.09 - 2.51 (m, 12H), 2.50 (s, 4H), 2.20 (m, 1H).

**Example 30**

**(3aR,11as)-5-(2-(2-acryloyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-f][1,4]diazocine-2,11(3H)-dione**

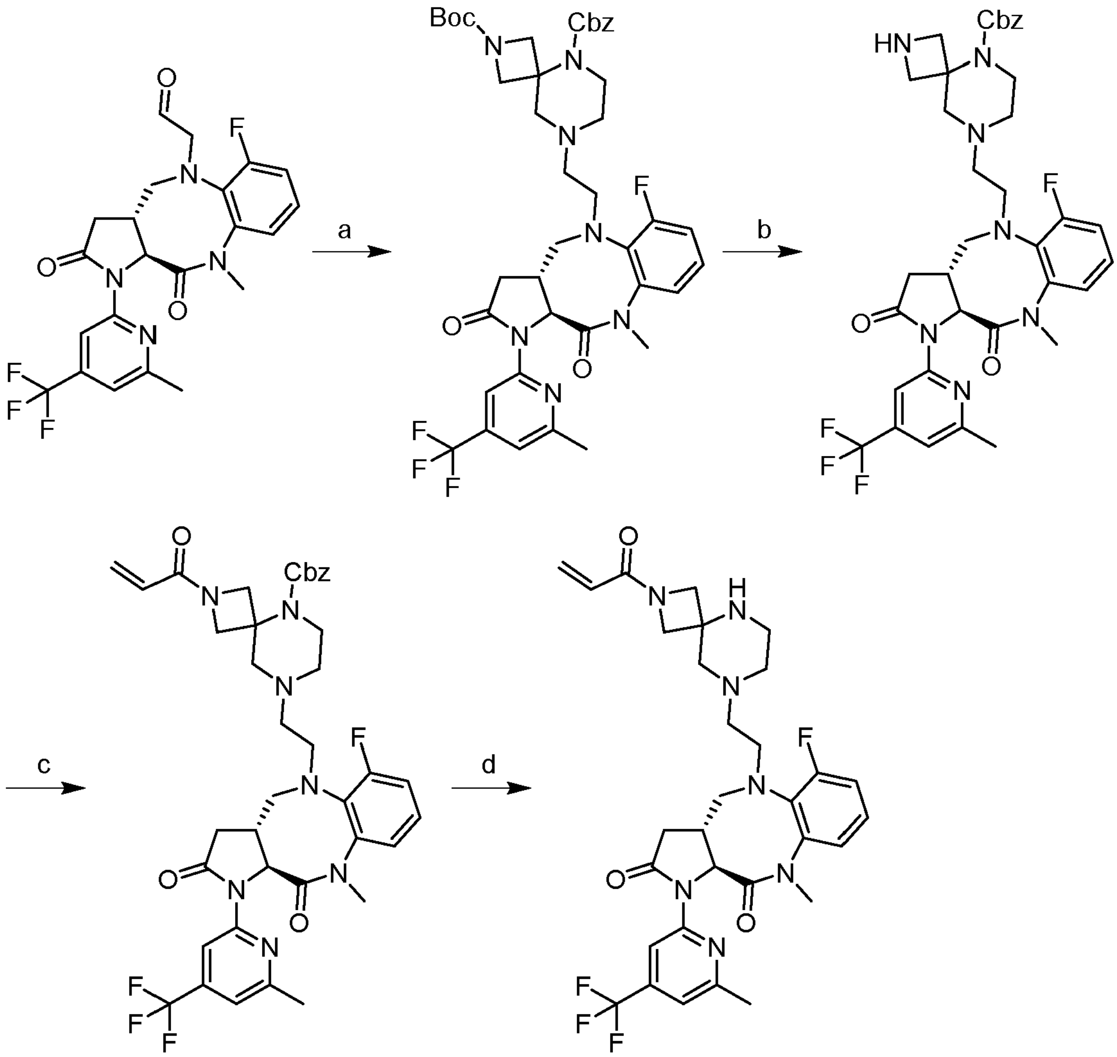


**Step a.** To a solution of **Intermediate 7a** (80 mg, 0.17 mmol) and **Intermediate A4** (62 mg, 0.17 mmol) in MeOH (1 mL) was added 4 Å molecular sieves (10 mg) and acetic acid (1 mg, 0.02 mmol). The mixture was stirred at rt for 5 min, and then $NaBH_3CN$ (13 mg, 0.21 mmol) was added. The mixture was stirred at rt for a further 25 min. Upon completion, the mixture was diluted with sat. aq. $NaHCO_3$ (5 mL), and then extracted with EtOAc (3 x 5 mL). The combined organic layers were washed with brine (10 mL), dried over $Na_2SO_4$ and evaporated. The crude product was purified by Prep-TLC (PE/EtOAc = 1/1) to give 5-benzyl *2-(tert-butyl)* 8-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5H-benzo[b]pyrrolo[2,3-*f*][1,4]diazocin-5-yl)ethyl)-2,5,8-triazaspiro[3.5]nonane-2,5-dicarboxylate (80 mg, 45% yield) as a yellow solid.

m/z ES+ $[M+H]^+$ 810.3.

**Step b.** To a solution of 5-benzyl *2-(tert-butyl)* 8-(2-((3aR,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,5,8-triazaspiro[3.5]nonane-2,5-dicarboxylate (70 mg, 0.07 mmol) in DCM (1 mL) was added TFA (308 mg, 2.70 mmol). The mixture was stirred at rt for 30 min. Upon completion, the mixture was diluted with DCM (3 mL) and then basified to pH 8 with sat. aq. $NaHCO_3$. The organic layer was separated and the aqueous was further extracted with DCM (2 x 4 mL). The combined organic layers were washed with brine (6 mL), dried over $Na_2SO_4$ and evaporated to give benzyl 8-(2-((3aR,11aS)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-t][1,4]diazocin-5-yl)ethyl)-2,5,8-triazaspiro[3.5]nonane-5-carboxylate as a TFA salt (60 mg, crude) as a yellow oil.

m/z ES+ $[M+H]^+$ 710.2.

**Step c.** To a solution of benzyl 8-(2-((3aR,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,5,8-triazaspiro[3.5]nonane-5-carboxylate (60 mg, 0.08 mmol) in DCM (0.5 mL) was added DIPEA (22 mg, 0.17 mmol) ) and subsequently cooled to 0 °C. Acryloyl chloride (8 mg, 0.09 mmol) in DCM (0.1 mL) was added dropwise. The mixture was stirred at 0 °C for

10 min. Upon completion, the reaction mixture was evaporated under vacuum. The crude product was purified by reverse phase flash chromatography (water (0.1% $NH_4OH$)/MeCN) to give benzyl 2-acryloyl-8-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5H-benzo[b]pyrrolo[2,3-t][1,4]diazocin-5-yl)ethyl)-2,5,8-triazaspiro[3.5]nonane-5-carboxylate (40 mg, 55% yield) as a white solid.

m/z ES+ $[M+H]^+$ 764.3; $^1H$ NMR (400 MHz, $CDCl_3$) δ ppm 8.33 (s, 1H), 7.36 - 7.34 (m, 6H), 7.23 - 7.22 (m, 1H), 7.15 (t, *J* = 8.8 Hz, 1H), 7.06 (s, 1H), 6.39 - 6.31 (m, 1H), 5.70 (d, *J* = 9.6 Hz, 1H), 5.31 (s, 2H), 5.20 - 5.10 (m, 2H), 4.59 (d, *J* = 8.8 Hz, 1H), 4.26 - 3.67 (m, 5H), 3.61 - 3.40 (m, 3H), 3.35 (s, 3H), 3.33 - 3.12 (m, 2H), 2.99 - 2.66 (m, 5H), 2.49 (s, 3H), 2.44 - 2.16 (m, 3H).

**Step d.** A mixture of benzyl 2-acryloyl-8-(2-((3aR,11aS)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-f][1,4]diazocin-5-yl)ethyl)-2,5,8-triazaspiro[3.5]nonane-5-carboxylate (40 mg, 0.051 mmol) and TFA (0.5 mL) was stirred at 45 °C for 16 h. Upon completion, the mixture was evaporated under vacuum, and the residue was dissolved in DCM (5 mL) and basified to pH 9 with sat. aq. $Na_2CO_3$. The organic layer was separated and the aqueous was extracted with DCM (2 x 5 mL). The combined organic layers were washed with brine (10 mL), dried over $Na_2SO_4$ and evaporated. The crude product was purified by Prep-HPLC to give the title compound (11 mg, 35% yield) as a yellow solid.

m/z ES+ $[M+H]^+$ 630.3; $^1H$ NMR (400 MHz, $CDCl_3$) δ ppm 8.33 (s, 1H), 7.35 - 7.33 (m, 1H), 7.22 (d, *J* = 7.6 Hz, 1H), 7.14 (t, *J* = 9.2 Hz, 1H), 7.06 (s, 1H), 6.40 - 6.31 (m, 1H), 6.27 - 6.16 (m, 1H), 5.72 - 5.66 (m, 1H), 4.61 (d, *J* = 9.2 Hz, 1H), 4.04 (d, *J* = 8.4 Hz, 1H), 3.92 - 3.84 (m, 2H), 3.72 (d, *J* = 10.8 Hz, 1H), 3.60 - 3.52 (m, 1H), 3.35 (d, *J* = 2.0 Hz, 3H), 3.24 - 3.16 (m, 2H), 2.98 - 2.78 (m, 4H), 2.72 - 2.68 (m, 1H), 2.61 - 2.50 (m, 1H), 2.49 (s, 3H), 2.48 - 2.37 (m, 3H), 2.37 - 2.26 (m, 2H), 2.21 (m, 1H).

The **Examples** in **Table 3** were prepared using methods similar to those described in the synthesis of **Example 30,** using the listed **Intermediates** in step a.

The **Examples** in **Table 4** were prepared using methods similar to those described in the synthesis of **Example 30,** using the listed **Intermediates** in step a, and an additional chiral SFC step was conducted after step a.

**Table 3**

| Example Number | Structure | Name | Intermediates | $^1$H NMR (400 MHz) δ ppm | MI |
|---|---|---|---|---|---|
| **31** | 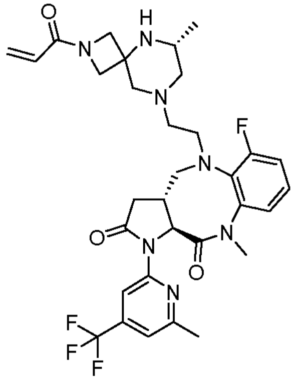 | (3a*R*,11a*S*)-5-(2-((R)-2-acryloyl-6-methyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7a** and **Intermediate A5** | ($CDCl_3$) 8.33 (s, 1H), 7.38 - 7.31 (m, 1H), 7.23 (d, *J* = 8.0 Hz, 1H), 7.14 (t, *J* = 9.2 Hz, 1H), 7.06 (s, 1H), 6.40 - 6.30 (m, 1H), 6.28 - 6.12 (m, 1H), 5.75 - 5.64 (m, 1H), 4.60 (d, *J* = 9.2 Hz, 1H), 4.11 (dd, *J* = 2.0, 7.2 Hz, 1H), 3.98 - 3.86 (m, 2H), 3.84 - 3.72 (m, 1H), 3.62 - 3.53 (m, 1H), 3.35 (s, 3H), 3.21 (s, 2H), 2.97 - 2.82 (m, 4H), 2.77 - 2.67 (m, 2H), 2.49 (s, 3H), 2.42 (dd, *J* = 5.6, 12.4 Hz, 1H), 2.36 - 2.28 (m, 1H), 2.26 - 2.03 (m, 3H), 1.75 - 1.73 (m, 1H), 1.07 (d, *J* = 5.2 Hz, 3H) | 644.4 |
| **32** | 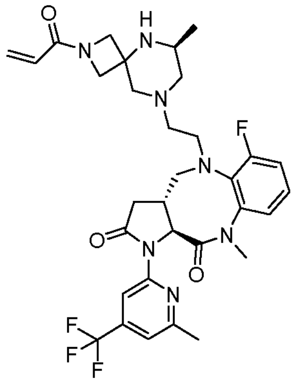 | (3a*R*,11a*S*)-5-(2-((S)-2-acryloyl-6-methyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7a** and **Intermediate A6** | ($CDCl_3$) 8.32 (s, 1H), 7.38 - 7.30 (m, 1H), 7.22 (d, *J* = 8.0 Hz, 1H), 7.18 - 7.10 (m, 1H), 7.06 (s, 1H), 6.39 - 6.28 (m, 1H), 6.25 - 6.15 (m, 1H), 5.69 (dd, *J* = 2.0, 10.4 Hz, 1H), 4.60 (dd, *J* = 3.2, 9.2 Hz, 1H), 4.15 (d, *J* = 13.6 Hz, 1H), 4.10 - 3.94 (m, 2H), 3.92 - 3.80 (m, 1H), 3.55 (d, *J* = 12.4 Hz, 1H), 3.36 (s, 3H), 3.30 - 3.13 (m, 2H), 3.03 - 2.81 (m, 4H), 2.78 - 2.61 (m, 2H), 2.49 (s, 3H), 2.45 - 2.31 (m, 2H), 2.29 - 2.13 (m, 3H), 1.85 - 1.80 (m, 1H), 1.10 (d, *J* = 4.4 Hz, 3H) | 644.4 |

(continued)

| Example Number | Structure | Name | Intermediates | $^1$H NMR (400 MHz) δ ppm | MI |
|---|---|---|---|---|---|
| **33** | 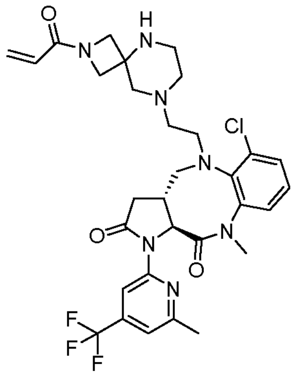 | (3a*R*,11a*S*)-5-(2-(2-acryloyl-2,5,8-triazaspiro[3.5]nonan-8-yl)ethyl)-6-chloro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 7b** and **Intermediate A4** | ($CDCl_3$) 8.34 (s, 1H), 7.48 - 7.42 (m, 1H), 7.35 - 7.28 (m, 2H), 7.06 (s, 1H), 6.40 - 6.30 (m, 1H), 6.25 - 6.13 (m, 1H), 5.75 - 5.65 (m, 1H), 4.62 (d, *J* = 9.2 Hz, 1H), 4.14 - 3.99 (m, 1H), 3.99 - 3.93 (m, 1H), 3.92 - 3.77 (m, 2H), 3.63 - 3.51 (m, 1H), 3.38 (s, 3H), 3.22 - 3.08 (m, 2H), 3.06 - 2.93 (m, 2H), 2.92 - 2.80 (m, 2H), 2.76 - 2.69 (m, 1H), 2.65 - 2.49 (m, 2H), 2.48 (s, 3H), 2.47 - 2.30 (m, 4H), 2.27 - 2.19 (m, 1H) | 646.3 |

**Table 4**

| Example Number | Structure | Name | Intermediates | $^1$H NMR (400 MHz) δ ppm | MI |
|---|---|---|---|---|---|
| **34a** | 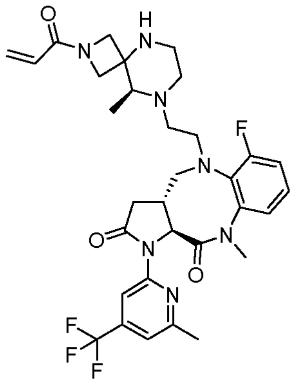 | (3a*R*,11a*S*)-5-(2-((S/R)-2-acryloyl-9-methyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Intermediate 7a** and **Intermediate A7** | ($CDCl_3$) 8.33 (s, 1H), 7.38 - 7.30 (m, 1H), 7.22 (d, *J* = 8.0 Hz, 1H), 7.19 - 7.11 (m, 1H), 7.06 (s, 1H), 6.39 - 6.30 (m, 1H), 6.30 - 6.13 (m, 1H), 5.74 - 5.62 (m, 1H), 4.69 - 4.55 (m, 1H), 4.03-4.01 (m, 2H), 3.87 - 3.85 (m, 1H), 3.84 - 3.68 (m, 1H), 3.63 - 3.53 (m, 2H), 3.36 (s, 3H), 3.21 - 3.11 (m, 2H), 2.96 - 2.81 (m, 5H), 2.78 - 2.65 (m, 2H), 2.50 (s, 3H), 2.47 - 2.29 (m, 3H), 2.21 - 2.17(m, 1H), 0.92 (s, 3H) | 644.3 |
| **34b** | 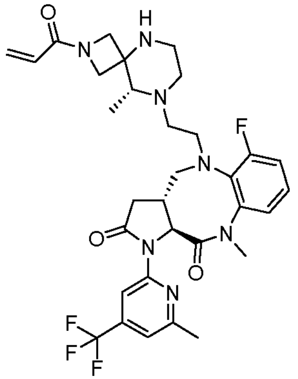 | (3a*R*,11a*S*)-5-(2-((R/S)-2-acryloyl-9-methyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | | ($CDCl_3$) 8.33 (s, 1H), 7.34 -7.32 (m, 1H), 7.22 (d, *J* = 8.0 Hz, 1H), 7.15 (t, *J* = 9.2 Hz, 1H), 7.06 (s, 1H), 6.38 - 6.31 (m, 1H), 6.27 - 6.15 (m, 1H), 5.68 (dd, *J* = 2.0, 10.2 Hz, 1H), 4.61 (d, *J* = 8.8 Hz, 1H), 4.12 - 3.82 (m, 4H), 3.80 - 3.55 (m, 2H), 3.36 (s, 3H), 3.24 - 3.07 (m, 2H), 2.99 - 2.67 (m, 6H), 2.62 - 2.51 (m, 1H), 2.49 (s, 3H), 2.47 - 2.27 (m, 3H), 2.25 - 2.16 (m, 1H), 1.05 - 0.88 (m, 3H) | 644.3 |

**Example 35**

**(3aR,11aS)-5-(2-(2-acryloyl-5-ethyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione**

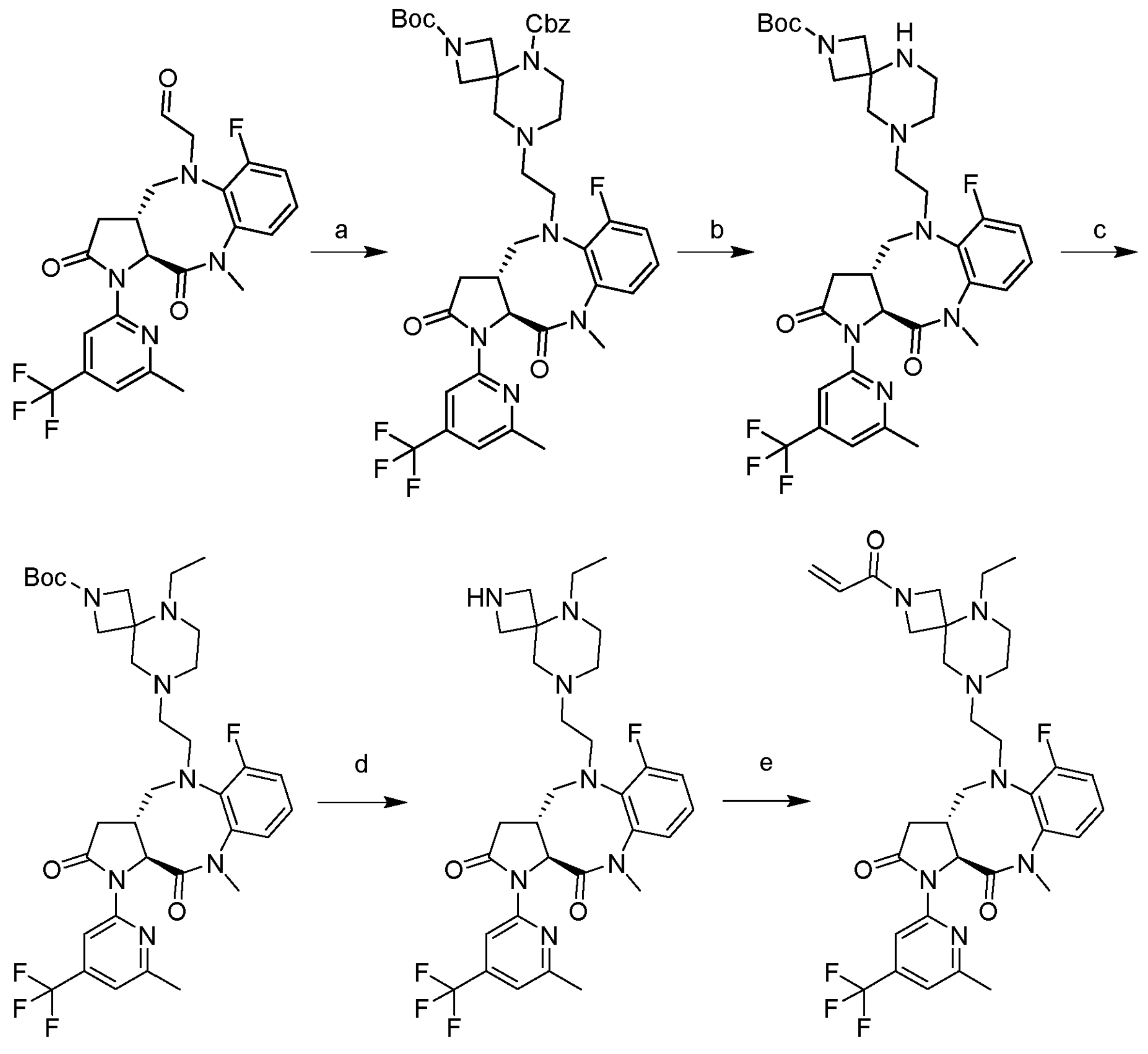


Step a. This step was conducted as described in Example 30, step a, using Intermediate 7a and Intermediate A4.

**Step b.** To a solution of 5-benzyl 2-(*tert*-butyl) 8-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-f][1,4]diazocin-5-yl)ethyl)-2,5,8-triazaspiro[3.5]nonane-2,5-dicarboxylate (110 mg, 0.14 mmol) in THF (4 mL) was added 10% Pd/C (29 mg), and the mixture was stirred at 25 °C for 1 h under a $H_2$ atmosphere (15 psi). Upon completion, the reaction mixture was filtered and evaporated to give *tert*-butyl 8-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,5,8-triazaspiro[3.5]nonane-2-carboxylate (90 mg, 98% yield) as a colourless solid.

m/z ES+ $[M+H]^+$ 676.4.

**Step c.** To a solution of *tert*-butyl 8-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,5,8-triazaspiro[3.5]nonane-2-carboxylate (90 mg, 0.13 mmol) and acetaldehyde (18 mg, 0.40 mmol) in MeOH (3 mL) was added acetic acid (40 mg, 0.67 mmol), 4 Å molecular sieves (100 mg) and $NaBH_3CN$ (17 mg, 0.27 mmol). The mixture was stirred at 25 °C for 1 h. Upon completion, the reaction mixture was filtered and evaporated to give *tert-butyl* 5-ethyl-8-(2-((3aR,11aS)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)ethyl)-2,5,8-triazaspiro[3.5]nonane-2-carboxylate (90 mg, crude) as a colourless solid.

m/z ES+ $[M+H]^+$ 704.4.

**Step d.** To a solution of *tert-butyl* 5-ethyl-8-(2-((3aR,11aS)-6-fluoro-10-methyl-1-(6-methyl-4-(trifluoromethyl) pyridin-2-yl)-2,11-dioxo-1,2,3,3a,4,10,11,11a-octahydro-5*H*-benzo[b]pyrrolo[2,3-f][1,4]diazocin-5-yl)ethyl)-2,5,8-triazaspiro[3.5]nonane-2-carboxylate (90 mg, 0.13 mmol) in DCM (3 mL) was added TFA (1.46 g, 12.8 mmol) and the mixture was stirred at 25 °C for 30 min. Upon completion, the reaction mixture was evaporated to give (3aR,11aS)-5-(2-(5-ethyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione as a TFA salt (90 mg, crude) as a yellow solid.

m/z ES+ $[M+H]^+$ 604.4.

**Step e.** To a solution of (3aR,11aS)-5-(2-(5-ethyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione (90 mg, 0.13 mmol) in DCM (3 mL) was added DIPEA (81 mg, 0.63 mmol) and acryloyl chloride (17 mg, 0.19 mmol). The mixture was stirred at 25 °C for 30 min. Upon completion, the reaction mixture was evaporated and the resulting residue was purified by Prep-HPLC to give the title compound (47 mg, 56% yield) as a yellow solid.

m/z ES+ $[M+H]^+$ 658.4; $^1H$ NMR (400 MHz, $CDCl_3$) δ ppm 8.32 (s, 1H), 7.33 (td, *J* = 8.4, 5.6 Hz, 1H), 7.21 (d, *J* = 7.6 Hz, 1H), 7.13 (t, *J* = 9.2 Hz, 1H), 7.05 (s, 1H), 6.38 - 6.31 (m, 1H), 6.28 - 6.15 (m, 1H), 5.69 - 5.65 (m, 1H), 4.60 (d, *J* = 9.2 Hz, 1H), 4.21 - 4.14 (m, 1H), 4.05 (d, *J* = 11.2 Hz, 1H), 3.78 (d, *J* = 9.2 Hz, 1H), 3.62 (dd, *J* = 10.8, 6.4 Hz, 1H), 3.58 - 3.52 (m, 1H), 3.34 (d, *J* = 2.0 Hz, 3H), 3.25 - 3.15 (m, 2H), 2.96 - 2.82 (m, 2H), 2.70 (dd, *J* = 16.4, 8.0 Hz, 2H), 2.63 - 2.57(m, 4H), 2.49 (s, 3H), 2.47 - 2.39 (m, 3H), 2.37 - 2.27 (m, 2H), 2.19 (dd, *J* = 16.8, 11.2 Hz, 1H),1.08 (t, *J* = 7.2 Hz, 3H).

## Example 36

**(3a*R*,11a*S*)-5-(2-(5-acetyl-2-acryloyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

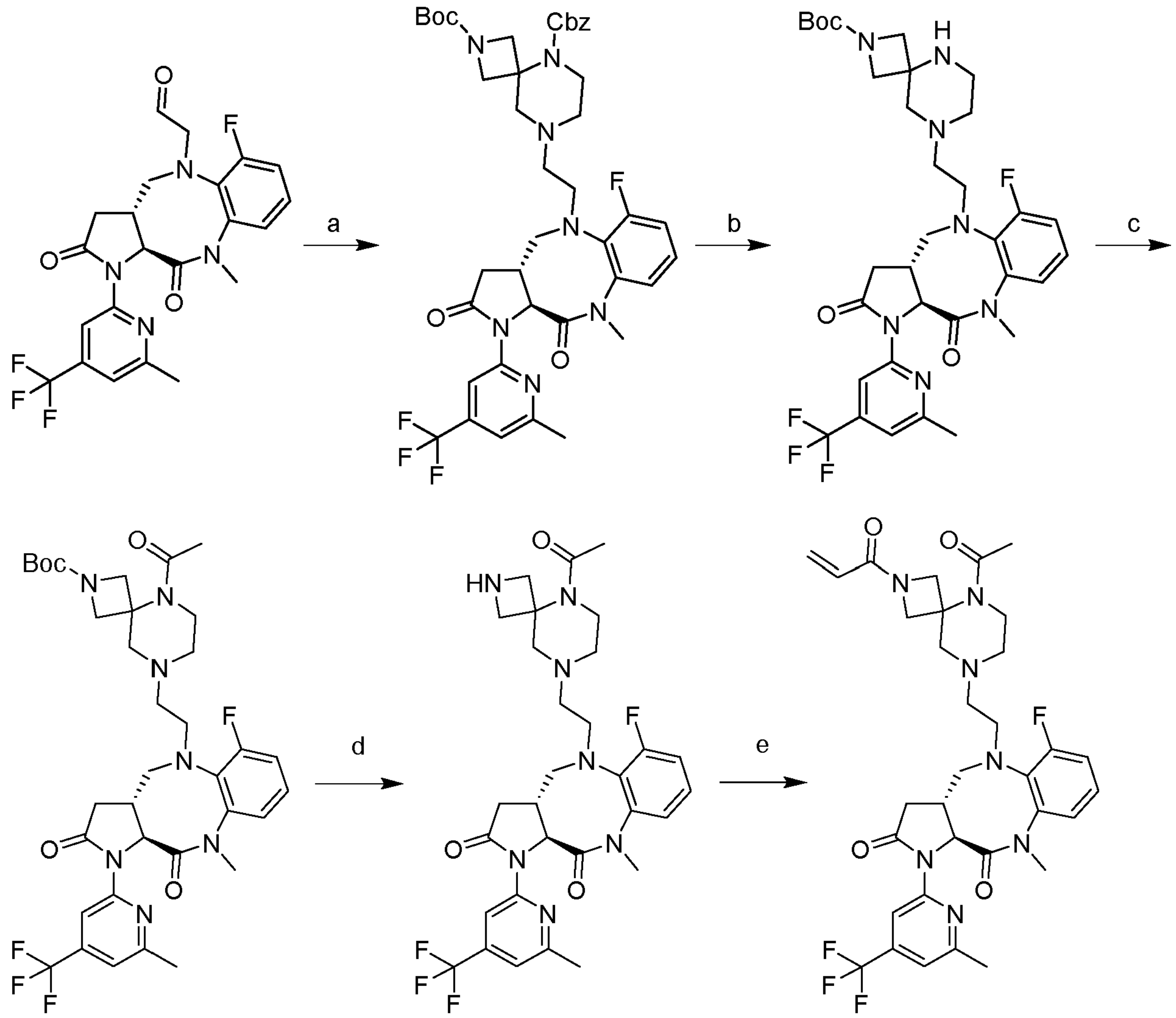


**Steps a-b.** These steps were conducted as described in **Example 35,** steps a-b, using **Intermediate 7a and Intermediate A4.**

**Step c.** To a solution of *tert-butyl* 8-(2-((3aR,11aS)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,5,8-triazaspiro[3.5]nonane-2-carboxylate (150 mg, 0.22 mmol) and acetic acid (17 mg, 0.29 mmol) in DCM (2 mL) was added DIPEA (72 mg, 0.55 mmol) and HATU (101 mg, 0.27 mmol). The mixture was stirred at 25 °C for 30 min. Upon completion, the mixture was diluted with water (5 mL) and extracted with EtOAc (3 x 5 mL). The combined organic layers were washed with brine (10 mL), dried over $Na_2SO_4$ and evaporated. The residue was purified by Prep-TLC (EtOAc) to give *tert-butyl* 5-acetyl-8-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5H-benzo[*b*]pyrrolo[2,3-*f*][1,4]diazocin-5-yl)ethyl)-2,5,8-triazaspiro[3.5]nonane-2-carboxylate (145 mg, 91% yield) as a white solid.

m/z ES+ $[M+H]^+$ 718.3; $^1$H NMR (400 MHz, $CDCl_3$) δ ppm 8.33 (s, 1H), 7.38 - 7.31 (m, 1H), 7.23 (d, *J* = 8.0 Hz, 1H), 7.18 - 7.12 (m, 1H), 7.06 (s, 1H), 4.61 (d, *J* = 9.2 Hz, 1H), 4.11 - 4.04 (m, 1H), 4.03 - 3.90 (m, 1H), 3.78 (dd, *J* = 9.2, 16.0 Hz, 2H), 3.60 - 3.53 (m, 1H), 3.35 (s, 3H), 3.32 - 3.23 (m, 1H), 3.20 (m, 2H), 2.97 - 2.85 (m, 2H), 2.81 (s, 4H), 2.72 (dd, *J* = 8.0, 16.8 Hz, 1H), 2.66 - 2.53 (m, 2H), 2.50 (s, 3H), 2.48 - 2.37 (m, 2H), 2.36 - 2.26 (m, 2H), 2.20 (dd, *J* = 11.2, 16.8 Hz, 1H), 1.45 (s, 9H).

**Step d.** To a solution of *tert-butyl* 5-acetyl-8-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,5,8-triazaspiro[3.5]nonane-2-carboxylate (120 mg, 0.17 mmol) in DCM (2 mL) was added TFA (1 mL). The mixture was stirred at 25 °C for 30 min. Upon completion, the mixture was adjusted to pH 8 with sat. aq. $NaHCO_3$ and then extracted with DCM (3 x 5 mL). The combined organic layers were washed with brine (10 mL), dried over $Na_2SO_4$ and evaporated to give (3a*R*,11a*S*)-5-(2-(5-acetyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (100 mg, crude) as a white

solid.

m/z ES+ $[M+H]^+$ 618.6.

**Step e.** To a solution of (3a*R*,11a*S*)-5-(2-(5-acetyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione (100 mg, 0.16 mmol) in DCM (1 mL) was added DIPEA (42 mg, 0.32 mmol). A solution of acryloyl chloride (19 mg, 0.21 mmol) in DCM (0.1 mL) was added dropwise at 0 °C. The mixture was stirred at 0 °C for 10 min. Upon completion, the mixture was diluted with water (10 mL) and extracted with EtOAc (3 x 10 mL). The combined organic layers were washed with brine (20 mL), dried over $Na_2SO_4$ and evaporated. The crude product was purified by Prep-HPLC to give the title compound (37 mg, 31% yield) as a white solid.

m/z ES+ $[M+H]^+$ 672.3; $^1H$ NMR (400 MHz, $CDCl_3$) δ ppm 8.32 (s, 1H), 7.39 - 7.31 (m, 1H), 7.28 - 7.23 (m, 1H), 7.18 - 7.11 (m, 1H), 7.05 (s, 1H), 6.39 - 6.30 (m, 1H), 6.26 - 6.13 (m, 1H), 5.69 - 5.67 (m, 1H), 4.60 (d, *J* = 9.2 Hz, 1H), 4.55 - 4.40 (m, 1H), 4.15 - 4.01 (m, 2H), 3.95 (s, 1H), 3.58 - 3.50 (m, 1H), 3.48 - 3.36 (m, 1H), 3.34 (d, *J* = 3.2 Hz, 3H), 3.30 - 3.14 (m, 3H), 2.98 - 2.79 (m, 2H), 2.77 - 2.64 (m, 2H), 2.64 - 2.55 (m, 1H), 2.54 - 2.38 (m, 5H), 2.36 - 2.29 (m, 1H), 2.29 - 2.14 (m, 2H), 2.06 (d, *J* = 2.0 Hz, 3H).

**Examples 37a and 37b**

**(3aR,11aS)-5-(2-((6R,9S/R)-2-acryloyl-6,9-dimethyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-1f][1,4] diazocine-2,11(3*H*)-dione and (3aR,11aS)-5-(2-((6R,9R/S)-2-acryloyl-6,9-dimethyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*] pyrrolo[2,3-*f*][1,4]diazocine-2,11(3*H*)-dione**

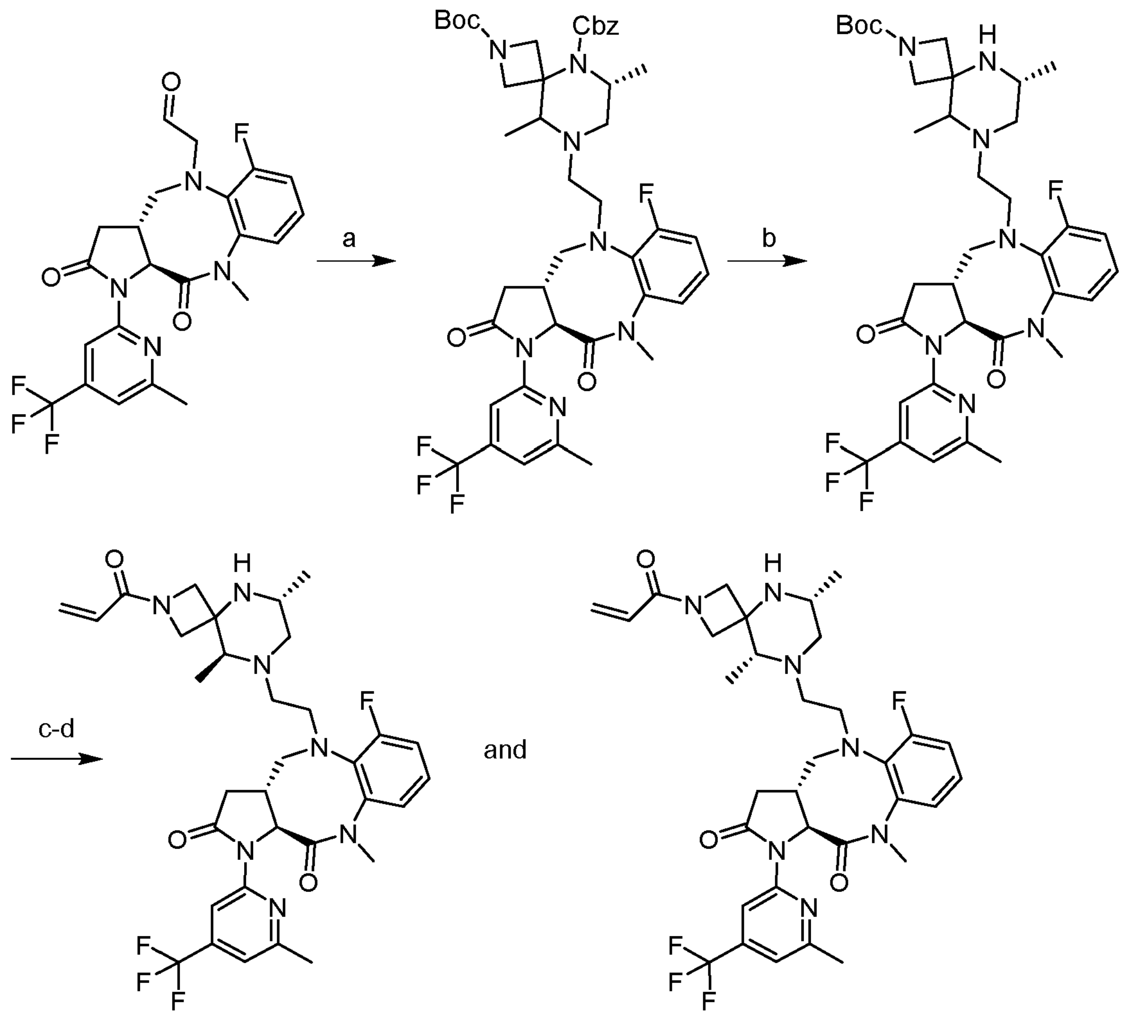

**Step** a. A mixture of **Intermediate 7a** (0.3 g, 0.65 mmol), **Intermediate A8** (503 mg, 1.29 mmol), acetic acid (193 mg, 3.23 mmol) and 4 Å molecular sieves (0.3 g) in MeOH (3 mL) was stirred at 25 °C for 30 min, after which, $NaBH_3CN$ (41 mg, 0.65 mmol) was added and the mixture was stirred at 25 °C for a further 1 h. Upon completion, the mixture was filtered and evaporated. The residue was purified by Prep-HPLC to give 5-benzyl *2-(tert*-butyl*)* (6*R*)-8-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5H-benzo[b] pyrrolo[2,3-*f*][1,4]diazocin-5-yl)ethyl)-6,9-dimethyl-2,5,8-triazaspiro[3.5]nonane-2,5-dicarboxylate (300 mg, 55% yield) as a white solid. m/z ES+ $[M+H]^+$ 838.6.

**Step b.** A mixture 5-benzyl *2-(tert-butyl)* (6R)-8-(2-((3aR,11aS)-6-fluoro-10-methyl-1-(6-methyl-4-(trifluoromethyl)pyridin-2-yl)-2,11-dioxo-1,2,3,3a,4,10,11,11a-octahydro-5H-benzo[b]pyrrolo[2,3-*f*][1,4]diazocin-5-yl)ethyl)-6,9-dimethyl-2,5,8-triazaspiro[3.5]nonane-2,5-dicarboxylate (100 mg, 0.12 mmol) and 10% Pd/C (20 mg) in 2-propanol (1 mL) was stirred at 25 °C under a $H_2$ atmosphere (15 psi) for 12 h. Upon completion, the mixture was filtered and evaporated to give tert-butyl (6R)-8-(2-((3aR,11aS)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-t][1,4]diazocin-5-yl)ethyl)-6,9-dimethyl-2,5,8-triazaspiro[3.5] nonane-2-carboxylate (83 mg, crude) as a white solid.

m/z ES+ $[M+H]^+$ 704.4.

**Step c.** A mixture of tert-butyl (6R)-8-(2-((3aR,11aS)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-t][1,4]diazocin-5-yl)ethyl)-6,9-dimethyl-2,5,8-triazaspiro[3.5]nonane-2-carboxylate (83 mg, 0.12 mmol) and TFA (1 mL) in DCM (1 mL) was stirred at 25 °C for 1 h. Upon completion, the mixture was adjusted pH to 8~9 with sat. aq. $NaHCO_3$ and diluted with water (30 mL). The mixture was extracted with EtOAc (2 x 30 mL). The combined organic layers were dried over $Na_2SO_4$ and evaporated to give (3a*R*,11aS)-5-(2-((6R)-6,9-dimethyl-2,5,8-triazaspiro[3.5]nonan-8-yl)ethyl)-6-fluoro-10-methyl-1-(6-methyl-4-(trifluoromethyl)pyridin-2-yl)-1 ,3a,4,5, 1 0, 11a-hexahydro-2H-benzo[b]pyrrolo[2,3-*f*][1,4]diazocine-2,11(3*H*)-dione (60 mg, 84% yield) as a colourless oil.

m/z ES+ $[M+H]^+$ 604.3.

**Step d.** To a solution of (3aR,11aS)-5-(2-((6R)-6,9-dimethyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (60 mg, 0.099 mmol), acrylic acid (11 mg, 0.15 mmol) and HATU (49 mg, 0.13 mmol) in DCM (1 mL) was added DIPEA (26 mg, 0.20 mmol). The mixture was stirred at 25 °C for 1 h. Upon completion, the mixture was evaporated. The residue was purified by Prep-HPLC to give two diastereoisomers with unknown absolute configuration.

**Example 37a:** (15 mg, 23% yield) as an off-white solid.

m/z ES+ $[M+H]^+$ 658.3; $^1H$ NMR (400 MHz, $CDCl_3$) δ ppm 8.34 (s, 1H), 7.40 - 7.32 (m, 1H), 7.26 - 7.21 (m, 1H), 7.16 (t, *J* = 9.2 Hz, 1H), 7.07 (s, 1H), 6.39 - 6.31 (m, 1H), 6.28 - 6.14 (m, 1H), 5.77 - 5.60 (m, 1H), 4.62 (d, *J* = 8.8 Hz, 1H), 4.10 - 3.78 (m, 1H), 4.10 - 3.78 (m, 2H), 3.76 - 3.48 (m, 3H), 3.37 (s, 3H), 3.26 - 3.15 (m, 2H), 2.99 - 2.81 (m, 2H), 2.80 - 2.60 (m, 3H), 2.51 (s, 3H), 2.41 - 2.15 (m, 3H), 2.04 - 1.89 (m, 1H), 1.56 (t, *J* = 7.2 Hz, 1H), 1.16 - 0.99 (m, 6H).

**Example 37b:** (15 mg, 23% yield) as a white solid.

m/z ES+ $[M+H]^+$ 658.3; $^1H$ NMR (400 MHz, $CDCl_3$) δ ppm 8.34 (s, 1H), 7.36 (dt, *J* = 5.6, 8.4 Hz, 1H), 7.24 (d, *J* = 8.0 Hz, 1H), 7.16 (t, *J* = 9.2 Hz, 1H), 7.07 (s, 1H), 6.41 - 6.31 (m, 1H), 6.31 - 6.13 (m, 1H), 5.74 - 5.64 (m, 1H), 4.71 - 4.57 (m, 1H), 4.17 - 4.01 (m, 1H), 3.98 - 3.84 (m, 2H), 3.77 - 3.53 (m, 2H), 3.44 - 3.30 (m, 3H), 3.26 - 3.05 (m, 2H), 3.00 - 2.80 (m, 4H), 2.79 - 2.67 (m, 1H), 2.51 (s, 3H), 2.47 - 2.30 (m, 3H), 2.28 - 2.09 (m, 2H), 1.57 (t, *J* = 7.2 Hz, 1H), 1.12 - 0.99 (m, 3H), 0.88 (d, *J* = 6.4 Hz, 3H).

**Example 38**

**(3a*R*,11a*S*)-5-((1-(1-acryloylazetidin-3-yl)-1*H*-imidazol-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

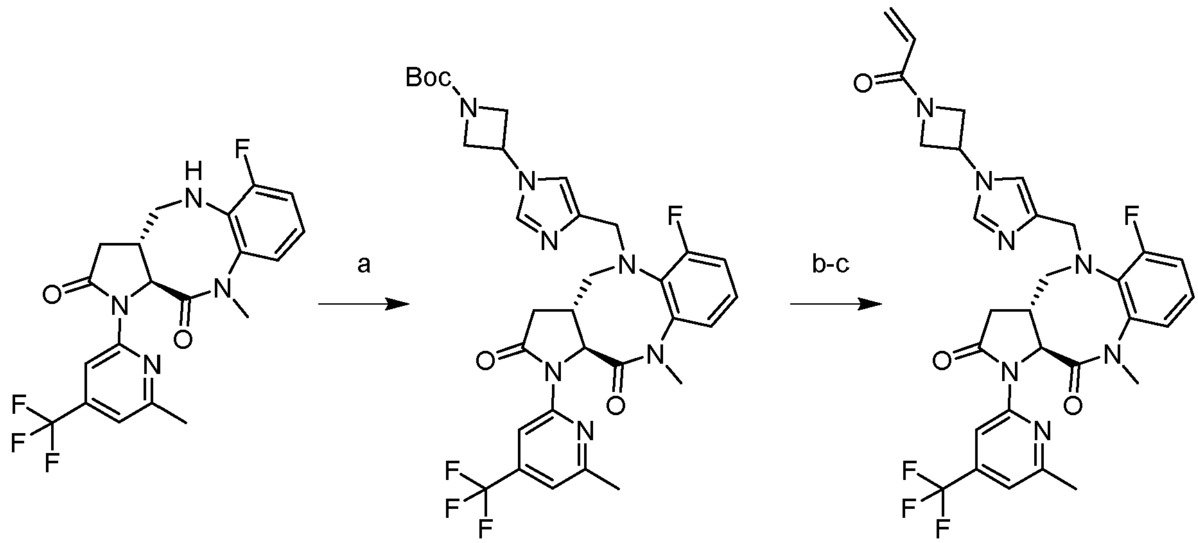


**Step a.** A mixture of **Intermediate B1** (140 mg, 0.56 mmol), **Intermediate 5a** (150 mg, 0.36 mmol) and acetic acid (107 mg, 1.78 mmol) in MeOH (3 mL) was stirred at 25 °C for 30 min, then $NaBH_3CN$ (44.6 mg, 0.71 mmol) was added. The reaction mixture was stirred at 25 °C for 12 h. Upon completion, the reaction mixture was filtered and evaporated. The residue was purified by Prep-TLC (EtOAc/MeOH = 10/1) to give *tert*-butyl 3-(4-(((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)-1H-imidazol-1-yl)azetidine-1-carboxylate (100 mg, 40% yield) as a yellow oil.

m/z ES+ $[M+H]^+$ 658.3.

**Step b.** To a solution of *tert-butyl* 3-(4-(((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)-1*H*-imidazol-1-yl) azetidine-1-carboxylate (60 mg, 0.091 mmol) in DCM (3 mL) was added TFA (1 mL). The reaction mixture was stirred at 20 °C for 30 min. Upon completion, the reaction mixture was evaporated. The residue was basified with sat. aq. $NaHCO_3$ (30 mL) and extracted with EtOAc (30 mL). The organic layer was washed with brine (30 mL), dried over $Na_2SO_4$ and evaporated to give (3aR,11aS)-5-((1-(azetidin-3-yl)-1H-imidazol-4-yl)methyl)-6-fluoro-10-methyl-1-(6-methyl-4-(trifluoromethyl)pyridin-2-yl)-1 ,3a,4,5, 1 0, 11a-hexahydro-2H-benzo[b]pyrrolo[2,3-*f*][1,4]diazocine-2,11(3*H*)-dione as a TFA salt (50 mg, crude) as a yellow gum.

m/z ES+ $[M+H]^+$ 558.2.

**Step c.** To a solution of (3aR,11aS)-5-[[1-(azetidin-3-yl)imidazol-4-yl]methyl]-6-fluoro-10-methyl-1-[6-methyl-4-(trifluoromethyl)-2-pyridyl]-3,3a,4,11a-tetrahydropyrrolo[2,3-c][1,6]benzodiazocine-2,11-dione (TFA salt, 50 mg, 0.09 mmol) and DIPEA (35 mg, 0.27 mmol) in DCM (2 mL) was added acryloyl chloride (12 mg, 0.14 mmol). The reaction mixture was stirred at 20 °C for 10 min. Upon completion, the reaction mixture was diluted with water (20 mL) and extracted with EtOAc (20 mL). The organic layer was washed with brine (20 mL), dried over $Na_2SO_4$ and evaporated. The residue was purified by Prep-HPLC to give the title compound (31 mg, 54% yield) as a white solid.

m/z ES+ $[M+H]^+$ 612.2; $^1H$ NMR (400 MHz, $CDCl_3$) δ ppm 8.86 (s, 1H), 8.30 (s, 1H), 7.42 - 7.32 (m, 1H), 7.25 - 7.10 (m, 3H), 7.06 (s, 1H), 6.46 - 6.30 (m, 1H), 6.27 - 6.12 (m, 1H), 5.75 (d, *J* = 10.0 Hz, 1H), 5.36 - 5.34 (m, 1H), 4.76 - 4.73 (m, 1H), 4.61 (d, *J* = 9.2 Hz, 2H), 4.52 - 4.76 (m, 2H), 4.26 - 4.20 (m, 2H), 3.77 - 3.67 (m, 1H), 3.28 (s, 3H), 3.07 - 2.84 (m, 2H), 2.82 - 2.68 - 2.55 (m, 1H), 2.49 (s, 3H), 2.26 - 2.10 (m, 1H).

**Example 39**

**(3a*R*,11a*S*)-5-((1-(1-acryloylazetidin-3-yl)-1H-imidazol-4-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

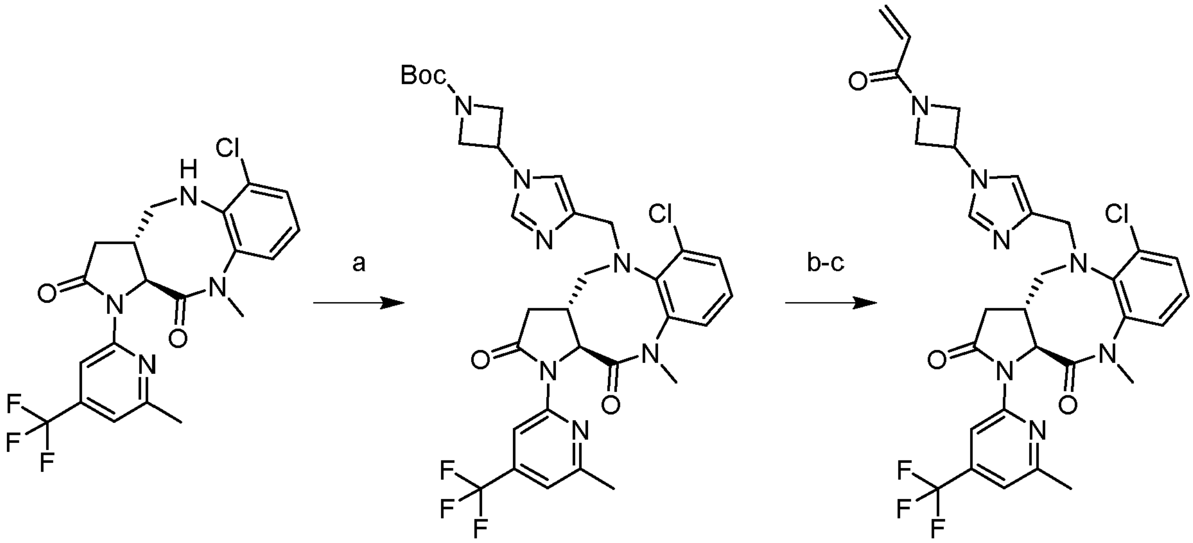


**Step a.** To a solution of **Intermediate 5b** (349 mg, 0.80 mmol), **Intermediate B1** (300 mg, 1.19 mmol) in DMF (3 mL) was added TMSCI (216 mg, 1.99 mmol). The mixture was stirred at 0 °C for 30 min. A solution of borane tetrahydrofuran complex (1 M in THF, 0.80 mL) in THF (7 mL) was added and the reaction mixture was stirred at 0 °C for 90 min, and then at 20 °C for 14 h. (LCMS showed some Boc-group cleavage). Upon completion, the reaction mixture was basified with $NaHCO_3$ (201 mg, 2.39 mmol), and di-tert-butyl dicarbonate (347 mg, 1.59 mmol) was added. The mixture was stirred at 20 °C for 3 h. Upon completion, the reaction mixture was quenched with methanol (4 mL) at 0°C, filtered and evaporated. The crude product was purified by reverse phase flash chromatography (water (0.1 FA)/MeCN) to give *tert-butyl* 3-(4-(((3aR,11aS)-6-chloro-10-methyl-1-(6-methyl-4- (trifluoromethyl) pyridin-2-yl)-2,11-dioxo-1,2,3,3a,4,10,11,11a-octahydro-5H-benzo[b]pyrrolo[2,3-f][1,4]diazocin-5-yl)methyl)-1H-imidazol-1-yl)azetidine-1-carboxylate (200 mg, 34% yield) as light yellow oil.

m/z ES+ $[M+H]^+$ 674.2.

**Steps b-c.** These 2 steps were conducted in a similar manner to **Example 38,** steps b-c.

m/z ES+ $[M+H]^+$ 628.1; $^1H$ NMR (400 MHz, $CDCl_3$) δ ppm 8.32 (s, 1H), 7.53 (s, 1H), 7.46 (dd, *J* = 2.4, 7.2 Hz, 1H), 7.34 - 7.28 (m, 2H), 7.13 (s, 1H), 7.05 (s, 1H), 6.45 - 6.36 (m, 1H), 6.27 - 6.17 (m, 1H), 5.76 (d, *J* = 10.4 Hz, 1H), 5.00 - 4.96 (m, 1H), 4.75 - 4.57 (m, 3H), 4.37 - 4.34 (m, 1H), 4.33 - 4.21 (m, 2H), 4.19 - 4.11 (m, 1H), 3.68 - 3.63 (m, 1H), 3.35 - 3.33 (m, 3H), 3.10 - 2.99 (m, 1H), 2.98 - 2.83 (m, 1H), 2.74 - 2.62 (m, 1H), 2.48 (s, 3H), 2.24 - 2.16 (m, 1H).

The **Examples** in **Table 5** were prepared using methods similar to those described in the synthesis of **Example 38** or **Example 39, using** the listed **Intermediates** in step a, and the appropriate warhead in step c.

**Table 5**

| Example Number | Structure | Name | Intermediates | $^1$H NMR (400 MHz) δ ppm | MI |
|---|---|---|---|---|---|
| **40** | 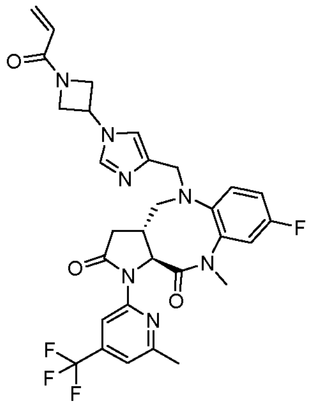 | (3a*R*,11a*S*)-5-((1-(1-acryloylazetidin-3-yl)-1*H*-imidazol-4-yl) methyl)-8-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4] diazocine-2,11(3*H*)-dione | **Intermediate 5c** and **Intermediate B1** | ($CDCl_3$) 8.30 (s, 1H), 7.89 (s, 1H), 7.58 - 7.49 (m, 1H), 7.16 - 6.98 (m, 4H), 6.43 - 6.34 (m, 1H), 6.25 - 6.14 (m, 1H), 5.75 (d, *J* = 10.4 Hz, 1H), 5.14 - 5.01 (m, 1H), 4.79 - 4.67 (m, 1H), 4.66 - 4.53 (m, 2H), 4.42 - 4.30 (m, 1H), 4.29 - 4.17 (m, 2H), 4.16 - 4.07 (m, 1H), 3.72 - 3.58 (m, 1H), 3.29 (s, 3H), 2.86 - 2.75 (m, 2H), 2.72 - 2.59 (m, 1H), 2.51 (s, 3H), 2.22 - 2.10 (m, 1H) | 612.3 |
| **41** | 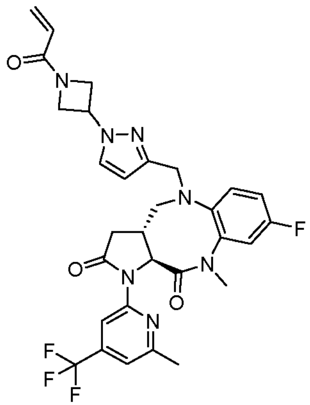 | (3a*R*,11a*S*)-5-((1-(1-acryloylazetidin-3-yl)-1*H*-pyrazol-3-yl) methyl)-8-fluoro-10-methyl-1-(6-methyl-4-(trifluoromethyl)pyridin-2-yl)-1,3a,4,5,10,11a-hexahydro-2*H*-benzo[b]pyrrolo[2,3-f][1,4] diazocine-2,11(3*H*)-dione | **Intermediate 5c** and **Intermediate B2** | ($CDCl_3$) 8.30 (s, 1H), 7.50 (dd, *J* = 6.0, 8.8 Hz, 1H), 7.39 (s, 1H), 7.17 - 7.05 (m, 3H), 6.42 - 6.34 (m, 1H), 6.27 - 6.17 (m, 2H), 5.73 (d, *J* = 10.4 Hz, 1H), 5.08 - 5.00 (m, 1H), 4.66 - 4.47 (m, 4H), 4.43 - 4.35 (m, 1H), 4.30 (d, *J* = 13.6 Hz, 1H), 4.12 (dd, *J* = 3.2, 13.6 Hz, 1H), 3.59 - 3.49 (m, 1H), 3.30 (d, *J* = 3.2 Hz, 3H), 2.84 - 2.74 (m, 2H), 2.64 (dd, *J* = 7.6, 16.8 Hz, 1H), 2.51 (s, 3H), 2.20 - 2.10 (m, 1H) | 612.1 |

(continued)

| Example Number | Structure | Name | Intermediates | $^1$H NMR (400 MHz) δ ppm | MI |
|---|---|---|---|---|---|
| **42** | 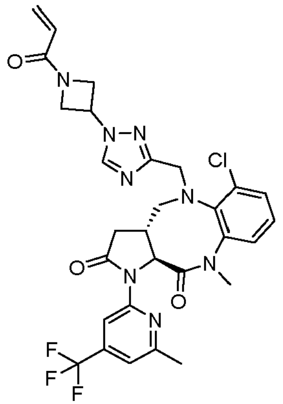 | (3a*R*,11a*S*)-5-((1-(1-acryloylazetidin-3-yl)-1H-1,2,4-triazol-3-yl)methyl)-6-chloro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 5b** and **Intermediate B3** | ($CDCl_3$) δ ppm 8.30 (s, 1H), 8.04 (s, 1H), 7.44 (d, *J* = 5.6 Hz, 1H), 7.31 (d, *J* = 6.4 Hz, 2H), 7.04 (s, 1H), 6.46 - 6.33 (m, 1H), 6.28 - 6.18 (m, 1H), 5.73 (t, *J* = 8.0 Hz, 1H), 5.21 - 5.06 (m, 1H), 4.78 - 4.63 (m, 2H), 4.61 (d, *J* = 9.2 Hz, 1H), 4.54 - 4.52 (m, 1H), 4.48 - 4.35 (m, 2H), 4.24 - 4.07 (m, 1H), 3.83 - 3.78 (m, 1H), 3.40 - 3.20 (m, 3H), 3.13 - 2.63 (m, 3H), 2.48 (s, 3H), 2.27 - 2.12 (m, 1H) | 629.1 |

**Example 43**

**(3a*R*,11a*S*)-5-(3-((1-((E)-4-(dimethylamino)but-2-enoyl)azetidin-3-yl)oxy)propyl)-6-fluoro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione**

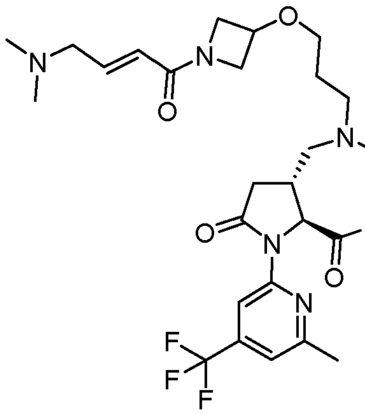

The title compound was prepared in a similar manner to **Example 38,** using **Intermediate 5a** and **Intermediate B4** in step a. The final step was conducted in a similar manner to the amide coupling described for **Example 27.**

m/z ES+ $[M+H]^+$ 647.4; $^1H$ NMR (400 MHz, DMSO-$d_6$) δ ppm 8.17 (s, 1H), 7.43 - 7.20 (m, 4H), 7.00 - 6.92 (m, 1H), 6.67 - 6.60 (m, 1H), 6.60 - 6.54 (m, 1H), 6.08 - 6.04 (m, 1H), 4.58 (d, *J* = 9.2 Hz, 1H), 4.37 - 4.30 (m, 1H), 4.26 - 4.20 (m, 1H), 4.10 - 3.90 (m, 3H), 3.63 - 3.58 (m, 1H), 3.50 - 3.45 (m, 1H), 3.42 - 3.40 (m, 1H), 3.24 (s, 1H), 3.19 - 3.14 (m, 1H), 3.13 - 3.04 (m, 1H), 3.04 - 2.90 (m, 2H), 2.12 (d, *J* = 2.4 Hz, 2H), 2.04 - 1.90 (m, 2H), 1.68 - 1.61 (m, 1H), 1.60 - 1.42 (m, 2H), 1.41 - 1.20 (m, 6H), 0.94 (t, *J* = 7.4 Hz, 1H), 0.88 - 0.83 (m, 1H).

**Example 44**

**(3a*R*,11a*S*)-6-chloro-5-(3-((1-((E)-4-(dimethylamino)but-2-enoyl)azetidin-3-yl)oxy)propyl)-10-methyl-1-(6-methyl-4-(trifluoromethyl)pyridin-2-yl)-1,3a,4,5,10,11a-hexahydro-2H-benzo[b]pyrrolo[2,3-f][1,4]diazocine-2,11(3H)-dione**

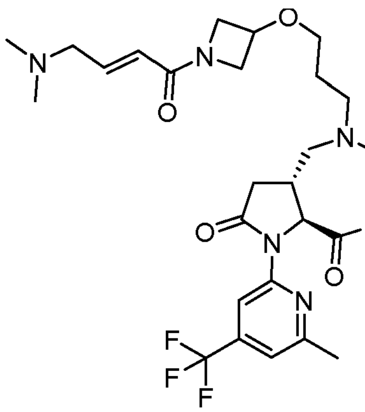

The title compound was prepared in a similar manner to **Example 43,** using **Intermediate 5b** and **Intermediate B4** in step a.

m/z ES+ $[M+H]^+$ 663.1; $^1H$ NMR (400 MHz, $CDCl_3$) δ ppm 8.33 (s, 1H), 7.49 - 7.42 (m, 1H), 7.34 - 7.29 (m, 2H), 7.05 (s, 1H), 6.90 - 6.76 (m, 1H), 6.41 - 6.22 (m, 1H), 4.63 (d, *J* = 9.2 Hz, 1H), 4.47 - 4.35 (m, 1H), 4.32 - 4.15 (m, 2H), 4.13 - 3.99 (m, 1H), 3.95 - 3.78 (m, 1H), 3.59 - 3.52 (m, 1H), 3.50 - 3.31 (m, 7H), 3.18 - 2.93 (m, 4H), 2.74 (dd, *J* = 7.6, 16.8 Hz, 1H), 2.62 - 2.50 (m, 6H), 2.48 (s, 3H), 2.30 - 2.17 (m, 1H), 1.75 - 1.53 (m, 2H).

**Example 45**

**(3aR,11aS)-5-(2-((S)-8-acryloyl-1-oxooctahydro-2H-pyrazino[1,2-a]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione**

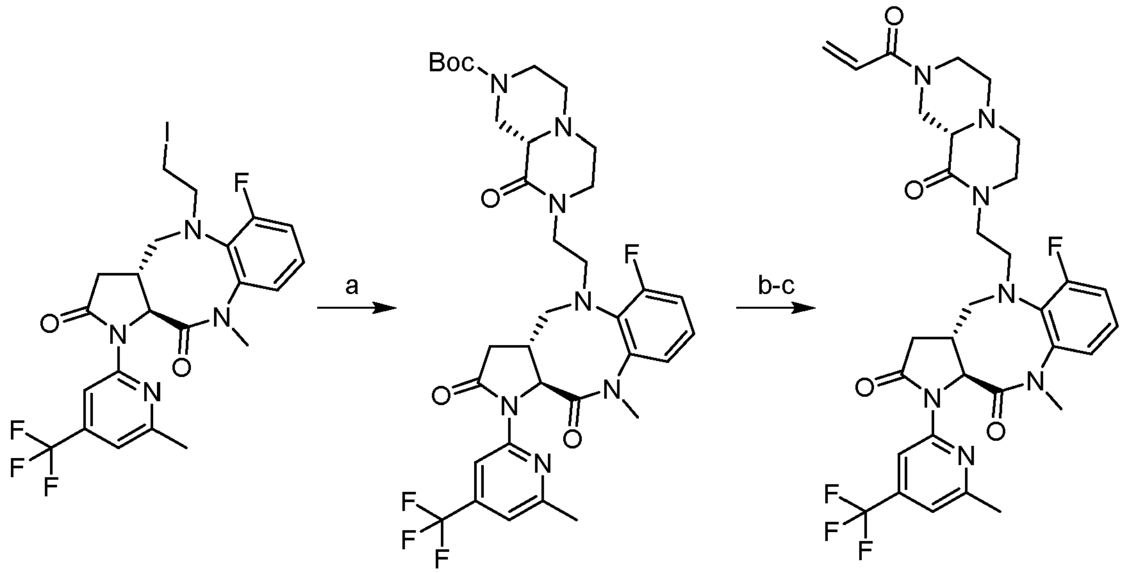


**Step a.** To a solution of **Intermediate C1** (177 mg, 0.69 mmol) in DMF (5 mL) was added NaH (21 mg, 0.52 mmol, 60% dispersion in mineral oil). The mixture was stirred at 0 °C for 30 min, then **Intermediate 8** (200 mg, 0.35 mmol) was added. The mixture was stirred at 20 °C for 30 min. Upon completion, the mixture was poured into sat. aq. $NH_4Cl$ (10 mL) at 0 °C and extracted with EtOAc (3 x 10 mL). The combined organic layers were dried over $Na_2SO_4$ and evaporated. The crude product was purified by reverse phase flash chromatography (water (0.1 FA)/MeCN) to give *tert-butyl* (S)-8-(2-((3aR,11aS)-6-fluoro-10-methyl-1-(6-methyl-4-(trifluoromethyl)pyridin-2-yl)-2,11-dioxo-1,2,3,3a,4,10,11,11a-octahydro-5H-benzo[b]pyrrolo[2,3-f][1,4]diazocin-5-yl)ethyl)-9-oxooctahydro-2H-pyrazino[1,2-a]pyrazine-2-carboxylate (80 mg, 33% yield) as a white solid.

$^1$H NMR (400 MHz, $CDCl_3$) δ ppm 8.33 (s, 1H), 7.40 - 7.31 (m, 1H), 7.23 (d, *J* = 8.0 Hz, 1H), 7.16 (t, *J* = 9.2 Hz, 1H), 7.07 (s, 1H), 4.63 (d, *J* = 9.2 Hz, 1H), 4.58 - 4.46 (m, 1H), 4.18 - 3.94 (m, 1H), 3.78 - 3.54 (m, 2H), 3.49 - 3.41 (m, 1H), 3.40 (s, 3H), 3.38 - 3.27 (m, 2H), 3.26 - 3.16 (m, 2H), 3.01 - 2.67 (m, 7H), 2.66 - 2.54 (m, 2H), 2.51 (s, 3H), 2.30 - 2.13 (m, 2H), 1.47 (s, 9H).

**Step b.** To a solution of *tert-butyl* (S)-8-(2-((3aR,11aS)-6-fluoro-10-methyl-1-(6-methyl-4-(trifluoromethyl)pyridin-2-yl)-2,11-dioxo-1,2,3,3a,4,10,11,11a-octahydro-5H-benzo[b]pyrrolo[2,3-t][1,4]diazocin-5-yl)ethyl)-9-oxooctahydro-2H-pyrazino[1,2-a]pyrazine-2-carboxylate (60 mg, 0.085 mmol) in DCM (1 mL) was added TFA (770 mg, 6.75 mmol). The mixture was stirred at 15 °C for 30 min. Upon completion, the reaction mixture was evaporated to give (3aR,11aS)-6-fluoro-10-methyl-1-(6-methyl-4-(trifluoromethyl)pyridin-2-yl)-5-(2-((S)-1-oxooctahydro-2H-pyrazino[1,2-a]pyrazin-2-yl) ethyl)-1,3a,4,5,10,11a-hexahydro-2H-benzo[b]pyrrolo[2,3-f][1,4]diazocine-2,11(3H)-dione as a TFA salt (60 mg, crude) as white solid.

m/z ES+ $[M+H]^+$ 604.2.

**Step c.** To a solution of (3aR,11aS)-6-fluoro-10-methyl-1-(6-methyl-4-(trifluoromethyl)pyridin-2-yl)-5-(2-((S)-1-oxooctahydro-2H-pyrazino[1,2-a]pyrazin-2-yl)ethyl)-1,3a,4,5,10,11a-hexahydro-2H-benzo[b]pyrrolo[2,3-f][1,4]diazocine-2,11(3H)-dione (60 mg, 0.084 mmol) and TEA (25 mg, 0.25 mmol) in DCM (2 mL) was added acryloyl chloride (8 mg, 0.084 mmol). The mixture was stirred at 20 °C for 30 min. Upon completion, the reaction mixture was evaporated. The resulting residue was purified by Prep-HPLC to give the title compound (39 mg, 70% yield) as white solid.

m/z ES+ $[M+H]^+$ 658.3; $^1$H NMR (400 MHz, $CDCl_3$) δ ppm 8.32 (s, 1H), 7.40 - 7.32 (m, 1H), 7.23 (d, *J* = 8.0 Hz, 1H), 7.19 - 7.12 (m, 1H), 7.06 (s, 1H), 6.73 - 6.54 (m, 1H), 6.34 (dd, *J* = 1.2, 16.8 Hz, 1H), 5.80 - 5.77 (m, 1H), 4.63 - 4.45 (m, 2H), 4.08 - 3.89 (m, 1H), 3.78 - 3.57 (m, 3H), 3.45 - 3.00 (m, 13H), 2.92 - 2.69 (m, 4H), 2.50 (s, 3H), 2.25 - 2.17 (m, 1H).

The **Examples** in **Table 6** were prepared using methods similar to those described in the synthesis of **Example 45,** using the listed **Intermediates** in step a.

For **Example 49** and **Example 50,** step a using *tert*-butyl 1,4,6,7-tetrahydro-5H-imidazo[4,5-c]pyridine-5-carboxylate (CAS: 1202800-68-7) formed a mixture of regioisomers which were separated by chiral SFC and used in the subsequent steps.

**Table 6**

| Example Number | Structure | Name | Intermediates | $^1$H NMR (400 MHz) δ ppm | MI |
|---|---|---|---|---|---|
| **46** | 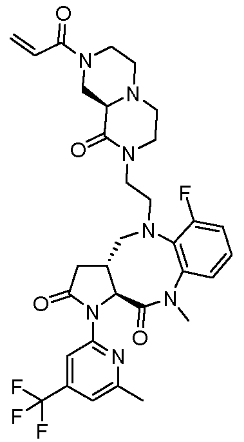 | (3a*R*,11a*S*)-5-(2-((*R*)-8-acryloyl-1-oxooctahydro-2*H*-pyrazino[1,2-*a*]pyrazin-2-yl) ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4] diazocine-2,11(3H)-dione | **Intermediate 8** and **Intermediate C2** | ($CDCl_3$) 8.32 (s, 1H), 7.40 - 7.32 (m, 1H), 7.23 (d, *J* = 8.0 Hz, 1H), 7.16 (t, *J* = 9.2 Hz, 1H), 7.06 (s, 1H), 6.63 (s, 1H), 6.32 (d, *J* = 16.8 Hz, 1H), 5.78 (d, *J* = 11.2 Hz, 1H), 4.99 - 3.76 (m, 5H), 3.73 - 3.05 (m, 14H), 2.96 - 2.66 (m, 4H), 2.49 (s, 3H), 2.27 - 2.15 (m, 1H) | 658.3 |
| **47** | 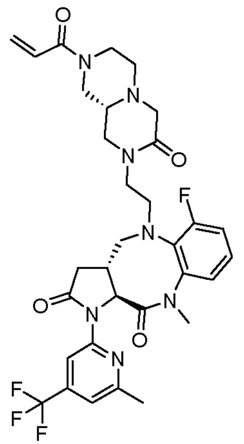 | (3a*R*,11a*S*)-5-(2-((*S*)-8-acryloyl-3-oxooctahydro-2H-pyrazino[1,2-a]pyrazin-2-yl) ethyl)-6-fluoro-10-methyl-1-(6-methyl-4-(trifluoromethyl)pyridin-2-yl)-1,3a,4,5,10,11a-hexahydro-2H-benzo[*b*]pyrrolo[2,3-*f*][1,4] diazocine-2,11(3*H*)-dione | **Intermediate 8** and **Intermediate C3** | ($CDCl_3$) 8.31 (s, 1H), 7.38 - 7.30 (m, 1H), 7.22 (d, *J* = 8.0 Hz, 1H), 7.15 7.13 (m, 1H), 7.05 (s, 1H), 6.58 - 6.52 (m, 1H), 6.34 - 6.39 (m, 1H), 5.75 - 5.73 (m, 1H), 4.59 (d, *J* = 9.2 Hz, 2H), 4.06 - 3.83 (m, 1H), 3.68 - 3.50 (m, 3H), 3.41 (s, 4H), 3.36 - 3.26 (m, 2H), 3.23 - 3.07 (m, 3H), 3.03 - 2.82 (m, 5H), 2.78 - 2.74 (m, 1H), 2.50 - 2.49 (m, 4H), 2.25 - 2.09 (m, 2H) | 658.3 |

(continued)

| Example Number | Structure | Name | Intermediates | $^1$H NMR (400 MHz) δ ppm | MI |
|---|---|---|---|---|---|
| **48** | 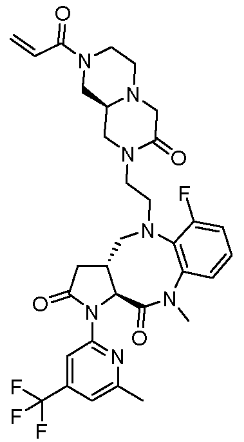 | (3a*R*,11a*S*)-5-(2-((R)-8-acryloyl-3-oxooctahydro-2*H*-pyrazino[1,2-a]pyrazin-2-yl) ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4] diazocine-2,11(3*H*)-dione | **Intermediate 8** and **Intermediate C4** | ($CDCl_3$) 8.34 (s, 1H), 7.39 - 7.32 (m, 1H), 7.24 (d, *J* = 7.6 Hz, 1H), 7.16 (t, *J* = 9.2 Hz, 1H), 7.07 (s, 1H), 6.63 - 6.49 (m, 1H), 6.33 (d, *J* = 16.8 Hz, 1H), 5.75 (d, *J* = 10.8 Hz, 1H), 4.66 - 4.58 (m, 2H), 4.00 - 3.86 (m, 1H), 3.70 - 3.51 (m, 2H), 3.50 - 3.45 (m, 1H), 3.43 (s, 3H), 3.41 - 3.33 (m, 2H), 3.32 - 3.11 (m, 4H), 2.95 - 2.79 (m, 5H), 2.77 - 2.61 (m, 1H), 2.51 (s, 3H), 2.46 (m, 1H), 2.30 - 2.11 (m, 2H) | 658.1 |
| **49** | 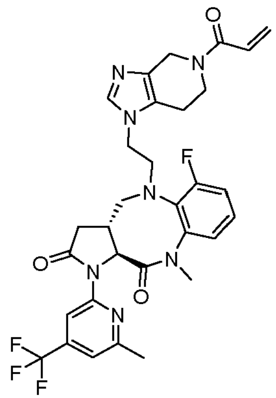 | (3a*R*,11a*S*)-5-(2-(5-acryloyl-4,5,6,7-tetrahydro-1*H*-imidazo[4,5-*c*]pyridin-1-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 8** and *tert-butyl* 1,4,6,7-tetrahydro-5*H*-imidazo[4,5-*c*]pyridine-5-carboxylate (CAS: 1202800-68-7) | ($CDCl_3$) 9.29 (s, 1H), 8.28 (s, 1H), 7.37 - 7.28 (m, 1H), 7.25 - 7.12 (m, 2H), 7.06 (s, 1H), 6.63 - 6.57 (m, 1H), 6.34 - 6.29 (m, 1H), 5.80 - 5.76 (m, 1H), 4.91 - 4.85 (m, 1H), 4.72 - 4.51 (m, 3H), 4.30 - 4.15 (m, 2H), 3.80 - 3.75 (m, 1H), 3.72 - 3.65 (m, 2H), 3.30 - 3.13 (m, 2H), 3.22 (s, 3H), 3.00 - 2.87 (m, 2H), 2.70 - 2.60 (m, 2H), 2.47 (s, 3H), 2.26 - 2.18 (m, 1H) | 626.1 |

(continued)

| Example Number | Structure | Name | Intermediates | $^1$H NMR (400 MHz) δ ppm | MI |
|---|---|---|---|---|---|
| 50 | 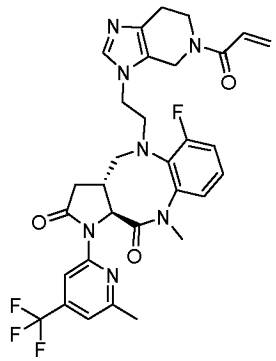 | (3a*R*,11a*S*)-5-(2-(5-acryloyl-4,5,6,7-tetrahydro-3*H*-imidazo[4,5-*c*]pyridin-3-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 8** and *tert*-butyl 1,4,6,7-tetrahydro-5*H*-imidazo[4,5-*c*]pyridine-5-carboxylate (CAS: 1202800-68-7) | ($CDCl_3$) 8.32 (s, 1H), 7.41 - 7.33 (m, 2H), 7.25 - 7.13 (m, 2H), 7.05 (s, 1H), 6.69 - 6.58 (m, 1H), 6.31 (dd, *J* =16.8, 1.6 Hz, 1H), 5.77 - 5.70 (m, 1H), 4.75 - 4.70 (m, 1H), 4.59 (d, *J* =9.4 Hz, 1H), 4.51 (d, *J* =15.6 Hz, 1H), 3.90 - 3.71 (m, 4H), 3.54 - 3.39 (m, 2H), 3.37 - 3.30 (m, 1H), 3.26 (s, 3H), 3.04 - 2.93 (m, 1H), 2.90 - 2.81 (m, 1H), 2.79 - 2.68 (m, 3H), 2.48 (s, 3H),2.20 (dd, *J* =16.4, 11.2 Hz, 1H) | 626.1 |

**Example 51**

**(3a*R*,11a*S*)-5-(2-(4-(1-acryloylazetidin-3-yl)piperazin-1-yl)-2-oxoethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

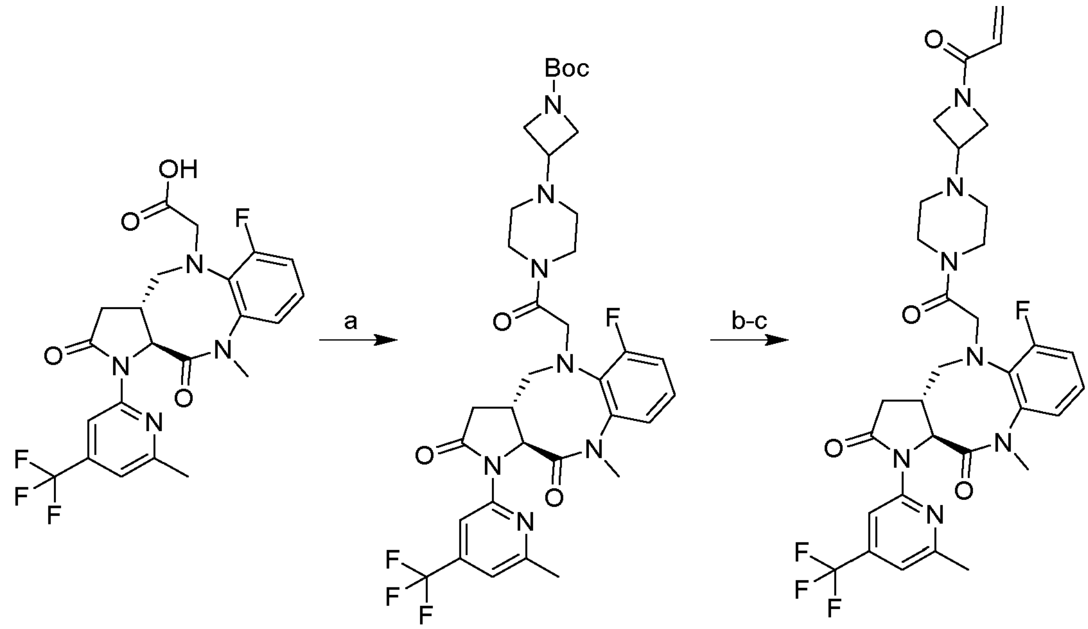


**Step a.** To a solution of **Intermediate 9a** (90 mg, 0.19 mmol) in DCM (2 mL) was added DIPEA (73 mg, 0.56 mmol), HATU (86 mg, 0.23 mmol) and **Intermediate A1** (90 mg, 0.38 mmol). The mixture was stirred at 20 °C for 1 h. Upon completion, the reaction mixture was diluted with water (5 mL) and extracted with EtOAc (3 x 2.5 mL). The combined organic layers were washed with brine (5 mL), dried over $Na_2SO_4$ and evaporated. The crude product was purified by Prep-TLC (EtOAc/MeOH = 10/1) to give *tert*-butyl 3-(4-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)acetyl)piperazin-1-yl) azetidine-1-carboxylate (100 mg, 75% yield) as white solid.

m/z ES+ $[M+H]^+$ 704.2; $^1H$ NMR (400 MHz, $CDCl_3$) $\delta$ ppm 8.33 (s, 1H), 7.38 - 7.35 (m, 1H), 7.24 - 7.22 (m, 1H), 7.17 - 7.15 (m, 1H), 7.06 (s, 1H), 4.64 (d, *J* = 9.2 Hz, 1H), 4.03 - 3.90 (m, 3H), 3.84 - 3.73 (m, 3H), 3.71 - 3.43 (m, 5H), 3.39 (s, 3H), 3.14 - 3.04 (m, 1H), 3.01 - 2.86 (m, 2H), 2.75 - 2.70 (m, 1H), 2.49 (s, 3H), 2.42 - 2.15 (m, 5H), 1.43 (s, 9H).

**Step b.** To a solution of *tert*-butyl 3-(4-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)acetyl)piperazin-1-yl) azetidine-1-carboxylate (100 mg, 0.14 mmol) in DCM (1 mL) was added TFA (1.54 g, 13.5 mmol). The mixture was stirred at 20 °C for 30 min. Upon completion, the reaction mixture was evaporated to give (3a*R*,11a*S*)-5-(2-(4-(azetidin-3-yl) piperazin-1-yl)-2-oxoethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione as a TFA salt (100 mg, crude) as yellow oil.

m/z ES+ $[M+H]^+$ 604.3.

**Step c.** To a solution of (3a*R*,11a*S*)-5-(2-(4-(azetidin-3-yl)piperazin-1-yl)-2-oxoethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (100 mg, 0.14 mmol) in DCM (2 mL) was added TEA (42 mg, 0.42 mmol) and acryloyl chloride (13 mg, 0.14 mmol). The mixture was stirred at 20 °C for 30 min. Upon completion, the reaction mixture was evaporated. The crude product was purified by Prep-HPLC to give the title compound (41 mg, 43% yield) as a white solid.

m/z ES+ $[M+H]^+$ 658.3; $^1H$ NMR (400 MHz, $CDCl_3$) $\delta$ ppm 8.32 (s, 1H), 7.39 - 7.36 (m, 1H), 7.25 -7.23 (m, 1H), 7.17 (t, *J* = 9.2 Hz, 1H), 7.06 (s, 1H), 6.38 - 6.28 (m, 1H), 6.22 - 6.12 (m, 1H), 5.70 - 5.67 (m, 1H), 4.63 (d, *J* = 8.8 Hz, 1H), 4.30 - 4.22 (m, 1H), 4.19 - 4.08 (m, 2H), 4.06 - 3.93 (m, 2H), 3.81 - 3.77 (m, 2H), 3.66 - 3.46 (m, 4H), 3.39 (s, 3H), 3.32 - 3.22 (m, 1H), 2.99 - 2.87 (m, 2H), 2.78 - 2.74 (m, 1H), 2.59 - 2.29 (m, 7H), 2.24 - 2.19 (m, 1H).

The **Example** in **Table 7** was prepared using methods similar to those described in the synthesis of **Example 51,** using the listed **Intermediates** in step a.

**Table 7**

| Example Number | Structure | Name | Intermediates | $^1H$ NMR (400 MHz) δ ppm | MI |
|---|---|---|---|---|---|
| **52** | 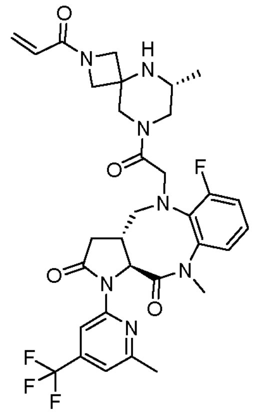 | (3a*R*,11a*S*)-5-(2-((*R*)-2-acryloyl-6-methyl-2,5,8-triazaspiro[3.5]nonan-8-yl)-2-oxoethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 9a** and **Intermediate A9** | ($CDCl_3$) 8.32 (s, 1H), 7.41 - 7.33 (m, 1H), 7.23 (d, *J* =7.8 Hz, 1H), 7.17 (s, 1H), 7.06 (s, 1H), 6.38 - 6.26 (m, 1H), 6.26 - 6.01 (m, 1H), 5.71 (t, *J* =10.4 Hz, 1H), 4.69 - 4.62 (m, 1H), 4.61 - 4.37 (m, 1H), 4.13 - 4.09 (m, 2H), 4.00 - 3.87 (m, 4H), 3.85 - 3.75 (m, 1H), 3.65 - 3.57 (m, 1H), 3.46 - 3.38 (m, 3H), 3.13 - 3.10 (m, 1H), 3.00 - 2.97 (m, 2H), 2.88 - 2.86 (m, 1H), 2.78 - 2.73 (m, 2H), 2.49 (s, 3H), 2.46 - 2.27 (m, 1H), 1.32 - 1.16 (m, 3H) | 658.2 |

**Example 53**

**(3a*R*,11a*S*)-5-(2-(4-acryloylpiperazin-1-yl)pyridin-4-yl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

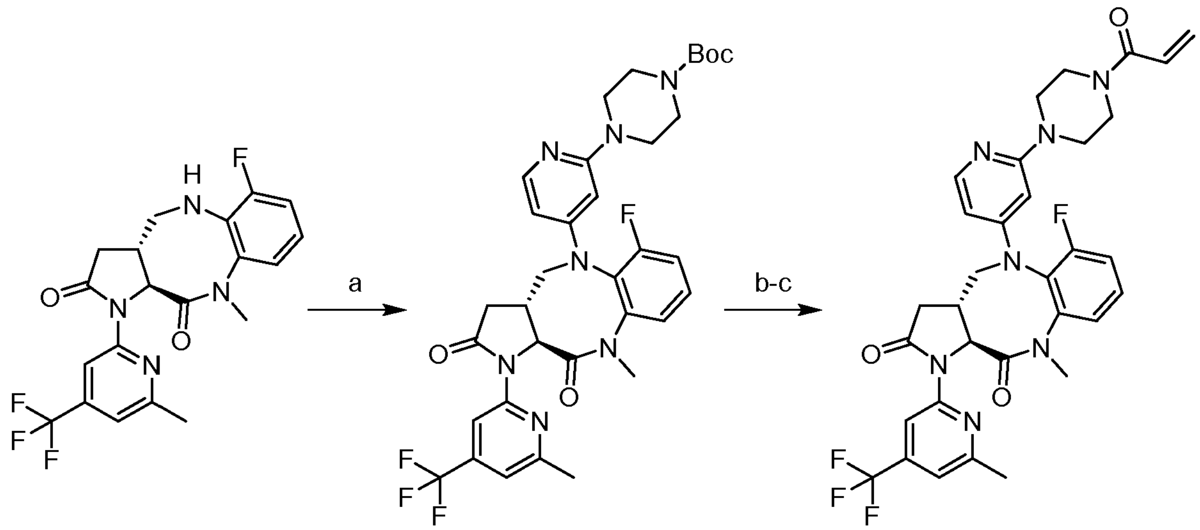


**Step a.** A mixture of **Intermediate 5a** (200 mg, 0.47 mmol), *tert*-butyl 4-(4-bromopyridin-2-yl)piperazine-1-carboxylate (CAS: 1197294-80-6; 243 mg, 0.71 mmol), RuPhos (44 mg, 0.095 mmol), $Cs_2CO_3$ (462 mg, 1.42 mmol) and RuPhos-Pd-G2 (37 mg, 0.047 mmol) in 1,4-dioxane (10 mL) was stirred at 100 °C for 20 h under an $N_2$ atmosphere. Upon completion, the reaction mixture was diluted with water (50 mL) and extracted with EtOAc (3 x 20 mL). The combined organic layers were washed with brine (50 mL), dried over $Na_2SO_4$ and evaporated. The crude product was purified by reverse phase flash chromatography (water (0.1 FA)/MeCN) to give *tert*-butyl 4-(4-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)pyridin-2-yl)piperazine-1-carboxylate (80 mg, 24% yield) as a yellow solid.

m/z ES+ $[M+H]^+$ 684.4; $^1H$ NMR (400 MHz, $CDCl_3$) δ ppm 8.32 (s, 1H), 7.89 (d, *J* = 6.0 Hz, 1H), 7.64 - 7.54 (m, 1H), 7.39 - 7.31 (m, 2H), 7.08 (s, 1H), 5.79 - 5.71 (m, 1H), 5.58 (s, 1H), 4.82 (d, *J* = 9.2 Hz, 1H), 4.52 - 4.40 (m, 1H), 3.55 - 3.51 (m, 4H), 3.48 - 3.39 (m, 4H), 3.24 (s, 1H), 3.19 (s, 3H), 3.15 - 3.07 (m, 1H), 2.96 - 2.86 (m, 1H), 2.50 (s, 3H), 2.40 - 2.29 (m, 1H), 1.48 (s, 9H).

**Step b.** To a mixture of *tert*-butyl 4-(4-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)pyridin-2-yl)piperazine-1-carboxylate (99 mg, 0.14 mmol) in DCM (5 mL) was added TFA (3.81 g, 33.4 mmol). The reaction mixture was stirred at 25 °C for 1 h. Upon completion, the reaction mixture was evaporated to give (3a*R*,11a*S*)-6-fluoro-10-methyl-1-(6-methyl-4-(trifluoromethyl)pyridin-2-yl)-5-(2-(piperazin-1-yl)pyridin-4-yl)-1,3a,4,5,10,11a-hexahydro-2*H*-benzo[*b*]pyrrolo[2,3-*f*][1,4]diazocine-2,11(3*H*)-dione as a TFA salt (100 mg, 99% yield) as a yellow oil.

m/z ES+ $[M+H]^+$ 584.2.

**Step c.** To a mixture of (3a*R*,11a*S*)-6-fluoro-10-methyl-1-(6-methyl-4-(trifluoromethyl)pyridin-2-yl)-5-(2-(piperazin-1-yl)pyridin-4-yl)-1,3a,4,5,10,11a-hexahydro-2*H*-benzo[*b*]pyrrolo[2,3-*f*][1,4]diazocine-2,11(3*H*)-dione (100 mg, 0.14 mmol) and DIPEA (37 mg, 0.29 mmol) in DCM (4 mL) was added acryloyl chloride (14 mg, 0.16 mmol). The reaction mixture was stirred at 25 °C for 30 min. Upon completion, the reaction mixture was quenched with water (50 mL) and extracted with DCM (3 x 20 mL). The combined organic layers were washed with brine (50 mL), dried over $Na_2SO_4$ and evaporated. The crude product was purified by Prep-HPLC to give the title compound (60 mg, 63% yield) as white solid.

m/z ES+ $[M+H]^+$ 638.3; $^1H$ NMR (400 MHz, $CDCl_3$) δ ppm 8.32 (s, 1H), 7.88 (d, *J* = 6.0 Hz, 1H), 7.69 - 7.59 (m, 1H), 7.45 - 7.33 (m, 2H), 7.09 (s, 1H), 6.63 - 6.53 (m, 1H), 6.39 - 6.30 (m, 1H), 5.94 - 5.50 (m, 3H), 4.82 (d, *J* = 9.6 Hz, 1H), 4.56 - 4.46 (m, 1H), 3.90 - 3.68 (m, 6H), 3.54 - 3.36 (m, 2H), 3.34 - 3.25 (m, 1H), 3.20 (s, 3H), 3.13 - 3.03 (m, 1H), 2.98 - 2.89 (m, 1H), 2.51 (s, 3H), 2.42 - 2.31 (m, 1H).

The **Examples** in **Table 8** were prepared using methods similar to those described in the synthesis of **Example 53,** using the listed **Intermediates** in step a.

**Table 8**

| Example Number | Structure | Name | Intermediates | $^1$H NMR (400 MHz) δ ppm | MI |
|---|---|---|---|---|---|
| **54** | 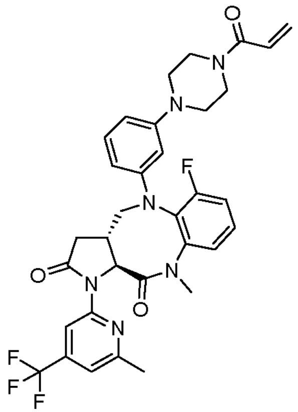 | (3a*R*,11a*S*)-5-(3-(4-acryloylpiperazin-1-yl)phenyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and *tert*-butyl 4-(3-bromophenyl)pipe razine-1-carboxylate (CAS: 327030-39-7) | ($CDCl_3$) 8.32 (s, 1H), 7.58 - 7.50 (m, 1H), 7.38 - 7.28 (m, 2H), 7.13 - 7.04 (m, 2H), 6.64 - 6.53 (m, 1H), 6.45 - 6.29 (m, 2H), 6.22 - 5.83 (m, 2H), 5.78 - 5.71 (m, 1H), 4.84 (d, *J* = 9.2 Hz, 1H), 4.52 - 4.41 (m, 1H), 4.00 - 3.55 (m, 4H), 3.30 - 3.13 (m, 4H), 3.12 (s, 3H), 3.11 - 3.00 (m, 2H), 2.94 - 2.84 (m, 1H), 2.50 (s, 3H), 2.38 - 2.28 (m, 1H) | 637.3 |
| **55** | 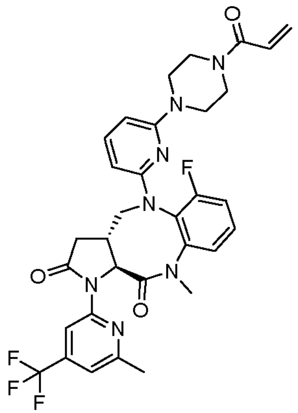 | (3a*R*,11a*S*)-5-(6-(4-acryloylpiperazin-1-yl)pyridin-2-yl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and *tert*-butyl 4-(6-bromopyridin-2-yl)piperazine-1-carboxylate (CAS: 331767-56-7) | ($CDCl_3$) 8.35 (s, 1H), 7.57 - 7.48 (m, 1H), 7.38 - 7.28 (m, 2H), 7.22 (t, *J* = 8.0 Hz, 1H), 7.06 (s, 1H), 6.63 (dd, *J* = 10.4, 16.8 Hz, 1H), 6.35 (dd, *J = 1.6,* 16.8 Hz, 1H), 6.01 (d, *J* = 8.0 Hz, 1H), 5.76 (dd, *J* = 1.6, 10.4 Hz, 1H), 5.45 - 5.33 (m, 1H), 5.16 - 5.03 (m, 1H), 4.83 (d, *J* = 9.2 Hz, 1H), 3.85 - 3.45 (m, 8H), 3.29 - 3.18 (m, 1H), 3.16 (s, 3H), 3.02 - 2.92 (m, 1H), 2.87 - 2.82 (m, 1H), 2.50 (s, 3H), 2.39 - 2.27 (m, 1H) | 638.3 |

(continued)

| Example Number | Structure | Name | Intermediates | $^1$H NMR (400 MHz) δ ppm | MI |
|---|---|---|---|---|---|
| **56** | 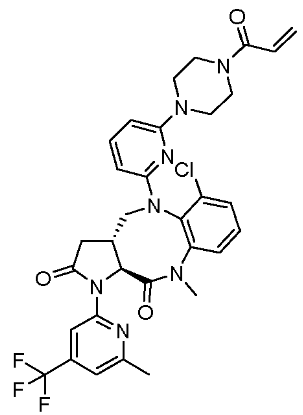 | (3a*R*,11a*S*)-5-(6-(4-acryloylpiperazin-1-yl) pyridin-2-yl)-6-chloro-10-methyl-1-(6-methyl-4-(trifluoromethyl)pyridin-2-yl)-1,3a,4,5,10,11a-hexahydro-2*H*-benzo [*b*]pyrrolo[2,3-*f*][1,4]diazocine-2,11(3*H*)-dione | **Intermediate 5b** and *tert*-butyl 4-(6-bromopyridin-2-yl)piperazine-1-carboxylate (CAS: 331767-56-7) | ($CDCl_3$) 8.36 (s, 1H), 7.62 (dd, *J* = 1.2, 7.6 Hz, 1H), 7.55 - 7.44 (m, 2H), 7.23 (t, *J* = 8.0 Hz, 1H), 7.07 (s, 1H), 6.64 (dd, *J* = 10.4, 16.8 Hz, 1H), 6.43 - 6.30 (m, 1H), 6.03 (d, *J* = 8.4 Hz, 1H), 5.77 (dd, *J* = 1.6, 10.4 Hz, 1H), 5.46 - 5.21 (m, 1H), 5.17 - 4.93 (m, 1H), 4.81 (d, *J* = 9.6 Hz, 1H), 3.91 - 3.68 (m, 4H), 3.66 - 3.46 (m, 4H), 3.32 - 3.21 (m, 1H), 3.15 (s, 3H), 2.99 (t, *J* = 12.6 Hz, 1H), 2.87 (dd, *J* = 8.8, 16.8 Hz, 1H), 2.51 (s, 3H), 2.36 (dd, *J* = 12.0, 16.8 Hz, 1H) | 654.1 |
| **57** | 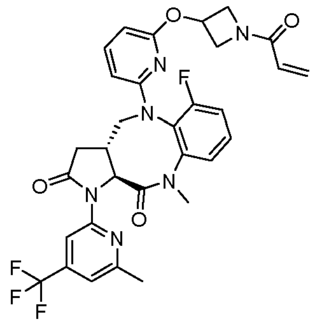 | (3a*R*,11a*S*)-5-(6-((1-acryloylazetidin-3-yl) oxy)pyridin-2-yl)-6-fluoro-10-methyl-1-(6-methyl-4-(trifluoromethyl)pyridin-2-yl)-1,3a,4,5,10,11a-hexahydro-2*H*-benzo [*b*]pyrrolo[2,3-*f*][1,4]diazocine-2,11(3*H*)-dione | **Intermediate 5a** and **Intermediate D1** | ($CDCl_3$) 8.36 (s, 1H), 7.56 (m, 1H), 7.40 - 7.28 (m, 3H), 7.07 (s, 1H), 6.41 - 6.34 (m, 1H), 6.27 - 6.18 (m, 1H), 6.17 (d, *J* = 8.0 Hz, 1H), 5.80 - 5.57 (m, 2H), 5.34 - 5.14 (m, 1H), 5.02 - 4.87 (m, 1H), 4.84 (d, *J* = 9.2 Hz, 1H), 4.55 - 4.08 (m, 4H), 3.16 (s, 3H), 3.15 - 3.09 (m, 1H), 3.08 - 2.98 (m, 1H), 2.88 (m, 1H), 2.50 (s, 3H), 2.36 (dd, *J* = 11.2, 16.8 Hz, 1H) | 625.5 |

**Example 58**

**(3a*R*,11a*S*)-5-(2-((7*R*/*S*,9a*R*/*S*)-8-acryloyl-7-methyl-6-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

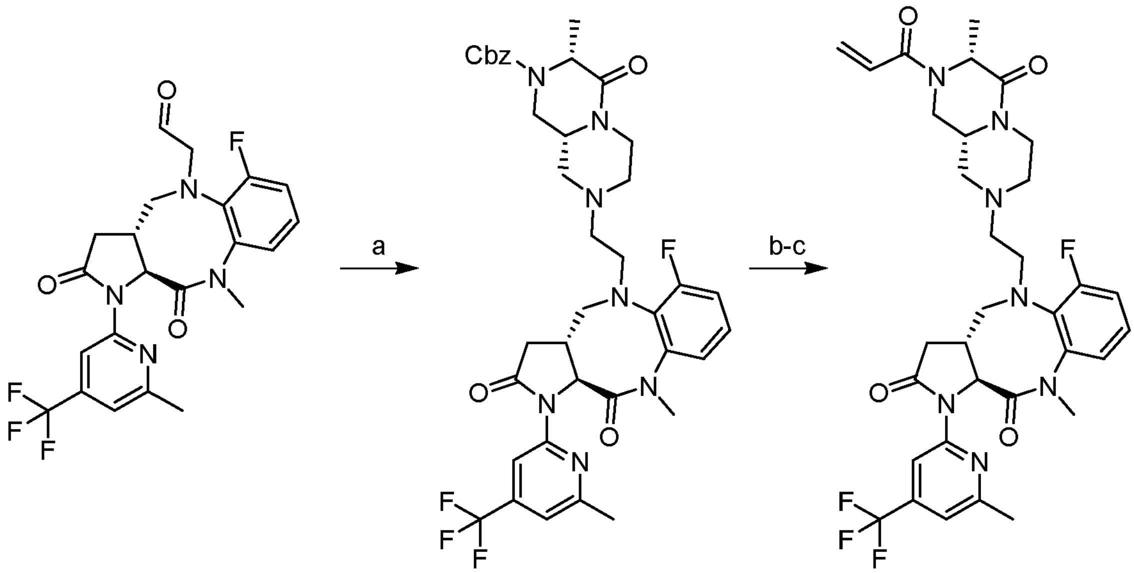


**Step a.** This step was conducted in a similar manner to **Example 1,** step a, using **Intermediate 7a** and **Intermediate A19a.**

**Step b.** A mixture of benzyl (3*R*/*S*,9a*R*/*S*)-8-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)ethyl)-3-methyl-4-oxooctahydro-2*H*-pyrazino[1,2-*a*]pyrazine-2-carboxylate (50 mg, 0.07 mmol) and 10% Pd/C (5 mg) in MeOH (1.5 mL) was stirred at 30 °C for 1 h under a $H_2$ atmosphere (15 psi). Upon completion, the reaction mixture was filtered and evaporated to give (3a*R*,11a*S*)-6-fluoro-10-methyl-1-(6-methyl-4-(trifluoromethyl)pyridin-2-yl)-5-(2-((7*R*/*S*,9a*S*/*R*)-7-methyl-6-oxooctahydro-2*H*-pyrazino[1,2-a]pyrazin-2-yl)ethyl)-1,3a,4,5,10,11a-hexahydro-2*H*-benzo[*b*]pyrrolo[2,3-*f*][1,4]diazocine-2,11(3*H*)-dione (40 mg, crude) as a yellow solid.

m/z ES+ $[M+H]^+$618.3.

**Step c.** This step was conducted in a similar manner to **Example 1,** step c.

m/z ES+ $[M+H]^+$ 672.3; $^1H$ NMR (400 MHz, $CDCl_3$) δ ppm 8.32 (s, 1H), 7.40 - 7.31 (m, 1H), 7.24 (d, *J* = 7.6 Hz, 1H), 7.15 (t, *J* = 9.2 Hz, 1H), 7.06 (s, 1H), 6.66 - 6.32 (m, 2H), 5.79 (d, *J* = 10.4 Hz, 1H), 5.21 - 4.68 (m, 1H), 4.67 - 4.39 (m, 3H), 3.57 (d, *J* = 9.2 Hz, 1H), 3.53 - 3.43 (m, 1H), 3.37 (s, 3H), 3.32 - 3.11 (m, 2H), 3.00 - 2.81 (m, 4H), 2.81 - 2.63 (m, 3H), 2.50 (s, 3H), 2.48 - 2.38 (m, 2H), 2.26 - 2.16 (m, 1H), 2.12 - 1.95 (m, 1H), 1.80 - 1.72 (m, 1H), 1.52 (d, *J* = 6.4 Hz, 3H).

**Example 59**

**(3a*R*,11a*S*)-5-(2-((7*S*/*R*,9a*S*/*R*)-8-acryloyl-7-methyl-6-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

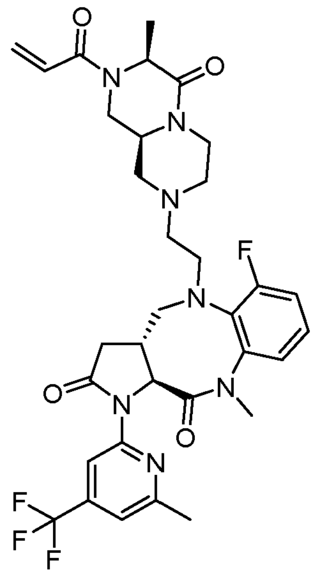

The title compound was prepared in a similar manner to **Example 58,** using **Intermediate A19b** in step a.

m/z ES+ $[M+H]^+$ 672.3; $^1H$ NMR (400 MHz, $CDCl_3$) δ ppm 8.32 (s, 1H), 7.38 - 7.31 (m, 1H), 7.23 (d, *J* = 7.6 Hz, 1H), 7.15 (t, *J* = 9.2 Hz, 1H), 7.06 (s, 1H), 6.54 - 6.35 (m, 2H), 5.79 (d, *J* = 11.2 Hz, 1H), 4.80 - 4.65 (m, 1H), 4.64 - 4.42 (m, 3H), 3.58 - 3.53 (m, 1H), 3.52 - 3.50 (m, 1H), 3.36 (s, 3H), 3.28 - 3.21 (m, 2H), 2.97 - 2.82 (m, 4H), 2.80 - 2.68 (m, 3H), 2.50 (s, 3H), 2.48 - 2.38 (m, 2H), 2.25 - 2.17 (m, 1H), 2.08 - 1.98 (m, 1H), 1.87 - 1.72 (m, 1H), 1.57 - 1.47 (m, 3H).

**Example 60**

**(3a*R*,11a*S*)-5-(2-((7*R*/*S*,9a*S*/*R*)-8-acryloyl-7-methyl-6-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo [2,3-*f*][1,4]diazocine-2,11(3*H*)-dione**

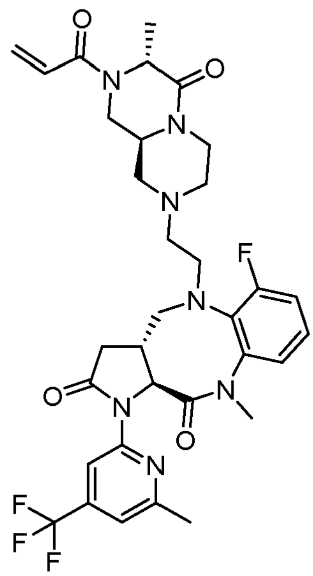

The title compound was prepared in a similar manner to **Example 58,** using **Intermediate A19c** in step a.

m/z ES+ $[M+H]^+$ 672.1; $^1H$ NMR (400 MHz, $CDCl_3$) δ ppm 8.33 (s, 1H), 7.39 - 7.30 (m, 1H), 7.23 (d, *J* = 8.0 Hz, 1H), 7.14 (t, *J* = 9.2 Hz, 1H), 7.06 (s, 1H), 6.55 - 6.45 (m, 1H), 6.45 - 6.33 (m, 1H), 5.79 (d, *J* = 10.0 Hz, 1H), 5.41 - 4.61 (m, 1H), 4.61 - 4.34 (m, 3H), 4.04 - 3.58 (m, 1H), 3.58 - 3.44 (m, 2H), 3.37 (s, 3H), 3.32 - 3.24 (m, 1H), 3.20 - 3.08 (m, 1H), 3.00 - 2.86 (m, 2H), 2.85 - 2.79 (m, 1H), 2.77 - 2.63 (m, 3H), 2.50 (s, 3H), 2.40 - 2.30 (m, 2H), 2.20 (dd, *J* = 16.8, 11.6 Hz, 1H), 2.10 - 1.93

(m, 2H), 1.51 (d, *J* = 6.8 Hz, 3H).

**Example 61**

**(3a*R*,11a*S*)-5-(2-((7*S*/*R*,9a*R*/*S*)-8-acryloyl-7-methyl-6-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

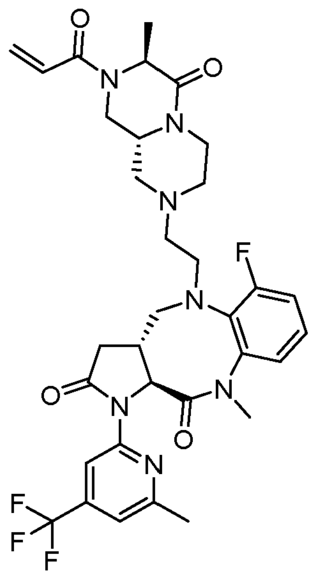

The title compound was prepared in a similar manner to **Example 58,** using **Intermediate A19d** in step a.

m/z ES+ $[M+H]^+$ 672.3; $^1H$ NMR (400 MHz, $CDCl_3$) δ ppm 8.32 (s, 1H), 7.37 - 7.32 (m, 1H), 7.23 (d, *J* = 8.0 Hz, 1H), 7.11 (t, *J* = 9.2 Hz, 1H), 7.06 (s, 1H), 6.53 - 6.37 (m, 2H), 5.80 - 5.78 (m, 2H), 4.61 - 4.50 (m, 3H), 3.55 - 3.48 (m, 2H), 3.35 (s, 3H), 3.25 - 3.17 (m, 2H), 2.90 - 2.68 (m, 6H), 2.50 (s, 3H), 2.40 - 2.30 (m, 2H), 2.24 - 2.17 (m, 1H), 2.10 - 1.93 (m, 2H), 1.51 - 1.28 (m, 4H).

**Example 62a: (3a*R*,11a*S*)-5-(2-((*R*/*S*)-8-acryloyl-6-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione and**

**Example 62b: (3a*R*,11a*S*)-5-(2-((*S*/*R*)-8-acryloyl-6-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

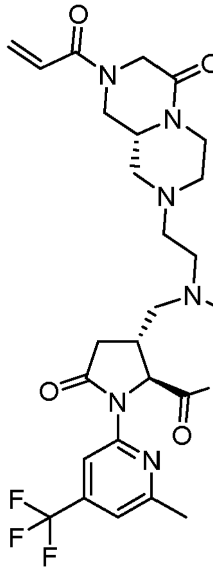

and

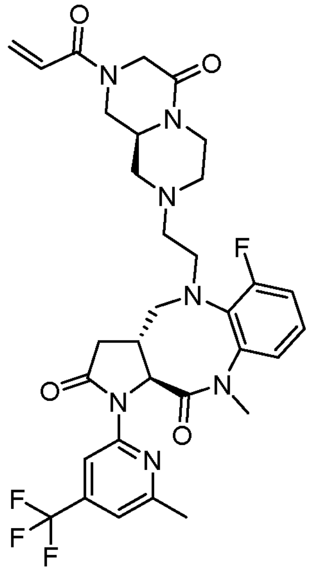

The title compounds were prepared in a similar manner to **Example 58,** using **Intermediate A22** in step a, and an additional chiral SFC step was conducted after step a.

**Example 62a:**

m/z ES+ $[M+H]^+$ 658.3; $^1H$ NMR (400 MHz, $CDCl_3$) δ ppm 8.32 (s, 1H), 7.40 - 7.30 (m, 1H), 7.23 (d, *J* = 8.0 Hz, 1H), 7.20 - 7.10 (m, 1H), 7.06 (s, 1H), 6.60 - 6.43 (m, 1H), 6.42 - 6.31 (m, 1H), 5.83 - 5.75 (m, 1H), 4.60 (d, *J* = 9.2 Hz, 1H), 4.51 (d, *J* = 11.6 Hz, 1H), 4.43 - 3.89 (m, 3H), 3.65 - 3.43 (d, *J* = 9.2 Hz, 2H), 3.36 (s, 3H), 3.32 - 3.13 (m, 3H), 3.02 - 2.63 (m, 6H), 2.50 (s, 3H), 2.46 - 2.31 (m, 2H), 2.26 - 2.14 (m, 1H), 2.10 - 1.81 (m, 2H).

**Example 62b:**

m/z ES+ $[M+H]^+$ 658.3; $^1H$ NMR (400 MHz, $CDCl_3$) δ ppm 8.32 (s, 1H), 7.39 - 7.31 (m, 1H), 7.25 - 7.22 (m, 1H), 7.15 (t, *J* = 9.2 Hz, 1H), 7.06 (s, 1H), 6.61 - 6.43 (m, 1H), 6.41 - 6.33 (m, 1H), 5.81 - 5.76 (m, 1H), 4.60 (d, *J* = 9.2 Hz, 1H), 4.57 - 4.46 (m, 1H), 4.39 - 4.27 (m, 1H), 4.12 - 4.05 (m, 1H), 3.58 - 3.50 (m, 2H), 3.36 (s, 3H), 3.33 - 3.11 (m, 3H), 2.99 - 2.65 (m, 6H), 2.50 (s, 3H), 2.45 - 2.35 (m, 2H), 2.26 - 2.14 (m, 1H), 2.08 - 2.01 (m, 1H), 1.94 - 1.78 (m, 1H), 1.72 - 1.60 (m, 1H).

**Example 63a: (3a*R*,11a*S*)-5-(2-((6*R*/*S*,9a*S*/*R*)-8-acryloyl-6-methyloctahydro-2*H*-pyrazino[1,2-*a*]pyrazin-2-yl) ethyl)-6-fluoro-10-methyl-1-(6-methyl-4-(trifluoromethyl)pyridin-2-yl)-1,3a,4,5,10,11a-hexahydro-2*H*-benzo[*b*] pyrrolo[2,3-*f*][1,4]diazocine-2,11(3*H*)-dione and**

**Example 63b: (3a*R*,11a*S*)-5-(2-((6*S*/*R*,9a*R*/*S*)-8-acryloyl-6-methyloctahydro-2*H*-pyrazino[1,2-*a*]pyrazin-2-yl) ethyl)-6-fluoro-10-methyl-1-(6-methyl-4-(trifluoromethyl)pyridin-2-yl)-1,3a,4,5,10,11a-hexahydro-2*H*-benzo[*b*] pyrrolo[2,3-*f*][1,4]diazocine-2,11(3*H*)-dione**

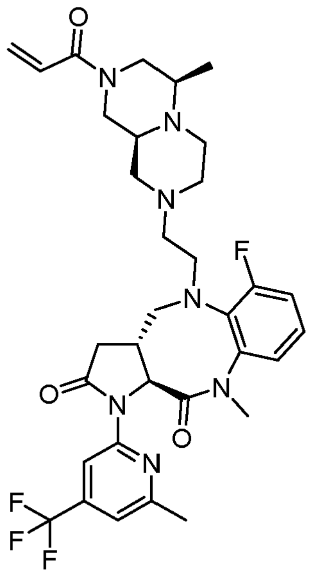

and

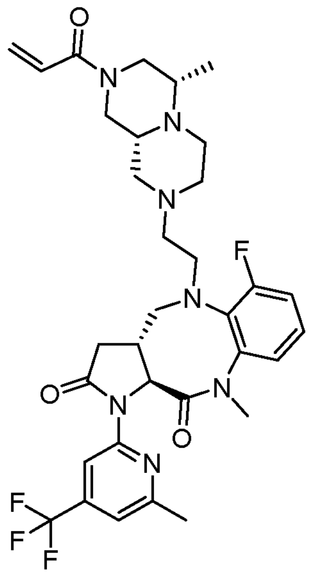

The title compounds were prepared in a similar manner to **Example 58,** using **Intermediate A24a** in step a, and an additional chiral SFC step was conducted after step a.

**Example 63a:**

m/z ES+ $[M+H]^+$ 658.4; $^1H$ NMR (400 MHz, $CDCl_3$) δ ppm 8.33 (s, 1H), 7.38 - 7.30 (m, 1H), 7.22 (d, *J* = 8.0 Hz, 1H), 7.15 (t, *J* = 9.2 Hz, 1H), 7.06 (s, 1H), 6.66 - 6.50 (m, 1H), 6.38 - 6.21 (m, 1H), 5.71 (d, *J* = 8.8 Hz, 1H), 4.60 (d, *J* = 9.2 Hz, 1H), 4.56 - 4.40 (m, 1H), 3.83 - 3.67 (m, 1H), 3.62 - 3.53 (m, 1H), 3.35 (s, 3H), 3.32 - 3.22 (m, 1H), 3.22 - 3.04 (m, 2H), 3.03 - 2.56 (m, 7H), 2.50 (s, 3H), 2.48 - 2.25 (m, 3H), 2.25 - 2.13 (m, 3H), 2.13 - 2.00 (m, 1H), 1.94 - 1.71 (m, 1H), 1.09 (d, *J* = 5.6 Hz, 3H).

**Example 63b:**

m/z ES+ $[M+H]^+$ 658.3; $^1H$ NMR (400 MHz, $CDCl_3$) δ ppm 8.31 (s, 1H), 7.38 - 7.29 (m, 1H), 7.22 (d, *J* = 8.0 Hz, 1H), 7.14 (t, *J* = 9.2 Hz, 1H), 7.05 (s, 1H), 6.55 (dd, *J* = 16.8, 10.4 Hz, 1H), 6.28 (dd, *J* = 16.8, 1.6 Hz, 1H), 5.70 (d, *J* = 10.4 Hz, 1H), 4.60 (d, *J* = 9.2 Hz, 1H), 4.55 - 4.32 (m, 1H), 3.85 - 3.64 (m, 1H), 3.60 - 3.50 (m, 1H), 3.36 (s, 3H), 3.31 - 3.18 (m, 2H), 3.13 - 3.05 (m, 1H), 3.00 - 2.79 (m, 4H), 2.75 - 2.65 (m, 2H), 2.51 - 2.48 (m, 4H), 2.45 - 2.30 (m, 3H), 2.25 - 2.15 (m, 3H), 2.12 - 1.98 (m, 1H), 1.97 - 1.83 (m, 1H), 1.09 (s, 3H).

**Example 64a: (3a*R*,11a*S*)-5-(2-((6*R*/*S*,9a*R*/*S*)-8-acryloyl-6-methyloctahydro-2*H*-pyrazino[1,2-*a*]pyrazin-2-yl) ethyl)-6-fluoro-10-methyl-1-(6-methyl-4-(trifluoromethyl)pyridin-2-yl)-1,3a,4,5,10,11a-hexahydro-2*H*-benzo[*b*] pyrrolo[2,3-*f*][1,4]diazocine-2,11(3*H*)-dione and**

**Example 64b: (3aR,11a*S*)-5-(2-((6*S*/*R*,9a*S*/*R*)-8-acryloyl-6-methyloctahydro-2*H*-pyrazino[1,2-*a*]pyrazin-2-yl) ethyl)-6-fluoro-10-methyl-1-(6-methyl-4-(trifluoromethyl)pyridin-2-yl)-1,3a,4,5,10,11a-hexahydro-2*H*-benzo[*b*] pyrrolo[2,3-*f*][1,4]diazocine-2,11(3*H*)-dione**

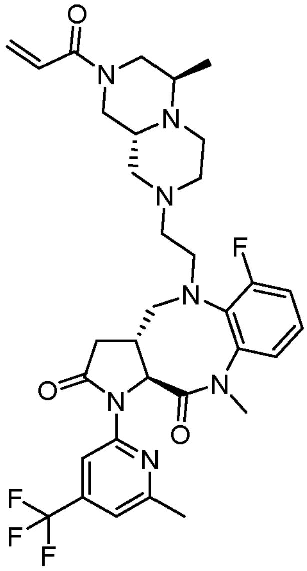

and

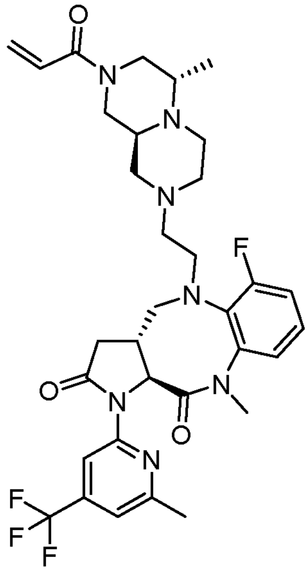

The title compounds were prepared in a similar manner to **Example 58,** using **Intermediate A24b** in step a, and an additional chiral SFC step was conducted after step a.

**Example 64a:**

m/z ES+ $[M+H]^+$ 658.4; $^1H$ NMR (400 MHz, $CDCl_3$) δ ppm 8.32 (s, 1H), 7.37 - 7.29 (m, 1H), 7.22 (d, *J* = 7.6 Hz, 1H), 7.18 - 7.10 (m, 1H), 7.05 (s, 1H), 6.67 - 6.45 (m, 1H), 6.38 - 6.22 (m, 1H), 5.70 (dd, *J* = 10.4, 1.6 Hz, 1H), 4.60 (d, *J* = 9.2 Hz, 1H), 4.50 - 4.38 (m, 1H), 3.83 - 3.68 (m, 1H), 3.64 - 3.40 (m, 2H), 3.35 (s, 3H), 3.30 - 3.13 (m, 2H), 3.08 - 2.98 (m, 1H), 2.96 - 2.86 (m, 2H), 2.83 - 2.54 (m, 6H), 2.49 (s, 3H), 2.46 - 2.13 (m, 5H), 1.89 - 1.70 (m, 1H), 0.99 (d, *J* = 6.4 Hz, 3H).

**Example 64b:**

m/z ES+ $[M+H]^+$ 658.4; $^1H$ NMR (400 MHz, $CDCl_3$) δ ppm 8.33 (s, 1H), 7.38 - 7.31 (m, 1H), 7.23 (d, *J* = 7.6 Hz, 1H), 7.20 - 7.12 (m, 1H), 7.07 (s, 1H), 6.70 - 6.45 (m, 1H), 6.31 (d, *J* = 16.8 Hz, 1H), 5.71 (d, *J* = 10.4 Hz, 1H), 4.62 (d, *J* = 9.2 Hz, 1H), 4.46 (d, *J* = 11.2 Hz, 1H), 3.73 (d, *J* = 12.4 Hz, 1H), 3.63 - 3.51 (m, 1H), 3.37 (s, 3H), 3.31 - 3.13 (m, 2H), 3.10 - 2.82 (m, 4H), 2.81 - 2.60 (m, 5H), 2.58 - 2.50 (m, 4H), 2.48 - 2.28 (m, 3H), 2.21 (dd, *J* = 16.8, 11.2 Hz, 2H), 1.88 - 1.77 (m, 1H), 0.99 (d, *J* = 6.4 Hz, 3H).

**Example 65**

***N*-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-1-(2-((dimethylamino)methyl)acryloyl)-N-methylazetidine-3-carboxamide**

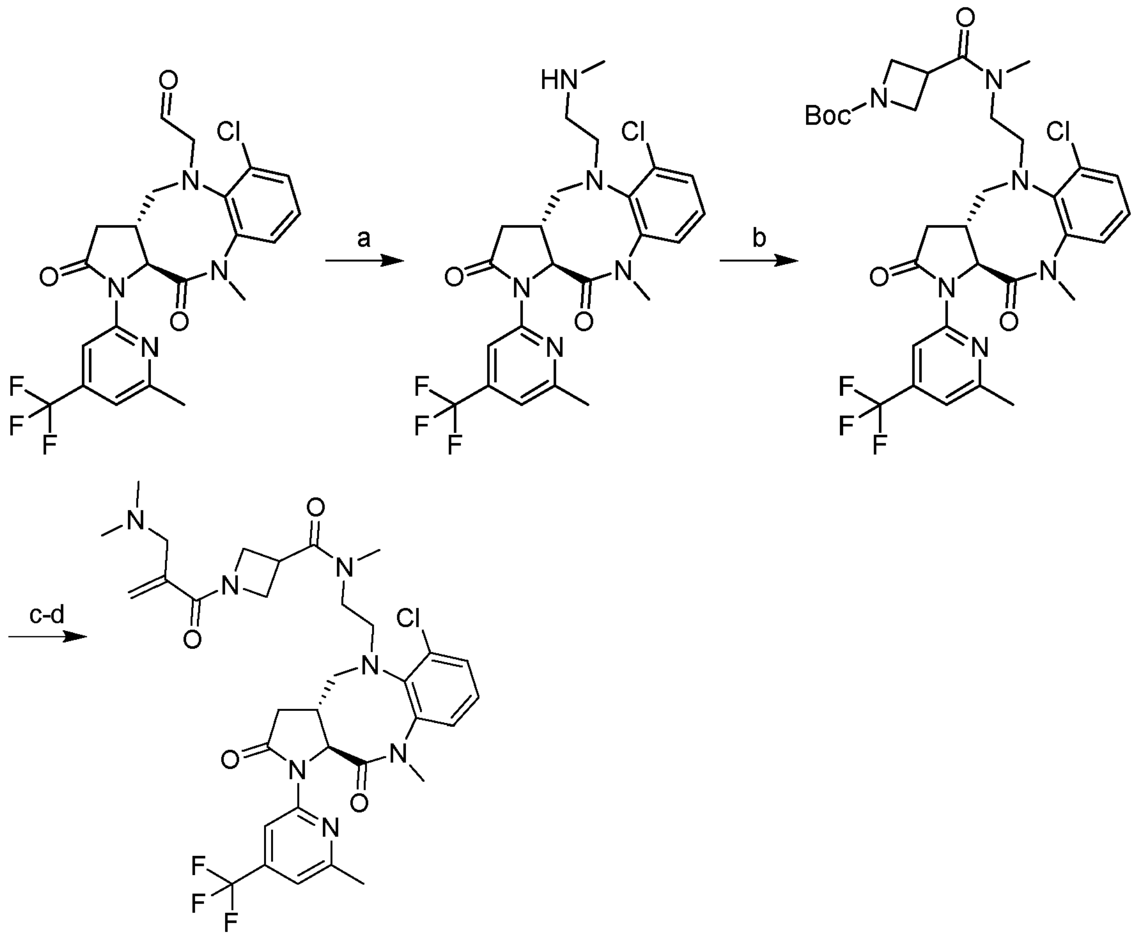


**Step a.** This step was conducted in a similar manner to **Example 1,** step a, using **Intermediate 7b** and methanamine (2 M in THF).

**Step b.** To a solution of (3a*R*,11a*S*)-6-chloro-10-methyl-1-(6-methyl-4-(trifluoromethyl)pyridin-2-yl)-5-(2-(methylamino)ethyl)-1,3a,4,5,10,11a-hexahydro-2*H*-benzo[*b*]pyrrolo[2,3-*f*][1,4]diazocine-2,11(3*H*)-dione (60 mg, 0.093 mmol) and 1-(*tert*-butoxycarbonyl)azetidine-3-carboxylic acid (24 mg, 0.12 mmol) in DCM (1.2 mL) was added DIPEA (24 mg, 0.19 mmol) and HATU (43 mg, 0.11 mmol). The mixture was stirred at 20 °C for 30 min. Upon completion, the reaction mixture was quenched with $H_2O$ (5 mL) and extracted with EtOAc (3 x 5 mL). The combined organic layers were washed with brine (15 mL), dried over $Na_2SO_4$ and evaporated. The residue was purified by Prep-TLC (EtOAc) to give *tert*-butyl 3-((2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)(methyl)carbamoyl)azetidine-1-carboxylate (50 mg, 79% yield) as a yellow solid.

m/z ES+ $[M+H]^+$679.1.

**Steps c-d.** These 2 steps were conducted in a similar manner to **Example 27,** steps b-c, using **Intermediate E1** in step d.

m/z ES+ $[M+H]^+$ 690.1; $^1$H NMR (400 MHz, $CDCl_3$) δ ppm 8.33 (s, 1H), 7.50 - 7.41 (m, 1H), 7.37 - 7.29 (m, 2H), 7.05 (s, 1H), 5.91 (s, 1H), 5.73 (s, 1H), 4.67 - 4.51 (m, 2H), 4.45 - 4.28 (m, 2H), 4.19 - 4.03 (m, 1H), 3.78 - 3.50 (m, 4H), 3.42 - 3.32 (m, 4H), 3.25 - 3.08 (m, 3H), 3.01 - 2.87 (m, 5H), 2.80 - 2.72 (m, 1H), 2.62 - 2.54 (m, 6H), 2.49 - 2.46 (m, 3H), 2.24 - 2.17 (m, 1H).

## Example 66

**(3a*R*,11a*S*)-5-(2-(((3*R*,4*S*)-1-acryloyl-3-fluoropiperidin-4-yl)amino)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

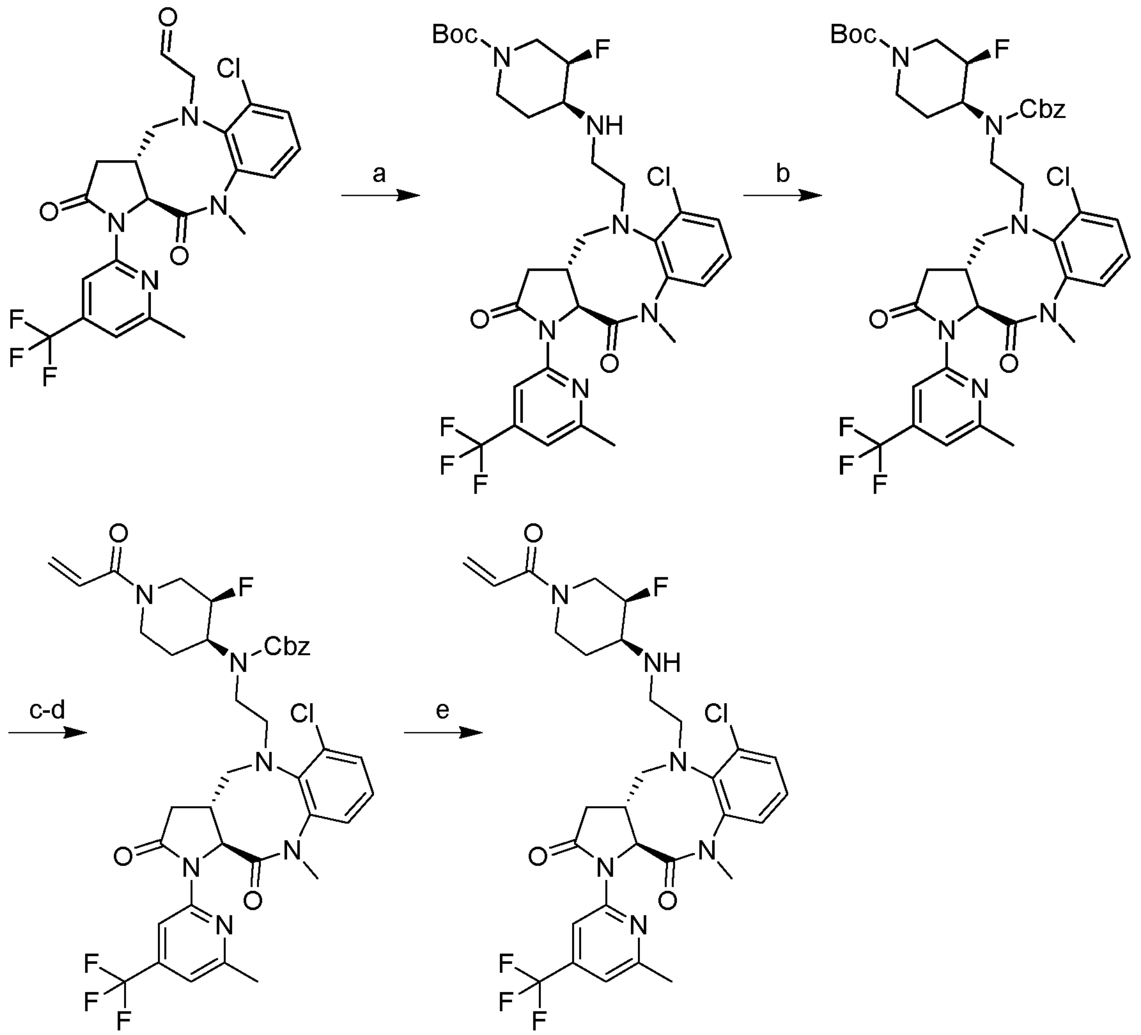


**Step** a. This step was conducted in a similar manner to **Example 1,** step a, using **Intermediate 7b** and *tert*-butyl (3*R*,4*S*)-4-amino-3-fluoropiperidine-1-carboxylate (CAS: 907544-17-6).

**Step b.** To a solution of *tert*-butyl (3*R*,4*S*)-4-((2-((3a*R*,11a*S*)-6-chloro-10-methyl-1-(6-methyl-4-(trifluoromethyl) pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)amino)-3-fluoropiperidine-1-carboxylate (120 mg, 0.15 mmol) in THF (1.5 mL) and $H_2O$ (1.5 mL) was added $NaHCO_3$ (26 mg, 0.31 mmol) and CbzCl (34 mg, 0.2 mmol). The mixture was stirred at 25 °C for 30 min. Upon completion, the reaction mixture was quenched with $H_2O$ (5 mL) and extracted with EtOAc (3 x 5 mL). The combined organic layers were washed with brine (12 mL), dried over $Na_2SO_4$ and evaporated. The residue was purified by Prep-TLC (PE/EtOAc = 1/1) to give *tert*-butyl (3*R*,4*S*)-4-(((benzyloxy)carbonyl)(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)amino)-3-fluoropiperidine-1-carboxylate (110 mg, 86% yield) as an off-white solid.

m/z ES+ $[M+H]^+$ 817.3; $^1H$ NMR (400 MHz, $CDCl_3$) δ ppm 8.31 (s, 1H), 7.43 (d, *J* = 8.8 Hz, 1H), 7.40 - 7.27 (m, 6H), 7.26 (s, 1H), 7.04 (s, 1H), 5.15 - 5.06 (m, 2H), 4.68 - 4.58 (m, 1H), 4.53 (d, *J* = 9.2 Hz, 1H), 4.40 - 4.31 (m, 1H), 4.27 - 4.17 (m, 1H), 3.50 - 3.22 (m, 4H), 3.16 (s, 3H), 3.07 - 2.87 (m, 3H), 2.85 - 2.69 (m, 3H), 2.53 - 2.42 (m, 4H), 2.04 - 2.02 (m, 1H), 1.55 - 1.47 (m, 2H), 1.46 (s, 9H).

**Step c.** This step was conducted in a similar manner to **Example 1,** step b.

**Step d.** To a solution of benzyl (2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)((3*R*,4*S*)-3-fluoropiperidin-4-yl)carbamate (96 mg, 0.13 mmol) and acrylic acid (13 mg, 0.17 mmol) in DCM (2 mL) was added DIPEA (35 mg, 0.27 mmol) and HATU (61 mg, 0.16 mmol). The mixture was stirred at 25 °C for 1 h. Upon completion, the reaction mixture was quenched with $H_2O$ (5 mL) and extracted with EtOAc (3 x 5 mL). The combined organic layers were washed with brine (10 mL), dried over $Na_2SO_4$ and evaporated. The residue was purified by Prep-TLC (EtOAc) to give benzyl ((3*R*,4*S*)-1-acryloyl-3-fluoropiperidin-4-yl)(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)carbamate (45 mg, 42% yield) as a

white solid. m/z ES+ [M+H]+ 771.2.

**Step e.** A solution of benzyl ((3*R*,4*S*)-1-acryloyl-3-fluoropiperidin-4-yl)(2-((3a*R*,11a*S*)-6-chloro-10-methyl-1-(6-methyl-4-(trifluoromethyl) pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)carbamate (40.0 mg, 0.05 mmol) in TFA (1 mL) was stirred at 45 °C for 12 h. Upon completion, the reaction mixture was evaporated. The residue was purified by Prep-HPLC to give the title compound (11 mg, 33% yield) as a white solid.

m/z ES+ [M+H]+ 637.2; $^1H$ NMR (400 MHz, $CDCl_3$) δ ppm 8.33 (s, 1H), 7.46 (d, *J* = 6.4 Hz, 1H), 7.35 - 7.28 (m, 2H), 7.06 (s, 1H), 6.65 - 6.50 (m, 1H), 6.35 - 6.23 (m, 1H), 5.71 (dd, *J* = 10.4, 1.6 Hz, 1H), 4.98 - 4.76 (m, 1H), 4.71 - 4.56 (m, 2H), 4.37 - 3.95 (m, 1H), 3.59 (d, *J* = 10.0 Hz, 1H), 3.39 (s, 3H), 3.33 - 3.11 (m, 3H), 3.02 - 2.81 (m, 4H), 2.80 - 2.70 (m, 2H), 2.65 - 2.56 (m, 1H), 2.48 (s, 3H), 2.28 - 2.20 (m, 1H), 1.83 - 1.77 (m, 2H), 1.72 - 1.65 (m, 1H).

**Example 67**

**(3a*R*,11a*S*)-5-(2-(((3R,4S)-1-acryloyl-3-fluoropiperidin-4-yl)(methyl)amino)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

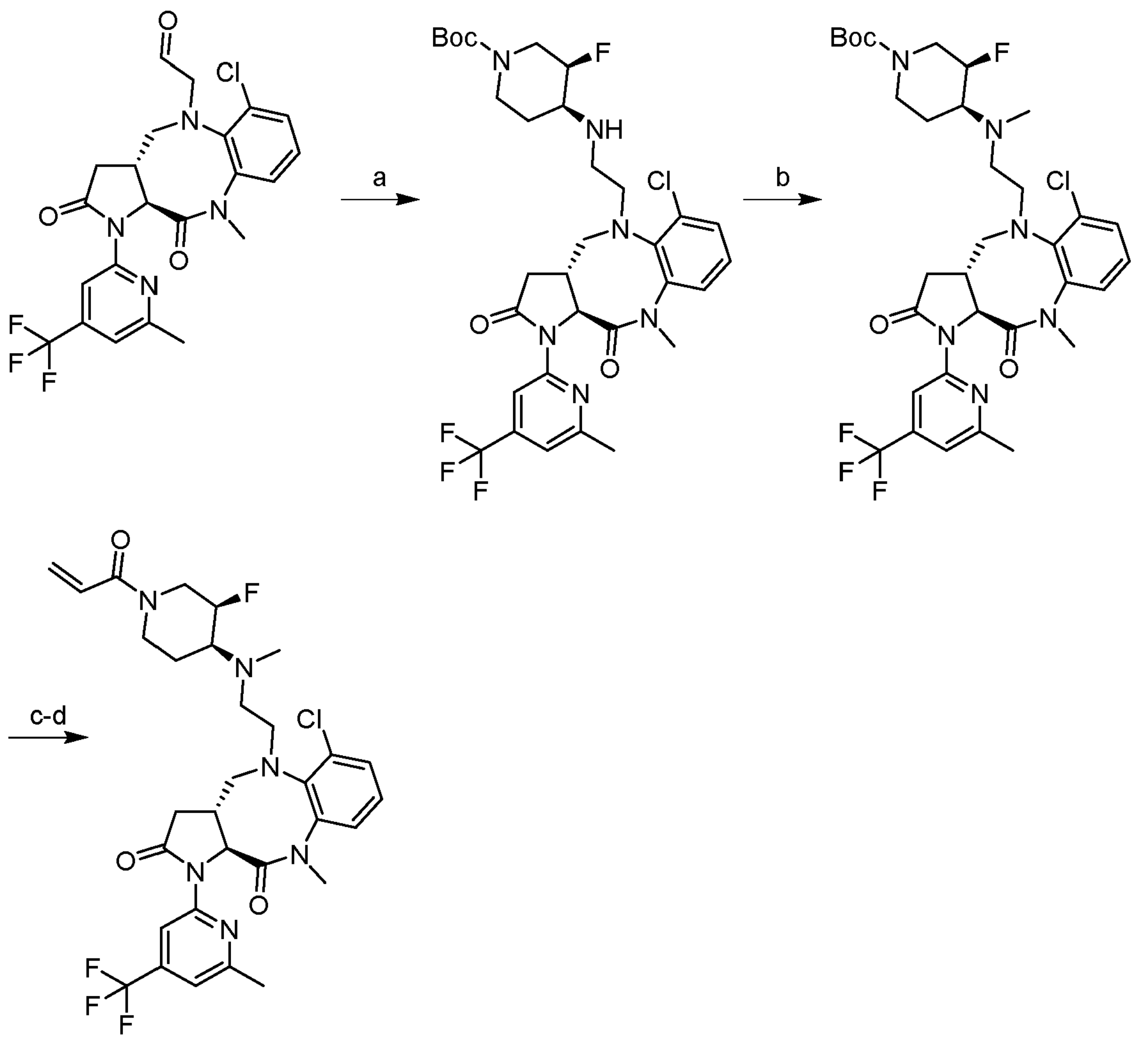


**Step a.** This step was conducted as described in **Example 66,** step a.

**Step b.** To a solution of *tert*-butyl (3*R*,4*S*)-4-((2-((3a*R*,11a*S*)-6-chloro-10-methyl-1-(6-methyl-4-(trifluoromethyl) pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)amino)-3-fluoropiperidine-1-carboxylate (55 mg, 0.08 mmol) and paraformaldehyde (24 mg, 0.81 mmol) in MeOH (2 mL) was added $NaBH_3CN$ (15 mg, 0.24 mmol) and acetic acid (10 mg, 0.16 mmol). The mixture was stirred at 40 °C for 12 h. Upon

completion, the reaction mixture was diluted with sat. aq. $NaHCO_3$ (5 mL), and then extracted with EtOAc (3 x 5 mL). The combined organic layers were washed with brine (15 mL), dried over $Na_2SO_4$ and evaporated. The crude product was purified by Prep-TLC (PE/EtOAc = 1/1) to give *tert*-butyl (3*R*,4*S*)-4-((2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)(methyl)amino)-3-fluoropiperidine-1-carboxylate (45 mg, 78% yield) as a white solid.

m/z ES+ $[M+H]^+$ 697.3; $^1H$ NMR (400 MHz, $CDCl_3$) δ ppm 8.33 (s, 1H), 7.45 (d, *J* = 7.2 Hz, 1H), 7.36 - 7.29 (m, 2H), 7.05 (s, 1H), 4.94 - 4.68 (m, 1H), 4.65 - 4.58 (m, 1H), 4.47 - 4.26 (m, 2H), 3.59 - 3.56 (m, 1H), 3.38 (s, 3H), 3.23 - 3.07 (m, 2H), 3.05 - 2.86 (m, 3H), 2.82 - 2.54 (m, 5H), 2.48 (s, 3H), 2.35 - 2.25 (m, 2H), 2.25 - 2.20 (m, 1H), 1.97 - 1.86 (m, 1H), 1.64 - 1.54 (m, 2H), 1.46 (s, 9H).

**Steps c-d.** These 2 steps were conducted in a similar manner to **Example** 1, steps b-c.

m/z ES+ $[M+H]^+$ 651.3; $^1H$ NMR (400 MHz, $CDCl_3$) δ ppm 8.33 (s, 1H), 7.47 - 7.43 (m, 1H), 7.35 - 7.28 (m, 2H), 7.05 (s, 1H), 6.65 - 6.48 (m, 1H), 6.35 - 6.20 (m, 1H), 5.71 (d, *J* = 10.4 Hz, 1H), 5.11 - 4.91 (m, 1H), 4.90-4.77 (m, 1H), 4.61 (d, *J* = 9.2 Hz, 1H), 4.29 - 4.10 (m, 1H), 3.57 (d, *J* = 10.0 Hz, 1H), 3.38 (s, 3H), 3.31 - 3.05 (m, 3H), 3.04 - 2.93 (m, 2H), 2.86 - 2.53 (m, 5H), 2.49 (s, 3H), 2.48 - 2.27 (m, 3H), 2.21 (dd, *J* = 16.8, 11.2 Hz, 1H), 2.02 - 1.89 (m, 1H), 1.75 - 1.65 (m, 1H).

**Example 68**

**(3a*R*,11a*S*)-5-((*S*/*R*)-3-((1-acryloylazetidin-3-yl)(methyl)amino)-2-hydroxypropyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

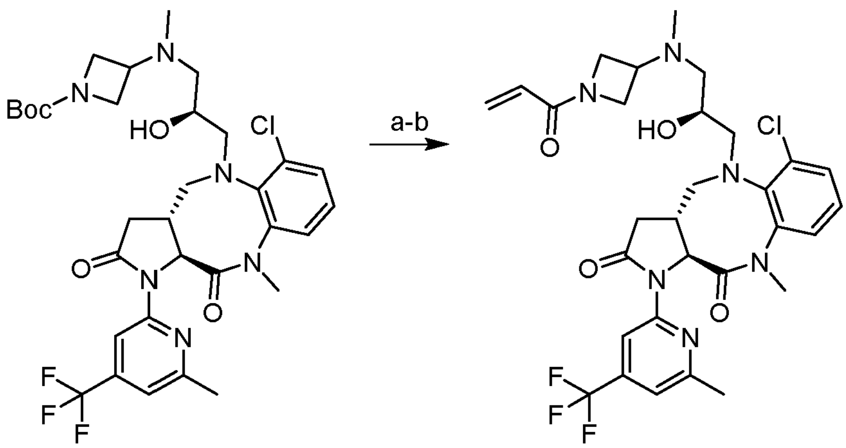


**Steps a-b.** These 2 steps were conducted in a similar manner to **Example 66,** steps c-d, using **Intermediate 12a.**

m/z ES+ $[M+H]^+$ 635.3; $^1H$ NMR (400 MHz, $CDCl_3$) δ ppm 8.34 (s, 1H), 7.50 - 7.45 (m, 1H), 7.36 - 7.29 (m, 2H), 7.06 (s, 1H), 6.38 - 6.28 (m, 1H), 6.24 - 6.13 (m, 1H), 5.67 (d, *J* = 10.4 Hz, 1H), 4.66 - 4.58 (m, 1H), 4.30 - 4.18 (m, 1H), 4.15 - 3.99 (m, 2H), 3.96 - 3.86 (m, 1H), 3.74 - 3.56 (m, 2H), 3.53 - 3.41 (m, 1H), 3.39 (s, 3H), 3.32 - 3.14 (m, 2H), 3.09 - 3.01 (m, 1H), 3.00 - 2.86 (m, 2H), 2.80 - 2.71 (m, 1H), 2.49 (s, 3H), 2.46 - 2.38 (m, 1H), 2.34 - 2.28 (m, 1H), 2.26 (s, 3H), 2.24 - 2.18 (m, 1H).

**Example 69**

**(3a*R*,11a*S*)-5-((*R*/*S*)-3-((1-acryloylazetidin-3-yl)(methyl)amino)-2-hydroxypropyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

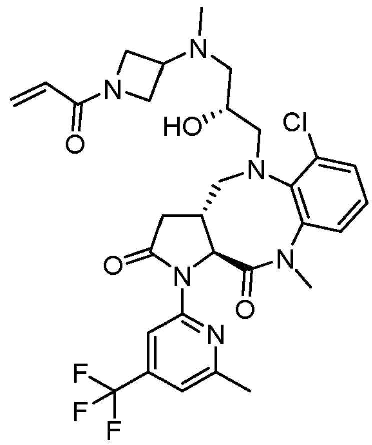

The title compound was prepared in a similar manner to **Example 68,** using **Intermediate 12b** in step a.

m/z ES+ $[M+H]^+$ 635.3; $^1H$ NMR (400 MHz, $CDCl_3$) δ ppm 8.34 (s, 1H), 7.47 (dd, *J* = 7.2, 2.4 Hz, 1H), 7.38 - 7.29 (m, 2H), 7.06 (s, 1H), 6.39 - 6.28 (m, 1H), 6.24 - 6.12 (m, 1H), 5.68 (d, *J* = 10.4 Hz, 1H), 4.71 - 4.57 (m, 1H), 4.30 - 4.15 (m, 1H), 4.16 - 4.02 (m, 2H), 4.00 - 3.85 (m, 1H), 3.83 - 3.58 (m, 2H), 3.55 - 3.25 (m, 5H), 3.16 - 2.89 (m, 4H), 2.76 (dd, *J* = 16.8, 7.6 Hz, 1H), 2.49 (s, 3H), 2.46 - 2.16 (m, 6H).

**Example 70**

**(3a*R*,11a*S*)-5-(3-((1-acryloylazetidin-3-yl)amino)propyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

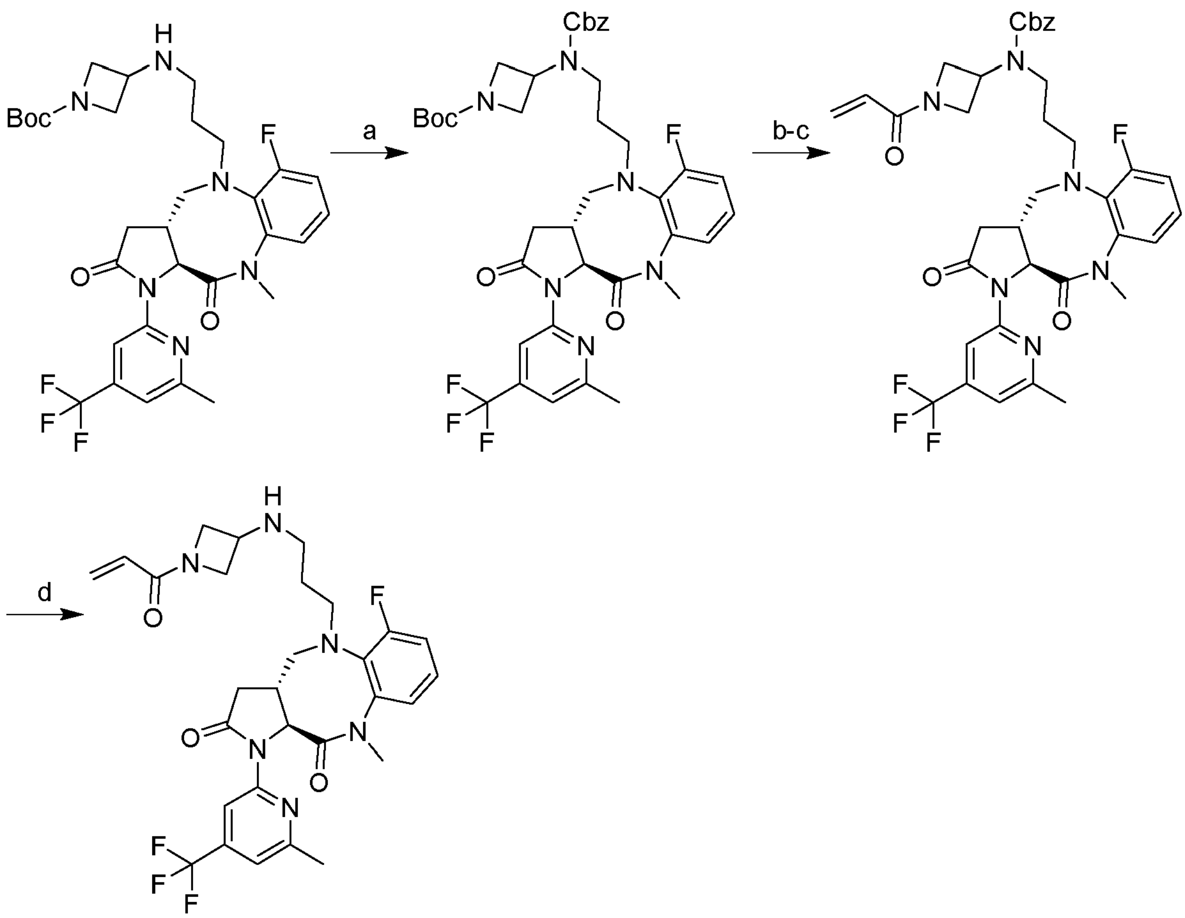

**Step a.** To a solution of **Intermediate 13** (0.17 g, 0.27 mmol) in THF (3 mL) and water (1 mL) was added CbzCl (69 mg, 0.40 mmol) and $Na_2CO_3$ (85 mg, 0.80 mmol). The mixture was stirred at 25 °C for 1 h. Upon completion, the reaction mixture was quenched with sat. aq. $NH_4Cl$ (20 mL), diluted with water (30 mL) and extracted with EtOAc (3 x 30 mL). The combined organic layers were washed with brine (20 mL), dried over $Na_2SO_4$ and evaporated. Purification by column chromatography (PE/EtOAc = 1/1) afforded *tert*-butyl 3-(((benzyloxy)carbonyl)(3-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propyl)amino)azetidine-1-carboxylate (180 mg, 87% yield) as a yellow solid.

m/z ES+ $[M+H]^+$ 769.1; $^1H$ NMR (400 MHz, $CDCl_3$) δ ppm 8.33 (s, 1H), 7.38 - 7.30 (m, 6H), 7.20 (d, *J* = 8.4 Hz, 1H), 7.13 (t, *J* = 9.2 Hz, 1H), 7.05 (s, 1H), 5.12 (s, 2H), 4.61 (d, *J* = 8.4 Hz, 2H), 4.26 - 4.02 (m, 3H), 3.99 - 3.85 (m, 2H), 3.51 - 3.40 (m, 1H), 3.32 - 3.25 (m, 5H), 3.10 - 2.96 (m, 2H), 2.90 - 2.75 (m, 2H), 2.70 - 2.57 (m, 1H), 2.49 (s, 3H), 2.20 - 2.13 (m, 1H), 1.56 - 1.51 (m, 1H), 1.44 (s, 9H).

**Steps b-c.** These 2 steps were conducted in a similar manner to **Example 67,** steps c-d.

**Step d.** To a solution of benzyl (1-acryloylazetidin-3-yl)(3-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propyl)carbamate (150 mg, 0.21 mmol) in DCM (3 mL) was added $BCl_3$ (1 M in DCM, 2.08 mL) and the mixture was stirred at 0 °C for 30 min. Upon completion, the reaction mixture was quenched with sat. aq. $NaHCO_3$ (30 mL), diluted with water (10 mL) and extracted with DCM (3 x 30 mL). The combined organic layers were washed with sat. aq. $NaHCO_3$ (3 x 20 mL), dried over $Na_2SO_4$ and evaporated. The crude product was purified by Prep-HPLC to give the title compound (55 mg, 45% yield) as a white solid.

m/z ES+ $[M+H]^+$ 589.1; $^1H$ NMR (400 MHz, $CDCl_3$) δ ppm 8.32 (s, 1 H), 7.37 - 7.30 (m, 1H), 7.22 (d, *J* = 8.0 Hz, 1H), 7.14 (t, *J* = 9.2 Hz, 1H), 7.05 (s, 1H), 6.35 - 6.28 (m, 1H), 6.23 - 6.14 (m, 1H), 5.65 (d, *J* = 10.4 Hz, 1H), 4.61 (d, *J* = 9.2 Hz, 1H), 4.38 - 4.31 (m, 1H), 4.26 - 4.18 (m, 1H), 3.91 - 3.84 (m, 1H), 3.74 - 3.70 (m, 1H), 3.65 - 3.62 (m, 1H), 3.56 - 3.52 (m, 1H), 3.35 (s, 3H), 3.23 - 3.08 (m, 2H), 2.95 - 2.82 (m, 2H), 2.74 - 2.69 (m, 1H), 2.67 - 2.55 (m, 2H), 2.49 (s, 3H), 2.24 - 2.16 (m, 1H), 1.54 - 1.47 (m, 3H).

**Example 71**

**(3a*R*,11a*S*)-5-(3-((1-acryloylazetidin-3-yl)(methyl)amino)propyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

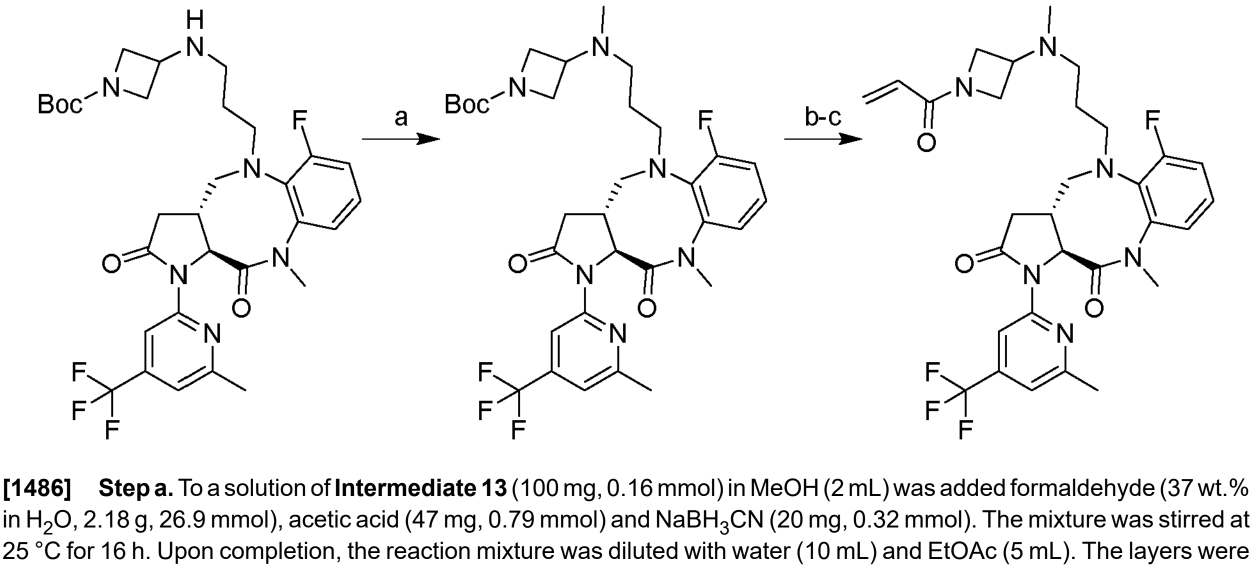


**Step a.** To a solution of **Intermediate 13** (100 mg, 0.16 mmol) in MeOH (2 mL) was added formaldehyde (37 wt.% in $H_2O$, 2.18 g, 26.9 mmol), acetic acid (47 mg, 0.79 mmol) and $NaBH_3CN$ (20 mg, 0.32 mmol). The mixture was stirred at 25 °C for 16 h. Upon completion, the reaction mixture was diluted with water (10 mL) and EtOAc (5 mL). The layers were separated and the aqueous layer was further extracted with EtOAc (2 x 5 mL), dried over $Na_2SO_4$ and evaporated to give *tert*-butyl 3-((3-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propyl)(methyl)amino)azetidine-1-carboxylate (100 mg, crude) as yellow oil.

m/z ES+ $[M+H]^+$ 649.2.

**Steps b-c.** These 2 steps were conducted in a similar manner to **Example 67,** steps c-d.

m/z ES+ $[M+H]^+$ 603.3; $^1H$ NMR (400 MHz, $CDCl_3$) δ ppm 8.33 (s, 1H), 7.38 - 7.31 (m, 1H), 7.22 (d, *J* = 8.0 Hz, 1H), 7.19 - 7.11 (m, 1H), 7.06 (s, 1H), 6.37 - 6.28 (m, 1H), 6.25 - 6.14 (m, 1H), 5.68 (m, 1H), 4.62 (d, *J* = 9.2 Hz, 1H), 4.34 - 3.84 (m,

4H), 3.57 - 3.54 (m, 1H), 3.43 - 3.30 (m, 4H), 3.05 (m, 2H), 2.97 - 2.68 (m, 4H), 2.50 (s, 4H), 2.34 - 2.12 (m, 4H), 1.68 - 1.51 (m, 2H).

**Example 72**

**(3a*R*,11a*S*)-5-(2-(4-(1-acryloyl-3-methylazetidin-3-yl)piperazin-1-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

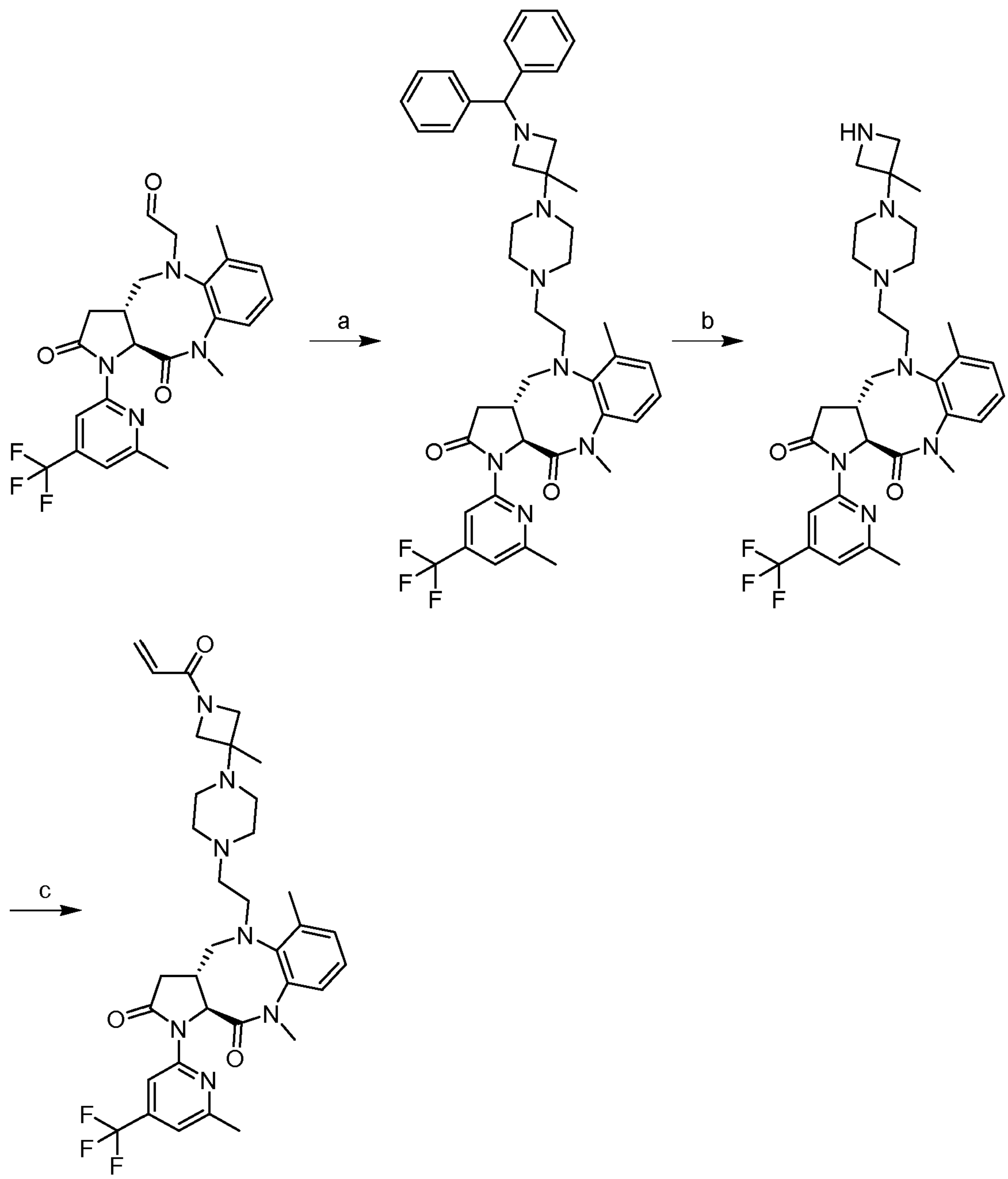


**Step** a. This step was conducted in a similar manner to **Example 1,** step a, using **Intermediate 7i** and **Intermediate A25.**

**Step b.** To a mixture of (3a*R*,11a*S*)-5-(2-(4-(1-benzhydryl-3-methylazetidin-3-yl)piperazin-1-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (100 mg, 0.13 mmol) in 2-propanol (10 mL) was added 10% Pd/C (20 mg), 10% $Pd(OH)_2$/C (20 mg) and

4 M HCl in dioxane (33 uL). The reaction mixture was stirred at 40 °C for 28 hr under a $H_2$ atmosphere (15 psi). Upon completion, the reaction mixture was filtered through a pad of Celite, washing with MeOH (2 x 3 mL). The filtrate was evaporated to give (3aR,11aS)-6,10-dimethyl-1-(6-methyl-4-(trifluoromethyl)pyridin-2-yl)-5-(2-(4-(3-methylazetidin-3-yl) piperazin-1-yl)ethyl)-1,3a,4,5,10,11a-hexahydro-2*H*-benzo[*b*]pyrrolo[2,3-*f*][1,4]diazocine-2,11(3*H*)-dione (60 mg, 76% yield) as a yellow oil.

m/z ES+ $[M+H]^+$600.3.

**Step c.** This step was conducted in a similar manner to **Example 1,** step c.

m/z ES+ $[M+H]^+$ 654.1; $^1H$ NMR (400 MHz, $CDCl_3$) δ ppm 8.33 (s, 1H), 7.27 - 7.19 (m, 3H), 7.05 (s, 1H), 6.38 - 6.29 (m, 1H), 6.24 - 6.12 (m, 1H), 5.70 - 5.63 (m, 1H), 4.69 (d, *J* = 9.2 Hz, 1H), 4.02 (d, *J* = 8.0 Hz, 1H), 3.88 (d, *J* = 9.6 Hz, 1H), 3.79 (d, *J* = 7.6 Hz, 1H), 3.72 - 3.58 (m, 2H), 3.38 (s, 3H), 3.27 - 2.87 (m, 4H), 2.86 - 2.67 (m, 3H), 2.60 - 2.50 (m, 2H), 2.49 (s, 3H), 2.48 - 2.40 (m, 4H), 2.39 (s, 3H), 2.38 - 2.26 (m, 2H), 2.24 - 2.14 (m, 1H), 1.32 (s, 3H).

**Example 73a: (3a*R*,11a*S*)-6-chloro-5-(((*R*/*S*)-7-(2-chloroacetyl)-6-methyl-5,6,7,8-tetrahydroimidazo[1,2-*a*]pyrazin-2-yl)methyl)-10-methyl-1-(6-methyl-4-(trifluoromethyl)pyridin-2-yl)-1,3a,4,5,10,11a-hexahydro-2*H*-benzo[*b*] pyrrolo[2,3-*f*][1,4]diazocine-2,11(3*H*)-dione and**

**Example 73b: (3a*R*,11a*S*)-6-chloro-5-(((*S*/*R*)-7-(2-chloroacetyl)-6-methyl-5,6,7,8-tetrahydroimidazo[1,2-*a*]pyrazin-2-yl)methyl)-10-methyl-1-(6-methyl-4-(trifluoromethyl)pyridin-2-yl)-1,3a,4,5,10,11a-hexahydro-2*H*-benzo[*b*] pyrrolo[2,3-*f*][1,4]diazocine-2,11(3*H*)-dione**

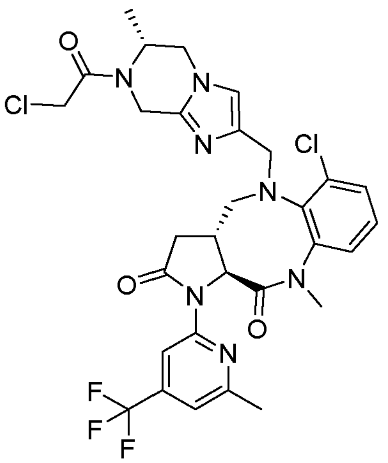

and

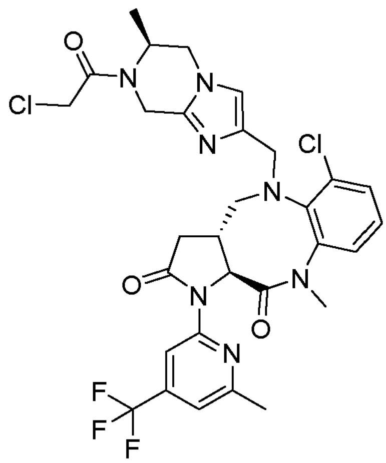

The title compounds were prepared in a similar manner to **Example 39,** using **Intermediate 5b** and **Intermediate B26** in step a, and 2-chloroacetyl chloride in step c. An additional chiral SFC step was conducted after step a.

**Example 73a:**

m/z ES+ $[M+H]^+$ 664.2; $^1H$ NMR (400 MHz, $CDCl_3$) δ ppm 8.31 (s, 1H), 7.47 (dd, *J* = 7.2, 2.4 Hz, 1H), 7.35 - 7.29 (m, 2H), 7.06 (s, 1H), 6.90 (s, 1H), 5.44 - 5.22 (m, 1H), 5.00 - 4.47 (m, 3H), 4.41 - 4.20 (m, 2H), 4.19 - 4.10 (m, 3H), 3.96 - 3.83 (m, 1H), 3.67 - 3.61 (m, 1H), 3.33 (s, 3H), 3.10 - 3.03 (m, 1H), 3.02 - 2.93 (m, 1H), 2.69 (dd, *J* = 16.8, 8.0 Hz, 1H), 2.49 (s, 3H), 2.20 (dd, *J* = 16.8, 11.6 Hz, 1H), 1.36 - 1.20 (m, 3H).

**Example 73b:**

m/z ES+ $[M+H]^+$ 664.1; $^1H$ NMR (400 MHz, $CDCl_3$) δ ppm 8.31 (s, 1H), 7.47 (dd, *J* = 6.8, 2.8 Hz, 1H), 7.38 - 7.29 (m, 2H), 7.06 (s, 1H), 6.91 (s, 1H), 5.51 - 5.24 (m, 1H), 5.11 - 4.57 (m, 3H), 4.53 - 4.24 (m, 2H), 4.22 - 4.13 (m, 3H), 3.95 - 3.80 (m, 1H), 3.65 - 3.60 (m, 1H), 3.33 (s, 3H), 3.12 - 2.90 (m, 2H), 2.70 (dd, *J* = 16.8, 8.4 Hz, 1H), 2.49 (s, 3H), 2.22 - 2.15 (m, 1H), 1.37 - 1.19 (m, 3H).

**Example 74**

**(3a*R*,11a*S*)-5-((7-acryloyl-3-methyl-5,6,7,8-tetrahydroimidazo[1,2-*a*]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

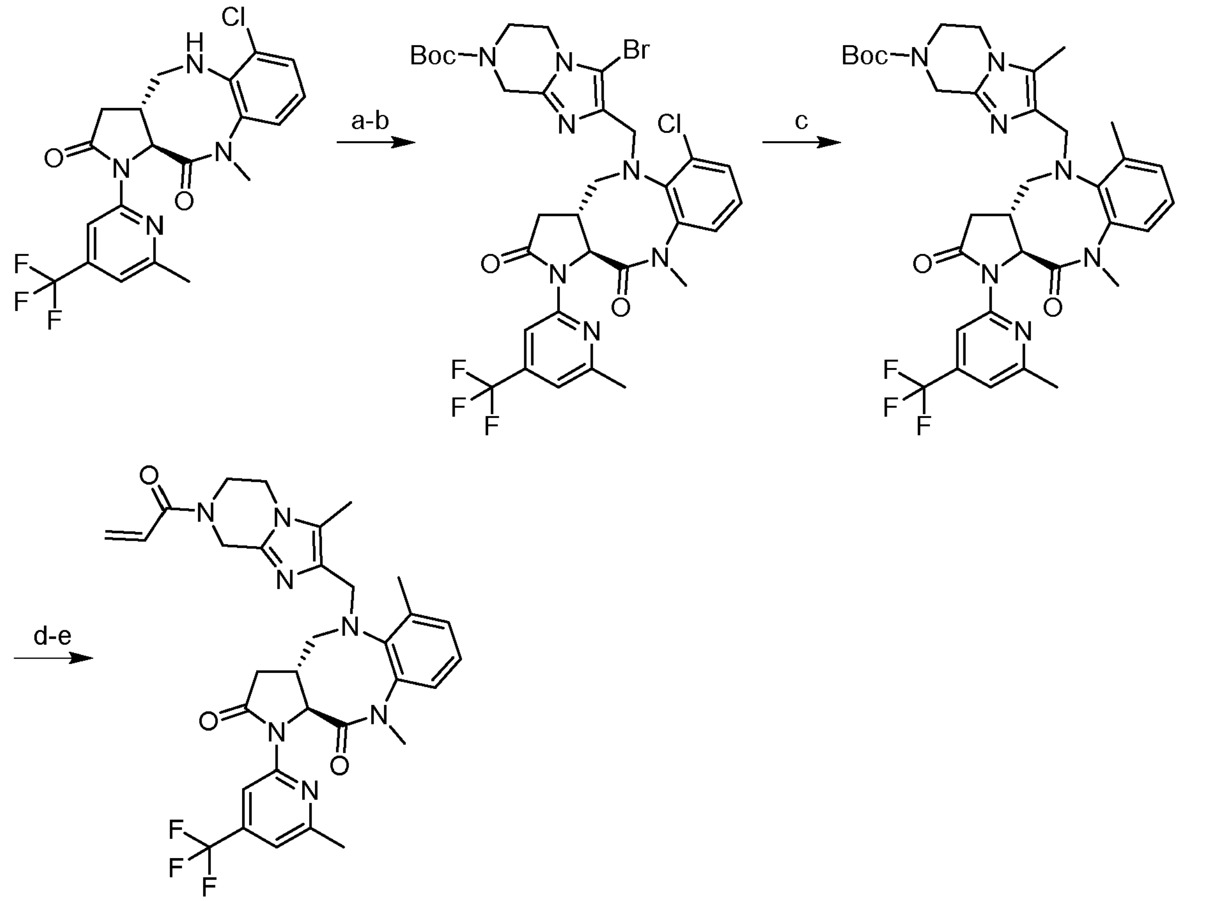


**Step a.** This step was conducted in a similar manner to **Example 39,** step a, using **Intermediate 5b** and **Intermediate B19.**

**Step b.** A solution of *tert*-butyl 2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,6-dihydroimidazo [1,2-*a*]pyrazine-7(8*H*)-carboxylate (280 mg, 0.42 mmol) and NBS (70 mg, 0.39 mmol) in MeCN (20 mL) was stirred at 20 °C for 1 h. Upon completion, the mixture was evaporated and the residue was purified by reverse phase flash chromatography (water (0.1% FA)/MeCN) to give *tert*-butyl 3-bromo-2-(((3a*R*,11a*S*)-6-chloro-10-methyl-1-(6-methyl-4-(trifluoromethyl) pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,6-dihydroimidazo[1,2-*a*]pyrazine-7(8*H*)-carboxylate (160 mg, 51% yield) as a white solid.

m/z ES+ $[M+H]^+$ 754.1.

**Step c.** A mixture of methylboronic acid (238 mg, 3.98 mmol), *tert*-butyl 3-bromo-2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo [2,3-*f*][1,4]diazocin-5-yl)methyl)-5,6-dihydroimidazo[1,2-a]pyrazine-7(8H)-carboxylate (150 mg, 0.20 mmol), XPhos-Pd-G2 (31 mg, 0.40 mmol) and $Cs_2CO_3$ (194 mg, 0.60 mmol) in toluene (10 mL) was degassed and purged with $N_2$ 3 times. The reaction mixture was stirred at 100 °C for 3 h. Upon completion, the mixture was filtered and the filtrate was evaporated. The residue was purified by reverse phase flash chromatography (water (0.1% FA)/MeCN) to give *tert*-butyl 2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-3-methyl-5,6-dihydroimidazo[1,2-*a*]pyrazine-7(8*H*)-carboxylate (120 mg, 90% yield) as a yellow solid.

m/z ES+ $[M+H]^+$ 668.3.

**Steps d-e.** These 2 steps were conducted in a similar manner to **Example 39,** steps b-c.

m/z ES+ $[M+H]^+$ 622.2; $^1H$ NMR (400 MHz, $CDCl_3$) δ ppm 8.36 (s, 1H), 7.34 - 7.30 (m, 1H), 7.27 - 7.24 (m, 2H),

7.09 (s, 1H), 6.75 - 6.60 (m, 1H), 6.43 (d, *J* = 16.4 Hz, 1H), 5.86 (d, *J* = 11.2 Hz, 1H), 4.86 (s, 2H), 4.81 - 4.74 (m, 1H), 4.65 - 4.47 (m, 1H), 4.24 - 4.01 (m, 3H), 3.91 - 3.83 (m, 2H), 3.65 - 3.55 (m, 1H), 3.42 (s, 3H), 3.17 - 2.99 (m, 1H), 2.81 - 2.65 (m, 2H), 2.54 (s, 3H), 2.43 (s, 3H), 2.24 - 2.17 (m, 1H), 2.15 (s, 3H).

**Example 75**

**(3a*R*,11a*S*)-5-(2-(4-(1-acryloylazetidin-3-yl)piperazin-1-yl)-2-oxoethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

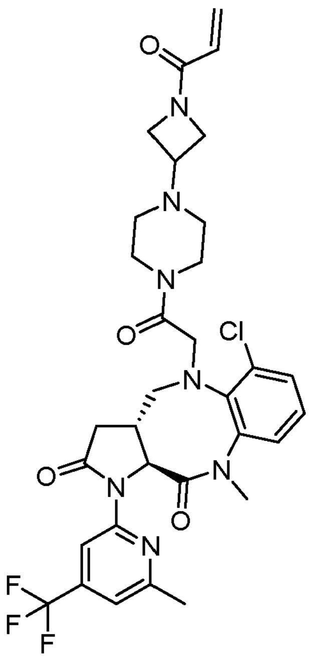

The title compound was prepared in a similar manner to **Example 51,** using **Intermediate 9b** in step a.

m/z ES+ $[M+H]^+$ 674.3; $^1$H NMR (400 MHz, $CD_3OD$) δ ppm 8.30 (s, 1H), 7.59 - 7.49 (m, 2H), 7.46 - 7.36 (m, 1H), 7.22 (s, 1H), 6.42 - 6.23 (m, 2H), 5.85 - 5.76 (m, 1H), 4.70 (d, *J* = 9.6 Hz, 1H), 4.67 - 4.59 (m, 2H), 4.41 - 4.30 (m, 2H), 4.27 - 4.17 (m, 1H), 4.15 - 3.68 (m, 6H), 3.62 (dd, *J* = 14.4, 4.4 Hz, 1H), 3.41 (s, 6H), 3.30 - 3.22 (m, 1H), 3.17 - 3.13 (m, 1H), 3.09 - 2.94 (m, 1H), 2.68 (dd, *J* = 16.8, 8.4 Hz, 1H), 2.54 (s, 3H), 2.38 (dd, *J* = 16.8, 11.6 Hz, 1H).

**Example 76a: (3a*R*,11a*S*)-5-(2-((*R*/*S*)-8-acryloyloctahydro-2*H*-pyrazino[1,2-*a*]pyrazin-2-yl)-2-oxoethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione and**

**Example 76b: (3a*R*,11a*S*)-5-(2-((*S*/*R*)-8-acryloyloctahydro-2*H*-pyrazino[1,2-*a*]pyrazin-2-yl)-2-oxoethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

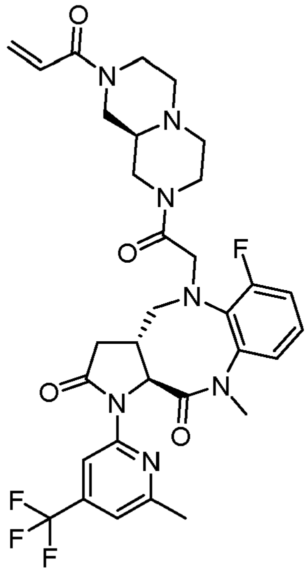

and

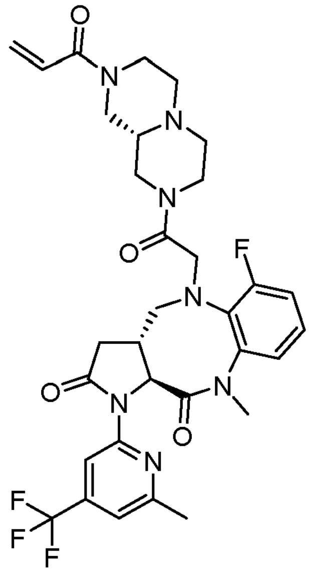

The title compounds were prepared in a similar manner to **Example 51,** using *tert*-butyl octahydro-2*H*-pyrazino [1,2-*a*]pyrazine-2-carboxylate (CAS: 1159825-34-9) in step a, and an additional chiral SFC step was conducted after step a.

**Example 76a:**

m/z ES+ $[M+H]^+$ 658.2; $^1H$ NMR (400 MHz, $CDCl_3$) δ ppm 8.33 (s, 1H), 7.43 - 7.32 (m, 1H), 7.23 (d, *J* = 8.0 Hz, 1H), 7.16 (t, *J* = 9.2 Hz, 1H), 7.09 - 7.02 (m, 1H), 6.62 - 6.44 (m, 1H), 6.30 (d, *J* = 16.4 Hz, 1H), 5.74 (d, *J* = 10.4 Hz, 1H), 4.76 - 4.35 (m, 3H), 4.03 - 3.52 (m, 5H), 3.47 - 3.28 (m, 4H), 3.16 - 2.64 (m, 7H), 2.57 - 2.37 (m, 4H), 2.34 - 1.86 (m, 4H).

**Example 76b:**

m/z ES+ $[M+H]^+$ 658.3; $^1H$ NMR (400 MHz, $CDCl_3$) δ ppm 8.33 (s, 1H), 7.44 - 7.34 (m, 1H), 7.27 - 7.13 (m, 2H), 7.06 (s, 1H), 6.65 - 6.45 (m, 1H), 6.40 - 6.23 (m, 1H), 5.87 - 5.62 (m, 1H), 4.79 - 4.43 (m, 3H), 4.20 - 3.57 (m, 6H), 3.40 (m, 4H), 3.10 - 2.68 (m, 7H), 2.49 (s, 4H), 2.37 - 2.12 (m, 3H).

**Example 77**

**(3a*R*,11a*S*)-5-(3-((1-(2-((dimethylamino)methyl)acryloyl)azetidin-3-yl)oxy)propyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

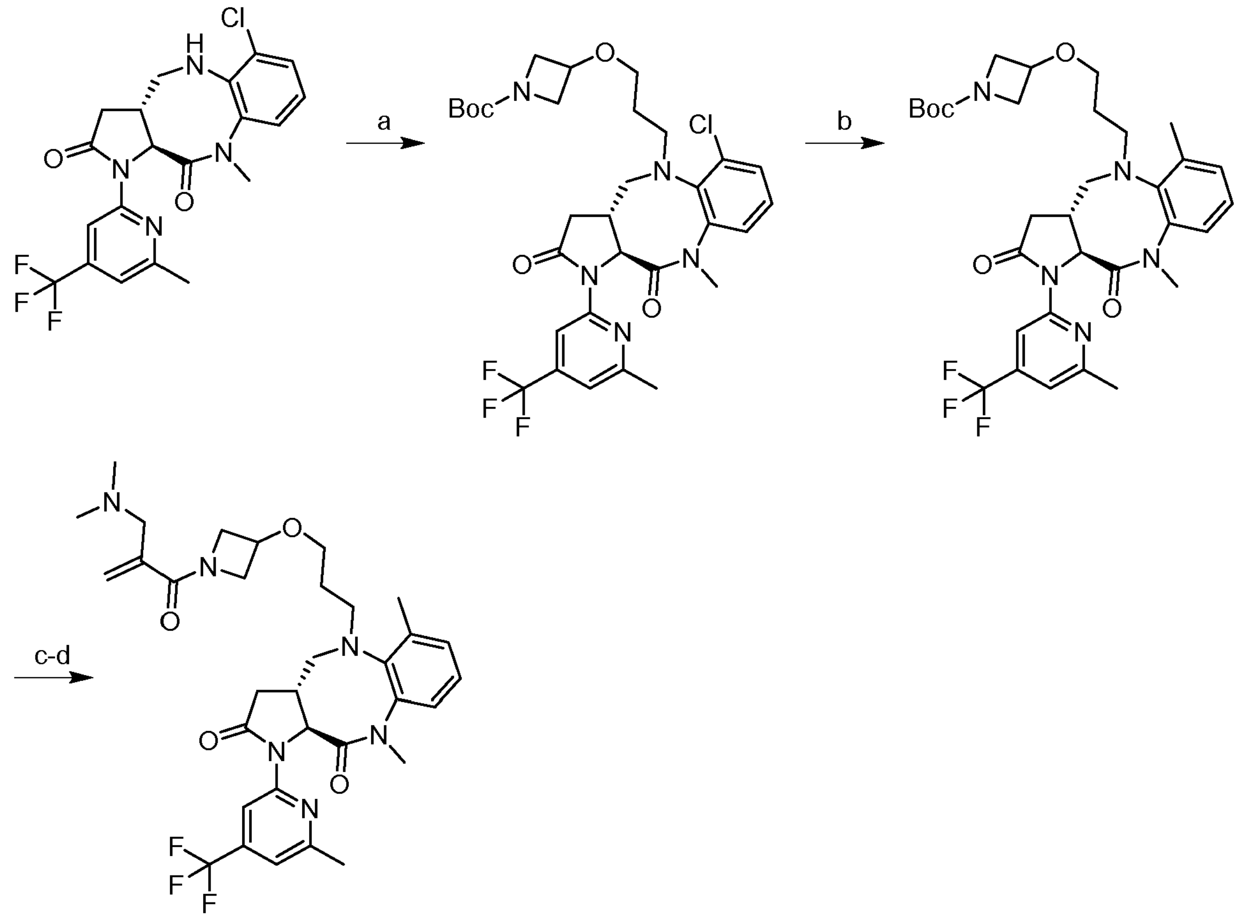


**Step a.** This step was conducted in a similar manner to **Example 39,** step a, using **Intermediate 5b** and **Intermediate B4.**

**Step b.** A mixture of *tert*-butyl 3-(3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propoxy)azetidine-1-carboxylate (300 mg, 0.46 mmol), methylboronic acid (551 mg, 9.20 mmol), XPhos-Pd-G2 (36.2 mg, 0.046 mmol) and $Cs_2CO_3$ (375 mg, 1.15 mmol) in toluene (5 mL) was degassed and purged with $N_2$ 3 times. The reaction mixture was stirred at 110 °C for 12 h under a $N_2$ atmosphere. Upon completion, the mixture was filtered, evaporated and purified by reverse phase flash chromatography (water (0.1% TFA)/MeCN) to give *tert*-butyl 3-(3-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)propoxy)azetidine-1-carboxylate (310 mg, 85% yield) as a white solid.

m/z ES+ $[M+H]^+$632.2.

**Steps c-d.** These 2 steps were conducted in a similar manner to **Example 27,** steps b-c, using **Intermediate E1** in step d.

m/z ES+ $[M+H]^+$ 643.3; $^1$H NMR (400 MHz, $CDCl_3$) δ ppm 8.33 (s, 1H), 7.27 - 7.21 (m, 3H), 7.04 (s, 1H), 5.61 (s, 1H), 5.49 (s, 1H), 4.70 (d, *J* = 9.6 Hz, 1H), 4.37 - 4.33 (m, 1H), 4.25 - 4.20 (m, 2H), 4.10 - 4.01 (m, 1H), 3.95 - 3.79 (m, 1H), 3.60 - 3.56 (m, 1H), 3.42 - 3.27 (m, 6H), 3.13 - 2.94 (m, 4H), 2.84 - 2.69 (m, 2H), 2.49 (s, 3H), 2.37 (s, 3H), 2.29 (s, 6H), 2.23 - 2.16 (m, 1H), 1.69 - 1.62 (m, 2H).

## Example 78

**2-(3-(3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propoxy)azetidine-1-carbonyl)allyl acetate**

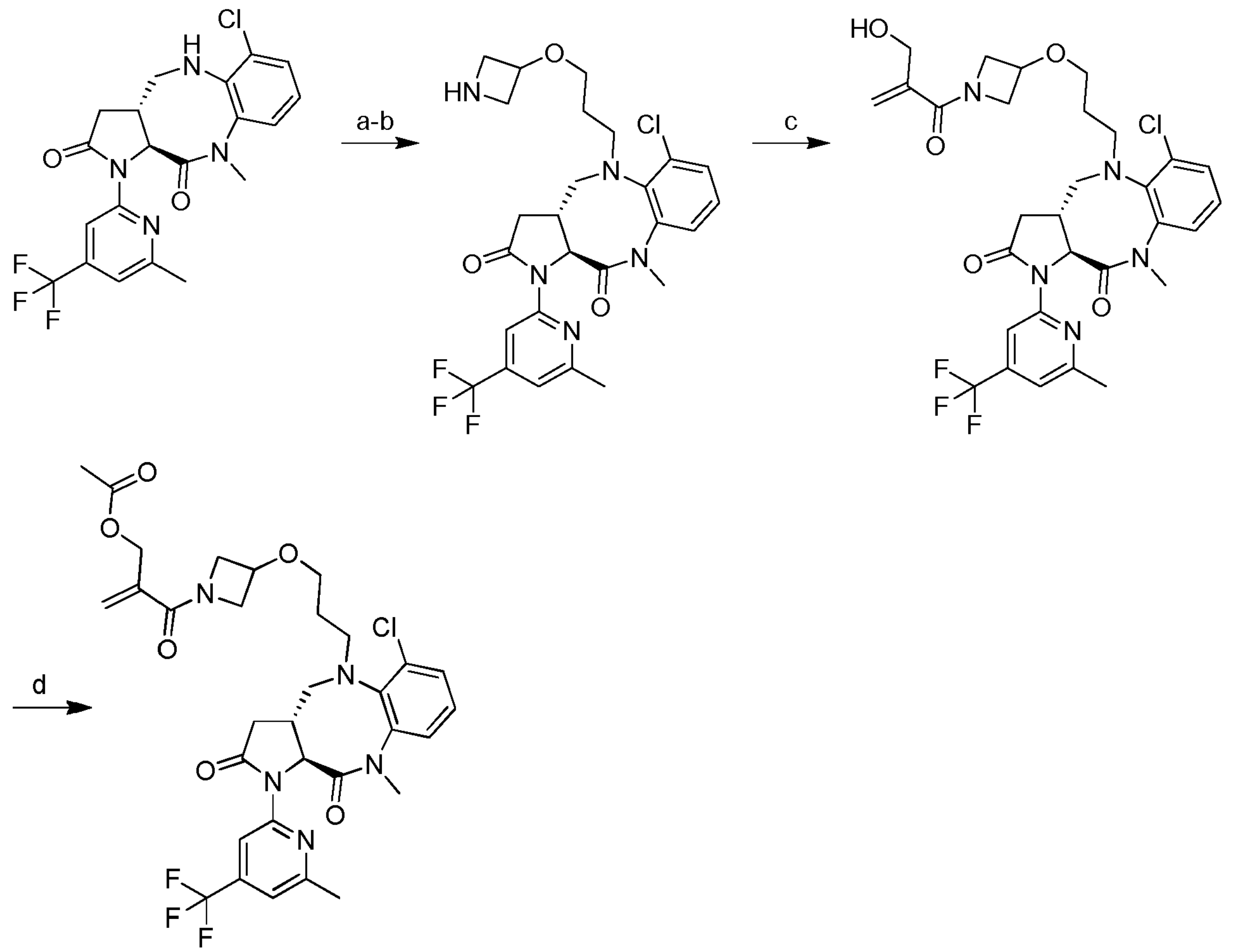


**Steps a-b.** These 2 steps were conducted in a similar manner to **Example 39,** steps a-b, using **Intermediate 5b** and **Intermediate B4** in step a.

**Step c.** This step was conducted in a similar manner to **Example 77,** step d, using **Intermediate E2.**

**Step d.** Acetyl chloride (9 mg, 0.11 mmol) was added to a solution of (3a*R*,11a*S*)-6-chloro-5-(3-((1-(2-(hydroxymethyl)acryloyl)azetidin-3-yl)oxy)propyl)-10-methyl-1-(6-methyl-4-(trifluoromethyl)pyridin-2-yl)-1 ,3a,4,5, 1 0, 11a-hexahydro-2*H*-benzo[*b*]pyrrolo[2,3-*f*][1,4]diazocine-2,11(3*H*)-dione (40 mg, 0.063 mmol) and TEA (26 mg, 0.25 mmol) in THF (3 mL) at 0 °C. The reaction mixture was stirred at 20 °C for 2 h. Upon completion, the mixture was diluted with water (20 mL), extracted with EtOAc (20 mL) and evaporated. Purification by by Prep-TLC (EtOAc) afforded the title compound (14 mg, 33% yield) as a white solid.

m/z ES+ $[M+H]^+$ 678.2; $^1$H NMR (400 MHz, $CDCl_3$) δ ppm 8.33 (s, 1H), 7.45 (dd, *J* = 7.2, 2.0 Hz, 1H), 7.35 - 7.28 (m, 2H), 7.05 (s, 1H), 5.64 (s, 1H), 5.50 (s, 1H), 4.77 (s, 2H), 4.63 (d, *J* = 9.2 Hz, 1H), 4.39 - 4.17 (m, 3H), 4.10 - 3.78 (m, 2H), 3.62 - 3.51 (m, 1H), 3.44 (t, *J* = 5.2 Hz, 2H), 3.38 (s, 3H), 3.20 - 3.07 (m, 2H), 3.05 - 2.87 (m, 2H), 2.74 (dd, *J* = 16.8, 8.0 Hz, 1H), 2.48 (s, 3H), 2.23 (dd, *J* = 16.8, 11.2 Hz, 1H), 2.10 (s, 3H), 1.64 - 1.55 (m, 2H).

**Example 79**

**(3a*R*,11a*S*)-5-(3-((1-((*Z*)-2-chloro-4-(dimethylamino)but-2-enoyl)azetidin-3-yl)oxy)propyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4] diazocine-2,11(3*H*)-dione**

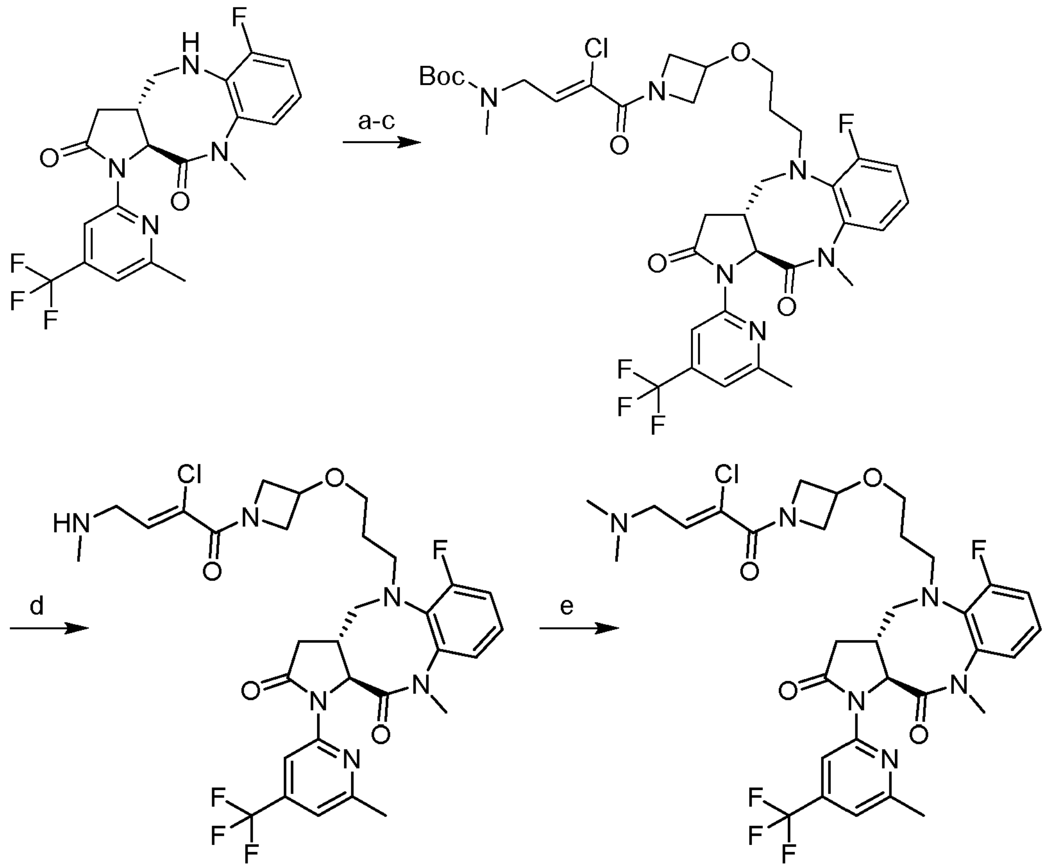


**Steps a-c**. These 3 steps were conducted in a similar manner to **Example 78,** steps a-c, using **Intermediate 5a** and **Intermediate B4** in step a, and **Intermediate E3** in step c.

**Step d.** To a solution of tert-butyl ((*Z*)-3-chloro-4-(3-(3-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propoxy) azetidin-1-yl)-4-oxobut-2-en-1-yl)(methyl)carbamate (30 mg, 0.039 mmol) in DCM (0.5 mL) was added TFA (0.1 mL). The mixture was stirred at 25 °C for 1 h. Upon completion, the reaction mixture was evaporated. The residue was diluted with sat. aq. $K_2CO_3$ (5 mL) and extracted with EtOAc (3 x 3 mL). The combined organic layers were washed with brine, dried over $Na_2SO_4$, and evaporated to give (3a*R*,11a*S*)-5-(3-((1-((*Z*)-2-chloro-4-(methylamino)but-2-enoyl)azetidin-3-yl)oxy) propyl)-6-fluoro-10-methyl-1-(6-methyl-4-(trifluoromethyl)pyridin-2-yl)-1,3a,4,5,10,11a-hexahydro-2*H*-benzo[*b*]pyrrolo [2,3-*f*][1,4]diazocine-2,11(3*H*)-dione (30 mg, crude) as light yellow oil.

m/z ES+ $[M+H]^+$667.0.

**Step e.** To a solution of (3a*R*,11a*S*)-5-(3-((1-((*Z*)-2-chloro-4-(methylamino)but-2-enoyl)azetidin-3-yl)oxy)propyl)-6-fluoro-10-methyl-1-(6-methyl-4-(trifluoromethyl)pyridin-2-yl)-1,3a,4,5,10,11a-hexahydro-2*H*-benzo[*b*]pyrrolo [2,3-*f*][1,4]diazocine-2,11(3*H*)-dione (25 mg, 0.038 mmol) in MeOH (0.5 mL) was added acetic acid (2 mg, 0.038 mmol), $NaBH_3CN$ (5 mg, 0.075 mmol) and formaldehyde (37 wt.% in $H_2O$; 9 mg, 0.11 mmol). The mixture was stirred at 25 °C for 16 h. Upon completion, the reaction mixture was evaporated and the residue was purified by Prep-TLC (EtOAc/MeOH = 9/1) followed by Prep-HPLC to give the title compound (1.4 mg, 5% yield) as a yellow solid.

m/z ES+ $[M+H]^+$ 681.2; $^1$H NMR (400 MHz, $CDCl_3$) δ ppm 8.32 (s, 1H), 7.37 - 7.31 (m, 1H), 7.22 (d, *J* = 7.6 Hz, 1H), 7.18 - 7.11 (m, 1H), 7.06 (s, 1H), 6.71 (t, *J* = 6.4 Hz, 1H), 4.62 (d, *J* = 9.2 Hz, 1H), 4.57 - 4.44 (m, 1H), 4.31 - 4.13 (m, 3H), 3.97 - 3.84 (m, 1H), 3.57 - 3.48 (m, 1H), 3.40 (t, *J* = 6.4 Hz, 2H), 3.35 (s, 3H), 3.24 - 3.11 (m, 4H), 2.97 - 2.81 (m, 2H), 2.75 - 2.69 (m, 1H), 2.50 (s, 3H), 2.30 - 2.24 (m, 6H), 2.23 - 2.13 (m, 1H), 1.39 - 1.24 (m, 2H).

**Example 80**

**(3a*R*,11a*S*)-5-(3-((1-((*E*)-2-((dimethylamino)methyl)but-2-enoyl)azetidin-3-yl)oxy)propyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4] diazocine-2,11(3*H*)-dione**

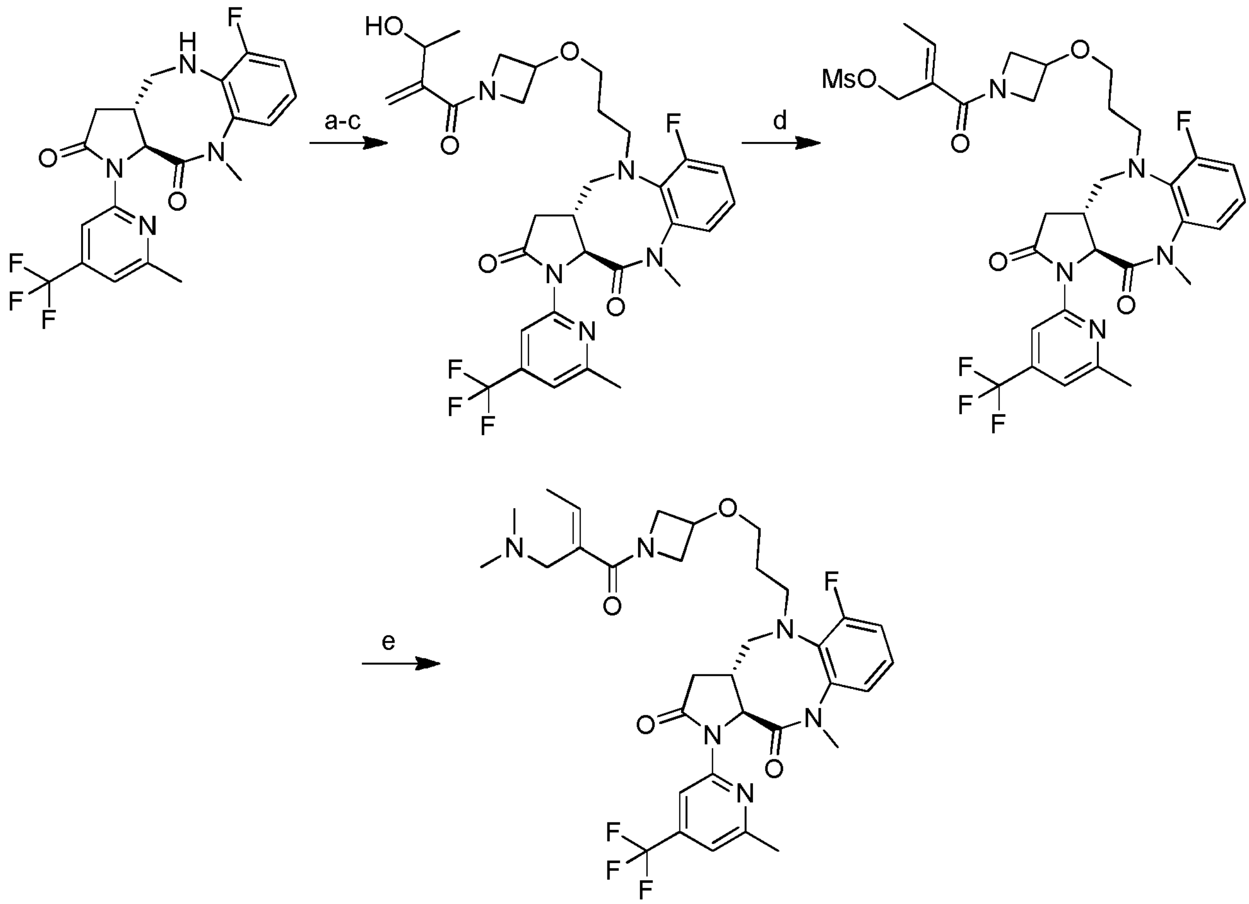


**Steps a-c**. These 3 steps were conducted in a similar manner to **Example 78,** steps a-c, using **Intermediate 5a** and **Intermediate B4** in step a, and **Intermediate E4** in step c.

**Step d.** To a mixture of (3a*R*,11a*S*)-6-fluoro-5-(3-((1-(3-hydroxy-2-methylenebutanoyl)azetidin-3-yl)oxy)propyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (150 mg, 0.24 mmol) and TEA (479 mg, 4.73 mmol) in DCM (10 mL) was added methanesulfonic anhydride (206 mg, 1.18 mmol). The mixture was stirred at 25 °C for 1 h under a $N_2$ atmosphere. Upon completion, the reaction mixture was diluted with DCM (50 mL) and washed with water (3 x 15 mL), dried over $Na_2SO_4$ and evaporated to give (*E*)-2-(3-(3-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propoxy)azetidine-1-carbonyl)but-2-en-1-yl methanesulfonate (150 mg, crude) as a yellow oil.

m/z ES+ $[M+H]^+$ 712.2.

**Step e.** To a mixture of (*E*)-2-(3-(3-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)propoxy)azetidine-1-carbonyl)but-2-en-1-yl methanesulfonate (150 mg, 0.21 mmol) in THF (2 mL) was added dimethylamine (2 M in THF, 0.53 mL). The mixture was stirred at 25 °C for 30 min under a $N_2$ atmosphere. Upon completion, the reaction mixture was evaporated and purified by Prep-HPLC to give the title compound (9 mg, 6% yield) as an off-white solid.

m/z ES+ $[M+H]^+$ 661.3; $^1H$ NMR (400 MHz, $CDCl_3$) δ ppm 8.33 (s, 1H), 7.37 - 7.33 (m, 1H), 7.23 (d, *J* = 8.0 Hz, 1H), 7.19 - 7.13 (m, 1H), 7.07 (s, 1H), 6.20 - 6.11 (m, 1H), 4.63 (d, *J* = 8.8 Hz, 1H), 4.38 - 4.19 (m, 3H), 4.10 - 3.99 (m, 1H), 3.93 - 3.80 (m, 1H), 3.59 - 3.51 (m, 1H), 3.43 - 3.33 (m, 6H), 3.23 - 3.14 (m, 3H), 2.97 - 2.84 (m, 2H), 2.79 - 2.69 (m, 1H), 2.51 (s, 3H), 2.31 - 2.17 (m, 7H), 1.83 (d, *J* = 7.2 Hz, 3H), 1.63 - 1.55 (m, 2H).

**Example 81**

**(3a*R*,11a*S*)-5-(3-((1-(2-((dimethylamino)methyl)acryloyl)azetidin-3-yl)oxy)propyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

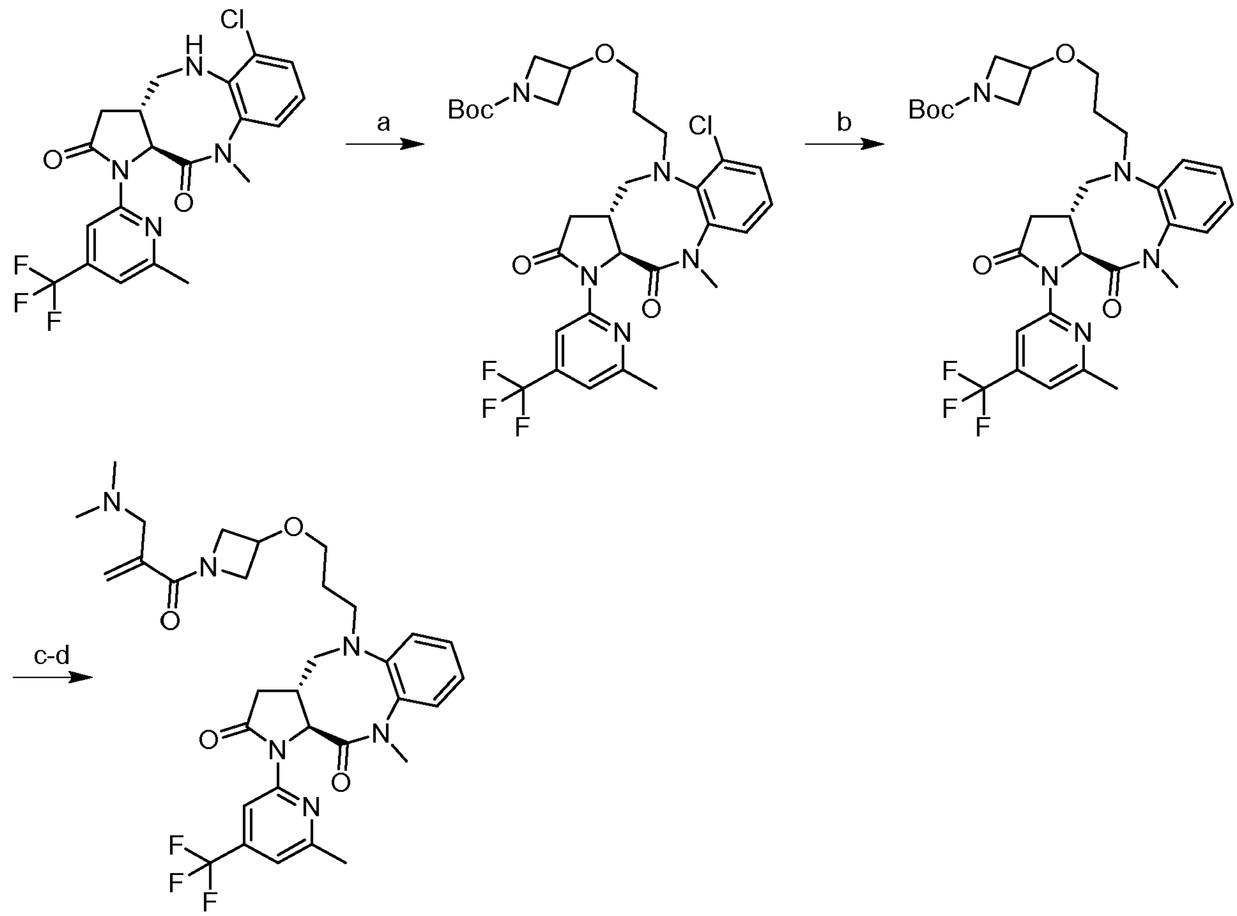


**Step a.** This step was conducted as described in **Example 77,** step a.

**Step b.** To a solution of *tert*-butyl 3-(3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propoxy)azetidine-1-carboxylate (90 mg, 0.14 mmol) in MeOH (2 mL) was added 10% Pd/C (20 mg). The reaction mixture was stirred at 40 °C for 12 hr under a $H_2$ atmosphere (15 psi). Upon completion, the reaction mixture was filtered and evaporated to give *tert*-butyl 3-(3-((3a*R*,11a*S*)-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propoxy)azetidine-1-carboxylate(40 mg, 46% yield) as a white solid.

m/z ES+ $[M+H]^+$618.1.

**Steps c-d.** These 2 steps were conducted in a similar manner to **Example 77,** steps c-d, using **Intermediate E1** in step d.

m/z ES+ $[M+H]^+$ 629.3; $^1H$ NMR (400 MHz, $CDCl_3$) $\delta$ ppm 8.31 (s, 1H), 7.47 - 7.39 (m, 3H), 7.32 - 7.38 (m, 1H), 7.05 (s, 1H), 5.62 (s, 1H), 5.50 (s, 1H), 4.61 (d, *J* = 8.4 Hz, 1H), 4.50 - 4.30 (m, 1H), 4.30 - 4.15 (m, 2H), 4.14 - 3.96 (m, 1H), 3.96 - 3.80 (m, 1H), 3.45 - 3.57 (m, 1H), 3.25 - 3.44 (m, 6H), 3.05 - 3.24 (m, 3H), 2.79 - 2.89 (m, 2H), 2.75 - 2.65 (m, 1H), 2.51 (s, 3H), 2.43 - 2.25 (m, 6H), 2.21 - 2.12 (m, 1H), 1.63 - 1.53 (m, 2H).

**Example 82**

**(3a*R*,11a*S*)-6,10-dimethyl-1-(6-methyl-4-(trifluoromethyl)pyridin-2-yl)-5-(3-((1-(1,2,5,6-tetrahydropyridine-3-carbonyl)azetidin-3-yl)oxy)propyl)-1,3a,4,5,10,11a-hexahydro-2*H*-benzo[*b*]pyrrolo[2,3-*f*][1,4]diazocine-2,11(3*H*)-dione**

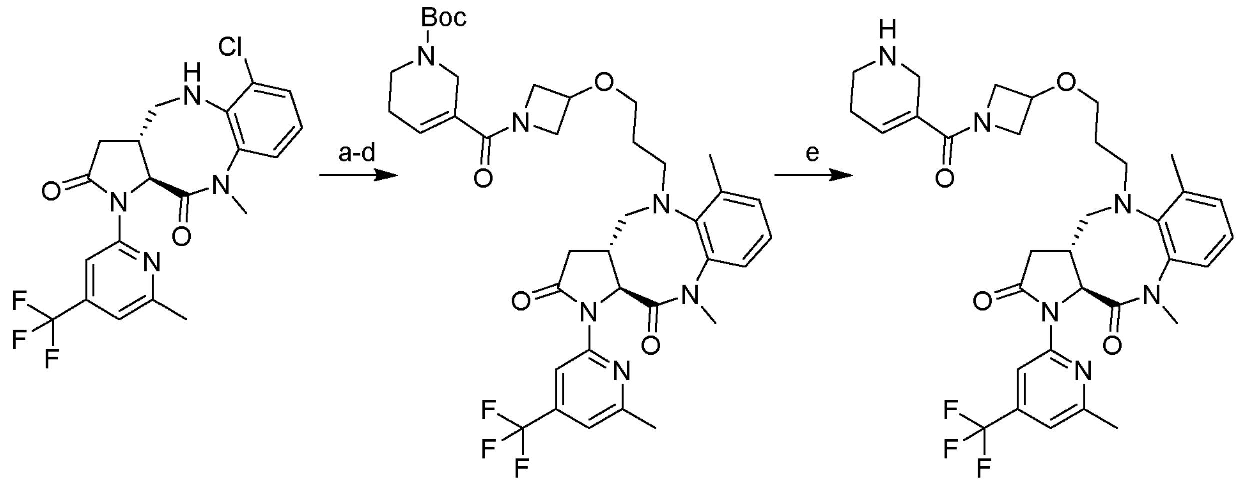


**Steps a-d**. These 4 steps were conducted in a similar manner to **Example 77,** steps a-d, using **Intermediate 5b** and **Intermediate B4** in step a, and 1-(*tert*-butoxycarbonyl)-1,2,5,6-tetrahydropyridine-3-carboxylic acid (CAS: 86447-11-2) in step d.

**Step e.** To a solution of *tert*-butyl 5-(3-(3-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propoxy)azetidine-1-carbonyl)-3,6-dihydropyridine-1(2*H*)-carboxylate (50 mg, 0.067 mmol) in DCM (1 mL) was added TFA (0.3 mL). The reaction mixture was stirred at 20 °C for 30 min. Upon completion, the mixture was evaporated and the residue was purified by reverse phase flash chromatography (water (0.1% $NH_4OH$)/MeCN) to give the title compound (40 mg, 89% yield) as a yellow solid.

m/z ES+ $[M+H]^+$ 641.2; $^1H$ NMR (400 MHz, $CDCl_3$) δ ppm 8.33 (s, 1H), 7.26 - 7.18 (m, 3H), 7.05 (s, 1H), 6.32 (s, 1H), 4.70 (d, *J* = 9.6 Hz, 1H), 4.37 - 4.20 (m, 2H), 3.87 - 3.71 (m, 2H), 3.59 (dd, *J* = 14.4, 4.0 Hz, 1H), 3.43 - 3.30 (m, 6H), 3.23 - 2.96 (m, 9H), 2.86 - 2.65 (m, 3H), 2.49 (s, 3H), 2.37 (s, 3H), 2.20 (dd, *J* = 16.8, 11.6 Hz, 1H), 1.75 - 1.55 (m, 2H).

## Example 83

**(*E*)-*N*-(5-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)pentyl)-4-(dimethylamino)but-2-enamide**

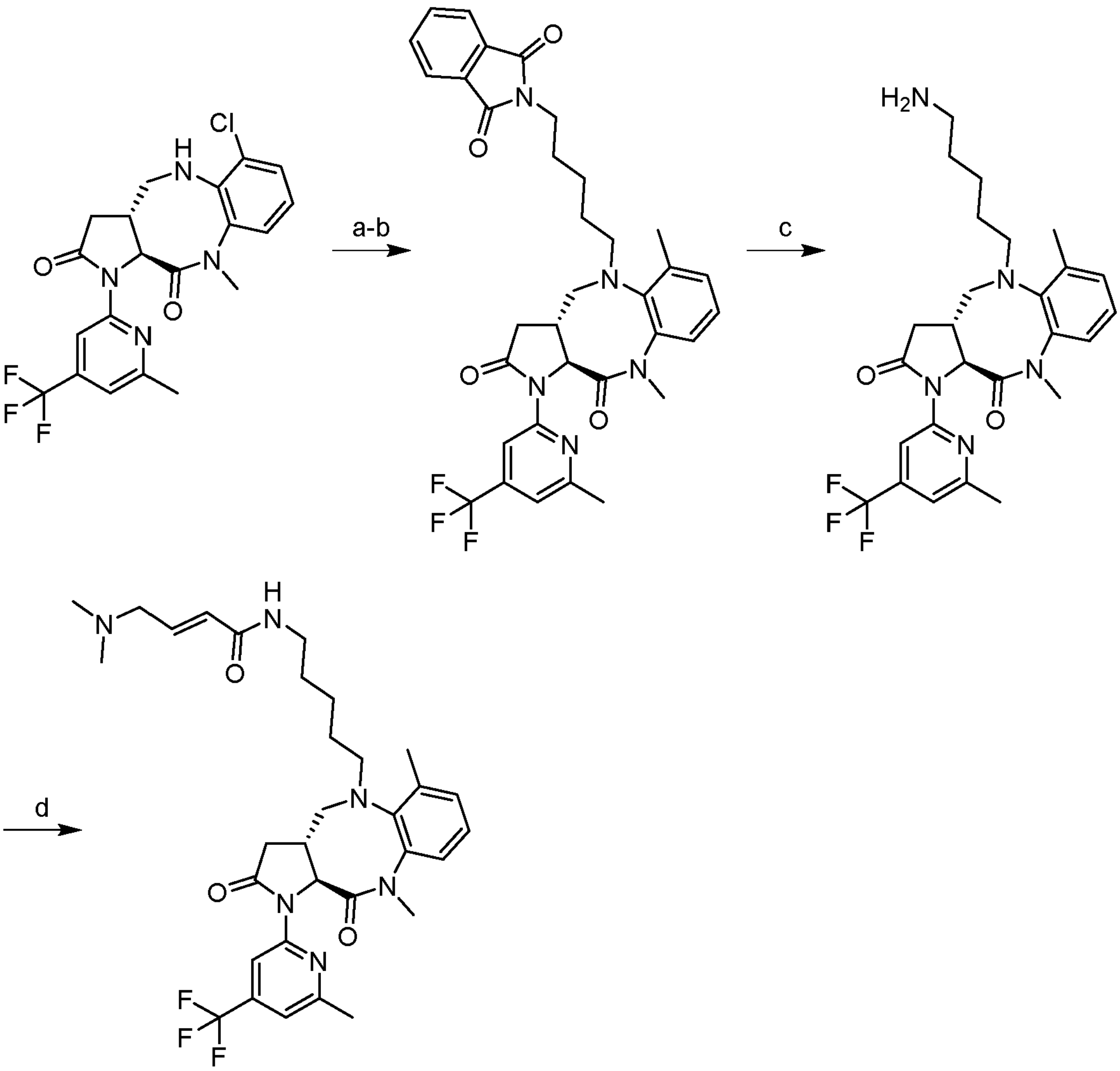


**Steps a-b.** These 2 steps were conducted in a similar manner to **Example 77.** Steps a-b, using **Intermediate 5b** and **Intermediate** B36 in step a.

**Step c.** To a solution of (3a*R*,11a*S*)-5-(5-(1,3-dioxoisoindolin-2-yl)pentyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (140 mg, 0.22 mmol) in EtOH (2.5 mL) was added hydrazine monohydrate (112 mg, 2.21 mmol). The mixture was stirred at 25 °C for 2 h. Upon completion, the reaction mixture was filtered and evaporated to give (3aR,11a*S*)-5-(5-aminopentyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (110 mg, crude) as a yellow solid.

m/z ES+ $[M+H]^+$ 504.0.

**Step d.** This step was conducted in a similar manner to **Example 27,** step c.

m/z ES+ $[M+H]^+$ 615.3; $^1$H NMR (400 MHz, $CDCl_3$) δ ppm 8.34 (s, 1H), 7.25 - 7.20 (m, 3H), 7.06 (s, 1H), 6.84 - 6.76 (m, 1H), 6.10 - 6.00 (m, 2H), 4.67 (d, *J* = 9.2 Hz, 1H), 3.55 (dd, *J* = 14.0, 4.4 Hz, 1H), 3.45 - 3.36 (m, 1H), 3.35 (s, 3H), 3.24 - 3.17 (m, 1H), 3.17 - 3.07 (m, 2H), 3.06 - 2.98 (m, 1H), 2.96 - 2.85 (m, 2H), 2.81 (d, *J* = 14.0 Hz, 1H), 2.71 (dd, *J* = 16.8, 8.4 Hz, 1H), 2.51 (s, 3H), 2.37 (s, 3H), 2.32 (s, 6H), 2.18 (dd, *J* = 16.8, 11.6 Hz, 1H), 1.54 - 1.28 (m, 6H).

**Example 84**

***N*-(5-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)pentyl)-2-((dimethylamino)methyl)acrylamide**

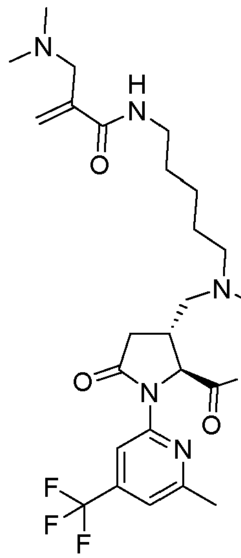

The title compound was prepared in a similar manner to **Example 83,** using **Intermediate E1** in step d.

m/z ES+ $[M+H]^+$ 615.3; $^1H$ NMR (400 MHz, $CDCl_3$) δ ppm 9.09 (br s, 1H), 8.33 (s, 1H), 7.25 - 7.18 (m, 3H), 7.04 (s, 1H), 6.20 (d, *J* = 1.6 Hz, 1H), 5.37 (s, 1H), 4.70 (d, *J* = 9.6 Hz, 1H), 3.58 (dd, *J* = 14.4, 4.4 Hz, 1H), 3.35 (s, 3H), 3.30 - 3.21 (m, 2H), 3.09 (s, 2H), 3.00 - 2.85 (m, 3H), 2.82 - 2.68 (m, 2H), 2.49 (s, 3H), 2.37 (s, 3H), 2.23 - 2.15 (m, 7H), 1.53 - 1.45 (m, 2H), 1.44 - 1.35 (m, 2H), 1.35 - 1.29 (m, 2H).

**Example 85**

***N*-(2-(2-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethoxy)ethyl)-2-((dimethylamino)methyl)acrylamide**

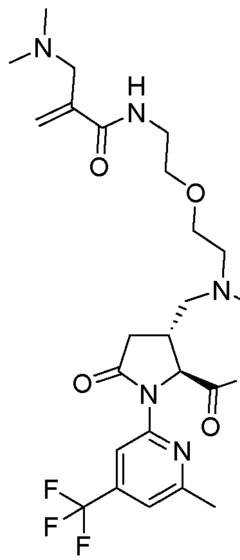

The title compound was prepared in a similar manner to **Example 83,** using **Intermediate B35** in step a, and **Intermediate E1** in step d.

m/z ES+ $[M+H]^+$ 617.3; $^1H$ NMR (400 MHz, DMSO-$d_6$) δ ppm 8.83 - 8.70 (m, 1H), 8.17 (s, 1H), 7.35 (s, 1H), 7.32 - 7.21 (m, 3H), 5.86 (d, *J* = 2.0 Hz, 1H), 5.39 (s, 1H), 4.62 (d, *J* = 9.2 Hz, 1H), 3.55 - 3.47 (m, 1H), 3.39 - 3.36 (m, 2H), 3.32 - 3.29 (m, 2H), 3.29 - 3.24 (m, 2H), 3.24 (s, 3H), 3.11 - 3.05 (m, 1H), 3.04 (s, 2H), 2.93 - 2.74 (m, 3H), 2.59 - 2.55 (m, 1H), 2.48 (s, 3H), 2.42 - 2.36 (m, 1H), 2.33 (s, 3H), 2.10 (s, 6H).

**Example 86**

**(3a*R*,11a*S*)-6-chloro-5-(3-(4-(2-((dimethylamino)methyl)acryloyl)piperazin-1-yl)-3-oxopropyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

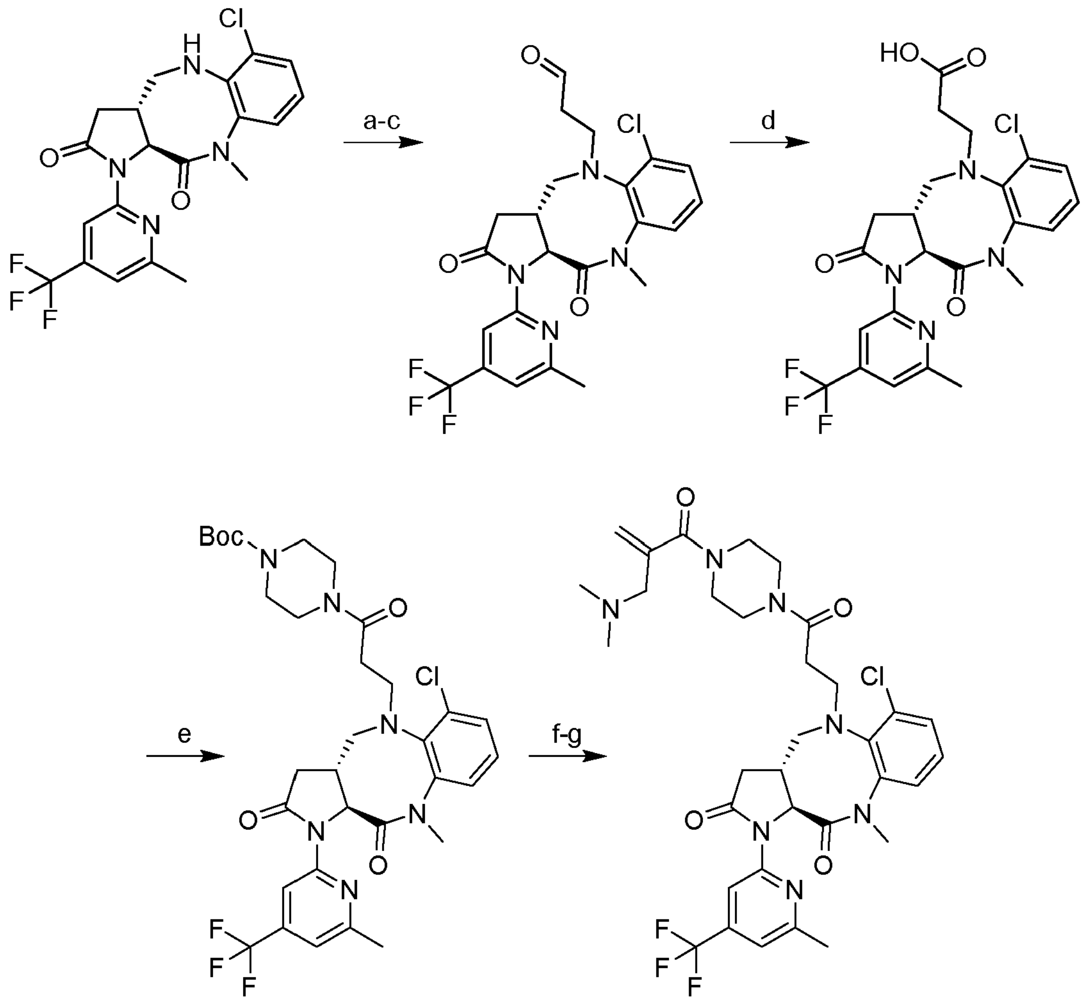


**Steps a-c**. These 3 steps were conducted in a similar manner to **Intermediate 13,** steps a-c, using **Intermediate 5b** in step a.

**Step d.** To a solution of 3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propanal (360 mg, 0.73 mmol), $NaH_2PO_4$ (261 mg, 2.18 mmol) and 2-methylbut-2-ene (714 mg, 10.1 mmol) in tert-butanol (4 mL) and $H_2O$ (4 mL) was added sodium chlorite (197 mg, 2.18 mmol). The mixture was stirred at 20 °C for 1 h. Upon completion, the reaction mixture was acidified to pH 5 with the slow addition of 0.5 M HCl. The aqueous mixture was extracted with EtOAc (3 x 10 mL). The combined organic layers were washed with brine (30 mL), dried over $Na_2SO_4$ and evaporated. The residue was purified by reverse phase flash chromatography (water (0.1% FA)/MeCN) to give 3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propanoic acid (160 mg, 43% yield) as a white solid.

m/z ES+ $[M+H]^+$ 511.0.

**Step e.** HATU (167 mg, 0.44 mmol) was added to a solution of 3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propanoic acid (150 mg, 0.29 mmol), tert-butyl piperazine-1-carboxylate (109 mg, 0.59 mmol) and DIPEA (76 mg, 0.59 mmol) in DCM (3 mL) at 20 °C. The reaction mixture was stirred for 1 h. Upon completion, the mixture was evaporated and the residue was purified by reverse phase flash chromatography (water (0.1% $NH_4OH$)/MeCN) to give *tert*-butyl

4-(3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propanoyl)piperazine-1-carboxylate (90 mg, 45% yield) as a yellow oil.

m/z ES+ $[M+H]^+$ 679.3.

**Steps f-g.** These 2 steps were conducted in a similar manner to **Example 77,** steps c-d.

m/z ES+ $[M+H]^+$ 690.2; $^1H$ NMR (400 MHz, $CDCl_3$) δ ppm 8.32 (s, 1H), 7.47 - 7.43 (m, 1H), 7.36 - 7.28 (m, 2H), 7.05 (s, 1H), 6.19 (s, 1H), 5.73 (s, 1H), 4.58 (d, *J* = 9.2 Hz, 1H), 3.88 (s, 2H), 3.82 - 3.61 (m, 7H), 3.55 (s, 2H), 3.50 - 3.40 (m, 2H), 3.38 (s, 3H), 3.03 - 2.92 (m, 1H), 2.84 (s, 6H), 2.75 (dd, *J* = 16.8, 8.4 Hz, 1H), 2.48 (s, 3H), 2.47 - 2.28 (m, 2H), 2.25 - 2.01 (m, 2H).

## Example 87

**N-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)acrylamide**

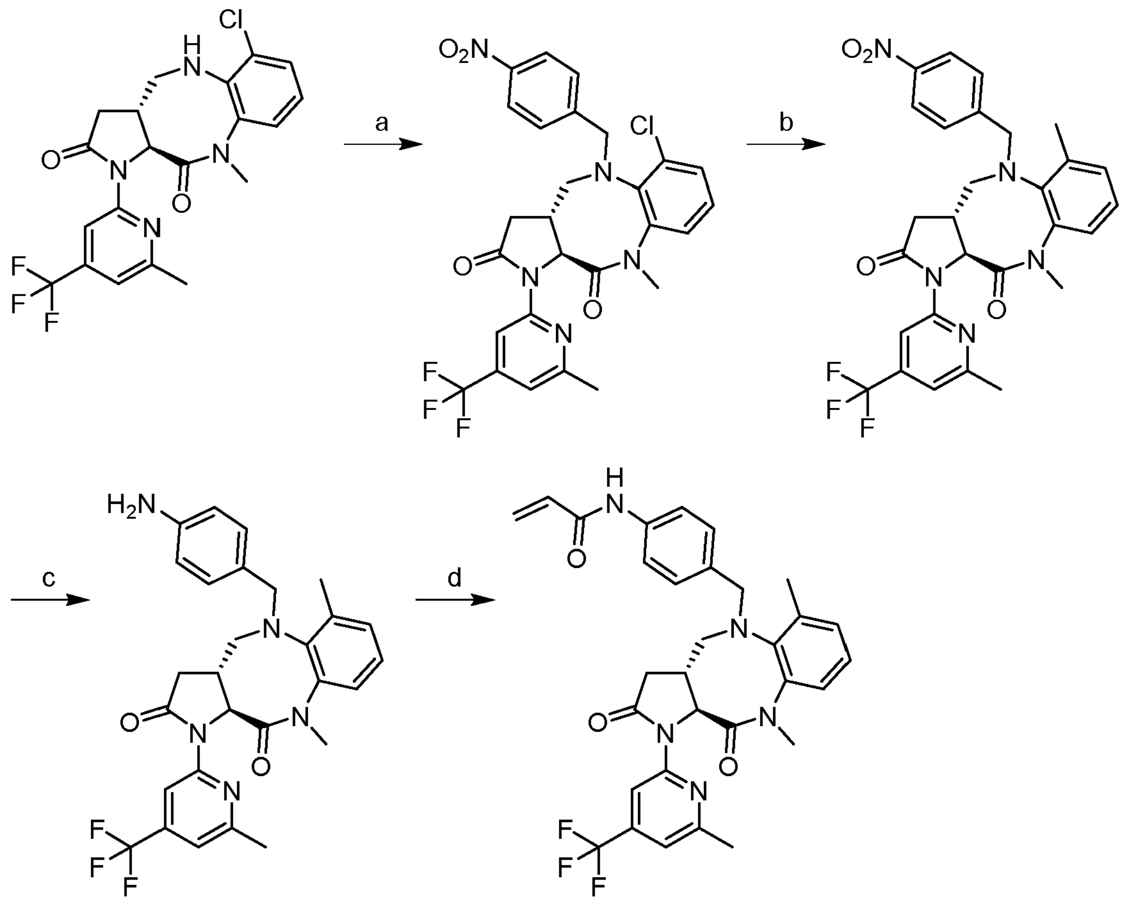


**Step a.** This step was conducted in a similar manner to **Example 39,** step a, using **Intermediate 5b** and 4-nitrobenzaldehyde (CAS: 555-16-8).

**Step b.** This step was conducted in a similar manner to **Example 77,** step b.

**Step c.** To a solution of (3a*R*,11a*S*)-6,10-dimethyl-1-(6-methyl-4-(trifluoromethyl)pyridin-2-yl)-5-(4-nitrobenzyl)-1,3a,4,5,10,11a-hexahydro-2*H*-benzo[*b*]pyrrolo[2,3-*f*][1,4]diazocine-2,11(3*H*)-dione (100 mg, 0.18 mmol) in EtOAc (3 mL) was added 3% Pt-V/C (50 mg, 0.006 mmol). The rection mixture was stirred at 40 °C for 12 hr under a $H_2$ atmosphere (15 psi).

Upon completion, the reaction mixture was filtered and evaporated to give (3a*R*,11a*S*)-5-(4-aminobenzyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (80 mg, 85% yield) as a yellow gum.

m/z ES+ $[M+H]^+$ 524.2.

**Step d.** This step was conducted in a similar manner to **Example 27,** step c, using acrylic acid.

m/z ES+ $[M+H]^+$ 578.2; $^1H$ NMR (400MHz, $CDCl_3$) δ ppm 8.34 (s, 1H), 7.71 (s, 1H), 7.60 (d, *J* = 7.6 Hz, 2H), 7.34 (d, *J* = 8.4 Hz, 2H), 7.31 - 7.27 (m, 2H), 7.27 - 7.24 (m, 1H), 7.08 (s, 1H), 6.51 - 6.40 (m, 1H), 6.31 - 6.19 (m, 1H), 5.76 (d, *J* = 10.4 Hz, 1H), 4.78 (d, *J* = 9.6 Hz, 1H), 4.15 (d, *J* = 13.6 Hz, 1H), 3.99 (d, *J* = 13.6 Hz, 1H), 3.49 - 3.39 (m, 4H), 3.07 - 2.91 (m, 1H), 2.79 - 2.68 (m, 1H), 2.62 (dd, *J* = 16.8, 8.8 Hz, 1H), 2.53 (s, 3H), 2.49 (s, 3H), 2.20 - 2.10 (m, 1H).

**Example 88**

***N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-1,2,5,6-tetrahydropyridine-3-carboxamide**

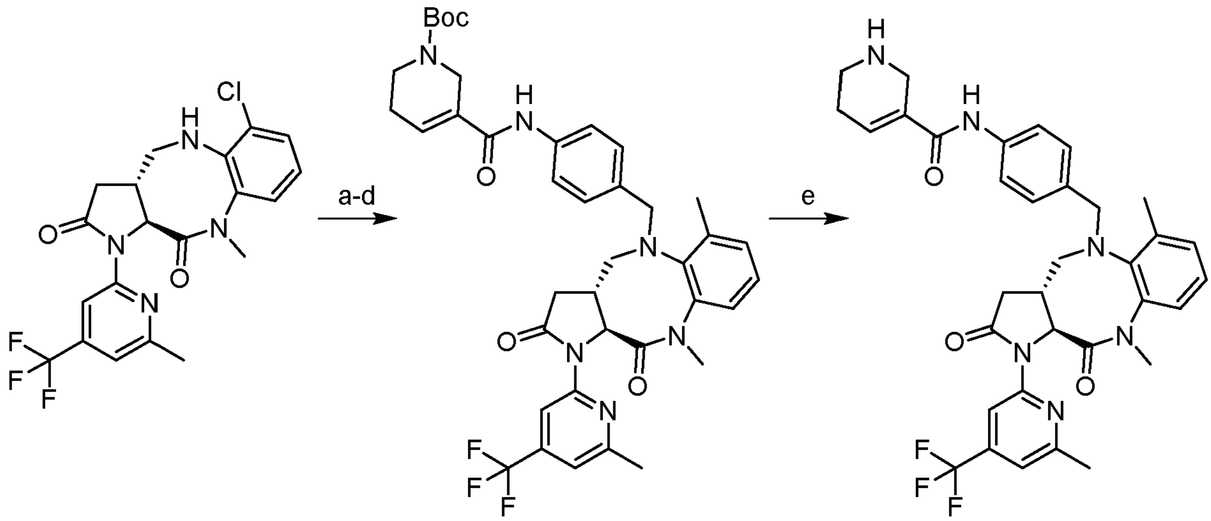


**Steps a-d.** These 4 steps were conducted in a similar manner to **Example 87,** steps a-d, using **Intermediate 5b** and 4-nitrobenzaldehyde (CAS: 555-16-8) in step a, 1-(*tert*-butoxycarbonyl)-1,2,5,6-tetrahydropyridine-3-carboxylic acid (CAS: 86447-11-2) in step d.

**Step e.** This step was conducted in a similar manner to **Example 82,** step e.

m/z ES+ $[M+H]^+$ 633.2; $^1H$ NMR (400 MHz, $CDCl_3$) δ ppm 8.31 (s, 1H), 7.87 (br s, 1H), 7.58 - 7.48 (m, 2H), 7.36 - 7.28 (m, 2H), 7.27 - 7.19 (m, 3H), 7.04 (s, 1H), 6.72 (s, 1H), 4.78 - 4.69 (m, 1H), 4.18 - 4.06 (m, 1H), 3.98 - 3.95 (m, 1H), 3.78 - 3.71 (m, 1H), 3.47 - 3.31 (m, 4H), 3.08 - 2.91 (m, 2H), 2.75 - 2.57 (m, 2H), 2.50 (s, 3H), 2.46 (s, 3H), 2.39 - 2.31 (m, 2H), 2.20 - 2.08 (m, 4H).

**Example 89**

**(*E*)-*N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-2-((dimethylamino)methyl)but-2-enamide**

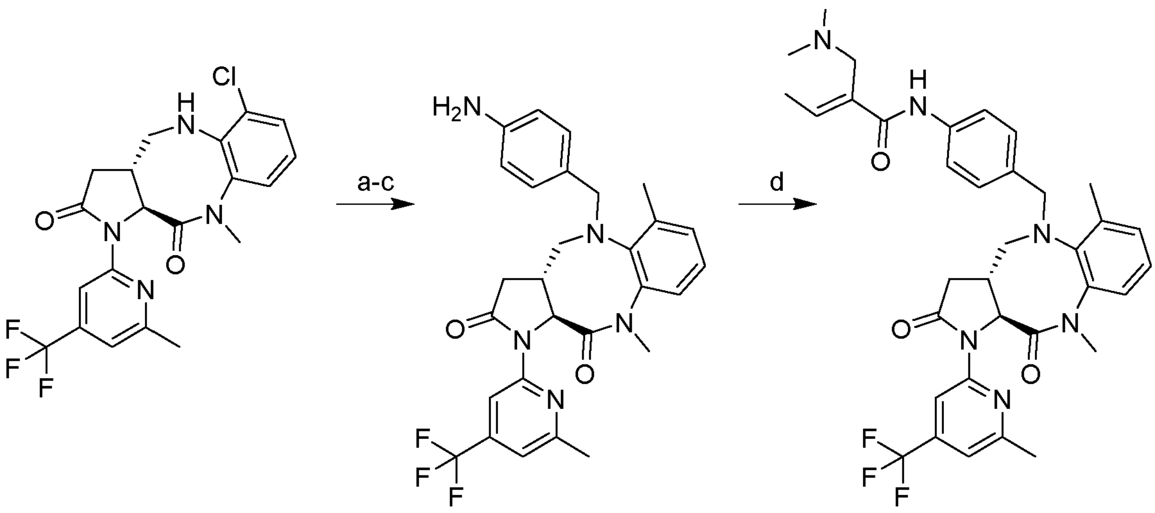


**Steps a-c**. These 3 steps were conducted as described in **Example 87,** steps a-c.

**Step d.** A mixture of (3a*R*,11a*S*)-5-(4-aminobenzyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (100 mg, 0.19 mmol), **Intermediate E6** (113 mg, 0.57 mmol) and TEA (193 mg, 1.91 mmol) in DCM (1 mL) was degassed and purged with $N_2$ 3 times. The reaction mixture was stirred at 25 °C for 1 h under a $N_2$ atmosphere. Upon completion, the reaction mixture was filtered and evaporated. The residue was purified by Prep-HPLC to give the title compound (19 mg, 15% yield) as a white solid.

m/z ES+ $[M+H]^+$ 649.3; $^1H$ NMR (400 MHz, $CDCl_3$) δ ppm 11.55 (s, 1H), 8.34 (s, 1H), 7.57 (d, *J* = 8.4 Hz, 2H), 7.31 (d, *J* = 8.4 Hz, 2H), 7.28 - 7.23 (m, 3H), 7.12 - 7.04 (m, 2H), 4.77 (d, *J* = 9.6 Hz, 1H), 4.17 - 4.09 (m, 1H), 4.00 - 3.94 (m, 1H), 3.48 - 3.41 (m, 4H), 3.25 (s, 2H), 3.09 - 2.94 (m, 1H), 2.75 - 2.61 (m, 2H), 2.52 (s, 3H), 2.48 (s, 3H), 2.37 (s, 6H), 2.14 (dd, *J* = 16.8, 11.6 Hz, 1H), 1.89 (d, *J* = 7.2 Hz, 3H).

## Example 90

***N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-methoxyphenyl)acrylamide**

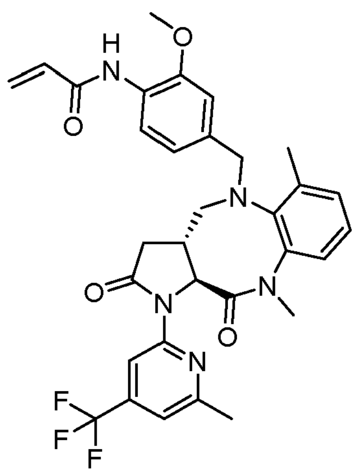

The title compound was prepared in a similar manner to **Example 89,** using **Intermediate B37** in step a, and acryloyl chloride in step d.

m/z ES+ $[M+H]^+$ 608.1; $^1H$ NMR (400 MHz, $CDCl_3$) δ ppm 8.41 (d, *J* = 8.0 Hz, 1H), 8.33 (s, 1H), 7.87 (s, 1H), 7.27 -7.24 (s, 3H), 7.05 (s, 1H), 6.97 - 6.90 (m, 2H), 6.47 - 6.39 (m, 1H), 6.34 - 6.25 (m, 1H), 5.77 (dd, *J* = 10.0, 1.2 Hz, 1H), 4.74 (d, *J* = 9.6 Hz, 1H), 4.15 - 4.02 (m, 2H), 3.93 (s, 3H), 3.46 (dd, *J* = 14.0, 4.8 Hz, 1H), 3.40 (s, 3H), 3.00 - 2.87 (m, 1H), 2.75 (dd, *J* = *14.0,* 11.6 Hz, 1H), 2.64 (dd, *J* = 16.8, 8.4 Hz, 1H), 2.50 (s, 6H), 2.13 (dd, *J* = 16.8, 11.6 Hz, 1H).

**Example 91**

***N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-((2-(dimethylamino)ethyl)(methyl)amino)phenyl) acrylamide**

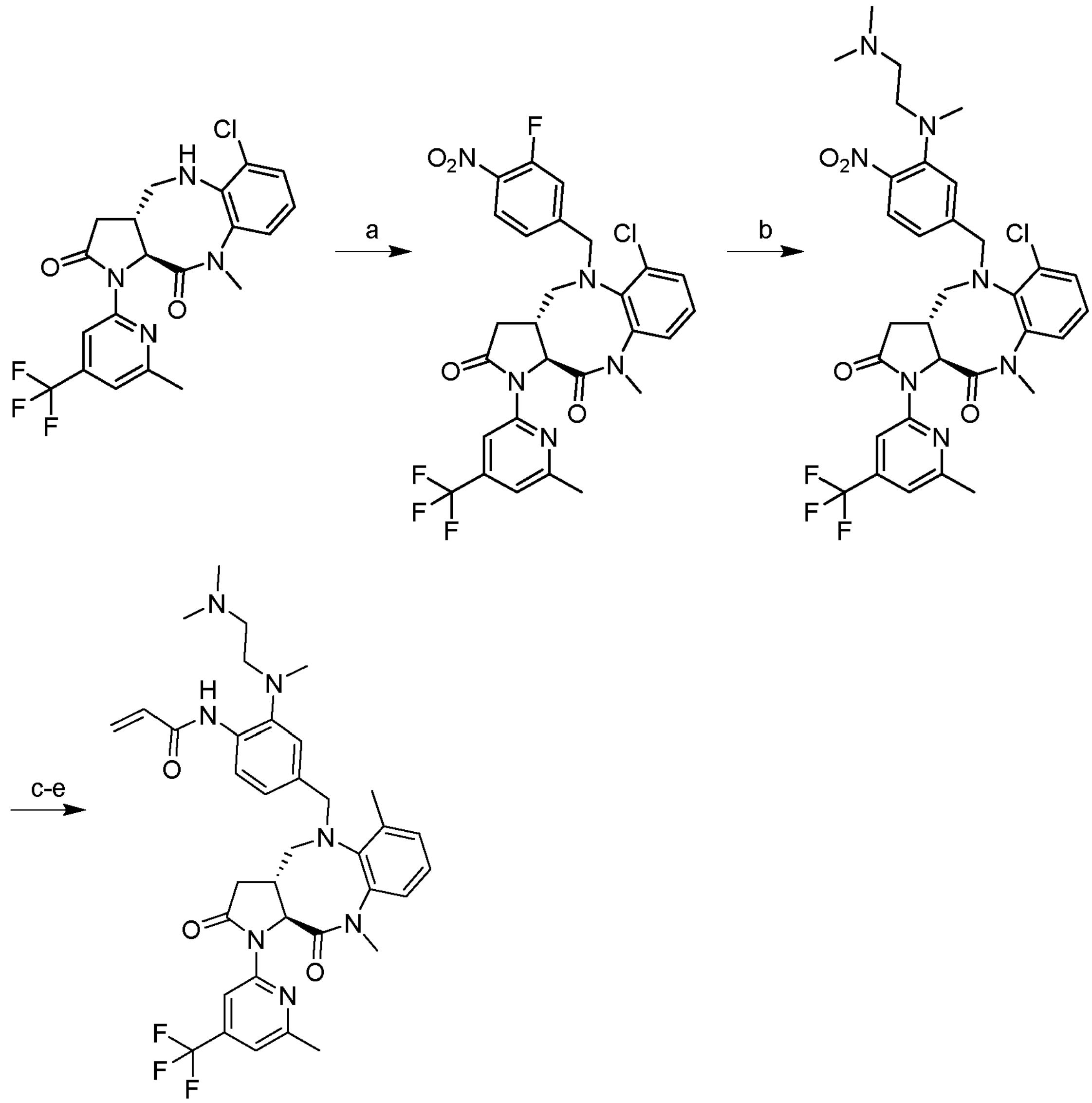


**Step a.** This step was conducted in a similar manner to **Example 39,** step a, using **Intermediate 5b** and 3-fluoro-4-nitrobenzaldehyde (CAS: 160538-51-2).

**Step b.** To a solution of (3aR,11aS)-6-chloro-5-(3-fluoro-4-nitrobenzyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (180 mg, 0.30 mmol) in MeCN (10 mL) was added $K_2CO_3$ (126 mg, 0.91 mmol) and *N*[1],*N*[1],*N*[2]-trimethylethane-1,2-diamine (CAS: 142-25-6; 37 mg, 0.37 mmol). The mixture was stirred at 40 °C for 3 h. Upon completion, the reaction mixture was diluted with $H_2O$ (10 mL) and extracted with EtOAc (3 x 5 mL). The combined organic layers were dried over $Na_2SO_4$, filtered and evaporated. The residue was purified by reverse phase flash chromatography (water (0.1% FA)/MeCN) to give (3a*R*,11a*S*)-6-chloro-5-(3-((2-(dimethylamino)ethyl)(methyl)amino)-4-nitrobenzyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (200 mg, 96% yield) as red oil.

m/z ES+ $[M+H]^+$ 674.1.

**Steps c-e.** These 3 steps were conducted in a similar manner to **Example 89,** steps b-d, using acryloyl chloride in

step d.

m/z ES+ $[M+H]^+$ 678.4; $^1H$ NMR (400 MHz, $CDCl_3$) δ ppm 10.11 (br s, 1H), 8.44 (s, 1H), 8.33 (s, 1H), 7.27 - 7.22 (m, 4H), 7.16 (s, 1H), 7.11 (d, *J* = 8.4 Hz, 1H), 7.05 (s, 1H), 6.45 (d, *J* = 16.0 Hz, 1H), 5.73 (d, *J* = 11.6 Hz, 1H), 4.73 (d, *J* = 9.2 Hz, 1H), 4.12 - 4.01 (m, 2H), 3.48 - 3.42 (m, 1H), 3.38 (s, 3H), 2.97 - 2.84 (m, 2H), 2.76 (d, *J* = 13.6 Hz, 1H), 2.72 (s, 4H), 2.67 - 2.58 (m, 2H), 2.51 (s, 4H), 2.48 (s, 4H), 2.43 - 2.25 (m, 4H), 2.20 - 2.10 (m, 2H).

**Example 92**

**(3a*R*,11a*S*)-5-((1-(1-acryloylazetidin-3-yl)piperidin-4-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

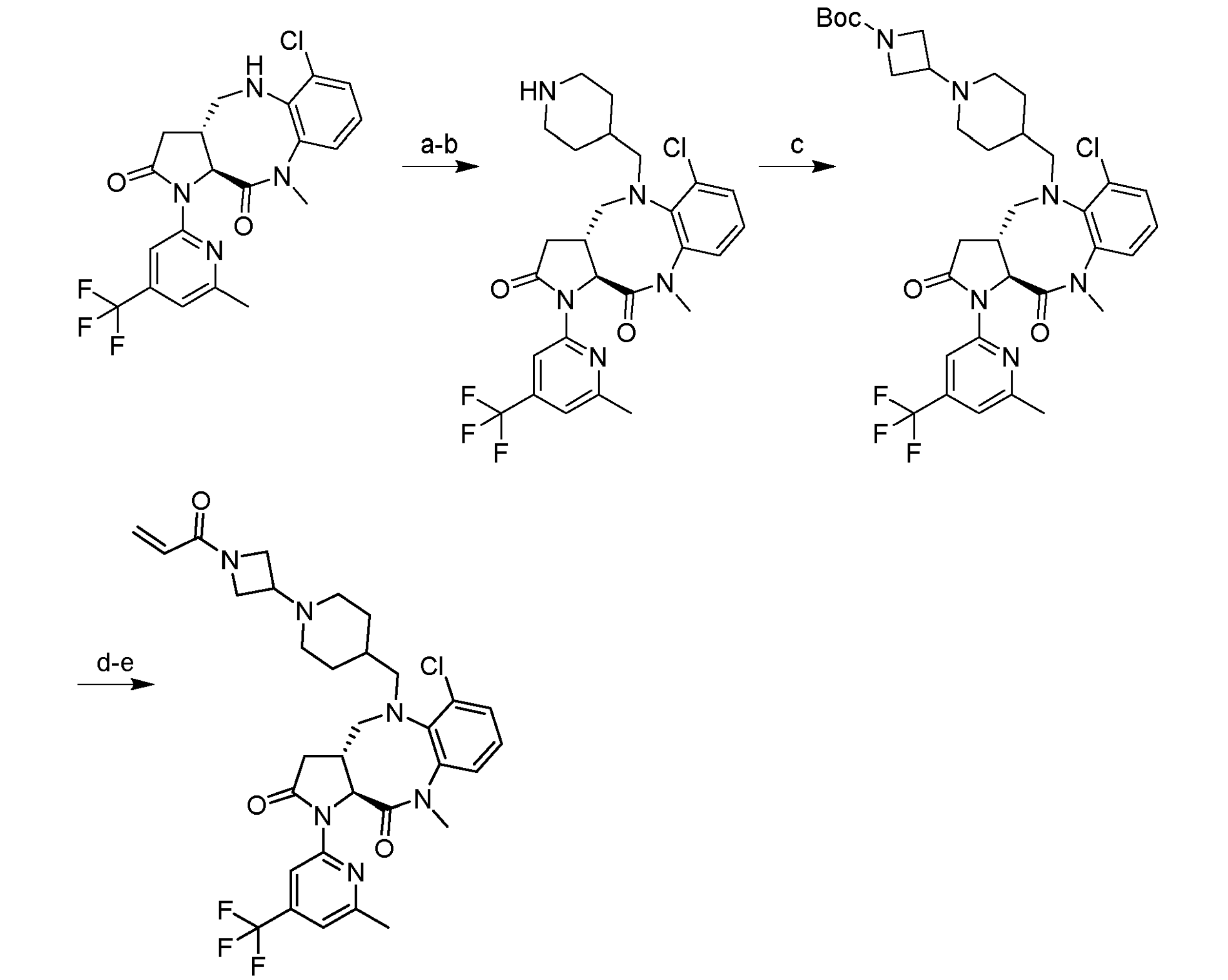


**Steps a-b.** These 2 steps were conducted in a similar manner to **Example 39,** steps a-b, using **Intermediate 5b** and *tert*-butyl 4-formylpiperidine-1-carboxylate (CAS: 137076-22-3) in step a.

**Step c.** To a solution of (3a*R*,11a*S*)-6-chloro-10-methyl-1-(6-methyl-4-(trifluoromethyl)pyridin-2-yl)-5-(piperidin-4-ylmethyl)-1,3a,4,5,10,11a-hexahydro-2*H*-benzo[*b*]pyrrolo[2,3-*f*][1,4]diazocine-2,11(3*H*)-dione (300 mg, 0.56 mmol) and *tert*-butyl 3-oxoazetidine-1-carboxylate (115 mg, 0.67 mmol) in MeOH (5 mL) was added 4 Å molecular sieves (100 mg) and acetic acid (3 mg, 0.056 mmol). The mixture was stirred at 20 °C for 30 min and then $NaBH_3CN$ (70 mg, 1.12 mmol) was added. The reaction mixture was stirred at 50 °C for 15 h 30 min. Upon completion, the reaction mixture was filtered and evaporated. Sat. aq. $NaHCO_3$ (10 mL) was added to the residue and the aqueous mixture was further diluted with water (10 mL) and extracted with EtOAc (3 x 20 mL). The combined organic layers were washed with brine (3 x 20 mL), dried over $Na_2SO_4$ and evaporated. The crude product was purified by column chromatography (EtOAc) to give *tert*-butyl 3-(4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)piperidin-1-yl)azetidine-1-carboxylate (240 mg, 62% yield) as a white solid.

m/z ES+ $[M+H]^+$ 691.4; $^1H$ NMR (400 MHz, $CDCl_3$) δ ppm 8.32 (s, 1H), 7.44 (dd, *J* = 7.2, 2.4 Hz, 1H), 7.33 - 7.27 (m, 2H), 7.05 (s, 1H), 4.62 (d, *J* = 9.2 Hz, 1H), 3.90 (m, 2H), 3.80 (m, 2H), 3.51 (dd, *J* = 14.0, 4.4 Hz, 1H), 3.36 (s, 3H), 3.07 - 2.67 (m, 8H), 2.48 (s, 3H), 2.22 (dd, *J* = 16.8, 11.2 Hz, 1H), 1.93 - 1.65 (m, 4H), 1.42 (s, 9H), 1.35 1.29 (m, 1H), 1.23 - 0.99 (m, 2H). **Steps d-e.** These 2 steps were conducted in a similar manner to **Example 38,** steps b-c.

m/z ES+ $[M+H]^+$ 645.1; $^1H$ NMR (400 MHz, $CDCl_3$) δ ppm 8.32 (s, 1H), 7.45 (dd, *J* = 7.2, 2.4 Hz, 1H), 7.33 - 7.28 (m, 2H), 7.05 (s, 1H), 6.37 - 6.27 (m, 1H), 6.23 - 6.11 (m, 1H), 5.66 (dd, *J* = 10.4, 1.6 Hz, 1H), 4.62 (d, *J* = 9.2 Hz, 1H), 4.26 - 4.17 (m, 1H), 4.15 - 3.68 (m, 3H), 3.51 (dd, *J* = 14.0, 4.0 Hz, 1H), 3.41 - 3.30 (m, 3H), 3.25 - 3.12 (m, 1H), 3.07 - 2.79 (m, 6H), 2.73 (dd, *J* = 16.8, 8.4 Hz, 1H), 2.48 (s, 3H), 2.22 (dd, *J* = 16.8, 11.2 Hz, 1H), 1.90 - 1.81 (m, 3H), 1.39 - 1.13 (m, 4H).

**Example 93**

**(3a*R*,11a*S*)-5-((1-(1-acryloylazetidin-3-yl)piperidin-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

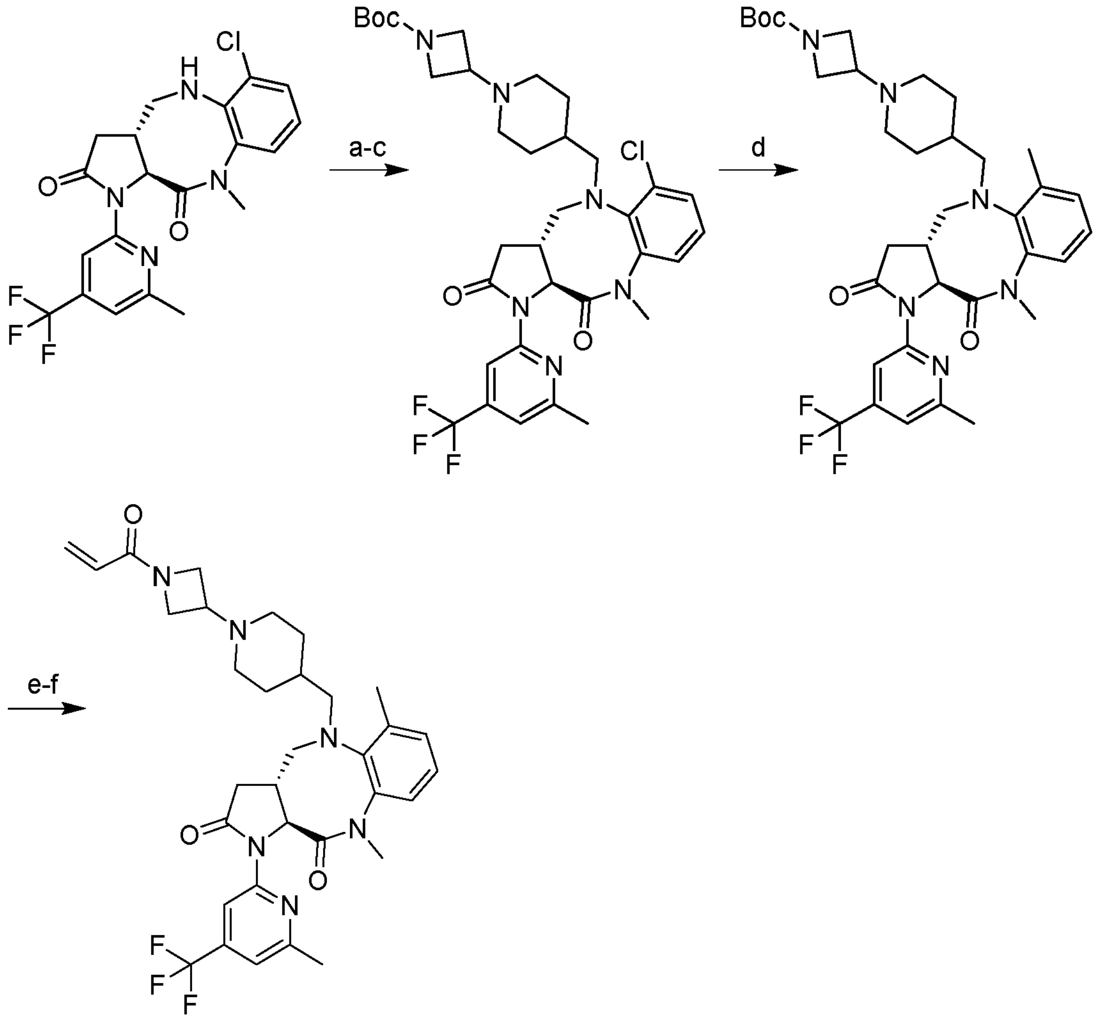


**Steps a-c**. These 3 steps were conducted as described in **Example 92,** steps **a-c**.

**Step d.** This step was conducted in a similar manner to **Example 77,** step b.

**Steps e-f.** These 2 steps were conducted in a similar manner to **Example 92,** steps d-e.

m/z ES+ $[M+H]^+$ 625.4; $^1H$ NMR (400 MHz, $CDCl_3$) δ ppm 8.31 (s, 1H), 7.27 - 7.19 (m, 3H), 7.05 (s, 1H), 6.33 (dd, *J* = 16.8, 1.6 Hz, 1H), 6.20 - 6.09 (m, 1H), 5.72 (dd, *J* = 10.4, 1.2 Hz, 1H), 4.97 - 4.71 (m, 1H), 4.68 (d, *J* = 9.2 Hz, 1H), 4.58 - 4.18 (m, 3H), 3.84 - 3.65 (m, 1H), 3.63 - 3.27 (m, 5H), 3.04 - 2.79 (m, 4H), 2.72 (dd, *J* = 16.8, 8.4 Hz, 1H), 2.60 - 2.48 (m, 4H), 2.37 (s, 3H), 2.20 (dd, *J* = 16.8, 11.2 Hz, 1H), 2.09 - 1.55 (m, 7H).

**Example 94**

**(3a*R*,11a*S*)-5-((1-(1-acryloylazetidin-3-yl)-4-fluoropiperidin-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

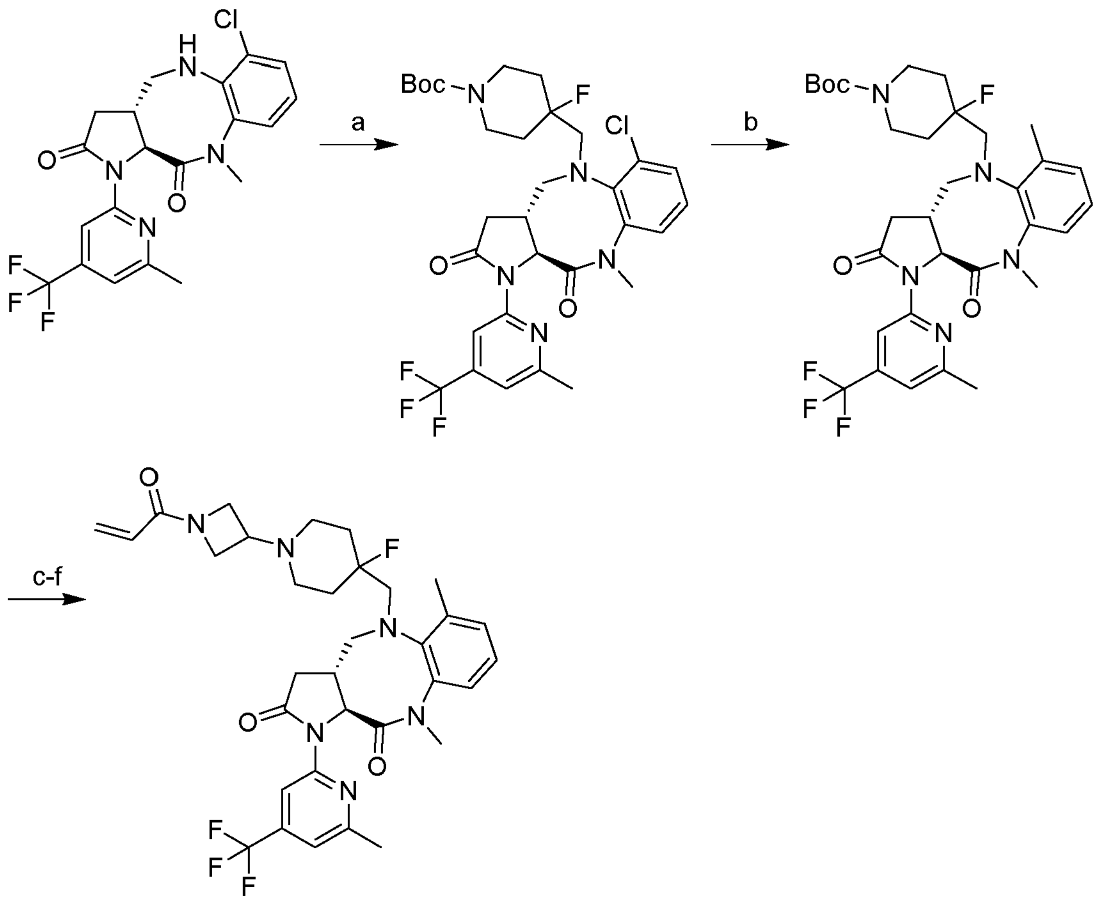


**Step a.** This step was conducted in a similar manner to **Example 92,** step a, using **Intermediate 5b** and **Intermediate B38.**

**Step b.** This step was conducted in a similar manner to **Example 77,** step b.

**Steps c-f.** These 4 steps were conducted in a similar manner to **Example 92,** steps b-e.

m/z ES+ $[M+H]^+$ 643.1; $^1H$ NMR (400 MHz, $CDCl_3$) δ ppm 8.31 (s, 1H), 7.27 - 7.22 (s, 3H), 7.04 (s, 1H), 6.38 - 6.25 (m, 1H), 6.23 - 6.11 (m, 1H), 5.66 (d, *J* = 10.4 Hz, 1H), 4.66 (d, *J* = 9.6 Hz, 1H), 4.25 - 4.17 (m, 1H), 4.15 - 3.98 (m, 2H), 3.94 - 3.84 (m, 1H), 3.73 - 3.61 (m, 1H), 3.35 (s, 3H), 3.23 - 3.08 (m, 2H), 3.07 - 2.91 (m, 2H), 2.86 - 2.75 (m, 1H), 2.72 - 2.65 (m, 1H), 2.63 - 2.55 (m, 2H), 2.49 (s, 3H), 2.43 (s, 3H), 2.22 - 2.12 (m, 3H), 2.01 - 1.92 (m, 2H), 1.66 - 1.40 (m, 2H).

**Example 95a: (3a*R*,11a*S*)-5-((1-((*S*/*R*)-1-acryloylpyrrolidin-3-yl)piperidin-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione and**

**Example 95b: (3a*R*,11a*S*)-5-((1-((*R*/*S*)-1-acryloylpyrrolidin-3-yl)piperidin-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

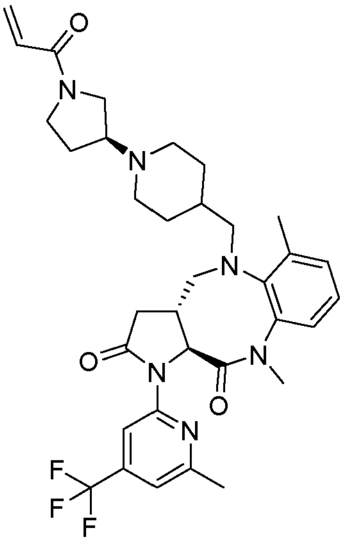

and

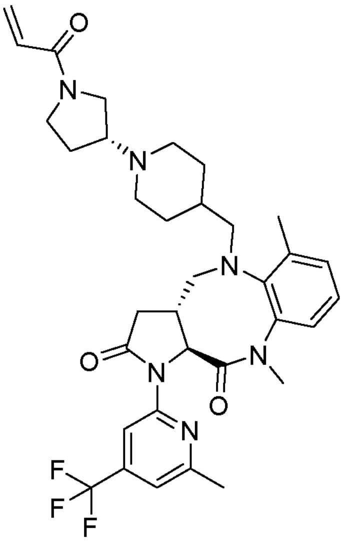

The title compounds were prepared in a similar manner to **Example 94,** using **Intermediate 5b** and *tert*-butyl 4-formylpiperidine-1-carboxylate (CAS: 137076-22-3) in step a, and *tert*-butyl 3-oxopyrrolidine-1-carboxylate (CAS: 101385-93-7) in step d. An additional chiral SFC step was conducted after step d.

**Example 95a:**

m/z ES+ $[M+H]^+$ 639.2; $^1H$ NMR (400 MHz, $CDCl_3$) $\delta$ ppm 8.32 (s, 1H), 7.26 - 7.19 (m, 3H), 7.05 (s, 1H), 6.47 - 6.33 (m, 2H), 5.75 - 5.65 (m, 1H), 4.69 (d, $J$ = 9.2 Hz, 1H), 4.05 - 3.67 (m, 2H), 3.61 - 3.41 (m, 2H), 3.35 (s, 3H), 3.06 - 2.67 (m, 7H), 2.49 (s, 3H), 2.39 (s, 3H), 2.24 - 2.16 (m, 2H), 2.12 - 1.89 (m, 2H), 1.75 - 1.55 (m, 5H), 1.48 - 1.06 (m, 3H).

**Example 95b:**

m/z ES+ $[M+H]^+$ 639.3; $^1H$ NMR (400 MHz, $CDCl_3$) $\delta$ ppm 8.32 (s, 1H), 7.25 - 7.19 (m, 3H), 7.04 (s, 1H), 6.45 - 6.32 (m, 2H), 5.72 - 5.65 (m, 1H), 4.71 - 4.66 (m, 1H), 3.98 - 3.71 (m, 2H), 3.59 - 3.45 (m, 2H), 3.45 - 3.31 (m, 4H), 3.11 - 2.66 (m, 8H), 2.49 (s, 3H), 2.39 (s, 3H), 2.29 - 1.88 (m, 5H), 1.85 - 1.56 (m, 4H), 1.30 - 1.19 (m, 1H).

**Example 96**

**(3a*R*,11a*S*)-5-((1-(1-acryloylazetidin-3-yl)-3-hydroxypiperidin-4-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

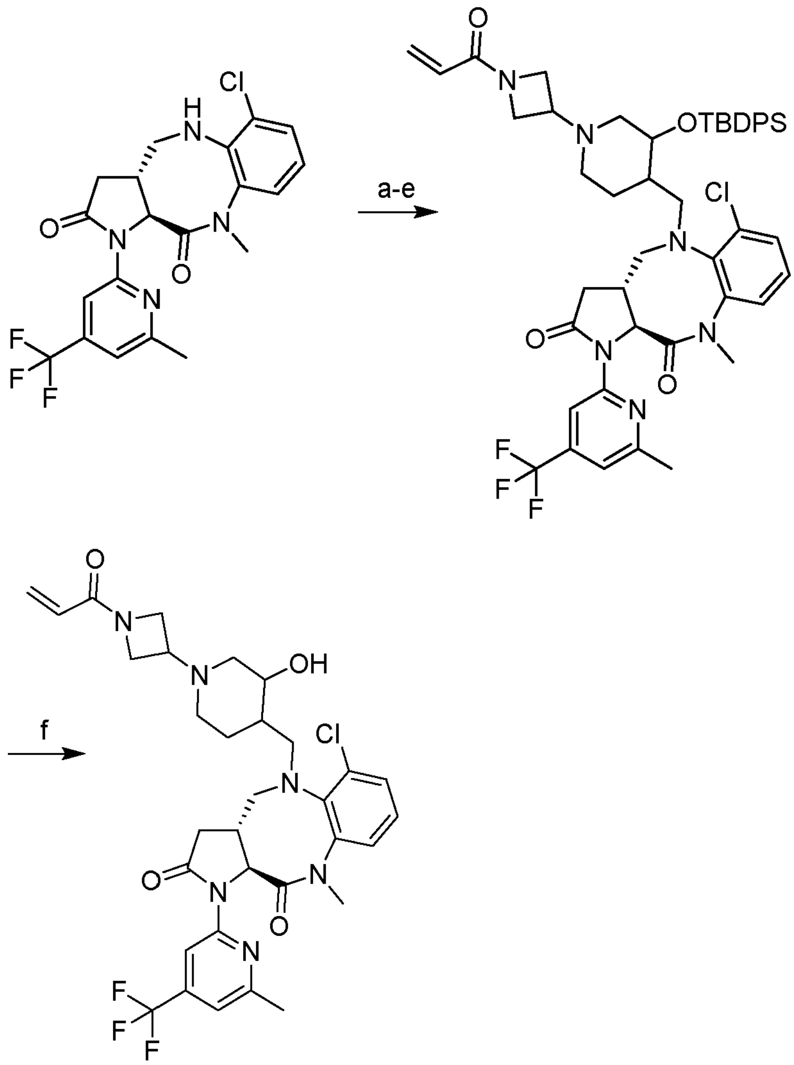


**Steps a-e.** These 5 steps were conducted in a similar manner to Example 92, steps a-e, using **Intermediate 5b** and **Intermediate B39** in step a.

**Step f.** A mixture of (3a*R*,11a*S*)-5-((1-(1-acryloylazetidin-3-yl)-3-((*tert*-butyldiphenylsilyl)oxy)piperidin-4-yl) methyl)-6-chloro-10-methyl-1-(6-methyl-4-(trifluoromethyl)pyridin-2-yl)-1,3a,4,5,10,11a-hexahydro-2*H*-benzo[*b*]pyrrolo [2,3-*f*][1,4]diazocine-2,11(3*H*)-dione (20 mg, 0.022 mmol) and TBAF (1 M in THF, 0.13 mL) in THF (1 mL) was stirred at 15 °C for 12 h. Upon completion, the mixture was quenched with water (1 mL), further diluted with water (30 mL) and extracted with EtOAc (2 x 30 mL). The combined organic layers were evaporated and the residue was purified by reverse phase flash chromatography (water (0.1% FA)/MeCN) followed by Prep-HPLC to give the title compound (0.7 mg, 5% yield) as a white solid.

m/z ES+ $[M+H]^+$ 661.1; $^1$H NMR (400 MHz, $CDCl_3$) δ ppm 8.34 (s, 1H), 7.48 (dd, *J* = 6.8, 2.8 Hz, 1H), 7.35 - 7.29 (m, 2H), 7.05 (s, 1H), 6.41 - 6.28 (m, 1H), 6.25 - 6.13 (m, 1H), 5.67 (d, *J* = 11.6 Hz, 1H), 4.62 (d, *J* = 8.8 Hz, 1H), 4.29 - 3.86 (m, 4H), 3.66 - 3.50 (m, 2H), 3.40 (s, 3H), 3.34 - 3.17 (m, 2H), 3.12 - 2.70 (m, 7H), 2.47 (s, 3H), 2.31 - 2.19 (m, 1H), 1.89 - 1.82 (m, 3H), 1.38 - 1.28 (m, 2H).

### Example 97

**(3a*R*,11a*S*)-5-(2-(4-(1-acryloylazetidin-3-yl)piperazin-1-yl)propyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

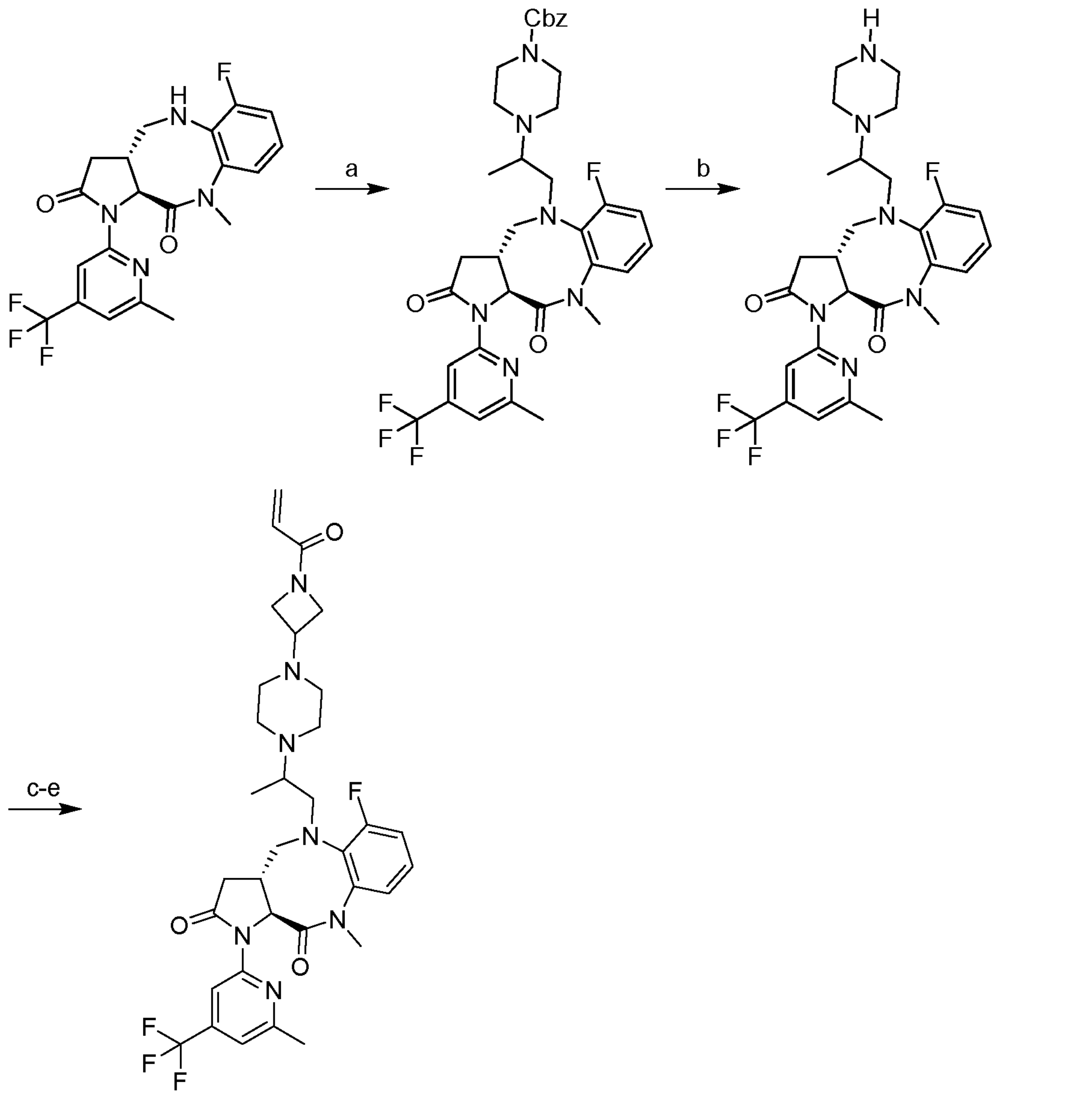


**Step a.** This step was conducted in a similar manner to Example 92, step a, using **Intermediate 5a** and **Intermediate B46** in step a.

**Step b.** To a solution of benzyl 4-(1-((3aR,11aS)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propan-2-yl)piperazine-1-carboxylate (50 mg, 0.073 mmol) in MeOH (2 mL) was added 10% Pd/C (50 mg). The mixture was stirred at 30 °C for 16 h under a $H_2$ atmosphere (15 psi). Upon completion, the reaction mixture was filtered and evaporated to give (3a*R*,11a*S*)-6-fluoro-10-methyl-1-(6-methyl-4-(trifluoromethyl)pyridin-2-yl)-5-(2-(piperazin-1-yl)propyl)-1,3a,4,5,10,11a-hexahydro-2*H*-benzo[*b*]pyrrolo[2,3-*f*][1,4]diazocine-2,11(3*H*)-dione (34 mg, crude) as white solid.

m/z ES+ $[M+H]^+$ 549.3.

**Steps c-e.** These 3 steps were conducted in a similar manner **Example 92,** steps c-e.

m/z ES+ $[M+H]^+$ 658.3; $^1$H NMR (400 MHz, $CDCl_3$) δ ppm 8.32 (s, 1H), 7.41 - 7.29 (m, 1H), 7.26 - 7.22 (m, 1H), 7.19 - 7.13 (m, 1H), 7.06 (s, 1H), 6.37 - 6.27 (m, 1H), 6.22 - 6.12 (m, 1H), 5.69 - 5.65 (m, 1H), 4.61 - 4.56 (m, 1H), 4.26 - 4.17 (m, 1H), 4.15 - 3.99 (m, 2H), 3.94 - 3.83 (m, 1H), 3.62 - 3.55 (m, 1H), 3.37 (s, 3H), 3.30 - 3.19 (m, 1H), 3.09 - 2.80 (m, 5H), 2.78 - 2.51 (m, 5H), 2.50 (s, 3H), 2.25 - 2.15 (m, 1H), 1.80 - 1.41 (m, 4H), 1.37 - 0.88 (m, 3H).

**Example 98**

**(3a*R*,11a*S*)-5-((5-(1-acryloylazetidin-3-yl)-1*H*-1,2,4-triazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

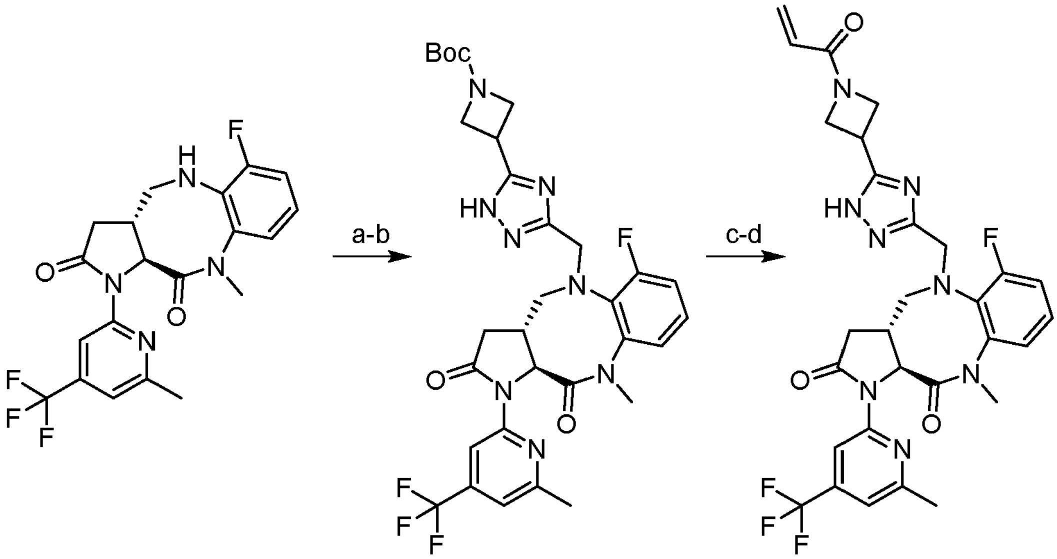


**Steps a-b.** These 2 steps were conducted as described in **Intermediate 11a** and **Intermediate 11b,** steps a-b.

**Step c.** This step was conducted in a similar manner to Example 38, step b.

**Step d.** To a mixture of (3a*R*,11a*S*)-5-((5-(azetidin-3-yl)-1*H*-1,2,4-triazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (30 mg, 0.045 mmol, TFA salt), acrylic acid (3 mg, 0.045 mmol) and DIPEA (17 mg, 0.13 mmol) in DCM (2 mL) was added HATU (20 mg, 0.054 mmol). The reaction mixture was stirred at 20 °C for 30 min. Upon completion, the reaction mixture was evaporated and the residue was purified by Prep-TLC (DCM/MeOH = 20/1) followed by Prep-HPLC to give the title compound (12 mg, 43% yield) as a white solid. m/z ES+ $[M+H]^+$ 613.1; $^1H$ NMR (400 MHz, $CDCl_3$) δ ppm 8.32 (s, 1H), 7.45 - 7.36 (m, 1H), 7.26 - 7.17 (m, 2H), 7.08 (s, 1H), 6.38 - 6.30 (m, 1H), 6.26 - 6.15 (m, 1H), 5.74 - 5.65 (m, 1H), 4.66 (d, *J* = 9.6 Hz, 1H), 4.59 - 4.37 (m, 5H), 4.32 - 4.20 (m, 1H), 4.04 - 3.93 (m, 1H), 3.71 - 3.60 (m, 1H), 3.33 (s, 3H), 3.12 - 3.01 (m, 1H), 2.99 - 2.87 (m, 1H), 2.78 - 2.68 (m, 1H), 2.51 (s, 3H), 2.27 - 2.17 (m, 1H).

**Example 99**

**(3a*R*,11a*S*)-5-((3-(1-acryloylazetidin-3-yl)-1-methyl-1*H*-1,2,4-triazol-5-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

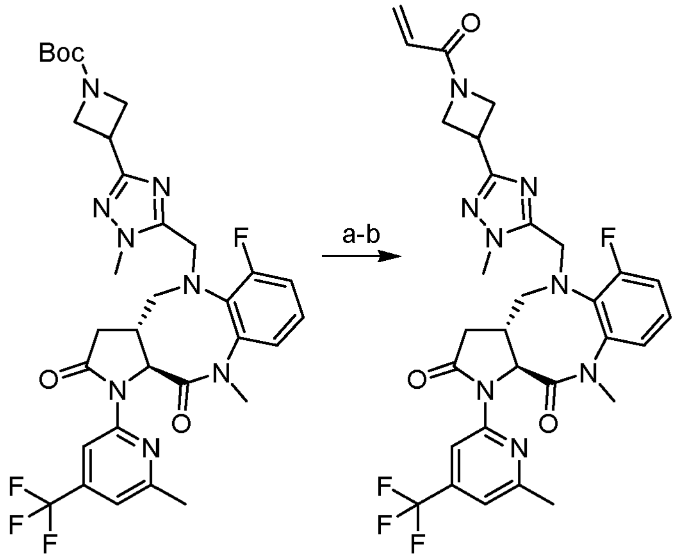


**Steps a-b.** These 2 steps were conducted in a similar manner to **Example 38,** steps b-c, using **Intermediate 11a** in step a.

m/z ES+ $[M+H]^+$ 627.3; $^1H$ NMR (400 MHz, $CDCl_3$) δ ppm 8.30 (s, 1H), 7.42 - 7.34 (m, 1H), 7.23 - 7.13 (m, 2H), 7.06 (s, 1H), 6.40 - 6.30 (m, 1H), 6.25 - 6.16 (m, 1 H), 5.68 (d, *J* =10.4 Hz, 1H), 4.61 (dd, *J* = 9.6, 1.6 Hz, 1H), 4.53 - 4.47 (m, 1H), 4.47 - 4.41 (m, 2H), 4.40 - 4.32 (m, 2H), 4.13 - 4.06 (m, 1H), 3.90 - 3.85 (m, 1H), 3.84 (d, *J* = 4.0 Hz, 3H), 3.66 - 3.56 (m, 1H), 3.26 (d, *J* = 13.6 Hz, 3H), 3.06 - 2.93 (m, 1H), 2.79 (dd, *J* = 9.6, 4.8 Hz, 1H), 2.74 - 2.66 (m, 1H), 2.48 (s, 3H), 2.20 (dd, *J* = 16.8, 11.6 Hz, 1H).

**Example 100**

**(3a*R*,11a*S*)-5-((5-(1-acryloylazetidin-3-yl)-1-methyl-1*H*-1,2,4-triazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

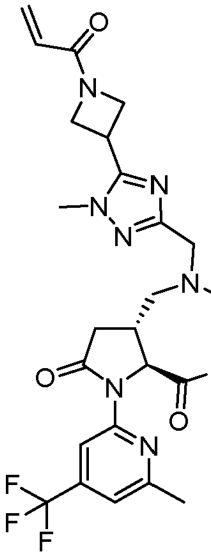

The title compound was prepared in a similar manner to **Example 99,** using **Intermediate 11b** in step a.

m/z ES+ $[M+H]^+$ 627.3; $^1H$ NMR (400 MHz, $CDCl_3$) δ ppm 8.30 (s, 1H), 7.36 - 7.28 (m, 1H), 7.20 (d, *J* = 8.4 Hz, 1H), 7.17 - 7.10 (m, 1H), 7.04 (s, 1H), 6.41 - 6.30 (m, 1H), 6.26 - 6.16 (m, 1H), 5.70 (t, *J* = 9.6 Hz, 1H), 4.73 - 4.53 (m, 3H), 4.50 - 4.41 (m, 1H), 4.39 - 4.31 (m, 1H), 4.26 - 4.08 (m, 2H), 3.94 - 3.74 (m, 2H), 3.72 (d, *J* = 4.4 Hz, 3H), 3.35 - 3.21 (m, 3H), 3.05 -

2.75 (m, 2H), 2.72 - 2.63 (m, 1H), 2.48 (s, 3H), 2.26 - 2.11 (m, 1H).

**Example 101**

**(3a*R*,11a*S*)-5-((5-(1-acryloylazetidin-3-yl)-1,2,4-oxadiazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

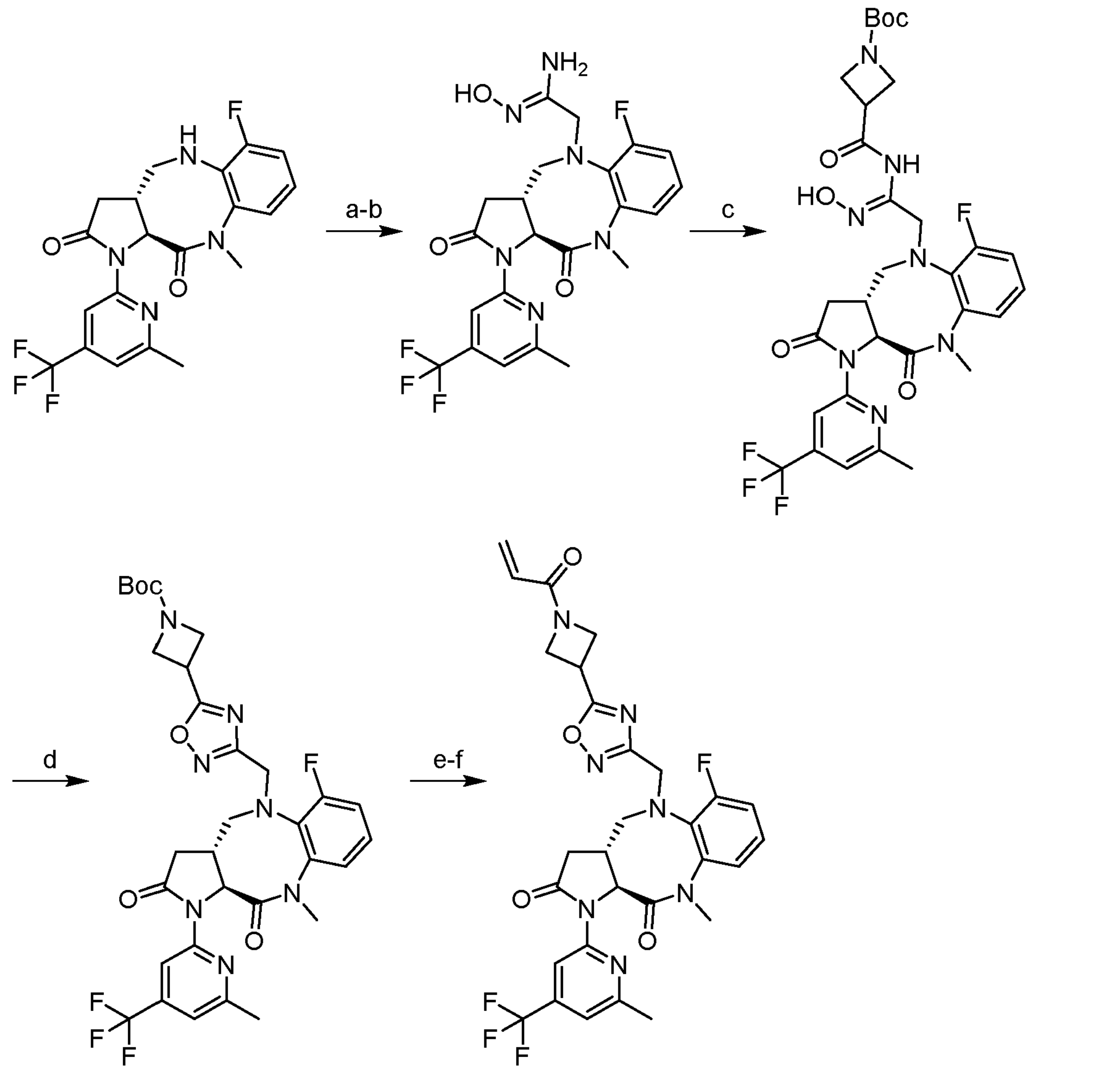


**Step** a. This step was conducted as described in **Intermediate 11a** and **Intermediate 11b,** step a.

**Step** b. A mixture of 2-((3aR,11aS)-6-fluoro-10-methyl-1-(6-methyl-4-(trifluoromethyl)pyridin-2-yl)-2,11-dioxo-1,2,3,3a,4,10,11,11a-octahydro-5H-benzo[b]pyrrolo[2,3-f][1,4]diazocin-5-yl)acetonitrile (300 mg, 0.65 mmol), hydroxylamine hydrochloride (90 mg, 1.30 mmol) and $K_2CO_3$ (269 mg, 1.95 mmol) in EtOH (3 mL) was stirred at 100 °C for 16 hr. Upon completion, the reaction mixture was diluted with water (10 ml) and extracted with EtOAc (2 x 10 ml). The combined organic layers were evaporated and the residue was purified by Prep-TLC (EtOAc) to give 2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo [2,3-*f*][1,4]diazocin-5-yl)-*N*'-hydroxyacetimidamide (150 mg, 46% yield) as a white solid.

$^1H$ NMR (400 MHz, $CDCl_3$) δ ppm 8.34 (s, 1H), 7.46 - 7.36 (m, 1H), 7.26 (d, *J* = 8.4 Hz, 1H), 7.23 - 7.16 (m, 1H), 7.08 (s, 1H), 5.57 (br s, 2H), 4.73 - 4.60 (m, 1H), 4.03 - 3.69 (m, 3H), 3.63 - 3.54 (m, 1H), 3.44 - 3.38 (m, 3H), 3.07 - 2.70 (m, 3H), 2.51 (s, 3H), 2.28 - 2.13 (m, 1H).

**Step c.** To a solution of 1-(*tert*-butoxycarbonyl)azetidine-3-carboxylic acid (63 mg, 0.32 mmol) in DMF (1 mL) was added CDI (79 mg, 0.49 mmol). The reaction mixture was stirred at 25 °C for 6 h, after which, 2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-*N'*-hydroxyacetimidamide (120 mg, 0.24 mmol) was added and the reaction mixture was stirred at 25 °C for a further 10 h. Upon completion, the reaction mixture was concentrated to give *tert*-butyl 3-((2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-1-(hydroxyimino)ethyl)carbamoyl)azetidine-1-carboxylate (140 mg, crude) as a yellow oil.

m/z ES+ $[M+H]^+$ 678.0.

**Step d.** To a solution of *tert*-butyl 3-((2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-1-(hydroxyimino)ethyl)carbamoyl)azetidine-1-carboxylate (140 mg, 0.21 mmol) in 1,4-dioxane (1 mL) was added DBU (63 mg, 0.41 mmol) and the mixture was stirred at 25 °C for 3 h. Upon completion, the reaction mixture was evaporated and the residue was purified by column chromatography (EtOAc) to give *tert-butyl* 3-(3-(((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-1,2,4-oxadiazol-5-yl)azetidine-1-carboxylate (120 mg, 79% yield) as a white solid.

m/z ES+ $[M+H]^+$ 660.1.

**Steps e-f.** These 2 steps were conducted in a similar manner to **Example 98,** steps c-d.

m/z ES+ $[M+H]^+$ 614.1; $^1H$ NMR (400 MHz, $CDCl_3$) $\delta$ ppm 8.33 (s, 1H), 7.43 - 7.36 (m, 1H), 7.27 - 7.16 (m, 2H), 7.07 (s, 1H), 6.45 - 6.35 (m, 1H), 6.28 - 6.17 (m, 1H), 5.79 - 5.70 (m, 1H), 4.68 - 4.62 (m, 2H), 4.61 - 4.42 (m, 3H), 4.37 - 4.21 (m, 2H), 4.12 - 4.07 (m, 1H), 3.83 - 3.72 (m, 1H), 3.32 (s, 3H), 3.12 - 3.02 (m, 1H), 3.00 - 2.79 (m, 1H), 2.78 - 2.69 (m, 1H), 2.50 (s, 3H), 2.27 - 2.14 (m, 1H).

**Example 102**

**(3a*R*,11a*S*)-5-((3-(1-acryloylazetidin-3-yl)-1,2,4-oxadiazol-5-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

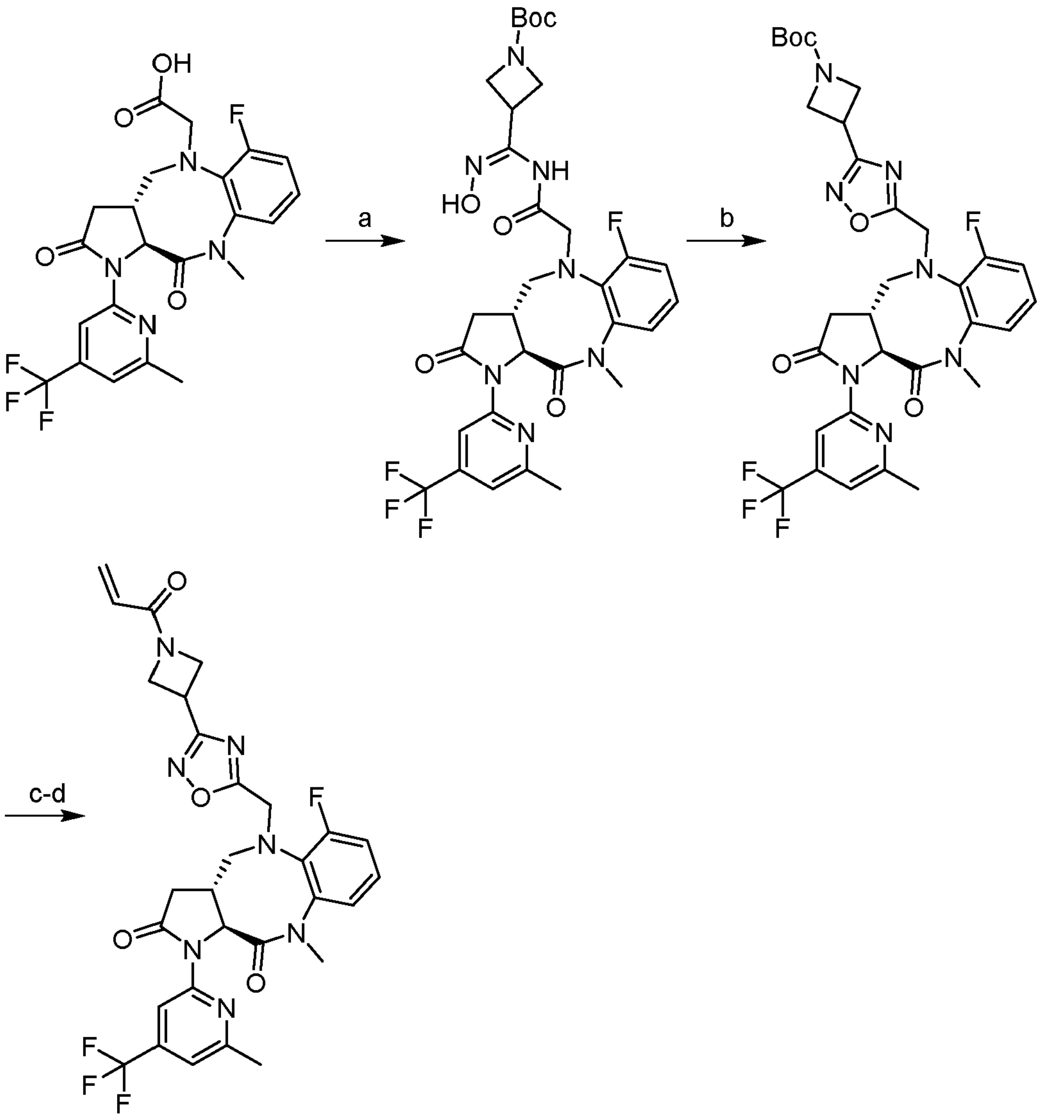


**Step** a. To a solution of **Intermediate 9a** (170 mg, 0.35 mmol) in DMF (2 mL) was added CDI (207 mg, 1.27 mmol) and the mixture was stirred at 25 °C for 6 h, after which, *tert*-butyl 3-(*N*'-hydroxycarbamimidoyl)azetidine-1-carboxylate (CAS: 1309207-05-3; 91 mg, 0.43 mmol) was added. The reaction mixture was stirred at 25 °C for a further 10 h. Upon completion, the mixture was evaporated to give *tert*-butyl 3-(N-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)acetyl)-*N*'-hydroxycarbamimidoyl)azetidine-1-carboxylate (240 mg, crude) as a white solid.

m/z ES+ $[M+H]^+$ 678.2.

**Step b.** This step was conducted in a similar manner to **Example 101,** step d.

**Steps c-d.** These 2 steps were conducted in a similar manner to **Example 38,** steps b-c.

m/z ES+ $[M+H]^+$ 614.2; $^1$H NMR (400 MHz, $CDCl_3$) δ ppm 8.32 (s, 1H), 7.43 - 7.36 (m, 1H), 7.27 - 7.23 (m, 1H), 7.22 - 7.15 (m, 1H), 7.06 (s, 1H), 6.41 - 6.32 (m, 1H), 6.26 - 6.15 (m, 1H), 5.75 - 5.67 (m, 1H), 4.63 (d, *J* = 9.6 Hz, 1H), 4.60 - 4.54 (m, 1H), 4.52 (s, 2H), 4.49 - 4.41 (m, 2H), 4.26 - 4.14 (m, 1H), 4.02 - 3.98 (m, 1H), 3.80 - 3.69 (m, 1H), 3.32 (d, *J* = 17.2 Hz, 3H), 3.16 - 3.06 (m, 1H), 3.03 - 2.82 (m, 1H), 2.75 (dd, *J* = 16.8, 8.4 Hz, 1H), 2.49 (s, 3H), 2.22 (dd, *J* = 16.8, 11.6 Hz, 1H).

**Example 103**

**(3a*R*,11a*S*)-5-((5-(1-acryloylazetidin-3-yl)-1,3,4-oxadiazol-2-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

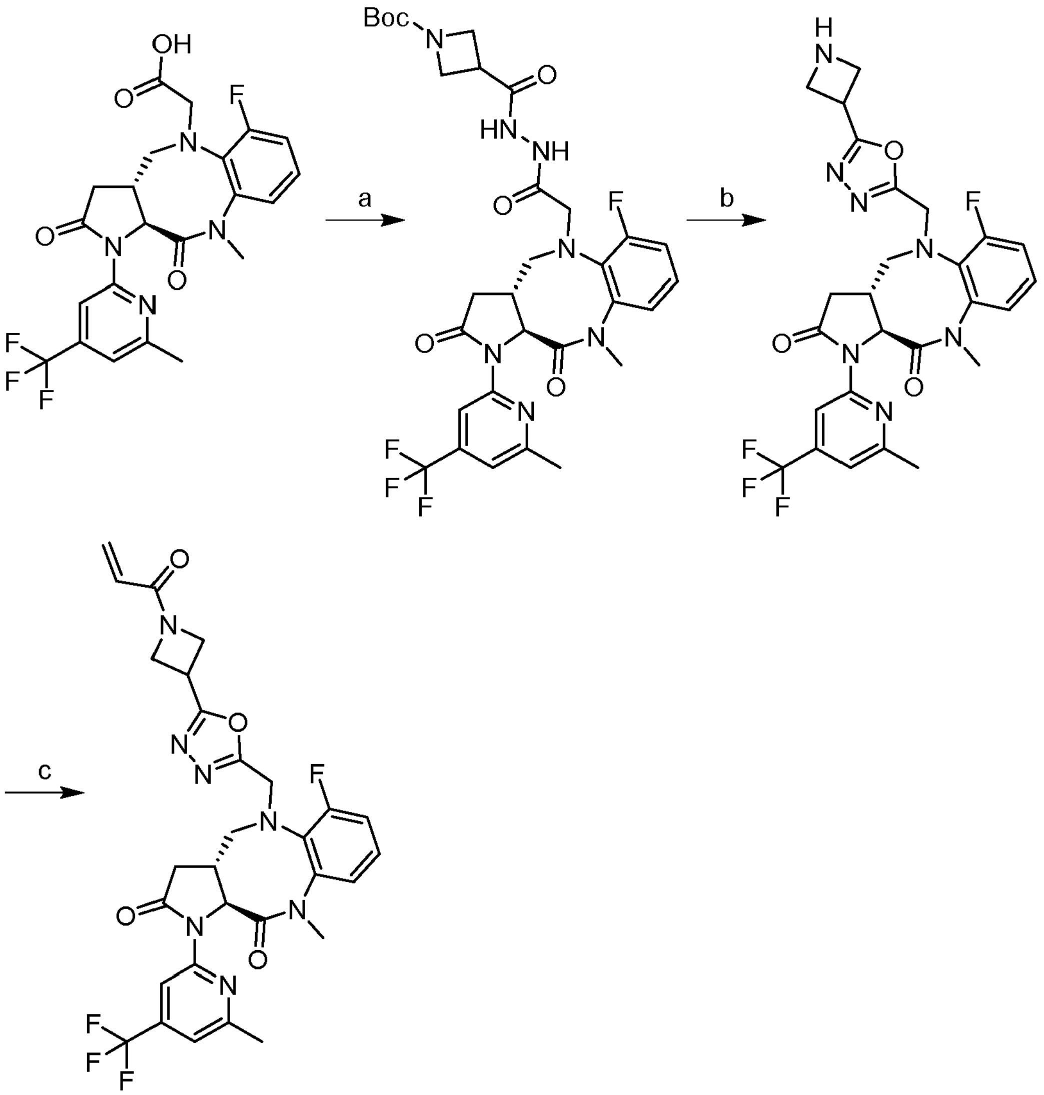


**Step a.** To a solution of **Intermediate 9a** (200 mg, 0.42 mmol) and *tert*-butyl 3-(hydrazinecarbonyl)azetidine-1-carboxylate (CAS: 1001907-44-3; 107 mg, 0.5 mmol) in DCM (4 mL) was added EDCI (96 mg, 0.5 mmol) and HOBt (73 mg, 0.54 mmol). The mixture was stirred at 25 °C for 5 h. Upon completion, the reaction mixture was diluted in water (10 mL) and extracted with EtOAc (5 mL). The aqueous layer was further extracted with EtOAc (2 x 10 mL). The combined organic layers were dried over $Na_2SO_4$ and evaporated. The residue was purified by column chromatography (EtOAc/MeOH = 20/1) to give *tert*-butyl 3-(2-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)acetyl)hydrazine-1-carbonyl)azetidine-1-carboxylate (135 mg, 46% yield) as a white solid.

m/z ES+ $[M+H]^+$ 678.1; $^1H$ NMR (400 MHz, $CDCl_3$) δ ppm 9.12 (d, *J* = 3.2 Hz,1H), 8.33 (s, 1H), 7.95 (d, *J* = 4.8 Hz,1H), 7.43 - 7.40 (m, 1H), 7.27 - 7.19 (m, 2H), 7.08 (s, 1H), 4.73 - 4.68 (m, 1H), 4.16 - 4.06 (m, 4H), 3.83 (s, 2H), 3.67 - 3.64 (m, 1H), 3.43 (s, 3H), 3.30 - 3.22 (m, 1H), 3.11 - 3.01 (m, 2H), 2.85 - 2.74 (m, 1H), 2.50 (s, 3H), 2.28 - 2.23 (m, 1H), 1.44 (s, 9H).

**Step b.** To a solution of *tert*-butyl 3-(2-(2-((3a*R*,11a*S*)-6-fluoro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)acetyl)hydrazine-1-carbonyl)azetidine-1-carboxylate

(100 mg, 0.15 mmol) in MeCN (1 mL) was added $POCl_3$ (68 mg, 0.44 mmol). The mixture was stirred at 80 °C for 4 h. Upon completion, the reaction mixture was quenched with sat. aq. $NaHCO_3$ (20 mL). The aqueous mixture was extracted with EtOAc (3 x 25 mL). The combined organic layers were washed with brine (50 mL), dried over $Na_2SO_4$ and evaporated to give (3a*R*,11a*S*)-5-((5-(azetidin-3-yl)-1,3,4-oxadiazol-2-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (100 mg, crude) was obtained as a yellow solid.

m/z ES+ $[M+H]^+$ 560.2.

**Step c.** This step was conducted in a similar manner to **Example 38,** step c.

m/z ES+ $[M+H]^+$ 614.2; $^1H$ NMR (400 MHz, $CDCl_3$) δ ppm 8.32 (s, 1H), 7.45 - 7.30 (m, 2H), 7.24 - 7.17 (m, 1H), 7.07 (s, 1H), 6.44 - 6.32 (m, 1H), 6.30 - 6.18 (m, 1H), 5.75 - 5.70 (m, 1H), 4.71 - 4.57 (m, 4H), 4.53 - 4.46 (m, 1H), 4.40 - 4.22 (m, 2H), 4.19 - 4.05 (m, 1H), 3.82 - 3.69 (m, 1H), 3.27 - 3.21 (m, 3H), 3.13 - 3.00 (m, 1H), 2.79 - 2.58 (m, 2H), 2.49 (s, 3H), 2.27 - 2.12 (m, 1H).

The **Examples** in **Table 9** were prepared using methods similar to those described in the synthesis of **Example 1,** using the listed **Intermediates** in step a.

**Table 9**

| Example Number | Structure | Name | Intermediates | $^1$H NMR (400 MHz) δ ppm | MI |
|---|---|---|---|---|---|
| **104** | 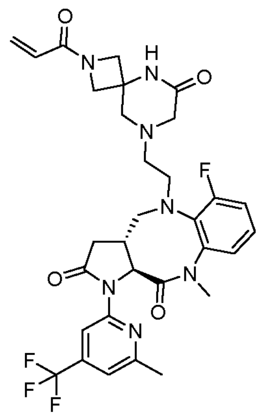 | (3a*R*,11a*S*)-5-(2-(2-acryloyl-6-oxo-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Intermediate 7a** and **Intermediate A26** | ($CDCl_3$) 8.32 (s, 1H), 7.40 - 7.32 (m, 1H), 7.23 (d, *J* = 8.0 Hz, 1H), 7.15 (t, *J* = 9.2 Hz, 1H), 7.06 (s, 1H), 7.01 (br s, 1H), 6.44 - 6.32 (m, 1H), 6.24 - 6.10 (m, 1H), 5.72 (d, *J* = 10.4 Hz, 1H), 4.60 (d, *J* = 8.8 Hz, 1H), 4.24 - 4.08 (m, 2 H), 4.06 - 3.89 (m, 2H), 3.60 - 3.50 (m, 1H), 3.34 (s, 3H), 3.30 - 3.20 (m, 2H), 3.18 - 3.04 (m, 2H), 3.02 - 2.89 (m, 2H), 2.90 - 2.83 (m, 1H), 2.80 - 2.68 (m, 2H), 2.49 (s, 3H), 2.45 (s, 2H), 2.25 - 2.16 (m, 1H) | 644.2 |
| **105** | 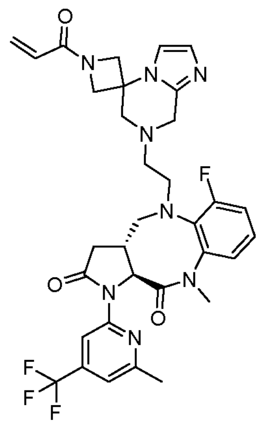 | (3a*R*,11a*S*)-5-(2-(1-acryloyl-6'*H*-spiro[azetidine-3,5'-imidazo[1,2-*a*]pyrazin]-7'(8'*H*)-yl)ethyl)-6-fluoro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Intermediate 7a** and **Intermediate A28** | ($CDCl_3$) 8.33 (s, 1H), 7.28 - 7.41 (m, 3H), 7.25 (d, *J* = 7.6 Hz, 1H), 7.15 (*J* = 9.2 Hz, 1H), 7.06 (s, 1H) , 6.45 (d, *J* = 17.2 Hz, 1H), 6.28 - 6.18 (m, 1H), 5.82 (d, *J* = 10.4 Hz, 1H), 4.60 (d, *J* = 9.2 Hz, 1H), 4.51 - 4.24 (m, 4H) , 4.10 - 3.76 (m, 2H), 3.57 (dd, *J* = 13.8, 4.6 Hz, 1H), 3.40 - 3.30 (m, 4H), 3.29 - 3.13 (m, 3H), 3.03 - 2.93 (m, 1H), 2.91 - 2.80 (m, 1H), 2.78 - 2.70 (m, 1H), 2.68 - 2.61 (m, 2H), 2.49 (s, 3H) ,2.26 - 2.18 (m, 1H) | 667.2 |

(continued)

| Example Number | Structure | Name | Intermediates | $^1$H NMR (400 MHz) δ ppm | MI |
|---|---|---|---|---|---|
| **106** | 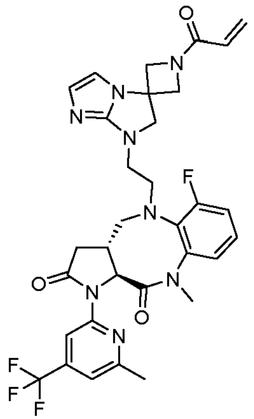 | (3a*R*,11a*S*)-5-(2-(1-acryloylspiro[azetidine-3,3'-imidazo[1,2-*a*]imidazol]-1'(2'*H*)-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 7a** and **Intermediate A29** | ($CDCl_3$) 8.32 (s, 1H), 7.37 - 7.33 (m, 1H), 7.27 - 7.20 (m, 1H), 7.15 - 7.11 (m, 1H), 7.04 (s, 1H), 6.75 - 6.60 (m, 2H), 6.42 - 6.35 (m, 1H), 6.23 - 6.15 (m, 1H), 5.75 - 5.73 (m, 1H), 4.62 - 4.59 (m, 1H), 4.55 - 4.25 (m, 3H), 4.30 - 3.80 (m, 2H), 3.75 - 3.10 (m, 9H), 3.00 - 2.55 (m, 3H), 2.50 (s, 3H), 2.25 - 2.18 (m, 1H). | 653.2 |
| **107** | 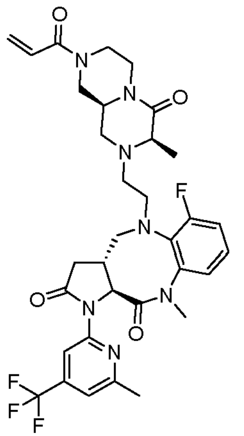 | (3a*R*,11a*S*)-5-(2-((3*R*/*S*,9a*S*/*R*)-8-acryloyl-3-methyl-4-oxooctahydro-2H-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 7a** and **Intermediate A20a** | ($CDCl_3$) 8.33 (s, 1H), 7.38 - 7.30 (m, 1H), 7.22 (d, *J* = 8.0 Hz, 1H), 7.14 (t, *J* = 9.2 Hz, 1H), 7.06 (s, 1H), 6.60 - 6.53 (m, 1H), 6.39 - 6.30 (m, 1H), 5.77 (dd, *J* = 10.4, 1.6 Hz, 1H), 4.62 - 4.57 (m, 3H), 4.05 - 3.81 (m, 1H), 3.57 - 3.52 (m, 1H), 3.50 - 3.42 (m, 1H), 3.36 (s, 3H), 3.34 - 3.28 (m, 1H), 3.27 - 3.05 (m, 3H), 2.98 - 2.82 (m, 3H), 2.81 - 2.52 (m, 5H), 2.49 (s, 3H), 2.48 - 2.44 (m, 1H), 2.25 - 2.16 (m, 1H), 1.28 (d, *J* = 6.0 Hz, 3H) | 672.3 |

(continued)

| Example Number | Structure | Name | Intermediates | $^1$H NMR (400 MHz) δ ppm | MI |
|---|---|---|---|---|---|
| **108** | 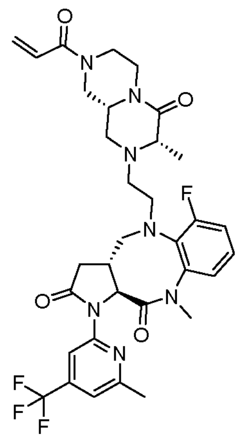 | (3a*R*,11a*S*)-5-(2-((3*S*/*R*,9a*R*/*S*)-8-acryloyl-3-methyl-4-oxooctahydro-2H-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 7a** and **Intermediate A20b** | ($CDCl_3$) 8.34 (s, 1H), 7.41 - 7.32 (m, 1H), 7.24 (d, *J* = 8.0 Hz, 1H), 7.16 (t, *J* = 8.4 Hz, 1H), 7.07 (s, 1H), 6.60 - 6.53 (dd, *J* = 16.8, 10.4 Hz, 1H), 6.35 (dd, *J* = 16.8, 1.6 Hz, 1H), 5.78 (d, *J* = 10.8 Hz, 1H), 4.62 - 4.58 (m, 3H), 4.20 - 3.90 (m, 1H), 3.62 - 3.44 (m, 2H), 3.42 - 3.32 (m, 4H), 3.30 - 3.05 (m, 3H), 3.00 - 2.64 (m, 6H), 2.60 - 2.30 (m, 6H), 2.24 - 2.17 (m, 1H), 1.30 (s, 3H) | 672.3 |
| **109** | 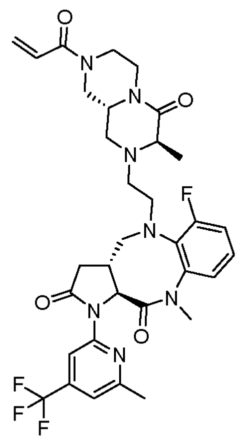 | (3a*R*,11a*S*)-5-(2-((3*R*/*S*,9a*R*/*S*)-8-acryloyl-3-methyl-4-oxooctahydro-2H-pyrazino[1,2-a]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 7a** and **Intermediate A20c** | ($CDCl_3$) 8.33 (s, 1H), 7.41 - 7.32 (m, 1H), 7.24 (d, *J* = 8.0 Hz, 1H), 7.20 - 7.12 (m, 1H), 7.06 (s, 1H), 6.67 - 6.50 (m, 1H), 6.34 (dd, *J* = 16.8, 1.6 Hz, 1H), 5.76 (d, *J* = 10.4 Hz, 1H), 4.70 - 4.50 (m, 3H), 4.03 - 3.87 (m, 1H), 3.58 - 3.53 (m, 1H), 3.51 - 3.41 (m, 1H), 3.36 (s, 3H), 3.29 - 3.04 (m, 5H), 3.01 - 2.80 (m, 3H), 2.78 - 2.56 (m, 4H), 2.50 (s, 3H), 2.40 - 2.26 (m, 1H), 2.25 - 2.18 (m, 1H), 1.28 (s, 3H) | 672.2 |

(continued)

| Example Number | Structure | Name | Intermediates | $^1$H NMR (400 MHz) δ ppm | MI |
|---|---|---|---|---|---|
| **110** | 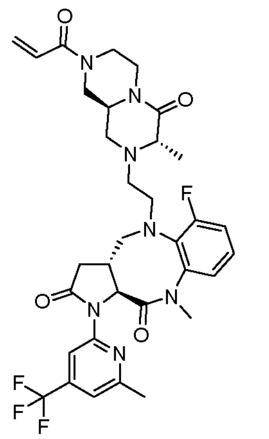 | (3a*R*,11a*S*)-5-(2-((3*S*/*R*,9a*S*/*R*)-8-acryloyl-3-methyl-4-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 7a** and **Intermediate A20d** | ($CDCl_3$) 8.33 (s, 1H), 7.35 - 7.32 (m, 1H), 7.24 (d, *J* = 8.0 Hz, 1H), 7.17 - 7.13 (m, 1H), 7.06 (s, 1H), 6.60 - 6.53 (m, 1H), 6.36 - 6.31 (m, 1H), 5.77 - 5.74 (m, 1H), 4.64 - 4.57 (m, 3H), 4.00 - 3.93 (m, 1H), 3.56 - 3.41 (m, 2H), 3.36 (s, 3H), 3.29 - 3.6 (m, 5H), 3.01 - 2.80 (m, 3H), 2.78 - 2.60 (m, 4H), 2.59 (s, 3H), 2.40 - 2.32 (m, 1H), 2.25 - 2.21 (m, 1H), 1.32 - 1.18 (m, 3H). | 672.3 |
| **111** | 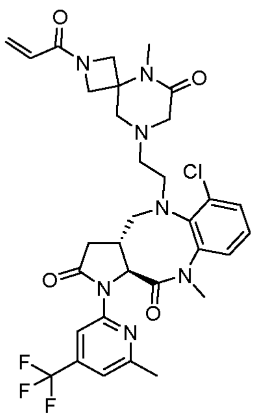 | (3a*R*,11a*S*)-5-(2-(2-acryloyl-5-methyl-6-oxo-2,5,8-triazaspiro[3.5]nonan-8-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 7b** and **Intermediate A27** | ($CDCl_3$) 8.34 (s, 1H), 7.47 - 7.44 (m, 1H), 7.36 - 7.30 (m, 2H), 7.06 (s, 1H), 6.42 - 6.36 (m, 1H), 6.24 - 6.15 (m, 1H), 5.79 - 5.71 (m, 1H), 4.61 (d, *J* = 9.2 Hz, 1H), 4.34 - 4.30 (m, 1H), 4.26 - 4.20 (m, 1H), 4.12 - 3.98 (m, 1H), 3.94 - 3.81 (m, 1H), 3.58 - 3.52 (m, 1H), 3.37 (s, 3H), 3.28 - 3.10 (m, 4H), 3.05 (s, 3H), 3.04 - 3.00 (m, 2H), 2.95 - 2.87 (m, 2H), 2.84 - 2.70 (m, 2H), 2.48 (s, 3H), 2.47 - 2.35 (m, 1H), 2.27-2.19 (m, 1H) | 674.2 |

(continued)

| Example Number | Structure | Name | Intermediates | $^1H$ NMR (400 MHz) δ ppm | MI |
|---|---|---|---|---|---|
| **112** | 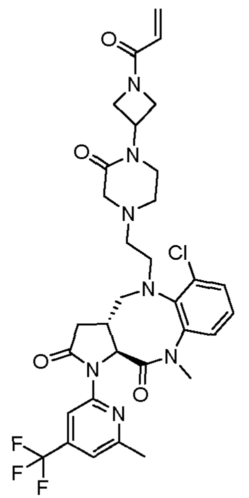 | (3a*R*,11a*S*)-5-(2-(4-(1-acryloylazetidin-3-yl)-3-oxopiperazin-1-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 7b** and **Intermediate A30** | ($CDCl_3$) 8.33 (s, 1H), 7.48 - 7.41 (m, 1H), 7.37 - 7.28 (m, 2H), 7.06 (s, 1H), 6.40 - 6.30 (m, 1H), 6.22 - 6.13 (m, 1H), 5.70 (d, *J* = 10.4 Hz, 1H), 5.35 - 5.22 (m, 1H), 4.61 (d, *J* = 9.2 Hz, 1H), 4.43 (t, *J* = 8.8 Hz, 1H), 4.33 - 4.21 (m, 2H), 4.20 - 4.09 (m, 1H), 3.61 - 3.52 (m, 1H), 3.52 - 3.38 (m, 2H), 3.36 (s, 3H), 3.25 - 3.07 (m, 4H), 3.06 - 2.97 (m, 1H), 2.96 - 2.84 (m, 1H), 2.83 - 2.67 (m, 3H), 2.48 (s, 3H), 2.48 - 2.41 (m, 2H), 2.27 - 2.17 (m, 1H) | 674.1 |
| **113** | 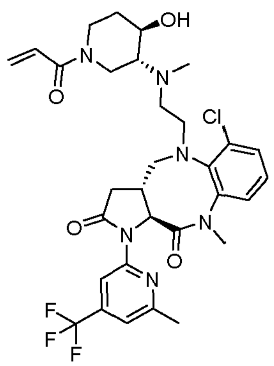 | (3a*R*,11a*S*)-5-(2-(((*rel-trans*)-1-acryloyl-4-hydroxypiperidin-3-yl)(methyl)amino)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 7b** and **Intermediate A31** | ($CDCl_3$) 8.33 (s, 1H), 7.46 (d, *J* = 6.0 Hz, 1H), 7.32 - 7.28 (m, 2H), 7.05 (s, 1H), 6.64 - 6.48 (m, 1H), 6.26 (d, *J* = 16.8 Hz, 1H), 5.70 (d, *J* = 10.4 Hz, 1H), 4.75 - 4.71 (m, 1H), 4.65 - 4.61 (m, 1H), 4.05 - 3.95 (m, 1H), 3.64 - 3.53 (m, 2H), 3.43 - 3.36 (m, 3H), 3.22 - 3.08 (m, 2H), 3.06 - 2.90 (m, 3H), 2.78 - 2.71 (m, 2H), 2.74 - 2.55 (m, 2H), 2.48 (s, 3H), 2.47 - 2.42 (m, 1H), 2.33 (s, 2H), 2.27 (s, 2H), 2.24 - 2.19 (m, 1H), 2.15 - 2.08 (m, 1H), 1.50 - 1.38 (m, 1H) | 649.2 |

(continued)

| Example Number | Structure | Name | Intermediates | $^1$H NMR (400 MHz) δ ppm | MI |
|---|---|---|---|---|---|
| **114** | 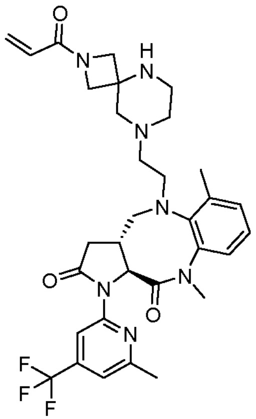 | (3a*R*,11a*S*)-5-(2-(2-acryloyl-2,5,8-triazaspiro[3.5]nonan-8-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 7i** and **Intermediate A32** | ($CDCl_3$) 8.34 (s, 1H), 7.26 - 7.18 (m, 3H), 7.05 (s, 1H), 6.39 - 6.31 (m, 1H), 6.25 - 6.13 (m, 1H), 5.71 - 5.66 (m, 1H), 4.70 (d, *J* = 9.6 Hz, 1H), 4.05 - 3.83 (m, 3H), 3.76 - 3.72 (m, 1H), 3.63 - 3.57 (m, 1H), 3.37 (s, 3H), 3.16 - 2.92 (m, 3H), 2.90 - 2.81 (m, 2H), 2.80 - 2.67 (m, 2H), 2.49 (s, 4H), 2.46 - 2.26 (m, 8H), 2.20 (dd, *J* = 16.8, 11.6 Hz, 1H), 1.26 - 1.23 (m, 1H) | 626.2 |
| **115** | 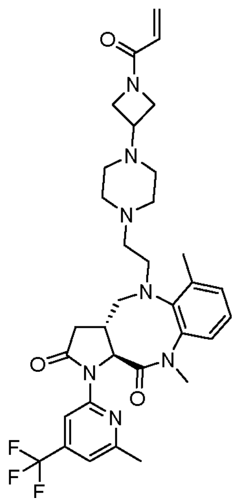 | (3a*R*,11a*S*)-5-(2-(4-(1-acryloylazetidin-3-yl)piperazin-1-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 7i** and **Intermediate A1** | ($CDCl_3$) 8.33 (s, 1H), 7.26 - 7.17 (m, 3H), 7.05 (s, 1H), 6.35 - 6.29 (m, 1H), 6.23 - 6.11 (m, 1H), 5.66 (dd, *J* = 10.4, 1.6 Hz, 1H), 4.70 (d, *J* = 9.6 Hz, 1H), 4.21 - 4.19 (m, 1H), 4.14 - 4.01 (m, 2H), 3.94 - 3.92 (m, 1H), 3.69 - 3.58 (m, 1H), 3.38 (s, 3H), 3.27 - 2.89 (m, 5H), 2.85 - 2.65 (m, 3H), 2.49 (s, 3H) 2.48 - 2.40 (m, 8H), 2.39 (s, 3H), 2.19 (dd, *J* = 16.8, 11.6 Hz, 1H) | 640.3 |

The **Examples** in **Table 10** were prepared using methods similar to those described in the synthesis of **Example 1,** using the listed **Intermediates** in step a, and an additional chiral SFC step was conducted.

**Table 10**

| Example Number | Structure | Name | Intermediates | $^1$H NMR (400 MHz) δ ppm | MI |
|---|---|---|---|---|---|
| **116a** | 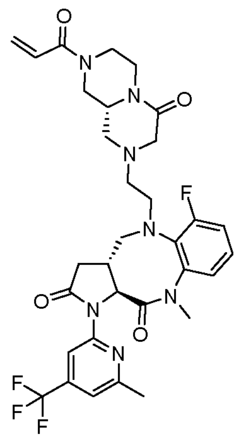 | (3a*R*,11a*S*)-5-(2-((*R*/*S*)-8-acryloyl-4-oxooctahydro-2*H*-pyrazino[1,2-a]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 7a** and **Intermediate A21** | ($CDCl_3$) 8.33 (s, 1H), 7.42 - 7.32 (m, 1H), 7.24 (d, *J* = 8.4 Hz, 1H), 7.16 (t, *J* = 9.2 Hz, 1H), 7.06 (s, 1H), 6.61 - 6.51 (m, 1H), 6.38 - 6.30 (m, 1H), 5.81 - 5.74 (m, 1H), 4.60 (d, *J* = 9.2 Hz, 3H), 4.06 - 3.86 (m, 1H), 3.70 - 3.51 (m, 2H), 3.38 (s, 3H), 3.37 - 3.25 (m, 3H), 3.24 (s, 3H), 3.00 - 2.67 (m, 5H), 2.66 - 2.50 (m, 3H), 2.50 (s, 3H), 2.26 - 2.17 (m, 1H) | 658.3 |
| **116b** | 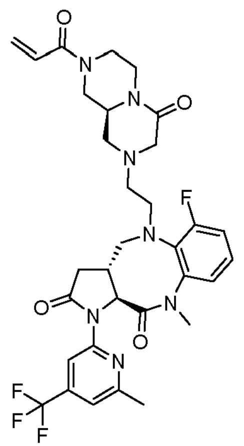 | (3a*R*,11a*S*)-5-(2-((*S*/*R*)-8-acryloyl-4-oxooctahydro-2H-pyrazino[1,2-a]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | ($CDCl_3$) 8.34 (s, 1H), 7.42 - 7.33 (m, 1H), 7.25 (d, *J* = 8.0 Hz, 1H), 7.17 (t, *J* = 9.2 Hz, 1H), 7.07 (s, 1H), 6.65 - 6.48 (m, 1H), 6.35 (dd, *J* = 16.8, 1.6 Hz, 1H), 5.77 (d, *J* = 10.8 Hz, 1H), 4.62 (d, *J* = 9.2 Hz, 3H), 4.10 - 3.86 (m, 1H), 3.56 (dd, *J*= 13.2, 4.4 Hz, 2H), 3.36 (s, 3H), 3.35 - 3.21 (m, 3H), 3.20 - 3.10 (m, 1H), 3.09 - 3.02 (m, 1H), 3.01 - 2.90 (m, 2H), 2.90 - 2.81 (m, 1H), 2.80 - 2.60 (m, 3H), 2.58 - 2.51 (m, 4H), 2.49 - 2.36 (m, 2H), 2.23 (dd, *J* = 16.4, 11.2 Hz, 1H) | 658.1 |

(continued)

| Example Number | Structure | Name | Intermediates | $^1$H NMR (400 MHz) δ ppm | MI |
|---|---|---|---|---|---|
| **117a** | 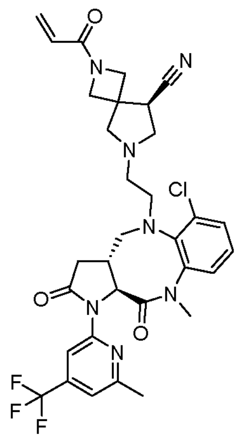 | (*R*/*S*)-2-acryloyl-6-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,6-diazaspiro[3.4]octane-8-carbonitrile | **Intermediate 7a** and **Intermediate A33** | ($CDCl_3$) 8.26 (s, 1H), 7.39 (d, *J* = 6.0 Hz, 1H), 7.29 - 7.27 (m, 2H), 6.99 (s, 1H), 6.28 (dd, *J* = 16.8, 1.6 Hz, 1H), 6.18 - 5.98 (m, 1H), 5.65 (d, *J* = 10.4 Hz, 1H), 4.53 (d, *J* = 9.2 Hz, 1H), 4.48 - 4.30 (m, 1H), 4.17 - 3.89 (m, 3H), 3.55 - 3.47 (m, 1H), 3.40 - 3.35 (m, 4H), 3.25 - 3.05 (m, 3H), 3.04 - 2.75 (m, 5H), 2.70 - 2.64 (m, 2H), 2.59 - 2.45 (m, 1H), 2.41 (s, 3H), 2.15 (dd, *J* = 16.4, 11.2 Hz, 1H) | 656.3 |
| **117b** | 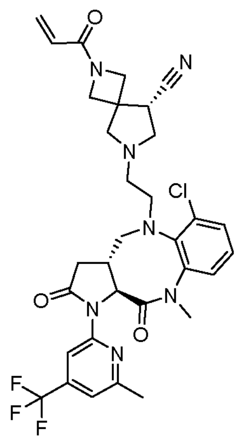 | (*S*/*R*)-2-acryloyl-6-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,6-diazaspiro[3.4]octane-8-carbonitrile | | ($CDCl_3$) 8.34 (s, 1H), 7.48 - 7.44 (m, 1H), 7.36 - 7.29 (m, 2H), 7.06 (s, 1H), 6.41 - 6.31 (m, 1H), 6.26 - 6.09 (m, 1H), 5.75 - 5.70 (m, 1H), 4.61 (d, *J* = 9.2 Hz, 1H), 4.56 - 4.33 (m, 1H), 4.21 - 3.99 (m, 2H), 3.96 (s, 1H), 3.61 - 3.48 (m, 1H), 3.38 (s, 3H), 3.19 - 3.12 (m, 3H), 3.09 - 2.80 (m, 5H), 2.79 - 2.69 (m, 2H), 2.61 - 2.52 (m, 1H), 2.50 - 2.45 (m, 4H), 2.22 (dd, *J* = 16.4, 10.8 Hz, 1H) | 656.3 |

(continued)

| Example Number | Structure | Name | Intermediates | $^1$H NMR (400 MHz) δ ppm | MI |
|---|---|---|---|---|---|
| **118a** | 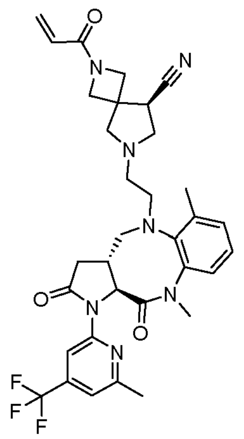 | (*R*/*S*)-2-acryloyl-6-(2-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,6-diazaspiro[3.4]octane-8-carbonitrile | **Intermediate 7i** and **Intermediate A33** | ($CDCl_3$) 8.34 (s, 1H), 7.27 - 7.20 (m, 3H), 7.05 (s, 1H), 6.41 - 6.31 (m, 1H), 6.27 - 6.08 (m, 1H), 5.73 (d, $J$ = 10.4 Hz, 1H), 4.69 (d, $J$ = 9.2 Hz, 1H), 4.56 - 4.34 (m, 1H), 4.22 - 3.92 (m, 3H), 3.67 - 3.53 (m, 1H), 3.38 (s, 3H), 3.30 - 3.05 (m, 3H), 3.04 - 2.90 (m, 3H), 2.89 - 2.81 (m, 2H), 2.80 - 2.68 (m, 2H), 2.67 - 2.51 (m, 2H), 2.50 (s, 3H), 2.38 (s, 3H), 2.26 - 2.14 (m, 1H) | 636.3 |
| **118b** | 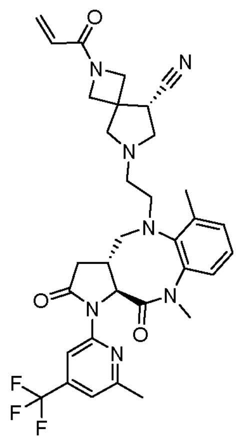 | (*S*/*R*)-2-acryloyl-6-(2-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,6-diazaspiro[3.4]octane-8-carbonitrile | | ($CDCl_3$) 8.34 (s, 1H), 7.27 - 7.20 (m, 3H), 7.05 (s, 1H), 6.40 - 6.33 (m, 1H), 6.26 - 6.05 (m, 1H), 5.76 - 5.69 (m, 1H), 4.69 (d, $J$ = 9.2 Hz, 1H), 4.58 - 4.34 (m, 1H), 4.24 - 3.92 (m, 3H), 3.68 - 3.54 (m, 1H), 3.38 (s, 3H), 3.30 - 3.02 (m, 3H), 3.01 - 2.88 (m, 3H), 2.87 - 2.79 (m, 2H), 2.78 - 2.70 (m, 2H), 2.66 - 2.50 (m, 2H), 2.50 (s, 3H), 2.39 (s, 3H), 2.25 - 2.15 (m, 1H) | 636.3 |

(continued)

| Example Number | Structure | Name | Intermediates | $^1$H NMR (400 MHz) δ ppm | MI |
|---|---|---|---|---|---|
| **119a** | 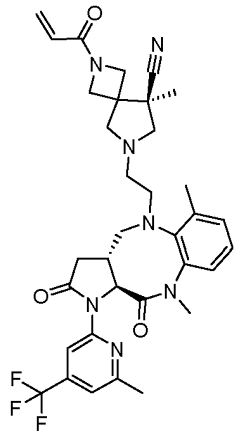 | (*R*/*S*)-2-acryloyl-6-(2-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-8-methyl-2,6-diazaspiro[3.4]octane-8-carbonitrile | **Intermediate 7i** and **Intermediate A34** | ($CDCl_3$) 8.34 (s, 1H), 7.27 - 7.23 (m, 3H), 7.05 (s, 1H), 6.43 - 6.32 (m, 1H), 6.24 - 6.08 (m, 1H), 5.73 (d, *J* = 10.4 Hz, 1H), 4.70 (d, *J* = 9.2 Hz, 1H), 4.49 - 3.99 (m, 3H), 3.95 - 3.73 (m, 1H), 3.60 (dd, *J* = 14.4, 4.4 Hz, 1H), 3.38 (s, 3H), 3.19 (d, *J* = 9.6 Hz, 1H), 3.12 - 2.89 (m, 5H), 2.86 - 2.69 (m, 3H), 2.54 (d, *J* = 7.6 Hz, 2H), 2.49 (s, 3H), 2.39 (s, 3H), 2.20 (dd, *J* = 16.8, 11.6 Hz, 1H), 1.48 - 1.43 (m, 3H) | 650.3 |
| **119b** | 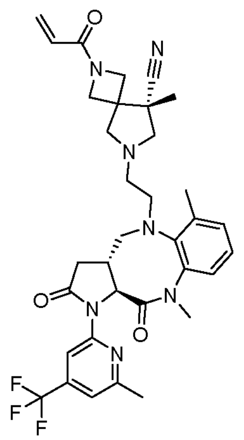 | (*S*/*R*)-2-acryloyl-6-(2-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-8-methyl-2,6-diazaspiro[3.4]octane-8-carbonitrile | | ($CDCl_3$) 8.34 (s, 1H), 7.26 - 7.20 (m, 3H), 7.05 (s, 1H), 6.43 - 6.31 (m, 1H), 6.27 - 6.11 (m, 1H), 5.73 (dd, *J* = 10.4, 1.6 Hz, 1H), 4.69 (d, *J* = 9.6 Hz, 1H), 4.47 - 4.04 (m, 3H), 4.01 - 3.70 (m, 1H), 3.65 - 3.49 (m, 1H), 3.37 (s, 3H), 3.19 - 2.96 (m, 5H), 2.83 - 2.66 (m, 3H), 2.62 - 2.51 (m, 3H), 2.49 (s, 3H), 2.39 (s, 3H), 2.19 (dd, *J* = 16.8, 11.6 Hz, 1H), 1.47 (s, 3H) | 650.4 |

The **Examples** in **Table 11** were prepared using methods similar to those described in the synthesis of **Example 38** or **Example 39,** using the listed **Intermediates** in step a, and the appropriate warhead in step c.

**Table 11**

| Example Number | Structure | Name | Intermediates | $^1$H NMR (400 MHz) δ ppm | MI |
|---|---|---|---|---|---|
| **120** | 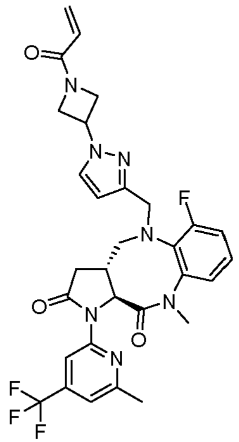 | (3a*R*,11a*S*)-5-((1-(1-acryloylazetidin-3-yl)-1*H*-pyrazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B2** | ($CDCl_3$) 8.29 (s, 1H), 7.39 (d, *J* = 1.6 Hz, 1H), 7.35 - 7.29 (m, 1H), 7.22 - 7.11 (m, 2H), 7.05 (s, 1H), 6.41 - 6.34 (m, 1H), 6.31 - 6.16 (m, 2H), 5.72 (d, *J* = 10.4 Hz, 1H), 5.07 - 5.04 (m, 1H), 4.67 - 4.56 (m, 3H), 4.53 - 4.41 (m, 2H), 4.39 - 4.29 (m, 1H), 4.15 - 4.07 (m, 1H), 3.55 (dd, *J* = 13.6, 4.4 Hz, 1H), 3.35 - 3.25 (m, 3H), 2.98 - 2.89 (m, 1H), 2.86 - 2.73 (m, 1H), 2.63 (dd, *J* = 16.8, 8.4 Hz, 1H), 2.49 (s, 3H), 2.16 (dd, *J* = 16.8, 11.6 Hz,1H) | 612.5 |
| **121** | 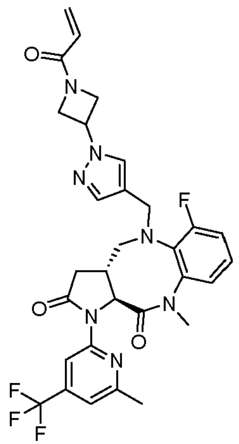 | (3a*R*,11a*S*)-5-((1-(1-acryloylazetidin-3-yl)-1*H*-pyrazol-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B5** | ($CDCl_3$) 8.31 (s, 1H), 7.45 (d, *J* = 1.2 Hz, 1H), 7.39 (s, 1H), 7.37 - 7.30 (m, 1H), 7.24 - 7.11 (m, 2H), 7.06 (s, 1H), 6.42 - 6.34 (m, 1H), 6.27 - 6.16 (m, 1H), 5.72 (d, *J* = 10.4 Hz, 1H), 5.09 - 5.06 (m, 1H), 4.69 - 4.57 (m, 3H), 4.56 - 4.46 (m, 1H), 4.40 - 4.31 (m, 1H), 4.26 - 4.17 (m, 1H), 4.15 - 4.06 (m, 1H), 3.55 (dd, *J* = 13.6, 5.2 Hz, 1H), 3.25 (s, 3H), 2.98 - 2.89 (m, 1H), 2.87 - 2.75 (m, 1H), 2.72 - 2.62 (m, 1H), 2.50 (s, 3H), 2.25 - 2.11 (m, 1H) | 612.3 |

(continued)

| Example Number | Structure | Name | Intermediates | $^1$H NMR (400 MHz) δ ppm | MI |
|---|---|---|---|---|---|
| **122** | 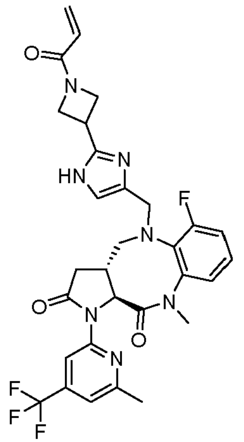 | (3a*R*,11a*S*)-5-((2-(1-acryloylazetidin-3-yl)-1*H*-imidazol-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B6** | ($CDCl_3$) 8.31 (s, 1H), 7.40 - 7.30 (m, 1H), 7.24 - 7.13 (m, 2H), 7.08 (s, 1H), 6.83 (s, 1H), 6.39 - 6.28 (m, 1H), 6.24 - 6.14 (m, 1H), 5.77 - 5.60 (m, 1H), 4.63 (d, *J* = 9.6 Hz, 1H), 4.56 - 4.54 (m, 1H), 4.51 - 4.31 (m, 2H), 4.29 - 4.11 (m, 2H), 4.04 - 3.91 (m, 1H), 3.62 - 3.58 (m, 1H), 3.25 (s, 3H), 3.02 - 2.91 (m, 1H), 2.90 - 2.76 (m, 1H), 2.73 - 2.59 (m, 1H), 2.58 - 2.37 (m, 4H), 2.22 - 2.14 (m, 1H) | 612.2 |
| **123** | 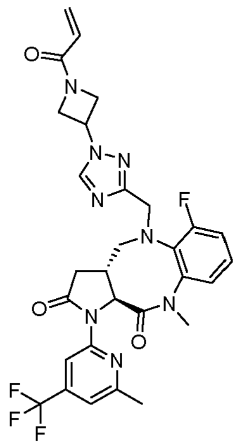 | (3a*R*,11a*S*)-5-((1-(1-acryloylazetidin-3-yl)-1*H*-1,2,4-triazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B3** | ($CDCl_3$) 8.30 (s, 1H), 8.06 (s, 1H), 7.37 - 7.30 (m, 1H), 7.24 - 7.10 (m, 2H), 7.04 (s, 1H), 6.49 - 6.33 (m, 1H), 6.30 - 6.13 (m, 1H), 5.82 - 5.68 (m, 1H), 5.16 - 5.10 (m, 1H), 4.76 - 4.31 (m, 6H), 4.29 - 4.14 (m, 1H), 3.91 - 3.75 (m, 1H), 3.40 - 3.18 (m, 3H), 3.08 - 2.61 (m, 3H), 2.48 (s, 3H), 2.27 - 2.11 (m, 1H) | 613.3 |

(continued)

| Example Number | Structure | Name | Intermediates | $^1H$ NMR (400 MHz) δ ppm | MI |
|---|---|---|---|---|---|
| **124** | 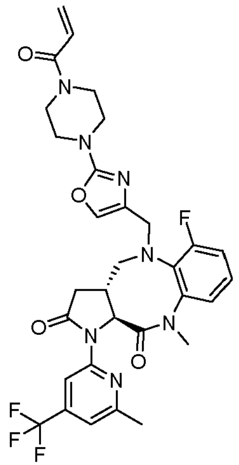 | (3a*R*,11a*S*)-5-((2-(4-acryloylpiperazin-1-yl)oxazol-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B7** | ($CDCl_3$) 8.42 - 8.29 (m, 1H), 7.51 - 7.29 (m, 2H), 7.25 - 7.11 (m, 2H), 7.08 (s, 1H), 6.61 - 6.54 (m, 1H), 6.37 - 6.30 (m, 1H), 5.79 - 5.75 (m, 1H), 4.89 - 4.62 (m, 1H), 4.30 - 4.26 (m, 1H), 4.05 - 4.01 (m, 1H), 3.94 - 3.49 (m, 9H), 3.41 - 3.25 (m, 3H), 3.07 - 2.78 (m, 2H), 2.72 - 2.67 (m, 1H), 2.52 (s, 3H), 2.27 - 2.14 (m, 1H) | 642.3 |
| **125** | 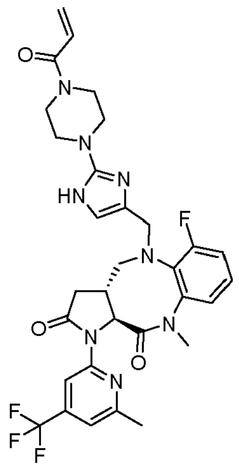 | (3a*R*,11a*S*)-5-((2-(4-acryloylpiperazin-1-yl)-1*H*-imidazol-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B8** | (DMSO-$d_6$) 10.88 - 10.64 (m, 1H), 8.16 (s, 1H), 7.41 - 7.33 (m, 2H), 7.31 - 7.22 (m, 2H), 6.88 - 6.78 (m, 1H), 6.44 (s, 1H), 6.16 - 6.08 (m, 1H), 5.73 - 5.65 (m, 1H), 4.60 (d, *J* = 9.6 Hz, 1H), 3.98 - 3.92 (m, 1H), 3.64 - 3.54 (m, 4H), 3.54 - 3.45 (m, 1H), 3.31 - 3.28 (m, 2H), 3.19 - 3.07 (m, 7H), 3.01 - 2.89 (m, 1H), 2.60 - 2.54 (m, 1H), 2.49 - 2.48 (m, 3H), 2.42 - 2.36 (m, 1H) | 641.3 |

(continued)

| Example Number | Structure | Name | Intermediates | $^1$H NMR (400 MHz) δ ppm | MI |
|---|---|---|---|---|---|
| **126** | 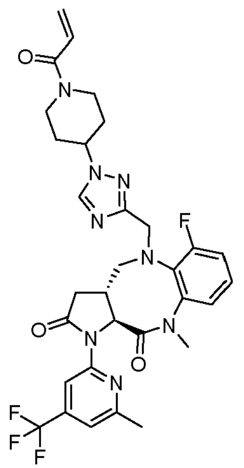 | (3a*R*,11a*S*)-5-((1-(1-acryloylpiperidin-4-yl)-1*H*-1,2,4-triazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B9** | ($CDCl_3$) 8.31 (s, 1H), 7.99 (s, 1H), 7.35 - 7.30 (m, 1H), 7.19 - 7.10 (m, 2H), 7.04 (s, 1H), 6.63 - 6.57 (m, 1H), 6.33 - 6.28 (m, 1H), 5.75 - 5.70 (m, 1H), 4.75 - 4.70 (m, 1H), 4.65 - 4.60 (m, 1H), 4.41 - 4.35 (m, 2H), 4.25 - 4.20 (m, 1H), 4.20 - 4.08 (m, 1H), 3.84 - 3.78 (m, 1H), 3.25 (s, 3H), 3.24 - 3.18 (m, 1H), 3.05 - 2.95 (m, 1H), 2.95 - 2.80 (m, 2H), 2.75 - 2.65 (m, 1H), 2.45 (s, 3H), 2.28 - 2.15 (m, 3H), 2.04 - 1.85 (m, 2H) | 641.3 |
| **127** | 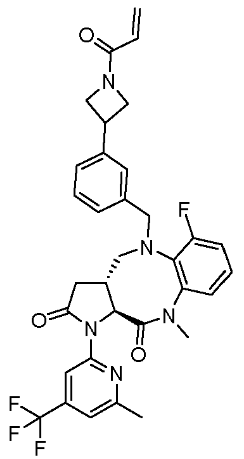 | (3a*R*,11a*S*)-5-(3-(1-acryloylazetidin-3-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B10** | ($CDCl_3$) 8.31 (s, 1H), 7.37 - 7.28 (m, 3H), 7.24 - 7.13 (m, 4H), 7.06 (s, 1H), 6.43 - 6.20 (m, 2H), 5.76 - 5.63 (m, 1H), 4.71 - 4.58 (m, 2H), 4.54 - 4.43 (m, 1H), 4.36 - 4.13 (m, 3H), 4.08 - 4.04 (m, 1H), 3.84 - 3.82 (m, 1H), 3.55 - 3.45 (m, 1H), 3.24 (d, *J* = 19.6 Hz, 3H), 3.01 - 2.90 (m, 1H), 2.88 - 2.69 (m, 1H), 2.64 - 2.57 ( m, 1H), 2.49 (s, 3H), 2.20 - 2.11 (m, 1H) | 622.0 |

(continued)

| Example Number | Structure | Name | Intermediates | $^1$H NMR (400 MHz) δ ppm | MI |
|---|---|---|---|---|---|
| **128** | 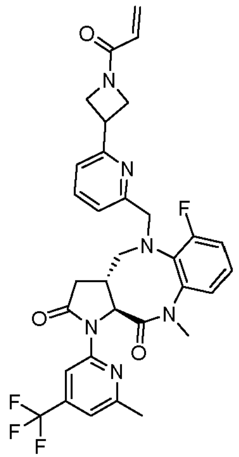 | (3a*R*,11a*S*)-5-((6-(1-acryloylazetidin-3-yl)pyridin-2-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B11** | ($CDCl_3$) 8.31 (s, 1H), 7.73 - 7.56 (m, 1H), 7.44 - 7.30 (m, 2H), 7.24 - 7.13 (m, 2H), 7.12 - 7.04 (m, 2H), 6.43 - 6.34 (m, 1H), 6.33 - 6.21 (m, 1H), 5.77 - 5.62 (m, 1H), 4.69 (t, *J* = 9.2 Hz, 1H), 4.60 - 4.37 (m, 4H), 4.35 - 4.24 (m, 1H), 4.22 - 4.13 (m, 1H), 3.98 - 3.86 (m, 1H), 3.65 - 3.51 (m, 1H), 3.39 - 3.17 (m, 3H), 3.09 - 2.99 (m, 1H), 2.96 - 2.80 (m, 1H), 2.70 - 2.60 (m, 1H), 2.50 (d, *J* = 2.4 Hz, 3H), 2.27 - 2.10 (m, 1H) | 623.2 |
| **129** | 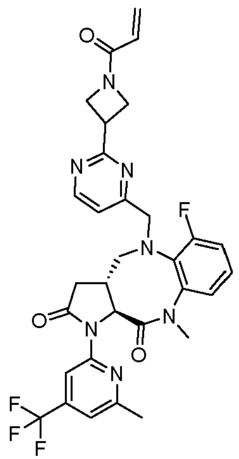 | (3a*R*,11a*S*)-5-((2-(1-acryloylazetidin-3-yl)pyrimidin-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B12** | ($CDCl_3$) 8.74 - 8.67 (m, 1H), 8.32 (s, 1H), 7.50 - 7.42 (m, 1H), 7.41 - 7.33 (m, 1H), 7.26 - 7.15 (m, 2H), 7.07 (s, 1H), 6.41 - 6.32 (m, 1H), 6.30 - 6.20 (m, 1H), 5.69 (d, *J* = 10.0 Hz, 1H), 4.70 (dd, *J* = 9.6, 3.2 Hz, 1H), 4.64 - 4.53 (m, 2H), 4.50 - 4.40 (m, 2H), 4.33 - 4.23 (m, 2H), 4.13 - 4.03 (m, 1H), 3.56 - 3.47 (m, 1H), 3.34 (d, *J* = 11.6 Hz, 3H), 3.14 - 3.03 (m, 1H), 3.01 - 2.90 (m, 1H), 2.73 - 2.63 (m, 1H), 2.50 (s, 3H), 2.26 - 2.13 (m, 1H) | 624.1 |

(continued)

| Example Number | Structure | Name | Intermediates | $^1$H NMR (400 MHz) δ ppm | MI |
|---|---|---|---|---|---|
| **130** | 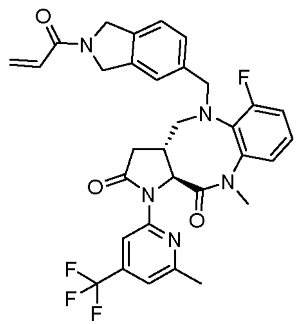 | (3a*R*,11a*S*)-5-((2-acryloylisoindolin-5-yl) methyl)-6-fluoro-10-methyl-1-(6-methyl-4-(trifluoromethyl)pyridin-2-yl)-1,3a,4,5,10,11a-hexahydro-2*H*-benzo [*b*]pyrrolo[2,3-*f*][1,4]diazocine-2,11(3*H*)-dione | **Intermediate 5a** and **Intermediate B18** | ($CDCl_3$) 8.31 (s, 1H), 7.36 - 7.25 (m, 2H), 7.24 - 7.12 (m, 4H), 7.06 (s, 1H), 6.60 - 6.42 (m, 2H), 5.78 - 5.74 (m, 1H), 4.89 (d, *J* = 6.4 Hz, 2H), 4.84 (s, 2H), 4.66 (d, *J* = 9.6 Hz, 1H), 4.35 - 4.19 (m, 2H), 3.56 - 3.46 (m, 1H), 3.27 (d, *J* = 6.4 Hz, 3H), 3.00 - 2.91 (m, 1H), 2.88 - 2.76 (m, 1H), 2.67 - 2.60 (m, 1H), 2.50 (d, *J* = 2.4 Hz, 3H), 2.20 - 2.13 (m, 1H) | 608.1 |
| **131** | 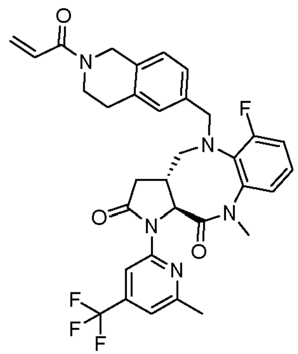 | (3a*R*,11a*S*)-5-((2-acryloyl-1,2,3,4-tetrahydroisoquinolin-6-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B17** | ($CDCl_3$) 8.31 (s, 1H), 7.37 - 7.29 (m, 1H), 7.22 - 6.99 (m, 6H), 6.67-6.60 (m, 1H), 6.35 - 6.30 (m, 1H), 5.75 - 5.71 (m, 1H), 4.79 - 4.67 (m, 2H), 4.66 (d, *J* = 9.6 Hz, 1H), 4.31 - 4.24 (m, 1H), 4.20 - 4.13 (m, 1H), 3.97 - 3.72 (m, 2H), 3.51 - 3.47 (m, 1H), 3.27 (s, 3H), 3.00 - 2.74 (m, 4H), 2.63 - 2.59 (m, 1H), 2.50 (s, 3H), 2.18 - 2.11 (m, 1H) | 622.0 |
| **132** | 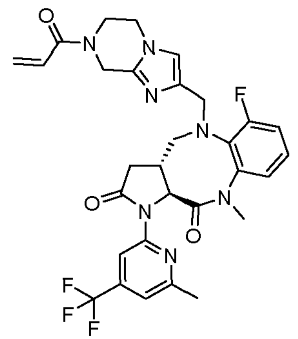 | (3aR,11aS)-5-((7-acryloyl-5,6,7,8-tetrahydroimidazo[1,2-a]pyrazin-2-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B19** | ($CDCl_3$) 8.30 (s, 1H), 7.37 - 7.31 (m, 1H), 7.23 (d, *J* = 8.0 Hz, 1H), 7.20 - 7.14 (m, 1H), 7.07 (s, 1H), 6.82 (s, 1H), 6.63 - 6.56 (m, 1H), 6.42 - 6.36 (m, 1H), 5.82 (dd, *J* = 10.4, 1.6 Hz, 1H), 4.85 (s, 2H), 4.65 (d, *J*= 9.6 Hz, 1H), 4.48 - 4.35 (m, 1H), 4.13 - 3.95 (m, 5H), 3.59 (dd, *J* = 13.6, 4.4 Hz, 1H), 3.32 (s, 3H), 3.00 - 2.92 (m, 1H), 2.91 - 2.81 (m, 1H), 2.66 (dd, *J* = 16.8, 8.0 Hz, 1H), 2.51 (s, 3H), 2.18 (dd, *J* = 16.8, 11.6 Hz, 1H) | 612.1 |

(continued)

| Example Number | Structure | Name | Intermediates | $^1$H NMR (400 MHz) δ ppm | MI |
|---|---|---|---|---|---|
| **133** | 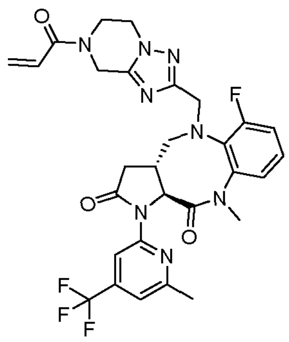 | (3aR,11aS)-5-((7-acryloyl-5,6,7,8-tetrahydro-[1,2,4]triazolo[1,5-a]pyrazin-2-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate** 5a and Intermediate B20 | ($CDCl_3$) 8.33 (s, 1H), 7.38 - 7.32 (m, 1H), 7.24 - 7.14 (m, 2H), 7.06 (s, 1H), 6.68 - 6.55 (m, 1H), 6.47 - 6.38 (m, 1H), 5.87 (dd, *J* = 10.4, 1.6 Hz, 1H), 4.89 (s, 2H), 4.66 (d, *J* = 9.2 Hz, 1H), 4.41 - 4.34 (m, 1H), 4.27 - 4.00 (m, 5H), 3.87 - 3.79 (m, 1H), 3.31 (s, 3H), 3.08 - 2.90 (m, 2H), 2.73 (dd, *J* = 16.8, 8.0 Hz, 1H), 2.50 (s, 3H), 2.21 (dd, *J* = 16.8, 11.2 Hz, 1H) | 613.3 |
| **134** | 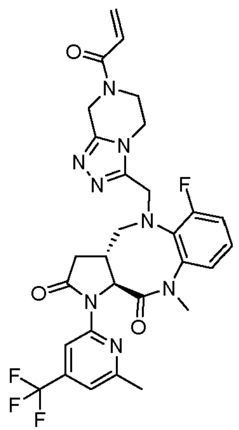 | (3a*R*,11a*S*)-5-((7-acryloyl-5,6,7,8-tetrahydro-[1,2,4]triazolo[4,3-a]pyrazin-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B21** | ($CDCl_3$) 8.31 (s, 1H), 7.42 - 7.37 (m, 1H), 7.26 - 7.16 (m, 2H), 7.07 (s, 1H), 6.63 - 6.56 (m, 1H), 6.47 - 6.32 (m, 1H), 5.85 (d, *J* = 10.8 Hz, 1H), 5.32 - 5.00 (m, 1H), 4.98 - 4.78 (m, 1H), 4.60 (d, *J* = 9.6 Hz, 2H), 4.44 - 4.30 (m, 1H), 4.22 - 4.15 (m, 1H), 4.08 - 3.96 (m, 1H), 3.89 - 3.78 (m, 1H), 3.65 (dd, *J* = 14.4, 4.4 Hz, 1H), 3.39 - 3.22 (m, 3H), 2.96 (t, *J* = 12.4 Hz, 1H), 2.77 - 2.65 (m, 2H), 2.50 (s, 3H), 2.26 - 2.12 (m, 2H) | 613.3 |

(continued)

| Example Number | Structure | Name | Intermediates | $^1$H NMR (400 MHz) δ ppm | MI |
|---|---|---|---|---|---|
| **135** | 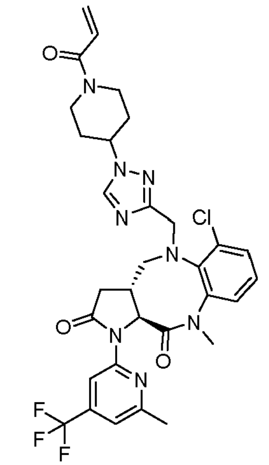 | (3a*R*,11a*S*)-5-((1-(1-acryloylpiperidin-4-yl)-1*H*-1,2,4-triazol-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B9** | ($CDCl_3$) 8.31 (s, 1H), 8.04 (s, 1H), 7.46 - 7.43 (m, 1H), 7.33 - 7.28 (m, 2H), 7.04 (s, 1H), 6.59 (dd, *J* = 16.8, 10.4 Hz, 1H), 6.31 (dd, *J* = 16.8, 1.6 Hz, 1H), 5.73 (dd, *J* = 10.6, 1.8 Hz, 1H), 4.84 - 4.66 (m, 1H), 4.62 (d, *J* = 9.6 Hz, 1H), 4.42 - 4.32 (m, 2H), 4.22 - 4.04 (m, 2H), 3.83 - 3.77 (m, 1H), 3.27 (s, 3H), 3.25 - 3.17 (m, 1H), 3.09 - 3.03 (m, 1H), 3.00 - 2.82 (m, 2H), 2.74 - 2.67 (m, 1H), 2.47 (s, 3H), 2.28 - 2.13 (m, 3H), 2.02 - 1.90 (m, 2H) | 657.3 |
| **136** | 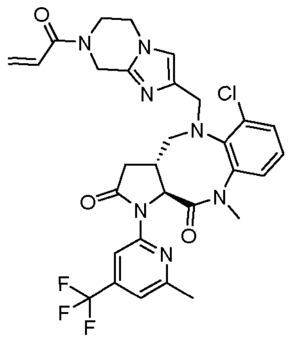 | (3a*R*,11a*S*)-5-((7-acryloyl-5,6,7,8-tetrahydroimidazo[1,2-a]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B19** | ($CDCl_3$) 8.31 (s, 1H), 7.50 - 7.45 (m, 1H), 7.34 - 7.29 (m, 2H), 7.06 (s, 1H), 6.87 (s, 1H), 6.63 - 6.55 (m, 1H), 6.41 - 6.36 (m, 1H), 5.83 - 5.78 (m, 1H), 4.80 (s, 2H), 4.65 (d, *J* = 9.2 Hz, 1H), 4.38 - 4.25 (m, 1H), 4.15 - 3.95 (m, 5H), 3.63 - 3.60 (m, 1H), 3.34 (s, 3H), 3.10 - 2.94 (m, 2H), 2.72 - 2.64 (m, 1H), 2.49 (s, 3H), 2.23 - 2.15 (m, 1H) | 628.3 |

(continued)

| Example Number | Structure | Name | Intermediates | $^1$H NMR (400 MHz) δ ppm | MI |
|---|---|---|---|---|---|
| **137** | 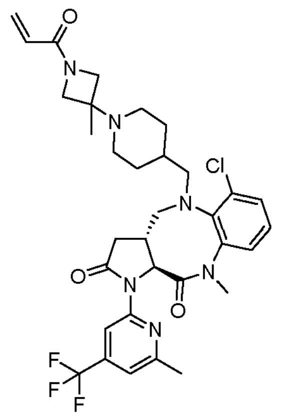 | (3a*R*,11a*S*)-5-((1-(1-acryloyl-3-methylazetidin-3-yl)piperidin-4-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B22** | ($CDCl_3$) 8.34 (s, 1H), 7.48 - 7.45 (m, 1H), 7.36 - 7.30 (m, 2H), 7.07 (s, 1H), 6.37 - 6.32 (m, 1H), 6.24 - 6.11 (m, 1H), 5.70 - 5.63 (m, 1H), 4.64 (d, *J* = 9.2 Hz, 1H), 3.85 - 3.73 (m, 2H), 3.54 - 3.49 (m, 1H), 3.38 (s, 3H), 3.07 - 3.00 (m, 1H), 2.98 - 2.80 (m, 4H), 2.74 (m, 2H), 2.50 (s, 3H), 2.29 - 2.20 (m, 2H), 2.00 - 1.90 (m, 2H), 1.87 - 1.80 (m, 1H), 1.78 - 1.49 (m, 5H), 1.41 (s, 3H) | 659.3 |
| **138** | 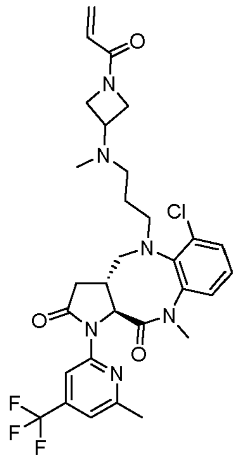 | (3a*R*,11a*S*)-5-(3-((1-acryloylazetidin-3-yl)(methyl)amino)propyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B23** | ($CD_3OD$) 8.32 (s, 1H), 7.58 - 7.49 (m, 2H), 7.44 - 7.36 (m, 1H), 7.23 (s, 1H), 6.41 - 6.30 (m, 1H), 6.29 - 6.22 (m, 1H), 5.81 - 5.70 (m, 1H), 4.70 (d, *J* = 9.6 Hz, 1H), 4.38 - 4.28 (m, 1H), 4.15 - 3.97 (m, 2H), 3.93 - 3.76 (m, 1H), 3.61 (m, 1H), 3.41 (s, 3H), 3.32 - 3.28 (m, 1H), 3.20 - 3.04 (m, 3H), 2.98 - 2.84 (m, 1H), 2.71 - 2.70 (m, 1H), 2.55 (s, 3H), 2.46 - 2.34 (m, 3H), 2.16 (s, 3H), 1.71 - 1.44 (m, 2H) | 619.3 |

(continued)

| Example Number | Structure | Name | Intermediates | $^1$H NMR (400 MHz) δ ppm | MI |
|---|---|---|---|---|---|
| **139** | 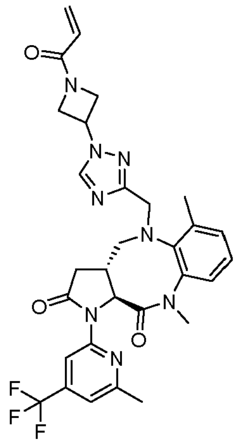 | (3a*R*,11a*S*)-5-((1-(1-acryloylazetidin-3-yl)-1*H*-1,2,4-triazol-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 6c** and **Intermediate B3** | ($CDCl_3$) 8.33 (s, 1H), 8.04 (s, 1H), 7.25 - 7.20 (m, 3H), 7.04 (s, 1H), 6.46 - 6.34 (m, 1H), 6.28 - 6.16 (m, 1H), 5.75 (d, *J* = 10.0 Hz, 1H), 5.18 - 5.10 (m, 1H), 4.74 (d, *J* = 9.2 Hz, 1H), 4.68 - 4.48 (m, 3H), 4.45 - 4.29 (m, 1H), 4.28 - 4.16 (m, 2H), 3.76 (dd, *J* = 14.4, 4.4 Hz, 1H), 3.45 - 3.33 (m, 3H), 3.17 - 2.93 (m, 1H), 2.90 - 2.82 (m, 1H), 2.79 - 2.67 (m, 1H), 2.49 (s, 3H), 2.38 (s, 3H), 2.19 (dd, *J* = 16.8, 11.4 Hz, 1H) | 609.3 |

The **Examples** in **Table 12** were prepared using methods similar to those described in the synthesis of **Example 38** or **Example 39,** using the listed **Intermediates** in step a, and an additional chiral SFC step was conducted.

**Table 12**

| Example Number | Structure | Name | Intermediates | $^1$H NMR (400 MHz) δ ppm | MI |
|---|---|---|---|---|---|
| **140a** | 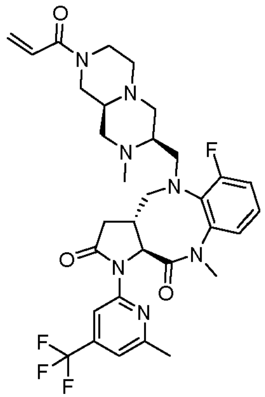 | (3a*R*,11a*S*)-5-(((3*S*,9a*S*/*R*)-8-acryloyl-2-methyloctahydro-2*H*-pyrazino[1,2-a]pyrazin-3-yl) methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B24** | ($CDCl_3$) 8.30 (s, 1H), 7.42 - 7.34 (m, 1H), 7.24 (d, *J* = 9.2 Hz, 1H), 7.20 - 7.14 (m, 1H), 7.07 (s, 1H), 6.57 - 6.46 (m, 1H), 6.33 - 6.24 (m, 1H), 5.71 (d, *J* = 11.2 Hz, 1H), 4.64 - 4.48 (m, 2H), 3.98 - 3.69 (m, 2H), 3.53 - 5.40 (m, 2H), 3.36 (s, 3H), 3.31 - 3.15 (m, 2H), 3.06 - 2.80 (m, 6H), 2.79 - 2.65 (m, 3H), 2.51 (s, 4H), 2.48 - 2.25 (m, 3H), 2.24 - 2.17 (m, 2H) | 644.3 |
| **140b** | 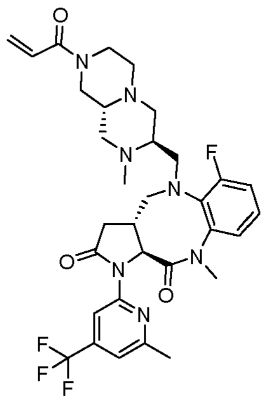 | (3a*R*,11a*S*)-5-(((3S,9a*R*/*S*)-8-acryloyl-2-methyloctahydro-2*H*-pyrazino[1,2-*a*]pyrazin-3-yl) methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | ($CDCl_3$) 8.32 (s, 1H), 7.44 - 7.38 (m, 1H), 7.27 - 7.25 (m, 1H), 7.22 - 7.16 (m, 1H), 7.08 (s, 1H), 6.57 - 6.46 (m, 1H), 6.34 - 6.26 (m, 1H), 5.72 (d, *J* = 9.6 Hz, 1H), 4.64 - 4.48 (m, 2H), 4.00 - 3.82 (m, 1H), 3.67 - 3.51 (m, 3H), 3.34 (s, 3H), 3.05 - 2.98 (m, 1H), 2.93 - 2.81 (m 5H), 2.80 - 2.59 (m, 5H), 2.50 (s, 3H), 2.49 - 2.33 (m, 4H), 2.25 - 2.16 (m, 2H) | 644.3 |

(continued)

| Example Number | Structure | Name | Intermediates | $^1H$ NMR (400 MHz) δ ppm | MI |
|---|---|---|---|---|---|
| **141a** | 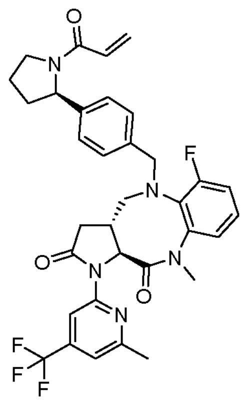 | (3a*R*,11a*S*)-5-(4-((*R*/*S*)-1-acryloylpyrrolidin-2-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5a** and **Intermediate B16** | ($CDCl_3$) 8.30 (s, 1H), 7.35 - 7.30 (m, 1H), 7.32 - 7.28 (m, 1H), 7.26 - 7.22 (m, 1H), 7.20 - 7.13 (m, 2 H), 7.12 - 7.08 (m, 2H), 7.06 (s, 1H), 6.60 - 6.07 (m, 2H), 5.71 - 5.42 (m, 1 H), 5.25 - 5.01 (m, 1H), 4.65 (d, *J* = 9.6 Hz, 1H), 4.33 - 4.10 (m, 2H), 3.85 - 3.69 (m, 2H), 3.55 - 3.46 (m, 1H), 3.22 (s, 3H), 3.01 - 2.86 (m, 1H), 2.85 - 2.75 (m, 1H), 2.66 - 2.59 (m, 1H), 2.49 (s, 3H), 2.42 - 2.23 (m, 1H), 2.21 - 2.09 (m, 1H), 2.03 - 1.93 (m, 1H), 1.92 - 1.83 (m, 2H) | 636.2 |
| **141b** | 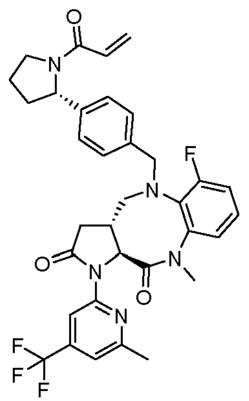 | (3a*R*,11a*S*)-5-(4-((*S*/*R*)-1-acryloylpyrrolidin-2-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | ($CDCl_3$) 8.30 (s, 1H), 7.32 - 7.27 (m, 1H), 7.24 (d, *J* = 8.0 Hz, 2H), 7.21 - 7.07 (m, 4H), 7.06 (s, 1H), 6.59 - 6.02 (m, 2H), 5.72 - 5.40 (m, 1H), 5.27 - 5.00 (m, 1H), 4.72 - 4.60 (m, 1H), 4.32 - 4.10 (m, 2H),3.88 - 3.68 (m, 2H), 3.57 - 3.45 (m, 1H), 3.27 - 3.18 (m, 3H), 3.01 - 2.89 (m, 1H), 2.89 - 2.79 (m, 1H), 2.69 - 2.60 (m, 1H), 2.52 - 2.45 (m, 3H), 2.44 - 2.10 (m, 2H), 2.02 - 1.92 (m, 1H), 1.91 - 1.87 (m, 1H), 1.87 - 1.82 (m, 1H) | 636.1 |

(continued)

| Example Number | Structure | Name | Intermediates | $^1$H NMR (400 MHz) δ ppm | MI |
|---|---|---|---|---|---|
| **142a** | 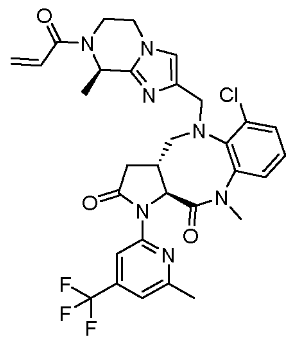 | (3a*R*,11a*S*)-5-(((*R*/*S*)-7-acryloyl-8-methyl-5,6,7,8-tetrahydroimidazo[1,2-a]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B25** | ($CDCl_3$) 8.31 (s, 1H), 7.48 - 7.45 (m, 1H), 7.37 - 7.28 (m, 2H), 7.06 (s, 1H), 6.86 (s, 1H), 6.66 - 6.54 (m, 1H), 6.47 - 6.35 (m, 1H), 5.84 - 5.80 (m, 1H), 5.53 - 5.25 (m, 1H), 5.15 - 4.85 (m, 1H), 4.65 (d, *J* = 9.6 Hz, 1H), 4.49 - 4.29 (m, 1H), 4.17 - 4.12 (m, 1H), 4.05 - 3.89 (m, 2H), 3.67 - 3.51 (m, 1H), 3.34 (s, 3H), 3.33 - 3.15 (s, 1H), 3.10 - 3.03 (m, 1H), 3.01 - 2.88 (m, 1H), 2.68 (dd, *J* = 16.8, 8.4 Hz, 1H), 2.49 (s, 3H), 2.23 - 2.15 (m, 1H), 1.64 - 1.62 (m, 3H) | 642.1 |
| **142b** | 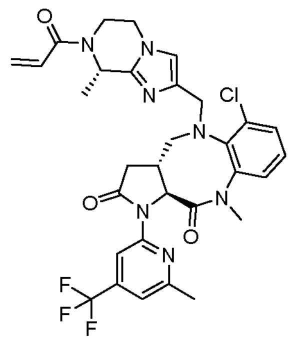 | (3a*R*,11a*S*)-5-(((*S*/*R*)-7-acryloyl-8-methyl-5,6,7,8-tetrahydroimidazo[1,2-a]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | ($CDCl_3$) 8.32 (s, 1H), 7.48 - 7.45 (m, 1H), 7.36 - 7.28 (m, 2H), 7.06 (s, 1H), 6.86 (s, 1H), 6.65 - 6.55 (m, 1H), 6.46 - 6.34 (m, 1H), 5.83 - 5.80 (m, 1H), 5.47 - 5.20 (m, 1H), 5.10 - 4.80 (m, 1H), 4.65 (d, *J* = 9.6 Hz, 1H), 4.42 - 4.25 (m, 1H), 4.16 - 4.12 (m, 1H), 4.05 - 3.92 (m, 2H), 3.66 - 3.62 (m, 1H), 3.40 - 3.15 (m, 4H), 3.11 - 2.89 (m, 2H), 2.68 (dd, *J* = 16.8, 8.0 Hz, 1H), 2.49 (s, 3H), 2.24 - 2.16 (m, 1H), 1.64 - 1.62 (m, 3H) | 642.1 |

(continued)

| Example Number | Structure | Name | Intermediates | $^1$H NMR (400 MHz) δ ppm | MI |
|---|---|---|---|---|---|
| **143a** | 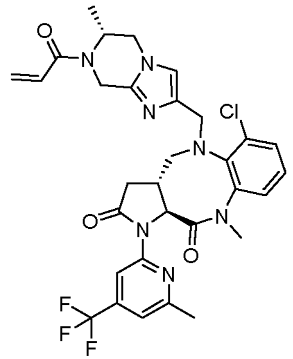 | (3a*R*,11a*S*)-5-(((*R*/*S*)-7-acryloyl-6-methyl-5,6,7,8-tetrahydroimidazo[1,2-a]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B26** | ($CDCl_3$) 8.31 (s, 1H), 7.49 - 7.47 (m, 1H), 7.37 - 7.31 (m, 2H), 7.06 (s, 1H), 6.99 (s, 1H), 6.63 - 6.54 (m, 1H), 6.43 - 6.36 (m, 1H), 5.85 (dd, *J* = 10.4, 1.6 Hz, 1H), 5.45 - 5.40 (m, 1H), 4.72 - 4.61 (m, 2H), 4.48 (d, *J* = 16.4 Hz, 1H), 4.22 (s, 1H), 4.20 - 4.18 (m, 1H), 4.16 (d, *J* = 4.4 Hz, 1H), 3.95 (d, *J* = 12.8 Hz, 1H), 3.64 (dd, *J* = 14.4, 5.2 Hz, 1H), 3.32 (s, 3H), 3.14 - 3.07 (m, 1H), 3.01 - 2.88 (m, 1H), 2.71 (dd, *J* = 16.8, 8.4 Hz, 1H), 2.50 (s, 3H), 2.20 (dd, *J* = 16.8, 11.6 Hz, 1H), 1.28 (d, *J* = 7.2 Hz, 3H) | 642.1 |
| **143b** | 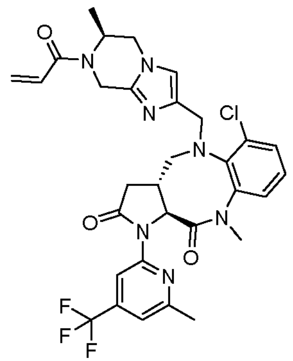 | (3a*R*,11a*S*)-5-(((*S*/*R*)-7-acryloyl-6-methyl-5,6,7,8-tetrahydroimidazo[1,2-a]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | ($CDCl_3$) 8.31 (s, 1H), 7.48 - 7.44 (m, 1H), 7.34 - 7.28 (m, 2H), 7.05 (s, 1H), 6.84 (s, 1H), 6.62 - 6.53 (m, 1H), 6.40 - 6.35 (m, 1H), 5.80 (dd, *J* = 10.4, 1.6 Hz, 1H), 5.55 - 4.79 (m, 2H), 4.66 (d, *J* = 9.2 Hz, 1H), 4.61 - 4.38 (m, 1H), 4.36 - 4.24 (m, 1H), 4.18 - 4.05 (m, 2H), 3.82 (d, *J* = 12.8 Hz, 1H), 3.61 (dd, *J* = 13.6, 4.4 Hz, 1H), 3.33 (s, 3H), 3.11 - 2.90 (m, 2H), 2.68 (dd, *J* = 16.8, 8.4 Hz, 1H), 2.49 (s, 3H), 2.19 (dd, *J* = 16.8, 11.2 Hz, 1H), 1.23 (d, *J* = 6.8 Hz, 3H) | 642.1 |

(continued)

| Example Number | Structure | Name | Intermediates | $^1$H NMR (400 MHz) δ ppm | MI |
|---|---|---|---|---|---|
| **144a** | 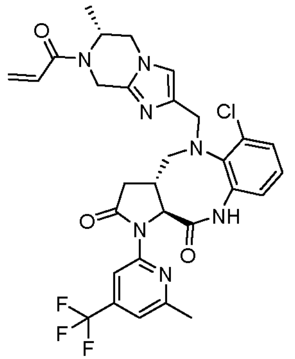 | (3a*R*,11a*S*)-5-(((*R*/*S*)-7-acryloyl-6-methyl-5,6,7,8-tetrahydroimidazo[1,2-*a*]pyrazin-2-yl)methyl)-6-chloro-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 14** and Intermediate B26 | ($CDCl_3$) 8.27 (s, 1H), 8.12 (br s, 1H), 7.49 - 7.39 (m, 1H), 7.27 - 7.23 (m, 2H), 7.05 (s, 1H), 6.88 (s, 1H), 6.62 - 6.52 (m, 1H), 6.37 (dd, *J* = 16.8, 1.6 Hz, 1H), 5.81 (dd, *J* = 10.4, 1.6 Hz, 1H), 5.26 - 5.09 (m, 1H), 4.64 (d, *J* = 9.6 Hz, 1H), 4.43 (d, *J* = 15.6 Hz, 1H), 4.15 (d, *J* = 15.6 Hz, 2H), 3.83 (d, *J* = 12.4 Hz, 1H), 3.65 (dd, *J* = 14.4, 4.8 Hz, 1H), 3.09 (dd, *J* = 14.4, 12.0 Hz, 1H), 2.92 - 2.81 (m, 1H), 2.70 (dd, *J* = 16.8, 8.8 Hz, 1H), 2.49 (s, 3H), 2.26 - 2.15 (m, 3H), 1.23 (d, *J* = 7.2 Hz, 3H) | 628.3 |
| **144b** | 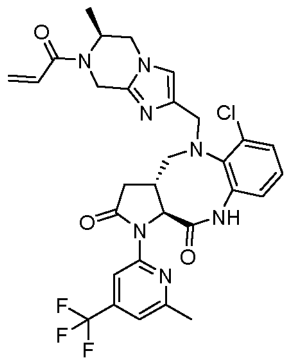 | (3a*R*,11a*S*)-5-(((*S*/*R*)-7-acryloyl-6-methyl-5,6,7,8-tetrahydroimidazo[1,2-*a*]pyrazin-2-yl)methyl)-6-chloro-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | ($CDCl_3$) 8.27 (s, 1H), 8.18 (s, 1H), 7.46 - 7.38 (m, 1H), 7.26 - 7.18 (m, 2H), 7.06 (s, 1H), 6.91 (s, 1H), 6.62 - 6.50 (m, 1H), 6.42 - 6.33 (m, 1H), 5.88 - 5.76 (m, 1H), 5.24 - 5.07 (m, 1H), 4.65 (d, *J* = 9.6 Hz, 1H), 4.61 - 4.43 (m, 2H), 4.38 (d, *J* = 15.2 Hz, 1H), 4.22 - 4.05 (m, 2H), 3.83 (d, *J* = 12.4 Hz, 1H), 3.66 (dd, *J* = 14.4, 4.4 Hz, 1H), 3.13 - 3.03 (m, 1H), 2.98 - 2.82 (m, 1H), 2.70 (dd, *J* = 16.8, 8.4 Hz, 1H), 2.49 (s, 3H), 2.22 (dd, *J* = 16.8, 11.6 Hz, 1H), 1.16 (d, *J* = 6.8 Hz, 3H) | 628.1 |

The **Examples** in **Table 13** were prepared using methods similar to those described in the synthesis of Example 39 or Example 43, using the listed **Intermediates** in step a, and the appropriate warhead in step c.

**Table 13**

| Example Number | Structure | Name | Intermediates | $^1$H NMR (400 MHz) δ ppm | MI |
|---|---|---|---|---|---|
| **145** | 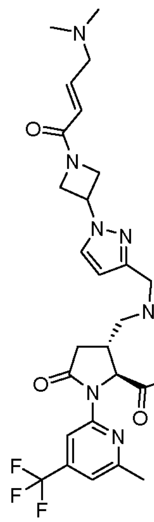 | (3a*R*,11a*S*)-5-((1-(1-((*E*)-4-(dimethylamino) but-2-enoyl)azetidin-3-yl)-1*H*-pyrazol-3-yl) methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and **Intermediate B2** **Step c:** (*E*)-4-(dimethylamino)but-2-enoic acid hydrochloride (CAS: 848133-35-7) | ($CDCl_3$) 8.30 (s, 1H), 7.39 (s, 1H), 7.36 - 7.29 (m, 1H), 7.23 - 7.10 (m, 2H), 7.05 (s, 1H), 6.94 - 6.87 (m, 1H), 6.26 (d, *J* = 9.6 Hz, 1H), 6.08 (d, *J* = 15.2 Hz, 1H), 5.07 - 4.99 (m, 1H), 4.67 - 4.58 (m, 3H), 4.51 - 4.41 (m, 2H), 4.34 - 4.31 (m, 1H), 4.15 - 4.07 (m, 1H), 3.55 (dd, *J* = 13.6, 3.6 Hz, 1H), 3.31 (d, *J* = 19.6 Hz, 3H), 3.12 (d, *J* = 5.6 Hz, 2H), 2.99 - 2.89 (m, 1H), 2.85 - 5.72 (m, 1H), 2.64 (dd, *J* = 16.8, 8.4 Hz, 1H), 2.49 (s, 3H), 2.29 (d, *J* = 3.2 Hz, 6H), 2.17 (dd, *J* = 16.8, 11.6 Hz, 1H) | 669.3 |
| **146** | 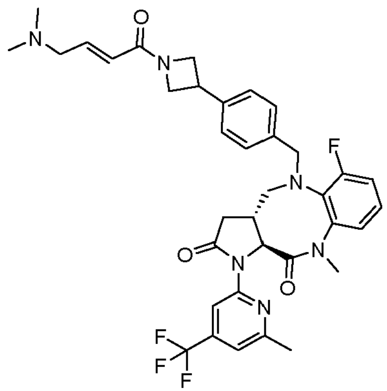 | (3a*R*,11a*S*)-5-(4-(1-((*E*)-4-(dimethylamino) but-2-enoyl)azetidin-3-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and **Intermediate B13** **Step c:** (*E*)-4-(dimethylamino)but-2-enoic acid hydrochloride (CAS: 848133-35-7) | ($CDCl_3$) 8.30 (s, 1H), 7.36 - 7.29 (m, 3H), 7.27 - 7.23 (m, 2H), 7.22 - 7.13 (m, 2H), 7.06 (s, 1H), 6.92 - 6.87 (m, 1H), 6.09 (d, *J* = 15.6 Hz, 1H), 4.66 (d, *J* = 9.6 Hz, 1H), 4.60 (t, *J* = 8.8 Hz, 1H), 4.46 (t, *J* = 9.6 Hz, 1H), 4.34 - 4.26 (m, 1H), 4.24 - 4.16 (m, 2H), 4.13 - 4.05 (m, 1H), 3.86 - 3.77 (m, 1H), 3.55 - 3.45 (m, 1H), 3.28 (d, *J* = 11.6 Hz, 3H), 3.12 (d, *J* = 6.0 Hz, 2H), 2.98 - 2.89 (m, 1H), 2.87 - 2.75 (m, 1H), 2.68 - 2.58 (m, 1H), 2.50 (s, 3H), 2.29 (s, 6H), 2.15 (dd, *J* = 16.4, 12.4 Hz, 1H) | 679.2 |

(continued)

| Example Number | Structure | Name | Intermediates | $^1H$ NMR (400 MHz) δ ppm | MI |
|---|---|---|---|---|---|
| **147** | 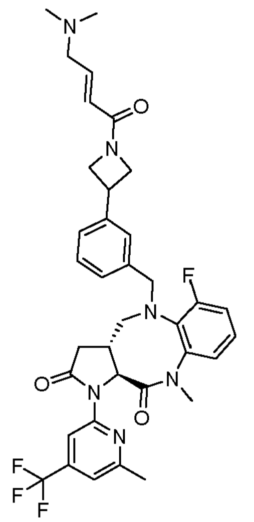 | (3a*R*,11a*S*)-5-(3-(1-((*E*)-4-(dimethylamino) but-2-enoyl)azetidin-3-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and **Intermediate B10** **Step c:** (*E*)-4-(dimethylamino)but-2-enoic acid hydrochloride (CAS: 848133-35-7) | ($CDCl_3$) 8.31 (s, 1H), 7.33 - 7.28 (m, 2H), 7.27 - 7.22 (m, 1H), 7.21 - 7.13 (m, 4H), 7.06 (s, 1H), 6.95 - 6.84 (m, 1H), 6.21 - 6.14 (m, 1H), 4.68 - 4.58 (m, 2H), 4.52 - 4.43 (m, 1H), 4.36 - 4.28 (m, 1H), 4.28 - 4.14 (m, 2H), 4.10 - 4.02 (m, 1H), 3.89 - 3.77 (m, 1H), 3.56 - 3.45 (m, 1H), 3.29 - 3.20 (m, 3H), 3.16 (m, 2H), 3.01 - 2.91 (m, 1H), 2.84 - 2.72 (m, 1H), 2.67 - 2.58 (m, 1H), 2.50 (d, *J* = 1.2 Hz, 3H), 2.31 (d, *J* = 16.4 Hz, 6H), 2.20 - 2.10 (m, 1H) | 679.1 |
| **148** | 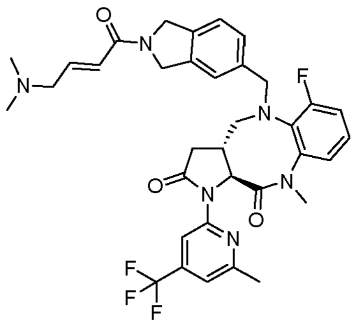 | (3a*R*,11a*S*)-5-((2-((*E*)-4-(dimethylamino)but-2-enoyl)isoindolin-5-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and **Intermediate B18** **Step c:** (*E*)-4-(dimethylamino)but-2-enoic acid hydrochloride (CAS: 848133-35-7) | ($CDCl_3$) 8.30 (s, 1H), 7.36 - 7.28 (m, 1H), 7.25 (s, 1H), 7.24 - 7.11 (m, 4H), 7.06 (s, 1H), 7.03 - 6.95 (m, 1H), 6.57 - 6.49 (m, 1H), 4.92 (d, *J* = 4.4 Hz, 2H), 4.83 (s, 2H), 4.66 (d, *J* = 9.6 Hz, 1H), 4.37 - 4.17 (m, 2H), 3.54 - 3.49 (m, 1H), 3.34 - 3.22 (m, 5H), 3.00 - 2.91 (m, 1H), 2.88 - 2.75 (m, 1H), 2.66 - 2.59 (m, 1H), 2.49 (d, *J* = 3.6 Hz, 3H), 2.41 (d, *J* = 6.0 Hz, 6H), 2.20 - 2.12 (m, 1H) | 665.2 |

(continued)

| Example Number | Structure | Name | Intermediates | $^1$H NMR (400 MHz) δ ppm | MI |
|---|---|---|---|---|---|
| **149** | 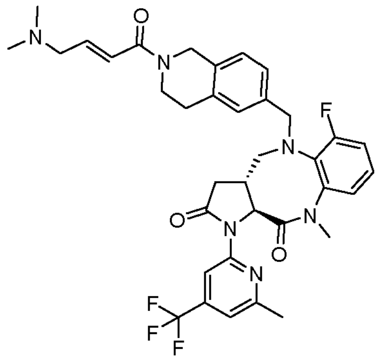 | (3a*R*,11a*S*)-5-((2-((*E*)-4-(dimethylamino)but-2-enoyl)-1,2,3,4-tetrahydroisoquinolin-6-yl) methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and **Intermediate B17** **Step c:** (*E*)-4-(dimethylamino)but-2-enoic acid hydrochloride (CAS: 848133-35-7) | ($CDCl_3$) 8.32 (s, 1H), 7.37 - 7.30 (m, 1H), 7.24 - 7.16 (m, 2H), 7.16 - 7.06 (m, 4H), 6.90 - 6.83 (m, 1H), 6.76 - 6.58 (m, 1H), 4.80 - 4.72 (m, 2H), 4.67 (d, *J* = 9.6 Hz, 1H), 4.32 - 4.15 (m, 2H), 3.96 - 3.77 (m, 2H), 3.53 - 2.48 (m, 1H), 3.30 - 3.27 (m, 5H), 2.98 - 2.77 (m, 4H), 2.68 - 2.61 (m, 1H), 2.51 (s, 3H), 2.44 (s, 6H), 2.21 - 2.13 (m, 1H) | 679.0 |
| **150** | 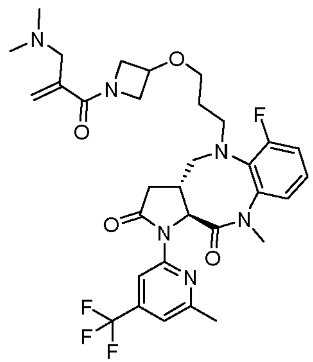 | (3a*R*,11a*S*)-5-(3-((1-(2-((dimethylamino) methyl) acryloyl)azetidin-3-yl)oxy)propyl)-6-fluoro-10-methyl-1-(6-methyl-4-(trifluoromethyl) pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and **Intermediate B4** **Step c: Intermediate E1** | ($CDCl_3$) 8.34 (s, 1H), 7.38 - 7.32 (m, 1H), 7.24 (d, *J* = 8.0 Hz, 1H), 7.16 (t, *J* = 9.2 Hz, 1H), 7.07 (s, 1H), 5.60 (s, 1H), 5.49 (s, 1H), 4.63 (d, *J* = 9.2 Hz, 1H), 4.41 - 4.32 (m, 1H), 4.25 (d, *J* = 4.4 Hz, 2H), 4.11 - 4.03 (m, 1H), 3.93 - 3.85 (m, 1H), 3.58 - 3.52 (m, 1H), 3.43 - 3.38 (m, 2H), 3.36 (s, 3H), 3.31 - 3.23 (m, 1H), 3.22 - 3.06 (m, 3H), 2.99 - 2.82 (m, 2H), 2.78 - 2.67 (m, 1H), 2.51 (s, 3H), 2.32 - 2.17 (m, 7H), 1.64 - 1.61 (m, 2H) | 647.3 |

(continued)

| Example Number | Structure | Name | Intermediates | $^1$H NMR (400 MHz) δ ppm | MI |
|---|---|---|---|---|---|
| **151** | 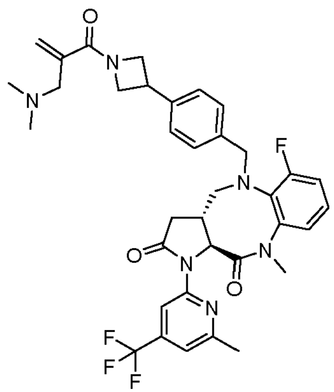 | (3a*R*,11a*S*)-5-(4-(1-(2-((dimethylamino)methyl) acryloyl)azetidin-3-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and **Intermediate B13** **Step c: Intermediate E1** | ($CDCl_3$) 8.30 (s, 1H), 7.36 - 7.29 (m, 4H), 7.27 - 7.24 (m, 1H), 7.22 - 7.13 (m, 2H), 7.06 (s, 1H), 5.58 - 5.52 (m, 2H), 4.66 (d, *J* = 9.6 Hz, 1H), 4.64 - 4.57 (m, 1H), 4.48 (t, *J* = 8.4 Hz, 1H), 4.34 - 4.17 (m, 3H), 4.15 - 4.04 (m, 1H), 3.87 - 3.75 (m, 1H), 3.55 - 3.45 (m, 1H), 3.33 - 3.21 (m, 4H), 3.20 - 3.08 (m, 1H), 3.00 - 2.89 (m, 1H), 2.88 - 2.72 (m, 1H), 2.69 - 2.57 (m, 1H), 2.50 (s, 3H), 2.30 (s, 6H), 2.16 (dd, *J* = 16.8, 11.6 Hz, 1H) | 679.2 |
| **152** | 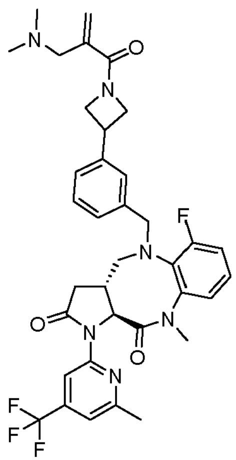 | (3a*R*,11a*S*)-5-(3-(1-(2-((dimethylamino)methyl) acryloyl)azetidin-3-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and **Intermediate B10** **Step c: Intermediate E1** | ($CDCl_3$) 8.31 (s, 1H), 7.36 - 7.27 (m, 3H), 7.24 - 7.12 (m, 4H), 7.06 (s, 1H), 5.73 - 5.54 (m, 2H), 4.66 (d, *J* = 9.6 Hz, 2H), 4.56 - 4.44 (m, 1H), 4.37 - 4.15 (m, 3H), 4.13 - 3.99 (m, 1H), 3.90 - 3.78 (m, 1H), 3.57 - 3.46 (m, 1H), 3.38 - 3.13 (m, 5H), 3.01 - 2.91 (m, 1H), 2.87 - 2.75 (m, 1H), 2.65 - 2.59 (m, 1H), 2.50 (s, 3H), 2.34 (s, 6H), 2.20 - 2.12 (m, 1H) | 679.1 |

(continued)

| Example Number | Structure | Name | Intermediates | $^1$H NMR (400 MHz) δ ppm | MI |
|---|---|---|---|---|---|
| **153** | 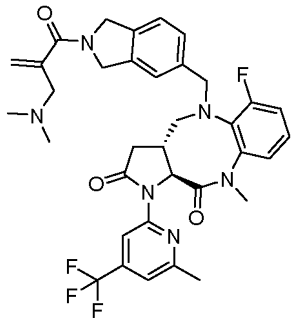 | (3a*R*,11a*S*)-5-((2-(2-((dimethylamino)methyl) acryloyl)isoindolin-5-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and **Intermediate B18** **Step c: Intermediate E1** | ($CDCl_3$) 8.30 (s, 1H), 7.36 - 7.29 (m, 1H), 7.24 (s, 2H), 7.22 - 7.12 (m, 3H), 7.06 (s, 1H), 5.97 (s, 1H), 5.81 (s, 1H), 4.91 (d, *J* = 3.6 Hz, 2H), 4.85 (s, 2H), 4.68 - 4.64 (m, 1H), 4.34 - 4.17 (m, 2H), 3.69 (s, 2H), 3.53 - 3.46 (m, 1H), 3.29 (s, 3H), 2.99 - 2.89 (m, 1H), 2.85 - 2.75 (m, 1H), 2.74 - 2.58 (m, 7H), 2.50 (s, 3H), 2.20 - 2.13 (m, 1H) | 665.2 |
| **154** | 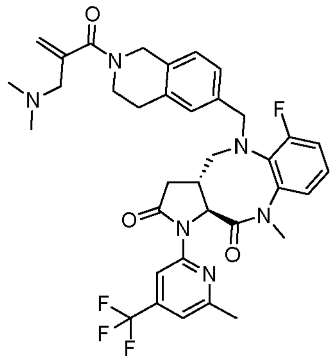 | (3a*R*,11a*S*)-5-((2-(2-((dimethylamino)methyl) acryloyl)-1,2,3,4-tetrahydroisoquinolin-6-yl) methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and **Intermediate B17** **Step c: Intermediate E1** | ($CDCl_3$) 8.30 (s, 1H), 7.37 - 7.29 (m, 1H), 7.22 - 7.15 (m, 2H), 7.15 - 7.00 (m, 4H), 6.05 - 5.68 (m, 1H), 5.52 (s, 1H), 4.75 (s, 2H), 4.66 (d, *J* = 9.6 Hz, 1H), 4.29 - 4.12 (m, 2H), 3.83 (s, 2H), 3.55 - 3.40 (m, 3H), 3.28 (s, 3H), 2.97 - 2.87 (m, 3H), 2.86 - 2.78 (m, 1H), 2.69 - 2.61 (m, 1H), 2.61 - 2.38 (m, 9H), 2.21 - 2.12 (m, 1H) | 679.1 |

(continued)

| Example Number | Structure | Name | Intermediates | $^1$H NMR (400 MHz) δ ppm | MI |
|---|---|---|---|---|---|
| **155** | 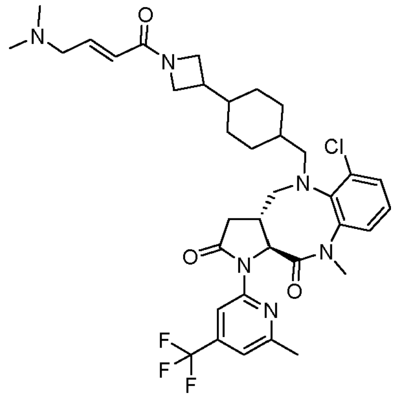 | (3a*R*,11a*S*)-6-chloro-5-((4-(1-((*E*)-4-(dimethylamino)but-2-enoyl)azetidin-3-yl)cyclohexyl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B14** **Step c:** (*E*)-4-(dimethylamino)but-2-enoic acid hydrochloride (CAS: 848133-35-7) | ($CDCl_3$) 8.32 (s, 1H), 7.44 (d, *J* = 7.6 Hz, 1H), 7.31 - 7.28 (m, 2H), 7.05 (s, 1H), 6.87 - 6.80 (m, 1H), 6.01 (d, *J* = 15.6 Hz, 1H), 4.62 (d, *J* = 8.8 Hz, 1H), 4.20 (t, *J* = 8.4 Hz, 1H), 4.15 - 3.96 (m, 1H), 3.90 - 3.78 (m, 1H), 3.77 - 3.65 (m, 1H), 3.51 (dd, *J* = 13.2, 3.6 Hz, 1H), 3.37 (s, 3H), 3.05 (d, *J* = 6.0 Hz, 2H), 3.02 - 2.91 (m, 2H), 2.83 - 2.68 (m, 3H), 2.48 (s, 3H), 2.25 (s, 6H), 2.23 - 2.18 (m, 1H), 1.96 - 1.91 (m, 2H), 1.80 - 1.70 (m, 2H), 1.60 - 1.30 (m, 3H), 1.26 - 1.09 (m, 1H), 0.94 - 0.72 (m, 3H) | 701.2 |
| **156** | 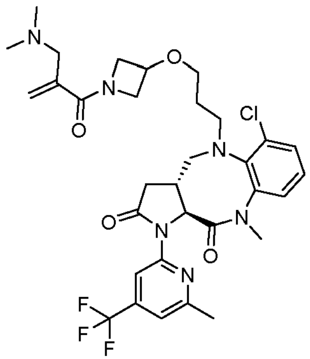 | (3a*R*,11a*S*)-6-chloro-5-(3-((1-(2-((dimethylamino)methyl) acryloyl)azetidin-3-yl)oxy)propyl)-10-methyl-1-(6-methyl-4-(trifluoromethyl) pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B4** **Step c: Intermediate E1** | ($CD_3OD$) 8.23 (s, 1H), 7.44 (t, *J* = 6.8 Hz, 2H), 7.35 - 7.27 (m, 1H), 7.15 (s, 1H), 6.02 (s, 1H), 5.92 (s, 1H), 4.60 (d, *J* = 9.6 Hz, 1H), 4.49 - 4.45 (m, 1H), 4.31 - 4.24 (m, 1H), 4.21 - 4.06 (m, 2H), 3.83 (s, 3H), 3.82 - 3.77 (m, 1H), 3.57 - 3.48 (m, 1H), 3.43 (t, *J* = 6.0 Hz, 2H), 3.26 - 3.21 (m, 2H), 3.08 (t, *J* = 6.8 Hz, 2H), 3.00 (dd, *J* = 14.4, 11.6 Hz, 1H), 2.79 (s, 7H), 2.61 (dd, *J* = 16.8, 8.4 Hz, 1H), 2.47 (s, 3H), 2.30 (dd, *J* = 16.8, 11.6 Hz, 1H), 1.65 - 1.51 (m, 2H) | 663.2 |

(continued)

| Example Number | Structure | Name | Intermediates | $^1$H NMR (400 MHz) δ ppm | MI |
|---|---|---|---|---|---|
| **157** | 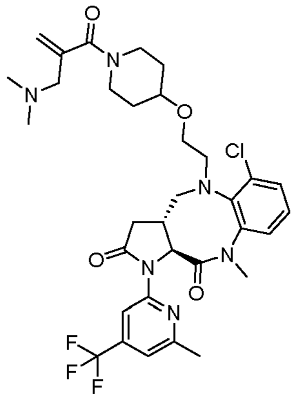 | (3a*R*,11a*S*)-6-chloro-5-(2-((1-(2-((dimethylamino)methyl) acryloyl)piperidin-4-yl)oxy) ethyl)-10-methyl-1-(6-methyl-4-(trifluoromethyl) pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B27** **Step c: Intermediate E1** | ($CDCl_3$) 8.33 (s, 1H), 7.44 (dd, *J* = 7.6, 2.0 Hz, 1H), 7.34 - 7.28 (m, 2H), 7.05 (s, 1H), 5.45 (s, 1H), 5.23 (s, 1H), 4.63 (d, *J* = 9.2 Hz, 1H), 3.93 - 3.68 (m, 2H), 3.67 - 3.58 (m, 1H), 3.52 - 3.40 (m, 3H), 3.38 (s, 3H), 3.36 - 3.25 (m, 3H), 3.24 - 3.12 (m, 3H), 3.02 - 2.95 (m, 2H), 2.75 -2.67 (m, 1H), 2.49 (s, 3H), 2.31 (s, 6H), 2.25 - 2.18 (m, 1H), 1.81 - 1.75 (m, 2H), 1.54 - 1.45 (m, 2H) | 677.2 |
| **158** | 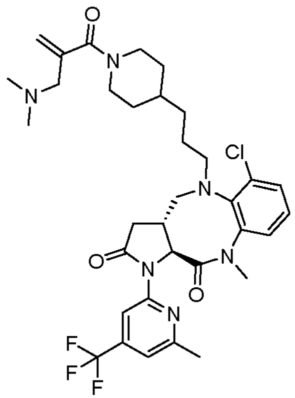 | (3a*R*,11a*S*)-6-chloro-5-(3-(1-(2-((dimethylamino)methyl) acryloyl)piperidin-4-yl)propyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B45** **Step c: Intermediate E1** | ($CDCl_3$) 8.33 (s, 1H), 7.45 (dd, *J* = 7.2, 2.4 Hz, 1H), 7.34 - 7.28 (m, 2H), 7.05 (s, 1H), 6.21 (s, 1H), 5.67 (s, 1H), 4.62 (d, *J* = 9.2 Hz, 1H), 4.57 - 4.38 (m, 1H), 4.22 - 4.00 (m, 1H), 3.87 - 3.68 (m, 2H), 3.53 (dd, *J* = 14.0, 4.0 Hz, 1H), 3.36 (s, 3H), 3.11 - 2.88 (m, 5H), 2.83 - 2.58 (m, 8H), 2.48 (s, 3H), 2.22 (dd, *J* = 16.8, 11.2 Hz, 1H), 1.50 - 1.44 (m, 1H), 1.40 - 1.20 (m, 6H), 1.18 - 1.04 (m, 2H) | 675.1 |

(continued)

| Example Number | Structure | Name | Intermediates | $^1$H NMR (400 MHz) δ ppm | MI |
|---|---|---|---|---|---|
| **159** | 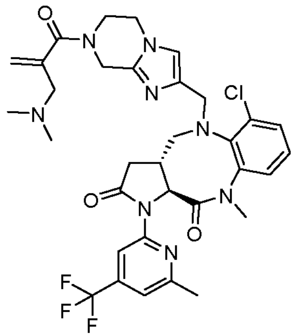 | (3a*R*,11a*S*)-6-chloro-5-((7-(2-((dimethylamino) methyl) acryloyl)-5,6,7,8-tetrahydroimidazo [1,2-a]pyrazin-2-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B19** **Step c: Intermediate E1** | ($CDCl_3$) 8.31 (s, 1H), 7.45 (dd, *J* = 7.2, 2.4 Hz, 1H), 7.34 - 7.28 (m, 2H), 7.05 (s, 1H), 6.86 (s, 1H), 5.73 (s, 1H), 5.45 (s, 1H), 4.84 (s, 2H), 4.65 (d, *J* = 9.2 Hz, 1H), 4.31 - 4.26 (m, 1H), 4.13 - 4.04 (m, 5H), 3.64 - 3.59 (m, 1H), 3.42 (s, 2H), 3.33 (s, 3H), 3.03 - 2.94 (m, 2H), 2.72 - 2.66 (m, 1H), 1.50 - 1.44 (m, 3H), 2.44 (s, 6H), 2.20 - 2.18 (m, 1H) | 685.3 |
| **160** | 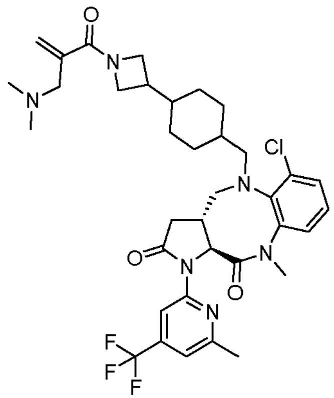 | (3a*R*,11a*S*)-6-chloro-5-((4-(1-(2-((dimethylamino)methyl) acryloyl)azetidin-3-yl)cyclohexyl) methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B14** **Step c: Intermediate E1** | ($CDCl_3$) 8.32 (s, 1H), 7.44 (dd, *J* = 7.2, 2.0 Hz, 1H), 7.33 - 7.27 (m, 2H), 7.05 (s, 1H), 6.58 (s, 1H), 5.97 (s, 1H), 4.62 (d, *J* = 9.2 Hz, 1H), 4.44 - 4.29 (m, 1H), 4.19 - 3.98 (m, 2H), 3.94 - 3.72 (m, 3H), 3.56 - 3.48 (m, 1H), 3.37 (s, 3H), 3.10 - 2.88 (m, 2H), 2.83 - 2.79 (m, 7H), 2.73 (dd, *J* = 16.8, 8.0 Hz, 1H), 2.48 (s, 3H), 2.41 - 2.31 (m, 1H), 2.22 (dd, *J* = 16.8, 10.0 Hz, 1H), 1.90 (d, *J* = 12.0 Hz, 2H), 1.77 - 1.72 (m, 2H), 1.56 - 1.34 (m, 2H), 1.32 - 1.21 (m, 1H), 0.96 - 0.70 (m, 4H) | 701.2 |

(continued)

| Example Number | Structure | Name | Intermediates | $^1$H NMR (400 MHz) δ ppm | MI |
|---|---|---|---|---|---|
| **161** | 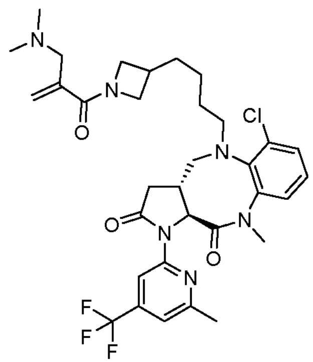 | (3a*R*,11a*S*)-6-chloro-5-(4-(1-(2-((dimethylamino)methyl) acryloyl)azetidin-3-yl)butyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B30** **Step c: Intermediate E1** | ($CDCl_3$) 8.33 (s, 1H), 7.45 (dd, *J* = 7.2, 2.4 Hz, 1H), 7.33 - 7.28 (m, 2H), 7.05 (s, 1H), 6.57 (s, 1H), 5.98 (s, 1H), 4.62 (d, *J* = 9.2 Hz, 1H), 4.43 (m, 1H), 4.22 - 4.12 (m, 1H), 4.01 - 3.78 (m, 3H), 3.73 - 3.63 (m, 1H), 3.58 - 3.49 (m, 1H), 3.37 (s, 3H), 3.05 - 2.87 (m, 4H), 2.83 - 2.69 (m, 7H), 2.68 - 2.59 (m, 1H), 2.48 (s, 3H), 2.22 (dd, *J* = 16.8, 11.2 Hz, 1H), 1.60 - 1.56 (m, 1H), 1.39 - 1.25 (m, 5H) | 661.1 |
| **162** | 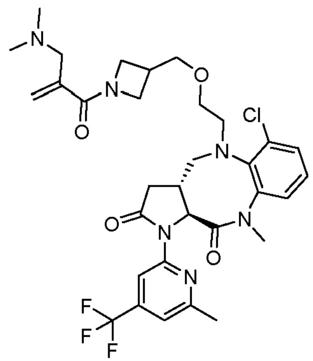 | (3a*R*,11a*S*)-6-chloro-5-(2-((1-(2-((dimethylamino)methyl) acryloyl)azetidin-3-yl)methoxy) ethyl)-10-methyl-1-(6-methyl-4-(trifluoromethyl) pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B31** **Step c: Intermediate E1** | ($CDCl_3$) 8.32 (s, 1H), 7.45 - 7.42 (m, 1H), 7.33 - 7.26 (m, 2H), 7.05 (s, 1H), 5.54 (s, 1H), 5.46 (d, *J* = 6.8 Hz, 1H), 4.61 (d, *J* = 9.2 Hz, 1H), 4.27 - 4.22 (m, 1H), 4.13 - 4.03 (m, 1H), 3.97 - 3.88 (m, 1H), 3.72 - 3.65 (m, 1H), 3.62 - 3.55 (m, 1H), 3.51 (d, *J* = 6.8 Hz, 2H), 3.45 - 3.41 (m, 2H), 3.35 (s, 3H), 3.29 - 3.14 (m, 3H), 3.14 - 3.06 (m, 1H), 3.02 - 2.87 (m, 2H), 2.85 - 2.75(m, 1H), 2.74 - 2.65 (m, 1H), 2.48 (s, 3H), 2.26 (s, 6H), 2.25 - 2.16 (m, 1H) | 663.1 |

(continued)

| Example Number | Structure | Name | Intermediates | $^1$H NMR (400 MHz) δ ppm | MI |
|---|---|---|---|---|---|
| **163** | 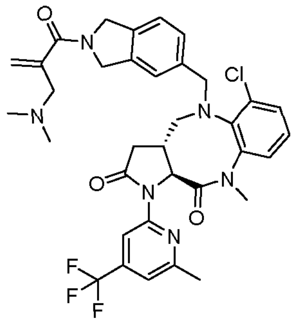 | (3a*R*,11a*S*)-6-chloro-5-((2-(2-((dimethylamino) methyl) acryloyl)isoindolin-5-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B18** **Step c: Intermediate E1** | ($CDCl_3$) 8.32 (s, 1H), 7.50 - 7.45 (m, 1H), 7.35 - 7.28 (m, 4H), 7.27 - 7.17 (m, 1H), 7.06 (s, 1H), 5.58 (s, 1H), 5.51 (s, 1H), 4.88 (s, 4H), 4.68 (dd, *J* = 9.2, 2.8 Hz, 1H), 4.20 (q, *J* = 14.0 Hz, 2H), 3.46 (dd, *J* = 14.0, 4.0 Hz, 1H), 3.38 - 3.22 (m, 5H), 3.03 - 2.96 (m, 1H), 2.95 - 2.85 (m, 1H), 2.65 (dd, *J* = 16.8, 8.0 Hz, 1H), 2.49 (s, 3H), 2.34 (s, 6H), 2.18 (dd, *J* = 16.8, 11.2 Hz, 1H) | 681.1 |
| **164** | 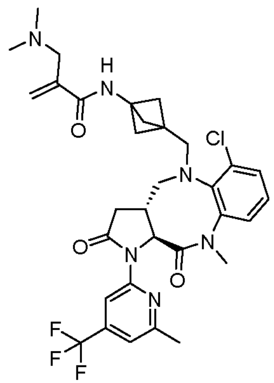 | *N*-(3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5H-benzo [*b*]pyrrolo[2,3-*f*][1,4]diazocin-5-yl)methyl)bicyclo[1.1.1]p entan-1-yl)-2-((dimethylamino) methyl) acrylamide | **Step a: Intermediate 5b** and **Intermediate B32** **Step c: Intermediate E1** | ($CDCl_3$) 8.33 (s, 1H), 7.45 (dd, *J* = 6.8, 2.8 Hz, 1H), 7.34 - 7.25 (m, 3H), 7.07 (s, 1H), 6.20 (d, *J* = 0.8 Hz, 1H), 5.50 (br s, 1H), 4.65 (d, *J* = 9.2 Hz, 1H), 3.55 - 3.48 (m, 1H), 3.41 - 3.35 (m, 4H), 3.32 - 3.14 (m, 1H), 3.13 - 2.89 (m, 4H), 2.73 (dd, *J* = 16.8, 8.0 Hz, 1H), 2.49 (s, 3H), 2.47 - 2.05 (m, 7H), 2.04 - 1.98 (m, 3H), 1.95 - 1.89 (m, 3H) | 645.1 |
| **165** | 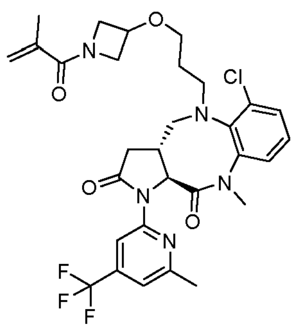 | (3a*R*,11a*S*)-6-chloro-5-(3-((1-methacryloylazetidin-3-yl)oxy)propyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B4** **Step c:** methacrylic acid (CAS: 79-41-4) | ($CDCl_3$) 8.34 (s, 1H), 7.40 - 7.37 (m, 1H), 7.33 - 7.28 (m, 2H), 7.05 (s, 1H), 5.37 (s, 1H), 5.29 (s, 1H), 4.63 (d, *J*= 9.2 Hz, 1H), 4.35 - 4.26 (m, 1H), 4.25 - 4.15 (m, 2H), 4.08 - 3.94 (m, 1H), 3.93 - 3.75 (m, 1H), 3.60 - 3.52 (m, 1H), 3.46 - 3.41 (m, 2H), 3.38 (s, 3H), 3.18 - 3.06 (m, 2H), 3.04 - 2.90 (m, 2H), 2.78 - 2.69 (m, 1H), 2.48 (s, 3H), 2.27 - 2.18 (m, 1H), 1.92 (s, 3H), 1.71 - 1.61 (m, 2H) | 620.1 |

(continued)

| Example Number | Structure | Name | Intermediates | $^1$H NMR (400 MHz) δ ppm | MI |
|---|---|---|---|---|---|
| **166** | 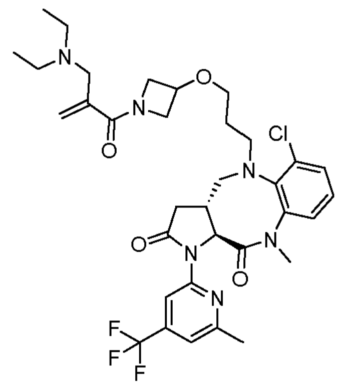 | (3a*R*,11a*S*)-6-chloro-5-(3-((1-(2-((diethylamino)methyl)ac ryloyl)azetidin-3-yl)oxy)propyl)-10-methyl-1-(6-methyl-4-(trifluoromethyl) pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B4** **Step c: Intermediate E7** | ($CDCl_3$) 8.34 (s, 1H), 7.47 - 7.42 (m, 1H), 7.34 - 7.27 (m, 2H), 7.05 (s, 1H), 5.86 - 5.29 (m, 2H), 4.63 (d, *J* = 8.8 Hz, 1H), 4.44 - 4.28 (m, 1H), 4.24 (d, *J* = 4.0 Hz, 2H), 4.10 - 3.97 (m, 1H), 3.92 - 3.75 (m, 1H), 3.58 - 3.52 (m, 1H), 3.46 - 3.41 (m, 2H), 3.38 (s, 3H), 3.34 - 3.21 (m, 1H), 3.17 - 3.06 (m, 2H), 3.05 - 2.88 (m, 2H), 2.78 - 2.52 (m, 4H), 2.48 (s, 3H), 2.23 (dd, *J* = 16.8, 10.8 Hz, 1H), 1.64 - 1.57 (m, 4H), 1.15 - 1.07 (m, 6H) | 691.0 |
| **167** | 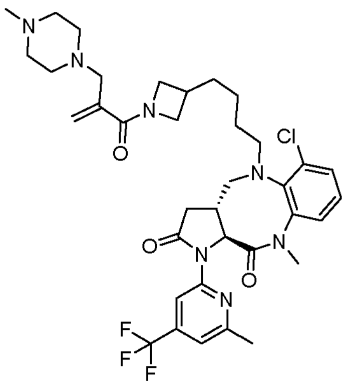 | (3a*R*,11a*S*)-6-chloro-10-methyl-1-(6-methyl-4-(trifluoromethyl)pyridin-2-yl)-5-(4-(1-(2-((4-methylpiperazin-1-yl)methyl) acryloyl)azetidi n-3-yl)butyl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B30** **Step c: Intermediate E8** | ($CDCl_3$) 8.33 (s, 1H), 7.46 - 7.43 (m, 1H), 7.34 - 7.28 (m, 2H), 7.05 (s, 1H), 5.52 - 5.48 (m, 2H), 4.62 (d, *J* = 9.2 Hz, 1H), 4.31 - 4.27 (m, 1H), 4.15 - 4.10 (m, 1H), 3.83 - 3.78 (m, 1H), 3.64 - 3.61 (m, 1H), 3.58 - 3.51 (m, 1H), 3.48 - 3.25 (m, 6H), 3.05 - 2.98 (m, 3H), 2.97 - 2.79 (m, 6H), 2.77 - 2.70 (m, 3H), 2.65 - 2.60 (m, 1H), 2.48 (s, 3H), 2.22 (dd, J = 16.8, 11.2 Hz, 1H), 1.66 - 1.52 (m, 4H), 1.44 - 1.23 (m, 5H) | 716.1 |

(continued)

| Example Number | Structure | Name | Intermediates | $^1$H NMR (400 MHz) δ ppm | MI |
|---|---|---|---|---|---|
| **168** | 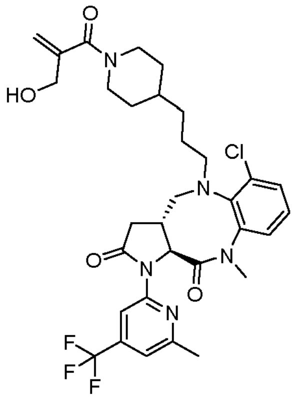 | (3a*R*,11a*S*)-6-chloro-5-(3-(1-(2-(hydroxymethyl)acryloyl) piperidin-4-yl)propyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B45** **Step c: Intermediate E2** | ($CDCl_3$) 8.33 (s, 1H), 7.47 - 7.42 (m, 1H), 7.36 - 7.28 (m, 2H), 7.05 (s, 1H), 5.45 (s, 1H), 5.18 (s, 1H), 4.62 (d, *J* = 9.6, 1H), 4.50 - 4.57 (m, 1H), 4.29 (s, 2H), 4.20 - 4.03 (m, 1H), 3.51 - 3.56 (m, 1H), 3.36 (s, 3H), 3.16 - 2.86 (m, 5H), 2.76 - 2.69 (m, 1H), 2.67 - 2.55 (m, 1H), 2.48 (s, 3H), 2.26 - 2.18 (m, 1H), 1.90 - 1.68 (m, 2H), 1.47 - 1.23 (m, 6H), 1.17 - 0.99 (m, 2H) | 648.1 |
| **169** | 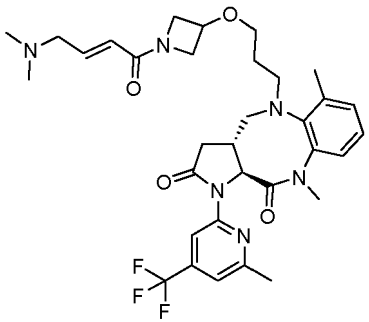 | (3a*R*,11a*S*)-5-(3-((1-((*E*)-4-(dimethylamino) but-2-enoyl)azetidin-3-yl)oxy)propyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 6c** and **Intermediate B4** **Step c:** (*E*)-4-(dimethylamino)but-2-enoic acid hydrochloride | ($CDCl_3$) 8.33 (s, 1H), 7.25 - 7.19 (m, 3H), 7.04 (s, 1H), 6.89 - 6.82 (m, 1H), 6.02 (d, *J* = 15.6 Hz, 1H), 4.70 (d, *J* = 9.6, 1H), 4.35 - 4.31 (m, 1H), 4.24 - 4.16 (m, 2H), 4.06 - 3.95 (m, 1H), 3.90 - 3.84 (m, 1H), 3.66 - 3.52 (m, 1H), 3.45 - 3.30 (m, 5H), 3.01 - 2.94 (m, 5H), 2.83 - 2.68 (m, 2H), 2.49 (s, 3H), 2.37 (s, 3H), 2.27-2.15 (m, 7H), 1.71 - 1.58 (m, 2H) | 643.4 |

The **Examples** in **Table 14** were prepared using methods similar to those described in the synthesis of **Example 39** or **Example 43,** using the listed **Intermediates** in step a, and the appropriate warhead in step c. An additional chiral SFC step was conducted after step a.

**Table 14**

| Example Number | Structure | Name | Intermediates | $^1$H NMR (400 MHz) δ ppm | MI |
|---|---|---|---|---|---|
| **170a** | 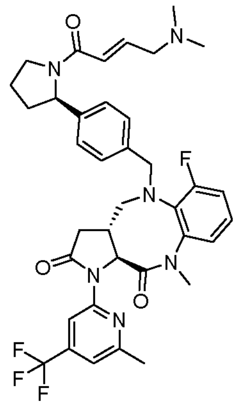 | (3*aR*,11*aS*)-5-(4-((*R*/*S*)-1-((*E*)-4-(dimethylamino) but-2-enoyl)pyrrolidin-2-yl)benzyl)-6-fluoro-10-methyl-1-(6-methyl-4-(trifluoromethyl)pyridin-2-yl)-1,3a,4,5,10,11a-hexahydro-2H-benzo[b]pyrrolo [2,3-*f*][1,4]diazocine-2,11(3*H*)-dione | **Step a: Intermediate 5a** and **Intermediate B16 Step c:** (*E*)-4-(dimethylamino)b ut-2-enoic acid hydrochloride | ($CDCl_3$) 8.29 (s, 1H), 7.36 - 7.29 (m, 1H), 7.26 - 7.19 (m, 2H), 7.19 - 7.13 (m, 2H), 7.12 - 7.08 (m, 2H), 7.07 - 7.04 (m, 1H), 6.93 - 6.75 (m, 1 H), 6.43 - 5.91 (m, 1H), 5.24 - 5.03 (m, 1H), 4.68 - 4.63 (m, 1H), 4.32 - 4.24 (m, 1H), 4.20 - 4.08 (m, 1H), 3.88 - 3.66 (m, 2H), 3.48 (dd, *J* = 13.6, 5.0 Hz, 1H), 3.32 - 3.21 (m, 3H), 3.12 (d, *J* = 6.0 Hz, 1H), 3.00 - 2.88 (m, 2H), 2.86 - 2.73 (m, 1H), 2.65 - 2.60 (m, 1H), 2.49 (s, 3H), 2.32 - 2.22 (m, 4H), 2.21 - 2.10 (m, 2H), 2.09 (s, 3H), 2.01 - 1.93 (m, 2H) | 693.3 |
| **170b** | 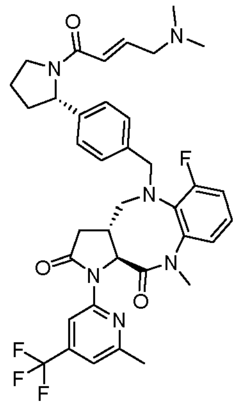 | (3*aR*,11*aS*)-5-(4-((*S*/*R*)-1-((*E*)-4-(dimethylamino) but-2-enoyl)pyrrolidin-2-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo [2,3-*f*][1,4]diazocine-2,11(3*H*)-dione | | ($CDCl_3$) 8.29 (s, 1H), 7.37 - 7.29 (m, 1H), 7.29 - 7.25 (m, 2H), 7.24 - 7.15 (m, 2H), 7.14 - 7.08 (m, 2H), 7.06 (s, 1H), 6.92 - 6.72 (m, 1H), 6.65 - 6.09 (m, 1H), 5.24 - 5.04 (m, 1H), 4.65 (d, *J* = 9.6 Hz, 1H), 4.34 - 4.23 (m, 1H), 4.20 - 4.08 (m, 1H), 3.90 - 3.69 (m, 2H), 3.52 - 3.44 (m, 2H), 3.31 - 3.19 (m, 4H), 2.98 - 2.86 (m, 1H), 2.84 - 2.73 (m, 1H), 2.67 - 2.60 (m, 1H), 2.60 - 2.55 (m, 2H), 2.49 (s, 3H), 2.45 - 2.22 (m, 5H), 2.21 - 2.09 (m, 1H), 2.03 - 1.83 (m, 3H) | 693.2 |

(continued)

| Example Number | Structure | Name | Intermediates | $^1$H NMR (400 MHz) δ ppm | MI |
|---|---|---|---|---|---|
| **171a** | 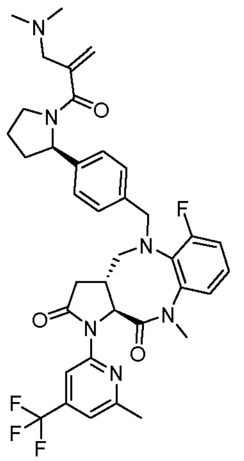 | (3a*R*,11a*S*)-5-(4-((*R*/*S*)-1-(2-((dimethylamino) methyl)acrylo yl)pyrrolidin-2-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo [2,3-*f*][1,4]diazocine-2,11(3*H*)-dione | **Step a: Intermediate 5a** and **Intermediate B16 Step c: Intermediate E1** | ($CDCl_3$) 8.30 (s, 1H), 7.35 - 7.28 (m, 1H), 7.27 - 7.14 (m, 5H), 7.14 - 7.03 (m, 2H), 5.65 - 5.47 (m, 1H), 5.37 - 4.90 (m, 2H), 4.65 (d, *J* = 9.2 Hz, 1H), 4.32 - 4.23 (m, 1H), 4.22 - 4.12 (m, 1H), 3.86 - 3.77 (m, 1H), 3.77 - 3.68 (m, 1H), 3.53 - 3.45 (m, 1H), 3.29 - 3.17 (m, 3H), 3.08 - 2.71 (m, 3H), 2.68 - 2.57 (m, 1H), 2.49 (s, 3H), 2.41 - 2.24 (m, 5H), 2.24 - 2.10 (m, 3H), 2.02 - 1.80 (m, 4H) | 693.2 |
| **171b** | 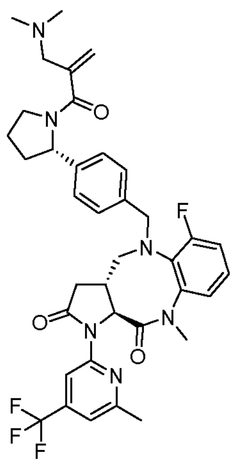 | (3a*R*,11a*S*)-5-(4-((*S*/*R*)-1-(2-((dimethylamino) methyl)acrylo yl)pyrrolidin-2-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo [2,3-*f*][1,4]diazocine-2,11(3*H*)-dione | | ($CDCl_3$) 8.30 (s, 1H), 7.39 - 7.28 (m, 2H), 7.27 - 7.24 (m, 2H), 7.21 - 7.10 (m, 3H), 7.05 (s, 1H), 5.79 - 5.50 (m, 1H), 5.45 - 4.97 (m, 2H), 4.65 (d, *J* = 9.6 Hz, 1H), 4.31 - 4.10 (m, 2H), 3.90 - 3.81 (m, 1H), 3.80 - 3.70 (m, 1H), 3.60 - 3.46 (m, 1H), 3.25 (s, 3H), 3.19 - 3.05 (m, 1H), 3.01 - 2.73 (m, 3H), 2.70 - 2.62 (m, 1H), 2.49 (s, 3H), 2.44 - 2.27 (m, 6H), 2.25 - 2.08 (m, 2H), 2.07 - 1.98 (m, 1H), 1.97 - 1.93 (m, 1H), 1.90 - 1.84 (m, 1H) | 693.2 |

(continued)

| Example Number | Structure | Name | Intermediates | $^1$H NMR (400 MHz) δ ppm | MI |
|---|---|---|---|---|---|
| **172a** | 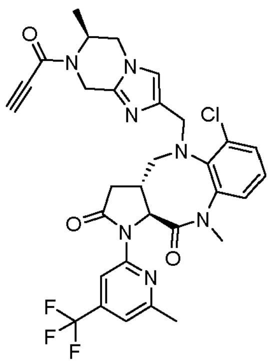 | (3*aR*,11*aS*)-6-chloro-10-methyl-1-(6-methyl-4-(trifluoromethyl)pyridin-2-yl)-5-(((*S*/*R*)-6-methyl-7-propioloyl-5,6,7,8-tetrahydroimidazo[1,2-a]pyrazin-2-yl)methyl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B26 Step c:** propiolic acid (CAS: 471-25-0) | ($CDCl_3$) 8.32 (s, 1H), 7.49 - 7.43 (m, 1H), 7.35 - 7.29 (m, 2H), 7.06 (s, 1H), 6.86 (d, *J* = 4.8 Hz, 1H), 5.40 - 5.01 (m, 2H), 4.67 (d, *J* = 9.6 Hz, 1H), 4.62 - 4.33 (m, 1H), 4.32 - 4.02 (m, 3H), 3.93 - 3.78 (m, 1H), 3.67 - 3.56 (m, 1H), 3.35 (d, *J* = 5.6 Hz, 3H), 3.23 (d, *J* = 16.8 Hz, 1H), 3.11 - 2.93 (m, 2H), 2.74 - 2.63 (m, 1H), 2.49 (s, 3H), 2.25 - 2.14 (m, 1H), 1.33 - 1.25 (m, 3H) | 640.1 |
| **172b** | 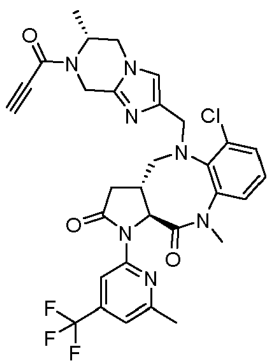 | (3*aR*,11*aS*)-6-chloro-10-methyl-1-(6-methyl-4-(trifluoromethyl)pyridin-2-yl)-5-(((*R*/*S*)-6-methyl-7-propioloyl-5,6,7,8-tetrahydroimidazo[1,2-a]pyrazin-2-yl)methyl)-1,3a,4,5,10,11a-hexahydro-2*H*-benzo[*b*]pyrrolo[2,3-*f*][1,4]diazocine-2,11(3*H*)-dione | | ($CDCl_3$) 8.32 (s, 1H), 7.47 (d, J = 7.2 Hz, 1H), 7.35 - 7.28 (m, 2H), 7.06 (s, 1H), 6.88 (s, 1H), 5.43 - 5.02 (m, 2H), 4.66 (d, *J* = 9.2 Hz, 1H), 4.63 - 4.23 (m, 2H), 4.21 - 4.05 (m, 2H), 3.95 - 3.81 (m, 1H), 3.69 - 3.58 (m, 1H), 3.33 (s, 3H), 3.27 - 3.20 (m, 1H), 3.11 - 2.93 (m, 2H), 2.73 - 2.64 (m, 1H), 2.49 (s, 3H), 2.25 - 2.14 (m, 1H), 1.32 - 1.25 (m, 3H) | 640.2 |

The **Examples** in **Table 15** were prepared using methods similar to those described in the synthesis of **Example 77,** using the listed **Intermediates** in step a, and the appropriate warhead in step d. For **Example 199,** an additional chiral SFC step was conducted after step b.

**Table 15**

| Example Number | Structure | Name | Intermediates | $^1$H NMR (400 MHz) δ ppm | MI |
|---|---|---|---|---|---|
| **173** | 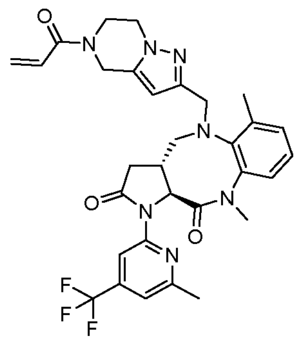 | (3a*R*,11a*S*)-5-((5-acryloyl-4,5,6,7-tetrahydropyrazolo[1,5-a]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B33** **Step d:** acrylic acid | ($CDCl_3$) 8.32 (s, 1H), 7.26 - 7.20 (m, 3H), 7.04 (s, 1H), 6.66 - 6.58 (m, 1H), 6.41 - 6.36 (m, 1H), 6.06 (s, 1H), 5.83 - 5.80 (m, 1H), 4.88 - 4.81 (m, 2H), 4.73 (d, *J* = 9.2 Hz, 1H), 4.21 - 4.11 (m, 3H), 4.10 - 3.95 (m, 3H), 3.58 - 3.52 (m, 1H), 3.39 (s, 3H), 3.10 - 2.95 (m, 1H), 2.78 - 2.64 (m, 2H), 2.49 (s, 3H), 2.44 (s, 3H), 2.18 - 2.10 (m, 1H) | 608.1 |
| **174** | 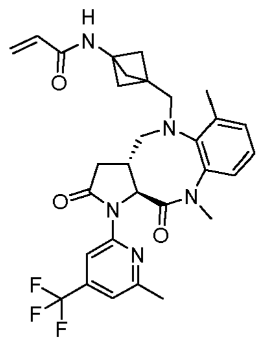 | *N*-(3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)acrylamide | **Step a: Intermediate 5b** and **Intermediate B32** **Step d:** acrylic acid | ($CDCl_3$) 8.33 (s, 1H), 7.25 - 7.20 (m, 2H), 7.19 - 7.16 (m, 1H), 7.04 (s, 1H), 6.25 (dd, *J* = 16.8, 1.2 Hz, 1H), 6.02 - 5.95 (m, 1H), 5.91 (s, 1H), 5.63 - 5.60 (m, 1H), 4.70 (d, *J* = 9.6 Hz, 1H), 3.58 (dd, *J* = 14.4, 4.8 Hz, 1H), 3.37 (s, 3H), 3.13 (s, 2H), 3.07 - 2.90 (m, 1H), 2.83 - 2.74 (m, 1H), 2.73 - 2.68 (m, 1H), 2.48 (s, 3H), 2.42 (s, 3H), 2.22 - 2.14 (m, 1H), 2.00 - 1.95 (m, 3H), 1.94 - 1.89 (m, 3H) | 568.1 |
| 175 | 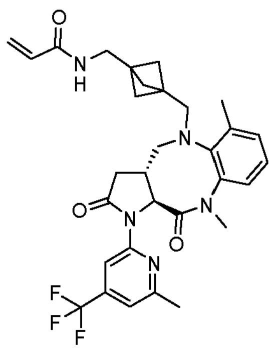 | *N*-((3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)methyl)acrylamide | **Step a: Intermediate 5b** and **Intermediate B34** **Step d:** acrylic acid | ($CDCl_3$) 8.32 (s, 1H), 7.25 - 7.14 (m, 3H), 7.04 (s, 1H), 6.32 - 6.23 (m, 1H), 6.15 - 6.04 (m, 1H), 5.64 (d, *J* = 10.4 Hz, 1H), 5.54 (br s, 1H), 4.69 (d, *J* = 9.6 Hz, 1H), 3.56 (dd, *J* = 14.4, 4.4 Hz, 1H), 3.38 (d, *J* = 6.0 Hz, 2H), 3.36 (s, 3H), 3.01 (s, 2H), 3.00 - 2.90 (m, 1H), 2.81 - 2.66 (m, 2H), 2.48 (s, 3H), 2.41 (s, 3H), 2.17 (dd, *J* = 16.8, 11.6 Hz, 1H), 1.57 - 1.54 (m, 3H), 1.51- 1.48 (m, 3H) | 582.2 |

(continued)

| Example Number | Structure | Name | Intermediates | $^1H$ NMR (400 MHz) δ ppm | MI |
|---|---|---|---|---|---|
| **176** | 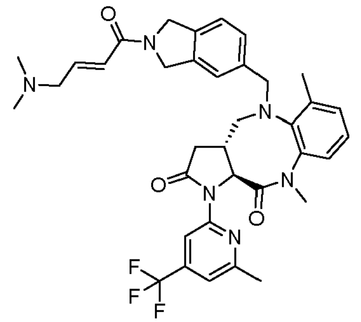 | (3a*R*,11a*S*)-5-((2-((*E*)-4-(dimethylamino)but-2-enoyl)isoindolin-5-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B18** **Step d:** (*E*)-4-(dimethylamino)but-2-enoic acid hydrochloride | ($CDCl_3$) 8.32 (s, 1H), 7.32 - 7.27 (m, 3H), 7.27 - 7.21 (m, 3H), 7.06 (s, 1H), 7.05 - 6.97 (m, 1H), 6.42 (d, *J*= 15.2 Hz, 1H), 4.93 (d, *J* = 4.4 Hz, 2H), 4.87 (s, 2H), 4.76 (dd, *J* = 9.6, 3.2 Hz, 1H), 4.22 - 4.14 (m, 1H), 4.07 - 3.98 (m, 1H), 3.48 - 3.42 (m, 1H), 3.40 (d, *J* = 10.0 Hz, 3H), 3.15 (d, *J* = 6.0 Hz, 2H), 3.04 - 2.92 (m, 1H), 2.79 - 2.69 (m, 1H), 2.65 - 2.61 (m, 1H), 2.51 (s, 3H), 2.47 (d, *J* = 3.2 Hz, 3H), 2.31 (s, 6H), 2.18 - 2.10 (m, 1H) | 661.2 |
| **177** | 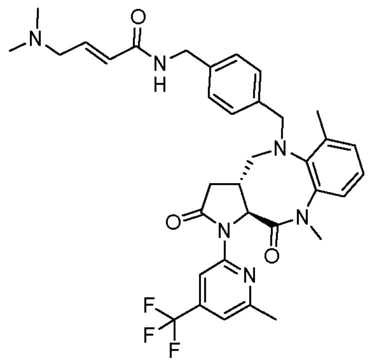 | (*E*)-*N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)benzyl)-4-(dimethylamino)but-2-enamide | **Step a: Intermediate 5b** and *tert*-butyl (4-formylbenzyl)carbamate (CAS: 156866-52-3) **Step d:** (*E*)-4-(dimethylamino)but-2-enoic acid hydrochloride | (DMSO-$d_6$) 8.49 (t, *J* = 6.0 Hz, 1H), 8.16 (d, *J* = 2.0 Hz, 1H), 7.34 (s, 1H), 7.30 - 7.25 (m, 5H), 7.24 - 7.18 (m, 2H), 6.63 - 6.55 (m, 1H), 6.10 - 6.00 (m, 1H), 4.66 (d, *J* = 8.8 Hz, 1H), 4.30 (d, *J* = 6.0 Hz, 2H), 4.05 - 4.01 (m, 1H), 3.92 - 3.88 (m, 1H), 3.28 (s, 3H), 3.27 - 3.25 (m, 1H), 3.01 - 2.99 (m, 2H), 2.91 - 2.81 (m, 2H), 2.60 - 2.56 (m, 1H), 2.49 (s, 3H), 2.37 (s, 3H), 2.35 - 2.31 (m, 1H), 2.14 (s, 6H) | 649.3 |
| **178** | 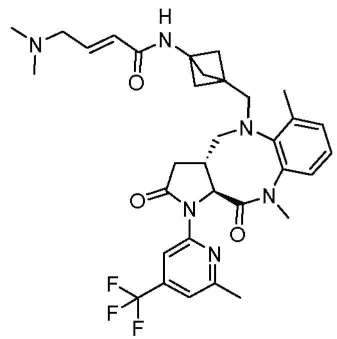 | (*E*)-*N*-(3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)-4-(dimethylamino)but-2-enamide | **Step a: Intermediate 5b** and **Intermediate B32** **Step d:** (*E*)-4-(dimethylamino)but-2-enoic acid hydrochloride | ($CDCl_3$) 8.32 (s, 1H), 7.23 - 7.20 (m, 2H), 7.19 - 7.15 (m, 1H), 7.04 (s, 1H), 6.81 - 6.74 (m, 1H), 5.90 - 5.80 (m, 2H), 4.70 (d, *J* = 9.6 Hz, 1H), 3.58 (dd, J = 14.4, 4.4 Hz, 1H), 3.37 (s, 3H), 3.12 (s, 2H), 3.05 (dd, *J* = 6.0, 1.2 Hz, 2H), 3.02 - 2.92 (m, 1H), 2.82 - 2.74 (m, 1H), 2.71 - 2.68 (m, 1H), 2.48 (s, 3H), 2.41 (s, 3H), 2.25 (s, 6H), 2.19 - 2.14 (m, 1H), 1.98 - 1.94 (m, 3H), 1.91 - 1.87 (m, 3H) | 625.2 |

(continued)

| Example Number | Structure | Name | Intermediates | $^1$H NMR (400 MHz) δ ppm | MI |
|---|---|---|---|---|---|
| 179 | 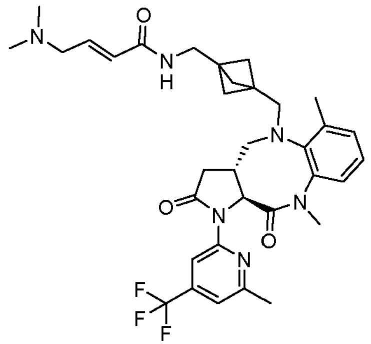 | (*E*)-*N*-((3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)methyl)-4-(dimethylamino)but-2-enamide | **Step a: Intermediate 5b** and **Intermediate B34** **Step d:** (*E*)-4-(dimethylamino)but-2-enoic acid hydrochloride | ($CDCl_3$) 8.32 (s, 1H), 7.23 - 7.14 (m, 3H), 7.04 (s, 1H), 6.84 - 6.76 (m, 1H), 5.95 (d, *J* = 15.2 Hz, 1H), 5.44 (t, *J* = 5.2 Hz, 1H), 4.69 (d, *J* = 9.6 Hz, 1H), 3.56 (dd, *J* = 14.4, 4.4 Hz, 1H), 3.38 - 3.34 (m, 5H), 3.06 (dd, *J* = 6.0, 1.2 Hz, 2H), 3.01 (s, 2H), 2.99 - 2.92 (m, 1H), 2.81 - 2.67 (m, 2H), 2.48 (s, 3H), 2.41 (s, 3H), 2.26 (s, 6H), 2.18 - 2.14 (m, 1H), 1.56 - 1.53 (m, 3H), 1.50 - 1.47 (m, 3H) | 639.2 |
| 180 | 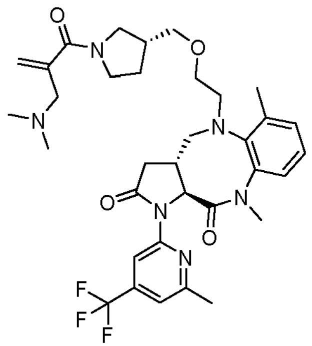 | (3a*R*,11a*S*)-5-(2-(((*R*)-1-(2-((dimethylamino)methyl)acrylo yl)pyrrolidin-3-yl)methoxy)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B29** **Step d: Intermediate E1** | ($CDCl_3$) 8.33 (s, 1H), 7.25 - 7.20 (m, 3H), 7.04 (s, 1H), 5.76 (s, 1H), 5.53 (s, 1H), 4.70 (dd, *J* = 9.6, 2.0 Hz, 1H), 3.75 - 3.58 (m, 3H), 3.57 - 3.44 (m, 2H), 3.44 - 3.29 (m, 9H), 3.28 - 3.13 (m, 2H), 3.10 - 2.96 (m, 2H), 2.84 - 2.68 (m, 2H), 2.49 (s, 3H), 2.45 (s, 6H), 2.38 (d, *J* = 4.8 Hz, 3H), 2.19 (dd, *J* = 16.8, 11.6 Hz, 1H), 2.02 - 1.97 (m, 1H), 1.69 - 1.65 (m, 1H) | 657.1 |
| 181 | 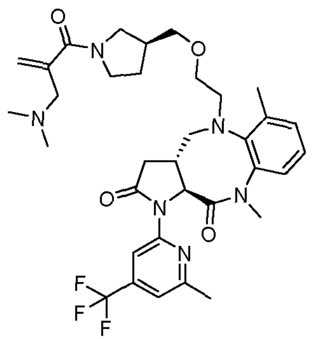 | (3a*R*,11a*S*)-5-(2-(((*S*)-1-(2-((dimethylamino)methyl)acrylo yl)pyrrolidin-3-yl)methoxy)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B28** **Step d: Intermediate E1** | ($CDCl_3$) 8.33 (s, 1H), 7.25 - 7.19 (m, 3H), 7.04 (s, 1H), 5.45 (s, 1H), 5.33 (s, 1H), 4.70 (d, *J* = 9.6 Hz, 1H), 3.70 - 3.58 (m, 3H), 3.55 - 3.47 (m, 1H), 3.43 - 3.35 (m, 6H), 3.30 - 3.19 (m, 3H), 3.18 - 2.94 (m, 4H), 2.83 - 2.75 (m, 1H), 2.74 - 2.67 (m, 1H), 2.49 (s, 3H), 2.48 - 2.42 (m, 1H), 2.39 (d, *J* = 4.8 Hz, 3H), 2.26 (s, 6H), 2.23 - 2.14 (m, 1H), 2.03 - 1.93 (m, 1H), 1.67 - 1.64 (m, 1H) | 657.3 |

(continued)

| Example Number | Structure | Name | Intermediates | $^1$H NMR (400 MHz) δ ppm | MI |
|---|---|---|---|---|---|
| **182** | 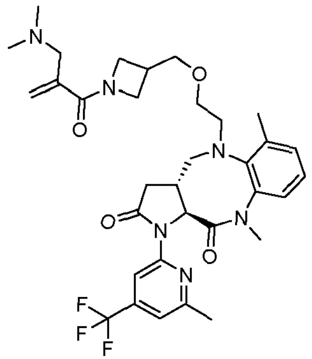 | (3a*R*,11a*S*)-5-(2-((1-(2-((dimethylamino)methyl) acrylo yl)azetidin-3-yl)methoxy)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B31** **Step d: Intermediate E1** | ($CDCl_3$) 8.32 (s, 1H), 7.26 - 7.16 (m, 3H), 7.04 (s, 1H), 5.57 (s, 1H), 5.47 (s, 1H), 4.70 (d, *J* = 9.6 Hz, 1H), 4.30 - 4.19 (m, 1H), 4.10 (t, *J* = 9.2 Hz, 1H), 3.96 - 3.87 (m, 1H), 3.78 - 3.68 (m, 1H), 3.63 (dd, *J* = 14.4, 4.8 Hz, 1H), 3.56 - 3.47 (m, 2H), 3.46 - 3.39 (m, 2H), 3.35 (s, 3H), 3.27 - 3.19 (m, 1H), 3.18 - 3.04 (m, 3H), 3.03 - 2.91 (m, 1H), 2.84 - 2.67 (m, 3H), 2.49 (s, 3H), 2.38 (s, 3H), 2.28 (s, 6H), 2.25 - 2.18 (m, 1H) | 643.2 |
| **183** | 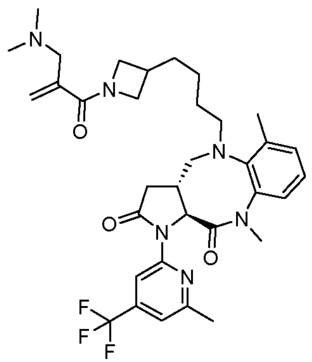 | (3a*R*,11a*S*)-5-(4-(1-(2-((dimethylamino)methyl) acrylo yl)azetidin-3-yl)butyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B30** **Step d: Intermediate E1** | ($CDCl_3$) 8.32 (s, 1H), 7.26 - 7.17 (m, 3H), 7.04 (s, 1H), 5.60 (s, 1H), 5.47 (s, 1H), 4.69 (d, *J* = 9.6 Hz, 1H), 4.28 (t, *J* = 8.4 Hz, 1H), 4.13 (t, *J* = 8.8 Hz, 1H), 3.78 (dd, *J* = 8.0, 6.0 Hz, 1H), 3.67 - 3.53 (m, 2H), 3.35 (s, 3H), 3.27 - 3.11 (m, 2H), 3.00 - 2.85 (m, 3H), 2.83 - 2.68 (m, 2H), 2.59 - 2.51 (m, 1H), 2.49 (s, 3H), 2.36 (s, 3H), 2.29 (s, 6H), 2.19 (dd, *J* = 16.8, 11.6 Hz, 1H), 1.57 - 1.56 (m, 2H), 1.36 (m, 2H), 1.29 - 1.20 (m, 2H) | 641.2 |

(continued)

| Example Number | Structure | Name | Intermediates | $^1$H NMR (400 MHz) δ ppm | MI |
|---|---|---|---|---|---|
| **184** | 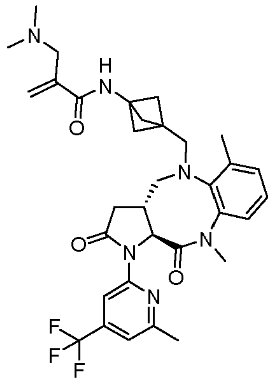 | *N*-(3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)-2-((dimethylamino)methyl)acryla mide | **Step a: Intermediate 5b** and **Intermediate B32**<br>**Step d: Intermediate E1** | ($CDCl_3$) 9.39 (s, 1H), 8.32 (s, 1H), 7.24 - 7.20 (m, 2H), 7.20 - 7.16 (m, 1H), 7.04 (s, 1H), 6.18 (d, *J* = 2.0 Hz, 1H), 5.35 (s, 1H), 4.70 (d, *J* = 9.6 Hz, 1H), 3.59 (dd, *J* = 14.4, 4.4 Hz, 1H), 3.37 (s, 3H), 3.18 - 3.08 (m, 2H), 3.06 (s, 2H), 3.03 - 2.92 (m, 1H), 2.84 - 2.67 (m, 2H), 2.48 (s, 3H), 2.42 (s, 3H), 2.20 (s, 6H), 2.19 - 2.16 (m, 1H), 1.97 - 1.87 (m, 6H) | 625.2 |
| **185** | 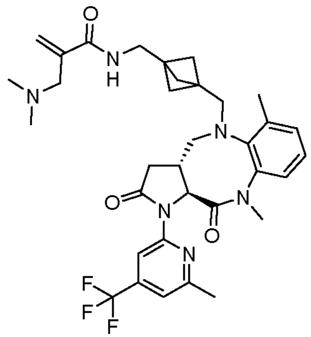 | *N*-((3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)methyl)-2-((dimethylamino)methyl)acryla mide | **Step a: Intermediate 5b** and **Intermediate B34**<br>**Step d: Intermediate E1** | ($CDCl_3$) 9.18 (s, 1H), 8.33 (s, 1H), 7.24 - 7.15 (m, 3H), 7.04 (s, 1H), 6.20 (d, *J* = 2.0 Hz, 1H), 5.39 (s, 1H), 4.70 (d, *J* = 9.6 Hz, 1H), 3.61 - 3.56 (m, 1H), 3.37 (s, 3H), 3.34 (d, *J* = 5.2 Hz, 2H), 3.11 (s, 2H), 3.02 (d, *J* = 2.0 Hz, 2H), 3.00 - 2.93 (m, 1H), 2.81 - 2.68 (m, 2H), 2.48 (s, 3H), 2.42 (s, 3H), 2.23 (s, 6H), 2.22 - 2.14 (m, 1H), 1.54 - 1.51 (m, 3H), 1.48 - 1.45 (m, 3H) | 639.2 |

(continued)

| Example Number | Structure | Name | Intermediates | $^1$H NMR (400 MHz) δ ppm | MI |
|---|---|---|---|---|---|
| **186** | 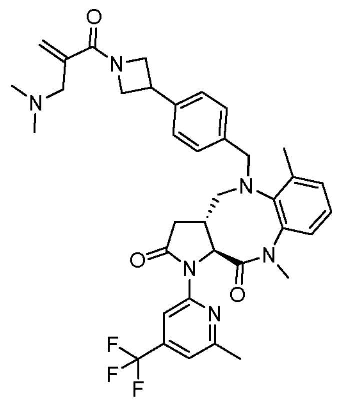 | (3a*R*,11a*S*)-5-(4-(1-(2-((dimethylamino)methyl) acrylo yl)azetidin-3-yl)benzyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B13** **Step d: Intermediate E1** | ($CDCl_3$) 8.32 (s, 1H), 7.39 - 7.34 (m, 2H), 7.33 - 7.28 (m, 2H), 7.27 - 7.22 (m, 3H), 7.06 (s, 1H), 5.88 (s, 1H), 5.69 (s, 1H), 4.76 (d, *J* = 9.6 Hz, 1H), 4.70 (t, *J* = 8.8 Hz, 1H), 4.52 (t, *J* = 9.6 Hz, 1H), 4.29 (d, *J* = 6.8 Hz, 1H), 4.21 - 4.10 (m, 2H), 3.99 (d, *J* = 14.0 Hz, 1H), 3.92 - 3.83 (m, 1H), 3.52 - 3.31 (m, 6H), 3.05 - 2.94 (m, 1H), 2.72 (dd, J = 14.4, 12.0 Hz, 1H), 2.64 (dd, *J* = 16.8, 8.8 Hz, 1H), 2.50 (s, 3H), 2.48 - 2.46 (m, 9H), 2.18 - 2.10 (m, 1H) | 675.3 |
| **187** | 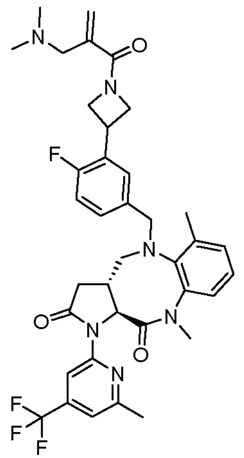 | (3a*R*,11a*S*)-5-(3-(1-(2-((dimethylamino)methyl) acrylo yl)azetidin-3-yl)-4-fluorobenzyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B15** **Step d: Intermediate E1** | ($CDCl_3$) 8.33 (s, 1H), 7.34 - 7.28 (m, 1H), 7.27 - 7.18 (m, 4H), 7.07 - 6.99 (m, 2H), 5.55 (s, 1H), 5.52 (s, 1H), 4.75 (d, *J* = 9.6 Hz, 1H), 4.64 (d, *J* = 4.4 Hz, 1H), 4.50 (t, *J* = 9.2 Hz, 1H), 4.30 (d, *J* = 6.8 Hz, 1H), 4.15 (d, *J* = 14.0 Hz, 2H), 4.07 (d, *J* = 4.8 Hz, 1H), 3.99 (d, *J* = 14.0 Hz, 1H), 3.49 - 3.36 (m, 4H), 3.27 - 3.17 (m, 1H), 3.12 - 3.04 (m, 1H), 3.03 - 2.92 (m, 1H), 2.81 - 2.72 (m, 1H), 2.66 (dd, *J* = 17.0, 8.8 Hz, 1H), 2.49 (d, *J* = 12.4 Hz, 6H), 2.25 (s, 6H), 2.15 (dd, *J* = 16.8, 11.6 Hz, 1H) | 693.3 |

(continued)

| Example Number | Structure | Name | Intermediates | $^1$H NMR (400 MHz) δ ppm | MI |
|---|---|---|---|---|---|
| **188** | 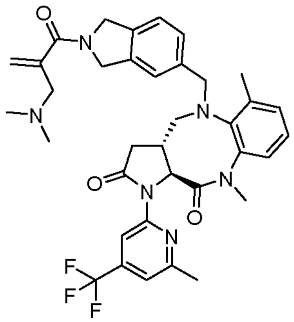 | (3a*R*,11a*S*)-5-((2-(2-((dimethylamino)methyl) acrylo yl)isoindolin-5-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B18** **Step d: Intermediate E1** | (DMSO-$d_6$) 9.33 (br s, 1H, HCl salt), 8.18 (s, 1H), 7.37 - 7.24 (m, 7H), 6.09 (d, *J* = 4.0 Hz, 1H), 6.03 (s, 1H), 4.94 (s, 2H), 4.76 (s, 2H), 4.68 (d, *J* = 9.2 Hz, 1H), 4.13 - 4.04 (m, 1H), 4.01 - 3.92 (m, 3H), 3.34 - 3.25 (m, 4H), 2.96 - 2.88 (m, 1H), 2.86 - 2.80 (m, 1H), 2.78 (d, *J* = 4.4 Hz, 6H), 2.57 - 2.52 (m, 1H), 2.50 (s, 3H), 2.39 (s, 3H), 2.37 - 2.31 (m, 1H) | 661.1 |
| **189** | 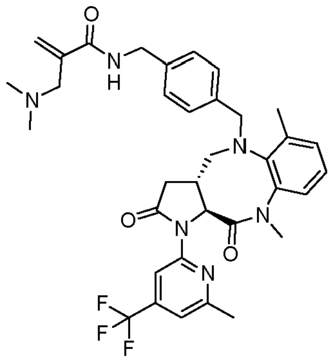 | *N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*] pyrrolo[2,3-*f*][1,4]diazocin-5-yl)methyl)benzyl)-2-((dimethylamino)methyl)acryla mide | **Step a: Intermediate 5b** and *tert*-butyl (4-formylbenzyl)carbamate (CAS: 156866-52-3) **Step d: Intermediate E1** | ($CDCl_3$) 9.58 (br s, 1H), 8.32 (s, 1H), 7.33 - 7.29 (m, 2H), 7.27 - 7.21 (m, 5H), 7.05 (s, 1H), 6.28 (d, *J* = 1.6 Hz, 1H), 5.45 (s, 1H), 4.76 (d, *J* = 9.6 Hz, 1H), 4.52 (d, *J* = 5.6 Hz, 2H), 4.15 (d, *J* = 14.0 Hz, 1H), 3.96 (d, *J* = 14.0 Hz, 1H), 3.46 - 3.39 (m, 4H), 3.17 (s, 2H), 3.07 - 2.94 (m, 1H), 2.75 - 2.60 (m, 2H), 2.50 (s, 3H), 2.46 (s, 3H), 2.22 (s, 6H), 2.14 - 2.09 (m, 1H) | 649.1 |
| **190** | 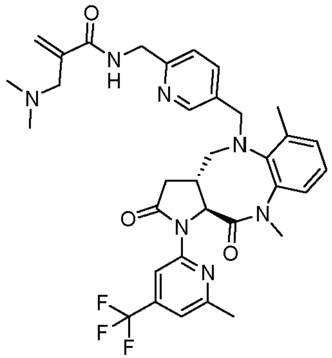 | *N*-((5-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*] pyrrolo[2,3-*f*][1,4]diazocin-5-yl)methyl)pyridin-2-yl)methyl)-2-((dimethylamino)methyl)acryla mide | **Step a: Intermediate 5b and Intermediate B40** **Step d: Intermediate E1** | ($CDCl_3$) 9.83 (br s, 1H), 8.50 (d, *J* = 1.6 Hz, 1H), 8.33 (s, 1H), 7.64 (dd, *J* = 8.0, 2.0 Hz, 1H), 7.29 (s, 1H), 7.27 - 7.22 (m, 3H), 7.06 (s, 1H), 6.28 (d, *J* = 1.6 Hz, 1H), 5.49 (s, 1H), 4.76 (d, *J* = 9.6 Hz, 1H), 4.64 (d, *J* = 5.6 Hz, 2H), 4.18 (d, *J* = 14.0 Hz, 1H), 3.99 (d, *J* = 14.0 Hz, 1H), 3.48 - 3.37 (m, 4H), 3.22 (s, 2H), 3.10 - 2.92 (m, 1H), 2.80 - 2.61 (m, 2H), 2.50 (s, 3H), 2.44 (s, 3H), 2.29 (s, 6H), 2.16 (dd, *J* = 16.8, 11.6 Hz, 1H) | 650.4 |

(continued)

| Example Number | Structure | Name | Intermediates | $^1H$ NMR (400 MHz) δ ppm | MI |
|---|---|---|---|---|---|
| **191** | 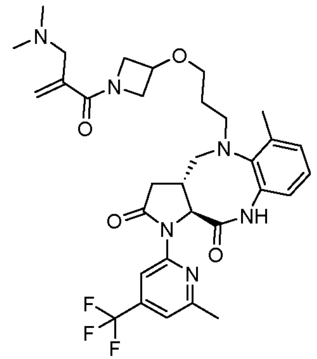 | (3a*R*,11a*S*)-5-(3-((1-(2-((dimethylamino)methyl) acrylo yl)azetidin-3-yl)oxy)propyl)-6-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 14** and **Intermediate B4** **Step d: Intermediate E1** | ($CDCl_3$) 8.31 (s, 1H), 7.55 (s, 1H), 7.25 - 7.16 (m, 2H), 7.14 - 7.09 (m, 1H), 7.06 (s, 1H), 5.62 (s, 1H), 5.50 (s, 1H), 4.70 (d, *J* = 9.2 Hz, 1H), 4.34 - 4.31 (m, 1H), 4.27 - 4.14 (m, 2H), 4.08 - 4.01 (m, 1H), 3.94 - 3.77 (m, 1H), 3.66 - 3.54 (m, 1H), 3.44 - 3.27 (m, 3H), 3.19 (s, 2H), 3.14 - 2.91 (m, 3H), 2.91 - 2.82 (m, 1H), 2.75 (dd, *J* = 16.8, 8.4 Hz, 1H), 2.50 (s, 3H), 2.37 (s, 3H), 2.33 (s, 6H), 1.65 - 1.56 (m, 2H) | 629.4 |
| **192** | 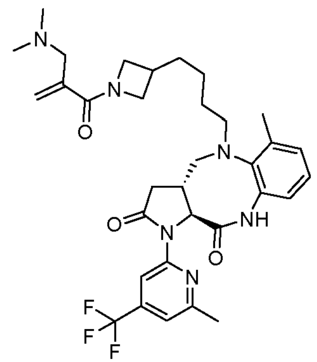 | (3a*R*,11a*S*)-5-(4-(1-(2-((dimethylamino)methyl) acrylo yl)azetidin-3-yl)butyl)-6-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 14** and **Intermediate B30** **Step d: Intermediate E1** | ($CDCl_3$) 8.32 (s, 1H), 7.31 (s, 1H), 7.25 - 7.21 (m, 1H), 7.18 (t, *J* = 7.6 Hz, 1H), 7.12 - 7.08 (m, 1H), 7.06 (s, 1H), 5.48 (s, 1H), 5.40 (s, 1H), 4.70 (d, *J* = 9.2 Hz, 1H), 4.27 - 4.23 (m, 1H), 4.13 - 4.08 (m, 1H), 3.80 - 3.72 (m, 1H), 3.70 - 3.56 (m, 2H), 3.16 - 3.04 (m, 3H), 3.02 - 2.94 (m, 2H), 2.89 - 2.81 (m, 1H), 2.76 (m, 1H), 2.52 - 2.51 (m, 1H), 2.50 (s, 3H), 2.37 (s, 3H), 2.27 - 2.17 (m, 7H), 1.58 - 1.51 (m, 2H), 1.43 - 1.30 (m, 2H), 1.29 - 1.20 (m, 2H) | 627.3 |

(continued)

| Example Number | Structure | Name | Intermediates | $^1$H NMR (400 MHz) δ ppm | MI |
|---|---|---|---|---|---|
| **193** | 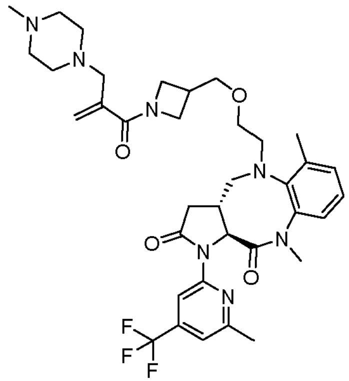 | (3*aR*,11*aS*)-6,10-dimethyl-1-(6-methyl-4-(trifluoromethyl)pyridin-2-yl)-5-(2-((1-(2-((4-methylpiperazin-1-yl)methyl)acryloyl)azetidin-3-yl)methoxy)ethyl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B31**<br>**Step d: Intermediate E8** | ($CDCl_3$) 8.28 (s, 1H), 7.24 - 7.16 (m, 3H), 7.04 (s, 1H), 6.30 (s, 1H), 5.95 (s, 1H), 4.66 (d, *J* = 9.2 Hz, 1H), 4.47 - 4.43 (m, 2H), 4.23 - 4.18 (m, 1H), 4.16 - 4.10 (m, 3H), 4.00 - 3.95 (m, 2H), 3.90 - 3.75 (m, 7H), 3.68 - 3.54 (m, 3H), 3.43 (s, 2H), 3.33 (s, 3H), 3.18 - 3.13 (m, 1H), 3.11 - 3.00 (m, 4H), 2.99 - 2.91 (m, 1H), 2.83 - 2.65 (m, 2H), 2.48 (s, 3H), 2.35 (s, 3H), 2.20 - 2.12 (m, 1H) | 698.3 |
| **194** | 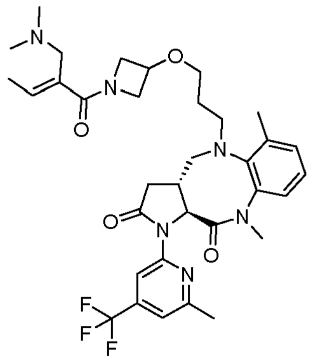 | (3*aR*,11*aS*)-5-(3-((1-((*E*)-2-((dimethylamino)methyl)but-2-enoyl)azetidin-3-yl)oxy)propyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B4**<br>**Step d: Intermediate E5** | ($CDCl_3$) 8.33 (s, 1H), 7.26 - 7.18 (m, 3H), 7.05 (s, 1H), 6.24 - 6.19 (m, 1H), 4.70 (d, *J* = 9.2 Hz, 1H), 4.40 - 4.17 (m, 3H), 4.09 - 3.77 (m, 2H), 3.59 - 3.56 (m, 2H), 3.37 (s, 6H), 3.19 - 2.87 (m, 4H), 2.86 - 2.69 (m, 2H), 2.49 (s, 3H) 2.48 - 2.38 (m, 6H), 2.37 (m, 3H), 2.23 - 2.15 (m, 1H), 1.86 (d, *J* = 7.0 Hz, 2H), 1.68 - 1.62 (m, 2H) | 657.3 |

(continued)

| Example Number | Structure | Name | Intermediates | $^1$H NMR (400 MHz) δ ppm | MI |
|---|---|---|---|---|---|
| **195** | 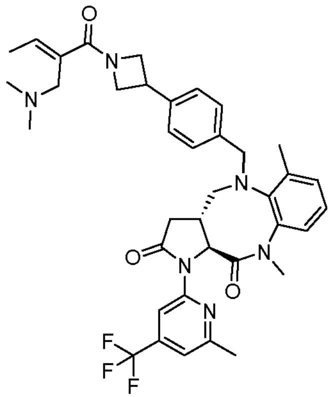 | (3*aR*,11*aS*)-5-(4-(1-((*E*)-2-((dimethylamino) methyl)but-2-enoyl)azetidin-3-yl)benzyl)-6,10-dimethyl-1-(6-methyl-4-(trifluoromethyl)pyridin-2-yl)-1,3a,4,5,10,11a-hexahydro-2*H*-benzo [*b*]pyrrolo[2,3-*f*][1,4]diazocine-2,11(3*H*)-dione | **Step a: Intermediate 5b** and **Intermediate B13** **Step d: Intermediate E5** | ($CDCl_3$) 8.34 (s, 1H), 7.38 - 7.30 (m, 4H), 7.27 - 7.25 (m, 3H), 7.07 (s, 1H), 6.20 - 5.68 (m, 1H), 4.78 (d, *J* = 9.6 Hz, 1H), 4.67 - 4.44 (m, 2H), 4.28 - 4.08 (m, 3H), 3.99 (d, *J* = 13.6 Hz, 1H), 3.86 - 3.77 (m, 1H), 3.49 - 3.38 (m, 4H), 3.34 - 3.14 (m, 2H), 3.11 - 2.96 (m, 1H), 2.78 - 2.62 (m, 2H), 2.50 (d, *J* = 11.5 Hz, 6H), 2.31 - 2.23 (m, 6H), 2.19 - 2.11 (m, 1H), 1.85 - 1.82 (m, 3H) | 689.3 |
| **196** | 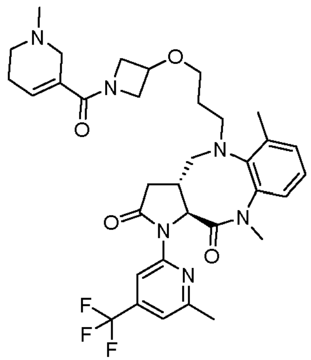 | (3*aR*,11*aS*)-6,10-dimethyl-5-(3-((1-(1-methyl-1,2,5,6-tetrahydropyridine-3-carbonyl) azetidin-3-yl)oxy)propyl)-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B4** **Step d:** 1-methyl-1,2,5,6-tetrahydropyridine-3-carboxylic acid (CAS: 6018-28-6) | ($CDCl_3$) 8.33 (s, 1H), 7.26 - 7.19 (m, 3H), 7.05 (s, 1H), 6.21 - 6.19 (m, 1H), 4.70 (d, *J* = 9.2 Hz, 1H), 4.42 - 4.15 (m, 3H), 4.09 - 3.79 (m, 2H), 3.59 - 3.56 (m, 1H), 3.36 (s, 5H), 3.14 (s, 2H), 3.07 - 3.02 (m, 2H), 3.01 - 2.91 (m, 1H), 2.85 - 2.69 (m, 2H), 2.55 - 2.52 (m, 2H), 2.49 (s, 3H), 2.37 (s, 3H), 2.39 - 2.32 (m, 5H), 2.24 - 2.14 (m, 1H), 1.63 - 1.57 (m, 2H) | 655.2 |

(continued)

| Example Number | Structure | Name | Intermediates | $^1$H NMR (400 MHz) δ ppm | MI |
|---|---|---|---|---|---|
| **197** | 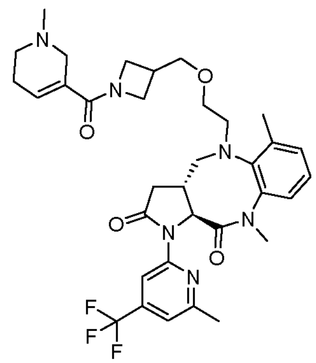 | (3a*R*,11a*S*)-6,10-dimethyl-5-(2-((1-(1-methyl-1,2,5,6-tetrahydropyridine-3-carbonyl)azetidin-3-yl)methoxy)ethyl)-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B31** **Step d:** 1-methyl-1,2,5,6-tetrahydropyridine-3-carboxylic acid (CAS: 6018-28-6) | ($CDCl_3$) 8.32 (s, 1H), 7.26 - 7.17 (m, 3H), 7.04 (s, 1H), 6.21 (s, 1H), 4.70 (d, *J* = 9.6 Hz, 1H), 4.32 - 4.17 (m, 1H), 4.15 - 4.02 (m, 1H), 4.00 - 3.85 (m, 1H), 3.78 - 3.67 (m, 1H), 3.64 - 3.60 (m, 1H), 3.50 - 3.46 (m, 2H), 3.46 - 3.40 (m, 2H), 3.35 (s, 3H), 3.21 - 3.12 (m, 3H), 3.12 - 3.04 (m, 1H), 3.02 - 2.93 (m, 1H), 2.83 - 2.74 (m, 2H), 2.73 - 2.67 (m, 1H), 2.54 - 2.51 (m, 2H), 2.49 (s, 3H), 2.41 (s, 3H), 2.37 (s, 3H), 2.34 - 2.30 (m, 2H), 2.22 - 2.16 (m, 1H) | 655.2 |
| **198** | 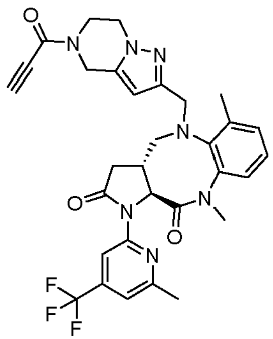 | (3a*R*,11a*S*)-6,10-dimethyl-1-(6-methyl-4-(trifluoromethyl)pyridin-2-yl)-5-((5-propioloyl-4,5,6,7-tetrahydropyrazolo[1,5-*a*]pyrazin-2-yl)methyl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B33** **Step d:** propiolic acid (CAS: 471-25-0) | ($CDCl_3$) 8.32 (s, 1H), 7.26 - 7.21 (m, 3H), 7.04 (s, 1H), 6.08 (d, *J* = 19.2 Hz, 1H), 5.00 (s, 1H), 4.91 - 4.79 (m, 1H), 4.77 - 4.71 (m, 1H), 4.31 - 4.22 (m, 2H), 4.21 - 4.13 (m, 2H), 4.13 - 3.95 (m, 2H), 3.57 - 3.52 (m, 1H), 3.40 (d, *J* = 2.4 Hz, 3H), 3.24 (d, *J* = 19.6 Hz, 1H), 3.11 - 2.95 (m, 1H), 2.82 - 2.73 (m, 1H), 2.71 - 2.65 (m, 1H), 2.50 (d, *J* = 3.6 Hz, 3H), 2.44 (d, *J* = 8.0 Hz, 3H), 2.21 - 2.10 (m, 1H) | 606.1 |
| **199** | 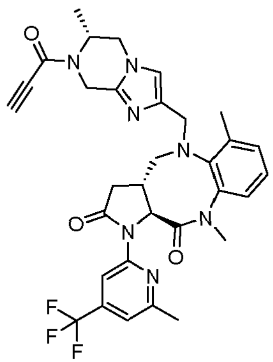 | (3a*R*,11a*S*)-6,10-dimethyl-1-(6-methyl-4-(trifluoromethyl)pyridin-2-yl)-5-(((*R*/*S*)-6-methyl-7-propioloyl-5,6,7,8-tetrahydroimidazo[1,2-a]pyrazin-2-yl)methyl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B26** **Step d:** propiolic acid (CAS: 471-25-0) | ($CDCl_3$) 8.32 (s, 1H), 7.26 - 7.21 (m, 3H), 7.05 (s, 1H), 6.75 (d, *J* = 8.8 Hz, 1H), 5.40 - 5.04 (m, 2H), 4.74 (dd, J = 9.6, 2.0 Hz, 1H), 4.63 - 4.23 (m, 1H), 4.22 - 4.01 (m, 3H), 3.90 - 3.78 (m, 1H), 3.66 - 3.55 (m, 1H), 3.37 (s, 3H), 3.24 (d, *J* = 18.0 Hz, 1H), 3.07 - 2.94 (m, 1H), 2.84 - 2.75 (m, 1H), 2.73 - 2.64 (m, 1H), 2.51 (s, 3H), 2.44 (s, 3H), 2.21 - 2.12 (m, 1H), 1.33 - 1.24 (m, 3H) | 620.2 |

(continued)

| Example Number | Structure | Name | Intermediates | $^1$H NMR (400 MHz) δ ppm | MI |
|---|---|---|---|---|---|
| **200** | 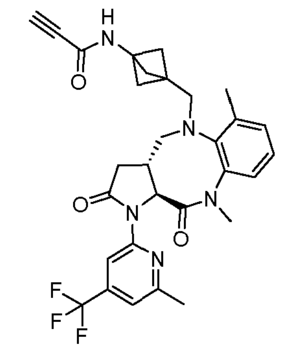 | *N*-(3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-fl[1,4]diazocin-5-yl)methyl)bicyclo[1.1.1]pentan-1-yl)propiolamide | **Step a: Intermediate 5b** and **Intermediate B32** **Step d:** propiolic acid (CAS: 471-25-0) | ($CDCl_3$) 8.33 (s, 1H), 7.24 - 7.19 (m, 3H), 7.04 (s, 1H), 6.16 (s, 1H), 4.70 (d, *J* = 9.6 Hz, 1H), 3.57 (dd, J = 14.4, 4.4 Hz, 1H), 3.39 (s, 3H), 3.16 - 3.10 (m, 2H), 3.05 - 2.92 (m, 1H), 2.86 - 2.78 (m, 1H), 2.76 - 2.68 (m, 2H), 2.48 (s, 3H), 2.41 (s, 3H), 2.20 - 2.14 (m, 1H), 1.99 - 1.88 (m, 6H) | 566.2 |
| **201** | 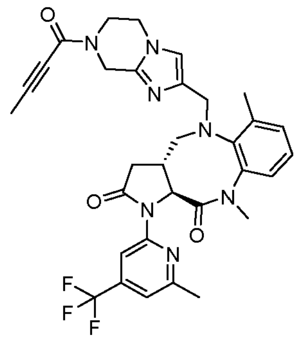 | (3a*R*,11a*S*)-5-((7-(but-2-ynoyl)-5,6,7,8-tetrahydroimidazo[1,2-a]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B19** **Step d:** but-2-ynoic acid (CAS: 590-93-2) | ($CDCl_3$) 8.32 (s, 1H), 7.26 - 7.20 (m, 3H), 7.05 (s, 1H), 6.82 - 6.69 (m, 1H), 4.99 (d, *J* = 2.4 Hz, 1H), 4.84 - 4.70 (m, 2H), 4.21 - 3.94 (m, 6H), 3.65 - 3.60 (m, 1H), 3.37 (s, 3H), 3.13 - 2.92 (m, 1H), 2.87 - 2.61 (m, 2H), 2.50 (s, 3H), 2.44 (s, 3H), 2.22 - 2.11 (m, 1H), 2.09 - 2.02 (m, 3H) | 620.2 |

The **Examples in Table 16** were prepared using methods similar to those described in the synthesis of **Example 77,** using the listed **Intermediates** in step a. The final step was conducted in a similar manner to the amide coupling with acryloyl chloride described for **Example 1.** For **Example 206a** and **Example 206b** an additional chiral SFC step was conducted after step b.

**Table 16**

| Example Number | Structure | Name | Intermediates | $^1$H NMR (400 MHz) δ ppm | MI |
|---|---|---|---|---|---|
| **202** | 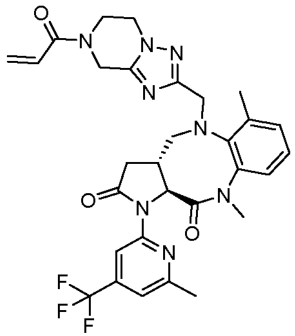 | (3*aR*,11*aS*)-5-((7-acryloyl-5,6,7,8-tetrahydro-[1,2,4]triazolo[1,5-a]pyrazin-2-yl) methyl)-6,10-dimethyl-1-(6-methyl-4-(trifluoromethyl)pyridin-2-yl)-1,3a,4,5,10,11a-hexahydro-2*H*-benzo [*b*]pyrrolo[2,3-*f*][1,4]diazocine-2,11(3*H*)-dione | **Intermediate 5b** and **Intermediate B20** | ($CDCl_3$) 8.35 (s, 1H), 7.26 - 7.22 (m, 3H), 7.06 (s, 1H), 6.69 - 6.56 (m, 1H), 6.47 - 6.39 (m, 1H), 5.87 (d, *J* = 10.4 Hz, 1H), 4.92 (s, 2H), 4.76 (d, *J* = 9.6 Hz, 1H), 4.32 - 4.14 (m, 4H), 4.13 - 3.98 (m, 2H), 3.82 - 3.70 (m, 1H), 3.40 (s, 3H), 3.25 - 3.07 (m, 1H), 2.91 - 2.70 (m, 2H), 2.51 (s, 3H), 2.41 (s, 3H), 2.24 - 2.16 (m, 1H) | 609.4 |
| **203** | 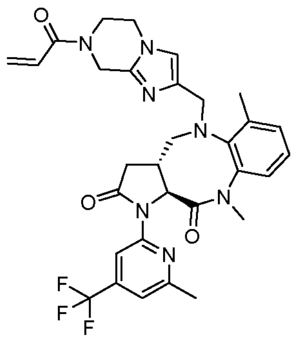 | (3*aR*,11*aS*)-5-((7-acryloyl-5,6,7,8-tetrahydroimidazo[1,2-a]pyrazin-2-yl) methyl)-6,10-dimethyl-1-(6-methyl-4-(trifluoromethyl)pyridin-2-yl)-1,3a,4,5,10,11a-hexahydro-2*H*-benzo [*b*]pyrrolo[2,3-*f*][1,4]diazocine-2,11(3*H*)-dione | **Intermediate 5b** and **Intermediate B19** | (DMSO-$d_6$) 8.17 (s, 1H), 7.35 (s, 1H), 7.31 - 7.19 (m, 3H), 6.96 (s, 1H), 6.94 - 6.85 (m, 1H), 6.17 (dd, *J* = 16.4, 2.0 Hz, 1H), 5.75 (d, *J* = 10.4 Hz, 1H), 4.78 (s, 1H), 4.70 - 4.60 (m, 2H), 4.03 - 3.87 (m, 5H), 3.73 (d, *J* = 14.4 Hz, 1H), 3.52 - 3.41 (m, 1H), 3.33 - 3.30 (m, 3H), 3.28 (s, 3H), 2.91 - 2.79 (m, 2H), 2.59 - 2.53 (m, 1H), 2.42 - 2.34 (m, 4H) | 608.4 |

(continued)

| Example Number | Structure | Name | Intermediates | $^1$H NMR (400 MHz) δ ppm | MI |
|---|---|---|---|---|---|
| **204** | 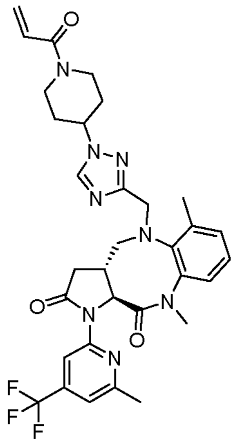 | (3a*R*,11a*S*)-5-((1-(1-acryloylpiperidin-4-yl)-1*H*-1,2,4-triazol-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B9** | ($CDCl_3$) 8.33 (s, 1H), 8.19 (s, 1H), 7.23 - 7.18 (m, 3H), 7.04 (s, 1H), 6.60 (dd, *J* = 16.8, 10.4 Hz, 1H), 6.32 (dd, *J* = 16.8, 1.6 Hz, 1H), 5.75 (dd, *J* = 10.4, 1.6 Hz, 1H), 4.86 - 4.65 (m, 2H), 4.44 - 4.33 (m, 1H), 4.29 - 4.22 (m, 1H), 4.19 - 4.05 (m, 2H), 3.76 (dd, *J* = 14.4, 4.4 Hz, 1H), 3.38 (s, 3H), 3.33 - 3.19 (m, 1H), 3.15 - 3.03 (m, 1H), 2.96 - 2.68 (m, 3H), 2.49 (s, 3H), 2.37 (s, 3H), 2.22 - 2.14 (m, 2H), 2.02 - 1.88 (m, 3H) | 637.4 |
| **205** | 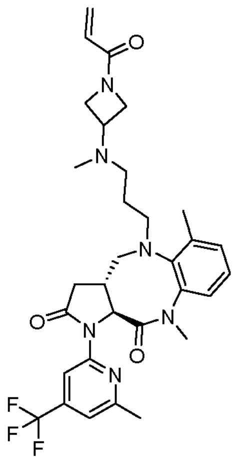 | (3a*R*,11a*S*)-5-(3-((1-acryloylazetidin-3-yl)(methyl)amino)propyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B23** | ($CDCl_3$) 8.34 (s, 1H), 7.28 - 7.25 (m, 3H), 7.08 (s, 1H), 6.36 (dd, *J* = 17.2, 1.6 Hz, 1H), 6.22 -6.10 (m, 1H), 5.75 (d, *J* = 11.2 Hz, 1H), 4.66 (d, *J* = 9.2 Hz, 1H), 4.52 - 4.19 (m, 3H), 4.08 - 3.73 (m, 1H), 3.72 - 3.60 (m, 1H), 3.38 (s, 3H), 3.22 - 3.13 (m, 1H), 3.10 - 3.03 (m, 1H), 2.99 - 2.59 (m, 7H), 2.52 (s, 3H), 2.41 (s, 3H), 2.26 - 2.18 (m, 1H), 1.96 - 1.84 (m, 1H), 1.78 - 1.50 (m, 3H) | 599.2 |

(continued)

| Example Number | Structure | Name | Intermediates | $^1$H NMR (400 MHz) δ ppm | MI |
|---|---|---|---|---|---|
| **206a** | 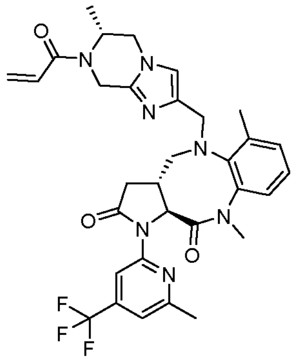 | (3aR,11aS)-5-(((R/S)-7-acryloyl-6-methyl-5,6,7,8-tetrahydroimidazo[1,2-a]pyrazin-2-yl)methyl)-6,10-dimethyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Intermediate 5b and Intermediate B26** | ($CDCl_3$) 8.32 (s, 1H), 7.26 - 7.21 (m, 3H), 7.05 (s, 1H), 6.77 (s, 1H), 6.65 - 6.53 (m, 1H), 6.44 - 6.35 (m, 1H), 5.85 - 5.79 (m, 1H), 5.55 - 4.85 (m, 2H), 4.74 (d, *J* = 9.6 Hz, 1H), 4.60 - 4.50 (m, 1H), 4.22 - 4.01 (m, 3H), 3.86 - 3.82 (m, 1H), 3.68 - 3.55 (m, 1H), 3.37 (s, 3H), 3.08 - 2.93 (m, 1H), 2.86 - 2.76 (m, 1H), 2.69 (dd, *J* = 16.8, 8.8 Hz, 1H), 2.51 (s, 3H), 2.44 (s, 3H), 2.17 (dd, *J* = 16.8, 11.6 Hz, 1H), 1.23 (d, *J* = 6.8 Hz, 3H) | 622.1 |
| **206b** | 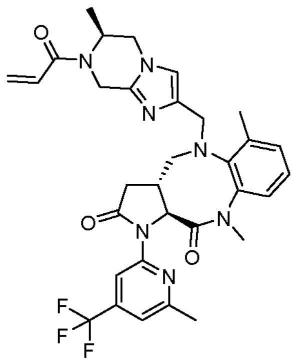 | (3aR,11aS)-5-(((S/R)-7-acryloyl-6-methyl-5,6,7,8-tetrahydroimidazo[1,2-a]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | ($CDCl_3$) 8.32 (s, 1H), 7.26 - 7.22 (m, 3H), 7.05 (s, 1H), 6.78 (s, 1H), 6.58 (dd, *J* = 16.4, 10.4 Hz, 1H), 6.42 - 6.35 (m, 1H), 5.82 (dd, *J* = 10.4, 1.2 Hz, 1H), 5.59 - 4.92 (m, 2H), 4.73 (d, *J* = 9.6 Hz, 1H), 4.66 - 4.41 (m, 1H), 4.20 - 4.07 (m, 3H), 3.87 (d, *J* = 12.4 Hz, 1H), 3.66 (dd, *J* = 14.0, 4.8 Hz, 1H), 3.36 (s, 3H), 3.09 - 2.97 (m, 1H), 2.88 - 2.77 (m, 1H), 2.69 (dd, *J* = 16.8, 8.4 Hz, 1H), 2.50 (s, 3H), 2.44 (s, 3H), 2.17 (dd, *J* = 16.8, 11.6 Hz, 1H), 1.25 (d, *J* = 6.8 Hz, 3H) | 622.3 |

The **Examples** in **Table 17** were prepared using methods similar to those described in the synthesis of **Example 87,** using the listed **Intermediates** in step a, and the appropriate warhead in step d.

**Table 17**

| Example Number | Structure | Name | Intermediates | $^1$H NMR (400 MHz) δ ppm | MI |
|---|---|---|---|---|---|
| **207** | 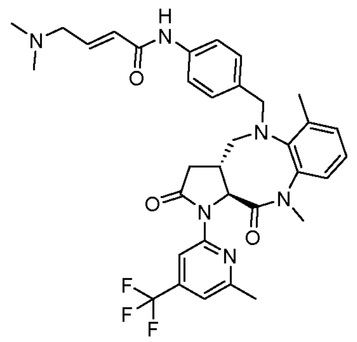 | (*E*)-*N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-4-(dimethylamino)but-2-enamide | **Step a: Intermediate 5b** and 4-nitrobenzaldehyde (CAS: 555-16-8) **Step d:** (*E*)-4-(dimethylamino)but-2-enoic acid hydrochloride | ($CDCl_3$) 8.32 (s, 1H), 7.60 (s, 1H), 7.56 (d, *J* = 8.0 Hz, 2H), 7.30 (d, *J* = 8.4 Hz, 2H), 7.27 - 7.21 (m, 3H), 7.05 (s, 1H), 7.01 - 6.90 (m, 1H), 6.16 (d, *J* = 15.2 Hz, 1H), 4.75 (d, *J* = 9.6 Hz, 1H), 4.12 (d, *J* = 13.6 Hz, 1H), 3.95 (d, *J* = 13.6 Hz, 1H), 3.46 - 3.38 (m, 4H), 3.16 (d, *J* = 5.6 Hz, 2H), 3.06 - 2.90 (m, 1H), 2.74 - 2.66 (m, 1H), 2.65 - 2.58 (m, 1H), 2.50 (s, 3H), 2.46 (s, 3H), 2.32 (s, 6H), 2.17 - 2.08 (m, 1H) | 635.3 |
| **208** | 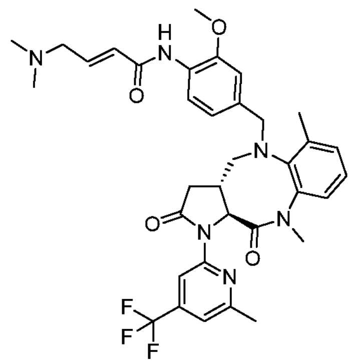 | (*E*)-*N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-methoxyphenyl)-4-(dimethylamino)but-2-enamide | **Step a: Intermediate 5b** and **Intermediate B37 Step d:** (*E*)-4-(dimethylamino)but-2-enoic acid hydrochloride | ($CDCl_3$) 8.39 (d, *J* = 7.6 Hz, 1H), 8.33 (s, 1H), 7.90 (s, 1H), 7.27 - 7.24 (m, 3H), 7.05 (s, 1H), 7.01 - 6.90 (m, 3H), 6.28 (d, *J* = 15.2 Hz, 1H), 4.74 (d, *J* = 9.6 Hz, 1H), 4.14 - 4.02 (m, 2H), 3.92 (s, 3H), 3.45 (dd, *J* = 14.0, 4.4 Hz, 1H), 3.40 (s, 3H), 3.31 (d, *J* = 6.0 Hz, 2H), 2.99 - 2.87 (m, 1H), 2.74 (dd, *J* = 14.0, 11.6 Hz, 1H), 2.63 (dd, *J* = 16.8, 8.4 Hz, 1H), 2.50 (d, *J* = 2.0 Hz, 6H), 2.44 (s, 6H), 2.16 - 2.09 (m, 1H) | 665.3 |

(continued)

| Example Number | Structure | Name | Intermediates | $^1$H NMR (400 MHz) δ ppm | MI |
|---|---|---|---|---|---|
| **209** | 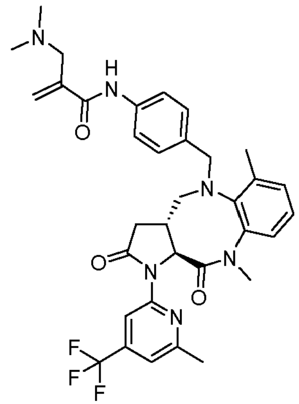 | *N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-2-((dimethylamino)methyl)acryla mide | **Step a: Intermediate 5b** and 4-nitrobenzaldehyde (CAS: 555-16-8) **Step d: Intermediate E1** | ($CDCl_3$) 11.57 (s, 1H), 8.32 (s, 1H), 7.58 (d, *J* = 8.4 Hz, 2H), 7.31 (d, *J* = 8.4 Hz, 2H), 7.27 - 7.22 (m, 3H), 7.05 (s, 1H), 6.35 (d, *J* = 1.8 Hz, 1H), 5.51 (s, 1H), 4.76 (d, *J* = 9.6 Hz, 1H), 4.15 - 4.10 (m, 1H), 4.01 - 3.95 (m, 1H), 3.46 - 3.39 (m, 4H), 3.25 (s, 2H), 3.07 - 2.92 (m, 1H), 2.73 - 2.60 (m, 2H), 2.50 (s, 3H), 2.46 (s, 3H), 2.36 (s, 6H), 2.16 - 2.08 (m, 1H) | 635.2 |
| **210** | 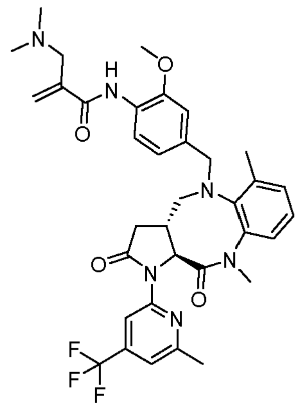 | *N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-methoxyphenyl)-2-((dimethylamino)methyl)acryla mide | **Step a: Intermediate 5b** and **Intermediate B37 Step d: Intermediate E1** | ($CDCl_3$) 11.86 (br s, 1H), 8.50 - 8.40 (m, 1H), 8.32 (s, 1H), 7.27 - 7.22 (m, 3H), 7.05 (s, 1H), 6.96 - 6.89 (m, 2H), 6.33 (s, 1H), 5.48 (br s, 1H), 4.74 (d, *J* = 9.6 Hz, 1H), 4.14 - 4.01 (m, 2H), 3.91 (s, 3H), 3.49 - 3.40 (m, 4H), 3.24 (s, 2H), 3.00 - 2.88 (m, 1H), 2.77 - 2.69 (m, 1H), 2.66 - 2.58 (m, 1H), 2.51 (d, *J* = 2.4 Hz, 6H), 2.35 (s, 6H), 2.12 (dd, J = 16.8, 11.6 Hz, 1H) | 665.3 |

(continued)

| Example Number | Structure | Name | Intermediates | $^1$H NMR (400 MHz) δ ppm | MI |
|---|---|---|---|---|---|
| **211** | 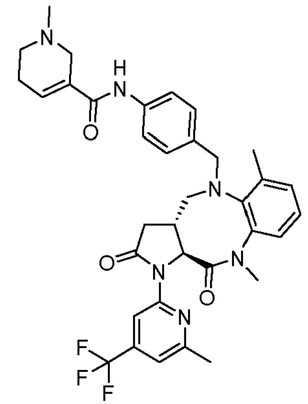 | *N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-1-methyl-1,2,5,6-tetrahydropyridine-3-carboxamide | **Step a: Intermediate 5b** and 4-nitrobenzaldehyde (CAS: 555-16-8) **Step d:** 1-methyl-1,2,5,6-tetrahydropyridine-3-carboxylic acid (CAS: 6018-28-6) | ($CDCl_3$) 8.32 (s, 1H), 7.51 (d, *J* = 8.4 Hz, 2H), 7.45 (s, 1H), 7.32 (d, *J* = 8.4 Hz, 2H), 7.26 - 7.21 (m, 3H), 7.05 (s, 1H), 6.62 (s, 1H), 4.79 - 4.73 (m, 2H), 4.13 (d, *J* = 13.6 Hz, 1H), 3.96 (d, *J* = 13.6 Hz, 1H), 3.46 - 3.39 (m, 4H), 3.27 (s, 2H), 2.74 - 2.60 (m, 2H), 2.59 - 2.53 (m, 2H), 2.50 (s, 3H), 2.46 (s, 6H), 2.45 - 2.40 (m, 2H), 2.17 - 2.06 (m, 1H) | 647.1 |

The **Examples** in **Table 18** were prepared using methods similar to those described in the synthesis of **Example 92,** using the listed **Intermediates** in step a, and the appropriate ketone in step c.

**Table 18**

| Example Number | Structure | Name | Intermediates | $^1$H NMR (400 MHz) δ ppm | MI |
|---|---|---|---|---|---|
| **212** | 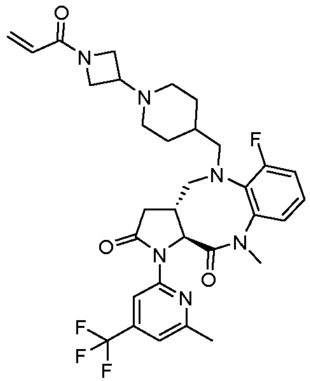 | (3a*R*,11as)-5-((1-(1-acryloylazetidin-3-yl) piperidin-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and *tert*-butyl **4-**formylpiperidine-1-carboxylate (CAS: 137076-22-3)<br><br>**Step c:** tert-butyl 3-oxoazetidine-1-carboxylate (CAS: 177947-96-5) | ($CDCl_3$) 8.30 (s, 1H), 7.35 - 7.30 (m, 1H), 7.21 (d, *J* = 8.0 Hz, 1H), 7.15-7.13 (m, 1H), 7.06 (s, 1H), 6.34 - 6.27 (m, 1H), 6.21 - 6.12 (m, 1H), 5.68 - 5.62 (m, 1H), 4.61 (d, *J* = 9.2 Hz, 1H), 4.23 - 4.16 (m, 1H), 4.11 - 4.01 (m, 2H), 3.95 - 3.86 (m, 1H), 3.49 (dd, *J* = 13.2, 4.4 Hz, 1H), 3.33 (s, 3H), 3.15 - 3.02 (m, 2H), 2.96 - 2.88 (m, 1H), 2.86 - 2.79 (m, 3H), 2.78 - 2.67 (m, 2H), 2.49 (s, 3H), 2.20 (dd, J = 16.8, 11.2 Hz, 1H), 1.91 - 1.75 (m, 4H), 1.37 - 1.26 (m, 1H), 1.23 - 1.05 (m, 2H) | 629.2 |
| **213** | 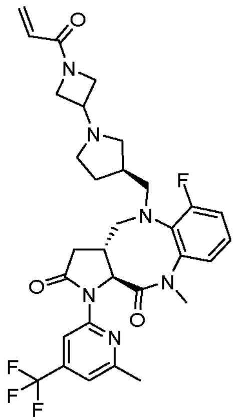 | (3a*R*,11a*S*)-5-(((*S*)-1-(1-acryloylazetidin-3-yl)pyrrolidin-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and **Intermediate B43 Step c:** *tert*-butyl 3-oxoazetidine-1-carboxylate (CAS: 177947-96-5) | ($CDCl_3$) 8.32 (s, 1H), 7.38 - 7.30 (m, 1H), 7.24 - 7.20 (m, 1H), 7.15 - 7.10 (m, 1H), 7.06 (s, 1H), 6.36 - 6.28 (m, 1H), 6.21 - 6.10 (m, 1H), 5.67 (d, *J* = 9.6 Hz, 1H), 4.61 (d, *J* = 8.8 Hz, 1H), 4.33 - 3.89 (m, 4H), 3.56 (d, *J* = 12.4 Hz, 1H), 3.36 (s, 4H), 3.19 - 3.01 (m, 2H), 2.96 - 2.84 (m, 2H), 2.83 - 2.52 (m, 4H), 2.49 (s, 3H), 2.42 - 2.15 (m, 3H), 2.06 - 1.93 (m, 1H), 1.59 - 1.42 (m, 1H) | 615.4 |

(continued)

| Example Number | Structure | Name | Intermediates | $^1$H NMR (400 MHz) δ ppm | MI |
|---|---|---|---|---|---|
| **214** | 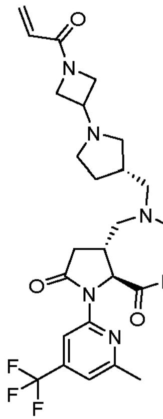 | (3a*R*,11a*S*)-5-(((*R*)-1-(1-acryloylazetidin-3-yl)pyrrolidin-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and **Intermediate B44 Step c:** *tert*-butyl 3-oxoazetidine-1-carboxylate (CAS: 177947-96-5) | ($CDCl_3$) 8.32 (s, 1H), 7.38 - 7.34 (m, 1H), 7.24 - 7.20 (m, 1H), 7.16 - 7.11 (m, 1H), 7.07 (s, 1H), 6.36 - 6.29 (m, 1H), 6.19 - 6.11 (m, 1H), 5.69 - 5.64 (m, 1H), 4.60 (d, *J* = 9.2 Hz, 1H), 4.31 - 3.95 (m, 4H), 3.62 - 3.57 (m, 1H), 3.39 (s, 3H), 3.35 - 3.11 (m, 2H), 3.05 - 2.60 (m, 6H), 2.50 (s, 3H), 2.43 - 2.15 (m, 3H), 2.10 - 1.93 (m, 1H), 1.62 - 1.50 (m, 2H) | 615.3 |
| **215** | 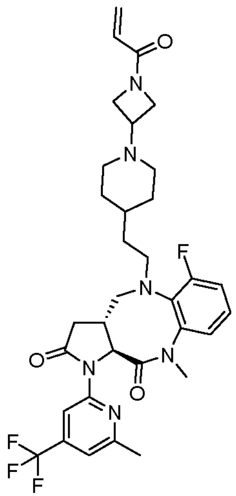 | (3a*R*,11a*S*)-5-(2-(1-(1-acryloylazetidin-3-yl)piperidin-4-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and *tert*-butyl 4-(2-oxoethyl)piperidine-1-carboxylate (CAS: 142374-19-4)<br>**Step c:** *tert*-butyl 3-oxoazetidine-1-carboxylate (CAS: 177947-96-5) | ($CDCl_3$) 8.32 (s, 1H), 7.39 - 7.30 (m, 1H), 7.22 (d, *J* = 8.0 Hz, 1H), 7.14 (t, *J* = 9.2 Hz, 1H), 7.06 (s, 1H), 6.36 - 6.27 (m, 1H), 6.22 - 6.09 (m, 1H), 5.67 (d, *J* = 10.0 Hz, 1H), 4.60 (d, *J* = 8.8 Hz, 1H), 4.28 - 3.96 (m, 4H), 3.58 - 3.45 (m, 1H), 3.34 (s, 3H), 3.32 - 3.22 (m, 1H), 3.18 - 3.05 (m, 2H), 3.01 - 2.79 (m, 4H), 2.72 (m, 1H), 2.50 (s, 3H), 2.24 - 2.17 (m, 1H), 2.11 - 1.91 (m, 2H), 1.70 - 1.66 (m, 2H), 1.51 - 1.42 (m, 1H), 1.40 - 1.25 (m, 4H) | 643.0 |

(continued)

| Example Number | Structure | Name | Intermediates | $^1H$ NMR (400 MHz) δ ppm | MI |
|---|---|---|---|---|---|
| **216** | 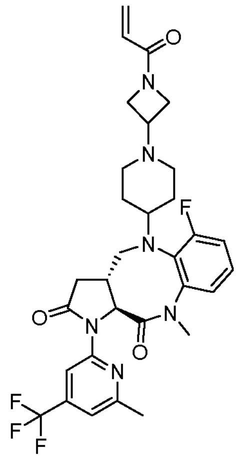 | (3a*R*,11a*S*)-5-(1-(1-acryloylazetidin-3-yl)piperidin-4-yl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5a** and *tert-butyl 4*-oxopiperidine-1-carboxylate (CAS: 79099-07-3) **Step c:** *tert-butyl 3*-oxoazetidine-1-carboxylate (CAS: 177947-96-5) | ($CDCl_3$) 8.31 (s, 1H), 7.42 - 7.34 (m, 1H), 7.28 - 7.23 (m, 1H), 7.15 (t, *J = 8.8* Hz, 1H), 7.08 (s, 1H), 6.43 - 6.29 (m, 1H), 6.23 - 6.09 (m, 1H), 5.68 (d, *J* = 11.2 Hz, 1H), 4.60 (d, *J* = 8.4 Hz, 1H), 4.30 - 4.20 (m, 1H), 4.16 - 4.08 (m, 2H), 4.02 - 3.85 (m, 1H), 3.77 - 3.66 (m, 1H), 3.37 (s, 3H), 3.32 - 3.08 (m, 2H), 2.89 - 2.62 (m, 5H), 2.52 (s, 3H), 2.31 - 2.15 (m, 1H), 2.10 - 1.86 (m, 3H), 1.58 - 1.45 (m, 3H) | 615.3 |
| **217** | 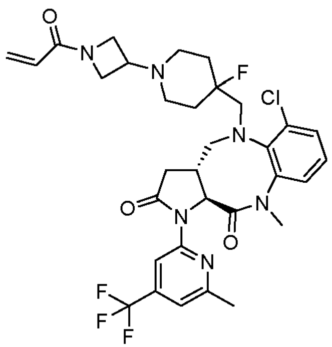 | (3a*R*,11a*S*)-5-((1-(1-acryloylazetidin-3-yl)-4-fluoropiperidin-4-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B38 Step c:** *tert-butyl 3*-oxoazetidine-1-carboxylate (CAS: 177947-96-5) | ($CDCl_3$) 8.31 (s, 1H), 7.46 (dd, J = 7.2, 2.4 Hz, 1H), 7.37 - 7.25 (m, 2H), 7.05 (s, 1H), 6.37 - 6.26 (m, 1H), 6.23 - 6.11 (m, 1H), 5.66 (d, *J* = 10.4 Hz, 1H), 4.59 (d, *J = 9.2* Hz, 1H), 4.22 (t, *J* = 7.6 Hz, 1H), 4.15 - 4.00 (m, 2H), 3.90 (dd, J = 10.4, 5.2 Hz, 1H), 3.59 (dd, J = 9.2, 5.6 Hz, 1H), 3.35 (s, 3H), 3.29 - 3.17 (m, 2H), 3.16 - 3.07 (m, 1H), 3.07 - 2.94 (m, 2H), 2.72 (dd, *J* = 16.8, 8.0 Hz, 1H), 2.63 (d, *J* = 6.8 Hz, 2H), 2.48 (s, 3H), 2.26 - 2.12 (m, 3H), 2.10 - 1.95 (m, 2H), 1.69 - 1.38 (m, 2H) | 663.1 |

(continued)

| Example Number | Structure | Name | Intermediates | $^1$H NMR (400 MHz) δ ppm | MI |
|---|---|---|---|---|---|
| **218** | 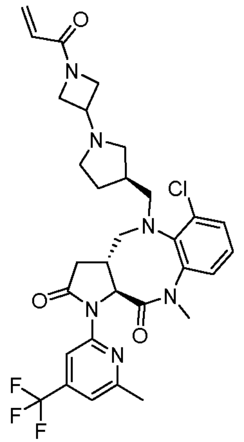 | (3*aR*,11*aS*)-5-(((*S*)-1-(1-acryloylazetidin-3-yl)pyrrolidin-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B43 Step c:** *tert-butyl* 3-oxoazetidine-1-carboxylate (CAS: 177947-96-5) | ($CDCl_3$) 8.34 (s, 1H), 7.46 - 7.43 (m, 1H), 7.33 - 7.28 (m, 2H), 7.06 (s, 1H), 6.36 - 6.27 (m, 1H), 6.22 - 6.11 (m, 1H), 5.65 (d, *J* = 10.2 Hz, 1H), 4.63 (d, *J* = 9.0 Hz, 1H), 4.30 - 4.21 (m, 1H), 4.19 - 4.05 (m, 2H), 4.00 - 3.91 (m, 1H), 3.60 - 3.53 (m, 1H), 3.39 (s, 3H), 3.38 - 3.23 (m, 1H), 3.20 - 3.04 (m, 1H), 3.03 - 2.99 (m, 1H), 2.97 - 2.90 (m, 2H), 2.77 - 2.70 (m, 2H), 2.68 - 2.51 (m, 2H), 2.48 (s, 3H), 2.40 - 2.15 (m, 3H), 2.06 - 1.94 (m, 1H), 1.61 - 1.45 (m, 1H) | 631.2 |
| **219** | 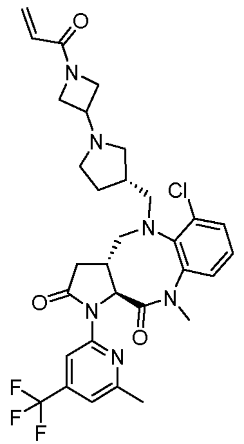 | (3*aR*,11*aS*)-5-(((*R*)-1-(1-acryloylazetidin-3-yl)pyrrolidin-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B44 Step c:** *tert-butyl* 3-oxoazetidine-1-carboxylate (CAS: 177947-96-5) | ($CDCl_3$) 8.34 (s, 1H), 7.46 - 7.43 (m, 1H), 7.33 - 7.27 (m, 2H), 7.05 (s, 1H), 6.37 - 6.27 (m, 1H), 6.23 - 6.12 (m, 1H), 5.65 (d, *J* = 10.4 Hz, 1H), 4.66 - 4.59 (m, 1H), 4.27 - 4.18 (m, 1H), 4.12 - 4.05 (m, 2H), 3.97 - 3.84 (m, 1H), 3.62 - 3.52 (m, 1H), 3.38 (d, *J* = 3.2 Hz, 3H), 3.32 - 3.25 (m, 1H), 3.16 - 2.86 (m, 4H), 2.73 (dd, J = 16.8, 8.0 Hz, 1H), 2.64 - 2.42 (m, 6H), 2.40 - 2.14 (m, 3H), 2.04 - 1.89 (m, 1H), 1.54 - 1.40 (m, 1H) | 631.2 |

(continued)

| Example Number | Structure | Name | Intermediates | $^1$H NMR (400 MHz) δ ppm | MI |
|---|---|---|---|---|---|
| **220** | 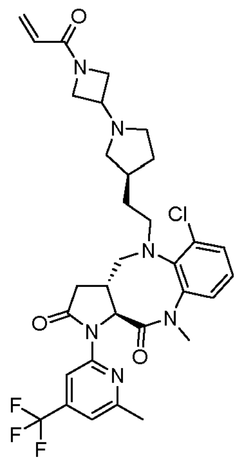 | (3a*R*,11a*S*)-5-(2-((*S*)-1-(1-acryloylazetidin-3-yl)pyrrolidin-3-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B42 Step c:** *tert-butyl* 3-oxoazetidine-1-carboxylate (CAS: 177947-96-5) | ($CDCl_3$) 8.34 (s, 1H), 7.45 (dd, J = 7.2, 2.4 Hz, 1H), 7.33 - 7.28 (m, 2H), 7.05 (s, 1H), 6.38 - 6.27 (m, 1H), 6.24 - 6.12 (m, 1H), 5.68 - 5.64 (m, 1H), 4.62 (d, *J* = 9.2 Hz, 1H), 4.28 - 4.17 (m, 1H), 4.13 - 4.08 (m, 2H), 3.99 - 3.94 (m, 1H), 3.59 - 3.50 (m, 1H), 3.37 (s, 4H), 3.10 - 2.87 (m, 4H), 2.76 - 2.70 (m, 2H), 2.66 - 2.57 (m, 1H), 2.48 (s, 4H), 2.33 - 2.18 (m, 2H), 2.16 - 1.93 (m, 2H), 1.51 - 1.32 (m, 3H) | 645.3 |
| **221** | 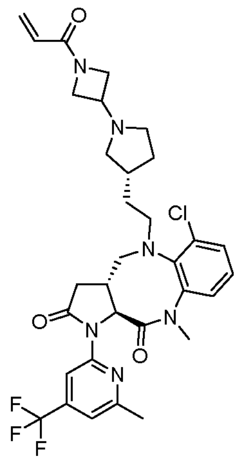 | (3a*R*,11a*S*)-5-(2-((*R*)-1-(1-acryloylazetidin-3-yl)pyrrolidin-3-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B41 Step c:** *tert-butyl* 3-oxoazetidine-1-carboxylate (CAS: 177947-96-5) | ($CDCl_3$) 8.33 (s, 1H), 7.49 - 7.43 (m, 1H), 7.36 - 7.29 (m, 2H), 7.06 (s, 1H), 6.38 - 6.30 (m, 1H), 6.20 - 6.08 (m, 1H), 5.72 (d, *J* = 10.4 Hz, 1H), 4.61 (d, *J* = 9.2 Hz, 1H), 4.55 - 4.33 (m, 2H), 4.32 - 4.12 (m, 1H), 3.95 - 3.76 (m, 1H), 3.59 - 3.49 (m, 1H), 3.38 (s, 3H), 3.28 - 2.82 (m, 7H), 2.79 - 2.68 (m, 1H), 2.58 - 2.50 (m, 1H), 2.48 (s, 3H), 2.29 - 2.16 (m, 2H), 1.97 - 1.39 (m, 5H) | 645.2 |

(continued)

| Example Number | Structure | Name | Intermediates | $^1$H NMR (400 MHz) δ ppm | MI |
|---|---|---|---|---|---|
| **222** | 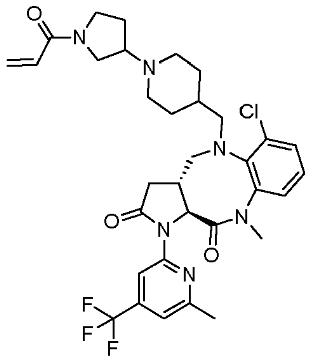 | (3a*R*,11a*S*)-5-((1-(1-acryloylpyrrolidin-3-yl)piperidin-4-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and *tert*-butyl 4-formylpiperidine-1-carboxylate (CAS: 137076-22-3) **Step c:** *tert*-butyl 3-oxopyrrolidine-1-carboxylate (CAS: 101385-93-7) | ($CDCl_3$) 8.32 (s, 1H), 7.45 (dd, J = 7.2, 2.4 Hz, 1H), 7.35 - 7.28 (m, 2H), 7.06 (s, 1H), 6.46 - 6.32 (m, 2H), 5.74 - 5.62 (m, 1H), 4.62 (d, *J* = 9.2 Hz, 1H), 4.08 - 3.69 (m, 2H), 3.60 - 3.25 (m, 6H), 3.10 - 2.67 (m, 7H), 2.48 (s, 3H), 2.32 - 1.61 (m, 7H), 1.57 - 0.98 (m, 4H) | 659.2 |
| **223** | 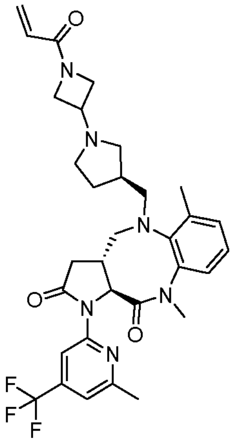 | (3a*R*,11a*S*)-5-(((*S*)-1-(1-acryloylazetidin-3-yl)pyrrolidin-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 6c** and **Intermediate B43 Step c:** *tert*-butyl 3-oxoazetidine-1-carboxylate (CAS: 177947-96-5) | ($CDCl_3$) 8.33 (s, 1H), 7.25 - 7.17 (m, 3H), 7.04 (s, 1H), 6.38 - 6.27 (m, 1H), 6.23 - 6.11 (m, 1H), 5.66 (dd, J = 10.4, 2.0 Hz, 1H), 4.70 (d, *J* = 9.2 Hz, 1H), 4.26 - 4.18 (m, 1H), 4.14 - 3.96 (m, 2H), 3.94 - 3.85 (m, 1H), 3.63 - 3.49 (m, 1H), 3.37 (s, 3H), 3.28 - 3.24 (m, 1H), 3.03 - 2.93 (m, 2H), 2.93 - 2.87 (m, 1H), 2.86 - 2.77 (m, 1H), 2.75 - 2.71 (m, 1H), 2.65 - 2.52 (m, 1H), 2.50 (s, 5H), 2.37 (s, 3H), 2.30 - 2.12 (m, 3H), 2.01 - 1.86 (m, 1H), 1.51 - 1.92 (m, 1H) | 611.3 |

(continued)

| Example Number | Structure | Name | Intermediates | $^1$H NMR (400 MHz) δ ppm | MI |
|---|---|---|---|---|---|
| **224** | 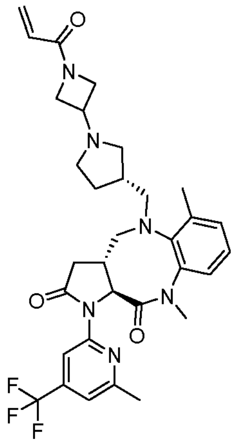 | (3a*R*,11a*S*)-5-(((*R*)-1-(1-acryloylazetidin-3-yl)pyrrolidin-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 6c** and **Intermediate B44 Step c:** *tert*-butyl 3-oxoazetidine-1-carboxylate (CAS: 177947-96-5) | ($CDCl_3$) 8.33 (s, 1H), 7.25 - 7.16 (m, 3H), 7.04 (s, 1H), 6.36 - 6.27 (m, 1H), 6.21 - 6.11 (m, 1H), 5.69 - 5.61 (m, 1H), 4.74 - 4.65 (m, 1H), 4.27 - 4.17 (m, 1H), 4.14 - 3.98 (m, 2H), 3.96 - 3.83 (m, 1H), 3.64 - 3.51 (m, 1H), 3.37 (s, 3H), 3.34 - 3.21 (m, 1H), 3.05 - 2.77 (m, 4H), 2.77 - 2.67 (m, 1H), 2.66 - 2.49 (m, 3H), 2.48 (s, 3H), 2.37 (s, 3H), 2.31 - 2.13 (m, 3H), 2.04 - 1.86 (m, 1H), 1.55 - 1.40 (m, 1H) | 611.2 |

The **Examples** in **Table 19** were prepared using methods similar to those described in the synthesis of **Example 92,** using the listed **Intermediates** in step a, and the appropriate ketone in step c. An additional chiral SFC step was conducted after step c.

**Table 19**

| Example Number | Structure | Name | Intermediates | $^1$H NMR (400 MHz) δ ppm | MI |
|---|---|---|---|---|---|
| **225a** | 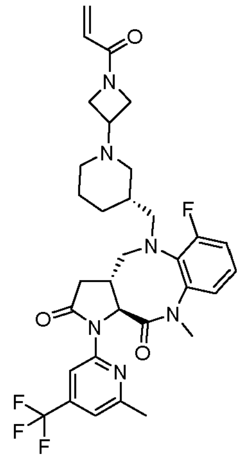 | (3aR,11aS)-5-(((R/S)-1-(1-acryloylazetidin-3-yl)piperidin-3-yl)methyl)-6-fluoro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione | **Step a: Intermediate 5a** and tert-butyl 3-formylpiperidine-1-carboxylate (CAS: 118156-93-7) Step c: tert-butyl 3-oxoazetidine-1-carboxylate (CAS: 177947-96-5) | ($CDCl_3$) 8.34 (s, 1H), 7.35 - 7.31 (m, 1H), 7.22 (d, *J* = 8.4 Hz, 1H), 7.18 - 7.11 (m, 1H), 7.06 (s, 1H), 6.40 - 6.18 (m, 2H), 5.68 - 5.65 (m, 1H), 4.62 - 4.59 (m, 1H), 4.37 - 4.13 (m, 2H), 4.12 - 3.86 (m, 2H), 3.53 - 3.50 (m, 1H), 3.32 (s, 3H), 3.31 - 3.25 (m, 1H), 3.23 - 3.10 (m, 1H), 3.10 - 2.78 (m, 4H), 2.76 - 2.69 (m, 1H), 2.63 - 2.56 (m, 1H), 2.52 - 2.46 (m, 3H), 2.23 - 2.15 (m, 1H) 1.80 - 1.65 (m, 4H), 1.59 - 1.42 (m, 2H), 0.95 - 0.80 (m, 1H) | 629.3 |
| **225b** | 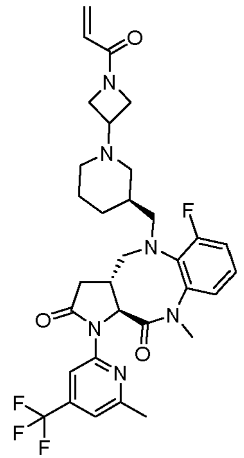 | (3a*R*,11a*S*)-5-(((*S*/*R*)-1-(1-acryloylazetidin-3-yl)piperidin-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | ($CDCl_3$) 8.32 (s, 1H), 7.36 - 7.29 (m, 1H), 7.24 - 7.19 (m, 1H), 7.17 - 7.10 (m, 1H), 7.06 (s, 1H), 6.37 - 6.29 (m, 1H), 6.27 - 6.15 (m, 1H), 5.68 - 5.64 (m, 1H), 4.64 - 4.59 (m, 1H), 4.25 - 4.18 (m, 1H), 4.16 - 4.00 (m, 2H), 4.00 - 3.84 (m, 1H), 3.58 - 3.47 (m, 1H), 3.36 (d, *J* = 3.6 Hz, 3H), 3.20 - 3.07 (m, 1H), 3.06 - 2.81 (m, 4H), 2.80 - 2.60 (m, 3H), 2.50 (s, 3H), 2.28 - 2.13 (m, 1H), 2.02 - 1.65 (m, 4H), 1.58 - 1.45 (m, 2H), 1.03 - 0.81 (m, 1H) | 629.3 |

(continued)

| Example Number | Structure | Name | Intermediates | $^1$H NMR (400 MHz) δ ppm | MI |
|---|---|---|---|---|---|
| **226a** | 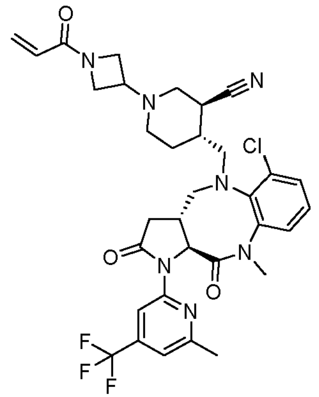 | *(3S/R,4R/S)-1-(1*-acryloylazetidin-3-yl)-4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)piperidine-3-carbonitrile | **Step a: Intermediate 5b** and **Intermediate B48b** **Step c:** tert-butyl 3-oxoazetidine-1-carboxylate (CAS: 177947-96-5) | ($CDCl_3$) 8.32 (s, 1H), 7.52 - 7.42 (m, 1H), 7.38 - 7.28 (m, 2H), 7.06 (s, 1H), 6.38 - 6.28 (m, 1H), 6.23 - 6.11 (m, 1H), 5.68 (d, *J* = 10.8 Hz, 1H), 4.63 (d, *J* = 9.2 Hz, 1H), 4.27 - 4.17 (m, 1H), 4.14 - 3.98 (m, 2H), 3.84 - 3.84 (m, 1H), 3.61 - 3.44 (m, 2H), 3.43 (s, 3H), 3.28 - 3.18 (m, 1H), 3.14 - 2.99 (m, 2H), 2.98 - 2.91 (m, 2H), 2.83 - 2.70 (m, 2H), 2.67 - 2.57 (m, 1H), 2.48 (s, 3H), 2.28 - 2.18 (m, 1H), 2.17 - 2.08 (m, 1H), 2.03 - 1.90 (m, 2H), 1.61 - 1.42 (m, 2H) | 670.2 |
| **226b** | 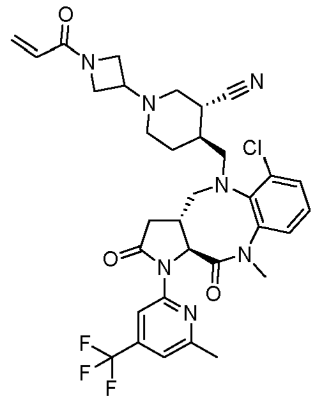 | *(3R/S,4S/R)-1-(1*-acryloylazetidin-3-yl)-4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)piperidine-3-carbonitrile | | ($CDCl_3$) 8.33 (s, 1H), 7.51 - 7.43 (m, 1H), 7.38 - 7.29 (m, 2H), 7.06 (s, 1H), 6.39 - 6.29 (m, 1H), 6.23 - 6.11 (m, 1H), 5.68 (d, *J* = 10.0 Hz, 1H), 4.68 (d, *J* = 9.2 Hz, 1H), 4.28 - 4.18 (m, 1H), 4.15 - 4.05 (m, 1H), 4.05 - 3.98 (m, 1H), 3.93 - 3.84 (m, 1H), 3.64 - 3.48 (m, 2H), 3.44 (d, *J* = 2.8 Hz, 3H), 3.30 - 3.17 (m, 1H), 3.14 - 3.04 (m, 1H), 3.03 - 2.95 (m, 1H), 2.94 - 2.85 (m, 1H), 2.84 - 2.70 (m, 3H), 2.59 - 2.50 (m, 1H), 2.48 (s, 3H), 2.29 - 2.18 (m, 2H), 2.17 - 2.07 (m, 1H), 2.03 - 1.87 (m, 1H), 1.34 - 1.12 (m, 2H) | 670.2 |
| **227a** | | *(3S/R,4S/R)-1-(1*-acryloylazetidin-3-yl)-4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)piperidine-3-carbonitrile | **Step a: Intermediate 5b** and **Intermediate B48a** **Step c:** *tert*-butyl 3-oxoazetidine-1-carboxylate (CAS: 177947-96-5) | ($CDCl_3$) 8.33 (s, 1H), 7.47 (dd, *J* = 7.2, 2.0 Hz, 1H), 7.36 - 7.28 (m, 2H), 7.06 (s, 1H), 6.40 - 6.30 (m, 1H), 6.25 - 6.12 (m, 1H), 5.68 (d, *J* = 10.4 Hz, 1H), 4.64 (dd, *J* = 8.8, 3.6 Hz, 1H), 4.30 - 4.18 (m, 1H), 4.15 - 4.03 (m, 2H), 3.99 - 3.90 (m, 1H), 3.60 - 3.49 (m, 1H), 3.41 (s, 3H), 3.36 - 3.17 (m, 3H), 3.15 - 2.85 (m, 5H), 2.75 (dd, *J* = 16.8, 7.6 Hz, 1H), 2.49 (s, 3H), 2.28 - 2.23 (m, 1H), 2.15 - 2.06 (m, 1H), 1.98 - 1.87 (m, 1H), 1.67 - 1.62 (m, 2H), 1.57 - 1.45 (m, 1H) | 670.2 |

(continued)

| Example Number | Structure | Name | Intermediates | $^1$H NMR (400 MHz) δ ppm | MI |
|---|---|---|---|---|---|
| **227b** | 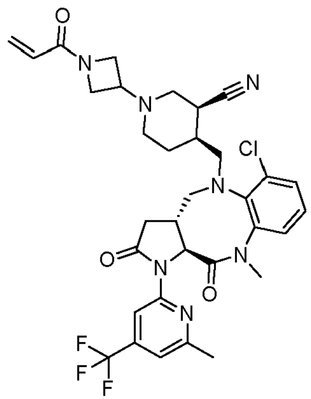 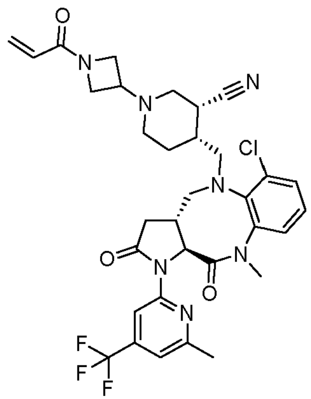 | (3*R*/*S*,4*R*/*S*)-1-(1-acryloylazetidin-3-yl)-4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)piperidine-3-carbonitrile | | ($CDCl_3$) 8.33 (s, 1H), 7.46 (dd, *J* = 6.8, 2.4 Hz, 1H), 7.37 - 7.29 (m, 2H), 7.06 (s, 1H), 6.40 - 6.27 (m, 1H), 6.23 - 6.10 (m, 1H), 5.69 - 5.65 (m, 1H), 4.61 (d, *J* = 9.2 Hz, 1H), 4.27 - 4.18 (m, 1H), 4.15 - 4.04 (m, 2H), 3.98 - 3.92 (m, 1H), 3.58 - 3.51 (m, 1H), 3.43 (s, 3H), 3.38 - 3.23 (m, 3H), 3.16 - 3.03 (m, 2H), 3.03 - 2.84 (m, 3H), 2.80 - 2.71 (m, 1H), 2.49 (s, 3H), 2.27 - 2.22 (m, 1H), 2.04 (d, *J* = 11.6 Hz, 1H), 1.95 - 1.85 (m, 1H), 1.69 - 1.55 (m, 3H) | 670.3 |

**Example 228**

**(3a*R*,11a*S*)-5-((6-acryloyl-6,7-dihydro-5*H*-pyrrolo[3,4-*b*]pyrazin-2-yl)methyl)-6,10-dimethyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione**

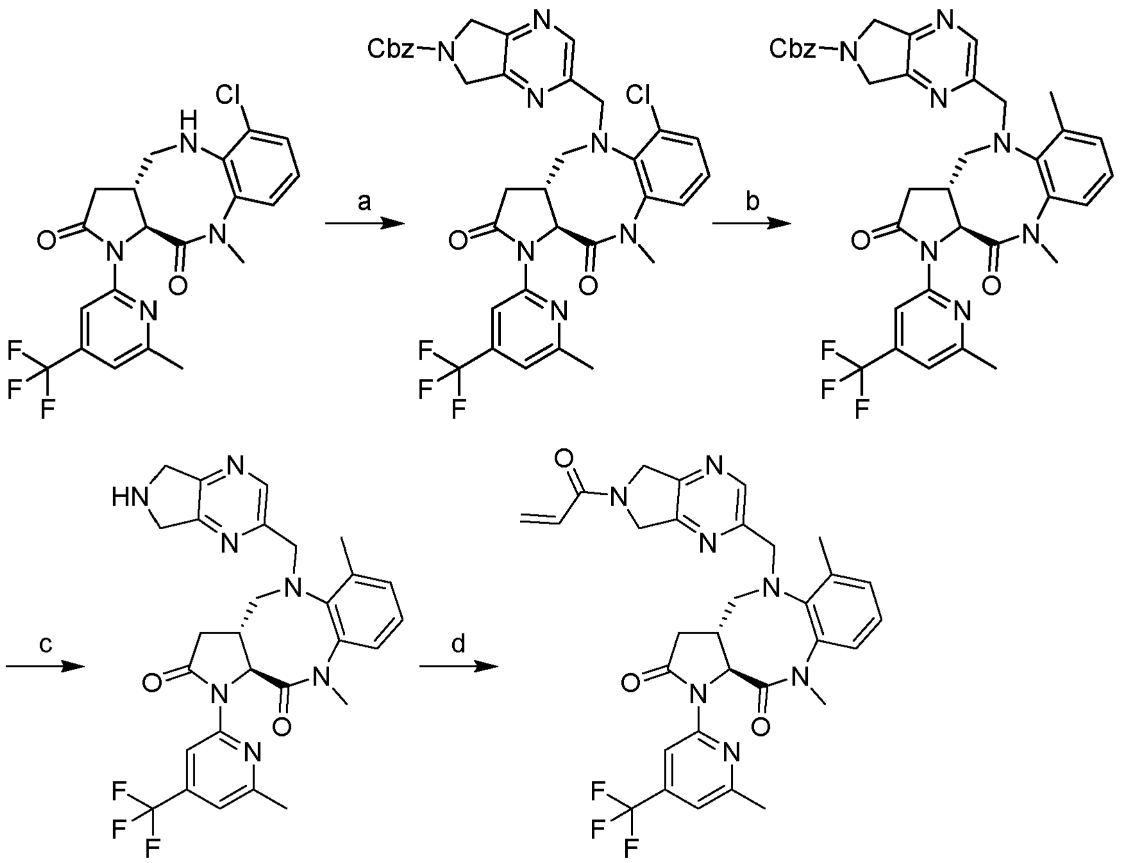


**Step** a. To a solution of **Intermediate 5b** (7.4 g, 16.7 mmol) and **Intermediate B49** (6.21 g, 21.9 mmol) in DMF (100 mL) was added TMSCl (4.58 g, 42.2 mmol). The mixture was stirred at 0 °C for 30 min. A solution of borane tetrahydrofuran complex (1 M in THF, 18.5 mL) in THF (186 mL) was added and the reaction mixture was stirred at 20 °C for 11 h 30 min. Upon completion, the reaction mixture was quenched with sat. aq. $NaHCO_3$ (50 mL) and $H_2O$ (300 mL). The aqueous mixture was extracted with EtOAc (3 x 300 mL). The combined organic layers were washed with brine (500 mL), dried over $Na_2SO_4$ and evaporated. The residue was purified by prep-TLC (PE/EtOAc = 1/3) followed by reverse phase flash chromatography (water (0.1% $NH_4OH$)/MeCN) to give benzyl 2-(((3*aR*,11*aS*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl) methyl)-5,7-dihydro-6H-pyrrolo[3,4-b]pyrazine-6-carboxylate (3.7 g, 31% yield) as a yellow solid.

m/z ES+ $[M+H]^+$ 706.2; $^1H$ NMR (400 MHz, $CDCl_3$) δ ppm 8.68 (d, *J* = 17.2 Hz, 1H), 8.32 (s, 1H), 7.51 - 7.45 (m, 1H), 7.44 - 7.37 (m, 4H), 7.37 - 7.30 (m, 3H), 7.06 (s, 1H), 5.24 (d, *J* = 1.6 Hz, 2H), 4.82 - 4.73 (m, 4H), 4.69 - 4.66 (m, 1H), 4.43 - 4.28 (m, 2H), 3.60 - 3.56 (m, 1H), 3.40 - 3.29 (m, 3H), 3.15 - 3.05 (m, 1H), 3.03 - 2.89 (m, 1H), 2.73 - 2.66 (m, 1H), 2.48 (s, 3H), 2.25 - 2.17 (m, 1H).

**Step b.** A mixture of benzyl 2-(((3a*R*,11a*S*)-6-chloro-10-methyl-1-(6-methyl-4-(trifluoromethyl)pyridin-2-yl)-2, 11-dioxo-1 ,2,3,3a,4, 1 0, 11,11 a-octahydro-5H-benzo[b]pyrrolo[2,3-*f*][1,4]diazocin-5-yl)methyl)-5,7-dihydro-6H-pyrrolo [3,4-*b*]pyrazine-6-carboxylate (2.66 g, 3.77 mmol), methylboronic acid (4.51 g, 75.3 mmol), $Cy_3P$-Pd-G3 (244 mg, 0.38 mmol) and $Cs_2CO_3$ (3.68 g, 11.3 mmol) in toluene (100 mL) was degassed and purged with $N_2$ 3 times. The mixture was stirred at 100 °C for 16 h under a $N_2$ atmosphere. Upon completion, the reaction mixture was diluted with $H_2O$ (150 mL) and extracted with EtOAc (3 x 150 mL). The combined organic layers were washed with brine (400 mL), dried over $Na_2SO_4$ and evaporated. The residue was purified by column chromatography (PE/EtOAc = 1/3) followed by reverse phase flash chromatography (water (0.1% $NH_4OH$)/MeCN) to give benzyl 2-(((3*aR*,11*aS*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,7-dihydro-6*H*-pyrrolo[3,4-*b*]pyrazine-6-carboxylate (2 g, 75% yield) as a yellow solid.

m/z ES+ $[M+H]^+$ 686.3; $^1H$ NMR (400 MHz, $CDCl_3$) δ ppm 8.49 (s, 1H), 8.33 (s, 1H), 7.45 - 7.32 (m, 5H), 7.25 (s, 3H), 7.05 (s, 1H), 5.25 (s, 2H), 4.84 - 4.73 (m, 5H), 4.34 - 4.33 (m, 1H), 4.22 - 4.15 (m, 1H), 3.55 - 3.54 (m, 1H), 3.40 (d, *J* = 16.4 Hz, 3H), 3.15 - 3.01 (m, 1H), 2.91 - 2.81 (m, 1H), 2.76 - 2.67 (m, 1H), 2.50 (s, 3H), 2.41 (s, 3H), 2.21 - 2.14 (m, 1H).

**Step c.** A mixture of benzyl 2-(((3a*R*,11a*S*)-6,10-dimethyl-1-(6-methyl-4-(trifluoromethyl)pyridin-2-yl)-2, 11-dioxo-1 ,2,3,3a,4, 1 0, 11,11 a-octahydro-5H-benzo[*b*]pyrrolo[2,3-*f*][1,4]diazocin-5-yl)methyl)-5,7-dihydro-6*H*-pyrrolo[3,4-*b*]pyrazine-6-carboxylate (2.0 g, 2.92 mmol) and 10% Pd/C (50% w/w $H_2O$; 400 mg) in 2-propanol (40 mL) was degassed and purged with $H_2$ (15 psi) 3 times. The mixture was stirred at 45 °C under a $H_2$ atmosphere (15 psi) for 3 h. Upon completion, the reaction mixture was filtered and evaporated to give (3*aR*,11*aS*)-5-((6,7-dihydro-5*H*-pyrrolo[3,4-*b*]pyrazin-2-yl)methyl)-6,10-dimethyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione (1.61 g, crude) as a yellow solid.

m/z ES+ $[M+H]^+$ 552.2; $^1H$ NMR (400 MHz, $CDCl_3$) δ ppm 8.41 (s, 1H), 8.33 (s, 1H), 7.26 - 7.22 (m, 3H), 7.05 (s, 1H), 4.76 (d, *J* = 9.6 Hz, 1H), 4.40 - 4.29 (m, 5H), 4.16 (d, *J* = 14.8 Hz, 1H), 4.07 - 3.99 (m, 1H), 3.59 (dd, *J* = 14.0, 4.4 Hz, 1H), 3.40 (s, 3H), 3.16 - 3.04 (m, 1H), 2.84 - 2.74 (m, 1H), 2.72 - 2.68 (m, 1H), 2.50 (s, 3H), 2.42 (s, 3H), 2.22 - 2.18 (m, 1H).

**Step d.** Acryloyl chloride (479 mg, 5.30 mmol) was added dropwise to a solution of (3a*R*,11a*S*)-5-((6,7-dihydro-5*H*-pyrrolo[3,4-*b*]pyrazin-2-yl)methyl)-6,10-dimethyl-1-(6-methyl-4-(trifluoromethyl)pyridin-2-yl)-1 ,3a,4,5, 1 0, 11a-hexahydro-2*H*-benzo[*b*]pyrrolo[2,3-*f*][1,4]diazocine-2,11(3*H*)-dione (1.95 g, 3.54 mmol) and DIPEA (913 mg, 7.07 mmol) in DCM (30 mL) at 0 °C. The reaction mixture was stirred at 0 °C for 10 min. Upon completion, the reaction mixture was evaporated and purified by column chromatography (EtOAc) followed by reverse phase flash chromatography (water (0.1% $NH_4OH$)/MeCN) to give the title compound (1.81 g, 85% yield) as a white solid.

m/z ES+ $[M+H]^+$ 606.2; $^1H$ NMR (400 MHz, $CDCl_3$) δ ppm 8.53 (d, *J* = 3.2 Hz, 1H), 8.33 (s, 1H), 7.27 - 7.21 (m, 3H), 7.05 (s, 1H), 6.61 - 6.49 (m, 2H), 5.88 - 5.82 (m, 1H), 4.97 (s, 2H), 4.91 (d, *J* = 8.4 Hz, 2H), 4.77 - 4.74 (m, 1H), 4.39 - 4.34 (m, 1H), 4.25 - 4.17 (m, 1H), 3.61 - 2.57 (m, 1H), 3.46 - 3.33 (m, 3H), 3.19 - 3.01 (m, 1H), 2.93 - 2.83 (m, 1H), 2.77 - 2.68 (m, 1H), 2.50 (s, 3H), 2.42 (d, *J* = 6.0 Hz, 3H), 2.22 - 2.15 (m, 1H).

**Example 229**

**(3a*R*,11a*S*)-5-((6-acryloyl-6,7-dihydro-5*H*-pyrrolo[3,4-*b*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

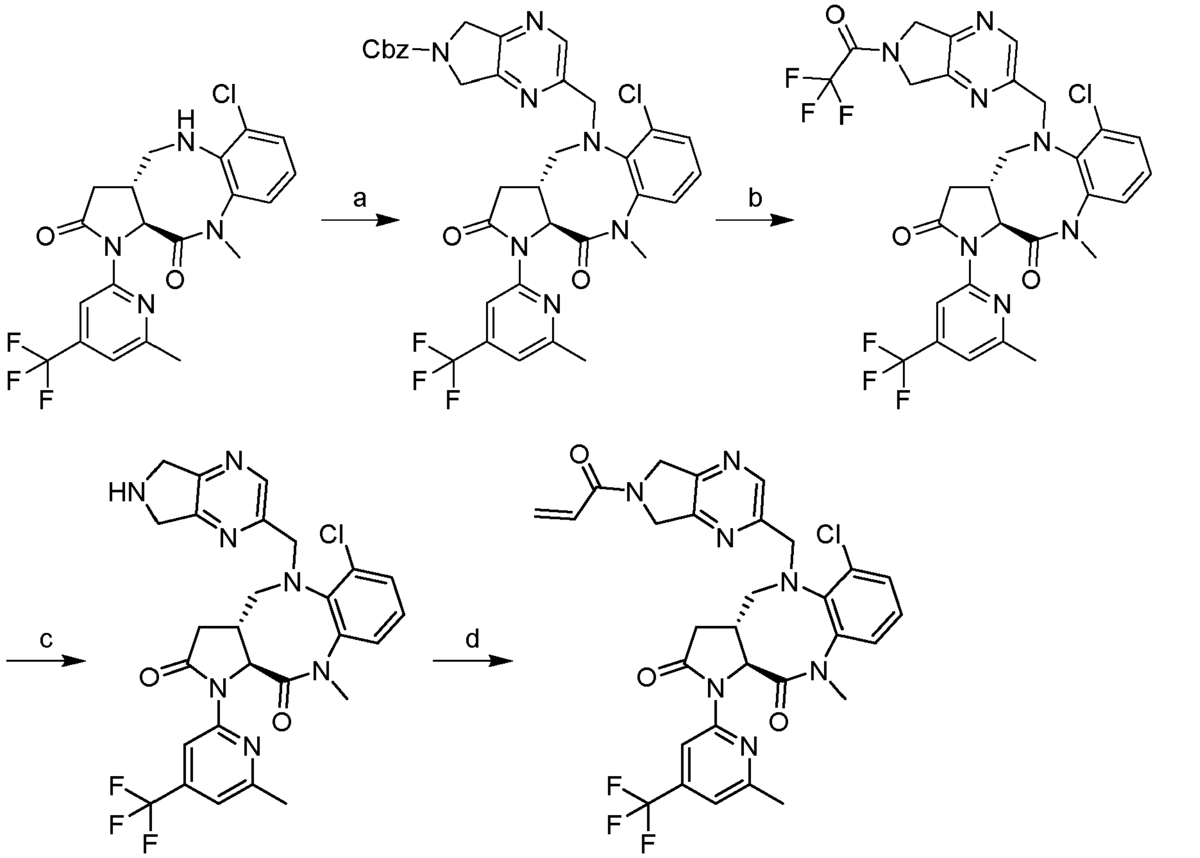


**Step a.** This step was conducted as described in Example 228, step a.

Step b. A solution of benzyl 2-(((3aR,11aS)-6-chloro-10-methyl-1-(6-methyl-4-(trifluoromethyl)pyridin-2-yl)-2, 11-dioxo-1 ,2,3,3a,4, 1 0, 11,11 a-octahydro-5H-benzo[b]pyrrolo[2,3-f][1,4]diazocin-5-yl)methyl)-5,7-dihydro-6H-pyrrolo [3,4-b]pyrazine-6-carboxylate (150 mg, 0.21 mmol) in TFA (2 mL) was stirred at 60 °C for 3 h. Upon completion, the reaction mixture was evaporated. The residue was dissolved in DCM (3 mL), basified to pH 8 with sat. aq. $Na_2CO_3$ and extracted with EtOAc (3 x 8 mL). The combined organic layers were washed with brine (15 mL), dried over $Na_2SO_4$ and evaporated to give (3a*R*,11a*S*)-6-chloro-10-methyl-1-(6-methyl-4-(trifluoromethyl)pyridin-2-yl)-5-((6-(2,2,2-trifluoroacetyl)-6,7-dihydro-5*H*-pyrrolo[3,4-*b*]pyrazin-2-yl)methyl)-1 ,3a,4,5, 1 0, 11a-hexahydro-2H-benzo[*b*]pyrrolo[2,3-*f*][1,4]diazocine-2,11(3*H*)-dione (150 mg, crude) as a black solid.

m/z ES+ $[M+H]^+$668.1.

**Step c.** To a solution of (3a*R*,11a*S*)-6-chloro-10-methyl-1-(6-methyl-4-(trifluoromethyl)pyridin-2-yl)-5-((6-(2,2,2-trifluoroacetyl)-6,7-dihydro-5*H*-pyrrolo[3,4-*b*]pyrazin-2-yl)methyl)-1,3a,4,5,10,11a-hexahydro-2*H*-benzo[*b*]pyrrolo[2,3-*f*][1,4]diazocine-2,11(3*H*)-dione (150 mg, 0.22 mmol) in MeOH (4 mL) and $H_2O$ (1 mL) was added $Na_2CO_3$ (48 mg, 0.45 mmol). The mixture was stirred at 20 °C for 1 h. Upon completion, the reaction mixture was diluted with $H_2O$ (8 mL) and extracted with EtOAc (3 x 10 mL). The combined layers were washed with brine (20 mL), dried over $Na_2SO_4$ and evaporated to give (3*aR*,11*aS*)-6-chloro-5-((6,7-dihydro-5*H*-pyrrolo[3,4-*b*]pyrazin-2-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (115 mg, crude) as a black solid.

m/z ES+ $[M+H]^+$ 572.0.

**Step d.** This step was conducted in a similar manner to **Example 228,** step c.

m/z ES+ $[M+H]^+$ 626.0; $^1$H NMR (400 MHz, $CDCl_3$) δ ppm 8.75 - 8.65 (m, 1H), 8.32 (s, 1H), 7.52 - 7.46 (m, 1H), 7.36 - 7.30 (m, 2H), 7.06 (s, 1H), 6.61 - 6.49 (m, 2H), 5.88 - 5.80 (m, 1H), 4.97 (s, 2H), 4.91 (d, *J* = 9.2 Hz, 2H), 4.70 - 4.66 (m, 1H), 4.44 - 4.31 (m, 2H), 3.62 - 3.57 (m, 1H), 3.42 - 3.25 (m, 3H), 3.16 - 3.07 (m, 1H), 3.06 - 2.91 (m, 1H), 2.73 - 2.67 (m, 1H), 2.48 (s, 3H), 2.25 - 2.18 (m, 1H).

**Example 230**

**(3a*R*,11a*S*)-5-(((*R*/*S*)-6-acryloyl-7-(hydroxymethyl)-6,7-dihydro-5*H*-pyrrolo[3,4-*b*]pyridin-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

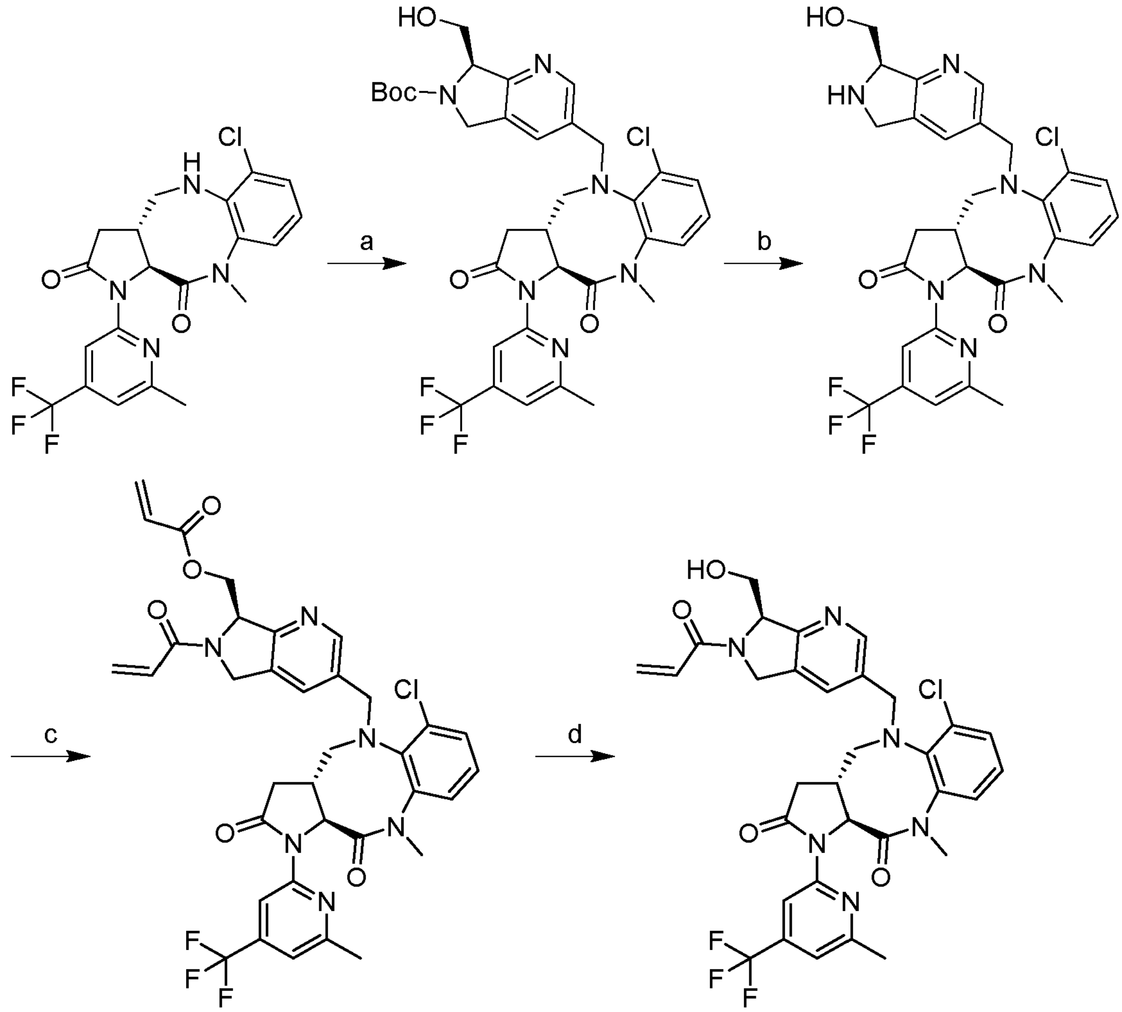


**Step** a. To a solution of Intermediate 5b (615 mg, 1.40 mmol), **Intermediate B51a** (550 mg, 1.40 mmol) in DMF (6 mL) was added TMSCl (381 mg, 3.50 mmol). The reaction mixture was stirred at 0 °C for 30 min. A solution of borane tetrahydrofuran complex (1 M in THF, 2.1 mL) in THF (6 mL) was added and the reaction mixture was stirred at 30 °C for 12 h. Upon completion, the reaction mixture was quenched with sat. aq. $NaHCO_3$ (10 mL), diluted with water (70 mL) and extracted with EtOAc (3 x 30 mL). The combined organic layers were washed with brine (3 x 30 mL), dried over $Na_2SO_4$ and evaporated. The residue was purified by column chromatography (PE/EtOAc = 1/3) to give tert-butyl (R/S)-3-(((3aR,11aS)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-7-(hydroxymethyl)-5,7-dihydro-6*H*-pyrrolo[3,4-*b*]pyridine-6-carboxylate (530 mg, 54% yield) as a yellow solid.

m/z ES+ $[M+H]^+$ 701.3.

**Step b.** To a solution of tert-butyl (*R*/*S*)-3-(((3a*R*,11a*S*)-6-chloro-10-methyl-1-(6-methyl-4-(trifluoromethyl)pyridin-2-yl)-2, 11-dioxo-1 ,2,3,3a,4, 1 0, 11,11 a-octahydro-5*H*-benzo[*b*]pyrrolo[2,3-*f*][1,4]diazocin-5-yl)methyl)-7-(hydroxymethyl)-5,7-dihydro-6*H*-pyrrolo[3,4-*b*]pyridine-6-carboxylate (480 mg, 0.69 mmol) in DCM (4 mL) was added TFA (1.54 g, 13.5 mmol). The reaction mixture was stirred for 1 h at 25 °C. Upon completion, the reaction mixture was evaporated to give (3a*R*,11a*S*)-6-chloro-5-(((*R*/*S*)-7-(hydroxymethyl)-6,7-dihydro-5H-pyrrolo[3,4-*b*]pyridin-3-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (411 mg, crude) as a yellow oil.

m/z ES+ $[M+H]^+$ 601.2.

**Step c.** To a solution of (3aR,11aS)-6-chloro-5-(((R/S)-7-(hydroxymethyl)-6,7-dihydro-5*H*-pyrrolo[3,4-*b*]pyridin-3-yl)methyl)-10-methyl-1-(6-methyl-4-(trifluoromethyl)pyridin-2-yl)-1,3a,4,5,10,11a-hexahydro-2*H*-benzo[*b*]pyrrolo [2,3-*f*][1,4]diazocine-2,11(3*H*)-dione (400 mg, 0.67 mmol) and DIPEA (258 mg, 2.00 mmol) in DCM (5 mL) was added acryloyl chloride (57 mg, 0.63 mmol) at -40 °C. The reaction mixture was stirred at -40 °C for 5 min. Upon completion, the mixture was quenched with water (1 mL), further diluted with water (30 mL) and extracted with DCM (2 x 30 mL). The combined organic layers were washed with brine (30 mL), dried over $Na_2SO_4$ and evaporated. The residue was purified by reverse phase flash chromatography (water (0.1% $NH_4OH$)/MeCN) to give ((*R*/*S*)-6-acryloyl-3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo [2,3-*f*][1,4]diazocin-5-yl)methyl)-6,7-dihydro-5*H*-pyrrolo[3,4-*b*]pyridin-7-yl)methyl acrylate (250 mg, 53% yield) as a white solid.

m/z ES+ $[M+H]^+$ 709.3.

**Step d.** To a solution of ((*R*/*S*)-6-acryloyl-3-(((3a*R*,11a*S*)-6-chloro-10-methyl-1-(6-methyl-4-(trifluoromethyl) pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)methyl acrylate (230 mg, 0.32 mmol) in THF (1 mL) and $H_2O$ (0.5 mL) was added lithium hydroxide monohydrate (41 mg, 0.97 mmol). The reaction mixture was stirred at 30 °C for 1 h. Upon completion, the mixture was quenched with water (1 mL), further diluted with water (30 mL) and extracted with EtOAc (2 x 30 mL). The combined organic layers were washed with brine (30 mL), dried over $Na_2SO_4$ and evaporated. The residue was purified by reverse phase flash chromatography (water (0.1% $NH_4OH$)/MeCN) to give the title compound (120 mg, 56% yield) as a white solid.

m/z ES+ $[M+H]^+$ 655.2; $^1H$ NMR (400 MHz, $CDCl_3$) δ ppm 8.45 (s, 1H), 8.33 (s, 1H), 7.85 - 7.68 (m, 1H), 7.54 - 7.45 (m, 1H), 7.35 - 7.30 (m, 2H), 7.07 (s, 1H), 6.64 - 6.48 (m, 2H), 5.91 - 5.82 (m, 1H), 5.45 - 5.36 (m, 1H), 4.96 (d, *J* = 3.2 Hz, 2H), 4.66 (d, *J* = 9.6 Hz, 1H), 4.33 - 4.16 (m, 3H), 4.00 - 3.83 (m, 1H), 3.52 - 3.46 (m, 1H), 3.34 - 3.28 (m, 3H), 3.11 - 3.01 (m, 1H), 2.97 - 2.81 (m, 1H), 2.73 - 2.63 (m, 1H), 2.49 (s, 3H), 2.25 - 2.16 (m, 1H).

**Example 231**

**(3a*R*,11a*S*)-5-(((*S*/*R*)-6-acryloyl-7-(hydroxymethyl)-6,7-dihydro-5*H*-pyrrolo[3,4-*b*]pyridin-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo [2,3-*f*][1,4]diazocine-2,11(3*H*)-dione**

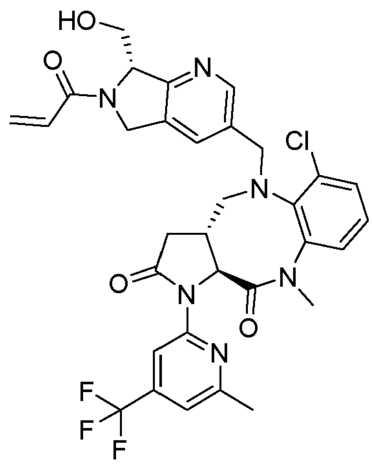

The title compound was prepared in a similar manner to **Example 230,** using **Intermediate B51b** in step a.

m/z ES+ $[M+H]^+$ 655.2; $^1H$ NMR (400 MHz, $CDCl_3$) δ ppm 8.53 - 8.44 (m, 1H), 8.32 (s, 1H), 7.79 - 7.67 (m, 1H), 7.50 - 7.47 (m 1H), 7.37 - 7.28 (m, 2H), 7.06 (s, 1H), 6.71 - 6.46 (m, 2H), 5.91 - 5.77 (m, 1H), 5.43 - 5.11 (m, 1H), 5.08 - 4.90 (m, 2H), 4.78 - 4.62 (m, 1H), 4.34 - 4.21 (m, 2H), 4.21 - 4.05 (m, 1H), 4.00 - 3.84 (m, 1H), 3.50 - 3.47 (m, 1H), 3.39 - 3.19 (m, 3H), 3.10 - 2.98 (m, 1H), 2.97 - 2.84 (m, 1H), 2.69 - 2.64 (m, 1H), 2.48 (s, 3H), 2.24 - 2.16 (m, 1H).

**Example 232**

**(3a*R*,11a*S*)-5-(((*R*/*S*)-6-acryloyl-7-(hydroxymethyl)-6,7-dihydro-5*H*-pyrrolo[3,4-*b*]pyridin-3-yl)methyl)-6,10-dimethyl-1-(6-methyl-4-(trifluoromethyl)pyridin-2-yl-1,3a,4,5,10,11a-hexahydro-2*H*-benzo[*b*]pyrrolo[2,3-*f*][1,4]diazocine-2,11(3*H*)-dione**

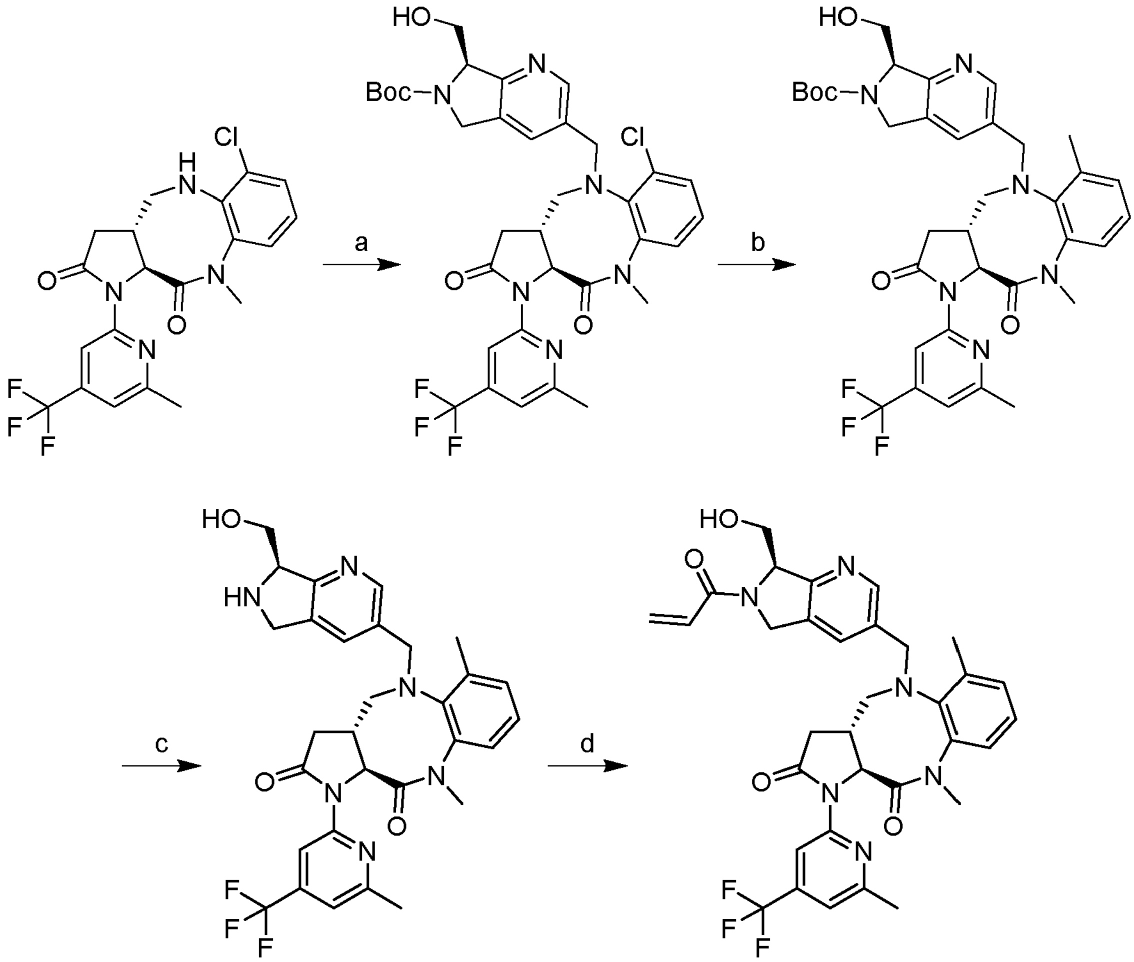


**Step a.** This step was conducted as described in **Example 230.**

**Step b.** This step was conducted in a similar manner as **Example 77,** step b.

m/z ES+ $[M+H]^+$681.4; $^1$H NMR (400 MHz, $CDCl_3$) δ ppm 8.43 (s, 1H), 8.33 (s, 1H), 7.66-7.51 (m, 1H), 7.27 (s, 3H), 7.06 (s, 1H), 5.19 - 4.89 (m, 1H), 4.79 - 4.40 (m, 4H), 4.18 (d, *J* = 14.4 Hz, 2H), 4.09 - 3.87 (m, 2H), 3.56 - 3.23 (m, 4H), 3.05 - 2.61 (m, 3H), 2.54 - 2.47 (m, 3H), 2.45 (s, 3H), 2.22 - 2.07 (m, 1H), 1.54 (s, 9H).

**Steps c-d.** These 2 steps were conducted in a similar manner to **Example 27,** steps b-c, using acrylic acid in step d.

m/z ES+ $[M+H]^+$ 635.5; $^1$H NMR (400 MHz, $CDCl_3$) δ ppm 8.56 - 8.49 (m, 1H), 8.32 (s, 1H), 7.89 - 7.64 (m, 1H), 7.31 - 7.27 (m, 3H), 7.07 (s, 1H), 6.72 - 6.48 (m, 2H), 5.93 - 5.80 (m, 1H), 5.53 - 5.30 (m, 1H), 5.17 - 4.78 (m, 2H), 4.78 - 4.70 (m, 1H), 4.36 - 4.27 (m, 1H), 4.26 - 4.17 (m, 1H), 4.17 - 4.06 (m, 1H), 4.06 - 3.86 (m, 1H), 3.45 - 3.50 (m, 1H), 3.42 - 3.34 (m, 3H), 3.07 - 2.87 (m, 1H), 2.87 - 2.78 (m, 1H), 2.60 - 2.75 (m, 1H), 2.51 (s, 3H), 2.47 (s, 3H), 2.21 - 2.12 (m, 1H).

**Example 233**

**(3a*R*,11a*S*)-5-(((*S*/*R*)-6-acryloyl-7-(hydroxymethyl)-6,7-dihydro-5*H*-pyrrolo[3,4-*b*]pyridin-3-yl)methyl)-6,10-dimethyl-1-(6-methyl-4-(trifluoromethyl)pyridin-2-yl-1,3a,4,5,10,11a-hexahydro-2*H*-benzo[b]pyrrolo[2,3-*f*][1,4]diazocine-2,11(3*H*)-dione**

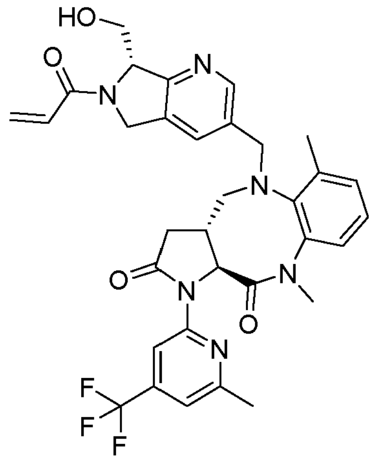

The title compound was prepared in a similar manner to **Example 232,** using **Intermediate B51b** in step a.

m/z ES+ $[M+H]^+$ 635.4; $^1$H NMR (400 MHz, $CDCl_3$) δ ppm 8.57 - 8.46 (m, 1H), 8.33 (s, 1H), 7.69 - 7.56 (m, 1H), 7.31 - 7.27 (m, 2H), 7.25 (s, 1H), 7.07 (s, 1H), 6.72 - 6.49 (m, 2H), 5.92-5.81 (m, 1H), 5.46 - 5.13 (m, 1H), 5.11 - 4.77 (m, 2H), 4.76 - 4.70 (m, 1H), 4.36 - 4.27 (m, 1H), 4.27 - 4.19 (m, 1H), 4.17 - 4.03 (m, 1H), 4.03 - 3.85 (m, 1H), 3.49 - 3.41 (m, 1H), 3.42 - 3.32 (m, 3H), 3.08 - 2.89 (m, 1H), 2.88 - 2.75 (m, 1H), 2.74 - 2.63 (m, 1H), 2.51 (s, 3H), 2.49 - 2.43 (m, 3H), 2.23 - 2.12 (m, 1H).

**Example 234**

**(3a*R*,11a*S*)-5-(((*S*/*R*)-6-acryloyl-7-(methoxymethyl)-6,7-dihydro-5*H*-pyrrolo[3,4-*b*]pyridin-3-yl)methyl)-6,10-dimethyl-1-(6-methyl-4-(trifluoromethyl)pyridin-2-yl-1,3a,4,5,10,11a-hexahydro-2*H*-benzo[b]pyrrolo[2,3-*f*][1,4]diazocine-2,11(3*H*)-dione**

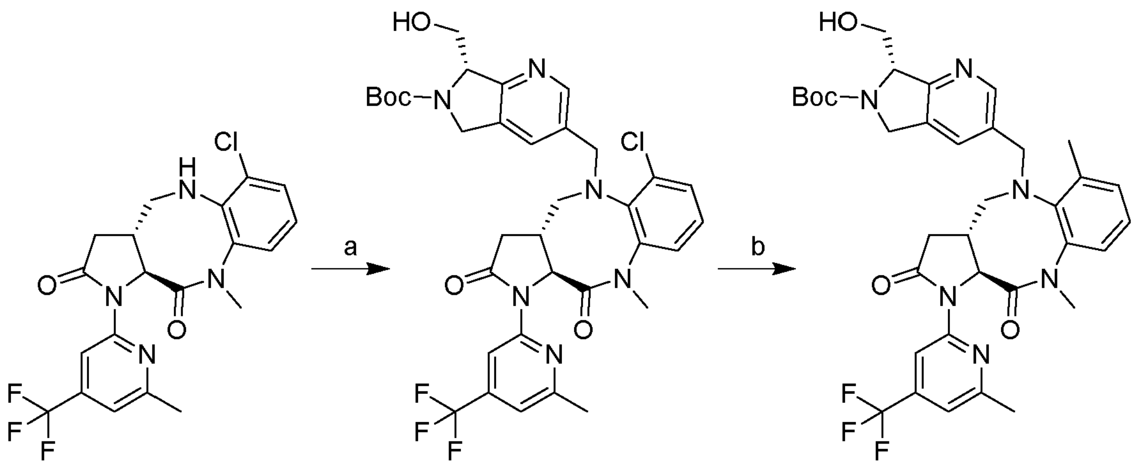

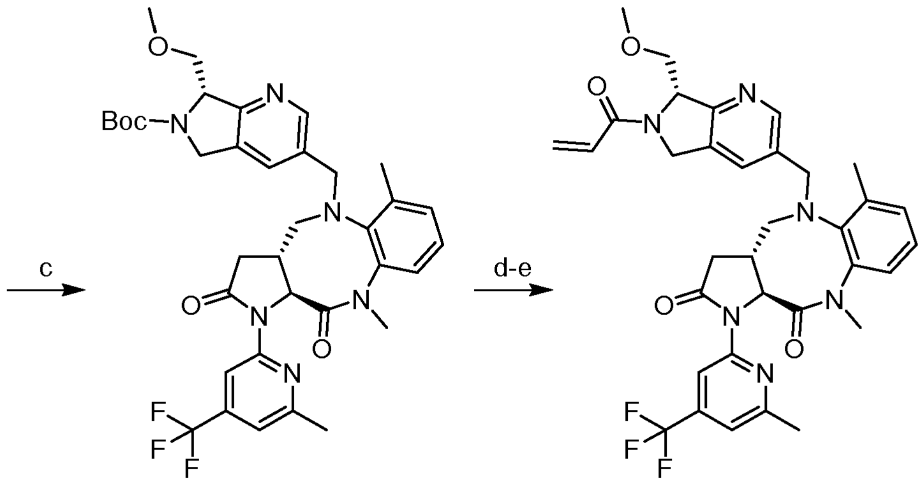


**Step a.** To a solution of **Intermediate B51b** (215 mg, 0.55 mmol) and **Intermediate 5b** (0.2 g, 0.46 mmol) in DMF (5 mL) was added TMSCl (124 mg, 1.14 mmol) at 0 °C. The mixture was stirred for 30 min, after which a solution of borane tetrahydrofuran complex (1 M in THF, 0.46 mL) in THF (5 mL) was added. The reaction mixture was stirred at 20 °C for 11 h 30 min. Upon completion, the reaction mixture was quenched with sat. aq. $NaHCO_3$ solution (30 mL) and extracted with EtOAc (30 mL). The organic layer was washed with brine (30 mL), dried over $Na_2SO_4$, evaporated and purified by column chromatography (EtOAc) to give *tert*-butyl (*S*/*R*)-3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-7-(hydroxymethyl)-5,7-dihydro-6*H*-pyrrolo[3,4-*b*]pyridine-6-carboxylate (220 mg, 64% yield) as a colourless gum.

m/z ES+ $[M+H]^+$ 701.5, $^1H$ NMR (400 MHz, $CDCl_3$) δ ppm 8.52 - 8.38 (m, 1H), 8.33 (s, 1H), 7.71 - 7.63 (m, 1H), 7.52 - 7.45 (m, 1H), 7.32 (d, *J* = 4.0 Hz, 2H), 7.06 (s, 1H), 5.16 - 4.87 (m, 1H), 4.84 - 4.56 (m, 3H), 4.34 - 4.25 (m, 1H), 4.24 - 4.16 (m, 3H), 3.98 - 3.86 (m, 1H), 3.53 - 3.44 (m, 1H), 3.36 - 3.24 (m, 3H), 3.09 - 2.99 (m, 1H), 2.98 - 2.85 (m, 1H), 2.74 - 2.63 (m, 1H), 2.48 (s, 3H), 2.26 - 2.15 (m, 1H), 1.53 (s, 9H).

**Step b.** A mixture of *tert*-butyl (*S*/*R*)-3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-7-(hydroxymethyl)-5,7-dihydro-6*H*-pyrrolo[3,4-b]pyridine-6-carboxylate (0.21 g, 0.30 mmol), methylboronic acid (717 mg, 12.0 mmol), $Cy_3P$-Pd-G3 (39 mg, 0.06 mmol) and $Cs_2CO_3$ (293 mg, 0.90 mmol) in 1,4-dioxane (6 mL) was stirred at 100 °C for 12 h under a $N_2$ atmosphere. Upon completion, the reaction mixture was evaporated, the residue was diluted with water (20 mL) and extracted with EtOAc (20 mL). The organic layer was washed with brine (20 mL), dried over $Na_2SO_4$, evaporated and purified by column chromatography (EtOAc) to give *tert*-butyl (*S*/*R*)-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-7-(hydroxymethyl)-5,7-dihydro-6H-pyrrolo[3,4-b]pyridine-6-carboxylate (160 mg, 78% yield) as a white solid.

m/z ES+ $[M+H]^+$681.3; $^1H$ NMR (400 MHz, $CDCl_3$) δ ppm 8.46 (s, 1H), 8.33 (s, 1H), 7.62-7.52 (m, 1H), 7.30 - 7.27 (m, 1H), 7.27 - 7.22 (m, 2H), 7.06 (s, 1H), 5.16 - 4.89 (m, 1H), 4.88 - 4.57 (m, 3H), 4.26 - 4.16 (m, 2H), 4.09 - 3.87 (m, 2H), 3.49 - 3.42 (m, 2H), 3.42 - 3.34 (m, 3H), 3.07 - 2.90 (m, 1H), 2.86 - 2.75 (m, 1H), 2.74 - 2.62 (m, 1H), 2.50 (s, 3H), 2.46 (s, 3H), 2.22 - 2.12 (m, 1H), 1.54 (s, 9H).

**Step c.** To a solution of *tert*-butyl (*S*/*R*)-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-7-(hydroxymethyl)-5,7-dihydro-6*H*-pyrrolo[3,4-*b*]pyridine-6-carboxylate (0.19 g, 0.28 mmol) in DMF (3 mL) was added NaH (13 mg, 0.34 mmol, 60% dispersion in mineral oil) at 0 °C. The mixture was stirred for 30 min, after which MeI (59 mg, 0.42 mmol) was added. The reaction mixture was stirred at 0 °C for 1 h. Upon completion, the reaction mixture was poured into sat. aq. $NH_4Cl$ (20 mL) and extracted with EtOAc (20 mL). The organic layer was washed with brine (20 mL), dried over $Na_2SO_4$, evaporated and purified by prep-TLC (EtOAc) to give *tert*-butyl (*S*/*R*)-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-7-(methoxymethyl)-5,7-dihydro-6*H*-pyrrolo[3,4-b]pyridine-6-carboxylate (40 mg, 19% yield) as a white solid.

m/z ES+ $[M+H]^+$695.3.

**Step d.** To a solution of *tert*-butyl (*S*/*R*)-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-7-(methoxymethyl)-5,7-dihydro-6*H*-pyrrolo[3,4-b]pyridine-6-carboxylate (40 mg, 0.58 mmol) in DCM (3 mL) was added TFA (1.54 g, 13.5 mmol). The reaction mixture was stirred at 25 °C for 30 min. Upon completion, the mixture was evaporated to give (3a*R*,11a*S*)-5-(((*S*/*R*)-7-(methoxymethyl)-6,7-dihydro-5*H*-pyrrolo[3,4-*b*]pyridin-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione as a TFA salt (40 mg, 98% yield) as a yellow gum.

m/z ES+ $[M+H]^+$595.2.

**Step e.** To a solution of (3a*R*,11a*S*)-5-(((*S*/*R*)-7-(methoxymethyl)-6,7-dihydro-5*H*-pyrrolo[3,4-*b*]pyridin-3-yl) methyl)-6,10-dimethyl-1-(6-methyl-4-(trifluoromethyl)pyridin-2-yl)-1,3a,4,5,10,11a-hexahydro-2*H*-benzo[*b*]pyrrolo [2,3-*f*][1,4]diazocine-2,11(3*H*)-dione (TFA salt, 40 mg, 0.057 mmol) in DCM (5 mL) was added DIPEA (37 mg, 0.28 mmol) and acryloyl chloride (6 mg, 68 mmol). The reaction mixture was stirred at 20 °C for 10 min. Upon completion, the reaction mixture was diluted with water (20 mL) and extracted with EtOAc (20 mL). The organic layer was washed with brine (20 mL), dried over $Na_2SO_4$, evaporated and purified by prep-TLC (EtOAc) to give the title compound (36 mg, 96% yield) as a white solid. m/z ES+ $[M+H]^+$ 649.3, $^1H$ NMR (400 MHz, $CDCl_3$) δ ppm 8.48 (d, *J* = 4.0 Hz, 1H), 8.33 (s, 1H), 7.58 (d, *J* = 18.0 Hz, 1H), 7.28 (s, 1H), 7.27 - 7.21 (m, 2H), 7.06 (s, 1H), 6.77 - 6.54 (m, 1H), 6.54 - 6.45 (m, 1H), 5.84 - 5.75 (m, 1H), 5.40 - 5.20 (m, 1H), 5.13 - 4.64 (m, 3H), 4.30-4.04 (m, 2H), 4.03 - 3.77 (m, 2H), 3.52 - 3.42 (m, 1H), 3.41 - 3.29 (m, 3H), 3.28 - 3.17 (m, 3H), 3.08 - 2.93 (m, 1H), 2.86 - 2.74 (m, 1H), 2.73 - 2.64 (m, 1H), 2.50 (s, 3H), 2.45 (d, *J* = 7.2 Hz, 3H), 2.22 - 2.11 (m, 1H).

### Example 235

**(3a*R*,11a*S*)-5-(((*R*/*S*)-6-acryloyl-7-(methoxymethyl)-6,7-dihydro-5*H*-pyrrolo[3,4-*b*]pyridin-3-yl)methyl)-6,10-dimethyl-1-(6-methyl-4-(trifluoromethyl)pyridin-2-yl-1,3a,4,5,10,11a-hexahydro-2*H*-benzo[b]pyrrolo[2,3-*f*][1,4]diazocine-2,11(3*H*)-dione**

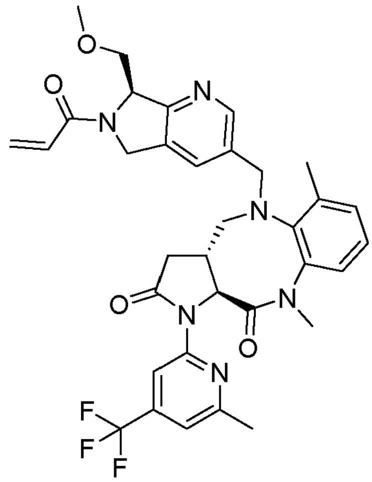

The title compound was prepared in a similar manner to **Example 234,** using **Intermediate B51a** in step a.

m/z ES+ $[M+H]^+$ 649.2, $^1H$ NMR (400 MHz, $CDCl_3$) δ ppm 8.52 - 8.41 (m, 1H), 8.32 (s, 1H), 7.67 - 7.56 (m, 1H), 7.31 - 7.28 (m, 1H), 7.25 (s, 2H), 7.06 (s, 1H), 6.76 - 6.54 (m, 1H), 6.54-6.44 (m, 1H), 5.88 - 5.72 (m, 1H), 5.43 - 5.19 (m, 1H), 5.10 - 4.68 (m, 3H), 4.29 - 4.10 (m, 2H), 4.09 - 3.79 (m, 2H), 3.48 (d, *J* = 13.2 Hz, 1H), 3.42 - 3.30 (m, 3H), 3.28 - 3.16 (m, 3H), 3.07-2.90 (m, 1H), 2.88 - 2.75 (m, 1H), 2.74 - 2.63 (m, 1H), 2.50 (s, 3H), 2.46 (d, *J* = 7.2 Hz, 3H), 2.23 - 2.12 (m, 1H).

### Example 236

**(3a*R*,11a*S*)-5-(((*S*/*R*)-6-acryloyl-7-(methoxymethyl)-6,7-dihydro-5*H*-pyrrolo[3,4-*b*]pyridin-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo [2,3-*f*][1,4]diazocine-2,11(3*H*)-dione**

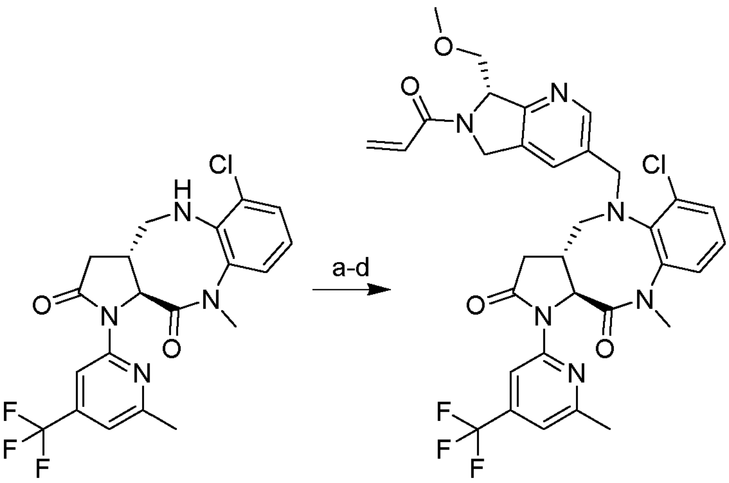


**Steps a-d.** These 4 steps were conducted in a similar manner to **Example 234,** steps a, c-e, using **Intermediate B51b** in step a.

m/z ES+ $[M+H]^+$ 669.2; $^1$H NMR (400 MHz, $CDCl_3$) δ ppm 8.53 - 8.43 (m, 1H), 8.33 (s, 1H), 7.72 - 7.63 (m, 1H), 7.52 - 7.45 (m, 1H), 7.37 - 7.28 (m, 2H), 7.06 (s, 1H), 6.75 - 6.44 (m, 2H), 5.83 - 5.74 (m, 1H), 5.37 - 5.19 (m, 1H), 5.11 - 4.63 (m, 3H), 4.37 - 4.28 (m, 1H), 3.96 - 3.90 (m, 1H), 4.28 - 3.76 (m, 2H), 3.57 - 3.43 (m, 1H), 3.35 - 3.16 (m, 6H), 3.11 - 2.86 (m, 2H), 2.76 - 2.63 (m, 1H), 2.48 (s, 3H), 2.27 - 2.14 (m, 1H).

## Example 237

**(3a*R*,11a*S*)-5-(((*R*/*S*)-6-acryloyl-7-(methoxymethyl)-6,7-dihydro-5*H*-pyrrolo[3,4-b]pyridin-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

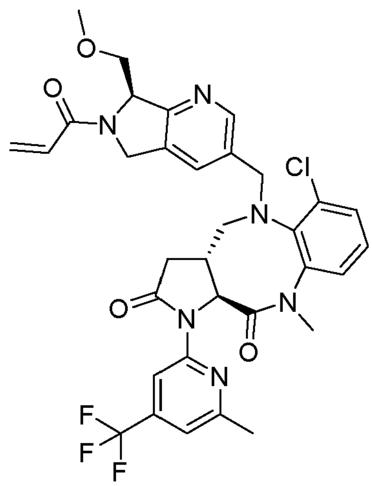

The title compound was prepared in a similar manner to **Example 236,** using **Intermediate B51a** in step a.

m/z ES+ $[M+H]^+$ 669.2, $^1$H NMR (400 MHz, $CDCl_3$) δ ppm 8.49 - 8.37 (m, 1H), 8.32 (s, 1H), 7.77 - 7.64 (m, 1H), 7.48 (d, *J* = 6.8 Hz, 1H), 7.36 - 7.28 (m, 2H), 7.12 - 7.01 (m, 1H), 6.76-6.36 (m, 2H), 5.85 - 5.73 (m, 1H), 5.38 - 5.17 (m, 1H), 5.10 - 4.79 (m, 2H), 4.73 - 4.60 (m, 1H), 4.35 - 4.12 (m, 2H), 3.96 - 3.76 (m, 2H), 3.58 - 3.46 (m, 1H), 3.35 - 3.15 (m, 6H), 3.11 - 2.85 (m, 2H), 2.75 - 2.63 (m, 1H), 2.48 (s, 3H), 2.27 - 2.20 (m, 1H).

## Example 238

**(3a*R*,11a*S*)-5-((6-acryloyl-5-(hydroxymethyl)-6,7-dihydro-5*H*-pyrrolo[3,4-*b*]pyridin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

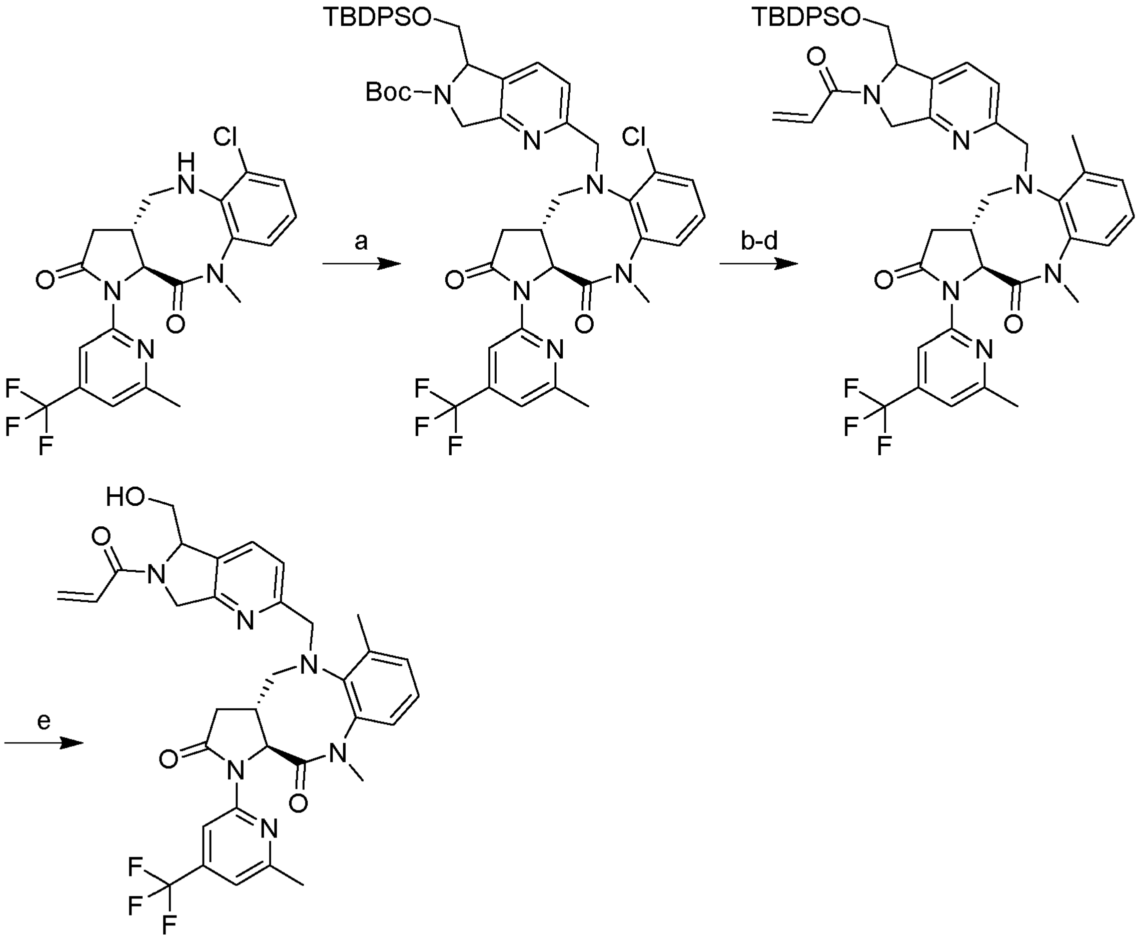


**Step a.** This step was conducted in a similar manner to **Example 228,** step a, using **Intermediate B52.**

m/z ES+ $[M+H]^+$ 939.4; $^1H$ NMR (400 MHz, $CDCl_3$) δ ppm 8.33 (s, 1H), 7.66 - 7.56 (m, 2H), 7.56 - 7.45 (m, 4H), 7.44 - 7.29 (m, 9H), 7.06 (s, 1H), 5.17 - 5.00 (m, 1H), 4.84 - 4.62 (m, 3H), 4.51 - 4.22 (m, 3H), 4.00 - 3.89 (m, 1H), 3.57 - 3.49 (m, 1H), 3.46 - 3.36 (m, 3H), 3.18 - 2.93 (m, 2H), 2.70 - 2.55 (m, 1H), 2.49 (s, 3H), 2.24 - 2.13 (m, 1H), 1.56 - 1.39 (m, 9H), 0.90 - 0.81 (m, 9H).

**Steps b-d.** These 3 steps were conducted in a similar manner to **Example 232,** steps b-d. m/z ES+ $[M+H]^+$ 873.1; $^1H$ NMR (400 MHz, $CDCl_3$) δ ppm 8.33 (s, 1H), 7.67 - 7.53 (m, 2H), 7.49 - 7.42 (m, 3H), 7.42 - 7.35 (m, 5H), 7.34 - 7.29 (m, 3H), 7.26 - 7.24 (m, 2H), 7.06 (s, 1H), 6.63 - 6.38 (m, 2H), 5.83 - 5.80 (m, 1H), 5.50 (s, 1H), 4.95 - 4.89 (m, 1H), 4.80 - 4.75 (m, 1H), 4.42 - 4.17 (m, 3H), 4.02 - 3.97 (m, 1H), 3.57 - 3.47 (m, 1H), 3.46 - 3.39 (m, 3H), 3.18 - 3.03 (m, 1H), 2.90 - 2.78 (m, 1H), 2.72 - 2.58 (m, 1H), 2.51 (s, 3H), 2.49 - 2.47 (m, 3H), 2.27 - 2.07 (m, 2H), 0.97 - 0.86 (m, 9H).

**Step e.** To a solution of (3a*R*,11a*S*)-5-((6-acryloyl-5-(((*tert*-butyldiphenylsilyl)oxy)methyl)-6,7-dihydro-5*H*-pyrrolo[3,4-*b*]pyridin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (20 mg, 0.023 mmol) in THF (0.5 mL) was added TBAF (1 M in THF, 23 μL). The mixture was stirred at 20 °C for 3 h. Upon completion, the reaction mixture was evaporated and purified by Prep-TLC (EtOAc/MeOH = 10/1) to give the title compound (11 mg, 73% yield) as an off-white solid.

m/z ES+ $[M+H]^+$ 635.3; $^1H$ NMR (400 MHz, $CDCl_3$) δ ppm 8.32 (s, 1H), 7.68 - 7.63 (m, 1H), 7.40 (t, *J* = 8.4 Hz, 1H), 7.27 - 7.22 (m, 3H), 7.05 (s, 1H), 6.66 - 6.50 (m, 2H), 5.91 - 5.88 (m, 1H), 5.54 (s, 1H), 4.93 (d, *J* = 4.8 Hz, 2H), 4.87 - 4.71 (m, 2H), 4.38 - 4.31 (m, 1H), 4.23 - 4.15 (m, 1H), 4.06 - 3.98 (m, 1H), 3.92 - 3.83 (m, 1H), 3.55 - 3.50 (m, 1H), 3.36 (d, *J* = 8.8 Hz, 3H), 3.15 - 3.03 (m, 1H), 2.89 - 2.85 (m, 1H), 2.73 - 2.64 (m, 1H), 2.50 (s, 3H), 2.47 (s, 3H), 2.21-2.12 (m, 1H).

**Example 239**

**(3a*R*,11a*S*)-5-((6-acryloyl-5-(hydroxymethyl)-6,7-dihydro-5*H*-pyrrolo[3,4-*b*]pyridin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

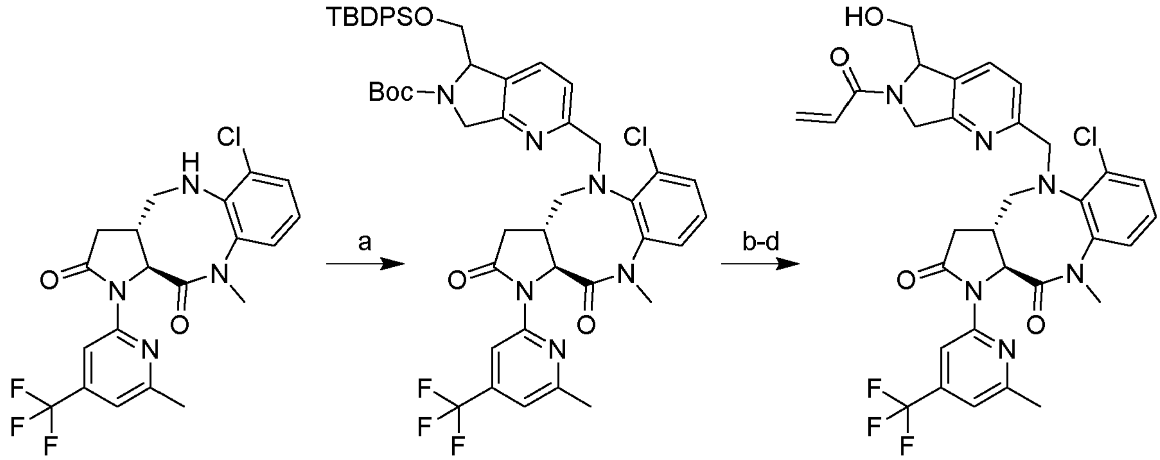


**Step a.** This step was conducted as described in **Example 238,** step a.

**Steps b-d.** These 3 steps were conducted in a similar manner to **Example 238,** steps c-e.

m/z ES+ $[M+H]^+$ 655.0; $^1H$ NMR (400 MHz, $CDCl_3$) δ ppm 8.32 (s, 1H), 7.68 - 7.63 (m, 1H), 7.61 - 7.55 (m, 1H), 7.51 - 7.47 (m, 1H), 7.35 - 7.30 (m, 2H), 7.06 (s, 1H), 6.65 - 6.50 (m, 2H), 5.90 - 5.87 (m, 1H), 5.54 (s, 1H), 4.93 (d, *J* = 4.8 Hz, 2H), 4.79 - 4.65 (m, 2H), 4.35 (s, 2H), 4.05 - 3.97 (m, 1H), 3.91 - 3.81 (m, 1H), 3.56 - 3.53 (m, 1H), 3.31 (d, *J* = 12.4 Hz, 3H), 3.15-2.98 (m, 2H), 2.73 - 2.63 (m, 1H), 2.48 (s, 3H), 2.25 - 2.15 (m, 1H).

## Example 240

**(3a*R*,11a*S*)-5-((6-acryloyl-6,7-dihydro-5*H*-pyrrolo[3,4-*b*]pyridin-2-yl)methyl)-6,10-dimethyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione**

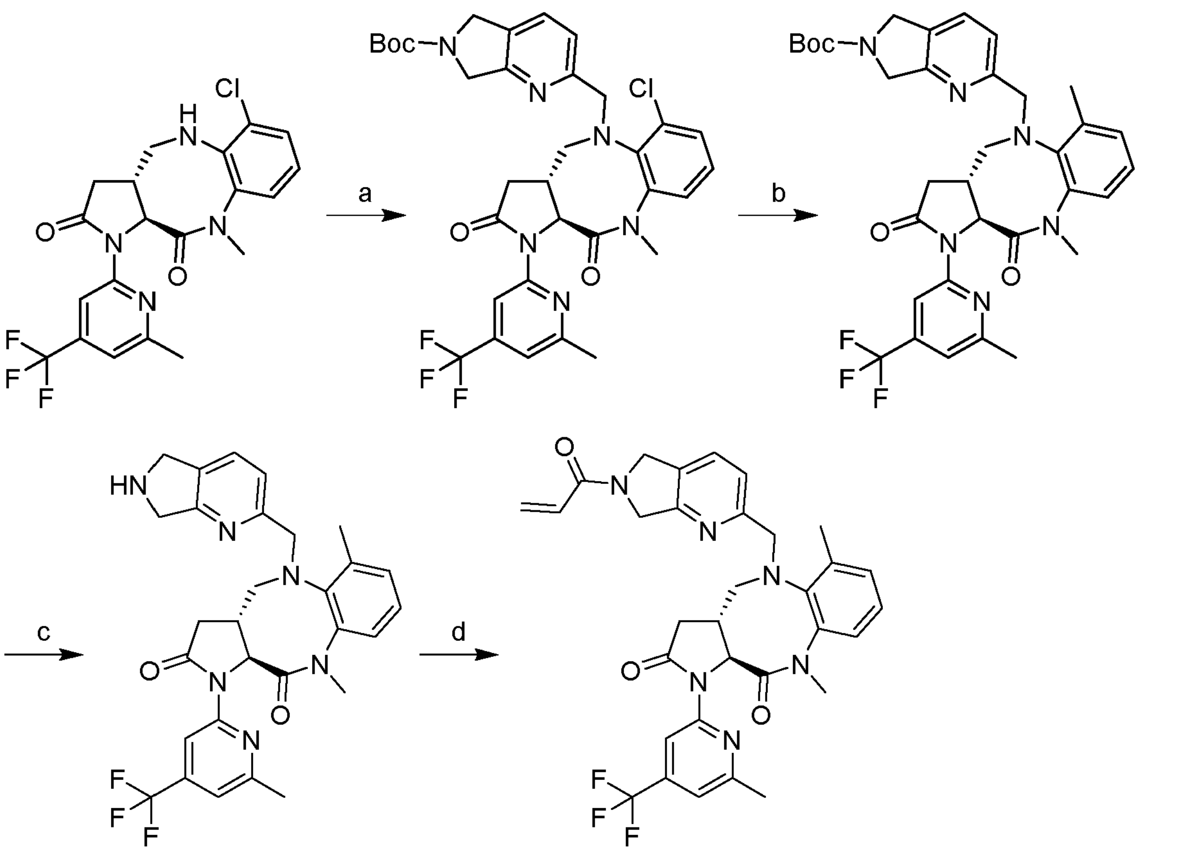


**Step** a. To a solution of **Intermediate 5b** (45.0 g, 102 mmol) and **Intermediate B53** (26.8 g, 102 mmol) in DMF (450 mL) was added TMSCl (27.8 g, 256 mmol) at 0 °C. The mixture was stirred at 0 °C for 30 min. A solution of borane tetrahydrofuran complex (1 M in THF, 102 mL) was then added dropwise at 0 °C. The reaction mixture was stirred at 25 °C for 3 h. Upon completion, the reaction mixture was quenched with MeOH (350 mL) and sat. aq. $NaHCO_3$ (1000 mL) was added. The aqueous mixture was extracted with EtOAc (3 x 1000 mL). The combined organic layers were washed with brine (1000 mL), dried over $Na_2SO_4$ and evaporated. The residue was purified by column chromatography (PE/EtOAc = 10/1 to 2/1) to give *tert*-butyl 2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,7-dihydro-6*H*-pyrrolo[3,4-*b*] pyridine-6-carboxylate (52.8 g, 72% yield) as a yellow solid.

m/z ES+ $[M+H]^+$671.4; $^1H$ NMR (400 MHz, $CDCl_3$) δ ppm 8.32 (s, 1H), 7.63 - 7.52 (m, 2H), 7.50 - 7.46 (m, 1H), 7.35 - 7.30 (m, 2H), 7.05 (s, 1H), 4.73 - 4.61 (m, 5H), 4.46 - 4.35 (m, 1H), 3.59 - 3.50 (m, 1H), 3.48 - 3.41 (m, 2H), 3.37 - 3.34 (m, 2H), 3.13 - 3.04 (m, 1H), 3.02 - 2.89 (m, 1H), 2.72 - 2.63 (m, 1H), 2.48 (s, 3H), 2.24 - 2.14 (m, 1H), 1.52 (s, 9H).

**Step b.** To a solution of *tert*-butyl 2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,7-dihydro-6*H*-pyrrolo[3,4-*b*]pyridine-6-carboxylate (52.8 g, 73.9 mmol) and $Cy_3P$-Pd-G3 (2.80 g, 3.81 mmol) in 1,4-dioxane (510 mL) was added $Cs_2CO_3$ (74.5 g, 228 mmol) at 25 °C. Methylboronic acid (27.3 g, 457 mmol) in 1,4-dioxane (150 mL) was then added dropwise at 90 °C. The resulting mixture was stirred at 110 °C for 4 h. Upon completion, the reaction mixture was diluted with water (1000 mL) and extracted with EtOAc (3 x 500 mL). The combined organic layers were dried over $Na_2SO_4$ and evaporated. The residue was purified by column chromatography (PE/EtOAc = 7/1 to 2/1) to give *tert*-butyl 2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,7-dihydro-6*H*-pyrrolo[3,4-*b*]pyridine-6-carboxylate (44.0 g, 67% yield) as a white solid.

m/z ES+ $[M+H]^+$651.4; $^1H$ NMR (400 MHz, $CDCl_3$) δ ppm 8.32 (s, 1H), 7.56 (dd, *J* = 16.4, 7.6 Hz, 1H), 7.32 (dd, *J* = 7.6, 3.2 Hz, 1H), 7.25 (s, 3H), 7.05 (s, 1H), 4.79 - 4.73 (m, 1H), 4.71-4.60 (m, 4H), 4.35 - 4.24 (m, 1H), 3.54 (td, *J* = 14.0, 4.0 Hz, 1H), 3.44 - 3.37 (m, 4H), 3.12-2.95 (m, 1H), 2.92 - 2.78 (m, 1H), 2.72 - 2.60 (m, 1H), 2.50 (s, 3H), 2.46 (s, 3H), 2.16 (dd, *J* = 16.8, 11.6 Hz, 1H), 1.53 (s, 9H).

**Step c.** To a solution of *tert*-butyl 2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,7-dihydro-6*H*-pyrrolo[3,4-*b*]pyridine-6-carboxylate (36.5 g, 41.5 mmol) in DCM (365 mL) was added TFA (54 mL, 730 mmol). The reaction mixture was stirred at 25 °C for 30 min. Upon completion, the reaction mixture was evaporated to give (3a*R*,11a*S*)-5-((6,7-dihydro-5*H*-pyrrolo[3,4-*b*]pyridin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione as a TFA salt (40.0 g, crude) as a brown oil that was used without further purification.

m/z ES+ $[M+H]^+$ 551.2.

**Step d.** DIPEA was added to a solution of (3a*R*,11a*S*)-5-((6,7-dihydro-5*H*-pyrrolo[3,4-*b*]pyridin-2-yl) methyl)-6,10-dimethyl-1-(6-methyl-4-(trifluoromethyl)pyridin-2-yl)-1,3a,4,5,10,11a-hexahydro-2*H*-benzo[*b*]pyrrolo [2,3-*f*][1,4]diazocine-2,11(3*H*)-dione (35.7 g, 53.7 mmol) in DCM (360 mL) until the pH of the mixture was ~7. Afterwhich, additional DIPEA (18.7 mL, 107 mmol) was added followed by the dropwise addition of acryloyl chloride (6.79 mL, 83.2 mmol) in DCM (36 mL) at 0 °C. The resulting mixture was stirred at 0 °C for 10 min. Upon completion, the reaction mixture was diluted with water (1000 mL) and extracted with EtOAc (3 x 500 mL). The combined organic layers were dried over $Na_2SO_4$, filtered and evaporated. The residue was purified by reverse phase flash chromatography (water (0.1% FA)/MeCN) to give the title compound (20.0 g, 60% yield) as a white solid.

m/z ES+ $[M+H]^+$ 605.2; $^1H$ NMR (400 MHz, $CDCl_3$) δ ppm 8.32 (s, 1H), 7.67 - 7.57 (m, 1H), 7.36 (d, *J* = 7.6 Hz, 1H), 7.27 - 7.23 (m, 3H), 7.05 (s, 1H), 6.62 - 6.47 (m, 2H), 5.83 - 5.78 (m, 1H), 4.95 - 4.86 (m, 4H), 4.76 (d, *J* = 9.6 Hz, 1H), 4.36 - 4.16 (m, 2H), 3.57 - 3.51 (m, 1H), 3.42 - 3.39 (m, 3H), 3.15 - 2.96 (m, 1H), 2.90 - 2.81 (m, 1H), 2.72 - 2.63 (m, 1H), 2.50 (s, 3H), 2.47 (s, 3H), 2.19 - 2.12 (m, 1H).

**Example 241**

**(3a*R*,11a*S*)-5-((5-acryloyl-1-methyl-1,4,5,6-tetrahydropyrrolo[3,4-*d*]imidazol-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

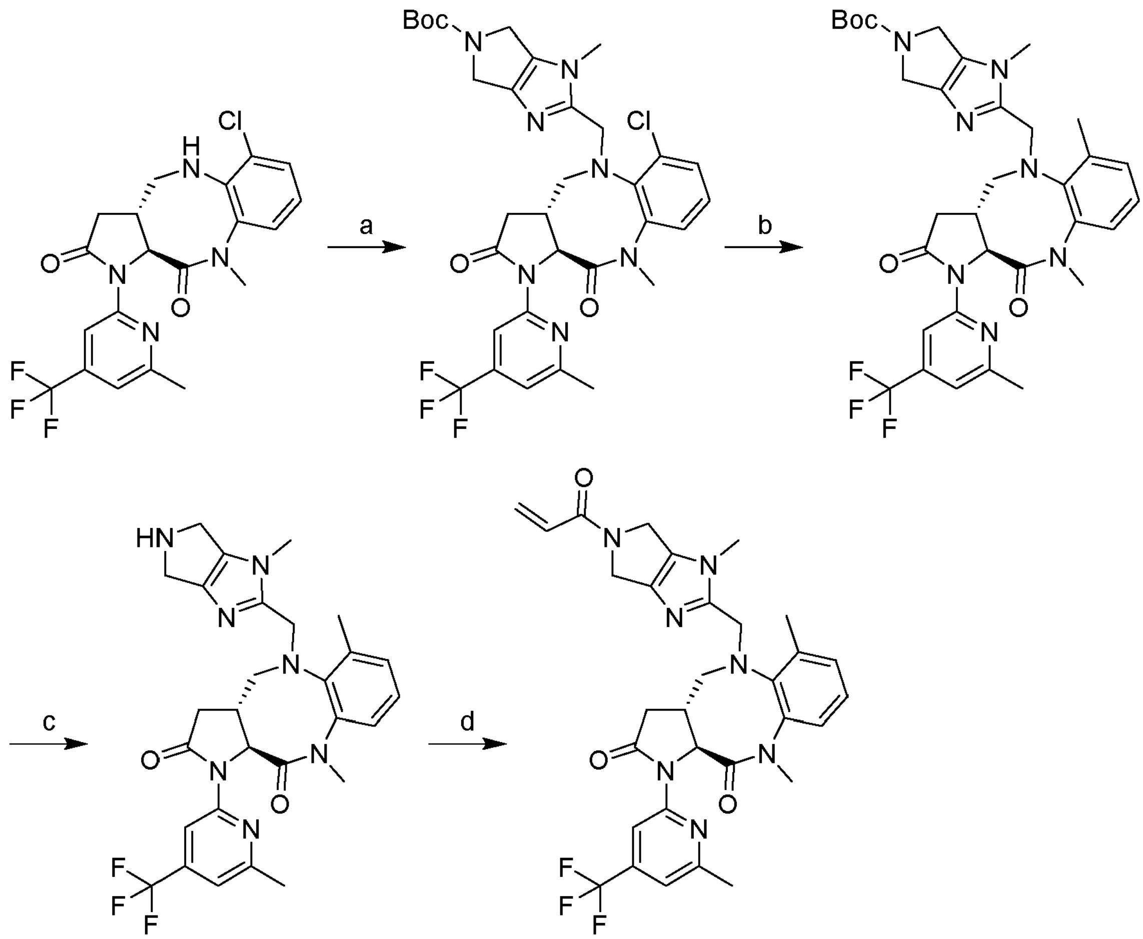


**Step a.** To a solution of **Intermediate B63** (500 mg, 1.99 mmol) and **Intermediate 5b** (873 mg, 1.99 mmol) in DMF (50 mL) was added TMSCl (540 mg, 4.97 mmol). The mixture was stirred at 0 °C for 30 min. A solution of borane

tetrahydrofuran complex (1 M in THF, 1.99 mL) in THF (40 mL) was added. The mixture was stirred at 25 °C for 12 h 30 min. Upon completion, the reaction mixture was evaporated and purified by reverse phase flash chromatography (water (0.1% FA)/MeCN) to give *tert*-butyl 2-(((3aR,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-1-methyl-4,6-dihydropyrrolo [3,4-*d*]imidazole-5(1H)-carboxylate (600 mg, 36% yield) as a yellow solid.

m/z ES+ $[M+H]^+$674.4; $^1$H NMR (400 MHz, $CDCl_3$) δ ppm 8.34 (s, 1H), 7.51 - 7.44 (m, 1H), 7.37 - 7.32 (m, 2H), 7.06 (s, 1H), 4.64 - 4.47 (m, 1H), 4.50 - 4.29 (m, 5H), 4.27 - 4.16 (m, 1H), 3.69 - 3.55 (m, 4H), 3.35 (d, *J* = 13.2 Hz, 3H), 3.09 - 2.93 (m, 2H), 2.80 - 2.74 (m, 1H), 2.48 (s, 3H), 2.24 - 2.17 (m, 1H), 1.52 (s, 9H).

**Step b.** A mixture of *tert*-butyl 2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-1-methyl-4,6-dihydropyrrolo[3,4-*d*]imidazole-5(1*H*)-carboxylate (560 mg, 0.83 mmol), methylboronic acid (2.49 g, 41.5 mmol), XPhos-Pd-G2 (65.4 mg, 0.083 mmol) and $Cs_2CO_3$ (676 mg, 2.08 mmol) in toluene (50 mL) was degassed and purged with $N_2$ 3 times. The reaction mixture was stirred at 110 °C for 12 h under a $N_2$ atmosphere. Upon completion, the reaction mixture was filtered, evaporated and purified by reverse phase flash chromatography (water (0.1% $NH_4OH$)/MeCN) to give *tert*-butyl 2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-1-methyl-4,6-dihydropyrrolo[3,4-*d*]imidazole-5(1*H*)-carboxylate (350 mg, 61% yield) as a colourless oil. m/z ES+ $[M+H]^+$654.3; $^1$H NMR (400 MHz, $CDCl_3$) δ ppm 8.34 (s, 1H), 7.28 - 7.27 (m, 3H), 7.06 (s, 1H), 4.72 - 4.68 (m, 1H), 4.51 - 4.27 (m, 5H), 4.16 - 4.01 (m, 1H), 3.73 - 3.61 (m, 1H), 3.57 (d, *J* = 1.6 Hz, 3H), 3.40 (d, *J* = 10.4 Hz, 3H), 3.19 - 2.91 (m, 1H), 2.84 - 2.72 (m, 2H), 2.50 (s, 3H), 2.41 - 2.40 (m, 3H), 2.21 - 2.18 (m, 1H), 1.52 (s, 9H).

**Step c.** A mixture of *tert*-butyl 2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-1-methyl-4,6-dihydropyrrolo [3,4-*d*]imidazole-5(1*H*)-carboxylate (300 mg, 0.46 mmol) and TFA (523 mg, 4.6 mmol) in DCM (1 mL) was stirred at 25 °C for 1 h under a $N_2$ atmosphere. Upon completion, the reaction mixture was evaporated to give (3a*R*,11a*S*)-6,10-dimethyl-5-((1-methyl-1,4,5,6-tetrahydropyrrolo[3,4-*d*]imidazol-2-yl)methyl)-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione as the TFA salt (300 mg, crude) as a yellow oil.

m/z ES+ $[M+H]^+$ 554.3.

**Step d.** To a solution of (3a*R*,11a*S*)-6,10-dimethyl-5-((1-methyl-1,4,5,6-tetrahydropyrrolo[3,4-*d*]imidazol-2-yl) methyl)-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (300 mg, 0.54 mmol), acrylic acid (51 mg, 0.70 mmol) in DCM (2 mL) was added DIPEA (210 mg, 1.6 mmol) and HATU (288 mg, 0.76 mmol). The mixture was stirred at 25 °C for 10 min. Upon completion, the reaction mixture was diluted with water (50 mL) and extracted with DCM (3 x 30 mL). The combined organic layers were washed with brine (30 mL), dried over $Na_2SO_4$ and evaporated. The residue was purified by reverse phase flash (water (0.1% $NH_4OH$)/-MeCN) to give the title compound (130 mg, 40% yield) as a white solid.

m/z ES+ $[M+H]^+$608.3; $^1$H NMR (400 MHz, $CDCl_3$) δ ppm 8.34 (s, 1H), 7.29 - 7.25 (m, 3H), 7.06 (s, 1H), 6.62 - 6.42 (m, 2H), 5.81 (dd, *J* = 7.6, 4.4 Hz, 1H), 4.83 - 4.52 (m, 5H), 4.39-4.28 (m, 1H), 4.17 - 3.99 (m, 1H), 3.74 - 3.54 (m, 4H), 3.50 - 3.34 (m, 3H), 3.21 - 2.93 (m, 1H), 2.88 - 2.71 (m, 2H), 2.51 (s, 3H), 2.45 - 2.37 (m, 3H), 2.19 (dd, *J* = 17.2, 11.2 Hz, 1H).

## Example 242

**(3a*R*,11a*S*)-5-((5-acryloyl-1-methyl-1,4,5,6-tetrahydropyrrolo[3,4-*d*]imidazol-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4] diazocine-2,11(3*H*)-dione**

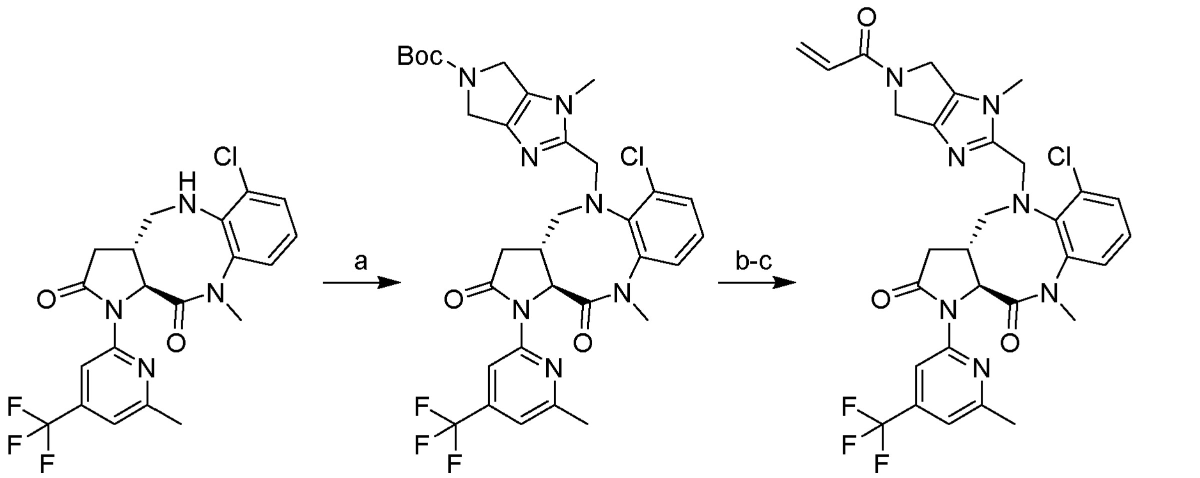


**Step a.** This step was conducted as described in **Example 241,** step a.

**Steps b-c.** These two steps were conducted in a similar manner to **Example 241,** steps c-d. m/z ES+ $[M+H]^+$ 628.2; $^1H$ NMR (400 MHz, $CDCl_3$) ppm 8.34 (s, 1H), 7.54 - 7.45 (m, 1H), 7.40 - 7.31 (m, 2H), 7.07 (s, 1H), 6.56 - 6.37 (m, 2H), 5.84 - 5.68 (m, 1H), 4.81 - 4.53 (m, 5H), 4.45 - 4.36 (m, 1H), 4.29 - 4.14 (m, 1H), 3.75 - 3.65 (m, 3H), 3.62 - 3.56 (m, 1H), 3.43 - 3.29 (m, 3H), 3.13 - 2.94 (m, 2H), 2.83 - 2.71 (m, 1H), 2.49 (s, 3H), 2.31 - 2.16 (m, 1H).

**Example 243a: (3a*R*,11a*S*)-5-(((*S*/*R*)-7-acryloyl-6-(difluoromethyl)-5,6,7,8-tetrahydroimidazo[1,2-*a*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione and**

**Example 243b: (3a*R*,11a*S*)-5-(((*R*/*S*)-7-acryloyl-6-(difluoromethyl)-5,6,7,8-tetrahydroimidazo[1,2-*a*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

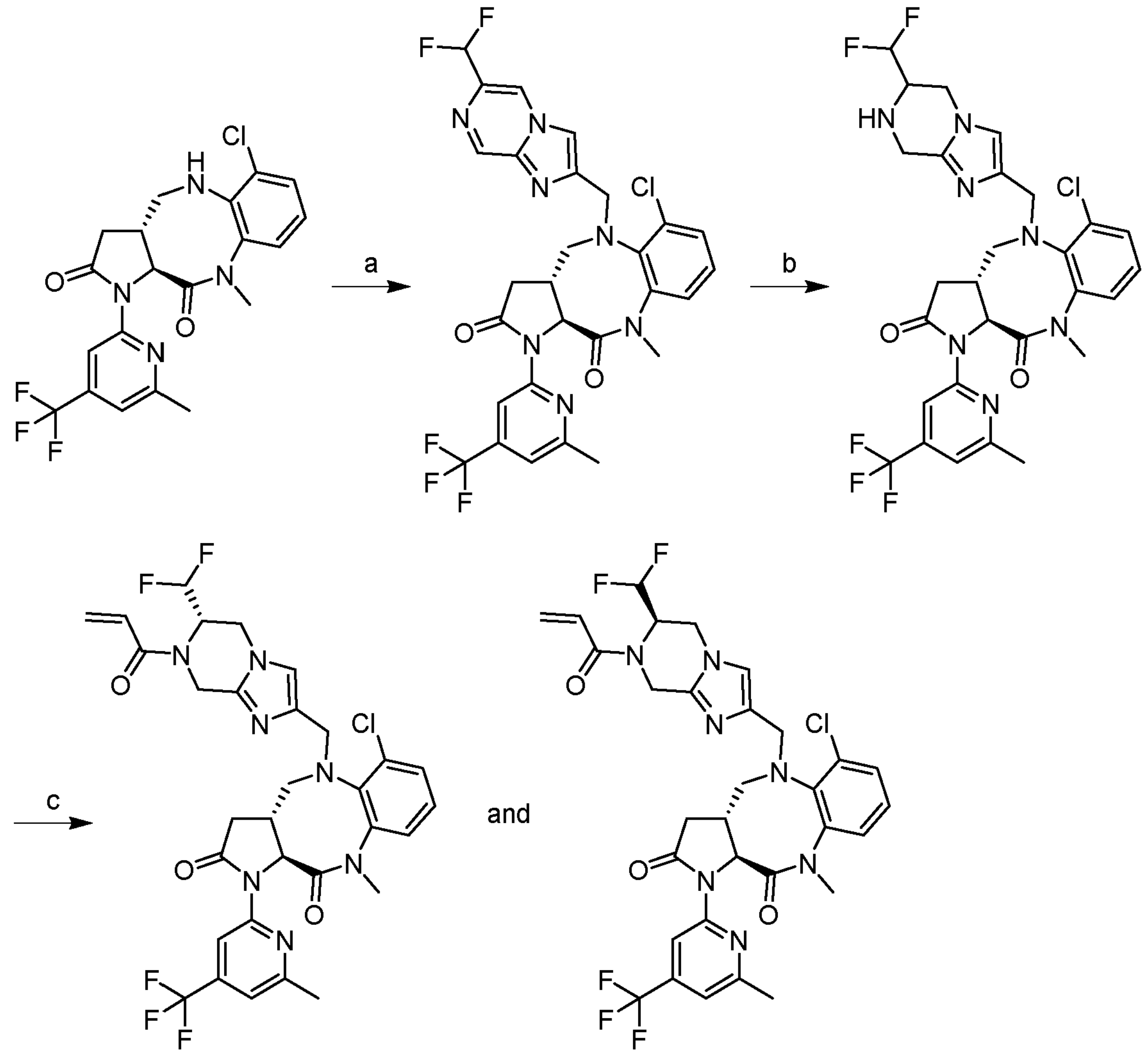


**Step a.** This step was conducted in a similar manner to **Example 39,** step a, using **Intermediate 5b** and **Intermediate B69.**

m/z ES+ $[M+H]^+$ 620.1; $^1$H NMR (400 MHz, $CDCl_3$) δ ppm 9.00 (s, 1H), 8.44 (s, 1H), 8.33 (s, 1H), 7.51 - 7.49 (m, 1H), 7.38 - 7.30 (m, 2H), 7.07 (s, 1H), 6.96 - 6.59 (m, 1H), 4.71 (d, *J* = 9.6 Hz, 1H), 4.68 - 4.42 (m, 2H), 3.64 - 3.59 (m, 1H), 3.42 (s, 1H), 3.39 (s, 3H), 3.15 - 3.11 (m, 1H), 3.09 - 2.98 (m, 1H), 2.71 - 2.65 (m, 1H), 2.50 (s, 3H), 2.25 - 2.18 (m, 1H).

**Step b.** To a solution of (3a*R*,11a*S*)-6-chloro-5-((6-(difluoromethyl)imidazo[1,2-a]pyrazin-2-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3H)-dione (150 mg, 0.24 mmol) in EtOH (7.5 mL) was added $NaBH_3CN$ (46 mg, 0.73 mmol) and acetic acid (3 mg, 0.048 mmol). The reaction mixture was stirred at 25-40 °C for 56 h. The mixture was diluted with water (10 mL) and extracted with EtOAc (3 x 5 mL). The combined organic layers were dried over $Na_2SO_4$, filtered and evaporated. The residue was purified by reverse phase flash chromatography (water (0.1% FA)/MeCN) to give (3a*R*,11a*S*)-6-chloro-5-((6-(difluoromethyl)-5,6,7,8-tetrahydroimidazo[1,2-*a*]pyrazin-2-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (120 mg, 80% yield) as a white solid.

m/z ES+ $[M+H]^+$624.3; $^1$H NMR (400 MHz, MeOD-$d_4$) δ ppm 8.30 (s, 1H), 7.53 - 7.41 (m, 2H), 7.44 - 7.34 (m, 1H), 7.22 (s, 1H), 7.01 (s, 1H), 6.26 - 5.83 (m, 1H), 4.20 - 4.07 (m, 4H), 4.02-3.89 (m, 2H), 3.69 - 3.46 (m, 3H), 3.32 (s, 4H), 3.17 - 3.06 (m, 1H), 2.98 - 2.87 (m, 1H), 2.67 - 2.61 (m, 1H), 2.53 (s, 3H), 2.39 - 2.35 (m, 1H).

**Step c.** This step was conducted in a similar manner to **Example 38,** step c. An additional chiral SFC step afforded the title compounds.

**Example 243a:**

m/z ES+ $[M+H]^+$ 678.1; $^1$H NMR (400 MHz, $CDCl_3$) δ ppm 8.31 (s, 1H), 7.47 - 7.45 (m, 1H), 7.37 - 7.28 (m, 2H),

7.06 (s, 1H), 6.90 (s, 1H), 6.68 - 6.57 (m, 1H), 6.55 - 6.38 (m, 1H), 6.11-5.58 (m, 2H), 5.56 - 4.92 (m, 2H), 4.77 - 4.52 (m, 2H), 4.43 - 4.23 (m, 2H), 4.22 - 4.07 (m, 2H), 3.62 - 3.57 (m, 1H), 3.33 (s, 3H), 3.10 - 2.90 (m, 2H), 2.70 - 2.64 (m, 1H), 2.49 (s, 3H), 2.23 - 2.16 (m, 1H).

**Example 243b:**

m/z ES+ $[M+H]^+$678.1; $^1H$ NMR (400 MHz, $CDCl_3$) δ ppm 8.32 (s, 1H), 7.47 - 7.45 (m, 1H), 7.35 - 7.28 (m, 2H), 7.05 (s, 1H), 6.90 (s, 1H), 6.72 - 6.56 (m, 1H), 6.55 - 6.36 (m, 1H), 6.07-5.64 (m, 2H), 5.57 - 4.92 (m, 2H), 4.78 - 4.50 (m, 2H), 4.41 - 4.20 (m, 2H), 4.20 - 4.09 (m, 2H), 3.63 (d, *J* = 9.2 Hz, 1H), 3.31 (s, 3H), 3.09 - 2.91 (m, 2H), 2.6 - 2.65 (m, 1H), 2.48 (s, 3H), 2.23 - 2.16 (m, 1H).

**Example 244**

**(3a*R*,11a*S*)-5-((7-acryloyl-6-(hydroxymethyl)-5,6,7,8-tetrahydroimidazo[1,2-*a*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

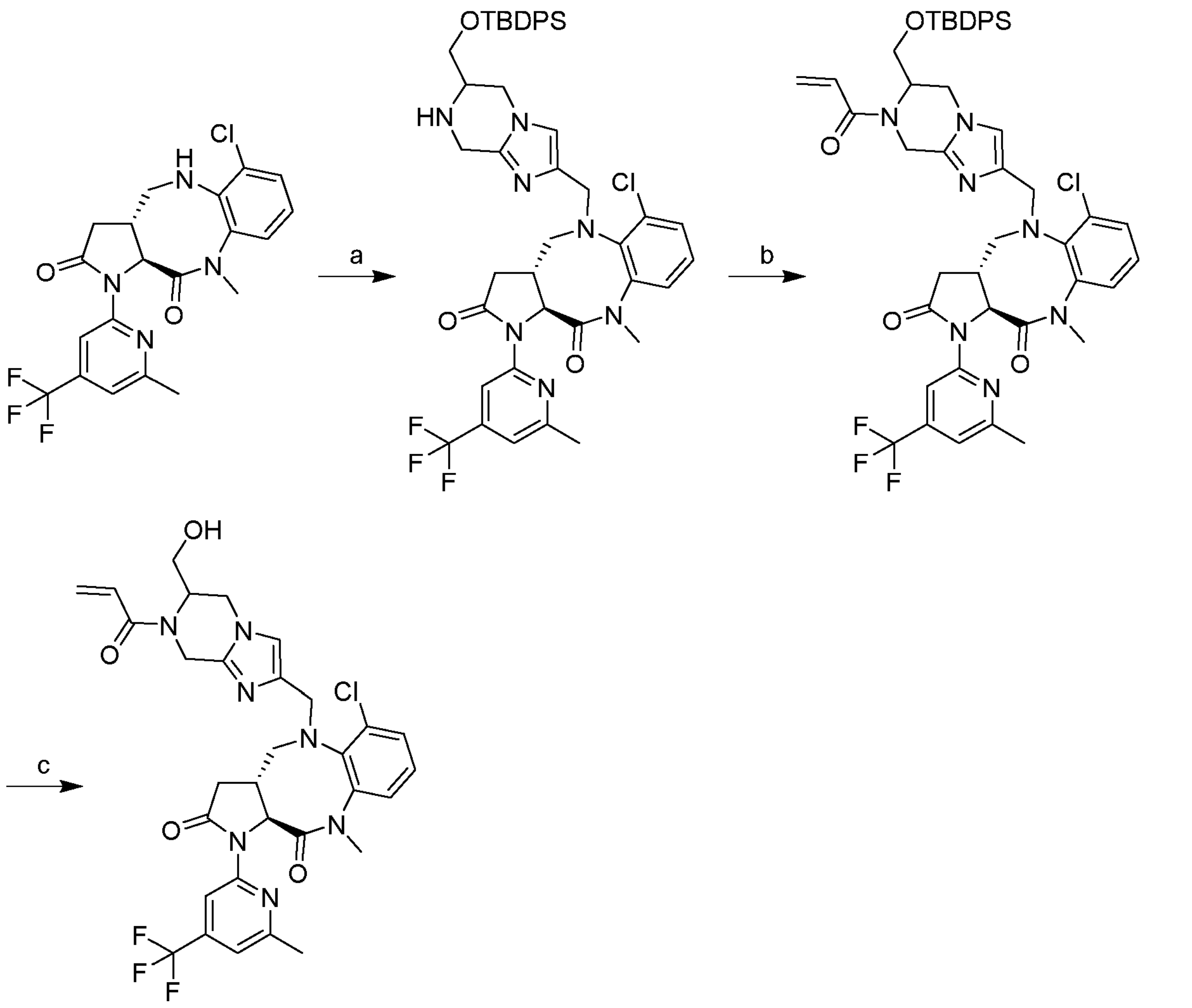


**Step a.** This step was conducted in a similar manner to **Example 39,** step a, using **Intermediate 5b** and **Intermediate B70.**

m/z ES+ $[M+H]^+$842.1; $^1H$ NMR (400 MHz, $CDCl_3$) δ ppm 8.31 (s, 1H), 7.67 - 7.65 (m, 4H), 7.46 - 7.39 (m, 7H), 7.35 - 7.28 (m, 3H), 7.05 (s, 1H), 6.80 (s, 1H), 4.70 - 4.63 (m, 1H), 4.35-4.22 (m, 1H), 4.20 - 4.09 (m, 2H), 4.05 - 4.01 (m, 1H), 3.94 - 3.85 (m, 1H), 3.84 - 3.69 (m, 3H), 3.68 - 3.56 (m, 1H), 3.34 (s, 3H), 3.29 - 3.21 (m, 1H), 3.08 - 2.95 (m, 2H), 2.74 - 2.63 (m, 1H), 2.49 (s, 3H), 2.25 - 2.13 (m, 1H), 1.08 (s, 9H).

Step b. This step was conducted in a similar manner to Example 38, step c.

m/z ES+ $[M+H]^+$ 896.1.

**Step c.** To a solution of (3a*R*,11a*S*)-5-((7-acryloyl-6-(((*tert*-butyldiphenylsilyl)oxy)methyl)-5,6,7,8-tetrahydroimidazo[1,2-*a*]pyrazin-2-yl)methyl)-6-chloro-10-methyl-1-(6-methyl-4-(trifluoromethyl)pyridin-2-yl)-1 ,3a,4,5, 1 0, 11a-hexahydro-2*H*-benzo[b]pyrrolo[2,3-*f*][1,4]diazocine-2,11(3*H*)-dione (5 mg, 0.006 mmol) in THF (0.5 mL) was added TBAF (1 M in THF, 7 μL). The reaction mixture was stirred at 20 °C for 3 h. Upon completion, the reaction mixture was diluted with water (2 mL) and extracted with a mixture of DCM/2-propanol (3/1; 3 x 1 mL). The combined organic layers were dried over $Na_2SO_4$, filtered and evaporated. The residue was purified by prep-HPLC to give the title compound (1 mg, 27% yield) as a brown solid.

m/z ES+ $[M+H]^+$ 658.2; $^1H$ NMR (400 MHz, $CDCl_3$) δ ppm 8.33 (s, 1H), 7.50 - 7.47 (m, 1H), 7.39 - 7.31 (m, 3H), 7.07 (s, 1H), 6.93 - 6.82 (m, 1H), 6.76 - 6.52 (m, 1H), 6.44 (d, *J* = 16.4 Hz, 1H), 5.96 - 5.78 (m, 1H), 5.46 - 5.27 (m, 1H), 5.25 - 4.94 (m, 1H), 4.83 - 4.46 (m, 2H), 4.44 - 4.22 (m, 2H), 4.19 - 3.97 (m, 2H), 3.77 - 3.70 (m, 2H), 3.67 - 3.57 (m, 1H), 3.34 (d, *J* = 3.2 Hz, 3H), 3.14 - 2.80 (m, 2H), 2.77 - 2.64 (m, 1H), 2.50 (s, 3H), 2.27 - 2.19 (m, 1H).

## Example 245

**(*E*)-*N*-(4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5H-benzo[b]pyrrolo[2,3-t][1,4]diazocin-5-yl)methyl)-2-fluorophenyl)-4-(dimethylamino)but-2-enamide**

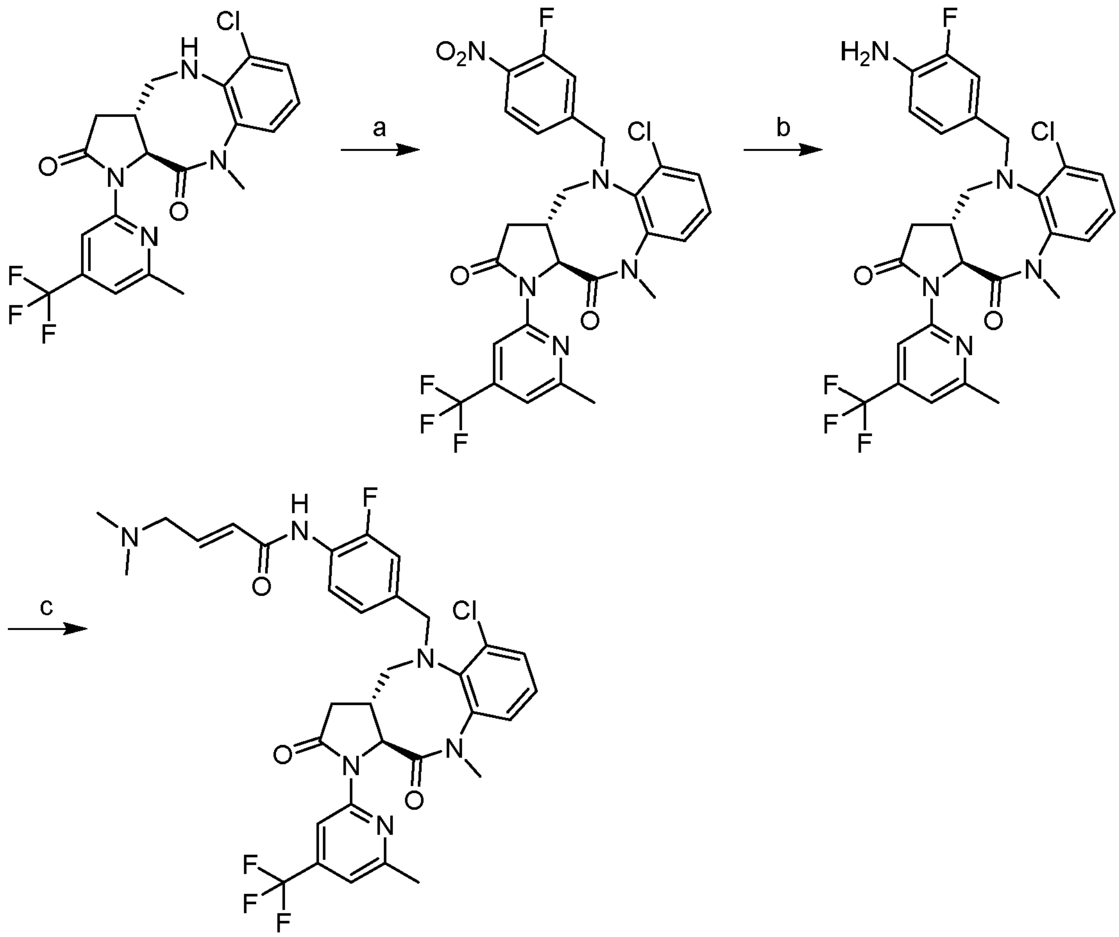


**Step a.** This step was conducted in a similar manner to **Example 87,** step a, using **Intermediate 5b** and 3-fluoro-4-nitrobenzaldehyde (CAS: 160538-51-2).

**Step b.** This step was conducted in a similar manner to **Example 87,** step c.

**Step c.** To a solution of (3a*R*,11a*S*)-5-(4-amino-3-fluorobenzyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (100 mg, 0.18

mmol) in pyridine (2.5 mL) was added $T_3P$ (340 mg, 0.53 mmol) and (*E*)-4-(dimethylamino)but-2-enoic acid hydrochloride (CAS: 848133-35-7; 35 mg, 0.21 mmol). The reaction mixture was stirred at 50 °C for 3 h. Upon completion, the mixture was diluted with water (10 mL) and extracted with EtOAc (3 x 5 mL). The combined organic layers were dried over $Na_2SO_4$, filtered and evaporated. The residue was purified by prep-HPLC to give the title compound (40 mg, 32% yield) as a pink solid.

m/z ES+ $[M+H]^+$673.1; $^1H$ NMR (400 MHz, $CDCl_3$) δ ppm 8.37 - 8.28 (m, 2H), 7.48 - 7.45 (m, 2H), 7.30 - 7.27 (m, 2H), 7.25 - 7.24 (m, 1H), 7.13 (d, *J* = 8.4 Hz, 1H), 7.05 (s, 1H), 7.01 - 6.97 (m, 1H), 6.20 (d, *J* = 15.2 Hz, 1H), 4.68 (d, *J* = 9.2 Hz, 1H), 4.23 - 4.16 (m, 1H), 4.12 - 4.06 (m, 1H), 3.51 - 3.44 (m, 1H), 3.36 (s, 3H), 3.19 (m, 2H), 3.04 - 2.91 (m, 2H), 2.67 (m, 1H), 2.48 (s, 3H), 2.34 (s, 6H), 2.22 - 2.15 (m, 1H).

**Example 246**

**(*E*)-*N*-(4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-3-fluorophenyl)-4-(dimethylamino)but-2-enamide**

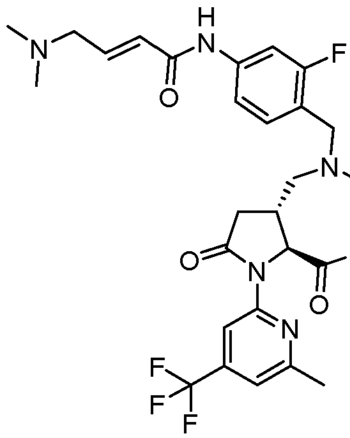

The title compound was prepared in a similar manner to **Example 245,** using 2-fluoro-4-nitrobenzaldehyde (CAS: 157701-72-9) in step a.

m/z ES+ $[M+H]^+$673.2; $^1H$ NMR (400 MHz, $CDCl_3$) δ ppm 8.43 (d, *J* = 11.6 Hz, 2H), 8.30 (s, 1H), 7.57 (d, *J* = 11.2 Hz, 1H), 7.47 - 7.44 (m, 1H), 7.29 (d, *J* = 4.8 Hz, 2H), 7.26 - 7.23 (m, 1H), 7.04 (s, 1H), 6.95 - 6.88 (m, 1H), 6.32 (d, *J* = 15.6 Hz, 1H), 4.64 (d, *J* = 9.2 Hz, 1H), 4.33 - 4.11 (m, 2H), 3.56 - 3.52 (m, 1H), 3.42 (d, *J* = 6.4 Hz, 2H), 3.24 (s, 3H), 3.00 (d, *J* = 14.0 Hz, 1H), 2.95 - 2.84 (m, 1H), 2.71 - 2.65 (m, 1H), 2.54 (s, 6H), 2.47 (s, 3H), 2.23 - 2.12 (m, 1H).

**Example 247**

**(*E*)-*N*-(3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)phenyl)-4-(dimethylamino) but-2-enamide**

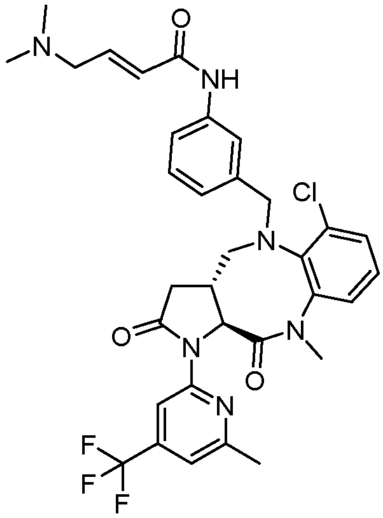

The title compound was prepared in a similar manner to **Example 245,** using 3-nitrobenzaldehyde in step a.

m/z ES+ $[M+H]^+$655.1; $^1H$ NMR (400 MHz, $CDCl_3$) δ ppm 8.33 (s, 1H), 7.75 (d, *J* = 7.6 Hz, 1H), 7.51 - 7.38 (m, 3H), 7.35 - 7.28 (m, 3H), 7.07 (s, 2H), 6.989 - 6.94 (m, 1H), 6.13 (d, *J* = 15.2 Hz, 1H), 4.66 (d, *J* = 9.6 Hz, 1H), 4.33 - 4.10 (m, 2H), 3.51 - 3.46 (m, 1H), 3.30 (s, 3H), 3.12 (d, *J* = 6.0 Hz, 2H), 3.07 - 2.98 (m, 1H), 2.96 - 2.84 (m, 1H), 2.68 - 2.61 (m, 1H), 2.50 (s, 3H), 2.28 (s, 6H), 2.20 - 2.13 (m, 1H).

**Example 248**

***N*-(4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-cyanophenyl)acrylamide**

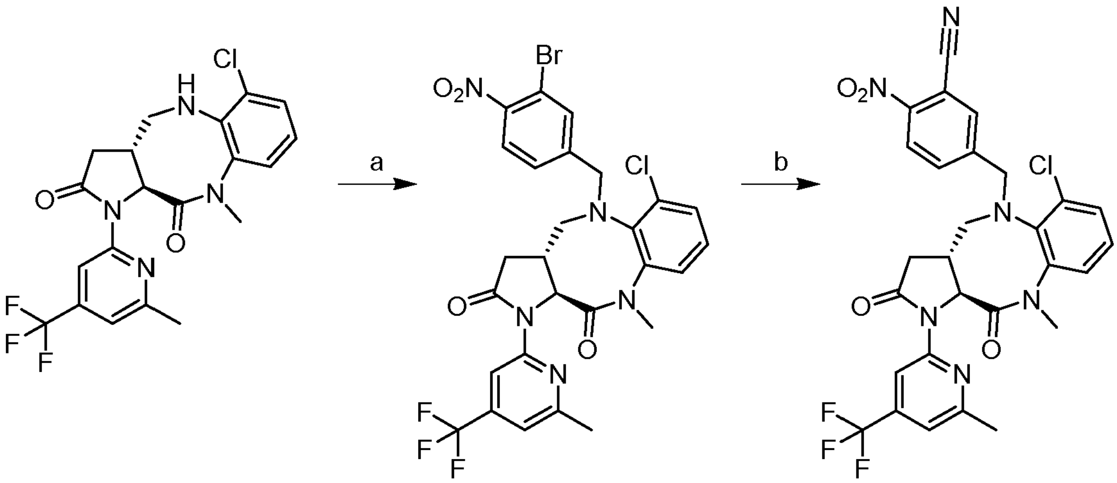

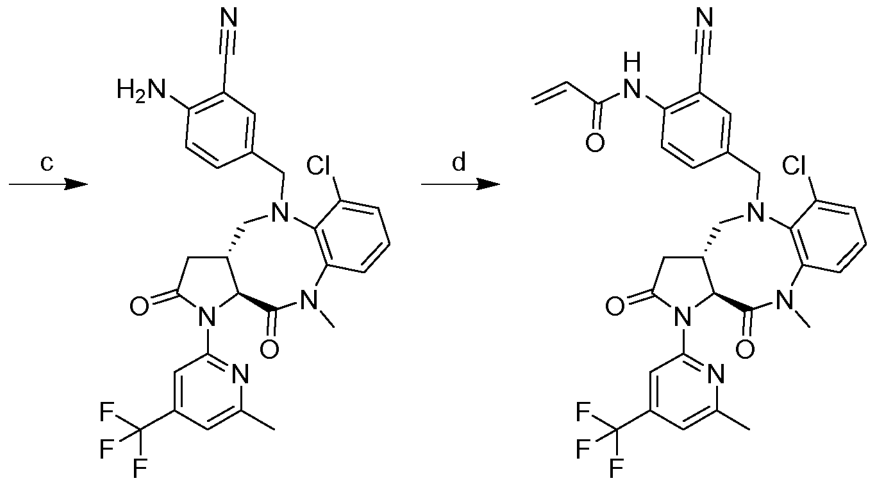


**Step a.** This step was conducted in a similar manner to **Example 87,** step a, using **Intermediate 5b** and 3-bromo-4-nitrobenzaldehyde (CAS: 101682-68-2).

**Step b.** To a solution of (3a*R*,11a*S*)-5-(3-bromo-4-nitrobenzyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (200 mg, 0.31 mmol) and $Zn(CN)_2$ (144 mg, 1.22 mmol) in DMA (1 mL) was added Pd(*t*-$Bu_3P)_2$ (CAS: 53199-31-8; 16 mg, 0.031 mmol). The mixture was purged with $N_2$ 3 times and stirred at 130 °C for 1 h under an $N_2$ atmosphere. Upon completion, the reaction mixture was diluted with water (10 mL) and extracted with EtOAc (3 x 10 mL). The combined organic layers were washed with brine (2 x 20 mL), dried over $Na_2SO_4$, and evaporated. The residue was purified by reverse phase flash (water (0.1% $NH_4OH$)/MeCN) to give 5-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-nitrobenzonitrile (110 mg, 60% yield) as a white solid.

m/z ES+ $[M+H]^+$ 599.2.

**Step c.** This step was conducted in a similar manner to **Example 87,** step c.

m/z ES+ $[M+H]^+$ 569.2.

**Step d.** This step was conducted in a similar manner to **Example 38,** step c.

m/z ES+ $[M+H]^+$623.1; $^1$H NMR (400 MHz, $CDCl_3$) δ ppm 8.49 (d, *J* = 8.8 Hz, 1H), 8.34 (s, 1H), 7.71 - 7.61 (m, 3H), 7.48 (t, *J* = 4.8 Hz, 1H), 7.31 (d, *J* = 4.4 Hz, 2H), 7.06 (s, 1H), 6.61-6.40 (m, 1H), 6.38 - 6.20 (m, 1H), 5.90 (d, *J* = 10.4 Hz, 1H), 4.69 (d, *J* = 9.6 Hz, 1H), 4.30-4.18 (m, 1H), 4.14 - 4.04 (m, 1H), 3.51 - 3.42 (m, 1H), 3.38 (s, 3H), 3.10 - 2.99 (m, 1H), 2.97 - 2.84 (m, 1H), 2.76 - 2.63 (m, 1H), 2.48 (s, 3H), 2.29 - 2.11 (m, 1H).

### Example 249

**(*E*)-4-(4-acetylpiperazin-1-yl)-*N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)but-2-enamide**

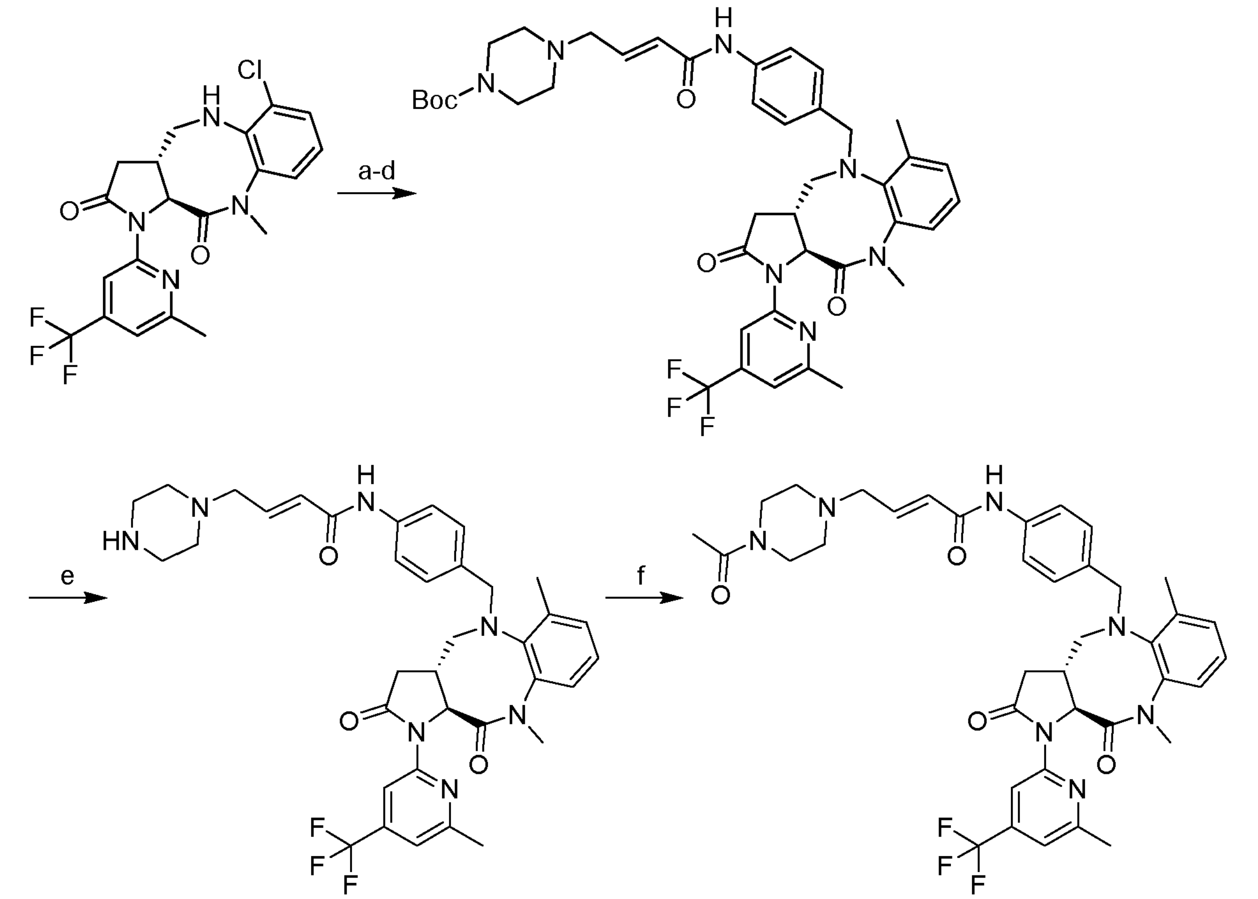


**Steps a-c.** These 3 steps were conducted as described in **Example 87,** steps a-c.

**Step d.** This step was conducted in a similar manner to **Example 87,** step d, using **Intermediate E10.**

m/z ES+ $[M+H]^+$ 776.2; $^1$H NMR (400 MHz, $CDCl_3$) $\delta$ ppm 8.33 (s, 1H), 7.61 - 7.51 (m, 2H), 7.35 - 7.30 (m, 3H), 7.27 - 7.23 (m, 4H), 7.05 (s, 1H), 7.03 - 6.93 (m, 1H), 4.77 (d, *J* = 9.6 Hz, 1H), 4.16 - 4.12 (m, 1H), 4.05 - 3.93 (m, 1H), 3.70 - 3.60 (m, 3H), 3.55 - 3.15 (m, 7H), 3.05-2.90 (m, 1H), 2.76 - 2.63 (m, 5H), 2.50 (d, *J* = 15.6 Hz, 7H), 2.21 - 2.10 (m, 1H), 1.49 (s, 9H). **Step e.** To a solution of *tert-butyl* 4-((E)-4-((4-(((3aR,11aS)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)amino)-4-oxobut-2-en-1-yl)piperazine-1-carboxylate (90 mg, 0.12 mmol) in DCM (5 mL) was added TFA (1.54 g, 13.5 mmol). The reaction mixture was stirred at 25 °C for 1 h. Upon completion, the reaction mixture was basified to pH 8 with sat. aq. $NaHCO_3$. The aqueous mixture was diluted with water (60 mL) and extracted with EtOAc (2 x 40mL). The combined organic layers were washed with brine (3 x 30mL), dried over $Na_2SO_4$ and evaporated to give (*E*)-*N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-4-(piperazin-1-yl)but-2-enamide (50 mg, 64% yield) as a white solid.

m/z ES+ $[M+H]^+$676.1.

**Step f.** A mixture of (*E*)-N-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-4-(piperazin-1-yl)but-2-enamide (50 mg, 0.074 mmol), acetic acid (9 mg, 0.16 mmol), HATU (37 mg, 0.096 mmol) and DIPEA (24 mg, 0.19 mmol) in DCM (1 mL) was stirred at 25 °C for 12 h under a $N_2$ atmosphere. Upon completion, the reaction mixture was evaporated. The residue was purified by reverse phase flash chromatography (water (0.1% TFA)/MeCN) followed by Prep-HPLC to give the title compound (27 mg, 47% yield) as a white solid.

m/z ES+ $[M+H]^+$ 718.4; $^1$H NMR (400 MHz, $CDCl_3$) $\delta$ ppm 8.32 (s, 1H), 7.56 (d, *J* = 8.0 Hz, 2H), 7.47 (s, 1H), 7.32 (d, *J* = 8.0 Hz, 2H), 7.25 (s, 3H), 7.05 (s, 1H), 7.01 - 6.92 (m, 1H), 6.17 (d, *J* = 15.2 Hz, 1H), 4.76 (d, *J* = 9.6 Hz, 1H), 4.13 (d, *J* = 13.6 Hz, 1H), 3.96 (d, *J* = 14.0 Hz, 1H), 3.68 (s, 2H), 3.58 - 3.48 (m, 2H), 3.46 - 3.36 (m, 4H), 3.24 (d, *J* = 5.6 Hz, 2H), 3.05 - 2.91 (m, 1H), 2.76 - 2.59 (m, 2H), 2.53 (d, *J* = 4.4 Hz, 2H), 2.50 (s, 5H), 2.46 (s, 3H), 2.20 - 2.08 (m, 4H).

**Example 250**

**(3a*R*,11a*S*)-5-((4-acryloyl-3,4-dihydro-2*H*-benzo[*b*][1,4]oxazin-6-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

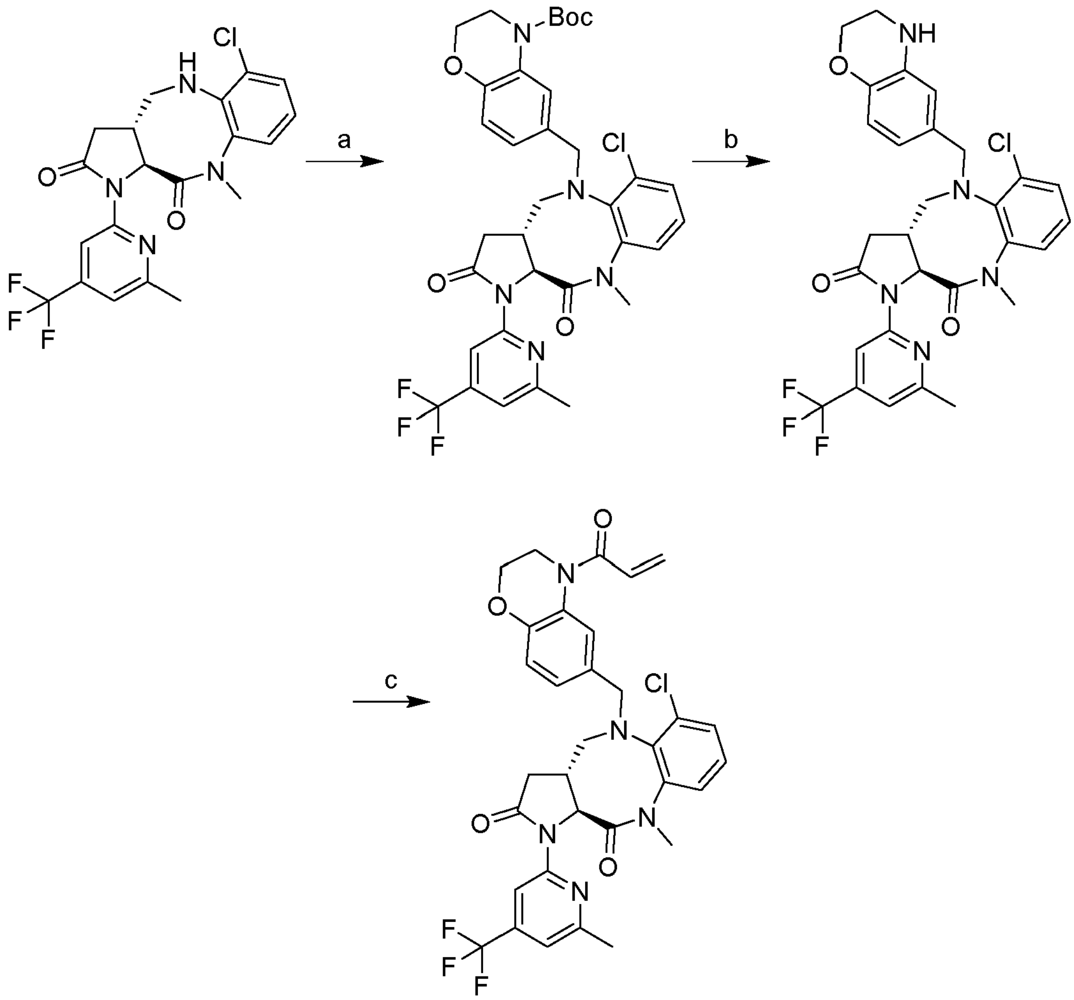


**Steps a.** This step was conducted in a similar manner to **Example 39,** step a, using **Intermediate 5b** and **Intermediate B75.**

m/z ES+ $[M+H]^+$686.3; $^1$H NMR (400 MHz, $CDCl_3$) δ ppm 8.32 (s, 1H), 7.83 (s, 1H), 7.50-7.40 (m, 1H), 7.33 - 7.27 (m, 2H), 7.11 - 7.01 (m, 2H), 6.85 (d, *J* = 8.4 Hz, 1H), 4.69 (d, *J* = 9.2 Hz, 1H), 4.24 (t, *J* = 4.4 Hz, 2H), 4.14 - 4.07 (m, 2H), 3.89 - 3.81 (m, 2H), 3.55 - 3.44 (m, 1H), 3.38 (s, 3H), 2.99 - 2.88 (m, 2H), 2.70 - 2.60 (m, 1H), 2.48 (s, 3H), 2.25 - 2.15 (m, 1H), 1.55 (s, 9H).

**Step b.** To a solution of *tert*-butyl 6-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2,3-dihydro-4*H*-benzo[*b*][1,4]oxazine-4-carboxylate (240 mg, 0.35 mmol) in DCM (12 mL) was added $ZnBr_2$ (788 mg, 3.5 mmol). The mixture was stirred at 20 °C for 1 h. Upon completion, the reaction mixture was diluted with water (10 mL) and extracted with EtOAc (3 x 5 mL). The combined organic layers were dried over $Na_2SO_4$, and evaporated to give (3a*R*,11a*S*)-6-chloro-5-((3,4-dihydro-2*H*-benzo[*b*][1,4]oxazin-6-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (200 mg, crude) as a light yellow solid.

m/z ES+ $[M+H]^+$586.2

**Step c.** This step was conducted in a similar manner to **Example 38,** step c.

m/z ES+ $[M+H]^+$ 640.1; $^1$H NMR (400 MHz, $CDCl_3$) δ ppm 8.33 (s, 1H), 7.51 - 7.43 (m, 1H), 7.34 - 7.28 (m, 2H), 7.23 (d, *J* = 7.6 Hz, 1H), 7.14 - 7.03 (m, 2H), 6.89 (d, *J* = 8.4 Hz, 1H), 6.72 - 6.61 (m, 1H), 6.57 - 6.49 (m, 1H), 5.82 - 5.79 (m, 1H), 4.68 (d, *J* = 8.8 Hz, 1H), 4.33 (t, *J* = 4.8 Hz, 2H), 4.18 (d, *J* = 14.0 Hz, 1H), 4.09 - 3.95 (m, 3H), 3.56 - 3.42 (m, 1H), 3.35 (s, 3H), 3.02 - 2.90 (m, 2H), 2.71 - 2.67 (m, 1H), 2.48 (s, 3H), 2.24 - 2.20 (m, 1H).

**Example 251**

**(3a*R*,11a*S*)-6-chloro-5-((4-((*E*)-4-(dimethylamino)but-2-enoyl)-3,4-dihydro-2*H*-benzo[*b*][1,4]oxazin-6-yl) methyl)-10-methyl-1-(6-methyl-4-(trifluoromethyl)pyridin-2-yl)-1,3a,4,5,10,11a-hexahydro-2*H*-benzo[*b*]pyrrolo [2,3-*f*][1,4]diazocine-2,11(3*H*)-dione**

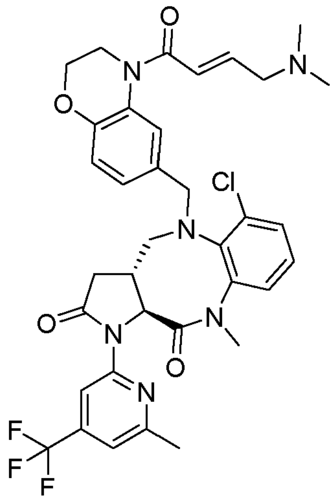

The title compound was prepared in a similar manner to **Example,** using **Intermediate 5b** and **Intermediate B75** in step a. The final step was conducted in a similar manner to the amide coupling described for **Example 27.**

m/z ES+ $[M+H]^+$ 697.1; $^1H$ NMR (400 MHz, $CDCl_3$) δ ppm 8.32 (s, 1H), 7.47 - 7.45 (m, 1H), 7.33 - 7.27 (m, 3H), 7.11 - 7.10 (m, 1H), 7.07 - 7.01 (m, 2H), 6.89 (d, *J* = 8.4 Hz, 1H), 6.60-6.53 (m, 1H), 4.69 (d, *J* = 9.2 Hz, 1H), 4.36 - 4.26 (m, 2H), 4.18 - 4.12 (m, 1H), 4.11 - 4.04 (m, 1H), 4.03 - 3.98 (m, 1H), 3.98 - 3.90 (m, 1H), 3.55 - 3.44 (m, 1H), 3.36 (s, 3H), 3.15 - 3.05 (m, 2H), 3.00 - 2.88 (m, 2H), 2.75 - 2.60 (m, 1H), 2.48 (s, 3H), 2.24 (s, 6H), 2.23 - 2.14 (m, 1H).

**Example 252**

**(*E*)-*N*-(4-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5H-benzo[*b*]pyrrolo[2,3-*f*][1,4]diazocin-5-yl)butyl)-4-(dimethylamino)but-2-enamide**

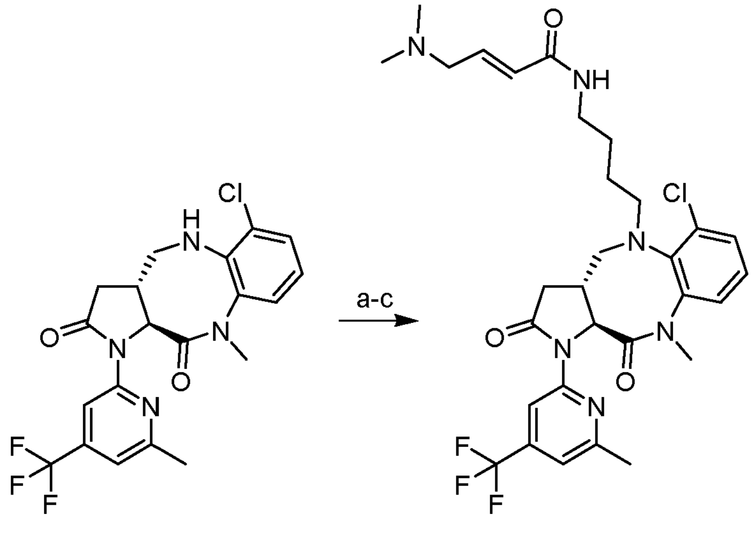

**Step a.** This step was conducted in a similar manner to **Example 83,** step a, using **Intermediate B82.**

**Steps b-c.** These two steps were conducted in a similar manner to **Example 83,** steps c-d. m/z ES+ $[M+H]^+$ 621.3; $^1H$ NMR (400 MHz, $CDCl_3$) δ ppm 8.36 (s, 1H), 7.47 - 7.45 (m, 1H), 7.34 - 7.30 (m, 2H), 7.08 (s, 1H), 6.85 - 6.78 (m, 1H), 5.96 (d, *J* = 15.6 Hz, 1H), 5.70 - 5.62 (m, 1H), 4.62 (d, *J* = 9.2 Hz, 1H), 3.55 - 3.45 (m, 1H), 3.38 (s, 3H), 3.35 - 3.25 (m, 2H), 3.18-3.12 (m, 1H), 3.06 (d, *J* = 5.6 Hz, 2H), 2.98 - 2.90 (m, 2H), 2.76 - 2.69 (m, 1H), 2.51 (s, 3H), 2.28 (s, 6H), 2.24 - 2.18 (m, 1H), 1.58 - 1.54 (m, 2H), 1.48 - 1.37 (m, 3H).

**Example 253**

**(*E*)-*N*-(5-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-4-fluoropentyl)-4-(dimethylamino)but-2-enamide**

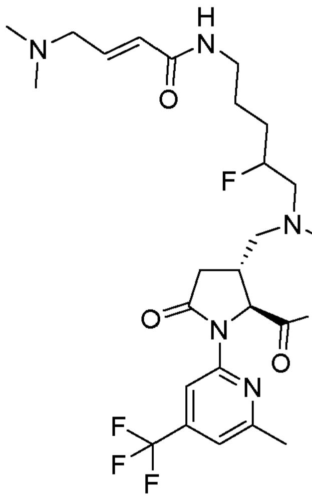

The title compound was prepared in a similar manner to **Example 252,** using **Intermediate B86** in step a.

m/z ES+ $[M+H]^+$ 653.2; $^1H$ NMR (400 MHz, $CDCl_3$) δ ppm 8.34 (s, 1H), 7.48 - 7.45 (m, 1H), 7.35 - 7.27 (m, 2H), 7.07 (s, 1H), 6.87 - 6.76 (m, 1H), 6.16 - 5.98 (m, 2H), 4.58 (d, *J* = 9.2 Hz, 1H), 4.53 - 4.32 (m, 1H), 3.62 - 3.44 (m, 2H), 3.43 - 3.29 (m, 4H), 3.29 - 2.89 (m, 6H), 2.77-2.66 (m, 1H), 2.50 (s, 3H), 2.26 - 2.15 (m, 7H), 1.83 - 1.74 (m, 2H), 1.67 - 1.42 (m, 2H).

**Example 254**

**(3a*R*,11a*S*)-5-(4-(1-((*Z*)-2-chloro-4-(dimethylamino)but-2-enoyl)azetidin-3-yl)butyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

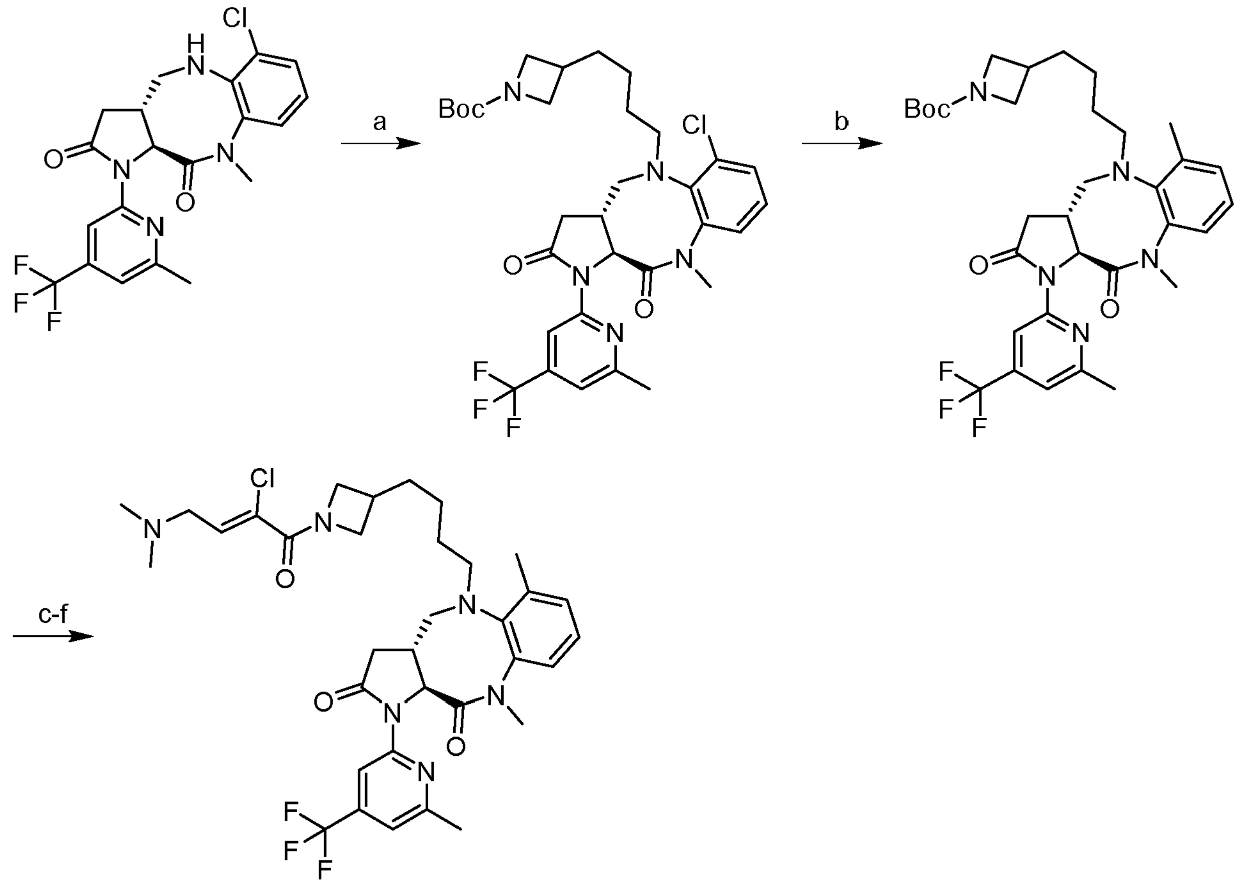


**Step a.** This step was conducted in a similar manner to **Example 79,** step a, using **Intermediate 5b** and **Intermediate B30.**

m/z ES+ $[M+H]^+$ 650.1; $^1$H NMR (400 MHz, $CDCl_3$) δ ppm 8.33 (s, 1H), 7.46 - 7.43 (m, 1H), 7.32 - 7.27 (m, 2H), 7.05 (s, 1H), 4.63 (d, *J* = 9.2 Hz, 1H), 4.00 - 3.92 (m, 2H), 3.58 - 3.45 (m, 3H), 3.36 (s, 3H), 3.05 - 2.87 (m, 4H), 2.85 - 2.70 (m, 1H), 2.48 (s, 3H), 2.46 - 2.39 (m, 1H), 2.25 - 2.20 (m, 1H), 1.56 - 1.49 (m, 2H), 1.43 (s, 9H), 1.39 - 1.30 (m, 2H), 1.29 - 1.25 (m, 2H). **Step b.** This step was conducted in a similar manner to **Example 77,** step b.

m/z ES+ $[M+H]^+$630.1.

**Steps c-f.** These 4 steps were conducted in a similar manner to **Example 79,** steps b-e, using **Intermediate E3** in step d.

m/z ES+ $[M+H]^+$675.3; $^1$H NMR (400 MHz, $CDCl_3$) δ ppm 8.33 (s, 1H) 7.16 - 7.26 (m, 3H) 7.04 (s, 1H) 6.71 - 6.63 (m, 1H) 4.70 (d, *J* = 9.2 Hz, 1H) 4.50 - 4.35 (m, 1H) 4.20 - 4.06 (m, 1H) 3.87 - 4.00 (m, 1H) 3.71 - 3.62 (m, 1H) 3.61 - 3.51 (m, 1H) 3.35 (s, 3H) 3.21 (d, *J* = 6.4 Hz, 2H) 2.84 - 3.01 (m, 3H) 2.67 - 2.83 (m, 2H) 2.51 - 2.61 (m, 1H) 2.49 (s, 3H) 2.37 (s, 3H) 2.28 (s, 6H) 2.23 - 2.14 (m, 1H) 1.51 - 1.63 (m, 2H) 1.22 - 1.39 (m, 4H).

**Example 255**

**(3a*R*,11a*S*)-5-((2-((*Z*)-2-chloro-4-(dimethylamino)but-2-enoyl)isoindolin-5-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

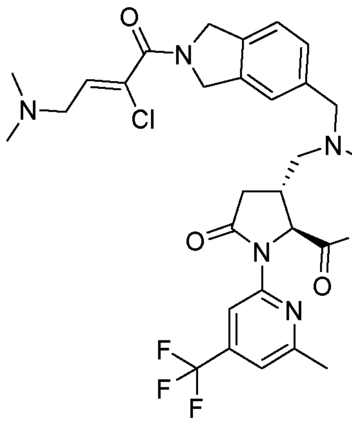

The title compound was prepared in a similar manner to **Example 254,** using **Intermediate 5b** and **Intermediate B18** in step a.

m/z ES+ $[M+H]^+$695.7; $^1$H NMR (400 MHz, $CDCl_3$) δ ppm 8.32 (s, 1H), 7.29 (s, 3H), 7.25 (s, 3H), 7.06 (s, 1H), 6.72 (t, *J* = 6.4 Hz, 1H), 4.97 (s, 2H), 4.88 (s, 2H), 4.77 - 4.74 (m, 1H), 4.20 - 4.15 (m, 1H), 4.08 - 3.99 (m, 1H), 3.94 (d, *J* = 6.8 Hz, 2H), 3.46 - 3.39 (m, 4H), 3.04-2.95 (m, 1H), 2.90 (s, 6H), 2.79 - 2.62 (m, 2H), 2.51 (s, 3H), 2.47 (d, *J* = 3.2 Hz, 3H), 2.20-2.09 (m, 1H).

**Example 256**

**(3a*R*,11a*S*)-5-(2-(4-acryloylpiperazin-1-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

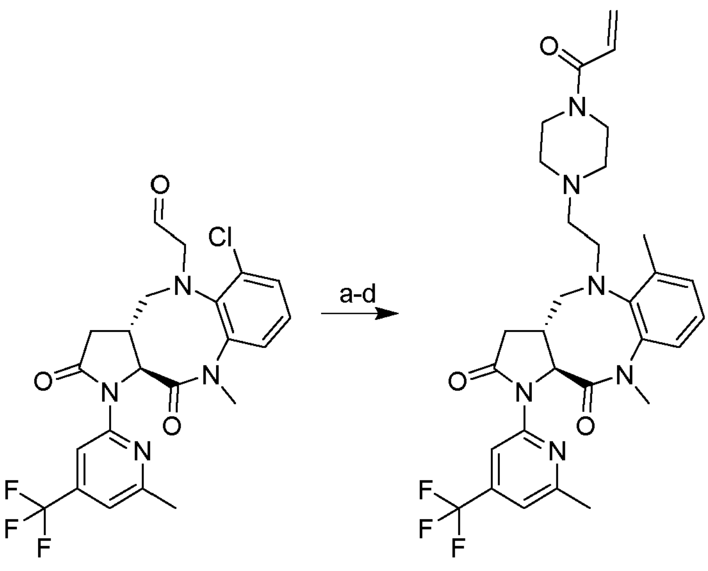


**Step a.** This step was conducted in a similar manner to **Example 1,** step a, using **Intermediate 7b** and *tert*-butyl piperazine-1-carboxylate.

m/z ES+ $[M+H]^+$ 651.3.

**Step b.** This step was conducted in a similar manner to **Example 77,** step b.

m/z ES+ $[M+H]^+$631.3.

**Steps c-d.** These 2 steps were conducted in a similar manner to **Example 1,** steps b-c.

m/z ES+ $[M+H]^+$ 585.3; $^1$H NMR (400 MHz, $CDCl_3$) δ ppm 8.33 (s, 1H), 7.26 - 7.18 (m, 3H), 7.05 (s, 1H), 6.61 - 6.46 (m, 1H), 6.29 (d, *J* = 16.0 Hz, 1H), 5.70 (d, *J* = 10.4 Hz, 1H), 4.70 (d, *J = 9.6* Hz, 1H), 3.79 - 3.44 (m, 5H), 3.38 (s, 3H), 3.30 - 3.03 (m, 2H), 3.03 - 2.92 (m, 1H), 2.86 - 2.77 (m, 1H), 2.76 - 2.68 (m, 1H), 2.49 (s, 3H), 2.49 - 2.40 (m, 4H), 2.40 (s, 3H), 2.39-2.26 (m, 2H), 2.24 - 2.15 (m, 1H).

**Example 257a: *N*-((*S*/*R*)-1-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)ethyl)-3,3-difluoropiperidin-4-yl)acrylamide and**

**Example 257b: *N*-((*R*/*S*)-1-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-3,3-difluoropiperidin-4-yl)acrylamide**

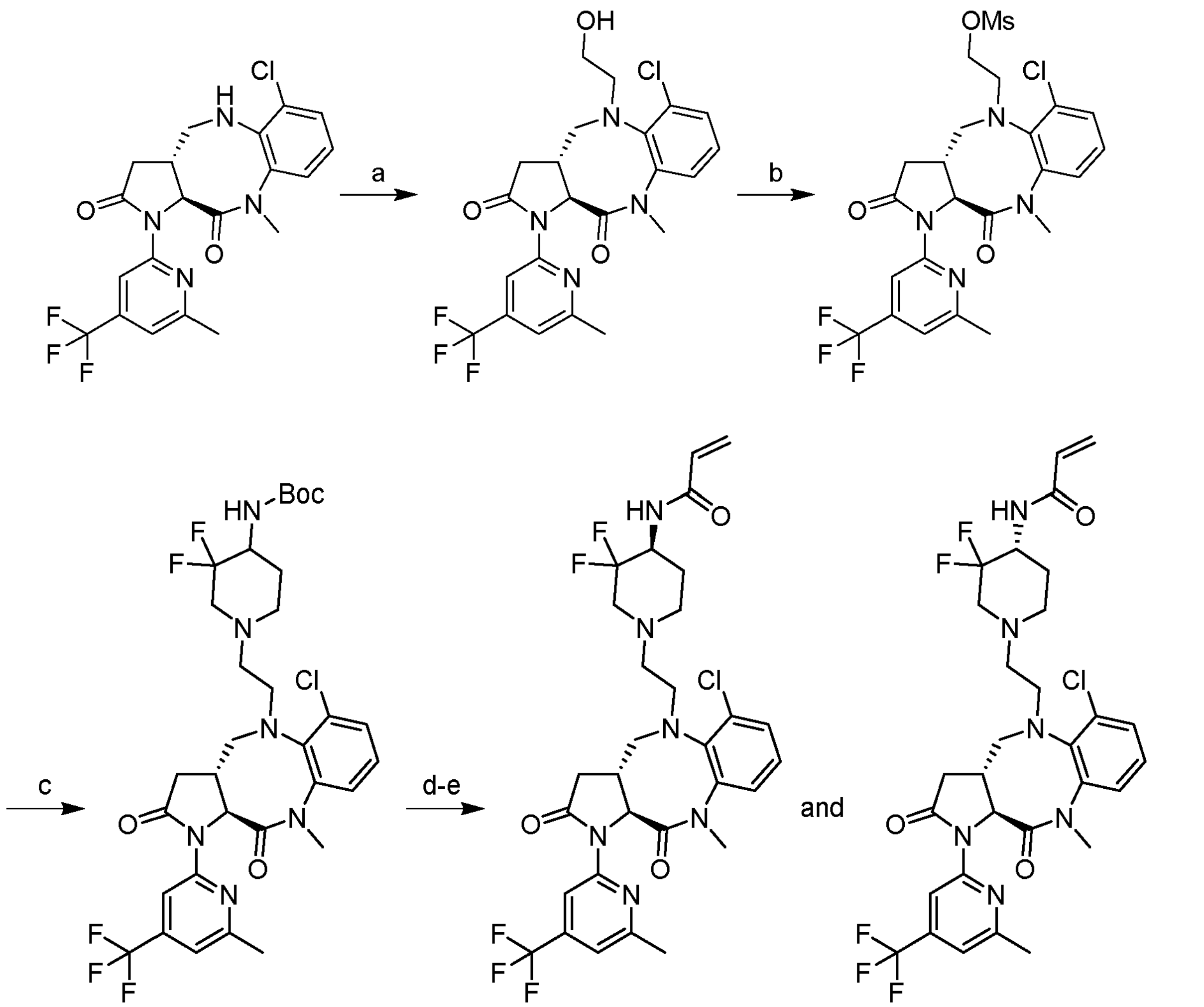


**Step a.** To a mixture of **Intermediate 5b** (2 g, 4.56 mmol), 1,4-dioxane-2,5-diol (CAS: 23147-58-2; 2.46 g, 2.05 mmol), 4 Å molecular sieves (1 g) and acetic acid (1.37 g, 22.8 mmol) in MeOH (20 mL) was added $NaBH_3CN$ (1.00 g, 16.0 mmol) at 0 °C. The reaction mixture was stirred at 20 °C for 16 h under a $N_2$ atmosphere. Upon completion, the reaction mixture was filtered and evaporated. The residue was purified by column chromatography (PE/EtOAc = 1/2) to give (3a*R*,11a*S*)-6-chloro-5-(2-hydroxyethyl)-10-methyl-1-(6-methyl-4-(trifluoromethyl)pyridin-2-yl)-1 ,3a,4,5, 1 0, 11a-hexahydro-2*H*-benzo[b]pyrrolo[2,3-*f*][1,4]diazocine-2,11(3*H*)-dione (1.7 g, 77% yield) as light yellow solid.

m/z ES+ $[M+H]^+$ 483.0; $^1$H NMR (400 MHz, $CDCl_3$) δ ppm 8.34 (s, 1H), 7.49 - 7.47 (m, 1H), 7.37 - 7.30 (m, 2H), 7.06 (s, 1H), 4.67 (d, *J* = 9.2 Hz, 1H), 3.67 - 3.55 (m, 2H), 3.51 - 3.42 (m, 1H), 3.39 (s, 3H), 3.31 - 3.19 (m, 1H), 3.10 - 3.06 (m, 1H), 3.05 - 2.91 (m, 2H), 2.80 - 2.71 (m, 1H), 2.49 (s, 4H), 2.50 - 2.26 (m, 1H).

**Step b.** A mixture of (3a*R*,11a*S*)-6-chloro-5-(2-hydroxyethyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (400 mg, 0.83 mmol), methanesulfonic anhydride (289 mg, 1.66 mmol) and TEA (838 mg, 8.28 mmol) in DCM (5 mL) was stirred at 25 °C for 3 h under a $N_2$ atmosphere. Upon completion, the reaction mixture was washed with water (3 x 15 mL) and brine (15 mL), dried over $Na_2SO_4$ and evaporated to give 2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)ethyl methanesulfonate (0.45 g, crude) as a yellow oil, which was used without further purification.

$^1$H NMR (400 MHz, $CDCl_3$) δ ppm 8.26 (s, 1H), 7.41 - 7.39 (m, 1H), 7.34 - 7.24 (m, 2H), 6.99 (s, 1H), 4.54 (d, *J* = 9.6 Hz, 1H), 4.15 - 4.05 (m, 1H), 4.03 - 3.93 (m, 1H), 3.55 - 3.48 (m, 1H), 3.44 - 3.34 (m, 3H), 3.32 (s, 3H), 2.98 (s, 3H), 2.91 - 2.81 (m, 1H), 2.73 (s, 3H), 2.67 - 2.65 (m, 1H), 2.18 - 2.11 (m, 1H).

**Step c.** A mixture of 2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl methanesulfonate (300 mg, 0.54 mmol), *tert-butyl* (3,3-difluoropiperidin-4-yl)carbamate (CAS: 1255666-48-8; 316 mg, 1.34 mmol) and TEA (162 mg, 1.60 mmol) in EtOH (1 mL) was stirred at 80 °C for 1 h under a $N_2$ atmosphere. Upon completion, the reaction mixture was evaporated. The residue was redissolved with EtOAc (15 mL) and washed with water (20 mL) and brine (3 x 15 mL), dried over $Na_2SO_4$ and evaporated. The residue was purified by column chromatography (PE/EtOAc = 3/1 to 1/1) to give *tert-butyl* (1-(2-((3a*R*,11aiS)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-3,3-difluoropiperidin-4-yl)carbamate (200 mg, 53% yield) as a white solid.

m/z ES+ $[M+H]^+$ 701.3; $^1$H NMR (400 MHz, $CDCl_3$) δ ppm 8.26 (s, 1H), 7.38 - 7.36 (m, 1H), 7.27 - 7.21 (m, 2H), 6.97 (s, 1H), 4.76 - 4.65 (m, 2H), 4.52 (t, *J* = 9.2 Hz, 1H), 3.56 - 3.44 (m, 1H), 3.29 (d, *J* = 5.2 Hz, 3H), 3.23 - 3.13 (m, 2H), 3.06 (t, *J* = 6.8 Hz, 1H), 3.02 - 2.96 (m, 2H), 2.95 - 2.84 (m, 2H), 2.82 - 2.72 (m, 2H), 2.64 - 2.54 (m, 2H), 2.41 (d, *J* = 1.2 Hz, 3H), 2.39-2.34 (m, 1H), 2.17 - 2.07 (m, 2H), 1.39 (s, 9H).

**Steps d-e.** These two steps were conducted in a similar manner to **Example 37a** and **Example 37b,** steps c-d.

**Example 257a:**

m/z ES+ $[M+H]^+$ 655.2; $^1$H NMR (400 MHz, $CDCl_3$) δ ppm 8.36 (s, 1H), 7.48 - 7.46 (m, 1H), 7.34 - 7.31 (m, 2H), 7.07 (s, 1H), 6.38 - 6.33 (m, 1H), 6.19 - 6.10 (m, 1H), 5.77 - 5.69 (m, 2H), 4.63 (d, *J* = 9.2 Hz, 1H), 4.41 - 4.23 (m, 1H), 3.64 (d, *J* = 9.6 Hz, 1H), 3.39 (s, 3H), 3.21 - 3.14 (m, 2H), 3.08 - 2.88 (m, 4H), 2.77 - 2.73 (m, 1H), 2.50 (s, 3H), 2.47 - 2.33 (m, 2H), 2.29 - 2.15 (m, 3H), 2.08 - 1.97 (m, 1H), 1.70 - 1.61 (m, 1H).

**Example 257b:**

m/z ES+ $[M+H]^+$ 655.2; $^1$H NMR (400 MHz, $CDCl_3$) δ ppm 8.35 (s, 1H), 7.49 - 7.46 (m, 1H), 7.37 - 7.31 (m, 2H), 7.07 (s, 1H), 6.38 - 6.34 (m, 1H), 6.20 - 6.12 (m, 1H), 5.79 - 5.72 (m, 2H), 4.60 (d, *J* = 9.6 Hz, 1H), 4.40 - 4.25 (m, 1H), 3.60 - 3.56 (m, 1H), 3.40 (s, 3H), 3.36 - 3.18 (m, 2H), 3.16 - 2.96 (m, 3H), 2.94 - 2.88 (m, 1H), 2.75 - 2.71 (m, 1H), 2.51 (s, 3H), 2.48 - 2.32 (m, 2H), 2.30 - 2.17 (m, 3H), 2.05 - 1.97 (m, 1H), 1.74 - 1.68 (m, 1H).

**Example 258a: 2-((*R*/*S*)-1-acryloyl-4-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)piperazin-2-yl)acetonitrile and**

**Example 258b: 2-((*S*/*R*)-1-acryloyl-4-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)piperazin-2-yl)acetonitrile**

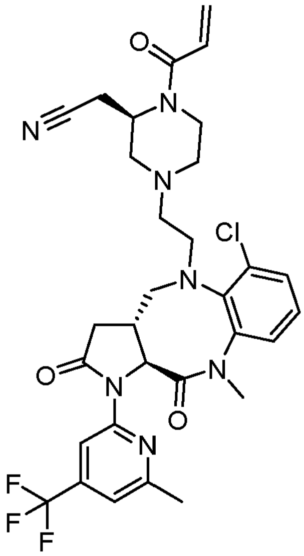

and

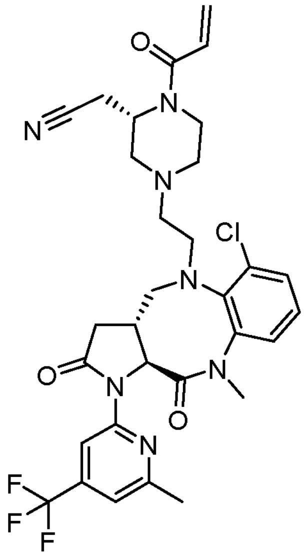

The title compound was prepared in a similar manner to **Example 257a** and **Example 257b,** using *tert*-butyl 2-(cyanomethyl)piperazine-1-carboxylate (CAS: 1808997-73-0) in step c.

**Example 258a:**

m/z ES+ $[M+H]^+$ 644.3; $^1H$ NMR (400 MHz, $CDCl_3$) $\delta$ ppm 8.35 (s, 1H), 7.48 - 7.43 (m, 1H), 7.35 - 7.29 (m, 2H), 7.06 (s, 1H), 6.61 - 6.43 (m, 1H), 6.39 - 6.27 (m, 1H), 5.82 - 5.68 (m, 1H), 5.06 - 4.87 (m, 1H), 4.64 (d, $J$ = 9.2 Hz, 1H), 3.82 - 3.71 (m, 1H), 3.65 - 3.53 (m, 1H), 3.40 (s, 3H), 3.27 - 3.05 (m, 3H), 3.03 - 2.89 (m, 4H), 2.79 - 2.72 (m, 2H), 2.69 - 2.58 (m, 1H), 2.48 (s, 3H), 2.46 - 2.32 (m, 2H), 2.30 - 2.15 (m, 2H), 2.13 - 1.95 (m, 1H).

**Example 258b:**

m/z ES+ $[M+H]^+$ 644.3; $^1H$ NMR (400 MHz, $CDCl_3$) $\delta$ ppm 8.34 (s, 1H), 7.53 - 7.47(m, 1H), 7.38 - 7.30 (m, 2H), 7.06 (s, 1H), 6.63 - 6.45 (m, 1H), 6.39 - 6.29 (m, 1H), 5.76 (d, $J$ = 10.4 Hz, 1H), 5.03 - 4.86 (m, 1H), 4.63 (d, $J$ = 8.8 Hz, 1H), 3.88 - 3.71 (m, 1H), 3.61 (d, $J$ = 9.6 Hz, 1H), 3.41 (s, 3H), 3.25 - 3.15 (m, 2H), 3.07 - 2.88 (m, 5H), 2.87 - 2.81 (m, 1H), 2.66 - 2.53 (m, 1H), 2.49 (s, 3H), 2.46 - 2.34 (m, 2H), 2.32 - 2.12 (m, 3H), 1.96 - 1.81 (m, 1H).

**Example 259**

**(3a*R*,11a*S*)-5-(2-(5-acryloyl-5,6-dihydropyrrolo[3,4-*d*]imidazol-1(4*H*)-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

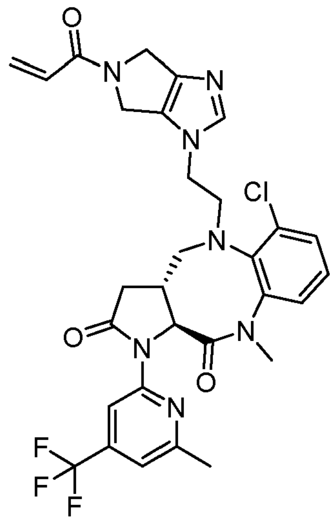

The title compound was prepared in a similar manner to **Example 257a** and **Example 257b,** using **Intermediate C5** in step c.

m/z ES+ $[M+H]^+$ 628.2; $^1$H NMR (400 MHz, $CDCl_3$) δ ppm 8.34 (s, 1H), 7.48 - 7.44 (m, 1H), 7.36 (s, 2H), 7.34 (d, *J* = 1.6 Hz, 1H), 7.06 (s, 1H), 6.55 - 6.44 (m, 2H), 5.84 - 5.75 (m, 1H), 4.67 - 4.60 (m, 4H), 4.59 - 4.54 (m, 1H), 3.97 - 3.84 (m, 2H), 3.56 - 3.46 (m, 1H), 3.38 (s, 3H), 3.35 - 3.30 (m, 1H), 3.09 - 2.98 (m, 1H), 2.95 - 2.83 (m, 1H), 2.80 - 2.72 (m, 1H), 2.48 (s, 3H), 2.27 - 2.21 (m, 1H), 2.08 - 1.96 (m, 1H).

## Example 260

**(3a*R*,11a*S*)-5-(2-((*S*)-4-acryloyl-2-methylpiperazin-1-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

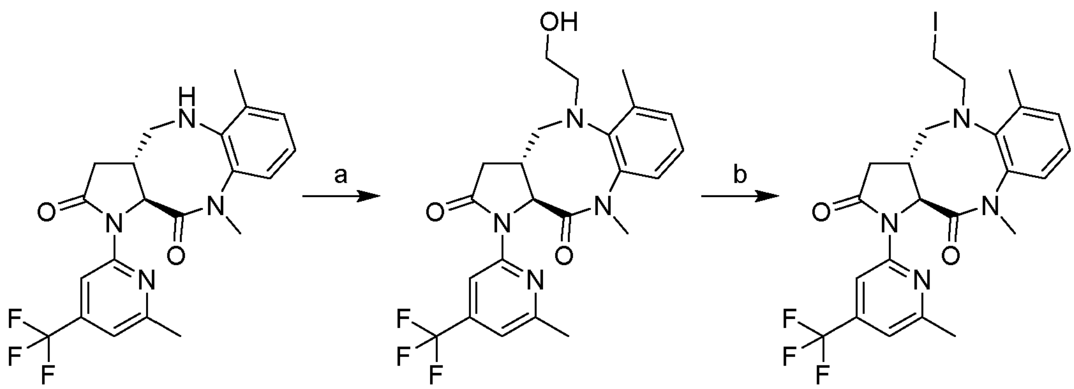

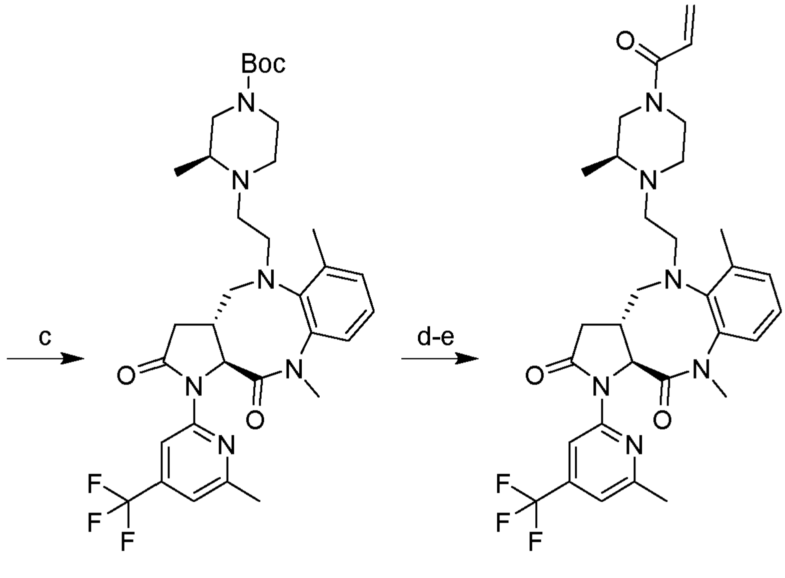


**Step a.** This step was conducted in a similar manner to **Example 257a** and **Example 257b**, step a, using **Intermediate 6c**.

m/z ES+ $[M+H]^+$ 463.1; $^1H$ NMR (400 MHz, $CDCl_3$) δ ppm 8.33 (s, 1H), 7.27 - 7.22 (m, 3H), 7.05 (s, 1H), 4.71 (d, *J* = 9.6 Hz, 1H), 3.69 - 3.48 (m, 3H), 3.37 (s, 3H), 3.25 - 3.15 (m, 1H), 3.15 - 3.06 (m, 1H), 3.05 - 2.92 (m, 1H), 2.91 - 2.81 (m, 1H), 2.74 (dd, *J* = 16.8, 8.4 Hz, 1H), 2.50 (s, 3H), 2.42 (s, 3H), 2.21 (dd, *J* = 16.8, 11.2 Hz, 1H), 2.06 (br s, 1H).

**Step b.** To a solution of (3a*R*,11a*S*)-5-(2-hydroxyethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (1.1 g, 2.38 mmol) in DCM (12 mL) was added imidazole (259 mg, 3.81 mmol), $PPh_3$ (936 mg, 3.57 mmol) and $I_2$ (905 mg, 3.57 mmol). The mixture was stirred at 25 °C for 12 h under a $N_2$atmosphere. Upon completion, the mixture was diluted with water (40 mL) and extracted with DCM (2 x 40 mL). The combined organic layers were washed with brine (40 mL), dried over $Na_2SO_4$ and evaporated. Purification by column chromatography (PE/EtOAc = 3/1) afforded (3a*R*,11a*S*)-5-(2-iodoethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (0.74 g, 54% yield) as a white solid.

m/z ES+ $[M+H]^+$ 573.2; $^1H$ NMR (400 MHz, $CDCl_3$) δ ppm 8.34 (s, 1H), 7.47 (d, *J* = 2.8 Hz, 1H), 7.26 - 7.23 (m, 2H), 7.05 (s, 1H), 4.71 (d, *J* = 9.2 Hz, 1H), 3.6 - 3.7 (m, 1H), 3.39 (s, 3H), 3.38 - 3.33 (m, 1H), 3.29 - 3.20 (m, 1H), 3.15 - 3.00 (m, 2H), 3.00 - 2.89 (m, 1H), 2.87 - 2.70 (m, 2H), 2.49 (s, 3H), 2.46 (s, 3H), 2.1 - 2.3 (m, 1H).

**Step c.** To a solution of (3a*R*,11a*S*)-5-(2-iodoethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (180 mg, 0.31 mmol) in MeCN (2 mL) was added DIPEA (81 mg, 0.63 mmol) and *tert*-butyl (*S*)-3-methylpiperazine-1-carboxylate (CAS: 147081-29-6; 95 mg, 0.47 mmol). The mixture was stirred at 60 °C for 12 h. Upon completion, water (30 ml) was added and the aqueous mixture was extracted with EtOAc (3 x 30 ml). The combined organic layers were washed with brine (75 mL), dried over $Na_2SO_4$ and evaporated. Purification by column chromatography (EtOAc) afforded *tert*-butyl (*S*)-4-(2-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-3-methylpiperazine-1-carboxylate (0.2 g, 91% yield) as a white solid.

m/z ES+ $[M+H]^+$645.4; $^1H$ NMR (400 MHz, $CDCl_3$) δ ppm 8.33 (s, 1H), 7.26 - 7.19 (m, 3H), 7.06 - 7.03 (m, 1H), 4.70 (d, *J* = 9.6 Hz, 1H), 3.77 - 3.51 (m, 3H), 3.38 (s, 3H), 3.27 - 2.94 (m, 4H), 2.91 - 2.66 (m, 5H), 2.49 (s, 3H), 2.40 (s, 3H), 2.37 - 2.27 (m, 1H), 2.24 - 2.08 (m, 3H), 1.45 (s, 9H), 0.90 (d, *J* = 6.4 Hz, 3H).

**Steps d-e.** These 2 steps were conducted in a similar manner to **Example 1,** steps b-c.

m/z ES+ $[M+H]^+$ 599.3; $^1H$ NMR (400 MHz, $CDCl_3$) δ ppm 8.34 (s, 1H), 7.27 - 7.20 (m, 3H), 7.06 (s, 1H), 6.61 - 6.50 (m, 1H), 6.24 - 6.35 (m, 1H), 5.70 (d, *J* = 10.8 Hz, 1H), 4.71 (d, *J* = 9.6 Hz, 1H), 4.15 - 3.86 (m, 1H), 3.77 - 3.54 (m, 2H), 3.39 (s, 4H), 3.18 - 2.63 (m, 8H), 2.51 (s, 3H), 2.41 (s, 4H), 2.27 - 2.14 (m, 3H), 0.94 (s, 3H).

**Example 261**

**(3a*R*,11a*S*)-5-(2-(3-acryloyl-3,6-diazabicyclo[3.1.1]heptan-6-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

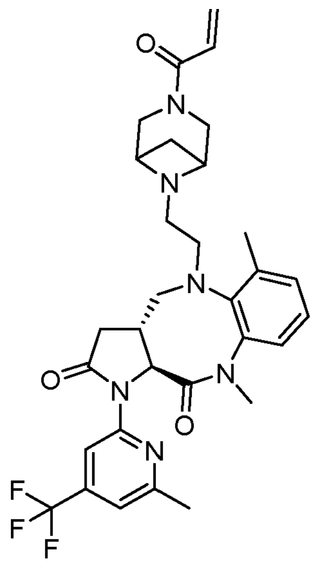

The title compound was prepared in a similar manner to **Example 260,** using *tert*-butyl 3,6-diazabicyclo[3.1.1] heptane-3-carboxylate (CAS: 1251017-66-9) in step c.

m/z ES+ $[M+H]^+$ 597.2; $^1H$ NMR (400 MHz, $CDCl_3$) δ ppm 8.32 (s, 1H), 7.25 - 7.17 (m, 3H), 7.04 (s, 1H), 6.62 - 6.51 (m, 1H), 6.49 - 6.39 (m, 1H), 5.7 - 5.85 (m, 1H), 4.65 - 4.75 (m, 1H), 3.78 (d, *J* = 11.2 Hz, 1H), 3.69 - 3.50 (m, 6H), 3.35 (d, *J* = 8.8 Hz, 3H), 3.07 - 2.85 (m, 3H), 2.84 - 2.67 (m, 2H), 2.62 - 2.51 (m, 1H), 2.48 (s, 3H), 2.47 - 2.43 (m, 1H), 2.41 (s, 1H), 2.37 (s, 3H), 2.1 - 2.25 (m, 1H), 1.48 (d, *J* = 8.8 Hz, 1H).

**Example 262**

**(3a*R*,11a*S*)-5-(2-(3-acryloyl-3,8-diazabicyclo[3.2.1]octan-8-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

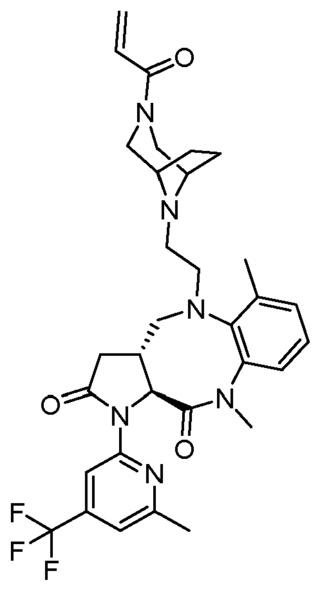

The title compound was prepared in a similar manner to **Example 260,** using *tert*-butyl 3,8-diazabicyclo[3.2.1] octane-3-carboxylate (CAS: 201162-53-0) in step c.

m/z ES+ $[M+H]^+$611.2; $^1H$ NMR (400 MHz, $CDCl_3$) δ ppm 8.33 (s, 1H), 7.26 - 7.19 (m, 3H), 7.05 (s, 1H), 6.55 - 6.44 (m, 1H), 6.2 - 6.35 (m, 1H), 5.67 (d, *J* = 10.4 Hz, 1H), 4.74 - 4.67 (m, 1H), 4.23 (d, *J* = 13.2 Hz, 1H), 3.72 - 3.49 (m, 2H), 3.38 (d, *J* = 3.6 Hz, 3H), 3.35 - 3.21 (m, 1H), 3.20 - 2.92 (m, 5H), 2.91 - 2.66 (m, 3H), 2.49 (s, 3H), 2.41 (s, 3H), 2.35 (d, *J* = 6.0 Hz, 2H), 2.15 - 2.25 (m, 1H), 1.84 (s, 2H), 1.62 - 1.47 (m, 2H).

**Example 263**

**(3a*R*,11a*S*)-5-(2-((1*R*,4*R*)-5-acryloyl-2,5-diazabicyclo[2.2.1]heptan-2-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

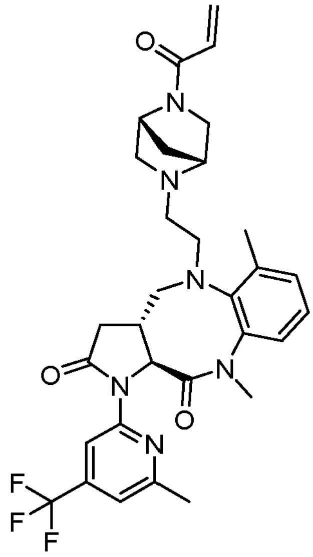

The title compound was prepared in a similar manner to **Example 260,** using *tert*-butyl (1*R*,4*R*)-2,5-diazabicyclo [2.2.1]heptane-2-carboxylate (CAS: 134003-84-2) in step c.

m/z ES+ $[M+H]^+$ 597.2; $^1H$ NMR (400 MHz, $CDCl_3$) δ ppm 8.33 (s, 1H), 7.26 - 7.18 (m, 3H), 7.04 (s, 1H), 6.48 - 6.26 (m, 2H), 5.69 (dd, *J* = 9.6, 2.4 Hz, 1H), 4.81 - 4.75 (m, 1H), 4.69 (dd, *J* = 9.6, 4.8 Hz, 1H), 3.70 - 3.46 (m, 3H), 3.36 (d, *J* = 9.2 Hz, 4H), 3.08 - 2.76 (m, 5H), 2.72 (dd, *J* = 16.8, 8.4 Hz, 1H), 2.66 - 2.53 (m, 2H), 2.49 (s, 3H), 2.49 - 2.39 (m, 1H), 2.37 (s, 3H), 2.24 - 2.13 (m, 1H), 1.68 - 1.61 (m, 2H).

**Example 264**

**(3a*R*,11a*S*)-6,10-dimethyl-1-(6-methyl-4-(trifluoromethyl)pyridin-2-yl)-5-(2-(4-propioloylpiperazin-1-yl)ethyl)-1,3a,4,5,10,11a-hexahydro-2*H*-benzo[*b*]pyrrolo[2,3-*f*][1,4]diazocine-2,11(3*H*)-dione**

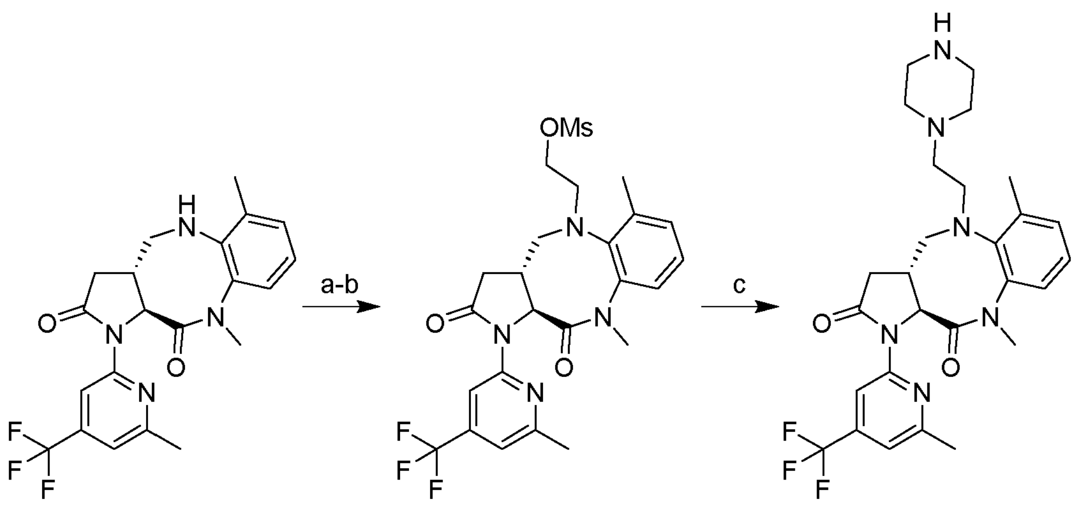

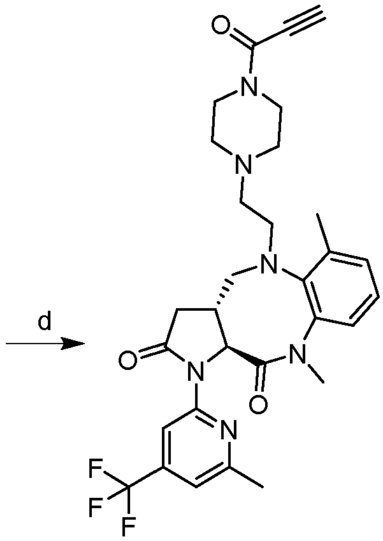


**Steps a-b.** These 2 steps were conducted in a similar manner to **Example 257a** and **Example 257b,** steps a-b, using **Intermediate 6c** in step a.

m/z ES+ $[M+H]^+$ 541.1; $^1$H NMR (400 MHz, $CDCl_3$) δ ppm 8.33 (s, 1H), 7.27 - 7.22 (m, 3H), 7.05 (s, 1H), 4.71 (d, *J* = 9.6 Hz, 1H), 4.15 - 4.10 (m, 1H), 3.68 - 3.50 (m, 3H), 3.39 - 3.30 (m, 4H), 3.21 - 3.02 (m, 1H), 3.00 (s, 3H), 2.96 - 2.73 (m, 2H), 2.49 (s, 3H), 2.42 (s, 3H), 2.21 (dd, *J* = 16.8, 11.4 Hz, 1H).

**Step c.** A solution of 2-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl methanesulfonate (800 mg, 1.48 mmol) and piperazine (1.27 g, 14.8 mmol) in MeCN (10 mL) was stirred at 50 °C for 12 h. Upon completion, the mixture was evaporated. The residue was purified by prep-HPLC to give (3a*R*,11a*S*)-6,10-dimethyl-1-(6-methyl-4-(trifluoromethyl)pyridin-2-yl)-5-(2-(piperazin-1-yl)ethyl)-1,3a,4,5,10,11a-hexahydro-2*H*-benzo[*b*]pyrrolo[2,3-*f*][1,4]diazocine-2,11(3*H*)-dione (700 mg, 89% yield) as a yellow solid.

m/z ES+ $[M+H]^+$ 531.3; $^1$H NMR (400 MHz, $CDCl_3$) δ ppm 8.32 (s, 1H), 7.27 - 7.18 (m, 3H), 7.04 (s, 1H), 4.70 (d, *J* = 9.6 Hz, 1H), 3.61 (dd, *J* = 14.2, 4.4 Hz, 1H), 3.37 (s, 3H), 3.20 - 2.95 (m, 3H), 2.88 - 2.85 (m, 4H), 2.82 - 2.68 (m, 2H), 2.49 (s, 3H), 2.39 - 2.32 (m, 9H), 2.18 (dd, *J* = 16.8, 11.6 Hz, 2H).

**Steps d.** This step was conducted in a similar manner to **Example 37a** and **Example 37b,** step d, using propiolic acid.

m/z ES+ $[M+H]^+$583.3; $^1$H NMR (400 MHz, $CDCl_3$) ppm 8.33 (s, 1H), 7.27 - 7.19 (m, 3H), 7.05 (s, 1H), 4.70 (d, *J* = 9.6 Hz, 1H), 3.78 - 3.71 (m, 2H), 3.69 - 3.53 (m, 3H), 3.38 (s, 3H), 3.17 2.92 (m, 4H), 2.86 - 2.68 (m, 2H), 2.49 (s, 3H), 2.47 - 2.41 (m, 3H), 2.40 (s, 6H), 2.31 - 2.02 (m, 1H).

**Example 265a: (3a*R*,11a*S*)-5-(3-((*S*/*R*)-4-acryloyl-3-(trifluoromethyl)piperazin-1-yl)propyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione and**

**Example 265b: (3a*R*,11a*S*)-5-(3-((*R*/*S*)-4-acryloyl-3-(trifluoromethyl)piperazin-1-yl)propyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

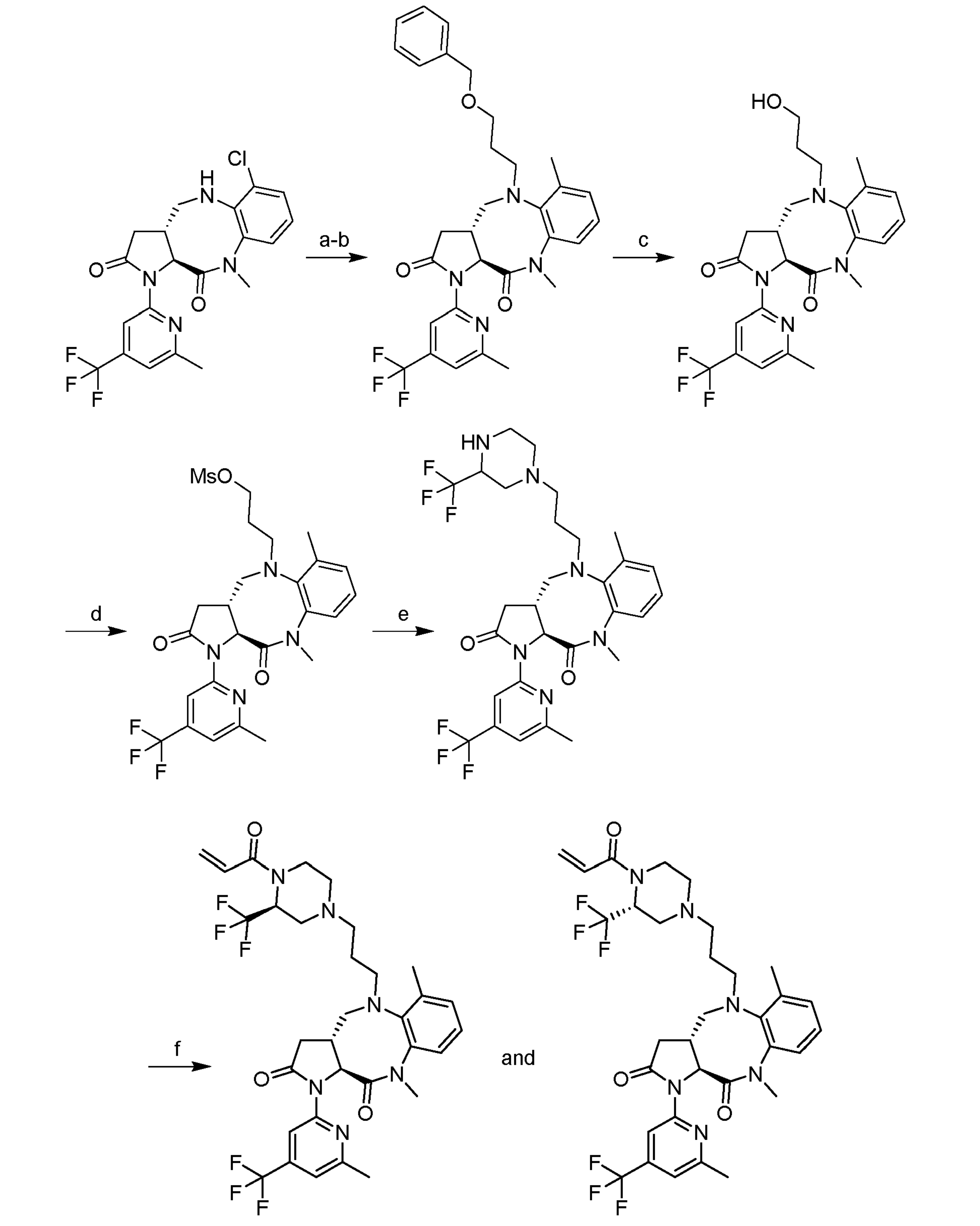


**Step** a. This step was conducted in a similar manner to **Example 38,** step a, using **Intermediate 5b** and **Intermediate B87.**

m/z ES+ $[M+H]^+$ 587.0; $^1H$ NMR (400 MHz, $CDCl_3$) δ ppm 8.31 (s, 1H), 7.43 - 7.39 (m, 1H), 7.34 - 7.28 (m, 4H), 7.26 (d, *J* = 6.4 Hz, 3H), 7.03 (s, 1H), 4.61 (d, *J* = 9.2 Hz, 1H), 4.44 (s, 2H), 3.51 (t, *J* = 6.0 Hz, 3H), 3.33 (s, 3H), 3.18 - 3.04 (m, 2H), 3.00 - 2.87 (m, 2H), 2.73 - 2.65 (m, 1H), 2.46 (s, 3H), 2.24 - 2.14 (m, 1H), 1.67 - 1.58 (m, 2H).

**Step b.** This step was conducted in a similar manner to **Example 77,** step b.

m/z ES+ $[M+H]^+$ 567.3; $^1H$ NMR (400 MHz, $CDCl_3$) δ ppm 8.33 (s, 1H), 7.36 - 7.27 (m, 5H), 7.24 - 7.17 (m, 3H), 7.04 (s, 1H), 4.70 (d, *J* = 9.6 Hz, 1H), 4.46 (s, 2H), 3.60 - 3.53 (m, 1H), 3.51 - 3.44 (m, 2H), 3.35 (s, 3H), 3.12 - 2.91 (m, 3H),

2.81 - 2.67 (m, 2H), 2.49 (s, 3H), 2.33 (s, 3H), 2.22 - 2.13 (m, 1H), 1.75 - 1.59 (m, 2H).

**Step c.** To a solution of (3a*R*,11a*S*)-5-(3-(benzyloxy)propyl)-6,10-dimethyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione (350 mg, 0.62 mmol) in THF (10 mL) was added 10% Pd/C (100 mg). The reaction mixture was stirred at 25 °C for 32 h under a $H_2$ atmosphere (15 psi). Upon completion, the reaction mixture was filtered through a pad of Celite and the solids were washed with EtOAc (2 x 10 mL). The filtrate were evaporated to give (3a*R*,11a*S*)-5-(3-hydroxypropyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione (290 mg, 98% yield) as a yellow solid.

m/z ES+ $[M+H]^+$ 477.1; $^1H$ NMR (400 MHz, $CDCl_3$) δ ppm 8.33 (s, 1H), 7.26 - 7.18 (m, 3H), 7.04 (s, 1H), 4.71 (d, *J* = 9.6 Hz, 1H), 3.69 (t, *J* = 6.2 Hz, 2H), 3.65 - 3.58 (m, 1H), 3.38 (s, 3H), 3.17 - 2.94 (m, 3H), 2.85 - 2.68 (m, 2H), 2.49 (s, 3H), 2.38 (s, 3H), 2.24 - 2.15 (m, 1H), 1.98 - 1.95 (m, 1H), 1.69 - 1.61 (m, 2H).

**Step d.** This step was conducted in a similar manner to **Example 257a** and **Example 257b,** step b.

m/z ES+ $[M+H]^+$ 555.2; $^1H$ NMR (400 MHz, $CDCl_3$) δ ppm 8.33 (s, 1H), 7.27 - 7.18 (m, 3H), 7.05 (s, 1H), 4.69 (d, *J = 9.6* Hz, 1H), 4.31 - 4.20 (m, 2H), 3.64 - 3.54 (m, 1H), 3.38 (s, 3H), 3.15 - 3.08 (m, 2H), 2.99 (s, 3H), 2.97 - 2.89 (m, 1H), 2.88 - 2.79 (m, 1H), 2.78 - 2.70 (m, 1H), 2.49 (s, 3H), 2.39 (s, 3H), 2.25 - 2.16 (m, 1H), 1.84 - 1.80 (m, 2H).

**Step e.** To a mixture of 3-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propyl methanesulfonate (280 mg, 0.50 mmol) and 2-(trifluoromethyl)piperazine (CAS: 131922-05-9; 233 mg, 1.51 mmol) in EtOH (4 mL) was added TEA (153 mg, 1.51 mmol). The reaction mixture was stirred at 80 °C for 4 h. Additional 2-(trifluoromethyl)piperazine (78 mg, 0.50 mmol) was added. The reaction mixture was stirred at 80 °C for a further 2 h. Upon completion, the reaction mixture was diluted with water (10 mL) and extracted with EtOAc (3 x 10 mL). The combined organic layers were washed with brine (2 x 20 mL), dried over $Na_2SO_4$ and evaporated. Purification by reverse phase flash chromatography (water (0.1% $NH_4OH$)/MeCN) afforded (3a*R*,11a*S*)-6,10-dimethyl-1-(6-methyl-4-(trifluoromethyl)pyridin-2-yl)-5-(3-(3-(trifluoromethyl)piperazin-1-yl)propyl)-1,3a,4,5,10,11a-hexahydro-2*H*-benzo[*b*]pyrrolo[2,3-*f*][1,4]diazocine-2,11(3*H*)-dione (200 mg, 65% yield) as a white solid.

m/z ES+ $[M+H]^+$613.3; $^1H$ NMR (400 MHz, $CDCl_3$) δ ppm 8.33 (s, 1H), 7.26 - 7.16 (m, 3H), 7.04 (s, 1H), 4.74 - 4.67 (m, 1H), 3.65 - 3.53 (m, 1H), 3.39 - 3.35 (m, 3H), 3.35 - 3.26 (m, 1H), 3.08 - 2.79 (m, 7H), 2.78 - 2.68 (m, 2H), 2.49 (s, 3H), 2.39 - 2.38 (m, 5H), 2.24 - 2.15 (m, 1H), 2.09 - 1.97 (m, 2H), 1.61 - 1.51 (m, 3H).

**Step f.** This step was conducted in a similar manner to **Example 38,** step c. An additional chiral SFC step afforded the title compounds.

**Example 265a:**

m/z ES+ $[M+H]^+$ 667.0; $^1H$ NMR (400 MHz, $CDCl_3$) δ ppm 8.33 (s, 1H), 7.26 - 7.19 (m, 3H), 7.04 (s, 1H), 6.61 - 6.46 (m, 1H), 6.40 - 6.30 (m, 1H), 5.82 - 5.74 (m, 1H), 5.37 - 5.09 (m, 1H), 4.69 (d, *J = 9.6* Hz, 1H), 4.63 - 4.20 (m, 1H), 3.86 - 3.48 (m, 2H), 3.36 (s, 3H), 3.14 (d, *J* = 12.4 Hz, 1H), 3.11 - 3.03 (m, 1H), 3.02 - 2.91 (m, 2H), 2.90 - 2.77 (m, 2H), 2.76 - 2.68 (m, 1H), 2.49 (s, 3H), 2.43 - 2.30 (m, 5H), 2.25 - 2.09 (m, 2H), 2.06 - 1.93 (m, 1H), 1.59 - 1.49 (m, 2H).

**Example 265b:**

m/z ES+ $[M+H]^+$667.0; $^1H$ NMR (400 MHz, $CDCl_3$) δ ppm 8.33 (s, 1H), 7.26 - 7.19 (m, 3H), 7.04 (s, 1H), 6.63 - 6.45 (m, 1H), 6.40 - 6.30 (m, 1H), 5.78 (d, *J* = 10.8 Hz, 1H), 5.40 - 5.15 (m, 1H), 4.69 (d, *J* = 9.6 Hz, 1H), 4.61 - 4.18 (m, 1H), 3.85 - 3.51 (m, 2H), 3.36 (s, 3H), 3.20 (d, *J* = 12.8 Hz, 1H), 3.15 - 3.05 (m, 1H), 3.03 - 2.90 (m, 2H), 2.87 - 2.77 (m, 2H), 2.76 - 2.68 (m, 1H), 2.49 (s, 3H), 2.38 (s, 3H), 2.38 - 2.27 (m, 2H), 2.25 - 2.08 (m, 2H), 2.04 - 1.92 (m, 1H), 1.57 - 1.47 (m, 2H).

**Example 266**

**(3a*R*,11a*S*)-5-(3-(4-acryloylpiperazin-1-yl)propyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

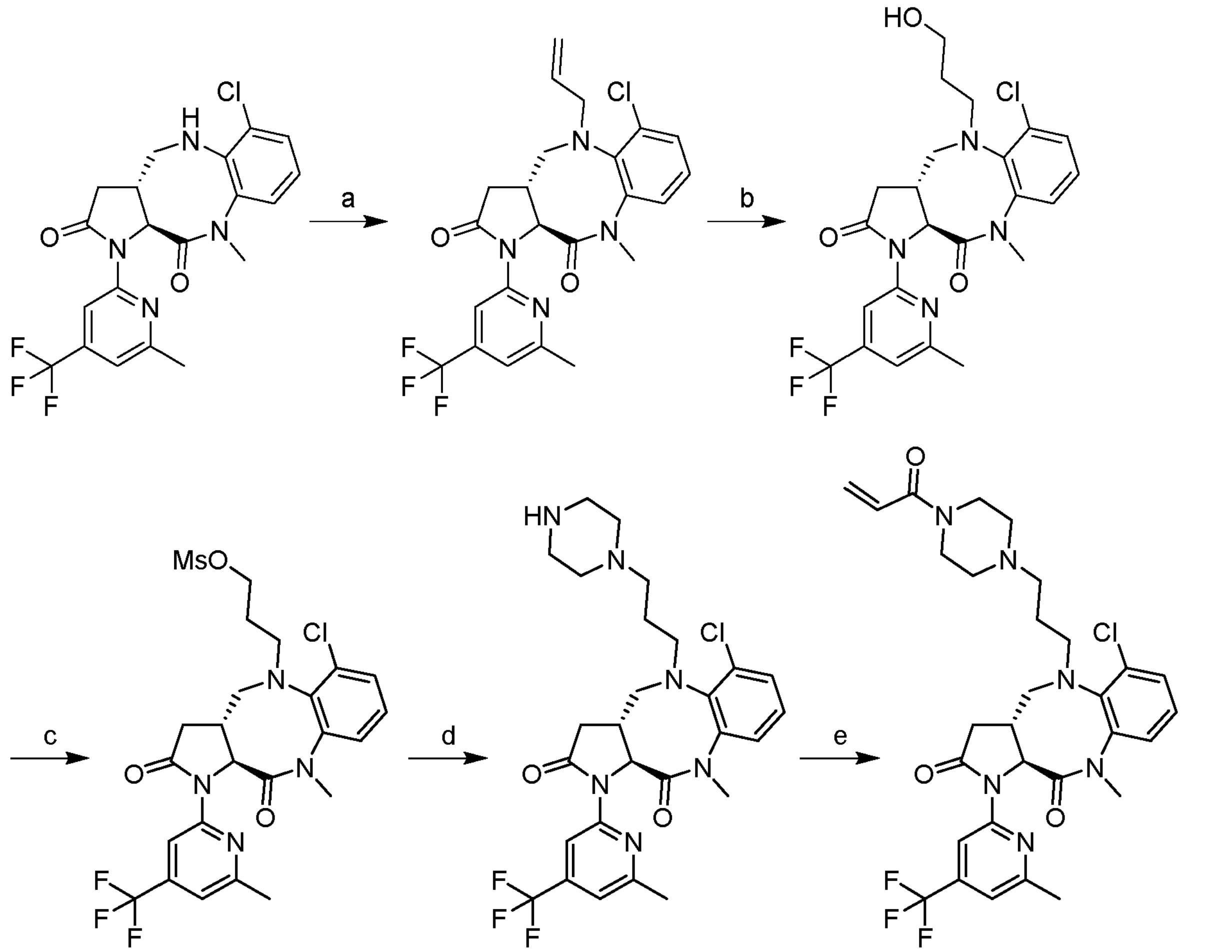


**Step a.** This step was conducted in a similar manner to **Intermediate 7a,** step a, using **Intermediate 5b.**

m/z ES+ $[M+H]^+$ 479.0; $^1H$ NMR (400 MHz, $CDCl_3$) $\delta$ ppm 8.30 (s, 1H), 7.43 - 7.37 (m, 1H), 7.27 - 7.21 (m, 2H), 7.02 (s, 1H), 5.79 - 5.64 (m, 1H), 5.22 - 5.13 (m, 1H), 5.09 - 5.00 (m, 1H), 4.62 (d, $J$ = 9.2 Hz, 1H), 3.67 - 3.48 (m, 3H), 3.34 (s, 3H), 2.99 - 2.86 (m, 2H), 2.74 - 2.63 (m, 1H), 2.45 (s, 3H), 2.22 - 2.11 (m, 1H).

**Step b.** To a solution of (3a*R*,11a*S*)-5-allyl-6-chloro-10-methyl-1-(6-methyl-4-(trifluoromethyl)pyridin-2-yl)-1 ,3a,4,5, 1 0, 11a-hexahydro-2*H*-benzo[b]pyrrolo[2,3-*f*][1,4]diazocine-2,11(3*H*)-dione (2 g, 4.18 mmol) in THF (20 mL) was added a solution of borane dimethyl sulfide complex (10 M in THF, 1.25 mL) in DCM (11 mL). The reaction mixture was stirred at 0 °C for 2 h. Upon completion, the reaction mixture was diluted with MeOH (10 mL) and evaporated. The residue was dissolved in THF (20 mL) and NaOH (501 mg, 12.5 mmol) and $H_2O_2$ (4.26 g, 37.6 mmol) were added. The resulting mixture was stirred at 0 °C for 1 h. Upon completion, the reaction mixture was quenched with sat. aq. $Na_2S_2O_3$ (100 mL). The aqueous mixture was extracted with EtOAc (3 x 50 mL). The combined organic layers were washed with water (2 x 100 mL), sat. aq. $Na_2S_2O_3$ (100 mL), brine (100 mL), dried over $Na_2SO_4$ and evaporated. The residue was purified by reverse phase flash chromatography (water (0.1% $NH_4OH$)/MeCN) to give (3a*R*,11a*S*)-6-chloro-5-(3-hydroxypropyl)-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione (1.3 g, 63% yield) as a yellow oil.

m/z ES+ $[M+H]^+$ 497.2; $^1H$ NMR (400 MHz, $CDCl_3$) $\delta$ ppm 8.25 (s, 1H), 7.42 - 7.35 (m, 1H), 7.28 - 7.19 (m, 2H), 7.00 - 6.95 (m, 1H), 4.62 - 4.52 (m, 1H), 3.69 - 3.59 (m, 2H), 3.58 - 3.47 (m, 1H), 3.35 - 3.28 (m, 3H), 3.19 - 2.98 (m, 2H), 2.93 - 2.88 (m, 1H), 2.71 - 2.61 (m, 1H), 2.43 - 2.38 (m, 3H), 2.23 - 2.08 (m, 1H), 1.69 - 1.41 (m, 2H), 1.08 - 1.00 (m, 1H).

**Step c.** This step was conducted in a similar manner to **Example 257a** and **Example 257b,** step b.

m/z ES+ $[M+H]^+$ 575.2.

**Step d.** To a solution of 3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propyl methanesulfonate (190 mg, 0.33 mmol) in EtOH (2 mL) was added piperazine (285 mg, 3.30 mmol). The reaction mixture was stirred for 1 h at 80 °C. Upon completion, the mixture was quenched with water (30 mL) and extracted with EtOAc (3 x 30 mL). The combined organic layers were washed with brine (30 mL), dried over $Na_2SO_4$ and evaporated to give (3a*R*,11a*S*)-6-chloro-10-methyl-1-(6-methyl-4-(trifluoromethyl)pyridin-2-yl)-5-(3-(piperazin-1-yl)propyl)-1,3a,4,5,10,11a-hexahydro-2*H*-benzo[*b*]pyrrolo[2,3-*f*][1,4]diazocine-2,11(3*H*)-dione (140 mg, crude) as a yellow solid.

m/z ES+ $[M+H]^+$ 565.3; $^1H$ NMR (400 MHz, $CDCl_3$) δ ppm 8.33 (s, 1H), 7.46 - 7.42 (m, 1H), 7.33 - 7.27 (m, 2H), 7.05 (s, 1H), 4.63 (d, *J* = 9.2 Hz, 1H), 3.62 - 3.50 (m, 1H), 3.38 (s, 3H), 3.06 (t, *J* = 6.8 Hz, 2H), 3.01 - 2.92 (m, 2H), 2.86 (t, *J* = 4.8 Hz, 4H), 2.78 - 2.67 (m, 1H), 2.48 (s, 3H), 2.43 - 2.31 (m, 7H), 2.26 - 2.18 (m, 1H), 1.54 (t, *J* = 7.2 Hz, 2H).

**Step e.** This step was conducted in a similar manner to **Example 38,** step c.

m/z ES+ $[M+H]^+$ 619.1; $^1H$ NMR (400 MHz, $CDCl_3$) δ ppm 8.32 (s, 1H), 7.49 - 7.41 (m, 1H), 7.35 - 7.29 (m, 2H), 7.05 (s, 1H), 6.58 - 6.47 (m, 1H), 6.35 - 6.24 (m, 1H), 5.81 - 5.67 (m, 1H), 4.61 (d, *J* = 9.2 Hz, 1H), 3.82 - 3.56 (m, 5H), 3.39 (s, 3H), 3.30 - 3.15 (m, 1H), 3.15 - 2.85 (m, 5H), 2.78 - 2.72 (m, 2H), 2.71 - 2.47 (m, 6H), 2.29 - 2.17 (m, 1H), 1.79 - 1.59 (m, 2H).

**Example 267a: 2-((*S*/*R*)-1-acryloyl-4-(3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)propyl)piperazin-2-yl)acetonitrile and**

**Example 267b: 2-((*R*/*S*)-1-acryloyl-4-(3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propyl)piperazin-2-yl)acetonitrile**

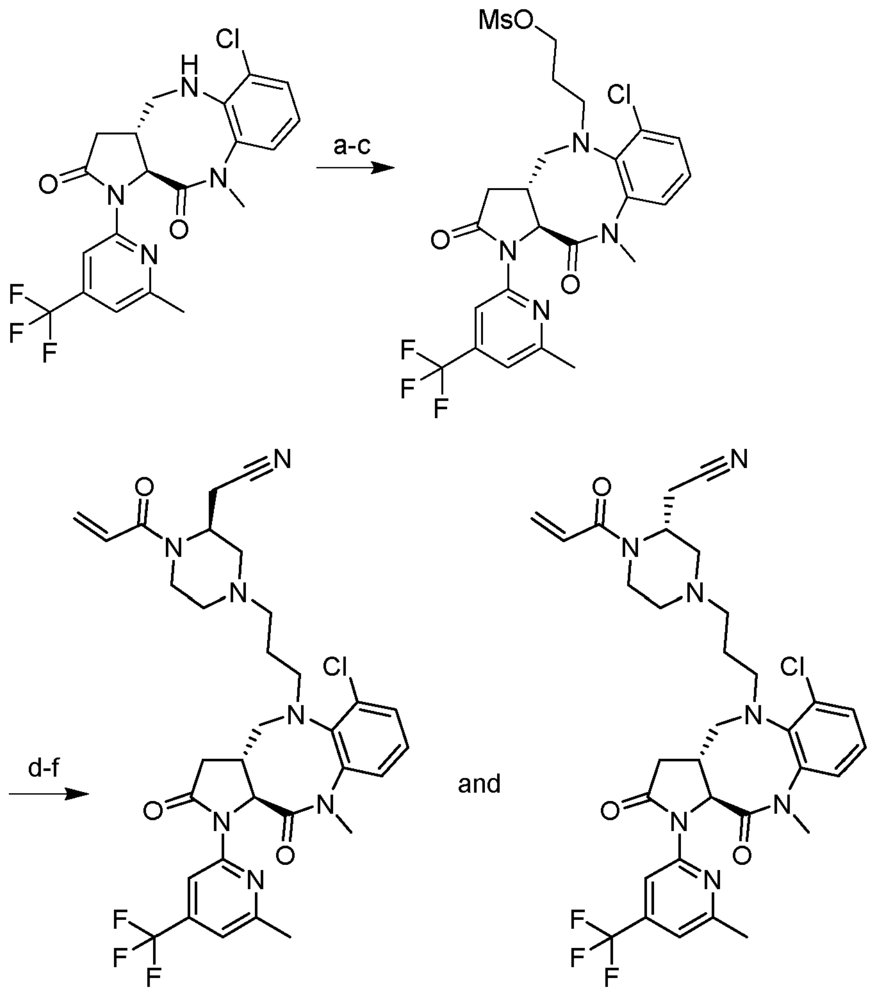


**Steps a-c.** These 3 steps were conducted as described in **Example 266,** steps a-c. **Steps d-f.** These 3 steps were conducted in a similar manner to **Example 257a** and **Example 257b,** steps c-e, using *tert*-butyl 2-(cyanomethyl) piperazine-1-carboxylate (CAS: 1808997-73-0) in step d.

**Example 267a:**

m/z ES+ $[M+H]^+$ 658.2; $^1H$ NMR (400 MHz, $CDCl_3$) δ ppm 8.34 (s, 1H), 7.50 - 7.43 (m, 1H), 7.35 - 7.29 (m, 2H),

7.06 (s, 1H), 6.63 - 6.43 (m, 1H), 6.37 - 6.26 (m, 1H), 5.75 (d, *J* = 9.6 Hz, 1H), 5.07 - 4.88 (m, 0.5H), 4.63 (d, *J* = 9.2 Hz, 1H), 3.85 - 3.69 (m, 0.5H), 3.61 - 3.51 (m, 1H), 3.39 (s, 3H), 3.34 - 3.09 (m, 2H), 3.08 - 2.94 (m, 3H), 2.93 - 2.71 (m, 5H), 2.68 - 2.55 (m, 1H), 2.49 (s, 3H), 2.47 - 2.40 (m, 2H), 2.26 - 2.19 (m, 2H), 2.10 - 1.98 (m, 1H), 1.54 - 1.47 (m, 2H).

**Example 267b:**

m/z ES+ $[M+H]^+$ 658.2; $^1H$ NMR (400 MHz, $CDCl_3$) δ ppm 8.34 (s, 1H), 7.48 - 7.41 (m, 1H), 7.35 - 7.28 (m, 2H), 7.06 (s, 1H), 6.63 - 6.44 (m, 1H), 6.37 - 6.28 (m, 1H), 5.76 (d, *J* = 11.2 Hz, 1H), 5.04 - 4.89 (m, 0.5H), 4.63 (d, *J* = 9.2 Hz, 1H), 3.86 - 3.68 (m, 0.5H), 3.62 - 3.52 (m, 1H), 3.39 (s, 3H), 3.35 - 3.14 (m, 1H), 3.13 - 2.71 (m, 9H), 2.69 - 2.56 (m, 1H), 2.49 - 2.46 (m, 4H), 2.42 - 2.30 (m, 1H), 2.28 - 2.19 (m, 1H), 2.19 - 2.10 (m, 1H), 2.09 - 1.98 (m, 1H), 1.56 - 1.52 (m, 2H).

**Example 268**

**(3a*R*,11a*S*)-5-((3-((*R*)-1-acryloylpyrrolidin-2-yl)-1,2,4-oxadiazol-5-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

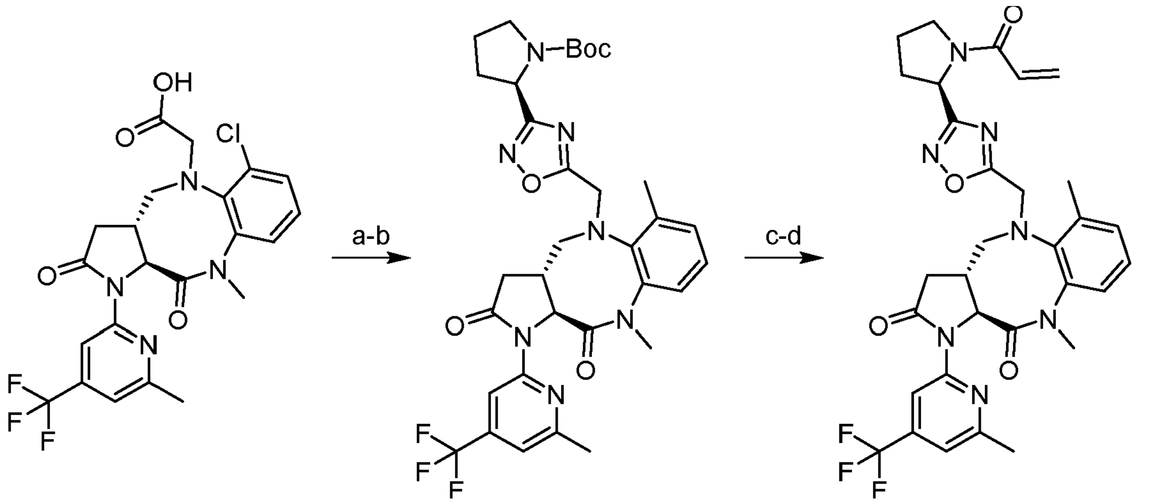


**Step a.** A mixture of **Intermediate 9b** (0.3 g, 0.60 mmol) and CDI (127 mg, 0.79 mmol) in NMP (5 mL) was stirred at 20 °C for 30 min, after which **Intermediate F1** (208 mg, 0.91 mmol) was added. The reaction mixture was stirred at 120 °C for 2 h. Upon completion, the residue was diluted with water (30 mL) and extracted with EtOAc (3 x 30 mL). The combined organic layers were washed with brine (75 mL), dried over $Na_2SO_4$, evaporated and purified by column chromatography (PE/EtOAc = 1/1) to give *tert*-butyl (*R*)-2-(5-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-1,2,4-oxadiazol-3-yl)pyrrolidine-1-carboxylate (0.27 g, 65% yield) as a colourless oil.

m/z ES+ $[M+H]^+$ 690.4; $^1H$ NMR (400 MHz, $CDCl_3$) δ ppm 8.31 (s, 1H), 7.48 - 7.43 (m, 1H), 7.35 - 7.31 (m, 2H), 7.03 (s, 1H), 5.30 ( s, 1H), 5.08 - 4.89 (m, 1H), 4.87 - 4.67 (m, 1H), 4.66 - 4.58 (m, 1H), 3.76 - 3.63 (m, 1H), 3.63 - 3.45 (m, 2H), 3.35 - 3.24 (m, 4H), 3.16 - 3.08 (m, 1H), 2.73 (dd, *J* = 16.8, 8.4 Hz, 1H), 2.46 (s, 3H), 2.25 - 2.19 (m, 2H), 1.96 - 1.78 (m, 3H), 1.45 - 1.41 (m, 9H).

**Step b.** This step was conducted in a similar manner to **Example 77,** step b.

**Steps c-d.** These 2 steps were conducted in a similar manner to **Example 51,** steps b-c.

m/z ES+ $[M+H]^+$624.3; $^1H$ NMR (400 MHz, $CDCl_3$) δ ppm 8.34 (s, 1H), 7.27 - 7.20 (m, 3H), 7.05 (s, 1H), 6.56 - 6.31 (m, 2H), 5.76 - 5.55 (m, 1H), 5.40 - 5.11 (m, 1H), 4.75 (d, *J* = 9.6 Hz, 1H), 4.48 - 4.36 (m, 1H), 4.32 - 4.13 (m, 1H), 3.91 - 3.60 (m, 3H), 3.44 - 3.33 (m, 3H), 3.14 - 2.99 (m, 1H), 2.97 - 2.84 (m, 1H), 2.82 - 2.71 (m, 1H), 2.49 (s, 3H), 2.41 - 2.36 (m, 3H), 2.34 - 2.14 (m, 3H), 2.13 - 1.99 (m, 2H).

**Example 269**

**(3a*R*,11a*S*)-5-((3-((*S*)-1-acryloylpyrrolidin-2-yl)-1,2,4-oxadiazol-5-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

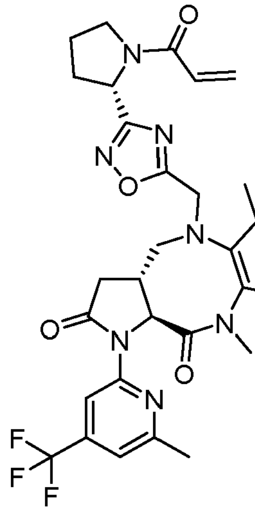

The title compound was prepared in a similar manner to **Example 268,** using **Intermediate 9b** and **Intermediate F2.**

m/z ES+ $[M+H]^+$ 624.3; $^1H$ NMR (400 MHz, $CDCl_3$) δ ppm 8.34 (s, 1H), 7.30 - 7.27 (m, 1H), 7.26 - 7.23 (m, 2H), 7.05 (s, 1H), 6.56 - 6.33 (m, 2H), 5.76 - 5.56 (m, 1H), 5.38 - 5.13 (m, 1H), 4.80 - 4.70 (m, 1H), 4.42 (d, *J* = 16.0 Hz, 1H), 4.27 - 4.17 (m, 1H), 3.91 - 3.63 (m, 3H), 3.38 (s, 3H), 3.14 - 2.99 (m, 1H), 2.96 - 2.83 (m, 1H), 2.82 - 2.69 (m, 1H), 2.49 (s, 3H), 2.41 - 2.35 (m, 3H), 2.30 - 2.14 (m, 3H), 2.11 - 1.98 (m, 2H).

## Example 270

**(3a*R*,11a*S*)-5-((5-((*R*)-1-acryloylpyrrolidin-2-yl)-1,2,4-oxadiazol-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

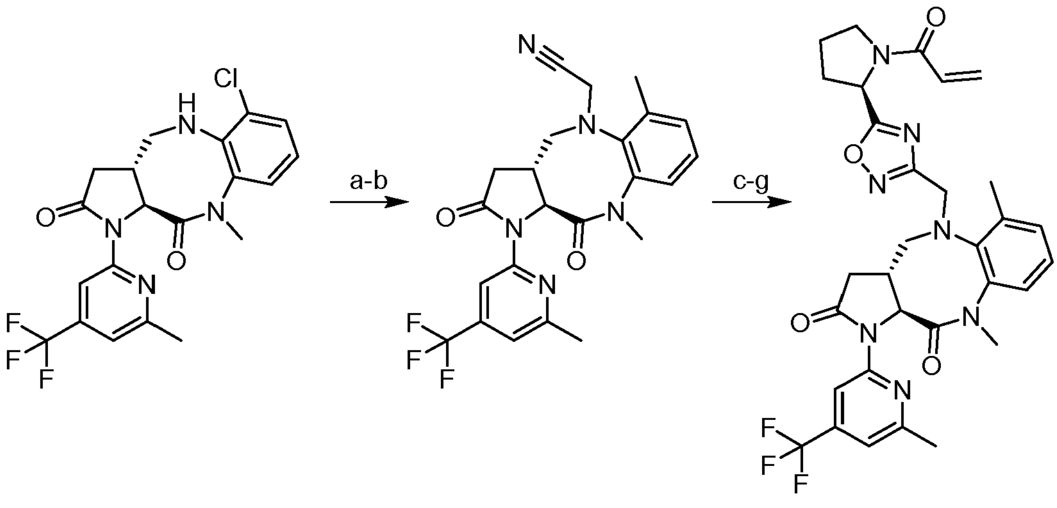


**Step** a. This step was conducted in a similar manner to **Example 101,** step a, using **Intermediate 5b.**

**Step b.** This step was conducted in a similar manner to **Example 77,** step b.

**Steps c-g.** These 5 steps were conducted in a similar manner to **Example 101,** steps b-f, using (*tert*-butoxycarbonyl)-*D*-proline in step d.

m/z ES+ $[M+H]^+$624.3; $^1H$ NMR (400 MHz, $CDCl_3$) δ ppm 8.33 (s, 1H), 7.26 - 7.18 (m, 3H), 7.04 (s, 1H), 6.54 - 6.25

(m, 2H), 5.79 - 5.62 (m, 1H), 5.42 - 5.24 (m, 1H), 4.73 (d, *J* = 9.6 Hz, 1H), 4.29 - 4.21 (m, 1H), 4.20 - 4.07 (m, 1H), 3.94 - 3.76 (m, 1H), 3.76 - 3.63 (m, 2H), 3.38-3.27 (m, 3H), 3.15 - 2.97 (m, 1H), 2.94 - 2.80 (m, 1H), 2.79 - 2.68 (m, 1H), 2.48 (s, 3H), 2.41 - 2.29 (m, 4H), 2.26 - 2.03 (m, 4H).

**Example 271**

**(3a*R*,11a*S*)-5-((5-((*S*)-1-acryloylpyrrolidin-2-yl)-1,2,4-oxadiazol-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

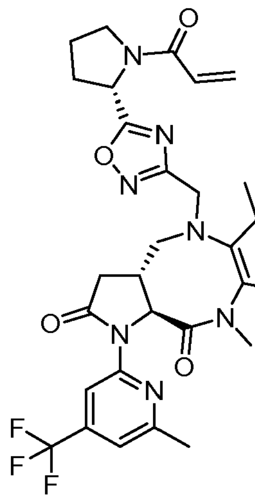

The title compound was prepared in a similar manner to **Example 270,** using (*tert*-butoxycarbonyl)-D-proline in step d.

m/z ES+ $[M+H]^+$624.3; $^1$H NMR (400 MHz, $CDCl_3$) δ ppm 8.33 (s, 1H), 7.26 - 7.19 (m, 3H), 7.04 (s, 1H), 6.57 - 6.21 (m, 2H), 5.80 - 5.59 (m, 1H), 5.40 - 5.21 (m, 1H), 4.77 - 4.69 (m, 1H), 4.30 - 4.21 (m, 1H), 4.19 - 4.08 (m, 1H), 3.96 - 3.78 (m, 1H), 3.76 - 3.64 (m, 2H), 3.38 - 3.27 (m, 3H), 3.14 - 2.95 (m, 1H), 2.91 - 2.80 (m, 1H), 2.78 - 2.68 (m, 1H), 2.49 (s, 3H), 2.41 - 2.29 (m, 4H), 2.27 - 2.05 (m, 4H).

**Example 272**

**(3a*R*,11a*S*)-5-((5-((*R*)-1-acryloylpyrrolidin-2-yl)-1,3,4-oxadiazol-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

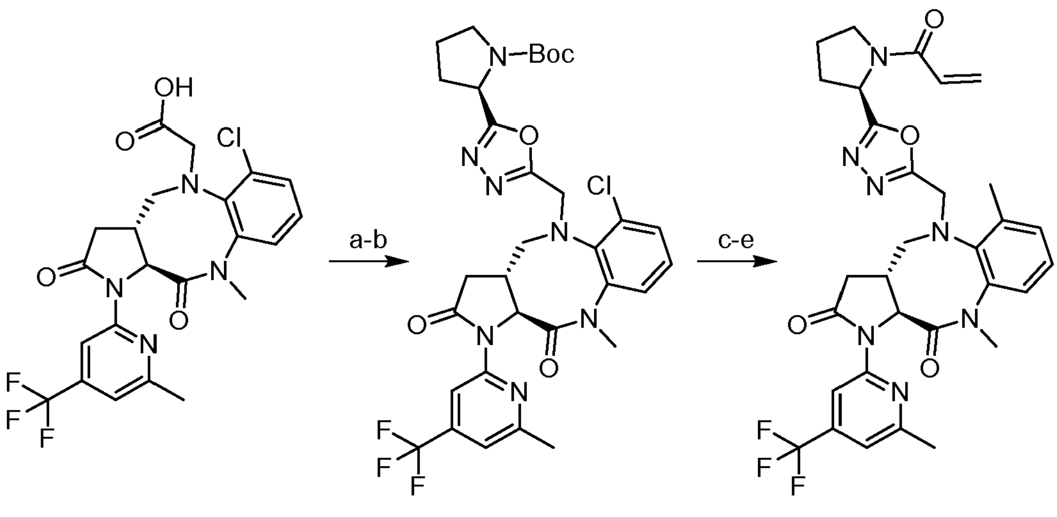

**Step a.** This step was conducted in a similar manner to **Example 103,** step a, using **Intermediate 9b** and **Intermediate G1.**

**Step b.** To a solution of *tert*-butyl (*R*)-2-(2-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)acetyl)hydrazine-1-carbonyl)pyrrolidine-1-carboxylate (300 mg, 0.42 mmol) in DCM (10 mL) was added DIPEA (547 mg, 4.24 mmol) and Burgess reagent (CAS: 29684-56-8; 1.01 g, 4.24 mmol). The mixture was stirred at 25 °C for 12 h. Upon completion, the reaction mixture was diluted with water (15 mL) and extracted with DCM (3 x 20 mL). The combined organic layers were washed with brine (40 mL), dried over $Na_2SO_4$, evaporated and purified by column chromatography (EtOAc) to give *tert*-butyl (*R*)-2-(5-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-1,3,4-oxadiazol-2-yl)pyrrolidine-1-carboxylate (180 mg, 57% yield) as a yellow oil.

m/z ES+ $[M+H]^+$690.3.

**Step c.** This step was conducted in a similar manner to **Example 77,** step b.

**Steps d-e.** These 2 steps were conducted in a similar manner to **Example 51,** steps b-c. m/z ES+ $[M+H]^+$ 624.4; $^1$H NMR (400 MHz, $CDCl_3$) δ ppm 8.34 (s, 1H), 7.27 - 7.19 (m, 3H), 7.10 - 6.97 (m, 1H), 6.54 - 6.43 (m, 1H), 6.43 - 6.35 (m, 1H), 5.91 - 5.58 (m, 1H), 5.44 - 5.25 (m, 1H), 4.71 (d, *J* = 9.2 Hz, 1H), 4.47 - 4.05 (m, 2H), 3.92 - 3.59 (m, 3H), 3.45 - 3.21 (m, 3H), 3.10 - 2.84 (m, 2H), 2.81 - 2.68 (m, 1H), 2.48 (s, 3H), 2.38 - 2.33 (m, 3H), 2.32 - 2.02 (m, 5H).

**Example 273**

**(3a*R*,11a*S*)-5-((5-((*S*)-1-acryloylpyrrolidin-2-yl)-1,3,4-oxadiazol-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

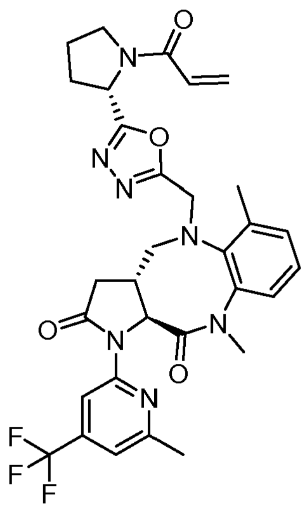

The title compound was prepared in a similar manner to **Example 272,** using **Intermediate 9b** and **Intermediate G2** in step a.

m/z ES+ $[M+H]^+$ 624.2; $^1$H NMR (400 MHz, DMSO-$d_6$) δ ppm 8.18 (s, 1H), 7.35 (s, 1H), 7.33-7.24 (m, 2H), 7.24 - 7.19 (m, 1H), 6.68 - 6.36 (m, 1H), 6.17 - 6.08 (m, 1H), 5.76 - 5.57 (m, 1H), 5.56 - 5.19 (m, 1H), 4.65 (d, *J* = 9.6 Hz, 1H), 4.38 - 4.30 (m, 1H), 4.29 - 4.21 (m, 1H), 3.79-3.43 (m, 3H), 3.28 - 3.24 (m, 3H), 3.06 - 2.96 (m, 1H), 2.86 - 2.73 (m, 1H), 2.63 - 2.53 (m, 1H), 2.48 (s, 3H), 2.44 - 2.36 (m, 1H), 2.32 - 2.20 (m, 4H), 2.16 - 1.78 (m, 3H).

**Example 274**

**(3a*R*,11a*S*)-5-((5-((*R*)-1-acryloylazetidin-2-yl)-1,3,4-oxadiazol-2-yl)methyl)-6,10-dimethyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione**

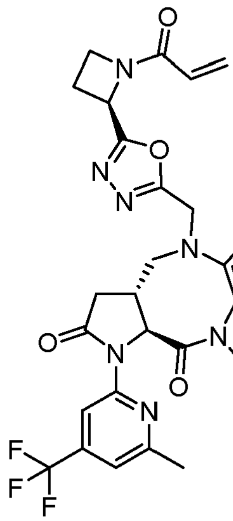

The title compound was prepared in a similar manner to **Example 272,** using **Intermediate 9b** and **Intermediate G3** in step a.

m/z ES+ $[M+H]^+$610.2; $^1$H NMR (400 MHz, $CDCl_3$) δ ppm 8.34 (s, 1H), 7.25 (s, 3H), 7.05 (s, 1H), 6.42 - 6.30 (m, 1H), 6.28 - 6.04 (m, 1H), 5.82 - 5.53 (m, 2H), 4.72 (d, *J* = 8.8 Hz, 1H), 4.54 - 4.13 (m, 4H), 3.76 - 3.62 (m, 1H), 3.48 - 3.31 (m, 3H), 2.98 - 2.62 (m, 5H), 2.50 (s, 3H), 2.38 (s, 3H), 2.26 - 2.14 (m, 1H).

**Example 275**

**(3a*R*,11a*S*)-5-((5-((*S*)-1-acryloylazetidin-2-yl)-1,3,4-oxadiazol-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione**

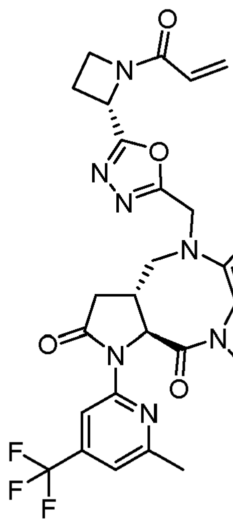

The title compound was prepared in a similar manner to **Example 272,** using **Intermediate 9b** and **Intermediate G4** in step a.

m/z ES+ $[M+H]^+$610.2; $^1$H NMR (400 MHz, $CDCl_3$) δ ppm 8.33 (s, 1H), 7.26 - 7.24 (m, 3H), 7.05 (s, 1H), 6.43 - 6.29 (m, 1H), 6.28 - 5.91 (m, 1H), 5.83 - 5.50 (m, 2H), 4.80 - 4.66 (m, 1H), 4.50 - 4.18 (m, 4H), 3.79 - 3.63 (m, 1H), 3.35 (s, 3H), 3.00 - 2.63 (m, 5H), 2.49 (s, 3H), 2.44-2.33 (m, 3H), 2.25 - 2.12 (m, 1H).

The **Examples** in **Table 20** were prepared using methods similar to those described in the synthesis of **Example 38** or **Example 39,** using the listed **Intermediates** in step a. For **Examples 287a/b-294a/b** an additional chiral SFC step was conducted after step b.

**Table 20**

| Example Number | Structure | Name | Intermediates | $^1$H NMR (400 MHz) δ ppm | MI |
|---|---|---|---|---|---|
| **276** | 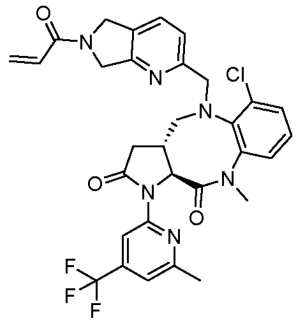 | (3a*R*,11a*S*)-5-((6-acryloyl-6,7-dihydro-5*H*-pyrrolo[3,4-*b*]pyridin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B53** | ($CDCl_3$) 8.32 (s, 1H), 7.69 - 7.46 (m, 3H), 7.33 - 7.29 (m, 2H), 7.06 (s, 1H), 6.62 - 6.48 (m, 2H), 5.86 - 5.75 (m, 1H), 4.96 - 4.85 (m, 4H), 4.70 - 4.68 (m, 1H), 4.46 - 4.28 (m, 2H), 3.57 - 3.52 (m, 1H), 3.39 - 3.34 (m, 3H), 3.19 - 2.82 (m, 2H), 2.74 - 2.63 (m, 1H), 2.48 (s, 3H), 2.26 - 2.15 (m, 1H) | 625.2 |
| **277** | 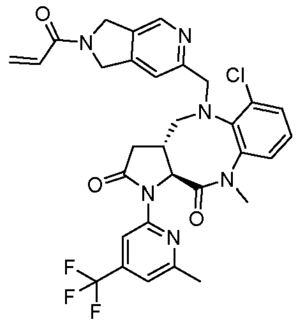 | (3a*R*,11a*S*)-5-((2-acryloyl-2,3-dihydro-1*H*-pyrrolo[3,4-*c*]pyridin-6-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B54** | ($CDCl_3$) 8.47 (d, *J* = 13.2 Hz, 1H), 8.32 (s, 1H), 7.62 (d, *J* = 15.6 Hz, 1H), 7.53 - 7.45 (m, 1H), 7.38 - 7.29 (m, 2H), 7.05 (d, *J* = 4.4 Hz, 1H), 6.62 - 6.42 (m, 2H), 5.86 - 5.72 (m, 1H), 4.95 (s, 2H), 4.89 (s, 2H), 4.73 - 4.66 (m, 1H), 4.50 - 4.27 (m, 2H), 3.63 - 3.49 (m, 1H), 3.36 (d, *J* = 12.0 Hz, 3H), 3.17 - 3.05 (m, 1H), 3.04 - 2.90 (m, 1H), 2.74 - 2.63 (m, 1H), 2.48 (d, *J* = 6.0 Hz, 3H), 2.26 - 2.15 (m, 1H) | 625.2 |
| **278** | 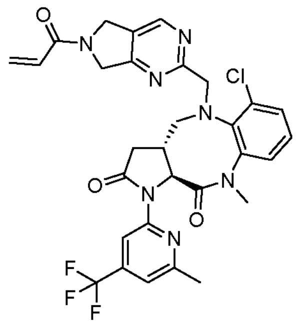 | (3a*R*,11a*S*)-5-((6-acryloyl-6,7-dihydro-5*H*-pyrrolo[3,4-*d*]pyrimidin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B56** | (DMSO-$d_6$) 8.71 - 8.69 (m, 1H), 8.16 (s, 1H), 7.54 - 7.51 (m, 1H), 7.47 - 7.46 (m, 1H), 7.42 - 7.38 (m, 1H), 7.34 (s, 1H), 6.74 - 6.64 (m, 1H), 6.27 - 6.21 (m, 1H), 5.81 - 5.75 (m, 1H), 5.00 (s, 1H), 4.93 (s, 1H), 4.70 (s, 1H), 4.68 - 4.52 (m, 2H), 4.33 - 4.24 (m, 2H), 3.68 - 3.64 (m, 1H), 3.15 - 3.08 (m, 4H), 2.87 - 2.75 (m, 1H), 2.60 - 2.57 (m, 1H), 2.48 (s, 3H), 2.27 - 2.14 (m, 1H) | 626.2 |

(continued)

| Example Number | Structure | Name | Intermediates | $^1$H NMR (400 MHz) δ ppm | MI |
|---|---|---|---|---|---|
| **279** | 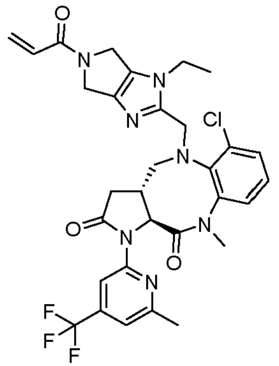 | (3a*R*,11a*S*)-5-((5-acryloyl-1-ethyl-1,4,5,6-tetrahydropyrrolo[3,4-d]imidazol-2-yl) methyl)-6-chloro-10-methyl-1-(6-methyl-4-(trifluoromethyl)pyridin-2-yl)-1,3a,4,5,10,11a-hexahydro-2*H*-benzo [*b*]pyrrolo[2,3-*f*][1,4]diazocine-2,11(3*H*)-dione | **Intermediate 5b** and **Intermediate B64** | ($CDCl_3$) 8.34 (s, 1H), 7.50 - 7.47 (m, 1H), 7.39 - 7.33 (m, 2H), 7.07 (s, 1H), 6.54 - 6.48 (m, 2H), 5.82 - 5.79 (m, 1H), 4.80 - 4.71 (m, 2H), 4.68 - 4.54 (m, 2H), 4.46 - 4.36 (m, 1H), 4.31 - 4.17 (m, 1H), 4.13 - 3.98 (m, 2H), 3.66 - 3.53 (m, 1H), 3.50 - 3.28 (m, 4H), 3.11 - 2.95 (m, 2H), 2.79 - 2.73 (m, 1H), 2.49 (s, 3H), 2.25 - 2.18 (m, 1H), 1.43 (t, *J* = 7.2 Hz, 3H) | 642.2 |
| **280** | 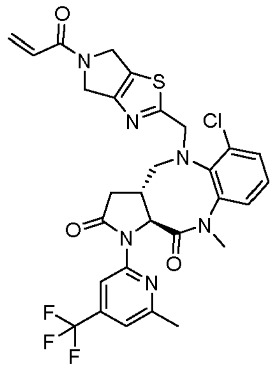 | (3a*R*,11a*S*)-5-((5-acryloyl-5,6-dihydro-4*H*-pyrrolo[3,4-*d*]thiazol-2-yl) methyl)-6-chloro-1 0-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B65** | ($CDCl_3$) 8.32 (s, 1H), 7.54 - 7.48 (m, 1H), 7.42 - 7.33 (m, 2H), 7.07 (s, 1H), 6.51 (d, *J* = 6.0 Hz, 2H), 5.86 - 5.68 (m, 1H), 4.98 - 4.61 (m, 6H), 4.52 - 4.35 (m, 1H), 3.69 - 3.56 (m, 1H), 3.43 (s, 3H), 3.26 - 3.12 (m, 1H), 3.10 - 2.97 (m, 1H), 2.79 - 2.63 (m, 1H), 2.49 (s, 3H), 2.30 - 2.12 (m, 1H) | 631.1 |

(continued)

| Example Number | Structure | Name | Intermediates | $^1$H NMR (400 MHz) δ ppm | MI |
|---|---|---|---|---|---|
| **281** | 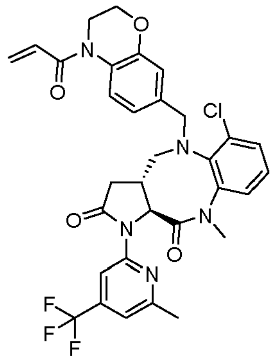 | (3a*R*,11a*S*)-5-((4-acryloyl-3,4-dihydro-2*H*-benzo[*b*][1,4]oxazin-7-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B76** | ($CDCl_3$) 8.34 (s, 1H), 7.50 - 7.48 (m, 1H), 7.35 - 7.29 (m, 2H), 7.23 - 7.10 (m, 1H), 7.07 (s, 1H), 7.02 (d, *J* = 1.6 Hz, 1H), 6.96 - 6.93 (m, 1H), 6.77 - 6.70 (m, 1H), 6.53 - 6.46 (m, 1H), 5.83 - 5.80 (m, 1H), 4.71 (d, *J* = 9.2 Hz, 1H), 4.34 (t, *J* = 4.8 Hz, 2H), 4.20 - 4.06 (m, 2H), 4.06 - 3.98 (m, 2H), 3.55 - 3.47 (m, 1H), 3.39 (s, 3H), 3.06 - 2.91 (m, 2H), 2.69 - 2.65 (m, 1H), 2.50 (s, 3H), 2.24 - 2.17 (m, 1H) | 640.2 |
| **282** | 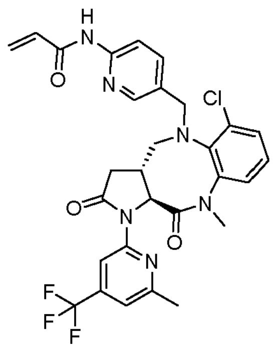 | *N*-(5-(((3aR,11aS)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)pyridin-2-yl)acrylamide | **Intermediate 5b** and *tert*-butyl (5-formylpyridin-2-yl)carbamate (CAS: 199296-40-7) | ($CDCl_3$) 8.33 (s, 1H), 8.29 - 8.23 (m, 2H), 8.09 (s, 1H), 7.79 - 7.76 (m, 1H), 7.49 - 7.44 (m, 1H), 7.29 (d, *J* = 4.8 Hz, 2H), 7.06 (s, 1H), 6.48 - 6.44 (m, 1H), 6.31 - 6.22 (m, 1H), 5.84 - 5.81 (m, 1H), 4.68 (d, *J* = 9.2 Hz, 1H), 4.26 (d, *J* = 14.0 Hz, 1H), 4.13 - 4.06 (m, 1H), 3.52 - 3.46 (m, 1H), 3.34 (s, 3H), 3.07 - 2.89 (m, 2H), 2.70 - 2.66 (m, 1H), 2.48 (s, 3H), 2.24 - 2.17 (m, 1H) | 599.0 |
| **283** | 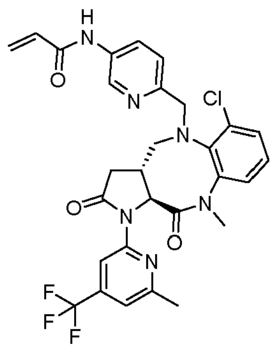 | *N*-(6-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)pyridin-3-yl)acrylamide | **Intermediate 5b** and **Intermediate B77** | ($CDCl_3$) 8.46 (s, 1H), 8.24 (s, 1H), 8.16 - 8.10 (m, 1H), 7.53 (d, *J* = 9.2 Hz, 1H), 7.42 - 7.38 (m, 1H), 7.28 - 7.20 (m, 3H), 6.97 (s, 1H), 6.46 - 6.34 (m, 1H), 6.20 - 6.13 (m, 1H), 5.75 (d, *J* = 9.6 Hz, 1H), 4.62 (d, *J* = 9.2 Hz, 1H), 4.25 (d, *J* = 2.4 Hz, 2H), 3.50 - 3.46 (m, 1H), 3.28 (s, 3H), 3.03 - 2.87 (m, 2H), 2.63 - 2.57 (m, 1H), 2.40 (s, 3H), 2.18 - 2.09 (m, 1H) | 599.2 |

(continued)

| Example Number | Structure | Name | Intermediates | $^1H$ NMR (400 MHz) δ ppm | MI |
|---|---|---|---|---|---|
| **284** | 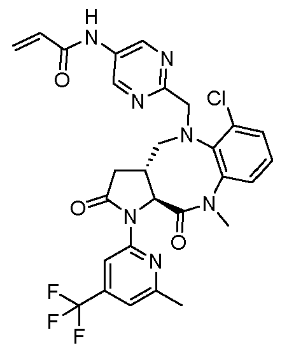 | *N*-(2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)pyrimidin-5-yl)acrylamide | **Intermediate 5b** and **Intermediate B78** | ($CDCl_3$) 9.01 (s, 2H), 8.27 (s, 1H), 8.09 (s, 1H), 7.49 - 7.46 (m, 1H), 7.34 - 7.28 (m, 2H), 7.03 (s, 1H), 6.42 (d, *J* = 16.8 Hz, 1H), 6.13 - 6.07 (m, 1H), 5.74 (d, *J* = 10.4 Hz, 1H), 4.61 (d, *J* = 9.2 Hz, 1H), 4.52 (d, *J* = 14.8 Hz, 1H), 4.31 (d, *J* = 14.8 Hz, 1H), 3.88 - 3.83 (m, 1H), 3.14 - 2.11 (m, 4H), 3.06 - 2.96 (m, 1H), 2.72 - 2.66 (m, 1H), 2.47 (s, 3H), 2.23 - 2.16 (m, 1H) | 600.1 |
| **285** | 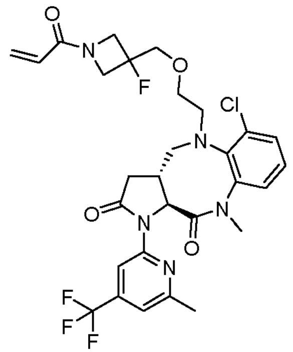 | (3a*R*,11a*S*)-5-(2-((1-acryloyl-3-fluoroazetidin-3-yl)methoxy)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B80** | ($CDCl_3$) 8.33 (s, 1H), 7.46 - 7.44 (m, 1H), 7.35 - 7.28 (m, 2H), 7.05 (s, 1H), 6.39 - 6.34 (m, 1H), 6.25 - 6.10 (m, 1H), 5.72 (d, *J* = 10.4 Hz, 1H), 4.61 (d, *J* = 9.2 Hz, 1H), 4.35 - 4.19 (m, 2H), 4.17 - 3.99 (m, 2H), 3.70 (s, 1H), 3.65 (s, 1H), 3.63 - 3.50 (m, 3H), 3.36 (s, 3H), 3.31 - 3.14 (m, 2H), 3.06 - 2.87 (m, 2H), 2.77 - 2.66 (m, 1H), 2.49 (s, 3H), 2.23 - 2.16 (m, 1H) | 624.3 |
| **286** | 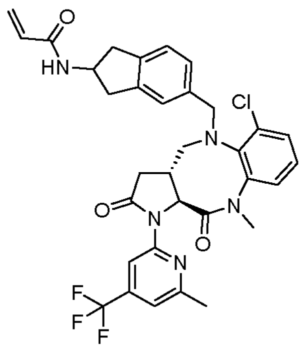 | *N*-(5-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2,3-dihydro-1*H*-inden-2-yl)acrylamide | **Intermediate 5b** and **Intermediate B81** | ($CDCl_3$) 8.32 (s, 1H), 7.51 - 7.44 (m, 1H), 7.48 - 7.28 (m, 3H), 7.18 (s, 2H), 7.06 (s, 1H), 6.29 (d, *J* = 16.0 Hz, 1H), 6.05 - 6.02 (m, 1H), 5.90 - 5.76 (m, 1H), 5.62 (d, *J* = 10.0 Hz, 1H), 4.89 - 4.76 (m, 1H), 4.67 - 4.64 (m, 1H), 4.24 - 4.08 (m, 2H), 3.49 - 3.35 (m, 1H), 3.37 - 3.27 (m, 5H), 2.99 - 2.93 (m, 1H), 2.86 - 2.81 (m, 3H), 2.65 - 2.60 (m, 1H), 2.49 (d, *J* = 3.6 Hz, 3H), 2.26 - 2.10 (m, 1H) | 638.3 |

(continued)

| Example Number | Structure | Name | Intermediates | $^1$H NMR (400 MHz) δ ppm | MI |
|---|---|---|---|---|---|
| **287a** | | 2-((*S*/*R*)-2-acryloyl-5-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)isoindolin-1-yl)acetonitrile | **Intermediate 5b** and **Intermediate B57** | ($CDCl_3$) 8.33 (s, 1H), 7.49 (t, *J* = 4.8 Hz, 1H), 7.43 - 7.39 (m, 1H), 7.37 (s, 1H), 7.36 - 7.33 (m, 1H), 7.31 (d, *J* = 4.4 Hz, 2H), 7.06 (s, 1H), 6.63 - 6.44 (m, 2H), 5.88 - 5.80 (m, 1H), 5.52 (d, *J* = 2.0 Hz, 1H), 5.08 - 4.97 (m, 1H), 4.94 - 4.86 (m, 1H), 4.68 (d, *J* = 9.2 Hz, 1H), 4.34 - 4.26 (m, 1H), 4.24 - 4.16 (m, 1H), 3.55 - 3.40 (m, 2H), 3.26 (s, 3H), 3.10 - 2.91 (m, 3H), 2.74 - 2.63 (m, 1H), 2.48 (s, 3H), 2.26 - 2.14 (m, 1H) | 663.1 |
| **287b** | 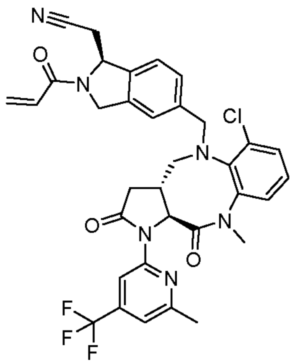 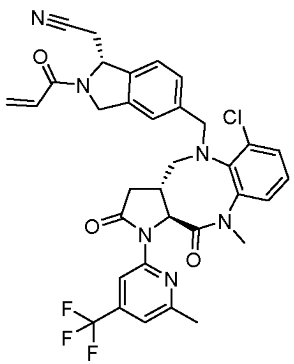 | 2-((*R*/*S*)-2-acryloyl-5-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)isoindolin-1-yl)acetonitrile | | ($CDCl_3$) 8.33 (s, 1H), 7.51 - 7.46 (m, 1H), 7.41 (s, 1H), 7.39 - 7.35 (m, 1H), 7.34 - 7.28 (m, 3H), 7.06 (s, 1H), 6.62 - 6.53 (m, 1H), 6.53 - 6.46 (m, 1H), 5.86 - 5.81 (m, 1H), 5.55 - 5.49 (m, 1H), 5.09 - 5.00 (m, 1H), 4.96 - 4.87 (m, 1H), 4.67 (d, *J* = 9.6 Hz, 1H), 4.34 - 4.19 (m, 2H), 3.54 - 3.43 (m, 2H), 3.30 (s, 3H), 3.10 - 2.98 (m, 1H), 2.97 - 2.84 (m, 2H), 2.72 - 2.61 (m, 1H), 2.49 (s, 3H), 2.25 - 2.12 (m, 1H) | 663.1 |

(continued)

| Example Number | Structure | Name | Intermediates | $^1$H NMR (400 MHz) δ ppm | MI |
|---|---|---|---|---|---|
| **288a** | 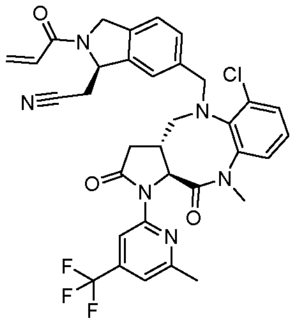 | 2-((*R*/*S*)-2-acryloyl-6-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)isoindolin-1-yl)acetonitrile | **Intermediate** 5b and **Intermediate B58** | ($CDCl_3$) 8.35 (s, 1H), 7.50 - 7.44 (m, 1H), 7.40 (s, 1H), 7.38 - 7.32 (m, 3H), 7.31 (d, *J* = 9.6 Hz, 1H), 7.06 (s, 1H), 6.63 - 6.43 (m, 2H), 5.89 - 5.79 (m, 1H), 5.52 (s, 1H), 5.08 - 4.98 (m, 1H), 4.94 - 4.85 (m, 1H), 4.66 (d, *J* = 9.6 Hz, 1H), 4.41 - 4.32 (m, 1H), 4.27 - 4.18 (m, 1H), 3.65 - 3.53 (m, 1H), 3.50 - 3.39 (m, 1H), 3.33 (s, 3H), 3.10 - 3.01 (m, 1H), 3.01 - 2.94 (m, 1H), 2.91 - 2.79 (m, 1H), 2.71 - 2.62 (m, 1H), 2.49 (s, 3H), 2.21 - 2.11 (m, 1H) | 663.2 |
| **288b** | 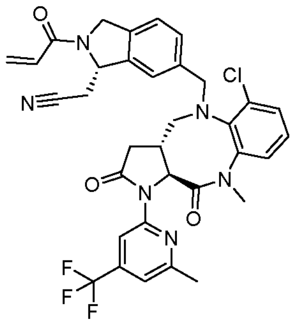 | 2-((*S*/*R*)-2-acryloyl-6-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)isoindolin-1-yl)acetonitrile | | ($CDCl_3$) 8.33 (s, 1H), 7.50 - 7.48 (m, 1H), 7.45 (s, 1H), 7.39 (d, *J* = 8.0 Hz, 1H), 7.35 - 7.28 (m, 3H), 7.07 (s, 1H), 6.61 - 6.47 (m, 2H), 5.85 - 5.82 (m, 1H), 5.56 - 5.51 (m, 1H), 5.07 - 5.00 (m, 1H), 4.93 - 4.87 (m, 1H), 4.68 (d, *J* = 9.2 Hz, 1H), 4.32 - 4.21 (m, 2H), 3.57 - 3.52 (m, 1H), 3.48 - 3.45 (m, 1H), 3.34 (s, 3H), 3.09 - 2.99 (m, 2H), 2.99 - 2.89 (m, 1H), 2.67 - 2.63 (m, 1H), 2.49 (s, 3H), 2.12 - 2.15 (m, 1H) | 663.1 |

(continued)

| Example Number | Structure | Name | Intermediates | $^1$H NMR (400 MHz) δ ppm | MI |
|---|---|---|---|---|---|
| **289a** | 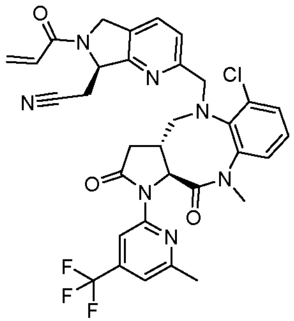 | 2-((*R*/*S*)-6-acryloyl-2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)-6,7-dihydro-5*H*-pyrrolo[3,4-*b*]pyridin-7-yl)acetonitrile | **Intermediate 5b** and **Intermediate B59** | ($CDCl_3$) 8.35 (s, 1H), 7.71 - 7.62 (m, 2H), 7.51 - 7.49 (m, 1H), 7.36 - 7.32 (m, 2H), 7.08 (s, 1H), 6.62 - 6.51 (m, 2H), 5.89 - 5.87 (m, 1H), 5.41 (s, 1H), 5.11 - 5.02 (m, 1H), 4.96 - 4.89 (m, 1H), 4.71 (d, *J* = 9.2 Hz, 1H), 4.43 (s, 2H), 3.73 - 3.68 (m, 1H), 3.58 -3.53 (m, 1H), 3.35 (s, 3H), 3.17 - 3.01 (m, 3H), 3.0 - 2.7 (m, 1H), 2.50 (s, 3H), 2.3 - 2.1 (m, 1H) | 664.1 |
| **289b** | 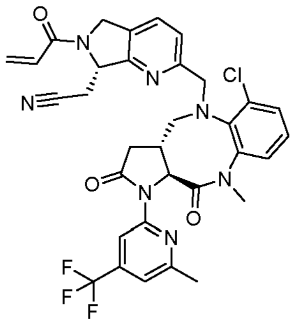 | 2-((*S*/*R*)-6-acryloyl-2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitrile | | ($CDCl_3$) 8.33 (s, 1H), 7.68 - 7.61 (m, 2H), 7.49 - 7.47 (m, 1H), 7.33 - 7.31 (m, 2H), 7.06 (s, 1H), 6.57 - 6.54 (m, 2H), 5.88 - 5.85 (m, 1H), 5.41 (s, 1H), 5.06 - 4.90 (m, 2H), , 4.71 (d, *J* = 9.2 Hz, 1H), 4.44 - 4.33 (m, 2H), 3.72 - 3.67 (m, 1H), 3.55 - 3.50 (m, 1H), 3.41 (s, 3H), 3.10 - 3.05 (m, 3H), 2.77 - 2.66 (m, 1H), 2.49 (s, 3H), 2.23 - 2.16 (m, 1H) | 664.5 |

(continued)

| Example Number | Structure | Name | Intermediates | $^1$H NMR (400 MHz) δ ppm | MI |
|---|---|---|---|---|---|
| **290a** | 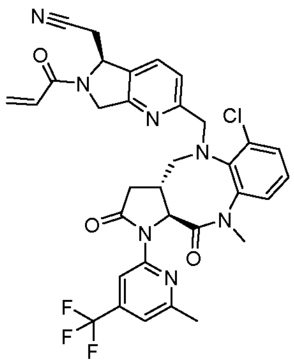 | 2-((*S*/*R*)-6-acryloyl-2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitrile | **Intermediate 5b** and **Intermediate B60** | ($CDCl_3$) 8.33 (s, 1H), 7.76 (d, *J* = 8.0 Hz, 1H), 7.64 (d, *J* = 8.0 Hz, 1H), 7.51 - 7.48 (m, 1H), 7.33 - 7.27 (m, 2H), 7.06 (s, 1H), 6.60 - 6.53 (m, 2H), 5.88 - 5.85 (m, 1H), 5.57 - 5.56 (m, 1H), 5.03 - 5.01 (m, 1H), 4.99 - 4.88 (m, 1H), 4.70 (d, *J* = 9.6 Hz, 1H), 4.39 (s, 2H), 3.56 - 3.52 (m, 1H), 3.36 (d, *J* = 6.0 Hz, 1H), 3.33 (s, 3H), 3.15 - 3.08 (m, 1H), 3.07 -3.01 (m, 2H), 2.71 - 2.65 (m, 1H), 2.49 (s, 3H), 2.23 - 2.16 (m, 1H) | 664.1 |
| **290b** | 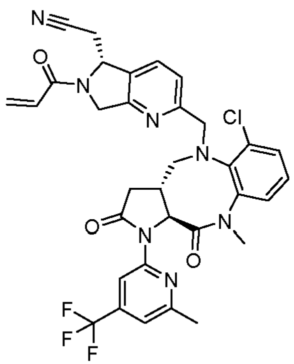 | 2-((*R*/*S*)-6-acryloyl-2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitrile | | ($CDCl_3$) 8.33 (s, 1H), 7.77 (d, *J* = 8.0 Hz, 1H), 7.66 (d, *J* = 8.0 Hz, 1H), 7.50 - 7.48 (m, 1H), 7.34 - 7.29 (m, 2H), 7.06 (s, 1H), 6.56 - 6.50 (m, 2H), 5.88 - 5.85 (m, 1H), 5.56 - 5.54 (m, 1H), 5.02 - 5.01 (m, 1H), 4.98 - 4.90 (m, 1H), 4.70 (d, *J* = 9.6 Hz, 1H), 4.39 (s, 2H), 3.58 - 3.36 (m, 1H), 3.33 - 3.32 (m, 1H), 3.31 (s, 3H), 3.16 - 3.04 (m, 3H), 2.74 - 2.70 (m, 1H), 2.48 (s, 3H), 2.25 - 2.18 (m, 1H) | 664.1 |

(continued)

| Example Number | Structure | Name | Intermediates | $^1$H NMR (400 MHz) δ ppm | MI |
|---|---|---|---|---|---|
| **291a** | 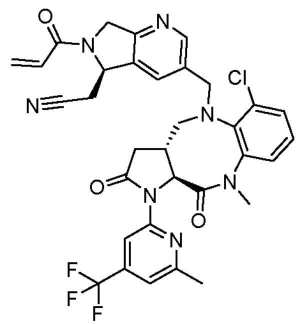 | 2-((*R*/*S*)-6-acryloyl-3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)-6,7-dihydro-5*H*-pyrrolo[3,4-*b*]pyridin-5-yl)acetonitrile | **Intermediate 5b** and **Intermediate B61** | ($CDCl_3$) 8.53 (s, 1H), 8.36 (s, 1H), 7.83 (s, 1H), 7.49 - 7.48 (m, 1H), 7.33 (d, *J* = 4.4 Hz, 2H), 7.07 (s, 1H), 7.57 - 7.50 (m, 2H), 5.89 - 5.87 (m, 1H), 5.58 (s, 1H), 5.06 - 4.91 (m, 2H), 4.66 (d, *J* = 9.6 Hz, 1H), 4.43 - 4.23 (m, 2H), 3.56 - 3.51 (m, 2H), 3.48 (s, 3H), 3.10 - 3.04 (m, 2H), 2.82 - 2.71 (m, 1H), 2.65 - 2.50 (m, 1H), 2.50 (s, 3H), 2.21 - 2.14 (m, 1H) | 664.0 |
| **291b** | 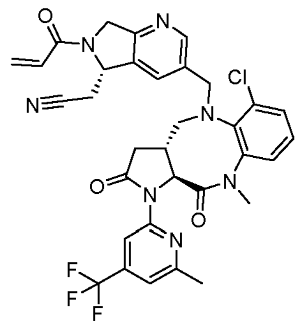 | 2-((*S*/*R*)-6-acryloyl-3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitrile | | ($CDCl_3$) 8.55 (d, *J* = 1.6 Hz, 1H), 8.34 (s, 1H), 7.88 (s, 1H), 7.51 - 7.48 (m, 1H), 7.34 - 7.33 (m, 2H), 7.07 (s, 1H), 6.56 - 6.50 (m, 2H), 5.89 - 5.86 (m, 1H), 5.58 (d, *J* = 3.2 Hz, 1H), 5.05 - 5.01 (m, 1H), 4.92 - 4.88 (m, 1H), 4.68 (d, *J* = 10.0 Hz, 1H), 4.30 (s, 2H), 3.47 - 3.42 (m, 2H), 3.36 (s, 3H), 3.15 - 3.06 (m, 2H), 2.98 - 2.81 (m, 1H), 2.72 - 2.66 (m, 1H), 2.50 (s, 3H), 2.25 - 2.17 (m, 1H) | 664.0 |

(continued)

| Example Number | Structure | Name | Intermediates | $^1H$ NMR (400 MHz) δ ppm | MI |
|---|---|---|---|---|---|
| **292a** | 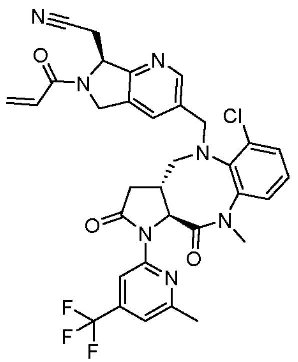 | 2-((*S*/*R*)-6-acryloyl-3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitrile | **Intermediate 5b** and **Intermediate B62** | ($CDCl_3$) 8.50 (s, 1H), 8.33 (s, 1H), 7.79 (s, 1H), 7.50 - 7.47 (m, 1H), 7.33 - 7.27 (m, 2H), 7.06 (s, 1H), 6.61 - 6.50 (m, 2H), 5.88 - 5.85 (m, 1H), 5.43 (s, 1H), 5.10 - 4.94 (m, 2H), 4.68 - 4.65 (m, 1H), 4.36 - 4.29 (m, 2H), 3.74 - 3.48 (m, 2H), 3.39 - 3.25 (m, 3H), 3.09 - 3.03 (m, 2H), 2.97 - 2.79 (m, 1H), 2.69 - 2.65 (m, 1H), 2.49 (s, 3H), 2.24 - 2.17 (m, 1H) | 664.1 |
| **292b** | 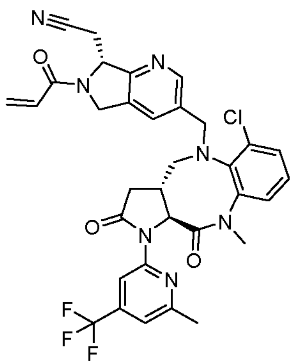 | 2-((*R*/*S*)-6-acryloyl-3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)-6,7-dihydro-5*H*-pyrrolo[3,4-*b*]pyridin-7-yl)acetonitrile | | ($CDCl_3$) 8.54 - 8.50 (m, 1H), 8.33 (s, 1H), 7.79 - 7.75 (m, 1H), 7.50 - 7.48 (m, 1H), 7.37 - 7.30 (m, 2H), 7.06 (s, 1H), 6.61 - 6.50 (m, 2H), 5.88 - 5.85 (m, 1H), 5.43 (d, *J* = 2.0 Hz, 1H), 5.09 - 5.04 (m, 1H), 4.97 - 4.92 (m, 1H), 4.66 - 4.64 (m, 1H), 4.39 - 4.20 (m, 2H), 3.71 - 3.66 (m, 1H), 3.52 - 3.48 (m, 1H), 3.30 - 3.22 (m, 3H), 3.13 - 3.02 (m, 2H), 3.02 - 2.92 (m, 1H), 2.78 - 2.67 (m, 1H), 2.48 (s, 3H), 2.27 - 2.16 (m, 1H) | 664.0 |

(continued)

| Example Number | Structure | Name | Intermediates | $^1$H NMR (400 MHz) δ ppm | MI |
|---|---|---|---|---|---|
| **293a** | 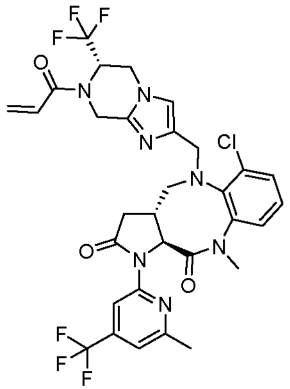 | (3a*R*,11a*S*)-5-(((*S*/*R*)-7-acryloyl-6-(trifluoromethyl)-5,6,7,8-tetrahydroimidazo[1,2-*a*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B71** | ($CDCl_3$) 8.31 (s, 1H), 7.47 - 7.45 (m, 1H), 7.35 - 7.29 (m, 2H), 7.06 (s, 1H), 6.89 (s, 1H), 6.68 - 6.61 (m, 1H), 6.55 - 6.47 (m, 1H), 5.94 (d, *J* = 10.0 Hz, 1H), 5.87 - 5.85 (m, 1H), 5.06 - 5.02 (m, 1H), 4.71 - 4.63 (m, 2H), 4.41 - 4.25 (m, 2H), 4.27 - 4.21 (m, 1H), 4.15 - 4.11 (m, 1H), 3.61 - 3.51 (m, 1H), 3.31 (s, 3H), 3.13 - 2.89 (m, 2H), 2.71 - 2.61 (m, 1H), 2.49 (s, 3H), 2.23 - 2.16 (m, 1H) | 696.1 |
| **293b** | 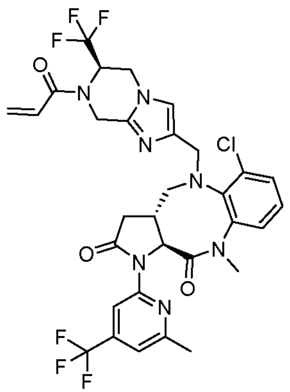 | (3a*R*,11a*S*)-5-(((*R*/*S*)-7-acryloyl-6-(trifluoromethyl)-5,6,7,8-tetrahydroimidazo[1,2-*a*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | ($CDCl_3$) 8.32 (s, 1H), 7.47 - 7.42 (m, 1H), 7.33 - 7.28 (m, 2H), 7.05 (s, 1H), 6.89 (s, 1H), 6.66 - 6.57 (m, 1H), 6.54 - 6.45 (m, 1H), 5.95 - 5.85 (m, 2H), 5.05 (d, *J* = 16.0 Hz, 1H), 4.75 - 4.61 (m, 2H), 4.36 ( d, *J* = 13.6 Hz, 1H), 4.27 - 4.16 (m, 3H), 3.66 - 3.59 (m, 1H), 3.31 (s, 3H), 3.09 - 2.96 (m, 2H), 2.71 - 2.65 (m, 1H), 2.48 (s, 3H), 2.23 - 2.16 (m, 1H) | 696.2 |

(continued)

| Example Number | Structure | Name | Intermediates | $^1$H NMR (400 MHz) δ ppm | MI |
|---|---|---|---|---|---|
| **294a** | 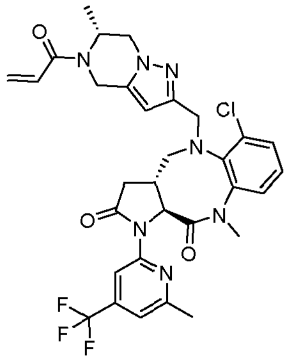 | (3a*R*,11a*S*)-5-(((*R*/*S*)-5-acryloyl-6-methyl-4,5,6,7-tetrahydropyrazolo[1,5-*a*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B72** | ($CDCl_3$) 8.33 (s, 1H), 7.48 - 7.46 (m, 1H), 7.35 - 7.30 (m, 2H), 7.06 (s, 1H), 6.64 - 6.57 (m, 1H), 6.41 - 6.36 (m, 1H), 6.20 (s, 1H), 5.82 - 5.79 (m, 1H), 5.3 - 5.23 (m, 1H), 5.51 - 4.88 (m, 1H), 4.68 (d, *J* = 9.2 Hz, 1H), 4.31 - 4.18 (m, 3H), 4.17 - 4.06 (m, 2H), 3.61 - 3.55 (m, 1H), 3.37 (s, 3H), 3.07 - 2.92 (m, 2H), 2.70 - 2.64 (m, 1H), 2.50 (s, 3H), 2.23 - 2.16 (m, 1H), 1.22 (d, *J* = 6.8 Hz, 3H) | 642.2 |
| **294b** | 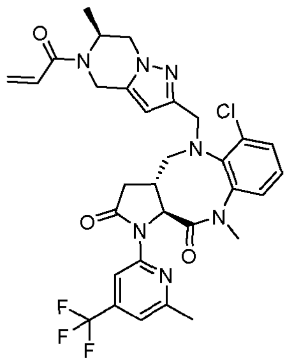 | (3a*R*,11a*S*)-5-(((*S*/*R*)-5-acryloyl-6-methyl-4,5,6,7-tetrahydropyrazolo[1,5-*a*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | ($CDCl_3$) 8.31 (s, 1H), 7.46 (dd, *J* = 7.2, 2.4 Hz, 1H), 7.35 - 7.28 (m, 2H), 7.05 (s, 1H), 6.66 - 6.54 (m, 1H), 6.37 (dd, *J* = 16.4, 1.6 Hz, 1H), 6.17 (s, 1H), 5.80 (d, *J* = 11.6 Hz, 1H), 5.43 - 4.99 (m, 2H), 4.66 (d, *J* = 9.6 Hz, 1H), 4.43 - 4.20 (m, 2H), 4.24 - 4.04 (m, 3H), 3.54 (d, *J* = 9.6 Hz, 1H), 3.36 (s, 3H), 3.11 - 2.85 (m, 2H), 2.67 (dd, *J* = 16.8, 8.4 Hz, 1H), 2.49 (s, 3H), 2.18 (dd, *J* = 16.8, 11.6 Hz, 1H), 1.24 (d, *J* = 6.8 Hz, 3H) | 642.2 |

The **Examples** in **Table 21** were prepared using methods similar to those described in the synthesis of **Example 39** or **Example 43,** using the listed **Intermediates** in step a, and the appropriate warhead in step c. For **Examples 310a/b-311a/b,** an additional chiral SFC step was conducted after step a.

**Table 21**

| Example Number | Structure | Name | Intermediates | $^1$H NMR (400 MHz) δ ppm | MI |
|---|---|---|---|---|---|
| **295** | 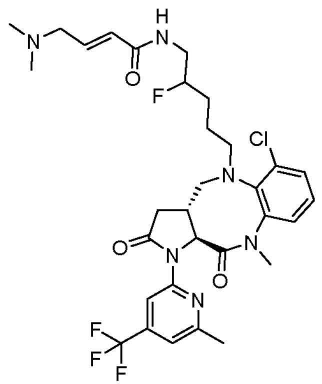 | (*E*)-*N*-(5-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-2-fluoropentyl)-4-(dimethylamino)but-2-enamide | **Step a: Intermediate 5b** and **Intermediate B88** **Step c:** *(E)-4-*(dimethylamino)but-2-enoic acid hydrochloride (CAS: 848133-35-7) | ($CDCl_3$) 8.34 (s, 1H), 7.48 - 7.41 (m, 1H), 7.35 - 7.28 (m, 2H), 7.06 (d, *J* = 3.2 Hz, 1H), 6.90 - 6.77 (m, 1H), 6.36 - 6.15 (m, 1H), 6.14 - 6.01 (m, 1H), 4.69 - 4.42 (m, 2H), 3.83 - 3.22 (m, 6H), 3.21 - 3.07 (m, 3H), 3.06 - 2.79 (m, 3H), 2.78 - 2.66 (m, 1H), 2.49 (d, *J* = 3.6 Hz, 3H), 2.32 (d, *J* = 4.8 Hz, 6H), 2.26 - 2.16 (m, 1H), 1.81 - 1.38 (m, 4H) | 653.2 |
| **296** | 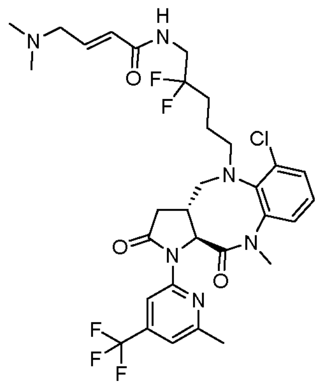 | (*E*)-N-(5-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-2,2-difluoropentyl)-4-(dimethylamino)but-2-enamide | **Step a: Intermediate 5b** and **Intermediate B89** **Step c:** *(E)-4-*(dimethylamino)but-2-enoic acid hydrochloride (CAS: 848133-35-7) | ($CDCl_3$) 8.34 (s, 1H), 7.46 - 7.44 (m, 1H), 7.37 - 7.29 (m, 2H), 7.07 (s, 1H), 6.93 - 6.78 (m, 2H), 6.37 (d, *J* = 15.6 Hz, 1H), 4.58 (d, *J* = 9.6 Hz, 1H), 3.92 - 3.75 (m, 1H), 3.53 (d, *J* = 4.4 Hz, 1H), 3.50 - 3.44 (m, 2H), 3.36 (s, 3H), 3.29 - 3.17 (m, 1H), 3.05 - 2.93 (m, 2H), 2.91 - 2.79 (m, 1H), 2.78 - 2.68 (m, 1H), 2.58 (s, 6H), 2.50 (s, 3H), 2.25 - 2.18 (m, 2H), 1.62 - 1.59 (m, 2H), 1.36 - 1.22 (m, 2H) | 671.1 |

(continued)

| Example Number | Structure | Name | Intermediates | $^1$H NMR (400 MHz) δ ppm | MI |
|---|---|---|---|---|---|
| **297** | 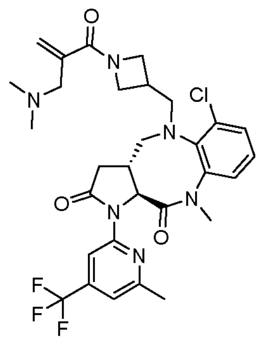 | (3a*R*,11a*S*)-6-chloro-5-((1-(2-((dimethylamino)methyl)acrylo yl)azetidin-3-yl) methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4] diazocine-2,11(3*H*)-dione | **Step a: Intermediate 5b** and *tert*-butyl 3-formylazetidine-1-carboxylate (CAS: 177947-96-5) **Step c: Intermediate E1** | ($CDCl_3$) 8.26 (s, 1H), 7.39 - 7.36 (m, 1H), 7.26 - 7.22 (m, 2H), 6.98 (s, 1H), 5.40 (s, 1H), 5.34 (s, 1H), 4.55 (d, *J* = 9.2 Hz, 1H), 4.17 - 4.13 (m, 1H), 4.07 - 3.92 (m, 1H), 3.79 - 3.67 (m, 1H), 3.66 - 3.55 (m, 1H), 3.48 (m, 1H), 3.32 (s, 2H), 3.38 - 3.27 (m, 1H), 3.15 (d, *J* = 6.4 Hz, 1H), 3.07 - 3.02 (m, 1H), 3.01 (s, 2H), 2.98 - 2.88 (m, 1H), 2.87 - 2.79 (m, 1H), 2.69 - 2.63 (m, 1H), 2.61 - 2.45 (m, 1H), 2.41 (s, 3H), 2.17 (d, *J* = 11.2 Hz, 1H), 2.13 (d, *J* = 2.4 Hz, 6H) | 619.3 |
| **298** | 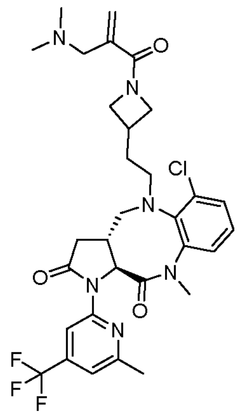 | (3a*R*,11a*S*)-6-chloro-5-(2-(1-(2-((dimethylamino)methyl)acrylo yl)azetidin-3-yl) ethyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B90** **Step c: Intermediate E1** | ($CDCl_3$) 8.35 (s, 1H), 7.48 - 7.46 (m, 1H), 7.36 - 7.31 (m, 2H), 7.07 (s, 1H), 6.10 - 5.88 (m, 1H), 5.77 - 5.58 (m, 1H), 4.63 (d, *J* = 9.2 Hz, 1H), 4.45 - 4.29 (m, 1H), 4.24 - 4.08 (m, 1H), 4.00 - 3.83 (m, 1H), 3.72 - 3.62 (m, 1H), 3.59 - 3.50 (m, 1H), 3.48 - 3.42 (m, 1H), 3.39 (s, 3H), 3.13 - 2.88 (m, 5H), 2.80 - 2.69 (m, 2H), 2.50 (s, 9H), 2.30 - 2.19 (m, 1H), 1.81 - 1.73 (m, 2H) | 633.3 |

(continued)

| Example Number | Structure | Name | Intermediates | $^1$H NMR (400 MHz) δ ppm | MI |
|---|---|---|---|---|---|
| **299** | 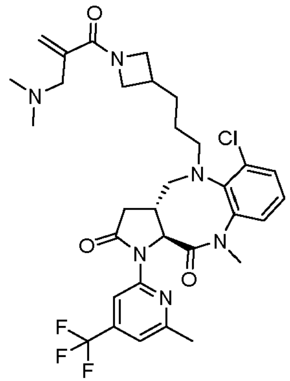 | (3a*R*,11a*S*)-6-chloro-5-(3-(1-(2-((dimethylamino)methyl)acrylo yl)azetidin-3-yl)propyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B91** **Step c: Intermediate E1** | ($CDCl_3$) 8.33 (s, 1H), 7.46 - 7.44 (m, 1H), 7.33 - 7.28 (m, 2H), 7.05 (s, 1H), 6.08 - 5.78 (m, 1H), 5.64 (s, 1H), 4.63 (d, *J* = 9.2 Hz, 1H), 4.37 - 4.31 (m, 1H), 4.20 - 4.09 (m, 1H), 3.89 - 3.76 (m, 1H), 3.70 - 3.58 (m, 1H), 3.57 - 3.33 (m, 6H), 3.04 - 2.88 (m, 4H), 2.77 - 2.71 (m, 1H), 2.61 - 2.53 (m, 4H), 2.52 (m, 6H), 2.26 - 2.19 (m, 1H), 1.68 - 1.59 (m, 2H), 1.36 - 1.22 (m, 2H) | 647.1 |
| **300** | 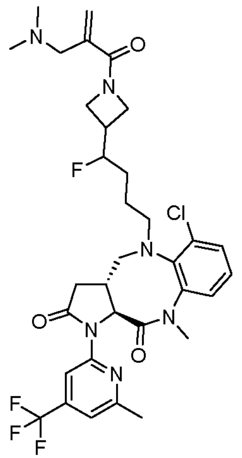 | (3a*R*,11a*S*)-6-chloro-5-(4-(1-(2-((dimethylamino)methyl)acrylo yl)azetidin-3-yl)-4-fluorobutyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B92** **Step c: Intermediate E1** | ($CDCl_3$) 8.35 (s, 1H), 7.47 - 7.45 (m, 1H), 7.35 - 7.30 (m, 2H), 7.07 (s, 1H), 6.48 - 6.20 (m, 1H), 5.89 (s, 1H), 4.76 - 4.53 (m, 2H), 4.50 - 4.03 (m, 4H), 4.01 - 3.64 (m, 3H), 3.59 - 3.52 (m, 1H), 3.39 (s, 3H), 3.14 - 2.89 (m, 5H), 2.77 - 2.66 (m, 6H), 2.50 (s, 3H), 2.27 - 2.20 (m, 1H), 1.58 - 1.43 (m, 4H) | 679.3 |

(continued)

| Example Number | Structure | Name | Intermediates | $^1$H NMR (400 MHz) δ ppm | MI |
|---|---|---|---|---|---|
| **301** | 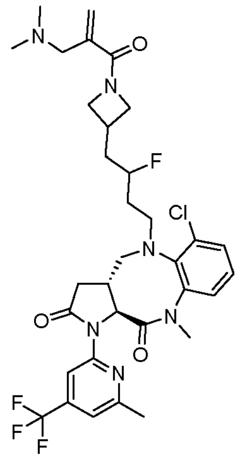 | (3a*R*,11a*S*)-6-chloro-5-(4-(1-(2-((dimethylamino)methyl)acrylo yl)azetidin-3-yl)-3-fluorobutyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B93** **Step c: Intermediate E1** | ($CDCl_3$) 8.33 (s, 1H), 7.47 - 7.45 (m, 1H), 7.34 - 7.28 (m, 2H), 7.06 (s, 1H), 5.66 (s, 1H), 5.54 (s, 1H), 4.76 - 4.55 (m, 2H), 4.37 - 4.25 (m, 1H), 4.21 (t, *J* = 9.2 Hz, 1H), 3.99 - 3.90 (m, 1H), 3.81 - 3.70 (m, 1H), 3.55 (d, *J* = 9.6 Hz, 1H), 3.37 (s, 3H), 3.35 - 3.16 (m, 3H), 3.09 - 2.92 (m, 3H), 2.91 - 2.80 (m, 1H), 2.79 - 2.69 (m, 1H), 2.48 (s, 3H), 2.44 - 2.26 (m, 6H), 2.26 - 2.19 (m, 1H), 2.03 - 1.90 (m, 2H), 1.84 -1.72 (m, 2H) | 679.2 |
| **302** | 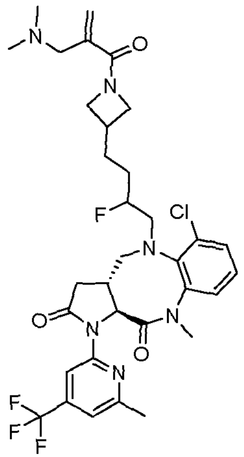 | (3*aR*,11a*S*)-6-chloro-5-(4-(1-(2-((dimethylamino)methyl)acrylo yl)azetidin-3-yl)-2-fluorobutyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B94** **Step c: Intermediate E1** | ($CDCl_3$) 8.36 (s, 1H), 7.49 - 7.47 (m, 1H), 7.38 - 7.32 (m, 2H), 7.06 (s, 1H), 5.88 (s, 1H), 5.64 (s, 1H), 4.64 (d, *J* = 9.2 Hz, 1H), 4.58 - 4.32 (m, 2H), 4.18 (t, *J* = 8.8 Hz, 1H), 3.94 - 3.86 (m, 1H), 3.70 - 3.68 (m, 1H), 3.64 - 3.58 (m, 1H), 3.44 - 3.38 (m, 3H), 3.38 (s, 2H), 3.30 - 3.16 (m, 2H), 3.10 - 2.96 (m, 2H), 2.79 - 2.74 (m, 1H), 2.68 - 2.62 (m, 1H), 2.50 (s, 3H), 2.48 (s, 5H), 2.27 - 2.20 (m, 2H), 1.84 - 1.64 (m, 4H) | 679.3 |

(continued)

| Example Number | Structure | Name | Intermediates | $^1$H NMR (400 MHz) δ ppm | MI |
|---|---|---|---|---|---|
| **303** | 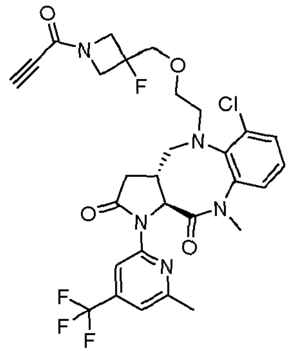 | (3a*R*,11a*S*)-6-chloro-5-(2-((3-fluoro-1-propioloylazetidin-3-yl)methoxy)ethyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B80** **Step c:** propiolic acid | ($CDCl_3$) 8.33 (s, 1H), 7.51 - 7.42 (m, 1H), 7.35 - 7.28 (m, 2H), 7.05 (s, 1H), 4.64 - 4.61 (m, 1H), 4.38 - 4.21 (m, 2H), 4.13 - 3.99 (m, 2H), 3.72 - 3.63 (m, 2H), 3.63 - 3.52 (m, 3H), 3.37 (s, 3H), 3.29 - 3.15 (m, 2H), 3.06 - 2.94 (m, 3H), 2.78 - 2.68 (m, 1H), 2.49 (s, 3H), 2.27 - 2.17 (m, 1H) | 622.3 |
| **304** | 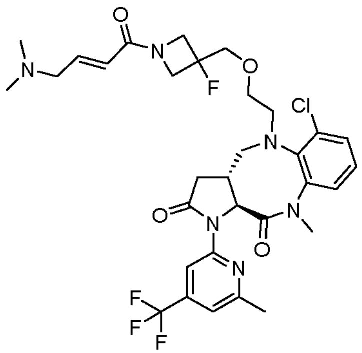 | (3a*R*,11a*S*)-6-chloro-5-(2-((1-((*E*)-4-(dimethylamino)but-2-enoyl)-3-fluoroazetidin-3-yl)methoxy)ethyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B80** **Step c:** *(E)-4-*(dimethylamino)but-2-enoic acid hydrochloride (CAS: 848133-35-7) | ($CDCl_3$) 8.33 (s, 1H), 7.47 - 7.45 (m, 1H), 7.36 - 7.28 (m, 2H), 7.06 (s, 1H), 6.93 - 6.80 (m, 1H), 6.44 (d, *J* = 15.2 Hz, 1H), 4.66 - 4.54 (m, 1H), 4.49 - 4.26 (m, 2H), 4.18 - 4.02 (m, 2H), 3.69 (s, 1H), 3.65 (s, 1H), 3.63 - 3.58 (m, 1H), 3.57 - 3.44 (m, 4H), 3.36 (d, *J* = 2.0 Hz, 3H), 3.31 - 3.17 (m, 2H), 3.04 - 2.87 (m, 2H), 2.75 - 2.65 (m, 1H), 2.62 (d, *J* = 5.2 Hz, 6H), 2.49 (s, 3H), 2.25 - 2.16 (m, 1H) | 681.4 |
| **305** | 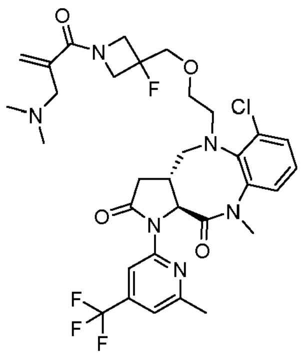 | (3a*R*,11a*S*)-6-chloro-5-(2-((1-(2-((dimethylamino)methyl)acrylo yl)-3-fluoroazetidin-3-yl)methoxy)ethyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B80** **Step c: Intermediate E1** | ($CDCl_3$) 8.32 (s, 1H), 7.46 - 7.44 (m, 1H), 7.36 - 7.28 (m, 2H), 7.05 (s, 1H), 5.93 (s, 1H), 5.71 (s, 1H), 4.61 (d, *J* = 8.8 Hz, 1H), 4.56 - 4.23 (m, 2H), 4.22 - 4.01 (m, 2H), 3.78 - 3.58 (m, 3H), 3.57 - 3.50 (m, 2H), 3.36 (s, 4H), 3.31 - 3.23 (m, 1H), 3.22 - 3.15 (m, 1H), 3.08 - 2.90 (m, 3H), 2.76 - 2.70 (m, 1H), 2.49 (s, 9H), 2.24 - 2.16 (m, 1H) | 681.4 |

(continued)

| Example Number | Structure | Name | Intermediates | $^1$H NMR (400 MHz) δ ppm | MI |
|---|---|---|---|---|---|
| **306** | 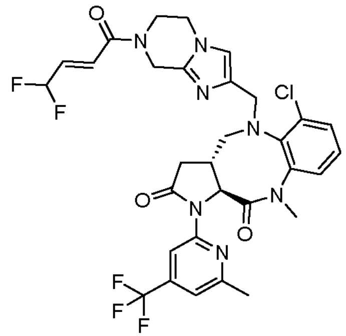 | (3a*R*,11a*S*)-6-chloro-5-((7-((*E*)-4,4-difluorobut-2-enoyl)-5,6,7,8-tetrahydroimidazo[1,2-a]pyrazin-2-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B19** **Step c: Intermediate E9** | ($CDCl_3$) 8.31 (s, 1H), 7.51 - 7.45 (m, 1H), 7.37 - 7.29 (m, 2H), 7.06 (s, 1H), 6.94 (s, 1H), 6.89 - 6.49 (m, 2H), 6.44 - 6.09 (m, 1H), 4.99 - 4.84 (m, 2H), 4.65 (d, *J* = 9.6 Hz, 1H), 4.48 - 4.30 (m, 1H), 4.22 - 3.87 (m, 5H), 3.68 - 3.54 (m, 1H), 3.34 (s, 3H), 3.12 - 3.02 (m, 1H), 3.01 - 2.89 (m, 1H), 2.73 - 2.63 (m, 1H), 2.49 (s, 3H), 2.24 - 2.15 (m, 1H) | 678.1 |
| **307** | 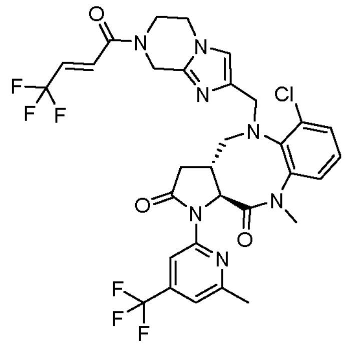 | (3a*R*,11a*S*)-6-chloro-10-methyl-1-(6-methyl-4-(trifluoromethyl)pyridin-2-yl)-5-((7-((*E*)-4,4,4-trifluorobut-2-enoyl)-5,6,7,8-tetrahydroimidazo[1,2-*a*]pyrazin-2-yl)methyl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B19** **Step c:** (*E*)-4,4,4-trifluorobut-2-enoic acid (CAS: 71027-02-6) | ($CDCl_3$) 8.31 (s, 1H), 7.50 - 7.44 (m, 1H), 7.36 - 7.28 (m, 2H), 7.06 (s, 1H), 7.03 - 6.94 (m, 1H), 6.91 (s, 1H), 6.87 - 6.74 (m, 1H), 4.94 - 4.77 (m, 2H), 4.66 (d, *J* = 9.2 Hz, 1H), 4.42 - 4.24 (m, 1H), 4.18 - 3.91 (m, 5H), 3.66 - 3.55 (m, 1H), 3.34 (s, 3H), 3.12 - 3.00 (m, 1H), 3.00 - 2.89 (m, 1H), 2.72 - 2.62 (m, 1H), 2.49 (s, 3H), 2.25 - 2.15 (m, 1H) | 696.1 |
| **308** | 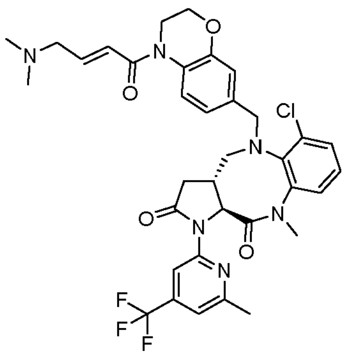 | (3a*R*,11a*S*)-6-chloro-5-((4-((*E*)-4-(dimethylamino)but-2-enoyl)-3,4-dihydro-2*H*-benzo[*b*][1,4]oxazin-7-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B76** **Step c:** *(E)-4-*(dimethylamino)but-2-enoic acid hydrochloride (CAS: 848133-35-7) | ($CDCl_3$) 8.35 (s, 1H), 7.51 - 7.49 (m, 1H), 7.35 - 7.31 (m, 2H), 7.23 - 7.12 (m, 1H), 7.10 - 7.00 (m, 3H), 6.95 - 6.93 (m, 1H), 6.57 (d, *J* = 15.2 Hz, 1H), 4.71 (d, *J* = 9.2 Hz, 1H), 4.33 (t, *J* = 4.8 Hz, 2H), 4.21 - 4.16 (m, 1H), 4.11 - 4.05 (m, 1H), 4.03 - 4.00 (m, 2H), 3.57 - 3.48 (m, 1H), 3.39 (s, 3H), 3.13 - 3.12 (m, 2H), 3.07 - 2.95 (m, 2H), 2.75 - 2.66 (m, 1H), 2.50 (s, 3H), 2.29 (s, 6H), 2.25 - 2.18 (m, 1H) | 697.4 |

(continued)

| Example Number | Structure | Name | Intermediates | $^1$H NMR (400 MHz) δ ppm | MI |
|---|---|---|---|---|---|
| **309** | 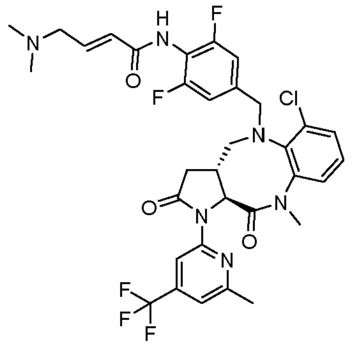 | (*E*)-*N*-(4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2,6-difluorophenyl)-4-(dimethylamino)but-2-enamide | **Step a: Intermediate 5b** and **Intermediate B79** **Step c:** *(E)-4*-(dimethylamino)but-2-enoic acid hydrochloride (CAS: 848133-35-7 | ($CDCl_3$) 8.32 (s, 1H), 7.50 - 7.48 (m, 2H), 7.35 - 7.29 (m, 2H), 7.13 - 7.04 (m, 3H), 6.97 -6.94 (m, 1H), 6.43 (d, *J* = 15.2 Hz, 1H), 4.69 (d, *J* = 9.2 Hz, 1H), 4.26 - 4.18 (m, 1H), 4.15 - 4.04 (m, 1H), 3.60 - 3.44 (m, 3H), 3.38 (s, 3H), 3.10 - 2.87 (m, 3H), 2.74 - 2.67 (m, 1H), 2.63 (s, 6H), 2.48 (s, 3H) | 691.1 |
| **310a** | 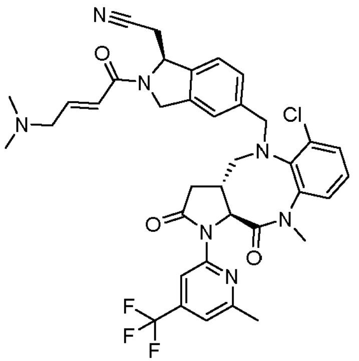 | 2-((*S*/*R*)-5-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-((*E*)-4-(dimethylamino)but-2-enoyl)isoindolin-1-yl)acetonitrile | **Step a: Intermediate 5b** and **Intermediate B57** **Step c:** (*E*)-4-(dimethylamino)but-2-enoic acid hydrochloride (CAS: 848133-35-7) | ($CDCl_3$) 8.33 (s, 1H), 7.49 (t, *J* = 4.8 Hz, 1H), 7.41 - 7.36 (m, 2H), 7.35 - 7.30 (m, 3H), 7.10 - 7.00 (m, 2H), 6.44 (d, *J* = 15.6 Hz, 1H), 5.51 (s, 1H), 5.07 - 4.99 (m, 1H), 4.95 - 4.86 (m, 1H), 4.68 (d, *J* = 9.2 Hz, 1H), 4.33 - 4.26 (m, 1H), 4.24 - 4.18 (m, 1H), 3.54 - 3.40 (m, 2H), 3.25 (s, 3H), 3.16 (d, *J* = 5.2 Hz, 2H), 3.09 - 3.00 (m, 1H), 2.99 - 2.90 (m, 2H), 2.75 - 2.61 (m, 1H), 2.49 (s, 3H), 2.32 (s, 6H), 2.25 - 2.14 (m, 1H) | 720.1 |
| **310b** | 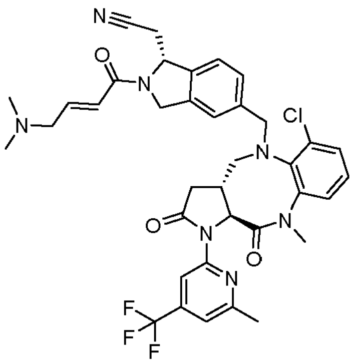 | 2-((*R*/*S*)-5-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)-2-((*E*)-4-(dimethylamino)but-2-enoyl)isoindolin-1-yl)acetonitrile | | ($CDCl_3$) 8.32 (s, 1H), 7.50 - 7.47 (m, 1H), 7.43 - 7.28 (m, 5H), 7.12 - 6.96 (m, 2H), 6.42 (d, *J* = 15.2 Hz, 1H), 5.50 (d, *J* = 2.4 Hz, 1H), 5.08 - 4.86 (m, 2H), 4.67 (d, *J* = 9.6 Hz, 1H), 4.27 (s, 2H), 3.54 - 3.43 (m, 2H), 3.29 (s, 3H), 3.15 (d, *J* = 6.0 Hz, 2H), 3.05 (s, 1H), 2. 93 - 2.88 (m, 2H), 2.70 - 2.60 (m, 1H), 2.49(s, 3H), 2.30 (s, 6H), 2.23 - 2.13 (m, 1H) | 720.1 |

(continued)

| Example Number | Structure | Name | Intermediates | $^1$H NMR (400 MHz) δ ppm | MI |
|---|---|---|---|---|---|
| **311a** | 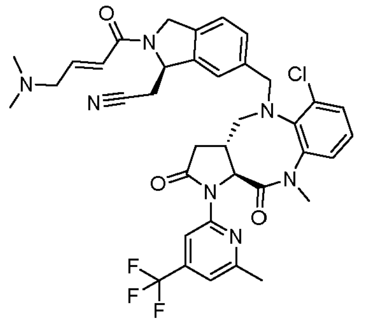 | 2-((*R*/*S*)-6-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-((*E*)-4-(dimethylamino)but-2-enoyl)isoindolin-1-yl)acetonitrile | **Step a: Intermediate 5b** and **Intermediate B58** **Step c:** (*E*)-4-(dimethylamino)but-2-enoic acid hydrochloride (CAS: 848133-35-7) | ($CDCl_3$) 8.35 (s, 1H), 7.49 - 7.47 (m, 1H), 7.42 - 7.28 (m, 5H), 7.10 - 6.99 (m, 2H), 6.42 (d, *J* = 15.2 Hz, 1H), 5.51 (s, 1H), 5.08 - 4.83 (m, 2H), 4.66 (d, *J* = 9.6 Hz, 1H), 4.41 - 4.19 (m, 2H), 3.59 - 3.54 (m, 1H), 3.50 - 3.41 (m, 1H), 3.33 (s, 3H), 3.15 (d, *J* = 5.6 Hz, 2H), 3.10 - 2.91 (m, 2H), 2.90 - 2.75 (m, 1H), 2.69 - 2.63 (m, 1H), 2.49 (s, 3H), 2.30 (s, 6H), 2.23 - 2.09 (m, 1H) | 720.1 |
| **311b** | 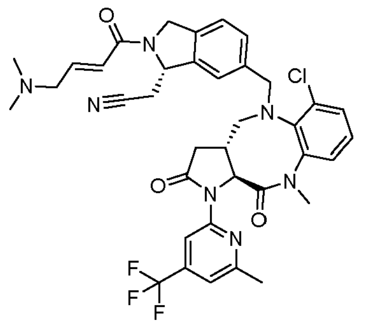 | 2-((*S*/*R*)-6-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)-2-((*E*)-4-(dimethylamino)but-2-enoyl)isoindolin-1-yl)acetonitrile | | ($CDCl_3$) 8.35 (s, 1H), 7.52 - 7.49 (m, 1H), 7.46 (s, 1H), 7.40 (d, *J* = 8.0 Hz, 1H), 7.36 - 7.32 (m, 2H), 7.31 (s, 1H), 7.10 - 7.06 (m, 1H), 7.06 - 7.01 (m, 1H), 6.50 (d, *J* = 15.6 Hz, 1H), 5.54 (s, 1H), 5.09 - 5.00 (m, 1H), 4.97 - 4.88 (m, 1H), 4.70 (d, *J* = 9.6 Hz, 1H), 4.35 - 4.22 (m, 2H), 3.60 - 3.52 (m, 1H), 3.52 - 3.43 (m, 1H), 3.36 (s, 3H), 3.29 - 3.19 (m, 2H), 3.10 - 3.05 (m, 1H), 3.04 - 2.99 (m, 1H), 2.99 - 2.90 (m, 1H), 2.73 - 2.64 (m, 1H), 2.51 (s, 3H), 2.38 (s, 6H), 2.25 - 2.16 (m, 1H) | 720.2 |

The **Examples in Table 22** were prepared using methods similar to those described in the synthesis of **Example 77,** using the listed **Intermediates** in step a. The final step was conducted in a similar manner to the amide coupling with acryloyl chloride described for **Example 1.** For **Examples 318a/b-324a/b,** an additional chiral SFC step was conducted after step b.

**Table 22**

| Example Number | Structure | Name | Intermediates | $^1$H NMR (400 MHz) δ ppm | MI |
|---|---|---|---|---|---|
| **312** | 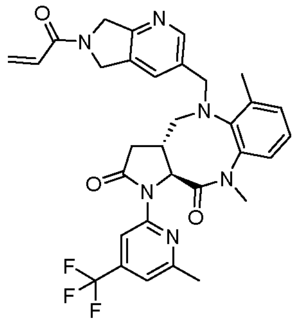 | (3a*R*,11a*S*)-5-((6-acryloyl-6,7-dihydro-5*H*-pyrrolo[3,4-*b*]pyridin-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B55** | ($CDCl_3$) 8.50 (s, 1H), 8.33 (s, 1H), 7.85 - 7.61 (m, 1H), 7.30 - 7.28 (m, 2H), 7.25 (s, 1H), 7.07 (s, 1H), 6.64 - 6.49 (m, 2H), 5.90 - 5.77 (m, 1H), 5.07 (s, 1H), 5.03 - 4.92 (m, 3H), 4.81 - 4.70 (m, 1H), 4.33 - 4.19 (m, 1H), 4.11 (t, *J* = 14.8 Hz, 1H), 3.50 - 3.41 (m, 3H), 3.37 (s, 1H), 2.90 - 2.79 (m, 1H), 2.76 - 2.60 (m, 1H), 2.51 (s, 3H), 2.47 - 2.44 (m, 3H), 2.24 - 2.11 (m, 2H) | 605.6 |
| **313** | 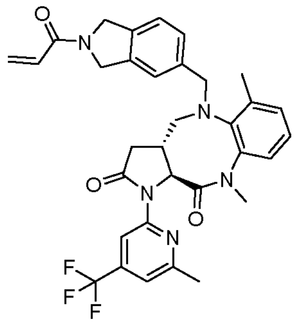 | (3a*R*,11a*S*)-5-((2-acryloylisoindolin-5-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B18** | ($CDCl_3$) 8.34 (s, 1H), 7.35 - 7.29 (m, 3H), 7.28 - 7.24 (m, 3H), 7.07 (s, 1H), 6.68 - 6.42 (m, 2H), 5.83 - 5.76 (m, 1H), 5.01 - 4.87 (m, 4H), 4.84 - 4.69 (m, 1H), 4.23 - 4.14 (m, 1H), 4.07 - 3.98 (m, 1H), 3.49 - 3.44 (m, 1H), 3.44 - 3.40 (m, 3H), 3.10 - 2.93 (m, 1H), 2.82 - 2.71 (m, 1H), 2.69 - 2.63 (m, 1H), 2.52 (s, 3H), 2.49 (d, *J* = 2.8 Hz, 3H), 2.21 - 2.11 (m, 1H) | 604.0 |
| **314** | 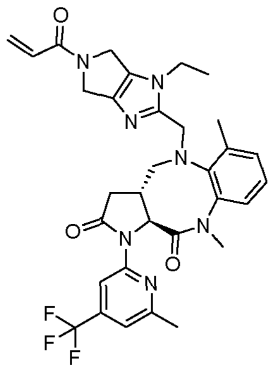 | (3a*R*,11a*S*)-5-((5-acryloyl-1-ethyl-1,4,5,6-tetrahydropyrrolo[3,4-d]imidazol-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B64** | ($CDCl_3$) 8.34 (s, 1H), 7.31 - 7.29 (m, 3H), 7.06 (s, 1H), 6.56 - 6.46 (m, 2H), 5.88 - 5.76 (m, 1H), 4.82 - 4.58 (m, 5H), 4.42 - 4.28 (m, 1H), 4.19 - 3.88 (m, 3H), 3.72 - 3.57 (m, 1H), 3.50 - 3.35 (m, 3H), 3.18 - 3.05 (m, 1H), 2.84 - 2.71 (m, 2H), 2.51 (s, 3H), 2.44 - 2.32 (m, 3H), 2.22 - 2.14 (m, 1H), 1.40 (t, *J* = 7.2 Hz, 3H) | 622.3 |

(continued)

| Example Number | Structure | Name | Intermediates | $^1$H NMR (400 MHz) δ ppm | MI |
|---|---|---|---|---|---|
| **315** | 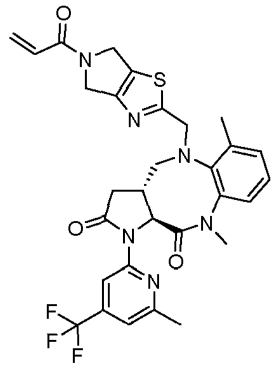 | (3a*R*,11a*S*)-5-((5-acryloyl-5,6-dihydro-4*H*-pyrrolo[3,4-d]thiazol-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B65** | ($CDCl_3$) 8.34 (s, 1H), 7.32 - 7.29 (m, 3H), 7.08 (s, 1H), 6.56 - 6.50 (m, 2H), 5.83 (t, *J* = 6.0 Hz, 1H), 4.93 - 4.76 (m, 5H), 4.64 - 4.30 (m, 2H), 3.73 - 3.65 (m, 1H), 3.45 (s, 3H), 3.18 - 3.04 (m, 1H), 3.01 - 2.86 (m, 1H), 2.78 - 2.69 (m, 1H), 2.57 - 2.51 (m, 6H), 2.28 - 2.16 (m, 1H) | 611.2 |
| **316** | 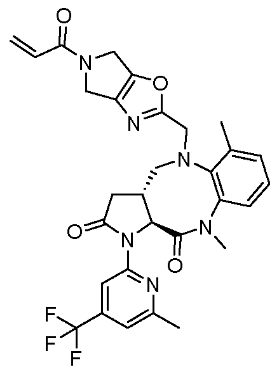 | (3a*R*,11a*S*)-5-((5-acryloyl-5,6-dihydro-4*H*-pyrrolo[3,4-*d*]oxazol-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B66** | ($CDCl_3$) 8.33 (s, 1H), 7.27 - 7.21 (m, 3H), 7.05 (s, 1H), 6.53 - 6.36 (m, 2H), 5.83 - 5.77 (m, 1H), 4.78 - 4.64 (m, 3H), 4.62 - 4.52 (m, 2H), 4.36 - 4.28 (m, 1H), 4.22 - 4.10 (m, 1H), 3.75 - 3.66 (m, 1H), 3.42 - 3.31 (m, 3H), 3.10 - 2.97 (m, 1H), 2.94 - 2.82 (m, 1H), 2.80 - 2.71 (m, 1H), 2.50 (s, 3H), 2.41 - 2.35 (m, 3H), 2.25 - 2.15 (m, 1H) | 595.2 |
| **317** | 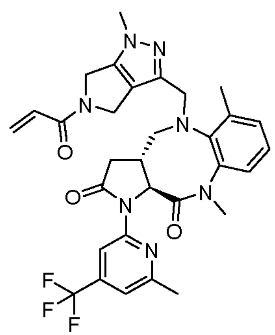 | (3a*R*,11a*S*)-5-((5-acryloyl-1-methyl-1,4,5,6-tetrahydropyrrolo[3,4-*c*]pyrazol-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B68** | ($CDCl_3$) 8.32 (s, 1H), 7.28 - 7.32 (m, 1H) , 7.24 (s, 2H), 7.05 (s, 1H), 6.42 - 6.53 (m, 2H), 5.74 - 5.86 (m, 1H), 4.61 - 4.78 (m, 4H), 4.52 - 4.60 (m, 1H), 4.11 - 4.28 (m, 1H), 3.85 - 4.06 (m, 1H), 3.76 - 3.83 (m, 3H), 3.48 - 3.59 (m, 1H), 3.38 - 3.46 (m, 3H), 2.89 - 3.13 (m, 1H), 2.65 - 2.81 (m, 2H), 2.47 - 2.52 (m, 3H), 2.43 - 2.40 (m, 3H), 2.18 - 2.14 (m, 1H) | 608.4 |

(continued)

| Example Number | Structure | Name | Intermediates | $^1$H NMR (400 MHz) δ ppm | MI |
|---|---|---|---|---|---|
| **318a** | | 2-((*R*/*S*)-6-acryloyl-2-(((3a*R*,11a*S*)-6,10-dimethyl-1-(6-methyl-4- | **Intermediate 5b** and **Intermediate B59** | ($CDCl_3$) 8.35 (s, 1H), 7.67 (d, *J* = 8.0 Hz, 1H), 7.44 (d, *J* = 8.0 Hz, 1H), 7.28 - 7.24 (m, 3H), 7.07 (s, 1H), 6.67 - 6.50 (m, 2H), | 644.1 |
| | 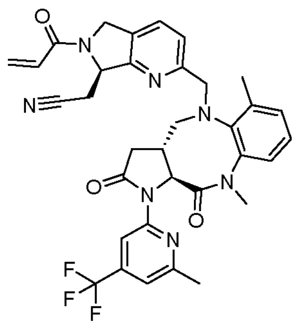 | (trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitrile | | 5.93 - 5.83 (m, 1H), 5.42 (s, 1H), 5.10 - 5.03 (m, 1H), 4.98 - 4.91 (m, 1H), 4.78 (d, *J* = 9.6 Hz, 1H), 4.45 - 4.34 (m, 1H), 4.29 - 4.20 (m, 1H), 3.74 - 3.69 (m, 1H), 3.58 - 3.54 (m, 1H), 3.44 - 3.40 (m, 3H), 3.12 ( s, 2H), 2.90 - 2.84 (m, 1H), 2.73 - 2.68 (m, 1H), 2.52 (s, 3H), 2.48 (s, 3H), 2.20 - 2.15 (m, 1H) | |
| **318b** | 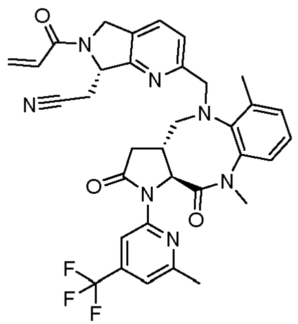 | 2-((S/R)-6-acryloyl-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitrile | | ($CDCl_3$) 8.33 (s, 1H), 7.66 (d, *J* = 8.0 Hz, 1H), 7.42 (d, *J* = 8.0 Hz, 1H), 7.26 (s, 3H), 7.06 (s, 1H), 6.61 - 6.48 (m, 2H), 5.91 - 5.80 (m, 1H), 5.43 - 5.30 (m, 1H), 5.10 - 4.86 (m, 2H), 4.78 (d, *J* = 9.6 Hz, 1H), 4.50 - 4.06 (m, 2H), 3.84 - 3.74 (m, 1H), 3.55 - 3.47 (m, 1H), 3.44 (s, 3H), 3.13 - 3.03 (m, 1H), 2.99 - 2.95 (m, 1H), 2.94 - 2.86 (m, 1H), 2.76 - 2.67 (m, 1H), 2.51 (s, 3H), 2.44 (s, 3H), 2.23 - 2.12 (m, 1H) | 644.5 |
| **319a** | | 2-((*S*/*R*)-6-acryloyl-2-(((3a*R*,11a*S*)-6,10-dimethyl-1-(6-methyl-4- | **Intermediate 5b** and **Intermediate B60** | ($CDCl_3$) 8.33 (s, 1H), 7.77 (d, *J* = 8.0 Hz, 1H), 7.45 (d, *J* = 8.0 Hz, 1H), 7.28 (s, 1H), 7.25 (s, 2H), 7.06 (s, 1H), 6.63 - 6.48 (m, | 644.4 |

(continued)

| Example Number | Structure | Name | Intermediates | $^1$H NMR (400 MHz) δ ppm | MI |
|---|---|---|---|---|---|
| | 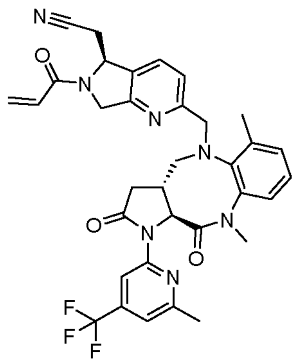 | (trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitrile | | 2H), 5.92 - 5.85 (m, 1H), 5.61 - 5.55 (m, 1H), 5.08 - 4.97 (m, 1H), 4.93 - 4.87 (m, 1H), 4.77 (d, *J* = 9.6 Hz, 1H), 4.41 - 4.34 (m, 1H), 4.27 - 4.20 (m, 1H), 3.58 - 3.51 (m, 1H), 3.37 (s, 3H), 3.35 - 3.26 (m, 1H), 3.13 - 3.03 (m, 2H), 2.92 - 2.83 (m, 1H), 2.74 - 2.63 (m, 1H), 2.50 (s, 3H), 2.48 (s, 3H), 2.23 - 2.12 (m, 1H) | |
| **319b** | 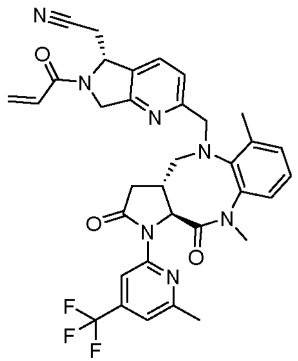 | 2-((*R*/*S*)-6-acryloyl-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitrile | | ($CDCl_3$) 8.33 (s, 1H), 7.77 (d, *J* = 8.0 Hz, 1H), 7.45 (d, *J* = 8.0 Hz, 1H), 7.27 - 7.24 (m, 3H), 7.05 (s, 1H), 6.66 - 6.44 (m, 2H), 5.97 - 5.77 (m, 1H), 5.64 - 5.48 (m, 1H), 5.06 - 4.97 (m, 1H), 4.94 - 4.85 (m, 1H), 4.76 (d, *J* = 9.6 Hz, 1H), 4.41 - 4.32 (m, 1H), 4.28 - 4.16 (m, 1H), 3.62 - 3.51 (m, 1H), 3.36 (s, 3H), 3.36 - 3.29 (m, 1H), 3.15 - 3.03 (m, 2H), 2.95 - 2.84 (m, 1H), 2.76 - 2.66 (m, 1H), 2.50 (s, 3H), 2.48 (s, 3H), 2.23 - 2.12 (m, 1H) | 644.3 |
| **320a** | | 2-((*R*/*S*)-6-acryloyl-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitrile | **Intermediate 5b** and**Intermediate B61** | ($CDCl_3$) 8.54 (s, 1H), 8.35 (s, 1H), 7.78 (s, 1H), 7.29 (s, 3H), 7.06 (s, 1H), 6.63 - 6.49 (m, 2H), 5.96 - 5.83 (m, 1H), 5.59 (s, 1H), 5.09 - 4.98 (m, 1H), 4.98 - 4.86 (m, 1H), 4.73 (d, *J* = 8.8 Hz, 1H), 4.21 (s, 2H), 3.43 (s, 3H), 3.40 (s, 1H), 3.37 (d, *J* = 5.6 Hz, 1H), 3.24 - 3.13 (m, 1H), 2.91 - 2.78 (m, 2H), 2.73 - 2.64 (m, 1H), 2.52 (s, 3H), 2.47 (s, 3H), 2.19 - 2.07 (m, 1H) | 644.6 |

(continued)

| Example Number | Structure | Name | Intermediates | $^1H$ NMR (400 MHz) δ ppm | MI |
|---|---|---|---|---|---|
| 320b | 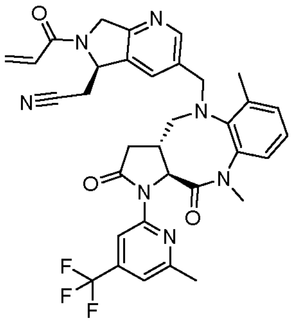 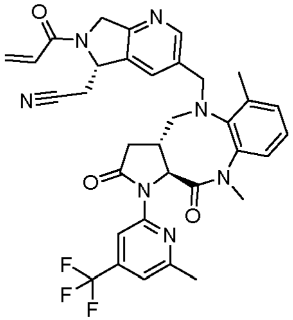 | 2-((*S*/*R*)-6-acryloyl-3-(((3a*R*,11a*S*)-6,10-dimethyl-1-(6-methyl-4-(trifluoromethyl) pyridin-2-yl)-2,11-dioxo-1,2,3,3a,4,10,11,11a-octahydro-5*H*-benzo[*b*]pyrrolo[2,3-*f*][1,4]diazocin-5-yl) methyl)-6,7-dihydro-5*H*-pyrrolo[3,4-*b*]pyridin-5-yl)acetonitrile | | ($CDCl_3$) 8.55 (s, 1H), 8.34 (s, 1H), 7.78 (s, 1H), 7.30 - 7.27 (m, 3H), 7.07 (s, 1H), 6.62 - 6.50 (m, 2H), 5.92 - 5.86 (m, 1H), 5.59 (d, *J* = 3.2 Hz, 1H), 5.08 - 4.87 (m, 2H), 4.76 (d, *J* = 9.6 Hz, 1H), 4.30 - 4.10 (m, 2H), 3.51 - 3.44 (m, 1H), 3.42 (s, 3H), 3.38 - 3.26 (m, 1H), 3.20 - 3.11 (m, 1H), 3.03 - 2.92 (m, 1H), 2.90 - 2.80 (m, 1H), 2.74 - 2.63 (m, 1H), 2.51 (s, 3H), 2.46 (s, 3H), 2.24 - 2.12 (m, 1H) | 644.2 |
| 321a | | 2-((*S*/*R*)-6-acryloyl-3-(((3a*R*,11a*S*)-6,10-dimethyl-1-(6-methyl-4- | **Intermediate 5b** and **Intermediate B62** | ($CDCl_3$) 8.54 (s, 1H), 8.34 (s, 1H), 7.66 (s, 1H), 7.30 - 7.28 (m, 2H), 7.27 - 7.23 (m, 1H), 7.07 (s, 1H), 6.63 - 6.51 (m, 2H), 5.91 | 644.3 |

(continued)

| Example Number | Structure | Name | Intermediates | $^1$H NMR (400 MHz) δ ppm | MI |
|---|---|---|---|---|---|
| | 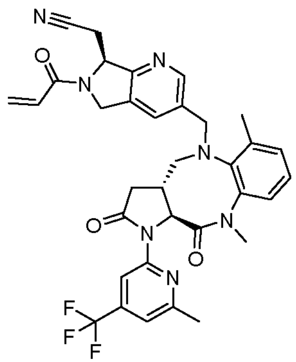 | (trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitrile | | - 5.85 (m, 1H), 5.50 - 5.42 (m, 1H), 5.15 - 5.05 (m, 1H), 5.01 - 4.91 (m, 1H), 4.75 (d, *J* = 9.6 Hz, 1H), 4.27 (d, *J* = 14.8 Hz, 1H), 4.08 (d, *J* = 14.8 Hz, 1H), 3.77 - 3.63 (m, 1H), 3.53 - 3.43 (m, 1H), 3.35 (s, 3H), 3.12 - 2.98 (m, 2H), 2.90 - 2.78 (m, 1H), 2.76 - 2.65 (m, 1H), 2.51 (s, 3H), 2.47 (s, 3H), 2.24 - 2.12 (m, 1H) | |
| 321b | 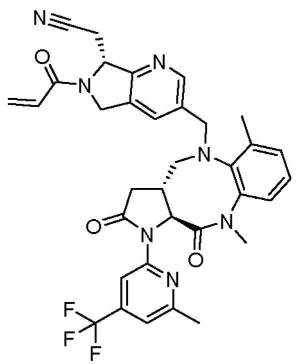 | 2-((*R*/*S*)-6-acryloyl-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitrile | | ($CDCl_3$) 8.54 (s, 1H), 8.33 (s, 1H), 7.68 (s, 1H), 7.31 - 7.28 (m, 3H), 7.07 (s, 1H), 6.65 - 6.48 (m, 2H), 5.94 - 5.81 (m, 1H), 5.45 (s, 1H), 5.16 - 5.04 (m, 1H), 5.03 - 4.93 (m, 1H), 4.74 (d, *J* = 9.2 Hz, 1H), 4.29 - 4.19 (m, 1H), 4.18 - 4.07 (m, 1H), 3.79 - 3.67 (m, 1H), 3.49 - 3.42 (m, 1H), 3.38 (s, 3H), 3.12 - 3.04 (m, 1H), 2.98 - 2.90 (m, 1H), 2.89 - 2.81 (m, 1H), 2.72 - 2.64 (m, 1H), 2.51 (s, 3H), 2.48 (s, 3H), 2.22 - 2.13 (m, 1H) | 644.3 |
| 322a | | (3a*R*,11a*S*)-5-(((*S*/*R*)-7-acryloyl-6-(trifluoromethyl)-5,6,7,8-tetrahydroimidazo[1,2-*a*]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B71** | ($CDCl_3$) 8.32 (s, 1H), 7.24 (s, 3H), 7.05 (s, 1H), 6.77 (s, 1H), 6.70 - 6.59 (m, 1H), 6.56 - 6.44 (m, 1H), 6.03 - 5.79 (m, 2H), 5.06 (d,*J* = 16.4 Hz, 1H), 4.74 (d, *J* = 9.6 Hz, 2H), 4.35 (d, *J* = 13.6 Hz, 1H), 4.26 - 4.18 (m, 1H), 4.14 - 4.02 (m, 2H), 3.66 - 3.55 (m, 1H), 3.36 (s, 3H), 3.08 - 2.94 (m, 1H), 2.88 - 2.75 (m, 1H), 2.73 - 2.63 (m, 1H), 2.51 (s, 3H), 2.42 (s, 3H), 2.22 - 2.12 (m, 1H) | 676.2 |

(continued)

| Example Number | Structure | Name | Intermediates | $^1H$ NMR (400 MHz) δ ppm | MI |
|---|---|---|---|---|---|
| **322b** | 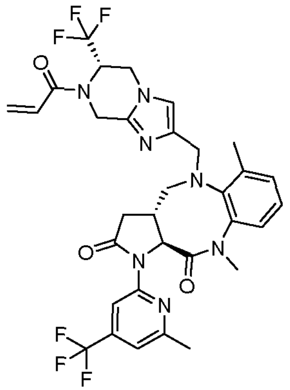 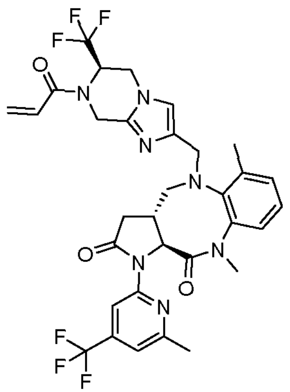 | (3a*R*,11a*S*)-5-(((*R*/*S*)-7-acryloyl-6-(trifluoromethyl)-5,6,7,8-tetrahydroimidazo[1,2-a]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | ($CDCl_3$) 8.32 (s, 1H), 7.26 - 7.20 (m, 3H), 7.05 (s, 1H), 6.79 (s, 1H), 6.68 - 6.58 (m, 1H), 6.55 - 6.45 (m, 1H), 6.02 - 5.81 (m, 2H), 5.05 (d, *J* = 16.0 Hz, 1H), 4.79 - 4.62 (m, 2H), 4.37 (d, *J* = 14.0 Hz, 1H), 4.24 - 4.19 (m, 1H), 4.18 - 4.10 (m, 1H), 4.07 - 3.97 (m, 1H), 3.66 - 3.57 (m, 1H), 3.36 (s, 3H), 3.12 - 2.96 (m, 1H), 2.84 - 2.74 (m, 1H), 2.73 - 2.64 (m, 1H), 2.50 (s, 3H), 2.43 (s, 3H), 2.22 - 2.12 (m, 1H) | 676.3 |
| **323a** | | (3a*R*,11a*S*)-5-(4-((*R*/*S*)-4-acryloylmorpholin-3-yl)benzyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B95** | (DMSO-$d_6$) 8.18 (s, 1H), 7.37 - 7.24 (m, 8H), 6.97 - 6.64 (m, 1H), 6.24 - 6.16 (m, 1H), 5.73 (d, *J* = 10.0 Hz, 1H), 5.59 - 5.14 (m, 1H), 4.68 (d, *J* = 8.8 Hz, 1H), 4.39 (d, *J* = 12.0 Hz, 1H), 4.30 - 3.95 (m, 2H), 3.92(d, *J* = 14.0 Hz, 1H), 3.86 - 3.70 (m, 2H), 3.52 - 3.41 (m, 1H), 3.27 (s, 4H), 3.26 - 3.04 (m, 1H), 2.93 - 2.82 (m, 2H), 2.58 - 2.51 (m, 1H), 2.49 (s, 3H), 2.40 (s, 3H), 2.38 - 2.30 (m, 1H) | 648.3 |

(continued)

| Example Number | Structure | Name | Intermediates | $^1H$ NMR (400 MHz) δ ppm | MI |
|---|---|---|---|---|---|
| **323b** | 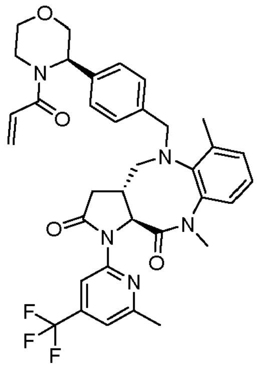 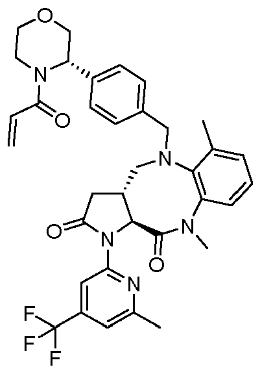 | (3a*R*,11a*S*)-5-(4-((*S*/*R*)-4-acryloylmorpholin-3-yl)benzyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | (DMSO-$d_6$) 8.47 (d, *J* = 1.6 Hz, 1H), 8.18 (s, 1H), 7.73 (d, *J* = 1.2 Hz, 1H), 7.38 - 7.23 (m, 5H), 7.01 - 6.64 (m, 1H), 6.17 (d, *J* = 16.8 Hz, 1H), 5.83 - 5.59 (m, 1H), 5.50 - 5.19 (m, 1H), 4.72 - 4.56 (m, 2H), 4.25 - 3.93 (m, 3H), 3.84 - 3.68 (m, 2H), 3.53 - 3.40 (m, 2H), 3.31 - 3.22 (m, 4H), 2.96 - 2.88 (m, 2H), 2.55 (s, 1H), 2.49 (s, 4H), 2.37 (s, 3H), 2.35 - 2.30 (m, 1H) | 648.3 |
| **324a** | | (3a*R*,11a*S*)-5-((6-((R/S)-4-acryloylmorpholin-3-yl)pyridin-3-yl)methyl)-6,10-dimethyl-1-(6-methyl-4-(trifluoromethyl)pyridin-2-yl)-1,3a,4,5,10,11a- | **Intermediate 5b** and **Intermediate B96** | ($CDCl_3$) 8.47 (s, 1H), 8.18 (s, 1H), 7.73 (d, *J* = 8.0 Hz, 1H), 7.35 (s, 1H), 7.33 - 7.29 (m, 1H), 7.29 - 7.17 (m, 3H), 7.01 - 6.63 (m, 1H), 6.18 (d, *J* = 15.2 Hz, 1H), 5.85 - 5.60 (m, 1H), 5.51 - 5.17 (m, 1H), 4.67 (d, *J* = | 649.3 |

(continued)

| Example Number | Structure | Name | Intermediates | $^1$H NMR (400 MHz) δ ppm | MI |
|---|---|---|---|---|---|
| | 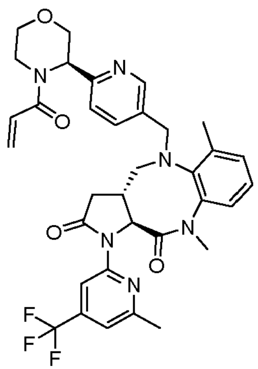 | hexahydro-2*H*-benzo[*b*]pyrrolo[2,3-*f*][1,4]diazocine-2,11(3*H*)-dione | | 8.8 Hz, 1H), 4.64 - 4.56 (m, 1H), 4.27 - 3.89 (m, 3H), 3.86 - 3.66 (m, 2H), 3.53 - 3.33 (m, 2H), 3.30 - 3.19 (m, 4H), 2.96 - 2.86 (m, 2H), 2.62 - 2.53 (m, 1H), 2.49 (s, 3H), 2.41 - 2.30 (m, 4H) | |
| **324b** | 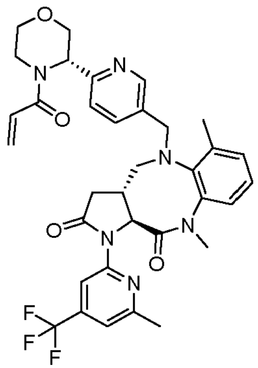 | (3a*R*,11a*S*)-5-((6-((*S*/*R*)-4-acryloylmorpholin-3-yl)pyridin-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | ($CDCl_3$) 8.47 (s, 1H), 8.18 (s, 1H), 7.73 (d, *J* = 1.2 Hz, 1H), 7.38 - 7.23 (m, 5H), 7.01 - 6.64 (m, 1H), 6.17 (d, *J* = 16.8 Hz, 1H), 5.83 - 5.59 (m, 1H), 5.50 - 5.19 (m, 1H), 4.72 - 4.56 (m, 2H), 4.25 - 3.93 (m, 3H), 3.84 - 3.68 (m, 2H), 3.53 - 3.40 (m, 2H), 3.31 - 3.22 (m, 4H), 2.96 - 2.88 (m, 2H), 2.55 (s, 1H), 2.49 (s, 4H), 2.37 (s, 3H) | 649.3 |
| **325a** | | (3a*R*,11a*S*)-5-((1-((3*S*/*R*,4*S*/*R*)-1-acryloyl-4-fluoropyrrolidin-3-yl)-1*H*-pyrazol-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B99a** | (400 MHz, $CDCl_3$) δ ppm 8.33 (s, 1H), 7.47 - 7.28 (m, 3H), 7.26 - 7.21 (m, 2H), 7.05 (s, 1H), 6.51 - 6.38 (m, 2H), 5.82 - 5.73 (m, 1H), 5.53 - 5.20 (m, 1H), 5.09 - 4.85 (m, 1H), 4.72 (d, *J* = 9.6 Hz, 1H), 4.24 - 3.80 (m, 6H), 3.57 - 3.46 (m, 1H), 3.39 - 3.26 (m, 3H), 3.03 - 2.84 (m, 1H), 2.80 - 2.63 (m, 2H), 2.53 - 2.47 (m, 3H), 2.40 (d, *J* = 5.2 Hz, 3H), 2.24 - 2.10 (m, 1H) | 640.3 |

(continued)

| Example Number | Structure | Name | Intermediates | $^1$H NMR (400 MHz) δ ppm | MI |
|---|---|---|---|---|---|
| **325b** | 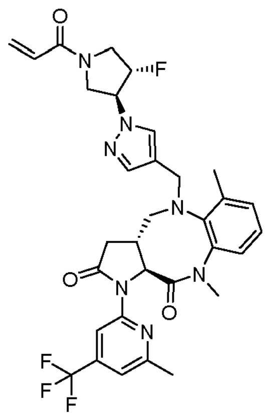 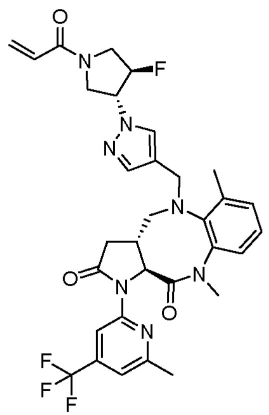 | (3a*R*,11a*S*)-5-((1-((3*R*/*S*,4*R*/*S*)-1-acryloyl-4-fluoropyrrolidin-3-yl)-1*H*-pyrazol-4-yl)methyl)-6,10-dimethyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione | **Intermediate 5b** and **Intermediate B99b** | ($CDCl_3$) 8.33 (s, 1H), 7.44 (s, 2H), 7.28 - 7.20 (m, 3H), 7.06 (s, 1H), 6.53 - 6.37 (m, 2H), 5.83 - 5.72 (m, 1H), 5.52 - 5.18 (m, 1H), 5.06 - 4.85 (m, 1H), 4.77 - 4.59 (m, 1H), 4.26 - 3.78 (m, 6H), 3.61 - 3.45 (m, 1H), 3.39 - 3.24 (m, 3H), 3.07 - 2.83 (m, 1H), 2.81 - 2.62 (m, 2H), 2.53 - 2.46 (m, 3H), 2.40 (s, 3H), 2.27 - 2.10 (m, 1H) | 640.3 |
| **325c** | | (3a*R*,11a*S*)-5-((1-((3*R*/*S*,4*S*/*R*)-1-acryloyl-4-fluoropyrrolidin-3-yl)-1*H*-pyrazol-4-yl)methyl)-6,10-dimethyl-1-(6-methyl-4- | **Intermediate 5b** and **Intermediate B99c** | ($CDCl_3$) 8.33 (s, 1H), 7.53 - 7.45 (m, 2H), 7.27 - 7.20 (m, 3H), 7.05 (s, 1H), 6.51 - 6.36 (m, 2H), 5.83 - 5.76 (m, 1H), 5.43 - 5.24 (m, 1H), 5.06 - 4.89 (m, 1H), 4.77 - | 640.3 |

(continued)

| Example Number | Structure | Name | Intermediates | $^1$H NMR (400 MHz) δ ppm | MI |
|---|---|---|---|---|---|
| | 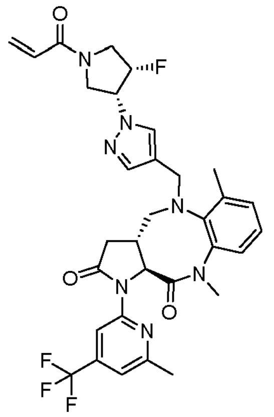 | (trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | 4.70 (m, 1H), 4.39 - 4.22 (m, 1H), 4.16 - 3.98 (m, 3H), 3.96 - 3.76 (m, 2H), 3.56 - 3.51 (m, 1H), 3.37 (d, *J* = 2.0 Hz, 3H), 3.02 - 2.87 (m, 1H), 2.80 - 2.63 (m, 2H), 2.50 (d, *J* = 2.0 Hz, 3H), 2.42 (d, *J* = 7.2 Hz, 3H), 2.20 - 2.13 (m, 1H) | |
| **325d** | 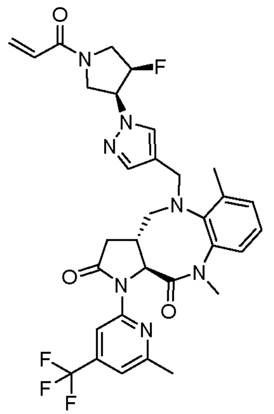 | (3a*R*,11a*S*)-5-((1-((3*S*/*R*,4*R*/*S*)-1-acryloyl-4-fluoropyrrolidin-3-yl)-1*H*-pyrazol-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B99d** | ($CDCl_3$) 8.33 (s, 1H), 7.55 - 7.44 (m, 2H), 7.27 - 7.20 (m, 3H), 7.06 (s, 1H), 6.58 - 6.34 (m, 2H), 5.87 - 5.70 (m, 1H), 5.46 - 5.22 (m, 1H), 5.06 - 4.86 (m, 1H), 4.74 (d, *J* = 9.6 Hz, 1H), 4.41 - 4.23 (m, 1H), 4.19 - 3.97 (m, 3H), 3.97 - 3.76 (m, 2H), 3.58 - 3.49 (m, 1H), 3.37 (d, *J* = 4.0 Hz, 3H), 3.03 - 2.86 (m, 1H), 2.81 - 2.61 (m, 2H), 2.50 (s, 3H), 2.42 (d, *J* = 4.4 Hz, 3H), 2.23 - 2.10 (m, 1H) | 640.3 |
| **326a** | | (3a*R*,11a*S*)-5-((1-((3S/R,4R/S)-1-acryloyl-4-fluoropyrrolidin-3-yl)-1*H*-imidazol-4-yl)methyl)-6,10-dimethyl-1-(6-methyl-4- | **Intermediate 5b** and **Intermediate B102a** | ($CDCl_3$) 8.32 (s, 1H), 7.66 - 7.61 (m, 1H), 7.25 (s, 3H), 7.05 (s, 1H), 7.00 (d, *J* = 10.0 Hz, 1H), 6.51 - 6.33 (m, 2H), 5.85 - 5.78 (m, 1H), 5.40 - 5.18 (m, 1H), 4.81 - 4.63 (m, | 640.3 |

(continued)

| Example Number | Structure | Name | Intermediates | $^1$H NMR (400 MHz) δ ppm | MI |
|---|---|---|---|---|---|
| | 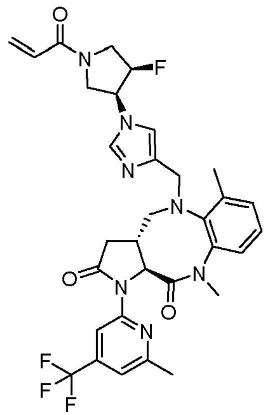 | (trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | | 2H), 4.36 - 4.17 (m, 1H), 4.16 - 4.11 (m, 2H), 4.10 - 3.78 (m, 3H), 3.64 - 3.58 (m, 1H), 3.36 (d, *J* = 3.2 Hz, 3H), 3.04 - 2.92 (m, 1H), 2.87 - 2.77 (m, 1H), 2.73 - 2.64 (m, 1H), 2.51 (s, 3H), 2.44 (d, *J* = 3.2 Hz, 3H), 2.17 (m, 1H) | |
| **326b** | 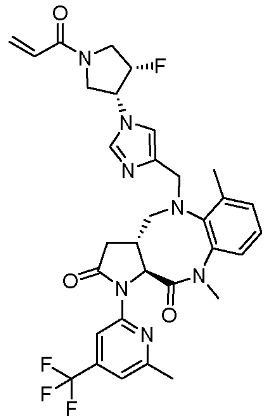 | (3a*R*,11a*S*)-5-((1-((3*R*/*S*,4*S*/*R*)-1-acryloyl-4-fluoropyrrolidin-3-yl)-1*H*-imidazol-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Intermediate 5b** and **Intermediate B102b** | ($CDCl_3$) 8.31 (s, 1H), 7.94 - 7.74 (m, 1H), 7.24 (s, 3H), 7.11 - 7.03 (m, 2H), 6.51 - 6.30 (m, 2H), 5.86 - 5.74 (m, 1H), 5.40 - 5.22 (m, 1H), 4.92 - 4.67 (m, 2H), 4.38 - 4.22 (m, 1H), 4.18 - 4.08 (m, 2H), 4.04 - 3.73 (m, 3H), 3.65 - 3.60 (m, 1H), 3.33 (d, *J* = 4.0 Hz, 3H), 3.05 - 2.93 (m, 1H), 2.88 - 2.78 (m, 1H), 2.74 - 2.65 (m, 1H), 2.51 (s, 3H), 2.42 ( d, *J* = 3.2 Hz, 3H), 2.22 - 2.15 (m, 1H) | 640.2 |

The **Examples** in **Table 23** were prepared using methods similar to those described in the synthesis of **Example 77,** using the listed **Intermediates** in step a, and the appropriate warhead in step d. For **Examples 329a/b-330a/b,** an additional chiral SFC step was conducted after step a. For **Examples 331-332,** epimerisation occurred during step a, and the isomers were separated by chiral SFC after step b. The absolute configuration of **Examples 331-332** was determined by 2D NMR.

**Table 23**

| Example Number | Structure | Name | Intermediates | $^1$H NMR (400 MHz) δ ppm | MI |
|---|---|---|---|---|---|
| **327** | 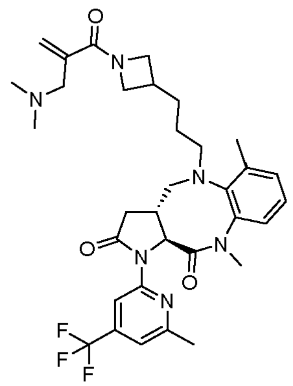 | (3a*R*,11a*S*)-5-(3-(1-(2-((dimethylamino) methyl)acryloyl) azetidin-3-yl)propyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione | **Step a: Intermediate 5b** and **Intermediate B91 Step d: Intermediate E1** | ($CDCl_3$) 8.33 (s, 1H), 7.26 - 7.18 (m, 3H), 7.04 (s, 1H), 5.58 (s, 1H), 5.46 (s, 1H), 4.70 (d, *J* = 9.2 Hz, 1H), 4.33 - 4.23 (m, 1H), 4.17 - 4.07 (m, 1H), 3.82 - 3.71 (m, 1H), 3.66 - 3.51 (m, 2H), 3.35 (s, 3H), 3.26 - 3.06 (m, 2H), 3.02 - 2.88 (m, 3H), 2.85 - 2.69 (m, 2H), 2.57 - 2.47 (m, 4H), 2.37 (s, 3H), 2.28 (s, 6H), 2.23 - 2.16 (m, 1H), 1.66 - 1.55 (m, 2H), 1.37 - 1.26 (m, 2H) | 627.3 |
| **328** | 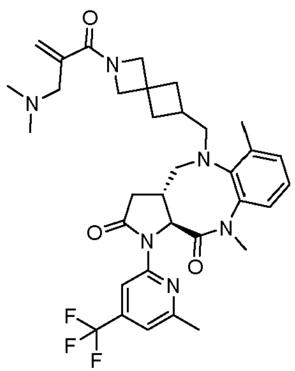 | (3a*R*,11a*S*)-5-((2-(2-((dimethylamino) methyl)acryloyl) -2-azaspiro[3.3]heptan-6-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4] diazocine-2,11(3*H*)-dione | **Step a: Intermediate 5b** and *tert*-butyl 6-formyl-2-azaspiro[3.3] heptane -2-carboxylate (CAS: 1440960-67-7) **Step d: Intermediate E1** | ($CDCl_3$) 8.33 (s, 1H), 7.26 - 7.16 (m, 3H), 7.04 (s, 1H), 5.61 (s, 1H), 5.48 (s, 1H), 4.68 (d, *J* = 9.2 Hz, 1H), 4.20 (s, 1H), 4.06 (d, *J* = 8.8 Hz, 2H), 3.93 (s, 1H), 3.59 - 3.46 (m, 1H), 3.36 (s, 3H), 3.18 (s, 2H), 2.96 (d, *J* = 6.0 Hz, 2H), 2.90 - 2.82 (m, 1H), 2.81 - 2.67 (m, 2H), 2.49 (s, 3H), 2.35 (s, 3H), 2.29 (s, 6H), 2.23 (d, *J* = 6.0 Hz, 2H), 2.20 - 2.12 (m, 2H), 1.84 (s, 2H) | 639.0 |

(continued)

| Example Number | Structure | Name | Intermediates | $^1$H NMR (400 MHz) δ ppm | MI |
|---|---|---|---|---|---|
| **329a** | 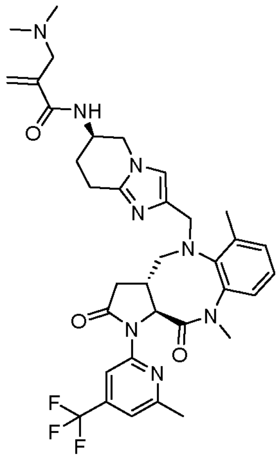 | N-((*R*/*S*)-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,6,7,8-tetrahydroimidazo[1,2-a]pyridin-6-yl)-2-((dimethylamino)methyl)acrylami de | **Step a: Intermediate 5b** and **Intermediate B73 Step d: Intermediate E1** | ($CDCl_3$) 9.95 (d, *J* = 6.8 Hz, 1H), 8.32 (s, 1H), 7.25 - 7.19 (m, 3H), 7.04 (s, 1H), 6.62 (s, 1H), 6.24 (d, *J* = 1.6 Hz, 1H), 5.42 (s, 1H), 4.74 (d, *J* = 9.6 Hz, 1H), 4.56 - 4.47 (m, 1H), 4.16 - 4.08 (m, 2H), 4.04 - 3.97 (m, 1H), 3.93 - 3.86 (m, 1H), 3.67 - 3.60 (m, 1H), 3.37 (s, 3H), 3.14 - 2.96 (m, 4H), 2.92 - 2.82 (m, 1H), 2.81 - 2.72 (m, 1H), 2.71 - 2.63 (m, 1H), 2.49 (s, 3H), 2.43 (s, 3H), 2.20 - 2.06 (m, 9H) | 679.3 |
| **329b** | 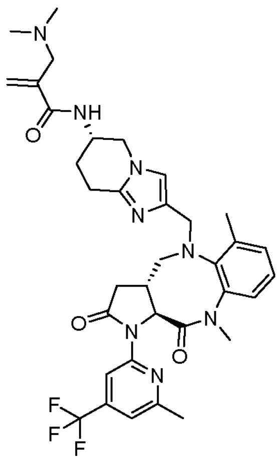 | N-(*S*/*R*)-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,6,7,8-tetrahydroimidazo[1,2-a]pyridin-6-yl)-2-((dimethylamino)methyl)acrylami de | | ($CDCl_3$) 9.93 (d, *J* = 7.6 Hz, 1H), 8.32 (s, 1H), 7.25 - 7.19 (m, 3H), 7.09 - 6.99 (m, 1H), 6.62 (s, 1H), 6.25 (d, *J* = 1.6 Hz, 1H), 5.42 (s, 1H), 4.74 (d, *J* = 9.6 Hz, 1H), 4.57 - 4.44 (m, 1H), 4.20 - 4.07 (m, 2H), 4.03 - 3.95 (m, 1H), 3.91 - 3.81 (m, 1H), 3.68 - 3.63 (m, 1H), 3.38 (s, 3H), 3.16 - 3.00 (m, 3H), 2.99 - 2.83 (m, 2H), 2.81 - 2.63 (m, 2H), 2.50 (s, 3H), 2.42 (s, 3H), 2.20 - 1.99 (m, 9H) | 679.4 |

(continued)

| Example Number | Structure | Name | Intermediates | $^1$H NMR (400 MHz) δ ppm | MI |
|---|---|---|---|---|---|
| **330a** | 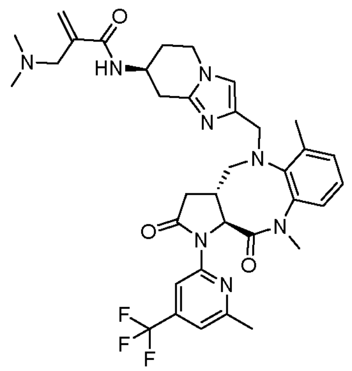 | *N*-((*S*/*R*)-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,6,7,8-tetrahydroimidazo[1,2-*a*]pyridin-7-yl)-2-((dimethylamino)methyl)acrylami de | **Step a: Intermediate 5b** and **Intermediate B74 Step d: Intermediate E1** | ($CDCl_3$) 9.71 - 9.68 (m, 1H), 8.32 (s, 1H), 7.27 - 7.20 (m, 3H), 7.05 (s, 1H), 6.70 (s, 1H), 6.24 (s, 1H), 5.41 (s, 1H), 4.74 (d, *J* = 9.6 Hz, 1H), 4.51 - 4.45 (m, 1H), 4.12 - 3.93 (m, 4H), 3.62 (m, 1H), 3.37 (s, 3H), 3.18 - 2.98 (m, 4H), 2.80 - 2.74 (m, 2H), 2.70 - 2.65 (m, 1H), 2.50 (s, 3H), 2.44 (s, 3H), 2.28 - 2.23 (m, 1H), 2.20 - 2.03 (m, 8H) | 679.6 |
| **330b** | 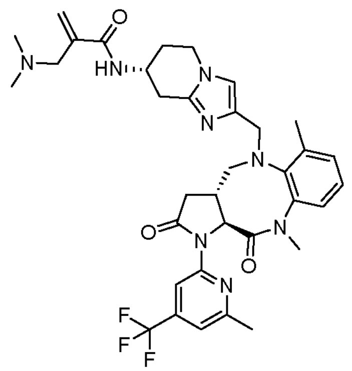 | *N*-((*R*/*S*)-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,6,7,8-tetrahydroimidazo[1,2-a]pyridin-7-yl)-2-((dimethylamino)methyl)acrylami de | | ($CDCl_3$) 9.69 (d, *J* = 7.2 Hz, 1H), 8.32 (s, 1H), 7.25 - 7.19 (m, 3H), 7.05 (s, 1H), 6.68 (s, 1H), 6.24 (d, *J* = 2.0 Hz, 1H), 5.41 (s, 1H), 4.74 (d, *J* = 9.6 Hz, 1H), 4.53 - 4.38 (m, 1H), 4.14 - 3.91 (m, 4H), 3.69 - 3.61 (m, 1H), 3.37 (s, 3H), 3.20 - 2.97 (m, 4H), 2.83 - 2.72 (m, 2H), 2.71 - 2.62 (m, 1H), 2.50 (s, 3H), 2.43 (s, 3H), 2.32 - 2.21 (m, 1H), 2.20 - 2.15 (m, 1H), 2.13 (s, 5H), 2.13 - 2.01 (m, 2H) | 679.6 |
| **331** | 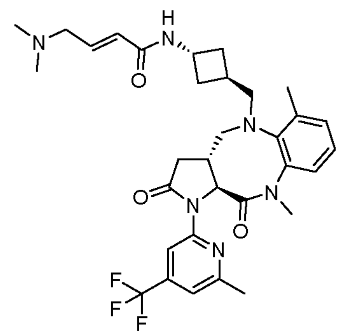 | (*E*)-*N*-((1*R*,3*r*)-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)cyclobutyl)-4-(dimethylamino)but-2-enamide | **Step a: Intermediate 5b** and *tert*-butyl ((*trans*)-3-formylcyclobutyl)car bamate (CAS: 171549-92-1)<br>**Step d:** (*E*)-4-(dimethylamino)but-2-enoic acid hydrochloride (CAS: 848133-35-7) | ($CDCl_3$) 8.33 (s, 1H), 7.24 - 7.17 (m, 3H), 7.04 (s, 1H), 6. 82 - 6.70 (m, Hz, 1H), 6.40 - 6.21 (m, 2H), 4.70 (d, *J* = 9.6 Hz, 1H), 4.49 - 4.36 (m, 1H), 3.61 - 3.54 (m, 1H), 3.48 (d, *J* = 6.0 Hz, 2H), 3.37 (s, 3H), 3.13 - 3.06 (m, 1H), 3.01 - 2.92 (m, 2H), 2.82 - 2.70 (m, 2H), 2.65 - 2.58 (m, 5H), 2.49 (s, 3H), 2.34 (s, 3H), 2.32 - 2.00 (m, 7H) | 613.4 |

(continued)

| Example Number | Structure | Name | Intermediates | $^1$H NMR (400 MHz) δ ppm | MI |
|---|---|---|---|---|---|
| **332** | 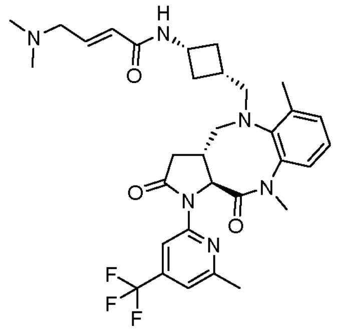 | (*E*)-*N*-((1*S*,3*s*)-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)cyclobutyl)-4-(dimethylamino)but-2-enamide | **Step a: Intermediate 5b** and *tert*-butyl ((*trans*)-3-formylcyclobutyl)car bamate (CAS: 171549-92-1)<br><br>**Step d:** *(E)-4-*(dimethylamino)but-2-enoic acid hydrochloride (CAS: 848133-35-7 | ($CDCl_3$) 8.33 (s, 1H), 7.25 - 7.17 (m, 3H), 7.04 (s, 1H), 6.84 - 6.72 (m, 1H), 5.95 (d, *J* = 15.6 Hz, 1H), 5.71 (d, *J* = 7.2 Hz, 1H), 4.69 (d, *J* = 9.6 Hz, 1H), 4.36 - 4.20 (m, 1H), 3.58 - 3.50 (m, 1H), 3.37 (s, 3H), 3.12 (d, *J* = 5.6 Hz, 2H), 3.05 - 2.88 (m, 3H), 2.84 - 2.66 (m, 2H), 2.49 (s, 3H), 2.48 - 2.39 (m, 2H), 2.35 (s, 3H), 2.31 (s, 6H), 2.22 - 2.13 (m, 1H), 2.11 - 1.96 (m, 1H), 1.59 - 1.43 (m, 2H) | 613.4 |

## BIOLOGICAL DATA

### Polθ Polymerase Domain $K_{inact}/K_I$ Assay

A PicoGreen assay was used to measure the $K_{inact}$ and $K_I$ values of covalent compounds that inhibit the activity of Polθ *in vitro.*

Human Polθ polymerase domain (aa1820-2590) was expressed in *E. Coli,* purified, aliquoted and stored at -80 °C until required. Polθ substrate was generated from a 1.2:1 mix of DNA II Short to DNA II Long to give a final concentration of 20 mM substrate in annealing buffer (20 mM Tris pH 7.5, 50 mM NaCl). The substrate was heated in 50 mL aliquots to 95 °C in a heating block for 5 min before the heating block switched off, the reaction left to cool to rt and stored at -20 °C until required.

| Name | Sequence |
|---|---|
| DNA II short | 5'-GCGGCTGTCATAAG-3' (SEQ ID NO: 1) |
| DNA II long | 5'-GCTACATTGACAATGGCA-TCAAATCTCAGATTGCGTCTTATGACAGCCGCG-3' (SEQ ID NO: 2) |

Assay measurements were performed with 1X buffer comprising 25 mM Tris pH 7.5, 12.5 mM NaCl, 0.5 mM NaCl, 5% (v/v) glycerol, 0.01 % v/v Triton x-100, 0.1 mg/ml BSA, 1 mM DTT. Test compounds were prepared by dilution in 100% DMSO to give a 12 pM intermediate stock of each (100x final top concentration). 100 nL of 23x 1:1.5-fold serial dilutions and a DMSO only control were dispensed using the Tecan dispenser into Greiner 384 well black low volume plates (product code 784076). DMSO concentration was maintained at 1% of the final assay volume by back filling with DMSO.

2x Working stock of substrate (200 nM of DNA substrate and 100 mM dNTPs) and enzyme (1.4 nM PolΘ) were made up in assay buffer. 5 μL / well of both enzyme and substrate 2x solutions were dispensed using a Tempest dispenser (Formulatrix) into assay plates that had been pre-dispensed with compound to give a final assay concentration of 100 nM DNA substrate, 50 mM dNTPs and 0.8 nM Polθ. To stop the reaction, 5 mL of a solution containing 25 mM Tris-HCl pH 7.5 and 20 mM EDTA was added at 6 timepoints (t=0, 2.5 , 5, 7.5, 10, 12.5, 15, 20, 25, 30, 35, 40, 45, 50, 55 and 60 min) using the Tempest's time delay function. The plates were covered during the time course to prevent evaporation. After completing the assay, 5 μL of detection reagent (25 mM Tris-HCl pH 7.5 and 2.5% (v/v) PicoGreen) was dispensed into the wells using a Tempest liquid handler (Formulatrix) and plates subsequently read on the CLARIOstar Plus (BMG Labtech) using the default optical settings for fluorescein and the auto gain / focus settings.

All data analysis was carried out using GraphPad Prism V.8 (GraphPad Software Inc, San Diego, CA) Time course data for each inhibitor concentration was fitted to equation 1 to determine $k_{obs}$ values.[1] Any time points where the control (DMSO alone) reaction was no longer linear were excluded from the analysis.

$$[P] = \frac{v_i}{k_{obs}}[1 - \exp(-k_{obs}\,t)]$$

(equation 1)

A secondary plot of $k_{obs}$ vs [I] is used to determine the $K_{inact}$ and $K_I$ parameters (for a 2-step covalent inhibitor) by fitting to equation 2.[1]

$$k_{obs} = \frac{k_{inact}\,[I]}{K_I + [I]}$$

(equation 2)

1) Evaluation of Enzyme Inhibitors in Drug Discovery (Ch9), R.A Copeland, Wiley, 2nd Edition 2013.

The compounds of **Examples 1 to 332** were tested in the above mentioned Polθ Polymerase Domain $K_{inact}/K_I$ Assay and the results are shown in the following table:

| Example Number | $K_{inact}/K_I$ ($\mu M^{-1}s^{-1}$) |
|---|---|
| **1** | 0.41 |
| **2** | 0.67 |
| **3** | 0.54 |
| **4** | 0.75 |
| **5** | 0.14 |
| **6** | 0.54 |
| **7** | 0.41 |
| **8** | 2.00 |
| **9** | 0.60 |
| **10** | 0.21 |
| **11** | 0.40 |
| **12** | 1.71 |
| **13** | 1.26 |
| **14** | 2.05 |
| **15** | 0.073 |
| **16** | 0.13 |
| **17** | 1.19 |
| **18** | 1.18 |
| **19** | 0.35 |
| **20** | 1.12 |
| **21a** | 0.054 |
| **21b** | 0.14 |
| **22a** | 0.17 |
| **22b** | 0.46 |
| **23a** | 0.14 |
| **23b** | 0.54 |
| **24a** | 1.63 |
| **24b** | 0.29 |
| **25a** | 0.20 |
| **25b** | 0.25 |
| **26a** | 0.69 |
| **26b** | 1.29 |
| **27** | 1.05 |
| **28** | 0.35 |
| **29** | 0.32 |
| **30** | 0.69 |
| **31** | 1.13 |
| **32** | 0.42 |
| **33** | 1.23 |
| **34a** | 1.20 |

(continued)

| Example Number | $K_{inact}/K_I$ ($\mu M^{-1}s^{-1}$) |
|---|---|
| **34b** | 1.69 |
| **35** | 0.87 |
| **36** | 1.47 |
| **37a** | 1.48 |
| **37b** | 0.58 |
| **38** | 2.39 |
| **39** | 3.42 |
| **40** | 0.85 |
| **41** | 1.38 |
| **42** | 0.79 |
| **43** | 1.90 |
| **44** | 2.22 |
| **45** | 0.31 |
| **46** | 0.48 |
| **47** | 0.14 |
| **48** | 0.11 |
| **49** | 0.34 |
| **50** | 0.19 |
| **51** | 0.21 |
| **52** | 0.19 |
| **53** | 0.024 |
| **54** | 0.11 |
| **55** | 0.015 |
| **56** | 0.036 |
| **57** | 0.034 |
| **58** | 0.50 |
| **59** | 1.08 |
| **60** | 0.30 |
| **61** | 0.50 |
| **62a** | 3.31 |
| **62b** | 1.26 |
| **63a** | 3.10 |
| **63b** | 0.19 |
| **64a** | 2.06 |
| **64b** | 0.27 |
| **65** | 4.96 |
| **66** | 2.11 |
| **67** | 2.32 |
| **68** | 0.66 |

(continued)

| Example Number | $K_{inact}/K_I$ ($\mu M^{-1}s^{-1}$) |
|---|---|
| **69** | 1.09 |
| **70** | 0.97 |
| **71** | 0.42 |
| **72** | 2.36 |
| **73a** | 0.039 |
| **73b** | 0.79 |
| **74** | 5.83 |
| **75** | 1.32 |
| **76a** | 0.22 |
| **76b** | 0.94 |
| **77** | 2.55 |
| **78** | 2.06 |
| **79** | 1.46 |
| **80** | 0.81 |
| **81** | 2.03 |
| **82** | 0.92 |
| **83** | 0.62 |
| **84** | 3.61 |
| **85** | 1.82 |
| **86** | 3.49 |
| **87** | 0.81 |
| **88** | 4.75 |
| **89** | 2.40 |
| **90** | 2.09 |
| **91** | 4.38 |
| **92** | 2.56 |
| **93** | 2.73 |
| **94** | 1.54 |
| **95a** | 2.63 |
| **95b** | 2.50 |
| **96** | 0.81 |
| **97** | 1.18 |
| **98** | 0.43 |
| **99** | 0.17 |
| **100** | 0.10 |
| **101** | 1.47 |
| **102** | 1.16 |
| **103** | 1.60 |
| **104** | 1.86 |

(continued)

| Example Number | $K_{inact}/K_I$ ($\mu M^{-1}s^{-1}$) |
|---|---|
| **105** | 1.04 |
| **106** | 1.14 |
| **107** | 0.42 |
| **108** | 2.35 |
| **109** | 0.79 |
| **110** | 1.00 |
| **111** | 2.49 |
| **112** | 0.92 |
| **113** | 1.72 |
| **114** | 0.58 |
| **115** | 2.35 |
| **116a** | 1.17 |
| **116b** | 0.58 |
| **117a** | 1.89 |
| **117b** | 3.50 |
| **118a** | 3.00 |
| **118b** | 3.36 |
| **119a** | 4.57 |
| **119b** | 3.87 |
| **120** | 0.89 |
| **121** | 0.54 |
| **122** | 0.61 |
| **123** | 0.27 |
| **124** | 0.011 |
| **125** | 2.53 |
| **126** | 0.46 |
| **127** | 0.65 |
| **128** | 0.66 |
| **129** | 0.40 |
| **130** | 0.71 |
| **131** | 0.53 |
| **132** | 1.86 |
| **133** | 1.70 |
| **134** | 0.062 |
| **135** | 2.00 |
| **136** | 6.47 |
| **137** | 0.98 |
| **138** | 1.08 |
| **139** | 2.37 |

(continued)

| Example Number | $K_{inact}/K_I$ ($\mu M^{-1}s^{-1}$) |
|---|---|
| **140a** | 0.022 |
| **140b** | 0.23 |
| **141a** | 0.20 |
| **141b** | 0.10 |
| **142a** | 1.75 |
| **142b** | 0.63 |
| **143a** | 1.00 |
| **143b** | 2.32 |
| **144a** | 1.06 |
| **144b** | 2.00 |
| **145** | 1.34 |
| **146** | 1.68 |
| **147** | 0.61 |
| **148** | 1.65 |
| **149** | 1.08 |
| **150** | 3.35 |
| **151** | 2.04 |
| **152** | 5.05 |
| **153** | 1.51 |
| **154** | 2.30 |
| **155** | 2.51 |
| **156** | 3.95 |
| **157** | 1.57 |
| **158** | 3.72 |
| **159** | 3.07 |
| **160** | 2.30 |
| **161** | 5.08 |
| **162** | 2.95 |
| **163** | 1.82 |
| **164** | 2.38 |
| **165** | 1.18 |
| **166** | 3.41 |
| **167** | 4.66 |
| **168** | 1.97 |
| **169** | 2.69 |
| **170a** | 0.88 |
| **170b** | 0.41 |
| **171a** | 2.29 |
| **171b** | 1.62 |

(continued)

| Example Number | $K_{inact}/K_I$ ($\mu M^{-1}s^{-1}$) |
|---|---|
| **172a** | 0.90 |
| **172b** | 0.28 |
| **173** | 2.92 |
| **174** | 1.08 |
| **175** | 0.37 |
| **176** | 1.37 |
| **177** | 0.26 |
| **178** | 1.60 |
| **179** | 0.67 |
| **180** | 2.72 |
| **181** | 2.26 |
| **182** | 5.23 |
| **183** | 3.80 |
| **184** | 1.33 |
| **185** | 1.27 |
| **186** | 3.23 |
| **187** | 1.41 |
| **188** | 2.31 |
| **189** | 3.35 |
| **190** | 2.93 |
| **191** | 1.40 |
| **192** | 2.18 |
| **193** | 2.58 |
| **194** | 4.38 |
| **195** | 5.22 |
| **196** | 2.06 |
| **197** | 1.67 |
| **198** | 0.70 |
| **199** | 1.38 |
| **200** | 0.69 |
| **201** | 2.80 |
| **202** | 5.56 |
| **203** | 4.32 |
| **204** | 0.98 |
| **205** | 1.67 |
| **206a** | 4.36 |
| **206b** | 4.19 |
| **207** | 1.82 |
| **208** | 4.36 |

(continued)

| Example Number | $K_{inact}/K_I$ ($\mu M^{-1}s^{-1}$) |
|---|---|
| **209** | 0.53 |
| **210** | 0.63 |
| **211** | 1.57 |
| **212** | 1.81 |
| **213** | 1.08 |
| **214** | 0.32 |
| **215** | 1.21 |
| **216** | 0.43 |
| **217** | 1.59 |
| **218** | 1.62 |
| **219** | 0.56 |
| **220** | 3.66 |
| **221** | 3.03 |
| **222** | 2.04 |
| **223** | 1.62 |
| **224** | 4.21 |
| **225a** | 0.32 |
| **225b** | 1.31 |
| **226a** | 0.23 |
| **226b** | 0.87 |
| **227a** | 0.23 |
| **227b** | 1.07 |
| **228** | 2.26 |
| **229** | 0.73 |
| **230** | 1.01 |
| **231** | 0.86 |
| **232** | 1.89 |
| **233** | 2.99 |
| **234** | 5.85 |
| **235** | 0.27 |
| **236** | 1.24 |
| **237** | 0.27 |
| **238** | 0.74 |
| **239** | 0.73 |
| **240** | 1.55 |
| **241** | 1.28 |
| **242** | 0.66 |
| **243a** | 1.03 |
| **243b** | 0.58 |

(continued)

| Example Number | $K_{inact}/K_I$ ($\mu M^{-1}s^{-1}$) |
|---|---|
| **244** | 3.83 |
| **245** | 1.90 |
| **246** | 0.42 |
| **247** | 0.98 |
| **248** | 0.81 |
| **249** | 0.11 |
| **250** | 0.53 |
| **251** | 0.37 |
| **252** | 1.19 |
| **253** | 1.42 |
| **254** | 2.13 |
| **255** | 0.98 |
| **256** | 1.11 |
| **257a** | 2.17 |
| **257b** | 2.56 |
| **258a** | 1.65 |
| **258b** | 1.53 |
| **259** | 0.15 |
| **260** | 1.54 |
| **261** | 0.34 |
| **262** | 0.71 |
| **263** | 1.06 |
| **264** | 1.25 |
| **265a** | 3.32 |
| **265b** | 1.58 |
| **266** | 0.59 |
| **267a** | 1.24 |
| **267b** | 3.03 |
| **268** | 0.41 |
| **269** | 0.97 |
| **270** | 0.11 |
| **271** | 0.20 |
| **272** | 0.033 |
| **273** | 0.06 |
| **274** | 0.47 |
| **275** | 0.80 |
| **276** | 1.76 |
| **277** | 0.08 |
| **278** | 0.77 |

(continued)

| Example Number | $K_{inact}/K_I$ ($\mu M^{-1}s^{-1}$) |
|---|---|
| **279** | 0.90 |
| **280** | 2.54 |
| **281** | 1.05 |
| **282** | 0.87 |
| **283** | 0.95 |
| **284** | 0.77 |
| **285** | 1.98 |
| **286** | 0.61 |
| **287a** | 0.93 |
| **287b** | 1.19 |
| **288a** | 0.63 |
| **288b** | 0.50 |
| **289a** | 1.26 |
| **289b** | 1.24 |
| **290a** | 2.02 |
| **290b** | 1.30 |
| **291a** | 1.17 |
| **291b** | 1.48 |
| **292a** | 2.97 |
| **292b** | 2.52 |
| **293a** | 0.88 |
| **293b** | 1.28 |
| **294a** | 1.61 |
| **294b** | 2.48 |
| **295** | 1.62 |
| **296** | 3.00 |
| **297** | 1.47 |
| **298** | 3.64 |
| **299** | 1.99 |
| **300** | 3.93 |
| **301** | 1.78 |
| **302** | 3.04 |
| **303** | 1.80 |
| **304** | 1.87 |
| **305** | 1.82 |
| **306** | 1.54 |
| **307** | 1.35 |
| **308** | 0.28 |
| **309** | 1.02 |

(continued)

| Example Number | $K_{inact}/K_I$ ($\mu M^{-1}s^{-1}$) |
|---|---|
| **310a** | 2.31 |
| **310b** | 1.46 |
| **311a** | 1.12 |
| **311b** | 1.22 |
| **312** | 1.85 |
| **313** | 0.66 |
| **314** | 1.74 |
| **315** | 1.79 |
| **316** | 0.61 |
| **317** | 0.23 |
| **318a** | 1.90 |
| **318b** | 1.56 |
| **319a** | 2.17 |
| **319b** | 1.66 |
| **320a** | 1.37 |
| **320b** | 2.48 |
| **321a** | 1.57 |
| **321b** | 0.69 |
| **322a** | 1.56 |
| **322b** | 1.13 |
| **323a** | 0.64 |
| **323b** | 0.33 |
| **324a** | 0.15 |
| **324b** | 0.47 |
| **325a** | 1.25 |
| **325b** | 0.87 |
| **325c** | 0.98 |
| **325d** | 1.77 |
| **326a** | 0.45 |
| **326b** | 1.52 |
| **327** | 3.64 |
| **328** | 4.11 |
| **329a** | 1.73 |
| **329b** | 5.73 |
| **330a** | 2.74 |
| **330b** | 1.15 |
| **331** | 1.85 |
| **332** | 1.30 |

## Claims

**1.** A compound of formula (I):

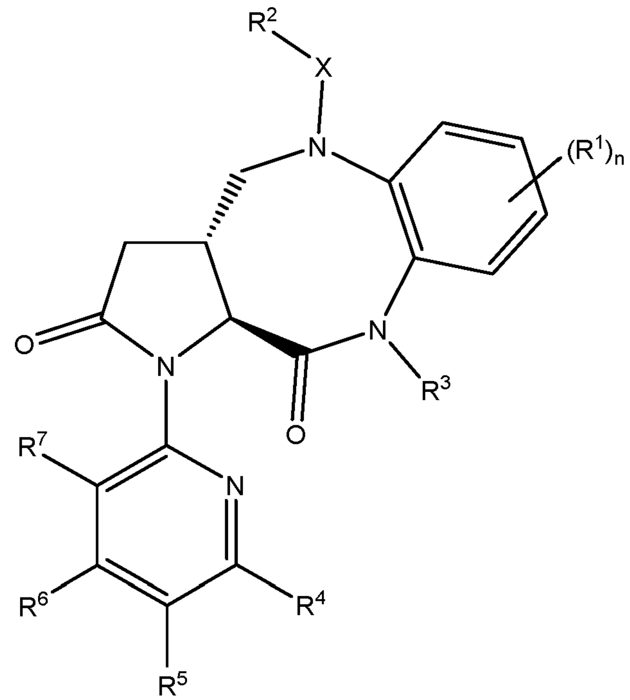

(I)

or a tautomeric or a stereochemically isomeric form, a pharmaceutically acceptable salt or a solvate thereof, wherein:

n represents an integer selected from 0, 1, 2, 3 or 4;
$R^1$ represents $C_{1-6}$ alkyl, $C_{2-6}$ alkenyl, $C_{1-6}$ alkoxy, hydroxy, halogen, halo$C_{1-6}$ alkyl, halo$C_{1-6}$ alkoxy, $C_{3-8}$ cycloalkyl, cyano or $-NR^xR^y$;
X represents a bond or a $C_1$-$C_6$ alkylene group optionally substituted by one or more $C_{1-6}$ alkyl, $NR^x$, O, hydroxy, halogen or CO groups;
$R^2$ represents a -Ring$^A$ or -Ring$^B$-Y-Ring$^C$ group;
or $-X-R^2$ represents a $-C_1$-$C_{12}$ alkylene-$NR^XR^Y$ group, wherein said alkylene group may be optionally substituted by one or more $C_{1-6}$ alkyl, $C_{2-6}$ alkenyl, $-NR^x$, O, hydroxy or CO groups;
Ring$^A$ represents carbocyclyl, heterocyclyl or heteroaryl which requires a substituent selected from a $-(CH_2)_m$-CO-$C_{1-6}$ alkyl, $-(CH_2)_m$-NHCO-$C_{1-6}$ alkyl, $-(CH_2)_m$-CO-$C_{2-6}$ alkenyl, - $(CH_2)_m$-NHCO-$C_{2-6}$ alkenyl, $-(CH_2)_m$-CO-$C_{2-6}$ alkynyl or $-(CH_2)_m$-NHCO-$C_{2-6}$ alkynyl group,
wherein said alkyl, alkenyl or alkynyl group may be optionally substituted by one or more (e.g. 1, 2 or 3) halogen, hydroxy, CO or $-NR^xR^y$ groups and wherein said carbocyclyl, heterocyclyl or heteroaryl groups may be optionally further substituted by one or more (e.g. 1, 2 or 3) substituents selected from halogen, $C_{1-6}$ alkyl, hydroxy, $C_{1-6}$ alkoxy, -CO-$C_{1-6}$ alkyl, oxo, $C_{1-6}$ alkylamino or cyano, wherein said $C_{1-6}$ alkyl group may be optionally substituted by one or more halogen, hydroxy or cyano groups;
m represents an integer selected from 0, 1, 2, 3 or 4;
Ring$^B$ represents carbocyclyl, heterocyclyl or heteroaryl, each of which may be optionally substituted by one or more (e.g. 1, 2 or 3) substituents selected from hydroxy, oxo, $C_{1-6}$ alkyl, $C_{2-6}$ alkenyl, $C_{2-6}$ alkynyl, $C_{1-6}$ alkoxy, halo$C_{1-6}$ alkyl, -CO-$C_{1-6}$ alkyl, cyano or halogen;
Ring$^C$ represents heterocyclyl substituted by one or more (e.g. 1, 2 or 3) substituents selected from halogen, $C_{1-6}$ alkyl, -CO-$C_{1-6}$ alkyl, and/or $-(CH_2)_m$-CO-$C_{2-6}$ alkenyl optionally substituted by an $-NR^xR^y$ group;
Y represents a bond, -O-, -NHCO-, CO, or a $C_1$-$C_6$ alkylene group optionally substituted by one or more $C_{1-6}$ alkyl, $C_{2-6}$ alkenyl, $-NR^x$, O, hydroxy or CO groups;
$R^3$ represents hydrogen or $C_{1-6}$ alkyl;
$R^4$, $R^5$, $R^6$ and $R^7$ independently represent hydrogen, $C_{1-6}$ alkyl, $C_{2-6}$ alkenyl, $C_{1-6}$ alkoxy, halogen, halo$C_{1-6}$ alkyl, halo$C_{1-6}$ alkoxy, $C_{3-8}$ cycloalkyl, cyano or $-NR^xR^y$; and

$R^x$ and $R^y$ independently represent hydrogen, $C_{1-6}$ alkyl, $C_{2-6}$ alkenyl, $C_{2-6}$ alkynyl, $C_{1-6}$ alkoxy, or $R^x$ and $R^y$ together with the nitrogen atom to which they are attached join to form a nitrogen containing heterocyclic ring which may be optionally substituted by one or more $C_{1-6}$ alkyl groups.

**2.** The compound as defined in claim 1, wherein n represents 0, 1, 2 or 3.

**3.** The compound as defined in claim 1 or claim 2, wherein $R^1$ represents $C_{1-6}$ alkyl (such as methyl), halogen (such as fluorine or chlorine) or $C_{3-8}$ cycloalkyl (such as cyclopropyl), such as wherein $R^1$ represents $C_{1-6}$ alkyl (such as methyl) or halogen (such as fluorine or chlorine), in particular wherein $R^1$ represents $C_{1-6}$ alkyl (such as methyl).

**4.** The compound as defined in any one of claims 1 to 3, wherein $R^2$ represents -Ring$^A$, such as wherein Ring$^A$ represents carbocyclyl (such as phenyl, cyclobutyl, 2,3-dihydro-1H-inden-2-yl or bicyclo[1.1.1]pentan-1-yl), a heterocyclyl ring (such as azetidinyl, pyrrolidinyl, piperidinyl, piperazinyl, isoindolin-1-yl, isoindolin-5-yl, tetrahydroisoquinolin-6-yl, diazaspiro[3.4]octan-6-yl, octahydro-2H-pyrazino[1,2-a]pyrazin-2-yl, octahydro-5H-pyrrolo[3,4-c]pyridin-5-yl, 5-oxa-2,8-diazaspiro[3.5]nonan-8-yl, 2,6-diazaspiro[3.4]octane, 9-oxa-3,7-diazabicyclo[3.3.1]nonan-3-yl, tetrahydro-1H-imidazo[4,5-c]pyridin-1-yl, tetrahydro-1'H-spiro[azetidine-3,4'-pyrrolo[1,2-a]pyrazin]-2'(3'*H*)-yl, tetrahydro-1'*H*-spiro[azetidine-3,6'-pyrazino[2,1-*c*][1,4]oxazin]-8'(7'*H*)-yl, tetrahydro-3*H*-imidazo[4,5-c]pyridin-3-yl, tetrahydro-[1,2,4]triazolo[1,5-a]pyrazin-2-yl, tetrahydroimidazo[1,2-a]pyrazin-2-yl, tetrahydropyrazolo[1,5-a]pyrazin-2-yl, octahydro-2H-pyrazino[1,2-a]pyrazin-3-yl, tetrahydro-[1,2,4]triazolo[4,3-a]pyrazin-3-yl, 2,5,8-triazaspiro[3.5]nonan-8-yl, spiro[azetidine-3,3'-imidazo[1,2-a]imidazol]-1'(2'H)-yl, dihydro-5H-pyrrolo[3,4-b]pyrazin-2-yl, dihydro-5H-pyrrolo[3,4-b]pyridin-3-yl, dihydro-5H-pyrrolo[3,4-b]pyridin-2-yl, dihydro-5H-pyrrolo[3,4-b]pyridin-7-yl, dihydro-5H-pyrrolo[3,4-b]pyridin-5-yl, tetrahydropyrrolo[3,4-d]imidazol-2-yl, dihydro-2H-benzo[b][1,4]oxazin-6-yl, dihydro-2H-benzo[b][1,4]oxazin-7-yl, dihydro-4H-pyrrolo[3,4-d]imidazol-1-yl, diazabicyclo[3.1.1]heptan-6-yl, diazabicyclo[3.2.1]octan-8-yl, diazabicyclo[2.2.1]heptan-2-yl, dihydro-1H-pyrrolo[3,4-c]pyridin-6-yl, dihydro-5H-pyrrolo[3,4-d]pyrimidin-2-yl, dihydro-4H-pyrrolo[3,4-d]thiazol-2-yl, dihydro-4H-pyrrolo[3,4-d]oxazo-2-yl, tetrahydropyrrolo[3,4-c]pyrazol-3-yl, diazaspiro[3.3]heptan-6-yl, tetrahydroimidazo[1,2-a]pyridin-6-yl, tetrahydroimidazo[1,2-a]pyridin-7-yl, or spiro[azetidine-3,5'-imidazo[1,2-a]pyrazin]-7'(8'H)-yl) or a heteroaryl ring (such as pyridyl or pyrimidinyl), each being substituted by a

$-(CH_2)_m-CO-C_{2-6}$ alkyl optionally substituted by a halogen group (such as $-CO-CH_2-Cl$);
$-(CH_2)_m-CO-C_{2-6}$ alkenyl optionally substituted by one or more halogen, hydroxy, CO or $-NR^xR^y$ group (such as $-COCH=CH_2$, $-CO-C(=CH_2)-Me$, $-CO-CH=CH-CH_2-N(Me)_2$, $-CO-C(=CH_2)-CH_2-N(Me)_2$, $-CO-C(=CH-Me)-CH_2-N(Me)_2$, $-CO-C(=CH_2)-CH_2-N(Me)_2$, $-CO-C(Cl)=CH-CH_2-N(Me)_2$, $-CO-C(=CH_2)-CH_2-OH$, $-CO-C(=CH_2)-CH_2-O-C(=O)-Me$, $-CO-CH=CH-CHF_2$, $-CO-C(=CH_2)-CH_2-NEt_2$);
$-(CH_2)_m-NHCO-C_{2-6}$ alkenyl optionally substituted by a $-NR^xR^y$ group (such as $-NH-CO-CH=CH_2$, $-NH-CO-C(=CH_2)-CH_2-N(Me)_2$, $-NH-CO-C(=CH-Me)-CH_2-N(Me)_2$, $-NH-CO-CH=CH-CH_2-N(Me)_2$, $-CH_2-NH-CO-CH=CH_2-$, $-CH_2-NH-CO-CH=CH-CH_2-N(Me)_2$, $-CH_2-NH-CO-C(=CH_2)-CH_2-N(Me)_2$);
$-(CH_2)_m-CO-C_{2-6}$ alkynyl (such as -CO-ethynyl or -CO-ethynyl-Me); or
$-(CH_2)_m-NHCO-C_{2-6}$ alkynyl group (such as -NH-CO-ethynyl),

wherein said carbocyclyl, heterocyclyl or heteroaryl group may be optionally substituted by one or more (e.g. 1, 2 or 3) further substituents selected from: halogen (such as fluorine); $C_{1-6}$ alkyl (such as methyl or ethyl) optionally substituted by one or more halogen (such as $-CF_3$ or $-CHF_2$), hydroxy (such as $-CH_2-OH$) or cyano (such as $-CH_2-CN$) groups; hydroxy; $C_{1-6}$ alkoxy (such as methoxy and $-CH_2-OMe$); $-CO-C_{1-6}$ alkyl (such as -CO-Me); oxo; $C_{1-6}$ alkylamino (such as $-N(Me)-(CH_2)_2-N(Me)_2$); or cyano),

in particular Ring$^A$ represents a heterocyclyl ring (such as azetidinyl, pyrrolidinyl, piperidinyl, piperazinyl, isoindolin-1-yl, isoindolin-5-yl, tetrahydroisoquinolin-6-yl, diazaspiro[3.4]octan-6-yl, octahydro-2*H*-pyrazino[1,2-*a*]pyrazin-2-yl, octahydro-5H-pyrrolo[3,4-c]pyridin-5-yl, 5-oxa-2,8-diazaspiro[3.5]nonan-8-yl, 2,6-diazaspiro[3.4]octane, 9-oxa-3,7-diazabicyclo[3.3.1]nonan-3-yl, tetrahydro-1*H*-imidazo[4,5-*c*]pyridin-1-yl, tetrahydro-1'H-spiro[azetidine-3,4'-pyrrolo[1,2-a]pyrazin]-2'(3'*H*)-yl, tetrahydro-1'*H*-spiro[azetidine-3,6'-pyrazino[2,1-*c*][1,4]oxazin]-8'(7'*H*)-yl, tetrahydro-3*H*-imidazo[4,5-*c*]pyridin-3-yl, tetrahydro-[1,2,4]triazolo[1,5-a]pyrazin-2-yl, tetrahydroimidazo[1,2-a]pyrazin-2-yl, tetrahydropyrazolo[1,5-a]pyrazin-2-yl, octahydro-2H-pyrazino[1,2-a]pyrazin-3-yl, tetrahydro-[1,2,4]triazolo[4,3-a]pyrazin-3-yl, 2,5,8-triazaspiro[3.5]nonan-8-yl, spiro[azetidine-3,3'-imidazo[1,2-a]imidazol]-1'(2'H)-yl, dihydro-5H-pyrrolo[3,4-b]pyrazin-2-yl, dihydro-5H-pyrrolo[3,4-b]pyridin-3-yl, dihydro-5H-pyrrolo[3,4-b]pyridin-2-yl, dihydro-5H-pyrrolo[3,4-b]pyridin-7-yl, dihydro-5H-pyrrolo[3,4-b]pyridin-5-yl, tetrahydropyrrolo[3,4-d]imidazol-2-yl, dihydro-2H-benzo[b][1,4]oxazin-6-yl, dihydro-2H-benzo[b][1,4]oxazin-7-yl, dihydro-4H-pyrrolo[3,4-d]imidazol-1-yl, diazabicyclo[3.1.1]heptan-6-yl, diazabicyclo[3.2.1]octan-8-yl, diazabicyclo[2.2.1]heptan-2-yl, dihydro-1H-pyrrolo[3,4-c]pyridin-6-yl, dihydro-5H-pyrrolo[3,4-d]pyrimidin-2-yl, dihydro-4H-pyrrolo[3,4-d]thiazol-2-yl, dihydro-4H-pyrrolo[3,4-d]oxazo-2-yl, tetrahydropyrrolo[3,4-c]pyrazol-3-yl, diazaspiro[3.3]heptan-6-yl, tetrahydroimidazo[1,2-a]pyridin-6-yl, tetrahydroimidazo[1,2-a]pyridin-7-yl, or spiro[azetidine-3,5'-imidazo[1,2-a]pyrazin]-7'(8'H)-yl) each being substituted by a

$-(CH_2)_m-CO-C_{2-6}$ alkyl optionally substituted by a halogen group (such as $-CO-CH_2-Cl$);
$-(CH_2)_m-CO-C_{2-6}$ alkenyl optionally substituted by one or more halogen, hydroxy, CO or $-NR^xR^y$ group (such as $-COCH=CH_2$, $-CO-C(=CH_2)-Me$, $-CO-CH=CH-CH_2-N(Me)_2$, $-CO-C(=CH_2)-CH_2-N(Me)_2$, $-CO-C(=CH-Me)-CH_2-N(Me)_2$, $-CO-C(=CH_2)-CH_2-N(Me)_2$, $-CO-C(Cl)=CH-CH_2-N(Me)_2$, $-CO-C(=CH_2)-CH_2-OH$, $-CO-C(=CH_2)-CH_2-O-C(=O)-Me)$, $-CO-CH=CH-CHF_2$, $-CO-C(=CH_2)-CH_2-NEt_2$;
$-(CH_2)_m-NHCO-C_{2-6}$ alkenyl optionally substituted by a $-NR^xR^y$ group (such as $-NH-CO-CH=CH_2$, $-NH-CO-C(=CH_2)-CH_2-N(Me)_2$, $-NH-CO-C(=CH-Me)-CH_2-N(Me)_2$, $-NH-CO-CH=CH-CH_2-N(Me)_2$, $-CH_2-NH-CO-CH=CH_2-$, $-CH_2-NH-CO-CH=CH-CH_2-N(Me)_2$, $-CH_2-NH-CO-C(=CH_2)-CH_2-N(Me)_2$);
$-(CH_2)_m-CO-C_{2-6}$ alkynyl (such as -CO-ethynyl or -CO-ethynyl-Me); or
$-(CH_2)_m-NHCO-C_{2-6}$ alkynyl group (such as -NH-CO-ethynyl),
wherein said heterocyclyl or heteroaryl group may be optionally substituted by one or more (e.g. 1, 2 or 3) further substituents selected from: halogen (such as fluorine); $C_{1-6}$ alkyl (such as methyl or ethyl) optionally substituted by one or more halogen (such as $-CF_3$ or $-CHF_2$), hydroxy (such as $-CH_2-OH$) or cyano (such as $-CH_2-CN$) groups, hydroxy, $C_{1-6}$ alkoxy (such as methoxy and $-CH_2-OMe$), $-CO-C_{1-6}$ alkyl (such as -CO-Me), oxo, $C_{1-6}$ alkylamino (such as $-N(Me)-(CH_2)_2-N(Me)_2$) or cyano), more particularly $Ring^A$ represents dihydro-5H-pyrrolo[3,4-b]pyridinyl, such as dihydro-5H-pyrrolo[3,4-b]pyridin-3-yl, dihydro-5H-pyrrolo[3,4-b]pyridin-2-yl, dihydro-5H-pyrrolo[3,4-b]pyridin-7-yl, or dihydro-5H-pyrrolo[3,4-b]pyridin-5-yl each being substituted by a
$-(CH_2)_m-CO-C_{2-6}$ alkenyl optionally substituted by one or more halogen, hydroxy, CO or $-NR^xR^y$ group (such as $-COCH=CH_2$, $-CO-C(=CH_2)-Me$, $-CO-CH=CH-CH_2-N(Me)_2$, $-CO-C(=CH_2)-CH_2-N(Me)_2$, $-CO-C(=CH-Me)-CH_2-N(Me)_2$, $-CO-C(=CH_2)-CH_2-N(Me)_2$, $-CO-C(Cl)=CH-CH_2-N(Me)_2$, $-CO-C(=CH_2)-CH_2-OH$, $-CO-C(=CH_2)-CH_2-O-C(=O)-Me)$, $-CO-CH=CH-CHF_2$, $-CO-C(=CH_2)-CH_2-NEt_2$;
wherein said heterocyclyl group may be optionally substituted by one or more (e.g. 1, 2 or 3) further substituents selected from: halogen (such as fluorine); $C_{1-6}$ alkyl (such as methyl or ethyl) optionally substituted by one or more halogen (such as $-CF_3$ or $-CHF_2$), hydroxy (such as $-CH_2-OH$) or cyano (such as $-CH_2-CN$) groups, hydroxy, $C_{1-6}$ alkoxy (such as methoxy and $-CH_2-OMe$), $-CO-C_{1-6}$ alkyl (such as -CO-Me), oxo, $C_{1-6}$ alkylamino (such as $-N(Me)-(CH_2)_2-N(Me)_2$) or cyano),

especially $Ring^A$ represents dihydro-5H-pyrrolo[3,4-b]pyridinyl, such as dihydro-5H-pyrrolo[3,4-b]pyridin-3-yl, dihydro-5H-pyrrolo[3,4-b]pyridin-2-yl, dihydro-5H-pyrrolo[3,4-b]pyridin-7-yl, or dihydro-5H-pyrrolo[3,4-b]pyridin-5-yl each being substituted by a $-COCH=CH_2$,
more especially $Ring^A$ represents dihydro-5H-pyrrolo[3,4-b]pyridin-2-yl substituted by a $-COCH=CH_2$,
most especially $Ring^A$ represents 6,7-dihydro-5H-pyrrolo[3,4-b]pyridin-2-yl substituted by a $-COCH=CH_2$.

**5.** The compound as defined in any one of claims 1 to 3, wherein $R^2$ represents $-Ring^B-Y-Ring^C$, such as wherein $Ring^B$ represents carbocyclyl (such as cyclohexyl or phenyl), heterocyclyl (such as azetidinyl, pyrrolidinyl, piperidinyl, piperazinyl, diazepanyl or hexahydropyrrolo[3,4-*b*][1,4]oxazin-6(2*H*)-yl) or heteroaryl (such as imidazolyl, pyrazolyl, triazolyl, pyridinyl, pyrimidinyl, oxazolyl or oxadiazolyl), each of which may be optionally substituted by one or more (e.g. 1, 2 or 3) substituents selected from hydroxy, oxo, $C_{1-6}$ alkyl (such as methyl), halo$C_{1-6}$ alkyl (such as $-CH_2-F$), cyano or halogen (such as fluorine).

**6.** The compound as defined in any one of claims 1 to 5, wherein Y represents a bond, -O-, -NHCO-, CO, or a $C_1$-$C_6$ alkylene group optionally substituted by one or more $C_{1-6}$ alkyl, $C_{2-6}$ alkenyl, $-NR^x$, O, hydroxy or CO groups (such as $-CO-C(=CH_2)-CH_2-$ or $-NHCO-CH=CH-CH_2-$), such as wherein Y represents a bond, alternatively wherein Y represents -O-.

**7.** The compound as defined in any one of claims 1 to 6, wherein $Ring^C$ represents a monocyclic heterocyclyl ring having at least one nitrogen atom. In a further embodiment, $Ring^C$ represents azetidinyl, pyrrolidinyl, piperazinyl or tetrahydropyridinyl, each of which being substituted by a halogen (such as fluorine), $C_{1-6}$ alkyl (such as methyl), $-CO-C_{1-6}$ alkyl (such as -CO-methyl) and/or a $-(CH_2)_m-CO-C_{2-6}$ alkenyl optionally substituted by an $-NR^xR^y$ group (such as $-COCH=CH_2$, $-CO-C(=CH_2)-CH_2-N(Me)_2$, $-CO-C(=CH-Me)-CH_2-N(Me)_2$, or $-CO-CH=CH-CH_2-N(Me)_2$).

**8.** The compound as defined in any one of claims 1 to 7, wherein X represents:

(a) a bond or a $C_1$-$C_4$ alkylene group optionally substituted by one or more $C_{1-6}$ alkyl (such as methyl), $-NR^x$ (such

as -NH or -N-methyl), O, hydroxy, halogen (such as fluorine) or CO groups; or
(b) $-CH_2-$; or
(c) a $C_1$-$C_4$ alkylene group (such as $-CH_2-$, $-(CH_2)_2-$, $-(CH_2)_3-$ or $-(CH_2)_4-$) optionally substituted by one or more $C_{1-6}$ alkyl (such as $-(CH_2)$-C(Me)-), O (such as $-(CH_2)_2$-O-, - $(CH_2)_3$-O- or $-(CH_2)_2$-O-$CH_2$-) or CO (such as $-(CH_2)$-CO- or $-(CH_2)_2$-CO-) $-NR^x$ (such as - $(CH_2)_2$-NH-, $-(CH_2)_3$-NH-, $-(CH_2)_2$-N(Me)- or $-(CH_2)_3$-N(Me)-) groups or a CO and $-NR^x$ group (such as $-(CH_2)_2$-N(Me)-CO-) or a hydroxy and $-NR^x$ group (such as $-(CH_2)$-CH(OH)-$CH_2$-N(Me)- or halogen (such as $-(CH_2)_3$-CHF-, $-(CH_2)_2$-CHF-$CH_2$-, or -$CH_2$-CHF-$(CH_2)_2$-); or
(d) a bond, $-CH_2-$, $-(CH_2)_2-$, $-(CH_2)_3-$, $-(CH_2)_4-$, $-(CH_2)$-C(Me)-, $-(CH_2)_2$-O-, $-(CH_2)_3$-O-, - $(CH_2)_2$-O-$CH_2$-, $-(CH_2)$-CO-, $-(CH_2)_2$-CO-, $-(CH_2)_2$-NH-, $-(CH_2)_3$-NH-, $-(CH_2)_2$-N(Me)-, - $(CH_2)_3$-N(Me)-, $-(CH_2)_2$-N(Me)-CO- $-(CH_2)$-CH(OH)-$CH_2$-N(Me)-, $-(CH_2)_3$-CHF-, $-(CH_2)_2$-CHF-$CH_2$-, or -$CH_2$-CHF-$(CH_2)_2$-.

**9.** The compound as defined in any one of claims 1 to 8, wherein -X-$R^2$ represents a - $C_1$-$C_{10}$ alkylene-$NR^xR^y$ group, wherein said alkylene group may be optionally substituted by one or more $C_{2-6}$ alkenyl, $NR^x$, O or CO groups, such as $-(CH_2)_2$-O-$(CH_2)_2$-NH-CO-C(=$CH_2$)-$CH_2$-N$(Me)_2$, $-(CH_2)_5$-NH-CO-CH=CH-$CH_2$-N$(Me)_2$,$-(CH_2)_5$-NH-CO-C(=$CH_2$)-$CH_2$-N$(Me)_2$, - $(CH_2)_4$-NHCOCH=CH-$CH_2$-$NMe_2$, or -$CH_2$-CHF-$(CH_2)_3$-NHCO-CH=CH$CH_2$$NMe_2$, $-(CH_2)_3$-CHF-$CH_2$NHCO-CH=CH$CH_2$$NMe_2$, or $-(CH_2)_3$-$CF_2$-$CH_2$NHCO-CH=CH$CH_2$$NMe_2$.

**10.** The compound as defined in any one of claims 1 to 9, wherein:

(a) $R^3$ represents hydrogen or $C_{1-6}$ alkyl (such as methyl or ethyl), such as wherein $R^3$ represents hydrogen or $C_{1-6}$ alkyl (such as methyl), in particular wherein $R^3$ represents $C_{1-6}$ alkyl (such as methyl), especially wherein $R^3$ represents methyl; and/or
(b) $R^4$ represents:

hydrogen;
$C_{1-6}$ alkyl (such as methyl or ethyl);
$C_{2-6}$ alkenyl (such as ethenyl);$C_{1-6}$ alkoxy (such as methoxy);
halogen (such as chlorine); or
$-NR^xR^y$ (such as -N$(Me)_2$ or -N(Me)(Et)),

such as wherein $R^4$ represents $C_{1-6}$ alkyl (such as methyl), in particular wherein $R^4$ represents methyl; and/or
(c) $R^5$ represents:

hydrogen;
halogen (such as chlorine); or
$C_{1-6}$ alkyl (such as methyl),

such as wherein $R^5$ represents hydrogen; and/or
(d) $R^6$ represents:

$C_{1-6}$ alkyl (such as methyl, ethyl or isopropyl);
$C_{2-6}$ alkenyl (such as -C(=$CH_2$)(Me));
halogen (such as bromine);
halo$C_{1-6}$ alkyl (such as trifluoromethyl or -C(H)(Me)-$CF_3$); or
halo$C_{1-6}$ alkoxy (such as difluoromethoxy),

such as wherein $R^6$ represents halo$C_{1-6}$ alkyl (such as trifluoromethyl), in particular wherein $R^6$ represents trifluoromethyl; and/or
(e) $R^7$ represents hydrogen or cyano, such as hydrogen; and/or
(f) $R^x$ and $R^y$ independently represent hydrogen or $C_{1-6}$ alkyl (such as methyl or ethyl), such as wherein $R^x$ and $R^y$ independently represent $C_{1-6}$ alkyl (such as methyl), in particular wherein both of $R^x$ and $R^y$ represent $C_{1-6}$ alkyl (such as methyl).

**11.** The compound as defined in claim 1 which is a compound of formula $(I)^a$:

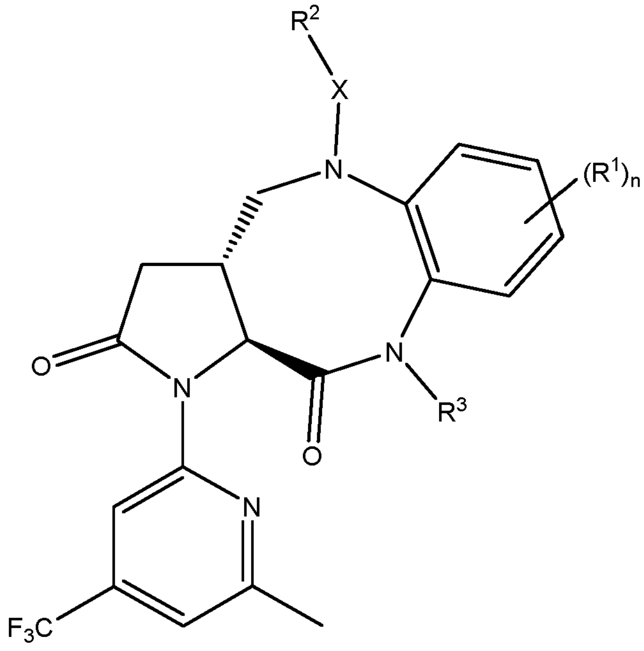

(I)[a]

or a tautomeric or a stereochemically isomeric form, a pharmaceutically acceptable salt or a solvate thereof, wherein n, $R^1$, X, $R^2$ and $R^3$ are as defined in claim 1.

**12.** The compound as defined in claim 1, wherein the compound is the free base of a compound of Examples 1-332:

| Example Number | Name |
|---|---|
| 1 | (3a*R*,11a*S*)-5-(2-(4-(1-Acryloylazetidin-3-yl)piperazin-1-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 2 | (3a*R*,11a*S*)-5-(2-((*R*)-1-Acryloyltetrahydro-1'H-spiro[azetidine-3,4'-pyrrolo[1,2-a]pyrazin]-2'(3'*H*)-yl)ethyl)-6-fluoro-10-methyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione |
| 3 | (3a*R*,11a*S*)-5-(2-(4-(1-Acryloylazetidin-3-yl)-1,4-diazepan-1-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 4 | (3a*R*,11a*S*)-5-(2-(2-acryloyl-5-oxa-2,8-diazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 5 | (3a*R*,11a*S*)-5-(2-(7-acryloyl-9-oxa-3,7-diazabicyclo[3.3.1]nonan-3-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 6 | (3a*R*,11a*S*)-5-(2-((*S*)-2-acryloyl-6-(methoxymethyl)-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 7 | (3a*R*,11a*S*)-5-(2-((7'*R*/*S*,9a'*S*)-1-acryloyl-7'-methyltetrahydro-1'*H*-spiro[azetidine-3,6'-pyrazino[2,1-*c*][1,4]oxazin]-8'(7'*H*)-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 8 | (3a*R*,11a*S*)-5-(2-((7'*S*/*R*,9a'*S*)-1-acryloyl-7'-methyltetrahydro-1'*H*-spiro[azetidine-3,6'-pyrazino[2,1-*c*][1,4]oxazin]-8'(7'*H*)-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 9 | (3a*R*,11a*S*)-5-(2-((7*S*,9a*S*/*R*)-8-acryloyl-7-methyloctahydro-2*H*-pyrazino[1,2-*a*]pyrazin-2-yl) ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 10 | (3a*R*,11a*S*)-5-(2-((7*S*,9a*R*/*S*)-8-acryloyl-7-methyloctahydro-2*H*-pyrazino[1,2-*a*]pyrazin-2-yl) ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 11 | (3a*R*,11a*S*)-5-(2-((*rel-trans*)-3-((1-acryloylazetidin-3-yl)oxy)-4-hydroxypyrrolidin-1-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 12 | (3a*R*,11a*S*)-5-(2-(4-(1-acryloylazetidin-3-yl)piperazin-1-yl)ethyl)-6-chloro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 13 | (3a*R*,11a*S*)-5-(2-((*R*)-2-acryloyl-6-methyl-2,5,8-triazaspiro[3.5]nonan-8-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 14 | (3a*R*,11a*S*)-5-(2-(8-acryloyloctahydro-2*H*-pyrazino[1,2-*a*]pyrazin-2-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 15 | (3a*R*,11a*S*)-5-(2-(4-(1-acryloylazetidin-3-yl)piperazin-1-yl)ethyl)-8-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 16 | (3a*R*,11a*S*)-5-(2-(4-(1-acryloylazetidin-3-yl)piperazin-1-yl)ethyl)-6,8-difluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 17 | (3a*R*,11a*S*)-5-(2-(4-(1-acryloylazetidin-3-yl)piperazin-1-yl)ethyl)-6-chloro-8-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 18 | (3a*R*,11a*S*)-5-(2-(4-(1-acryloylazetidin-3-yl)piperazin-1-yl)ethyl)-8-fluoro-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 19 | (3a*R*,11a*S*)-5-(2-(4-(1-acryloylazetidin-3-yl)piperazin-1-yl)ethyl)-6-cyclopropyl-8-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 20 | (3a*R*,11a*S*)-5-(2-(4-(1-acryloylazetidin-3-yl)piperazin-1-yl)ethyl)-6-chloro-8,9-difluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 21a | (3a*R*,11a*S*)-5-(2-((*S*/*R*)-1-acryloyl-1,6-diazaspiro[3.4]octan-6-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 21b | (3a*R*,11a*S*)-5-(2-((*R*/*S*)-1-acryloyl-1,6-diazaspiro[3.4]octan-6-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 22a | (3a*R*,11aS)-5-(2-((4a*S*/*R*,7a*S*/*R*)-4-(1-acryloylazetidin-3-yl)hexahydropyrrolo[3,4-*b*][1,4]oxazin-6(2*H*)-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 22b | (3a*R*,11a*S*)-5-(2-((4a*R*/*S*,7a*R*/*S*)-4-(1-acryloylazetidin-3-yl)hexahydropyrrolo[3,4-*b*][1,4]oxazin-6(2*H*)-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 23a | (3a*R*,11a*S*)-5-(2-((*S*/*R*)-8-acryloyloctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 23b | (3a*R*,11a*S*)-5-(2-((*R*/*S*)-8-acryloyloctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 24a | (3a*R*,11a*S*)-5-(2-((*R*/*S*)-1-acryloyltetrahydro-1'*H*-spiro[azetidine-3,6'-pyrazino[2,1-*c*][1,4]oxazin]-8'(7'*H*)-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 24b | (3a*R*,11a*S*)-5-(2-((*S*/*R*)-1-acryloyltetrahydro-1'*H*-spiro[azetidine-3,6'-pyrazino[2,1-*c*][1,4]oxazin]-8'(7'*H*)-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 25a | (3a*R*,11a*S*)-5-(2-((3a*S*/*R*,7a*S*/*R*)-2-acryloyloctahydro-5*H*-pyrrolo[3,4-*c*]pyridin-5-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 25b | (3a*R*,11a*S*)-5-(2-((3a*R*/*S*,7a*R*/*S*)-2-acryloyloctahydro-5*H*-pyrrolo[3,4-*c*]pyridin-5-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 26a | (3a*R*,11a*S*)-5-(2-((*R*/*S*)-1-acryloyltetrahydro-1'*H*-spiro[azetidine-3,6'-pyrazino[2,1-*c*][1,4]oxazin]-8'(7'*H*)-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 26b | (3a*R*,11a*S*)-5-(2-((*S*/*R*)-1-acryloyltetrahydro-1'*H*-spiro[azetidine-3,6'-pyrazino[2,1-*c*][1,4]oxazin]-8'(7'*H*)-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 27 | (3a*R*,11a*S*)-5-(2-(2-((*E*)-4-(dimethylamino)but-2-enoyl)-5-oxa-2,8-diazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione hydrochloride |
| 28 | (3a*R*,11a*S*)-5-(2-((*R*)-4-(1-acryloylazetidin-3-yl)-3-(fluoromethyl)piperazin-1-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 29 | (3a*R*,11a*S*)-5-(2-((*S*)-4-(1-acryloylazetidin-3-yl)-6-fluoro-1,4-diazepan-1-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 30 | (3a*R*,11a*S*)-5-(2-(2-acryloyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 31 | (3a*R*,11a*S*)-5-(2-((*R*)-2-acryloyl-6-methyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 32 | (3a*R*,11a*S*)-5-(2-((*S*)-2-acryloyl-6-methyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 33 | (3a*R*,11a*S*)-5-(2-(2-acryloyl-2,5,8-triazaspiro[3.5]nonan-8-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 34a | (3a*R*,11a*S*)-5-(2-((*S*/*R*)-2-acryloyl-9-methyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 34b | (3a*R*,11a*S*)-5-(2-((*R*/*S*)-2-acryloyl-9-methyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 35 | (3a*R*,11a*S*)-5-(2-(2-acryloyl-5-ethyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 36 | (3a*R*,11a*S*)-5-(2-(5-acetyl-2-acryloyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 37a | (3a*R*,11a*S*)-5-(2-((6*R*,9*S*/*R*)-2-acryloyl-6,9-dimethyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 37b | (3a*R*,11a*S*)-5-(2-((6*R*,9*R*/*S*)-2-acryloyl-6,9-dimethyl-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 38 | (3a*R*,11a*S*)-5-((1-(1-acryloylazetidin-3-yl)-1*H*-imidazol-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 39 | (3a*R*,11a*S*)-5-((1-(1-acryloylazetidin-3-yl)-1*H*-imidazol-4-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 40 | (3a*R*,11a*S*)-5-((1-(1-acryloylazetidin-3-yl)-1*H*-imidazol-4-yl)methyl)-8-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 41 | (3a*R*,11a*S*)-5-((1-(1-acryloylazetidin-3-yl)-1*H*-pyrazol-3-yl)methyl)-8-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 42 | (3a*R*,11a*S*)-5-((1-(1-acryloylazetidin-3-yl)-1H-1,2,4-triazol-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 43 | (3a*R*,11a*S*)-5-(3-((1-((*E*)-4-(dimethylamino)but-2-enoyl)azetidin-3-yl)oxy)propyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 44 | (3a*R*,11a*S*)-6-chloro-5-(3-((1-((*E*)-4-(dimethylamino)but-2-enoyl)azetidin-3-yl)oxy)propyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 45 | (3a*R*,11a*S*)-5-(2-((*S*)-8-acryloyl-1-oxooctahydro-2H-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 46 | (3a*R*,11a*S*)-5-(2-((*R*)-8-acryloyl-1-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3H)-dione |
| 47 | (3a*R*,11a*S*)-5-(2-((*S*)-8-acryloyl-3-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 48 | (3a*R*,11a*S*)-5-(2-((*R*)-8-acryloyl-3-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 49 | (3a*R*,11a*S*)-5-(2-(5-acryloyl-4,5,6,7-tetrahydro-1*H*-imidazo[4,5-*c*]pyridin-1-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 50 | (3a*R*,11a*S*)-5-(2-(5-acryloyl-4,5,6,7-tetrahydro-3*H*-imidazo[4,5-*c*]pyridin-3-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 51 | (3a*R*,11a*S*)-5-(2-(4-(1-acryloylazetidin-3-yl)piperazin-1-yl)-2-oxoethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 52 | (3a*R*,11a*S*)-5-(2-((*R*)-2-acryloyl-6-methyl-2,5,8-triazaspiro[3.5]nonan-8-yl)-2-oxoethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 53 | (3a*R*,11a*S*)-5-(2-(4-acryloylpiperazin-1-yl)pyridin-4-yl)-6-fluoro-10-methyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione |
| 54 | (3a*R*,11a*S*)-5-(3-(4-acryloylpiperazin-1-yl)phenyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 55 | (3a*R*,11a*S*)-5-(6-(4-acryloylpiperazin-1-yl)pyridin-2-yl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 56 | (3a*R*,11a*S*)-5-(6-(4-acryloylpiperazin-1-yl)pyridin-2-yl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 57 | (3a*R*,11a*S*)-5-(6-((1-acryloylazetidin-3-yl)oxy)pyridin-2-yl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 58 | (3a*R*,11a*S*)-5-(2-((7*R*/*S*,9a*R*/*S*)-8-acryloyl-7-methyl-6-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 59 | (3a*R*,11a*S*)-5-(2-((7*S*/*R*,9a*S*/*R*)-8-acryloyl-7-methyl-6-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 60 | (3a*R*,11a*S*)-5-(2-((7*R*/*S*,9a*S*/*R*)-8-acryloyl-7-methyl-6-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 61 | (3a*R*,11aS)-5-(2-((7*S*/*R*,9a*R*/*S*)-8-acryloyl-7-methyl-6-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 62a | (3a*R*,11a*S*)-5-(2-((*R*/*S*)-8-acryloyl-6-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 62b | (3a*R*,11a*S*)-5-(2-((*S*/*R*)-8-acryloyl-6-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione |
| 63a | (3a*R*,11a*S*)-5-(2-((6*R*/*S*,9a*S*/*R*)-8-acryloyl-6-methyloctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 63b | (3a*R*,11a*S*)-5-(2-((6*S*/*R*,9a*R*/*S*)-8-acryloyl-6-methyloctahydro-2*H*-pyrazino[1,2-*a*]pyrazin-2-yl) ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 64a | (3a*R*,11a*S*)-5-(2-((6*R*/*S*,9a*R*/*S*)-8-acryloyl-6-methyloctahydro-2*H*-pyrazino[1,2-*a*]pyrazin-2-yl) ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 64b | (3a*R*,11a*S*)-5-(2-((6*S*/*R*,9a*S*/*R*)-8-acryloyl-6-methyloctahydro-2*H*-pyrazino[1,2-*a*]pyrazin-2-yl) ethyl)-6-fluoro-10-methyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione |
| 65 | *N*-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-1-(2-((dimethylamino)methyl)acryloyl)-*N*-methylazetidine-3-carboxamide |
| 66 | (3a*R*,11a*S*)-5-(2-(((3*R*,4*S*)-1-acryloyl-3-fluoropiperidin-4-yl)amino)ethyl)-6-chloro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 67 | (3a*R*,11a*S*)-5-(2-(((3*R*,4*S*)-1-acryloyl-3-fluoropiperidin-4-yl)(methyl)amino)ethyl)-6-chloro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 68 | (3a*R*,11a*S*)-5-((*S*/*R*)-3-((1-acryloylazetidin-3-yl)(methyl)amino)-2-hydroxypropyl)-6-chloro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 69 | (3a*R*,11a*S*)-5-((*R*/*S*)-3-((1-acryloylazetidin-3-yl)(methyl)amino)-2-hydroxypropyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 70 | (3a*R*,11a*S*)-5-(3-((1-acryloylazetidin-3-yl)amino)propyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 71 | (3a*R*,11a*S*)-5-(3-((1-acryloylazetidin-3-yl)(methyl)amino)propyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 72 | (3a*R*,11a*S*)-5-(2-(4-(1-acryloyl-3-methylazetidin-3-yl)piperazin-1-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 73a | (3a*R*,11a*S*)-6-chloro-5-(((*R*/*S*)-7-(2-chloroacetyl)-6-methyl-5,6,7,8-tetrahydroimidazo[1,2-*a*]pyrazin-2-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 73b | (3a*R*,11a*S*)-6-chloro-5-(((*S*/*R*)-7-(2-chloroacetyl)-6-methyl-5,6,7,8-tetrahydroimidazo[1,2-*a*]pyrazin-2-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 74 | (3a*R*,11a*S*)-5-((7-acryloyl-3-methyl-5,6,7,8-tetrahydroimidazo[1,2-*a*]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 75 | (3a*R*,11a*S*)-5-(2-(4-(1-acryloylazetidin-3-yl)piperazin-1-yl)-2-oxoethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 76a | (3a*R*,11aS)-5-(2-((*R*/*S*)-8-acryloyloctahydro-2*H*-pyrazino[1,2-*a*]pyrazin-2-yl)-2-oxoethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 76b | (3a*R*,11a*S*)-5-(2-((*S*/*R*)-8-acryloyloctahydro-2*H*-pyrazino[1,2-*a*]pyrazin-2-yl)-2-oxoethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 77 | (3a*R*,11a*S*)-5-(3-((1-(2-((dimethylamino)methyl)acryloyl)azetidin-3-yl)oxy)propyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 78 | 2-(3-(3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propoxy)azetidine-1-carbonyl)allyl acetate |
| 79 | (3a*R*,11a*S*)-5-(3-((1-((*Z*)-2-chloro-4-(dimethylamino)but-2-enoyl)azetidin-3-yl)oxy)propyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 80 | (3a*R*,11a*S*)-5-(3-((1-((*E*)-2-((dimethylamino)methyl)but-2-enoyl)azetidin-3-yl)oxy)propyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 81 | (3a*R*,11a*S*)-5-(3-((1-(2-((dimethylamino)methyl)acryloyl)azetidin-3-yl)oxy)propyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 82 | (3a*R*,11a*S*)-6,10-dimethyl-1-(6-methyl-4-(trifluoromethyl)pyridin-2-yl)-5-(3-((1-(1,2,5,6-tetrahydropyridine-3-carbonyl)azetidin-3-yl)oxy)propyl)-1,3a,4,5,10,11a-hexahydro-2*H*-benzo[*b*]pyrrolo[2,3-*f*][1,4]diazocine-2,11(3*H*)-dione |
| 83 | (*E*)-*N*-(5-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)pentyl)-4-(dimethylamino)but-2-enamide |
| 84 | *N*-(5-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)pentyl)-2-((dimethylamino)methyl)acrylamide |
| 85 | *N*-(2-(2-((3a*R*,11a*S*)-6,10-dimethyl-1-(6-methyl-4-(trifluoromethyl)pyridin-2-yl) 2,11-dioxo-1,2,3,3a,4,10,11,11a-octahydro-5*H*-benzo[*b*]pyrrolo[2,3-*f*][1,4]diazocin-5-yl)ethoxy) ethyl)-2-((dimethylamino)methyl)acrylamide |
| 86 | (3a*R*,11a*S*)-6-chloro-5-(3-(4-(2-((dimethylamino)methyl)acryloyl)piperazin-1-yl)-3-oxopropyl)-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 87 | *N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl) acrylamide |
| 88 | *N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-1,2,5,6-tetrahydropyridine-3-carboxamide |
| 89 | (*E*)-*N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-2-((dimethylamino)methyl)but-2-enamide |
| 90 | *N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-methoxyphenyl)acrylamide |
| 91 | *N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-((2-(dimethylamino)ethyl)(methyl)amino)phenyl)acrylamide |

(continued)

| Example Number | Name |
|---|---|
| 92 | (3a*R*,11a*S*)-5-((1-(1-acryloylazetidin-3-yl)piperidin-4-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 93 | (3a*R*,11a*S*)-5-((1-(1-acryloylazetidin-3-yl)piperidin-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 94 | (3a*R*,11a*S*)-5-((1-(1-acryloylazetidin-3-yl)-4-fluoropiperidin-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 95a | (3a*R*,11a*S*)-5-((1-((*S*/*R*)-1-acryloylpyrrolidin-3-yl)piperidin-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 95b | (3a*R*,11a*S*)-5-((1-((*R*/*S*)-1-acryloylpyrrolidin-3-yl)piperidin-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 96 | (3a*R*,11a*S*)-5-((1-(1-acryloylazetidin-3-yl)-3-hydroxypiperidin-4-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 97 | (3a*R*,11a*S*)-5-(2-(4-(1-acryloylazetidin-3-yl)piperazin-1-yl)propyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 98 | (3a*R*,11a*S*)-5-((5-(1-acryloylazetidin-3-yl)-1*H*-1,2,4-triazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 99 | (3a*R*,11a*S*)-5-((3-(1-acryloylazetidin-3-yl)-1-methyl-1*H*-1,2,4-triazol-5-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 100 | (3a*R*,11a*S*)-5-((5-(1-acryloylazetidin-3-yl)-1-methyl-1*H*-1,2,4-triazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 101 | (3a*R*,11a*S*)-5-((5-(1-acryloylazetidin-3-yl)-1,2,4-oxadiazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 102 | (3a*R*,11a*S*)-5-((3-(1-acryloylazetidin-3-yl)-1,2,4-oxadiazol-5-yl)methyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 103 | (3a*R*,11a*S*)-5-((5-(1-acryloylazetidin-3-yl)-1,3,4-oxadiazol-2-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 104 | (3a*R*,11a*S*)-5-(2-(2-acryloyl-6-oxo-2,5,8-triazaspiro[3.5]nonan-8-yl)ethyl)-6-fluoro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 105 | (3a*R*,11a*S*)-5-(2-(1-acryloyl-6'*H*-spiro[azetidine-3,5'-imidazo[1,2-*a*]pyrazin]-7'(8'*H*)-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 106 | (3a*R*,11a*S*)-5-(2-(1-acryloylspiro[azetidine-3,3'-imidazo[1,2-*a*]imidazol]-1'(2'*H*)-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 107 | (3a*R*,11a*S*)-5-(2-((3*R*/*S*,9a*S*/*R*)-8-acryloyl-3-methyl-4-oxooctahydro-2H-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 108 | (3a*R*,11a*S*)-5-(2-((3*S*/*R*,9a*R*/*S*)-8-acryloyl-3-methyl-4-oxooctahydro-2H-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 109 | (3a*R*,11a*S*)-5-(2-((3*R*/*S*,9a*R*/*S*)-8-acryloyl-3-methyl-4-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 110 | (3a*R*,11a*S*)-5-(2-((3*S*/*R*,9a*S*/*R*)-8-acryloyl-3-methyl-4-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 111 | (3a*R*,11a*S*)-5-(2-(2-acryloyl-5-methyl-6-oxo-2,5,8-triazaspiro[3.5]nonan-8-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 112 | (3a*R*,11a*S*)-5-(2-(4-(1-acryloylazetidin-3-yl)-3-oxopiperazin-1-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 113 | (3a*R*,11a*S*)-5-(2-(((*rel-trans*)-1-acryloyl-4-hydroxypiperidin-3-yl)(methyl)amino)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 114 | (3a*R*,11a*S*)-5-(2-(2-acryloyl-2,5,8-triazaspiro[3.5]nonan-8-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 115 | (3a*R*,11a*S*)-5-(2-(4-(1-acryloylazetidin-3-yl)piperazin-1-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 116a | (3a*R*,11a*S*)-5-(2-((*R*/*S*)-8-acryloyl-4-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 116b | (3a*R*,11a*S*)-5-(2-((*S*/*R*)-8-acryloyl-4-oxooctahydro-2*H*-pyrazino[1,2-*a*]pyrazin-2-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 117a | (*R*/*S*)-2-acryloyl-6-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,6-diazaspiro[3.4]octane-8-carbonitrile |
| 117b | (*S*/*R*)-2-acryloyl-6-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,6-diazaspiro[3.4]octane-8-carbonitrile |
| 118a | (*R*/*S*)-2-acryloyl-6-(2-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,6-diazaspiro[3.4]octane-8-carbonitrile |
| 118b | (*S*/*R*)-2-acryloyl-6-(2-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,6-diazaspiro[3.4]octane-8-carbonitrile |
| 119a | (*R*/*S*)-2-acryloyl-6-(2-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-8-methyl-2,6-diazaspiro[3.4]octane-8-carbonitrile |

(continued)

| Example Number | Name |
|---|---|
| 119b | (*S*/*R*)-2-acryloyl-6-(2-((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl) ethyl)-8-methyl-2,6-diazaspiro[3.4]octane-8-carbonitrile |
| 120 | (3a*R*,11a*S*)-5-((1-(1-acryloylazetidin-3-yl)-1*H*-pyrazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 121 | (3a*R*,11a*S*)-5-((1-(1-acryloylazetidin-3-yl)-1*H*-pyrazol-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 122 | (3a*R*,11a*S*)-5-((2-(1-acryloylazetidin-3-yl)-1*H*-imidazol-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 123 | (3a*R*,11a*S*)-5-((1-(1-acryloylazetidin-3-yl)-1*H*-1,2,4-triazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 124 | (3a*R*,11a*S*)-5-((2-(4-acryloylpiperazin-1-yl)oxazol-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 125 | (3a*R*,11a*S*)-5-((2-(4-acryloylpiperazin-1-yl)-1*H*-imidazol-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 126 | (3a*R*,11a*S*)-5-((1-(1-acryloylpiperidin-4-yl)-1*H*-1,2,4-triazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 127 | (3a*R*,11a*S*)-5-(3-(1-acryloylazetidin-3-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 128 | (3a*R*,11a*S*)-5-((6-(1-acryloylazetidin-3-yl)pyridin-2-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 129 | (3a*R*,11a*S*)-5-((2-(1-acryloylazetidin-3-yl)pyrimidin-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 130 | (3a*R*,11a*S*)-5-((2-acryloylisoindolin-5-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 131 | (3a*R*,11a*S*)-5-((2-acryloyl-1,2,3,4-tetrahydroisoquinolin-6-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 132 | (3a*R*,11a*S*)-5-((7-acryloyl-5,6,7,8-tetrahydroimidazo[1,2-*a*]pyrazin-2-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 133 | (3a*R*,11a*S*)-5-((7-acryloyl-5,6,7,8-tetrahydro-[1,2,4]triazolo[1,5-*a*]pyrazin-2-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 134 | (3a*R*,11a*S*)-5-((7-acryloyl-5,6,7,8-tetrahydro-[1,2,4]triazolo[4,3-*a*]pyrazin-3-yl)methyl)-6-fluoro-10-methyl-1-(6-methyl-4-(trifluoromethyl)pyridin-2-yl)-1,3a,4,5,10,11a-hexahydro-*2H*-benzo[*b*]pyrrolo[2,3-*f*][1,4]diazocine-2,11(3*H*)-dione |

(continued)

| Example Number | Name |
|---|---|
| 135 | (3a*R*,11a*S*)-5-((1-(1-acryloylpiperidin-4-yl)-1*H*-1,2,4-triazol-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 136 | (3a*R*,11a*S*)-5-((7-acryloyl-5,6,7,8-tetrahydroimidazo[1,2-*a*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 137 | (3a*R*,11a*S*)-5-((1-(1-acryloyl-3-methylazetidin-3-yl)piperidin-4-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 138 | (3a*R*,11a*S*)-5-(3-((1-acryloylazetidin-3-yl)(methyl)amino)propyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 139 | (3a*R*,11a*S*)-5-((1-(1-acryloylazetidin-3-yl)-1*H*-1,2,4-triazol-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 140a | (3a*R*,11a*S*)-5-(((3*S*,9a*S*/*R*)-8-acryloyl-2-methyloctahydro-2*H*-pyrazino[1,2-*a*]pyrazin-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 140b | (3a*R*,11a*S*)-5-(((3*S*,9a*R*/*S*)-8-acryloyl-2-methyloctahydro-2*H*-pyrazino[1,2-*a*]pyrazin-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 141a | (3a*R*,11a*S*)-5-(4-((*R*/*S*)-1-acryloylpyrrolidin-2-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 141b | (3a*R*,11a*S*)-5-(4-((*S*/*R*)-1-acryloylpyrrolidin-2-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 142a | (3a*R*,11a*S*)-5-(((*R*/*S*)-7-acryloyl-8-methyl-5,6,7,8-tetrahydroimidazo[1,2-*a*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 142b | (3a*R*,11a*S*)-5-(((*S*/*R*)-7-acryloyl-8-methyl-5,6,7,8-tetrahydroimidazo[1,2-*a*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 143a | (3a*R*,11a*S*)-5-(((*R*/*S*)-7-acryloyl-6-methyl-5,6,7,8-tetrahydroimidazo[1,2-*a*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 143b | (3a*R*,11a*S*)-5-(((*S*/*R*)-7-acryloyl-6-methyl-5,6,7,8-tetrahydroimidazo[1,2-*a*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 144a | (3a*R*,11a*S*)-5-(((*R*/*S*)-7-acryloyl-6-methyl-5,6,7,8-tetrahydroimidazo[1,2-*a*]pyrazin-2-yl)methyl)-6-chloro-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 144b | (3a*R*,11a*S*)-5-(((*S*/*R*)-7-acryloyl-6-methyl-5,6,7,8-tetrahydroimidazo[1,2-*a*]pyrazin-2-yl)methyl)-6-chloro-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 145 | (3a*R*,11a*S*)-5-((1-(1-((*E*)-4-(dimethylamino)but-2-enoyl)azetidin-3-yl)-1*H*-pyrazol-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 146 | (3a*R*,11a*S*)-5-(4-(1-((*E*)-4-(dimethylamino)but-2-enoyl)azetidin-3-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 147 | (3a*R*,11a*S*)-5-(3-(1-((*E*)-4-(dimethylamino)but-2-enoyl)azetidin-3-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 148 | (3a*R*,11a*S*)-5-((2-((*E*)-4-(dimethylamino)but-2-enoyl)isoindolin-5-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 149 | (3a*R*,11a*S*)-5-((2-((*E*)-4-(dimethylamino)but-2-enoyl)-1,2,3,4-tetrahydroisoquinolin-6-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 150 | (3a*R*,11a*S*)-5-(3-((1-(2-((dimethylamino)methyl)acryloyl)azetidin-3-yl)oxy)propyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 151 | (3a*R*,11aS)-5-(4-(1-(2-((dimethylamino)methyl)acryloyl)azetidin-3-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 152 | (3a*R*,11a*S*)-5-(3-(1-(2-((dimethylamino)methyl)acryloyl)azetidin-3-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 153 | (3a*R*,11a*S*)-5-((2-(2-((dimethylamino)methyl)acryloyl)isoindolin-5-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 154 | (3a*R*,11a*S*)-5-((2-(2-((dimethylamino)methyl)acryloyl)-1,2,3,4-tetrahydroisoquinolin-6-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 155 | (3a*R*,11a*S*)-6-chloro-5-((4-(1-((*E*)-4-(dimethylamino)but-2-enoyl)azetidin-3-yl)cyclohexyl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 156 | (3a*R*,11a*S*)-6-chloro-5-(3-((1-(2-((dimethylamino)methyl)acryloyl)azetidin-3-yl)oxy)propyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 157 | (3a*R*,11a*S*)-6-chloro-5-(2-((1-(2-((dimethylamino)methyl)acryloyl)piperidin-4-yl)oxy)ethyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 158 | (3a*R*,11a*S*)-6-chloro-5-(3-(1-(2-((dimethylamino)methyl)acryloyl)piperidin-4-yl)propyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 159 | (3a*R*,11a*S*)-6-chloro-5-((7-(2-((dimethylamino)methyl)acryloyl)-5,6,7,8-tetrahydroimidazo[1,2-*a*]pyrazin-2-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 160 | (3a*R*,11a*S*)-6-chloro-5-((4-(1-(2-((dimethylamino)methyl)acryloyl)azetidin-3-yl)cyclohexyl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 161 | (3a*R*,11a*S*)-6-chloro-5-(4-(1-(2-((dimethylamino)methyl)acryloyl)azetidin-3-yl)butyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 162 | (3a*R*,11a*S*)-6-chloro-5-(2-((1-(2-((dimethylamino)methyl)acryloyl)azetidin-3-yl)methoxy) ethyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 163 | (3a*R*,11a*S*)-6-chloro-5-((2-(2-((dimethylamino)methyl)acryloyl)isoindolin-5-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 164 | *N*-(3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo [1.1.1]pentan-1-yl)-2-((dimethylamino)methyl)acrylamide |
| 165 | (3a*R*,11a*S*)-6-chloro-5-(3-((1-methacryloylazetidin-3-yl)oxy)propyl)-10-methyl-1-(6-methyl-4-(trifluoromethyl)pyridin-2-yl)-1,3a,4,5,10,11a-hexahydro-2*H*-benzo[*b*]pyrrolo[2,3-*f*] [1,4]diazocine-2,11(3*H*)-dione |
| 166 | (3a*R*,11a*S*)-6-chloro-5-(3-((1-(2-((diethylamino)methyl)acryloyl)azetidin-3-yl)oxy)propyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 167 | (3a*R*,11a*S*)-6-chloro-10-methyl-1-(6-methyl-4-(trifluoromethyl)pyridin-2-yl)-5-(4-(1-(2-((4-methylpiperazin-1-yl)methyl)acryloyl)azetidin-3-yl)butyl)-1,3a,4,5,10,11a-hexahydro-2*H*-benzo [*b*]pyrrolo[2,3-*f*][1,4]diazocine-2,11(3*H*)-dione |
| 168 | (3a*R*,11a*S*)-6-chloro-5-(3-(1-(2-(hydroxymethyl)acryloyl)piperidin-4-yl)propyl)-10-methyl-1-(6-methyl-4-(trifluoromethyl)pyridin-2-yl)-1,3a,4,5,10,11a-hexahydro-2*H*-benzo[*b*]pyrrolo[2,3-*f*] [1,4]diazocine-2,11(3*H*)-dione |
| 169 | (3a*R*,11a*S*)-5-(3-((1-((*E*)-4-(dimethylamino)but-2-enoyl)azetidin-3-yl)oxy)propyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 170a | (3a*R*,11a*S*)-5-(4-((*R*/*S*)-1-((*E*)-4-(dimethylamino)but-2-enoyl)pyrrolidin-2-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 170b | (3a*R*,11a*S*)-5-(4-((*S*/*R*)-1-((*E*)-4-(dimethylamino)but-2-enoyl)pyrrolidin-2-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 171a | (3a*R*,11a*S*)-5-(4-((*R*/*S*)-1-(2-((dimethylamino)methyl)acryloyl)pyrrolidin-2-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 171b | (3a*R*,11a*S*)-5-(4-((*S*/*R*)-1-(2-((dimethylamino)methyl)acryloyl)pyrrolidin-2-yl)benzyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 172a | (3a*R*,11a*S*)-6-chloro-10-methyl-1-(6-methyl-4-(trifluoromethyl)pyridin-2-yl)-5-(((*S*/*R*)-6-methyl-7-propioloyl-5,6,7,8-tetrahydroimidazo[1,2-*a*]pyrazin-2-yl)methyl)-1,3a,4,5,10,11a-hexahydro-2*H*-benzo[*b*]pyrrolo[2,3-*f*][1,4]diazocine-2,11(3*H*)-dione |
| 172b | (3a*R*,11a*S*)-6-chloro-10-methyl-1-(6-methyl-4-(trifluoromethyl)pyridin-2-yl)-5-(((*R*/*S*)-6-methyl-7-propioloyl-5,6,7,8-tetrahydroimidazo[1,2-*a*]pyrazin-2-yl)methyl)-1,3a,4,5,10,11a-hexahydro-2*H*-benzo[*b*]pyrrolo[2,3-*f*][1,4]diazocine-2,11(3*H*)-dione |
| 173 | (3a*R*,11a*S*)-5-((5-acryloyl-4,5,6,7-tetrahydropyrazolo[1,5-*a*]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 174 | *N*-(3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo [1.1.1]pentan-1-yl)acrylamide |

(continued)

| Example Number | Name |
|---|---|
| 175 | *N*-((3-(((3a*R*,11a*S*)-6,10-dimethyl-1-(6-methyl-4- (trifluoromethyl) pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo [1.1.1]pentan-1-yl)methyl)acrylamide |
| 176 | (3a*R*,11a*S*)-5-((2-((*E*)-4-(dimethylamino)but-2-enoyl)isoindolin-5-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*] [1,4]diazocine-2,11(3*H*)-dione |
| 177 | (*E*)-*N*-(4-(((3a*R*,11a*S*)-6,10-dimethyl-1-(6-methyl-4-(trifluoromethyl) pyridin-2-yl)-2,11-dioxo-1,2,3,3a,4,10,11,11a-octahydro-5*H*-benzo[*b*]pyrrolo[2,3-*f*][1,4]diazocin-5-yl)methyl)benzyl)-4-(dimethylamino)but-2-enamide |
| 178 | (*E*)-*N*-(3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo [1.1.1]pentan-1-yl)-4-(dimethylamino)but-2-enamide |
| 179 | (*E*)-*N*-((3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo [1.1.1]pentan-1-yl)methyl)-4-(dimethylamino)but-2-enamide |
| 180 | (3a*R*,11a*S*)-5-(2-(((*R*)-1-(2-((dimethylamino)methyl)acryloyl)pyrrolidin-3-yl)methoxy) ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 181 | (3a*R*,11a*S*)-5-(2-(((S)-1-(2-((dimethylamino)methyl)acryloyl)pyrrolidin-3-yl)methoxy) ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 182 | (3a*R*,11a*S*)-5-(2-((1-(2-((dimethylamino)methyl)acryloyl)azetidin-3-yl)methoxy)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 183 | (3a*R*,11a*S*)-5-(4-(1-(2-((dimethylamino)methyl)acryloyl)azetidin-3-yl)butyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*] [1,4]diazocine-2,11(3*H*)-dione |
| 184 | *N*-(3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo [1.1.1]pentan-1-yl)-2-((dimethylamino)methyl)acrylamide |
| 185 | *N*-((3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo [1.1.1]pentan-1-yl)methyl)-2-((dimethylamino)methyl)acrylamide |
| 186 | (3a*R*,11a*S*)-5-(4-(1-(2-((dimethylamino)methyl)acryloyl)azetidin-3-yl)benzyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 187 | (3a*R*,11a*S*)-5-(3-(1-(2-((dimethylamino)methyl)acryloyl)azetidin-3-yl)-4-fluorobenzyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 188 | (3a*R*,11a*S*)-5-((2-(2-((dimethylamino)methyl)acryloyl)isoindolin-5-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 189 | *N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)benzyl)-2-((dimethylamino)methyl)acrylamide |
| 190 | *N*-((5-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)pyridin-2-yl)methyl)-2-((dimethylamino)methyl)acrylamide |

(continued)

| Example Number | Name |
|---|---|
| 191 | (3a*R*,11a*S*)-5-(3-((1-(2-((dimethylamino)methyl)acryloyl)azetidin-3-yl)oxy)propyl)-6-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 192 | (3a*R*,11a*S*)-5-(4-(1-(2-((dimethylamino)methyl)acryloyl)azetidin-3-yl)butyl)-6-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 193 | (3a*R*,11a*S*)-6,10-dimethyl-1-(6-methyl-4-(trifluoromethyl)pyridin-2-yl)-5-(2-((1-(2-((4-methylpiperazin-1-yl)methylacryloyl)azetidin-3-yl)methoxy)ethyl)-1,3a,4,5,10,11a-hexahydro-2*H*-benzo[*b*]pyrrolo[2,3-*f*][1,4]diazocine-2,11(3*H*)-dione |
| 194 | (3a*R*,11a*S*)-5-(3-((1-((*E*)-2-((dimethylamino)methyl)but-2-enoyl)azetidin-3-yl)oxy)propyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 195 | (3a*R*,11a*S*)-5-(4-(1-((*E*)-2-((dimethylamino)methyl)but-2-enoyl)azetidin-3-yl)benzyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 196 | (3a*R*,11a*S*)-6,10-dimethyl-5-(3-((1-(1-methyl-1,2,5,6-tetrahydropyridine-3-carbonyl)azetidin-3-yl)oxy)propyl)-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 197 | (3a*R*,11a*S*)-6,10-dimethyl-5-(2-((1-(1-methyl-1,2,5,6-tetrahydropyridine-3-carbonyl)azetidin-3-yl)methoxy)ethyl)-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 198 | (3a*R*,11a*S*)-6,10-dimethyl-1-(6-methyl-4-(trifluoromethyl)pyridin-2-yl)-5-((5-propioloyl-4,5,6,7-tetrahydropyrazolo[1,5-*a*]pyrazin-2-yl)methyl)-1,3a,4,5,10,11a-hexahydro-2*H*-benzo[*b*]pyrrolo[2,3-*f*][1,4]diazocine-2,11(3*H*)-dione |
| 199 | (3a*R*,11a*S*)-6,10-dimethyl-1-(6-methyl-4-(trifluoromethyl)pyridin-2-yl)-5-(((*R*/*S*)-6-methyl-7-propioloyl-5,6,7,8-tetrahydroimidazo[1,2-*a*]pyrazin-2-yl)methyl)-1,3a,4,5,10,11a-hexahydro-2*H*-benzo[*b*]pyrrolo[2,3-*f*][1,4]diazocine-2,11(3*H*)-dione |
| 200 | *N*-(3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)propiolamide |
| 201 | (3a*R*,11a*S*)-5-((7-(but-2-ynoyl)-5,6,7,8-tetrahydroimidazo[1,2-*a*]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 202 | (3a*R*,11a*S*)-5-((7-acryloyl-5,6,7,8-tetrahydro-[1,2,4]triazolo[1,5-*a*]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 203 | (3a*R*,11a*S*)-5-((7-acryloyl-5,6,7,8-tetrahydroimidazo[1,2-*a*]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 204 | (3a*R*,11a*S*)-5-((1-(1-acryloylpiperidin-4-yl)-1*H*-1,2,4-triazol-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 205 | (3a*R*,11a*S*)-5-(3-((1-acryloylazetidin-3-yl)(methyl)amino)propyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 206a | (3a*R*,11a*S*)-5-(((*R*/*S*)-7-acryloyl-6-methyl-5,6,7,8-tetrahydroimidazo[1,2-*a*]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 206b | (3a*R*,11a*S*)-5-(((S/R)-7-acryloyl-6-methyl-5,6,7,8-tetrahydroimidazo[1,2-*a*]pyrazin-2-yl) methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 207 | (*E*)-*N*-(4-(((3a*R*,11a*S*)-6,10-dimethyl-1-(6-methyl-4-(trifluoromethyl) pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-4-(dimethylamino)but-2-enamide |
| 208 | (*E*)-*N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-methoxyphenyl)-4-(dimethylamino)but-2-enamide |
| 209 | *N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-2-((dimethylamino)methyl)acrylamide |
| 210 | *N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-methoxyphenyl)-2-((dimethylamino)methyl)acrylamide |
| 211 | *N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-1-methyl-1,2,5,6-tetrahydropyridine-3-carboxamide |
| 212 | (3a*R*,11a*S*)-5-((1-(1-acryloylazetidin-3-yl)piperidin-4-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 213 | (3a*R*,11a*S*)-5-(((*S*)-1-(1-acryloylazetidin-3-yl)pyrrolidin-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 214 | (3a*R*,11a*S*)-5-(((*R*)-1-(1-acryloylazetidin-3-yl)pyrrolidin-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 215 | (3a*R*,11a*S*)-5-(2-(1-(1-acryloylazetidin-3-yl)piperidin-4-yl)ethyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 216 | (3a*R*,11a*S*)-5-(1-(1-acryloylazetidin-3-yl)piperidin-4-yl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 217 | (3a*R*,11a*S*)-5-((1-(1-acryloylazetidin-3-yl)-4-fluoropiperidin-4-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 218 | (3a*R*,11a*S*)-5-(((*S*)-1-(1-acryloylazetidin-3-yl)pyrrolidin-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 219 | (3a*R*,11a*S*)-5-(((*R*)-1-(1-acryloylazetidin-3-yl)pyrrolidin-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 220 | (3a*R*,11a*S*)-5-(2-((*S*)-1-(1-acryloylazetidin-3-yl)pyrrolidin-3-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 221 | (3a*R*,11a*S*)-5-(2-((*R*)-1-(1-acryloylazetidin-3-yl)pyrrolidin-3-yl)ethyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 222 | (3a*R*,11a*S*)-5-((1-(1-acryloylpyrrolidin-3-yl)piperidin-4-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 223 | (3a*R*,11a*S*)-5-(((*S*)-1-(1-acryloylazetidin-3-yl)pyrrolidin-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 224 | (3a*R*,11a*S*)-5-(((*R*)-1-(1-acryloylazetidin-3-yl)pyrrolidin-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 225a | (3a*R*,11a*S*)-5-(((*R*/*S*)-1-(1-acryloylazetidin-3-yl)piperidin-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 225b | (3a*R*,11a*S*)-5-(((*S*/*R*)-1-(1-acryloylazetidin-3-yl)piperidin-3-yl)methyl)-6-fluoro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 226a | (3*S*/*R*,4*R*/*S*)-1-(1-acryloylazetidin-3-yl)-4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)piperidine-3-carbonitrile |
| 226b | (3*R*/*S*,4*S*/*R*)-1-(1-acryloylazetidin-3-yl)-4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)piperidine-3-carbonitrile |
| 227a | (3*S*/*R*,4*S*/*R*)-1-(1-acryloylazetidin-3-yl)-4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)piperidine-3-carbonitrile |
| 227b | (3*R*/*S*,4*R*/*S*)-1-(1-acryloylazetidin-3-yl)-4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)piperidine-3-carbonitrile |
| 228 | (3a*R*,11a*S*)-5-((6-acryloyl-6,7-dihydro-5*H*-pyrrolo[3,4-*b*]pyrazin-2-yl)methyl)-6,10-dimethyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 229 | (3a*R*,11a*S*)-5-((6-acryloyl-6,7-dihydro-5*H*-pyrrolo[3,4-*b*]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 230 | (3a*R*,11a*S*)-5-(((*R*/*S*)-6-acryloyl-7-(hydroxymethyl)-6,7-dihydro-5*H*-pyrrolo[3,4-*b*]pyridin-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 231 | (3a*R*,11a*S*)-5-(((*S*/*R*)-6-acryloyl-7-(hydroxymethyl)-6,7-dihydro-5*H*-pyrrolo[3,4-*b*]pyridin-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 232 | (3a*R*,11a*S*)-5-(((*R*/*S*)-6-acryloyl-7-(hydroxymethyl)-6,7-dihydro-5*H*-pyrrolo[3,4-*b*]pyridin-3-yl)methyl)-6,10-dimethyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 233 | (3a*R*,11a*S*)-5-(((*S*/*R*)-6-acryloyl-7-(hydroxymethyl)-6,7-dihydro-5*H*-pyrrolo[3,4-*b*]pyridin-3-yl)methyl)-6,10-dimethyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 234 | (3a*R*,11a*S*)-5-(((*S*/*R*)-6-acryloyl-7-(methoxymethyl)-6,7-dihydro-5*H*-pyrrolo[3,4-*b*]pyridin-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 235 | (3a*R*,11a*S*)-5-(((*R*/*S*)-6-acryloyl-7-(methoxymethyl)-6,7-dihydro-5*H*-pyrrolo[3,4-*b*]pyridin-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 236 | (3a*R*,11a*S*)-5-(((*S*/*R*)-6-acryloyl-7-(methoxymethyl)-6,7-dihydro-5*H*-pyrrolo[3,4-*b*]pyridin-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 237 | (3a*R*,11a*S*)-5-(((*R*/*S*)-6-acryloyl-7-(methoxymethyl)-6,7-dihydro-5*H*-pyrrolo[3,4-*b*]pyridin-3-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 238 | (3a*R*,11a*S*)-5-((6-acryloyl-5-(hydroxymethyl)-6,7-dihydro-5*H*-pyrrolo[3,4-*b*]pyridin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 239 | (3a*R*,11a*S*)-5-((6-acryloyl-5-(hydroxymethyl)-6,7-dihydro-5*H*-pyrrolo[3,4-*b*]pyridin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 240 | (3a*R*,11a*S*)-5-((6-acryloyl-6,7-dihydro-5*H*-pyrrolo[3,4-*b*]pyridin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 241 | (3a*R*,11a*S*)-5-((5-acryloyl-1-methyl-1,4,5,6-tetrahydropyrrolo[3,4-*d*]imidazol-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 242 | (3a*R*,11a*S*)-5-((5-acryloyl-1-methyl-1,4,5,6-tetrahydropyrrolo[3,4-*d*]imidazol-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 243a | (3a*R*,11a*S*)-5-(((*S*/*R*)-7-acryloyl-6-(difluoromethyl)-5,6,7,8-tetrahydroimidazo[1,2-a]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 243b | (3*a*R,11a*S*)-5-(((*R*/*S*)-7-acryloyl-6-(difluoromethyl)-5,6,7,8-tetrahydroimidazo[1,2-a]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 244 | (3a*R*,11a*S*)-5-((7-acryloyl-6-(hydroxymethyl)-5,6,7,8-tetrahydroimidazo[1,2-a]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 245 | (*E*)-*N*-(4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-fluorophenyl)-4-(dimethylamino)but-2-enamide |
| 246 | (*E*)-*N*-(4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-3-fluorophenyl)-4-(dimethylamino)but-2-enamide |
| 247 | (*E*)-*N*-(3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-4-(dimethylamino)but-2-enamide |
| 248 | *N*-(4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-cyanophenyl)acrylamide |
| 249 | (*E*)-4-(4-acetylpiperazin-1-yl)-*N*-(4-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)but-2-enamide |

(continued)

| Example Number | Name |
|---|---|
| 250 | (3a*R*,11a*S*)-5-((4-acryloyl-3,4-dihydro-2*H*-benzo[*b*][1,4]oxazin-6-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 251 | (3a*R*,11a*S*)-6-chloro-5-((4-((*E*)-4-(dimethylamino)but-2-enoyl)-3,4-dihydro-2*H*-benzo[*b*][1,4]oxazin-6-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 252 | (*E*)-*N*-(4-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)butyl)-4-(dimethylamino)but-2-enamide |
| 253 | (*E*)-*N*-(5-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-4-fluoropentyl)-4-(dimethylamino)but-2-enamide |
| 254 | (3a*R*,11a*S*)-5-(4-(1-((*Z*)-2-chloro-4-(dimethylamino)but-2-enoyl)azetidin-3-yl)butyl)-6,10-dimethyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 255 | (3a*R*,11a*S*)-5-((2-((*Z*)-2-chloro-4-(dimethylamino)but-2-enoyl)isoindolin-5-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 256 | (3a*R*,11a*S*)-5-(2-(4-acryloylpiperazin-1-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 257a | *N*-((*S*/*R*)-1-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-3,3-difluoropiperidin-4-yl)acrylamide |
| 257b | *N*-((*R*/*S*)-1-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-3,3-difluoropiperidin-4-yl)acrylamide |
| 258a | 2-((R/S)-1-acryloyl-4-(2-((3aR,11aS)-6-chloro-10-methyl-1-(6-methyl-4-(trifluoromethyl)pyridin-2-yl)-2,11-dioxo-1,2,3,3a,4,10,11,11a-octahydro-5H-benzo[*b*]pyrrolo[2,3-*f*][1,4]diazocin-5-yl)ethyl)piperazin-2-yl)acetonitrile |
| 258b | 2-((*S*/*R*)-1-acryloyl-4-(2-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)piperazin-2-yl)acetonitrile |
| 259 | (3a*R*,11a*S*)-5-(2-(5-acryloyl-5,6-dihydropyrrolo[3,4-d]imidazol-1(4*H*)-yl)ethyl)-6-chloro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 260 | (3aR,11aS)-5-(2-((S)-4-acryloyl-2-methylpiperazin-1-yl)ethyl)-6,10-dimethyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione |
| 261 | (3a*R*,11a*S*)-5-(2-(3-acryloyl-3,6-diazabicyclo[3.1.1]heptan-6-yl)ethyl)-6,10-dimethyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3H)-dione |
| 262 | (3a*R*,11a*S*)-5-(2-(3-acryloyl-3,8-diazabicyclo[3.2.1]octan-8-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 263 | (3a*R*,11a*S*)-5-(2-((1*R*,4*R*)-5-acryloyl-2,5-diazabicyclo[2.2.1]heptan-2-yl)ethyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 264 | (3a*R*,11a*S*)-6,10-dimethyl-1-(6-methyl-4-(trifluoromethyl)pyridin-2-yl)-5-(2-(4-propioloylpiperazin-1-yl)ethyl)-1,3a,4,5,10,11a-hexahydro-2*H*-benzo[b]pyrrolo[2,3-*f*][1,4]diazocine-2,11(3*H*)-dione |
| 265a | (3a*R*,11a*S*)-5-(3-((*S*/*R*)-4-acryloyl-3-(trifluoromethyl)piperazin-1-yl)propyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 265b | (3aR,11aS)-5-(3-((*R*/*S*)-4-acryloyl-3-(trifluoromethyl)piperazin-1-yl)propyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 266 | (3a*R*,11aS)-5-(3-(4-acryloylpiperazin-1-yl)propyl)-6-chloro-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione |
| 267a | 2-((*S*/*R*)-1-acryloyl-4-(3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propyl)piperazin-2-yl)acetonitrile |
| 267b | 2-((*R*/*S*)-1-acryloyl-4-(3-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propyl)piperazin-2-yl)acetonitrile |
| 268 | (3a*R*,11a*S*)-5-((3-((*R*)-1-acryloylpyrrolidin-2-yl)-1,2,4-oxadiazol-5-yl)methyl)-6,10-dimethyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 269 | (3a*R*,11a*S*)-5-((3-((S)-1-acryloylpyrrolidin-2-yl)-1,2,4-oxadiazol-5-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 270 | (3a*R*,11a*S*)-5-((5-((*R*)-1-acryloylpyrrolidin-2-yl)-1,2,4-oxadiazol-3-yl)methyl)-6,10-dimethyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione |
| 271 | (3a*R*,11a*S*)-5-((5-((*S*)-1-acryloylpyrrolidin-2-yl)-1,2,4-oxadiazol-3-yl)methyl)-6,10-dimethyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione |
| 272 | (3a*R*,11a*S*)-5-((5-((*R*)-1-acryloylpyrrolidin-2-yl)-1,3,4-oxadiazol-2-yl)methyl)-6,10-dimethyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione |
| 273 | (3a*R*,11a*S*)-5-((5-((*S*)-1-acryloylpyrrolidin-2-yl)-1,3,4-oxadiazol-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 274 | (3a*R*,11a*S*)-5-((5-((*R*)-1-acryloylazetidin-2-yl)-1,3,4-oxadiazol-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 275 | (3a*R*,11a*S*)-5-((5-((*S*)-1-acryloylazetidin-2-yl)-1,3,4-oxadiazol-2-yl)methyl)-6,10-dimethyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione |
| 276 | (3a*R*,11aS)-5-((6-acryloyl-6,7-dihydro-5*H*-pyrrolo[3,4-b]pyridin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 277 | (3a*R*,11a*S*)-5-((2-acryloyl-2,3-dihydro-1*H*-pyrrolo[3,4-*c*]pyridin-6-yl)methyl)-6-chloro-10-methyl-1-(6-methyl-4-(trifluoromethyl)pyridin-2-yl)-1,3a,4,5,10,11a-hexahydro-2H-benzo[b]pyrrolo[2,3-*f*][1 ,4]diazocine-2,11(3*H*)-dione |

(continued)

| Example Number | Name |
|---|---|
| 278 | (3a*R*,11a*S*)-5-((6-acryloyl-6,7-dihydro-5*H*-pyrrolo[3,4-*d*]pyrimidin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 279 | (3a*R*,11a*S*)-5-((5-acryloyl-1-ethyl-1,4,5,6-tetrahydropyrrolo[3,4-d]imidazol-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 280 | (3a*R*,11a*S*)-5-((5-acryloyl-5,6-dihydro-4*H*-pyrrolo[3,4-*d*]thiazol-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 281 | (3aR,11a*S*)-5-((4-acryloyl-3,4-dihydro-2*H*-benzo[*b*][1,4]oxazin-7-yl)methyl)-6-chloro-1 0-methyl-1-(6-methyl-4-(trifluoromethyl)pyridin-2-yl)-1 ,3a,4,5, 1 0, 11 a-hexahydro-2*H*-benzo[*b*]pyrrolo[2,3-*f*][1,4]diazocine-2,11(3*H*)-dione |
| 282 | N-(5-(((3aR,11aS)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)pyridin-2-yl)acrylamide |
| 283 | N-(6-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)pyridin-3-yl)acrylamide |
| 284 | N-(2-(((3aR,11aS)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)pyrimidin-5-yl)acrylamide |
| 285 | (3aR,11aS)-5-(2-((1-acryloyl-3-fluoroazetidin-3-yl)methoxy)ethyl)-6-chloro-10-methyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione |
| 286 | N-(5-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2,3-dihydro-1*H*-inden-2-yl)acrylamide |
| 287a | 2-((*S*/*R*)-2-acryloyl-5-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)isoindolin-1-yl)acetonitrile |
| 287b | 2-((*R*/*S*)-2-acryloyl-5-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)isoindolin-1-yl)acetonitrile |
| 288a | 2-((*R*/S)-2-acryloyl-6-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)isoindolin-1-yl)acetonitrile |
| 288b | 2-((*S*/*R*)-2-acryloyl-6-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)isoindolin-1-yl)acetonitrile |
| 289a | 2-((*R*/*S*)-6-acryloyl-2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitrile |
| 289b | 2-((*S*/*R*)-6-acryloyl-2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)-6,7-dihydro-5*H*-pyrrolo[3,4-*b*]pyridin-7-yl)acetonitrile |
| 290a | 2-((*S*/*R*)-6-acryloyl-2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)-6,7-dihydro-5*H*-pyrrolo[3,4-*b*]pyridin-5-yl)acetonitrile |

(continued)

| Example Number | Name |
|---|---|
| 290b | 2-((*R*/*S*)-6-acryloyl-2-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl) methyl)-6,7-dihydro-5*H*-pyrrolo[3,4-*b*]pyridin-5-yl)acetonitrile |
| 291a | 2-((*R*/*S*)-6-acryloyl-3-(((3aR,11aS)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl) methyl)-6,7-dihydro-5*H*-pyrrolo[3,4-*b*]pyridin-5-yl)acetonitrile |
| 291b | 2-((*S*/*R*)-6-acryloyl-3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl) methyl)-6,7-dihydro-5*H*-pyrrolo[3,4-*b*]pyridin-5-yl)acetonitrile |
| 292a | 2-((*S*/*R*)-6-acryloyl-3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl) methyl)-6,7-dihydro-5*H*-pyrrolo[3,4-*b*]pyridin-7-yl)acetonitrile |
| 292b | 2-((*R*/*S*)-6-acryloyl-3-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl) methyl)-6,7-dihydro-5*H*-pyrrolo[3,4-*b*]pyridin-7-yl)acetonitrile |
| 293a | (3a*R*,11a*S*)-5-(((*S*/*R*)-7-acryloyl-6-(trifluoromethyl)-5,6,7,8-tetrahydroimidazo[1,2-a]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 293b | (3a*R*,11a*S*)-5-(((*R*/*S*)-7-acryloyl-6-(trifluoromethyl)-5,6,7,8-tetrahydroimidazo[1,2-a]pyrazin-2-yl)methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 294a | (3aR,11aS)-5-(((*R*/*S*)-5-acryloyl-6-methyl-4,5,6,7-tetrahydropyrazolo[1,5-*a*]pyrazin-2-yl) methyl)-6-chloro-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 294b | (3a*R*,11a*S*)-5-(((*S*/*R*)-5-acryloyl-6-methyl-4,5,6,7-tetrahydropyrazolo[1,5-*a*]pyrazin-2-yl) methyl)-6-chloro-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 295 | (*E*)-*N*-(5-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5H-benzo[*b*]pyrrolo[2,3-*f*][1,4]diazocin-5-yl)-2-fluoropentyl)-4-(dimethylamino)but-2-enamide |
| 296 | (*E*)-*N*-(5-((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-2,2-difluoropentyl)-4-(dimethylamino)but-2-enamide |
| 297 | (3a*R*,11a*S*)-6-chloro-5-((1-(2-((dimethylamino)methyl)acryloyl)azetidin-3-yl)methyl)-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 298 | (3a*R*,11a*S*)-6-chloro-5-(2-(1-(2-((dimethylamino)methyl)acryloyl)azetidin-3-yl)ethyl)-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 299 | (3a*R*,11a*S*)-6-chloro-5-(3-(1-(2-((dimethylamino)methyl)acryloyl)azetidin-3-yl)propyl)-10-methyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 300 | (3a*R*,11a*S*)-6-chloro-5-(4-(1-(2-((dimethylamino)methyl)acryloyl)azetidin-3-yl)-4-fluorobutyl)-10-methyl-1-(6-methyl-4-(trifluoromethyl)pyridin-2-yl)-1,3a,4,5,10,11a-hexahydro-2*H*-benzo [*b*]pyrrolo[2,3-*f*][1,4]diazocine-2,11(3*H*)-dione |
| 301 | (3a*R*,11a*S*)-6-chloro-5-(4-(1-(2-((dimethylamino)methyl)acryloyl)azetidin-3-yl)-3-fluorobutyl)-10-methyl-1-(6-methyl-4-(trifluoromethyl)pyridin-2-yl)-1,3a,4,5,10,11a-hexahydro-2*H*-benzo [*b*]pyrrolo[2,3-*f*][1,4]diazocine-2,11(3*H*)-dione |

(continued)

| Example Number | Name |
|---|---|
| 302 | (3a*R*,11a*S*)-6-chloro-5-(4-(1-(2-((dimethylamino)methyl)acryloyl)azetidin-3-yl)-2-fluorobutyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 303 | (3a*R*,11a*S*)-6-chloro-5-(2-((3-fluoro-1-propioloylazetidin-3-yl)methoxy)ethyl)-10-methyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione |
| 304 | (3a*R*,11a*S*)-6-chloro-5-(2-((1-((*E*)-4-(dimethylamino)but-2-enoyl)-3-fluoroazetidin-3-yl)methoxy)ethyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 305 | (3a*R*,11a*S*)-6-chloro-5-(2-((1-(2-((dimethylamino)methyl)acryloyl)-3-fluoroazetidin-3-yl)methoxy)ethyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 306 | (3a*R*,11a*S*)-6-chloro-5-((7-((*E*)-4,4-difluorobut-2-enoyl)-5,6,7,8-tetrahydroimidazo[1,2-a]pyrazin-2-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 307 | (3a*R*,11a*S*)-6-chloro-10-methyl-1-(6-methyl-4-(trifluoromethyl)pyridin-2-yl)-5-((7-((*E*)-4,4,4-trifluorobut-2-enoyl)-5,6,7,8-tetrahydroimidazo[1,2-*a*]pyrazin-2-yl)methyl)-1,3a,4,5,10,11a-hexahydro-2*H*-benzo[b]pyrrolo[2,3-*f*][1,4]diazocine-2,11(3*H*)-dione |
| 308 | (3a*R*,11a*S*)-6-chloro-5-((4-((*E*)-4-(dimethylamino)but-2-enoyl)-3,4-dihydro-2*H*-benzo[*b*][1,4]oxazin-7-yl)methyl)-10-methyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 309 | (*E*)-*N*-(4-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2,6-difluorophenyl)-4-(dimethylamino)but-2-enamide |
| 310a | 2-((*S*/*R*)-5-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-((*E*)-4-(dimethylamino)but-2-enoyl)isoindolin-1-yl)acetonitrile |
| 310b | 2-((*R*/*S*)-5-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-((*E*)-4-(dimethylamino)but-2-enoyl)isoindolin-1-yl)acetonitrile |
| 311a | 2-((*R*/*S*)-6-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-((*E*)-4-(dimethylamino)but-2-enoyl)isoindolin-1-yl)acetonitrile |
| 311b | 2-((*S*/*R*)-6-(((3a*R*,11a*S*)-6-chloro-10-methyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-((*E*)-4-(dimethylamino)but-2-enoyl)isoindolin-1-yl)acetonitrile |
| 312 | (3a*R*,11a*S*)-5-((6-acryloyl-6,7-dihydro-5*H*-pyrrolo[3,4-*b*]pyridin-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 313 | (3a*R*,11a*S*)-5-((2-acryloylisoindolin-5-yl)methyl)-6,10-dimethyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 314 | (3a*R*,11a*S*)-5-((5-acryloyl-1-ethyl-1,4,5,6-tetrahydropyrrolo[3,4-d]imidazol-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 315 | (3a*R*,11a*S*)-5-((5-acryloyl-5,6-dihydro-4*H*-pyrrolo[3,4-d]thiazol-2-yl)methyl)-6,1 0-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 316 | (3a*R*, 11 aS)-5-((5-acryloyl-5,6-dihydro-4H-pyrrolo[3,4-d]oxazol-2-yl)methyl)-6,1 0-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 317 | (3a*R*,11a*S*)-5-((5-acryloyl-1-methyl-1,4,5,6-tetrahydropyrrolo[3,4-*c*]pyrazol-3-yl)methyl)-6,10-dimethyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione |
| 318a | 2-((*R*/*S*)-6-acryloyl-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitrile |
| 318b | 2-((*S*/*R*)-6-acryloyl-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[b]pyrrolo[2,3-*f*][1,4]diazocin-5-yl)methyl)-6,7-dihydro-5*H*-pyrrolo[3,4-*b*]pyridin-7-yl)acetonitrile |
| 319a | 2-((*S*/*R*)-6-acryloyl-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitrile |
| 319b | 2-((*R*/*S*)-6-acryloyl-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitrile |
| 320a | 2-((*R*/*S*)-6-acryloyl-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitrile |
| 320b | 2-((*S*/*R*)-6-acryloyl-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitrile |
| 321a | 2-((*S*/*R*)-6-acryloyl-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitrile |
| 321b | 2-((*R*/*S*)-6-acryloyl-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitrile |
| 322a | (3a*R*,11a*S*)-5-(((*S*/*R*)-7-acryloyl-6-(trifluoromethyl)-5,6,7,8-tetrahydroimidazo[1,2-a]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 322b | (3a*R*,11a*S*)-5-(((*R*/*S*)-7-acryloyl-6-(trifluoromethyl)-5,6,7,8-tetrahydroimidazo[1,2-a]pyrazin-2-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 323a | (3a*R*,11a*S*)-5-(4-((*R*/*S*)-4-acryloylmorpholin-3-yl)benzyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 323b | (3a*R*,11a*S*)-5-(4-((*S*/*R*)-4-acryloylmorpholin-3-yl)benzyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 324a | (3a*R*,11a*S*)-5-((6-((*R*/*S*)-4-acryloylmorpholin-3-yl)pyridin-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 324b | (3a*R*,11a*S*)-5-((6-((*S*/*R*)-4-acryloylmorpholin-3-yl)pyridin-3-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |

(continued)

| Example Number | Name |
|---|---|
| 325a | (3a*R*,11a*S*)-5-((1-((3*S*/*R*,4*S*/*R*)-1-acryloyl-4-fluoropyrrolidin-3-yl)-1*H*-pyrazol-4-yl)methyl)-6,10-dimethyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione |
| 325b | (3a*R*,11a*S*)-5-((1-((3*R*/*S*,4*R*/*S*)-1-acryloyl-4-fluoropyrrolidin-3-yl)-1*H*-pyrazol-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 325c | (3a*R*,11a*S*)-5-((1-((3*R*/*S*,4*S*/*R*)-1-acryloyl-4-fluoropyrrolidin-3-yl)-1*H*-pyrazol-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 325d | (3a*R*,11a*S*)-5-((1-((3*S*/*R*,4*R*/*S*)-1-acryloyl-4-fluoropyrrolidin-3-yl)-1*H*-pyrazol-4-yl)methyl)-6,10-dimethyl-1-(6-methyl-4-(trifluoromethyl)pyridin-2-yl)-1,3a,4,5,10,11a-hexahydro-2*H*-benzo[*b*]pyrrolo[2,3-*f*][1,4]diazocine-2,11(3*H*)-dione |
| 326a | (3a*R*,11a*S*)-5-((1-((3*S*/*R*,4*R*/*S*)-1-acryloyl-4-fluoropyrrolidin-3-yl)-1*H*-imidazol-4-yl)methyl)-6,10-dimethyl-1-(6-methyl-4-(trifluoromethyl)pyridin-2-yl)-1,3a,4,5,10,11a-hexahydro-2H-benzo[b]pyrrolo[2,3-*f*][1,4]diazocine-2,11(3*H*)-dione |
| 326b | (3a*R*,11a*S*)-5-((1-((3*R*/*S*,4*S*/*R*)-1-acryloyl-4-fluoropyrrolidin-3-yl)-1*H*-imidazol-4-yl)methyl)-6,10-dimethyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 327 | (3a*R*,11a*S*)-5-(3-(1-(2-((dimethylamino)methyl)acryloyl)azetidin-3-yl)propyl)-6,10-dimethyl-1-(6-methyl-4-(trifluoromethyl)pyridin-2-yl)-1,3a,4,5,10,11a-hexahydro-2H-benzo[*b*]pyrrolo[2,3-*f*][1,4]diazocine-2,11(3*H*)-dione |
| 328 | (3a*R*,11a*S*)-5-((2-(2-((dimethylamino)methyl)acryloyl)-2-azaspiro[3.3]heptan-6-yl)methyl)-6,10-dimethyl-1-(6-methyl-4-(trifluoromethyl)pyridin-2-yl)-1,3a,4,5,10,11a-hexahydro-2*H*-benzo[b]pyrrolo[2,3-*f*][1,4]diazocine-2,11(3*H*)-dione |
| 329a | *N*-((*R*/*S*)-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,6,7,8-tetrahydroimidazo[1,2-*a*]pyridin-6-yl)-2-((dimethylamino)methyl)acrylamide |
| 329b | *N*-((*S*/*R*)-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,6,7,8-tetrahydroimidazo[1,2-*a*]pyridin-6-yl)-2-((dimethylamino)methyl)acrylamide |
| 330a | *N*-((*S*/*R*)-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,6,7,8-tetrahydroimidazo[1,2-a]pyridin-7-yl)-2-((dimethylamino)methyl)acrylamide |
| 330b | *N*-((*R*/*S*)-2-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,6,7,8-tetrahydroimidazo[1,2-*a*]pyridin-7-yl)-2-((dimethylamino)methyl)acrylamide |
| 331 | (*E*)-*N*-((1*R*,3*r*)-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)cyclobutyl)-4-(dimethylamino)but-2-enamide |
| 332 | (*E*)-*N*-((1*S*,3*s*)-3-(((3a*R*,11a*S*)-6,10-dimethyl-1-(6-methyl-4-(trifluoromethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)cyclobutyl)-4-(dimethylamino)but-2-enamide |

or a pharmaceutically acceptable salt or solvate thereof.

**13.** A pharmaceutical composition comprising a compound of formula (I) as defined in any of claims 1 to 12, optionally in combination with one or more therapeutic agents.

**14.** A compound as defined in any of claims 1 to 12 for use in:

(a) therapy; or
(b) the prophylaxis or treatment of cancer.

**15.** A process for preparing a compound of formula (I) as defined in claim 1 which comprises:

(a) deprotection of a compound of formula (II);

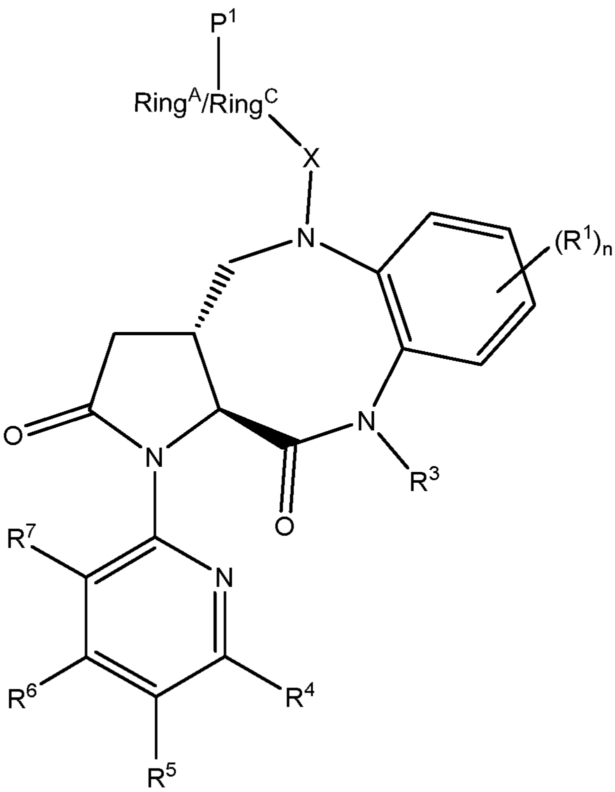

(II)

wherein $R^1$, n, X, $R^3$, $R^4$, $R^5$, $R^6$, $R^7$, $Ring^A$ and $Ring^C$ are as defined in claim 1 and $P^1$ represents a suitable protecting group, such as Boc;
(b) reacting a compound of formula (III):

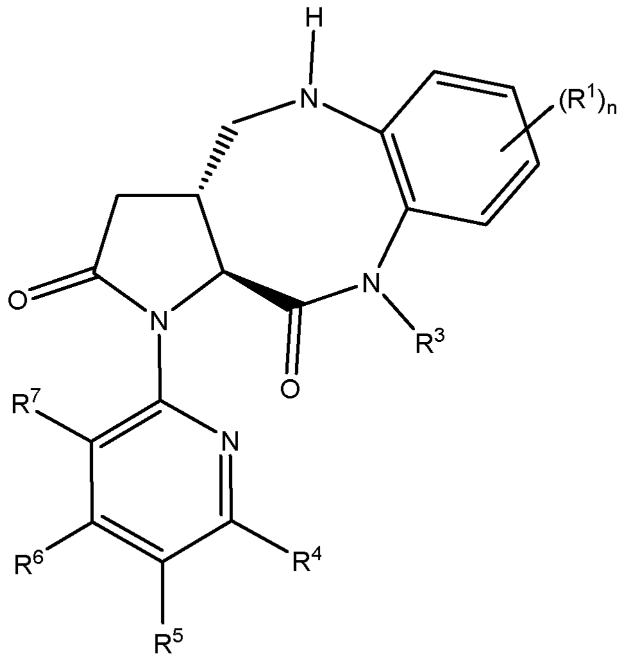

(III)

wherein $R^1$, n, $R^3$, $R^4$, $R^5$, $R^6$ and $R^7$ are as defined in claim 1, with a compound of formula $L^1$-X-$R^2$, wherein X and $R^2$ are as defined in claim 1 and $L^1$ represents a suitable leaving group such as halogen (i.e. chlorine) or a hydroxy group;
(c) deprotection of a protected derivative of a compound of formula (I);
(d) interconversion of a compound of formula (I) or protected derivative thereof to a further compound of formula (I) or protected derivative thereof; and
(e) optional formation of a pharmaceutically acceptable salt of a compound of formula (I).

**Patentansprüche**

**1.** Verbindung der Formel (I):

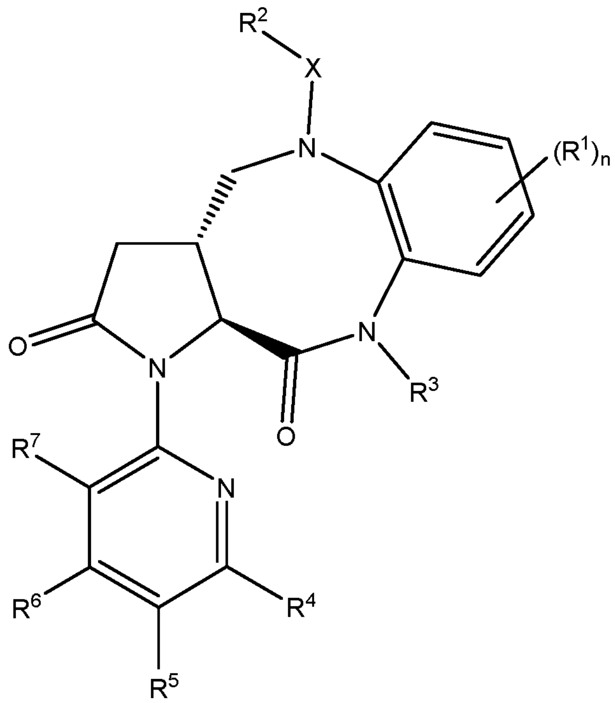

(I)

oder eine tautomere oder stereochemisch isomere Form, ein pharmazeutisch verträgliches Salz oder ein Solvat davon, wobei:

n für eine ganze Zahl steht, ausgewählt aus 0, 1, 2, 3 oder 4;
$R^1$ für $C_{1-6}$-Alkyl, $C_{2-6}$-Alkenyl, $C_{1-6}$-Alkoxy, Hydroxy, Halogen, Halogen-$C_{1-6}$-alkyl, Halogen-$C_{1-6}$-alkoxy, $C_{3-8}$-Cycloalkyl, Cyano oder $-NR^xR^y$ steht;
X für eine Bindung oder eine $C_1$-$C_6$-Alkylengruppe steht, die optional substituiert ist durch eine oder mehrere $C_{1-6}$-Alkyl-, $NR^x$-, O-, Hydroxy-, Halogen- oder CO-Gruppen;
$R^2$ für eine -Ring$^A$- oder eine -Ring$^B$-Y-Ring$^C$-Gruppe steht;
oder $-X-R^2$ für eine $-C_1-C_{12}$-Alkylen-$NR^xR^y$-Gruppe steht, wobei die Alkylengruppe optional substituiert sein kann durch eine oder mehrere $C_{1-6}$-Alkyl-, $C_{2-6}$-Alkenyl-, $-NR^x$-, O-, Hydroxy- oder CO-Gruppen;
Ring$^A$ für Carbocyclyl, Heterocyclyl oder Heteroaryl steht, das einen Substituenten erfordert ausgewählt aus einer $-(CH_2)_m$-CO-$C_{1-6}$-Alkyl-, $-(CH_2)_m$-NHCO-$C_{1-6}$-Alkyl-, $-(CH_2)_m$-CO-$C_{2-6}$-Alkenyl-, - $(CH_2)_m$-NHCO-$C_{2-6}$-Alkenyl-, $-(CH_2)_m$-CO-$C_{2-6}$-Alkinyl- oder $-(CH_2)_m$-NHCO-$C_{2-6}$-Alkinylgruppe, wobei die Alkyl-, Alkenyl- oder Alkinylgruppe optional substituiert sein kann durch eine oder mehrere (z. B. 1, 2 oder 3) Halogen-, Hydroxy-, CO- oder $-NR^xR^y$-Gruppen und wobei die Carbocyclyl-, Heterocyclyl- oder Heteroarylgruppe optional ferner substituiert sein kann durch einen oder mehrere (z. B. 1, 2 oder 3) Substituenten ausgewählt aus Halogen, $C_{1-6}$-Alkyl, Hydroxy, $C_{1-6}$-Alkoxy, -CO-$C_{1-6}$-Alkyl, Oxo, $C_{1-6}$-Alkylamino oder Cyano, wobei die $C_{1-6}$-Alkylgruppe optional substituiert sein kann durch eine oder mehrere Halogen-, Hydroxy- oder Cyanogruppen;
m für eine ganze Zahl steht, ausgewählt aus 0, 1, 2, 3 oder 4;
Ring$^B$ für Carbocyclyl, Heterocyclyl oder Heteroaryl steht, das jeweils optional substituiert sein kann durch einen oder mehrere (z. B. 1, 2 oder 3) Substituenten ausgewählt aus Hydroxy, Oxo, $C_{1-6}$-Alkyl, $C_{2-6}$-Alkenyl, $C_{2-6}$-Alkinyl, $C_{1-6}$-Alkoxy, Halogen-$C_{1-6}$-alkyl, -CO-$C_{1-6}$-Alkyl, Cyano oder Halogen;
Ring$^C$ für Heterocyclyl steht, das substituiert ist durch einen oder mehrere (z. B. 1, 2 oder 3) Substituenten ausgewählt aus Halogen, $C_{1-6}$-Alkyl, -CO-$C_{1-6}$-Alkyl und/oder $-(CH_2)_m$-CO-$C_{2-6}$-Alkenyl optional substituiert durch eine $-NR^xR^y$-Gruppe;
Y für eine Bindung, -O-, -NHCO-, CO oder eine $C_1$-$C_6$-Alkylengruppe steht optional substituiert durch eine oder mehrere $C_{1-6}$-Alkyl-, $C_{2-6}$-Alkenyl-, $-NR^x$-, O-, Hydroxy- oder CO-Gruppen;
$R^3$ für Wasserstoff oder $C_{1-6}$-Alkyl steht;
$R^4$, $R^5$, $R^6$ und $R^7$ unabhängig für Wasserstoff, $C_{1-6}$-Alkyl, $C_{2-6}$-Alkenyl, $C_{1-6}$-Alkoxy, Halogen, Halogen-$C_{1-6}$-Alkyl, Halogen-$C_{1-6}$-alkoxy, $C_{3-8}$-Cycloalkyl, Cyano oder $-NR^xR^y$ stehen; und
$R^x$ und $R^y$ unabhängig für Wasserstoff, $C_{1-6}$-Alkyl, $C_{2-6}$-Alkenyl, $C_{2-6}$-Alkinyl, $C_{1-6}$-Alkoxy stehen oder $R^x$ und $R^y$ sich zusammen mit dem Stickstoffatom, an das sie gebunden sind, verbinden, um einen stickstoffhaltigen

heterocyclischen Ring zu bilden, der optional substituiert sein kann durch eine oder mehrere $C_{1-6}$-Alkylgruppen.

2. Verbindung nach Anspruch 1, wobei n für 0, 1, 2 oder 3 steht.

3. Verbindung nach Anspruch 1 oder Anspruch 2, wobei $R^1$ für $C_{1-6}$-Alkyl (wie etwa Methyl), Halogen (wie etwa Fluor oder Chlor) oder $C_{3-8}$-Cycloalkyl (wie etwa Cyclopropyl) steht, wie etwa wobei $R^1$ für $C_{1-6}$-Alkyl (wie etwa Methyl) oder Halogen (wie etwa Fluor oder Chlor) steht, insbesondere wobei $R^1$ für $C_{1-6}$-Alkyl (wie etwa Methyl) steht.

4. Verbindung nach einem der Ansprüche 1 bis 3, wobei $R^2$ für einen -Ring$^A$ steht, wie etwa wobei Ring$^A$ für Carbocyclyl (wie etwa Phenyl, Cyclobutyl, 2,3-Dihydro-1H-inden-2-yl oder Bicyclo[1.1.1]pentan-1-yl), einen Heterocyclylring (wie etwa Azetidinyl, Pyrrolidinyl, Piperidinyl, Piperazinyl, Isoindolin-1-yl, Isoindolin-5-yl, Tetrahydroisoquinolin-6-yl, Diazaspiro[3.4]octan-6-yl, Octahydro-2*H*-pyrazino[1,2-a]pyrazin-2-yl, Octahydro-5*H*-pyrrolo[3,4-c]pyridin-5-yl, 5-Oxa-2,8-diazaspiro[3.5]nonan-8-yl, 2,6-diazaspiro[3.4]octan, 9-Oxa-3,7-diazabicyclo[3.3.1]nonan-3-yl, Tetrahydro-1H-imidazo[4,5-c]pyridin-1-yl, Tetrahydro-1'H-spiro[azetidin-3,4'-pyrrolo[1,2-a]pyrazin]-2' (3'H)-yl, Tetrahydro-1'*H*-spiro[azetidin-3,6'-pyrazino[2,1-*c*][1,4]oxazin]-8'(7'*H*)-yl, Tetrahydro-3H-imidazo[4,5-c]pyridin-3-yl, Tetrahydro-[1,2,4]triazolo[1,5-a]pyrazin-2-yl, Tetrahydroimidazo[1,2-a]pyrazin-2-yl, Tetrahydropyrazolo[1,5-a]pyrazin-2-yl, Octahydro-2H-pyrazino[1,2-a]pyrazin-3-yl, Tetrahydro-[1,2,4]triazolo[4,3-a]pyrazin-3-yl, 2,5,8-Triazaspiro[3.5]nonan-8-yl, spiro[Azetidin-3,3'-imidazo[1,2-a]imidazol]-1'(2'H)-yl, Dihydro-5H-pyrrolo[3,4-b]pyrazin-2-yl, Dihydro-5H-pyrrolo[3,4-b]pyridin-3-yl, Dihydro-5H-pyrrolo[3,4-b]pyridin-2-yl, Dihydro-5H-pyrrolo[3,4-b]pyridin-7-yl, Dihydro-5H-pyrrolo[3,4-b]pyridin-5-yl, Tetrahydropyrrolo[3,4-d]imidazol-2-yl, Dihydro-2H-benzo[b] [1,4]oxazin-6-yl, Dihydro-2H-benzo[b][1,4]oxazin-7-yl, Dihydro-4H-pyrrolo[3,4-d]imidazol-1-yl, Diazabicyclo[3.1.1]heptan-6-yl, Diazabicyclo[3.2.1]octan-8-yl, Diazabicyclo[2.2.1]heptan-2-yl, Dihydro-1H-pyrrolo[3,4-c]pyridin-6-yl, Dihydro-5H-pyrrolo [3,4-d]pyrimidin-2-yl, Dihydro-4H-pyrrolo[3,4-d]thiazol-2-yl, Dihydro-4H-pyrrolo[3,4-d]oxazo-2-yl, Tetrahydropyrrolo [3,4-c]pyrazol-3-yl, Diazaspiro[3.3]heptan-6-yl, Tetrahydroimidazo[1,2-a]pyridin-6-yl, Tetrahydroimidazo[1,2-a]pyridin-7-yl oder Spiro[azetidin-3,5'-imidazo[1,2-a]pyrazin]-7'(8'H)-yl) oder einen Heteroarylring (wie etwa Pyridyl oder pyrimidinyl) steht, jeweils substituiert durch ein(e)

$-(CH_2)_m-CO-C_{2-6}$-Alkyl optional substituiert durch eine Halogengruppe (wie etwa $-CO-CH_2-Cl$);
$-(CH_2)_m-CO-C_{2-6}$-Alkenyl optional substituiert durch eine oder mehrere Halogen-, Hydroxy-, CO- oder $-NR^xR^y$-Gruppen (wie etwa $-COCH=CH_2$, $-CO-C(=CH_2)-Me$, $-CO-CH=CH-CH_2-N(Me)_2$, $-CO-C(=CH_2)-CH_2-N(Me)_2$, $-CO-C(=CH-Me)-CH_2-N(Me)_2$, $-CO-C (=CH_2)-CH_2-N(Me)_2$, $-CO-C(Cl)=CH-CH_2-N(Me)_2$, $-CO-C (=CH_2)-CH_2-OH$, $-CO-C (=CH_2) - CH_2-O-C(=O)-Me$, $-CO-CH=CH-CHF_2$, $-CO-C(=CH_2)-CH_2-NEt_2$);
$-(CH_2)_m-NHCO-C_{2-6}$-Alkenyl optional substituiert durch eine - $NR^xR^y$-Gruppe (wie etwa $-NH-CO-CH=CH_2$, $-NH-CO-C(=CH_2)-CH_2-N(Me)_2$, $-NH-CO-C (=CH-Me)-CH_2-N(Me)_2$, $-NH-CO-CH=CH-CH_2-N(Me)_2$, $-CH_2-NH-CO-CH=CH_2-$, $-CH_2-NH-CO-CH=CH-CH_2-N (Me)_2$, $-CH_2-NH-CO-C(=CH_2)-CH_2-N (Me) 2)$ ;
$-(CH_2)_m-CO-C_{2-6}$-Alkinyl (wie etwa -CO-Ethinyl oder -CO-Ethinyl-Me); oder
$-(CH_2)_m-NHCO-C_{2-6}$-Alkinylgruppe (wie etwa -NH-CO-Ethinyl),
wobei die Carbocyclyl-, Heterocyclyl- oder Heteroarylgruppe optional substituiert sein kann durch einen oder mehrere (z. B. 1, 2 oder 3) weitere(n) Substituenten ausgewählt aus: Halogen (wie etwa Fluor); $C_{1-6}$-Alkyl (wie etwa Methyl oder Ethyl) optional substituiert durch eine oder mehrere Halogen- (wie etwa $-CF_3$ oder $-CHF_2$), Hydroxy- (wie etwa $-CH_2-OH$) oder Cyanogruppen (wie etwa $-CH_2-CN$); Hydroxy; $C_{1-6}$-Alkoxy (wie etwa Methoxy und $-CH_2-OMe$); $-CO-C_{1-6}$-Alkyl (wie etwa -CO-Me); Oxo; $C_{1-6}$-Alkylamino (wie etwa $-N(Me)-(CH_2)_2-N(Me)_2$) oder Cyano),
Ring$^A$ insbesondere für einen Heterocyclylring (wie etwa Azetidinyl, Pyrrolidinyl, Piperidinyl, Piperazinyl, Isoindolin-1-yl, Isoindolin-5-yl, Tetrahydroisoquinolin-6-yl, Diazaspiro[3.4]octan-6-yl, Octahydro-2*H*-pyrazino[1,2-a]pyrazin-2-yl, Octahydro-5*H*-pyrrolo[3,4-*c*]pyridin-5-yl, 5-Oxa-2,8-diazaspiro[3.5]nonan-8-yl, 2,6-Diazaspiro [3.4]octan, 9-Oxa-3,7-diazabicyclo[3.3.1]nonan-3-yl, Tetrahydro-1H-imidazo[4,5-c]pyridin-1-yl, Tetrahydro-1'H-spiro[azetidin-3,4'-pyrrolo[1,2-a]pyrazin]-2'(3'*H*)-yl, Tetrahydro-1'*H*-spiro[azetidin-3,6'-pyrazino[2,1-*c*] [1,4]oxazin]-8'(7'*H*)-yl, Tetrahydro-3H-imidazo[4,5-c]pyridin-3-yl, Tetrahydro-[1,2,4]triazolo[1,5-a]pyrazin-2-yl, Tetrahydroimidazo[1,2-a]pyrazin-2-yl, Tetrahydropyrazolo[1,5-a]pyrazin-2-yl, Octahydro-2H-pyrazino[1,2-a]pyrazin-3-yl, Tetrahydro-[1,2,4]triazolo[4,3-a]pyrazin-3-yl, 2,5,8-Triazaspiro[3.5]nonan-8-yl, spiro[Azetidin-3,3'-imidazo[1,2-a]imidazol]-1'(2'H)-yl, Dihydro-5H-pyrrolo[3,4-b]pyrazin-2-yl, Dihydro-5H-pyrrolo[3,4-b]pyridin-3-yl, Dihydro-5H-pyrrolo[3,4-b]pyridin-2-yl, Dihydro-5H-pyrrolo[3,4-b]pyridin-7-yl, Dihydro-5H-pyrrolo[3,4-b]pyridin-5-yl, Tetrahydropyrrolo[3,4-d]imidazol-2-yl, Dihydro-2H-benzo[b][1,4]oxazin-6-yl, Dihydro-2H-benzo[b][1,4] oxazin-7-yl, Dihydro-4H-pyrrolo[3,4-d]imidazol-1-yl, Diazabicyclo[3.1.1]heptan-6-yl, Diazabicyclo[3.2.1]octan-8-yl, Diazabicyclo[2.2.1]heptan-2-yl, Dihydro-1H-pyrrolo[3,4-c]pyridin-6-yl, Dihydro-5H-pyrrolo[3,4-d]pyrimidin-2-yl, Dihydro-4H-pyrrolo[3,4-d]thiazol-2-yl, Dihydro-4H-pyrrolo[3,4-d]oxazo-2-yl, Tetrahydropyrrolo[3,4-c]pyrazol-3-yl, Diazaspiro[3.3]heptan-6-yl, Tetrahydroimidazo[1,2-a]pyridin-6-yl, Tetrahydroimidazo[1,2-a]pyridin-7-yl oder Spiro[azetidin-3,5'-imidazo[1,2-a]pyrazin]-7'(8'H)-yl) steht, jeweils substituiert durch ein(e)

$-(CH_2)_m-CO-C_{2-6}$-Alkyl optional substituiert durch eine Halogengruppe (wie etwa $-CO-CH_2-Cl$);
$-(CH_2)_m-CO-C_{2-6}$-Alkenyl optional substituiert durch eine oder mehrere Halogen-, Hydroxy-, CO- oder $-NR^xR^y$-Gruppen (wie etwa $-COCH=CH_2$, -CO-C $(=CH_2)$-Me, $-CO-CH=CH-CH_2-N(Me)_2$, -CO-$C(=CH_2)-CH_2-N(Me)_2$, $-CO-C(=CH-Me)-CH_2-N(Me)_2$, $-CO-C(=CH_2)-CH_2-N(Me)_2$, -CO-C (Cl)=CH-$CH_2-N(Me)_2$, -CO-C $(=CH_2)$ $-CH_2-OH$, -CO-C $(=CH_2)$ - $CH_2-O-C(=O)-Me)$, $-CO-CH=CH-CHF_2$, -CO-C $(=CH_2)-CH_2-NEt_2$;
$-(CH_2)_m-NHCO-C_{2-6}$-Alkenyl optional substituiert durch eine - $NR^xR^y$-Gruppe (wie etwa $-NH-CO-CH=CH_2$, $-NH-CO-C(=CH_2)-CH_2-N(Me)_2$, -NH-CO-C $(=CH-Me)-CH_2-N(Me)_2$, $-NH-CO-CH=CH-CH_2-N$ $(Me)_2$, $-CH_2-NH-CO-CH=CH_2$-, $-CH_2-NH-CO-CH=CH-CH_2-N$ $(Me)_2$, $-CH_2-NH-CO-C(=CH_2)-CH_2-N$ (Me) 2) ;
- $(CH_2)_m-CO-C_{2-6}$-Alkinyl (wie etwa -CO-Ethinyl oder -CO-Ethinyl-Me); oder
- $(CH_2)_m-NHCO-C_{2-6}$-Alkinylgruppe (wie etwa -NH-CO-Ethinyl),
wobei die Heterocyclyl- oder Heteroarylgruppe optional substituiert sein kann durch einen oder mehrere (z. B. 1, 2 oder 3) weitere(n) Substituenten ausgewählt aus: Halogen (wie etwa Fluor); $C_{1-6}$-Alkyl (wie etwa Methyl oder Ethyl) optional substituiert durch eine oder mehrere Halogen- (wie etwa $-CF_3$ oder $-CHF_2$), Hydroxy- (wie etwa $-CH_2-OH$) oder Cyanogruppen (wie etwa $-CH_2-CN$), Hydroxy, $C_{1-6}$-Alkoxy (wie etwa Methoxy und $-CH_2-OMe$), $-CO-C_{1-6}$-Alkyl (wie etwa -CO-Me), Oxo, $C_{1-6}$-Alkylamino (wie etwa $-N(Me)-(CH_2)_2-N(Me)_2$) oder Cyano),

$Ring^A$ konkreter für Dihydro-5H-pyrrolo[3,4-b]pyridinyl steht, wie etwa Dihydro-5H-pyrrolo[3,4-b]pyridin-3-yl, Dihydro-5H-pyrrolo[3,4-b]pyridin-2-yl, Dihydro-5H-pyrrolo[3,4-b]pyridin-7-yl oder Dihydro-5H-pyrrolo[3,4-b]pyridin-5-yl, jeweils substituiert durch ein

- $(CH_2)_m-CO-C_{2-6}$-Alkenyl optional substituiert durch eine oder mehrere Halogen-, Hydroxy-, CO- oder $-NR^xR^y$-Gruppen (wie etwa $-COCH=CH_2$, -CO-C $(=CH_2)$-Me, $-CO-CH=CH-CH_2-N(Me)_2$, -CO-C $(=CH_2)-CH_2-N(Me)_2$, -CO-C $(=CH-Me)-CH_2-N(Me)_2$, -CO-C $(=CH_2)-CH_2-N(Me)_2$, -CO-C (Cl)=CH-$CH_2-N(Me)_2$, -CO-C $(=CH_2)$ $-CH_2-OH$, -CO-C $(=CH_2)$ - $CH_2-O-C(=O)-Me)$, $-CO-CH=CH-CHF_2$, -CO-C $(=CH_2)-CH_2-NEt_2$;
wobei die Heterocyclylgruppe optional substituiert sein kann durch einen oder mehrere (z. B. 1, 2 oder 3) weitere(n) Substituenten ausgewählt aus: Halogen (wie etwa Fluor); $C_{1-6}$-Alkyl (wie etwa Methyl oder Ethyl) optional substituiert durch eine oder mehrere Halogen- (wie etwa $-CF_3$ oder $-CHF_2$), Hydroxy-(wie etwa $-CH_2-OH$) oder Cyanogruppen (wie etwa $-CH_2-CN$), Hydroxy, $C_{1-6}$-Alkoxy (wie etwa Methoxy und $-CH_2-O-Me$), $-CO-C_{1-6}$-Alkyl (wie etwa -CO-Me), Oxo, $C_{1-6}$-Alkylamino (wie etwa $-N(Me)-(CH_2)_2-N(Me)_2$) oder Cyano),

Ring $^A$ insbesondere für Dihydro-5H-pyrrolo[3,4-b]pyridinyl, wie etwa Dihydro-5H-pyrrolo[3,4-b]pyridin-3-yl, Dihydro-5H-pyrrolo[3,4-b]pyridin-2-yl, Dihydro-5H-pyrrolo[3,4-b]pyridin-7-yl oder Dihydro-5H-pyrrolo[3,4-b]pyridin-5-yl steht, jeweils substituiert durch ein $-COCH=CH_2$,
$Ring^A$ konkreter für Dihydro-5H-pyrrolo[3,4-b]pyridin-2-yl steht substituiert durch ein $-COCH=CH_2$,
$Ring^A$ konkreter für 6,7-Dihydro-5H-pyrrolo[3,4-b]pyridin-2-yl steht substituiert durch ein $-COCH=CH_2$.

**5.** Verbindung nach einem der Ansprüche 1 bis 3, wobei $R^2$ für einen $-Ring^B-Y-Ring^C$ steht, wie etwa wobei $Ring^B$ für Carbocyclyl (wie etwa Cyclohexyl oder Phenyl), Heterocyclyl (wie etwa Azetidinyl, Pyrrolidinyl, Piperidinyl, Piperazinyl, Diazepanyl oder Hexahydropyrrolo[3,4-b] [1,4]oxazin-6(2H)-yl) oder Heteroaryl (wie etwa Imidazolyl, Pyrazolyl, Triazolyl, Pyridinyl, Pyrimidinyl, Oxazolyl oder Oxadiazolyl) steht, die jeweils optional substituiert sein können durch einen oder mehrere (z. B. 1, 2 oder 3) Substituenten ausgewählt aus Hydroxy, Oxo, $C_{1-6}$-Alkyl (wie etwa Methyl), Halogen-$C_{1-6}$-alkyl (wie etwa $-CH_2-F$), Cyano oder Halogen (wie etwa Fluor).

**6.** Verbindung nach einem der Ansprüche 1 bis 5, wobei Y für eine Bindung, -O-, -NHCO-, CO- oder eine $C_1-C_6$-Alkylengruppe steht optional substituiert durch eine oder mehrere $C_{1-6}$-Alkyl-, $C_{2-6}$-Alkenyl-, $-NR^x$-, O-, Hydroxy- oder CO-Gruppen (wie etwa $-CO-C(=CH_2)-CH_2$- oder $-NHCO-CH=CH-CH_2$-), wie etwa wobei Y für eine Bindung steht, alternativ wobei Y für -O- steht.

**7.** Verbindung nach einem der Ansprüche 1 bis 6, wobei $Ring^A$ für einen monocyclischen Heterocyclylring, der mindestens ein Stickstoffatom aufweist, steht. In einer weiteren Ausführungsform steht $Ring^A$ für Azetidinyl, Pyrrolidinyl, Piperazinyl oder Tetrahydropyridinyl, die jeweils substituiert sind durch ein Halogen (wie etwa Fluor), $C_{1-6}$-Alkyl (wie etwa Methyl), $-CO-C_{1-6}$-Alkyl (wie etwa -CO-Methyl) und/oder ein - $(CH_2)_m-CO-C_{2-6}$-Alkenyl optional

substituiert durch eine -$NR^xR^y$-Gruppe (wie etwa -$COCH=CH_2$, -CO-C (=$CH_2$)-$CH_2$-N(Me)$_2$, -CO-C (=CH-Me)-$CH_2$-N(Me)$_2$ oder -CO-CH=CH-$CH_2$-N(Me)$_2$).

**8.** Verbindung nach einem der Ansprüche 1 bis 7, wobei X für Folgendes steht:

(a) eine Bindung oder eine $C_1$-$C_4$-Alkylengruppe optional substituiert durch eine oder mehrere $C_{1-6}$-Alkyl- (wie etwa Methyl), -$NR^x$- (wie etwa -NH oder -N-Methyl), O-, Hydroxy-, Halogen (wie etwa Fluor) oder CO-Gruppen; oder
(b) -$CH_2$-; oder
(c) eine $C_1$-$C_4$-Alkylengruppe (wie etwa -$CH_2$-, - ($CH_2$) $_2$-, - ($CH_2$) $_3$- oder -($CH_2$)$_4$-) optional substituiert durch eine oder mehrere $C_{1-6}$-Alkyl- (wie etwa - ($CH_2$) -C(Me-)), O- (wie etwa - ($CH_2$) $_2$-O-, - ($CH_2$) $_3$-O- oder -($CH_2$)$_2$-O-$CH_2$-) oder CO- (wie etwa -($CH_2$)-CO- oder - ($CH_2$) $_2$-CO-)-$NR^x$- (wie etwa - ($CH_2$) $_2$-NH-, - ($CH_2$) $_3$-NH-, - ($CH_2$) $_2$-N (Me) - oder - ($CH_2$) $_3$-N(Me)-)-Gruppen oder eine CO- und -$NR^x$-Gruppe (wie etwa - ($CH_2$) $_2$-N(Me) -CO-) oder eine Hydroxy- und -$NR^x$-Gruppe (wie etwa - ($CH_2$) -CH (OH) -$CH_2$-N(Me) -) oder Halogen (wie etwa - ($CH_2$)$_3$-CHF-, - ($CH_2$) $_2$-CHF-$CH_2$- oder -$CH_2$-CHF- ($CH_2$) $_2$-); oder
(d) eine Bindung, -$CH_2$-, -($CH_2$)$_2$-, -($CH_2$)$_3$-, -($CH_2$)$_4$-, -($CH_2$)-C(Me)-, -($CH_2$)$_2$-O-, -($CH_2$)$_3$-O-, -($CH_2$)$_2$-O-$CH_2$-, -($CH_2$)-CO-, - ($CH_2$) 2-CO-, - ($CH_2$) 2-NH-, - ($CH_2$) 3-NH-, - ($CH_2$) 2-N (Me)-, - ($CH_2$) 3-N (Me) -, -($CH_2$)$_2$-N(Me)-CO- - ($CH_2$) -CH(OH)-$CH_2$-N(Me)-($CH_2$)$_3$-CHF-, - ($CH_2$)$_2$-CHF-$CH_2$- oder -$CH_2$-CHF-($CH_2$)$_2$-.

**9.** Verbindung nach einem der Ansprüche 1 bis 8, wobei -X-$R^2$ für eine -$C_1$-$C_{10}$-Alkylen-$NR^xR^y$-Gruppe steht, wobei die Alkylengruppe optional substituiert sein kann durch eine oder mehrere $C_{2-6}$-Alkenyl-, $NR^x$-, O oder CO-Gruppen, wie etwa - ($CH_2$) $_2$-O- ($CH_2$) $_2$-NH-CO-C (=$CH_2$)-$CH_2$-N(Me)$_2$, - ($CH_2$) $_5$-NH-CO-CH=CH-$CH_2$-N (Me)$_2$, - ($CH_2$)$_5$-NH-CO-C(=$CH_2$)-$CH_2$-N(Me)$_2$, - ($CH_2$)$_4$-NHCOCH=CH-$CH_2$-$NMe_2$ oder -$CH_2$-CHF- ($CH_2$) $_3$-NHCO-CH=$CHCH_2NMe_2$, - ($CH_2$) $_3$-CHF-$CH_2$NHCO-CH=$CHCH_2NMe_2$ oder - ($CH_2$) $_3$-$CF_2$-$CH_2$NHCO-CH=$CHCH_2NMe_2$.

**10.** Verbindung nach einem der Ansprüche 1 bis 9, wobei:

(a) $R^3$ für Wasserstoff oder $C_{1-6}$-Alkyl steht (wie etwa Methyl oder Ethyl), wie etwa wobei $R^3$ für Wasserstoff oder $C_{1-6}$-Alkyl steht (wie etwa Methyl), insbesondere wobei $R^3$ für $C_{1-6}$-Alkyl steht (wie etwa Methyl), insbesondere wobei $R^3$ für Methyl steht; und/oder
(b) $R^4$ für Folgendes steht:

Wasserstoff;
$C_{1-6}$-Alkyl (wie etwa Methyl oder Ethyl);
$C_{2-6}$-Alkenyl (wie etwa Ethenyl); C $_{1-6}$-Alkoxy (wie etwa Methoxy);
Halogen (wie etwa Chlor); oder
-$NR^xR^y$ (wie etwa -N(Me)$_2$ oder -N(Me)(Et)),

wie etwa wobei $R^4$ für $C_{1-6}$-Alkyl steht (wie etwa Methyl), insbesondere wobei $R^4$ für Methyl steht; und/oder
(c) $R^5$ für Folgendes steht:

Wasserstoff;
Halogen (wie etwa Chlor); oder
$C_{1-6}$-Alkyl (wie etwa Methyl);

wie etwa wobei $R^5$ für Wasserstoff steht; und/oder
(d) $R^6$ für Folgendes steht:

$C_{1-6}$-Alkyl (wie etwa Methyl, Ethyl oder Isopropyl);
$C_{2-6}$-Alkenyl (wie etwa -C(=$CH_2$)(Me));
Halogen (wie etwa Brom);
Halogen-$C_{1-6}$-Alkyl (wie etwa Trifluormethyl oder -C(H)(Me)-$CF_3$) ; oder
Halogen-$C_{-1-6}$-Alkoxy (wie etwa Difluormethoxy),

wie etwa wobei $R^6$ für Halogen-$C_{1-6}$ Alkyl steht (wie etwa Trifluormethyl), insbesondere wobei $R^6$ für Trifluormethyl steht; und/oder
(e) $R^7$ für Wasserstoff oder Cyano steht, wie etwa Wasserstoff; und/oder
(f) $R^x$ und $R^y$ unabhängig für Wasserstoff oder $C_{1-6}$-Alkyl stehen (wie etwa Methyl oder Ethyl), wie etwa wobei $R^x$

und $R^y$ unabhängig für $C_{1-6}$-Alkyl stehen (wie etwa Methyl), insbesondere wobei sowohl $R^x$ als auch $R^y$ für $C_{1-6}$-Alkyl stehen (wie etwa Methyl) .

**11.** Verbindung nach Anspruch 1, die eine Verbindung der Formel $(I)^a$ ist:

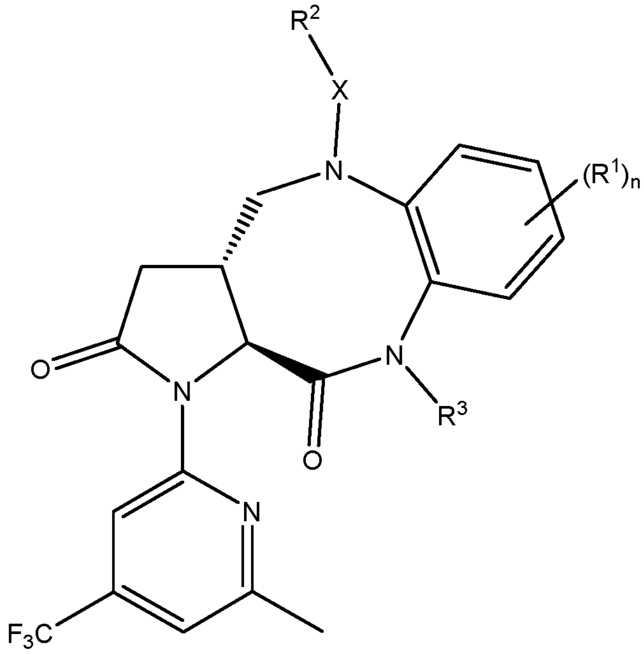

$(I)^a$

oder eine tautomere oder eine stereochemisch isomere Form, ein pharmazeutisch verträgliches Salz oder ein Solvat davon, wobei n, $R^1$, X, $R^2$ und $R^3$ wie in Anspruch 1 definiert sind.

**12.** Verbindung nach Anspruch 1, wobei die Verbindung die freie Base einer Verbindung der Beispiele 1-332 ist:

| Beispieln ummer | Bezeichnung |
|---|---|
| 1 | (3a*R*,11a*S*)-5-(2-(4-(1-Acryloylazetidin-3-yl)piperazin-1-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo [2,3-*f*][1,4]diazocin-2,11(3*H*)-dion |
| 2 | (3a*R*,11a*S*)-5-(2-((*R*)-1-Acryloyltetrahydro-1'H-spiro[azetidin-3,4'-pyrrolo[1,2-a]pyrazin]-2'(3'*H*)-yl)ethyl)-6-fluor-10-methyl-1-(6-methyl-4-(trifluormethyl)pyridin-2-yl)-1,3a,4,5,10,11a-hexahydro-2H-benzo[*b*]pyrrolo[2,3-*f*][1,4]diazocin-2,11(3*H*)-dion |
| 3 | (3a*R*,11a*S*)-5-(2-(4-(1-Acryloylazetidin-3-yl)-1,4-diazepan-1-yl)ethyl)-6-fluor-10-methyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 4 | (3a*R*,11a*S*)-5-(2-(2-Acryloyl-5-oxa-2,8-diazaspiro[3.5]nonan-8-yl)ethyl)-6-fluor-10-methyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 5 | (3a*R*,11a*S*)-5-(2-(7-Acryloyl-9-oxa-3,7-diazabicyclo[3.3.1]nonan-3-yl)ethyl)-6-fluor-10-methyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 6 | (3a*R*,11a*S*)-5-(2-((*S*)-2-Acryloyl-6-(methoxymethyl)-2,5,8-triazaspiro[3.5]nonan-8-yl) ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-f][1,4]diazocin-2,11(3*H*)-dion |

(continued)

| Beispieln ummer | Bezeichnung |
|---|---|
| 7 | (3a*R*,11a*S*)-5-(2-((7'*R*/*S*,9a'*S*)-1-Acryloyl-7'-methyltetrahydro-1'H-spiro[azetidin-3,6'-pyrazino[2,1-*c*][1,4]oxazin]-8'(7'*H*)-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 8 | (3a*R*,11a*S*)-5-(2-((7'*S*/*R*,9a'S)-1-Acryloyl-7'-methyltetrahydro-1'*H*-spiro[azetidin-3,6'-pyrazino[2,1-*c*][1,4]oxazin]-8'(7'*H*)-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 9 | (3a*R*,11a*S*)-5-(2-((7S,9a*S*/*R*)-8-Acryloyl-7-methyloctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 10 | (3a*R*,11a*S*)-5-(2-((7*S*,9a*R*/*S*)-8-Acryloyl-7-methyloctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 11 | (3a*R*,11a*S*)-5-(2-((*rel-trans*)-3-((1-Acryloylazetidin-3-yl)oxy)-4-hydroxypyrrolidin-1-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 12 | (3a*R*,11a*S*)-5-(2-(4-(1-Acryloylazetidin-3-yl)piperazin-1-yl)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-f][1,4]diazocin-2,11(3*H*)-dion |
| 13 | (3a*R*,11a*S*)-5-(2-((*R*)-2-Acryloyl-6-methyl-2,5,8-triazaspiro[3.5]nonan-8-yl)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 14 | (3a*R*,11a*S*)-5-(2-(8-Acryloyloctahydro-2*H*-pyrazino[1,2-*a*]pyrazin-2-yl)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 15 | (3a*R*,11a*S*)-5-(2-(4-(1-Acryloylazetidin-3-yl)piperazin-1-yl)ethyl)-8-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 16 | (3a*R*,11a*S*)-5-(2-(4-(1-Acryloylazetidin-3-yl)piperazin-1-yl)ethyl)-6,8-difluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 17 | (3a*R*,11a*S*)-5-(2-(4-(1-Acryloylazetidin-3-yl)piperazin-1-yl)ethyl)-6-chlor-8-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 18 | (3a*R*,11a*S*)-5-(2-(4-(1-Acryloylazetidin-3-yl)piperazin-1-yl)ethyl)-8-fluor-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3H)-dion |
| 19 | (3a*R*,11a*S*)-5-(2-(4-(1-Acryloylazetidin-3-yl)piperazin-1-yl)ethyl)-6-cyclopropyl-8-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 20 | (3a*R*,11a*S*)-5-(2-(4-(1-Acryloylazetidin-3-yl)piperazin-1-yl)ethyl)-6-chlor-8,9-difluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 21a | (3a*R*,11a*S*)-5-(2-((*S*/*R*)-1-Acryloyl-1,6-diazaspiro[3.4]octan-6-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 21b | (3a*R*,11a*S*)-5-(2-((*R*/*S*)-1-Acryloyl-1,6-diazaspiro[3.4]octan-6-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 22a | (3a*R*,11a*S*)-5-(2-((4a*S*/R,7a*S*/*R*)-4-(1-Acryloylazetidin-3-yl)hexahydropyrrolo[3,4-*b*][1,4]oxazin-6(2*H*)-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 22b | (3a*R*,11a*S*)-5-(2-((4a*R*/*S*,7a*R*/*S*)-4-(1-Acryloylazetidin-3-yl)hexahydropyrrolo[3,4-b][1,4]oxazin-6(2*H*)-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 23a | (3a*R*,11a*S*)-5-(2-((*S*/*R*)-8-Acryloyloctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 23b | (3a*R*,11a*S*)-5-(2-((*R*/*S*)-8-Acryloyloctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 24a | (3a*R*,11a*S*)-5-(2-((*R*/*S*)-1-Acryloyltetrahydro-1’*H*-spiro[azetidin-3,6’-pyrazino[2,1-*c*][1,4]oxazin]-8’(7’*H*)-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 24b | (3a*R*,11a*S*)-5-(2-((*S*/*R*)-1-Acryloyltetrahydro-1’*H*-spiro[azetidin-3,6’-pyrazino[2,1-*c*][1,4]oxazin]-8’(7’*H*)-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 25a | (3a*R*,11a*S*)-5-(2-((3a*S*/*R*,7a*S*/*R*)-2-Acryloyloctahydro-5*H*-pyrrolo[3,4-*c*]pyridin-5-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 25b | (3a*R*,11a*S*)-5-(2-((3a*R*/*S*,7a*R*/*S*)-2-Acryloyloctahydro-5*H*-pyrrolo[3,4-*c*]pyridin-5-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 26a | (3a*R*,11a*S*)-5-(2-((*R*/*S*)-1-Acryloyltetrahydro-1’H-spiro[azetidin-3,6’-pyrazino[2,1-c][1,4]oxazin]-8’(7’*H*)-yl)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 26b | (3a*R*,11a*S*)-5-(2-((*S*/*R*)-1-Acryloyltetrahydro-1’*H*-spiro[azetidin-3,6’-pyrazino[2,1-*c*][1,4]oxazin]-8’(7’H)-yl)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 27 | (3a*R*,11a*S*)-5-(2-(2-((*E*)-4-(Dimethylamino)but-2-enoyl)-5-oxa-2,8-diazaspiro[3.5]nonan-8-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion hydrochlorid |
| 28 | (3a*R*,11a*S*)-5-(2-((*R*)-4-(1-Acryloylazetidin-3-yl)-3-(fluormethyl)piperazin-1-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 29 | (3a*R*,11a*S*)-5-(2-((*S*)-4-(1-Acryloylazetidin-3-yl)-6-fluor-1,4-diazepan-1-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 30 | (3a*R*,11a*S*)-5-(2-(2-Acryloyl-2,5,8-triazaspiro[3.5]nonan-8-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 31 | (3a*R*,11a*S*)-5-(2-((*R*)-2-Acryloyl-6-methyl-2,5,8-triazaspiro[3.5]nonan-8-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 32 | (3a*R*,11a*S*)-5-(2-((*S*)-2-Acryloyl-6-methyl-2,5,8-triazaspiro[3.5]nonan-8-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 33 | (3a*R*,11a*S*)-5-(2-(2-Acryloyl-2,5,8-triazaspiro[3.5]nonan-8-yl)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 34a | (3a*R*,11a*S*)-5-(2-((*S*/*R*)-2-Acryloyl-9-methyl-2,5,8-triazaspiro[3.5]nonan-8-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 34b | (3a*R*,11a*S*)-5-(2-((*R*/*S*)-2-Acryloyl-9-methyl-2,5,8-triazaspiro[3.5]nonan-8-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 35 | (3a*R*,11a*S*)-5-(2-(2-Acryloyl-5-ethyl-2,5,8-triazaspiro[3.5]nonan-8-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 36 | (3a*R*,11a*S*)-5-(2-(5-Acetyl-2-acryloyl-2,5,8-triazaspiro[3.5]nonan-8-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 37a | (3a*R*,11a*S*)-5-(2-((6*R*,9*S*/*R*)-2-Acryloyl-6,9-dimethyl-2,5,8-triazaspiro[3.5]nonan-8-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 37b | (3a*R*,11a*S*)-5-(2-((6*R*,9R/*S*)-2-Acryloyl-6,9-dimethyl-2,5,8-triazaspiro[3.5]nonan-8-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 38 | (3a*R*,11a*S*)-5-((1-(1-Acryloylazetidin-3-yl)-1*H*-imidazol-4-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 39 | (3a*R*,11a*S*)-5-((1-(1-Acryloylazetidin-3-yl)-1*H*-imidazol-4-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3H)-dion |
| 40 | (3a*R*,11a*S*)-5-((1-(1-Acryloylazetidin-3-yl)-1*H*-imidazol-4-yl)methyl)-8-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 41 | (3a*R*,11aS)-5-((1-(1-Acryloylazetidin-3-yl)-1*H*-pyrazol-3-yl)methyl)-8-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 42 | (3a*R*,11a*S*)-5-((1-(1-Acryloylazetidin-3-yl)-1*H*-1,2,4-triazol-3-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 43 | (3a*R*,11a*S*)-5-(3-((1-((*E*)-4-(Dimethylamino)but-2-enoyl)azetidin-3-yl)oxy)propyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 44 | (3a*R*,11a*S*)-6-Chlor-5-(3-((1-((*E*)-4-(dimethylamino)but-2-enoyl)azetidin-3-yl)oxy)propyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 45 | (3a*R*,11a*S*)-5-(2-((*S*)-8-Acryloyl-1-oxooctahydro-2H-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 46 | (3a*R*,11a*S*)-5-(2-((*R*)-8-Acryloyl-1-oxooctahydro-2H-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 47 | (3a*R*,11a*S*)-5-(2-((*S*)-8-Acryloyl-3-oxooctahydro-2H-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 48 | (3a*R*,11a*S*)-5-(2-((*R*)-8-Acryloyl-3-oxooctahydro-2H-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 49 | (3a*R*,11a*S*)-5-(2-(5-Acryloyl-4,5,6,7-tetrahydro-1H-imidazo[4,5-*c*]pyridin-1-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 50 | (3a*R*,11a*S*)-5-(2-(5-Acryloyl-4,5,6,7-tetrahydro-3*H*-imidazo[4,5-*c*]pyridin-3-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 51 | (3a*R*,11a*S*)-5-(2-(4-(1-Acryloylazetidin-3-yl)piperazin-1-yl)-2-oxoethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 52 | (3a*R*,11a*S*)-5-(2-((*R*)-2-Acryloyl-6-methyl-2,5,8-triazaspiro[3.5]nonan-8-yl)-2-oxoethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 53 | (3a*R*,11a*S*)-5-(2-(4-Acryloylpiperazin-1-yl)pyridin-4-yl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 54 | (3a*R*,11a*S*)-5-(3-(4-Acryloylpiperazin-1-yl)phenyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 55 | (3a*R*,11a*S*)-5-(6-(4-Acryloylpiperazin-1-yl)pyridin-2-yl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 56 | (3a*R*,11a*S*)-5-(6-(4-Acryloylpiperazin-1-yl)pyridin-2-yl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 57 | (3a*R*,11a*S*)-5-(6-((1-Acryloylazetidin-3-yl)oxy)pyridin-2-yl)-6-fluor-10-methyl-1-(6-methyl-4-(trifluormethyl)pyridin-2-yl)-1,3a,4,5,10,11a-hexahydro-2H-benzo[b]pyrrolo[2,3-f][1,4]diazocin-2,11(3H)-dion |
| 58 | (3a*R*,11a*S*)-5-(2-((7*R*/*S*,9a*R*/*S*)-8-Acryloyl-7-methyl-6-oxooctahydro-2H-pyrazino[1,2-a]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 59 | (3a*R*,11a*S*)-5-(2-((7*S*/*R*,9a*S*/*R*)-8-Acryloyl-7-methyl-6-oxooctahydro-2H-pyrazino[1,2-a]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 60 | (3a*R*,11a*S*)-5-(2-((7*R*/*S*,9a*S*/*R*)-8-Acryloyl-7-methyl-6-oxooctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 61 | (3a*R*,11a*S*)-5-(2-((7*S*/*R*,9a*R*/*S*)-8-Acryloyl-7-methyl-6-oxooctahydro-2*H*-pyrazino[1,2-a]pyrazin-2-yl)ethyl)-6-fluor-10-methyl-1-(6-methyl-4-(trifluormethyl)pyridin-2-yl)-1,3a,4,5,10,11a-hexahydro-2H-benzo[*b*]pyrrolo[2,3-*f*][1,4]diazocin-2,11(3*H*)-dion |

(continued)

| Beispieln ummer | Bezeichnung |
|---|---|
| 62a | (3a*R*,11a*S*)-5-(2-((*R*/*S*)-8-Acryloyl-6-oxooctahydro-2H-pyrazino[1,2-*a*]pyrazin-2-yl) ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 62b | (3a*R*,11a*S*)-5-(2-((*S*/*R*)-8-Acryloyl-6-oxooctahydro-2*H*-pyrazino[1,2-*a*]pyrazin-2-yl) ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 63a | (3a*R*,11a*S*)-5-(2-((6*R*/*S*,9a*S*/*R*)-8-Acryloyl-6-methyloctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 63b | (3a*R*,11a*S*)-5-(2-((6*S*/*R*,9a*R*/*S*)-8-Acryloyl-6-methyloctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 64a | (3a*R*,11a*S*)-5-(2-((6*R*/*S*,9a*R*/*S*)-8-Acryloyl-6-methyloctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 64b | (3a*R*,11a*S*)-5-(2-((6*S*/*R*,9*a*S/*R*)-8-Acryloyl-6-methyloctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 65 | *N*-(2-((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl) ethyl)-1-(2-((dimethylamino)methyl)acryloyl)-*N*-methylazetidin-3-carboxamid |
| 66 | (3a*R*,11a*S*)-5-(2-(((3*R*,4*S*)-1-Acryloyl-3-fluorpiperidin-4-yl)amino)ethyl)-6-chlor-10-methyl-1-(6-methyl-4-(trifluormethyl)pyridin-2-yl)-1,3a,4,5,10,11a-hexahydro-2H-benzo[*b*] pyrrolo[2,3-*f*][1,4]diazocin-2,11(3*H*)-dion |
| 67 | (3a*R*,1a*S*)-5-(2-(((3*R*,4*S*)-1-Acryloyl-3-fluorpiperidin-4-yl)(methyl)amino)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 68 | (3a*R*,11a*S*)-5-((*S*/*R*)-3-((1-Acryloylazetidin-3-yl)(methyl)amino)-2-hydroxypropyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 69 | (3a*R*,11a*S*)-5-((*R*/*S*)-3-((1-Acryloylazetidin-3-yl)(methyl)amino)-2-hydroxypropyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 70 | (3a*R*,11a*S*)-5-(3-((1-Acryloylazetidin-3-yl)amino)propyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*] [1,4]diazocin-2,11(3*H*)-dion |
| 71 | (3a*R*,11a*S*)-5-(3-((1-Acryloylazetidin-3-yl)(methyl)amino)propyl)-6-fluor-10-methyl-1-(6-methyl-4-(trifluormethyl)pyridin-2-yl)-1,3a,4,5,10,11a-hexahydro-2*H*-benzo[*b*]pyrrolo [2,3-*f*][1,4]diazocin-2,11(3*H*)-dion |
| 72 | (3a*R*,11a*S*)-5-(2-(4-(1-Acryloyl-3-methylazetidin-3-yl)piperazin-1-yl)ethyl)-6,10-dimethyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 73a | (3a*R*,11a*S*)-6-Chlor-5-(((*R*/*S*)-7-(2-chloracetyl)-6-methyl-5,6,7,8-tetrahydroimidazo[1,2-a] pyrazin-2-yl)methyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 73b | (3a*R*,11a*S*)-6-Chlor-5-(((*S*/*R*)-7-(2-chloracetyl)-6-methyl-5,6,7,8-tetrahydroimidazo[1,2-*a*] pyrazin-2-yl)methyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 74 | (3a*R*,11a*S*)-5-((7-Acryloyl-3-methyl-5,6,7,8-tetrahydroimidazo[1,2-*a*]pyrazin-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 75 | (3a*R*,11a*S*)-5-(2-(4-(1-Acryloylazetidin-3-yl)piperazin-1-yl)-2-oxoethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 76a | (3a*R*,11a*S*)-5-(2-((*R*/*S*)-8-Acryloyloctahydro-2*H*-pyrazino[1,2-*a*]pyrazin-2-yl)-2-oxoethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 76b | (3a*R*,11a*S*)-5-(2-((*S*/*R*)-8-Acryloyloctahydro-2*H*-pyrazino[1,2-*a*]pyrazin-2-yl)-2-oxoethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 77 | (3a*R*,11a*S*)-5-(3-((1-(2-((Dimethylamino)methyl)acryloyl)azetidin-3-yl)oxy)propyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 78 | 2-(3-(3-((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propoxy)azetidin-1-carbonyl)allylacetat |
| 79 | (3a*R*,11a*S*)-5-(3-((1-((*Z*)-2-Chlor-4-(dimethylamino)but-2-enoyl)azetidin-3-yl)oxy)propyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 80 | (3a*R*,11a*S*)-5-(3-((1-((*E*)-2-((Dimethylamino)methyl)but-2-enoyl)azetidin-3-yl)oxy)propyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 81 | (3a*R*,11a*S*)-5-(3-((1-(2-((Dimethylamino)methyl)acryloyl)azetidin-3-yl)oxy)propyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 82 | (3a*R*,11a*S*)-6,10-Dimethyl-1-(6-methyl-4-(trifluormethyl)pyridin-2-yl)-5-(3-((1-(1,2,5,6-tetrahydropyridin-3-carbonyl)azetidin-3-yl)oxy)propyl)-1,3a,4,5,10,11a-hexahydro-2*H*-benzo[*b*]pyrrolo[2,3-*f*][1,4]diazocin-2,11(3*H*)-dion |
| 83 | (*E*)-*N*-(5-((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)pentyl)-4-(dimethylamino)but-2-enamid |
| 84 | *N*-(5-((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)pentyl)-2-((dimethylamino)methyl)acrylamid |
| 85 | *N*-(2-(2-((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethoxy)ethyl)-2-((dimethylamino)methyl)acrylamid |
| 86 | (3a*R*,11a*S*)-6-Chlor-5-(3-(4-(2-((dimethylamino)methyl)acryloyl)piperazin-1-yl)-3-oxopropyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 87 | *N*-(4-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)acrylamid |
| 88 | *N*-(4-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-1,2,5,6-tetrahydropyridin-3-carboxamid |

(continued)

| Beispieln ummer | Bezeichnung |
|---|---|
| 89 | (*E*)-*N*-(4-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-2-((dimethylamino)methyl)but-2-enamid |
| 90 | *N*-(4-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-methoxyphenyl)acrylamid |
| 91 | *N*-(4-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-((2-(dimethylamino)ethyl)(methyl)amino)phenyl)acrylam id |
| 92 | (3a*R*,11a*S*)-5-((1-(1-Acryloylazetidin-3-yl)piperidin-4-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-f][1,4]diazocin-2,11(3*H*)-dion |
| 93 | (3a*R*,11a*S*)-5-((1-(1-Acryloylazetidin-3-yl)piperidin-4-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 94 | (3a*R*,11a*S*)-5-((1-(1-Acryloylazetidin-3-yl)-4-fluorpiperidin-4-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 95a | (3a*R*,11a*S*)-5-((1-((*S*/*R*)-1-Acryloylpyrrolidin-3-yl)piperidin-4-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 95b | (3a*R*,11a*S*)-5-((1-((*R*/*S*)-1-Acryloylpyrrolidin-3-yl)piperidin-4-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 96 | (3a*R*,11a*S*)-5-((1-(1-Acryloylazetidin-3-yl)-3-hydroxypiperidin-4-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 97 | (3a*R*,11a*S*)-5-(2-(4-(1-Acryloylazetidin-3-yl)piperazin-1-yl)propyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 98 | (3a*R*,11a*S*)-5-((5-(1-Acryloylazetidin-3-yl)-1*H*-1,2,4-triazol-3-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 99 | (3a*R*,11a*S*)-5-((3-(1-Acryloylazetidin-3-yl)-1-methyl-1*H*-1,2,4-triazol-5-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 100 | (3a*R*,11a*S*)-5-((5-(1-Acryloylazetidin-3-yl)-1-methyl-1*H*-1,2,4-triazol-3-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 101 | (3a*R*,11a*S*)-5-((5-(1-Acryloylazetidin-3-yl)-1,2,4-oxadiazol-3-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 102 | (3a*R*,11a*S*)-5-((3-(1-Acryloylazetidin-3-yl)-1,2,4-oxadiazol-5-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 103 | (3a*R*,11a*S*)-5-((5-(1-Acryloylazetidin-3-yl)-1,3,4-oxadiazol-2-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 104 | (3a*R*,11a*S*)-5-(2-(2-Acryloyl-6-oxo-2,5,8-triazaspiro[3.5]nonan-8-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 105 | (3a*R*,11a*S*)-5-(2-(1-Acryloyl-6'*H*-spiro[azetidin-3,5'-imidazo[1,2-*a*]pyrazin]-7'(8'*H*)-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 106 | (3a*R*,11a*S*)-5-(2-(1-Acryloylspiro[azetidin-3,3'-imidazo[1,2-*a*]imidazol]-1'(2'*H*)-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 107 | (3a*R*,11a*S*)-5-(2-((3*R*/*S*,9a*S*/*R*)-8-Acryloyl-3-methyl-4-oxooctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 108 | (3a*R*,11a*S*)-5-(2-((3*S*/*R*,9a*R*/*S*)-8-Acryloyl-3-methyl-4-oxooctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 109 | (3a*R*,11a*S*)-5-(2-((3*R*/*S*,9a*R*/*S*)-8-Acryloyl-3-methyl-4-oxooctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 110 | (3a*R*,11a*S*)-5-(2-((3*S*/*R*,9a*S*/*R*)-8-Acryloyl-3-methyl-4-oxooctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 111 | (3a*R*,11a*S*)-5-(2-(2-Acryloyl-5-methyl-6-oxo-2,5,8-triazaspiro[3.5]nonan-8-yl)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 112 | (3a*R*,11a*S*)-5-(2-(4-(1-Acryloylazetidin-3-yl)-3-oxopiperazin-1-yl)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 113 | (3a*R*,11a*S*)-5-(2-(((*rel-trans*)-1-Acryloyl-4-hydroxypiperidin-3-yl)(methyl)amino)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 114 | (3a*R*,11a*S*)-5-(2-(2-Acryloyl-2,5,8-triazaspiro[3.5]nonan-8-yl)ethyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 115 | (3a*R*,11a*S*)-5-(2-(4-(1-Acryloylazetidin-3-yl)piperazin-1-yl)ethyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 116a | (3a*R*,11a*S*)-5-(2-((*R*/*S*)-8-Acryloyl-4-oxooctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 116b | (3a*R*,11a*S*)-5-(2-((*S*/*R*)-8-Acryloyl-4-oxooctahydro-2*H*-pyrazino[1,2-*a*]pyrazin-2-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 117a | (*R*/*S*)-2-Acryloyl-6-(2-((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,6-diazaspiro[3.4]octan-8-carbonitril |
| 117b | (*S*/*R*)-2-Acryloyl-6-(2-((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,6-diazaspiro[3.4]octan-8-carbonitril |

(continued)

| Beispielnummer | Bezeichnung |
|---|---|
| 118a | (*R*/*S*)-2-Acryloyl-6-(2-((3a*R*,11a*S*)-6,10-dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,6-diazaspiro[3.4]octan-8-carbonitril |
| 118b | (*S*/*R*)-2-Acryloyl-6-(2-((3a*R*,11a*S*)-6,10-dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-2,6-diazaspiro[3.4]octan-8-carbonitril |
| 119a | (*R*/*S*)-2-Acryloyl-6-(2-((3a*R*,11a*S*)-6,10-dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-8-methyl-2,6-diazaspiro[3.4]octan-8-carbonitril |
| 119b | (*S*/*R*)-2-Acryloyl-6-(2-((3a*R*,11a*S*)-6,10-dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-8-methyl-2,6-diazaspiro[3.4]octan-8-carboni |
| 120 | (3a*R*,11a*S*)-5-((1-(1-Acryloylazetidin-3-yl)-1*H*-pyrazol-3-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 121 | (3a*R*,11a*S*)-5-((1-(1-Acryloylazetidin-3-yl)-1*H*-pyrazol-4-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 122 | (3a*R*,11a*S*)-5-((2-(1-Acryloylazetidin-3-yl)-1*H*-imidazol-4-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 123 | (3a*R*,11a*S*)-5-((1-(1-Acryloylazetidin-3-yl)-1*H*-1,2,4-triazol-3-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 124 | (3a*R*,11a*S*)-5-((2-(4-Acryloylpiperazin-1-yl)oxazol-4-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 125 | (3a*R*,11a*S*)-5-((2-(4-Acryloylpiperazin-1-yl)-1*H*-imidazol-4-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 126 | (3a*R*,11a*S*)-5-((1-(1-Acryloylpiperidin-4-yl)-1*H*-1,2,4-triazol-3-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 127 | (3a*R*,11a*S*)-5-(3-(1-Acryloylazetidin-3-yl)benzyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 128 | (3a*R*,11a*S*)-5-((6-(1-Acryloylazetidin-3-yl)pyridin-2-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 129 | (3a*R*,11a*S*)-5-((2-(1-Acryloylazetidin-3-yl)pyrimidin-4-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 130 | (3a*R*,11a*S*)-5-((2-Acryloylisoindolin-5-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 131 | (3a*R*,11a*S*)-5-((2-Acryloyl-1,2,3,4-tetrahydroisoquinolin-6-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 132 | (3a*R*,11a*S*)-5-((7-Acryloyl-5,6,7,8-tetrahydroimidazo[1,2-a]pyrazin-2-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 133 | (3a*R*,11a*S*)-5-((7-Acryloyl-5,6,7,8-tetrahydro-[1,2,4]triazolo[1,5-*a*]pyrazin-2-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 134 | (3a*R*,11a*S*)-5-((7-Acryloyl-5,6,7,8-tetrahydro-[1,2,4]triazolo[4,3-*a*]pyrazin-3-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 135 | (3a*R*,11a*S*)-5-((1-(1-Acryloylpiperidin-4-yl)-1*H*-1,2,4-triazol-3-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 136 | (3a*R*,11a*S*)-5-((7-Acryloyl-5,6,7,8-tetrahydroimidazo[1,2-*a*]pyrazin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 137 | (3a*R*,11a*S*)-5-((1-(1-Acryloyl-3-methylazetidin-3-yl)piperidin-4-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 138 | (3a*R*,11a*S*)-5-(3-((1-Acryloylazetidin-3-yl)(methyl)amino)propyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 139 | (3a*R*,11a*S*)-5-((1-(1-Acryloylazetidin-3-yl)-1*H*-1,2,4-triazol-3-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 140a | (3a*R*,11a*S*)-5-(((3*S*,9a*S*/*R*)-8-Acryloyl-2-methyloctahydro-2*H*-pyrazino[1,2-*a*]pyrazin-3-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 140b | (3a*R*,11a*S*)-5-(((3*S*,9a*R*/*S*)-8-Acryloyl-2-methyloctahydro-2*H*-pyrazino[1,2-*a*]pyrazin-3-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 141a | (3a*R*,11a*S*)-5-(4-((*R*/*S*)-1-Acryloylpyrrolidin-2-yl)benzyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 141b | (3a*R*,11a*S*)-5-(4-((*S*/*R*)-1-Acryloylpyrrolidin-2-yl)benzyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 142a | (3a*R*,11a*S*)-5-(((*R*/*S*)-7-Acryloyl-8-methyl-5,6,7,8-tetrahydroimidazo[1,2-*a*]pyrazin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 142b | (3a*R*,11a*S*)-5-(((*S*/*R*)-7-Acryloyl-8-methyl-5,6,7,8-tetrahydroimidazo[1,2-*a*]pyrazin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 143a | (3a*R*,11a*S*)-5-(((*R*/*S*)-7-Acryloyl-6-methyl-5,6,7,8-tetrahydroimidazo[1,2-*a*]pyrazin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 143b | (3a*R*,11a*S*)-5-(((*S*/*R*)-7-Acryloyl-6-methyl-5,6,7,8-tetrahydroimidazo[1,2-*a*]pyrazin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 144a | (3a*R*,11a*S*)-5-(((*R*/*S*)-7-Acryloyl-6-methyl-5,6,7,8-tetrahydroimidazo[1,2-*a*]pyrazin-2-yl) methyl)-6-chlor-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 144b | (3a*R*,11a*S*)-5-(((*S*/*R*)-7-Acryloyl-6-methyl-5,6,7,8-tetrahydroimidazo[1,2-*a*]pyrazin-2-yl) methyl)-6-chlor-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 145 | (3a*R*,11a*S*)-5-((1-(1-((*E*)-4-(Dimethylamino)but-2-enoyl)azetidin-3-yl)-1*H*-pyrazol-3-yl) methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 146 | (3a*R*,11a*S*)-5-(4-(1-((*E*)-4-(Dimethylamino)but-2-enoyl)azetidin-3-yl)benzyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 147 | (3a*R*,11a*S*)-5-(3-(1-((*E*)-4-(Dimethylamino)but-2-enoyl)azetidin-3-yl)benzyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 148 | (3a*R*,11a*S*)-5-((2-((*E*)-4-(Dimethylamino)but-2-enoyl)isoindolin-5-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 149 | (3a*R*,11a*S*)-5-((2-((*E*)-4-(Dimethylamino)but-2-enoyl)-1,2,3,4-tetrahydroisoquinolin-6-yl) methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 150 | (3a*R*,11a*S*)-5-(3-((1-(2-((Dimethylamino)methyl)acryloyl)azetidin-3-yl)oxy)propyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 151 | (3a*R*,11a*S*)-5-(4-(1-(2-((Dimethylamino)methyl)acryloyl)azetidin-3-yl)benzyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 152 | (3a*R*,11a*S*)-5-(3-(1-(2-((Dimethylamino)methyl)acryloyl)azetidin-3-yl)benzyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 153 | (3a*R*,11a*S*)-5-((2-(2-((Dimethylamino)methyl)acryloyl)isoindolin-5-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 154 | (3a*R*,11a*S*)-5-((2-(2-((Dimethylamino)methyl)acryloyl)-1,2,3,4-tetrahydroisoquinolin-6-yl) methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 155 | (3a*R*,11a*S*)-6-chlor-5-((4-(1-((*E*)-4-(Dimethylamino)but-2-enoyl)azetidin-3-yl)cyclohexyl) methyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 156 | (3a*R*,11a*S*)-6-Chlor-5-(3-((1-(2-((dimethylamino)methyl)acryloyl)azetidin-3-yl)oxy)propyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 157 | (3a*R*,11a*S*)-6-Chlor-5-(2-((1-(2-((dimethylamino)methyl)acryloyl)piperidin-4-yl)oxy) ethyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 158 | (3a*R*,11a*S*)-6-Chlor-5-(3-(1-(2-((dimethylamino)methyl)acryloyl)piperidin-4-yl)propyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 159 | (3a*R*,11a*S*)-6-Chlor-5-((7-(2-((dimethylamino)methyl)acryloyl)-5,6,7,8-tetrahydroimidazo[1,2-*a*]pyrazin-2-yl)methyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 160 | (3a*R*,11a*S*)-6-Chlor-5-((4-(1-(2-((dimethylamino)methyl)acryloyl)azetidin-3-yl)cyclohexyl)methyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 161 | (3a*R*,11a*S*)-6-Chlor-5-(4-(1-(2-((dimethylamino)methyl)acryloyl)azetidin-3-yl)butyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 162 | (3a*R*,11a*S*)-6-Chlor-5-(2-((1-(2-((dimethylamino)methyl)acryloyl)azetidin-3-yl)methoxy)ethyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 163 | (3a*R*,11a*S*)-6-Chlor-5-((2-(2-((dimethylamino)methyl)acryloyl)isoindolin-5-yl)methyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 164 | *N*-(3-(((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)-2-((dimethylamino)methyl)acrylamid |
| 165 | (3a*R*,11a*S*)-6-Chlor-5-(3-((1-methacryloylazetidin-3-yl)oxy)propyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 166 | (3a*R*,11a*S*)-6-Chlor-5-(3-((1-(2-((diethylamino)methyl)acryloyl)azetidin-3-yl)oxy)propyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 167 | (3a*R*,11a*S*)-6-Chlor-10-methyl-1-(6-methyl-4-(trifluormethyl)pyridin-2-yl)-5-(4-(1-(2-((4-methylpiperazin-1-yl)methyl)acryloyl)azetidin-3-yl)butyl)-1,3a,4,5,10,11a-hexahydro-2*H*-benzo[*b*]pyrrolo[2,3-*f*][1,4]diazocin-2,11(3*H*)-dion |
| 168 | (3a*R*,11a*S*)-6-Chlor-5-(3-(1-(2-(hydroxymethyl)acryloyl)piperidin-4-yl)propyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 169 | (3a*R*,11a*S*)-5-(3-((1-((*E*)-4-(Dimethylamino)but-2-enoyl)azetidin-3-yl)oxy)propyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 170a | (3a*R*,11a*S*)-5-(4-((*R*/*S*)-1-((*E*)-4-(Dimethylamino)but-2-enoyl)pyrrolidin-2-yl)benzyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 170b | (3a*R*,11a*S*)-5-(4-((*S*/*R*)-1-((*E*)-4-(Dimethylamino)but-2-enoyl)pyrrolidin-2-yl)benzyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 171a | (3a*R*,11a*S*)-5-(4-((*R*/*S*)-1-(2-((Dimethylamino)methyl)acryloyl)pyrrolidin-2-yl)benzyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 171b | (3a*R*,11a*S*)-5-(4-((*S*/*R*)-1-(2-((Dimethylamino)methyl)acryloyl)pyrrolidin-2-yl)benzyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 172a | (3a*R*,11a*S*)-6-Chlor-10-methyl-1-(6-methyl-4-(trifluormethyl)pyridin-2-yl)-5-(((*S*/*R*)-6-methyl-7-propioloyl-5,6,7,8-tetrahydroimidazo[1,2-*a*]pyrazin-2-yl)methyl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 172b | (3a*R*,11a*S*)-6-Chlor-10-methyl-1-(6-methyl-4-(trifluormethyl)pyridin-2-yl)-5-(((*R*/*S*)-6-methyl-7-propioloyl-5,6,7,8-tetrahydroimidazo[1,2-*a*]pyrazin-2-yl)methyl)-1,3a,4,5,10,11a-hexahydro-2*H*-benzo[*b*]pyrrolo[2,3-*f*][1,4]diazocin-2,11(3*H*)-dion |
| 173 | (3a*R*,11a*S*)-5-((5-Acryloyl-4,5,6,7-tetrahydropyrazolo[1,5-*a*]pyrazin-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 174 | *N*-(3-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)acrylamid |
| 175 | *N*-((3-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)methyl)acrylamid |
| 176 | (3a*R*,11a*S*)-5-((2-((*E*)-4-(Dimethylamino)but-2-enoyl)isoindolin-5-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 177 | (*E*)-*N*-(4-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)benzyl)-4-(dimethylamino)but-2-enamid |
| 178 | (*E*)-*N*-(3-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)-4-(dimethylamino)but-2-enamid |
| 179 | (*E*)-*N*-((3-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)methyl)-4-(dimethylamino)but-2-enamid |
| 180 | (3a*R*,11a*S*)-5-(2-(((*R*)-1-(2-((Dimethylamino)methyl)acryloyl)pyrrolidin-3-yl)methoxy)ethyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 181 | (3a*R*,11a*S*)-5-(2-(((*S*)-1-(2-((Dimethylamino)methyl)acryloyl)pyrrolidin-3-yl)methoxy)ethyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 182 | (3a*R*,11a*S*)-5-(2-((1-(2-((Dimethylamino)methyl)acryloyl)azetidin-3-yl)methoxy)ethyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 183 | (3a*R*,11a*S*)-5-(4-(1-(2-((Dimethylamino)methyl)acryloyl)azetidin-3-yl)butyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 184 | *N*-(3-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)-2-((dimethylamino)methyl)acrylamid |
| 185 | *N*-((3-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)methyl)-2-((dimethylamino)methyl)acrylamid |
| 186 | (3a*R*,11a*S*)-5-(4-(1-(2-((Dimethylamino)methyl)acryloyl)azetidin-3-yl)benzyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 187 | (3a*R*,11a*S*)-5-(3-(1-(2-((Dimethylamino)methyl)acryloyl)azetidin-3-yl)-4-fluorbenzyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 188 | (3a*R*,11a*S*)-5-((2-(2-((Dimethylamino)methyl)acryloyl)isoindolin-5-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 189 | *N*-(4-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)benzyl)-2-((dimethylamino)methyl)acrylamid |
| 190 | *N*-((5-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)pyridin-2-yl)methyl)-2-((dimethylamino)methyl)acrylamid |
| 191 | (3a*R*,11a*S*)-5-(3-((1-(2-((Dimethylamino)methyl)acryloyl)azetidin-3-yl)oxy)propyl)-6-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 192 | (3a*R*,11a*S*)-5-(4-(1-(2-((Dimethylamino)methyl)acryloyl)azetidin-3-yl)butyl)-6-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 193 | (3aR,11aS)-6,10-Dimethyl-1-(6-methyl-4-(trifluormethyl)pyridin-2-yl)-5-(2-((1-(2-((4-methylpiperazin-1-yl)methyl)acryloyl)azetidin-3-yl)methoxy)ethyl)-1,3a,4,5,10,11a-hexahydro-2*H*-benzo[*b*]pyrrolo[2,3-*f*][1,4]diazocin-2,11(3*H*)-dion |
| 194 | (3a*R*,11a*S*)-5-(3-((1-((*E*)-2-((Dimethylamino)methyl)but-2-enoyl)azetidin-3-yl)oxy)propyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 195 | (3a*R*,11a*S*)-5-(4-(1-((*E*)-2-((Dimethylamino)methyl)but-2-enoyl)azetidin-3-yl)benzyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 196 | (3a*R*,11a*S*)-6,10-Dimethyl-5-(3-((1-(1-methyl-1,2,5,6-tetrahydropyridin-3-carbonyl)azetidin-3-yl)oxy)propyl)-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 197 | (3a*R*,11a*S*)-6,10-Dimethyl-5-(2-((1-(1-methyl-1,2,5,6-tetrahydropyridin-3-carbonyl)azetidin-3-yl)methoxy)ethyl)-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3H)-dion |
| 198 | (3a*R*,11a*S*)-6,10-Dimethyl-1-(6-methyl-4-(trifluormethyl)pyridin-2-yl)-5-((5-propioloyl-4,5,6,7-tetrahydropyrazolo[1,5-*a*]pyrazin-2-yl)methyl)-1,3a,4,5,10,11a-hexahydro-2*H*-benzo[*b*]pyrrolo[2,3-*f*][1,4]diazocin-2,11(3*H*)-dion |
| 199 | (3a*R*,11a*S*)-6,10-Dimethyl-1-(6-methyl-4-(trifluormethyl)pyridin-2-yl)-5-(((*R*/*S*)-6-methyl-7-propioloyl-5,6,7,8-tetrahydroimidazo[1,2-*a*]pyrazin-2-yl)methyl)-1,3a,4,5,10,11a-hexahydro-2*H*-benzo[*b*]pyrrolo[2,3-*f*][1,4]diazocin-2,11(3*H*)-dion |
| 200 | *N*-(3-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)bicyclo[1.1.1]pentan-1-yl)propiolamid |
| 201 | (3a*R*,11a*S*)-5-((7-(But-2-ynoyl)-5,6,7,8-tetrahydroimidazo[1,2-*a*]pyrazin-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 202 | (3a*R*,11a*S*)-5-((7-Acryloyl-5,6,7,8-tetrahydro-[1,2,4]triazolo[1,5-*a*]pyrazin-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 203 | (3a*R*,11a*S*)-5-((7-Acryloyl-5,6,7,8-tetrahydroimidazo[1,2-*a*]pyrazin-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 204 | (3a*R*,11a*S*)-5-((1-(1-Acryloylpiperidin-4-yl)-1*H*-1,2,4-triazol-3-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 205 | (3a*R*,11a*S*)-5-(3-((1-Acryloylazetidin-3-yl)(methyl)amino)propyl)-6,10-dimethyl-1-(6-1,3a,4,5,10,11a-hexahydro-2*H*-benzo[*b*]pyrrolo[2,3-*f*][1,4]diazocin-2,11(3*H*)-dion |
| 206a | (3a*R*,11a*S*)-5-(((*R*/*S*)-7-Acryloyl-6-methyl-5,6,7,8-tetrahydroimidazo[1,2-*a*]pyrazin-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 206b | (3a*R*,11a*S*)-5-(((*S*/*R*)-7-Acryloyl-6-methyl-5,6,7,8-tetrahydroimidazo[1,2-*a*]pyrazin-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 207 | (*E*)-*N*-(4-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-4-(dimethylamino)but-2-enamid |
| 208 | (*E*)-*N*-(4-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-methoxyphenyl)-4-(dimethylamino)but-2-enamid |
| 209 | *N*-(4-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-2-((dimethylamino)methyl)acrylamid |
| 210 | *N*-(4-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-methoxyphenyl)-2-((dimethylamino)methyl)acrylamid |
| 211 | *N*-(4-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-1-methyl-1,2,5,6-tetrahydropyridin-3-carboxamid |
| 212 | (3a*R*,11a*S*)-5-((1-(1-Acryloylazetidin-3-yl)piperidin-4-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 213 | (3a*R*,11a*S*)-5-(((*S*)-1-(1-Acryloylazetidin-3-yl)pyrrolidin-3-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 214 | (3a*R*,11a*S*)-5-(((*R*)-1-(1-Acryloylazetidin-3-yl)pyrrolidin-3-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 215 | (3a*R*,11a*S*)-5-(2-(1-(1-Acryloylazetidin-3-yl)piperidin-4-yl)ethyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 216 | (3a*R*,11a*S*)-5-(1-(1-Acryloylazetidin-3-yl)piperidin-4-yl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 217 | (3a*R*,11a*S*)-5-((1-(1-Acryloylazetidin-3-yl)-4-fluorpiperidin-4-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 218 | (3a*R*,11a*S*)-5-(((*S*)-1-(1-Acryloylazetidin-3-yl)pyrrolidin-3-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 219 | (3a*R*,11a*S*)-5-(((*R*)-1-(1-Acryloylazetidin-3-yl)pyrrolidin-3-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 220 | (3a*R*,11a*S*)-5-(2-((*S*)-1-(1-Acryloylazetidin-3-yl)pyrrolidin-3-yl)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*) -dion |
| 221 | (3a*R*,11a*S*)-5-(2-((*R*)-1-(1-Acryloylazetidin-3-yl)pyrrolidin-3-yl)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 222 | (3a*R*,11a*S*)-5-((1-(1-Acryloylpyrrolidin-3-yl)piperidin-4-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 223 | (3a*R*,11a*S*)-5-(((*S*)-1-(1-Acryloylazetidin-3-yl)pyrrolidin-3-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 224 | (3a*R*,11a*S*)-5-(((*R*)-1-(1-Acryloylazetidin-3-yl)pyrrolidin-3-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 225a | (3a*R*,11a*S*)-5-(((*R*/*S*)-1-(1-Acryloylazetidin-3-yl)piperidin-3-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 225b | (3a*R*,11a*S*)-5-(((*S*/*R*)-1-(1-Acryloylazetidin-3-yl)piperidin-3-yl)methyl)-6-fluor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 226a | (3*S*/*R*,4*R*/*S*)-1-(1-Acryloylazetidin-3-yl)-4-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)piperidin-3-carbonitril |
| 226b | (3*R*/*S*,4*S*/*R*)-1-(1-Acryloylazetidin-3-yl)-4-(((3aR,11aS)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)piperidin-3-carbonitril |
| 227a | (3*S*/*R*,4*S*/*R*)-1-(1-Acryloylazetidin-3-yl)-4-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)piperidin-3-carbonitril |
| 227b | (3*R*/*S*,4*R*/*S*)-1-(1-Acryloylazetidin-3-yl)-4-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)piperidin-3-carbonitril |
| 228 | (3a*R*,11a*S*)-5-((6-Acryloyl-6,7-dihydro-5*H*-pyrrolo[3,4-*b*]pyrazin-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 229 | (3a*R*,11a*S*)-5-((6-Acryloyl-6,7-dihydro-5*H*-pyrrolo[3,4-*b*]pyrazin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 230 | (3a*R*,11a*S*)-5-(((*R*/*S*)-6-Acryloyl-7-(hydroxymethyl)-6,7-dihydro-5*H*-pyrrolo[3,4-*b*]pyridin-3-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 231 | (3a*R*,11a*S*)-5-(((*S*/*R*)-6-Acryloyl-7-(hydroxymethyl)-6,7-dihydro-5*H*-pyrrolo[3,4-*b*]pyridin-3-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 232 | (3a*R*,11a*S*)-5-(((*R*/*S*)-6-Acryloyl-7-(hydroxymethyl)-6,7-dihydro-5*H*-pyrrolo[3,4-*b*]pyridin-3-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 233 | (3a*R*,11a*S*)-5-(((*S*/*R*)-6-Acryloyl-7-(hydroxymethyl)-6,7-dihydro-5*H*-pyrrolo[3,4-*b*]pyridin-3-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 234 | (3a*R*,11a*S*)-5-(((*S*/*R*)-6-Acryloyl-7-(methoxymethyl)-6,7-dihydro-5*H*-pyrrolo[3,4 -*b*]pyridin-3-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 235 | (3a*R*,11a*S*)-5-(((*R*/*S*)-6-Acryloyl-7-(methoxymethyl)-6,7-dihydro-5*H*-pyrrolo[3,4-*b*]pyridin-3-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 236 | (3a*R*,11a*S*)-5-(((*S*/*R*)-6-Acryloyl-7-(methoxymethyl)-6,7-dihydro-5*H*-pyrrolo[3,4-*b*]pyridin-3-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 237 | (3a*R*,11a*S*)-5-(((*R*/*S*)-6-Acryloyl-7-(methoxymethyl)-6,7-dihydro-5*H*-pyrrolo[3,4-*b*]pyridin-3-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 238 | (3a*R*,11a*S*)-5-((6-Acryloyl-5-(hydroxymethyl)-6,7-dihydro-5*H*-pyrrolo[3,4-*b*]pyridin-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 239 | (3a*R*,11a*S*)-5-((6-Acryloyl-5-(hydroxymethyl)-6,7-dihydro-5*H*-pyrrolo[3,4-*b*]pyridin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 240 | (3a*R*,11a*S*)-5-((6-Acryloyl-6,7-dihydro-5*H*-pyrrolo[3,4-b]pyridin-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 241 | (3a*R*,11a*S*)-5-((5-Acryloyl-1-methyl-1,4,5,6-tetrahydropyrrolo[3,4-*d*]imidazol-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 242 | (3a*R*,11a*S*)-5-((5-Acryloyl-1-methyl-1,4,5,6-tetrahydropyrrolo[3,4-*d*]imidazol-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 243a | (3a*R*,11a*S*)-5-(((*S*/*R*)-7-Acryloyl-6-(difluormethyl)-5,6,7,8-tetrahydroimidazo[1,2-*a*]pyrazin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 243b | (3a*R*,11a*S*)-5-(((*R*/*S*)-7-Acryloyl-6-(difluormethyl)-5,6,7,8-tetrahydroimidazo[1,2-*a*]pyrazin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 244 | (3a*R*,11a*S*)-5-((7-Acryloyl-6-(hydroxymethyl)-5,6,7,8-tetrahydroimidazo[1,2-*a*]pyrazin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 245 | (*E*)-*N*-(4-(((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-fluorphenyl)-4-(dimethylamino)but-2-enamid |
| 246 | (*E*)-*N*-(4-(((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-3-fluorphenyl)-4-(dimethylamino)but-2-enamid |

(continued)

| Beispieln ummer | Bezeichnung |
|---|---|
| 247 | (*E*)-*N*-(3-(((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)-4-(dimethylamino)but-2-enamid |
| 248 | *N*-(4-(((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-cyanophenyl)acrylamid |
| 249 | (*E*)-4-(4-Acetylpiperazin-1-yl)-*N*-(4-(((3a*R*,11a*S*)-6,10-dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)phenyl)but-2-enamid |
| 250 | (3a*R*,11a*S*)-5-((4-Acryloyl-3,4-dihydro-2*H*-benzo[*b*][1,4]oxazin-6-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 251 | (3a*R*,11a*S*)-6-Chlor-5-((4-((*E*)-4-(dimethylamino)but-2-enoyl)-3,4-dihydro-2H-benzo[*b*][1,4]oxazin-6-yl)methyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 252 | (*E*)-*N*-(4-((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)butyl)-4-(dimethylamino)but-2-enamid |
| 253 | (*E*)-*N*-(5-((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-4-fluorpentyl)-4-(dimethylamino)but-2-enamid |
| 254 | (3a*R*,11a*S*)-5-(4-(1-((*Z*)-2-Chlor-4-(dimethylamino)but-2-enoyl)azetidin-3-yl)butyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 255 | (3a*R*,11a*S*)-5-((2-((*Z*)-2-Chlor-4-(dimethylamino)but-2-enoyl)isoindolin-5-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 256 | (3a*R*,11a*S*)-5-(2-(4-Acryloylpiperazin-1-yl)ethyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 257a | *N*-((*S*/*R*)-1-(2-((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5H-benzo[*b*]pyrrolo[2,3-*f*][1,4]diazocin-5-yl)ethyl)-3,3-difluorpiperidin-4-yl)acrylamid |
| 257b | *N*-((*R*/*S*)-1-(2-((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)-3,3-difluorpiperidin-4-yl)acrylamid |
| 258a | 2-((*R*/*S*)-1-Acryloyl-4-(2-((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)piperazin-2-yl)acetonitril |
| 258b | 2-((*S*/*R*)-1-Acryloyl-4-(2-((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)ethyl)piperazin-2-yl)acetonitril |
| 259 | (3a*R*,11a*S*)-5-(2-(5-Acryloyl-5,6-dihydropyrrolo[3,4-*d*]imidazol-1(4*H*)-yl)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 260 | (3a*R*,11a*S*)-5-(2-((*S*)-4-Acryloyl-2-methylpiperazin-1-yl)ethyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 261 | (3a*R*,11a*S*)-5-(2-(3-Acryloyl-3,6-diazabicyclo[3.1.1]heptan-6-yl)ethyl)-6,10-dimethyl-1-(6-methyl-4-(trifluormethyl)pyridin-2-yl)-1,3a,4,5,10,11a-hexahydro-2*H*-benzo[*b*]pyrrolo [2,3-*f*][1,4]diazocin-2,11(3*H*)-dion |
| 262 | (3a*R*,11a*S*)-5-(2-(3-Acryloyl-3,8-diazabicyclo[3.2.1]octan-8-yl)ethyl)-6,10-dimethyl-1-(6-methyl-4-(trifluormethyl)pyridin-2-yl)-1,3a,4,5,10,11a-hexahydro-2*H*-benzo[*b*]pyrrolo [2,3-*f*][1,4]diazocin-2,11(3*H*)-dion |
| 263 | (3a*R*,11a*S*)-5-(2-((1*R*,4*R*)-5-Acryloyl-2,5-diazabicyclo[2.2.1]heptan-2-yl)ethyl)-6,10-dimethyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 264 | (3a*R*,11a*S*)-6,10-Dimethyl-1-(6-methyl-4-(trifluormethyl)pyridin-2-yl)-5-(2-(4-propioloylpiperazin-1-yl)ethyl)-1,3a,4,5,10,11a-hexahydro-2*H*-benzo[*b*]pyrrolo[2,3-*f*][1,4]diazocin-2,11(3*H*)-dion |
| 265a | (3a*R*,11a*S*)-5-(3-((*S*/*R*)-4-Acryloyl-3-(trifluormethyl)piperazin-1-yl)propyl)-6,10-dimethyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 265b | (3a*R*,11a*S*)-5-(3-((*R*/*S*)-4-Acryloyl-3-(trifluormethyl)piperazin-1-yl)propyl)-6,10-dimethyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 266 | (3a*R*,11a*S*)-5-(3-(4-Acryloylpiperazin-1-yl)propyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 267a | 2-((*S*/*R*)-1-Acryloyl-4-(3-((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propyl)piperazin-2-yl)acetonitril |
| 267b | 2-((*R*/*S*)-1-Acryloyl-4-(3-((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)propyl)piperazin-2-yl)acetonitril |
| 268 | (3a*R*,11a*S*)-5-((3-((*R*)-1-Acryloylpyrrolidin-2-yl)-1,2,4-oxadiazol-5-yl)methyl)-6,10-dimethyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 269 | (3a*R*,11a*S*)-5-((3-((*S*)-1-Acryloylpyrrolidin-2-yl)-1,2,4-oxadiazol-5-yl)methyl)-6,10-dimethyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 270 | (3a*R*,11a*S*)-5-((5-((*R*)-1-Acryloylpyrrolidin-2-yl)-1,2,4-oxadiazol-3-yl)methyl)-6,10-dimethyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 271 | (3a*R*,11a*S*)-5-((5-((*S*)-1-Acryloylpyrrolidin-2-yl)-1,2,4-oxadiazol-3-yl)methyl)-6,10-dimethyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 272 | (3a*R*,11a*S*)-5-((5-((*R*)-1-Acryloylpyrrolidin-2-yl)-1,3,4-oxadiazol-2-yl)methyl)-6,10-dimethyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 273 | (3a*R*,11a*S*)-5-((5-((*S*)-1-Acryloylpyrrolidin-2-yl)-1,3,4-oxadiazol-2-yl)methyl)-6,10-dimethyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 274 | (3a*R*,11a*S*)-5-((5-((*R*)-1-Acryloylazetidin-2-yl)-1,3,4-oxadiazol-2-yl)methyl)-6,10-dimethyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |

(continued)

| Beispieln ummer | Bezeichnung |
|---|---|
| 275 | (3a*R*,11a*S*)-5-((5-((*S*)-1-Acryloylazetidin-2-yl)-1,3,4-oxadiazol-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 276 | (3a*R*,11a*S*)-5-((6-Acryloyl-6,7-dihydro-5*H*-pyrrolo[3,4-*b*]pyridin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 277 | (3a*R*,11a*S*)-5-((2-Acryloyl-2,3-dihydro-1*H*-pyrrolo[3,4-*c*]pyridin-6-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 278 | (3a*R*,11a*S*)-5-((6-Acryloyl-6,7-dihydro-5*H*-pyrrolo[3,4-*d*]pyrimidin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 279 | (3a*R*,11a*S*)-5-((5-Acryloyl-1-ethyl-1,4,5,6-tetrahydropyrrolo[3,4-*d*]imidazol-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 280 | (3a*R*,11a*S*)-5-((5-Acryloyl-5,6-dihydro-4*H*-pyrrolo[3,4-*d*]thiazol-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 281 | (3a*R*,11a*S*)-5-((4-Acryloyl-3,4-dihydro-2*H*-benzo[*b*][1,4]oxazin-7-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 282 | *N*-(5-(((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)pyridin-2-yl)acrylamid |
| 283 | *N*-(6-(((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)pyridin-3-yl)acrylamid |
| 284 | *N*-(2-(((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)pyrimidin-5-yl)acrylamid |
| 285 | (3a*R*,11a*S*)-5-(2-((1-Acryloyl-3-fluorazetidin-3-yl)methoxy)ethyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 286 | *N*-(5-(((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2,3-dihydro-1*H*-inden-2-yl)acrylamid |
| 287a | 2-((*S*/*R*)-2-Acryloyl-5-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)isoindolin-1-yl)acetonitril |
| 287b | 2-((*R*/*S*)-2-Acryloyl-5-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)isoindolin-1-yl)acetonitril |
| 288a | 2-((*R*/*S*)-2-Acryloyl-6-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)isoindolin-1-yl)acetonitril |
| 288b | 2-((*S*/*R*)-2-Acryloyl-6-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)isoindolin-1-yl)acetonitril |

(continued)

| Beispieln ummer | Bezeichnung |
|---|---|
| 289a | 2-((*R*/*S*)-6-Acryloyl-2-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitril |
| 289b | 2-((*S*/*R*)-6-Acryloyl-2-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitril |
| 290a | 2-((*S*/*R*)-6-Acryloyl-2-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitril |
| 290b | 2-((*R*/*S*)-6-Acryloyl-2-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitril |
| 291a | 2-((*R*/*S*)-6-Acryloyl-3-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitril |
| 291b | 2-((*S*/*R*)-6-Acryloyl-3-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitril |
| 292a | 2-((*S*/*R*)-6-Acryloyl-3-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitril |
| 292b | 2-((*R*/*S*)-6-Acryloyl-3-(((3a*R*,11a*S*)-6-chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitril |
| 293a | (3a*R*,11a*S*)-5-(((*S*/*R*)-7-Acryloyl-6-(trifluormethyl)-5,6,7,8-tetrahydroimidazo[1,2-a]pyrazin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 293b | (3a*R*,11a*S*)-5-(((*R*/*S*)-7-Acryloyl-6-(trifluormethyl)-5,6,7,8-tetrahydroimidazo[1,2-*a*]pyrazin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 294a | (3a*R*,11a*S*)-5-(((*R*/*S*)-5-Acryloyl-6-methyl-4,5,6,7-tetrahydropyrazolo[1,5-*a*]pyrazin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 294b | (3a*R*,11a*S*)-5-(((*S*/*R*)-5-Acryloyl-6-methyl-4,5,6,7-tetrahydropyrazolo[1,5-*a*]pyrazin-2-yl)methyl)-6-chlor-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 295 | (*E*)-*N*-(5-((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-2-fluorpentyl)-4-(dimethylamino)but-2-enamid |
| 296 | (*E*)-*N*-(5-((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)-2,2-difluorpentyl)-4-(dimethylamino)but-2-enamid |
| 297 | (3a*R*,11a*S*)-6-Chlor-5-((1-(2-((dimethylamino)methyl)acryloyl)azetidin-3-yl)methyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 298 | (3a*R*,11a*S*)-6-Chlor-5-(2-(1-(2-((dimethylamino)methyl)acryloyl)azetidin-3-yl)ethyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 299 | (3a*R*,11a*S*)-6-Chlor-5-(3-(1-(2-((dimethylamino)methyl)acryloyl)azetidin-3-yl)propyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 300 | (3a*R*,11a*S*)-6-Chlor-5-(4-(1-(2-((dimethylamino)methyl)acryloyl)azetidin-3-yl)-4-fluorbutyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 301 | (3a*R*,11a*S*)-6-Chlor-5-(4-(1-(2-((dimethylamino)methyl)acryloyl)azetidin-3-yl)-3-fluorbutyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 302 | (3a*R*,11a*S*)-6-Chlor-5-(4-(1-(2-((dimethylamino)methyl)acryloyl)azetidin-3-yl)-2-fluorbutyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 303 | (3a*R*,11a*S*)-6-Chlor-5-(2-((3-fluor-1-propioloylazetidin-3-yl)methoxy)ethyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 304 | (3a*R*,11a*S*)-6-Chlor-5-(2-((1-((*E*)-4-(dimethylamino)but-2-enoyl)-3-fluorazetidin-3-yl)methoxy)ethyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 305 | (3a*R*,11a*S*)-6-Chlor-5-(2-((1-(2-((dimethylamino)methyl)acryloyl)-3-fluorazetidin-3-yl)methoxy)ethyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 306 | (3a*R*,11a*S*)-6-Chlor-5-((7-((*E*)-4,4-difluorbut-2-enoyl)-5,6,7,8-tetrahydroimidazo[1,2-*a*]pyrazin-2-yl)methyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 307 | (3a*R*,11a*S*)-6-Chlor-10-methyl-1-(6-methyl-4-(trifluormethyl)pyridin-2-yl)-5-((7-((*E*)-4,4,4-trifluorbut-2-enoyl)-5,6,7,8-tetrahydroimidazo[1,2-*a*]pyrazin-2-yl)methyl)-1,3a,4,5,10,11a-hexahydro-2*H*-benzo[*b*]pyrrolo[2,3-*f*][1,4]diazocin-2,11(3*H*)-dion |
| 308 | (3a*R*,11a*S*)-6-Chlor-5-((4-((*E*)-4-(dimethylamino)but-2-enoyl)-3,4-dihydro-2*H*-benzo[*b*][1,4]oxazin-7-yl)methyl)-10-methyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 309 | (*E*)-*N*-(4-(((*3aR,11aS*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2,6-difluorphenyl)-4-(dimethylamino)but-2-enamid |
| 310a | 2-((*S*/*R*)-5-(((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-((*E*)-4-(dimethylamino)but-2-enoyl)isoindolin-1-yl)acetonitril |
| 310b | 2-((*R*/*S*)-5-(((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-((*E*)-4-(dimethylamino)but-2-enoyl)isoindolin-1-yl)acetonitril |
| 311a | 2-((*R*/*S*)-6-(((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-((*E*)-4-(dimethylamino)but-2-enoyl)isoindolin-1-yl)acetonitril |
| 311b | 2-((*S*/*R*)-6-(((3a*R*,11a*S*)-6-Chlor-10-methyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-2-((*E*)-4-(dimethylamino)but-2-enoyl)isoindolin-1-yl)acetonitril |
| 312 | (3a*R*,11a*S*)-5-((6-Acryloyl-6,7-dihydro-5*H*-pyrrolo[3,4-*b*]pyridin-3-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |

(continued)

| Beispieln ummer | Bezeichnung |
|---|---|
| 313 | (3a*R*,11a*S*)-5-((2-Acryloylisoindolin-5-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-f][1,4]diazocin-2,11(3*H*)-dion |
| 314 | (3a*R*,11a*S*)-5-((5-Acryloyl-1-ethyl-1,4,5,6-tetrahydropyrrolo[3,4-*d*]imidazol-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 315 | (3a*R*,11a*S*)-5-((5-Acryloyl-5,6-dihydro-4*H*-pyrrolo[3,4-*d*]thiazol-2-yl)methyl)-6,10-dimethyl-1-(6-methyl-4-(trifluormethyl)pyridin-2-yl)-1,3a,4,5,10,11a-hexahydro-2*H*-benzo[*b*] pyrrolo[2,3-*f*][1,4]diazocin-2,11(3*H*)-dion |
| 316 | (3a*R*,11a*S*)-5-((5-Acryloyl-5,6-dihydro-4*H*-pyrrolo[3,4-*d*]oxazol-2-yl)methyl)-6,10-dimethyl-1-(6-methyl-4-(trifluormethyl)pyridin-2-yl)-1,3a,4,5,10,11a-hexahydro-2*H*-benzo[b] pyrrolo[2,3-*f*][1,4]diazocin-2,11(3*H*)-dion |
| 317 | (3a*R*,11a*S*)-5-((5-Acryloyl-1-methyl-1,4,5,6-tetrahydropyrrolo[3,4-*c*]pyrazol-3-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 318a | 2-((*R*/*S*)-6-Acryloyl-2-(((3a*R*,11a*S*)-6,10-dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitril |
| 318b | 2-((*S*/*R*)-6-Acryloyl-2-(((3a*R*,11a*S*)-6,10-dimethyl-1-(6-methyl-4-(trifluormethyl)pyridin-2-yl)-2,11-dioxo-1,2,3,3a,4,10,11,11a-octahydro-5H-benzo[*b*]pyrrolo[2,3-*f*][1,4]diazocin-5-yl)methyl)-6,7-dihydro-5*H*-pyrrolo[3,4-b]pyridin-7-yl)acetonitril |
| 319a | 2-((*S*/*R*)-6-Acryloyl-2-(((3a*R*,11a*S*)-6,10-dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitril |
| 319b | 2-((*R*/*S*)-6-Acryloyl-2-(((3a*R*,11a*S*)-6,10-dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitril |
| 320a | 2-((*R*/*S*)-6-Acryloyl-3-(((3a*R*,11a*S*)-6,10-dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitril |
| 320b | 2-((*S*/*R*)-6-Acryloyl-3-(((3a*R*,11a*S*)-6,10-dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-5-yl)acetonitril |
| 321a | 2-((*S*/*R*)-6-Acryloyl-3-(((3a*R*,11a*S*)-6,10-dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5H-pyrrolo[3,4-*b*]pyridin-7-yl)acetonitril |
| 321b | 2-((*R*/*S*)-6-Acryloyl-3-(((3a*R*,11a*S*)-6,10-dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-6,7-dihydro-5*H*-pyrrolo[3,4-*b*]pyridin-7-yl)acetonitril |
| 322a | (3a*R*,11a*S*)-5-(((*S*/*R*)-7-Acryloyl-6-(trifluormethyl)-5,6,7,8-tetrahydroimidazo[1,2-a]pyrazin-2-yl)methyl)-6,10-dimethyl-1-(6-methyl-4-(trifluormethyl)pyridin-2-yl)-1,3a,4,5,10,11a-hexahydro-2*H*-benzo[b]pyrrolo[2,3-*f*][1,4]diazocin-2,11(3*H*)-dion |
| 322b | (3a*R*,11a*S*)-5-(((*R*/*S*)-7-Acryloyl-6-(trifluormethyl)-5,6,7,8-tetrahydroimidazo[1,2-a]pyrazin-2-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 323a | (3a*R*,11a*S*)-5-(4-((*R*/*S*)-4-Acryloylmorpholin-3-yl)benzyl)-6,10-dimethyl-1-(6-methyl-4-(trifluormethyl)pyridin-2-yl)-1,3a,4,5,10,11a-hexahydro-2*H*-benzo[b]pyrrolo[2,3-*f*][1,4]diazocin-2,11(3*H*)-dion |

(continued)

| Beispieln ummer | Bezeichnung |
|---|---|
| 323b | (3a*R*,11a*S*)-5-(4-((*S*/*R*)-4-Acryloylmorpholin-3-yl)benzyl)-6,10-dimethyl-1-(6-methyl-4-(trifluormethyl)pyridin-2-yl)-1,3a,4,5,10,11a-hexahydro-2*H*-benzo[b]pyrrolo[2,3-*f*][1,4]diazocin-2,11(3*H*)-dion |
| 324a | (3a*R*,11a*S*)-5-((6-((*R*/*S*)-4-Acryloylmorpholin-3-yl)pyridin-3-yl)methyl)-6,10-dimethyl-1-(6-methyl-4-(trifluormethyl)pyridin-2-yl)-1,3a,4,5,10,11a-hexahydro-2*H*-benzo[b]pyrrolo[2,3-f][1,4]diazocin-2,11(3*H*)-dion |
| 324b | (3a*R*,11a*S*)-5-((6-((*S*/*R*)-4-Acryloylmorpholin-3-yl)pyridin-3-yl)methyl)-6,10-dimethyl-1-(6-methyl-4-(trifluormethyl)pyridin-2-yl)-1,3a,4,5,10,11a-hexahydro-2*H*-benzo[b]pyrrolo[2,3-f][1,4]diazocin-2,11(3*H*)-dion |
| 325a | (3a*R*,11a*S*)-5-((1-((3*S*/*R*,4*S*/*R*)-1-Acryloyl-4-fluorpyrrolidin-3-yl)-1*H*-pyrazol-4-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 325b | (3a*R*,11a*S*)-5-((1-((3*R*/S,4*R*/*S*)-1-Acryloyl-4-fluorpyrrolidin-3-yl)-1*H*-pyrazol-4-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 325c | (3a*R*,11a*S*)-5-((1-((3*R*/*S*,4*S*/*R*)-1-Acryloyl-4-fluorpyrrolidin-3-yl)-1*H*-pyrazol-4-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3H)-dion |
| 325d | (3a*R*,11a*S*)-5-((1-((3*S*/*R*,4*R*/*S*)-1-Acryloyl-4-fluorpyrrolidin-3-yl)-1*H*-pyrazol-4-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 326a | (3a*R*,11a*S*)-5-((1-((3*S*/*R*,4*R*/*S*)-1-Acryloyl-4-fluorpyrrolidin-3-yl)-1*H*-imidazol-4-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 326b | (3a*R*,11a*S*)-5-((1-((3*R*/S,4*S*/*R*)-1-Acryloyl-4-fluorpyrrolidin-3-yl)-1*H*-imidazol-4-yl)methyl)-6,10-dimethyl-1-(6-methyl-4-(trifluormethyl)pyridin-2-yl)-1,3a,4,5,10,11a-hexahydro-2H-benzo[*b*]pyrrolo[2,3-*f*][1,4]diazocin-2,11(3*H*)-dion |
| 327 | (3a*R*,11a*S*)-5-(3-(1-(2-((Dimethylamino)methyl)acryloyl)azetidin-3-yl)propyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 328 | (3a*R*,11a*S*)-5-((2-(2-((Dimethylamino)methyl)acryloyl)-2-azaspiro[3.3]heptan-6-yl)methyl)-6,10-dimethyl-1-(6-methyl-4-(trifluormethyl)pyridin-2-yl)-1,3a,4,5,10,11a-hexahydro-2*H*-benzo[*b*]pyrrolo[2,3-*f*][1,4]diazocin-2,11(3*H*)-dion |
| 329a | *N*-((*R*/*S*)-2-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,6,7,8-tetrahydroimidazo[1,2-a]pyridin-6-yl)-2-((dimethylamino)methyl)acrylamid |
| 329b | *N*-((*S*/*R*)-2-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,6,7,8-tetrahydroimidazo[1,2-*a*]pyridin-6-yl)-2-((dimethylamino)methyl)acrylamid |
| 330a | *N*-((*S*/*R*)-2-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-.5,6,7,8-tetrahydroimidazo[1,2-*a*]pyridin-7-yl)-2-((dimethylamino)methyl)acrylamid |
| 330b | *N*-((*R*/*S*)-2-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)-5,6,7,8-tetrahydroimidazo[1,2-*a*]pyridin-7-yl)-2-((dimethylamino)methyl)acrylamid |
| 331 | (*E*)-*N*-((1*R,3r*)-*3*-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)cyclobutyl)-4-(dimethylamino)but-2-enamid |

(continued)

| Beispieln ummer | Bezeichnung |
|---|---|
| 332 | (*E*)-*N*-((1*S*,3*s*)-3-(((3a*R*,11a*S*)-6,10-Dimethyl-1-(6-methyl-4-(trifluormethyl)pyridin-2-yl)-2,11-dioxo-1,2,3,3a,4,10,11,11a-octahydro-5*H*-benzo[*b*]pyrrolo[2,3-*f*][1,4]diazocin-5-yl)methyl)cyclobutyl)-4-(dimethylamino)but-2-enamid |

oder ein pharmazeutisch verträgliches Salz oder Solvat davon.

**13.** Pharmazeutische Zusammensetzung, umfassend eine Verbindung der Formel (I) nach einem der Ansprüche 1 bis 12 optional in Kombination mit einem oder mehreren therapeutischen Mitteln.

**14.** Verbindung nach einem der Ansprüche 1 bis 12 zur Verwendung bei Folgenden:

(a) der Therapie; oder
(b) der Prophylaxe oder Behandlung von Krebs.

**15.** Prozess zum Herstellen einer Verbindung der Formel (I) nach Anspruch 1, der Folgendes umfasst:

(a) Entschützung einer Verbindung der Formel (II),

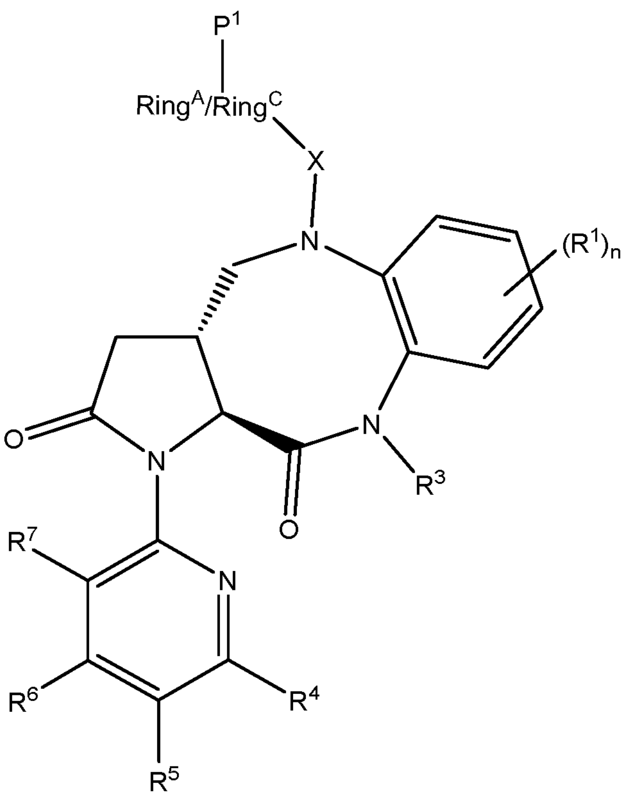

(II)

wobei $R^1$, n, X, $R^3$, $R^4$, $R^5$, $R^6$, $R^7$, $Ring^A$ und $Ring^C$ wie in Anspruch 1 definiert sind und $P^1$ für eine geeignete Schutzgruppe, wie etwa Boc, steht;
(b) Umsetzen einer Verbindung der Formel (III):

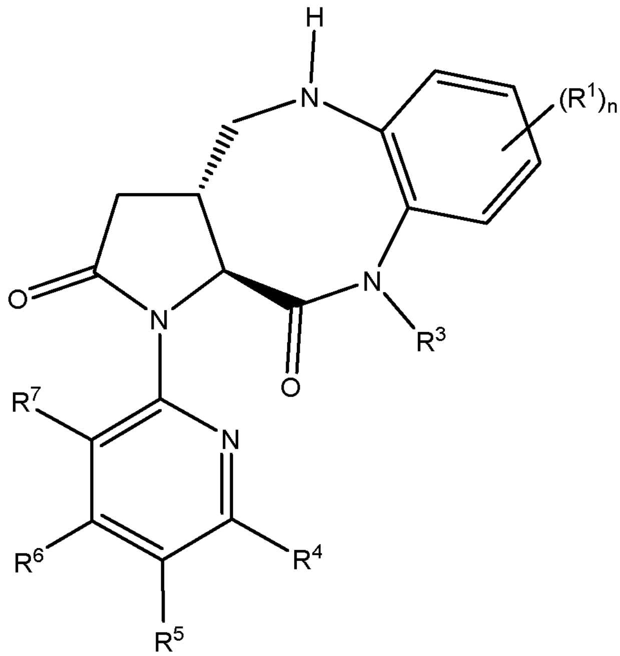

(III)

wobei $R^1$, n, $R^3$, $R^4$, $R^5$, $R^6$ und $R^7$ wie in Anspruch 1 definiert sind, mit einer Verbindung der Formel $L^1$-X-$R^2$, wobei X und $R^2$ wie in Anspruch 1 definiert sind und $L^1$ für eine geeignete Abgangsgruppe, wie etwa Halogen (d. h. Chlor) oder eine Hydroxygruppe, steht;
(c) Entschützung eines geschützten Derivats einer Verbindung der Formel (I);
(d) Umwandlung einer Verbindung der Formel (I) oder eines geschützten Derivats davon in eine weitere Verbindung der Formel (I) oder ein geschütztes Derivat davon; und
(e) optionale Bildung eines pharmazeutisch verträglichen Salzes einer Verbindung der Formel (I).

**Revendications**

1. Composé de formule (I) :

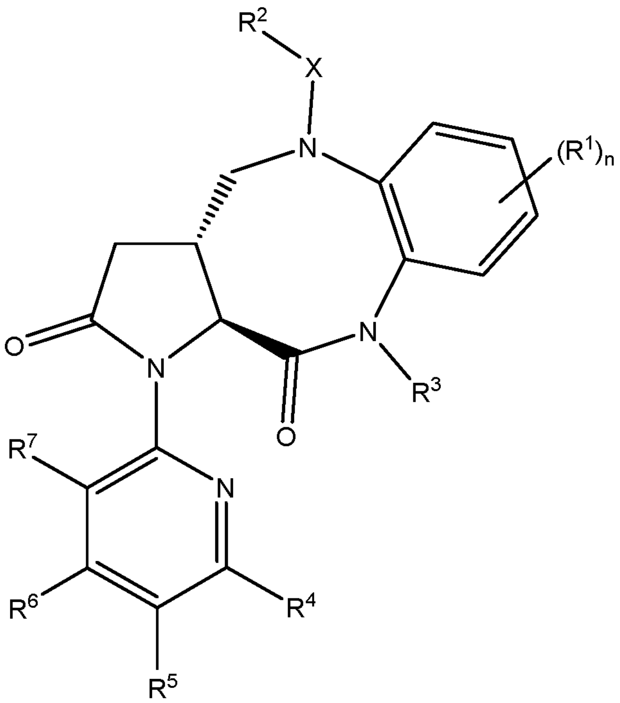

(I)

ou une forme tautomère ou stéréochimiquement isomère, ou un sel ou un solvate pharmaceutiquement acceptable de celui-ci, dans lequel :

n représente un nombre entier choisi parmi 0, 1, 2, 3 ou 4 ;
$R^1$ représente alkyle en $C_{1-6}$, alcényle en $C_{2-6}$, alcoxy en $C_{1-6}$, hydroxy, halogène, halogénoalkyle en $C_{1-6}$, halogénoalcoxy en $C_{1-6}$, cycloalkyle en $C_{3-8}$, cyano ou $-NR^xR^y$ ;
X représente une liaison ou un groupe alkylène en $C_1$-$C_6$ facultativement substitué par un ou plusieurs groupes alkyle en $C_{1-6}$, $NR^x$, O, hydroxy, halogène ou CO ;
$R^2$ représente un groupe cycle$^A$ ou cycle$^B$-Y-cycle$^C$ ;
ou -X-$R^2$ représente un groupe alkylène en $C_1$-$C_{12}$-$NR^xR^y$, dans lequel ledit groupe alkylène peut être facultativement substitué par un ou plusieurs groupes alkyle en $C_{1-6}$, alcényle en $C_{2-6}$, - $NR^x$, O, hydroxy ou CO ;
cycle$^A$ représente carbocyclyle, hétérocyclyle ou hétéroaryle qui nécessite un substituant choisi parmi un groupe $-(CH_2)_m$-CO-alkyle en $C_{1-6}$, $-(CH_2)_m$-NHCO-alkyle en $C_{1-6}$, $-(CH_2)_m$-CO-alcényle en $C_{2-6}$, $-(CH_2)_m$-NHCO-alcényle en $C_{2-6}$, $-(CH_2)_m$-CO-alcynyle en $C_{2-6}$ ou - $(CH_2)_m$-NHCO-alcynyle en $C_{2-6}$, dans lequel ledit groupe alkyle, alcényle ou alcynyle peut être facultativement substitué par un ou plusieurs (par exemple 1, 2 ou 3) groupes halogène, hydroxy, CO ou $-NR^xR^y$ et dans lequel lesdits groupes carbocyclyle, hétérocyclyle ou hétéroaryle peuvent être facultativement également substitués par un ou plusieurs (par exemple 1, 2 ou 3) substituants choisis parmi halogène, alkyle en $C_{1-6}$, hydroxy, alcoxy en $C_{1-6}$, -CO-alkyle en $C_{1-6}$, oxo, $C_{1-6}$ alkylamino ou cyano, dans lequel ledit groupe alkyle en $C_{1-6}$ peut être facultativement substitué par un ou plusieurs groupes halogène, hydroxy ou cyano ;
m représente un nombre entier choisi parmi 0, 1, 2, 3 ou 4 ;
cycle$^B$ représente carbocyclyle, hétérocyclyle ou hétéroaryle, chacun d'entre eux pouvant être facultativement substitué par un ou plusieurs (par exemple 1, 2 ou 3) substituants choisis parmi hydroxy, oxo, alkyle en $C_{1-6}$, alcényle en $C_{2-6}$, alcynyle en $C_{2-6}$, alcoxy en $C_{1-6}$, un halogénoalkyle en $C_{1-6}$, -CO-alkyle en $C_{1-6}$, cyano ou halogène ;
cycle$^C$ représente hétérocyclyle substitué par un ou plusieurs (par exemple 1, 2 ou 3) substituants choisis parmi halogène, alkyle en $C_{1-6}$, -CO-alkyle en $C_{1-6}$, et/ou $-(CH_2)_m$-CO-alcényle en $C_{2-6}$ facultativement substitué par un groupe $-NR^xR^y$ ;
Y représente une liaison, -O-, -NHCO-, CO ou un groupe alkylène en $C_1$-$C_6$ facultativement substitué par un ou plusieurs groupes alkyle en $C_{1-6}$, alcényle en $C_{2-6}$, $-NR^x$, O, hydroxy ou CO ;
$R^3$ représente hydrogène ou alkyle en $C_{1-6}$ ;

$R^4$, $R^5$, $R^6$ et $R^7$ représentent indépendamment hydrogène, alkyle en $C_{1-6}$, alcényle en $C_{2-6}$, alcoxy en $C_{1-6}$, halogène, halogénoalkyle en $C_{1-6}$, halogénoalcoxy en $C_{1-6}$, cycloalkyle en $C_{3-8}$, cyano ou - $NR^xR^y$ ; et
$R^x$ et $R^y$ représentent indépendamment hydrogène, alkyle en $C_{1-6}$, alcényle en $C_{2-6}$, alcynyle en $C_{2-6}$, alcoxy en $C_{1-6}$, ou $R^x$ et $R^y$ ensemble avec l'atome d'azote auquel ils sont attachés se joignent pour former un cycle hétérocyclique contenant de l'azote qui peut être facultativement substitué par un ou plusieurs groupes alkyle en $C_{1-6}$.

**2.** Composé selon la revendication 1, dans lequel n représente 0, 1, 2 ou 3.

**3.** Composé selon la revendication 1 ou la revendication 2, dans lequel $R^1$ représente alkyle en $C_{1-6}$ (tel que méthyle), halogène (tel que fluor ou chlore) ou cycloalkyle en $C_{3-8}$ (tel que cyclopropyle), tel que dans lequel $R^1$ représente alkyle en $C_{1-6}$ (tel que méthyle) ou halogène (tel que fluor ou chlore), en particulier dans lequel $R^1$ représente alkyle en $C_{1-6}$ (tel que méthyle) .

**4.** Composé tel que défini dans l'une quelconque des revendications 1 à 3, dans lequel $R^2$ représente cycle$^A$, tel que dans lequel cycle$^A$ représente carbocyclyle (tel que phényle, cyclobutyle, 2,3-dihydro-1H-indén-2-yle ou bicyclo [1.1.1]pentan-1-yle), un cycle hétérocyclyle (tel qu'azétidinyle, pyrrolidinyle, pipéridinyle, pipérazinyle, isoindolin-1-yle, isoindolin-5-yle, tétrahydroisoquinoléin-6-yle, diazaspiro[3.4]octan-6-yle, octahydro-2*H*-pyrazino[1,2-a]pyrazin-2-yle, octahydro-5H-pyrrolo[3,4-c]pyridin-5-yle, 5-oxa-2,8-diazaspiro[3.5]nonan-8-yle, 2,6-diazaspiro[3.4]octane, 9-oxa-3,7-diazabicyclo[3.3.1]nonan-3-yle, tétrahydro-1*H*-imidazo[4,5-c]pyridin-1-yle, tétrahydro-1'H-spiro [azétidin-3,4'-pyrrolo[1,2-a]pyrazin]-2'(3'*H*)-yle, tétrahydro-1'*H*-spiro[azétidin-3,6'-pyrazino[2,1-*c*][1,4]oxazin]-8'(7'*H*)-yle, tétrahydro-3*H*-imidazo[4,5-*c*]pyridin-3-yle, tétrahydro-[1,2,4]triazolo[1,5-a]pyrazin-2-yle, tétrahydroimidazo[1,2-a]pyrazin-2-yle, tétrahydropyrazolo[1,5-a]pyrazin-2-yle, octahydro-2H-pyrazino[1,2-a]pyrazin-3-yle, tétrahydro-[1,2,4]triazolo[4,3-a]pyrazin-3-yle, 2,5,8-triazaspiro[3,5]nonan-8-yle, spiro[azétidin-3,3'-imidazo [1,2-a]imidazol]-1' (2'H)-yle, dihydro-5H-pyrrolo[3,4-b]pyrazin-2-yle, dihydro-5H-pyrrolo[3,4-b]pyridin-3-yle, dihydro-5H-pyrrolo[3,4-b]pyridin-2-yle, dihydro-5H-pyrrolo[3,4-b]pyridin-7-yle, dihydro-5H-pyrrolo[3,4-b]pyridin-5-yle, tétrahydropyrrolo[3,4-d]imidazol-2-yle, dihydro-2H-benzo[b][1,4]oxazin-6-yle, dihydro-2H-benzo[b][1,4]oxazin-7-yle, dihydro-4H-pyrrolo[3,4-d]imidazol-1-yle, diazabicyclo[3.1.1]heptan-6-yle, diazabicyclo[3.2.1]octan-8-yle, diazabicyclo[2.2.1]heptan-2-yle, dihydro-1H-pyrrolo[3,4-c]pyridin-6-yle, dihydro-5H-pyrrolo[3,4-d]pyrimidin-2-yle, dihydro-4H-pyrrolo[3,4-d]thiazol-2-yle, dihydro-4H-pyrrolo[3,4-d]oxazo-2-yle, tétrahydropyrrolo[3,4-c]pyrazol-3-yle, diazaspiro[3.3]heptan-6-yle, tétrahydroimidazo[1,2-a]pyridin-6-yle, tétrahydroimidazo[1,2-a]pyridin-7-yle ou spiro[azétidin-3,5'-imidazo[1,2-a]pyrazin]-7'(8'H)-yle) ou un cycle hétéroaryle (tel que pyridyle ou pyrimidinyle), chacun étant substitué par

$-(CH_2)_m$-CO-alkyle en $C_{2-6}$ facultativement substitué par un groupe halogène (tel que $-CO-CH_2-Cl$) ;
$-(CH_2)_m$-CO-alcényle en $C_{2-6}$ facultativement substitué par un ou plusieurs groupes halogène, hydroxy, CO ou groupe $-NR^xR^y$ (tels que $-COCH=CH_2$, $-CO-C(=CH_2)-Me$, $-CO-CH=CH-CH_2-N(Me)_2$, $-CO-C(=CH_2)-CH_2-N(Me)_2$, $-CO-C(=CH-Me)-CH_2-N(Me)_2$, $-CO-C(=CH_2)-CH_2-N(Me)_2$, - $CO-C(Cl)=CH-CH_2-N(Me)_2$, $-CO-C(=CH_2)-CH_2-OH$, $-CO-C(=CH_2)-CH_2-OC(=O)-Me)$, $-CO-CH=CH-CHF_2$, $-CO-C(=CH_2)-CH_2-NEt_2)$ ;
$-(CH_2)_m$-NHCO-alcényle en $C_{2-6}$ facultativement substitué par un groupe $-NR^xR^y$ (tel que $-NH-CO-CH=CH_2$, $-NH-CO-C(=CH_2)-CH_2-N(Me)_2$, $-NH-CO-C(=CH-Me)-CH_2-N(Me)_2$, $-NH-CO-CH=CH-CH_2-N(Me)_2$, - $CH_2-NH-CO-CH=CH_2$-, $-CH_2-NH-CO-CH=CH-CH_2-N(Me)_2$, $-CH_2-NH-CO-C(=CH_2)-CH_2-N(Me)_2$) ;
- $(CH_2)_m$-CO-alcynyle en $C_{2-6}$ (tel que -CO-éthynyle ou -CO-éthynyl-Me) ; ou
$-(CH_2)_m$-NHCO-groupe alcynyle en $C_{2-6}$ (tel que -NH-CO-éthynyle),

dans lequel ledit groupe carbocyclyle, hétérocyclyle ou hétéroaryle peut être facultativement substitué par un ou plusieurs (par exemple 1, 2 ou 3) autres substituants choisis parmi : halogène (tel que fluor) ; alkyle en $C_{1-6}$ (tel que méthyle ou éthyle) facultativement substitué par un ou plusieurs groupes halogène (tels que $-CF_3$ ou $-CHF_2$), hydroxy (tel que $-CH_2-OH$) ou cyano (tel que $-CH_2-CN$) ; hydroxy ; alcoxy en $C_{1-6}$ (tel que méthoxy et $-CH_2-OMe$) ; -CO-alkyle en $C_{1-6}$ (tel que -CO-Me) ; oxo ; alkylamino en $C_{1-6}$ (tel que $-N(Me)-(CH_2)_2-N(Me)_2$) ; ou cyano), en particulier cycle$^A$ représente un cycle hétérocyclyle (tel qu'azétidinyle, pyrrolidinyle, pipéridinyle, pipérazinyle, isoindolin-1-yle, isoindolin-5-yle, tétrahydroisoquinoléin-6-yle, diazaspiro[3.4]octan-6-yle, octahydro-2*H*-pyrazino[1,2-a]pyrazin-2-yle, octahydro-5H-pyrrolo[3,4-c]pyridin-5-yle, 5-oxa-2,8-diazaspiro[3.5]nonan-8-yle, 2,6-diazaspiro[3.4]octane, 9-oxa-3,7-diazabicyclo[3.3.1]nonan-3-yle, tétrahydro-1*H*-imidazo[4,5-*c*]pyridin-1-yle, tétrahydro-1'*H*-spiro[azétidin-3,4'-pyrrolo[1,2-a]pyrazin]-2'(3'*H*)-yle, tétrahydro-1'*H*-spiro[azétidin-3,6'-pyrazino [2,1-*c*][1,4]oxazin]-8'(7'*H*)-yle, tétrahydro-3*H*-imidazo[4,5-c]pyridin-3-yle, tétrahydro-[1,2,4]triazolo[1,5-a]pyrazin-2-yle, tétrahydroimidazo[1,2-a]pyrazin-2-yle, tétrahydropyrazolo[1,5-a]pyrazin-2-yle, octahydro-2H-pyrazino[1,2-a]pyrazin-3-yle, tétrahydro-[1,2,4]triazolo[4,3-a]pyrazin-3-yle, 2,5,8-triazaspiro[3,5]nonan-8-yle, spiro [azétidin-3,3'-imidazo[1,2-a]imidazol]-1' (2'H)-yle, dihydro-5H-pyrrolo[3,4-b]pyrazin-2-yle, dihydro-5H-pyrrolo[3,4-b]pyridin-3-yle, dihydro-5H-pyrrolo[3,4-b]pyridin-2-yle, dihydro-5H-pyrrolo[3,4-b]pyridin-7-yle, dihydro-5H-pyrrolo[3,4-b]pyridin-5-yle, tétrahydropyrrolo[3,4-d]imidazol-2-yle, dihydro-2H-benzo[b][1,4]oxazin-6-yle, dihydro-2H-benzo[b][1,4]oxazin-7-yle, dihydro-4H-pyrrolo[3,4-d]imidazol-1-yle, diazabicyclo[3.1.1]heptan-6-yle, diazabicyclo[3.2.1]octan-8-yle, diazabicyclo[2.2.1]heptan-2-yle, dihydro-1H-pyrrolo[3,4-c]pyridin-6-yle, dihydro-5H-pyrrolo[3,4-d]pyrimidin-2-yle, dihydro-4H-pyrrolo[3,4-d]thiazol-2-yle, dihydro-4H-pyrrolo[3,4-d]oxazo-2-yle, tétrahydropyrrolo[3,4-c]pyrazol-3-yle, diazaspiro[3.3]heptan-6-yle, tétrahydroimidazo[1,2-a]pyridin-6-yle, tétrahydroimidazo[1,2-a]pyridin-7-yle ou spiro[azétidin-3,5'-imidazo[1,2-a]pyrazin]-7'(8'H)-yle), chacun étant substitué par

-$(CH_2)_m$-CO-alkyle en $C_{2-6}$ facultativement substitué par un groupe halogène (tel que -CO-$CH_2$-Cl) ;
-$(CH_2)_m$-CO-alcényle en $C_{2-6}$ facultativement substitué par un ou plusieurs groupes halogène, hydroxy, CO ou groupe -$NR^xR^y$ (tels que -COCH=$CH_2$, -CO-C (=$CH_2$)-Me, -CO-CH=CH-$CH_2$-N$(Me)_2$, -CO-C (=$CH_2$)-$CH_2$-N$(Me)_2$, -CO-C(=CH-Me)-$CH_2$-N$(Me)_2$, -CO-C(=$CH_2$)-$CH_2$-N$(Me)_2$, - CO-C (Cl)=CH-$CH_2$-N$(Me)_2$, -CO-C(=$CH_2$)-$CH_2$-OH, -CO-C (=$CH_2$)-$CH_2$-OC(=O)-Me), -CO-CH=CH-$CHF_2$, -CO-C (=$CH_2$)-$CH_2$-$NEt_2$ ;
-$(CH_2)_m$-NHCO-alcényle en $C_{2-6}$ facultativement substitué par un groupe -$NR^xR^y$ (tel que -NH-CO-CH=$CH_2$, -NH-CO-C(=$CH_2$)-$CH_2$-N$(Me)_2$, -NH-CO-C(=CH-Me)-$CH_2$-N$(Me)_2$, -NH-CO-CH=CH-$CH_2$-N$(Me)_2$, -$CH_2$-NH-CO-CH=$CH_2$-, -$CH_2$-NH-CO-CH=CH-$CH_2$-N$(Me)_2$, -$CH_2$-NH-CO-C(=$CH_2$)-$CH_2$-N$(Me)_2$) ;
- $(CH_2)_m$-CO-alcynyle en $C_{2-6}$ (tel que -CO-éthynyle ou -CO-éthynyl-Me) ; ou
-$(CH_2)_m$-NHCO-groupe alcynyle en $C_{2-6}$ (tel que -NH-CO-éthynyle),
dans lequel ledit groupe hétérocyclyle ou hétéroaryle peut être facultativement substitué par un ou plusieurs (par exemple 1, 2 ou 3) autres substituants choisis parmi : halogène (tel que fluor) ; alkyle en $C_{1-6}$ (tel que méthyle ou éthyle) facultativement substitué par un ou plusieurs groupes halogène (tels que -$CF_3$ ou -$CHF_2$), hydroxy (tel que -$CH_2$-OH) ou cyano (tel que -$CH_2$-CN), hydroxy , alcoxy en $C_{1-6}$ (tel que méthoxy et -$CH_2$-OMe) ; -CO-alkyle en $C_{1-6}$ (tel que -CO-Me) ; oxo ; alkylamino en $C_{1-6}$ (tel que -N(Me)-$(CH_2)_2$-N$(Me)_2$) ; ou cyano),

plus particulièrement, cycle$^A$ représente dihydro-5H-pyrrolo[3,4-b]pyridinyle, tel que dihydro-5H-pyrrolo[3,4-b] pyridin-3-yle, dihydro-5H-pyrrolo[3,4-b]pyridin-2-yle, dihydro-5H-pyrrolo[3,4-b]pyridin-7-yle ou dihydro-5H-pyrrolo[3,4-b]pyridin-5-yle, chacun étant substitué par

-$(CH_2)_m$-CO-alcényle en $C_{2-6}$ facultativement substitué par un ou plusieurs groupes halogène, hydroxy, CO ou -$NR^xR^y$ (tels que - COCH=$CH_2$, -CO-C (=$CH_2$)-Me, -CO-CH=CH-$CH_2$-N$(Me)_2$, -CO-C (=$CH_2$)-$CH_2$-N$(Me)_2$, -CO-C(=CH-Me)-$CH_2$-N$(Me)_2$, -CO-C(=$CH_2$)-$CH_2$-N$(Me)_2$, -CO-C(Cl)=CH-$CH_2$-N$(Me)_2$, -CO-C(=$CH_2$)-$CH_2$-OH, -CO-C(=$CH_2$)-$CH_2$-O-C(=O)-Me), -CO-CH=CH-$CHF_2$, -CO-C(=$CH_2$)-$CH_2$-$NEt_2$ ;
dans lequel ledit groupe hétérocyclyle peut être facultativement substitué par un ou plusieurs (par exemple 1, 2 ou 3) autres substituants choisis parmi : halogène (tel que fluor) ; alkyle en $C_{1-6}$ (tel que méthyle ou éthyle) facultativement substitué par un ou plusieurs groupes halogène (tels que -$CF_3$ ou -$CHF_2$), hydroxy (tel que -$CH_2$-OH) ou cyano (tel que -$CH_2$-CN), hydroxy , alcoxy en $C_{1-6}$ (tel que méthoxy et -$CH_2$-OMe) ; -CO-alkyle en $C_{1-6}$ (tel que -CO-Me) ; oxo ; alkylamino en $C_{1-6}$ (tel que -N(Me)-$(CH_2)_2$-N$(Me)_2$) ; ou cyano),

spécifiquement cycle$^A$ représente dihydro-5H-pyrrolo[3,4-b]pyridinyle, tel que dihydro-5H-pyrrolo[3,4-b]pyridin-3-yle, dihydro-5H-pyrrolo[3,4-b]pyridin-2-yle, dihydro-5H-pyrrolo[3,4-b]pyridin-7-yle ou dihydro-5H-pyrrolo [3,4-b]pyridin-5-yle, chacun étant substitué par -COCH=$CH_2$,
plus spécifiquement cycle$^A$ représente dihydro-5H-pyrrolo[3,4-b]pyridin-2-yle substitué par -COCH=$CH_2$,
plus spécifiquement cycle$^A$ représente dihydro-5H-pyrrolo[3,4-b]pyridin-2-yle substitué par -COCH=$CH_2$.

**5.** Composé tel que défini dans l'une quelconque des revendications 1 à 3, dans lequel $R^2$ représente un cycle$^B$-cycle $^Y$, tel que dans lequel cycle$^B$ représente carbocyclyle (tel que cyclohexyle ou phényle), hétérocyclyle (tel qu'azétidinyle, pyrrolidinyle, pipéridinyle, pipérazinyle, diazépanyle ouhexahydropyrrolo[3,4-*b*][1,4]oxazin-6(2*H*)-yle) ou hétéroaryle (tel qu'imidazolyle, pyrazolyle, triazolyle, pyridinyle, pyrimidinyle, oxazolyle ou oxadiazolyle), chacun pouvant être facultativement substitué par un ou plusieurs (par exemple 1, 2 ou 3) substituants choisis parmi hydroxy, oxo, alkyle en $C_{1-6}$ (tel que méthyle), haloalkyle en $C_{1-6}$ (tel que $CH_2$-F), cyano ou halogène (tel que fluor).

**6.** Composé tel que défini dans l'une quelconque des revendications 1 à 5, dans lequel Y représente une liaison, -O-, -NHCO-, CO ou un groupe alkylène en $C_1$-$C_6$ facultativement substitué par un ou plusieurs groupes alkyle en $C_{1-6}$, alcényle en $C_{2-6}$, -$NR^x$, O, hydroxy ou CO (tels que -CO-C(=$CH_2$)-$CH_2$- ou - NHCO-CH=CH-$CH_2$-), tel que dans lequel

Y représente une liaison, en variante dans lequel Y représente -O-.

**7.** Composé tel que défini dans l'une quelconque des revendications 1 à 6, dans lequel cycle$^C$ représente un cycle hétérocyclyle monocyclique ayant au moins un atome d'azote. Dans un autre mode de réalisation, cycle$^C$ représente azétidinyle, pyrrolidinyle, pipérazinyle ou tétrahydropyridinyle, chacun d'entre eux étant substitué par halogène (tel que fluor), alkyle en $C_{1-6}$ (tel que méthyle), -CO-alkyle en $C_{1-6}$ (tel que -CO-méthyle) et/ou $-(CH_2)_m$-CO-alcényle en $C_{2-6}$ facultativement substitué par un groupe $-NR^xR^y$ (tel que $-COCH=CH_2$, $-CO-C(=CH_2)-CH_2-N(Me)_2$, $-CO-C(=CH-Me)-CH_2-N(Me)_2$ ou $-CO-CH=CH-CH_2-N(Me)_2$).

**8.** Composé tel que défini dans l'une quelconque des revendications 1 à 7, dans lequel X représente :

(a) une liaison ou un groupe alkylène en $C_1$-$C_4$ facultativement substitué par un ou plusieurs groupes alkyle en $C_{1-6}$ (tel que méthyle), $-NR^x$ (tel que -NH ou -N-méthyle), O, hydroxy, halogène (tel que fluor) ou CO ; ou
(b) $-CH_2-$ ; ou
(c) un groupe alkylène en $C_1$-$C_4$ (tel que $-CH_2-$, $-(CH_2)_2-$, $-(CH_2)_3-$ ou $-(CH_2)_4-$) facultativement substitué par un ou plusieurs groupes alkyle en $C_{1-6}$ (tels que $-(CH_2)-C(Me)-$), O (tels que - $(CH_2)2-O-$, $-(CH_2)_3-O-$ ou $-(CH_2)2-O-CH_2-$) ou CO (tels que - $(CH_2)-CO-$ ou $-(CH_2)_2-CO-$) $-NR^x$ (tels que $-(CH_2)_2-NH-$, $-(CH_2)_3-NH-$, - $(CH_2)_2-N(Me)-$ ou $-(CH_2)_3-N(Me)-$) ou un groupe CO et $-NR^x$ (tels que $-(CH_2)_2-N(Me)-CO-$) ou un groupe hydroxy et $-NR^x$ (tel que - $(CH_2)-CH(OH)-CH_2-N(Me)-$ ou halogène (tel que $-(CH_2)_3-CHF-$, - $(CH_2)_2-CHF-CH_2-$, ou $CH_2-CHF-(CH_2)_2-$) ; ou
(d) une liaison, -CH2-, $-(CH_2)_2-$, $-(CH_2)_3-$, $-(CH_2)_4-$, $-(CH_2)-C(Me)-$, $-(CH_2)_2-O-$, $-(CH_2)_3-O-$, $-(CH_2)_2-O-CH_2-$, $-(CH_2)-CO-$, - $(CH_2)2-CO-$, - $(CH_2)2-NH-$, - $(CH_2)3-NH-$, - $(CH_2)2-N(Me)-$, - $(CH_2)3-N(Me)$ -, $-(CH_2)_2-N(Me)-CO-$ $-(CH_2)-CH(OH)-CH_2-N(Me)-$, $-(CH_2)_3-CHF-$, - $(CH_2)2-CHF-CH2-$ ou $-CH_2-CHF-(CH_2)_2-$.

**9.** Composé tel que défini dans l'une quelconque des revendications 1 à 8, dans lequel $-X-R^2$ représente un groupe alkylène en $C_1$-$C_{10}$-$NR^xR^y$, dans lequel ledit groupe alkylène peut être facultativement substitué par un ou plusieurs groupes alcényle en $C_{2-6}$, $NR^x$, O ou CO, tels que $-(CH_2)_2-O-(CH_2)_2-NH-CO-C(=CH_2)-CH_2-N(Me)_2$, $-(CH_2)_5-NH-CO-CH=CH-CH_2-N(Me)_2$, $-(CH_2)_5-NH-CO-C(=CH_2)-CH_2-N(Me)$ 2, $-(CH_2)_4-NHCOCH=CH-CH_2-NMe_2$ ou $-CH_2-CHF-(CH_2)_3-NHCO-CH=CHCH_2NMe_2$, $-(CH_2)_3-CHF-CH_2NHCO-CH=CHCH_2NMe_2$ ou $-(CH_2)_3-CF_2-CH_2NHCO-CH=CHCH_2NMe_2$.

**10.** Composé tel que défini dans l'une quelconque des revendications 1 à 9, dans lequel :

(a) $R^3$ représente hydrogène ou alkyle en $C_{1-6}$ (tel que méthyle ou éthyle), tel que dans lequel $R^3$ représente hydrogène ou alkyle en $C_{1-6}$ (tel que méthyle), en particulier dans lequel $R^3$ représente alkyle en $C_{1-6}$ (tel que méthyle), spécifiquement dans lequel $R^3$ représente méthyle ; et/ou
(b) $R^4$ représente :

hydrogène ;
alkyle en $C_{1-6}$ (tel que méthyle ou éthyle) ;
alcényle en $C_{2-6}$ (tel qu'éthényle) ; alcoxy en $C_{1-6}$ (tel que méthoxy) ;
halogène (tel que chlore) ; ou
$-NR^xR^y$ (tel que $-N(Me)_2$ ou -N(Me)(Et)),

tel que dans lequel $R^4$ représente alkyle en $C_{1-6}$ (tel que méthyle), en particulier dans lequel $R^4$ représente méthyle ; et/ou
(c) $R^5$ représente :

hydrogène ;
halogène (tel que chlore) ; ou
alkyle en $C_{1-6}$ (tel que méthyle),

tel que dans lequel $R^5$ représente hydrogène ; et/ou
(d) $R^6$ représente :

alkyle en $C_{1-6}$ (tel que méthyle, éthyle ou isopropyle),
alcényle en $C_{2-6}$ (tel que $-C(=CH_2)(Me)$) ;
halogène (tel que brome) ;
halogénoalkyle en $C_{1-6}$ (tel que trifluorométhyle ou - $C(H)(Me)-CF_3$) ; ou

halogénoalcoxy en $C_{1-6}$ (tel que difluorométhoxy), tel que dans lequel $R^6$ représente halogénoalkyle en $C_{1-6}$ (tel que trifluorométhyle), en particulier dans lequel $R^6$ représente trifluorométhyle ; et/ou

(e) $R^7$ représente hydrogène ou cyano, tel qu'hydrogène ; et/ou
(f) $R^x$ et $R^y$ représentent indépendamment hydrogène ou alkyle en $C_{1-6}$ (tel que méthyle ou éthyle), tel que dans lequel $R^x$ et $R^y$ représentent indépendamment alkyle en $C_{1-6}$ (tel que méthyle), en particulier dans lequel $R^x$ et $R^y$ représentent tous deux alkyle en $C_{1-6}$ (tel que méthyle).

**11.** Composé selon la revendication 1 qui est un composé de formule (I)a :

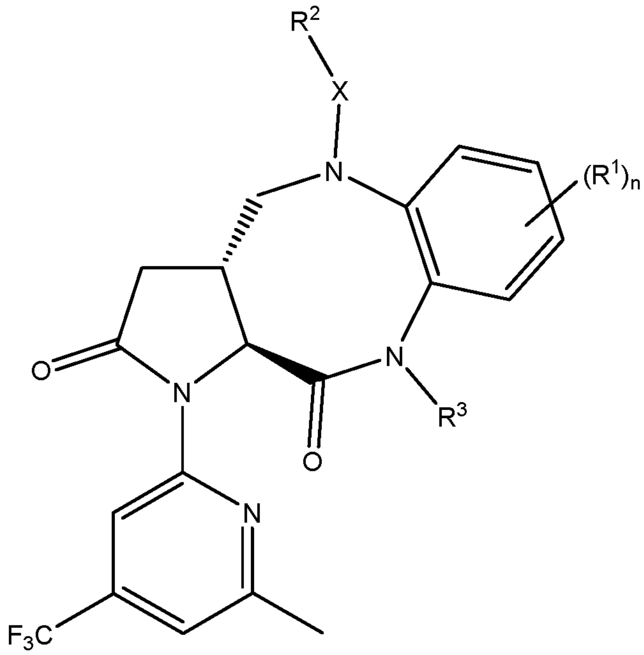

(I)a

ou une forme tautomère ou stéréochimiquement isomérique, un sel ou un solvate pharmaceutiquement acceptable de celui-ci, dans lequel n, $R^1$, X, $R^2$ et $R^3$ sont tels que définis dans la revendication 1.

**12.** Composé selon la revendication 1, dans lequel le composé est la base libre d'un composé des exemples 1 à 332 :

| Numéro de l'exemple | Nom |
|---|---|
| 1 | (3a*R*,11a*S*)-5-(2-(4-(1-acryloylazétidin-3-yl)pipérazin-1-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 2 | (3a*R*,11a*S*)-5-(2-((*R*)-1-acryloyltétrahydro-1'*H*-spiro[azétidin-3,4'-pyrrolo[1,2-a]pyrazin]-2'(3'*H*)-yl)éthyl)-6-fluoro-10-méthyl-1-(6-méthyl-4-(trifluorométhyl)pyridin-2-yl)-1,3a,4,5,10,11a-hexahydro-2H-benzo[b]pyrrolo[2,3-*f*][1,4]diazocin-2,11(3*H*)-dione |
| 3 | (3a*R*,11a*S*)-5-(2-(4-(1-acryloylazétidin-3-yl)-1,4-diazépan-1-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 4 | (3a*R*,11a*S*)-5-(2-(2-acryloyl-5-oxa-2,8-diazaspiro[3.5]nonan-8-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 5 | (3a*R*,11a*S*)-5-(2-(7-acryloyl-9-oxa-3,7-diazabicyclo[3.3.1]nonan-3-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 6 | (3a*R*,11a*S*)-5-(2-((*S*)-2-acryloyl-6-(méthoxyméthyl)-2,5,8-triazaspiro[3.5]nonan-8-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 7 | (3a*R*,11a*S*)-5-(2-((7'*R*/*S*,9a'*S*)-1-acryloyl-7'-méthyltétrahydro-1'*H*-spiro[azétidin-3,6'-pyrazino[2,1-*c*][1,4]oxazin]-8'(7'*H*)-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 8 | (3a*R*,11a*S*)-5-(2-((7'*S*/*R*,9a'*S*)-1-acryloyl-7'-méthyltétrahydro-1'*H*-spiro[azétidin-3,6'-pyrazino[2,1-*c*][1,4]oxazin]-8'(7'*H*)-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 9 | (3a*R*,11a*S*)-5-(2-((7*S*,9a*S*/*R*)-8-acryloyl-7-méthyloctahydro-2*H*-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 10 | (3a*R*,11a*S*)-5-(2-((7*S*,9a*R*/*S*)-8-acryloyl-7-méthyloctahydro-2*H*-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 11 | (3a*R*,11a*S*)-5-(2-((*rel-trans*)-3-((1-acryloylazétidin-3-yl)oxy)-4-hydroxypyrrolidin-1-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 12 | (3a*R*,11a*S*)-5-(2-(4-(1-acryloylazétidin-3-yl)pipérazin-1-yl)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 13 | (3a*R*,11a*S*)-5-(2-((*R*)-2-acryloyl-6-méthyl-2,5,8-triazaspiro[3.5]nonan-8-yl)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 14 | (3a*R*,11a*S*)-5-(2-(8-acryloyloctahydro-2*H*-pyrazino[1,2-*a*]pyrazin-2-yl)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 15 | (3a*R*,11a*S*)-5-(2-(4-(1-acryloylazétidin-3-yl)pipérazin-1-yl)éthyl)-8-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 16 | (3a*R*,11a*S*)-5-(2-(4-(1-acryloylazétidin-3-yl)pipérazin-1-yl)éthyl)-6,8-difluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 17 | (3a*R*,11a*S*)-5-(2-(4-(1-acryloylazétidin-3-yl)pipérazin-1-yl)éthyl)-6-chloro-8-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 18 | (3a*R*,11a*S*)-5-(2-(4-(1-acryloylazétidin-3-yl)pipérazin-1-yl)éthyl)-8-fluoro-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 19 | (3a*R*,11a*S*)-5-(2-(4-(1-acryloylazétidin-3-yl)pipérazin-1-yl)éthyl)-6-cyclopropyl-8-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 20 | (3a*R*,11a*S*)-5-(2-(4-(1-acryloylazétidin-3-yl)pipérazin-1-yl)éthyl)-6-chloro-8,9-difluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 21a | (3a*R*,11a*S*)-5-(2-((*S*/*R*)-1-acryloyl-1,6-diazaspiro[3.4]octan-6-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 21b | (3a*R*,11a*S*)-5-(2-((*R*/*S*)-1-acryloyl-1,6-diazaspiro[3.4]octan-6-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 22a | (3a*R*,11a*S*)-5-(2-((4a*S*/*R*,7a*S*/*R*)-4-(1-acryloylazétidin-3-yl)hexahydropyrrolo[3,4-*b*][1,4]oxazin-6(2*H*)-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 22b | (3a*R*,11a*S*)-5-(2-((4a*R*/*S*,7a*R*/*S*)-4-(1-acryloylazétidin-3-yl)hexahydropyrrolo[3,4-*b*][1,4]oxazin-6(2*H*)-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 23a | (3a*R*,11a*S*)-5-(2-((*S*/*R*)-8-acryloyloctahydro-2*H*-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 23b | (3a*R*,11a*S*)-5-(2-((*R*/*S*)-8-acryloyloctahydro-2*H*-pyrazino[1,2-a]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 24a | (3a*R*,11a*S*)-5-(2-((*R*/*S*)-1-acryloyltétrahydro-1'*H*-spiro[azétidin-3,6'-pyrazino[2,1-*c*][1,4]oxazin]-8'(7'*H*)-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 24b | (3aR,11aS)-5-(2-((*S*/R)-1-acryloyltétrahydro-1'*H*-spiro[azétidin-3,6'-pyrazino[2,1-*c*][1,4]oxazin]-8'(7'*H*)-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 25a | (3a*R*,11a*S*)-5-(2-((3a*S*/*R*,7a*S*/*R*)-2-acryloyloctahydro-5*H*-pyrrolo[3,4-*c*]pyridin-5-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 25b | (3a*R*,11a*S*)-5-(2-((3a*R*/*S*,7a*R*/*S*)-2-acryloyloctahydro-5*H*-pyrrolo[3,4-*c*]pyridin-5-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 26a | (3a*R*,11a*S*)-5-(2-((*R*/*S*)-1-acryloyltétrahydro-1'*H*-spiro[azétidin-3,6'-pyrazino[2,1-*c*][1,4]oxazin]-8'(7'*H*)-yl)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 26b | (3aR,11aS)-5-(2-((*S*/*R*)-1-acryloyltétrahydro-1'*H*-spiro[azétidin-3,6'-pyrazino[2,1-*c*][1,4]oxazin]-8'(7'*H*)-yl)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 27 | Chlorhydrate de (3a*R*,11a*S*)-5-(2-(2-((*E*)-4-(diméthylamino)but-2-énoyl)-5-oxa-2,8-diazaspiro[3.5]nonan-8-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 28 | (3a*R*,11a*S*)-5-(2-((*R*)-4-(1-acryloylazétidin-3-yl)-3-(fluorométhyl)pipérazin-1-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 29 | (3a*R*,11a*S*)-5-(2-((*S*)-4-(1-acryloylazétidin-3-yl)-6-fluoro-1,4-diazépan-1-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 30 | (3a*R*,11a*S*)-5-(2-(2-acryloyl-2,5,8-triazaspiro[3.5]nonan-8-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 31 | (3a*R*,11a*S*)-5-(2-((*R*)-2-acryloyl-6-méthyl-2,5,8-triazaspiro[3.5]nonan-8-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 32 | (3a*R*,11a*S*)-5-(2-((*S*)-2-acryloyl-6-méthyl-2,5,8-triazaspiro[3.5]nonan-8-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 33 | (3a*R*,11a*S*)-5-(2-(2-acryloyl-2,5,8-triazaspiro[3.5]nonan-8-yl)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 34a | (3a*R*,11a*S*)-5-(2-((*S*/*R*)-2-acryloyl-9-méthyl-2,5,8-triazaspiro[3.5]nonan-8-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 34b | (3a*R*,11a*S*)-5-(2-((*R*/*S*)-2-acryloyl-9-méthyl-2,5,8-triazaspiro[3.5]nonan-8-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 35 | (3a*R*,11a*S*)-5-(2-(2-acryloyl-5-éthyl-2,5,8-triazaspiro[3.5]nonan-8-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 36 | (3a*R*,11a*S*)-5-(2-(5-acétyl-2-acryloyl-2,5,8-triazaspiro[3.5]nonan-8-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 37a | (3a*R*,11a*S*)-5-(2-((6*R*,9*S*/*R*)-2-acryloyl-6,9-diméthyl-2,5,8-triazaspiro[3.5]nonan-8-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 37b | (3a*R*,11a*S*)-5-(2-((6*R*,9*R*/*S*)-2-acryloyl-6,9-diméthyl-2,5,8-triazaspiro[3.5]nonan-8-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 38 | (3a*R*,11a*S*)-5-((1-(1-acryloylazétidin-3-yl)-1*H*-imidazol-4-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 39 | (3a*R*,11a*S*)-5-((1-(1-acryloylazétidin-3-yl)-1H-imidazol-4-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 40 | (3a*R*,11a*S*)-5-((1-(1-acryloylazétidin-3-yl)-1*H*-imidazol-4-yl)méthyl)-8-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 41 | (3a*R*,11a*S*)-5-((1-(1-acryloylazétidin-3-yl)-1*H*-pyrazol-3-yl)méthyl)-8-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 42 | (3a*R*,11a*S*)-5-((1-(1-acryloylazétidin-3-yl)-1H-1,2,4-triazol-3-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 43 | (3a*R*,11a*S*)-5-(3-((1-((*E*)-4-(diméthylamino)but-2-énoyl)azétidin-3-yl)oxy)propyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 44 | (3a*R*,11a*S*)-6-chloro-5-(3-((1-((*E*)-4-(diméthylamino)but-2-énoyl)azétidin-3-yl)oxy)propyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 45 | (3a*R*,11a*S*)-5-(2-((*S*)-8-acryloyl-1-oxooctahydro-2H-pyrazino[1,2-a]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 46 | (3a*R*,11a*S*)-5-(2-((*R*)-8-acryloyl-1-oxooctahydro-2*H*-pyrazino[1,2-a]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 47 | (3a*R*,11a*S*)-5-(2-((*S*)-8-acryloyl-3-oxooctahydro-2*H*-pyrazino[1,2-a]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 48 | (3a*R*,11a*S*)-5-(2-((*R*)-8-acryloyl-3-oxooctahydro-2*H*-pyrazino[1,2-a]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 49 | (3a*R*,11a*S*)-5-(2-(5-acryloyl-4,5,6,7-tétrahydro-1*H*-imidazo[4,5-*c*]pyridin-1-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 50 | (3a*R*,11a*S*)-5-(2-(5-acryloyl-4,5,6,7-tétrahydro-3*H*-imidazo[4,5-c]pyridin-3-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 51 | (3a*R*,11a*S*)-5-(2-(4-(1-acryloylazétidin-3-yl)pipérazin-1-yl)-2-oxoéthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 52 | (3a*R*,11a*S*)-5-(2-((*R*)-2-acryloyl-6-méthyl-2,5,8-triazaspiro[3.5]nonan-8-yl)-2-oxoéthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 53 | (3a*R*,11a*S*)-5-(2-(4-acryloylpipérazin-1-yl)pyridin-4-yl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 54 | (3a*R*,11a*S*)-5-(3-(4-acryloylpipérazin-1-yl)phényl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 55 | (3a*R*,11a*S*)-5-(6-(4-acryloylpipérazin-1-yl)pyridin-2-yl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 56 | (3a*R*,11a*S*)-5-(6-(4-acryloylpipérazin-1-yl)pyridin-2-yl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 57 | (3a*R*,11a*S*)-5-(6-((1-acryloylazétidin-3-yl)oxy)pyridin-2-yl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 58 | (3a*R*,11a*S*)-5-(2-((7*R*/*S*,9a*R*/*S*)-8-acryloyl-7-méthyl-6-oxooctahydro-2*H*-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 59 | (3a*R*,11a*S*)-5-(2-((7*S*/*R*,9a*S*/*R*)-8-acryloyl-7-méthyl-6-oxooctahydro-2*H*-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 60 | (3a*R*,11a*S*)-5-(2-((7*R*/*S*,9a*S*/*R*)-8-acryloyl-7-méthyl-6-oxooctahydro-2*H*-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2H-benzo[*b*]pyrrolo[2,3-*f*][1,4]diazocin-2,11(3*H*)-dione |

(continued)

| Numéro de l'exemple | Nom |
|---|---|
| 61 | (3a*R*,11a*S*)-5-(2-((7*S*/*R*,9a*R*/*S*)-8-acryloyl-7-méthyl-6-oxooctahydro-2*H*-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 62a | (3a*R*,11a*S*)-5-(2-((*R*/*S*)-8-acryloyl-6-oxooctahydro-2*H*-pyrazino[1,2-a]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 62b | (3a*R*,11a*S*)-5-(2-((*S*/*R*)-8-acryloyl-6-oxooctahydro-2*H*-pyrazino[1,2-a]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 63a | (3a*R*,11a*S*)-5-(2-((6*R*/*S*,9a*S*/*R*)-8-acryloyl-6-méthyloctahydro-2H-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 63b | (3a*R*,11a*S*)-5-(2-((6*S*/*R*,9a*R*/*S*)-8-acryloyl-6-méthyloctahydro-2*H*-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 64a | (3a*R*,11a*S*)-5-(2-((6*R*/*S*,9a*R*/*S*)-8-acryloyl-6-méthyloctahydro-2*H*-pyrazino[1,2-a]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 64b | (3a*R*,11a*S*)-5-(2-((6*S*/*R*,9a*S*/*R*)-8-acryloyl-6-méthyloctahydro-2*H*-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 65 | *N*-(2-((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5H-benzo[b]pyrrolo[2,3-f][1,4]diazocin-5-yl) éthyl)-1-(2-((diméthylamino)méthyl)acryloyl)-*N*-méthylazétidin-3-carboxamide |
| 66 | (3a*R*,11a*S*)-5-(2-(((3*R*,4*S*)-1-acryloyl-3-fluoropipéridin-4-yl)amino)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 67 | (3a*R*,11a*S*)-5-(2-(((3*R*,4*S*)-1-acryloyl-3-fluoropipéridin-4-yl)(méthyl)amino)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 68 | (3a*R*,11a*S*)-5-((*S*/*R*)-3-((1-acryloylazétidin-3-yl)(méthyl)amino)-2-hydroxypropyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 69 | (3a*R*,11a*S*)-5-((*R*/*S*)-3-((1-acryloylazétidin-3-yl)(méthyl)amino)-2-hydroxypropyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 70 | (3a*R*,11a*S*)-5-(3-((1-acryloylazétidin-3-yl)amino)propyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3H)-dione |
| 71 | (3a*R*,11a*S*)-5-(3-((1-acryloylazétidin-3-yl)(méthyl)amino)propyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3H)-dione |
| 72 | (3a*R*,11a*S*)-5-(2-(4-(1-acryloyl-3-méthylazétidin-3-yl)pipérazin-1-yl)éthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 73a | (3a*R*,11a*S*)-6-chloro-5-(((*R*/*S*)-7-(2-chloroacétyl)-6-méthyl-5,6,7,8-tétrahydroimidazo[1,2-*a*]pyrazin-2-yl)méthyl)-10-méthyl-1-(6-méthyl-4-(trifluorométhyl)pyridin-2-yl)-1,3a,4,5,10,11a-hexahydro-2H-benzo[*b*]pyrrolo[2,3-*f*][1,4]diazocin-2,11(3*H*)-dione |

(continued)

| Numéro de l'exemple | Nom |
|---|---|
| 73b | (3a*R*,11a*S*)-6-chloro-5-(((*S*/*R*)-7-(2-chloroacétyl)-6-méthyl-5,6,7,8-tétrahydroimidazo[1,2-*a*]pyrazin-2-yl)méthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 74 | (3a*R*,11a*S*)-5-((7-acryloyl-3-méthyl-5,6,7,8-tétrahydroimidazo[1,2-*a*]pyrazin-2-yl)méthyl)-6,10-diméthyl-1-(6-méthyl-4-(trifluorométhyl)pyridin-2-yl)-1,3a,4,5,10,11a-hexahydro-2H-benzo[*b*]pyrrolo[2,3-*f*][1,4]diazocin-2,11(3*H*)-dione |
| 75 | (3a*R*,11a*S*)-5-(2-(4-(1-acryloylazétidin-3-yl)pipérazin-1-yl)-2-oxoéthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 76a | (3a*R*,11a*S*)-5-(2-((*R*/*S*)-8-acryloyloctahydro-2*H*-pyrazino[1,2-*a*]pyrazin-2-yl)-2-oxoéthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 76b | (3a*R*,11a*S*)-5-(2-((*S*/*R*)-8-acryloyloctahydro-2*H*-pyrazino[1,2-*a*]pyrazin-2-yl)-2-oxoéthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 77 | (3a*R*,11a*S*)-5-(3-((1-(2-((diméthylamino)méthyl)acryloyl)azétidin-3-yl)oxy)propyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 78 | acétate de 2-(3-(3-((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5H-benzo[b]pyrrolo[2,3-*f*][1,4]diazocin-5-yl)propoxy)azétidin-1-carbonyl)allyle |
| 79 | (3a*R*,11a*S*)-5-(3-((1-((*Z*)-2-chloro-4-(diméthylamino)but-2-énoyl)azétidin-3-yl)oxy)propyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 80 | (3a*R*,11a*S*)-5-(3-((1-((*E*)-2-((diméthylamino)méthyl)but-2-énoyl)azétidin-3-yl)oxy)propyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 81 | (3a*R*,11a*S*)-5-(3-((1-(2-((diméthylamino)méthyl)acryloyl)azétidin-3-yl)oxy)propyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 82 | (3a*R*,11a*S*)-6,10-diméthyl-1-(6-méthyl-4-(trifluorométhyl)pyridin-2-yl)-5-(3-((1-(1,2,5,6-tétrahydropyridin-3-carbonyl)azétidin-3-yl)oxy)propyl)-1,3a,4,5,10,11a-hexahydro-2*H*-benzo[*b*]pyrrolo[2,3-*f*][1,4]diazocin-2,11(3*H*)-dione |
| 83 | (*E*)-*N*-(5-((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-f][1,4]diazocin-5-yl)pentyl)-4-(diméthylamino)but-2-énamide |
| 84 | *N*-(5-((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)pentyl)-2-((diméthylamino)méthyl)acrylamide |
| 85 | *N*-(2-(2-((3a*R*,11a*S*)-6,10-diméthy1-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)éthoxy)éthyl)-2-((diméthylamino)méthyl)acrylamide |
| 86 | (3a*R*,11a*S*)-6-chloro-5-(3-(4-(2-((diméthylamino)méthyl)acryloyl)pipérazin-1-yl)-3-oxopropyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 87 | *N*-(4-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)phényl)acrylamide |

(continued)

| Numéro de l'exemple | Nom |
|---|---|
| 88 | *N*-(4-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)phényl)-1,2,5,6-tétrahydropyridin-3-carboxamide |
| 89 | (*E*)-*N*-(4-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)phényl)-2-((diméthylamino)méthyl)but-2-énamide |
| 90 | *N*-(4-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-2-méthoxyphényl)acrylamide |
| 91 | *N*-(4-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-2-((2-(diméthylamino)éthyl)(méthyl)amino)phényl)acry lamide |
| 92 | (3a*R*,11a*S*)-5-((1-(1-acryloylazétidin-3-yl)pipéridin-4-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 93 | (3a*R*,11a*S*)-5-((1-(1-acryloylazétidin-3-yl)pipéridin-4-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 94 | (3a*R*,11a*S*)-5-((1-(1-acryloylazétidin-3-yl)-4-fluoropipéridin-4-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 95a | (3a*R*,11a*S*)-5-((1-((*S*/*R*)-1-acryloylpyrrolidin-3-yl)pipéridin-4-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 95b | (3a*R*,11a*S*)-5-((1-((R/S)-1-acryloylpyrrolidin-3-yl)pipéridin-4-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 96 | (3a*R*,11a*S*)-5-((1-(1-acryloylazétidin-3-yl)-3-hydroxypipéridin-4-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 97 | (3a*R*,11a*S*)-5-(2-(4-(1-acryloylazétidin-3-yl)pipérazin-1-yl)propyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 98 | (3a*R*,11a*S*)-5-((5-(1-acryloylazétidin-3-yl)-1*H*-1,2,4-triazol-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 99 | (3a*R*,11a*S*)-5-((3-(1-acryloylazétidin-3-yl)-1-méthyl-1*H*-1,2,4-triazol-5-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 100 | (3a*R*,11a*S*)-5-((5-(1-acryloylazétidin-3-yl)-1-méthyl-1*H*-1,2,4-triazol-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 101 | (3a*R*,11a*S*)-5-((5-(1-acryloylazétidin-3-yl)-1,2,4-oxadiazol-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 102 | (3a*R*,11a*S*)-5-((3-(1-acryloylazétidin-3-yl)-1,2,4-oxadiazol-5-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 103 | (3a*R*,11a*S*)-5-((5-(1-acryloylazétidin-3-yl)-1,3,4-oxadiazol-2-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 104 | (3a*R*,11a*S*)-5-(2-(2-acryloyl-6-oxo-2,5,8-triazaspiro[3.5]nonan-8-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 105 | (3a*R*,11a*S*)-5-(2-(1-acryloyl-6'*H*-spiro[azétidin-3,5'-imidazo[1,2-*a*]pyrazin]-7'(8'*H*)-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 106 | (3a*R*,11a*S*)-5-(2-(1-acryloylspiro[azétidin-3,3'-imidazo[1,2-*a*]imidazol]-1'(2'*H*)-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 107 | (3a*R*,11a*S*)-5-(2-((3*R*/*S*,9a*S*/*R*)-8-acryloyl-3-méthyl-4-oxooctahydro-2H-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 108 | (3a*R*,11a*S*)-5-(2-((3*S*/*R*,9a*R*/*S*)-8-acryloyl-3-méthyl-4-oxooctahydro-2H-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 109 | (3a*R*,11a*S*)-5-(2-((3*R*/*S*,9a*R*/*S*)-8-acryloyl-3-méthyl-4-oxooctahydro-2H-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 110 | (3a*R*,11a*S*)-5-(2-((3*S*/*R*,9a*S*/*R*)-8-acryloyl-3-méthyl-4-oxooctahydro-2*H*-pyrazino[1,2-*a*]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 111 | (3a*R*,11a*S*)-5-(2-(2-acryloyl-5-méthyl-6-oxo-2,5,8-triazaspiro[3.5]nonan-8-yl)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 112 | (3a*R*,11a*S*)-5-(2-(4-(1-acryloylazétidin-3-yl)-3-oxopipérazin-1-yl)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 113 | (3a*R*,11a*S*)-5-(2-(((*rel-trans*)-1-acryloyl-4-hydroxypipéridin-3-yl)(méthyl)amino)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 114 | (3a*R*,11a*S*)-5-(2-(2-acryloyl-2,5,8-triazaspiro[3.5]nonan-8-yl)éthyl)-6,10-diméthyl-1-(6-méthyl-4-(trifluorométhyl)pyridin-2-yl)-1,3a,4,5,10,11a-hexahydro-2H-benzo[*b*]pyrrolo[2,3-*f*][1,4]diazocin-2,11(3*H*)-dione |
| 115 | (3a*R*,11a*S*)-5-(2-(4-(1-acryloylazétidin-3-yl)-pipérazin-1-yl)éthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 116a | (3a*R*,11a*S*)-5-(2-((*R*/*S*)-8-acryloyl-4-oxooctahydro-2*H*-pyrazino[1,2-a]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 116b | (3a*R*,11a*S*)-5-(2-((*S*/*R*)-8-acryloyl-4-oxooctahydro-2*H*-pyrazino[1,2-a]pyrazin-2-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 117a | (*R*/*S*)-2-acryloyl-6-(2-((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)éthyl)-2,6-diazaspiro[3.4]octan-8-carbonitrile |

(continued)

| Numéro de l'exemple | Nom |
|---|---|
| 117b | (*S*/*R*)-2-acryloyl-6-(2-((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)éthyl)-2,6-diazaspiro[3.4]octan-8-carbonitrile |
| 118a | (*R*/*S*)-2-acryloyl-6-(2-((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl) éthyl)-2,6-diazaspiro[3.4]octan-8-carbonitrile |
| 118b | (*S*/*R*)-2-acryloyl-6-(2-((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5H-benzo[*b*]pyrrolo[2,3-*f*][1,4]diazocin-5-yl) éthyl)-2,6-diazaspiro[3.4]octan-8-carbonitrile |
| 119a | (*R*/*S*)-2-acryloyl-6-(2-((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl) éthyl)-8-méthyl-2,6-diazaspiro[3.4]octan-8-carbonitrile |
| 119b | (*S*/*R*)-2-acryloyl-6-(2-((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5H-benzo[*b*]pyrrolo[2,3-*f*][1,4]diazocin-5-yl) éthyl)-8-méthyl-2,6-diazaspiro[3.4]octan-8-carbonitrile |
| 120 | (3a*R*,11a*S*)-5-((1-(1-acryloylazétidin-3-yl)-1*H*-pyrazol-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 121 | (3a*R*,11a*S*)-5-((1-(1-acryloylazétidin-3-yl)-1*H*-pyrazol-4-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 122 | (3a*R*,11a*S*)-5-((2-(1-acryloylazétidin-3-yl)-1*H*-imidazol-4-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 123 | (3a*R*,11a*S*)-5-((1-(1-acryloylazétidin-3-yl)-1*H*-1,2,4-triazol-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 124 | (3a*R*,11a*S*)-5-((2-(4-acryloylpipérazin-1-yl)oxazol-4-yl)méthyl)-6-fluoro-10-méthyl-1-(6-méthyl-4-(trifluorométhyl)pyridin-2-yl)-1,3a,4,5,10,11a-hexahydro-2H-benzo[*b*]pyrrolo[2,3-*f*][1,4] diazocin-2,11(3*H*)-dione |
| 125 | (3a*R*,11a*S*)-5-((2-(4-acryloylpipérazin-1-yl)-1*H*-imidazol-4-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 126 | (3a*R*,11a*S*)-5-((1-(1-acryloylpipéridin-4-yl)-1*H*-1,2,4-triazol-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 127 | (3a*R*,11a*S*)-5-(3-(1-acryloylazétidin-3-yl)benzyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 128 | (3a*R*,11a*S*)-5-((6-(1-acryloylazétidin-3-yl)pyridin-2-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4] diazocin-2,11(3*H*)-dione |
| 129 | (3a*R*,11a*S*)-5-((2-(1-acryloylazétidin-3-yl)pyrimidin-4-yl)méthyl)-6-fluoro-10-méthyl-1-(6-méthyl-4-(trifluorométhyl)pyridin-2-yl)-1,3a,4,5,10,11a-hexahydro-2H-benzo[*b*]pyrrolo[2,3-*f*][1,4] diazocin-2,11(3*H*)-dione |
| 130 | (3a*R*,11a*S*)-5-((2-acryloylisoindolin-5-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 131 | (3a*R*,11a*S*)-5-((2-acryloyl-1,2,3,4-tétrahydroisoquinoléin-6-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 132 | (3a*R*,11a*S*)-5-((7-acryloyl-5,6,7,8-tétrahydroimidazo[1,2-*a*]pyrazin-2-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 133 | (3a*R*,11a*S*)-5-((7-acryloyl-5,6,7,8-tétrahydro-[1,2,4]triazolo[1,5-*a*]pyrazin-2-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 134 | (3a*R*,11a*S*)-5-((7-acryloyl-5,6,7,8-tétrahydro-[1,2,4]triazolo[4,3-*a*]pyrazin-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 135 | (3a*R*,11a*S*)-5-((1-(1-acryloylpipéridin-4-yl)-1*H*-1,2,4-triazol-3-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 136 | (3a*R*,11a*S*)-5-((7-acryloyl-5,6,7,8-tétrahydroimidazo[1,2-*a*]pyrazin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 137 | (3a*R*,11a*S*)-5-((1-(1-acryloyl-3-méthylazétidin-3-yl)pipéridin-4-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 138 | (3a*R*,11a*S*)-5-(3-((1-acryloylazétidin-3-yl)(méthyl)amino)propyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 139 | (3a*R*,11a*S*)-5-((1-(1-acryloylazétidin-3-yl)-1*H*-1,2,4-triazol-3-yl)méthyl)-6,10-diméthyl-1-(6-méthyl-4-(trifluorométhyl)pyridin-2-yl)-1,3a,4,5,10,11a-hexahydro-2iH-benzo[*b*]pyrrolo[2,3-*f*][1,4]diazocin-2,11(3*H*)-dione |
| 140a | (3a*R*,11a*S*)-5-(((3*S*,9a*S*/*R*)-8-acryloyl-2-méthyloctahydro-2*H*-pyrazino[1,2-*a*]pyrazin-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 140b | (3a*R*,11a*S*)-5-(((3*S*,9a*R*/*S*)-8-acryloyl-2-méthyloctahydro-2*H*-pyrazino[1,2-*a*]pyrazin-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 141a | (3a*R*,11a*S*)-5-(4-((R/S)-1-acryloylpyrrolidin-2-yl)benzyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 141b | (3a*R*,11a*S*)-5-(4-((*S*/*R*)-1-acryloylpyrrolidin-2-yl)benzyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 142a | (3a*R*,11a*S*)-5-(((*R*/*S*)-7-acryloyl-8-méthyl-5,6,7,8-tétrahydroimidazo[1,2-*a*]pyrazin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 142b | (3a*R*,11a*S*)-5-(((*S*/*R*)-7-acryloyl-8-méthyl-5,6,7,8-tétrahydroimidazo[1,2-*a*]pyrazin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 143a | (3a*R*,11a*S*)-5-(((*R*/*S*)-7-acryloyl-6-méthyl-5,6,7,8-tétrahydroimidazo[1,2-*a*]pyrazin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 143b | (3a*R*,11a*S*)-5-(((*S*/*R*)-7-acryloyl-6-méthyl-5,6,7,8-tétrahydroimidazo[1,2-a]pyrazin-2-yl)méthyl)-6-chloro-10-méthyl-1-(6-méthyl-4-(trifluorométhyl)pyridin-2-yl)-1,3a,4,5,10,11a-hexahydro-2H-benzo[*b*]pyrrolo[2,3-*f*][1,4]diazocin-2,11(3*H*)-dione |
| 144a | (3a*R*,11a*S*)-5-(((*R*/*S*)-7-acryloyl-6-méthyl-5,6,7,8-tétrahydroimidazo[1,2-*a*]pyrazin-2-yl)méthyl)-6-chloro-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 144b | (3a*R*,11a*S*)-5-(((*S*/*R*)-7-acryloyl-6-méthyl-5,6,7,8-tétrahydroimidazo[1,2-*a*]pyrazin-2-yl)méthyl)-6-chloro-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 145 | (3a*R*,11a*S*)-5-((1-(1-((*E*)-4-(diméthylamino)but-2-énoyl)azétidin-3-yl)-1*H*-pyrazol-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 146 | (3a*R*,11a*S*)-5-(4-(1-((E)-4-(diméthylamino)but-2-énoyl)azétidin-3-yl)benzyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 147 | (3a*R*,11a*S*)-5-(3-(1-((*E*)-4-(diméthylamino)but-2-énoyl)azétidin-3-yl)benzyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 148 | (3a*R*,11a*S*)-5-((2-((*E*)-4-(diméthylamino)but-2-énoyl)isoindolin-5-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 149 | (3a*R*,11a*S*)-5-((2-((*E*)-4-(diméthylamino)but-2-énoyl)-1,2,3,4-tétrahydroisoquinoléin-6-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 150 | (3a*R*,11a*S*)-5-(3-((1-(2-((diméthylamino)méthyl)acryloyl)azétidin-3-yl)oxy)propyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 151 | (3a*R*,11a*S*)-5-(4-(1-(2-((diméthylamino)méthyl)acryloyl)azétidin-3-yl)benzyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 152 | (3a*R*,11a*S*)-5-(3-(1-(2-((diméthylamino)méthyl)acryloyl)azétidin-3-yl)benzyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 153 | (3a*R*,11a*S*)-5-((2-(2-((diméthylamino)méthyl)acryloyl)isoindolin-5-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 154 | (3a*R*,11a*S*)-5-((2-(2-((diméthylamino)méthyl)acryloyl)-1,2,3,4-tétrahydroisoquinoléin-6-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 155 | (3a*R*,11a*S*)-6-chloro-5-((4-(1-((*E*)-4-(diméthylamino)but-2-énoyl)azétidin-3-yl)cyclohexyl)méthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 156 | (3a*R*,11a*S*)-6-chloro-5-(3-((1-(2-((diméthylamino)méthyl)acryloyl)azétidin-3-yl)oxy)propyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 157 | (3a*R*,11a*S*)-6-chloro-5-(2-((1-(2-((diméthylamino)méthyl)acryloyl)pipéridin-4-yl)oxy)éthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 158 | (3a*R*,11a*S*)-6-chloro-5-(3-(1-(2-((diméthylamino)méthyl)acryloyl)pipéridin-4-yl)propyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 159 | (3a*R*,11a*S*)-6-chloro-5-((7-(2-((diméthylamino)méthyl)acryloyl)-5,6,7,8-tétrahydroimidazo[1,2-*a*]pyrazin-2-yl)méthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 160 | (3a*R*,11a*S*)-6-chloro-5-((4-(1-(2-((diméthylamino)méthyl)acryloyl)azétidin-3-yl)cyclohexyl)méthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 161 | (3a*R*,11a*S*)-6-chloro-5-(4-(1-(2-((diméthylamino)méthyl)acryloyl)azétidin-3-yl)butyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 162 | (3a*R*,11a*S*)-6-chloro-5-(2-((1-(2-((diméthylamino)méthyl)acryloyl)azétidin-3-yl)méthoxy)éthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 163 | (3a*R*,11a*S*)-6-chloro-5-(((2-(2-((diméthylamino)méthyl)acryloyl)isoindolin-5-yl)méthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 164 | *N*-(3-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)bicyclo[1.1.1]pentan-1-yl)-2-((diméthylamino)méthyl)acrylamide |
| 165 | (3a*R*,11a*S*)-6-chloro-*S*-(3-((1-méthacryloylazétidin-3-yl)oxy)propyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 166 | (3a*R*,11a*S*)-6-chloro-5-(3-((1-(2-((diéthylamino)méthyl)acryloyl)azétidin-3-yl)oxy)propyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 167 | (3a*R*,11a*S*)-6-chloro-10-méthyl-1-(6-méthyl-4-(trifluorométhyl)pyridin-2-yl)-5-(4-(1-(2-((4-méthylpipérazin-1-yl)méthyl)acryloyl)azétidin-3-yl)butyl)-1,3a,4,5,10,11a-hexahydro-2*H*-benzo[*b*]pyrrolo[2,3-*f*][1,4]diazocin-2,11(3*H*)-dione |
| 168 | (3a*R*,11a*S*)-6-chloro-5-(3-(1-(2-(hydroxyméthyl)acryloyl)pipéridin-4-yl)propyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 169 | (3a*R*,11a*S*)-5-(3-((1-((*E*)-4-(diméthylamino)but-2-énoyl)azétidin-3-yl)oxy)propyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 170a | (3a*R*,11a*S*)-5-(4-((*R*/*S*)-1-((*E*)-4-(diméthylamino)but-2-énoyl)pyrrolidin-2-yl)benzyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 170b | (3a*R*,11a*S*)-5-(4-((*S*/*R*)-1-((*E*)-4-(diméthylamino)but-2-énoyl)pyrrolidin-2-yl)benzyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 171a | (3a*R*,11a*S*)-5- (4-((*R*/*S*)-1-(2-((diméthylamino)méthyl)acryloyl)pyrrolidin-2-yl)benzyl)-6-fluoro-10-méthyl-1-(6-méthyl-4-(trifluorométhyl)pyridin-2-yl)-1,3a,4,5,10,11a-hexahydro-2*H*-benzo[b]pyrrolo[2,3-*f*][1,4]diazocin-2,11(3*H*)-dione |
| 171b | (3a*R*,11a*S*)-5-(4-((*S*/*R*)-1-(2-((diméthylamino)méthyl)acryloyl)pyrrolidin-2-yl)benzyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 172a | (3a*R*,11a*S*)-6-chloro-10-méthyl-1-(6-méthyl-4-(trifluorométhyl)pyridin-2-yl)-5-(((*S*/*R*)-6-méthyl-7-propioloyl-5,6,7,8-tétrahydroimidazo[1,2-*a*]pyrazin-2-yl)méthyl)-1,3a,4,5,10,11a-hexahydro-2*H*-benzo[*b*]pyrrolo[2,3-*f*][1,4]diazocin-2,11(3*H*)-dione |
| 172b | (3a*R*,11a*S*)-6-chloro-10-méthyl-1-(6-méthyl-4-(trifluorométhyl)pyridin-2-yl)-5-(((*R*/*S*)-6-méthyl-7-propioloyl-5,6,7,8-tétrahydroimidazo[1,2-*a*]pyrazin-2-yl)méthyl)-1,3a,4,5,10,11a-hexahydro-2*H*-benzo[*b*]pyrrolo[2,3-*f*][1,4]diazocin-2,11(3*H*)-dione |
| 173 | (3a*R*,11a*S*)-5-((5-acryloyl-4,5,6,7-tétrahydropyrazolo[1,5-*a*]pyrazin-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 174 | *N*-(3-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)bicyclo[1.1.1]pentan-1-yl)acrylamide |
| 175 | *N*-((3-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)bicyclo[1.1.1]pentan-1-yl)méthyl)acrylamide |
| 176 | (3a*R*,11a*S*)-5-((2-((*E*)-4-(diméthylamino)but-2-énoyl)isoindolin-5-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 177 | (*E*)-*N*-(4-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)benzyl)-4-(diméthylamino)but-2-énamide |
| 178 | (*E*)-N-(3-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)bicyclo[1.1.1]pentan-1-yl)-4-(diméthylamino)but-2-énamide |
| 179 | (*E*)-*N*-((3-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)bicyclo[1.1.1]pentan-1-yl)méthyl)-4-(diméthylamino)but-2-énamide |
| 180 | (3a*R*,11a*S*)-5-(2-(((*R*)-1-(2-((diméthylamino)méthyl)acryloyl)pyrrolidin-3-yl)méthoxy)éthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 181 | (3a*R*,11a*S*)-5-(2-(((*S*)-1-(2-((diméthylamino)méthyl)acryloyl)pyrrolidin-3-yl)méthoxy)éthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 182 | (3a*R*,11a*S*)-5-(2-((1-(2-((diméthylamino)méthyl)acryloyl)azétidin-3-yl)méthoxy)éthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 183 | (3a*R*,11a*S*)-5-(4-(1-(2-((diméthylamino)méthyl)acryloyl)azétidin-3-yl)butyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 184 | *N*-(3-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)bicyclo[1.1.1]pentan-1-yl)-2-((diméthylamino)méthyl)acrylamide |
| 185 | *N*-((3-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)bicyclo[1.1.1]pentan-1-yl)méthyl)-2-((diméthylamino)méthyl)acrylamide |
| 186 | (3a*R*,11a*S*)-5-(4-(1-(2-((diméthylamino)méthyl)acryloyl)azétidin-3-yl)benzyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 187 | (3a*R*,11a*S*)-5-(3-(1-(2-((diméthylamino)méthyl)acryloyl)azétidin-3-yl)-4-fluorobenzyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 188 | (3a*R*,11a*S*)-5-((2-(2-((diméthylamino)méthyl)acryloyl)isoindolin-5-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 189 | *N*-(4-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)benzyl)-2-((diméthylamino)méthyl)acrylamide |
| 190 | *N*-((5-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)pyridin-2-yl)méthyl)-2-((diméthylamino)méthyl)acrylamide |
| 191 | (3a*R*,11a*S*)-5-(3-((1-(2-((diméthylamino)méthyl)acryloyl)azétidin-3-yl)oxy)propyl)-6-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 192 | (3a*R*,11a*S*)-5-(4-(1-(2-((diméthylamino)méthyl)acryloyl)azétidin-3-yl)butyl)-6-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 193 | (3a*R*,11a*S*)-6,10-diméthyl-1-(6-méthyl-4-(trifluorométhyl)pyridin-2-yl)-5-(2-((1-(2-((4-méthylpipérazin-1-yl)méthyl)acryloyl)azétidin-3-yl)méthoxy)éthyl)-1,3a,4,5,10,11a-hexahydro-2*H*-benzo[*b*]pyrrolo[2,3-*f*][1,4]diazocin-2,11(3*H*)-dione |
| 194 | (3a*R*,11a*S*)-5-(3-((1-((*E*)-2-((diméthylamino)méthyl)but-2-énoyl)azétidin-3-yl)oxy)propyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 195 | (3a*R*,11a*S*)-5-(4-(1-((*E*)-2-((diméthylamino)méthyl)but-2-énoyl)azétidin-3-yl)benzyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 196 | (3a*R*,11a*S*)-6,10-diméthyl-5-(3-((1-(1-méthyl-1,2,5,6-tétrahydropyridin-3-carbonyl)azétidin-3-yl)oxy)propyl)-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 197 | (3a*R*,11a*S*)-6,10-diméthyl-5-(2-((1-(1-méthyl-1,2,5,6-tétrahydropyridin-3-carbonyl)azétidin-3-yl)méthoxy)éthyl)-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 198 | (3a*R*,11a*S*)-6,10-diméthyl-1-(6-méthyl-4-(trifluorométhyl)pyridin-2-yl)-5-((5-propioloyl-4,5,6,7-tétrahydropyrazolo[1,5-*a*]pyrazin-2-yl)méthyl)-1,3a,4,5,10,11a-hexahydro-2*H*-benzo[b]pyrrolo[2,3-*f*][1,4]diazocin-2,11(3*H*)-dione |
| 199 | (3a*R*,11a*S*)-6,10-diméthyl-1-(6-méthyl-4-(trifluorométhyl)pyridin-2-yl)-5-(((*R*/*S*)-6-méthyl-7-propioloyl-5,6,7,8-tétrahydroimidazo[1,2-*a*]pyrazin-2-yl)méthyl)-1,3a,4,5,10,11a-hexahydro-2*H*-benzo[*b*]pyrrolo[2,3-*f*][1,4]diazocin-2,11(3*H*)-dione |
| 200 | *N*-(3-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)bicyclo[1.1.1]pentan-1-yl)propiolamide |
| 201 | (3a*R*,11a*S*)-5-((7-(but-2-ynoyl)-5,6,7,8-tétrahydroimidazo[1,2-*a*]pyrazin-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 202 | (3a*R*,11a*S*)-5-((7-acryloyl-5,6,7,8-tétrahydro-[1,2,4]triazolo[1,5-*a*]pyrazin-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 203 | (3a*R*,11a*S*)-5-((7-acryloyl-5,6,7,8-tétrahydroimidazo[1,2-*a*]pyrazin-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 204 | (3a*R*,11a*S*)-5-((1-(1-acryloylpipéridin-4-yl)-1*H*-1,2,4-triazol-3-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 205 | (3a*R*,11a*S*)-5-(3-((1-acryloylazétidin-3-yl)(méthyl)amino)propyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 206a | (3a*R*,11a*S*)-5-(((*R*/*S*)-7-acryloyl-6-méthyl-5,6,7,8-tétrahydroimidazo[1,2-*a*]pyrazin-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 206b | (3a*R*,11a*S*)-5-(((*S*/*R*)-7-acryloyl-6-méthyl-5,6,7,8-tétrahydroimidazo[1,2-*a*]pyrazin-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 207 | (*E*)-*N*-(4-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)phényl)-4-(diméthylamino)but-2-énamide |
| 208 | (*E*)-*N*-(4-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-2-méthoxyphényl)-4-(diméthylamino)but-2-énamide |
| 209 | *N*-(4-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)phényl)-2-((diméthylamino)méthyl)acrylamide |
| 210 | *N*-(4-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)-2-méthoxyphényl)-2-((diméthylamino)méthyl)acrylamide |
| 211 | *N*-(4-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)phényl)-1-méthyl-1,2,5,6-tétrahydropyridin-3-carboxamide |
| 212 | (3a*R*,11a*S*)-5-((1-(1-acryloylazétidin-3-yl)pipéridin-4-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 213 | (3a*R*,11a*S*)-5-(((*S*)-1-(1-acryloylazétidin-3-yl)pyrrolidin-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 214 | (3a*R*,11a*S*)-5-(((*R*)-1-(1-acryloylazétidin-3-yl)pyrrolidin-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 215 | (3a*R*,11a*S*)-5-(2-(1-(1-acryloylazétidin-3-yl)pipéridin-4-yl)éthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 216 | (3a*R*,11a*S*)-5-(1-(1-acryloylazétidin-3-yl)pipéridin-4-yl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 217 | (3a*R*,11a*S*)-5-((1-(1-acryloylazétidin-3-yl)-4-fluoropipéridin-4-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 218 | (3a*R*,11a*S*)-5-(((*S*)-1-(1-acryloylazétidin-3-yl)pyrrolidin-3-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 219 | (3a*R*,11a*S*)-5-(((*R*)-1-(1-acryloylazétidin-3-yl)pyrrolidin-3-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 220 | (3a*R*,11a*S*)-5-(2-((*S*)-1-(1-acryloylazétidin-3-yl)pyrrolidin-3-yl)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 221 | (3a*R*,11a*S*)-5-(2-((*R*)-1-(1-acryloylazétidin-3-yl)pyrrolidin-3-yl)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 222 | (3a*R*,11a*S*)-5-((1-(1-acryloylpyrrolidin-3-yl)pipéridin-4-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 223 | (3a*R*,11a*S*)-5-(((*S*)-1-(1-acryloylazétidin-3-yl)pyrrolidin-3-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 224 | (3a*R*,11a*S*)-5-(((*R*)-1-(1-acryloylazétidin-3-yl)pyrrolidin-3-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 225a | (3a*R*,11a*S*)-5-(((*R*/*S*)-1-(1-acryloylazétidin-3-yl)pipéridin-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 225b | (3a*R*,11a*S*)-5-(((*S*/*R*)-1-(1-acryloylazétidin-3-yl)pipéridin-3-yl)méthyl)-6-fluoro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 226a | (3*S*/*R*,4*R*/*S*)-1-(1-acryloylazétidin-3-yl)-4-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)pipéridin-3-carbonitrile |
| 226b | (3*R*/*S*,4*S*/*R*)-1-(1-acryloylazétidin-3-yl)-4-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)pipéridin-3-carbonitrile |
| 227a | (3*S*/*R*,4*S*/*R*)-1-(1-acryloylazétidin-3-yl)-4-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)pipéridin-3-carbonitrile |
| 227b | (3*R*/*S*,4*R*/*S*)-1-(1-acryloylazétidin-3-yl)-4-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)pipéridin-3-carbonitrile |
| 228 | (3a*R*,11a*S*)-5-((6-acryloyl-6,7-dihydro-5*H*-pyrrolo[3,4-*b*]pyrazin-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 229 | (3a*R*,11a*S*)-5-((6-acryloyl-6,7-dihydro-5*H*-pyrrolo[3,4-*b*]pyrazin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 230 | (3a*R*,11aS)-5-(((*R*/*S*)-6-acryloyl-7-(hydroxyméthyl)-6,7-dihydro-5*H*-pyrrolo[3,4-*b*]pyridin-3-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 231 | (3a*R*,11aS)-5-(((*S*/*R*)-6-acryloyl-7-(hydroxyméthyl)-6,7-dihydro-5*H*-pyrrolo[3,4-*b*]pyridin-3-yl) méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 232 | (3a*R*,11aS)-5-(((*R*/*S*)-6-acryloyl-7-(hydroxyméthyl)-6,7-dihydro-5*H*-pyrrolo[3,4-*b*]pyridin-3-yl) méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 233 | (3a*R*,11aS)-5-(((*S*/*R*)-6-acryloyl-7-(hydroxyméthyl)-6,7-dihydro-5*H*-pyrrolo[3,4-*b*]pyridin-3-yl) méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 234 | (3a*R*,11a*S*)-5-(((*S*/*R*)-6-acryloyl-7-(méthoxyméthyl)-6,7-dihydro-5*H*-pyrrolo[3,4-*b*]pyridin-3-yl) méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 235 | (3a*R*,11a*S*)-5-(((*R*/*S*)-6-acryloyl-7-(méthoxyméthyl)-6,7-dihydro-5*H*-pyrrolo[3,4-*b*]pyridin-3-yl) méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 236 | (3a*R*,11aS)-5-(((*S*/*R*)-6-acryloyl-7-(méthoxyméthyl)-6,7-dihydro-5*H*-pyrrolo[3,4-*b*]pyridin-3-yl) méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 237 | (3a*R*,11aS)-5-(((*R*/*S*)-6-acryloyl-7-(méthoxyméthyl)-6,7-dihydro-5*H*-pyrrolo[3,4-*b*]pyridin-3-yl) méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 238 | (3a*R*,11a*S*)-5-((6-acryloyl-5-(hydroxyméthyl)-6,7-dihydro-5H-pyrrolo[3,4-b]pyridin-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 239 | (3a*R*,11a*S*)-5-((6-acryloyl-5-(hydroxyméthyl)-6,7-dihydro-5H-pyrrolo[3,4-b]pyridin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 240 | (3a*R*,11a*S*)-5-((6-acryloyl-6,7-dihydro-5*H*-pyrrolo[3,4-*b*]pyridin-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 241 | (3a*R*,11a*S*)-5-((5-acryloyl-1-méthyl-1,4,5,6-tétrahydropyrrolo[3,4-*d*]imidazol-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 242 | (3a*R*,11a*S*)-5-((5-acryloyl-1-méthyl-1,4,5,6-tétrahydropyrrolo[3,4-*d*]imidazol-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 243a | (3a*R*,11a*S*)-5-(((S/R)-7-acryloyl-6-(difluorométhyl)-5,6,7,8-tétrahydroimidazo[1,2-*a*]pyrazin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 243b | (3a*R*,11a*S*)-5-(((*R*/*S*)-7-acryloyl-6-(difluorométhyl)-5,6,7,8-tétrahydroimidazo[1,2-*a*]pyrazin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 244 | (3a*R*,11a*S*)-5-((7-acryloyl-6-(hydroxyméthyl)-5,6,7,8-tétrahydroimidazo[1,2-*a*]pyrazin-2-yl) méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 245 | (*E*)-*N*-(4-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-2-fluorophényl)-4-(diméthylamino)but-2-énamide |

(continued)

| Numéro de l'exemple | Nom |
|---|---|
| 246 | (*E*)-*N*-(4-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-3-fluorophényl)-4-(diméthylamino)but-2-énamide |
| 247 | (*E*)-*N*-(3-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl) phényl)-4-(diméthylamino)but-2-énamide |
| 248 | *N*-(4-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)-2-cyanophényl)acrylamide |
| 249 | (*E*)-4-(4-acétylpipérazin-1-yl)-*N*-(4-(((3a*R*,11a*S*)-6,10-diméthyl-1-(6-méthyl-4-(trifluorométhyl) pyridin-2-yl)-2,11-dioxo-1,2,3,3a,4,10,11,11a-octahydro-5H-benzo[*b*]pyrrolo[2,3-*f*][1,4]diazocin-5-yl)méthyl)phényl)but-2-énamide |
| 250 | (3a*R*,11a*S*)-5-((4-acryloyl-3,4-dihydro-2*H*-benzo[*b*][1,4]oxazin-6-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dine |
| 251 | (3a*R*,11a*S*)-6-chloro-5-((4-((*E*)-4-(diméthylamino)but-2-énoyl)-3,4-dihydro-2*H*-benzo[*b*][1,4] oxazin-6-yl)méthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 252 | (*E*)-*N*-(4-((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)butyl)-4-(diméthylamino)but-2-énamide |
| 253 | (*E*)-*N*-(5-((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)-4-fluoropentyl)-4-(diméthylamino)but-2-énamide |
| 254 | (3a*R*,11a*S*)-5-(4-(1-((*Z*)-2-chloro-4-(diméthylamino)but-2-énoyl)azétidin-3-yl)butyl)-6,10-diméthyl-1-(6-méthyl-4-(trifluorométhyl)pyridin-2-yl)-1,3a,4,5,10,11a-hexahydro-2H-benzo[*b*] pyrrolo[2,3-*f*][1,4]diazocin-2,11(3*H*)-dione |
| 255 | (3a*R*,11a*S*)-5-((2-((*Z*)-2-chloro-4-(diméthylamino)but-2-énoyl)isoindolin-5-yl)méthyl)-6,10-diméthyl-1-(6-méthyl-4-(trifluorométhyl)pyridin-2-yl)-1,3a,4,5,10,11a-hexahydro-2*H*-benzo[*b*] pyrrolo[2,3-*f*][1,4]diazocin-2,11(3*H*)-dione |
| 256 | (3a*R*,11a*S*)-5-(2-(4-acryloylpipérazin-1-yl)éthyl)-6,10-diméthyl-1-(6-méthyl-4-(trifluorométhyl) pyridin-2-yl)-1,3a,4,5,10,11a-hexahydro-2*H*-benzo[*b*]pyrrolo[2,3-*f*][1,4]diazocin-2,11(3*H*)-dione |
| 257a | *N*-((*S*/*R*)-1-(2-((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl) éthyl)-3,3-difluoropipéridin-4-yl)acrylamide |
| 257b | *N*-((*R*/*S*)-1-(2-((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl) éthyl)-3,3-difluoropipéridin-4-yl)acrylamide |
| 258a | 2-((*R*/*S*)-1-acryloyl-4-(2-((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)éthyl)pipérazin-2-yl)acétonitrile |
| 258b | 2-((*S*/*R*)-1-acryloyl-4-(2-((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)éthyl)pipérazin-2-yl)acétonitrile |
| 259 | (3a*R*,11a*S*)-5-(2-(5-acryloyl-5, 6-dihydropyrrolo[3,4-*d*]imidazol-1(4*H*)-yl)éthyl)-6-chloro-10-méthyl-1-(6-méthyl-4-(trifluorométhyl)pyridin-2-yl)-1,3a,4,5,10,11a-hexahydro-2*H*-benzo[*b*] pyrrolo[2,3-*f*][1,4]diazocin-2,11(3*H*)-dione |

(continued)

| Numéro de l'exemple | Nom |
|---|---|
| 260 | (3a*R*,11a*S*)-5-(2-((*S*)-4-acryloyl-2-méthylpipérazin-1-yl)éthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 261 | (3a*R*,11a*S*)-5-(2-(3-acryloyl-3,6-diazabicyclo[3.1.1]heptan-6-yl)éthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 262 | (3a*R*,11a*S*)-5-(2-(3-acryloyl-3,8-diazabicyclo[3.2.1]octan-8-yl)éthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 263 | (3a*R*,11a*S*)-5-(2-((1*R*,4*R*)-5-acryloyl-2,5-diazabicyclo[2.2.1]heptan-2-yl)éthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 264 | (3a*R*,11a*S*)-6,10-diméthyl-1-(6-méthyl-4-(trifluorométhyl)pyridin-2-yl)-5-(2-(4-propioloylpipérazin-1-yl)éthyl)-1,3a,4,5,10,11a-hexahydro-2*H*-benzo[*b*]pyrrolo[2,3-*f*][1,4]diazocin-2,11(3*H*)-dione |
| 265a | (3a*R*,11a*S*)-5-(3-((*S*/*R*)-4-acryloyl-3-(trifluorométhyl)pipérazin-1-yl)propyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 265b | (3a*R*,11a*S*)-5-(3-((*R*/*S*)-4-acryloyl-3-(trifluorométhyl)pipérazin-1-yl)propyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 266 | (3a*R*,11a*S*)-5-(3-(4-acryloylpipérazin-1-yl)propyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 267a | 2-((*S*/*R*)-1-acryloyl-4-(3-((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)propyl)pipérazin-2-yl)acétonitrile |
| 267b | 2-((*R*/*S*)-1-acryloyl-4-(3-((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)propyl)pipérazin-2-yl)acétonitrile |
| 268 | (3a*R*,11a*S*)-5-((3-((*R*)-1-acryloylpyrrolidin-2-yl)-1,2,4-oxadiazol-5-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 269 | (3a*R*,11a*S*)-5-((3-((*S*)-1-acryloylpyrrolidin-2-yl)-1,2,4-oxadiazol-5-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 270 | (3a*R*,11a*S*)-5-((5-((*R*)-1-acryloylpyrrolidin-2-yl)-1,2,4-oxadiazol-3-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 271 | (3a*R*,11a*S*)-5-((5-((*S*)-1-acryloylpyrrolidin-2-yl)-1,2,4-oxadiazol-3-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 272 | (3a*R*,11a*S*)-5-((5-((*R*)-1-acryloylpyrrolidin-2-yl)-1,3,4-oxadiazol-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 273 | (3a*R*,11a*S*)-5-((5-((S)-1-acryloylpyrrolidin-2-yl)-1,3,4-oxadiazol-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 274 | (3a*R*,11a*S*)-5-((5-((*R*)-1-acryloylazétidin-2-yl)-1,3,4-oxadiazol-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 275 | (3a*R*,11a*S*)-5-((5-((*S*)-1-acryloylazétidin-2-yl)-1,3,4-oxadiazol-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 276 | (3aR,11aS)-5-((6-acryloyl-6,7-dihydro-5*H*-pyrrolo[3,4-*b*]pyridin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 277 | (3a*R*,11a*S*)-5-((2-acryloyl-2,3-dihydro-1H-pyrrolo[3,4-*c*]pyridin-6-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 278 | (3a*R*,11a*S*)-5-((6-acryloyl-6,7-dihydro-5*H*-pyrrolo[3,4-*d*]pyrimidin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 279 | (3a*R*,11a*S*)-5-((5-acryloyl-1-éthyl-1,4,5,6-tétrahydropyrrolo[3,4-*d*]imidazol-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 280 | (3a*R*,11a*S*)-5-((5-acryloyl-5,6-dihydro-4*H*-pyrrolo[3,4-*d*]thiazol-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 281 | (3a*R*,11a*S*)-5-((4-acryloyl-3,4-dihydro-2*H*-benzo[*b*][1,4]oxazin-7-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 282 | *N*-(5-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)pyridin-2-yl)acrylamide |
| 283 | *N*-(6-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)pyridin-3-yl)acrylamide |
| 284 | *N*-(2-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)pyrimidin-5-yl)acrylamide |
| 285 | (3a*R*,11a*S*)-5-(2-((1-acryloyl-3-fluoroazétidin-3-yl)méthoxy)éthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 286 | *N*-(5-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-2,3-dihydro-1*H*-indén-2-yl)acrylamide |
| 287a | 2-((*S*/*R*)-2-acryloyl-5-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)isoindolin-1-yl)acétonitrile |
| 287b | 2-((*R*/*S*)-2-acryloyl-5-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)isoindolin-1-yl)acétonitrile |
| 288a | 2-((*R*/*S*)-2-acryloyl-6-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)isoindolin-1-yl)acétonitrile |

(continued)

| Numéro de l'exemple | Nom |
|---|---|
| 288b | 2-((*S*/*R*)-2-acryloyl-6-(((3aR,11aS)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)isoindolin-1-yl)acétonitrile |
| 289a | 2-((*R*/*S*)-6-acryloyl-2-(((3a*R*,11aS)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-6,7-dihydro-5*H*-pyrrolo[3,4-*b*]pyridin-7-yl)acétonitrile |
| 289b | 2-((*S*/*R*)-6-acryloyl-2-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)-6,7-dihydro-5*H*-pyrrolo[3,4-*b*]pyridin-7-yl)acétonitrile |
| 290a | 2-((*S*/*R*)-6-acryloyl-2-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)-6,7-dihydro-5*H*-pyrrolo[3,4-*b*]pyridin-5-yl)acétonitrile |
| 290b | 2-((*R*/*S*)-6-acryloyl-2-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)-6,7-dihydro-5*H*-pyrrolo[3,4-b]pyridin-5-yl)acétonitrile |
| 291a | 2-((*R*/*S*)-6-acryloyl-3-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-6,7-dihydro-5*H*-pyrrolo[3,4-*b*]pyridin-5-yl)acétonitrile |
| 291b | 2-((*S*/*R*)-6-acryloyl-3-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-6,7-dihydro-5*H*-pyrrolo[3,4-b]pyridin-5-yl)acétonitrile |
| 292a | 2-((*S*/*R*)-6-acryloyl-3-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-6,7-dihydro-5*H*-pyrrolo[3,4-*b*]pyridin-7-yl)acétonitrile |
| 292b | 2-((*R*/*S*)-6-acryloyl-3-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-6,7-dihydro-5*H*-pyrrolo[3,4-*b*]pyridin-7-yl)acétonitrile |
| 293a | (3a*R*,11a*S*)-5-(((*S*/*R*)-7-acryloyl-6-(trifluorométhyl)-5,6,7,8-tétrahydroimidazo[1,2-*a*]pyrazin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 293b | (3a*R*,11a*S*)-5-(((*R*/*S*)-7-acryloyl-6-(trifluorométhyl)-5,6,7,8-tétrahydroimidazo[1,2-*a*]pyrazin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 294a | (3a*R*,11a*S*)-5-(((*R*/*S*)-5-acryloyl-6-méthyl-4,5,6,7-tétrahydropyrazolo[1,5-*a*]pyrazin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 294b | (3a*R*,11a*S*)-5-(((*S*/*R*)-5-acryloyl-6-méthyl-4,5,6,7-tétrahydropyrazolo[1,5-*a*]pyrazin-2-yl)méthyl)-6-chloro-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 295 | (*E*)-N-(5-((3aR,11aS)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)-2-fluoropentyl)-4-(diméthylamino)but-2-énamide |
| 296 | (*E*)-*N*-(5-((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)-2,2-difluoropentyl)-4-(diméthylamino)but-2-énamide |
| 297 | (3a*R*,11a*S*)-6-chloro-5-((1-(2-((diméthylamino)méthyl)acryloyl)azétidin-3-yl)méthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 298 | (3a*R*,11a*S*)-6-chloro-5-(2-(1-(2-((diméthylamino)méthyl)acryloyl)azétidin-3-yl)éthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3H)-dione |
| 299 | (3a*R*,11a*S*)-6-chloro-5-(3-(1-(2-((diméthylamino)méthyl)acryloyl)azétidin-3-yl)propyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 300 | (3a*R*,11a*S*)-6-chloro-5-(4-(1-(2-((diméthylamino)méthyl)acryloyl)azétidin-3-yl)-4-fluorobutyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 301 | (3a*R*,11a*S*)-6-chloro-5-(4-(1-(2-((diméthylamino)méthyl)acryloyl)azétidin-3-yl)-3-fluorobutyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 302 | (3a*R*,11a*S*)-6-chloro-5-(4-(1-(2-((diméthylamino)méthyl)acryloyl)azétidin-3-yl)-2-fluorobutyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 303 | (3a*R*,11a*S*)-6-chloro-5-(2-((3-fluoro-1-propioloylazétidin-3-yl)méthoxy)éthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 304 | (3a*R*,11a*S*)-6-chloro-5-(2-((1-((*E*)-4-(diméthylamino)but-2-énoyl)-3-fluoroazétidin-3-yl)méthoxy)éthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 305 | (3a*R*,11a*S*)-6-chloro-5-(2-((1-(2-((diméthylamino)méthyl)acryloyl)-3-fluoroazétidin-3-yl)méthoxy)éthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 306 | (3a*R*,11a*S*)-6-chloro-5-((7-((*E*)-4,4-difluorobut-2-énoyl)-5,6,7,8-tétrahydroimidazo[1,2-*a*]pyrazin-2-yl)méthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 307 | (3a*R*,11a*S*)-6-chloro-10-méthyl-1-(6-méthyl-4-(trifluorométhyl)pyridin-2-yl)-5-((7-((*E*)-4,4,4-trifluorobut-2-énoyl)-5,6,7,8-tétrahydroimidazo[1,2-*a*]pyrazin-2-yl)méthyl)-1,3a,4,5,10,11a-hexahydro-2*H*-benzo[*b*]pyrrolo[2,3-*f*][1,4]diazocin-2,11(3*H*)-dione |
| 308 | (3a*R*,11a*S*)-6-chloro-5-((4-((*E*)-4-(diméthylamino)but-2-énoyl)-3,4-dihydro-2*H*-benzo[*b*][1,4]oxazin-7-yl)méthyl)-10-méthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 309 | (*E*)-N-(4-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-2,6-difluorophényl)-4-(diméthylamino)but-2-énamide |
| 310a | 2-((*S*/*R*)-5-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][[1,4]diazocin-5-yl)méthyl)-2-((*E*)-4-(diméthylamino)but-2-énoyl)isoindolin-1-yl)acétonitrile |
| 310b | 2-((*R*/*S*)-5-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][[1,4]diazocin-5-yl)méthyl)-2-((*E*)-4-(diméthylamino)but-2-énoyl)isoindolin-1-yl)acétonitrile |
| 311a | 2-((*R*/*S*)-6-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-2-((*E*)-4-(diméthylamino)but-2-énoyl)isoindolin-1-yl)acétonitrile |
| 311b | 2-((*S*/*R*)-6-(((3a*R*,11a*S*)-6-chloro-10-méthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-2-((*E*)-4-(diméthylamino)but-2-énoyl)isoindolin-1-yl)acétonitrile |

(continued)

| Numéro de l'exemple | Nom |
|---|---|
| 312 | (3a*R*,11a*S*)-5-((6-acryloyl-6,7-dihydro-5*H*-pyrrolo[3,4-*b*]pyridin-3-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 313 | (3a*R*,11a*S*)-5-((2-acryloylisoindolin-5-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 314 | (3a*R*,11a*S*)-5-((5-acryloyl-1-éthyl-1,4,5,6-tétrahydropyrrolo[3,4-*d*]imidazol-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 315 | (3a*R*,11a*S*)-5-((5-acryloyl-5,6-dihydro-4*H*-pyrrolo[3,4-*d*]thiazol-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 316 | (3a*R*,11a*S*)-5-((5-acryloyl-5,6-dihydro-4*H*-pyrrolo[3,4-*d*]oxazol-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 317 | (3a*R*,11a*S*)-5-((5-acryloyl-1-méthyl-1,4,5,6-tétrahydropyrrolo[3,4-*c*]pyrazol-3-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 318a | 2-((*R*/*S*)-6-acryloyl-2-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-6,7-dihydro-5*H*-pyrrolo[3,4-*b*]pyridin-7-yl)acétonitrile |
| 318b | 2-((*S*/*R*)-6-acryloyl-2-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)-6,7-dihydro-5*H*-pyrrolo[3,4-*b*]pyridin-7-yl)acétonitrile |
| 319a | 2-((*S*/*R*)-6-acryloyl-2-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-6,7-dihydro-5*H*-pyrrolo[3,4-*b*]pyridin-5-yl)acétonitrile |
| 319b | 2-((*R*/*S*)-6-acryloyl-2-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-6,7-dihydro-5*H*-pyrrolo[3,4-b]pyridin-5-yl)acétonitrile |
| 320a | 2-((*R*/*S*)-6-acryloyl-3-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-6,7-dihydro-5*H*-pyrrolo[3,4-b]pyridin-5-yl)acétonitrile |
| 320b | 2-((*S*/*R*)-6-acryloy1-3-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-f] [1,4]diazocin-5-yl)méthyl)-6,7-dihydro-5*H*-pyrrolo[3,4-b]pyridin-5-yl)acétonitrile |
| 321a | 2-((*S*/*R*)-6-acryloyl-3-(((3aR,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-6,7-dihydro-5*H*-pyrrolo[3,4-*b*]pyridin-7-yl)acétonitrile |
| 321b | 2-((*R*/*S*)-6-acryloyl-3-(((3aR,11aS)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-6,7-dihydro-5*H*-pyrrolo[3,4-*b*]pyridin-7-yl)acétonitrile |
| 322a | (3aR,11aS)-5-(((*S*/*R*)-7-acryloyl-6-(trifluorométhyl)-5,6,7,8-tétrahydroimidazo[1,2-*a*]pyrazin-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 322b | (3a*R*,11aS)-5-(((*R*/*S*)-7-acryloyl-6-(trifluorométhyl)-5,6,7,8-tétrahydroimidazo[1,2-*a*]pyrazin-2-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |

(continued)

| Numéro de l'exemple | Nom |
|---|---|
| 323a | (3a*R*,11a*S*)-5-(4-((*R*/*S*)-4-acryloylmorpholin-3-yl)benzyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 323b | (3a*R*,11a*S*)-5-(4-((*S*/*R*)-4-acryloylmorpholin-3-yl)benzyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-f][1,4]diazocin-2,11(3*H*)-dione |
| 324a | (3a*R*,11a*S*)-5-((6-((*R*/*S*)-4-acryloylmorpholin-3-yl)pyridin-3-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 324b | (3a*R*,11a*S*)-5-((6-((*S*/*R*)-4-acryloylmorpholin-3-yl)pyridin-3-yl)méthyl)-6,10-diméthyl-1-(6-méthyl-4-(trifluorométhyl)pyridin-2-yl)-1,3a,4,5,10,11a-hexahydro-2H-benzo[*b*]pyrrolo[2,3-*f*][1,4]diazocin-2,11(3*H*)-dione |
| 325a | (3a*R*,11a*S*)-5-((1-((3*S*/*R*,4*S*/*R*)-1-acryloyl-4-fluoropyrrolidin-3-yl)-1*H*-pyrazol-4-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 325b | (3a*R*,11a*S*)-5-((1-((3*R*/*S*,4*R*/*S*)-1-acryloyl-4-fluoropyrrolidin-3-yl)-1*H*-pyrazol-4-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 325c | (3a*R*,11a*S*)-5-((1-((3*R*/*S*,4*S*/*R*)-1-acryloyl-4-fluoropyrrolidin-3-yl)-1*H*-pyrazol-4-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 325d | (3a*R*,11a*S*)-5-((1-((3*S*/*R*,4*R*/*S*)-1-acryloyl-4-fluoropyrrolidin-3-yl)-1*H*-pyrazol-4-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 326a | (3a*R*,11a*S*)-5-((1-((3*S*/*R*,4*R*/*S*)-1-acryloyl-4-fluoropyrrolidin-3-yl)-1*H*-imidazol-4-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 326b | (3a*R*,11a*S*)-5-((1-((3*R*/*S*,4*S*/*R*)-1-acryloyl-4-fluoropyrrolidin-3-yl)-1*H*-imidazol-4-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 327 | (3a*R*,11a*S*)-5-(3-(1-(2-((diméthylamino)méthyl)acryloyl)azétidin-3-yl)propyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 328 | (3a*R*,11a*S*)-5-((2-(2-((diméthylamino)méthyl)acryloyl)-2-azaspiro[3.3]heptan-6-yl)méthyl)-6,10-diméthyl-1-(6-méthyl-4-(trifluorométhyl)pyridin-2-yl)-1,3a,4,5,10,11a-hexahydro-2*H*-benzo[*b*]pyrrolo[2,3-*f*][1,4]diazocin-2,11(3*H*)-dione |
| 329a | *N*-((*R*/*S*)-2-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-5,6,7,8-tétrahydroimidazo[1,2-*a*]pyridin-6-yl)-2-((diméthylamino)méthyl)acrylamide |
| 329b | *N*-((*S*/*R*)-2-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)-5,6,7,8-tétrahydroimidazo[1,2-*a*]pyridin-6-yl)-2-((diméthylamino)méthyl)acrylamide |
| 330a | *N*-((*S*/*R*)-2-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[b]pyrrolo[2,3-*f*][1,4]diazocin-5-yl)méthyl)-5,6,7,8-tétrahydroimidazo[1,2-*a*]pyridin-7-yl)-2-((diméthylamino)méthyl)acrylamide |
| 330b | *N*-((*R*/*S*)-2-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-f][1,4]diazocin-5-yl)méthyl)-5,6,7,8-tétrahydroimidazo[1,2-*a*]pyridin-7-yl)-2-((diméthylamino)méthyl)acrylamide |

(continued)

| Numéro de l'exemple | Nom |
|---|---|
| 331 | (*E*)-*N*-((1*R*,3*r*)-3-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)cyclobutyl)-4-(diméthylamino)but-2-énamide |
| 332 | (*E*)-*N*-((1*S*,3*s*)-3-(((3a*R*,11a*S*)-6,10-diméthyl-1-(6-méthyl-4-(trifluorométhyl)pyridin-2-yl)-2,11-dioxo-1,2,3,3a,4,10,11,11a-octahydro-5*H*-benzo[*b*]pyrrolo[2,3-*f*][1,4]diazocin-5-yl)méthyl)cyclobutyl)-4-(diméthylamino)but-2-énamide |

ou un sel ou un solvate pharmaceutiquement acceptable de celui-ci.

**13.** Composition pharmaceutique comprenant un composé de formule (I) tel que défini dans l'une quelconque des revendications 1 à 12, facultativement en combinaison avec un ou plusieurs agents thérapeutiques.

**14.** Composé tel que défini dans l'une quelconque des revendications 1 à 12 pour une utilisation :

(a) en thérapie ; ou
(b) dans la prophylaxie ou le traitement du cancer.

**15.** Processus de préparation d'un composé de formule (I) selon la revendication 1 qui comprend :

(a) la déprotection d'un composé de formule (II) ;

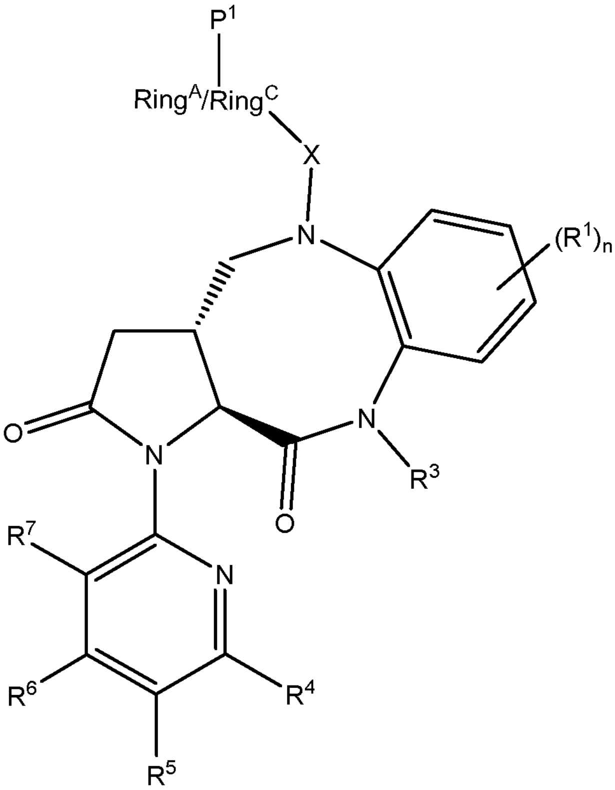

(II)

dans laquelle $R^1$, n, X, $R^3$, $R^4$, $R^5$, $R^6$, $R^7$, cycle$^A$ et cycle$^C$ sont tels que définis dans la revendication 1 et $P^1$ représente un groupe protecteur approprié, tel que Boc ;
(b) la réaction d'un composé de formule (III) :

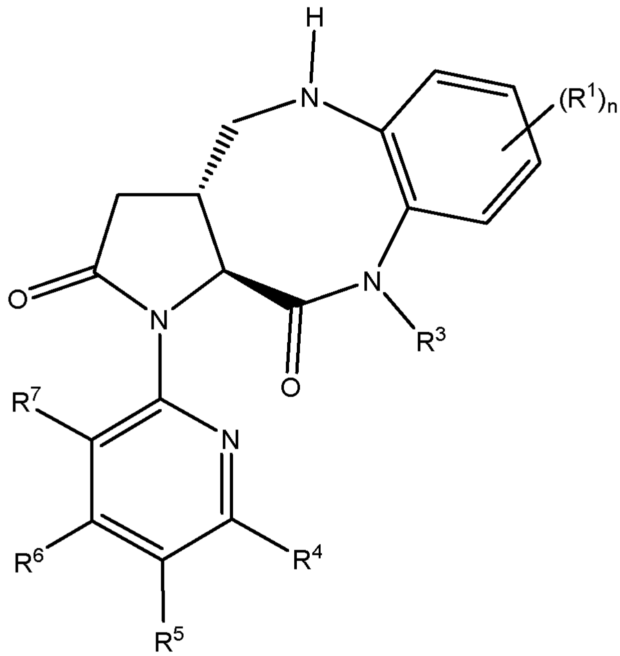

(III)

dans laquelle $R^1$, n, $R^3$, $R^4$, $R^5$, $R^6$ et $R^7$ sont tels que définis dans la revendication 1, avec un composé de formule $L^1$-X-$R^2$, dans laquelle X et $R^2$ sont tels que définis dans la revendication 1 et $L^1$ représente un groupe partant approprié tel qu'halogène (c'est-à-dire chlore) ou un groupe hydroxy ;

(c) la déprotection d'un dérivé protégé d'un composé de formule (I) ;

(d) l'interconversion d'un composé de formule (I) ou d'un dérivé protégé de celui-ci en un autre composé de formule (I) ou en un dérivé protégé de celui-ci ; et

(e) la formation facultative d'un sel pharmaceutiquement acceptable d'un composé de formule (I).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017062754 A


### Non-patent literature cited in the description


- **TRUONG et al.** *PNAS*, 2013, vol. 110 (19), 7720-7725
- **KENT et al.** *Nature Structural & Molecular Biology*, 2015, vol. 22 (3), 230-237
- **MATEOS-GOMEZ et al.** *Nature*, 2015, vol. 518 (7538), 254-257
- **WOOD** ; **DOUBLIÉ**. *DNA Repair*, 2016, vol. 44, 22-32
- **CECCALDI et al.** *Nature*, 2015, vol. 518 (7538), 258-262
- **HIGGINS et al.** *Oncotarget*, 2010, vol. 1, 175-184
- **LEMÉE et al.** *PNAS*, 2010, vol. 107 (30), 13390-13395
- **KAWAMURA et al.** *International Journal of Cancer*, 2004, vol. 109 (1), 9-16
- Pharmaceutical Salts: Properties, Selection, and Use. August 2002, 388
- **BERGE** ; **BIGHLEY** ; **MONKHOUSE**. *J. Pharm. Sci.*, 1977, vol. 66, 1-19
- **EMAMI S et al.** *BioImpacts*, 2018, vol. 8 (4), 305-320
- **JERRY MARCH**. Advanced Organic Chemistry. Wiley Interscience
- **L. W. DEADY**. *Syn. Commun*, 1977, vol. 7, 509-514
- **JERRY MARCH**. Advanced Organic Chemistry. John Wiley & Sons, 1992
- **JIRO TSUJI**. Palladium Reagents and Catalysts. Wiley
- Handbook of OrganoPalladium Chemistry for Organic Synthesis. Wiley, vol. 1
- **T. GREEN** ; **P. WUTS**. Protective Groups in Organic Synthesis. John Wiley and Sons, 2007
- Remington's Pharmaceutical Sciences. Mack Publishing Company
- **R. G. STRICKLY**. Solubilizing Excipients in oral and injectable formulations. *Pharmaceutical Research*, 2004, vol. 21 (2), 201-230
- Nanoparticle Technology for Drug Delivery. Informa Healthcare, 13 March 2006
- Nanoparticles for drug delivery are also described. *J. Control. Release*, 2003, vol. 91 (1-2), 167-172
- **SINHA et al.** *Mol. Cancer Ther.*, 01 August 2006, vol. 5, 1909
- **CHIOU** ; **RIEGELMAN**. *J. Pharm. Sci.*, 1971, vol. 60, 1281-1300
- **CHOU TC** ; **TALALAY P**. Quantitative analysis of dose-effect relationships: the combined effects of multiple drugs or enzyme inhibitors. *Adv Enzyme Regulat*, 1984, vol. 22, 27-55
- *CHEMICAL ABSTRACTS*, 81477-94-3
- *CHEMICAL ABSTRACTS*, 624-49-7
- *CHEMICAL ABSTRACTS*, 1808186-23-3
- **J. S. BANDER**. *Chem. Sci.*, 2015, vol. 6, 1537
- *CHEMICAL ABSTRACTS*, 17809-36-8
- *CHEMICAL ABSTRACTS*, 769-11-9
- *CHEMICAL ABSTRACTS*, 364-78-3
- *CHEMICAL ABSTRACTS*, 364-30-7
- *CHEMICAL ABSTRACTS*, 2613-32-3
- *CHEMICAL ABSTRACTS*, 26189-59-3
- *CHEMICAL ABSTRACTS*, 451459-17-9
- *CHEMICAL ABSTRACTS*, 1001907-44-3
- *CHEMICAL ABSTRACTS*, 454703-20-9
- *CHEMICAL ABSTRACTS*, 193269-78-2
- *CHEMICAL ABSTRACTS*, 2106-49-2
- *CHEMICAL ABSTRACTS*, 398489-26-4
- *CHEMICAL ABSTRACTS*, 31166-44-6
- *CHEMICAL ABSTRACTS*, 112275-50-0
- *CHEMICAL ABSTRACTS*, 278788-66-2
- *CHEMICAL ABSTRACTS*, 34582-32-6
- *CHEMICAL ABSTRACTS*, 14316-06-4
- *CHEMICAL ABSTRACTS*, 2491-20-5
- *CHEMICAL ABSTRACTS*, 184719-80-0
- *CHEMICAL ABSTRACTS*, 86123-95-7
- *CHEMICAL ABSTRACTS*, 126264-49-1
- *CHEMICAL ABSTRACTS*, 1187933-47-6
- *CHEMICAL ABSTRACTS*, 181955-79-3
- *CHEMICAL ABSTRACTS*, 31865-25-5
- *CHEMICAL ABSTRACTS*, 141699-55-0
- *CHEMICAL ABSTRACTS*, 138026-93-4
- *CHEMICAL ABSTRACTS*, 105258-93-3
- *CHEMICAL ABSTRACTS*, 859518-35-7

- *CHEMICAL ABSTRACTS*, 5445-17-0
- *CHEMICAL ABSTRACTS*, 40320-63-6
- *CHEMICAL ABSTRACTS*, 78818-15-2
- *CHEMICAL ABSTRACTS*, 254454-54-1
- *CHEMICAL ABSTRACTS*, 161157-50-2
- *CHEMICAL ABSTRACTS*, 3674-13-3
- *CHEMICAL ABSTRACTS*, 140-28-3
- *CHEMICAL ABSTRACTS*, 93102-05-7
- *CHEMICAL ABSTRACTS*, 1153949-11-1
- *CHEMICAL ABSTRACTS*, 17325-26-7
- *CHEMICAL ABSTRACTS*, 15366-34-4
- *CHEMICAL ABSTRACTS*, 4928-88-5
- *CHEMICAL ABSTRACTS*, 96-33-3
- *CHEMICAL ABSTRACTS*, 51105-90-9
- *CHEMICAL ABSTRACTS*, 78162-58-0
- *CHEMICAL ABSTRACTS*, 143238-38-4
- *CHEMICAL ABSTRACTS*, 301673-14-3
- *CHEMICAL ABSTRACTS*, 1064194-10-0
- *CHEMICAL ABSTRACTS*, 870987-63-6
- *CHEMICAL ABSTRACTS*, 1034901-50-2
- *CHEMICAL ABSTRACTS*, 1873-77-4
- *CHEMICAL ABSTRACTS*, 26218-75-7
- *CHEMICAL ABSTRACTS*, 1209459-78-8
- *CHEMICAL ABSTRACTS*, 59433-50-0
- *CHEMICAL ABSTRACTS*, 893566-74-0
- *CHEMICAL ABSTRACTS*, 50978-45-5
- *CHEMICAL ABSTRACTS*, 7688-25-7
- *CHEMICAL ABSTRACTS*, 201940-08-1
- *CHEMICAL ABSTRACTS*, 1053656-22-6
- *CHEMICAL ABSTRACTS*, 31954-27-5
- *CHEMICAL ABSTRACTS*, 816-27-3
- *CHEMICAL ABSTRACTS*, 1215852-11-1
- *CHEMICAL ABSTRACTS*, 1126-09-6
- *CHEMICAL ABSTRACTS*, 19838-08-5
- *CHEMICAL ABSTRACTS*, 5521-58-4
- *CHEMICAL ABSTRACTS*, 199174-24-8
- *CHEMICAL ABSTRACTS*, 138108-72-2
- *CHEMICAL ABSTRACTS*, 152537-03-6
- *CHEMICAL ABSTRACTS*, 1099-45-2
- *CHEMICAL ABSTRACTS*, 142253-56-3
- *CHEMICAL ABSTRACTS*, 676371-64-5
- *CHEMICAL ABSTRACTS*, 83249-10-9
- *CHEMICAL ABSTRACTS*, 929-06-6
- *CHEMICAL ABSTRACTS*, 2508-29-4
- *CHEMICAL ABSTRACTS*, 704-13-2
- *CHEMICAL ABSTRACTS*, 71233-25-5
- *CHEMICAL ABSTRACTS*, 89809-65-4
- *CHEMICAL ABSTRACTS*, 204688-60-8
- *CHEMICAL ABSTRACTS*, 204688-61-9
- *CHEMICAL ABSTRACTS*, 156185-63-6
- *CHEMICAL ABSTRACTS*, 13682-77-4
- *CHEMICAL ABSTRACTS*, 1841081-37-5
- *CHEMICAL ABSTRACTS*, 5326-23-8
- *CHEMICAL ABSTRACTS*, 1394117-24-8
- *CHEMICAL ABSTRACTS*, 57381-37-0
- *CHEMICAL ABSTRACTS*, 1375325-71-5
- *CHEMICAL ABSTRACTS*, 7560-83-0
- *CHEMICAL ABSTRACTS*, 57381-49-4
- *CHEMICAL ABSTRACTS*, 1227601-71-9
- *CHEMICAL ABSTRACTS*, 16640-68-9
- *CHEMICAL ABSTRACTS*, 1314538-55-0
- *CHEMICAL ABSTRACTS*, 1651823-59-4
- *CHEMICAL ABSTRACTS*, 55304-73-9
- *CHEMICAL ABSTRACTS*, 137628-17-2
- *CHEMICAL ABSTRACTS*, 1189513-50-5
- *CHEMICAL ABSTRACTS*, 190141-99-2
- *CHEMICAL ABSTRACTS*, 16982-21-1
- *CHEMICAL ABSTRACTS*, 1251012-01-7
- *CHEMICAL ABSTRACTS*, 657428-42-7
- *CHEMICAL ABSTRACTS*, 72788-94-4
- *CHEMICAL ABSTRACTS*, 69816-38-2
- *CHEMICAL ABSTRACTS*, 4487-50-7
- *CHEMICAL ABSTRACTS*, 719310-31-3
- *CHEMICAL ABSTRACTS*, 142166-01-6
- *CHEMICAL ABSTRACTS*, 67515-76-8
- *CHEMICAL ABSTRACTS*, 56621-90-0
- *CHEMICAL ABSTRACTS*, 67567-26-4
- *CHEMICAL ABSTRACTS*, 1126650-66-5
- *CHEMICAL ABSTRACTS*, 174349-93-0
- *CHEMICAL ABSTRACTS*, 13325-10-5
- *CHEMICAL ABSTRACTS*, 4799-68-2
- *CHEMICAL ABSTRACTS*, 737000-77-0
- *CHEMICAL ABSTRACTS*, 496807-97-7
- *CHEMICAL ABSTRACTS*, 158602-43-8
- *CHEMICAL ABSTRACTS*, 2356-16-3
- *CHEMICAL ABSTRACTS*, 26218-78-0
- *CHEMICAL ABSTRACTS*, 114214-49-2
- *CHEMICAL ABSTRACTS*, 37622-90-5
- *CHEMICAL ABSTRACTS*, 314741-39-4
- *CHEMICAL ABSTRACTS*, 53844-02-3
- *CHEMICAL ABSTRACTS*, 438631-77-7
- *CHEMICAL ABSTRACTS*, 1217650-60-6
- *CHEMICAL ABSTRACTS*, 1217791-74-6
- *CHEMICAL ABSTRACTS*, 144100-07-2
- *CHEMICAL ABSTRACTS*, 72707-66-5
- *CHEMICAL ABSTRACTS*, 10029-04-6
- *CHEMICAL ABSTRACTS*, 867-13-0
- *CHEMICAL ABSTRACTS*, 123387-72-4
- *CHEMICAL ABSTRACTS*, 148992-43-2
- *CHEMICAL ABSTRACTS*, 1117-71-1
- *CHEMICAL ABSTRACTS*, 228244-20-0
- *CHEMICAL ABSTRACTS*, 228244-04-0
- *CHEMICAL ABSTRACTS*, 73323-65-6
- *CHEMICAL ABSTRACTS*, 59936-29-7
- *CHEMICAL ABSTRACTS*, 1260593-39-2
- *CHEMICAL ABSTRACTS*, 107020-12-2
- *CHEMICAL ABSTRACTS*, 814-68-6
- *CHEMICAL ABSTRACTS*, 1251011-05-8
- *CHEMICAL ABSTRACTS*, 478647-20-0

- *CHEMICAL ABSTRACTS*, 1159825-34-9
- *CHEMICAL ABSTRACTS*, 1148044-31-8
- *CHEMICAL ABSTRACTS*, 236406-56-7
- *CHEMICAL ABSTRACTS*, 848133-35-7
- *CHEMICAL ABSTRACTS*, 1202800-68-7
- *CHEMICAL ABSTRACTS*, 1197294-80-6
- *CHEMICAL ABSTRACTS*, 327030-39-7
- *CHEMICAL ABSTRACTS*, 331767-56-7
- *CHEMICAL ABSTRACTS*, 907544-17-6
- *CHEMICAL ABSTRACTS*, 86447-11-2
- *CHEMICAL ABSTRACTS*, 555-16-8
- *CHEMICAL ABSTRACTS*, 160538-51-2
- *CHEMICAL ABSTRACTS*, 142-25-6
- *CHEMICAL ABSTRACTS*, 137076-22-3
- *CHEMICAL ABSTRACTS*, 101385-93-7
- *CHEMICAL ABSTRACTS*, 1309207-05-3
- *CHEMICAL ABSTRACTS*, 79-41-4
- *CHEMICAL ABSTRACTS*, 471-25-0
- *CHEMICAL ABSTRACTS*, 156866-52-3
- *CHEMICAL ABSTRACTS*, 6018-28-6
- *CHEMICAL ABSTRACTS*, 177947-96-5
- *CHEMICAL ABSTRACTS*, 142374-19-4
- *CHEMICAL ABSTRACTS*, 79099-07-3
- *CHEMICAL ABSTRACTS*, 118156-93-7
- *CHEMICAL ABSTRACTS*, 157701-72-9
- *CHEMICAL ABSTRACTS*, 101682-68-2
- *CHEMICAL ABSTRACTS*, 53199-31-8
- *CHEMICAL ABSTRACTS*, 23147-58-2
- *CHEMICAL ABSTRACTS*, 1255666-48-8
- *CHEMICAL ABSTRACTS*, 1808997-73-0
- *CHEMICAL ABSTRACTS*, 147081-29-6
- *CHEMICAL ABSTRACTS*, 1251017-66-9
- *CHEMICAL ABSTRACTS*, 201162-53-0
- *CHEMICAL ABSTRACTS*, 134003-84-2
- *CHEMICAL ABSTRACTS*, 131922-05-9
- *CHEMICAL ABSTRACTS*, 29684-56-8
- *CHEMICAL ABSTRACTS*, 199296-40-7
- *CHEMICAL ABSTRACTS*, 1440960-67-7
- *CHEMICAL ABSTRACTS*, 171549-92-1
- **R.A COPELAND**. Evaluation of Enzyme Inhibitors in Drug Discovery (Ch9). Wiley, 2013